(19) Europäisches Patentamt
European Patent Office
Office européen des brevets

(11) EP 1 490 491 B1

(12) FASCICULE DE BREVET EUROPEEN

(45) Date de publication et mention de la délivrance du brevet:
**14.03.2007 Bulletin 2007/11**

(21) Numéro de dépôt: **03740540.4**

(22) Date de dépôt: **28.03.2003**

(51) Int Cl.:
*C12N 15/11 (2006.01)* *C07K 14/35 (2006.01)*
*C30B 7/00 (2006.01)* *C12Q 1/68 (2006.01)*
*G01N 33/68 (2006.01)* *C12N 9/02 (2006.01)*

(86) Numéro de dépôt international:
**PCT/FR2003/000990**

(87) Numéro de publication internationale:
**WO 2003/082911 (09.10.2003 Gazette 2003/41)**

# (54) UTILISATION DE LA PROTEINE MABA (FABG1) DE MYCOBACTERIUM TUBERCULOSIS POUR LA CONCEPTION ET LE CRIBLAGE D'ANTIBIOTIQUES

VERWENDUNG VON PROTEIN MABA (FABG1) AUS MYCOBACTERIUM TUBERCULOSIS ZUR HERSTELLUNG UND NACHWEIS VON ANTIBIOTIKA

USE OF THE PROTEIN MABA (FABG1) OF I MYCOBACTERIUM TUBERCULOSIS /I FOR DESIGNING AND SCREENING ANTIBIOTICS

(84) Etats contractants désignés:
**AT BE BG CH CY CZ DE DK EE ES FI FR GB GR HU IE IT LI LU MC NL PT RO SE SI SK TR**

(30) Priorité: **29.03.2002 FR 0204018**

(43) Date de publication de la demande:
**29.12.2004 Bulletin 2004/53**

(73) Titulaires:
- **CENTRE NATIONAL DE LA RECHERCHE SCIENTIFIQUE (CNRS)
75794 Paris Cedex 16 (FR)**
- **INSTITUT NATIONAL DE LA SANTE ET DE LA RECHERCHE MEDICALE (INSERM)
75654 Paris Cédex 13 (FR)**
- **Université de Montpellier I
34006 Montpellier Cedex 1 (FR)**

(72) Inventeurs:
- **QUEMARD, Annaïk
F-31450 Montgiscard (FR)**
- **LABESSE, Gilles
F-34000 Montpellier (FR)**
- **DAFFE, Mamadou
F-31400 Toulouse (FR)**
- **MARRAKCHI, Hedia
314 00 Toulouse (FR)**
- **DOUGUET, Dominique
F-34090 Montpellier (FR)**
- **COHEN-GONSAUD, Martin
F-30000 Nimes (FR)**
- **DUCASSE, Stéphanie
F-31400 Toulouse (FR)**

(74) Mandataire: **Grosset-Fournier, Chantal Catherine
Grosset-Fournier & Demachy,
54, rue Saint-Lazare
75009 Paris (FR)**

(56) Documents cités:
**WO-A-94/26312** **WO-A-94/26765**

- **M COHEN-GONSAUD ET AL.: "Crystal structure and fluorescence spectroscopy of MabA from Mycobacterium tuberculosis, a reductase involved in long-chain fatty acid biosynthesis" BIOPHYSICAL JOURNAL, vol. 82, no. 1 part 2, janvier 2002 (2002-01), page 453A XP002229792 NEW YORK, US, US ISSN: 0006-3495**
- **A BANERJEE ET AL.: "The mabA gene from the inhA operon of Mycobacterium tuberculosis encodes a 3-ketoacyl reductase that fails to confer isoniazid resistance" MICROBIOLOGY, vol. 144, no. 10, octobre 1998 (1998-10), pages 2697-2704, XP002229443 CONSULTANTS BUREAU, NEW YORK, NY, US ISSN: 0026-2617**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

(Cont. page suivante)

- **DATABASE BIOSIS [en ligne] BIOSCIENCES INFORMATION SERVICE, PHILADELPHIA, PA, US; 2000 P KIEPIELA ET AL: "Genomic mutations in the katG, inhA and aphC are useful for the prediction of isoniazid resistance in Mycobacterium tuberculosis isolates from Kwazulu Natal, South Africa" Database accession no. PREV200000182099 XP002229447 & TUBERCLE AND LUNG DISEASE, vol. 80, no. 1, 2000, pages 47-56,**
- **A C PRICE ET AL.: "Structure of beta-ketoacyl-[acyl carrier protein] reductase from Escherichia coli: negative cooperativity and its structural basis" BIOCHEMISTRY., vol. 40, no. 43, 2001, pages 12772-12781, XP002229444 AMERICAN CHEMICAL SOCIETY. EASTON, PA., US ISSN: 0006-2960**
- **H MARRAKCHI ET AL.: "MabA (FabG1), a Mycobacterium tuberculosis protein involved in the long-chain fatty acid elongation system FAS-II" MICROBIOLOGY, vol. 148, no. 4, avril 2002 (2002-04), pages 951-960, XP002229445 CONSULTANTS BUREAU, NEW YORK, NY, US ISSN: 0026-2617**
- **M COHEN-GONSAUD ET AL.: "Crystal structure of MabA from Mycobacterium tuberculosis, a reductase involved in long-chain fatty acid biosynthesis" JOURNAL OF MOLECULAR BIOLOGY, vol. 320, no. 2, 5 juillet 2002 (2002-07-05), pages 249-261, XP002229446 LONDON, GB ISSN: 0022-2836**

LISTE DE SEQUENCES

<110> CNRS

<120> UTILISATION DE LA PROTEINE MabA (FABG1) DE MYCOBACTERIUM TUBERCULOSIS POUR LA CONCEPTION ET LE CRIBLAGE D'ANTIBIOTIQUES

<130> IFB 02 AY CNR MABA

<140> FR0204018
<141> 2002-03-29

<160> 41

<170> PatentIn Ver. 2.1

<210> 1
<211> 247
<212> PRT
<213> Mycobacterium tuberculosis

<400> 1

```
Met Thr Ala Thr Ala Thr Glu Gly Ala Lys Pro Pro Phe Val Ser Arg
 1               5                  10                  15

Ser Val Leu Val Thr Gly Gly Asn Arg Gly Ile Gly Leu Ala Ile Ala
            20                  25                  30

Gln Arg Leu Ala Ala Asp Gly His Lys Val Ala Val Thr His Arg Gly
        35                  40                  45

Ser Gly Ala Pro Lys Gly Leu Phe Gly Val Glu Cys Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Ser
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
                245
```

<210> 2
<211> 741
<212> ADN
<213> Séquence artificielle

<220>
<223> Description de la séquence artificielle: séquence codant pour une protéine dérivée de la protéine MabA de Mycobacterium tuberculosis

<220>
<221> CDS
<222> (1) .. (741)

<400> 2

```
atg act gcc aca gcc act gaa ggg gcc aaa ccc cca ttc gta tcc cgt     48
Met Thr Ala Thr Ala Thr Glu Gly Ala Lys Pro Pro Phe Val Ser Arg
1               5                   10                  15

tca gtc ctg gtt acc gga gga aac cgg ggg atc ggg ctg gcg atc gca     96
Ser Val Leu Val Thr Gly Gly Asn Arg Gly Ile Gly Leu Ala Ile Ala
            20                  25                  30

cag cgg ctg gct gcc gac ggc cac aag gtg gcc gtc acc cac cgt gga    144
Gln Arg Leu Ala Ala Asp Gly His Lys Val Ala Val Thr His Arg Gly
        35                  40                  45

tcc gga gcg cca aag ggg ctg ttt ggc gtc gaa gtt gac gtc acc gac    192
Ser Gly Ala Pro Lys Gly Leu Phe Gly Val Glu Val Asp Val Thr Asp
    50                  55                  60

agc gac gcc gtc gat cgc gcc ttc acg gcg gta gaa gag cac cag ggt    240
Ser Asp Ala Val Asp Arg Ala Phe Thr Ala Val Glu Glu His Gln Gly
65                  70                  75                  80

ccg gtc gag gtg ctg gtg tcc aac gcc ggc cta tcc gcg gac gca ttc    288
Pro Val Glu Val Leu Val Ser Asn Ala Gly Leu Ser Ala Asp Ala Phe
                85                  90                  95

ctc atg cgg atg acc gag gaa aag ttc gag aag gtc atc aac gcc aac    336
Leu Met Arg Met Thr Glu Glu Lys Phe Glu Lys Val Ile Asn Ala Asn
            100                 105                 110

ctc acc ggg gcg ttc cgg gtg gct caa cgg gca tcg cgc agc atg cag    384
Leu Thr Gly Ala Phe Arg Val Ala Gln Arg Ala Ser Arg Ser Met Gln
        115                 120                 125
```

```
cgc aac aaa ttc ggt cga atg ata ttc ata ggt tcg gtc tcc ggc agc     432
Arg Asn Lys Phe Gly Arg Met Ile Phe Ile Gly Ser Val Ser Gly Ser
    130                 135                 140

tgg ggc atc ggc aac cag gcc aac tac gca gcc tcc aag gcc gga gtg     480
Trp Gly Ile Gly Asn Gln Ala Asn Tyr Ala Ala Ser Lys Ala Gly Val
145                 150                 155                 160

att ggc atg gcc cgc tcg atc gcc cgc gag ctg tcg aag gca aac gtg     528
Ile Gly Met Ala Arg Ser Ile Ala Arg Glu Leu Ser Lys Ala Asn Val
                165                 170                 175

acc gcg aat gtg gtg gcc ccg ggc tac atc gac acc gat atg acc cgc     576
Thr Ala Asn Val Val Ala Pro Gly Tyr Ile Asp Thr Asp Met Thr Arg
            180                 185                 190

gcg ctg gat gag cgg att cag cag ggg gcg ctg caa ttt atc cca gcg     624
Ala Leu Asp Glu Arg Ile Gln Gln Gly Ala Leu Gln Phe Ile Pro Ala
        195                 200                 205

aag cgg gtc ggc acc ccc gcc gag gtc gcc ggg gtg gtc agc ttc ctg     672
Lys Arg Val Gly Thr Pro Ala Glu Val Ala Gly Val Val Ser Phe Leu
    210                 215                 220

gct tcc gag gat gcg agc tat atc tcc ggt gcg gtc atc ccg gtc gac     720
Ala Ser Glu Asp Ala Ser Tyr Ile Ser Gly Ala Val Ile Pro Val Asp
225                 230                 235                 240

ggc ggc atg ggt atg ggc cac                                         741
Gly Gly Met Gly Met Gly His
                245
```

<210> 3
<211> 247
<212> PRT
<213> Séquence artificielle
<223> Description de la séquence artificielle: séquence codant pour une protéine dérivée de la protéine MabA de Mycobacterium tuberculosis

<400> 3

```
Met Thr Ala Thr Ala Thr Glu Gly Ala Lys Pro Pro Phe Val Ser Arg
1               5                   10                  15

Ser Val Leu Val Thr Gly Gly Asn Arg Gly Ile Gly Leu Ala Ile Ala
            20                  25                  30

Gln Arg Leu Ala Ala Asp Gly His Lys Val Ala Val Thr His Arg Gly
        35                  40                  45

Ser Gly Ala Pro Lys Gly Leu Phe Gly Val Glu Val Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Ser
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
                245
```

<210> 4
<211> 741
<212> ADN
<213> Séquence artificielle

<220>
<223> Description de la séquence artificielle: séquence codant pour une protéine dérivée de la protéine MabA de

Mycobacterium tuberculosis

<220>
<221> CDS
<222> (1)..(741)

<400> 4

```
atg act gcc aca gcc act gaa ggg gcc aaa ccc cca ttc gta tcc cgt      48
Met Thr Ala Thr Ala Thr Glu Gly Ala Lys Pro Pro Phe Val Ser Arg
 1               5                  10                  15

tca gtc ctg gtt acc gga gga aac cgg ggg atc ggg ctg gcg atc gca      96
Ser Val Leu Val Thr Gly Gly Asn Arg Gly Ile Gly Leu Ala Ile Ala
            20                  25                  30

cag cgg ctg gct gcc gac ggc cac aag gtg gcc gtc acc cac cgt gga     144
Gln Arg Leu Ala Ala Asp Gly His Lys Val Ala Val Thr His Arg Gly
        35                  40                  45
```

```
tcc gga gcg cca aag ggg ctg ttt ggc gtc gaa tgt gac gtc acc gac     192
Ser Gly Ala Pro Lys Gly Leu Phe Gly Val Glu Cys Asp Val Thr Asp
     50                  55                  60

agc gac gcc gtc gat cgc gcc ttc acg gcg gta gaa gag cac cag ggt     240
Ser Asp Ala Val Asp Arg Ala Phe Thr Ala Val Glu Glu His Gln Gly
 65                  70                  75                  80

ccg gtc gag gtg ctg gtg tcc aac gcc ggc cta tcc gcg gac gca ttc     288
Pro Val Glu Val Leu Val Ser Asn Ala Gly Leu Ser Ala Asp Ala Phe
                 85                  90                  95

ctc atg cgg atg acc gag gaa aag ttc gag aag gtc atc aac gcc aac     336
Leu Met Arg Met Thr Glu Glu Lys Phe Glu Lys Val Ile Asn Ala Asn
            100                 105                 110

ctc acc ggg gcg ttc cgg gtg gct caa cgg gca tcg cgc agc atg cag     384
Leu Thr Gly Ala Phe Arg Val Ala Gln Arg Ala Ser Arg Ser Met Gln
        115                 120                 125

cgc aac aaa ttc ggt cga atg ata ttc ata ggt tcg gtc tcc ggc ctc     432
Arg Asn Lys Phe Gly Arg Met Ile Phe Ile Gly Ser Val Ser Gly Leu
    130                 135                 140

tgg ggc atc ggc aac cag gcc aac tac gca gcc tcc aag gcc gga gtg     480
Trp Gly Ile Gly Asn Gln Ala Asn Tyr Ala Ala Ser Lys Ala Gly Val
145                 150                 155                 160

att ggc atg gcc cgc tcg atc gcc cgc gag ctg tcg aag gca aac gtg     528
Ile Gly Met Ala Arg Ser Ile Ala Arg Glu Leu Ser Lys Ala Asn Val
                165                 170                 175

acc gcg aat gtg gtg gcc ccg ggc tac atc gac acc gat atg acc cgc     576
Thr Ala Asn Val Val Ala Pro Gly Tyr Ile Asp Thr Asp Met Thr Arg
            180                 185                 190

gcg ctg gat gag cgg att cag cag ggg gcg ctg caa ttt atc cca gcg     624
Ala Leu Asp Glu Arg Ile Gln Gln Gly Ala Leu Gln Phe Ile Pro Ala
        195                 200                 205

aag cgg gtc ggc acc ccc gcc gag gtc gcc ggg gtg gtc agc ttc ctg     672
Lys Arg Val Gly Thr Pro Ala Glu Val Ala Gly Val Val Ser Phe Leu
    210                 215                 220

gct tcc gag gat gcg agc tat atc tcc ggt gcg gtc atc ccg gtc gac     720
Ala Ser Glu Asp Ala Ser Tyr Ile Ser Gly Ala Val Ile Pro Val Asp
225                 230                 235                 240

ggc ggc atg ggt atg ggc cac                                         741
Gly Gly Met Gly Met Gly His
                245
```

<210> 5
<211> 247
<212 PRT
<213> Séquence artificielle
<223> Description de la séquence artificielle: séquence codant pour une protéine dérivée de la protéine MabA de

Mycobacterium tuberculosis

<400> 5

```
Met Thr Ala Thr Ala Thr Glu Gly Ala Lys Pro Pro Phe Val Ser Arg
1               5                   10                  15

Ser Val Leu Val Thr Gly Gly Asn Arg Gly Ile Gly Leu Ala Ile Ala
            20                  25                  30

Gln Arg Leu Ala Ala Asp Gly His Lys Val Ala Val Thr His Arg Gly
        35                  40                  45

Ser Gly Ala Pro Lys Gly Leu Phe Gly Val Glu Cys Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Leu
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
                245
```

<210> 6
<211> 741
<212> ADN
<213> Séquence artificielle

<220>

<223> Description de la séquence artificielle: séquence codant pour une protéine dérivée de la protéine MabA de Mycobacterium tuberculosis

<220>
<221> CDS
<222> (1) .. (741)

<400> 6

```
atg act gcc aca gcc act gaa ggg gcc aaa ccc cca ttc gta tcc cgt    48
Met Thr Ala Thr Ala Thr Glu Gly Ala Lys Pro Pro Phe Val Ser Arg
1               5                   10                  15

tca gtc ctg gtt acc gga gga aac cgg ggg atc ggg ctg gcg atc gca    96
Ser Val Leu Val Thr Gly Gly Asn Arg Gly Ile Gly Leu Ala Ile Ala
            20                  25                  30

cag cgg ctg gct gcc gac ggc cac aag gtg gcc gtc acc cac cgt gga    144
Gln Arg Leu Ala Ala Asp Gly His Lys Val Ala Val Thr His Arg Gly
        35                  40                  45

tcc gga gcg cca aag ggg ctg ttt ggc gtc gaa gtt gac gtc acc gac    192
Ser Gly Ala Pro Lys Gly Leu Phe Gly Val Glu Val Asp Val Thr Asp
    50                  55                  60

agc gac gcc gtc gat cgc gcc ttc acg gcg gta gaa gag cac cag ggt    240
Ser Asp Ala Val Asp Arg Ala Phe Thr Ala Val Glu Glu His Gln Gly
65                  70                  75                  80

ccg gtc gag gtg ctg gtg tcc aac gcc ggc cta tcc gcg gac gca ttc    288
Pro Val Glu Val Leu Val Ser Asn Ala Gly Leu Ser Ala Asp Ala Phe
                85                  90                  95

ctc atg cgg atg acc gag gaa aag ttc gag aag gtc atc aac gcc aac    336
Leu Met Arg Met Thr Glu Glu Lys Phe Glu Lys Val Ile Asn Ala Asn
            100                 105                 110

ctc acc ggg gcg ttc cgg gtg gct caa cgg gca tcg cgc agc atg cag    384
Leu Thr Gly Ala Phe Arg Val Ala Gln Arg Ala Ser Arg Ser Met Gln
        115                 120                 125

cgc aac aaa ttc ggt cga atg ata ttc ata ggt tcg gtc tcc ggc ctc    432
Arg Asn Lys Phe Gly Arg Met Ile Phe Ile Gly Ser Val Ser Gly Leu
    130                 135                 140

tgg ggc atc ggc aac cag gcc aac tac gca gcc tcc aag gcc gga gtg    480
Trp Gly Ile Gly Asn Gln Ala Asn Tyr Ala Ala Ser Lys Ala Gly Val
145                 150                 155                 160

att ggc atg gcc cgc tcg atc gcc cgc gag ctg tcg aag gca aac gtg    528
Ile Gly Met Ala Arg Ser Ile Ala Arg Glu Leu Ser Lys Ala Asn Val
                165                 170                 175

acc gcg aat gtg gtg gcc ccg ggc tac atc gac acc gat atg acc cgc    576
Thr Ala Asn Val Val Ala Pro Gly Tyr Ile Asp Thr Asp Met Thr Arg
            180                 185                 190

gcg ctg gat gag cgg att cag cag ggg gcg ctg caa ttt atc cca gcg    624
Ala Leu Asp Glu Arg Ile Gln Gln Gly Ala Leu Gln Phe Ile Pro Ala
        195                 200                 205

aag cgg gtc ggc acc ccc gcc gag gtc gcc ggg gtg gtc agc ttc ctg    672
Lys Arg Val Gly Thr Pro Ala Glu Val Ala Gly Val Val Ser Phe Leu
    210                 215                 220
```

```
gct tcc gag gat gcg agc tat atc tcc ggt gcg gtc atc ccg gtc gac    720
Ala Ser Glu Asp Ala Ser Tyr Ile Ser Gly Ala Val Ile Pro Val Asp
225                 230                 235                 240

ggc ggc atg ggt atg ggc cac                                        741
Gly Gly Met Gly Met Gly His
                245
```

<210> 7
<211> 247
<212> PRT
<213> Séquence artificielle
<223> Description de la séquence artificielle: séquence codant pour une protéine dérivée de la protéine MabA de Mycobacterium tuberculosis

<400> 7

Met Thr Ala Thr Ala Thr Glu Gly Ala Lys Pro Pro Phe Val Ser Arg
1 5 10 15

Ser Val Leu Val Thr Gly Gly Asn Arg Gly Ile Gly Leu Ala Ile Ala
20 25 30

Gln Arg Leu Ala Ala Asp Gly His Lys Val Ala Val Thr His Arg Gly
35 40 45

Ser Gly Ala Pro Lys Gly Leu Phe Gly Val Glu Val Asp Val Thr Asp
50 55 60

Ser Asp Ala Val Asp Arg Ala Phe Thr Ala Val Glu Glu His Gln Gly
65 70 75 80

Pro Val Glu Val Leu Val Ser Asn Ala Gly Leu Ser Ala Asp Ala Phe
85 90 95

Leu Met Arg Met Thr Glu Glu Lys Phe Glu Lys Val Ile Asn Ala Asn
100 105 110

Leu Thr Gly Ala Phe Arg Val Ala Gln Arg Ala Ser Arg Ser Met Gln
115 120 125

Arg Asn Lys Phe Gly Arg Met Ile Phe Ile Gly Ser Val Ser Gly Leu
130 135 140

Trp Gly Ile Gly Asn Gln Ala Asn Tyr Ala Ala Ser Lys Ala Gly Val
145 150 155 160

Ile Gly Met Ala Arg Ser Ile Ala Arg Glu Leu Ser Lys Ala Asn Val
165 170 175

Thr Ala Asn Val Val Ala Pro Gly Tyr Ile Asp Thr Asp Met Thr Arg
180 185 190

Ala Leu Asp Glu Arg Ile Gln Gln Gly Ala Leu Gln Phe Ile Pro Ala
195 200 205

Lys Arg Val Gly Thr Pro Ala Glu Val Ala Gly Val Val Ser Phe Leu
210 215 220

Ala Ser Glu Asp Ala Ser Tyr Ile Ser Gly Ala Val Ile Pro Val Asp
225 230 235 240

Gly Gly Met Gly Met Gly His
245

<210> 8
<211> 247
<212> PRT
<213> Séquence artificielle

<220>
<223> Mutant de la protéine MabA

<220>
<221> MISC_FEATURE
<222> (139)..(139)
<223> Ala ou Ser

<400> 8

```
Met Thr Ala Thr Ala Thr Glu Gly Ala Lys Pro Pro Phe Val Ser Arg
1               5                   10                  15

Ser Val Leu Val Thr Gly Gly Asn Arg Gly Ile Gly Leu Ala Ile Ala
            20                  25                  30

Gln Arg Leu Ala Ala Asp Gly His Lys Val Ala Val Thr His Arg Gly
        35                  40                  45

Ser Gly Ala Pro Lys Gly Leu Phe Gly Val Glu Val Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Xaa Ser Val Ser Gly Leu
    130                 135                 140
```

```
Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
                245
```

<210> 9
<211> 247
<212> PRT
<213> Séquence artificielle

<220>
<223> Mutant de la protéine MabA

<220>
<221> MISC_FEATURE
<222> (24)..(24)
<223> Asp ou Glu

<220>
<221> MISC_FEATURE
<222> (46)..(46)
<223> Asp ou His

<400> 9

```
Met Thr Ala Thr Ala Thr Glu Gly Ala Lys Pro Pro Phe Val Ser Arg
1               5                   10                  15

Ser Val Leu Val Thr Gly Gly Xaa Arg Gly Ile Gly Leu Ala Ile Ala
            20                  25                  30

Gln Arg Leu Ala Ala Asp Gly His Lys Val Ala Val Thr Xaa Arg Gly
        35                  40                  45
```

```
Ser Gly Ala Pro Lys Gly Leu Phe Gly Val Glu Cys Asp Val Thr Asp
    50                  55                      60

Ser Asp Ala Val Asp Arg Ala Phe Thr Ala Val Glu Glu His Gln Gly
65                  70                      75                  80

Pro Val Glu Val Leu Val Ser Asn Ala Gly Leu Ser Ala Asp Ala Phe
                85                  90                      95

Leu Met Arg Met Thr Glu Glu Lys Phe Glu Lys Val Ile Asn Ala Asn
            100                 105                     110

Leu Thr Gly Ala Phe Arg Val Ala Gln Arg Ala Ser Arg Ser Met Gln
        115                 120                     125

Arg Asn Lys Phe Gly Arg Met Ile Phe Ile Gly Ser Val Ser Gly Ser
    130                 135                     140

Trp Gly Ile Gly Asn Gln Ala Asn Tyr Ala Ala Ser Lys Ala Gly Val
145                 150                     155                 160

Ile Gly Met Ala Arg Ser Ile Ala Arg Glu Leu Ser Lys Ala Asn Val
                165                     170                     175

Thr Ala Asn Val Val Ala Pro Gly Tyr Ile Asp Thr Asp Met Thr Arg
            180                     185                     190

Ala Leu Asp Glu Arg Ile Gln Gln Gly Ala Leu Gln Phe Ile Pro Ala
        195                     200                     205

Lys Arg Val Gly Thr Pro Ala Glu Val Ala Gly Val Val Ser Phe Leu
    210                     215                     220

Ala Ser Glu Asp Ala Ser Tyr Ile Ser Gly Ala Val Ile Pro Val Asp
225                     230                     235                     240

Gly Gly Met Gly Met Gly His
                245
```

<210> 10
<211> 247
<212> PRT
<213> Séquence artificielle

<220>
<223> Mutant de la protéine MabA

<220>
<221> MISC_FEATURE
<222> (24)..(24)
<223> Asp ou Glu

<220>
<221> MISC_FEATURE
<222> (46)..(46)
<223> Asp ou His

<400> 10

```
Met Thr Ala Thr Ala Thr Glu Gly Ala Lys Pro Pro Phe Val Ser Arg
1               5                   10                  15

Ser Val Leu Val Thr Gly Gly Xaa Arg Gly Ile Gly Leu Ala Ile Ala
            20                  25                  30

Gln Arg Leu Ala Ala Asp Gly His Lys Val Ala Val Thr Xaa Arg Gly
        35                  40                  45

Ser Gly Ala Pro Lys Gly Leu Phe Gly Val Glu Val Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Ser
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190
```

Ala Leu Asp Glu Arg Ile Gln Gln Gly Ala Leu Gln Phe Ile Pro Ala
195 200 205

Lys Arg Val Gly Thr Pro Ala Glu Val Ala Gly Val Val Ser Phe Leu
210 215 220

Ala Ser Glu Asp Ala Ser Tyr Ile Ser Gly Ala Val Ile Pro Val Asp
225 230 235 240

Gly Gly Met Gly Met Gly His
245

<210> 11
<211> 247
<212> PRT
<213> Séquence artificielle

<220>
<223> Mutant de la protéine de la protéine MabA

<220>
<221> MISC_FEATURE
<222> (24). .(24)
<223> Asp ou Glu

<220>
<221> MISC_FEATURE
<222> (46)..(46)
<223> Asp ou His

<400> 11

Met Thr Ala Thr Ala Thr Glu Gly Ala Lys Pro Pro Phe Val Ser Arg
1               5                   10                  15

Ser Val Leu Val Thr Gly Gly Xaa Arg Gly Ile Gly Leu Ala Ile Ala
            20                  25                  30

Gln Arg Leu Ala Ala Asp Gly His Lys Val Ala Val Thr Xaa Arg Gly
        35                  40                  45

Ser Gly Ala Pro Lys Gly Leu Phe Gly Val Glu Cys Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
100 105 110

Leu Thr Gly Ala Phe Arg Val Ala Gln Arg Ala Ser Arg Ser Met Gln
115 120 125

Arg Asn Lys Phe Gly Arg Met Ile Phe Ile Gly Ser Val Ser Gly Leu
130 135 140

Trp Gly Ile Gly Asn Gln Ala Asn Tyr Ala Ala Ser Lys Ala Gly Val
145 150 155 160

Ile Gly Met Ala Arg Ser Ile Ala Arg Glu Leu Ser Lys Ala Asn Val
165 170 175

Thr Ala Asn Val Val Ala Pro Gly Tyr Ile Asp Thr Asp Met Thr Arg
180 185 190

Ala Leu Asp Glu Arg Ile Gln Gln Gly Ala Leu Gln Phe Ile Pro Ala
195 200 205

Lys Arg Val Gly Thr Pro Ala Glu Val Ala Gly Val Val Ser Phe Leu
210 215 220

Ala Ser Glu Asp Ala Ser Tyr Ile Ser Gly Ala Val Ile Pro Val Asp
225 230 235 240

Gly Gly Met Gly Met Gly His
245

<210> 12
<211> 247
<212> PRT
<213> Séquence artificielle

<220>
<223> Mutant de la protéine MabA

<220>
<221> MISC_FEATURE
<222> (24)..(24)
<223> Asp ou Glu

<220>
<221> MISC_FEATURE
<222> (46).. (46)
<223> Asp ou His

<400> 12

Met Thr Ala Thr Ala Thr Glu Gly Ala Lys Pro Pro Phe Val Ser Arg
1               5                   10                  15

Ser Val Leu Val Thr Gly Gly Xaa Arg Gly Ile Gly Leu Ala Ile Ala
            20                  25                  30

Gln Arg Leu Ala Ala Asp Gly His Lys Val Ala Val Thr Xaa Arg Gly
        35                  40                  45

Ser Gly Ala Pro Lys Gly Leu Phe Gly Val Glu Val Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Leu
    130                 135                 140

Trp Gly Ile Gly Asn Gln Ala Asn Tyr Ala Ala Ser Lys Ala Gly Val
145                 150                 155                 160

Ile Gly Met Ala Arg Ser Ile Ala Arg Glu Leu Ser Lys Ala Asn Val
                165                 170                 175

Thr Ala Asn Val Val Ala Pro Gly Tyr Ile Asp Thr Asp Met Thr Arg
            180                 185                 190

Ala Leu Asp Glu Arg Ile Gln Gln Gly Ala Leu Gln Phe Ile Pro Ala
        195                 200                 205

Lys Arg Val Gly Thr Pro Ala Glu Val Ala Gly Val Val Ser Phe Leu
    210                 215                 220

Ala Ser Glu Asp Ala Ser Tyr Ile Ser Gly Ala Val Ile Pro Val Asp
225                 230                 235                 240

Gly Gly Met Gly Met Gly His
245

<210> 13
<211> 247
<212> PRT
<213> Séquence artificielle

<220>
<223> Mutant de la protéine MabA

<220>
<221> MISC_FEATURE
<222> (24)..(24)
<223> Asp ou Glu

<220>
<221> MISC_FEATURE
<222> (46).. (46)
<223> Asp ou His

<400> 13

Met Thr Ala Thr Ala Thr Glu Gly Ala Lys Pro Pro Phe Val Ser Arg
1               5                   10                  15

Ser Val Leu Val Thr Gly Gly Xaa Arg Gly Ile Gly Leu Ala Ile Ala
            20                  25                  30

Gln Arg Leu Ala Ala Asp Gly His Lys Val Ala Val Thr Xaa Arg Gly
        35                  40                  45

Ser Gly Ala Pro Lys Gly Leu Phe Gly Val Glu Val Asp Val Thr Asp
    50                  55                  60

Ser Asp Ala Val Asp Arg Ala Phe Thr Ala Val Glu Glu His Gln Gly
65                  70                  75                  80

Pro Val Glu Val Leu Val Ser Asn Ala Gly Leu Ser Ala Asp Ala Phe
                85                  90                  95

Leu Met Arg Met Thr Glu Glu Lys Phe Glu Lys Val Ile Asn Ala Asn
            100                 105                 110

Leu Thr Gly Ala Phe Arg Val Ala Gln Arg Ala Ser Arg Ser Met Gln
        115                 120                 125

Arg Asn Lys Phe Gly Arg Met Ile Phe Ile Gly Ser Val Ser Gly Leu

130 135 140

Trp Gly Ile Gly Asn Gln Ala Asn Tyr Ala Ala Ser Lys Ala Gly Val
145 150 155 160

Ile Gly Met Ala Arg Ser Ile Ala Arg Glu Leu Ser Lys Ala Asn Val
165 170 175

Thr Ala Asn Val Val Ala Pro Gly Tyr Ile Asp Thr Asp Met Thr Arg
180 185 190

Ala Leu Asp Glu Arg Ile Gln Gln Gly Ala Leu Gln Phe Ile Pro Ala
195 200 205

Lys Arg Val Gly Thr Pro Ala Glu Val Ala Gly Val Val Ser Phe Leu
210 215 220

Ala Ser Glu Asp Ala Ser Tyr Ile Ser Gly Ala Val Ile Pro Val Asp
225 230 235 240

Gly Gly Met Gly Met Gly His
245

<210> 14
<211> 20
<212> PRT
<213> Séquence artificielle

<220>
<223> Etiquette poly-histidine

<400> 14

Met Gly Ser Ser His His His His His His Ser Ser Gly Leu Val Pro
1 5 10 15

Arg Gly Ser His
20

<210> 15
<211> 267
<212> PRT
<213> Séquence artificielle

<220>
<223> Mutant de la protéine MabA

<400> 15

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Asn Arg Gly Ile Gly
35 40 45

Leu Ala Ile Ala Gln Arg Leu Ala Ala Asp Gly His Lys Val Ala Val
50 55 60

Thr His Arg Gly Ser Gly Ala Pro Lys Gly Leu Phe Gly Val Glu Cys
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Ser Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 16
<211> 267
<212> PRT
<213> Séquence artificielle

<220>
<223> Mutant.de la protéine MabA

<400> 16

```
Met Gly Ser Ser His His His His His His Ser Ser Gly Leu Val Pro
1               5                   10                  15

Arg Gly Ser His Met Thr Ala Thr Ala Thr Glu Gly Ala Lys Pro Pro
            20                  25                  30

Phe Val Ser Arg Ser Val Leu Val Thr Gly Gly Asn Arg Gly Ile Gly
        35                  40                  45

Leu Ala Ile Ala Gln Arg Leu Ala Ala Asp Gly His Lys Val Ala Val
    50                  55                  60

Thr His Arg Gly Ser Gly Ala Pro Lys Gly Leu Phe Gly Val Glu Val
65                  70                  75                  80

Asp Val Thr Asp Ser Asp Ala Val Asp Arg Ala Phe Thr Ala Val Glu
                85                  90                  95

Glu His Gln Gly Pro Val Glu Val Leu Val Ser Asn Ala Gly Leu Ser
            100                 105                 110

Ala Asp Ala Phe Leu Met Arg Met Thr Glu Glu Lys Phe Glu Lys Val
        115                 120                 125

Ile Asn Ala Asn Leu Thr Gly Ala Phe Arg Val Ala Gln Arg Ala Ser
    130                 135                 140

Arg Ser Met Gln Arg Asn Lys Phe Gly Arg Met Ile Phe Ile Gly Ser
145                 150                 155                 160

Val Ser Gly Ser Trp Gly Ile Gly Asn Gln Ala Asn Tyr Ala Ala Ser
                165                 170                 175

Lys Ala Gly Val Ile Gly Met Ala Arg Ser Ile Ala Arg Glu Leu Ser
            180                 185                 190
```

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 17
<211> 267
<212> PRT
<213> Séquence artificielle

<220>
<223> Mutant de la protéine MabA

<400> 17

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Asn Arg Gly Ile Gly
35 40 45

Leu Ala Ile Ala Gln Arg Leu Ala Ala Asp Gly His Lys Val Ala Val
50 55 60

Thr His Arg Gly Ser Gly Ala Pro Lys Gly Leu Phe Gly Val Glu Cys
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val

115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Leu Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 18
<211> 267
<212 > PRT
<213> Séquence artificielle

<220>
<223> Mutant de la protéine MabA

<400> 18

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Asn Arg Gly Ile Gly
35 40 45

Leu Ala Ile Ala Gln Arg Leu Ala Ala Asp Gly His Lys Val Ala Val
50 55 60

Thr His Arg Gly Ser Gly Ala Pro Lys Gly Leu Phe Gly Val Glu Val
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Leu Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 19
<211> 267
<212> PRT

<213> Séquence artificielle

<220>
<223> Mutant de la protéine MabA

<220>
<221> MISC_FEATURE
<222> (44)..(44)
<223> Asp ou Glu

<220>
<221> MISC_FEATURE
<222> (66).. (66)
<223> Asp ou His

<400> 19

Met Gly Ser Ser His His His His His His Ser Ser Gly Leu Val Pro
1               5                   10                  15

Arg Gly Ser His Met Thr Ala Thr Ala Thr Glu Gly Ala Lys Pro Pro
            20                  25                  30

Phe Val Ser Arg Ser Val Leu Val Thr Gly Gly Xaa Arg Gly Ile Gly
        35                  40                  45

Leu Ala Ile Ala Gln Arg Leu Ala Ala Asp Gly His Lys Val Ala Val
    50                  55                  60

Thr Xaa Arg Gly Ser Gly Ala Pro Lys Gly Leu Phe Gly Val Glu Cys
65                  70                  75                  80

Asp Val Thr Asp Ser Asp Ala Val Asp Arg Ala Phe Thr Ala Val Glu
                85                  90                  95

Glu His Gln Gly Pro Val Glu Val Leu Val Ser Asn Ala Gly Leu Ser
            100                 105                 110

Ala Asp Ala Phe Leu Met Arg Met Thr Glu Glu Lys Phe Glu Lys Val
        115                 120                 125

Ile Asn Ala Asn Leu Thr Gly Ala Phe Arg Val Ala Gln Arg Ala Ser
    130                 135                 140

Arg Ser Met Gln Arg Asn Lys Phe Gly Arg Met Ile Phe Ile Gly Ser
145                 150                 155                 160

Val Ser Gly Ser Trp Gly Ile Gly Asn Gln Ala Asn Tyr Ala Ala Ser
                165                 170                 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 20
<211> 267
<212> PRT
<213> Séquence artificielle

<220>
<223> Mutant de la protéine MabA

<220>
<221> MISC_FEATURE
<222> (44)..(44)
<223> Asp ou Glu

<220>
<221> MISC_FEATURE
<222> (66)..(66)
<223> Asp ou His

<400> 20

```
Met Gly Ser Ser His His His His His His Ser Ser Gly Leu Val Pro
1               5                   10                  15

Arg Gly Ser His Met Thr Ala Thr Ala Thr Glu Gly Ala Lys Pro Pro
            20                  25                  30

Phe Val Ser Arg Ser Val Leu Val Thr Gly Gly Xaa Arg Gly Ile Gly
        35                  40                  45

Leu Ala Ile Ala Gln Arg Leu Ala Ala Asp Gly His Lys Val Ala Val
    50                  55                  60
```

Thr Xaa Arg Gly Ser Gly Ala Pro Lys Gly Leu Phe Gly Val Glu Val
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Ser Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 21
<211> 267
<212> PRT
<213> Séquence artificielle

<220>

<223> Mutant de la protéine MabA

<220>
<221> MISC_FEATURE
<222> (44)..(44)
<223> Asp ou Glu

<220>
<221> MISC_FEATURE
<222> (66)..(66)
<223> Asp ou His

<400> 21

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Xaa Arg Gly Ile Gly
35 40 45

Leu Ala Ile Ala Gln Arg Leu Ala Ala Asp Gly His Lys Val Ala Val
50 55 60

Thr Xaa Arg Gly Ser Gly Ala Pro Lys Gly Leu Phe Gly Val Glu Cys
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Leu Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 22
<211> 267
<212> PRT
<213> Séquence artificielle

<220>
<223> Mutant de la protéine MabA

<220>
<221> MISC_FEATURE
<222> (44)..(44)
<223> Asp ou Glu

<220>
<221> MISC_FEATURE
<222> (66)..(66)
<223> Asp ou His

<400> 22

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Xaa Arg Gly Ile Gly
35 40 45

Leu Ala Ile Ala Gln Arg Leu Ala Ala Asp Gly His Lys Val Ala Val
50 55 60

Thr Xaa Arg Gly Ser Gly Ala Pro Lys Gly Leu Phe Gly Val Glu Val
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Leu Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 23
<211> 267
<212> PRT
<213> Séquence artificielle

<220>
<223> Mutant de la protéine MabA

<220>
<221> MISC_FEATURE

<222> (44)..(44)
<223> Asp ou Glu

<220>
<221> MISC_FEATURE
<222> (66)..(66)
<223> Asp ou His

<400> 23

Met Gly Ser Ser His His His His His His Ser Ser Gly Leu Val Pro
1 5 10 15

Arg Gly Ser His Met Thr Ala Thr Ala Thr Glu Gly Ala Lys Pro Pro
20 25 30

Phe Val Ser Arg Ser Val Leu Val Thr Gly Gly Xaa Arg Gly Ile Gly
35 40 45

Leu Ala Ile Ala Gln Arg Leu Ala Ala Asp Gly His Lys Val Ala Val
50 55 60

Thr Xaa Arg Gly Ser Gly Ala Pro Lys Gly Leu Phe Gly Val Glu Val
65 70 75 80

Asp Val Thr Asp Ser Asp Ala Val Asp Arg Ala Phe Thr Ala Val Glu
85 90 95

Glu His Gln Gly Pro Val Glu Val Leu Val Ser Asn Ala Gly Leu Ser
100 105 110

Ala Asp Ala Phe Leu Met Arg Met Thr Glu Glu Lys Phe Glu Lys Val
115 120 125

Ile Asn Ala Asn Leu Thr Gly Ala Phe Arg Val Ala Gln Arg Ala Ser
130 135 140

Arg Ser Met Gln Arg Asn Lys Phe Gly Arg Met Ile Phe Ile Gly Ser
145 150 155 160

Val Ser Gly Leu Trp Gly Ile Gly Asn Gln Ala Asn Tyr Ala Ala Ser
165 170 175

Lys Ala Gly Val Ile Gly Met Ala Arg Ser Ile Ala Arg Glu Leu Ser
180 185 190

Lys Ala Asn Val Thr Ala Asn Val Val Ala Pro Gly Tyr Ile Asp Thr
195 200 205

Asp Met Thr Arg Ala Leu Asp Glu Arg Ile Gln Gln Gly Ala Leu Gln
210 215 220

Phe Ile Pro Ala Lys Arg Val Gly Thr Pro Ala Glu Val Ala Gly Val
225 230 235 240

Val Ser Phe Leu Ala Ser Glu Asp Ala Ser Tyr Ile Ser Gly Ala Val
245 250 255

Ile Pro Val Asp Gly Gly Met Gly Met Gly His
260 265

<210> 24
<211> 250
<212> PRT
<213> Séquence artificielle

<220>
<223> Mutant de la protéine MabA

<400> 24

```
Gly Ser His Met Thr Ala Thr Ala Thr Glu Gly Ala Lys Pro Pro Phe
1               5                   10                  15

Val Ser Arg Ser Val Leu Val Thr Gly Gly Asn Arg Gly Ile Gly Leu
            20                  25                  30

Ala Ile Ala Gln Arg Leu Ala Ala Asp Gly His Lys Val Ala Val Thr
        35                  40                  45

His Arg Gly Ser Gly Ala Pro Lys Gly Leu Phe Gly Val Glu Cys Asp
    50                  55                  60

Val Thr Asp Ser Asp Ala Val Asp Arg Ala Phe Thr Ala Val Glu Glu
65                  70                  75                  80

His Gln Gly Pro Val Glu Val Leu Val Ser Asn Ala Gly Leu Ser Ala
                85                  90                  95

Asp Ala Phe Leu Met Arg Met Thr Glu Glu Lys Phe Glu Lys Val Ile
            100                 105                 110

Asn Ala Asn Leu Thr Gly Ala Phe Arg Val Ala Gln Arg Ala Ser Arg
        115                 120                 125

Ser Met Gln Arg Asn Lys Phe Gly Arg Met Ile Phe Ile Gly Ser Val
```

130 135 140

Ser Gly Ser Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 25
<211> 250
<212> PRT
<213> Séquence artificielle

<220>
<223> Mutant de la protéine MabA

<400> 25

Gly Ser His Met Thr Ala Thr Ala Thr Glu Gly Ala Lys Pro Pro Phe
1 5 10 15

Val Ser Arg Ser Val Leu Val Thr Gly Gly Asn Arg Gly Ile Gly Leu
20 25 30

Ala Ile Ala Gln Arg Leu Ala Ala Asp Gly His Lys Val Ala Val Thr
35 40 45

His Arg Gly Ser Gly Ala Pro Lys Gly Leu Phe Gly Val Glu Val Asp
50 55 60

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Ser Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 26
<211> 250
<212> PRT
<213> Séquence artificielle

<220>
<223> Mutant de la protéine MabA

<400> 26

Gly Ser His Met Thr Ala Thr Ala Thr Glu Gly Ala Lys Pro Pro Phe
1               5                   10                  15

Val Ser Arg Ser Val Leu Val Thr Gly Gly Asn Arg Gly Ile Gly Leu
            20                  25                  30

```
Ala Ile Ala Gln Arg Leu Ala Ala Asp Gly His Lys Val Ala Val Thr
        35                  40                  45

His Arg Gly Ser Gly Ala Pro Lys Gly Leu Phe Gly Val Glu Cys Asp
    50                  55                  60

Val Thr Asp Ser Asp Ala Val Asp Arg Ala Phe Thr Ala Val Glu Glu
65                  70                  75                  80

His Gln Gly Pro Val Glu Val Leu Val Ser Asn Ala Gly Leu Ser Ala
                85                  90                  95

Asp Ala Phe Leu Met Arg Met Thr Glu Glu Lys Phe Glu Lys Val Ile
            100                 105                 110

Asn Ala Asn Leu Thr Gly Ala Phe Arg Val Ala Gln Arg Ala Ser Arg
        115                 120                 125

Ser Met Gln Arg Asn Lys Phe Gly Arg Met Ile Phe Ile Gly Ser Val
    130                 135                 140

Ser Gly Leu Trp Gly Ile Gly Asn Gln Ala Asn Tyr Ala Ala Ser Lys
145                 150                 155                 160

Ala Gly Val Ile Gly Met Ala Arg Ser Ile Ala Arg Glu Leu Ser Lys
                165                 170                 175

Ala Asn Val Thr Ala Asn Val Val Ala Pro Gly Tyr Ile Asp Thr Asp
            180                 185                 190

Met Thr Arg Ala Leu Asp Glu Arg Ile Gln Gln Gly Ala Leu Gln Phe
        195                 200                 205

Ile Pro Ala Lys Arg Val Gly Thr Pro Ala Glu Val Ala Gly Val Val
    210                 215                 220

Ser Phe Leu Ala Ser Glu Asp Ala Ser Tyr Ile Ser Gly Ala Val Ile
225                 230                 235                 240

Pro Val Asp Gly Gly Met Gly Met Gly His
                245                 250
```

<210> 27
<211> 250
<212> PRT

<213> Séquence artificielle

<220>
<223> Mutant de la protéine MabA

<400> 27

Gly Ser His Met Thr Ala Thr Ala Thr Glu Gly Ala Lys Pro Pro Phe
1 5 10 15

Val Ser Arg Ser Val Leu Val Thr Gly Gly Asn Arg Gly Ile Gly Leu
20 25 30

Ala Ile Ala Gln Arg Leu Ala Ala Asp Gly His Lys Val Ala Val Thr
35 40 45

His Arg Gly Ser Gly Ala Pro Lys Gly Leu Phe Gly Val Glu Val Asp
50 55 60

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Leu Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 28
<211> 250
<212> PRT
<213> Séquence artificielle

<220>
<223> Mutant de la protéine MabA

<220>
<221> MISC_FEATURE
<222> (27)..(27)
<223> Asp ou Glu

<220>
<221> MISC_FEATURE
<222> (49) .. (49)
<223> Asp ou His

<400> 28

Gly Ser His Met Thr Ala Thr Ala Thr Glu Gly Ala Lys Pro Pro Phe
1 5 10 15

Val Ser Arg Ser Val Leu Val Thr Gly Gly Xaa Arg Gly Ile Gly Leu
20 25 30

Ala Ile Ala Gln Arg Leu Ala Ala Asp Gly His Lys Val Ala Val Thr
35 40 45

Xaa Arg Gly Ser Gly Ala Pro Lys Gly Leu Phe Gly Val Glu Cys Asp
50 55 60

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Ser Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 29
<211> 250
<212> PRT
<213> Séquence artificielle

<220>
<223> Mutant de la protéine MabA

<220>
<221> MISC_FEATURE
<222> (27)..(27)
<223> Asp ou Glu

<220>
<221> MISC_FEATURE
<222> (49).. (49)
<223> Asp ou His

<400> 29

Gly Ser His Met Thr Ala Thr Ala Thr Glu Gly Ala Lys Pro Pro Phe
1 5 10 15

Val Ser Arg Ser Val Leu Val Thr Gly Gly Xaa Arg Gly Ile Gly Leu

20 25 30

Ala Ile Ala Gln Arg Leu Ala Ala Asp Gly His Lys Val Ala Val Thr
35 40 45

Xaa Arg Gly Ser Gly Ala Pro Lys Gly Leu Phe Gly Val Glu Val Asp
50 55 60

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Ser Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 30
<211> 250
<212> PRT
<213> Séquence artificielle

<220>
<223> Mutant de la protéine MabA

<220>
<221> MISC_FEATURE
<222> (27)..(27)
<223> Asp ou Glu

<220>
<221> MISC_FEATURE
<222> (49).. (49)
<223> Asp ou His

<400> 30

```
Gly Ser His Met Thr Ala Thr Ala Thr Glu Gly Ala Lys Pro Pro Phe
1               5                   10                  15

Val Ser Arg Ser Val Leu Val Thr Gly Gly Xaa Arg Gly Ile Gly Leu
            20                  25                  30

Ala Ile Ala Gln Arg Leu Ala Ala Asp Gly His Lys Val Ala Val Thr
        35                  40                  45

Xaa Arg Gly Ser Gly Ala Pro Lys Gly Leu Phe Gly Val Glu Cys Asp
    50                  55                  60

Val Thr Asp Ser Asp Ala Val Asp Arg Ala Phe Thr Ala Val Glu Glu
65                  70                  75                  80

His Gln Gly Pro Val Glu Val Leu Val Ser Asn Ala Gly Leu Ser Ala
                85                  90                  95

Asp Ala Phe Leu Met Arg Met Thr Glu Glu Lys Phe Glu Lys Val Ile
            100                 105                 110

Asn Ala Asn Leu Thr Gly Ala Phe Arg Val Ala Gln Arg Ala Ser Arg
        115                 120                 125

Ser Met Gln Arg Asn Lys Phe Gly Arg Met Ile Phe Ile Gly Ser Val
    130                 135                 140

Ser Gly Leu Trp Gly Ile Gly Asn Gln Ala Asn Tyr Ala Ala Ser Lys
145                 150                 155                 160

Ala Gly Val Ile Gly Met Ala Arg Ser Ile Ala Arg Glu Leu Ser Lys
```

165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 31
<211> 250
<212> PRT
<213> Séquence artificielle

<220>
<223> Mutant de la protéine MabA

<220>
<221> MISC_FEATURE
<222> (27)..(27)
<223> Asp ou Glu

<220>
<221> MISC_FEATURE
<222> (49)..(49)
<223> Asp ou His

<400> 31

```
Gly Ser His Met Thr Ala Thr Ala Thr Glu Gly Ala Lys Pro Pro Phe
1               5                   10                  15

Val Ser Arg Ser Val Leu Val Thr Gly Gly Xaa Arg Gly Ile Gly Leu
            20                  25                  30

Ala Ile Ala Gln Arg Leu Ala Ala Asp Gly His Lys Val Ala Val Thr
        35                  40                  45

Xaa Arg Gly Ser Gly Ala Pro Lys Gly Leu Phe Gly Val Glu Val Asp
    50                  55                  60
```

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Leu Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 32
<211> 250
<212> PRT
<213> Séquence artificielle

<220>
<223> Mutant de la protéine MabA

<220>
<221> MISC_FEATURE

<222> (27)..(27)
<223> Asp ou Glu

<220>
<221> MISC_FEATURE
<222> (49).. (49)
<223> Asp ou His

<400> 32

Gly Ser His Met Thr Ala Thr Ala Thr Glu Gly Ala Lys Pro Pro Phe
1 5 10 15

Val Ser Arg Ser Val Leu Val Thr Gly Gly Xaa Arg Gly Ile Gly Leu
20 25 30

Ala Ile Ala Gln Arg Leu Ala Ala Asp Gly His Lys Val Ala Val Thr
35 40 45

Xaa Arg Gly Ser Gly Ala Pro Lys Gly Leu Phe Gly Val Glu Val Asp
50 55 60

Val Thr Asp Ser Asp Ala Val Asp Arg Ala Phe Thr Ala Val Glu Glu
65 70 75 80

His Gln Gly Pro Val Glu Val Leu Val Ser Asn Ala Gly Leu Ser Ala
85 90 95

Asp Ala Phe Leu Met Arg Met Thr Glu Glu Lys Phe Glu Lys Val Ile
100 105 110

Asn Ala Asn Leu Thr Gly Ala Phe Arg Val Ala Gln Arg Ala Ser Arg
115 120 125

Ser Met Gln Arg Asn Lys Phe Gly Arg Met Ile Phe Ile Gly Ser Val
130 135 140

Ser Gly Leu Trp Gly Ile Gly Asn Gln Ala Asn Tyr Ala Ala Ser Lys
145 150 155 160

Ala Gly Val Ile Gly Met Ala Arg Ser Ile Ala Arg Glu Leu Ser Lys
165 170 175

Ala Asn Val Thr Ala Asn Val Val Ala Pro Gly Tyr Ile Asp Thr Asp
180 185 190

Met Thr Arg Ala Leu Asp Glu Arg Ile Gln Gln Gly Ala Leu Gln Phe
195 200 205

Ile Pro Ala Lys Arg Val Gly Thr Pro Ala Glu Val Ala Gly Val Val
210 215 220

Ser Phe Leu Ala Ser Glu Asp Ala Ser Tyr Ile Ser Gly Ala Val Ile
225 230 235 240

Pro Val Asp Gly Gly Met Gly Met Gly His
245 250

<210> 33
<211> 240
<212> PRT
<213> Séquence artificielle

<220>
<223> Mutant de la protéine MabA

<400> 33

Gly Ala Lys Pro Pro Phe Val Ser Arg Ser Val Leu Val Thr Gly Gly
1 5 10 15

Asn Arg Gly Ile Gly Leu Ala Ile Ala Gln Arg Leu Ala Ala Asp Gly
20 25 30

His Lys Val Ala Val Thr His Arg Gly Ser Gly Ala Pro Lys Gly Leu
35 40 45

Phe Gly Val Glu Cys Asp Val Thr Asp Ser Asp Ala Val Asp Arg Ala
50 55 60

Phe Thr Ala Val Glu Glu His Gln Gly Pro Val Glu Val Leu Val Ser
65 70 75 80

Asn Ala Gly Leu Ser Ala Asp Ala Phe Leu Met Arg Met Thr Glu Glu
85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Ser Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 34
<211> 240
<212> PRT
<213> Séquence artificielle

<220>
<223> Mutant de la protéine MabA

<400> 34

Gly Ala Lys Pro Pro Phe Val Ser Arg Ser Val Leu Val Thr Gly Gly
1 5 10 15

Asn Arg Gly Ile Gly Leu Ala Ile Ala Gln Arg Leu Ala Ala Asp Gly
20 25 30

His Lys Val Ala Val Thr His Arg Gly Ser Gly Ala Pro Lys Gly Leu
35 40 45

Phe Gly Val Glu Val Asp Val Thr Asp Ser Asp Ala Val Asp Arg Ala
50 55 60

Phe Thr Ala Val Glu Glu His Gln Gly Pro Val Glu Val Leu Val Ser
65 70 75 80

Asn Ala Gly Leu Ser Ala Asp Ala Phe Leu Met Arg Met Thr Glu Glu
85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Ser Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 35
<211> 240
<212> PRT
<213> Séquence artificielle

<220>
<223> Mutant de la protéine MabA

<400> 35

```
Gly Ala Lys Pro Pro Phe Val Ser Arg Ser Val Leu Val Thr Gly Gly
1               5                   10                  15

Asn Arg Gly Ile Gly Leu Ala Ile Ala Gln Arg Leu Ala Ala Asp Gly
            20                  25                  30

His Lys Val Ala Val Thr His Arg Gly Ser Gly Ala Pro Lys Gly Leu
        35                  40                  45

Phe Gly Val Glu Cys Asp Val Thr Asp Ser Asp Ala Val Asp Arg Ala
    50                  55                  60

Phe Thr Ala Val Glu Glu His Gln Gly Pro Val Glu Val Leu Val Ser
65                  70                  75                  80

Asn Ala Gly Leu Ser Ala Asp Ala Phe Leu Met Arg Met Thr Glu Glu
```

85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Leu Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 36
<211> 240
<212> PRT
<213> Séquence artificielle

<220>
<223> Mutant de la protéine MabA

<400> 36

Gly Ala Lys Pro Pro Phe Val Ser Arg Ser Val Leu Val Thr Gly Gly
1 5 10 15

Asn Arg Gly Ile Gly Leu Ala Ile Ala Gln Arg Leu Ala Ala Asp Gly
20 25 30

His Lys Val Ala Val Thr His Arg Gly Ser Gly Ala Pro Lys Gly Leu
35 40 45

Phe Gly Val Glu Val Asp Val Thr Asp Ser Asp Ala Val Asp Arg Ala
50 55 60

Phe Thr Ala Val Glu Glu His Gln Gly Pro Val Glu Val Leu Val Ser
65 70 75 80

Asn Ala Gly Leu Ser Ala Asp Ala Phe Leu Met Arg Met Thr Glu Glu
85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Leu Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 37
<211> 240
<212> PRT
<213> Séquence artificielle

<220>
<223> Mutant de la protéine MabA

<220>
<221> MISC_FEATURE

<222> (17)..(17)
<223> Asp ou Glu

<220>
<221> MISC_FEATURE
<222> (39)..(39)
<223> Asp ou His

<400> 37

Gly Ala Lys Pro Pro Phe Val Ser Arg Ser Val Leu Val Thr Gly Gly
1               5                   10                  15

Xaa Arg Gly Ile Gly Leu Ala Ile Ala Gln Arg Leu Ala Ala Asp Gly
            20                  25                  30

His Lys Val Ala Val Thr Xaa Arg Gly Ser Gly Ala Pro Lys Gly Leu
        35                  40                  45

Phe Gly Val Glu Cys Asp Val Thr Asp Ser Asp Ala Val Asp Arg Ala
    50                  55                  60

Phe Thr Ala Val Glu Glu His Gln Gly Pro Val Glu Val Leu Val Ser
65                  70                  75                  80

Asn Ala Gly Leu Ser Ala Asp Ala Phe Leu Met Arg Met Thr Glu Glu
                85                  90                  95

Lys Phe Glu Lys Val Ile Asn Ala Asn Leu Thr Gly Ala Phe Arg Val
            100                 105                 110

Ala Gln Arg Ala Ser Arg Ser Met Gln Arg Asn Lys Phe Gly Arg Met
        115                 120                 125

Ile Phe Ile Gly Ser Val Ser Gly Ser Trp Gly Ile Gly Asn Gln Ala
    130                 135                 140

Asn Tyr Ala Ala Ser Lys Ala Gly Val Ile Gly Met Ala Arg Ser Ile
145                 150                 155                 160

Ala Arg Glu Leu Ser Lys Ala Asn Val Thr Ala Asn Val Val Ala Pro
                165                 170                 175

Gly Tyr Ile Asp Thr Asp Met Thr Arg Ala Leu Asp Glu Arg Ile Gln
            180                 185                 190

Gln Gly Ala Leu Gln Phe Ile Pro Ala Lys Arg Val Gly Thr Pro Ala
        195                 200                 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 38
<211> 240
<212> PRT
<213> Séquence artificielle

<220>
<223> Mutant de la protéine MabA

<220>
<221> MISC_FEATURE
<222> (17)..(17)
<223> Asp ou Glu

<220>
<221> MISC_FEATURE
<222> (39).. (39)
<223> Asp ou His

<400> 38

Gly Ala Lys Pro Pro Phe Val Ser Arg Ser Val Leu Val Thr Gly Gly
1 5 10 15

Xaa Arg Gly Ile Gly Leu Ala Ile Ala Gln Arg Leu Ala Ala Asp Gly
20 25 30

His Lys Val Ala Val Thr Xaa Arg Gly Ser Gly Ala Pro Lys Gly Leu
35 40 45

Phe Gly Val Glu Val Asp Val Thr Asp Ser Asp Ala Val Asp Arg Ala
50 55 60

Phe Thr Ala Val Glu Glu His Gln Gly Pro Val Glu Val Leu Val Ser
65 70 75 80

Asn Ala Gly Leu Ser Ala Asp Ala Phe Leu Met Arg Met Thr Glu Glu
85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Ser Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 39
<211> 240
<212> PRT
<213> Séquence artificielle

<220>
<223> Mutant de la protéine MabA

<220>
<221> MISC_FEATURE
<222> (17)..(17)
<223> Asp ou Glu

<220>
<221> MISC_FEATURE
<222> (39)..(39)
<223> Asp ou His

<400> 39

```
Gly Ala Lys Pro Pro Phe Val Ser Arg Ser Val Leu Val Thr Gly Gly
1               5                   10                  15

Xaa Arg Gly Ile Gly Leu Ala Ile Ala Gln Arg Leu Ala Ala Asp Gly
            20                  25                  30

His Lys Val Ala Val Thr Xaa Arg Gly Ser Gly Ala Pro Lys Gly Leu
        35                  40                  45
```

Phe Gly Val Glu Cys Asp Val Thr Asp Ser Asp Ala Val Asp Arg Ala
50 55 60

Phe Thr Ala Val Glu Glu His Gln Gly Pro Val Glu Val Leu Val Ser
65 70 75 80

Asn Ala Gly Leu Ser Ala Asp Ala Phe Leu Met Arg Met Thr Glu Glu
85 90 95

Lys Phe Glu Lys Val Ile Asn Ala Asn Leu Thr Gly Ala Phe Arg Val
100 105 110

Ala Gln Arg Ala Ser Arg Ser Met Gln Arg Asn Lys Phe Gly Arg Met
115 120 125

Ile Phe Ile Gly Ser Val Ser Gly Leu Trp Gly Ile Gly Asn Gln Ala
130 135 140

Asn Tyr Ala Ala Ser Lys Ala Gly Val Ile Gly Met Ala Arg Ser Ile
145 150 155 160

Ala Arg Glu Leu Ser Lys Ala Asn Val Thr Ala Asn Val Val Ala Pro
165 170 175

Gly Tyr Ile Asp Thr Asp Met Thr Arg Ala Leu Asp Glu Arg Ile Gln
180 185 190

Gln Gly Ala Leu Gln Phe Ile Pro Ala Lys Arg Val Gly Thr Pro Ala
195 200 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 40
<211> 240
<212> PRT
<213> Séquence artificielle

<220>
<223> Mutant de la protéine MabA

<220>
<221> MISC_FEATURE

<222> (17).. (17)
<223> Asp ou Glu

<220>
<221> MISC_FEATURE
<222> (39)..(39)
<223> Asp ou His

<400> 40

Gly Ala Lys Pro Pro Phe Val Ser Arg Ser Val Leu Val Thr Gly Gly
1               5                   10                  15

Xaa Arg Gly Ile Gly Leu Ala Ile Ala Gln Arg Leu Ala Ala Asp Gly
            20                  25                  30

His Lys Val Ala Val Thr Xaa Arg Gly Ser Gly Ala Pro Lys Gly Leu
        35                  40                  45

Phe Gly Val Glu Val Asp Val Thr Asp Ser Asp Ala Val Asp Arg Ala
    50                  55                  60

Phe Thr Ala Val Glu Glu His Gln Gly Pro Val Glu Val Leu Val Ser
65                  70                  75                  80

Asn Ala Gly Leu Ser Ala Asp Ala Phe Leu Met Arg Met Thr Glu Glu
                85                  90                  95

Lys Phe Glu Lys Val Ile Asn Ala Asn Leu Thr Gly Ala Phe Arg Val
            100                 105                 110

Ala Gln Arg Ala Ser Arg Ser Met Gln Arg Asn Lys Phe Gly Arg Met
        115                 120                 125

Ile Phe Ile Gly Ser Val Ser Gly Leu Trp Gly Ile Gly Asn Gln Ala
    130                 135                 140

Asn Tyr Ala Ala Ser Lys Ala Gly Val Ile Gly Met Ala Arg Ser Ile
145                 150                 155                 160

Ala Arg Glu Leu Ser Lys Ala Asn Val Thr Ala Asn Val Val Ala Pro
                165                 170                 175

Gly Tyr Ile Asp Thr Asp Met Thr Arg Ala Leu Asp Glu Arg Ile Gln
            180                 185                 190

Gln Gly Ala Leu Gln Phe Ile Pro Ala Lys Arg Val Gly Thr Pro Ala
        195                 200                 205

Glu Val Ala Gly Val Val Ser Phe Leu Ala Ser Glu Asp Ala Ser Tyr
210 215 220

Ile Ser Gly Ala Val Ile Pro Val Asp Gly Gly Met Gly Met Gly His
225 230 235 240

<210> 41
<211> 240
<212> PRT
<213> Séquence artificielle

<220>
<223> Mutant de la protéine MabA

<220>
<221> MISC_FEATURE
<222> (17)..(17)
<223> Asp ou Glu

<220>
<221> MISC_FEATURE
<222> (39)..(39)
<223> Asp ou His

<400> 41

```
Gly Ala Lys Pro Pro Phe Val Ser Arg Ser Val Leu Val Thr Gly Gly
1               5                   10                  15

Xaa Arg Gly Ile Gly Leu Ala Ile Ala Gln Arg Leu Ala Ala Asp Gly
            20                  25                  30

His Lys Val Ala Val Thr Xaa Arg Gly Ser Gly Ala Pro Lys Gly Leu
        35                  40                  45

Phe Gly Val Glu Val Asp Val Thr Asp Ser Asp Ala Val Asp Arg Ala
    50                  55                  60

Phe Thr Ala Val Glu Glu His Gln Gly Pro Val Glu Val Leu Val Ser
65                  70                  75                  80

Asn Ala Gly Leu Ser Ala Asp Ala Phe Leu Met Arg Met Thr Glu Glu
                85                  90                  95

Lys Phe Glu Lys Val Ile Asn Ala Asn Leu Thr Gly Ala Phe Arg Val
            100                 105                 110

Ala Gln Arg Ala Ser Arg Ser Met Gln Arg Asn Lys Phe Gly Arg Met
```

| | | | | | | | | | | | | | | | |
|---|---|---|---|---|---|---|---|---|---|---|---|---|---|---|---|
| | | 115 | | | | | 120 | | | | | 125 | | | |
| Ile | Phe | Ile | Gly | Ser | Val | Ser | Gly | Leu | Trp | Gly | Ile | Gly | Asn | Gln | Ala |
| | 130 | | | | | 135 | | | | | 140 | | | | |
| Asn | Tyr | Ala | Ala | Ser | Lys | Ala | Gly | Val | Ile | Gly | Met | Ala | Arg | Ser | Ile |
| 145 | | | | | 150 | | | | | 155 | | | | | 160 |
| Ala | Arg | Glu | Leu | Ser | Lys | Ala | Asn | Val | Thr | Ala | Asn | Val | Val | Ala | Pro |
| | | | | 165 | | | | | 170 | | | | | 175 | |
| Gly | Tyr | Ile | Asp | Thr | Asp | Met | Thr | Arg | Ala | Leu | Asp | Glu | Arg | Ile | Gln |
| | | | 180 | | | | | 185 | | | | | 190 | | |
| Gln | Gly | Ala | Leu | Gln | Phe | Ile | Pro | Ala | Lys | Arg | Val | Gly | Thr | Pro | Ala |
| | | 195 | | | | | 200 | | | | | 205 | | | |
| Glu | Val | Ala | Gly | Val | Val | Ser | Phe | Leu | Ala | Ser | Glu | Asp | Ala | Ser | Tyr |
| | 210 | | | | | 215 | | | | | 220 | | | | |
| Ile | Ser | Gly | Ala | Val | Ile | Pro | Val | Asp | Gly | Gly | Met | Gly | Met | Gly | His |
| 225 | | | | | 230 | | | | | 235 | | | | | 240 |

## Description

La présente invention a principalement pour objet l'utilisation de la protéine MabA, et de protéines dérivées, et plus particulièrement des coordonnées cristallographiques de ces protéines, dans le cadre de la mise en oeuvre de méthodes de conception et de criblage de ligands de ces protéines, et avantageusement de ligands inhibiteurs de l'activité enzymatique de ces protéines, à savoir d'antibiotiques susceptibles d'être utilisés dans le cadre du traitement de mycobactérioses.

La tuberculose est une des causes majeures de mortalité par un agent infectieux unique, *Mycobacterium tuberculosis.* De plus, depuis une quinzaine d'années, il y a une recrudescence de cette maladie dans les pays industrialisés. Ce phénomène est en partie lié à l'apparition de souches du bacille tuberculeux résistantes aux antibiotiques. Ainsi, la conception de nouveaux médicaments antituberculeux est devenue une priorité proclamée par l'Organisation Mondiale de la Santé.

Les cibles des antibiotiques antituberculeux utilisés actuellement en clinique font partie de métabolismes de biosynthèse de composants de l'enveloppe du bacille tuberculeux. En particulier, la cible de l'isoniazide (INH), antituberculeux de 1ère ligne, est impliquée dans la synthèse d'acides gras à très longues chaînes (C60-C90), les acides mycoliques. L'isoniazide inhibe l'activité de la protéine InhA, qui fait partie d'un complexe enzymatique, FAS-II, dont la fonction est de produire, par cycles d'élongation successifs, des acides gras à longues chaînes (C18-C32), précurseurs des acides mycoliques. InhA, une 2-trans-enoyl-ACP réductase, catalyse la 4ème étape d'un cycle d'élongation, qui comprend 4 étapes. L'INH est une pro-drogue qui forme, avec le coenzyme de InhA, le NADH, un adduit inhibiteur, l'INH-NAD(H). FAS-II comprend au moins 3 autres enzymes principales, celles-ci représentant donc des cibles potentielles pour de nouveaux antibiotiques. La protéine MabA catalyse la 2ème étape du cycle.

La présente invention fournit des méthodes pour la conception d'antibiotiques pour le traitement d'infections mycobactériennes, en particulier la tuberculose. Cette invention a trait au gène *mabA* (fabG1, Rv1483) de *Mycobacterium tuberculosis,* au produit de ce gène, la protéine MabA (FabG1), ainsi qu'au matériel et méthodes utilisées pour la production de la protéine en grande quantité, la détermination de sa structure tridimensionnelle à l'échelle atomique et l'étude de ses interactions avec différents ligands ou de leur effet sur son activité enzymatique. L'invention a pour principe d'utiliser la protéine MabA comme cible d'antibiotiques ; en particulier, l'étude de l'interaction de MabA avec différents ligands ou de leur effet sur son activité enzymatique, par différentes méthodes, est utilisée pour concevoir des inhibiteurs de l'activité enzymatique de MabA.

La présente invention apporte les outils méthodologiques et matériel nécessaires pour la conception de molécules représentant des antibiotiques anti-mycobactériens et antituberculeux potentiels.

La présente invention propose le matériel biologique et les méthodologies nécessaires à la production et la purification en grandes quantités, d'une cible potentielle d'antibiotiques antituberculeux, la protéine MabA. De plus, ces étapes peuvent être effectuées très rapidement grâce à la surproduction de MabA dotée d'une étiquette poly-Histidine chez *Escherichia coli* et sa purification en une seule étape par chromatographie de chélation de métaux, donnant une protéine à haut degré de pureté. La quantité et la qualité de la protéine purifiée permettent d'obtenir des résultats fiables lors des études d'activité enzymatique ou de fixation de ligands, mais permettent également la cristallisation de la protéine pour résoudre sa structure tridimensionnelle. La mise au point de conditions permettant la congélation des cristaux de MabA dans l'azote liquide a permis l'obtention de jeux de données à résolution atomique (2,05 Å contre 2,6 Å à température ambiante) et ouvre la voie à de meilleures données grâce à l'utilisation du rayonnement synchrotron. La structure cristalline congelée a révélé le rôle des composés (notamment le césium) nécessaires à la croissance des cristaux de MabA et permet d'envisager une optimisation rationnelle de la cristallogenèse. Le criblage *in cristallo* de « pools » de ligands est aussi réalisable. La quantité de protéine purifiée est également un critère important pour la réalisation de criblages à haut débit de chimiothèques (voir ci-dessous).

Les tests d'activité de la protéine MabA, et par conséquent les tests d'inhibition par des inhibiteurs potentiels, peuvent être suivis de façon aisée et rapide, par spectrophotométrie, en suivant l'oxydation du coenzyme de réduction, le NADPH, à 340 nm: Les constantes d'inhibition (IC50 et Ki) et le mécanisme d'inhibition (inhibition compétitive, non compétitive, incompétitive) pour chaque molécule peuvent en être déduits. De plus, des tests de fixation spécifique de ligands au site actif de MabA peuvent être réalisés également de façon aisée et rapide, par spectrofluorimétrie. La présence de l'unique résidu Trp de la protéine dans le site actif permet, par excitation à 303 nm, de détecter, à partir de la variation d'intensité d'émission de fluorescence au maximum d'émission, la fixation d'un ligand et d'en déduire la constante de dissociation (Kd). De façon similaire, des expériences de FRET (Fluorescence Resonance Energy Transfert) peuvent être réalisées en présence du coenzyme, le NADPH, permettant d'en conclure si le ligand occupe ou non le site de fixation du NADPH (compétition de fixation). La simplicité de ces méthodes de mesure, et les volumes relativement faibles qu'elles requièrent, permettront une miniaturisation des tests d'inhibition ou de fixation de ligand, pour le criblage automatique à haut débit de chimiothèques, grâce à un automate doté d'un spectrophotomètre ou d'un spectrofluorimètre.

La comparaison de la structure de MabA avec celle de la protéine InhA, protéine de la même super-famille

structurale (RED) et appartenant au même complexe enzymatique (voir ci-dessus), a permis de suggérer un effet inhibiteur de l'isoniazide sur l'activité de MabA et de mettre en évidence la forme active de l'isoniazide (antituberculeux), l'adduit INH-NADP(H). La fixation de l'adduit et l'inhibition de l'activité de MabA a ensuite été confirmée expérimentalement. De la même manière, grâce à une forte similarité de structure avec d'autres protéines, qui ont été ou seront cristallisées avec des ligands (par exemple, des dérivés de stéroïdes, co-cristallisés avec des stéroïdes deshydrogénases), la conception rationnelle d'inhibiteurs potentiels de MabA pourra être réalisée rapidement.

La protéine MabA présente un intérêt particulier en tant que cible d'antibiotiques anti-mycobactériens. En effet, elle fait partie du même système enzymatique que la protéine InhA, cible du médicament antituberculeux de 1ère ligne, l'isoniazide. D'autre part, jusqu'à ce jour, il n'a pas été mis en évidence de protéine homologue à MabA dans les cellules animales. De plus, la comparaison avec les protéines homologues, trouvées chez les bactéries ou les plantes, a fait ressortir des propriétés particulières de MabA, qui sont liées à sa fonction, puisqu'elle utilise des substrats à longue chaîne. Ces particularités se reflètent dans la structure de son site actif, ce qui permet d'envisager la conception d'inhibiteurs spécifiques à MabA (notamment, en terme d'encombrement et de caractère hydrophobe), et donc d'antibiotiques à spectre étroit. Ces différents points apportent à MabA des critères de crédibilité pharmacologique.

Ainsi, la présente invention a principalement pour but :

- la recherche et la conception de médicaments efficaces contre les infections mycobactériennes opportunistes (*M. avium, M. kansasii, M. fortuitum, M. chelonae*...) posant des problèmes dans les hôpitaux (stérilisation des instruments médicaux), et dans les cas d'immuno-défiscience humaine (SIDA, prise d'immunosuppresseurs lors d'une greffe, dans le cas de cancers, etc...).
- la recherche et la conception de médicaments efficaces contre les infections tuberculeuses, notamment de médicaments qui soient efficaces sur les souches de *M. tuberculosis* résistantes aux antibiotiques utilisés actuellement en thérapie antituberculeuse, et qui se propagent dans des populations à risque (milieu carcéral, milieux économiquement défavorisés, etc...).
- la recherche et la conception de médicaments efficaces contre d'autres infections bactériennes, en prenant comme cibles moléculaires des protéines homologues à MabA.

Biophysical Journal, 82 (1, second part), page 453a, et Microbiology, 144 (10) pages 2697-2704, font état de la crystallisation du MABA dérivant de la bactérie Mycobacterium tuberculosis.

La présente invention a principalement pour objet des dérivés de la protéine MabA, encore désignée protéine FabG1, par mutation d'un ou plusieurs acides aminés, lesdites protéines dérivées étant sous forme purifiée, et ayant une activité β-cétoacyl réductase NADPH dépendante.

L'invention a plus particulièrement pour objet des dérivés de la protéine MabA recombinante purifiée, ladite protéine étant une protéine de mycobactéries, telle que *Mycobacterium tuberculosis,* et plus particulièrement de *M. tuberculosis* souche H37Rv.

L'invention a également pour objet les protéines recombinantes dérivées susmentionnées, sous forme purifiée, telles qu'obtenues par transformation de souches d'*E.coli* avec un plasmide contenant une séquence comprenant le gène codant pour la protéine MabA, ou comprenant une séquence codant pour une protéine dérivée de MabA telle que définie ci-dessus, suivie par une étape de purification au cours de laquelle :

- les bactéries *E. coli* recombinantes susmentionnées sont lavées dans un tampon de lavage, puis reprises dans un tampon de lyse, et lysées par un cycle de congélation / décongélation en présence d'inhibiteurs de protéases et du lysozyme,
- après traitement par la DNAse I et la RNAse A, en présence de $MgCl_2$, et centrifugation, le surnageant de lyse des bactéries obtenu à l'étape précédente, auquel est additionné 10% (v/v) de glycérol, ou du $NADP^+$ 400 μM, est déposé sur une colonne de résine Ni-NTA agarose,
- après plusieurs lavages avec du tampon à 5 mM puis 50 mM imidazole, la protéine MabA, ou la protéine dérivée, est éluée avec le tampon d'élution.

Selon un mode de réalisation de l'invention: les protéines recombinantes dérivées susmentionnées, sous forme purifiée, sont obtenues selon le procédé décrit ci-dessus dans lequel les différents tampons de lavage des bactéries, de lyse, de lavage, et d'élution, sont les suivants :

- tampon de lavage des bactéries : phosphate de potassium 10 mM, pH 7,8, '
- tampon de lyse : phosphate de potassium 50 mM, pH 7,8 contenant 500 mM de NaCl, 5 mM d'imidazole,
- tampon de lavage : phosphate de potassium 50 mM, pH 7,8 contenant 500 mM de NaCl, 5 et 50 mM d'imidazole,
- tampon d'élution: phosphate de potassium 50 mM, pH 7,8 contenant 500 mM de NaCl, et 175 mM d'imidazole.

Avantageusement, les protéines obtenues à l'aide des tampons susmentionnés, sont utilisées dans le cadre d'études de cinétique enzymatique pour le criblage de ligands selon les méthodes décrites ci-après.

Selon un autre mode de réalisation de l'invention, les protéines recombinantes dérivées susmentionnées, sous forme purifiée, sont obtenues selon le procédé décrit ci-dessus dans lequel les différents tampons de lavage des bactéries, de lyse, de lavage, et d'élution, sont les suivants :

- tampon de lavage des bactéries : Tris 10 mM, pH8,0,
- tampon de lyse :
  - 50 mM tampon Tris, pH 8,0, supplémenté avec 300 mM $LiSO_4$ et 5 mM imidazole;
  - ou 50 mM tampon Tris, pH 8,0, supplémenté avec 300 mM KCl et 5 mM imidazole,
- tampon de lavage :
  - 50 mM tampon Tris, pH 8,0, supplémenté avec 300 mM $LiSO_4$ et 5 ou 50 mM imidazole,
  - ou 50 mM tampon Tris, pH 8,0, supplémenté avec 300 mM KCl et 5 ou 50 mM imidazole.
- tampon d'élution:
  - 20 mM tampon MES, pH6.4, $LiSO_4$ 300mM et 175-750 mM imidazole;
  - ou 20 mM tampon PIPES, pH 8,0, supplémenté avec 300 mM KCl et 175-750 mM imidazole.

Avantageusement, les protéines obtenues à l'aide des tampons susmentionnés, sont utilisées dans le cadre d'études de cristallographie pour la conception et le criblage de ligands selon les méthodes décrites ci-après.

L'invention concerne les protéines dérivées de la protéine MabA susmentionnées, et caractérisées en ce qu'elles correspondent à la protéine MabA dont la séquence en acides aminés SEQ ID NO : 1 est la suivante :

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

dans laquelle la cystéine en position 60 est remplacée par un résidu valine, et/ou la glycine en position 139 est remplacée par une alanine ou une sérine, et/ou la sérine en position 144 est remplacée par un résidu leucine.

A ce titre, l'invention a plus particulièrement pour objet la protéine dérivée de la protéine MabA telle que définie ci-dessus, et caractérisée en ce qu'elle correspond à la protéine MabA dans laquelle la cystéine en position 60 est remplacée par un résidu valine, ladite protéine dérivée, encore désignée C(60)V, correspondant à la séquence SEQ ID NO 3 suivante :

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

A ce titre également, l'invention a plus particulièrement pour objet la protéine dérivée de la protéine MabA telle que définie ci-dessus, et caractérisée en ce qu'elle correspond à la protéine MabA dans laquelle la sérine en position 144 est remplacée par un résidu leucine, ladite protéine dérivée, encore désignée S(144)L, correspondant à la séquence SEQ ID NO 5 suivante :

MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

A ce titre encore, l'invention a plus particulièrement pour objet la protéine dérivée de la protéine MabA telle que définie ci-dessus, et caractérisée en ce qu'elle correspond à la protéine MabA dans laquelle la cystéine en position 60 est remplacée par un résidu valine, et la sérine en position 144 est remplacée par un résidu leucine, ladite protéine dérivée, encore désignée C(60)V / S(144)L, correspondant à la séquence SEQ ID NO 7 suivante :

MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

L'invention a plus particulièrement pour objet également la protéine dérivée de la protéine MabA telle que définie ci-dessus, et caractérisée en ce qu'elle correspond à la protéine MabA dans laquelle la cystéine en position 60 est remplacée par un résidu valine, la glycine en position 139 est remplacée par une alanine ou une sérine, et la sérine en position 144 est remplacée par un résidu leucine, ladite protéine dérivée, encore désignée C(60)V / G(139)[A ou S] / S(144)L, correspondant à la séquence SEQ ID NO 8 suivante :

MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle X représente A ou S.

L'invention concerne également la protéine MabA correspondant à la séquence SEQ ID NO : 1, ou les protéines dérivées de la protéine MabA définies ci-dessus, telles que les protéines dérivées correspondant aux séquences SEQ ID NO : 3, 5, 7, ou 8, caractérisées en ce qu'elles sont modifiées de telle sorte qu'elles comprennent une ou plusieurs mutations permettant de changer la spécificité de la protéine du NADPH vers le NADH.

L'invention a plus particulièrement pour objet les protéines MabA modifiées susmentionnées, correspondant aux séquences suivantes :

- la séquence SEQ ID NO : 9, correspondant à la séquence SEQ ID NO : 1 comportant les mutations N24D(ou E), et/ou H46D, à savoir la séquence suivante :

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 10, correspondant à la séquence SEQ ID NO : 3 comportant les mutations N24D(ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 11, correspondant à la séquence SEQ ID NO : 5 comportant les mutations N24D (ou E), et/ou H46D, à savoir la séquence suivante :

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 12, correspondant à la séquence SEQ ID NO : 7 comportant les mutations N24D(ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ

GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 13, correspondant à la séquence SEQ ID NO : 8 comportant les mutations N24D(ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D.

L'invention a également pour objet la protéine MabA correspondant à la séquence SEQ ID NO : 1, ou les protéines dérivées de la protéine MabA définies ci-dessus, telles que les protéines dérivées correspondant aux séquences SEQ ID NO : 3, 5, 7, 8, 9, 10, 11, 12, ou 13, caractérisées en ce qu'elles sont modifiées par insertion du côté N-terminal d'une étiquette poly-histidine telle que la séquence SEQ ID NO : 14 suivante : MGSSHHHHHH SSGLVPRGSH.

L'invention a plus particulièrement pour objet les protéines MabA modifiées susmentionnées, correspondant aux séquences suivantes :

- la séquence SEQ ID NO : 15, correspondant à la combinaison de la séquence SEQ ID NO : 14 et de la séquence SEQ ID NO : 1, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 16, correspondant à la combinaison de la séquence SEQ ID NO : 14 et de la séquence SEQ ID NO : 3, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 17, correspondant à la combinaison de la séquence SEQ ID NO : 14 et de la séquence SEQ ID NO : 5, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG

PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 18, correspondant à la combinaison de la séquence SEQ ID NO : 14 et de la séquence SEQ ID NO : 7, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 19, correspondant à la combinaison de la séquence SEQ ID NO : 14 et de la séquence SEQ ID NO : 9, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 20, correspondant à la combinaison de la séquence SEQ ID NO : 14 et de la séquence SEQ ID NO : 10, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 21, correspondant à la combinaison de la séquence SEQ ID NO : 14 et de la séquence SEQ ID NO : 11, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 22, correspondant à la combinaison de la séquence SEQ ID NO : 14 et de la séquence SEQ ID NO : 12, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 23, correspondant à la combinaison de la séquence SEQ ID NO : 14 et de la séquence SEQ ID NO : 13, à savoir la séquence suivante :

MGSSHHHHHH SSGLVPRGSH MTATATEGAK PPFVSRSVLV TGGX1RGIGLA
IAQRLAADGH KVAVTX2RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D.

L'invention a les protéines dérivées de la protéine MabA définies ci-dessus, telles que les protéines dérivées correspondant aux séquences SEQ ID NO : 3, 5, 7, 8, 9, 10, 11, 12, ou 13, présentant une séquence GSH en N-terminal, à savoir les séquences suivantes :

- la séquence SEQ ID NO : 24 suivante, correspondant à la combinaison de la séquence GSH et de la séquence SEQ ID NO : 1,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 25 suivante, correspondant à la combinaison de la séquence GSH et de la séquence SEQ ID NO : 3,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 26 suivante, correspondant à la combinaison de la séquence GSH et de la séquence SEQ ID NO : 5,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 27 suivante, correspondant à la combinaison de la séquence GSH et de la séquence SEQ ID NO : 7,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 28 suivante, correspondant à la combinaison de la séquence GSH et de la séquence SEQ ID NO : 9,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 29 suivante, correspondant à la combinaison de la séquence GSH et de la séquence SEQ ID NO : 10,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 30 suivante, correspondant à la combinaison de la séquence GSH et de la séquence SEQ ID NO : 11,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV

VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 31 suivante, correspondant à la combinaison de la séquence GSH et de la séquence SEQ ID NO : 12,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 32 suivante, correspondant à la combinaison de la séquence GSH et de la séquence SEQ ID NO : 13,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D.

L'invention a les protéines dérivées de la protéine MabA définies ci-dessus, telles que les protéines dérivées correspondant aux séquences SEQ ID NO : 3, 5, 7, 8, 9, 10, 11, 12, ou 13, dans lesquelles les sept premiers acides aminés sont supprimés, à savoir les séquences suivantes :

- la séquence SEQ ID NO : 33 suivante, correspondant à la séquence SEQ ID NO : 1 dont les sept premiers acides aminés sont supprimés :

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 34 suivante, correspondant à la séquence SEQ ID NO : 3 dont les sept premiers acides aminés sont supprimés:

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 35 suivante, correspondant à la séquence SEQ ID NO : 5 dont les sept premiers acides aminés sont supprimés :

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 36 suivante, correspondant à la séquence SEQ ID NO : 7 dont les sept premiers acides aminés sont supprimés :

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 37 suivante, correspondant à la séquence SEQ ID NO : 9 dont les sept premiers acides aminés sont supprimés :

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 38 suivante, correspondant à la séquence SEQ ID NO : 10 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 39 suivante, correspondant à la séquence SEQ ID NO : 11 dont les sept premiers acides aminés sont supprimés :

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 40 suivante, correspondant à la séquence SEQ ID NO : 12 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 41 suivante, correspondant à la séquence SEQ ID NO : 13 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D.

L'invention concerne les protéines dérivées susmentionnées, caractérisées par leur activité enzymatique spécifique des substrats du type β-cétoacyl à longue chaîne, notament entre 8 à 20 atomes de carbone, tel que le β -cétooctanoyl-CoA, ou le β - cétododécanoyl-CoA.

L'invention a plus particulièrement pour objet les protéines dérivées susmentionnées, dont les principales caractéristiques de leur structure tridimensionnelle à une résolution de 1,6-2,0 angströms, mises en évidence par l'analyse par diffraction aux rayons X des cristaux desdites protéines, sont telles que représentées sur la figure 1 pour la protéine MabA recombinante recombinante correspondant à la séquence SEQ ID NO : 15, sur la figure 2 pour la protéine dérivée MabA C(60)V correspondant à la séquence SEQ ID NO : 16, et sur la figure 3 pour la protéine dérivée MabA C(60)V / S(144)L correspondant à la séquence SEQ ID NO : 17.

L'invention concerne également la protéine MabA et les protéines dérivées susmentionnées, sous forme cristallisée.

L'invention concerne plus particulièrement les cristaux de protéines susmentionnées, tels qu'obtenus par la méthode de diffusion de vapeur à goutte suspendue, en mélangeant lesdites protéines (1 μl d'une solution à 10 mg/ml) avec une solution de polyéthylène glycol, de CsCl (150-300 mM), et de glycérol (à 10%) dans un tampon (PIPES) à pH 6,2.

L'invention a également pour objet les cristaux de protéines susmentionnés, tels qu'obtenus selon la méthode de cristallisation décrite ci-dessus, ladite méthode étant effectuée à partir des protéines purifiées à l'aide des tampons susmentionnés plus particulièrement utilisés pour l'obtention de protéines de l'invention destinées à des études de cristallographie.

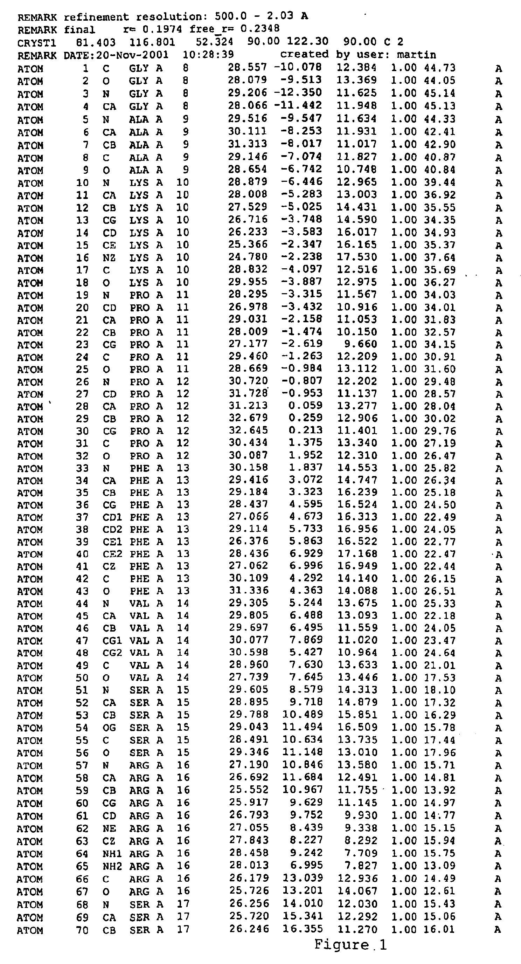

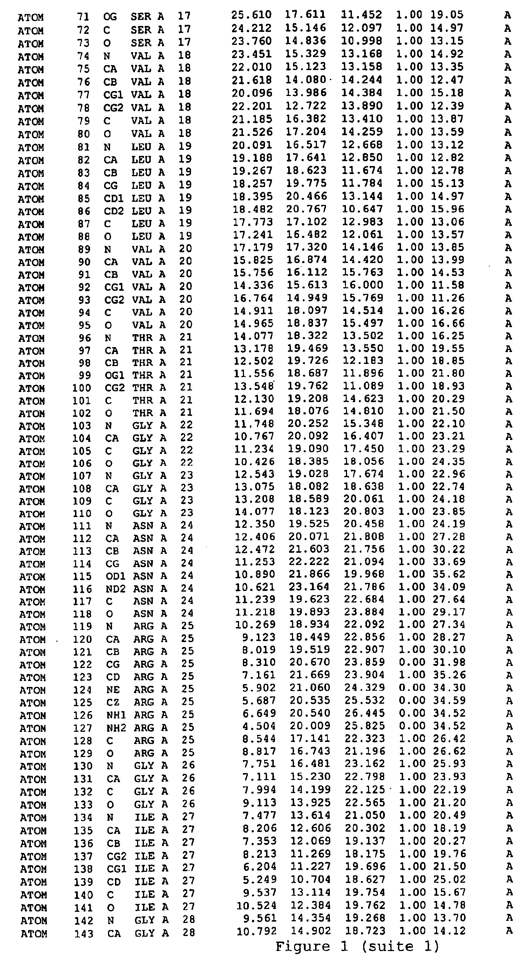

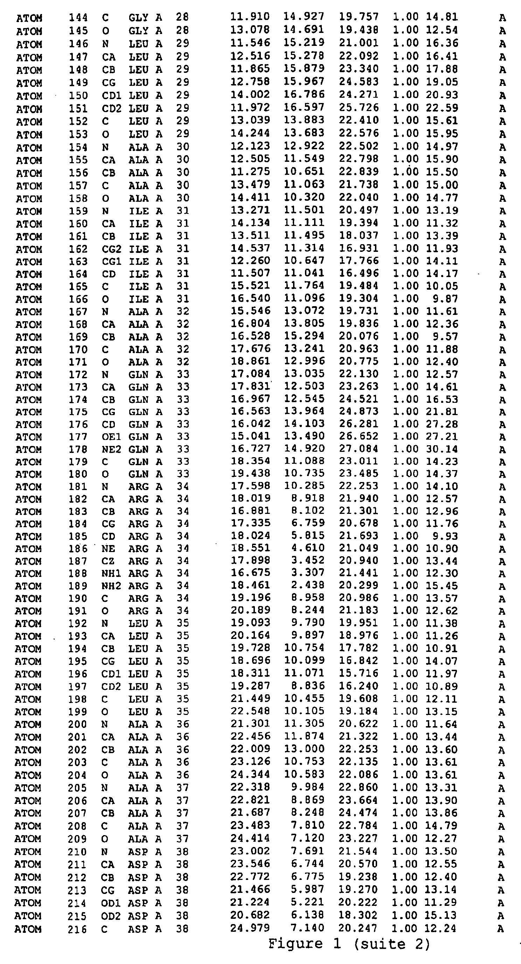

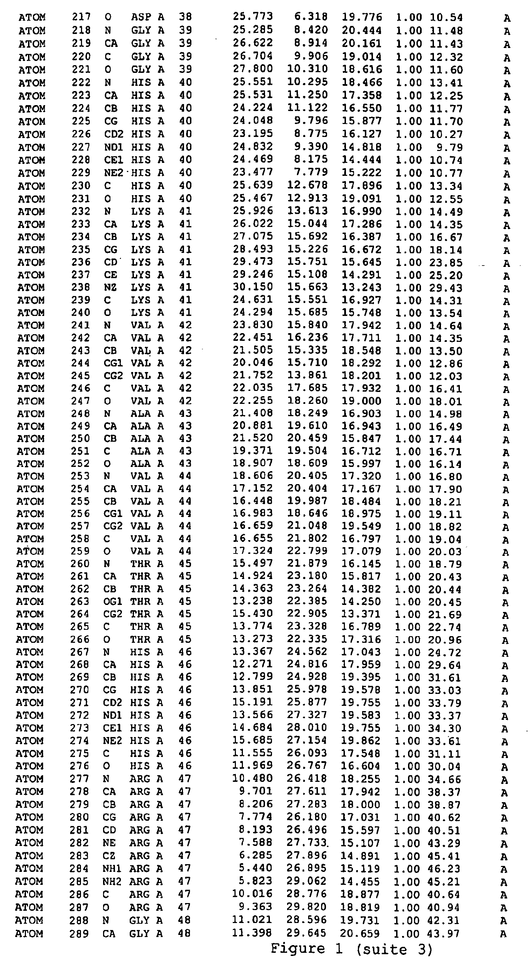

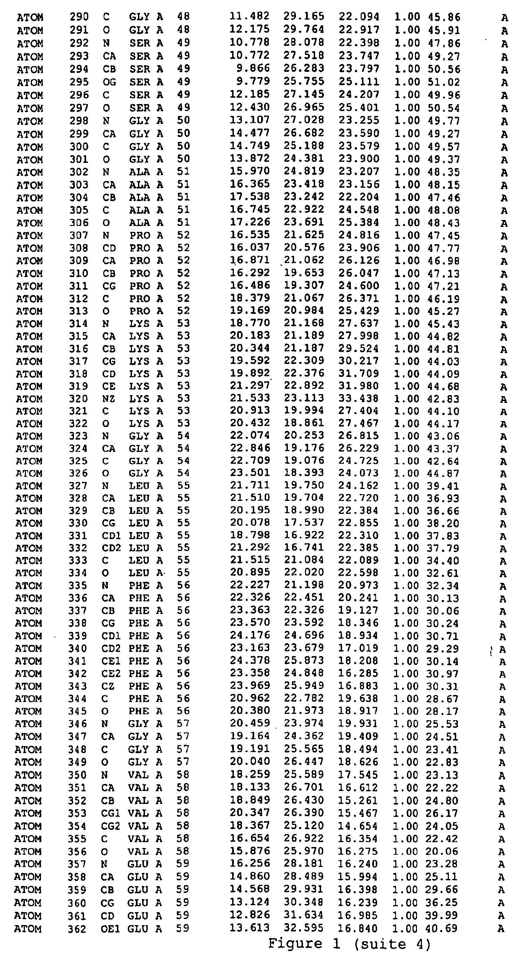

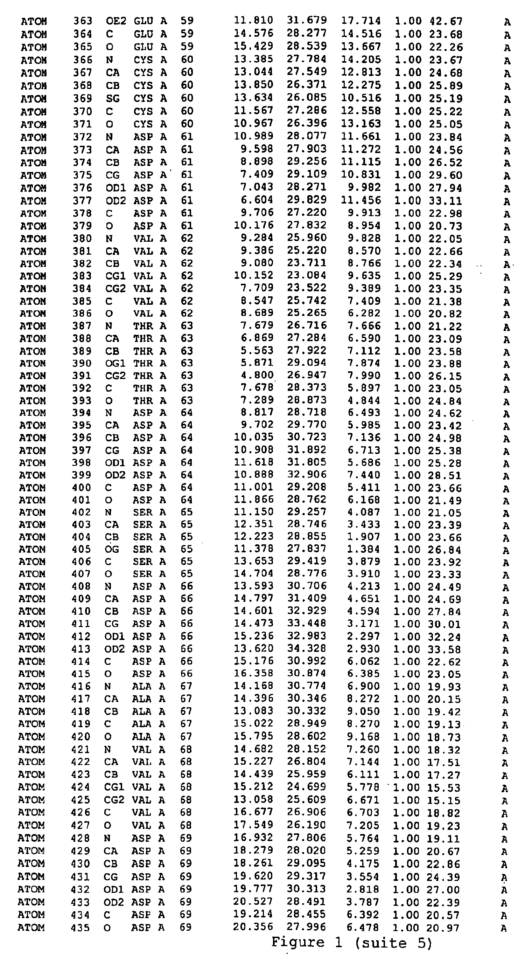

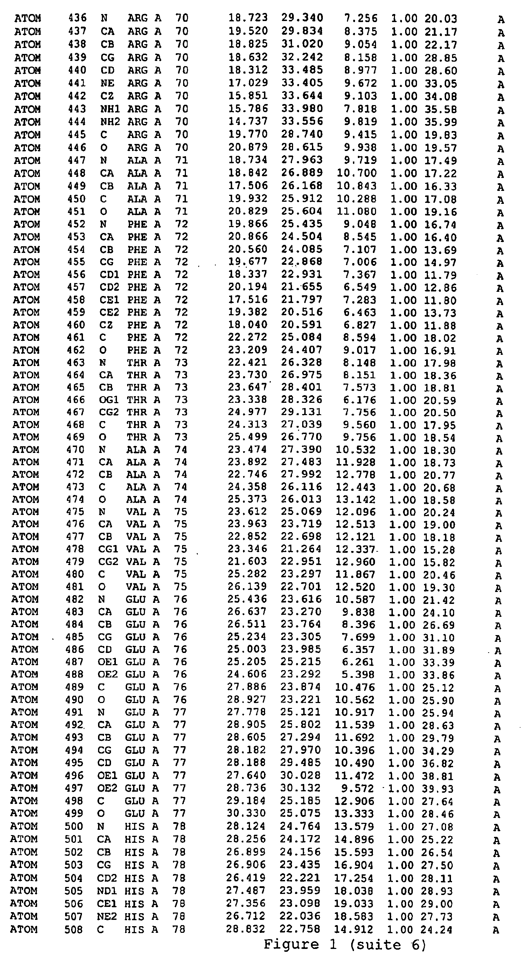

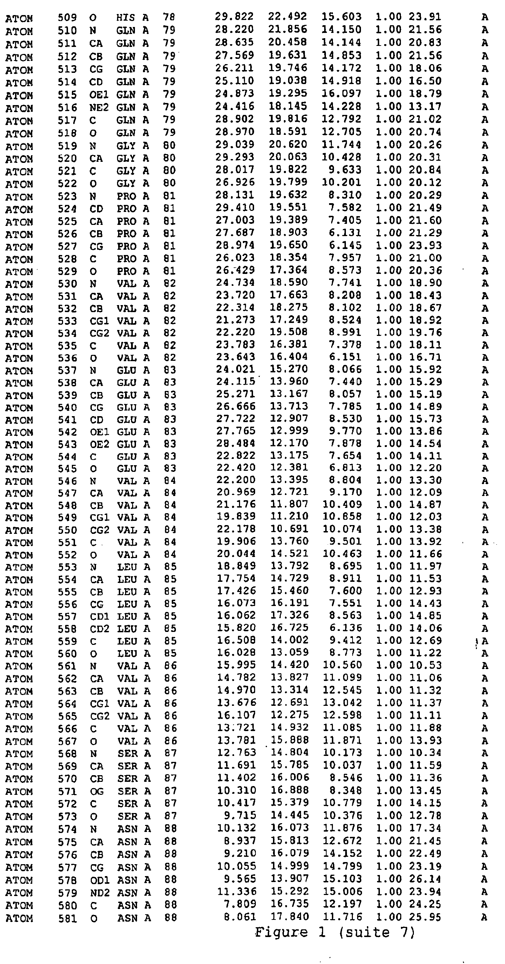

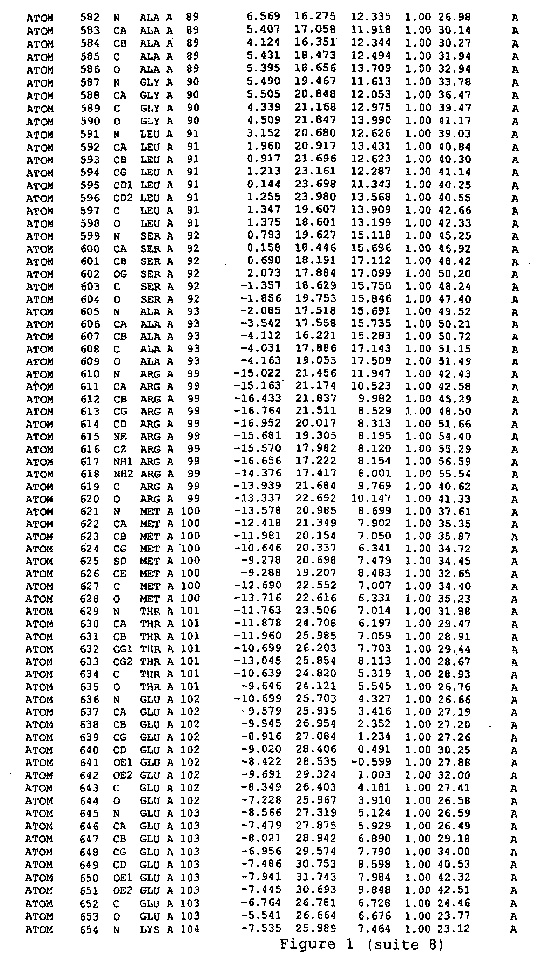

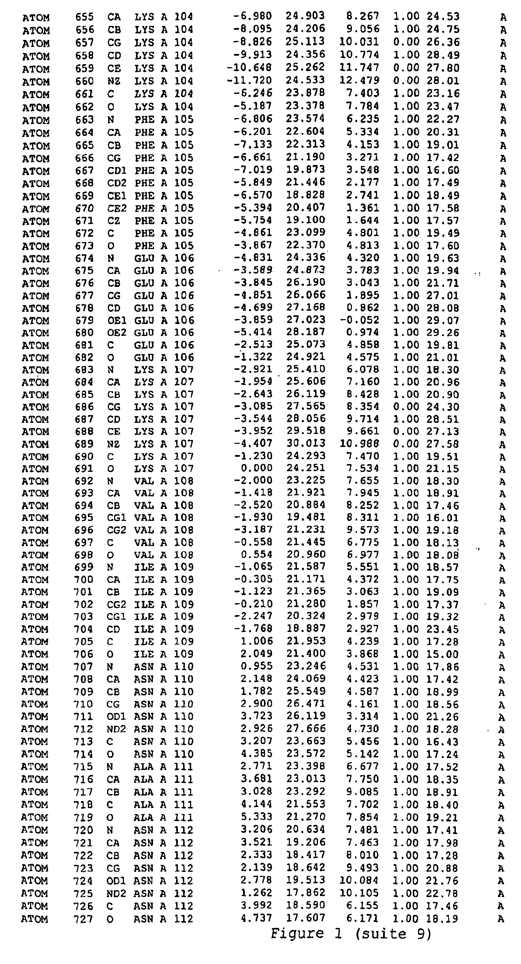

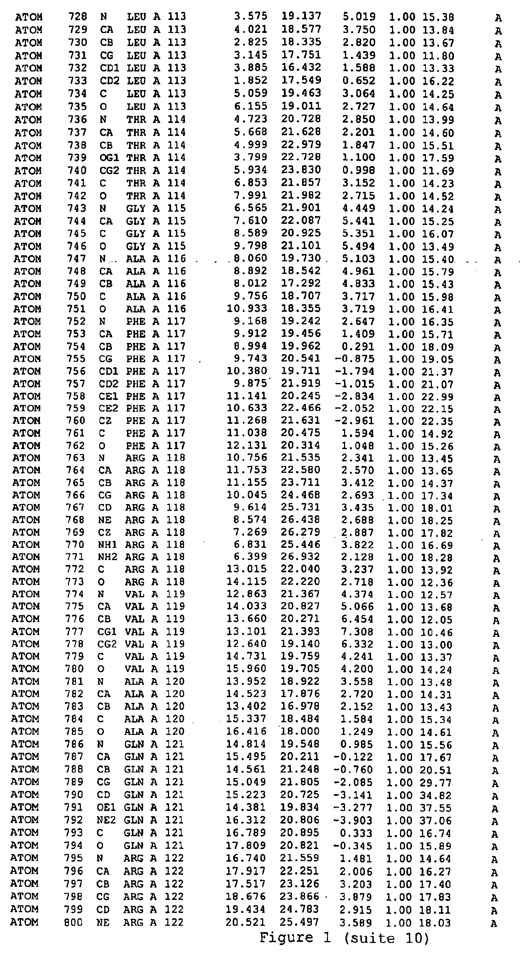

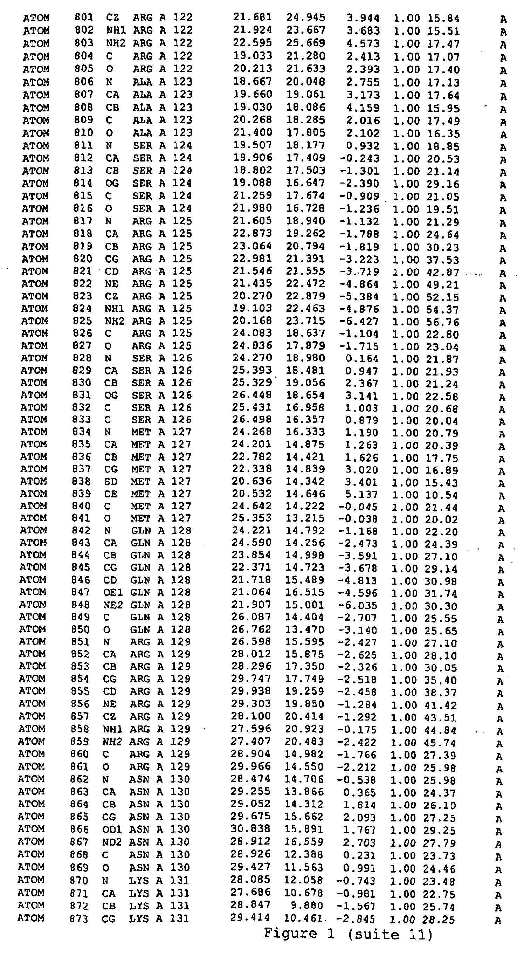

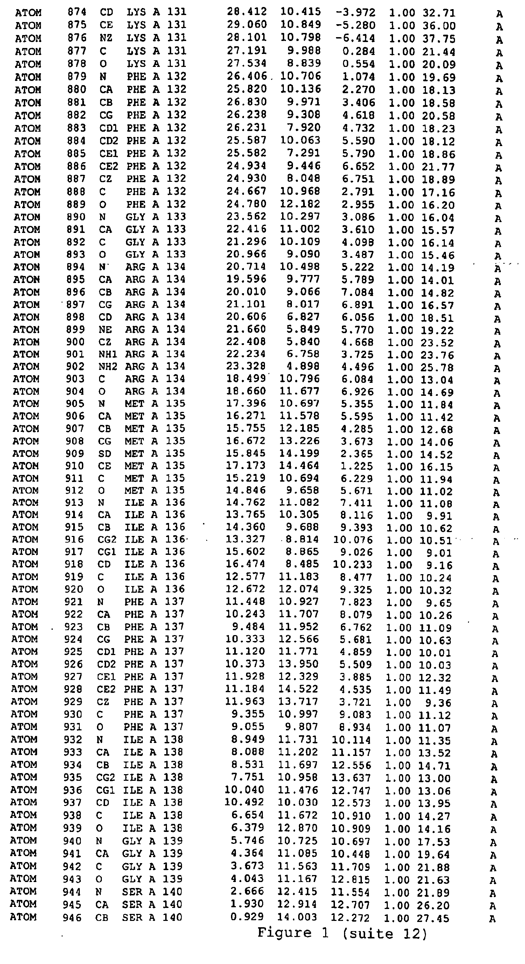

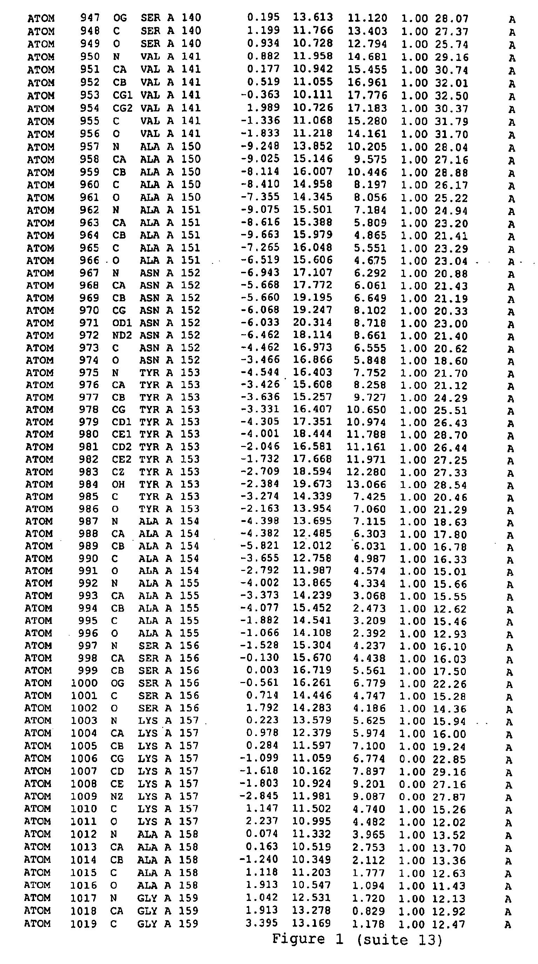

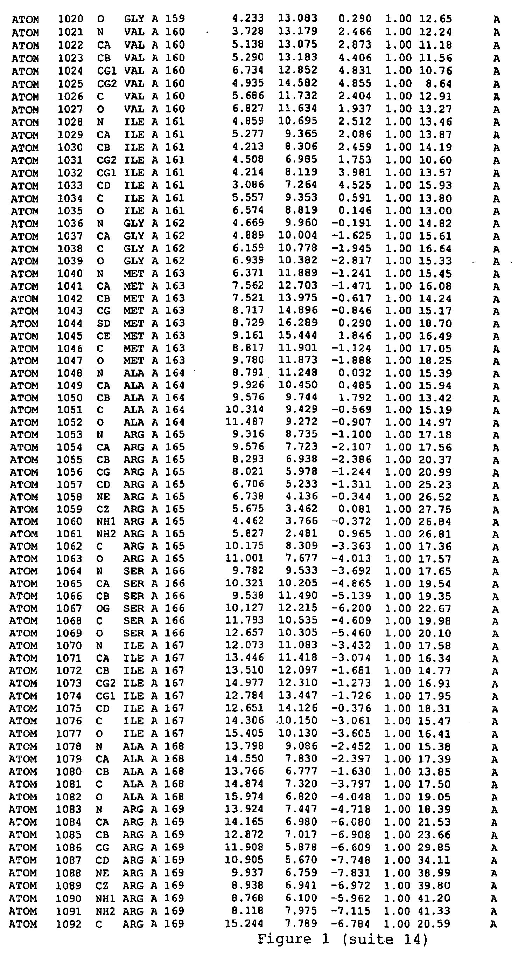

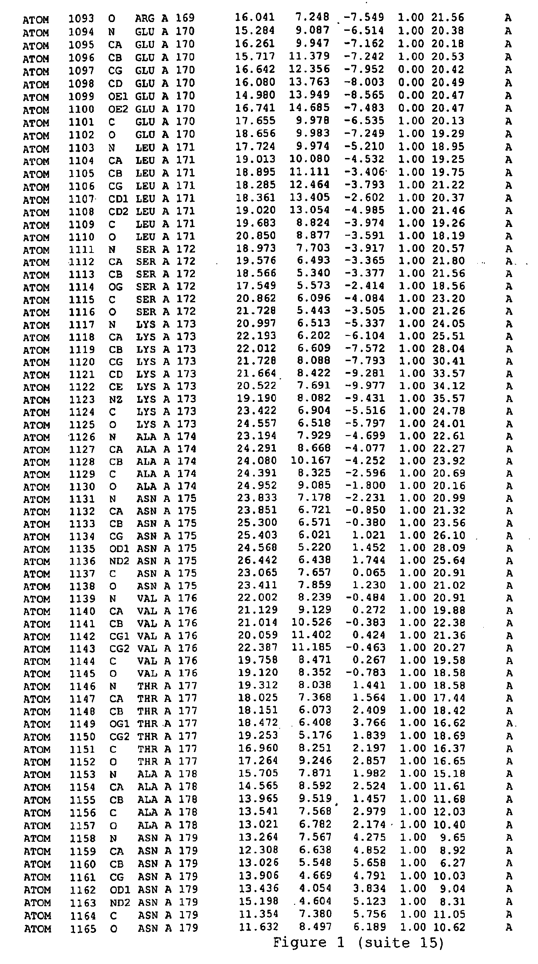

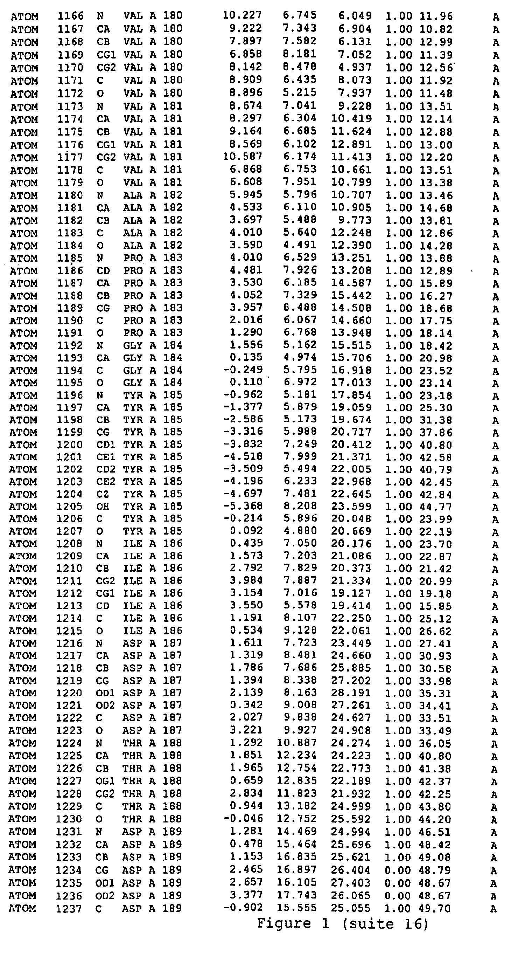

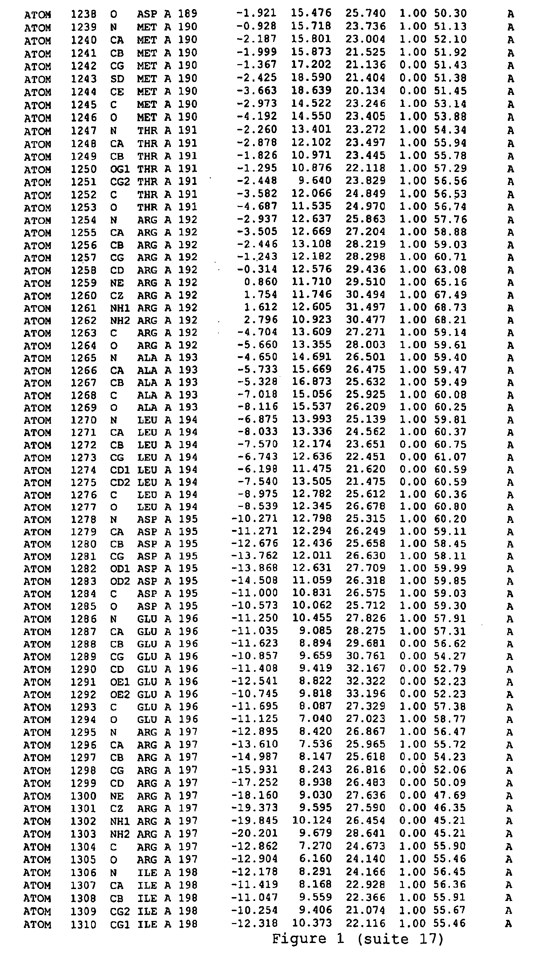

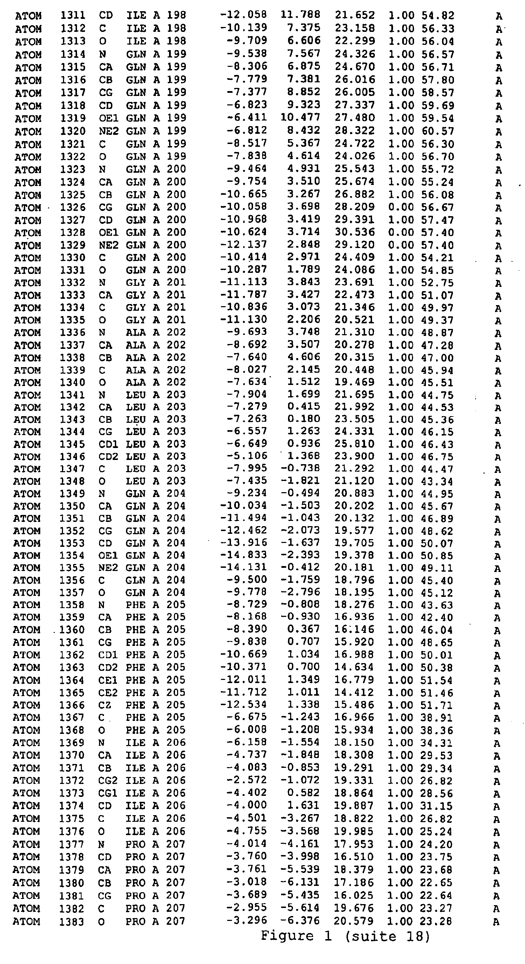

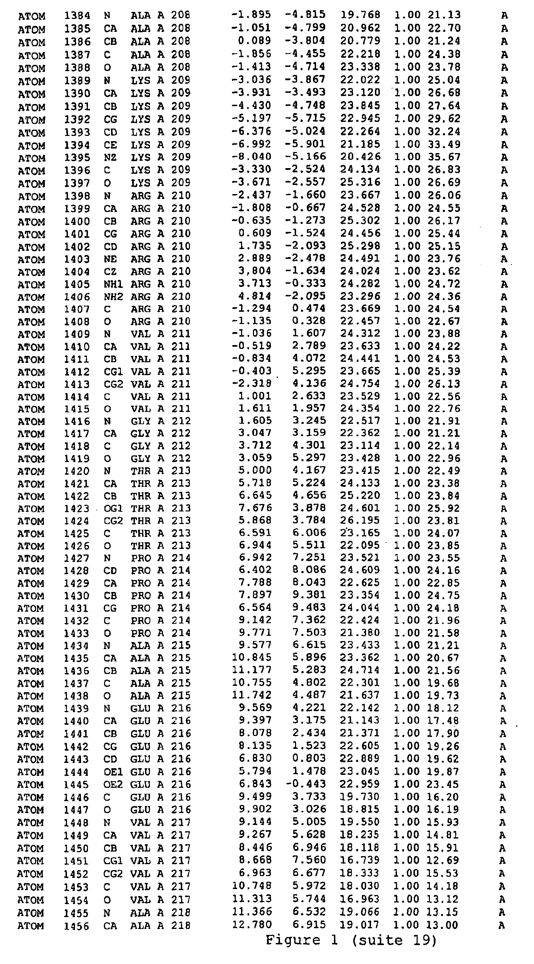

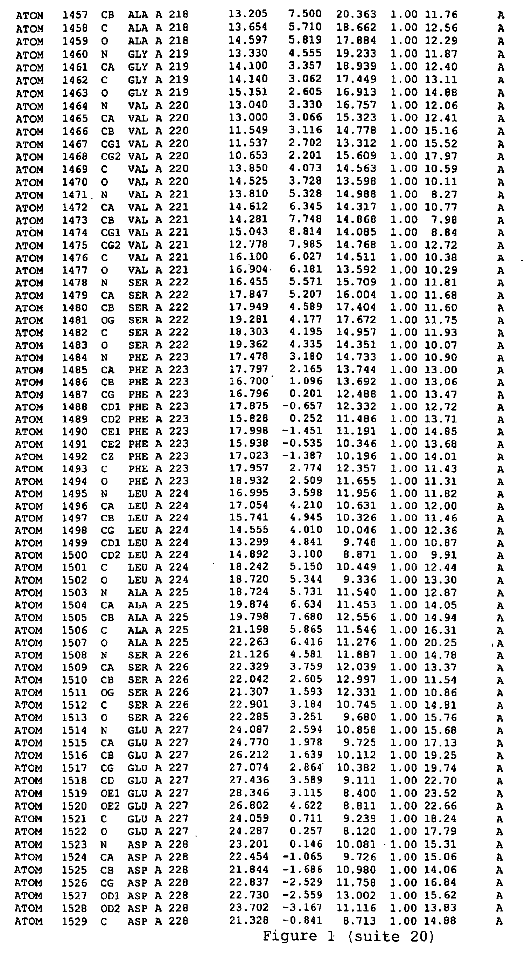

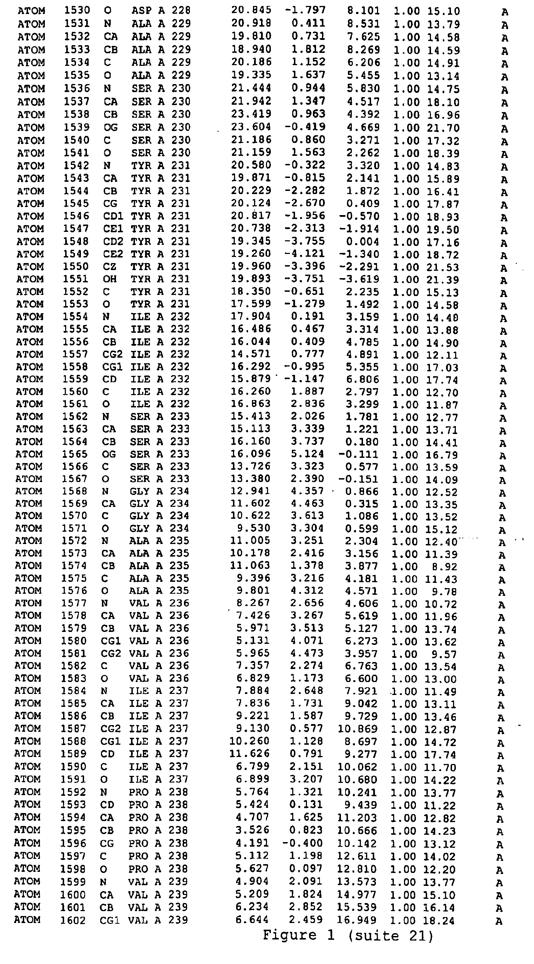

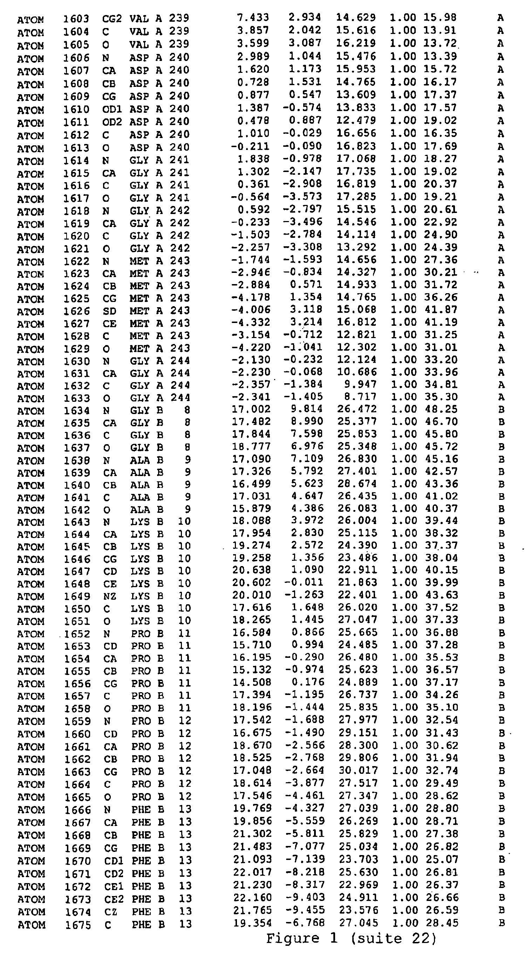

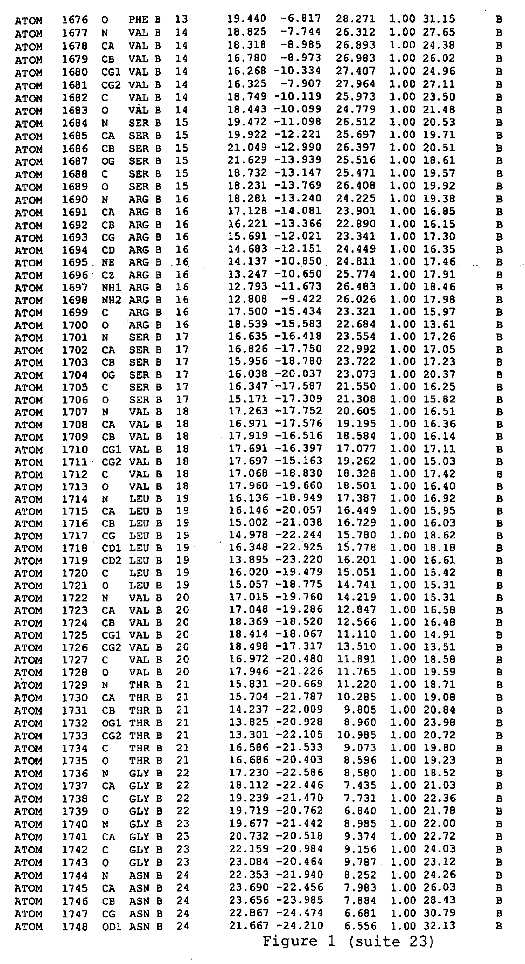

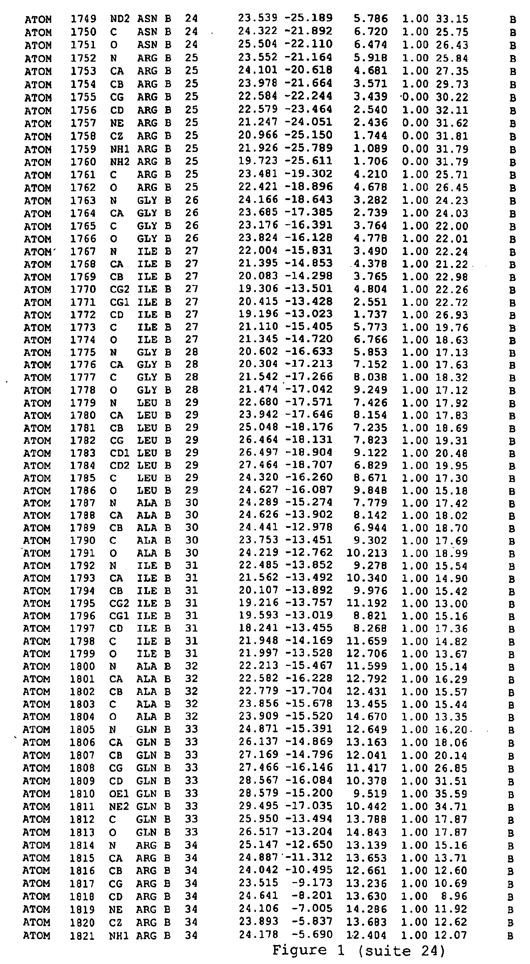

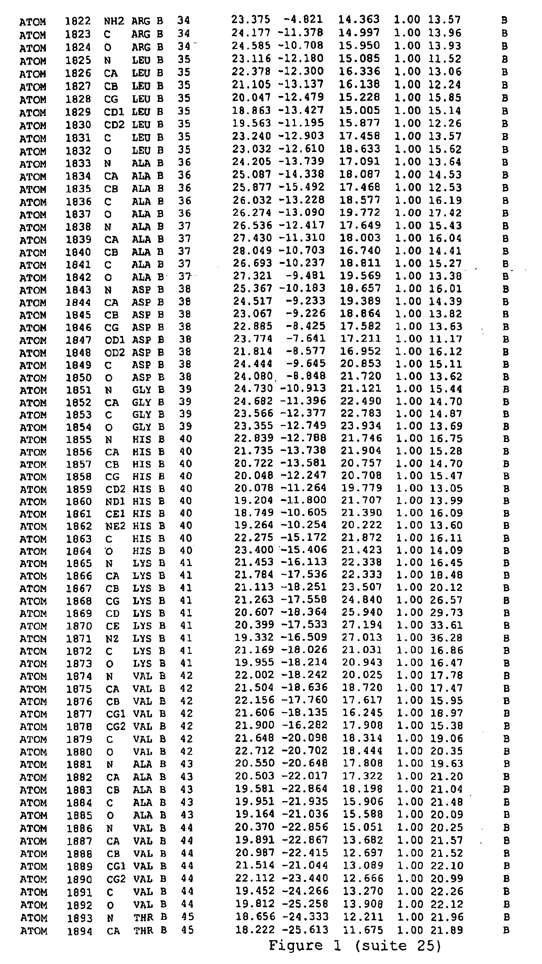

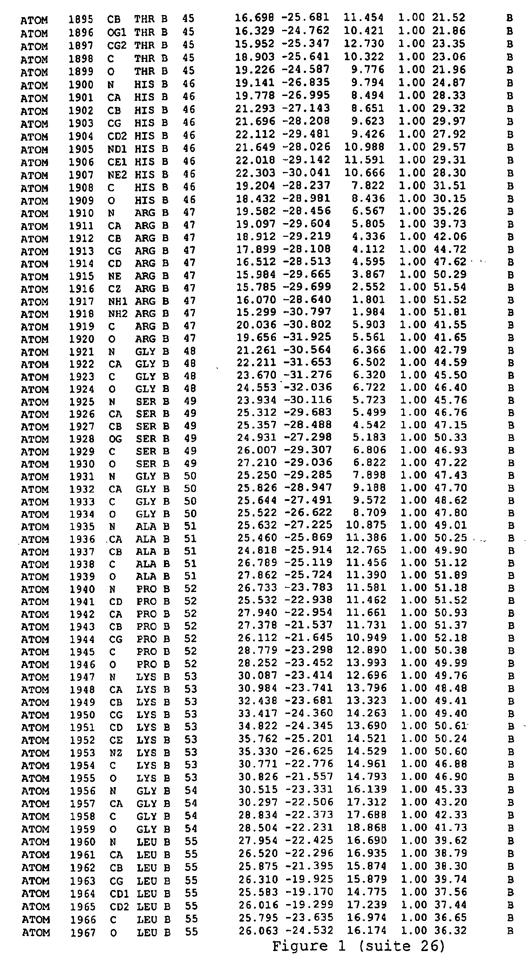

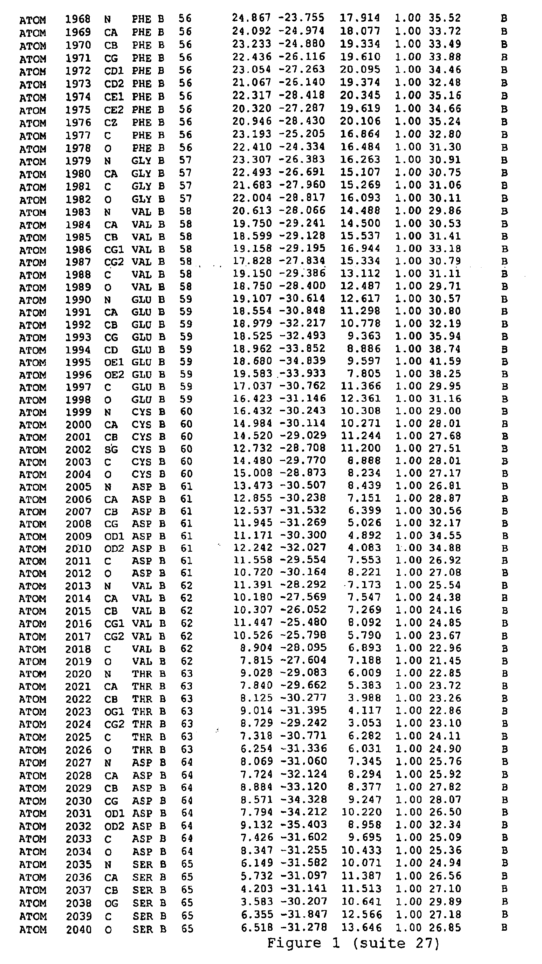

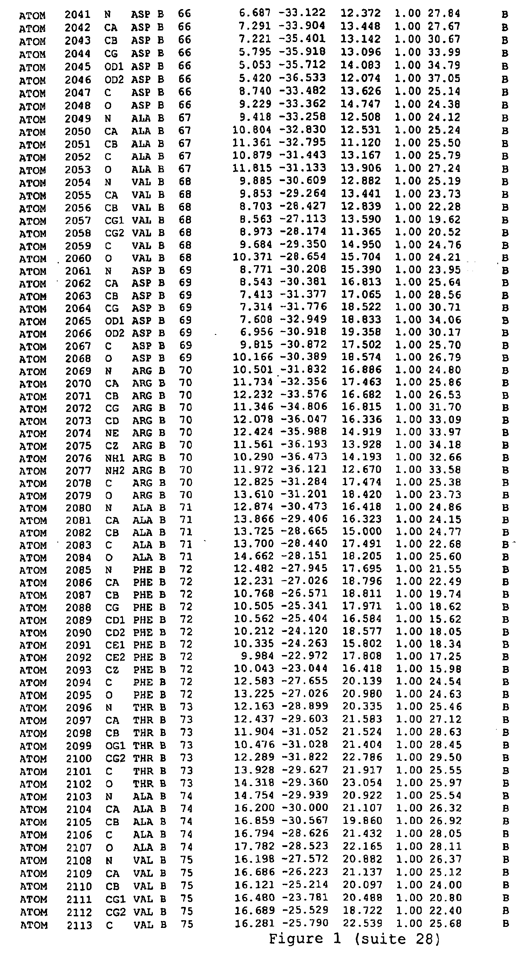

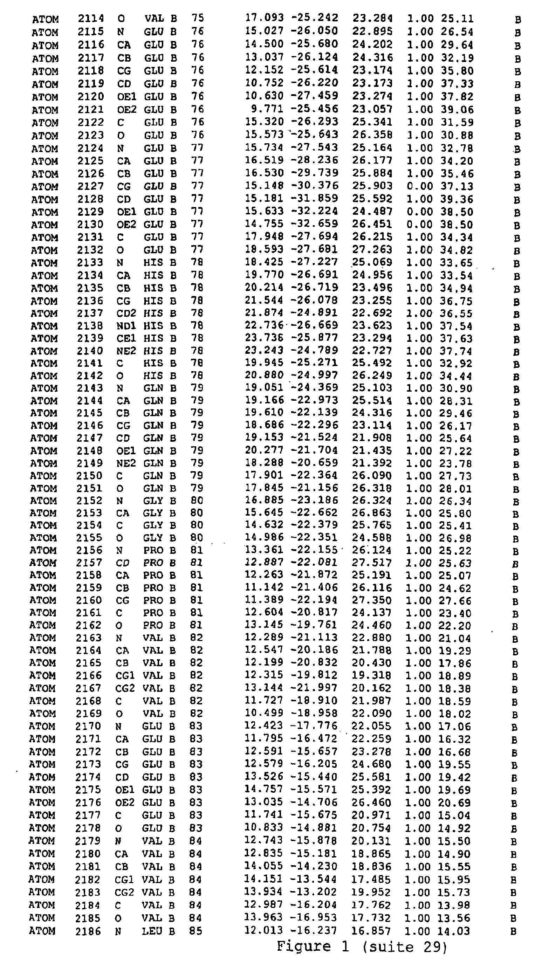

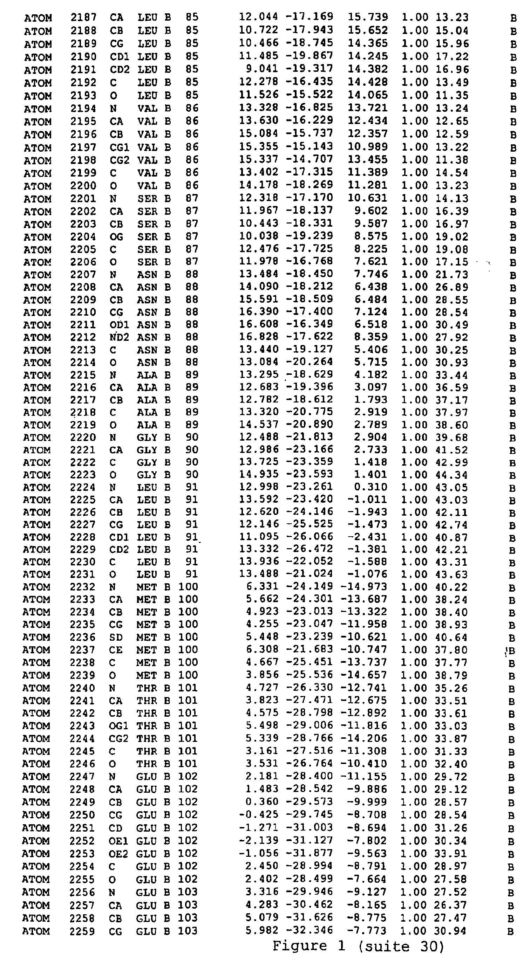

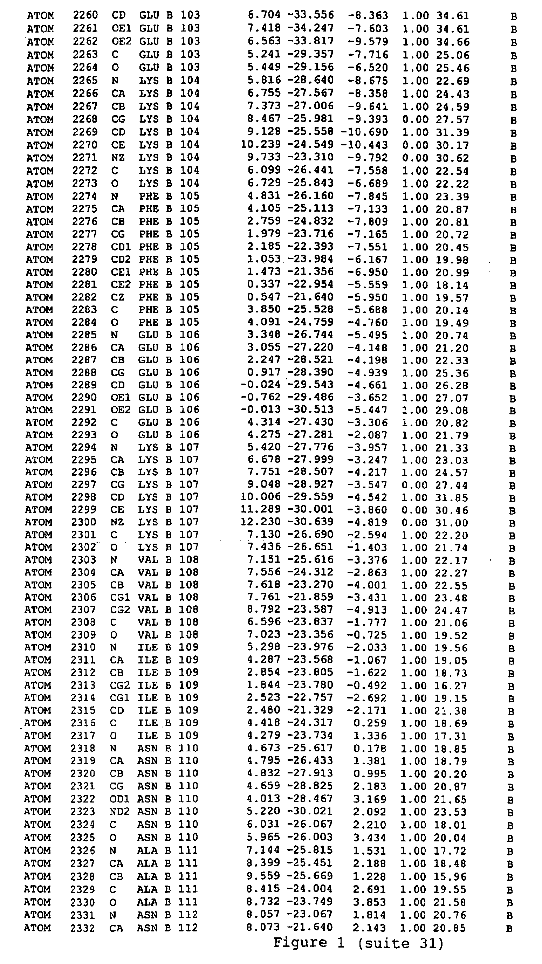

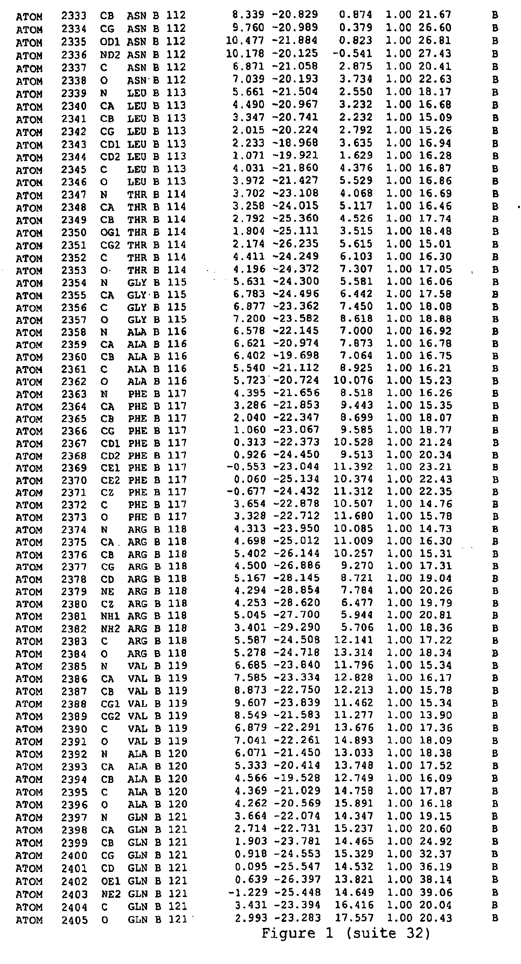

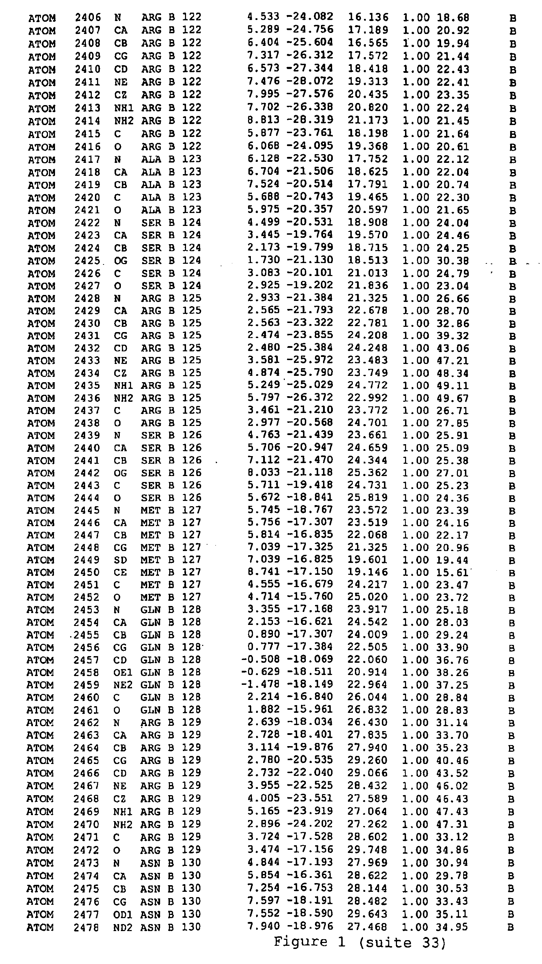

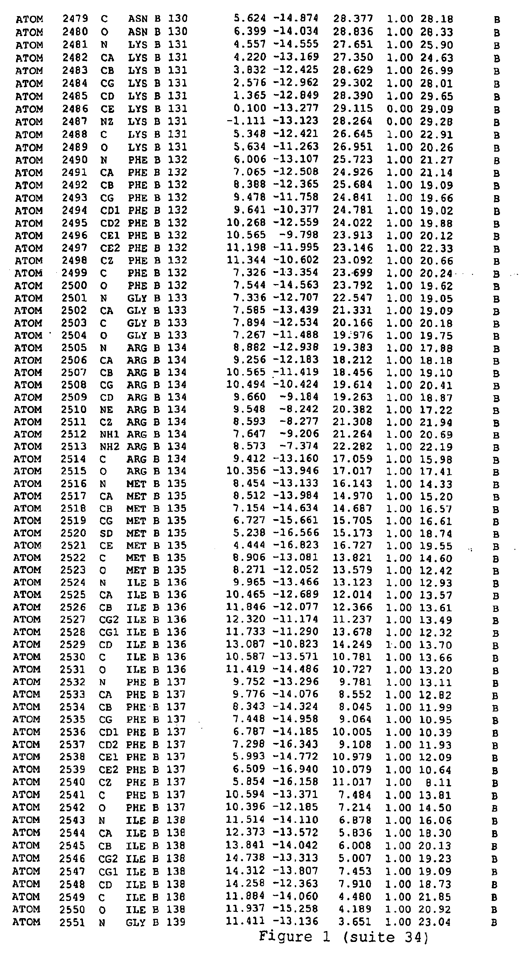

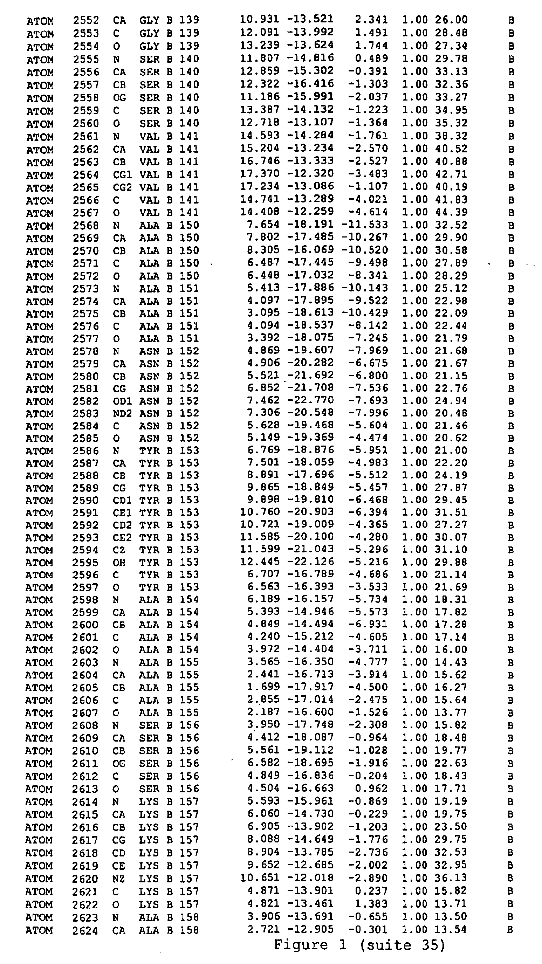

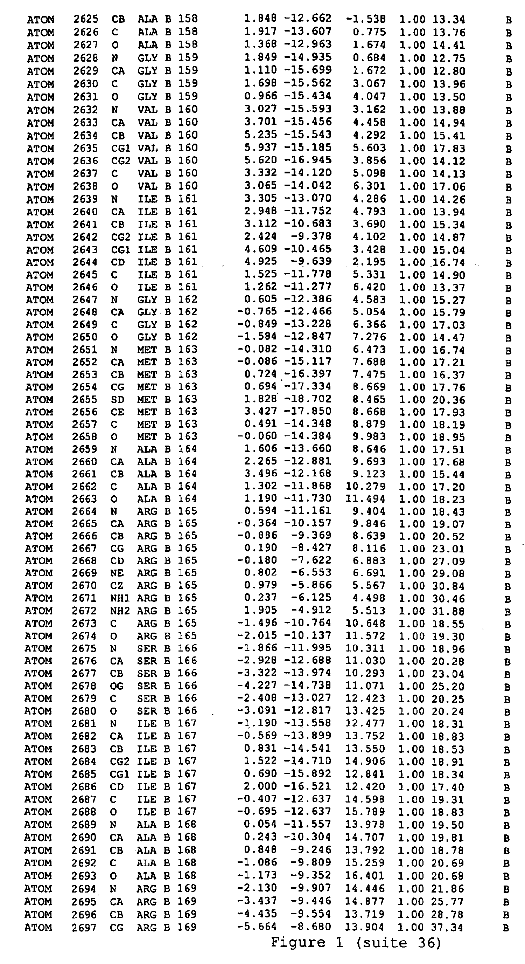

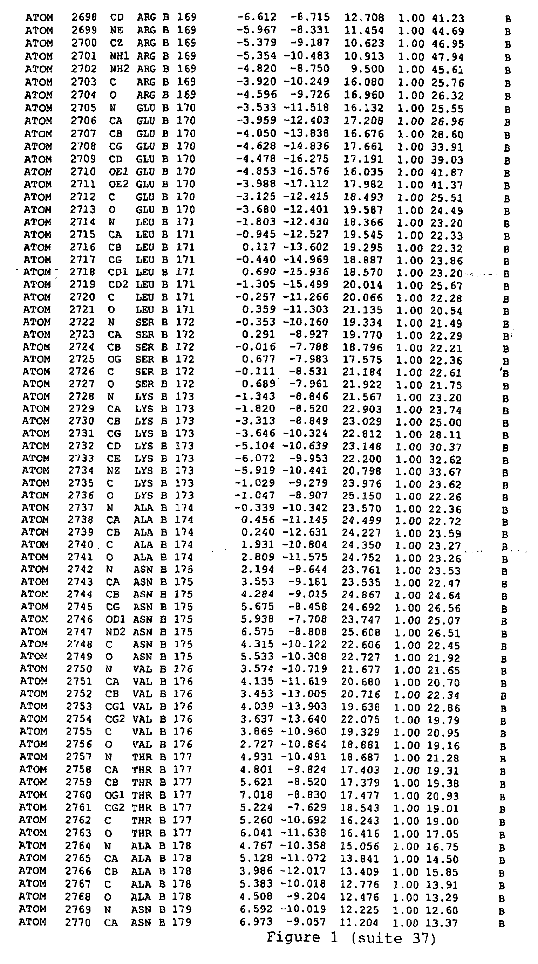

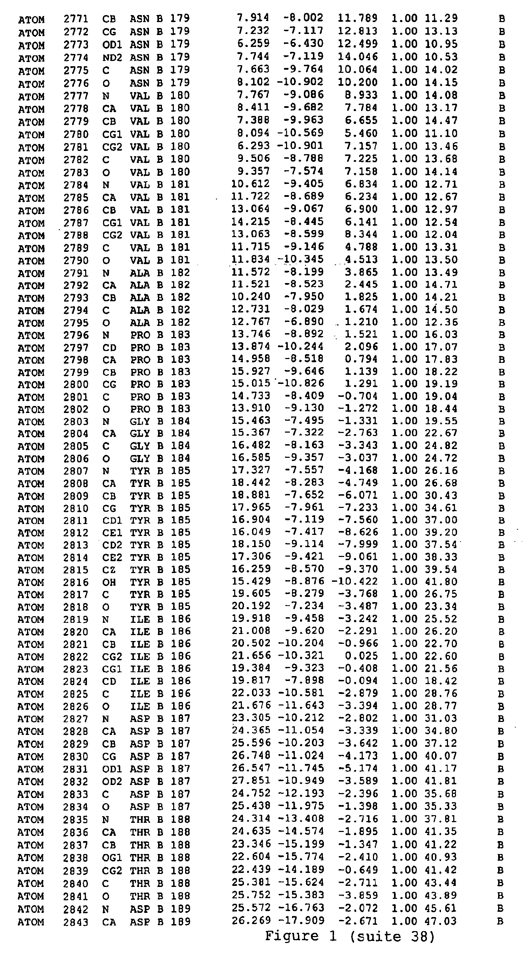

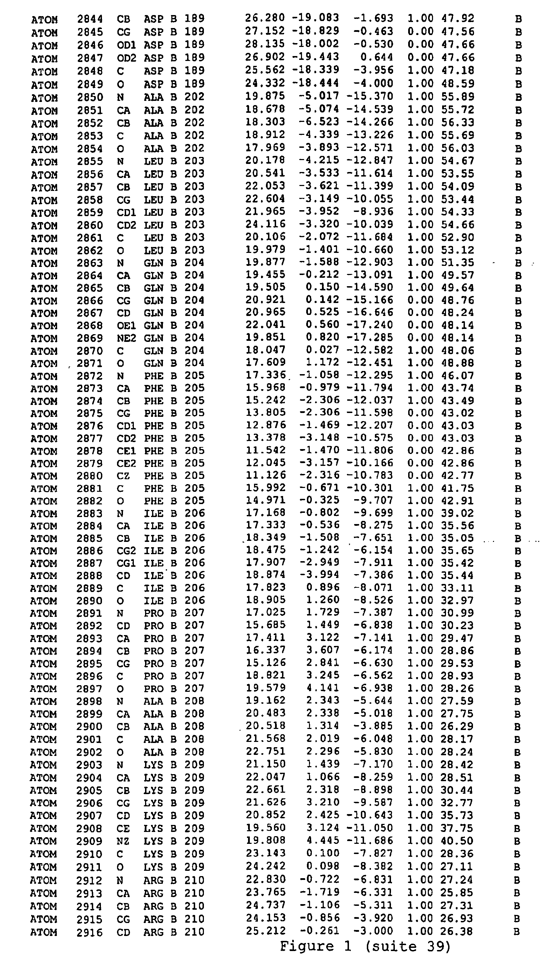

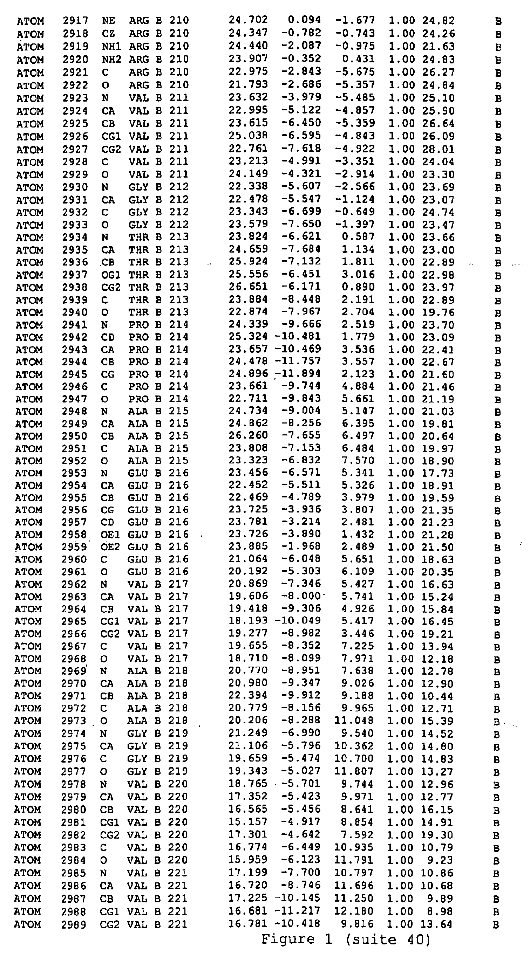

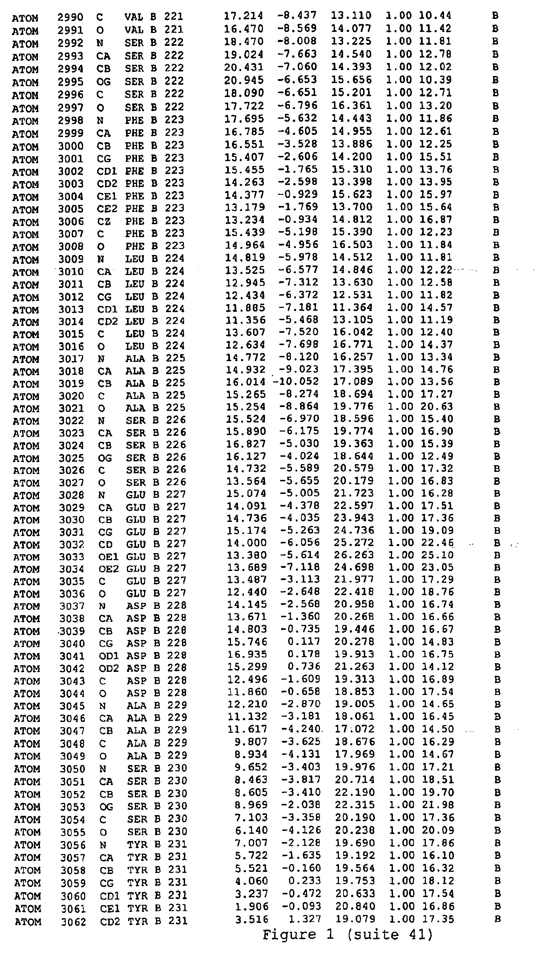

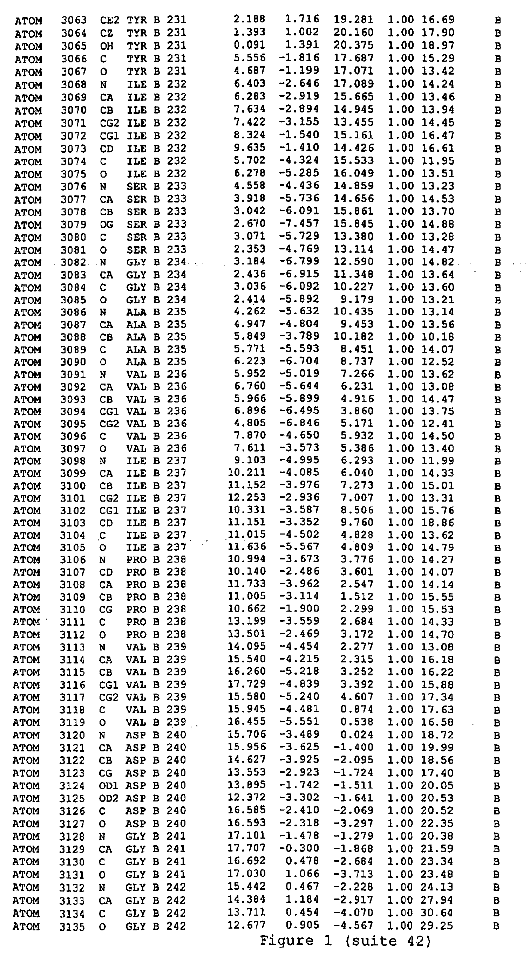

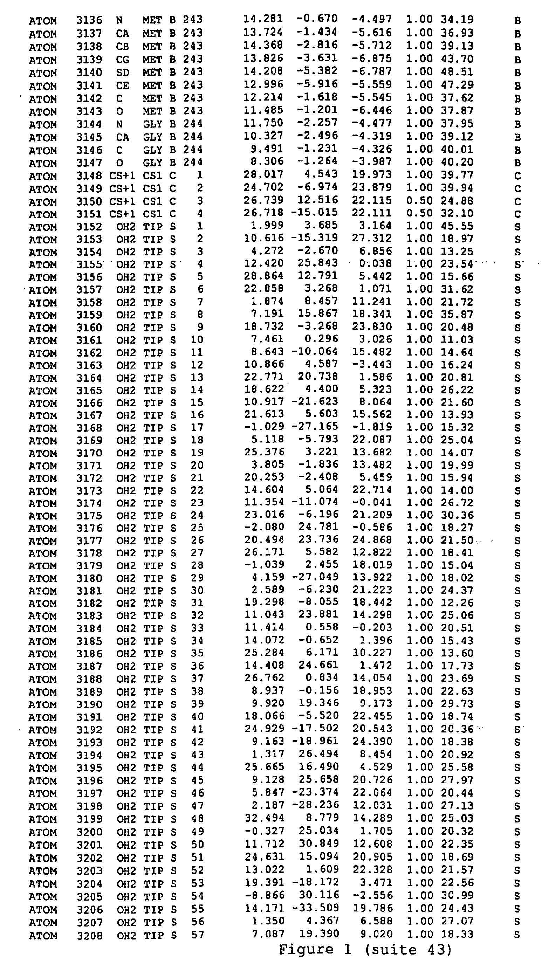

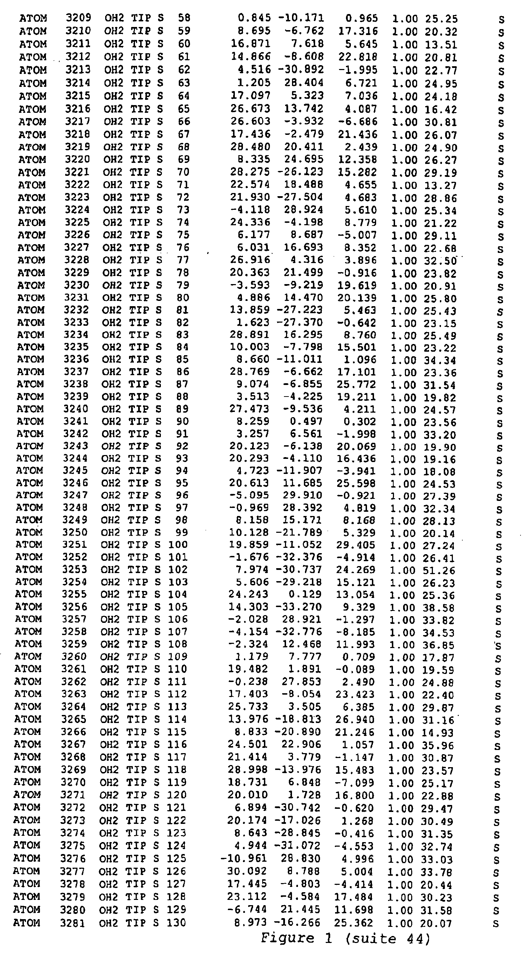

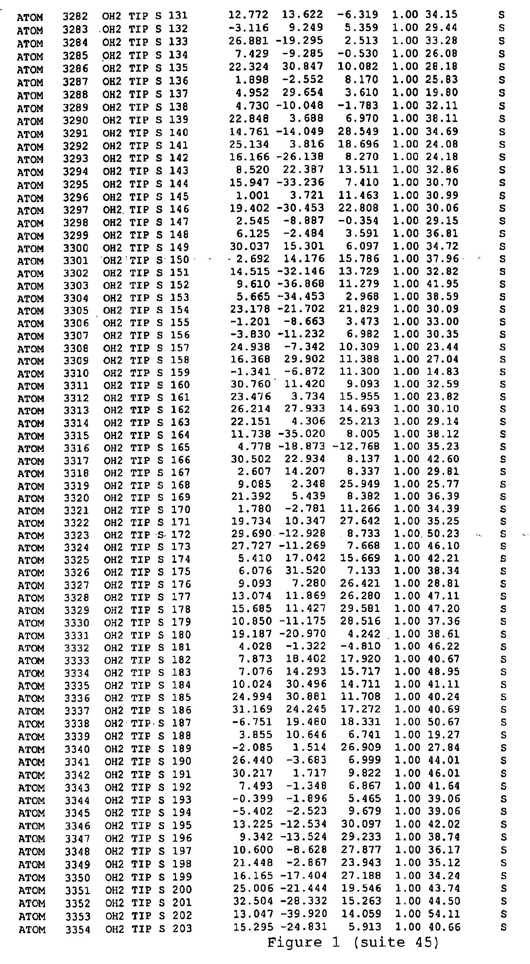

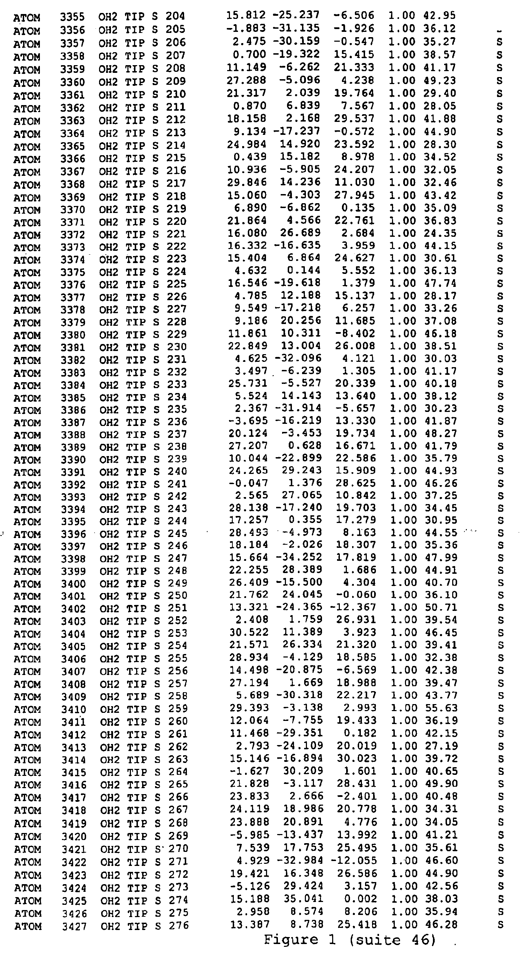

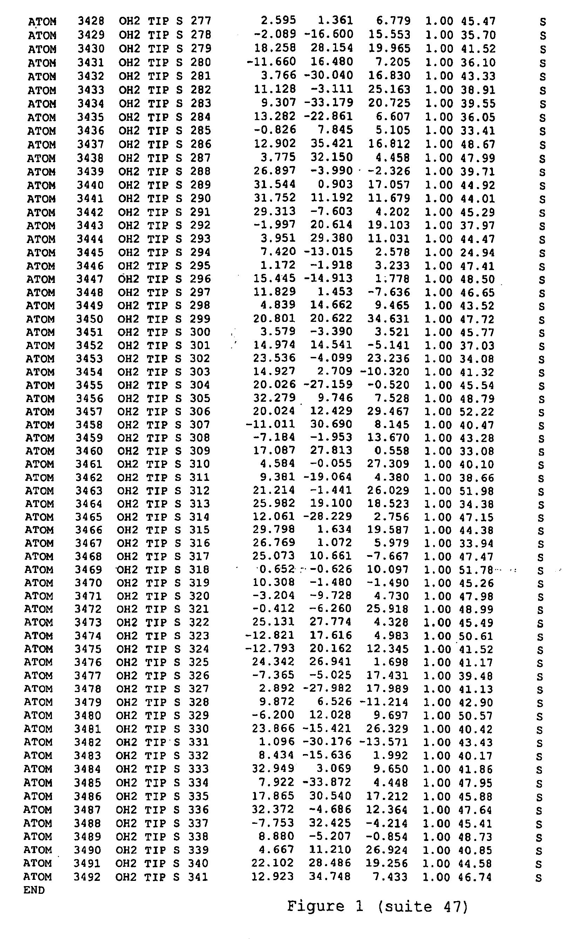

L'invention concerne également les cristaux susmentionnés de la protéine MabA recombinante correspondant à la séquence SEQ ID NO : 15, dont les coordonnées atomiques de la structure tridimensionnelle sont représentés sur la figure 1, et présentant les caractéristiques suivantes :

- paramètre de mailles :
  - a = 81.403 angströms, b = 116.801 angströms, c = 52.324 angströms,
  - $\alpha = \beta = 90.00$ °, $\gamma = 122.30$ °,
- groupe d'espace : C2,
- diffraction maximale = 2,05 angströms.

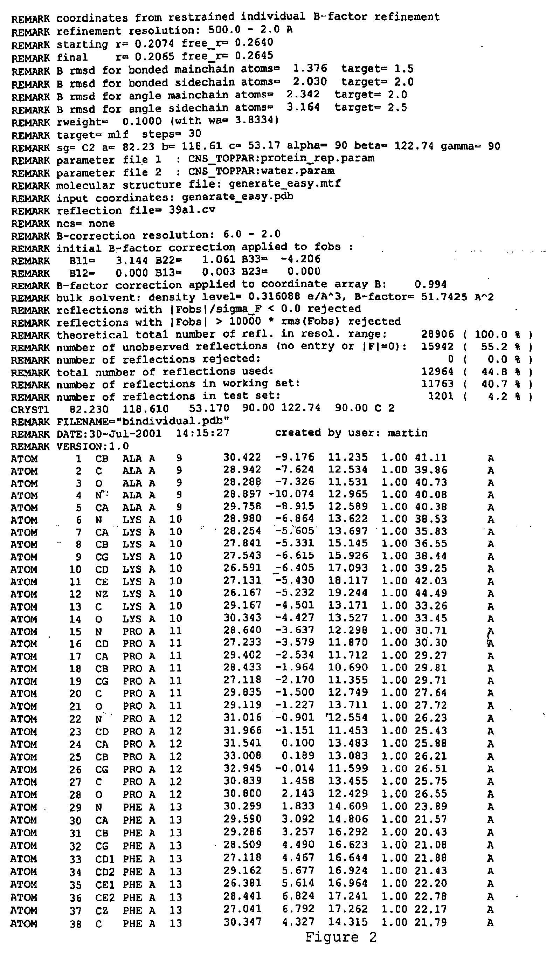

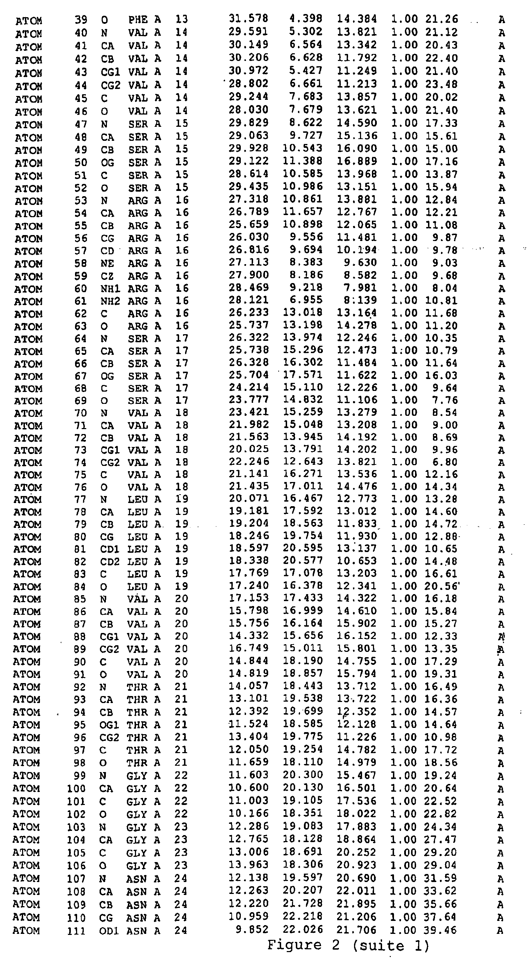

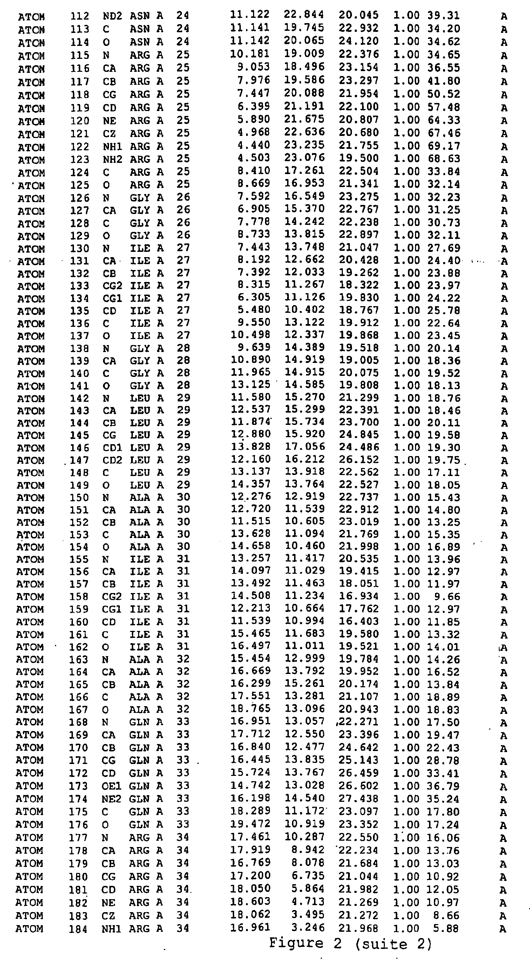

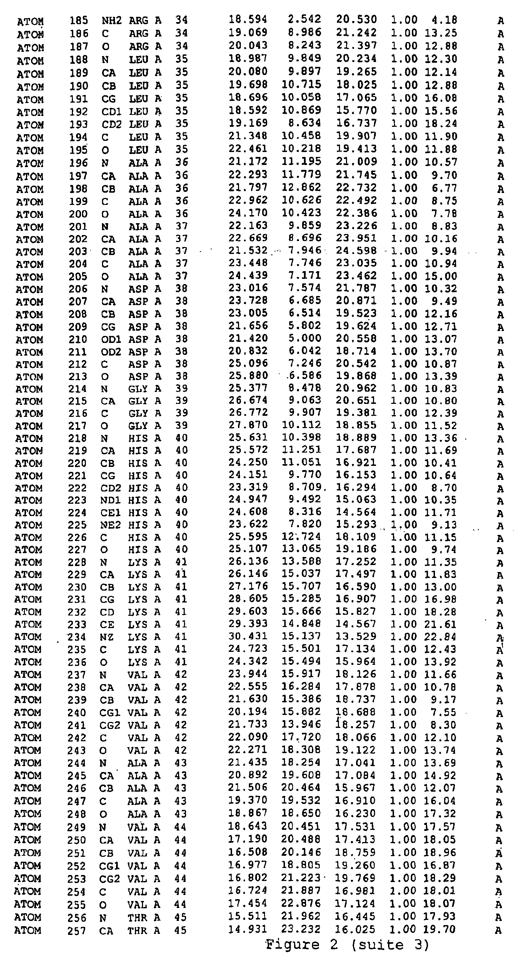

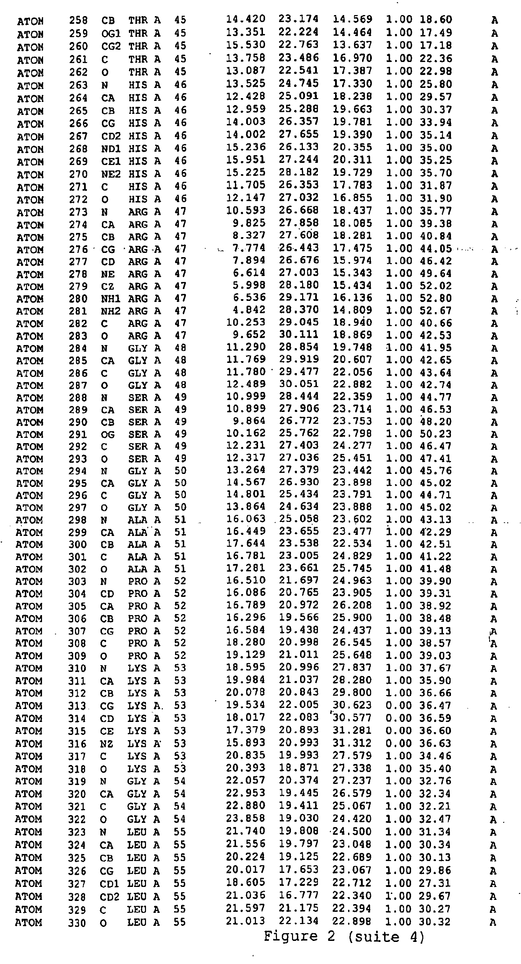

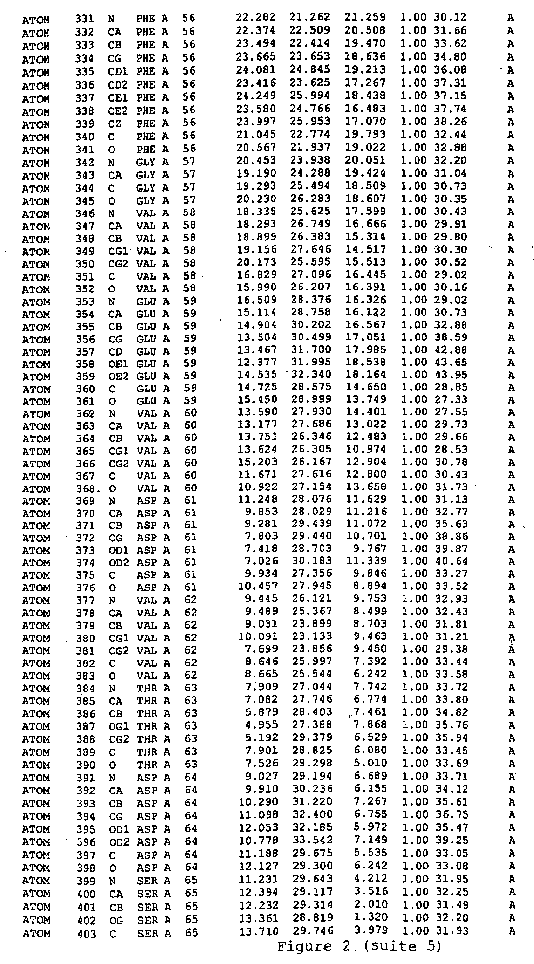

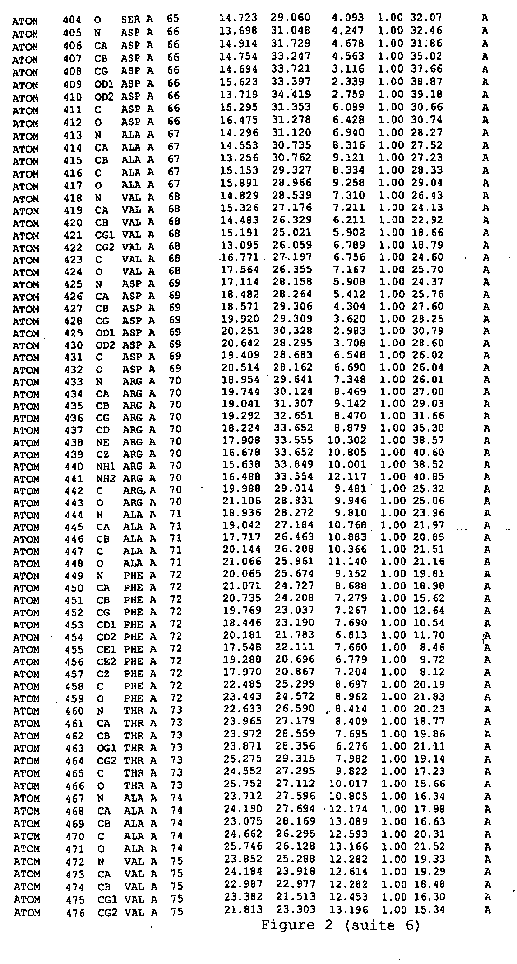

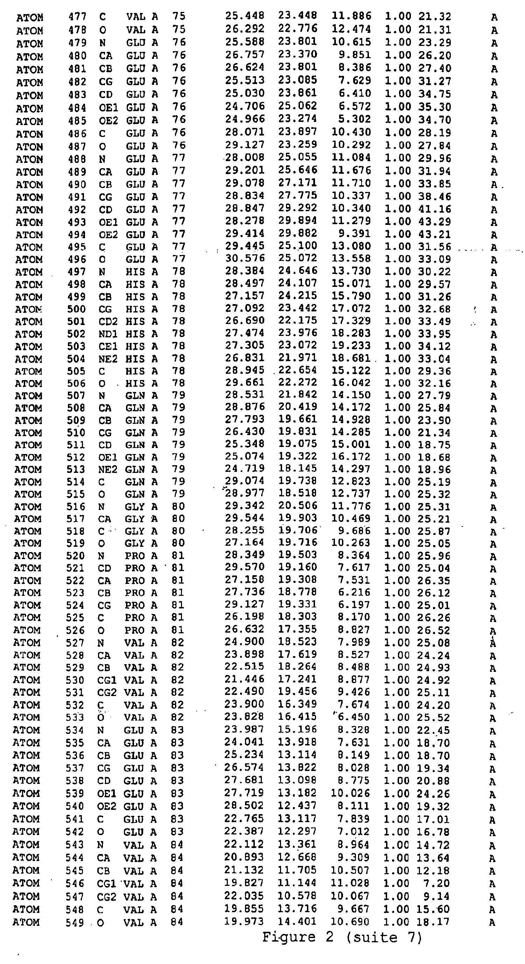

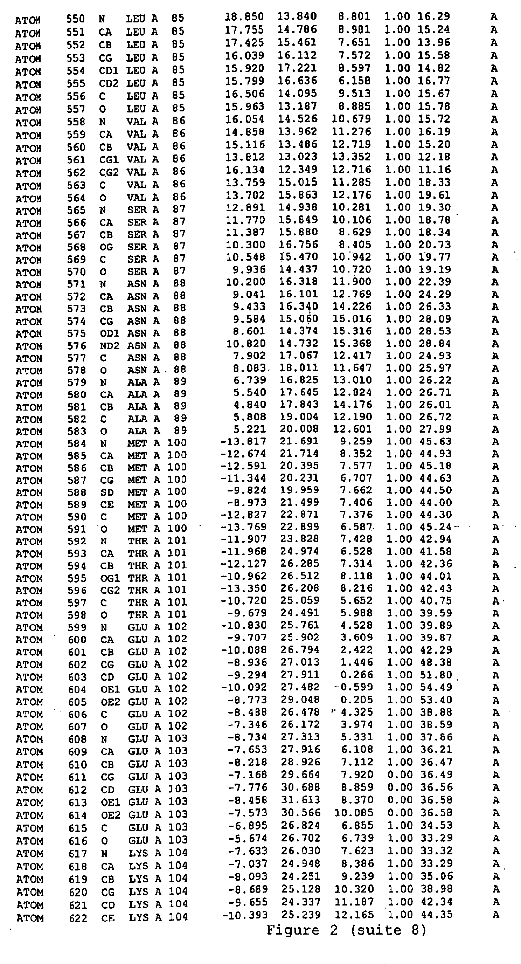

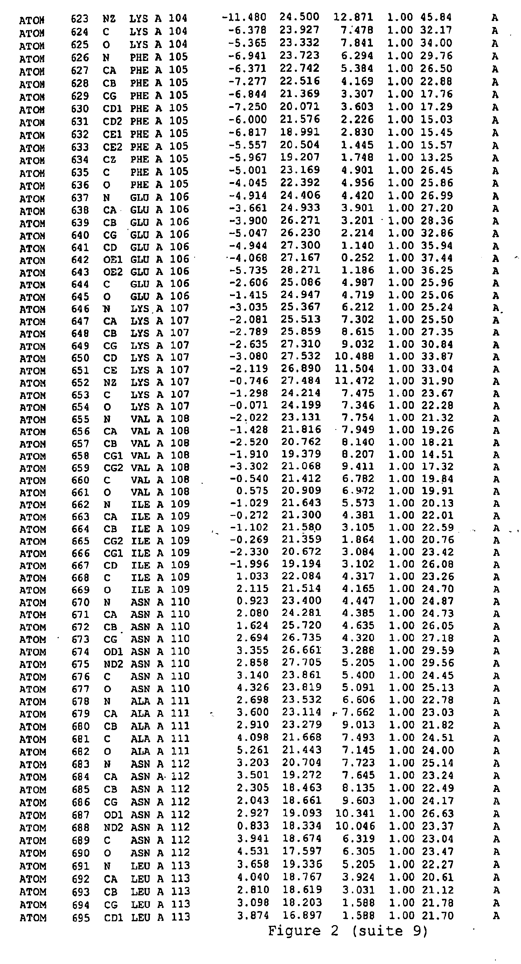

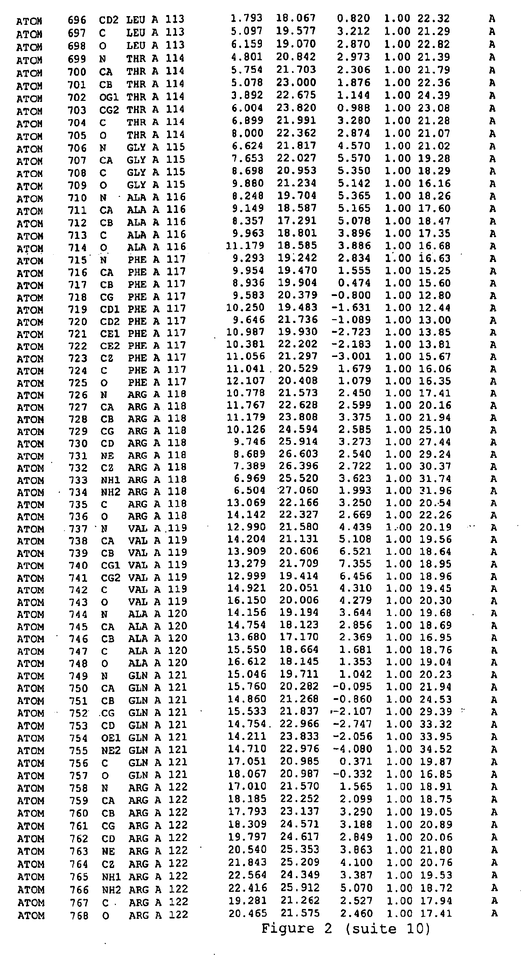

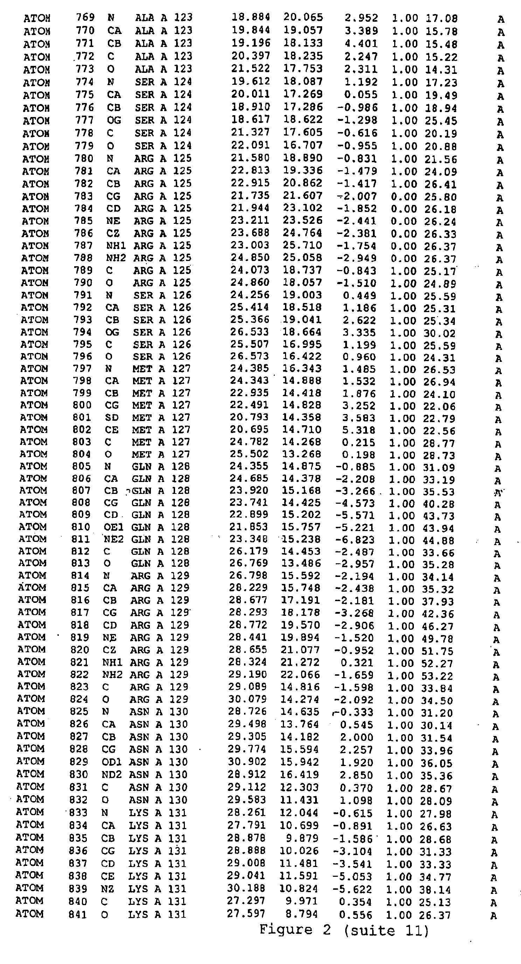

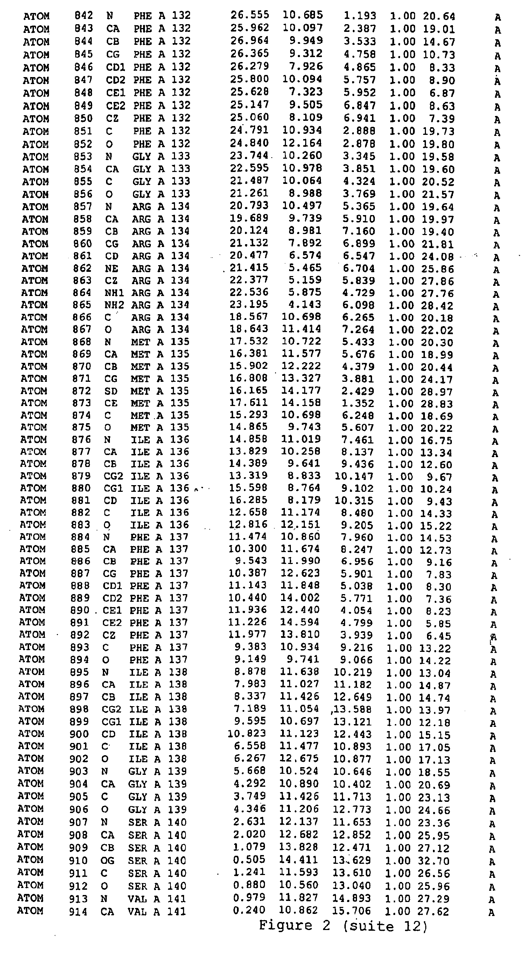

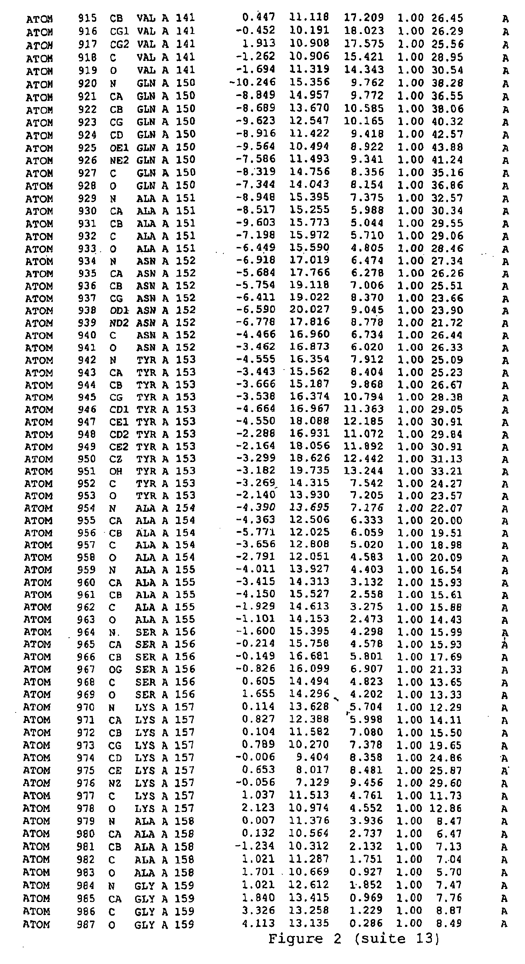

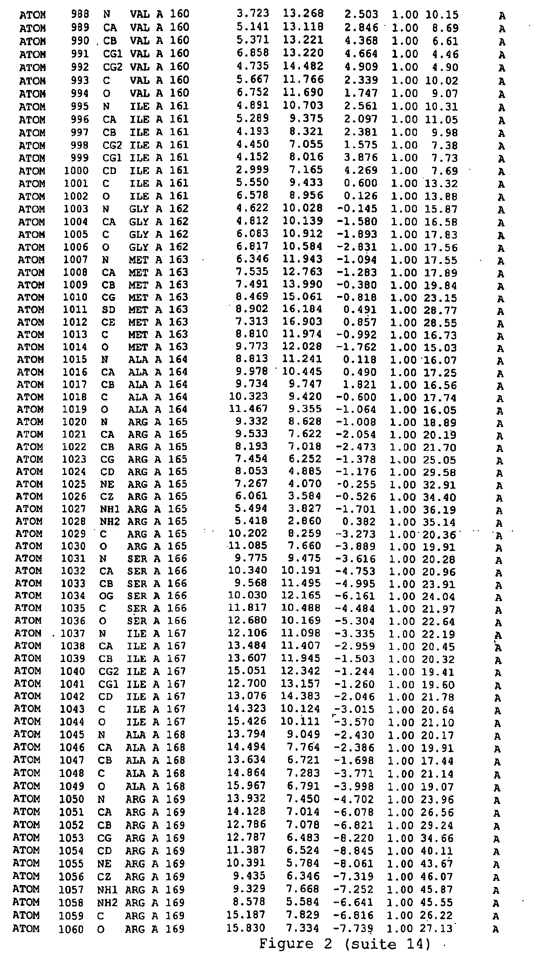

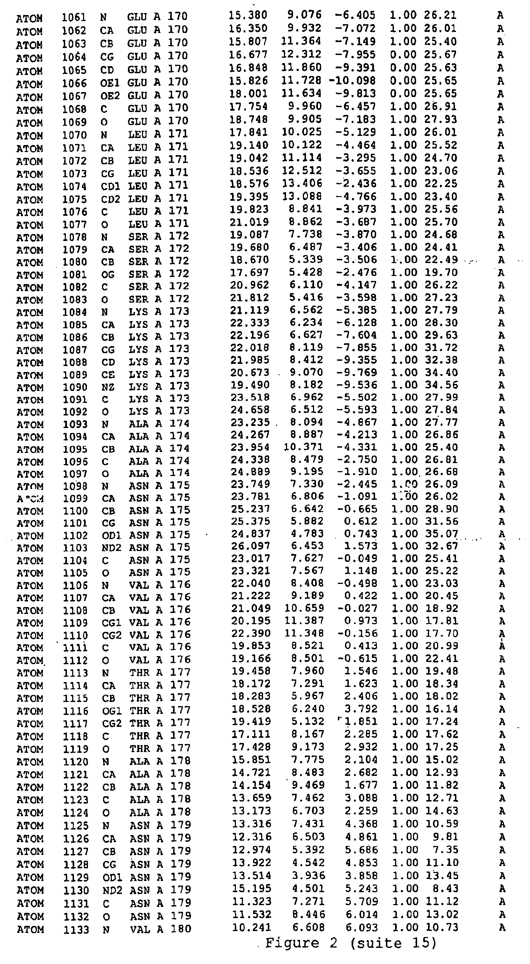

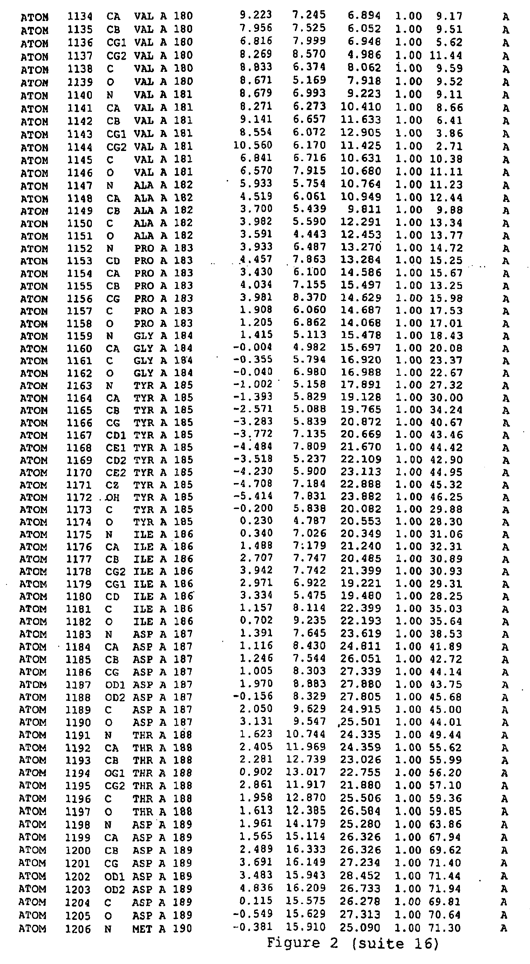

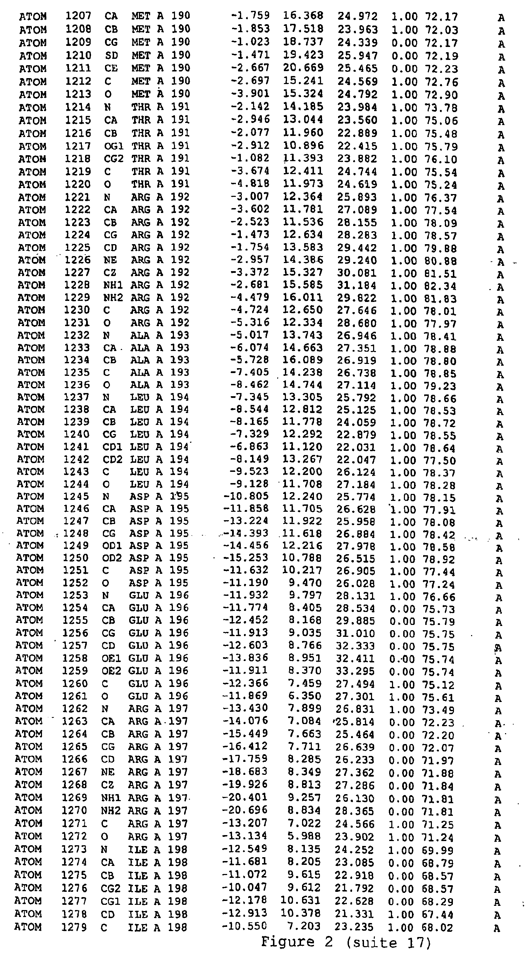

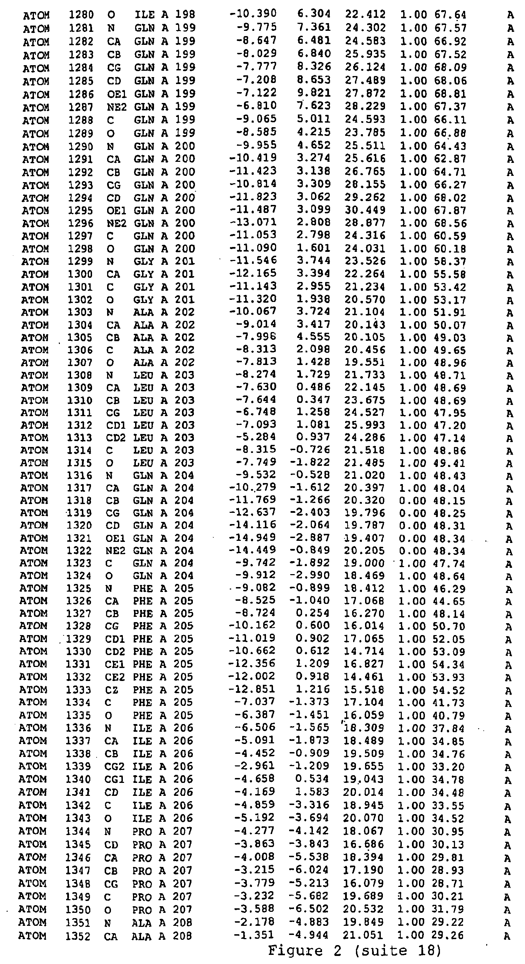

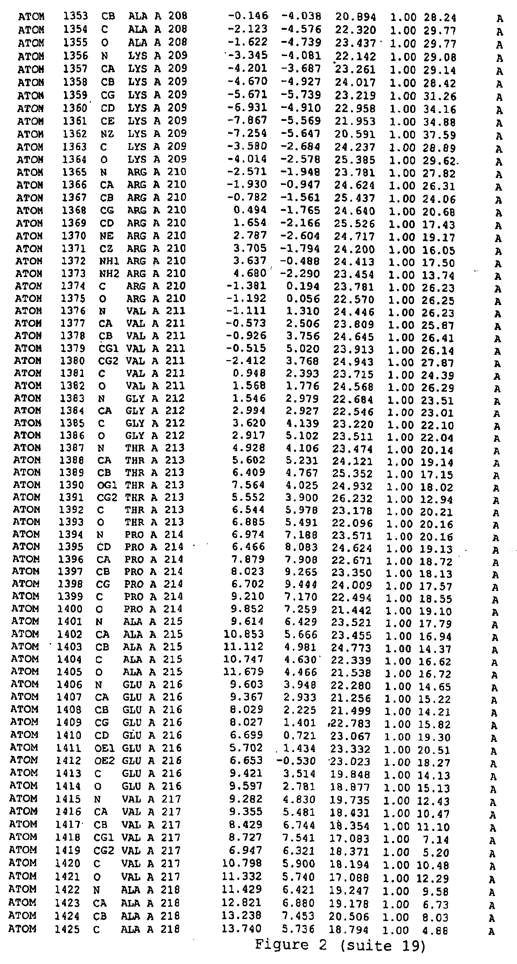

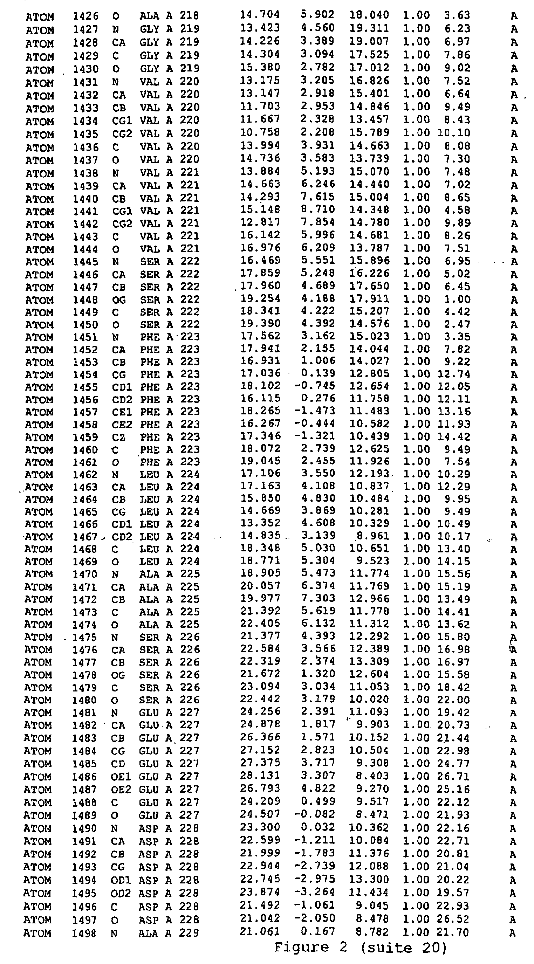

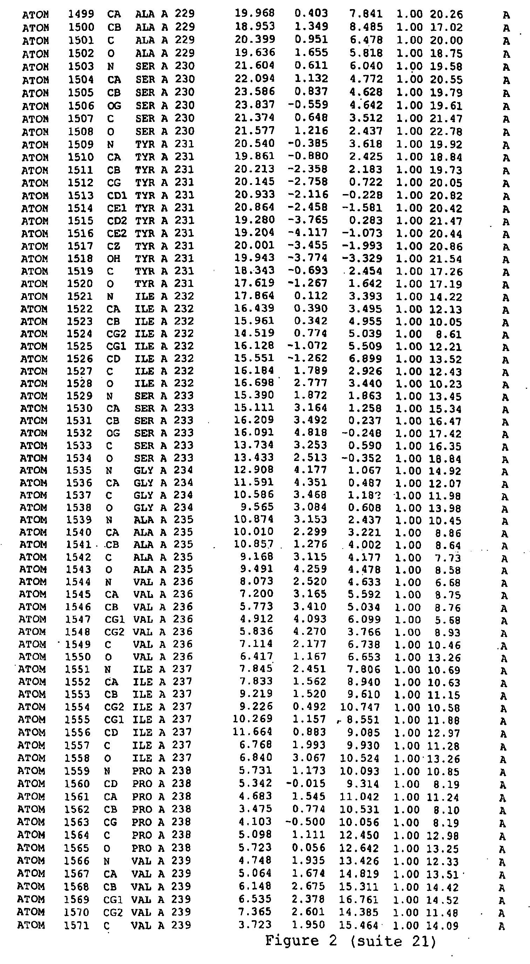

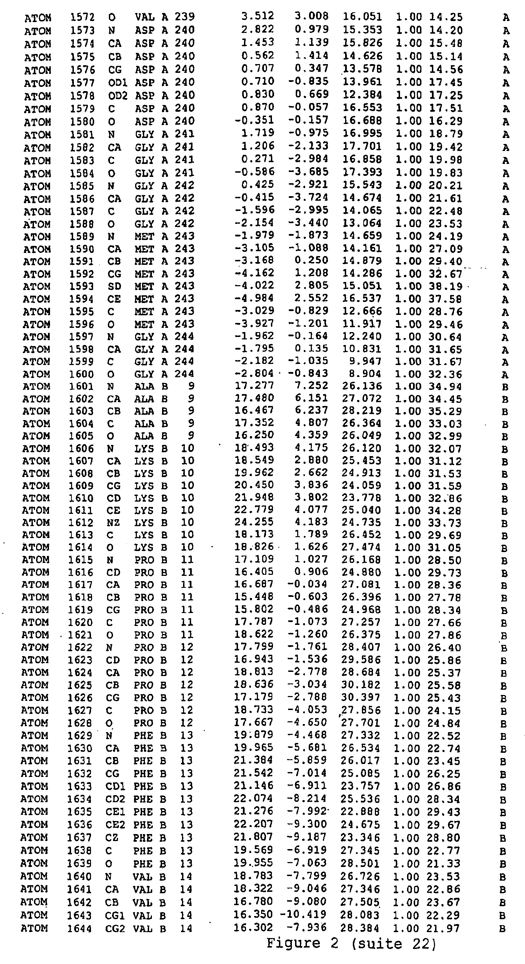

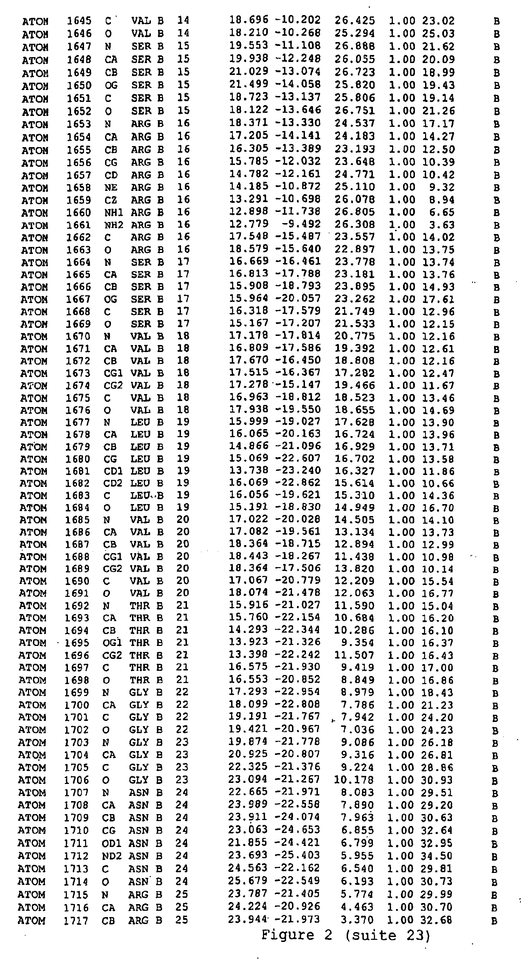

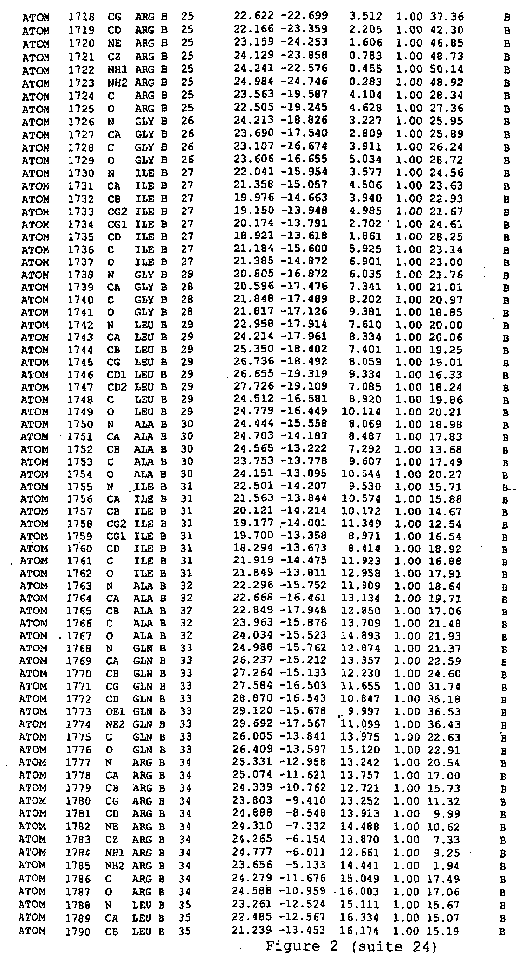

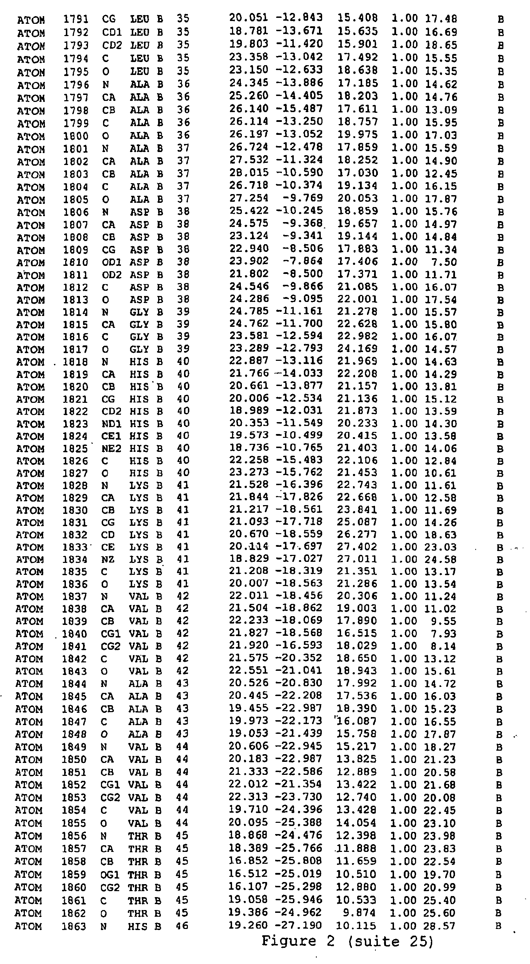

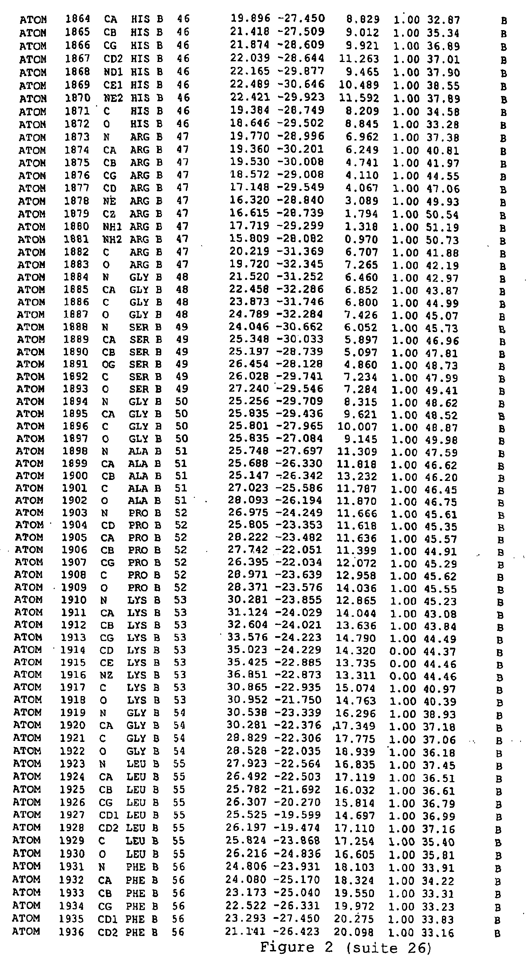

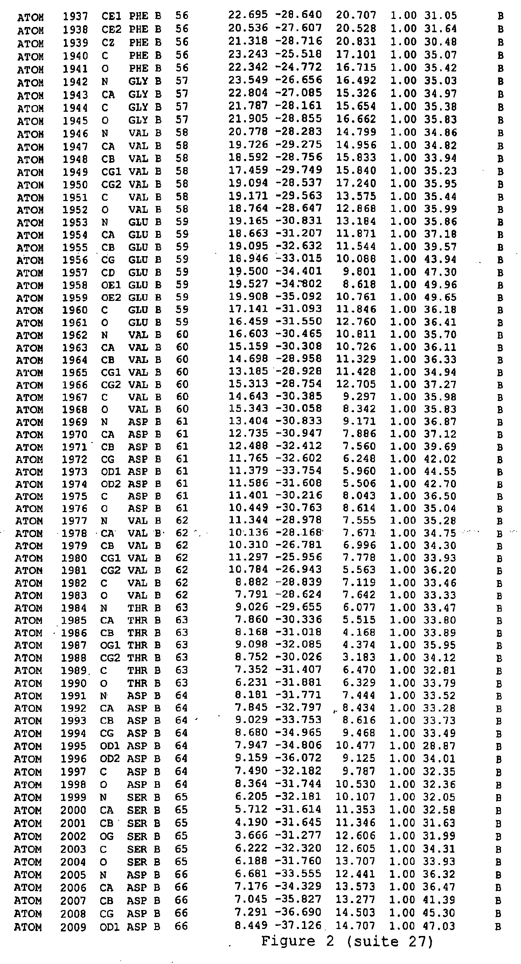

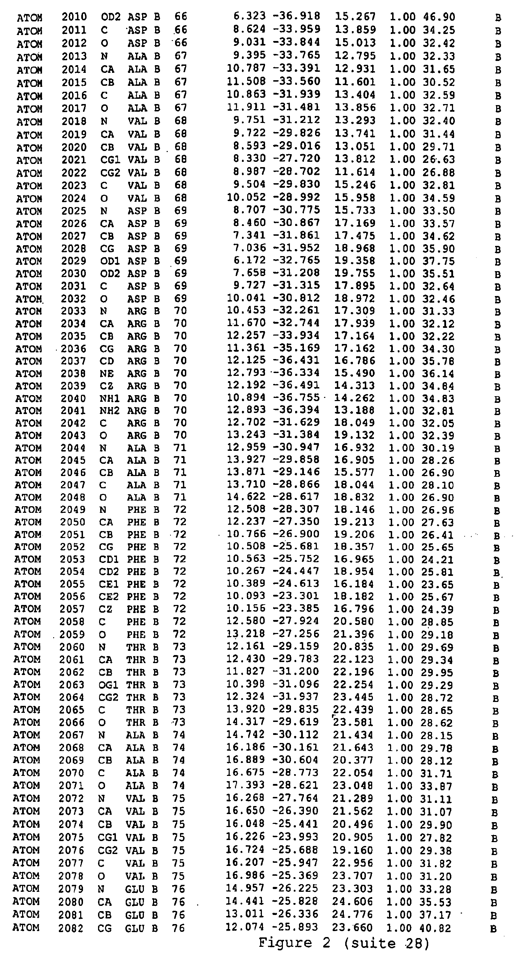

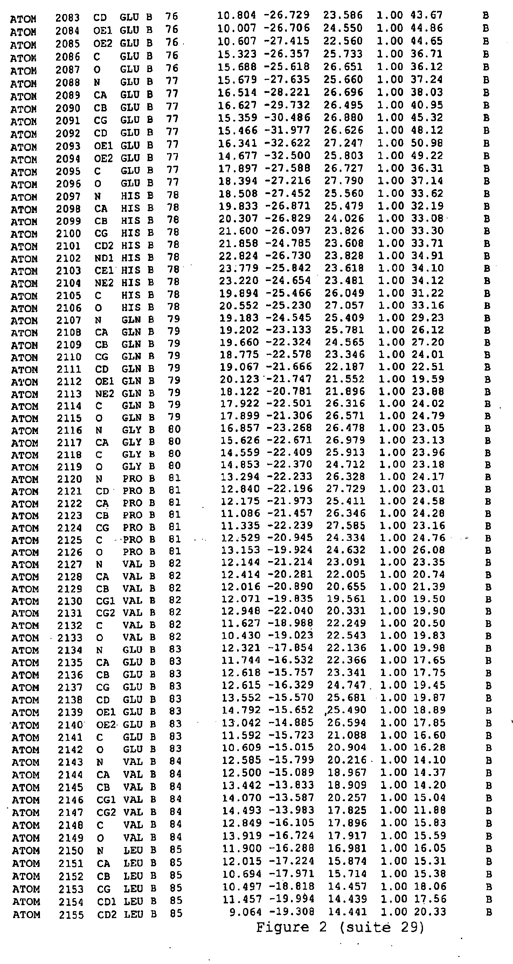

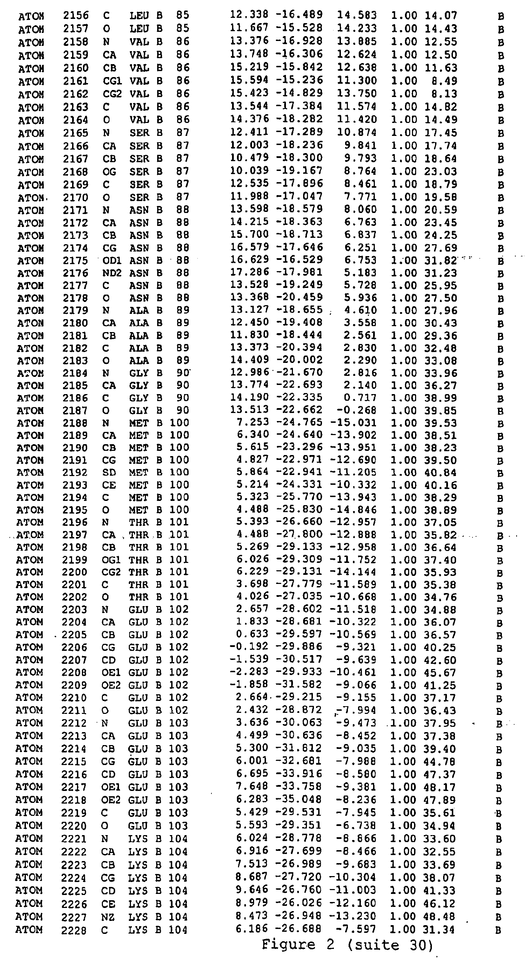

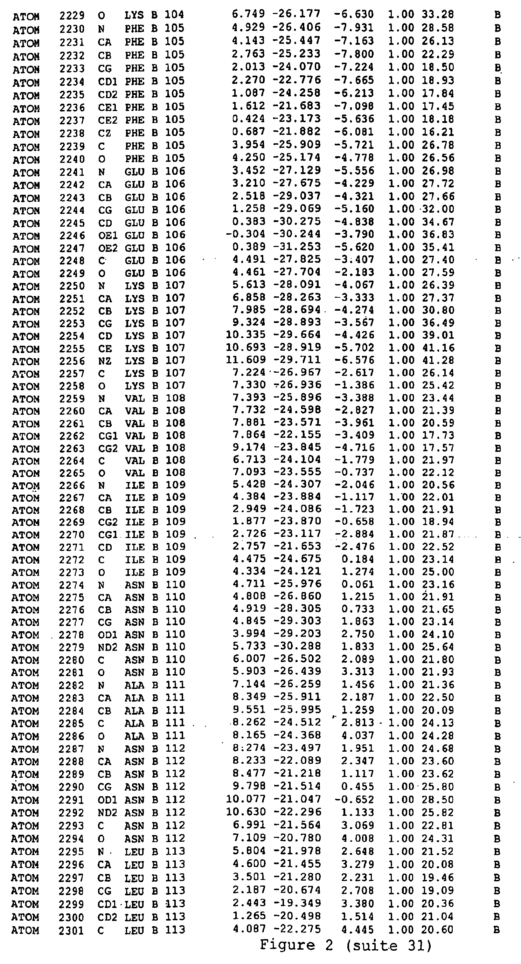

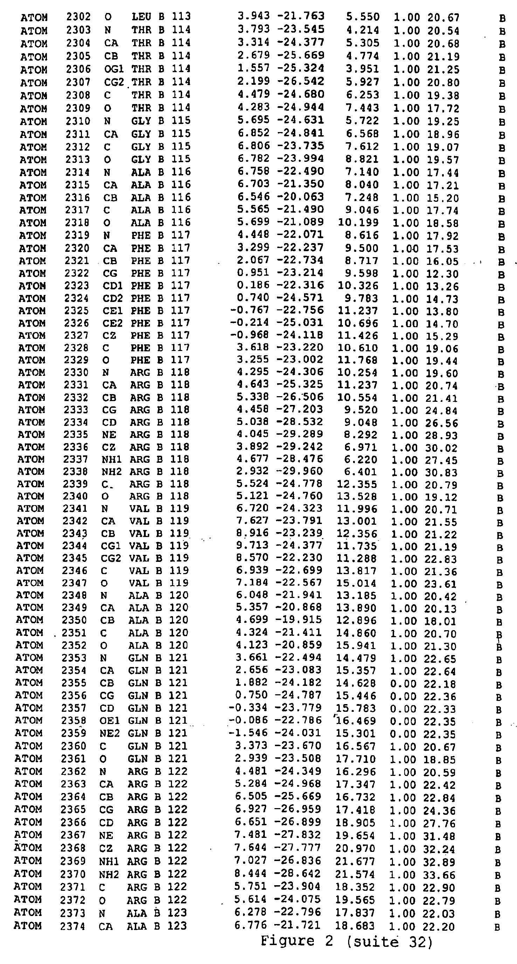

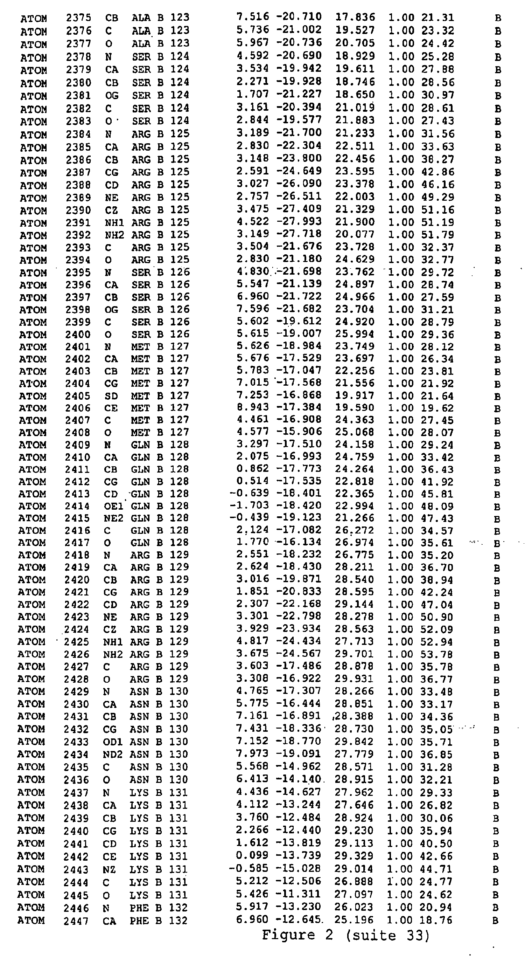

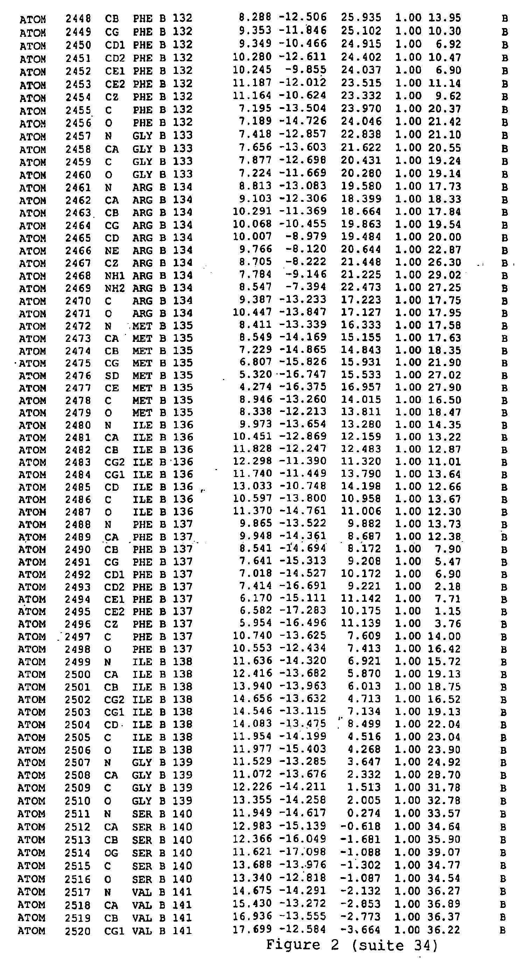

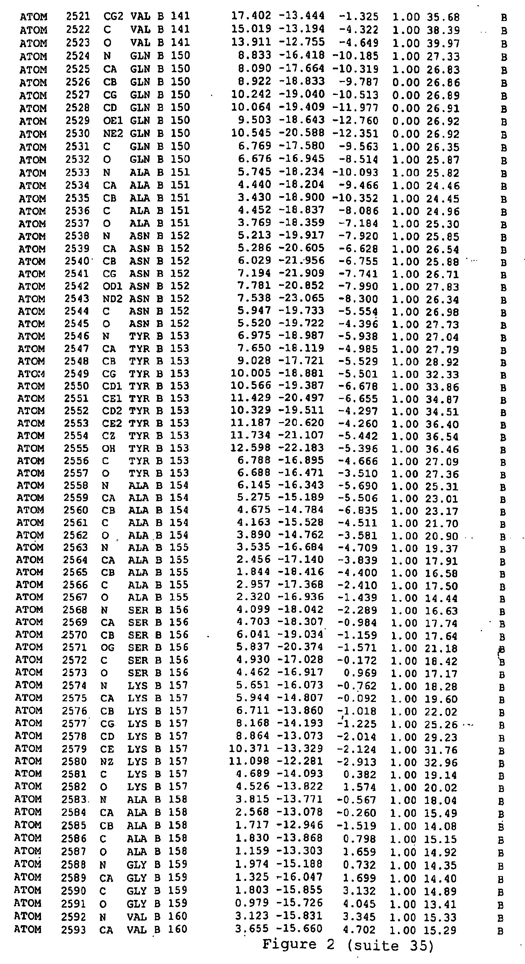

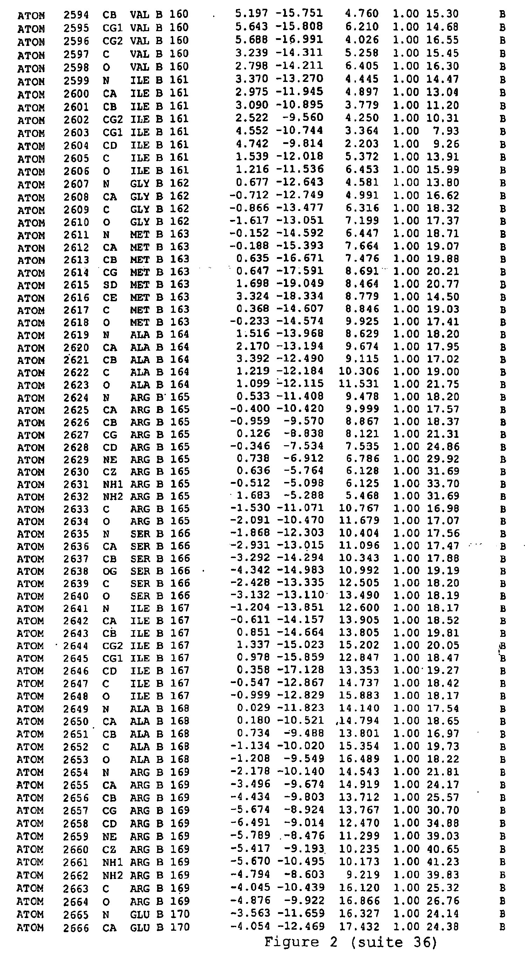

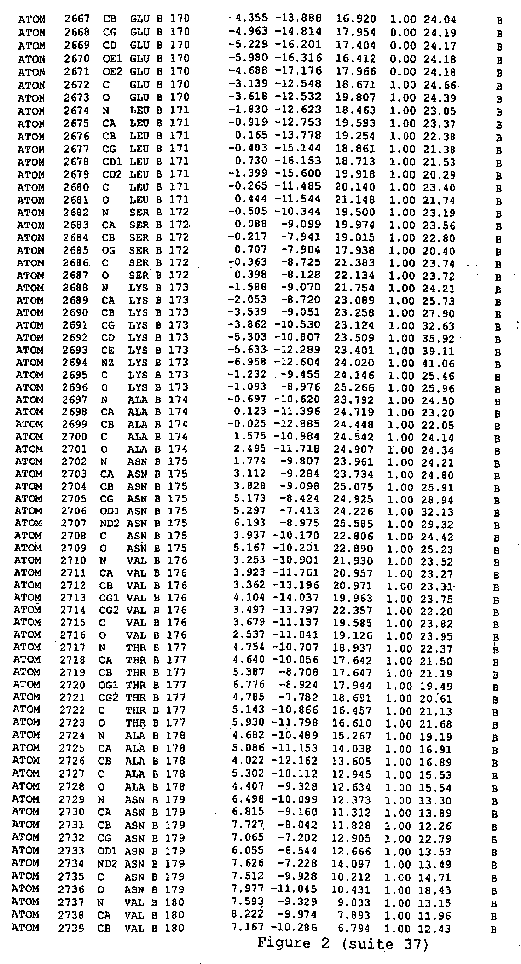

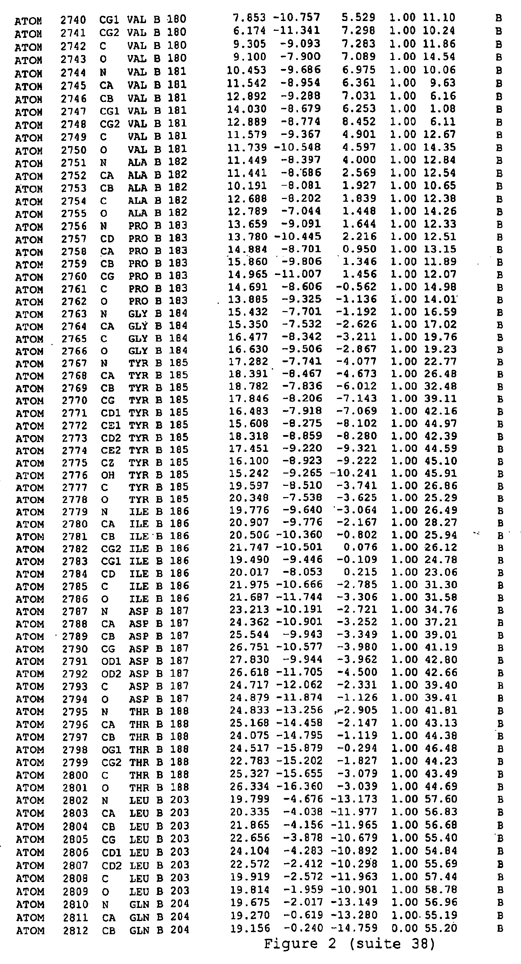

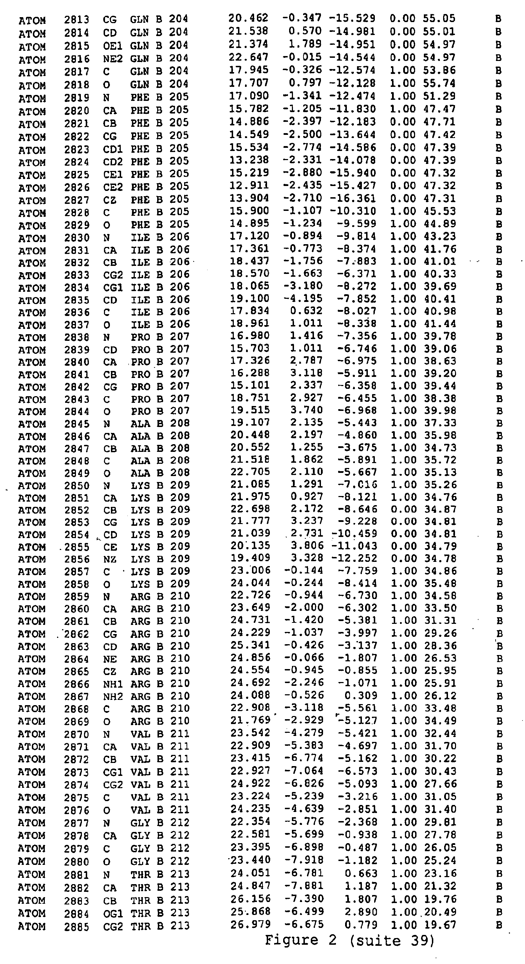

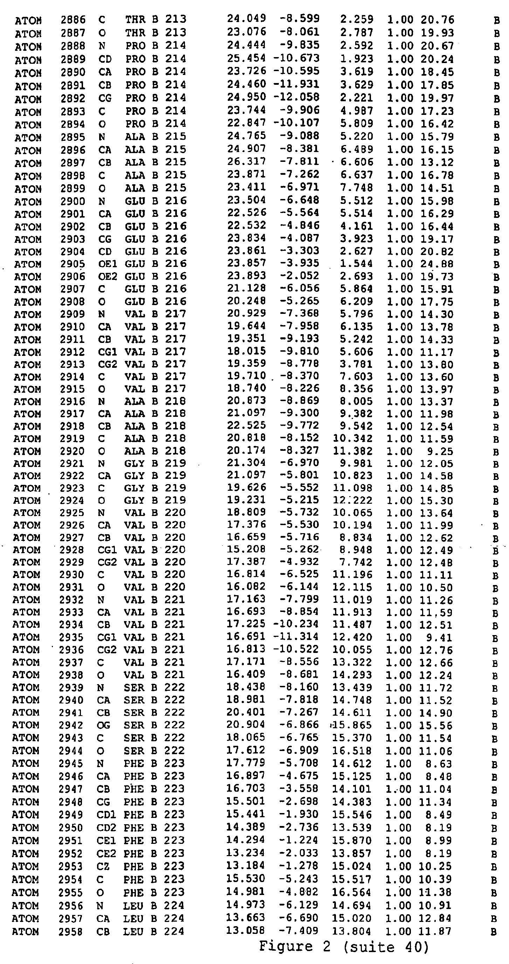

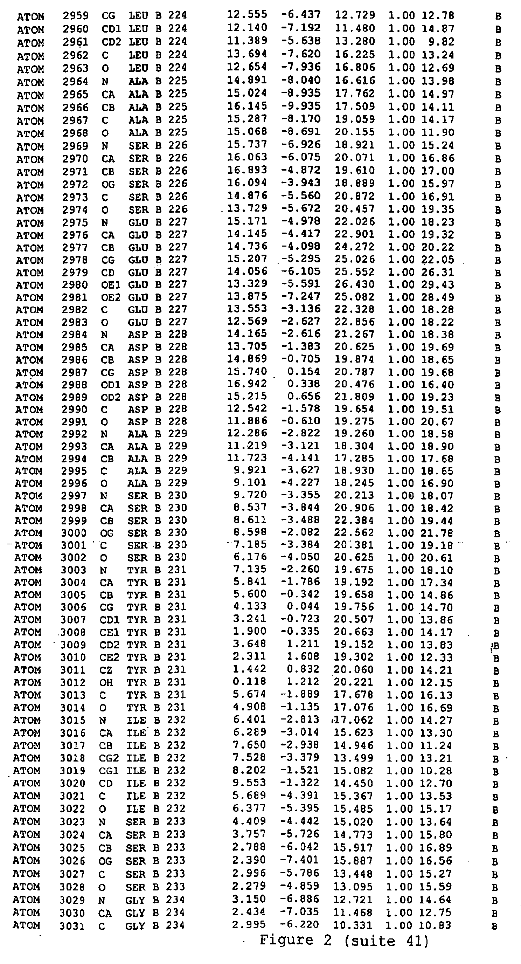

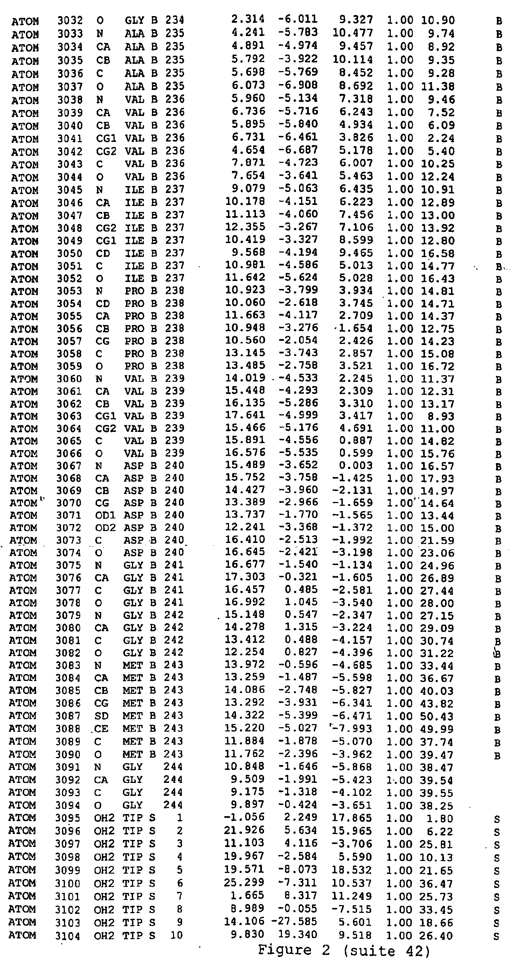

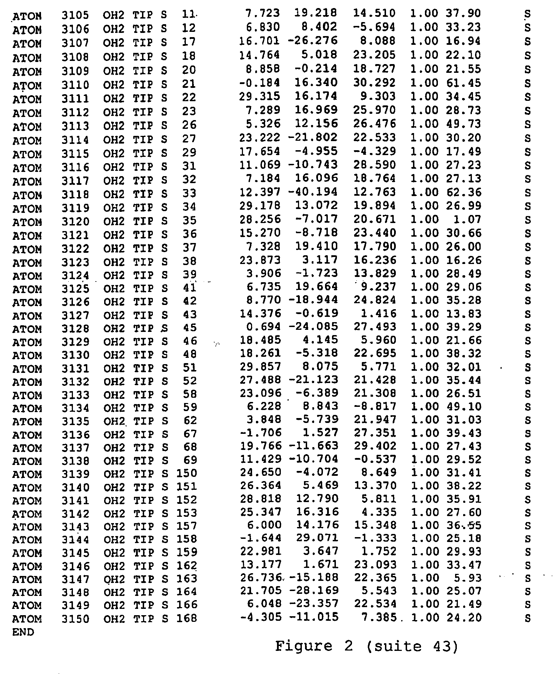

L'invention concerne également les cristaux susmentionnés de la protéine C(60)V correspondant à la séquence SEQ ID NO : 16, dont les coordonnées atomiques de la structure tridimensionnelle sont représentés sur la figure 2, et présentant les caractéristiques suivantes :

- paramètre de mailles :
  - a = 82.230 angströms, b =118.610 angströms, c = 53.170 angströms,
  - $\alpha = \beta = 90.00$ °, $\gamma = 122.74$ °,
- groupe d'espace : C2,
- diffraction maximale = 2,6 angströms.

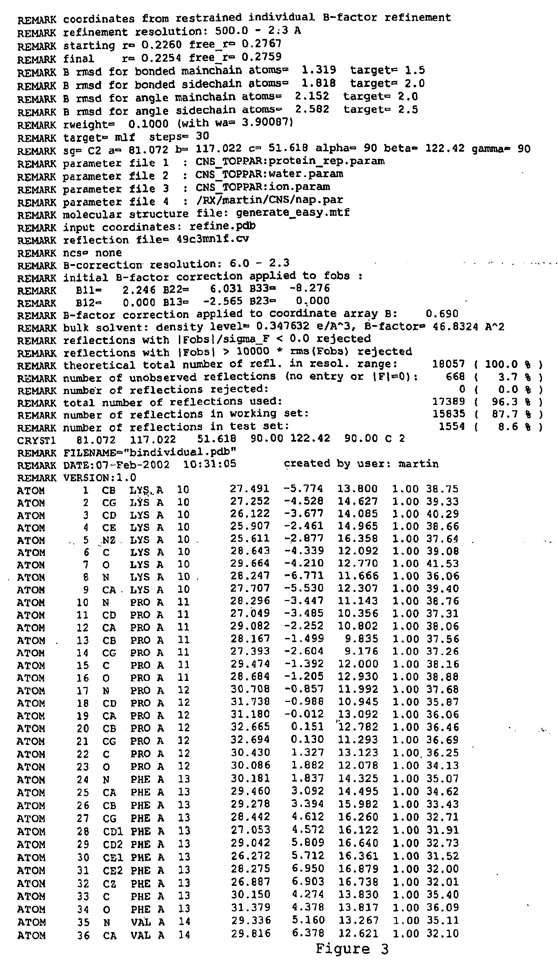

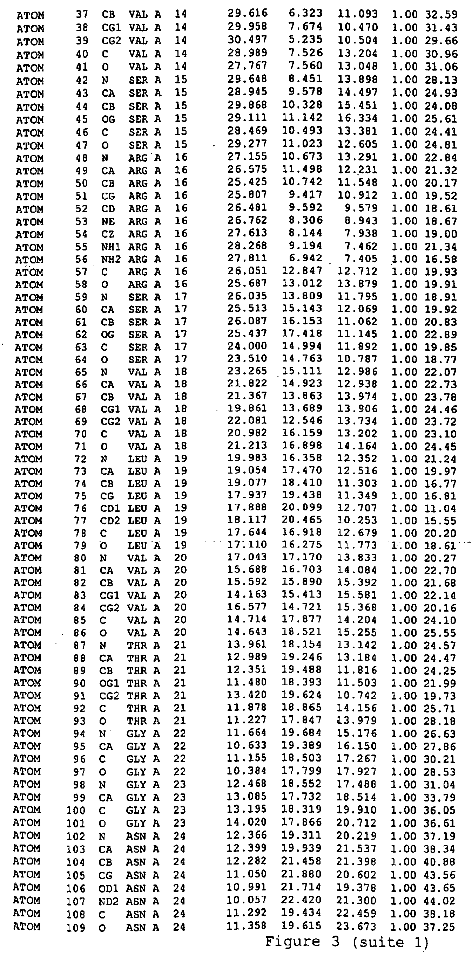

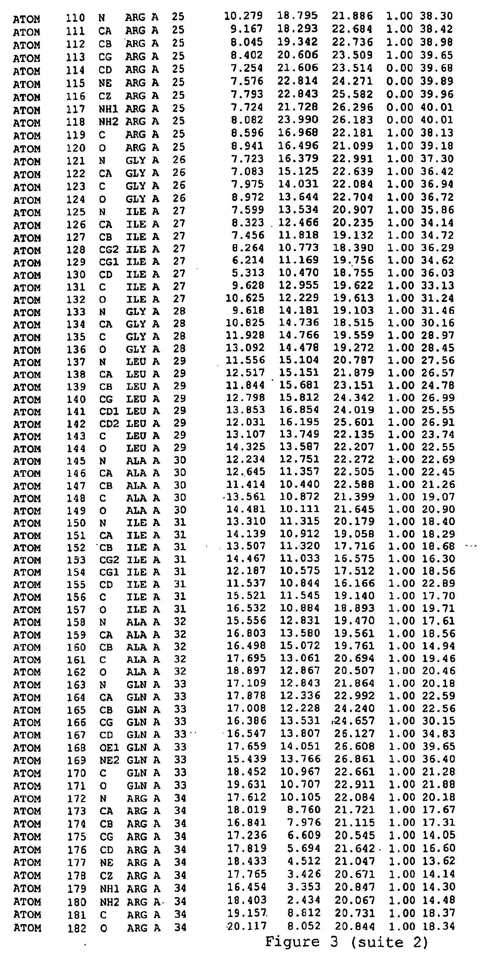

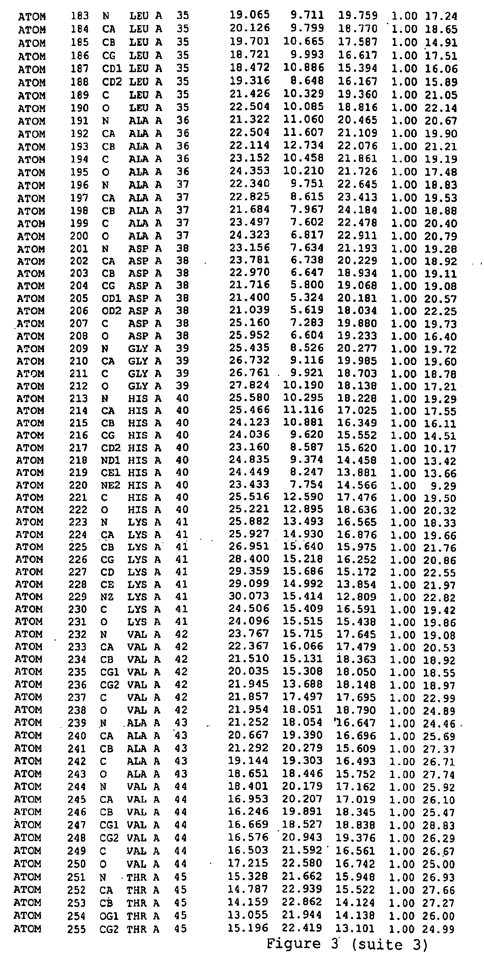

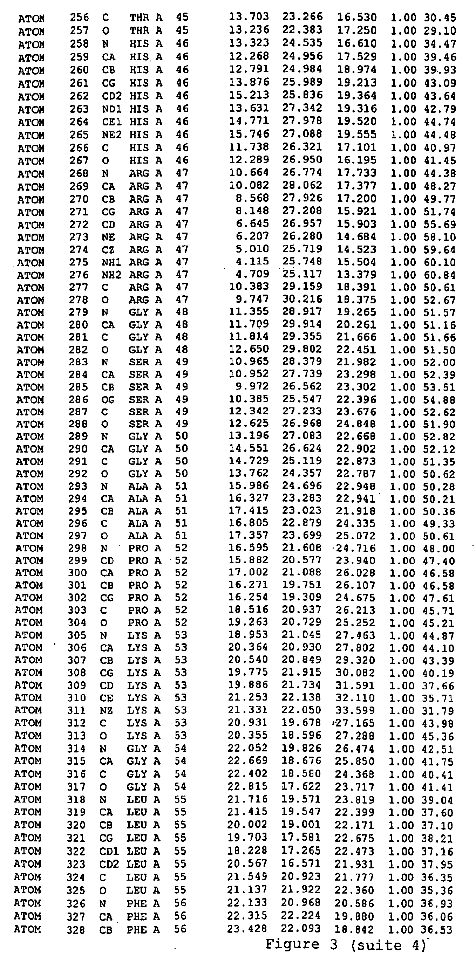

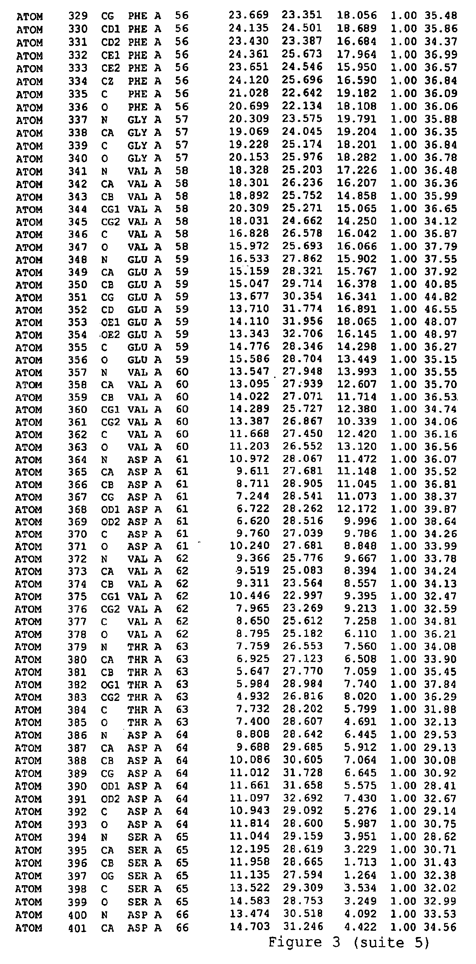

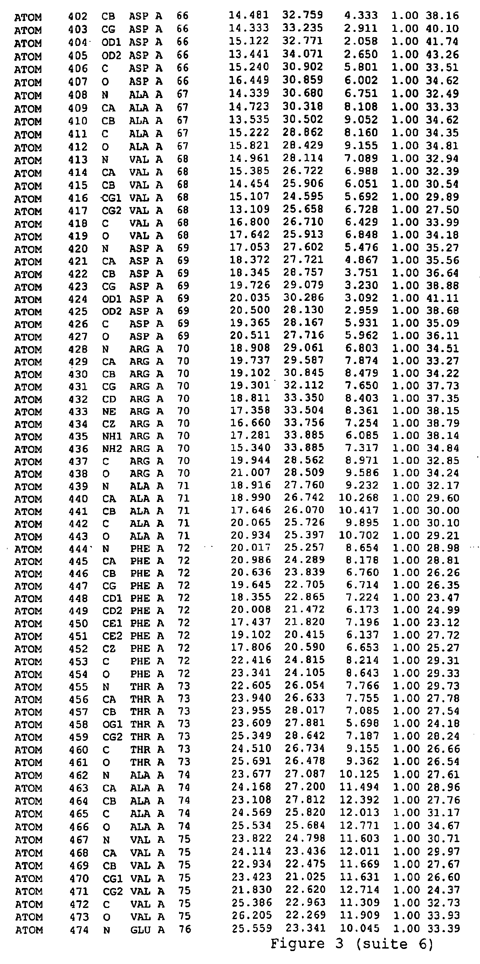

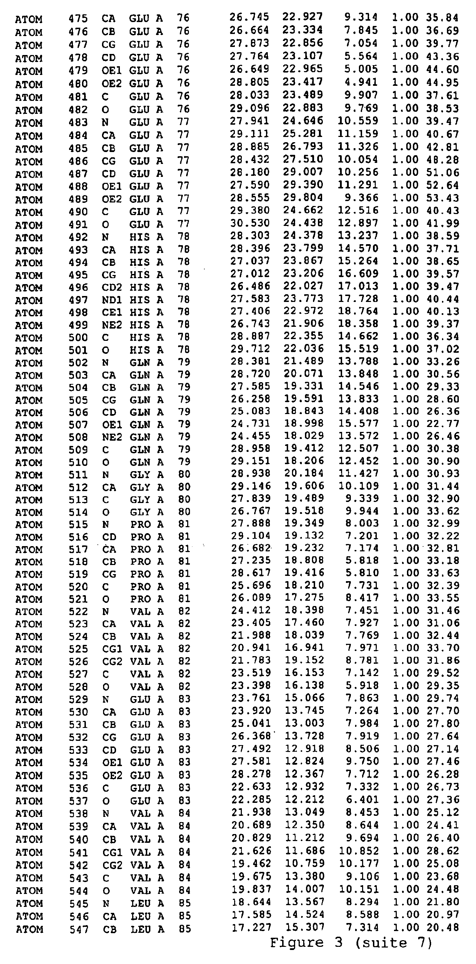

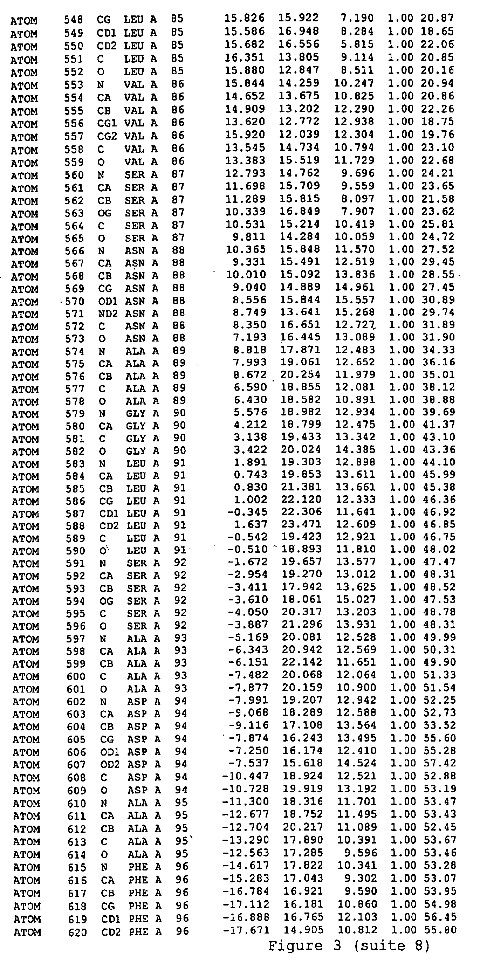

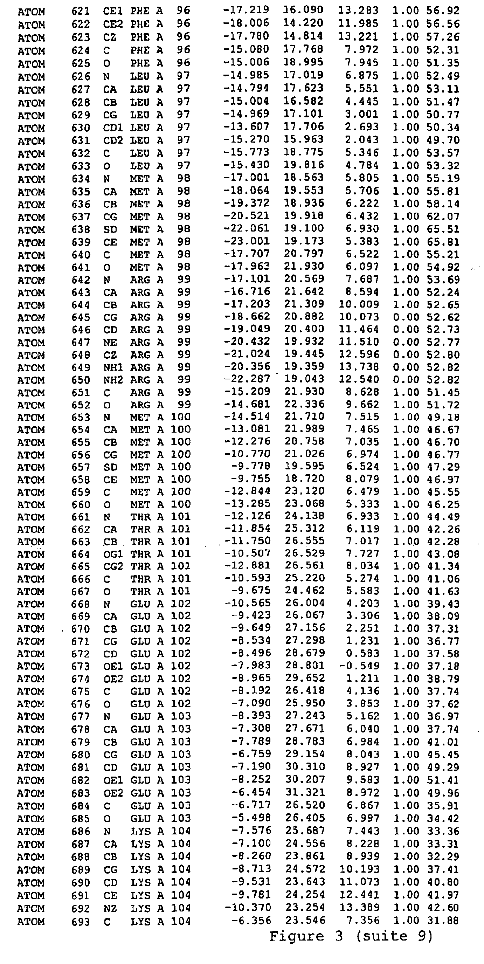

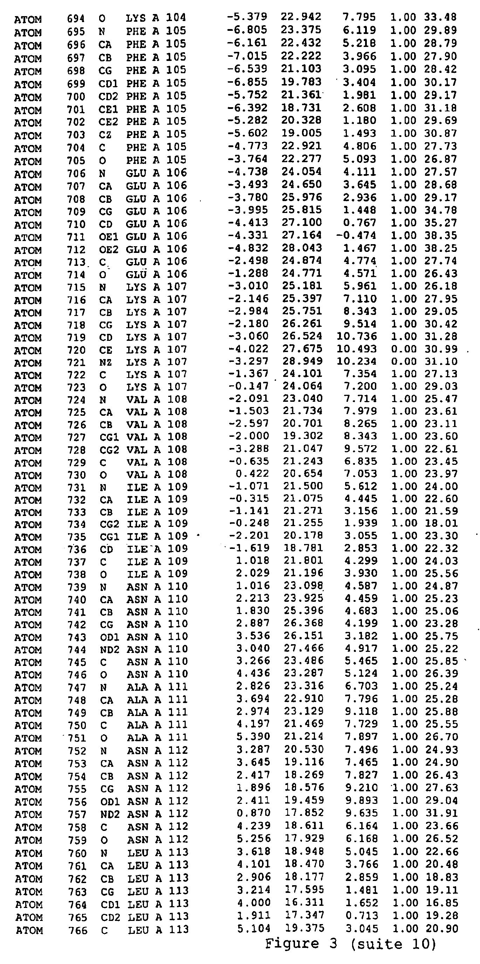

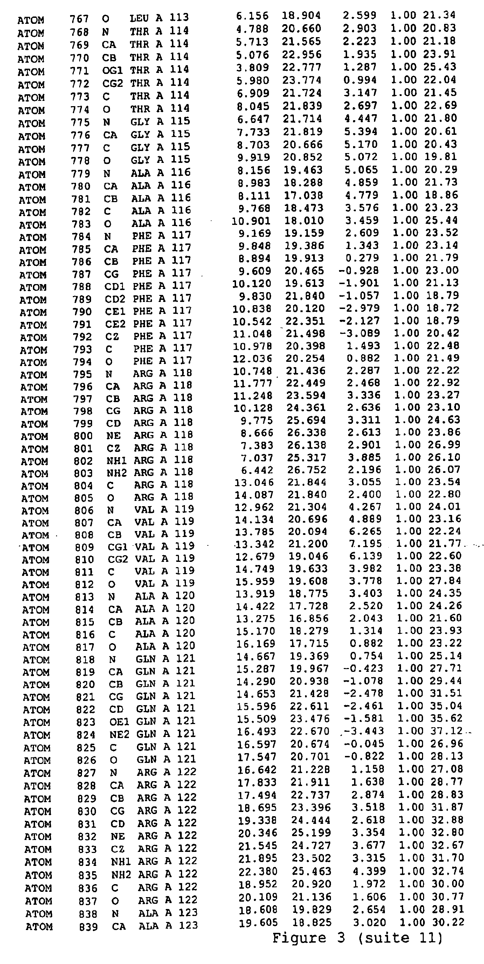

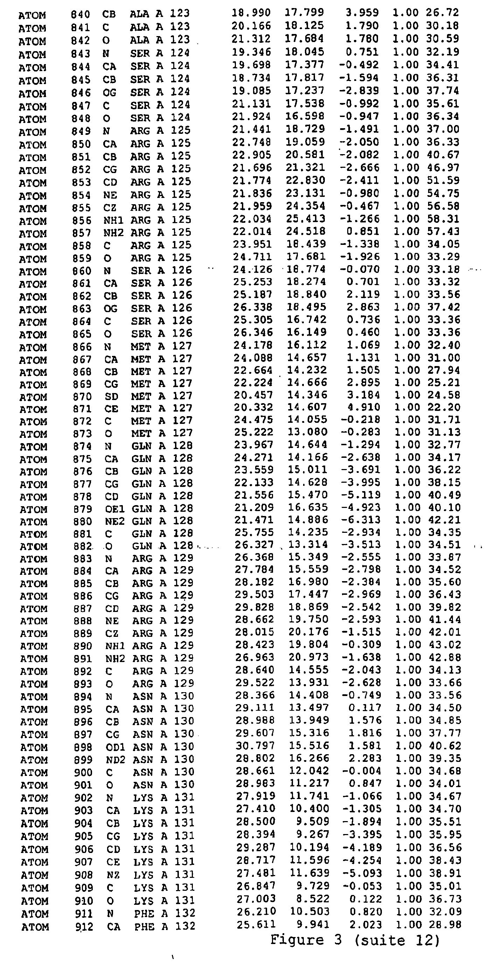

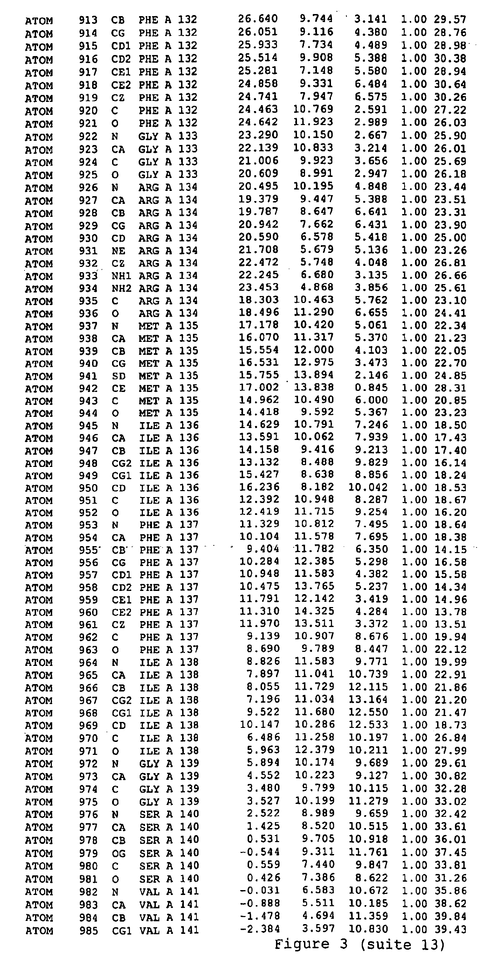

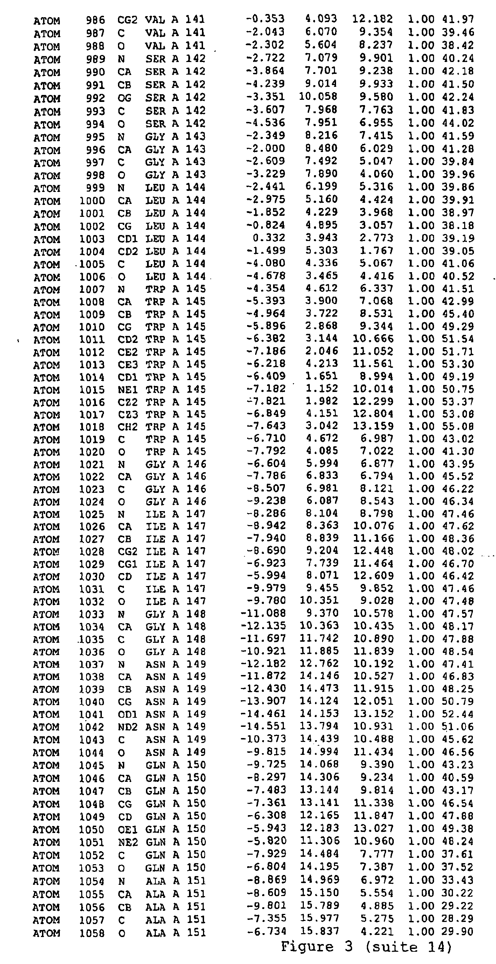

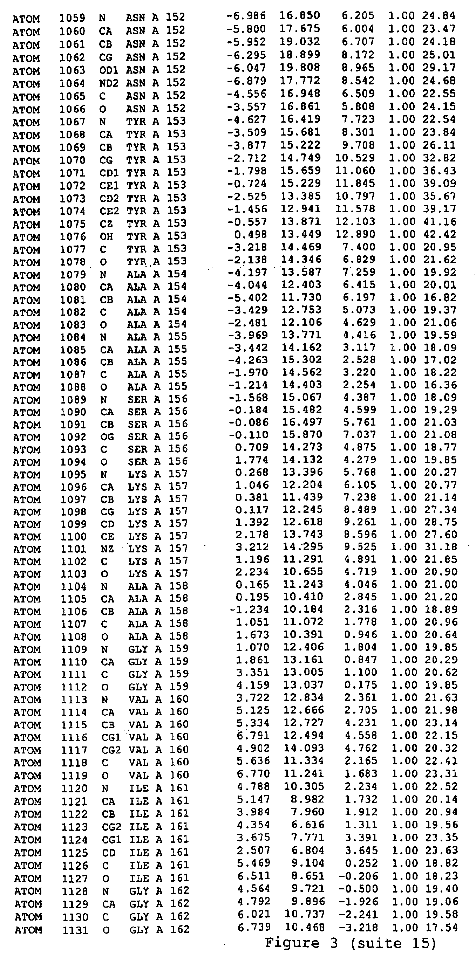

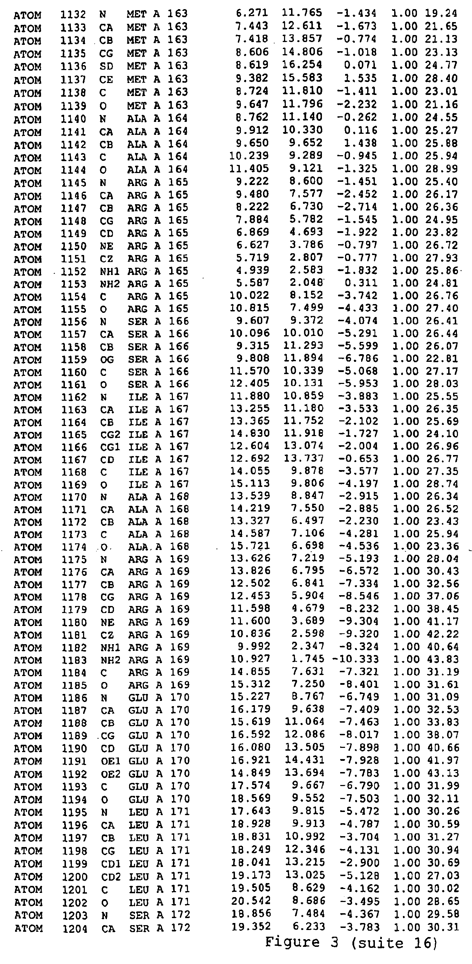

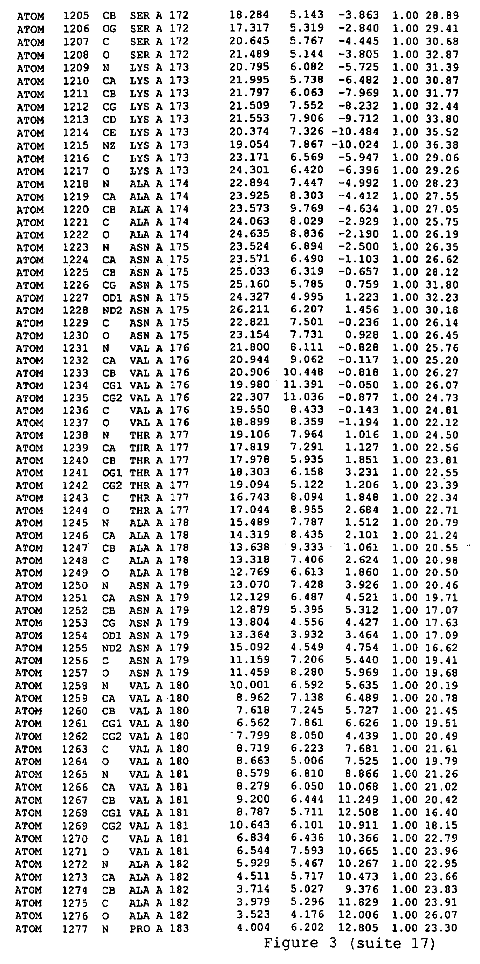

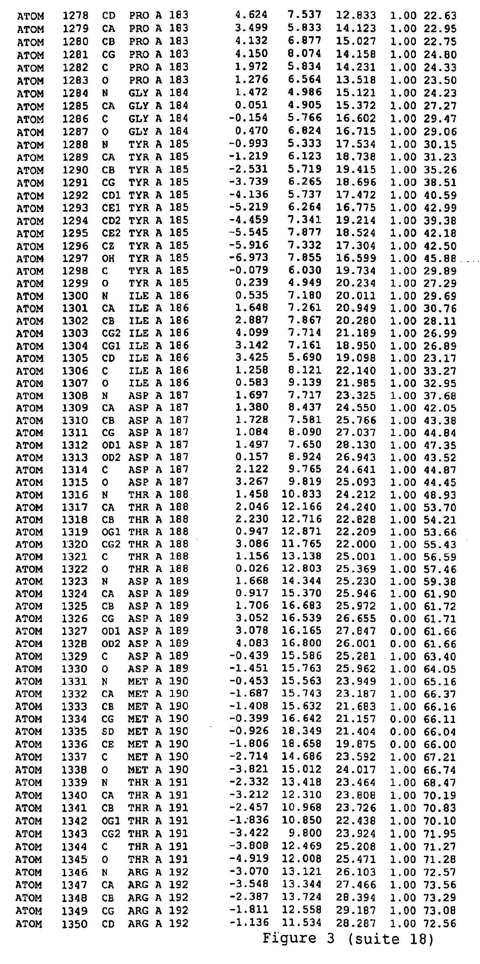

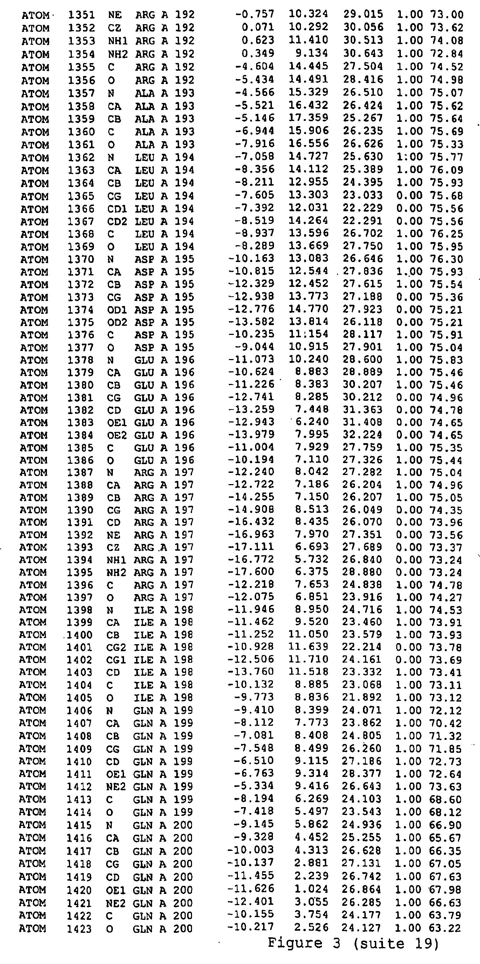

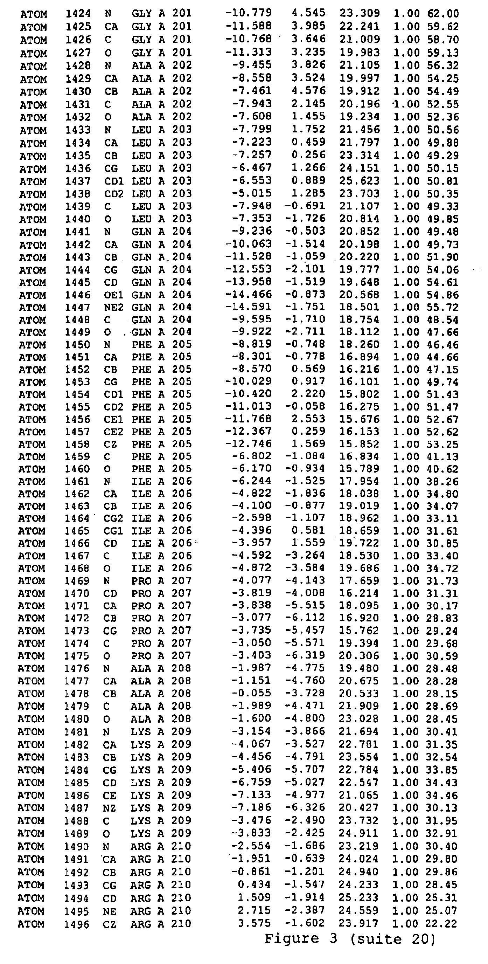

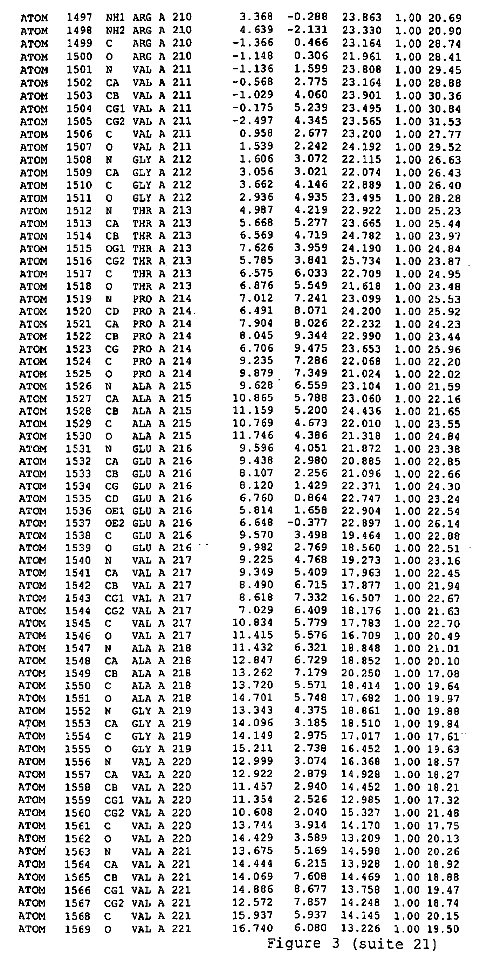

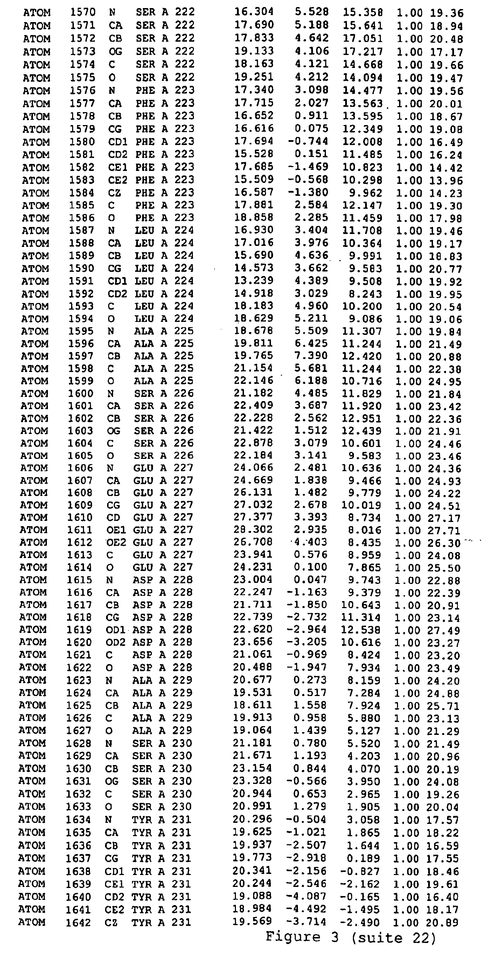

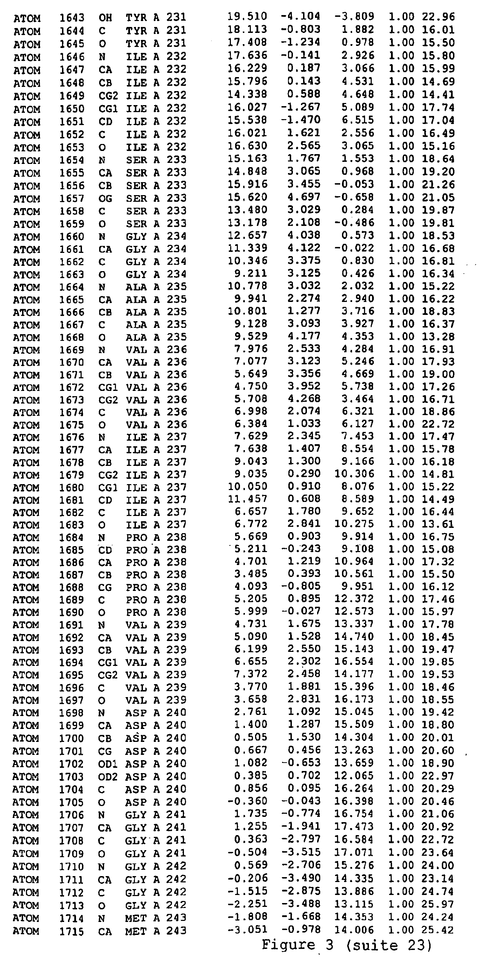

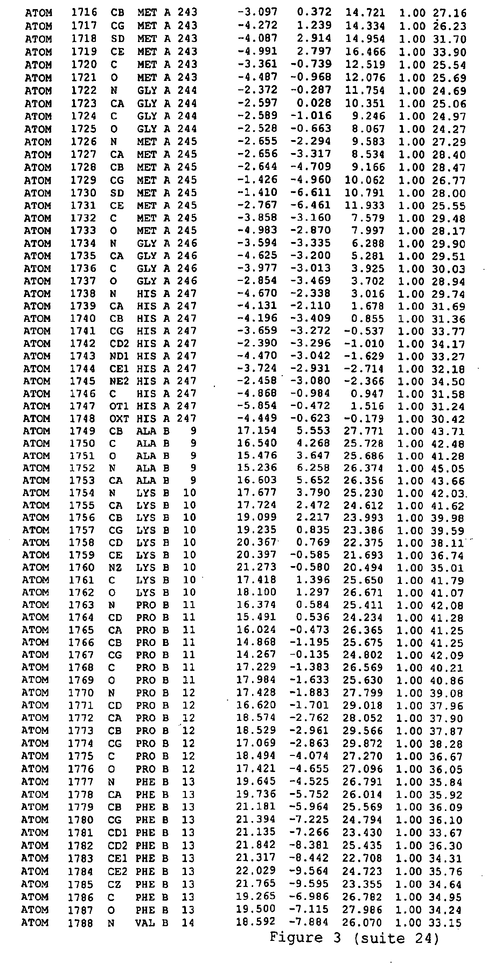

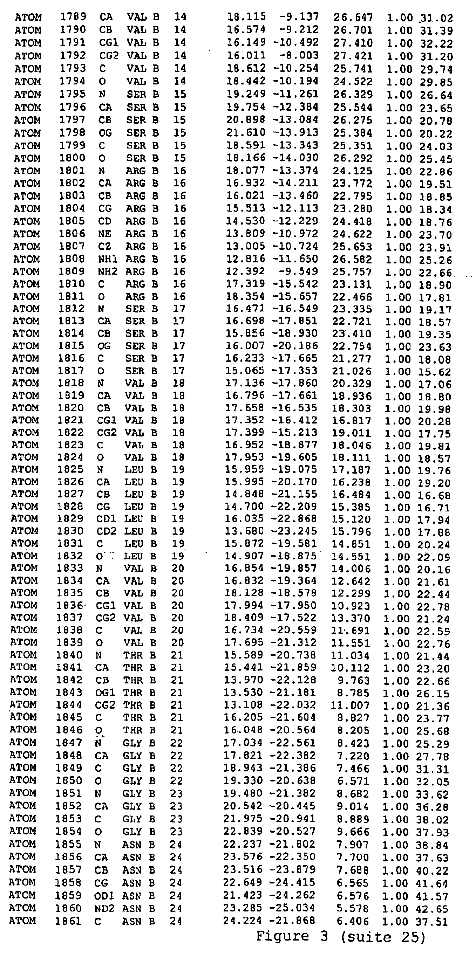

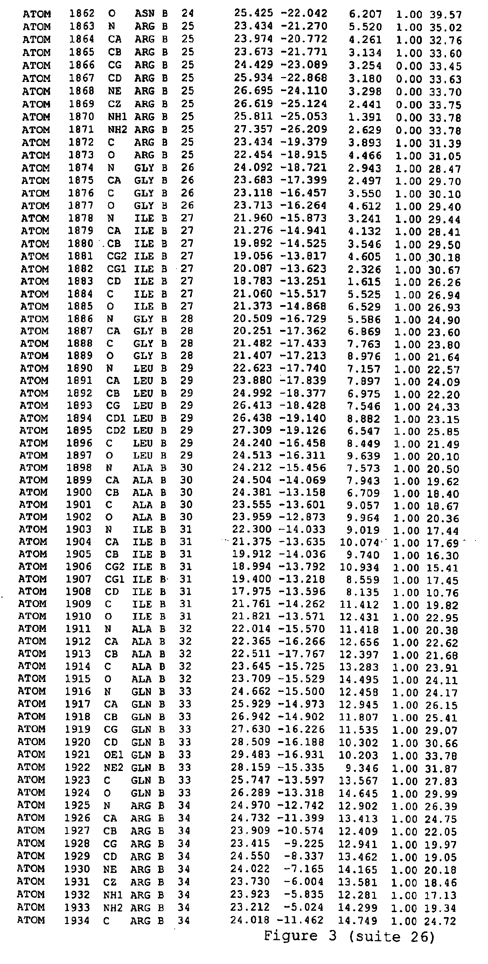

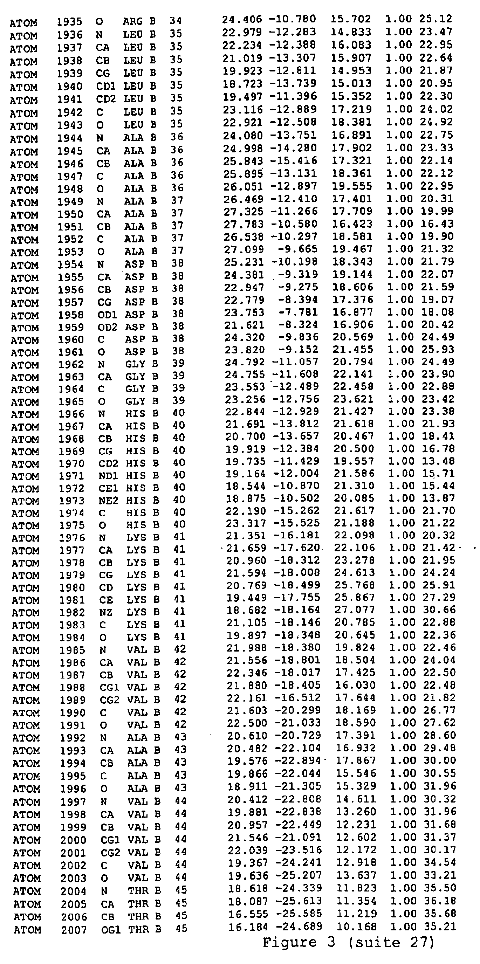

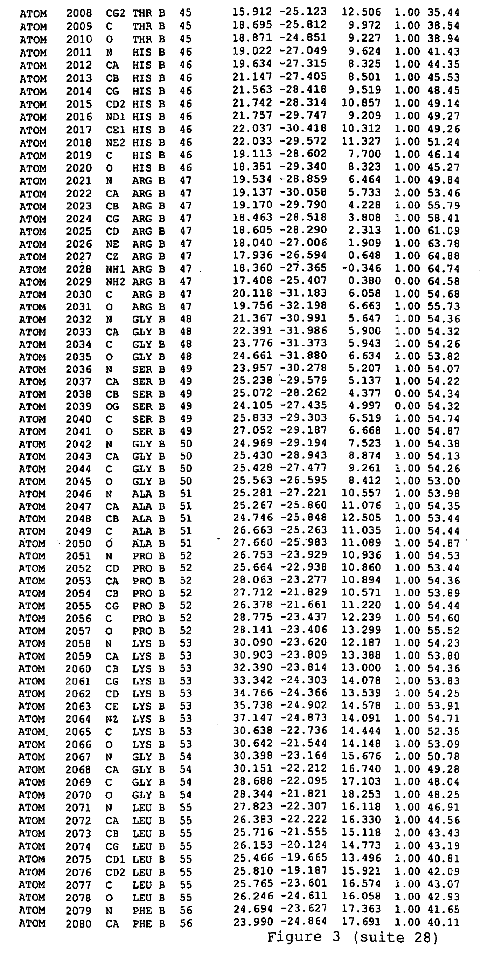

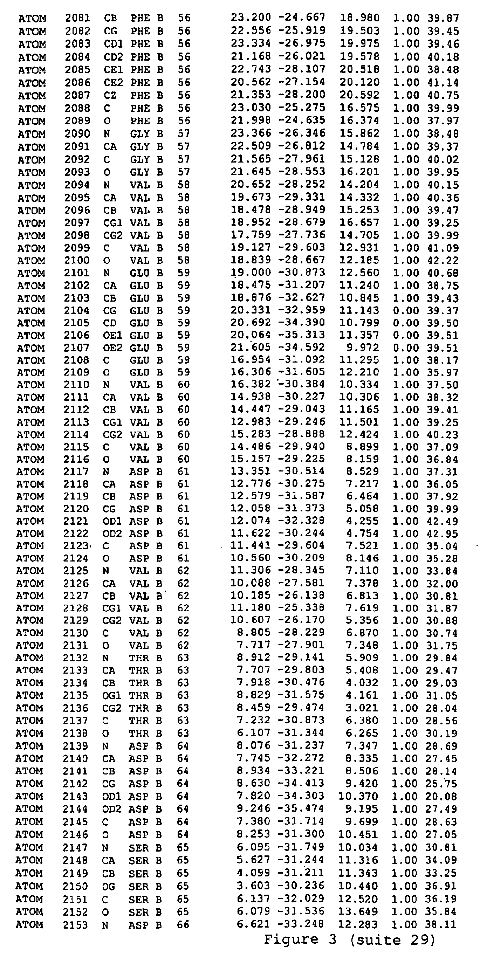

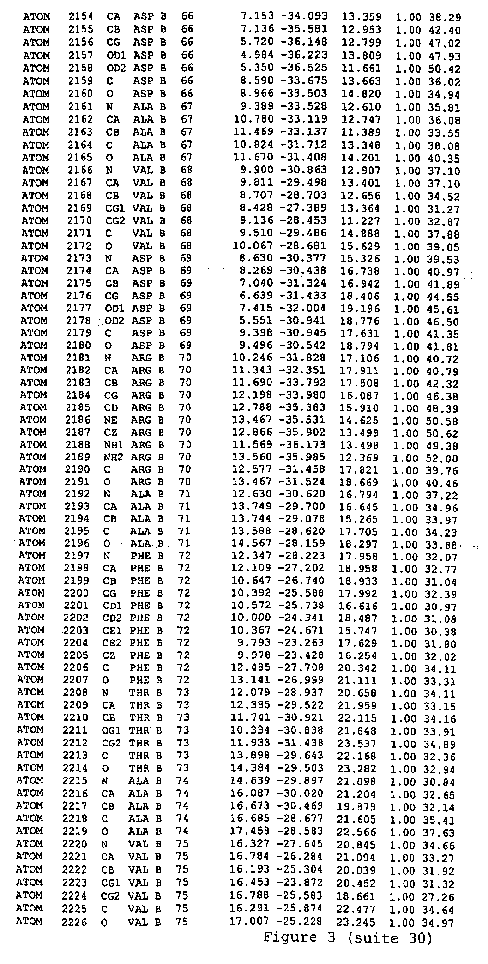

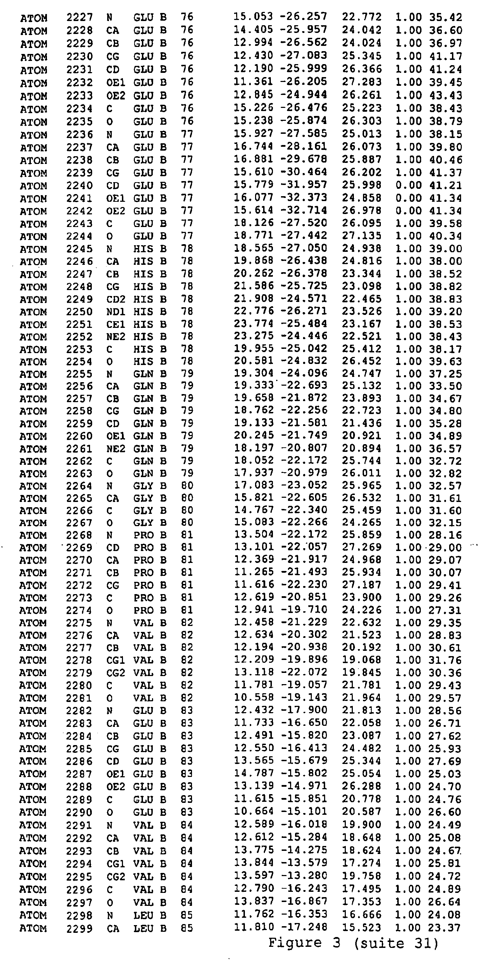

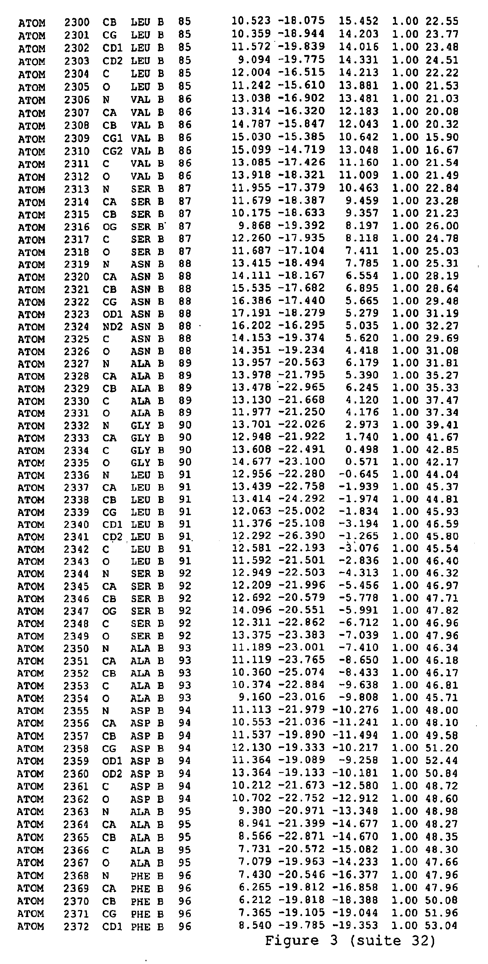

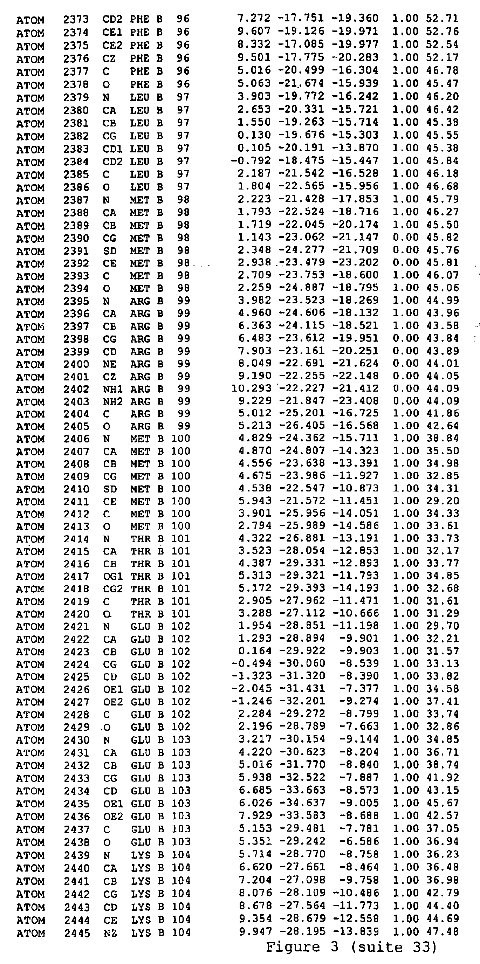

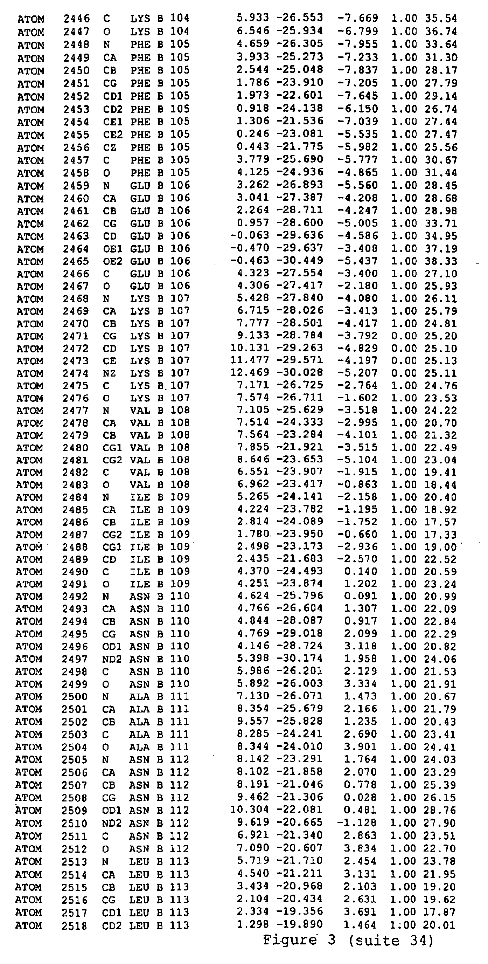

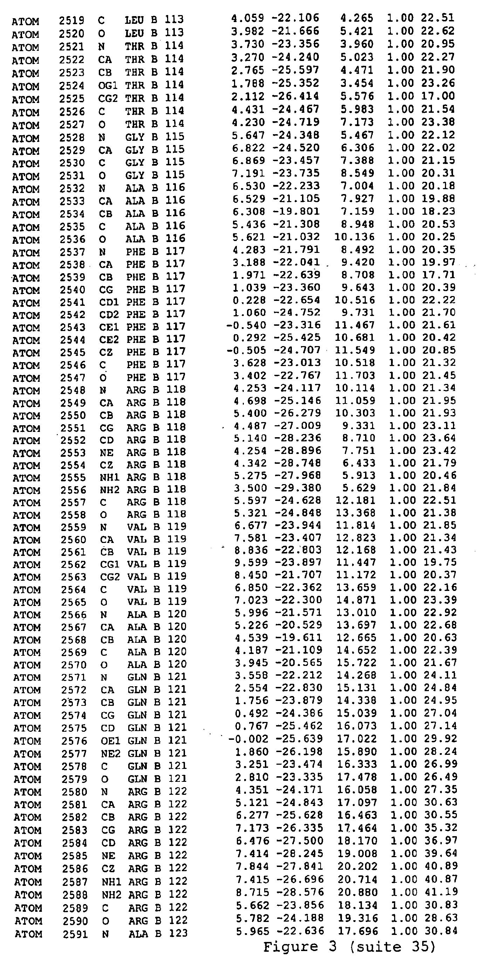

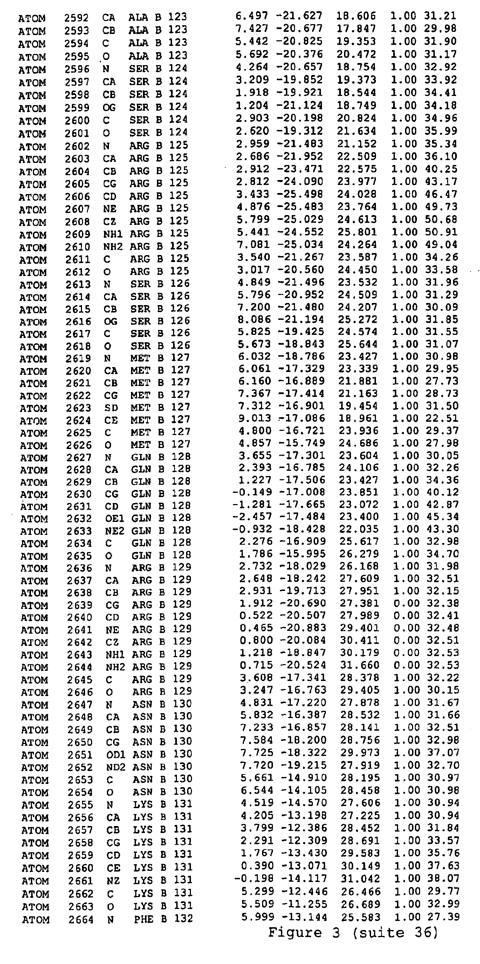

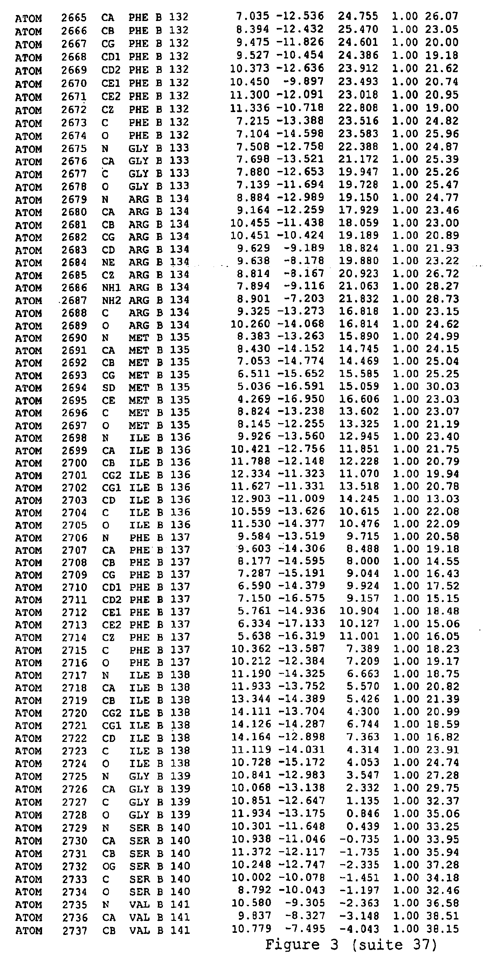

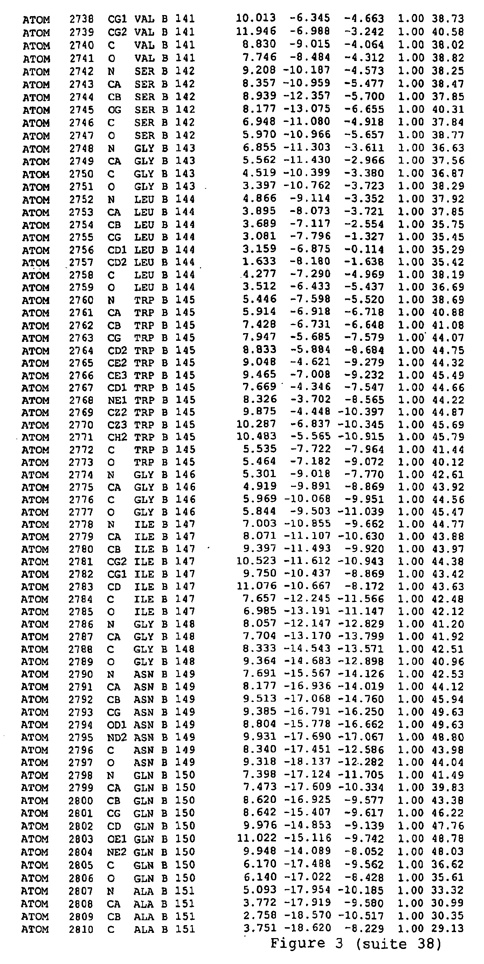

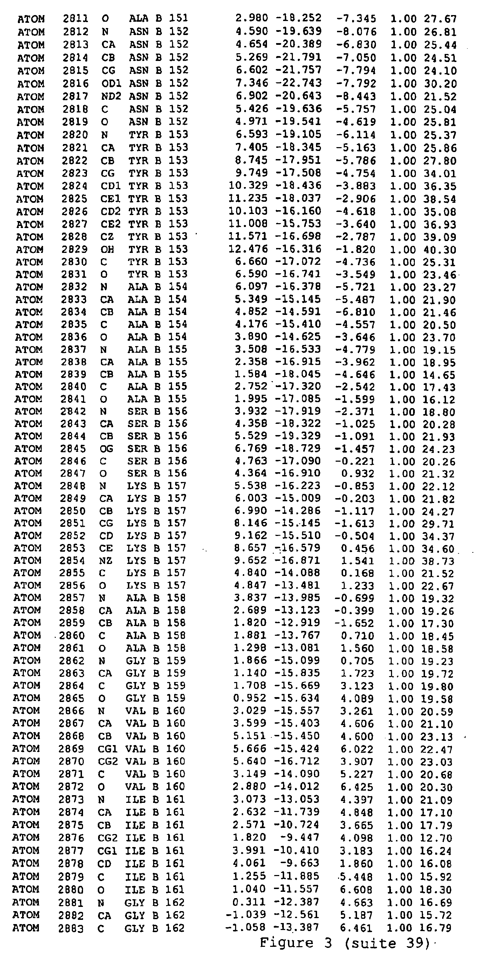

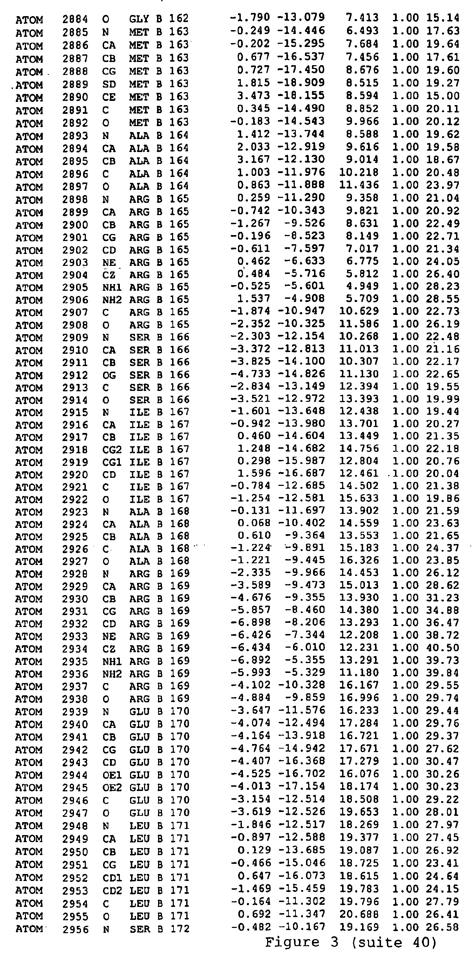

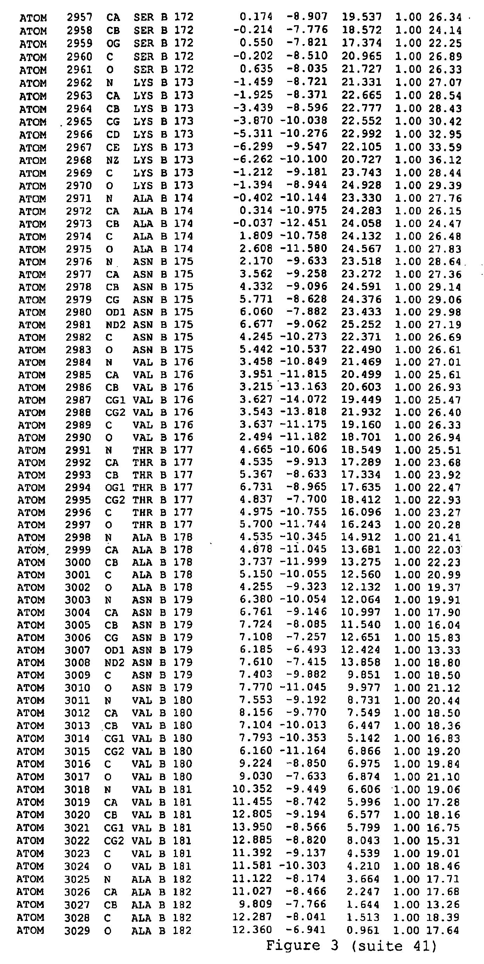

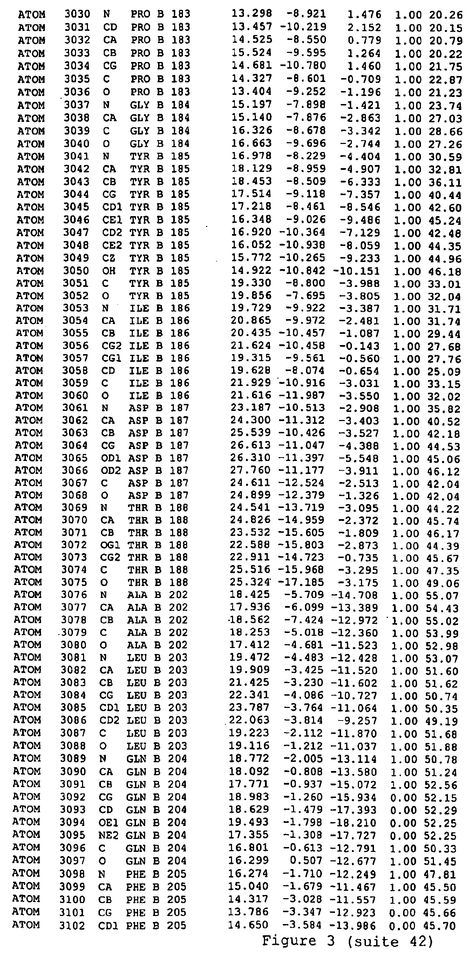

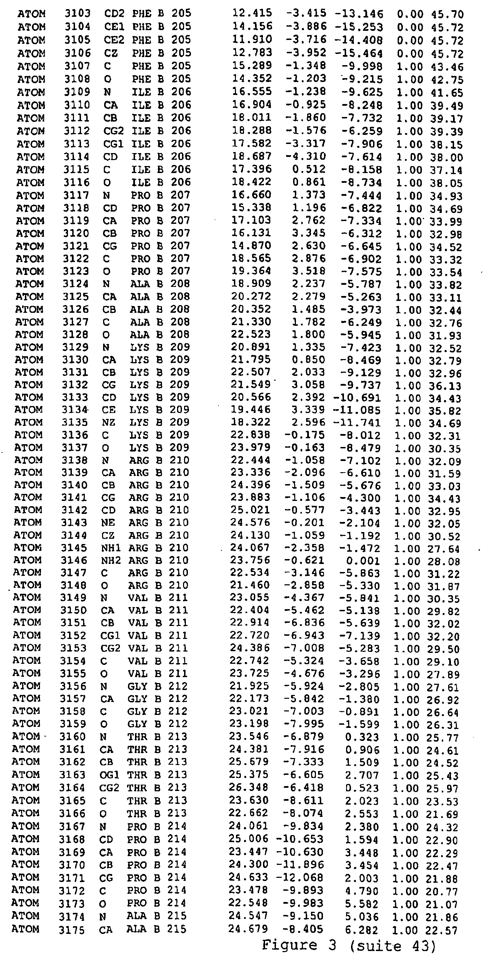

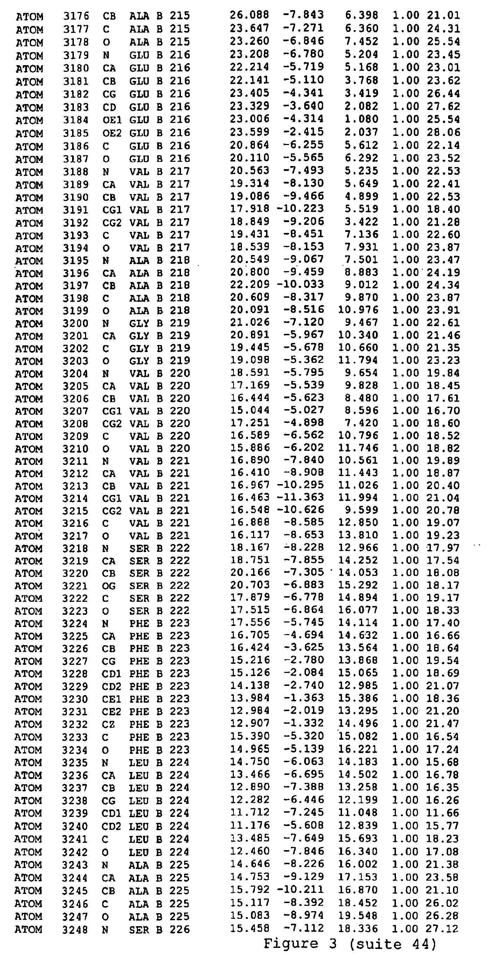

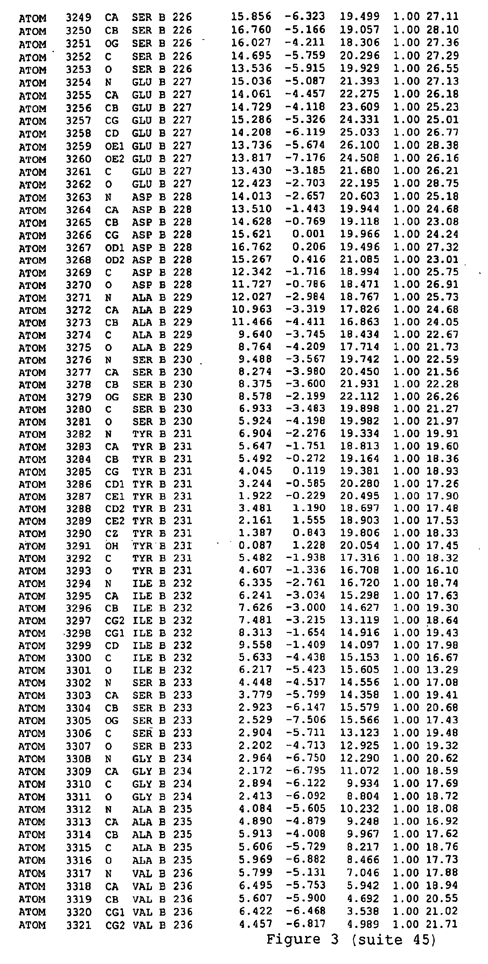

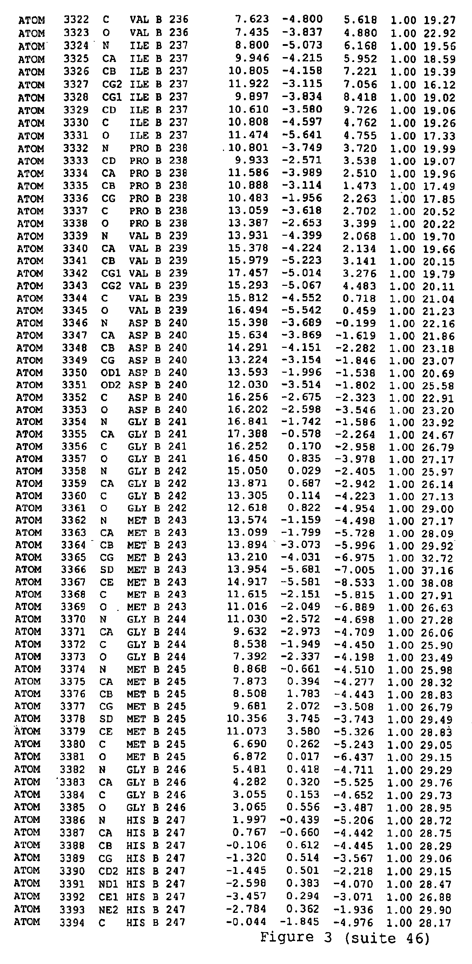

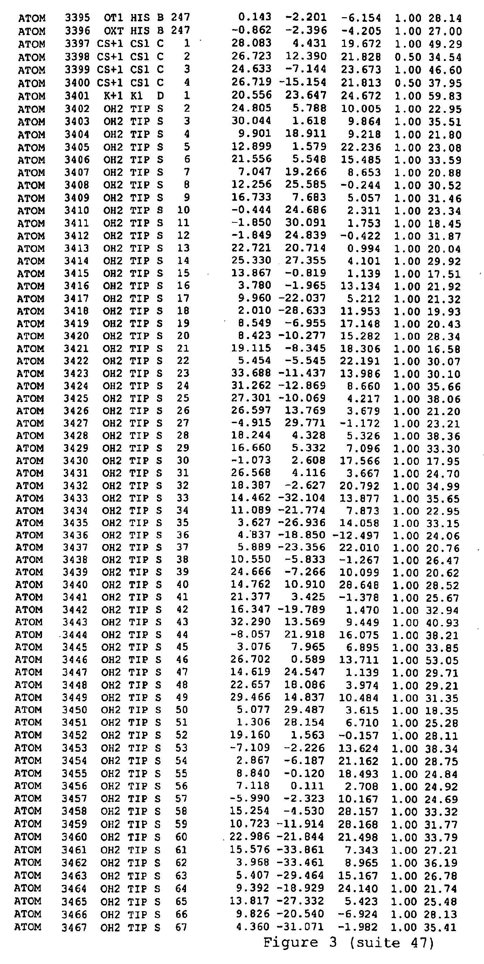

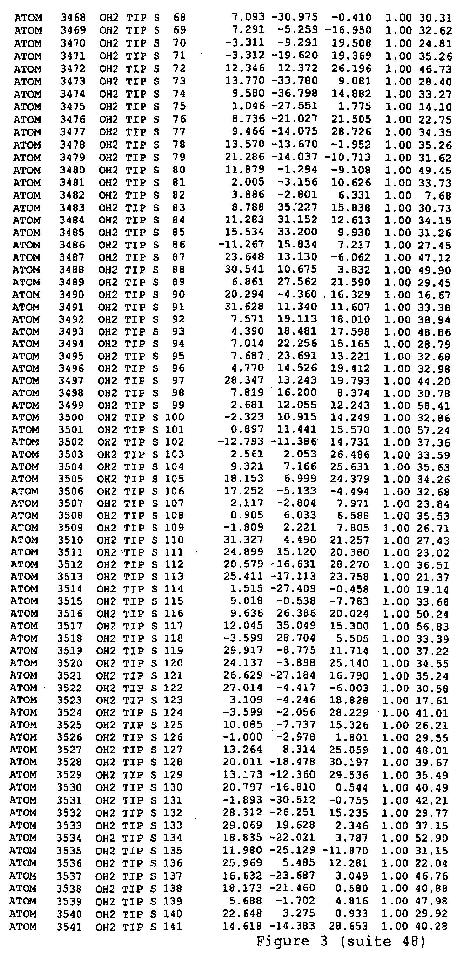

L'invention a également pour objet les cristaux susmentionnés de la protéine C(60)V / S(144)L correspondant à la séquence SEQ ID NO : 18, dont les coordonnées atomiques de la structure tridimensionnelle sont représentés sur la figure 3, et prés susmentionnés entant les caractéristiques suivantes :

- paramètre de mailles :
  - a = 81.072 angströms, b =117.022 angströms, c = 53.170 angströms,
  - $\alpha = \beta = 90.00$ °, $\gamma = 122.42$ °,
- groupe d'espace : C2,
- diffraction maximale =1,75 angströms.

L'invention a plus particulièrement pour objet les cristaux des protéines dérivées susmentionnées, dans lesquels lesdites protéines sont liées à un ligand, à savoir une molécule capable de se lier à la protéine MabA ou aux protéines dérivées de cette dernière, plus particulièrement au niveau de leur site actif principalement délimité par les acides aminés situés aux positions 21 à 28, 45 à 48, 60 à 63, 87 à 100, 112, 138 à 157, 183 à 212, et 240 à 247 des protéines correspondant aux séquences SEQ ID NO : 1, 3, 5, 7, 8, 9, 10, 11, ou 13, ou aux positions 41 à 48, 65 à 68, 80 à 83, 107 à 120, 132, 158 à 177, 203 à 232, et 260 à 267, des protéines correspondant aux séquences SEQ ID NO : 15 à 23, ou aux positions 24 à 31, 48 à 51, 63 à 66, 90 à 103, 115, 141 à 160, 186 à 215, et 243 à 250, des protéines correspondant aux séquences SEQ ID NO : 24 à 32, ou aux positions 14 à 11, 38 à 41, 53 à 56, 80 à 93, 105, 131 à 150, 176 à 205, et 233 à 240, des protéines correspondant aux séquences SEQ ID NO : 33 à 41, lesdits cristaux étant tels qu'obtenus par trempage ou co-cristallisation de la protéine MabA recombinante sous forme purifiée, ou d'une protéine recombinante dérivée de la protéine MabA susmentionnées, en présence dudit ligand, notamment dans les conditions de cristallisation définies ci-dessus.

L'invention concerne également les séquences nucléotidiques codant pour une protéine dérivée de la protéine MabA telle que définie ci-dessus.

A ce titre, l'invention a plus particulièrement pour objet la séquence nucléotidique codant pour la protéine dérivée C(60)V (SEQ ID NO : 3), et correspondant à la séquence SEQ ID NO : 2 suivante :

atgactgccacagccactgaaggggccaaacccccattcgtatcccgttcagtcctggttaccggaggaaaccgggggatcgggctggcgat
cgcacagcggctggctgccgacggccacaaggtggccgtcacccaccgtggatccggagcgccaaaggggctgtttggcgtcgaagttga
cgtcaccgacagcgacgccgtcgatcgcgccttcacggcggtagaagagcaccagggtccggtcgaggtgctggtgtccaacgccggccta
tccgcggacgcattcctcatgcggatgaccgaggaaaagttcgagaaggtcatcaacgccaacctcaccggggcgttccgggtggctcaacg
ggcatcgcgcagcatgcagcgcaacaaattcggtcgaatgatattcataggttcggtctccggcagctggggcatcggcaaccaggccaacta
cgcagcctccaaggccggagtgattggcatggcccgctcgatcgcccgcgagctgtcgaaggcaaacgtgaccgcgaatgtggtggcccc
gggctacatcgacaccgatatgacccgcgcgctggatgagcggattcagcagggggcgctgcaatttatcccagcgaagcgggtcggcacc
cccgccgaggtcgccggggtggtcagcttcctggcttccgaggatgcgagctatatctccggtgcggtcatcccggtcgacggcggcatggg
tatgggccac

ou toute séquence dérivée par dégénérescence du code génétique et codant pour la protéine C(60)V.

A ce titre également, l'invention a pour objet la séquence nucléotidique codant pour la protéine dérivée S(144)L (SEQ ID NO: 5), et correspondant à la séquence SEQ ID NO : 4 suivante:

atgactgccacagccactgaaggggccaaacccccattcgtatcccgttcagtcctggttaccggaggaaaccgggggatcgggctggcgat
cgcacagcggctggctgccgacggccacaaggtggccgtcacccaccgtggatccggagcgccaaaggggctgtttggcgtcgaatgtga
cgtcaccgacagcgacgccgtcgatcgcgccttcacggcggtagaagagcaccagggtccggtcgaggtgctggtgtccaacgccggccta
tccgcggacgcattcctcatgcggatgaccgaggaaaagttcgagaaggtcatcaacgccaacctcaccggggcgttccgggtggctcaacg
ggcatcgcgcagcatgcagcgcaacaaattcggtcgaatgatattcataggttcggtctccggcctctggggcatcggcaaccaggccaacta
cgcagcctccaaggccggagtgattggcatggcccgctcgatcgcccgcgagctgtcgaaggcaaacgtgaccgcgaatgtggtggcccc
gggctacatcgacaccgatatgacccgcgcgctggatgagcggattcagcagggggcgctgcaatttatcccagcgaagcgggtcggcacc
cccgccgaggtcgccggggtggtcagcttcctggcttccgaggatgcgagctatatctccggtgcggtcatcccggtcgacggcggcatggg
tatgggccac

ou toute séquence dérivée par dégénérescence du code génétique et codant pour la protéine S(144)L.

A ce titre encore, l'invention a pour objet la séquence nucléotidique codant pour la protéine dérivée C(60)V / S(144)L (SEQ ID NO : 7), et correspondant à la séquence SEQ ID NO : 6 suivante :

atgactgccacagccactgaaggggccaaacccccattcgtatcccgttcagtcctggttaccggaggaaaccgggggatcgggctggcgat
cgcacagcggctggctgccgacggccacaaggtggccgtcacccaccgtggatccggagcgccaaaggggctgtttggcgtcgaagttga
cgtcaccgacagcgacgccgtcgatcgcgccttcacggcggtagaagagcaccagggtccggtcgaggtgctggtgtccaacgccggccta
tccgcggacgcattcctcatgcggatgaccgaggaaaagttcgagaaggtcatcaacgccaacctcaccggggcgttccgggtggctcaacg
ggcatcgcgcagcatgcagcgcaacaaattcggtcgaatgatattcataggttcggtctccggcctctggggcatcggcaaccaggccaacta
cgcagcctccaaggccggagtgattggcatggcccgctcgatcgcccgcgagctgtcgaaggcaaacgtgaccgcgaatgtggtggcccc
gggctacatcgacaccgatatgacccgcgcgctggatgagcggattcagcagggggcgctgcaatttatcccagcgaagcgggtcggcacc
cccgccgaggtcgccggggtggtcagcttcctggcttccgaggatgcgagctatatctccggtgcggtcatcccggtcgacggcggcatggg
tatgggccac

ou toute séquence dérivée par dégénérescence du code génétique et codant pour la protéine C(60)V / S(144)L.

L'invention concerne également toute séquence nucléotidique recombinante comprenant la séquence nucléotidique codant pour la protéine MabA, ou comprenant une séquence nucléotidique codant pour une protéine dérivée de la protéine MabA, telles que définies ci-dessus, en association avec les éléments nécessaires à la transcription de cette séquence, notamment avec un promoteur et un terminateur de transcription.

L'invention a également pour objet tout vecteur, notamment plasmide, contenant une séquence nucléotidique telle que définie ci-dessus.

L'invention concerne également les cellules hôtes transformées par un vecteur susmentionné, lesdites cellules étant choisies notamment parmi les bactéries telles que *E. coli,* ou tout autre micro-organisme utilisé pour la production de protéines.

L'invention a également pour objet un procédé de préparation de la protéine MabA recombinante sous forme purifiée, ou de protéines recombinantes dérivées de la protéine MabA, telles que définies ci-dessus, caractérisé en ce qu'il comprend les étapes suivantes :

- transformation de cellules à l'aide d'un vecteur recombinant susmentionné,
- mise en culture des cellules ainsi transformées, et récupération desdites protéines produites par lesdites cellules,
- purification desdites protéines selon le procédé décrit de purification décrit ci-dessus.

L'invention concerne également l'utilisation de la protéine MabA recombinante sous forme purifiée, ou de protéines recombinantes dérivées de la protéine MabA telles que définies ci-dessus, ou de cristaux susmentionnés, pour la mise en oeuvre de méthodes de conception ou de criblage de ligands de la protéine MabA, et plus particulièrement de molécules susceptibles de se lier spécifiquement au niveau du site actif de la protéine MabA, ou de protéines de structure proche de la protéine MabA, et d'inhiber l'activité enzymatique de cette dernière, ces inhibiteurs étant choisis notamment parmi :

- les dérivés des stéroïdes,
- les dérivés de l'antibiotique antituberculeux isoniazide (hydrazide de l'acide isonicotinique), tels que les dérivés de l'adduit isonicotinoyl-NAD(P),
- les dérivés de la N-acétyl cystéamine ou d'autres types de dérivés simplifiés du coenzyme A, comprenant un fluorophore greffé permettant d'utiliser la méthode de la spectroscopie de fluorescence, en particulier résolue dans le temps, pour la détection d'interactions protéine-ligand,
- les dérivés inhibiteurs de la protéine InhA de *Mycobacterium tuberculosis.*

L'invention a plus particulièrement pour objet l'utilisation susmentionnée, de la protéine MabA recombinante sous forme purifiée, ou de protéines recombinantes dérivées de la protéine MabA telles que définies ci-dessus, ou de cristaux susmentionnés, pour la mise en oeuvre de méthodes de conception ou de criblage de ligands de la protéine MabA susceptibles d'être utilisés dans des compositions pharmaceutiques, notamment dans le cadre du traitement de pathologies liées à des infections mycobactériennes, telles que la tuberculose liée à l'infection par *Mycobacterium tuberculosis,* ou par *Mycobacterium africanium,* ou la lèpre liée à l'infection par *Mycobacterium leprae,* ou les mycobactérioses liées à l'infection par des mycobactéries opportunistes, telles que *Mycobacterium avium, Mycobacterium fortuitum, Mycobacterium kansasii, Mycobacterium chelonae.*

L'invention concerne également toute méthode de criblage de ligands de la protéine MabA, caractérisée en ce qu'elle comprend les étapes suivantes :

- mise en présence de la protéine MabA recombinante sous forme purifiée, ou d'une protéine recombinante dérivée de la protéine MabA telles que définies ci-dessus,
- détection de l'éventuelle liaison entre ladite protéine, et le ligand testé par mesure, après excitation de fluorescence, notamment à 300 nm, de l'intensité de fluorescence de ladite protéine émise entre 300 et 400 nm (correspondant essentiellement à l'émission de fluorescence de l'unique tryptophane W145), et comparaison de l'intensité de fluorescence émise dans un essai en absence de ligand, la fixation d'un ligand dans le site actif MabA étant caractérisée par un quenching de fluorescence.

L'invention a également pour objet toute méthode de criblage de ligands inhibiteurs de la protéine MabA, caractérisée en ce qu'elle comprend les étapes suivantes :

- mise en présence de la protéine MabA recombinante sous forme purifiée, ou d'une protéine recombinante dérivée de la protéine MabA telles que définies ci-dessus, dans un milieu réactionnel comprenant un substrat, tel qu'un dérivé β-cétoacyl défini ci-dessus, le coenzyme NADPH, et le ligand testé,

- détection d'un pouvoir inhibiteur potentiel du ligand testé, par mesure de l'activité enzymatique de ladite protéine par mesure cinétique de l'absorbance, notamment à 340 nm, et. comparaison de la pente de la courbe de densité optique en fonction du temps avec la pente obtenue dans un essai en absence de ligand.

L'invention concerne également toute méthode de criblage de ligands de la protéine MabA, caractérisée en ce qu'elle comprend les étapes suivantes :

- mise en présence d'une protéine recombinante dérivée de la protéine MabA telles que définies ci-dessus, avec le ligand testé,
- analyse de la structure tridimensionnelle du complexe formé en phase soluble entre ladite protéine et ledit ligand, notamment par RMN, et par fluorescence.

L'invention a plus particulièrement pour objet toute méthode de criblage de ligands de la protéine MabA, caractérisée en ce qu'elle comprend les étapes suivantes :

- co-cristallisation du ligand testé et d'une protéine recombinante dérivée de la protéine MabA telles que définies ci-dessus, notamment dans les conditions de cristallisation définies ci-dessus, de manière à obtenir des cristaux susmentionnés,
- ou, trempage des cristaux de la protéine MabA ou d'une protéine dérivée telles que définies ci-dessus, dans des solutions optimisées contenant des ligands potentiels,
- analyse de la structure tridimensionnelle des cristaux susmentionnés, notamment par diffraction des rayons X (en vue de sélectionner les ligands ayant une capacité optimale d'occupation et de blocage du site actif desdites protéines).

L'invention concerne également l'utilisation des coordonnées de la structure tridimensionnelle d'une protéine recombinante dérivée de la protéine MabA telles que définies ci-dessus, lesdites coordonnées étant représentées sur les figures 1 à 3, le cas échéant en association avec les coordonnées du site actif de ces protéines telles que définies ci-dessus, pour la mise en oeuvre de méthodes de conception ou de criblage de ligands de la protéine MabA (avantageusement assistées par ordinateur).

A ce titre, l'invention a plus particulièrement pour objet toute méthode de conception ou de criblage de ligands de la protéine MabA, comprenant l'utilisation des coordonnées de la structure tridimensionnelle d'une protéine recombinante dérivée de la protéine MabA telles que définies ci-dessus, lesdites coordonnées étant représentées sur les figures 1 à 3, pour cribler *in silico* des chimiothèques virtuelles de ligands potentiels, avantageusement à l'aide de logiciels informatiques appropriés, et la détection et l'optimisation structurale rationnelle des molécules susceptibles de se lier à ladite protéine.

L'invention a également pour objet toute méthode de conception rationnelle telle que définie ci-dessus, effectuée à partir d'inhibiteurs connus de MabA ou d'inhibiteurs de protéines homologues à MabA (de la même famille structurale des SDR ou RED, et présentant un taux d'identité supérieur à 10% avec MabA sur l'ensemble de la séquence peptidique), pour lesquels la structure tridimensionnelle fine du complexe entre ledit inhibiteur et la protéine MabA recombinante sous forme purifiée, ou une protéine recombinante dérivée de la protéine MabA, telles que définies ci-dessus, a été déterminée, et optimisation structurale rationnelle desdits inhibiteurs. Nous avons montré que l'activité de MabA était inhibée in vitro par un adduit INH-NADP(H). Ce mécanisme d'action de l'isoniazide (INH) sur MabA est similaire au mécanisme d'action de l'isoniazide sur la protéine InhA, cible de l'INH. D'autres protéines faisant partie de la superfamille des RED ont une structure tridimensionnelle comparable à celle de MabA, dont une stéroïde deshydrogénase (PDB1HSD), des tropinone réductases (ex : PDB1AE1), une trihydroxynaphthalène réductase (PDB1YBV) et une mannitol deshydrogénase (PDB1H5Q), et ont été co-cristallisées avec des inhibiteurs.

L'invention sera davantage illustrée à l'aide de la description détaillée qui suit de l'obtention de la protéine MabA recombinante, et des protéines MabA C(60)V, S(144)L, et C(60)V / S(144)L, sous forme purifiée, de leurs propriétés enzymatiques, ainsi que des cristaux de ces protéines et de leurs coordonnées atomiques.

Les coordonnées atomiques de la protéine MabA recombinante (correspondant à SEQ ID NO: 15), et des protéines MabA C(60)V(correspondant à SEQ ID NO : 16), et C(60)V / S(144)L(correspondant à SEQ ID NO : 18), sont respectivement représentées sur les figures 1 à 3, sur lesquelles sont indiquées de gauche à droite le numéro d'atome, le nom des résidus, le numéro de chaîne, les coordonnées x, y, z, l'occupation, et le facteur B.

PARTIE EXPERIMENTALE

La tuberculose, maladie infectieuse provoquée par *Mycobacterium tuberculosis,* demeure la cause majeure de mortalité dans le monde due à un agent infectieux unique. Selon l'Organisation Mondiale de la Santé, 8 millions de

cas de tuberculose apparaissent chaque année, résultant dans 3 millions de décès (Dolin et al., 1994). Alors qu'elle a toujours posé un grave problème de santé publique dans les pays en voie de développement, la tuberculose réapparaît dans les pays développés. Les conditions précaires de certains groupes dans la société et la détérioration des systèmes de santé, conséquences de la crise économique mondiale, ont favorisé cette recrudescence de la tuberculose. De même, l'endémie de l'infection par le virus de l'immunodéficience humaine (VIH) et l'apparition de souches de *M. tuberculosis* résistantes à un ou plusieurs antibiotiques ont aussi fortement contribué à ce phénomène (Barnes et al., 1991). L' émergence de la tuberculose multi-résistante, définie comme la résistance aux deux antibiotiques qui sont à la base du traitement antituberculeux, isoniazide (Rimifon, INH) et rifampicine (RMP), est une menace pour le contrôle de la tuberculose. Les patients infectés par les souches résistantes à plusieurs antibiotiques sont extrêmement difficiles à soigner et le traitement nécessaire est toxique et coûteux. Jusqu'à 30% de l'ensemble des isolats cliniques de *M. tuberculosis* résistants le sont à l'isoniazide (Cohn et al., 1997). Il est donc important de mieux comprendre les mécanismes de résistance à l'isoniazide mis en place par les mycobactéries afin de pouvoir, d'une part, élaborer des techniques rapides permettant la détection des résistances et, d'autre part, développer de nouveaux agents anti-mycobactériens qui soient efficaces contre les souches résistantes.

A partir de nombreux travaux réalisés sur l'isoniazide, une cible directe de cet antibiotique a pu être identifiée: il s'agit d'un métabolisme spécifique des mycobactéries, la biosynthèse des acides mycoliques [(Winder & Collins, 1970); (Takayama et al., 1972); (Quémard et al., 1991)]. Ces acides gras à très longue chaîne sont des constituants majeurs et caractéristiques de l'enveloppe mycobactérienne. Grâce à l'utilisation des outils de biologie moléculaire chez *M. tuberculosis,* une cible moléculaire de l'isoniazide, la protéine InhA, impliquée probablement dans la voie de biosynthèse des acides mycoliques a été caractérisée [(Banerjee et al., 1994); (Quémard et al., 1995)]. InhA appartient à un système enzymatique responsable de l'élongation des acides gras (Marrakchi et al., 2000). Ce système contenant la protéine InhA, cible de l'isoniazide, participe à la biosynthèse des acides mycoliques et représente de ce fait un complexe enzymatique dont les composantes sont intéressantes à étudier, en tant que cibles potentielles de nouveaux antibiotiques antituberculeux. Nous avons donc étudié les propriétés biochimiques de MabA, une des protéines du complexe contenant InhA. La modélisation moléculaire de la structure tridimensionnelle de cette protéine, qui catalyse.la réduction de dérivés β-cétoacyles, a montré que MabA et InhA font partie de la même famille structurale. L'étude de l'effet de l'isoniazide sur l'activité enzymatique de MabA suggère que l'antibiotique inhibe la protéine par un mécanisme similaire à l'action sur InhA. Ainsi, MabA représente une cible intéressante pour la conception d'inhibiteurs de la biosynthèse d'acides gras chez les mycobactéries.

Dans le cadre des travaux de la présente invention, le gène mabA de *M. tuberculosis* a été cloné chez *E. coli,* dans un vecteur d'expression. La protéine est produite en grande quantité par cette souche recombinante, en tant que protéine de fusion possédant une étiquette poly-Histidine en N-terminal. La purification de la protéine est effectuée en une seule étape par chromatographie sur colonne, donnant plusieurs mg de protéine purifiée. Un monomère sauvage de MabA possède 247 acides aminés et a une taille de 25,7 kDa ; le monomère de fusion est de 27,7 kDa. Plusieurs méthodes expérimentales (ultracentrifugation analytique, perméation sur gel, diffusion de la lumière, cristallographie...) nous ont permis de montrer que la protéine native était principalement tétramérique, provenant de l'auto-association de deux dimères. Des propriétés physico-chimiques (stabilité dans différents milieux tampons, à différentes températures, spectres d'émission de fluorescence...) et les principales propriétés enzymatiques de MabA (Kd, Km et kcat pour le coenzyme et des substrats -β-cétoacyl-CoA- de différentes longueurs de chaîne) ont été déterminées. La protéine recombinante purifiée est fonctionnelle ; c'est une β -cétoacyl réductase, NADPH-dépendante, et spécifique de substrats à longue chaîne (C12-C20). Nous avons montré que MabA faisait partie du système d'élongation d'acide gras mycobactérien, FAS-II, et catalyse la 2ème étape du cycle d'élongation.

La structure tridimensionnelle de la protéine MabA a été résolue par cristallographie à 2,05 Å de résolution après mise au point des conditions de cryostabilisation des cristaux. MabA fait partie de la super-famille structurale des protéines SDR (Short-Chain Reductases) ou RED (Reductases, Epimerases, Dehydrogenases). Elle est homologue aux KARs (ketoacyl-ACP reductases), mais représente un membre particulier de cette famille, par la structure de la poche de fixation du substrat. Celle-ci a un caractère plus hydrophobe que celle des protéines homologues. La présence du résidu tryptophane unique dans la poche de fixation du substrat nous a permis de réaliser des expériences de spectroscopie de fluorescence, qui ont mis en évidence une affinité plus marquée de MabA pour des substrats à longue chaîne (C8-C20) par rapport au substrat en C4. Ces résultats, qui corrèlent avec les données de cinétique enzymatique, mettent en évidence une relation structure-fonction entre l'hydrophobicité du site de fixation du substrat et l'affinité de MabA pour des substrats inhabituellement longs chez les bactéries.

Les propriétés distinctes de MabA par rapport aux autres protéines homologues en font une cible de choix pour la conception d'antibiotiques potentiels. Cette conception va faire appel à plusieurs démarches parallèles :

1. la conception rationnelle à partir d'inhibiteurs connus de MabA ou de protéines homologues
2. le criblage à haut débit de chimiothèques virtuelles
3. le criblage à haut débit de chimiothèques réelles.

Nous avons montré que l'activité de MabA était inhibée in vitro par un adduit INH-NADP(H). Ce mécanisme d'action de l'isoniazide (INH) sur MabA est similaire au mécanisme d'action de l'isoniazide sur la protéine InhA, cible de l'INH. D'autres protéines faisant partie de la super-famille des RED ont une structure tridimensionnelle comparable à celle de MabA, dont une stéroïde deshydrogénase (PDB1HSD), des tropinone réductases (ex: PDB1AE1), une trihydroxynaphthalène réductase (PDB1YBV) et une mannitol deshydrogénase (PDB1H5Q). Ainsi, la démarche (1) est basée sur l'utilisation de la structure des ligands (ex : dérivés de l'isoniazide, stéroïdes) de ces différentes protéines pour la conception d'autres inhibiteurs potentiels de MabA, de structures dérivées. La conception rationnelle implique bien-sûr l'utilisation de la structure cristalline de MabA et de la méthode de docking moléculaire assistée par ordinateur. De la même manière, si les démarches (2) et (3) apportent de nouveaux types de ligands potentiels, ceux-ci pourront être la base de nouvelles conceptions rationnelles.

L'invention procure donc une démarche conceptuelle pour le développement d'inhibiteurs de l'activité de la protéine MabA. D'autre part, elle offre une méthode de validation expérimentale d'une part, de la fixation spécifique de ces molécules au site actif de MabA (spectroscopie de fluorescence) et d'autre part, du pouvoir inhibiteur de ces molécules par un test enzymatique simple (cinétique enzymatique par suivi par spectrophotomètrie).

**I) ETUDE DE LA PROTEINE MABA (comparatif)**

Nous avons montré que le système d'élongation FAS-II contient la protéine InhA, une cible de l'isoniazide. De plus, le fait que ce système soit vraisemblablement impliqué dans la biosynthèse des acides mycoliques, composés spécifiques des mycobactéries, fait de FAS-II une cible de choix pour des agents anti-mycobactériens. L'étude des enzymes qui composent ce système est donc une approche intéressante pour rechercher de nouvelles cibles d'antibiotiques.

La forte inhibition de l'activité de FAS-II par l'isoniazide, et surtout le fait qu'aucun intermédiaire de biosynthèse, et en particulier les substrats de InhA, ne s'accumulent sous l'effet de l'INH suggèrent qu'il pourrait exister une autre cible de l'antibiotique en plus de InhA. La protéine MabA, codée par un gène contigu à inhA sur le chromosome de *M. tuberculosis,* fait probablement partie du même système enzymatique que InhA. Plusieurs données laissent supposer que MabA pourrait être une cible de l'isoniazide. D'une part, des mutations ponctuelles dans la région promotrice du locus mabA-inhA d'isolats cliniques de *M. tuberculosis* entraînent la surproduction des protéines en aval et corrèlent avec un phénotype de résistance à l'INH. Ceci suggère qu'en plus de la surproduction de InhA, induite par ces mutations, la surproduction de MabA pourrait également participer à la résistance, si cette protéine interagit avec l'isoniazide. D'autre part, l'étude de l'effet de l'isoniazide (2 mM) sur les enzymes purifiées du système FES isolé de *M. avium* a montré que deux étapes du système sont sensibles à l'INH, la β -cétoacyl réductase (93% d'inhibition et Ki =353 μM), qui est la plus sensible, et l'énoyl-réductase (26% d'inhibition et Ki=5,5 mM) (Kikuchi et al., 1989).

Nous avons donc décidé de purifier la protéine MabA et d'étudier certaines de ses propriétés biochimiques, en adoptant une stratégie de surproduction de la protéine dans un système procaryote.

**1. SURPRODUCTION DE MABA DANS *ESCHERICHIA COLI* (comparatif)**

Réaliser une étude enzymatique et produire des anticorps anti-MabA pour la localisation intracellulaire de MabA, imposait l'obtention de la protéine pure, sous forme soluble et en grande quantité. Pour surproduire MabA, nous avons utilisé un système d'expression et de purification dans *Escherichia coli,* qui est simple et très efficace pour la surexpression de gènes procaryotes. La mise au point d'une procédure expérimentale de manière à atteindre une surproduction suffisante, tout en obtenant la protéine sous forme soluble, a nécessité l'optimisation à plusieurs niveaux du schéma de surexpression et de purification.

**1.1 Clonage du gène mabA de *Mycobacterium tuberculosis* H37Rv (comparatif)**

La détermination de la séquence complète du génome de *Mycobacterium tuberculosis* H37Rv (Cole et al., 1998) et le développement des techniques de biologie moléculaire permettant la manipulation d'ADN recombinant, ont facilité la production et l'étude de la protéine d'intérêt, MabA.

**1.1.1 Description du système d'expression de *mabA* dans *Escherichia coli***

* Choix du vecteur d'expression pET (plasmid for Expression by T7 RNA polymerase)

Dans le vecteur d'expression utilisé, pET-15b (Novagen), le gène cible est cloné sous le contrôle des signaux de transcription et de traduction du bactériophage T7. Le gène mabA (fabG1) de 741 paires de bases, codant pour la protéine MabA a été amplifié par réaction de polymérisation en chaîne (PCR) à partir du cosmide MTCY277 (Institut

Pasteur), et cloné entre les sites de restriction NdeI et Xho du plasmide. Ce plasmide offre l'avantage de pouvoir obtenir en fusion NH2-terminale de la protéine recombinante, une séquence poly-histidine, clivable, permettant une purification rapide de la protéine par chromatographie d'affinité. La construction comprend donc en amont du gène *mabA,* une séquence codant pour 6 histidines successives et le site de clivage par la thrombine.

* Choix de la souche hôte

La souche hôte de *E. coli* choisie, BL21 (λDE3) (Novagen), présente l'avantage d'avoir les 2 gènes *ompT* et *lon* inactivés. Les gènes *ompT* [(Studier & Moffatt, 1986); (Studier et al., 1990)] et *lon* (Phillips et al., 1984) codent respectivement pour la protéase pariétale (responsable de la dégradation des protéines hétérologues) et la principale protéase cytoplasmique (responsable de la dégradation des protéines mal repliées ou instables). BL21(λDE3) est lysogène pour le bactériophage DE3 (dérivé de λ), et porte de ce fait une copie chromosomique du gène de l'ARN polymérase T7 sous le contrôle du promoteur lacUV5, qui est inductible à l'IPTG (isopropyl-β-D-thiogalactopyranoside). L'addition d'IPTG à une culture du lysogène induit l'expression de l'ARN polymérase T7, qui à son tour va transcrire l'ADN cible sur le plasmide.

**1.1.2 Transformation et sélection**

La transformation de la souche E. coli BL21(λDE3) compétente par le plasmide pET-15b::*mabA* a été effectuée par choc thermique (Matériel et Méthodes). L'efficacité de transformation obtenue est de 5,9 103 CFU/µg d'ADN. On note la faible efficacité de transformation caractérisant les souches de *coli* B dont dérive BL21(λDE3).

La sélection des cellules ayant incorporé le plasmide est réalisée grâce à l'acquisition de la résistance à l'ampicilline. Dans nos expériences de sélection, nous avons préféré utiliser la carbenicilline, un β-lactame stable, plutôt que l'ampicilline qui est connue pour être rapidement dégradée par les β -lactamases sécrétées par les bactéries résistantes.

**1.1.3 Vérification de la séquence du gène *mabA* cloné**

Afin de vérifier qu'aucune mutation n'a été introduite lors de l'étape d'amplification par PCR de *mabA,* la séquence du gène cloné a été analysée. Aucune mutation n'a été trouvée; la séquence clonée est identique à celle portée par le cosmide d'origine.

**1.2. Expression hétérologue et optimisation**

L'optimisation de l'expression d'un gène hétérologue nécessite une étude préalable à petite échelle pour déterminer le choix des conditions de culture et des paramètres de l'induction (DO, température, concentration de l'inducteur et temps d'induction). La mise au point de ces conditions nous a permis de définir la procédure à suivre pour obtenir une surproduction suffisante de la protéine qui soit visible en SDS-PAGE. Cependant, malgré nos efforts pour reproduire la surexpression à plus grande échelle, nous ne sommes pas arrivés à faire produire la protéine MabA par les bactéries induites. L'hypothèse la plus plausible était la perte du plasmide, en dépit du maintien des cultures en milieu contenant l'antibiotique. Face à ce problème, le test de stabilité de plasmide sur boîte (Matériel et Méthodes) nous offrait un moyen rapide et fiable de vérifier, dans les cultures avant induction, la présence du plasmide cible d'une part, et la capacité des bactéries transformées, en culture, à exprimer l'ADN hétérologue, d'autre part.

L'expression hétérologue de *mabA* dans *E. coli* s'est avérée particulièrement sensible aux conditions de culture qui affectent la stabilité du plasmide. Pour une expression optimale, deux conditions sont importantes à respecter:

* Les colonies transformantes doivent être fraîches (provenant directement d'une transformation ou d'un étalement par stries à partir d'un stock liquide conservé à -70°C).
* Le nombre de générations entre la transformation des bactéries par le plasmide et l'induction de l'expression doit être réduit au minimum (éviter les cultures intermédiaires).

**2. PURIFICATION DE MABA (comparatif)**

**2.1 Solubilité de la protéine surproduite et optimisation**

Pour établir la stratégie de purification, il est important de savoir si la protéine est produite sous forme soluble, ou localisée dans des corps d'inclusion (agrégats de protéines).

Les tests à petite échelle pour déterminer la solubilité dans les conditions d'induction optimales, ont révélé

certains points intéressants et inattendus. Le premier est que les variations opérées sur les paramètres d'induction (température, DO ou durée d'induction), visant à améliorer la solubilité, ne semblaient pas avoir de conséquences significatives sur la production préférentielle de la protéine MabA sous telle ou telle forme. En revanche, nous avons été surpris de constater que la technique adoptée pour lyser les bactéries modulait la répartition de la protéine entre les fractions soluble et insoluble. Par exemple, la sonication à froid dans un volume réduit (facteur de concentration de la culture FC> 20) est vraisemblablement responsable de la précipitation de MabA dans le culot (fraction insoluble). L'effet des températures locales élevées engendrées par les ultrasons pourrait expliquer ce phénomène d'agrégation-précipitation de MabA. La lyse d'une suspension bactérienne à plus faible densité cellulaire permet d'éviter la précipitation de MabA lors de la sonication.

Une étude effectuée sur les effets des ultrasons sur les enzymes (Coakley et al., 1973) révèle que les extraits cellulaires préparés par désintégration ultrasonique sont sensibles aux dommages causés par les radicaux libres, qui sont probablement générés par les ultrasons, ainsi que par l'effet de températures locales élevées. Ces auteurs montrent que l'effet "endommageant" durant la lyse des bactéries peut-être minimisé par une sonication à forte concentration en cellules et en présence de composants du milieu tels que les sucres (agissant comme des "scavengers" de radicaux). Ces conclusions ne semblent pas être en accord avec nos résultats qui montrent qu'à une plus faible densité cellulaire lors de la lyse de MabA, la protéine se trouve dans la fraction du "soluble". On ne peut toutefois rien avancer quant à l'activité de la protéine dans ces conditions.

Après comparaison de plusieurs techniques de lyse à l'échelle de production voulue, nous avons opté pour une lyse par le lysozyme, suivie d'un cycle de congélation-décongélation. Selon ce protocole, et dans les conditions d'induction adoptées [DO600 = 0.8, 2h à 37°C, 0.8 mM IPTG], une grande partie de la protéine MabA se retrouve dans la fraction soluble.

**2.2 Système de purification**

L'obtention de la protéine MabA avec une étiquette poly-His (H-MabA) facilite sa purification. En effet, la haute affinité des résidus histidine pour les ions métalliques permet d'utiliser la méthode de chromatographie d'affinité sur ion métallique immobilisé (IMAC). Une des matrices les plus utilisées pour son efficacité est le nickel-nitrilotriacétate Ni-NTA-agarose (Qiagen). Le groupement NTA présente 4 sites de chélation intéragissant avec 4 des 6 sites de coordination de l'ion métallique Ni. Les noyaux imidazole des résidus histidine se lient aux ions nickel sur la matrice Ni-NTA. L'ajout de molécules d'imidazole permet, par compétition avec les résidus histidine, de rompre les liaisons entre les protéines et la matrice, et d'éluer la protéine poly-His fixée. L'affinité d'une protéine pour la matrice Ni-NTA-agarose est fonction du nombre de résidus histidine qu'elle possède et qui sont exposés à la matrice. Ainsi, en jouant sur la concentration en imidazole, on peut éluer différentes espèces de protéines présentant des degrés d'affinité différents. L'affinité très élevée des protéines présentant une étiquette poly-His pour le Nickel permet de les séparer de la majorité des protéines co-produites par E. coli. Ainsi, si la fixation de la protéine H-MabA s'avère suffisamment spécifique, la purification se limitera à la seule étape de chromatographie d'affinité.

**2.3 Purification de MabA en conditions natives**

La mise au point des conditions d'élution de la protéine MabA sur résine Ni-NTA-agarose est effectuée à petite échelle (50 μl), en utilisant la méthode par sédimentation de résine dite en batch. Grâce à cette technique, nous avons pu déterminer les différentes concentrations en imidazole nécessaires à l'élution de la protéine MabA et à l'élimination des autres protéines.

La purification adaptée à plus grande échelle est effectuée en colonne ouverte avec 500 μl de résine en suspension (Matériel et Méthodes). Le passage de la purification en batch à la purification en colonne a demandé une étape de mise au point supplémentaire.

Les fractions protéiques correspondant aux différentes étapes de purification sont analysées par SDS-PAGE. Dans le lysat clarifié, la bande majoritaire obtenue entre 30 et 43 kDa et correspondant à MabA, témoigne d'une surproduction assez importante de la protéine sous forme soluble, supérieure à 50% des protéines solubles de E. coli. Après l'étape de fixation des protéines sur la résine, la fraction contenant les protéines non fixées sur la colonne est dépourvue de MabA, indiquant une fixation efficace de la protéine H-MabA à la matrice Ni-NTA. L'élimination d'autres protéines faiblement fixées à la matrice (par la présence d'histidines dispersées dans leur séquence), est obtenue après des lavages extensifs à 50 mM en imidazole. D faut une concentration en imidazole égale à 175 mM pour éluer la protéine H-MabA seule et en très grande quantité. La masse apparente de H-MabA déduite de sa migration électrophorétique est estimée à 35 kDa.

### 2.4 Problèmes de précipitation de MabA au cours de la purification

Durant la purification, nous avons constaté que la protéine MabA, éluée à très forte concentration, précipitait aussitôt dans le tube. Ce comportement souvent observé pour les protéines avec une étiquette poly-His, est probablement dû à des interactions protéine-protéine non spécifiques du fait de la très forte concentration locale en protéine lors de la purification (TALONTM Metal affinity Resin - User Manual CLONTECH).

Les tentatives de solubilisation de la protéine éluée avec des détergents (Triton X-100, NP-40) se sont avérées vaines. Il fallait donc intervenir avant l'élution de la protéine. Afin de prévenir la précipitation de la protéine, nous avons effectué un traitement avant et après purification. Pour vérifier si la protéine précipite, la fraction qui contient MabA après élution est centrifugée (5 min, à 12000g) puis le surnageant et le culot sont analysés par SDS-PAGE (Matériel et Méthodes). Le traitement pré-purification consiste à ajouter au lysat des agents "solubilisants" doux. Après purification, la protéine éluée est récupérée directement dans du glycérol (50% final) (le glycérol est un agent protecteur, très utilisé pour la préservation de l'activité des enzymes).

Trois conditions ont été testées:

* 10% (v/v) glycérol seul
* 10% (v/v) glycérol + 0.1 % (v/v) Triton X-100 (détergent non-ionique)
* 10% (v/v) glycérol + 0.05% (v/v) (7 mM) β-mercaptoéthanol (agent réducteur).

Les trois procédés ont permis d'améliorer la solubilité de la protéine MabA. On note toutefois, lors de l'utilisation du β-mercaptoéthanol à 7 mM, que H-MabA commence à être éluée à une concentration en imidazole bien plus faible (50 mM au lieu de 175 mM).

La solubilisation de MabA en présence des trois agents testés étant comparable, nous avons opté pour l'addition de glycérol 10% seul au lysat.

### 2.5 Protocole optimisé pour la surproduction et la purification de H-MabA

L'optimisation des conditions de surexpression et de purification de H-MabA nous a permis de retenir le protocole suivant :

* Cultiver 200 ml de *E. coli*/h-*mabA* sur LB + CBC 50 μg/ml jusqu'à DO600 = 0.8;
* Induire l'expression avec 0.8 mM IPTG pendant 2h, à 37°C;
* Sédimenter les bactéries par centrifugation 15 min à 10 000g, à 4°C. Reprendre le culot dans 9 ml de tampon de lyse (5 mM imidazole et 500 mM NaCl);
* Ajouter le lysozyme (0,5 mg/ml) et les inhibiteurs de protéases (0,113 mg/ml);
* Congeler une nuit à -70°C;
* Décongeler 1h à température ambiante et traiter par la DnaseI (5 μg/ml) et la RnaseA (10 μg/ml) en présence de MgC12 (10 mM), 15 min à 4°C;
* Centrifuger le lysat à 3 000g puis à 10 000g, et récupérer la fraction soluble;
* Centrifuger le surnageant 45 min à 44 000g, à 4°C et récupérer le "lysat clarifié";
* Ajouter 10% de glycérol pur (v/v) à la fraction soluble et la déposer sur une minicolonne (500 μl de phase Ni-NTA-agarose). Incuber 1h à 4°C sous agitation douce;
* Laver la phase avec 5 x 4 ml de tampon d'élution à 5 mM imidazole;
* Pré-éluer avec 8 x 500 μl à 50 mM imidazole;
* Éluer avec 8 x 500 μl à 175 mM imidazole;
* Laver avec 10 x 500 μl à 250 mM imidazole;
* Rassembler les fractions contenant la protéine, selon leur concentration et leur pureté. Collecter la protéine directement dans un volume égal de glycérol pur, dialyser contre du tampon phosphate de potassium 50 mM pH 7,2, contenant 50 % glycérol et conserver à -20°C.
* Tampon d'élution: 50 mM tampon phosphate de potassium, pH 7.8

### 2.6 Rendement de purification

Grâce au système d'expression et de purification utilisé, il a été possible de purifier la protéine H-MabA jusqu'à homogénéité en une seule étape. Pour connaître approximativement la concentration de la solution de protéine, son absorbance en ultraviolet à 280 nm a été déterminée. Connaissant l'absorbance des résidus de tyrosine et de tryptophane de la protéine (Matériel et Méthodes), le coefficient d'extinction molaire théorique de MabA a été déduit ($\varepsilon_{280\ nm}$ = 9530 $M^{-1}cm^{-1}$) et la concentration molaire de la solution purifiée a été estimée à 40 μM.

Le meilleur rendement de purification (pourcentage de protéine purifiée par rapport à l'ensemble des protéines totales déposées sur la colonne) obtenu est de 57%. A partir de 200 ml de culture, nous avons obtenu environ 20 mg de protéine MabA pure (rendement 100 mg/l de culture), ce qui est très satisfaisant.

## 3. CARACTERISATION DE LA PROTEINE MABA PURIFIEE (comparatif)

### 3.1. Vérification de la séquence peptidique

Le gène *mabA* cloné dans pET-15b a été séquencé, aucune mutation n'a été trouvée. La séquence primaire de la protéine MabA sauvage présente 247 acides aminés. L'étiquette poly-histidine de la protéine recombinante lui ajoute 19 acides aminés (266 acides aminés au total). Le séquençage des 20 premiers acides aminés de la protéine MabA surexprimée a été effectué (Biomérieux, Lyon). Nous avons pu vérifier l'identité de la protéine sur la partie amino-terminale et mettre en évidence la perte de la première méthionine de l'étiquette poly-His. L'élimination de la méthionine amino-terminale de protéines par protéolyse post-traductionnelle est très fréquente chez *E. coli.*

### 3.2. Contrôle de la pureté de l'échantillon

L'analyse par électrophorèse dénaturante (SDS-PAGE) et coloration au bleu de Coomassie de l'éluat MabA montre une bande unique, indiquant l'homogénéité de la préparation. La pureté de la protéine a été également vérifiée par SDS-PAGE après coloration au nitrate d'argent. Aucune bande de protéine contaminante n'est détectée par cette technique de révélation très sensible.

Afin de déterminer la masse de la protéine purifiée avec précision, l'analyse par spectrométrie de masse en électrospray (ESI-MS) a été réalisée.

### 3.3 Détermination de la masse moléculaire

La spectrométrie de masse permet de vérifier très rapidement que la protéine exprimée présente la masse attendue. Nous avons analysé un échantillon purifié par spectrométrie de masse/ionisation électrospray (ESI/MS), en collaboration avec B. Monsarrat (IPBS, Toulouse). Sur le type d'instrument utilisé, on détermine la masse moléculaire d'une protéine avec une précision de 0,01% (1/10 000). La masse de la protéine MabA prédite d'après la séquence du gène (en tenant compte de l'étiquette poly-His) est de 27 860 Da. L'analyse par ESI/MS en introduction directe révèle une masse majoritaire de 27 728 $\pm$ 2 Da.

La différence entre la masse théorique et la masse mesurée (131 unités de masse) correspond à la perte de la première méthionine en extrémité amino-terminale, mise en évidence par le séquençage N-terminal de la protéine. La masse moléculaire de la protéine H-MabA purifiée ainsi déterminée est de 27 728 Da.

La migration de H-MabA en électrophorèse dénaturante vers 35 000 Da pourrait être liée aux caractéristiques physico-chimiques de la protéine et/ou à sa forme native.

### 3.4 Détermination de la structure quaternaire de MabA par filtration sur gel

La chromatographie d'exclusion permet de déterminer la forme native (structure quaternaire) de la protéine en solution à une concentration donnée et dans les conditions définies de pH et de force ionique. Grâce à cette technique, il est possible d'établir une relation entre le volume d'élution de la protéine et son poids moléculaire, par l'intermédiaire d'une courbe de calibration. A partir des profils d'élution des protéines standard (Pharmacia), la courbe de calibration est déduite.

L'élution de la protéine MabA (0,66 mg) a été effectuée dans les mêmes conditions que celles des protéines standards. Sur le chromatogramme, on observe un pic asymétrique élué vers les hauts poids moléculaires. Le volume d'élution correspondant au sommet du pic, indique que la masse moléculaire majoritaire (94,6 kDa) est comprise entre 110 916 Da et 83 187 Da, correspondant à la forme tétramérique ou trimérique de H-MabA, respectivement. Le léger épaulement distingué sur le profil (vers 57,7 kDa) montre la présence, en moindre proportion, d'une forme dimérique (55 458 Da) de la protéine. Ces résultats suggèrent qu'il y a probablement un équilibre dimère-tétramère de la protéine MabA. L'étude de la structure tridimensionnelle par modélisation moléculaire de MabA favorise cette hypothèse (voir ci-après). Il est toutefois important de souligner que la détermination de l'oligomérie par filtration sur gel est dépendante des conditions testées et notamment de la concentration de la solution de protéine. La possibilité de présence de la protéine MabA in vivo sous forme tétramérique sera discutée ci-après.

### 3.5 Quelques propriétés physico-chimiques de MabA

Certaines propriétés physico-chimiques de MabA peuvent être déduites de sa séquence peptidique à l'aide d'un des programmes de calcul disponibles sur internet (aBi). La séquence de 266 acides aminés de la protéine H-MabA produite donne une masse calculée égale à 27729,37 Da. Elle correspond à celle déterminée par ESI/MS, à 1 unité de masse près. Les autres caractéristiques sont résumées dans le tableau I ci-après.

Tableau I: Caractéristiques physico-chimiques de H-MabA

| Paramètre | Valeur | Remarque |
|---|---|---|
| Séquence | 266 acides aminés | Absence de la méthionine N-terminale |
| Masse moléculaire | 27 729 Da | Vérifiée par ESI/MS |
| Coefficient d'extinction* molaire $\varepsilon_{280\ \nu\mu}$ | 9530 $M^{-1}cm^{-1}$ | Faible, présence d'un tryptophane et de trois tyrosines dans la séquence |
| Absorbance* à 280 nm d'une solution à 0,1 % (1 mg/ml) | $A_{280nm}^{0.1\%} = 0{,}348$ | |
| Point isoélectrique pI* | 9,79 | charge nette (+) à pH neutre |
| (*): estimé par calcul, d'après la séquence peptidique. | | |

### 4. ACTIVITE CATALYTIQUE DE MABA (comparatif)

L'attribution d'une activité potentielle à une protéine de fonction inconnue est souvent orientée par la similitude de séquence qu'elle présente avec des protéines connues. L'examen de la structure primaire de la protéine MabA met en évidence une forte identité avec la séquence de la β-cétoacyl-ACP réductase FabG de *E. coli* (44% d'identité sur 241AA), ainsi qu"avec les β -cétoacyl-ACP réductases d'autres bactéries ou de plantes. Cette activité enzymatique correspond à l'une des étapes de la voie classique de biosynthèse d'acides gras. L'élongation d'acides gras par le système mycobactérien FAS-II implique la protéine InhA, qui catalyse l'étape d'énoyl-ACP réduction NADH-dépendante. Le système d'élongation FAS-II étant composé de plusieurs enzymes agrégées, il était logique d'envisager la présence de la protéine MabA associée à InhA dans le même complexe enzymatique. Un argument fort en faveur de l'implication de MabA et InhA dans la même voie métabolique repose sur l'organisation en opéron des gènes *mabA* et *inhA* chez *M. tuberculosis.* Les gènes impliqués dans la biosynthèse d'acides gras sont souvent groupés en "cluster" comme par exemple chez *E. coli* (Rawlings & Cronan, 1992) et chez Vibrio harveyi (Shen & Byers, 1996).

Mettre en évidence l'activité β-cétoacyl réductase de la protéine MabA purifiée est la première étape de sa caractérisation en tant que partenaire potentiel de InhA dans la biosynthèse d'acides gras.

### 4.1 CARACTERISATION ENZYMATIQUE DE LA PROTEINE MABA (comparatif)

#### 4.1.1. Mise en évidence de l'activité catalytique de MabA

#### 4.1.1.1. Description du test enzymatique

L'activité de la protéine H-MabA purifiée a tout d'abord été testée en présence du seul β-cétoacyl-CoA commercialisé, l'acétoacétyl-CoA, et de NADPH comme donneur d'électrons. L'addition de MabA pure aux substrats déclenche la réaction. L'évolution de la réaction est suivie pendant 5 min par la mesure de la décroissance de l'absorbance à 340 nm, traduisant la disparition du co-substrat NADPH en faveur de sa forme oxydée $NADP^+$ (qui n'absorbe pas à cette longueur d'onde).

Dans les conditions d'essai enzymatique standard définies (voir Matériel et Méthodes), H-MabA est capable de réduire l'acétoacétyl-CoA. La protéine purifiée H-MabA est donc fonctionnelle: elle correspond à une β-cétoacyl réductase (KAR: keto-acyl reductase). La présence de l'étiquette poly-His en position N-terminale ne semble pas entraver son activité.

La Substitution du NADPH par le NADH à la même concentration dans le test cinétique entraîne une perte totale de l'activité. La protéine MabA est donc strictement NADPH-dépendante. La présence dans la séquence peptidique de MabA d'un motif de fixation du NADP(H) confirme ce résultat. Les KAR d'autres organismes sont le plus souvent NADPH-dépendantes et présentent une spécificité stricte pour le coenzyme nucléotidique.

#### 4.1.1.2 Paramètres affectant l'activité de la protéine MabA

L'activité d'une enzyme est directement affectée par la concentration de ses substrats, mais également par des paramètres tels que la nature du tampon, le pH, la force ionique, la température. Afin d'optimiser les conditions réactionnelles, nous avons étudié l'effet du pH et de la force ionique sur l'activité de MabA.

* Effet du pH

Nous avons évalué l'effet du pH sur l'activité enzymatique de MabA en utilisant dans le milieu réactionnel, des solutions de tampon phosphate de sodium de pH 5,0 à 8,0. La comparaison des résultats pour la gamme de pH choisie montre que l'activité optimale de MabA est obtenue pour un pH égal à 5,5. Toutefois, à pH acide (5,0 à 6,5), le NADPH est très instable et s'oxyde spontanément, ce qui entraîne une variation de l'absorbance au cours du temps en absence d'enzyme. Nous avons donc décidé de travailler à des pH proches du pH physiologique (entre 7,0 et 7,5), pour lesquels la ligne de base présente une pente négligeable par rapport à la pente de catalyse (inférieure à 5-10 %). D'autres β-cétoacyl-ACP réductases présentent un pH optimal acide (autour de 6,0-6,5) [(Shimakata & Stumpf, 1982); (Caughey & Kekwick, 1982)].

Si MabA présente une meilleure activité à pH 5,5, c'est probablement lié à un événement de protonation impliqué dans la fixation des substrats ou dans la catalyse. Cet événement pourrait concerner deux résidus His de la protéine, H46 et H247 (le pKa du noyau imidazole du résidu histidine est égal à 6,0-6,5), potentiellement impliqués dans le site actif, d'après le modèle structural de MabA.

* Effet de la force ionique

L'activité de MabA testée est constante pour des concentrations en tampon phosphate variant entre 20 et 100 mM. Nous avons opté pour un tampon 80 mM, pH7,0.

* Effet de dilution

Les tests enzymatiques nécessitant une préincubation de H-MabA au cours du temps ont révélé que l'activité catalytique décroît rapidement si l'enzyme est incubée à faible concentration (concentration molaire < 1 μM). L'inactivation par dilution des β-cétoacyl-ACP réductases de *E. coli* et de plantes (*Brassica napus, Persea americana*) a été déjà rapportée (Schulz & Wakil, 1971); (Sheldon et al., 1990) ; (Sheldon et al., 1992)].

#### 4.1.2. Détermination des paramètres cinétiques de MabA

La caractérisation d'une enzyme comprend généralement la détermination de la vitesse réactionnelle maximale, Vmax et de la "constante de Michaelis", Km, pour chaque substrat. La connaissance de ces paramètres s'avère très utile pour les études biochimiques (comparaison de l'affinité pour différents substrats, interaction avec d'autres molécules, comparaison d'isoenzymes de différents organismes) et notamment pour définir l'efficacité d'inhibiteurs ou d'activateurs de l'enzyme.

#### 4.1.2.1 Mesure du Km pour le NADPH

La détermination des paramètres cinétiques Vmax et Km commence par l'estimation de la valeur du Km, en testant deux concentrations de substrat, l'une faible et l'autre élevée. Les vitesses réactionnelles initiales sont ensuite déterminées pour une gamme de concentrations en substrat, de préférence large, couvrant si possible de Km/2 à 5 Km. Nous avons tracé la droite S/v = f (S) ou 1/v = f(1/S) pour visualiser les données, puis nous avons comparé les valeurs de Km et Vmax calculées par cette méthode et celles obtenues par la méthode des moindre-carrés. Les valeurs obtenues sont la moyenne de trois manipulations. La détermination S/v = f (S) donne des résultats proches de ceux obtenus par la méthode des moindre-carrés. La valeur de Km obtenue pour le NADPH, 39 μM, est environ cinq fois supérieure à celle de la protéine InhA pour son cofacteur NADH (8 μM). Ce Km plus élevé est probablement le reflet d'une affinité moins grande de MabA pour son coenzyme. Les Km des β-cétoacyl réductases d'autres organismes pour leur cofacteur sont du même ordre de grandeur que celui de obtenu pour MabA.

#### 4.1.2.2 Mesure du Km pour l'acétoacétyl-CoA

Le Km pour l'acétoacétyl-CoA, déterminé en présence de NADPH, est de 1582 μM. Ce Km, relativement élevé, est bien supérieur aux Km décrits pour d'autres β -cétoacyl-ACP réductases de plantes. Le fait que MabA présente un

Km plus élevé que ces enzymes qui appartiennent à des systèmes de synthèse de novo, donc spécifiques de substrats à chaîne courte suggérait que MabA pouvait avoir une préférence pour des substrats plus longs que 4 carbones. L'étude de la spécificité de MabA pour des substrats à chaîne hydrocarbonée plus longue nous semblait ainsi doublement importante, d'une part pour mieux caractériser l'activité enzymatique de cette protéine et d'autre part, pour comparer la spécificité de substrat de MabA et celle de InhA. La protéine InhA a été montrée spécifique de substrats à longue chaîne (12-24 atomes de carbone), ne présentant pas d'activité en présence du substrat à 4 carbones (crotonoyl-CoA), même à 8 mM (Quémard et al., 1995).

#### 4.1.3. Détermination des constantes cinétiques pour les substrats à longue chaîne

L'utilisation de substrats à longue chaîne (C8 à C20) pose des contraintes liées à leur concentration micellaire critique (CMC). Les acyl-CoAs à longue chaîne sont des composés amphiphiles et ne forment des solutions vraies qu'à faible concentration. Au delà de la CMC, une partie des molécules forme des micelles et la concentration en monomère est fixée à la CMC, donc différente de la concentration totale. Il était donc important d'utiliser des solutions de concentrations inférieures à la CMC. Dans une étude des propriétés physiques des acyl-CoA (Constantinides & Steim, 1985), les CMC de solutions aqueuses de palmitoyl-CoA (C16-CoA) et de stéaroyl-CoA (C18-CoA) déterminées sont respectivement de 70 et 12 μM. La présence d'une insaturation (en position 9) dans le cas de l'oléyl-CoA (C18:1-CoA) élève sa CMC à 33 ?M. La présence d'une fonction cétone sur la chaîne aurait en théorie un effet similaire par rapport à la CMC. Utilisant ces données, nous avons tenté de préparer des solutions de β-cétothioester dont la concentration soit en-dessous de la CMC. Les solutions mères utilisées pour les tests cinétiques sont de 400 μM et 100 μM pour les β-cétothioesters en C8 et C12, respectivement.

#### 4.1.3.1. Mesure du Km pour les β-cétoacyl-CoA en C8 et en C12

Nous avons mesuré les paramètres cinétiques de MabA pour le β-cétooctanoyl-CoA (C8) et le β-cétododécanoyl-CoA (C12). La protéine présente un Km (60 μM) pour le substrat en C8 25 fois inférieur à celui du C4 (1582 μM). Le dérivé en C12 s'avère encore un bien meilleur substrat (Km de 9 μM). Là encore, les valeurs obtenues par la méthode de Hanes et celle des « moindres-carrés » sont proches. Nous avons calculé le rapport Km/Vmax qui reflète l'affinité de l'enzyme pour ses substrats. Km/Vmax est d'autant plus bas que la longueur de chaîne de substrat est longue. Cette corrélation est due non seulement aux plus faibles Km, mais également à des Vmax plus élevés pour les plus longues chaînes.

Les constantes cinétiques Km et Vmax pour le C16 et C20 n'ont pu être déterminées. Pour ces β-cétoacyl-CoA de plus de 12 atomes de carbone, des problèmes d'inhibition par le substrat ont été rencontrés, décrits également dans le cas de l'utilisation de substrats de InhA de taille supérieure à C16. Nous avons donc comparé les vitesses initiales de réaction à une même concentration (2 μM), en présence des différents β-cétoacyl-CoA (C4 à C20). Pour mesurer l'activité, il était nécessaire d'utiliser des solutions d'enzymes à différentes concentrations pour les divers substrats β-cétothioesters. La protéine MabA présente une large préférence pour le substrat à 12 carbones par rapport aux substrats courts, et les β-cétothioesters en C16 et C20 s'avèrent au moins aussi bons substrats que le C8. La baisse de la vitesse de réaction observée pour les longues chaînes de β-cétoesters pourrait être liée à leur faible solubilité (dans le cas où la concentration réelle en molécules libres serait inférieure à 2 μM).

#### 4.1.3.2. Spécificité de substrat et implication dans une voie d'élongation?

Bien qu'elle présente une activité en présence du β-cétoacyl à 4 carbones, la protéine MabA montre néanmoins une nette préférence pour les substrats en C12-C16. L'affinité de MabA pour les substrats à longue chaîne hydrocarbonée est compatible avec la taille et la nature hydrophobe de la poche de fixation du substrat. La protéine InhA présente quant à elle une affinité légèrement différente, avec une préférence pour des substrats plus longs C16-C24 (Quémard et al., 1995). Les propriétés enzymatiques de MabA et InhA, notamment leur spécificité pour des substrats à chaîne moyenne à longue, va dans le sens de leur appartenance au même système d'élongation d'acides gras, FAS-II, qui est lui-même spécifique de substrats en C12-C18.

La spécificité de substrat de InhA diffère de celle des énoyl réductases des systèmes de type II de Spinacea oleracea (Shimakata & Stumpf, 1982)) ou de E. coli (Weeks & Wakil, 1968), qui ont une préférence pour des substrats en C6 et C8. De plus, la β-hydroxyacyl deshydratase du système de type II de E. coli (Birge & Vagelos, 1972) est spécifique de substrats à courte chaîne (C4 à C12), alors qu'elle est très peu active en présence de substrat en C16. Ces données mettent en avant la spécificité de substrat particulière du système FAS-II mycobactérien.

#### 4.1.4. MabA et ACP-dépendance?

Le complexe enzymatique contenant InhA que nous avons identifié comme le système d'élongation FAS-II, outre sa spécificité pour les substrats C12-C18, a comme propriété d'être ACP-dépendant. L'ACP-dépendance de la protéine InhA est illustrée par son affinité beaucoup plus marquée pour les substrats dérivés d'ACP (le Km pour l'octènoyl-ACP est de 2 ordres de grandeur inférieur à celui pour le dérivé de CoA en C8). Déterminer la préférence de MabA pour des dérivés d'ACP nécessite la synthèse de ces dérivés (non commerciaux) et la comparaison des constantes cinétiques avec celles des dérivés de CoA. Les KAR de plantes sont ACP-dépendantes, propriété qui a été corrélée à leur appartenance à un système de type II. Les nombreux arguments en faveur de l'appartenance de MabA à FAS-II suggèrent fortement l'ACP-dépendance de la β -cétoacyl réductase.

#### Conclusion

Après mise au point de la surproduction de la protéine MabA dans *Escherichia coli* et purification, nous avons réalisé une étude enzymatique de cette protéine. Ainsi, nous avons montré que son activité catalytique correspond à la réduction NADPH-dépendante de β-cétoesters, ce qui correspond à l'une des étapes de la voie d'élongation d'acides gras. La détermination de l'activité de MabA en présence de substrats de plusieurs longueurs de chaîne a permis de montrer la préférence de cette enzyme pour des substrats de taille supérieure ou égale à 12 atomes de carbone, en accord avec son implication potentielle dans un système d'élongation d'acides gras. Nous avons donc recherché la protéine MabA dans le complexe enzymatique FAS-II contenant InhA, et étudié l'implication de MabA dans l'activité d'élongation de ce système.

### 5. APPORT DE LA MODELISATION MOLECULAIRE A L'ETUDE DE LA PROTEINE MABA (comparatif)

La modélisation moléculaire permet d'accéder à un ensemble d'informations concernant les caractéristiques structurales de la protéine, l'architecture du site catalytique, mais également d'apprécier les possibilités d'interaction avec des ligands (substrats, inhibiteurs, molécules affines). L'élaboration du modèle tridimensionnel de la protéine MabA est présentée ci-dessous.

#### 5.1. Recherche, de protéines présentant une forte similitude de séquence avec MabA

La recherche de séquences peptidiques similaires à celle de MabA (*M. tuberculosis*) dans les banques de données avec le logiciel Psi-blast (Altschul et al., 1997) a montré qu'il existait des β-cétoacyl-ACP réductases présentant un fort taux d'identité avec MabA (87%, 84%,69%, respectivement) dans d'autres espèces mycobactériennes (avium, smegmatis, leprae). Des protéines homologues à MabA, appelées FabG, sont également présentes dans d'autres organismes, essentiellement des bactéries (exemple dans Streptomyces ceolicolor, 57% d'identité) et des plantes. Cependant, aucune structure de β-cétoacyl-ACP réductase n'a jamais été résolue. Réaliser un modèle moléculaire de MabA représentait donc un intérêt dans l'étude des FabG.

#### 5.2. Elaboration du modèle de MabA

La modélisation structurale de MabA a été effectuée en utilisant le programme Modeller 4 (Sali & Blundell, 1993). Le modèle est basé sur les structures de protéines cristallisées en complexe avec le NAD(P)(H) et présentant le plus fort taux d'identité et le plus faible score de probabilité (E) avec MabA. Ces protéines "support", sélectionnées grâce au logiciel Psi-blast (Altschul *et al.,* 1997) dans la principale banque de données de structure protéiques, la PDB (Protein Data Bank, (Berman *et al.,* 2000)), sont: PDB2HSD (34%/NAD); PDB1YBV (33%/NADPH); PDB2AE2 (29%/NADP); PDB1FMC (28%/NADH); PDB1CYD (28%/NADPH) et PDB1BDB (27%/NAD). Ces protéines, d'origine très diverses, catalysent toutes soit la réduction d'un carbonyle (comme MabA), soit la réaction inverse. L'alignement de séquences utilisé pour la modélisation a été réalisé en considérant les régions bien conservées entre MabA et les supports, d'une part, et entre MabA et les autres β-cétoacyl-ACP réductases (FabG) connues, d'autre part. Pour vérifier que le modèle est énergétiquement stable, deux programmes ont été utilisés, TITO (Labesse & Momon, 1998) et Verify-3D (Luthy *et al.,* 1992), qui ont donné des scores satisfaisants.

La structure monomérique donnée par le modèle de MabA indique que la protéine appartient à la superfamille structurale α/β, avec six hélices α et sept brins β. Notons que la boucle β6-α6" comporte deux hélices appelées α6 et α6'. MabA possède un seul domaine, dont la topologie est similaire au repliement $(\beta/\alpha)_6$ de Rossmann (Rossmann *et al.,* 1974), typique des protéines fixant un dinucléotide diphosphate (dinucléotide diphosphate-binding protein, DDBP) (Persson *et al.,* 1991). Toutefois, contrairement aux DDBP à deux domaines, la symétrie n'est pas respectée, puisque les hélices de la moitié C-terminale (α4, α5) sont plus longues que les structures secondaires de la partie N-terminale.

Ces caractéristiques, ainsi que la présence d'un brin supplémentaire (β7), sont typiques de la superfamille des protéines RED (Reductase/Epimerase/Deshydrogenase) (Labesse *et al.,* 1994). La présence d'une cystéine unique (C60), probablement enfouie, dans MabA exclut la possibilité de formation de pont disulfure intra- ou inter-chaîne au sein de la protéine.

♣ Le cofacteur NADPH fixé se trouve dans une conformation étendue reposant sur les extrémités C-terminales des brins β1-β5 qui forment un feuillet. Le brin β2 des protéines RED qui est impliqué dans la fixation du ribose lié à l'adénine du cofacteur, présente dans la séquence de MabA le motif [***xxr] ♣ , spécifique des enzymes NADP(H)-dépendantes (Labesse *et al.,* 1994). Ceci est en accord avec les données enzymatiques montrant la spécificité stricte de MabA pour le NADPH et indique que le phosphate supplémentaire est probablement important pour la stabilisation du cofacteur dans la bonne orientation pour la catalyse. Le résidu chargé positivement R47, faisant partie du motif [VAVTHR] du brin β2, est probablement impliqué dans l'interaction de la protéine avec le phosphate, par des liaisons électrostatiques. *: résidu hydrophobe, x: n'importe quel acide aminé. En lettres majuscules et minuscules les résidus strictement conservés et les plus fréquemment rencontrés, respectivement.

Comme dans les autres protéines RED, le site de liaison du substrat de MabA est probablement délimité par les extrémités C-terminales des brins β4, β5, β6, β7 et les hélices α4, α5, α6 (α6, α6', α6") (Fig. 5.18; (Labesse *et al.,* 1994)); la partie nicotinamide du NADPH, impliquée dans les échanges ioniques, est orientée vers le fond de la cavité. Les résidus du site actif très bien conservés, et constituant en partie la signature des protéines RED, sont présents dans le site catalytique de MabA: la triade catalytique, S140, Y153, K157 et N112, T188.

**5.3. Relation entre structure et fonction de MabA**

D'après les coordonnées atomiques du modèle de MabA, l'unique tryptophane (W145), situé au niveau de la boucle β5-α5, est probablement impliqué dans la poche catalytique. Celle-ci apparaît très hydrophobe en raison de l'implication du bras C-terminal (riche en résidus hydrophobes) dans la structure de cette poche d'une part, et par la présence, en plus du W145, de résidus tels que I147 et F205, d'autre part. Dans les protéines FabG d'autres organismes et spécifiques de substrats à chaîne courte, ces deux derniers résidus sont remplacés par des résidus plus polaires, Asn (pour 1147) et Thr, Gln ou Asn (pour F205). La spécificité de MabA pour des substrats à longue chaîne est très probablement liée au caractère hydrophobe de la poche catalytique qui constitue ainsi un environnement favorable à l'accueil de longues chaînes aliphatiques, une relation structure-fonction entre l'hydrophobicité et la taille de la poche de fixation du substrat et l'affinité pour des molécules à longue chaîne a déjà été mise en évidence pour la protéine InhA (Rozwarski *et al.*, 1999), qui fait également partie des REDs.

La superposition du modèle de MabA avec la structure cristalline de InhA (complexe ternaire InhA-$NAD^+$-substrat en C16, (Rozwarski *et al.,* 1999) ; PDB1BVR) révèle que les poches de liaison du substrat des deux protéines présentent des tailles similaires, en accord avec leur affinité pour des substrats possédant des longueurs de chaîne similaires ($C_{12}$-$C_{24}$ pour InhA, $C_8$-$C_{20}$ pour MabA; [(Rozwarski *et al.,* 1999) ; (Quémard *et al.,* 1995)]. Toutefois, la poche de liaison de l'énoyl réductase InhA est encore plus hydrophobe que celle de MabA, ce qui pourrait expliquer le léger décalage dans la spécificité de substrats entre InhA (activité spécifique maximale en présence du $C_{16}$, (Quémard *et al.,* 1995)) et MabA (activité spécifique maximale en présence du $C_{12}$).

L'alignement de séquences de MabA avec les protéines supports et avec l'ensemble des protéines FabG connues indique que l'extrémité amino-terminale n'est pas conservée; cette région "flotte" à l'extérieur de la protéine et ne correspond pas à une structure secondaire définie. Ceci suggère que ce domaine de la protéine peut tolérer des variations, et qu'il n'est pas important pour la fonction de la protéine. Une donnée expérimentale en accord avec cette proposition est fournie par l'étude de l'activité catalytique de H-MabA. La protéine comporte une étiquette poly-histidine en $NH_2$-terminale dont la présence ne semble pas affecter l'activité catalytique.

**5.4. Structure quaternaire de MabA**

Du fait de leurs structures secondaire et tertiaire caractéristiques, toutes les protéines RED décrites sont dimériques ou tétramériques (dimère de dimères). La région C-terminale de MabA, correspondant à la boucle α6-β7 et au brin β7, présente une très forte similitude avec la région équivalente des REDs tétramériques connues, en particulier avec celle de PDB2HSD pour laquelle il a été montré que cette région était impliquée dans l'interface dimère-dimère de l'hétérotétramère (Persson *et al.,* 1991). La seconde interface entre deux monomères dans PDB2HSD implique les hélices α4 et α5. La conservation dans MabA de l'extrémité C-terminale et la présence d'acides aminés hydrophobes à la surface des hélices α4 et α5 suggèrent que MabA est tétramérique. Ces résultats sont en accord avec l'analyse par chromatographie d'exclusion, suggèrant un équilibre entre les formes dimérique et tétramérique de MabA. L'analyse des interfaces monomère-monomère et dimère-dimère dans un modèle tétramérique de MabA pourrait permettre de renforcer cette conclusion.

### 5.5. Interaction avec des antibiotiques

Il a été montré que la forme active de l'INH qui inhibe la protéine InhA serait un radical ou anion isonicotinoyl-NAD (Rozwarski *et al.,* 1998). Ces auteurs ont suggéré que l'adduit isonicotinoyl-NAD se forme dans le site catalytique de InhA, alors que Wilming et Johnsson ont montré que sa formation peut se faire en absence de l'enzyme (Wilming & Johnsson, 1999). Ainsi, un doute subsiste quant au mode d'action exact de l'INH sur InhA *in vivo.* La superposition du modèle de MabA avec la structure du complexe binaire InhA-isonicotinoyl-NAD (PDB1ZID) montre qu'il n'y a pas d'incompatibilité pour la fixation, dans le site actif de MabA, de molécules telles que l'isoniazide ou l'éthionamide. De même que pour la protéine InhA, l'adduit isonicotinoyl-NADP pourrait *a priori* se fixer sur MabA, une fois qu'il est formé. Toutefois, dans le cas où l'adduit se formerait au sein du site catalytique, on ne peut prévoir si l'isoniazide aurait une orientation adéquate et pourrait interagir avec le cofacteur NADPH. En tous les cas, une inhibition de l'activité de MabA par l'INH doit être vérifiée biochimiquement, car le modèle ne permet pas de renseigner de façon précise sur la topologie des chaînes latérales du site actif.

## 6. INHIBITION DE L'ACTIVITE DE MABA (comparatif)

Nous avons testé l'effet de l'isoniazide sur l'activité β-cétoacyl réductase de MabA en adoptant des conditions expérimentales similaires à celles permettant d'observer une inhibition de l'activité de InhA. La protéine MabA (150 nM) est préincubée 2 heures en présence de 100 μM ou 2 mM isoniazide, 100 μM NADPH et 1 μM $MnCl_2$. En présence de 100 μM en INH, l'activité de MabA, mise en évidence en présence d'acétoacétyl-CoA, est inhibée de 44 ± 3% par rapport au témoin sans INH et l'addition de 2 mM d'isoniazide, entraîne une inhibition de 62 ± 6%. Le fait que l'on n'observe pas d'inhibition totale de l'activité de MabA même en présence de 2 mM isoniazide pourrait être expliquée par une oxydation très lente de l'isoniazide par les ions $Mn^{3+}$ dans les conditions utilisées (en absence de catalyseur tel que KatG), et donc une concentration en forme active de l'antibiotique qui n'est pas proportionnelle à la concentration de départ en isoniazide. Cette explication suppose que MabA est inhibée par un mécanisme similaire à celui décrit pour InhA. Rappelons que le mécanisme d'inhibition de la protéine InhA par l'isoniazide nécessite au minimum la présence du cofacteur NADH, d'ions $Mn^{2+}$ et de l'oxygène moléculaire. Les ions $Mn^{2+}$ seraient oxydés en $Mn^{3+}$, qui, à leur tour catalysent l'oxydation de l'isoniazide. Ainsi, nous avons testé l'effet de l'absence de $MnCl_2$ ou de NADPH sur l'inhibition de MabA par l'INH. En absence de $MnCl_2$ dans la réaction, une baisse non significative de l'activité de l'enzyme est observée, indiquant que les ions $Mn^{2+}$ sont nécessaires pour obtenir un effet de l'INH. La détermination de l'implication du NADPH dans le processus d'inhibition est plus délicate à réaliser, car la préincubation de la protéine MabA en absence de ce cofacteur entraîne une baisse importante (-74%) de l'activité après 2h de préincubation sans antibiotique. On n'a donc pas pu évaluer l'implication du NADPH dans l'inhibition par l'isoniazide.

Nos résultats montrent que l'activité de la protéine MabA est inhibée par l'isoniazide, et suggèrent que le mécanisme d'action de cet antibiotique sur MabA ferait intervenir les ions $Mn^{2+}$. Etant donnée l'homologie de structure et de fonction des deux protéines, il est probable que le mécanisme d'inhibition soit analogue à celui de InhA, passant par la formation d'un adduit isonicotinoyl-$NADP^+$.

Chez les mycobactéries, une mutation ou une surexpression du gène *inhA* entraîne une résistance croisée aux deux antituberculeux isoniazide et éthionamide (ETH). L'éthionamide est probablement également une prodrogue puisque l'inhibition de la protéine InhA par cet antibiotique sous sa forme native n'a pas été observée *in vitro.* Cependant, le mode d'activation de l'ETH n'est pas connu. Nous avons néanmoins testé l'effet de l'éthionamide sur l'activité de InhA, dans les conditions expérimentales d'inhibition par l'INH, en présence de $MnCl_2$ et de NADH. En présence de 100 μM d'ETH, l'activité de InhA est inchangée. Le même résultat est obtenu sur MabA. Il n'a pas été possible de tester des concentrations supérieures en antibiotique du fait de la forte absorbance qu'il présente dans la région de longueur d'onde utilisée pour les tests enzymatiques. Néanmoins, les conditions d'oxydation de l'INH *in vitro,* adoptées pour le test de l'éthionamide, ne semblent pas celles requises pour l'activation de l'ETH. Le fait que la catalase-peroxydase KatG, qui accélère l'oxydation de l'isoniazide ne soit pas l'activateur de l'ETH [(Johnsson *et al.,* 1995) ; (Basso *et al.,* 1996)] est en accord avec cette conclusion. D'autre part, si une oxydation de l'ETH est requise pour son action sur sa (ses) cibles (s), l'oxydation d'une fonction thioamide s'avère plus difficile que celle d'une fonction hydrazide (INH) et devrait nécessiter un oxydant plus fort que les ions $Mn^{3+}$.

## 7. CONCLUSION ET DISCUSSION

L'étude de la structure tridimensionnelle de MabA par modélisation moléculaire, a permis de montrer que la protéine présente un seul domaine, avec une structure secondaire de type α/β, et qu'elle appartient à la superfamille structurale des RED (réductases/épimérases/deshydrogénases). La protéine MabA possède le motif spécifique des protéines fixant le NADP(H) et une poche de fixation du substrat dont la taille et l'hydrophobicité favoriseraient l'accueil de β-cétoesters à longue chaîne. Ces données structurales fournies par le modèle de MabA sont en accord avec les

résultats biochimiques obtenus précédemment. Le modèle de MabA indique que le résidu tryptophane (Trp), situé au niveau de la boucle β5-α5, serait impliqué dans la poche de fixation du substrat. Grâce à l'unicité de ce résidu Trp, des expériences de spectroscopie en fluorescence ont pu être réalisées. Elles ont permis de valider le modèle de MabA, confirmant d'une part, l'implication du Trp et d'au moins une des deux Met de l'extrémité C-terminale dans la poche de fixation du substrat, et la spécificité de MabA pour des substrats à longue chaîne, d'autre part. La superposition du modèle de MabA avec la structure cristalline de InhA (en complexe avec $NAD^+$ et le substrat en C16, (Rozwarski *et al.,* 1999) révèle que les deux protéines présentent des sites de liaison du substrat de taille équivalente et plus hydrophobes que leurs homologues d'autres organismes, impliqués dans une synthèse *de novo.* Ceci conforte l'hypothèse de co-implication de MabA et InhA dans le même complexe d'élongation d'acides gras.

Le modèle de MabA suggère que la protéine en solution est tétramérique, ce qui est en accord avec le résultat des expériences de filtration sur gel, ayant suggéré qu'il y avait, dans les conditions expérimentales testées, un équilibre dimère-tétramère de MabA. Cependant, l'association de MabA avec InhA, chacune sous forme tétramérique dans le complexe FAS-II, est incompatible avec la taille estimée du système. Ainsi, comme InhA et MabA présentent des topologies similaires, on pourrait postuler que ces deux protéines forment un hétérotétramère au sein du complexe FAS-II. Pour tester cette hypothèse, la modélisation moléculaire d'un complexe hétérotétramère MabA-InhA, utilisant les protéines RED tétramériques comme supports, pourra être réalisée. Pour confirmer la possibilité que InhA et MabA puissent s'associer en complexe, un pontage chimique entre les deux protéines, en présence de leurs cofacteurs respectifs, pourra être tenté.

La connaissance de l'organisation tridimensionnelle donnée par le modèle a permis de suggérer une interaction possible entre MabA et l'isoniazide. Nous avons pu montrer, par des études enzymatiques, que l'activité de MabA était effectivement inhibée *in vitro* par cet antibiotique et que le mécanisme d'inhibition de MabA est probablement comparable à celui décrit pour la protéine InhA.

## <u>II) MATERIEL ET METHODES</u>

### M.1. SUREXPRESSION DU GENE *mabA* DANS *E. COLI*

#### M.1.1. Construction du plasmide d'expression pET-15b::*mabA*

Le gène *mabA* de *M. tuberculosis* a été cloné entre les sites NdeI et Xho du plasmide pET-15b, en aval d'une séquence codant pour 6 histidines.

#### M.1.2. Transformation de *E. coli* BL21(λDE3) par le plasmide pET-15b::*mabA*

Après préparation des bactéries compétentes de *E. coli* BL21 (λDE3) (Sambrook *et al.,* 1989), un aliquot (100 μl) est incubé en présence du plasmide pET-15b::*mabA* (39 ng) pendant 30 min, dans la glace. La transformation est effectuée par choc thermique (90 sec. à 42°C, puis 2 min dans la glace). Du milieu LB est alors ajouté et la suspension est incubée 45 min à 37°C sous agitation (250 rpm) avant d'être étalée sur boîte LB-agar contenant 50 μg/ml de carbenicilline. L'incubation à 37°C pendant 18h environ permet d'obtenir des colonies de taille moyenne à grosse.

#### M.1.3. Induction de l'expression du gène cible

Quatre colonies de taille moyenne sont utilisées pour ensemencer quatre cultures de 50 ml en milieu LB + carbenicilline. La turbidité du milieu est mesurée par spectrophotométrie à 600 nm toutes les heures jusqu'à ce que la densité optique atteigne 0,8 (milieu de la phase exponentielle de croissance), c'est à dire après 4h d'incubation environ. L'expression du gène *mabA* est alors induite avec 0,8 mM d'IPTG pendant 2h à 37°C, puis vérifiée par SDS-PAGE. Un aliquot de culture non-induite est conservé et servira de témoin négatif de l'induction.

#### M.1.4. Vérification de la surexpression

Une fois l'expression de *mabA* induite, un aliquot de 100 μl de culture est analysé pour contrôler l'expression du gène. Après centrifugation (5 min à 12000g), le culot bactérien est repris dans du tampon de charge (Laemmli, 1970) pour être déposé sur gel de polyacrylamide 12% en conditions dénaturantes.

#### M.1.5. Test de solubilité de la protéine MabA à petite échelle

Les bactéries (10 ml) sont collectées par centrifugation 5 min à 3000g, à 4°C. Le culot est resuspendu dans du tampon phosphate de potassium (100 mM, pH 7,2) dans 1/20 du volume initial de la culture. La suspension est

soniquée à l'aide d'une microsonde (Vibracell, Bioblock), en quatre pulses de 10 sec. intercallés de temps de repos de 40 sec. (duty cycle: 60%, microtip limit: 5). L'extrait total obtenu est centrifugé 5 min à 12000g, à 4°C. La présence de la protéine MabA dans les fractions correspondant au total, surnageant (soluble) et culot (insoluble) sont analysées par SDS-PAGE (12% de polyacrylamide).

**M.2. PURIFICATION DE MABA**

Toutes les étapes sont effectuées au froid (0-4°C), pour réduire l'action des protéases.

**M.2.1. Préparation du lysat bactérien**

Les cultures rassemblées (4 x 50 ml) sont collectées par centrifugation (15 min à 16000g, à 4°C) puis lavées (tampon phosphate de potassium 50 mM, pH 7,8). Le culot obtenu (0,9 g/ 200 ml de culture) est repris dans 9 ml de tampon de lyse (tampon phosphate de potassium 50 mM, pH 7,8 contenant 500 mM de NaCl et 5 mM d'imidazole). Avant de congeler la suspension à - 70°C (une nuit), un mélange d'inhibiteurs de protéases (0,113 mg/ml, voir ci-dessous) et du lysozyme (0,5 mg/ml) y sont ajoutés. La suspension est décongelée, sous agitation douce, à température ambiante, puis traitée à la DNaseI (5 μg/ml) et RNaseA (10 μg/ml) en présence de $MgCl_2$ 10 mM pendant 15 min à 4°C, sous agitation douce. Les bactéries entières et les débris sont éliminés par centrifugation (15 min à 3000g, à 4°C). Une dernière ultracentrifugation à 44000g, 45 min à 4°C, permet d'éliminer tout matériel insoluble. 10% (v/v) de glycérol est ajouté au surnageant (lysat clarifié) avant d'être chargé sur la colonne.

**Mélange d'inhibiteurs de protéases :**

- ♣ leupeptine (inhibiteur de la chymotrypsine): 0,0023, g/l
- ♣ soybean (inhibiteur réversible de la trypsine) : 0,02 g/l
- ♣ TLCK (inhibiteur irréversible de la trypsine) : 0,0518 g/l
- ♣ Aprotinine 0,0016 g/l
- ♣ Pepepstatine (inhibiteur des pepsine-like) 0,0011 g/l
- ♣ PMSF (inhibiteur irréversible de la chymotrypsine) 0,0362 g/l

Remarque : Dans ces expériences, l'EDTA (inhibiteur des protéases métal-dépendantes) est omis du mélange d'inhibiteurs de protéases, à cause de sa capacité à chélater les ions nickel lors de la purification sur colonne Ni-NTA.

**M.2.2. Purification de H-MabA en minicolonne**

Dans une minicolonne vide (volume total 7,5 ml en polypropylène, Polylabo), 500 μl de résine Ni-NTA agarose (QIAGEN) (soit 1 ml de suspension à 50%) sont lavés avec 4 fois 2,5 ml de tampon de lyse*. Quatre ml de lysat bactérien clarifié (environ 15 mg de protéines totales) sont incubés avec la résine Ni-NTA sous agitation douce, pendant 1h à 4°C. Le matériel non fixé à la résine est récupéré par décantation, puis par "lavages" avec 32 VC (volumes de colonne) de tampon de lyse. Le reste des protéines contaminantes est élué avec 8 VC de tampon à 50 mM imidazole. La protéine MabA est éluée par 8 VC de tampon à 175 mM imidazole. La résine est ensuite nettoyée avec 10 VC de tampon à 250 mM imidazole est récupérée directement dans du glycérol pur de manière à avoir 50 % (v/v) de glycérol final. Ces précautions sont nécessaires pour éviter la précipitation de MabA en sortie de colonne.

Remarque: tous les tampons utilisés ici contiennent 50 mM de phosphate de potassium pH 7,8 et 500 mM de NaCl.

* tampon de lyse: tampon phosphate de potassium 50 mM, pH 7,8 contenant 500 mM NaCl et 5 mM d'imidazole.

**M.2.3. Dialyse de la solution de H-MabA purifiée**

La solution de protéine MabA après purification, récupérée dans du glycérol 50% (v/v), est dialysée 2 fois 1 h contre 40 volumes de tampon phosphate de potassium 50 mM pH 7,2 contenant 50% (v/v) de glycérol, à 4°C, dans un tube de dialyse (seuil de coupure 8-10 kDa, Spectra/Por, Spectrum) préalablement bouilli dans une solution d'EDTA 1 mM pour éliminer les traces de métaux lourds, puis rincé à l'eau osmosée. Le dialysat est ensuite aliquoté et conservé à -20°C.

**M.2.4. Détermination de la quantité de protéine purifiée par spectroscopie U.V**

Nous avons estimé la concentration des solutions de protéine purifiée d'après la loi de Beer-Lambert (DO = ε1C*) avec son coefficient d'extinction molaire théorique et son absorbance à 280 nm.

* s:coefficient d'extinction molaire, 1 : longueur du trajet optique et C : concentration molaire.

**Détermination théorique du coefficient d'extinction molaire (déduite de la séquence de la protéine)**

Les coefficients d'extinction molaire (CEM) de la protéine sont calculés d'après Gill et Von Hippel (Gill & von Hippel, 1989) (en présence de chlorure de guanidine 6M à pH 6,5). La relation est alors la suivante :

CEM = (a * ETyr) + (b * ETrp) + (c * ECys), où a, b et c sont respectivement le nombre de résidus, et Eaa leurs coefficients d'extinction molaire. A 280nm, ils sont respectivement égaux à : ETyr=1280 ETrp=5690 ECys=120

Le CEM ($\varepsilon$) de MabA à 280nm est de 9530 $M^{-1}cm^{-1}$ et ne tient pas compte de l'unique résidu Cys.

## M.3. ETUDE DES PROPRIETES DE MABA

### M.3.1. Détermination de la masse de H-MabA par spectrométrie de masse/ionisation électrospray (ESI/MS)

Un culot de 2 mg de protéine H-MabA purifiée, précipitée dans du tampon sans glycérol, est lavé 5 fois à l'eau (5 min de centrifugation à 12000g). 200 $\mu$l de mélange acétonitrile/eau (50/50) + TFA 0, 1 % (v/v) sont ajoutés, puis le tout est vortexé et centrifugé 2 min à 12000g. Un volume égal d'un mélange méthanol/eau (50/50) + acide acétique 0,5% (v/v) est ajouté à un aliquot du surnageant, l'ensemble est vortexé puis gardé 2h à 4°C avant d'être centrifugé 2 min à 12000g. 60 $\mu$l du surnageant sont introduits dans la source du spectromètre par l'intermédiaire d'une pompe seringue (HARVARD), à un débit de 5 $\mu$l/min, pour être analysé par spectrométrie de masse/ionisation électrospray (ESI/MS) sur un appareil Finnigan MAT (TSQ 700). Les paramètres de la source ESI correspondent à une alimentation de 5kV, une température du capillaire intermédiaire de 250°C et à 40 psi pour le l'azote (gaz de nébulisation).

### M.3.2. Détermination de la taille native par filtration sur gel

Les expériences de FPLC ont été réalisées avec le système BioCAD SPRINT (PerSeptive Biosystems, Cambridge, MA). Une colonne Sephacryl S-100 HR (HiPrep™ 16/60 Sephacryl High Resolution, Pharmacia) a été équilibrée avec 1 VC de tampon phosphate de potassium 50 mM, pH 6,8 contenant 100 mM NaCl. Cinq protéines standards de masses moléculaires connues, diluées dans ce même tampon, ont été appliquées sur la colonne (0,5 à 1 mg de chaque protéine): alcool deshydrogénase (150 kDa), albumine de sérum bovin (BSA, 67 kDa), ovalbumine (43 kDa), anhydrase carbonique (29 kDa) et RibonucléaseA (RNaseA, 13,7 kDa). Deux élutions successives ont été effectuées avec une combinaison différente de 3 protéines standards pour obtenir une meilleure résolution des pics, et les profils à 280 nm ont été superposés. La courbe de calibration a été obtenue en reportant le volume d'élution de chaque protéine standard en fonction du logarythme de la masse moléculaire. Une solution de H-MabA à 1,1 mg/ml (0,66 mg de protéine chargé) est déposée sur la colonne et éluée dans les mêmes conditions que les protéines standard. La masse moléculaire de H-MabA est estimée en se reportant à la courbe de calibration.

## M.4. ETUDE ENZYMATIQUE DE MABA

### M.4.1. Calibration des solutions de réactifs

La détermination des paramètres cinétiques pour les différents substrats exige des conditions d'essai enzymatique reproductibles d'une manipulation à l'autre. Les concentrations des solutions de substrat $\beta$-cétoester de CoA et de cofacteur (NADPH) sont donc déterminées avant utilisation. Le réactif à calibrer (par exemple $\beta$-cétoester de CoA) est ajouté à une concentration largement inférieure à celle du second substrat (NADPH). La réaction est déclenchée avec une concentration en enzyme suffisante pour obtenir l'utilisation rapide du substrat en concentration limitante. La différence de $DO_{340}$ observée permet de déduire la concentration réelle de ce substrat $\beta$-cétoester de CoA dans la réaction.

### M.4.1.2. Description du test enzymatique

L'activité catalytique de MabA purifiée a été démontrée par spectrophotométrie en présence d'acétoacétyl-CoA et de NADPH.

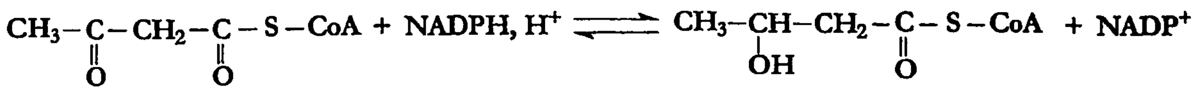

La cinétique de la réaction de β-cétoacyl réduction est suivie par la mesure de l'absorbance à 340 nm au cours du temps, qui décroît avec l'oxydation du NADPH. La réaction enzymatique est effectuée dans un volume final de 1 ml (en cuve de quartz, trajet optique 1 cm). Le spectrophotomètre (UVIKON 923, Bio-Tek Kontron Instruments) est relié à un bain thermostaté permettant de réguler la température de la cuve à 25°C. Une ligne de base est effectuée en absence d'enzyme. Le mélange réactionnel comprend 80 mM de tampon phosphate de sodium, et des concentrations variables en NADPH et β-cétoacyl-CoA. La réaction est déclenchée par l'addition de l'enzyme (36 nM à 144 nM). Les mesures sont effectuées sur 3 à 5 min.

Le $K_m$ pour le NADPH a été déterminé avec des concentrations en coenzyme variant de 5 à 200 μM et à concentration fixe (460 μM) en acétoacétyl-CoA. Les $K_m$ pour les β-cétoacyl-CoA ont été déterminés à concentration fixe, 100 μM, en NADPH. Une concentration supérieure à 100 μM entraînait un bruit de fond trop important au niveau des mesures. Il a été vérifié, en outre, que cette concentration était saturante.

Les $K_m$ et $V_{max}$ pour les β-cétoacyl-CoA ont été mesurés avec les concentrations suivantes: pour l'acétoacétyl-CoA ($C_4$), 100-8570 μM; pour le β-cétooctanoyl-CoA ($C_8$), 4-160 μM; pour le β-cétododécanoyl-CoA ($C_{12}$), 2-32 μM. Pour le β-cétohexadécanoyl-CoA et le β-cétoeicosanoyl-CoA ($C_{20}$), des problèmes d'inhibition par le substrat n'ont pu permettre de déterminer les paramètres cinétiques et la vitesse réactionnelle a été comparée à concentration fixe de 2 μM.

Au moins deux séries de points expérimentaux ont été réalisés pour chaque paramètre cinétique. La justesse de ces points a été vérifiée graphiquement par la représentation "en doubles inverses", 1/v = f(1/[S]) (équation (1)). Les paramètres cinétiques ont ensuite été déterminés graphiquement d'après la représentation de Hanes [S]/v = f([S]) (équation (2)) ou par calcul d'après la méthode des moindres-carrés, avec le logiciel GraphPad Prism. Version 2.01.

**Equation (1) (Lineweaver-Burk)**

$$\frac{1}{V} = \frac{K_m}{V_{max}} \cdot \frac{1}{S} + \frac{1}{V_{max}}$$

**Equation (2) (Hanes)**

$$\frac{S}{v} = \frac{S}{V\,max} + \frac{Km}{V\,max}$$

**III) MUTAGENESE DE LA PROTEINE MabA ET METHODES DE PURIFICATION OPTIMALES DE LA PROTEINE MabA, ET DES PROTEINES MabA, C(60)V, MabA S(144)L et MabA C(60)V / S(144)L**

1) mutagénèse de la protéine MabA

Le mutant MabA C(60)V a été obtenu par mutagénèse dirigée après avoir effectuée une PCR inverse. Les amorces nucléotidiques ont été choisises de manière à modifier le codon 60 du gène *mabA,* à savoir remplacement de TGT (cystéine) en GTT (valine). Le plasmide pET15b::*mabA* a été utilisé en tant que support pour l'amplification PCR par l'ADN polymérase PfuTurbo (Stratagene, USA).

Les produits PCR ont été digérés avec l'endonucléase *Dpn*1 afin de sélectionner les plasmides comportant le gène muté. Le gène muté a été entièrement séquencé afin de vérifier l'absence de mutation secondaire. Le plasmide portant le gène *mabA* C(60)V (pET15b::*mabA* C(60)V) a ensuite été utilisé pour transformer la souche surproductrice BL21(DE3).

L'obtention des mutants MabA C(60)V / S(144)L et MabA S(144)L a été effectuée selon la même méthode que précédemment.

2) purification des protéines MabA, MabA C(60)V, et MabA C(60)V / S(144)L

Quatre cultures de 50 ml en milieu LB + carbenicilline sont effectuées. La turbidité du milieu est mesurée par spectrophotométrie à 600 nm jusqu'à ce que la densité optique atteigne 0,8 (milieu de la phase exponentielle de croissance), c'est à dire après 4h d'incubation environ. L'expression du gène *mabA* est alors induite avec 0,8 mM d'IPTG pendant 2h à 37°C, puis vérifiée par SDS-PAGE.

Les cultures rassemblées (4 x 50 ml) sont collectées par centrifugation (15 min à 16000g, à 4°C) puis lavées. Le culot obtenu est repris dans 4 ml de tampon de lyse (voir ci-dessous). Avant de congeler la suspension à - 80°C (une nuit), un mélange d'inhibiteurs de protéases (0,113 mg/ml) et du lysozyme (0,5 mg/ml) y sont ajoutés. La suspension est décongelée, sous agitation douce, à température ambiante, puis traitée à la DNaseI (5 μg/ml) et RNaseA (10 μg/ml) en présence de $MgCl_2$ 10 mM pendant 15 min à 4°C, sous agitation douce. Les bactéries entières et les débris sont éliminés par centrifugation (15 min à 3000g, à 4°C). Une dernière ultracentrifugation à 44000g, 15 min à 4°C, permet d'éliminer tout matériel insoluble. Selon les cas, avant d'être chargé sur la colonne, le surnageant (lysat clarifié) peut être complémenté avec soit 10% (v/v) de glycérol (protéine pour études cinétiques, ou pour la cristallographie des protéines les moins stables), soit 400μM $NADP^+$ (étude cristallographique du complexe MabA-NADP).

Quatre ml de lysat bactérien clarifié (environ 30 mg de protéines totales) sont ajoutés à une colonne Ni-NTA agarose (500 μl, QIAGEN). Le matériel non fixé à la résine est récupéré par "lavages" avec du tampon à 5 mM puis 50 mM imidazole. La protéine MabA est éluée avec le tampon d'élution. Lorsque le tampon phosphate est utilisé, la protéine est récupérée directement dans 50% (v, v) glycérol final, pour éviter la précipitation. Pour la cristallographie, la protéine est concentrée à 10-15 mg/ml par ultrafiltration.

**Tampons utilisés:**

**Protéines pour études cinétiques:**

Tampon de lyse: phosphate de potassium 50 mM, pH 7,8 contenant 500 mM de NaCl, 5 mM d'imidazole

Tampons de lavage: phosphate de potassium 50 mM, pH 7,8 contenant 500 mM de NaCl, 5 et 50 mM d'imidazole

Tampon d'élution: phosphate de potassium 50 mM, pH 7,8 contenant 500 mM de NaCl, et 1.75 mM d'imidazole.

Protéines pour études de cristallographie:

Tampon de lyse: 50 mM tampon Tris, pH 8,0, supplémenté avec 300 mM $LiSO_4$ et 5 mM imidazole;
ou: 50 mM tampon Tris, pH 8,0, supplémenté avec 300 mM KCl et 5 mM imidazole.
Tampons de lavage : 50 mM tampon Tris, pH 8,0, supplémenté avec 300 mM $LiSO_4$ et 5 ou 50 mM imidazole;
ou: 50 mM tampon Tris, pH 8,0, supplémenté avec 300 mM KCl et 5 ou 50 mM imidazole.
Tampon d'élution: 20 mM MES buffer, pH6.4, $LiSO_4$ 300mM et 175-750 mM imidazole;
ou: 20 mM tampon PIPES, pH 8,0, supplémenté avec 300 mM KCl et 175-750 mM imidazole. Rmq: 1 mM DTT est ajouté à ces tampons dans le cas de la protéine sauvage.

(MES = acide 2-[N-morpholino]éthane sulfonique; PIPES = pipérazine-N,N'-bis[acide 2-éthane sulfonique])

3) séquences peptidiques des protéines obtenues et séquences nucléotidiques codant pour ces protéines

Séquence peptidique de la protéine MabA (FabG1) sauvage de *M. tuberculosis* H37Rv en fusion avec un tag poly-His (en gras):

**MGSSHHHHHH SSGLVPRGSH** MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

Séquence peptidique de la protéine MabA C60V (mutation en gras) en fusion avec un tag poly-His (en gras):

**MGSSHHHHHH SSGLVPRGSH** MTATATEGAK PPFVSRSVLV TGGNRGIGLA
IAQRLAADGH KVAVTHRGSG APKGLFGVE**V** DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

Séquence peptidique de la protéine MabA C60V/S144L (mutations en gras) en fusion avec un tag poly-His (en gras):

**MGSSHHHHHH SSGLVPRGSH** MTATATEGAK PPFVSRSVLV TGGNRGIGLA
IAQRLAADGH KVAVTHRGSG APKGLFGVE**V** DVTDSDAVDR AFTAVEEHQG
PVEVLVSNAG LSADAFLMRM TEEKFEKVIN ANLTGAFRVA QRASRSMQRN
KFGRMIFIGS VSG**L**WGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

Séquence nucléotidique du gène *mabA* (fabG1) sauvage de *M. tuberculosis* souche H37Rv, en fusion avec une séquence codant pour un tag poly-Histidine (en lettres capitales):

ATGGGCAGCAGCCATCATCATCATCATCACAGCAGCGGCCTGGTGCCGCGCGGCAGCCAT
atgactgccacagccactgaaggggccaaacccccattcgtatcccgttcagtcctggttaccggaggaaaccggggat
cgggctggcgatcgcacagcggctggctgccgacggccacaaggtggccgtcacccaccgtggatccggagcgccaaagg
ggctgtttggcgtcgaatgtgacgtcaccgacagcgacgccgtcgatcgcgccttcacggcggtagaagagcaccagggt
ccggtcgaggtgctggtgtccaacgccggcctatccgcggacgcattcctcatgcggatgaccgaggaaaagttcgagaa
ggtcatcaacgccaacctcaccggggcgttccgggtggctcaacgggcatcgcgcagcatgcagcgcaacaaattcggtc
gaatgatattcataggttcggtctccggcagctggggcatcggcaaccaggccaactacgcagcctccaaggccggagtg
attggcatggcccgctcgatcgcccgcgagctgtcgaaggcaaacgtgaccgcgaatgtggtggccccgggctacatcga
caccgatatgacccgcgcgctggatgagcggattcagcaggggggcgctgcaatttatcccagcgaagcgggtcggcaccc
ccgccgaggtcgccggggtggtcagcttcctggcttccgaggatgcgagctatatctccggtgcggtcatcccggtcgac
ggcggcatgggtatgggccac

Séquence nucléotidique du gène *mabA* (fabG1) C60V (codon muté en gras) de *M. tuberculosis* souche H37Rv, en fusion avec une séquence codant pour un tag poly-Histidine (en lettres capitales):

ATGGGCAGCAGCCATCATCATCATCATCACAGCAGCGGCCTGGTGCCGCGCGGCAGCCAT
atgactgccacagccactgaaggggccaaacccccattcgtatcccgttcagtcctggttaccggaggaaaccgggggat
cgggctggcgatcgcacagcggctggctgccgacggccacaaggtggccgtcacccaccgtggatccggagcgccaaagg
ggctgtttggcgtcgaa**GTT**gacgtcaccgacagcgacgccgtcgatcgcgccttcacggcggtagaagagcaccagggt
ccggtcgaggtgctggtgtccaacgccggcctatccgcggacgcattcctcatgcggatgaccgaggaaaagttcgagaa
ggtcatcaacgccaacctcaccggggcgttccgggtggctcaacgggcatcgcgcagcatgcagcgcaacaaattcggtc
gaatgatattcataggttcggtctccggcagctggggcatcggcaaccaggccaactacgcagcctccaaggccggagtg
attggcatggcccgctcgatcgcccgcgagctgtcgaaggcaaacgtgaccgcgaatgtggtggccccgggctacatcga
caccgatatgacccgcgcgctggatgagcggattcagcagggggcgctgcaatttatcccagcgaagcgggtcggcaccc
ccgccgaggtcgccggggtggtcagcttcctggcttccgaggatgcgagctatatctccggtgcggtcatcccggtcgac
ggcggcatgggtatgggccac

Séquence nucléotidique du gène *mabA* (fabG1) C60V/S144L (codons mutés en gras) de *M. tuberculosis* souche H37Rv, en fusion avec une séquence codant pour un tag poly-Histidine (en lettres capitales):

ATGGGCAGCAGCCATCATCATCATCATCACAGCAGCGGCCTGGTGCCGCGCGGCAGCCAT
atgactgccacagccactgaaggggccaaacccccattcgtatcccgttcagtcctggttaccggaggaaaccgggggat
cgggctggcgatcgcacagcggctggctgccgacggccacaaggtggccgtcacccaccgtggatccggagcgccaaagg
ggctgtttggcgtcgaa**GTT**gacgtcaccgacagcgacgccgtcgatcgcgccttcacggcggtagaagagcaccagggt
ccggtcgaggtgctggtgtccaacgccggcctatccgcggacgcattcctcatgcggatgaccgaggaaaagttcgagaa
ggtcatcaacgccaacctcaccggggcgttccgggtggctcaacgggcatcgcgcagcatgcagcgcaacaaattcggtc
gaatgatattcataggttcggtctccggc**CTC**tggggcatcggcaaccaggccaactacgcagcctccaaggccggagtg
attggcatggcccgctcgatcgcccgcgagctgtcgaaggcaaacgtgaccgcgaatgtggtggccccgggctacatcga
caccgatatgacccgcgcgctggatgagcggattcagcagggggcgctgcaatttatcccagcgaagcgggtcggcaccc
ccgccgaggtcgccggggtggtcagcttcctggcttccgaggatgcgagctatatctccggtgcggtcatcccggtcgac
ggcggcatgggtatgggccac

4) propriétés enzymatiques

Les mesures des cinétiques enzymatiques effectuées avec MabA sont les suivantes : acétoacétyl-CoA($C_4$ : $K_m$ = 1530 $\pm$ 81 $\mu$M, $k_{cat}$ = 1,9 $\pm$ 0,0 $s^{-1}$), le β-ketooctancyl-CoA ($C_8$ : $K_m$ = 70 $\pm$ 8 $\mu$M, $k_{cat}$ = 3,5 $\pm$ 0,0 $s^{-1}$), le β-ketododecanoyl-CoA ($C_{12}$ : $K_m$ = 8,3 $\pm$ 0,8 $\mu$M, $k_{cat}$ = 4,3 $\pm$ 0,2 $s^{-1}$).

5) étude cristallographique

Les coordonnées atomiques de la structure tridimensionnelle des cristaux de la protéine MabA sont représentées sur la figure 1, lesdits cristaux présentant en outre les caractéristiques suivantes :

- paramètre de mailles :
  - a = 81.403 angströms, b = 116.801 angströms, c = 52.324 angströms,
  - $\alpha = \beta$ = 90.00 °, $\gamma$ = 122.30 °,
- groupe d'espace : C2,
- diffraction maximale = 2,05 angströms.

Les coordonnées atomiques de la structure tridimensionnelle des cristaux de la protéine C(60)V sont représentées sur la figure 2, lesdits cristaux présentant en outre les caractéristiques suivantes :

- paramètre de mailles :
  - a = 82.230 angströms, b = 118.610 angströms, c = 53.170 angströms,
  - $\alpha = \beta$ = 90.00 °, $\gamma$ = 122.74 °,
- groupe d'espace : C2,
- diffraction maximale = 2,6 angströms.

Les coordonnées atomiques de la structure tridimensionnelle des cristaux de la protéine C(60)V / S(144)L sont représentées sur la figure 3, lesdits cristaux présentant en outre les caractéristiques suivantes :

- paramètre de mailles :
  - a = 81.072 angströms, b = 117.022 angströms, c = 53.170 angströms,
  - $\alpha = \beta = 90.00$ °, $\gamma = 122.42$ °,
- groupe d'espace : C2,
- diffraction maximale = 1,75 angströms.

***Références bibliographiques***

Altschul, S. F., Madden, T. L., Schäffer, A. A., Zhang, J., Zhang, Z., Miller, W. & Lipman, D. J. (1997). Gapped BLAST and PSI-BLAST: a new generation of protein database search programs. *Nucleic Acids Res.* 25, 3389-3402.

Banerjee, A., Dubnau, E., Quemard, A., Balasubramanian, V., Um, K. S., Wilson, T., Collins, D., de Lisle, G. & Jacobs, W. R. J. (1994). *inhA,* a gene encoding a target for isoniazid and ethionamide in *Mycobacterium tuberculosis. Science* 263, 227-30.

Barnes, P. F., Bloch, A. B., Davidson, P. T. & Snider, D. E. (1991). Tuberculosis in patients with immunodeficiency virus infection. *N. Engl. J. Med.* 234, 1644-50.

Basso, L. A., Zheng, R. & Blanchard, J. S. (1996). Kinetics of inactivation of WT and C243S mutant of *Mycobacterium tuberculosis* enoyl reductase by activated isoniazid. *J. Am. Chem. Soc.* 118,11301-11302.

Berman, H. M., Westbrook, J., Feng, Z., Gilliland, G., Bhat, T. N., Weissig, H., Shindyalov, I. N. & Bourne, P. E. (2000). The protein data bank. *Nucleic Acids Res.* 28, 235-242.

Birge, C. H. & Vagelos, P. R. (1972). Acyl carrier protein. Purification and properties of β-hydroxyacyl acyl carrier protein dehydrase. *J. Biol. chem.* 247, 4930-4938.

Caughey, I. & Kekwick, R. O. (1982). The Characteristics of Some Components of the Fatty Acid Synthetase System in the Plastids from the Mesocarp of Avocado (*Persea americana*) Fruit. *Eur. J. Biochem.* 123, 553-561.

Coakley, W. T., Brown, R. C. & James, C. J. (1973). The Inactivation of Enzymes by Ultrasonic Cavitation at 20 kHz. *Arch. Biochem. Biophys.* 159, 722-729.

Cohn, D. L., Bustreo, F. & Raviglione, M. C. (1997). Drug-resistant tuberculosis: review of the worldwide situation and the WHO/IUATLD global surveillance project. *Clinical Infectious Diseases. Clin Infect Dis* 24 (Suppl 1), S121-S130.

Cole, S. T., Brosch, R., Parkhill, J., Garnier, T., Churcher, C., Harris, D., Gordon, S. V., Eiglmeier, K., Gas, S., Barry, C. E., Tekaia, F., Badcock, K., Basham, D., Brown, D., Chillingworth, T., Connor, R., Davies, R., Devlin, K., Feltwell, T., Gentles, S., Hamlin, N., Holroyd, S., Homsby, T., Jagels, K. & Barrell, B. G. (1998). Deciphering the biology of *Mycobacterium tuberculosis* from the complete genome sequence. *Nature* 393, 537-44.

Constantinides, P. P. & Steim, J. M. (1985). Physical properties of fatty acyl-CoA. Critical micelle concentrations and micellar size and shape. *J Biol Chem* 260, 7573-7580.

Dolin, P. J., Raviglione, M. C. & Kochi, A. (1994). Global tuberculosis incidence and mortality during 1990-2000. *Bull World Health Organ* 72, 213-220.

Gill, S. C. & von Hippel, P. H. (1989). Calculation of protein extinction coefficients from amino acid sequence data [published erratum appears in Anal Biochem 1990 Sep;189(2):283]. *Anal Biochem* 182, 319-26.

Johnsson, K., King, D. S. & Shultz, P. G. (1995). Studies on the mechanism of action of isoniazid and ethionamide in the chemotherapy of tuberculosis. *J. Am. Chem. Soc.* 117, 5009-10.

Kikuchi, S., Takeuchi, T., Yasui, M., Kusaka, T. & Kolattukudi, P. E. (1989). A very long-chain fatty acid elongation system in *Mycobacterium avium* and a possible mode of action of isoniazid on the system. *Agric. Biol. Chem.* 53, 1689-98.

Labesse, G. & Momon, J. (1998). Incremental threading optimization (TITO) to help alignment and modelling of remote homologues. *Bioinformatics* 14, 206-211.

Labesse, G., Vidal-Cros, A., Chomilier, J., Gaudry, M. & Momon, J.-P. (1994). Structural comparisons lead to the definition of a new superfamily of NAD(P)(H)-accepting oxidoreductases : the single-domain reductases/epimerases/dehydrogenases (the RED family). *Biochem. J.* 304, 95-99.

Laemmli, U. K. (1970). Cleavage of structural proteins during the assembly of the head of bacteriophage T4. *Nature* 227, 680-685.

Luthy, R., Bowie, J. U. & Eisenberg, D. (1992). Assessment of protein models with three-dimensional profiles. *Nature* 356, 83-85.

Persson, B., Kpook, M. & Jômvall, H. (1991). Characteristics of short-chain alcohol dehydrogenases and related enzymes. *Eur. J. Biochem.* 200, 537-543.

Phillips, T. A., Van Bogelen, R. A. & Neidhardt, F. C. (1984). Ion gene product of *Escherichia coli* is a heat-shock protein. *J. Bacteriol.* 159, 283-287.

Quémard, A., Lacave, C. & Laneelle, G. (1991). Isoniazid inhibition of mycolic acid synthesis by cell extracts of sensitive and resistant strains of *Mycobacterium aurum. Antimicrob. Agents Chemother.* 35, 1035-9.

Quémard, A., Mazeres, S., Sut, A., Laneelle, G. & Lacave, C. (1995). Certain properties of isoniazid inhibition of mycolic acid synthesis in cell-free systems of *M. aurum* and *M. avium. Biochim Biophys Acta* 1254, 98-104.

Quémard, A., Sacchettini, J. C., Dessen, A., Vilcheze, C., Bittman, R., Jacobs, W. R. J. & Blanchard, J. S. (1995). Enzymatic characterization of the target for isoniazid in *Mycobacterium tuberculosis. Biochemistry* 34, 8235-41.

Rawlings, M. & Cronan, J. J. E. (1992). The gene encoding *Escherichia coli* acyl carrier protein lies within a cluster of fatty acid biosynthetic genes. *J. Biol. Chem.* 267, 5751-5754.

Rossmann, M. G., Moras, D. & Olsen, K. W. (1974). Chemical and biological evolution of nucleotide-binding protein. *Nature* 250, 194-199.

Rozwarski, D. A., Vilcheze, C., Sugantino, M., Bittman, R. & Sacchettini, J. C. (1999). Crystal structure of the *Mycobacterium tuberculosis* enoyl-ACP reductase, InhA, in complex with NAD+ and a C16 fatty acyl substrate. *J. Biol. Chem.* 274,15582-9.

Sali, A. & Blundell, T. L. (1993). Comparative protein modelling by satisfaction of spatial restraints. *J. Mol. Biol.* 234, 779-815.

Sambrook, J., Fritsch, E. F. & Maniatis, T. (1989). *Molecular cloning: a laboratory manual.* Cold Spring Harbor, NY: Cold Spring Harbor laboratory Press.

Schulz, H. & Wakil, S. J. (1971). Studies on the mechanism of fatty acid synthesis. XXV. On the mechanism of beta-ketoacylacyl carrier protein reductase from *Escherichia coli. J. Biol. Chem.* 246,1895-1901.

Sheldon, P. S., Kekwick, R. G. O., Sidebottom, C., Smith, C. G. & Slabas, A. R. (1990). 3-Oxoacyl-(acyl-carrier protein) reductase from avocado (*Persea americana*) fruit mesocarp. *Biochem. J.* 271, 713-720.

Sheldon, P. S., Kekwick, R. G. O., Smith, C. G., Sidebottom, C. & Slabas, A. R. (1992). 3-Oxoacyl-[ACP] reductase

from oilseed rape (*Brassica napus*). *Biochim. Biophys. Acta.* 1120, 151-159.

Shen, Z. & Byers, D. M. (1996). Isolation of *Vibrio harveyi* acyl carrier protein and the *fabG, acpP,* and *fabF* genes involved in fatty acid biosynthesis. *J. Bacteriol.* 178, 571-573.

Shimakata, T. & Stumpf, P. K. (1982). Purification and Characterizations of β-Ketoacyl-[Acyl-Carrier-Protein] Reductase, β-Hydroxyacyl-[Acyl-Carrier-Protein] Dehydrase, and Enoyl-[Acyl-Carrier-Protein] Reductase from *Spinacea oleracea* Leaves. *Arch. Biochem. Biophys.* 218, 77-91. Studier, F. W. & Moffatt, B. A. (1986). Use of bacteriophage T7 RNA polymerase to direct selective high-level cloned genes. *J. Mol. Biol.* 189, 113-130.

Studier, F. W., Rosenberg, A. H., Dunn, J. J. & Dubendorff, J. W. (1990). Use of T7 RNA polymerase to direct expression of cloned genes. *Meth. Enzymol.* 185, 60-89.

Takayama, K., Wang, L. & David, H. L. (1972). Effect of isoniazid on the *in vivo* mycolic acid synthesis, cell growth, and viability of *Mycobacterium tuberculosis. Antimicrob Agents Chemother* 2,29-35.

Weeks, G. & Wakil, S. J. (1968). Studies on the mechanism of fatty acid synthesis. 18. Preparation and general properties of the enoyl acyl carrier protein reductases from *Escherichia coli. J. Biol. Chem.* 243, 1180-1189.

Winder, F. G. & Collins, P. B. (1970). Inhibition by isoniazid of synthesis of mycolic acids in *Mycobacterium tuberculosis. J. Gen. Microbiol.* 63, 41-48.

## Revendications

1. Protéines dérivées de la protéine MabA **caractérisées en ce qu'**elles correspondent à la protéine MabA dont la séquence en acides aminés SEQ ID NO : 1 qui est la suivante :

   MTATATEGAK PPFVSRSVLV TGGNRGIGLA IAQRLAADGH KVAVTHRGSG
   APKGLFGVEC DVTDSDAVDR AFTAVEEHQG PVEVLVSNAG LSADAFLMRM
   TEEKFEKVIN ANLTGAFRVA QRASRSMQRN KFGRMIFIGS VSGSWGIGNQ
   ANYAASKAGV IGMARSIARE LSKANVTANV VAPGYIDTDM TRALDERIQQ
   GALQFIPAKR VGTPAEVAGV VSFLASEDAS YISGAVIPVD GGMGMGH

   est telle que :

   - la cystéine en position 60 est remplacée par un résidu valine, et/ou la glycine en position 139 est remplacée par une alanine ou une sérine, et/ou la sérine en position 144 est remplacée par un résidu leucine, et/ou
   - elle comporte les mutations N24D(ou E), et/ou H46D,
   - elle est modifiée par insertion du côté N-terminal d'une étiquette poly-histidine telle que la séquence SEQ ID NO : 14 suivante : MGSSHHHHHH SSGLVPRGSH,
   - elle présente une séquence GSH en N-terminal,
   - les sept premiers acides aminés sont supprimés.

2. Protéine dérivée de la protéine MabA selon la revendication 1, **caractérisée en ce qu'**elle correspond à la protéine MabA dans laquelle la cystéine en position 60 est remplacée par un résidu valine, ladite protéine dérivée, encore

désignée C(60)V, correspondant à la séquence SEQ ID NO 3 suivante :

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

3. Protéine dérivée de la protéine MabA selon l'une des revendications 1 ou 2, **caractérisée en ce qu'**elle correspond à la protéine MabA dans laquelle la sérine en position 144 est remplacée par un résidu leucine, ladite protéine dérivée, encore désignée S(144)L, correspondant à la séquence SEQ ID NO 5 suivante :

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ

ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

4. Protéine dérivée de la protéine MabA selon l'une des revendications 1 à 3, **caractérisée en ce qu'**elle correspond à la protéine MabA dans laquelle la cystéine en position 60 est remplacée par un résidu valine, et la sérine en position 144 est remplacée par un résidu leucine, ladite protéine dérivée, encore désignée C(60)V / S(144)L, correspondant à la séquence SEQ ID NO 7 suivante :

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

5. Protéine dérivée de la protéine MabA selon l'une des revendications 1 à 4, **caractérisée en ce qu'**elle correspond à la protéine MabA dans laquelle la cystéine en position 60 est remplacée par un résidu valine, la glycine en position 139 est remplacée par une alanine ou une sérine, et la sérine en position 144 est remplacée par un résidu leucine, ladite protéine dérivée, encore désignée C(60)V / G(139)[A ou S] / S(144)L, correspondant à la séquence SEQ ID NO 8 suivante :

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

dans laquelle X représente A ou S.

6. Protéines dérivées de la protéine MabA selon l'une des revendications 1 à 5, **caractérisées en ce qu'**elles sont modifiées de telle sorte qu'elles correspondent aux séquences suivantes :

- la séquence SEQ ID NO : 9, correspondant à la séquence SEQ ID NO : 1 comportant les mutations N24D(ou E), et/ou H46D, à savoir la séquence suivante :

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 9 ci-dessus, dans laquelle la cystéine en position 60 est remplacée par un résidu valine, et / ou la glycine en position 139 est remplacée par une alanine ou une sérine, et / ou la sérine en position 144 est remplacée par un résidu leucine,
- la séquence SEQ ID NO : 10, correspondant à la séquence SEQ ID NO : 3 comportant les mutations N24D (ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 11, correspondant à la séquence SEQ ID NO : 5 comportant les mutations N24D (ou E), et/ou H46D, à savoir la séquence suivante :

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 12, correspondant à la séquence SEQ ID NO : 7 comportant les mutations N24D (ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D,

- la séquence SEQ ID NO : 13, correspondant à la séquence SEQ ID NO : 8 comportant les mutations N24D (ou E), et/ou H46D, à savoir la séquence suivante :

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

dans laquelle $X_1$ représente D ou E, et $X_2$ représente H ou D.

**7.** Protéines dérivées de la protéine MabA selon l'une des revendications 1 à 6, **caractérisées en ce qu'**elles sont modifiées par insertion du côté N-terminal d'une étiquette poly-histidine telle que la séquence SEQ ID NO : 14 suivante : MGSSHHHHHH SSGLVPRGSH.

**8.** Protéines MabA modifiées selon la revendication 7, correspondant aux séquences suivantes:

- la séquence SEQ ID NO : 15, correspondant à la combinaison de la séquence SEQ ID NO : 14 et de la séquence SEQ ID NO : 1, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 16, correspondant à la combinaison de la séquence SEQ ID NO : 14 et de la séquence SEQ ID NO : 3, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 17, correspondant à la combinaison de la séquence SEQ ID NO : 14 et de la séquence SEQ ID NO : 5, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 18, correspondant à la combinaison de la séquence SEQ ID NO : 14 et de la séquence SEQ ID NO : 7, à savoir la séquence suivante :

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 19, correspondant à la combinaison de la séquence SEQ ID NO : 14 et de la séquence SEQ ID NO : 9, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEC DVTDSDAVDR AFTAVEEHQG
PVEVLVSNAG LSADAFLMRM TEEKFEKVIN ANLTGAFRVA QRASRSMQRN

KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,
- la séquence SEQ ID NO : 20, correspondant à la combinaison de la séquence SEQ ID NO : 14 et de la séquence SEQ ID NO : 10, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,
- la séquence SEQ ID NO : 21, correspondant à la combinaison de la séquence SEQ ID NO : 14 et de la séquence SEQ ID NO : 11, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,
- la séquence SEQ ID NO : 22, correspondant à la combinaison de la séquence SEQ ID NO : 14 et de la séquence SEQ ID NO : 12, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 23, correspondant à la combinaison de la séquence SEQ ID NO : 14 et de la séquence SEQ ID NO : 13, à savoir la séquence suivante :

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D.

9. Protéines dérivées de la protéine MabA selon l'une des revendications 1 à 6, présentant une séquence GSH en N-terminal, à savoir les séquences suivantes :

- la séquence SEQ ID NO : 24 suivante, correspondant à la combinaison de la séquence GSH et de la séquence SEQ ID NO : 1,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 25 suivante, correspondant à la combinaison de la séquence GSH et de la séquence SEQ ID NO : 3,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 26 suivante, correspondant à la combinaison de la séquence GSH et de la séquence SEQ ID NO : 5,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 27 suivante, correspondant à la combinaison de la séquence GSH et de la séquence SEQ ID NO : 7,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 28 suivante, correspondant à la combinaison de la séquence GSH et de la séquence SEQ ID NO : 9,

GSH MTATATEGAK PPFVSRSVLV TGGX$_1$RGIGLA
IAQRLAADGH KVAVTX$_2$RGSG APKGLFGVEC DVTDSDAVDR AFTAVEEHQG

PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 29 suivante, correspondant à la combinaison de la séquence GSH et de la séquence SEQ ID NO : 10,

GSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 30 suivante, correspondant à la combinaison de la séquence GSH et de la séquence SEQ ID NO : 11,

GSH MTATATEGAK PPFVSRSVLV TGGX1RGIGLA
IAQRLAADGH KVAVTX2RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 31 suivante, correspondant à la combinaison de la séquence GSH et de la séquence SEQ ID NO : 12,

GSH MTATATEGAK PPFVSRSVLV TGGX1RGIGLA
IAQRLAADGH KVAVTX2RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 32 suivante, correspondant à la combinaison de la séquence GSH et de la séquence SEQ ID NO : 13,

GSH MTATATEGAK PPFVSRSVLV TGGX1RGIGLA
IAQRLAADGH KVAVTX2RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS

YISGAVIPVD GGMGMGH

dans laquelle $X_1$ représente D ou E, et $X_2$ représente H ou D.

**10.** Protéines dérivées de la protéine MabA selon l'une des revendications 1 à 6, dans lesquelles les sept premiers acides aminés sont supprimés, à savoir les séquences suivantes :

- la séquence SEQ ID NO : 33 suivante, correspondant à la séquence SEQ ID NO : 1 dont les sept premiers acides aminés sont supprimés :

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 34 suivante, correspondant à la séquence SEQ ID NO : 3 dont les sept premiers acides aminés sont supprimés :

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 35 suivante, correspondant à la séquence SEQ ID NO : 5 dont les sept premiers acides aminés sont supprimés :

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 36 suivante, correspondant à la séquence SEQ ID NO : 7 dont les sept premiers acides aminés sont supprimés :

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- la séquence SEQ ID NO : 37 suivante, correspondant à la séquence SEQ ID NO : 9 dont les sept premiers acides aminés sont supprimés :

GAK PPFVSRSVLV TGGX$_1$RGIGLA

IAQRLAADGH KVAVTX$_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 38 suivante, correspondant à la séquence SEQ ID NO : 10 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 39 suivante, correspondant à la séquence SEQ ID NO : 11 dont les sept premiers acides aminés sont supprimés :

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 40 suivante, correspondant à la séquence SEQ ID NO : 12 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D,

- la séquence SEQ ID NO : 41 suivante, correspondant à la séquence SEQ ID NO : 13 dont les sept premiers acides aminés sont supprimés :

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV

VAPGYIDTDM TRALDERIQQ GALQFIPAKR VGTPAEVAGV VSFLASEDAS
YISGAVIPVD GGMGMGH

dans laquelle $X_1$ représente D ou E, et $X_2$ représente H ou D.

**11.** Protéines recombinantes dérivées sous forme purifiée selon l'une des la revendications 1 à 10, telles qu'obtenues par transformation de souches d'E.coli avec un plasmide contenant une séquence comprenant le gène codant pour une protéine dérivée de MabA telle que définie dans l'une des la revendications 1 à 10, suivie par une étape de purification au cours de laquelle :

- les bactéries E. coli recombinantes susmentionnées sont lavées dans un tampon de lavage, puis reprises

dans un tampon de lyse, et lysées par un cycle de congélation / décongélation en présence d'inhibiteurs de protéases et du lysozyme,
- après traitement par la DNAse I et la RNAse A, en présence de $MgCl_2$, et centrifugation, le surnageant de lyse des bactéries obtenu à l'étape précédente, auquel est additionné 10% (v/v) de glycérol, ou du $NADP^+$ 400 μM, est déposé sur une colonne de résine Ni-NTA agarose,
- après plusieurs lavages avec du tampon à 5 mM puis 50 mM imidazole, la protéine MabA, ou la protéine dérivée, est éluée avec le tampon d'élution.

**12.** Protéines recombinantes dérivées sous forme purifiée selon l'une des revendications 1 à 10, telles qu'obtenues selon le procédé de la revendication 11 dans lequel les différents tampons de lavage des bactéries, de lyse, de lavage, et d'élution, sont les suivants :

- tampon de lavage des bactéries : phosphate de potassium 10 mM, pH 7,8,
- tampon de lyse : phosphate de potassium 50 mM, pH 7,8 contenant 500 mM de NaCl, 5 mM d'imidazole,
- tampon de lavage : phosphate de potassium 50 mM, pH 7,8 contenant 500 mM de NaCl, 5 et 50 mM d'imidazole,
- tampon d'élution: phosphate de potassium 50 mM, pH 7,8 contenant 500 mM de NaCl, et 175 mM d'imidazole.

**13.** Protéines recombinantes dérivées sous forme purifiée selon l'une des revendications 1 à 10, telles qu'obtenues selon le procédé de la revendication 11 dans lequel les différents tampons de lavage des bactéries, de lyse, de lavage, et d'élution, sont les suivants :

- tampon de lavage des bactéries : Tris 10 mM, pH8,0,
- tampon de lyse :

  . 50 mM tampon Tris, pH 8,0, supplémenté avec 300 mM $LiSO_4$ et 5 mM imidazole;
  . ou 50 mM tampon Tris, pH 8,0, supplémenté avec 300 mM KCl et 5 mM imidazole,

- tampon de lavage :

  . 50 mM tampon Tris, pH 8,0, supplémenté avec 300 mM $LiSO_4$ et 5 ou 50 mM imidazole,
  . ou 50 mM tampon Tris, pH 8,0, supplémenté avec 300 mM KCl et 5 ou 50 mM imidazole.

- tampon d'élution:

  . 20 mM tampon MES, pH6.4, $LiSO_4$ 300mM et 175-750 mM imidazole;
  . ou 20 mM tampon PIPES, pH 8,0, supplémenté avec 300 mM KCl et 175-750 mM imidazole.

**14.** Protéines selon l'une des revendications à 13, **caractérisées par** leur activité enzymatique spécifique des substrats du type β-cétoacyl à longue chaîne, notament entre 8 à 20 atomes de carbone, tel que le β-cétooctanoyl-CoA, ou le β-cétododécanoyl-CoA.

**15.** Protéines selon l'une des revendications 1 à 14, dont les principales caractéristiques de leur structure tridimensionnelle à une résolution de 1,6-2,0 angströms, mises en évidence par l'analyse par diffraction aux rayons X des cristaux desdites protéines, sont telles que représentées sur la figure 1 pour la protéine MabA recombinante modifiée correspondant à la séquence SEQ ID NO : 15, sur la figure 2 pour la protéine dérivée MabA C(60)V correspondant à la séquence SEQ ID NO : 16, et sur la figure 3 pour la protéine dérivée MabA C(60)V / S(144)L correspondant à la séquence SEQ ID NO : 17.

**16.** Protéines selon l'une des revendications 1 à 15, sous forme cristallisée.

**17.** Cristaux de protéines selon la revendication 16, tels qu'obtenus par la méthode de diffusion de vapeur à goutte suspendue, en mélangeant lesdites protéines (1 μl d'une solution à 10 mg/ml) avec une solution de polyéthylène glycol, de CsCl (150-300 mM), et de glycérol (à 10%) dans un tampon (PIPES) à pH 6,2.

**18.** Cristaux de protéines selon la revendication 16 ou 17, tels qu'obtenus selon la méthode de cristallisation décrite dans la revendication 17, ladite méthode étant effectuée à partir des protéines purifiées selon la revendication 13.

**19.** Cristaux de la protéine MabA recombinante correspondant à la séquence SEQ ID NO : 15 selon l'une des revendications 16 à 18, dont les coordonnées atomiques de la structure tridimensionnelle sont représentés sur la figure 1, et présentant les caractéristiques suivantes :

- paramètre de mailles :

  . a = 81.403 angströms, b = 116.801 angströms, c = 52.324 angströms,
  . $\alpha = \beta = 90.00$ °, $\gamma = 122.30$ °,

- groupe d'espace : C2,
- diffraction maximale = 2,05 angströms.

**20.** Cristaux de la protéine C(60) V correspondant à la séquence SEQ ID NO : 16, selon l'une des revendications 16 à 18, dont les coordonnées atomiques de la structure tridimensionnelle sont représentés sur la figure 2, et présentant les caractéristiques suivantes :

- paramètre de mailles :

  . a = 82.230 angströms, b = 118.610 angströms, c = 53.170 angströms,
  . $\alpha = \beta = 90.00$ °, $\gamma = 122.74$ °,

- groupe d'espace : C2,
- diffraction maximale = 2,6 angströms.

**21.** Cristaux de la protéine C(60)V / S(144) L correspondant à la séquence SEQ ID NO : 18 selon l'une des revendications 16 à 18, dont les coordonnées atomiques de la structure tridimensionnelle sont représentés sur la figure 3, et présentant les caractéristiques suivantes

- paramètre de mailles :

  . a = 81.072 angströms, b = 117.022 angströms, c = 53.170 angströms,
  . $\alpha = \beta = 90.00$ °, $\gamma = 122.42$ °,

- groupe d'espace : C2,
- diffraction maximale = 1,75 angströms.

**22.** Cristaux de protéines selon l'une des revendications 17 à 21, dans lesquels lesdites protéines sont liées à un ligand, à savoir une molécule capable de se lier à la protéine MabA ou aux protéines dérivées de cette dernière, plus particulièrement au niveau de leur site actif principalement délimité par les acides aminés situés aux positions 21 à 28, 45 à 48, 60 à 63, 87 à 100, 112, 138 à 157, 183 à 212, et 240 à 247, des protéines décrites dans l'une des revendications 1 à 6, ou aux positions 41 à 48, 65 à 68, 80 à 83, 107 à 120, 132, 158 à 177, 203 à 232, et 260 à 267, des protéines décrites dans l'une des revendications 7 ou 8, ou aux positions 24 à 31, 48 à 51, 63 à 66, 90 à 103, 115, 141 à 160, 186 à 215, et 243 à 250, des protéines décrites dans la revendication 9, ou aux positions 14 à 11, 38 à 41, 53 à 56, 80 à 93, 105, 131 à 150, 176 à 205, et 233 à 240, des protéines décrites dans la revendication 10, lesdits cristaux étant tels qu'obtenus par trempage ou co-cristallisation d'une protéine recombinante dérivée de la protéine MabA selon l'une des revendications 1 à 16, en présence dudit ligand, notamment dans les conditions de cristallisation définies dans la revendication 17.

**23.** Séquence nucléotidique codant pour une protéine dérivée de la protéine MabA telle que définie dans l'une des revendications 1 à 16.

**24.** Séquence nucléotidique recombinante comprenant une séquence nucléotidique codant pour une protéine dérivée de la protéine MabA selon l'une des revendications 1 à 16, en association avec les éléments nécessaires à la transcription de cette séquence, notamment avec un promoteur et un terminateur de transcription.

**25.** Vecteur, notamment plasmide, contenant une séquence nucléotidique selon la revendication 24.

**26.** Cellules hôtes transformées par un vecteur selon la revendication 25, lesdites cellules étant choisies notamment parmi les bactéries telles que *E. coli,* ou tout autre micro-organisme utilisé pour la production de protéines.

**27.** Procédé de préparation de protéines recombinantes dérivées de la protéine MabA selon l'une des revendications 1 à 16, **caractérisé en ce qu'**il comprend les étapes suivantes :

- transformation de cellules à l'aide d'un vecteur recombinant selon la revendication 25,
- mise en culture des cellules ainsi transformées, et récupération desdites protéines produites par lesdites cellules,
- purification desdites protéines selon le procédé décrit dans les revendications 11 à 13.

**28.** Utilisation de protéines recombinantes dérivées de la protéine MabA selon l'une des revendications 1 à 16, ou de cristaux selon l'une des revendications 17 à 22, pour la mise en oeuvre de méthodes de criblage de ligands de la protéine MabA, et plus particulièrement de molécules susceptibles de se lier spécifiquement au niveau du site actif de la protéine MabA, ou de protéines de structure proche de la protéine MabA, et d'inhiber l'activité enzymatique de cette dernière, ces inhibiteurs étant choisis notamment parmi :

- les dérivés des stéroïdes,
- les dérivés de l'antibiotique antituberculeux isoniazide (hydrazide de l'acide isonicotinique), tels que les dérivés de l'adduit isonicotinoyl-NAD(P),
- les dérivés de la N-acétyl cystéamine ou d'autres types de dérivés simplifiés du coenzyme A, comprenant un fluorophore greffé permettant d'utiliser la méthode de la spectroscopie de fluorescence, en particulier résolue dans le temps, pour la détection d'interactions protéine-ligand,
- les dérivés inhibiteurs de la protéine InhA de *Mycobacterium tuberculosis.*

**29.** Utilisation selon la revendication 28, pour la mise en oeuvre de méthodes de criblage de ligands de la protéine MabA susceptibles d'être utilisés dans des compositions pharmaceutiques, notamment dans le cadre du traitement de pathologies liées à des infections mycobactériennes, telles que la tuberculose liée à l'infection par *Mycobacterium tuberculosis,* ou par *Mycobacterium africanium,* ou la lèpre liée à l'infection par *Mycobacterium leprae,* ou les mycobactérioses liées à l'infection par des mycobactéries opportunistes, telles que *Mycobacterium avium, Mycobacterium fortuitum, Mycobacterium kansasii, Mycobacterium chelonae.*

**30.** Méthode de criblage de ligands de la protéine MabA, **caractérisée en ce qu'**elle comprend les étapes suivantes :

- mise en présence d'une protéine recombinante dérivée de la protéine MabA selon l'une des revendications 1 à 16,
- détection de l'éventuelle liaison entre ladite protéine, et le ligand testé par mesure, après excitation de fluorescence, notamment à 300 nm, de l'intensité de fluorescence de ladite protéine émise entre 300 et 400 nm (correspondant essentiellement à l'émission de fluorescence de l'unique tryptophane W145), et comparaison de l'intensité de fluorescence émise dans un essai en absence de ligand, la fixation d'un ligand dans le site actif MabA étant **caractérisée par** un quenching de fluorescence.

**31.** Méthode de criblage de ligands inhibiteurs de la protéine MabA, **caractérisée en ce qu'**elle comprend les étapes suivantes :

- mise en présence d'une protéine recombinante dérivée de la protéine MabA selon l'une des revendications 1 à 16, dans un milieu réactionnel comprenant un substrat, tel qu'un dérivé β-cétoacyl défini dans la revendication 14, le coenzyme NADPH, et le ligand testé,
- détection d'un pouvoir inhibiteur potentiel du ligand testé, par mesure de l'activité enzymatique de ladite protéine par mesure cinétique de l'absorbance, notamment à 340 nm, et comparaison de la pente de la courbe de densité optique en fonction du temps avec la pente obtenue dans un essai en absence de ligand.

**32.** Méthode de criblage de ligands de la protéine MabA, **caractérisée en ce qu'**elle comprend les étapes suivantes :

- mise en présence d'une protéine recombinante dérivée de la protéine MabA selon l'une des revendications 1 à 16, avec le ligand testé,
- analyse de la structure tridimensionnelle du complexe formé en phase soluble entre ladite protéine et ledit ligand, notamment par RMN, et par fluorescence.

**33.** Méthode de criblage de ligands de la protéine MabA, **caractérisée en ce qu'**elle comprend les étapes suivantes :

- co-cristallisation du ligand testé et d'une protéine recombinante dérivée de la protéine MabA selon l'une des revendications 1 à 16, notamment dans les conditions de cristallisation définies dans la revendication 17, de manière à obtenir des cristaux selon la revendication 22,
- ou, trempage des cristaux d'une protéine recombinante dérivée selon l'une des revendications 17 à 22, dans des solutions optimisées contenant des ligands potentiels,
- analyse de la structure tridimensionnelle des cristaux susmentionnés, notamment par diffraction des rayons X (en vue de sélectionner les ligands ayant une capacité optimale d'occupation et de blocage du site actif desdites protéines).

## Claims

1. Proteins derived from the protein MabA, **characterized in that** they correspond to the protein MabA the amino acid sequence SEQ ID NO: 1 of which is the following:

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

in which:

- the cysteine in position 60 is replaced by a valine residue, and/or the glycine in position 139 is replaced by an alanine or a serine, and/or the serine in position 144 is replaced by a leucine residue, and/or
- it comprises the mutations N24D(or E), and/or H46D,
- it is modified by insertion on the N-terminal side of a polyhistidine tag such as the following sequence SEQ ID NO: 14: MGSSHHHHHH SSGLVPRGSH,
- it has an N-terminal GSH sequence,
- the first seven amino acids are deleted.

2. Protein derived from the protein MabA according to claim 1, **characterized in that** it corresponds to the protein MabA in which the cysteine in position 60 is replaced by a valine residue, said derived protein, also called C(60)V, corresponding to the following sequence SEQ ID NO 3:

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

3. Protein derived from the protein MabA according to one of claims 1 or 2, **characterized in that** it corresponds to the protein MabA in which the serine in position 144 is replaced by a leucine residue, said derived protein, also called S(144)L, corresponding to the following sequence SEQ ID NO 5:

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
```

TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

**4.** Protein derived from the protein MabA according to one of claims 1 to 3, **characterized in that** it corresponds to the protein MabA in which the cysteine in position 60 is replaced by a valine residue, and the serine in position 144 is replaced by a leucine residue, said derived protein, also called C(60)V / S(144)L, corresponding to the following sequence SEQ ID NO 7:

MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

**5.** Protein derived from the protein MabA according to one of claims 1 to 4, **characterized in that** it corresponds to the protein MabA in which the cysteine in position 60 is replaced by a valine residue, the glycine in position 139 is replaced by an alanine or a serine, and the serine in position 144 is replaced by a leucine residue, said derived protein, also called C(60)V / G(139)[A or S] / S(144)L, corresponding to the following sequence SEQ ID NO 8:

MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which X represents A or S.

**6.** Proteins derived from the protein MabA according to any one of claims 1 to 5, **characterized in that** they are modified as such that they correspond to the following sequences:

- the sequence SEQ ID NO: 9, corresponding to the sequence SEQ ID NO: 1 comprising the mutations N24D (or E), and/or H46D, namely the following sequence:

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which $X_1$ represents D or E, and $X_2$ represents H or D,
- the above sequence SEQ ID NO: 9, in which the cysteine in position 60 is replaced by a valine residue, and/or the glycine in position 139 is replaced by an alanine or a serine, and/or the serine in position 144 is replaced by a leucine residue,
- the sequence SEQ ID NO: 10, corresponding to the sequence SEQ ID NO: 3 comprising the mutations N24D (or E), and/or H46D, namely the following sequence:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which $X_1$ represents D or E, and $X_2$ represents H or D,
- the sequence SEQ ID NO: 11, corresponding to the sequence SEQ ID NO: 5 comprising the mutations N24D (or E), and/or H46D, namely the following sequence:

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which $X_1$ represents D or E, and $X_2$ represents H or D,
- the sequence SEQ ID NO: 12, corresponding to the sequence SEQ ID NO: 7 comprising the mutations N24D (or E), and/or H46D, namely the following sequence:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which $X_1$ represents D or E, and $X_2$ represents H or D,
- the sequence SEQ ID NO: 13, corresponding to the sequence SEQ ID NO: 8 comprising the mutations N24D (or E), and/or H46D, namely the following sequence:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

in which $X_1$ represents D or E, and $X_2$ represents H or D.

**7.** Proteins derived from the protein MabA according to one of claims 1 to 6, **characterized in that** they are modified by insertion, on the N-terminal side, of a poly-histidine tag such as the following sequence SEQ ID NO: 14: MGSSHHHHHH SSGLVPRGSH.

**8.** Modified proteins MabA according to claim 7, corresponding to the following sequences:

- the sequence SEQ ID NO: 15, corresponding to the combination of the sequence SEQ ID NO: 14 and the sequence SEQ ID NO: 1, namely the following sequence:

MGSSHHHHHH SSGLVPRGSH MTATATEGAK PPFVSRSVLV TGGNRGIGLA IAQRLAADGH KVAVTHRGSG APKGLFGVEC DVTDSDAVDR AFTAVEEHQG PVEVLVSNAG LSADAFLMRM TEEKFEKVIN ANLTGAFRVA QRASRSMQRN KFGRMIFIGS VSGSWGIGNQ ANYAASKAGV IGMARSIARE LSKANVTANV VAPGYIDTDM TRALDERIQQ GALQFIPAKR VGTPAEVAGV VSFLASEDAS YISGAVIPVD GGMGMGH

- the sequence SEQ ID NO: 16, corresponding to the combination of the sequence SEQ ID NO: 14 and the sequence SEQ ID NO: 3, namely the following sequence:

MGSSHHHHHH SSGLVPRGSH MTATATEGAK PPFVSRSVLV TGGNRGIGLA IAQRLAADGH KVAVTHRGSG APKGLFGVEV DVTDSDAVDR AFTAVEEHQG PVEVLVSNAG LSADAFLMRM TEEKFEKVIN ANLTGAFRVA QRASRSMQRN KFGRMIFIGS VSGSWGIGNQ ANYAASKAGV IGMARSIARE LSKANVTANV VAPGYIDTDM TRALDERIQQ GALQFIPAKR VGTPAEVAGV VSFLASEDAS YISGAVIPVD GGMGMGH

- the sequence SEQ ID NO: 17, corresponding to the combination of the sequence SEQ ID NO: 14 and the sequence SEQ ID NO: 5, namely the following sequence:

MGSSHHHHHH SSGLVPRGSH MTATATEGAK PPFVSRSVLV TGGNRGIGLA IAQRLAADGH KVAVTHRGSG APKGLFGVEC DVTDSDAVDR AFTAVEEHQG PVEVLVSNAG LSADAFLMRM TEEKFEKVIN ANLTGAFRVA QRASRSMQRN KFGRMIFIGS VSGLWGIGNQ ANYAASKAGV IGMARSIARE LSKANVTANV VAPGYIDTDM TRALDERIQQ GALQFIPAKR VGTPAEVAGV VSFLASEDAS YISGAVIPVD GGMGMGH

- the sequence SEQ ID NO: 18, corresponding to the combination of the sequence SEQ ID NO: 14 and the sequence SEQ ID NO: 7, namely the following sequence:

MGSSHHHHHH SSGLVPRGSH MTATATEGAK PPFVSRSVLV TGGNRGIGLA IAQRLAADGH KVAVTHRGSG APKGLFGVEV DVTDSDAVDR AFTAVEEHQG PVEVLVSNAG LSADAFLMRM TEEKFEKVIN ANLTGAFRVA QRASRSMQRN KFGRMIFIGS VSGLWGIGNQ ANYAASKAGV IGMARSIARE LSKANVTANV

VAPGYIDTDM TRALDERIQQ GALQFIPAKR VGTPAEVAGV VSFLASEDAS YISGAVIPVD GGMGMGH

- the sequence SEQ ID NO: 19, corresponding to the combination of the sequence SEQ ID NO: 14 and the sequence SEQ ID NO: 9, namely the following sequence:

MGSSHHHHHH SSGLVPRGSH MTATATEGAK PPFVSRSVLV TGG$X_1$RGIGLA IAQRLAADGH KVAVT$X_2$RGSG APKGLFGVEC DVTDSDAVDR AFTAVEEHQG PVEVLVSNAG LSADAFLMRM TEEKFEKVIN ANLTGAFRVA QRASRSMQRN KFGRMIFIGS VSGSWGIGNQ ANYAASKAGV IGMARSIARE LSKANVTANV VAPGYIDTDM TRALDERIQQ GALQFIPAKR VGTPAEVAGV VSFLASEDAS YISGAVIPVD GGMGMGH

in which $X_1$ represents D or E, and $X_2$ represents H or D,
- the sequence SEQ ID NO: 20, corresponding to the combination of the sequence SEQ ID NO: 14 and the sequence SEQ ID NO: 10, namely the following sequence:

MGSSHHHHHH SSGLVPRGSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the sequence SEQ ID NO: 21, corresponding to the combination of the sequence SEQ ID NO: 14 and the sequence SEQ ID NO: 11, namely the following sequence:

MGSSHHHHHH SSGLVPRGSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the sequence SEQ ID NO: 22, corresponding to the combination of the sequence SEQ ID NO: 14 and the sequence SEQ ID NO: 12, namely the following sequence:

MGSSHHHHHH SSGLVPRGSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the sequence SEQ ID NO: 23, corresponding to the combination of the sequence SEQ ID NO: 14 and the sequence SEQ ID NO: 13, namely the following sequence:

MGSSHHHHHH SSGLVPRGSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D.

**9.** Proteins derived from the protein MabA according to one of claims 1 to 6, having an N-terminal GSH sequence, namely the following sequences:

- the following sequence SEQ ID NO: 24, corresponding to the combination of the GSH sequence and the

sequence SEQ ID NO: 1,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 25, corresponding to the combination of the GSH sequence and the sequence SEQ ID NO: 3,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 26, corresponding to the combination of the GSH sequence and the sequence SEQ ID NO: 5,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 27, corresponding to the combination of the GSH sequence and the sequence SEQ ID NO: 7,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 28, corresponding to the combination of the GSH sequence and the sequence SEQ ID NO: 9,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the following sequence SEQ ID NO: 29, corresponding to the combination of the GSH sequence and the sequence SEQ ID NO: 10,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the following sequence SEQ ID NO: 30, corresponding to the combination of the GSH sequence and the sequence SEQ ID NO: 11,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the following sequence SEQ ID NO: 31, corresponding to the combination of the GSH sequence and the sequence SEQ ID NO:12,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV

VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,
- the following sequence SEQ ID NO: 32, corresponding to the combination of the GSH sequence and the sequence SEQ ID NO: 13,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D.

**10.** Proteins derived from the protein MabA according to one of claims 1 to 6, in which the first seven amino acids are deleted, namely the following sequences:

- the following sequence SEQ ID NO: 33, corresponding to the sequence SEQ ID NO: 1 the first seven amino acids of which are deleted:

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 34, corresponding to the sequence SEQ ID NO: 3 the first seven amino acids of which are deleted:

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 35, corresponding to the sequence SEQ ID NO: 5 the first seven amino acids of which are deleted:

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 36, corresponding to the sequence SEQ ID NO: 7 the first seven amino acids of which are deleted:

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- the following sequence SEQ ID NO: 37, corresponding to the sequence SEQ ID NO: 9 the first seven amino acids of which are deleted:

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,

- the following sequence SEQ ID NO: 38, corresponding to the sequence SEQ ID NO: 10 the first seven amino acids of which are deleted:

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,

- the following sequence SEQ ID NO: 39, corresponding to the sequence SEQ ID NO: 11 the first seven amino acids of which are deleted:

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,

- the following sequence SEQ ID NO: 40, corresponding to the sequence SEQ ID NO: 12 the first seven amino acids of which are deleted:

GAK PPFVSRSVLV TGG$X_1$RGIGLA

IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D,

- the following sequence SEQ ID NO: 41, corresponding to the sequence SEQ ID NO: 13 the first seven amino acids of which are deleted:

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

in which $X_1$ represents D or E, and $X_2$ represents H or D.

**11.** Derived recombinant proteins in purified form according to any one of claims 1 to 10, as obtained by transformation of strains of *E.coli* with a plasmid containing a sequence comprising the gene coding for a protein derived from MabA as defined in any one of claims 1 to 10, followed by a purification stage during which:

- the abovementioned recombinant *E. coli* bacteria are washed in a washing buffer, then taken up in a lysis buffer, and lysed by a freeze/thaw cycle in the presence of protease inhibitors and lysozyme,
- after treatment by DNAse I and RNAse A, in the presence of $MgCl_2$, and centrifugation, the lysis supernatant of the bacteria obtained in the preceding stage, to which 10% (v/v) of glycerol, or 400 $\mu$M of $NADP^+$ is added, is applied to an Ni-NTA agarose resin column,
- after several washings with 5 mM buffer then 50 mM imidazole, the protein MabA, or the derived protein, is eluted with the elution buffer.

**12.** Derived recombinant proteins in purified form according to one of claims 1 to 10, as obtained according to the process of claim 11 in which the different bacteria washing, lysis, washing, and elution buffers, are the following:

- bacteria washing buffer: 10 mM potassium phosphate, pH 7.8,
- lysis buffer: 50 mM potassium phosphate, pH 7.8 containing 500 mM of NaCl, 5 mM of imidazole,
- washing buffer: 50 mM potassium phosphate, pH 7.8 containing 500 mM of NaCl, 5 and 50 mM of imidazole,
- elution buffer: 50 mM potassium phosphate, pH 7.8 containing 500 mM of NaCl, and 175 mM of imidazole.

**13.** Derived recombinant proteins in purified form according to any one of claims 1 to 10, as obtained according to the process of claim 11 in which the different bacteria washing, lysis, washing, and elution buffers, are the following:

- bacteria washing buffer: Tris 10 mM, pH 8.0,
- lysis buffer:

  . 50 mM Tris buffer, pH 8.0, supplemented with 300 mM $LiSO_4$ and 5 mM imidazole;
  . or 50 mM Tris buffer, pH 8.0, supplemented with 300 mM KCl and 5 mM imidazole,

- washing buffer:

  . 50 mM Tris buffer, pH 8.0, supplemented with 300 mM $LiSO_4$ and 5 or 50 mM imidazole,
  . or 50 mM Tris buffer, pH 8.0, supplemented with 300 mM KCl and 5 or 50 mM imidazole.

- elution buffer:

. 20 mM MES buffer, pH 6.4, LiSO4 300 mM and 175-750 mM imidazole;
. or 20 mM PIPES buffer, pH 8.0, supplemented with 300 mM KCl and 175-750 mM imidazole;

**14.** Proteins according to one of claims 1 to 13, **characterized by** their specific enzymatic activity of the substrates of the long-chain type β-ketoacyl, in particular between 8 and 20 carbon atoms, such as β-ketooctanoyl-CoA, or β-ketododecanoyl-CoA.

**15.** Proteins according to one of claims 1 to 14, the main characteristics of the three-dimensional structure of which, at a resolution of 1.6-2.0 angströms, detected by X-ray diffraction analysis of the crystals of said proteins, are as represented in Figure 1 for the recombinant protein MabA corresponding to the sequence SEQ ID NO: 15, in Figure 2 for the derived protein MabA C(60)V corresponding to the sequence SEQ ID NO: 16, and in Figure 3 for the derived protein MabA C(60)V / S(144)L corresponding to the sequence SEQ ID NO: 17.

**16.** Proteins according to one of claims 1 to 15, in crystallized form.

**17.** Crystals of proteins according to claim 16, as obtained by the hanging-drop vapour diffusion method, by mixing said proteins (1 μl of a 10 mg/ml solution) with a solution of polyethylene glycol, CsCl (150-300 mM), and glycerol (10%) in a buffer (PIPES) at pH 6.2.

**18.** Crystals of proteins according to claim 16 or 17, as obtained according to the crystallization method described in claim 17, said method being carried out from the purified proteins according to claim 13.

**19.** Crystals of the recombinant protein MabA corresponding to the sequence SEQ ID NO: 15 according to one of claims 16 to 18, the atomic coordinates of the three-dimensional structure of which are represented in Figure 1, and having the following characteristics:

- cell parameters:

  . a = 81.403 angströms, b = 116.801 angströms, c = 52.324 angströms,
  . $\alpha = \beta = 90.00$ °, $\gamma = 122.30$ °,

- space group: C2,
- maximum diffraction = 2.05 angströms.

**20.** Crystals of the protein C(60) V corresponding to the sequence SEQ ID NO: 16, according to one of claims 16 to 18, the atomic coordinates of the three-dimensional structure of which are represented in Figure 2, and having the following characteristics:

- cell parameters:

  . a = 82.230 angströms, b = 118.610 angströms, c = 53.170 angströms,
  . $\alpha = \beta = 90.00$ °, $\gamma = 122.74$ °,

- space group: C2,
- maximum diffraction = 2.6 angströms.

**21.** Crystals of the protein C(60)V / S(144) L corresponding to the sequence SEQ ID NO: 18 according to one of claims 16 to 18, the atomic coordinates of the three-dimensional structure of which are represented in Figure 3, and having the following characteristics:

- cell parameters:

  . a = 81.072 angströms, b = 117.022 angströms, c = 53.170 angströms,
  . $\alpha = \beta = 90.00$ °, $\gamma = 122.42$ °,

- space group: C2,
- maximum diffraction = 1.75 angströms.

**22.** Crystals of proteins according to one of claims 17 to 21, in which said proteins are bound to a ligand, namely a molecule capable of binding to the protein MabA or to the proteins derived from the latter, more particularly at the level of their active site mainly delimited by the amino acids situated in positions 21 to 28, 45 to 48, 60 to 63, 87 to 100, 112, 138 to 157, 183 to 212, and 240 to 247, of the proteins described in one of claims 1 to 6, or in positions 41 to 48, 65 to 68, 80 to 83, 107 to 120, 132, 158 to 177, 203 to 232, and 260 to 267, of the proteins described in one of claims 7 or 8, or in positions 24 to 31, 48 to 51, 63 to 66, 90 to 103, 115, 141 to 160, 186 to 215, and 243 to 250, of the proteins described in claim 9, or in positions 14 to 11, 38 to 41, 53 to 56, 80 to 93, 105, 131 to 150, 176 to 205, and 233 to 240, of the proteins described in claim 10, said crystals being as obtained by soaking or co-crystallization of the recombinant protein MabA according to one of claims 1 to 16, in the presence of said ligand, in particular under the crystallization conditions defined in claim 17.

**23.** Nucleotide sequence coding for a protein derived from the protein MabA as defined in one of claims 1 to 16.

**24.** Recombinant nucleotide sequence comprising the nucleotide sequence coding for a protein derived from the protein MabA according to one of claims 1 to 16, in combination with the elements necessary for the transcription of this sequence, in particular with a transcription promoter and terminator.

**25.** Vector, in particular plasmid, containing a nucleotide sequence according to claim 24.

**26.** Host cells transformed by a vector according to claim 25, said cells being chosen in particular from the bacteria such as *E. coli,* or any other microorganism used for the production of proteins.

**27.** Process for the preparation of the recombinant proteins derived from the protein MabA according to one of claims 1 to 16, **characterized in that** it comprises the following stages:

- transformation of cells using a recombinant vector according to claim 25,
- culture of the cells thus transformed, and recovery of said proteins produced by said cells,
- purification of said proteins according to the process described in claims 11 to 13.

**28.** Use of the recombinant protein MabA in purified form, or of recombinant proteins derived from the protein MabA according to one of claims 1 to 16, or of crystals according to one of claims 17 to 22, for the implementation of methods for designing or screening ligands of the protein MabA, and more particularly molecules capable of binding specifically at the level of the active site of the protein MabA, or proteins similar in structure to the protein MabA, and inhibiting the enzymatic activity of the latter, these inhibitors being chosen in particular from:

- the steroid derivatives,
- the derivatives of the antituberculous antibiotic isoniazid (isonicotinic acid hydrazide), such as the derivatives of the isonicotinoyl-NAD(P) adduct,
- the derivatives of N-acetyl cysteamine or other simplified types of derivatives of the coenzyme A, comprising a grafted fluorophore making it possible to use the fluorescence spectroscopy method, in particular time-resolved, for the detection of protein-ligand interactions,
- the inhibiting derivatives of the protein InhA of *Mycobacterium tuberculosis.*

**29.** Use according to claim 28, for the implementation of methods for screening ligands of the protein MabA capable of being used in pharmaceutical compositions, in particular within the framework of the treatment of pathologies linked to mycobacterial infections, such as tuberculosis linked to infection by *Mycobacterium tuberculosis,* or by *Mycobacterium africanium,* or leprosy linked to infection by *Mycobacterium leprae,* or mycobacteriosis linked to infection by opportunist mycobacteria, such as *Mycobacterium avium, Mycobacterium fortuitum, Mycobacterium kansasii*, *Mycobacterium chelonae.*

**30.** Method for screening ligands of the protein MabA, **characterized in that** it comprises the following stages:

- being brought into the presence of a recombinant protein derived from the protein MabA according to one of claims 1 to 16,
- detection of any bond between said protein and the ligand tested by measurement, after fluorescence excitation, in particular at 300 run, of the intensity of fluorescence of said protein emitted between 300 and 400 nm (corresponding essentially to the emission of fluorescence of the single tryptophan W145), and comparison of the intensity of fluorescence emitted in a test in the absence of ligand, the binding of a ligand in the MabA active

site being **characterized by** a quenching of fluorescence.

**31.** Method for screening ligands inhibiting the protein MabA, **characterized in that** it comprises the following stages:

- being brought into the presence of a recombinant protein derived from the protein MabA according to one of claims 1 to 16, in a reaction medium comprising a substrate, such as a β-ketoacyl derivative defined in claim 14, the coenzyme NADPH and the ligand tested,
- detection of a potential inhibiting ability of the ligand tested, by measurement of the enzymatic activity of said protein by kinetic measurement of the absorbance, in particular at 340 nm, and comparison of the gradient of the optical density curve as a function of time with the gradient obtained in a test in the absence of ligand.

**32.** Method for screening ligands of the protein MabA, **characterized in that** it comprises the following stages:

- being brought into the presence of a recombinant protein derived from the protein MabA according to one of claims 1 to 16, with the ligand tested,
- analysis of the three-dimensional structure of the complex formed in soluble phase between said protein and said ligand, in particular by NMR, and by fluorescence.

**33.** Method for screening ligands of the protein MabA, **characterized in that** it comprises the following stages:

- co-crystallization of the ligand tested and a recombinant protein derived from the protein MabA according to one of claims 1 to 16, in particular under the crystallization conditions defined in claim 17, in order to obtain the crystals according to claim 22,
- or soaking of the crystals of the protein MabA or of a derived recombinant protein according to one of claims 17 to 22, in optimized solutions containing potential ligands,
- analysis of the three-dimensional structure of the abovementioned crystals, in particular by X-ray diffraction (with a view to selecting the ligands having an optimum ability to occupy and block the active site of said proteins).

## Patentansprüche

**1.** Proteine, die vom MabA-Protein abgeleitet sind, **gekennzeichnet dadurch, dass** diese dem MabA-Protein entsprechen, dessen Aminosäurensequenz SEQ ID NO: 1 die Folgende ist:

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

derart, dass:

- das Cystein an Position 60 durch einen Valinrest ersetzt wird und / oder das Glycin an Position 139 durch Alanin oder Serin ersetzt wird und / oder das Serin an Position 144 durch einen Leucinrest ersetzt wird, und / oder
- sie die Mutationen N24D (oder E) und / oder H46D umfasst,
- sie durch die Einfügung eines Polyhistidin-Tags an der N-Terminal-Seite modifiziert ist, sowie auch die folgende Sequenz SEQ ID NO: 14: MGSSHHHHHH SSGLVPRGSH,
- diese eine GSH-Sequenz am N-Terminal aufweist,
- die sieben ersten Aminosäuren unterdrückt sind.

**2.** Protein, das vom MabA-Protein abgeleitet ist, nach Anspruch 1, **gekennzeichnet dadurch, dass** dieses dem MabA-Protein entspricht, bei dem das Cystein an Position 60 durch einen Valinrest ersetzt wird, wobei besagtes abgeleitetes Protein, das wiederum mit C(60)V bezeichnet wird, der folgenden Sequenz SEQ ID NO: 3 entspricht:

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

3. Protein, das vom MabA-Protein abgeleitet ist, nach Anspruch 1 oder 2, **gekennzeichnet dadurch, dass** dieses dem MabA-Protein entspricht, bei dem das Serin an Position 144 durch einen Leucinrest substituiert wird, wobei besagtes abgeleitetes Protein, das wiederum mit S(144)L bezeichnet wird, der folgenden Sequenz SEQ ID NO: 5 entspricht:

```
MTATATEGAK PPFVSRSVLV TGGNRGIGLA IAQRLAADGH KVAVTHRGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

4. Protein, das vom MabA-Protein abgeleitet ist, nach einem der Ansprüche 1 bis 3, **gekennzeichnet dadurch, dass** dieses dem MabA-Protein entspricht, bei dem das Cystein an Position 60 durch einen Valinrest ersetzt wird und das Serin an Position 144 durch einen Leucinrest, wobei besagtes abgeleitetes Protein, das wiederum mit C(60)V / S(144)L bezeichnet wird, der folgenden Sequenz SEQ ID NO: 7 entspricht:

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

5. Protein, das vom MabA-Protein abgeleitet ist, nach einem der Ansprüche 1 bis 4, **gekennzeichnet dadurch, dass** dieses dem MabA-Protein entspricht, bei dem das Cystein an Position 60 durch einen Valinrest ersetzt wird, das Glycin an Position 139 durch Alanin oder Serin ersetzt wird und das Serin an Position 144 durch einen Leucinrest ersetzt wird, wobei besagtes abgeleitetes Protein, das wiederum mit C(60)V / G(139)[A oder S] / S(144)L bezeichnet wird, der folgenden Sequenz SEQ ID NO: 8 entspricht:

```
MTATATEGAK PPFVSRSVLV TGGNRGIGLA IAQRLAADGH KVAVTHRGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH
```

bei der X das A oder das S darstellt.

6. Proteine, die vom MabA-Protein abgeleitet sind, nach einem der Ansprüche 1 bis 5, **gekennzeichnet dadurch, dass** diese auf eine Weise modifiziert sind, dass sie den folgenden Sequenzen entsprechen:

- Sequenz SEQ ID NO: 9, die der Sequenz SEQ ID NO: 1 entspricht, die die Mutationen N24D (oder E) und / oder H46D aufweist, das heißt die folgende Sequenz:

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

bei der $X_1$ das D oder E und $X_2$ das H oder D darstellt.

- oben genannte Sequenz SEQ ID NO: 9, bei der das Cystein an Position 60 durch einen Valinrest ersetzt wird und oder das Glycin an Position 139 durch Alanin oder Serin ersetzt wird und / oder das Serin an Position 144 durch einen Leucinrest ersetzt wird,
- Sequenz SEQ ID NO: 10, die der Sequenz SEQ ID NO: 3 entspricht, die die Mutationen N24D (oder E) und / oder H46D aufweist, das heißt die folgende Sequenz:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGSWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

bei der $X_1$ das D oder E und $X_2$ das H oder D darstellt.

- Sequenz SEQ ID NO: 11, die der Sequenz SEQ ID NO: 5 entspricht, die die Mutationen N24D (oder E), und / oder H46D aufweist, das heißt die folgende Sequenz:

MTATATEGAK PPFVSRSVLV TGG$X_1$RGIGLA IAQRLAADGH KVAVT$X_2$RGSG
APKGLFGVEC DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

bei der $X_1$ das D oder E und $X_2$ das H oder D darstellt.

- Sequenz SEQ ID NO: 12, die der Sequenz SEQ ID NO: 7 entspricht, die die Mutationen N24D (oder E) und / oder H46D aufweist, das heißt die folgende Sequenz:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIGS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

bei der $X_1$ das D oder E und $X_2$ das H oder D darstellt.

- Sequenz SEQ ID NO: 13, die der Sequenz SEQ ID NO: 8 entspricht, wobei diese die Mutationen N24D (oder E) und / oder H46D aufweist, das heißt die folgende Sequenz:

MTATATEGAK PPFVSRSVLV TGG$X_1$RGIGLA IAQRLAADGH KVAVT$X_2$RGSG
APKGLFGVEV DVTDSDAVDR AFTAVEEHQG PVEVLVSNAG LSADAFLMRM
TEEKFEKVIN ANLTGAFRVA QRASRSMQRN KFGRMIFIXS VSGLWGIGNQ
ANYAASKAGV IGMARSIARE LSKANVTANV VAPGYIDTDM TRALDERIQQ
GALQFIPAKR VGTPAEVAGV VSFLASEDAS YISGAVIPVD GGMGMGH

bei der $X_1$ das D oder E und $X_2$ das H oder D darstellt.

7. Proteine, die vom MabA-Protein abgeleitet sind, nach einem der Ansprüche 1 bis 6, **gekennzeichnet dadurch, dass** diese durch Einfügung eines Polyhistidin-Tags an der N-Terminal-Seite auf eine Weise modifiziert sind wie die folgende Sequenz SEQ ID NO: 14: MGSSHHHHHH SSGLVPRGSH.

8. MabA-Proteine, die modifiziert sind, nach Anspruch 7, wobei diese den folgenden Sequenzen entsprechen:

- Sequenz SEQ ID NO: 15, die der Kombination aus der Sequenz SEQ ID NO: 14 und der Sequenz SEQ ID NO. 1 entspricht, das heißt der folgenden Sequenz:

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- Sequenz SEQ ID NO: 16, die der Kombination aus der Sequenz SEQ ID NO: 14 und der Sequenz SEQ ID NO: 3 entspricht, das heißt der folgenden Sequenz:

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- Sequenz SEQ ID NO: 17, die der Kombination aus der Sequenz SEQ ID NO: 14 und der Sequenz SEQ ID NO: 5 entspricht, das heißt der folgenden Sequenz:

MGSSHHHHHH SSGLVPRGSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV

VAPGYIDTDM TRALDERIQQ GALQFIPAKR VGTPAEVAGV VSFLASEDAS
YISGAVIPVD GGMGMGH

- Sequenz SEQ ID NO: 18, die der Kombination aus der Sequenz SEQ ID NO: 14 und der Sequenz SEQ ID NO: 7 entspricht, das heißt der folgenden Sequenz:

MGSSHHHHHH SSGLVPRGSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- Sequenz SEQ ID NO: 19, die der Kombination aus der Sequenz SEQ ID NO: 14 und der Sequenz SEQ ID NO: 9 entspricht, das heißt der folgenden Sequenz:

MGSSHHHHHH SSGLVPRGSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- Sequenz SEQ ID NO: 20, die der Kombination aus der Sequenz SEQ ID NO: 14 und der Sequenz SEQ ID NO: 10 entspricht, das heißt der folgenden Sequenz:

MGSSHHHHHH SSGLVPRGSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- Sequenz SEQ ID NO: 21, die der Kombination aus der Sequenz SEQ ID NO: 14 und der Sequenz SEQ ID NO: 11 entspricht, das heißt der folgenden Sequenz:

MGSSHHHHHH SSGLVPRGSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- Sequenz SEQ ID NO: 22, die der Kombination aus der Sequenz SEQ ID NO: 14 und der Sequenz SEQ ID NO: 12 entspricht, das heißt der folgenden Sequenz:

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,

- Sequenz SEQ ID NO: 23, die der Kombination aus der Sequenz SEQ ID NO: 14 und der Sequenz SEQ ID NO: 13 entspricht, das heißt der folgenden Sequenz:

MGSSHHHHHH SSGLVPRGSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt.

**9.** Proteine, die vom MabA-Protein abgeleitet sind, nach einem der Ansprüche 1 bis 6, wobei diese eine GSH-Sequenz am N-Terminal aufweisen, das heißt die folgenden Sequenzen:

- die folgende Sequenz SEQ ID NO: 24, die der Kombination aus der GSH-Sequenz und der Sequenz SEQ ID NO: 1 entspricht,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 25, die der Kombination aus der GSH-Sequenz und der Sequenz SEQ ID NO: 3 entspricht,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 26, die der Kombination aus der GSH-Sequenz und der Sequenz SEQ ID NO: 5 entspricht,

GSH MTATATEGAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 27, die der Kombination aus der GSH-Sequenz und der Sequenz SEQ ID NO: 7 entspricht,

GSH MTATATEGAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 28, die der Kombination aus der GSH-Sequenz und der Sequenz SEQ ID NO: 9 entspricht,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 29, die der Kombination aus der GSH-Sequenz und der Sequenz SEQ ID NO: 10 entspricht,

GSH MTATATEGAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 30, die der Kombination aus der GSH-Sequenz und der Sequenz SEQ ID NO: 11 entspricht,

GSH MTATATEGAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN

KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 31, die der Kombination aus der GSH-Sequenz und der Sequenz SEQ ID NO: 12 entspricht,

GSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 32, die der Kombination aus der GSH-Sequenz und der Sequenz SEQ ID NO: 13 entspricht,

GSH MTATATEGAK PPFVSRSVLV TGGX$_1$RGIGLA
IAQRLAADGH KVAVTX$_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt.

**10.** Proteine, die vom MabA-Protein abgeleitet sind, nach einem der Ansprüche 1 bis 6, bei denen die ersten sieben Aminosäuren unterdrückt sind, das heißt die folgenden Sequenzen:

- die folgende Sequenz SEQ ID NO: 33; die der Sequenz SEQ ID NO: 1 entspricht, wobei die sieben ersten Aminosäuren unterdrückt sind:

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 34, die der Sequenz SEQ ID NO: 3 entspricht, wobei die sieben ersten Aminosäuren unterdrückt sind:

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN

KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 35, die der Sequenz SEQ ID NO: 5 entspricht, wobei die sieben ersten Aminosäuren unterdrückt sind:

GAK PPFVSRSVLV TGGNRGIGLA
IAQRLAADGH KVAVTHRGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 36, die der Sequenz SEQ ID NO: 7 entspricht, wobei die sieben ersten Aminosäuren unterdrückt sind:

GAK PPFVSRSVLV TGGNRGIGLA
IAQRLAADGH KVAVTHRGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

- die folgende Sequenz SEQ ID NO: 37, die der Sequenz SEQ ID NO: 9 entspricht, wobei die sieben ersten Aminosäuren unterdrückt sind:

GAK PPFVSRSVLV TGG$X_1$RGIGLA
IAQRLAADGH KVAVT$X_2$RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 38, die der Sequenz SEQ ID NO: 10 entspricht, wobei die sieben ersten Aminosäuren unterdrückt sind:

GAK PPFVSRSVLV TGG$X_1$RGIGLA
IAQRLAADGH KVAVT$X_2$RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGSWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH

bei der $X_1$ das D oder E und $X_2$ das H oder D darstellt,
- die folgende Sequenz SEQ ID NO: 39, die der Sequenz SEQ ID NO: 11 entspricht, wobei die sieben ersten

Aminosäuren unterdrückt sind:

```
GAK PPFVSRSVLV TGGX1RGIGLA
IAQRLAADGH KVAVTX2RGSG APKGLFGVEC DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH
```

bei der $X_1$ das D oder E und $X_2$ das H oder D darstellt,
- die folgende Sequenz SEQ ID NO: 40, die der Sequenz SEQ ID NO: 12 entspricht, wobei die sieben ersten Aminosäuren unterdrückt sind:

```
GAK PPFVSRSVLV TGGX1RGIGLA
IAQRLAADGH KVAVTX2RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIGS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH
```

bei der $X_1$ das D oder E und $X_2$ das H oder D darstellt,
- die folgende Sequenz SEQ ID NO: 41, die der Sequenz SEQ ID NO: 13 entspricht, wobei die sieben ersten Aminosäuren unterdrückt sind:

```
GAK PPFVSRSVLV TGGX1RGIGLA
IAQRLAADGH KVAVTX2RGSG APKGLFGVEV DVTDSDAVDR AFTAVEEHQG
PVEVLVSNAG LSADAFLMRM TEEKFEKVIN ANLTGAFRVA QRASRSMQRN
KFGRMIFIXS VSGLWGIGNQ ANYAASKAGV IGMARSIARE LSKANVTANV
VAPGYIDTDM TRALDERIQQ GALQFIPAKR VGTPAEVAGV VSFLASEDAS
YISGAVIPVD GGMGMGH
```

bei der $X_1$ das D oder E und $X_2$ das H oder D darstellt.

**11.** Rekombinante abgeleitete Proteine, die in gereinigter Form sind, nach einem der Ansprüche 1 bis 10, wie sie durch Transformation der Bakterienstämme von E. Coli mit einem Plasmid erhalten werden, wobei dieses eine Sequenz enthält, die das Gen umfasst, das ein vom MabA-Protein ableitendes Protein kodiert, so wie es in einem der Ansprüche 1 bis 10 definiert wurde und gefolgt von einem Schritt der Reinigung, in dessen Verlauf:

- die oben genannten rekombinanten E. Coli-Bakterien in einem Waschpuffer gewaschen werden, dann in einen Lyse-Puffer gelegt werden und durch einen Gefrier-Auftau-Zyklus in Gegenwart eines Protease- oder Lysozymhemmers lysiert werden,
- nach der Behandlung durch die DNAse I und die RNAse A in Gegenwart von $MgCl_2$ und durch Zentrifugieren der Überstand der Bakterienlyse, der im vorangegangenen Schritt gewonnen wurde und zu dem 10 % (v/v) Glycerol gegeben wurden oder $NADP^+$ 400 $\mu$M, auf eine Ni-NTA-Agarose-Säule aus Harz gegeben wird,
- nach mehreren Waschungen mit dem Puffer mit 5 mM und dann mit 50 mM Imidazol das MabA-Protein oder das abgeleitete Protein mit dem Elutionspuffer eluiert wird.

**12.** Rekombinante Proteine, die in gereinigter Form nach einem der Ansprüche 1 bis 10 auf eine Weise abgeleitet sind, wie sie gemäß dem Verfahren von Anspruch 11 erhalten werden, bei dem die verschiedenen Waschpuffer für Bakterien, die Lysepuffer, Waschpuffer und Elutionspuffer die Folgenden sind:

- Waschpuffer für Bakterien: Potassiumphosphat 10 mM, pH-Wert 7,8,

- Lyse-Puffer: Potassiumphosphat 50 mM, pH-Wert 7,8, enthaltend 500 mM NaCl, 5 mM Imidazol,
- Waschpuffer: Potassiumphosphat 50 mM, pH-Wert 7,8, enthaltend 500 mM NaCl, 5 und 50 mM Imidazol,
- Elutionspuffer: Potassiumphosphat 50 mM, pH-Wert 7,8, enthaltend 500 mM NaCl und 175 mM Imidazol.

**13.** Rekombinante Proteine, die in gereinigter Form nach einem der Ansprüche 1 bis 10 auf eine Weise abgeleitet werden, wie sie nach dem Verfahren von Anspruch 11 erhalten werden, bei dem die verschiedenen Waschpuffer für Bakterien, die Lysepuffer, Waschpuffer und Elutionspuffer die Folgenden sind:

- Waschpuffer für Bakterien: 10 mM Tris, pH-Wert 8,0,
- Lyse-Puffer:

  . 50 mM Tris-Puffer, pH-Wert 8,0, ergänzt durch 300 mM $LiSO_4$ und 5 mM Imidazol,
  . oder 50 mM Tris-Puffer, pH-Wert 8,0, ergänzt durch 300 mM KCl und 5 mM Imidazol,

- Waschpuffer:

  . 50 mM Tris-Puffer, pH-Wert 8,0, ergänzt durch 300 mM $LiSO_4$ und 5 oder 50 mM Imidazol,
  . oder 50 mM Tris-Puffer, pH-Wert 8,0, ergänzt durch 300 mM KCl und 5 oder 50 mM Imidazol.

- Elutionspuffer:

  . 20 mM MES-Puffer, pH-Wert 6,4, 300 mM LiS04 und 175 bis 750 mM Imidazol,
  . oder 20 mM PIPES-Puffer, pH-Wert 8,0, ergänzt durch 300 mM KCl und 175 bis 750 mM Imidazol.

**14.** Proteine nach einem der Ansprüche 1 bis 13, **gekennzeichnet durch** ihre enzymatische Aktivität, die für Substrate des langkettigen β-Cetoscyl-Typus spezifisch ist, insbesondere mit 8 bis 20 Kohlenstoffatomen, wie das β-Cetooctanoyl-CoA oder das β-Cetododecanoyl-CoA.

**15.** Proteine nach einem der Ansprüche 1 bis 14, deren Hauptmerkmale ihrer Tertiärtruktur bei einer Auflösung von 1,6 bis 2,0 Angström durch die Analyse anhand der Beugung der Röntgenstrahlen an den Kristallen der besagten Proteine festgestellt werden, wobei diese derart sind, wie sie in Figur 1 für das rekombinante MabA-Protein, das entsprechend der Sequenz SEQ ID NO: 15 modifiziert wurde, dargestellt sind, und wie sie in Figur 2 für das abgeleitete MabA-Protein C(60)V, das der Sequenz SEQ ID NO: 16 entspricht, dargestellt sind, und so, wie sie in Figur 3 für das abgeleitete MabA-Protein C(60)V / S(144)L, das der Sequenz SEQ ID NO: 17 entspricht, dargestellt sind.

**16.** Proteine nach einem der Ansprüche 1 bis 15 in kristallisierter Form.

**17.** Proteinkristalle nach Anspruch 16, wie sie durch das Verfahren der Wasserdampfdiffusion mit einem hängenden Tropfen erhalten wurden, indem die besagten Proteine vermischt werden (1 μl einer Lösung aus 10 mg/ml) mit einer Polyethylen-Glycol-Lösung, CsCl (150 bis 300 mM) und Glycerol (zu 10 %) in einem Puffer (PIPES) bei einem pH-Wert von 6,2.

**18.** Proteinkristalle nach Anspruch 16 oder 17, wie sie gemäß dem Verfahren der Kristallisation, das in Anspruch 17 beschrieben ist, erhalten werden, wobei besagtes Verfahren ausgehend von den gereinigten Proteinen nach Anspruch 13 durchgeführt wird.

**19.** Kristalle des rekombinanten MabA-Proteins entsprechend der Sequenz SEQ ID NO: 15 nach einem der Ansprüche 16 bis 18, wobei die Atomkoordinaten der Tertiärstruktur in Figur 1 dargestellt sind und die folgenden Merkmale aufweisen:

- Maschenparameter:

  . a = 81.403 Angström, b = 116.801 Angström, c = 52.324 Angström,
  . $\alpha = \beta = 90.00$ °, $\gamma = 122.30$ °,

- Raumgruppe: C2,
- Maximale Beugung = 2,05 Angström.

**20.** Kristalle des C(60)V-Proteins entsprechend der Sequenz SEQ ID NO: 16 nach einem der Ansprüche 16 bis 18, wobei die Atomkoordinaten der Tertiärstruktur in Figur 2 dargestellt sind und die folgenden Merkmale aufweisen:

- Maschenparameter:

  . a = 82.230 Angström, b = 118.610 Angström, c = 53.170 Angström,
  . $\alpha = \beta = 90.00$ °, $\gamma = 122.74$ °,

- Raumgruppe: C2,
- Maximale Beugung = 2,6 Angström.

**21.** Kristalle des C(60)V / S(144)L-Proteins entsprechend der Sequenz SEQ ID NO: 18 nach einem der Ansprüche 16 bis 18, wobei die Atomkoordinaten der Tertiärstruktur in Figur 3 dargestellt sind und die folgenden Merkmale aufweisen:

- Maschenparameter:

  . a = 81.072 Angström, b = 117.022 Angström, c = 53.170 Angström,
  . $\alpha = \beta = 90.00$ °, $\gamma = 122.42$°,

- Raumgruppe: C2,
- Maximale Beugung = 1,75 Angström.

**22.** Proteinkristalle nach einem der Ansprüche 17 bis 21, bei denen besagte Proteine an einen Liganden gebunden sind, nämlich an ein Molekül, das in der Lage ist, sich an das MabA-Protein oder an Proteine, die aus diesem zuletzt genannten Protein abgeleitet sind, zu binden, insbesondere an ihre aktive Stelle, die hauptsächlich durch die Aminosäuren, die sich an den folgenden Positionen befinden, begrenzt wird: 21 bis 28, 45 bis 48, 60 bis 63, 87 bis 100, 112, 138 bis 157, 183 bis 212, und 240 bis 247, sowie Proteine, die in einem der Ansprüche 1 bis 6 beschrieben sind oder sich an den Positionen 41 bis 48, 65 bis 68, 80 bis 83, 107 bis 120, 132, 158 bis 177, 203 bis 232, und 260 bis 267 befinden, sowie Proteine, die in einem der Ansprüche 7 oder 8 beschrieben sind oder sich an den Positionen 24 bis 31, 48 bis 51, 63 bis 66, 90 bis 103, 115, 141 bis 160, 186 bis 215, und 243 bis 250 befinden, sowie Proteine, die in Anspruch 9 beschrieben sind oder sich an den Positionen 14 bis 11, 38 bis 41, 53 bis 56, 80 bis 93, 105, 131 bis 150, 176 bis 205, und 233 bis 240 befinden, sowie Proteine, die in Anspruch 10 beschrieben sind, wobei besagte Kristalle durch Einweichen oder durch Co-Kristallisation eines rekombinanten Proteines erhalten werden, das vom MabA-Protein nach einem der Ansprüche 1 bis 16 in der Gegenwart des besagten Liganden abgeleitet wurde, insbesondere unter den Bedingungen der Kristallisation, die in Anspruch 17 definiert sind.

**23.** Nukleotidsequenz, die ein Protein kodiert, das vom MabA-Protein abgeleitet wurde, wie es in einem der Ansprüche 1 bis 16 definiert ist.

**24.** Rekombinante Nukleotidsequenz, umfassend eine Nukleotidsequenz, die ein vom MabA-Protein ableitendes Protein kodiert, nach einem der Ansprüche 1 bis 16, zusammen mit den notwendigen Elementen für die Transkription dieser Sequenz, insbesondere mit einem Promotor und einem Transkriptionsterminator.

**25.** Vektor, insbesondere ein Plasmid, umfassend eine Nukleotidsequenz nach Anspruch 24.

**26.** Wirtszellen, die durch einen Vektor nach Anspruch 25 transformiert werden, wobei besagte Zellen insbesondere unter den Bakterien wie *E. Coli* ausgewählt werden, oder jeder andere Mikroorganismus; der für die Produktion von Proteinen verwendet wird.

**27.** Verfahren der Aufbereitung von rekombinanten Proteinen, die vom MabA-Protein nach einem der Ansprüche 1 bis 16 abgeleitet sind, **gekennzeichnet dadurch, dass** dieses die folgenden Schritte umfasst:

- Transformation der Zellen mit Hilfe eines rekombinanten Vektors nach Anspruch 25,
- Anlegen einer Kultur mit der auf diese Weise transformierten Zellen und Gewinnen der besagten Proteine, die durch besagte Zellen produziert werden,
- Reinigen der besagten Proteine nach dem Verfahren, das in den Ansprüchen 11 bis 13 beschrieben ist.

**28.** Verwendung von rekombinanten Proteinen, die vom MabA-Protein nach einem der Ansprüche 1 bis 16 abgeleitet sind, oder von Kristallen nach einem der Ansprüche 17 bis 22 für die Durchführung von Verfahren zum Screening von Liganden des MabA-Proteins und insbesondere von Molekülen, die dazu neigen, sich spezifisch an die aktive Stelle des MabA-Proteins zu binden oder Proteine mit ähnlicher Struktur wie das MabA-Protein, und Hemmen der enzymatischen Aktivität dieses Letzteren, wobei diese Hemmstoffe insbesondere ausgewählt sind unter den:

- Steroidderivaten,
- Derivaten des Tuberkulose-spezifischen Antiobiotikums Isoniazid (Isonicotinsäure-Hydrazid), sowie den Derivaten des Isonicotinoyl-NAD(P)-Adduktes,
- Derivaten aus N-Acetyl-Cysteamin oder anderen Arten von simplifizierten Derivaten des Coenzyms A, umfassend einen Fluorophor mit einem Pfropf, der die Anwendung des Verfahrens der Fluoreszenz-Spektroskopie gestattet, wobei diese für die Feststellung des Zusammenwirkens der Proteine und Liganden zeitlich aufgelöst ist,
- Derivaten, die das InhA-Protein des *Mycobacterium Tuberculosis* hemmen.

**29.** Verwendung nach Anspruch 28 für die Durchführung von Verfahren zum Screening von Liganden des MabA-Proteins, die in pharmazeutischen Zusammensetzungen verwendet werden können, insbesondere im Rahmen der Behandlung von Krankheiten, die in Verbindung stehen mit mykobakteriellen Infektionen wie der Tuberkulose, die verbunden ist mit der Infektion durch das *Mycobacterium Tuberculosis* oder durch das *Mycobacterium Africanium* oder Lepra, die in Verbindung steht mit der Infektion durch das *Mycobacterium Leprae* oder die Mykobakteriose, die in Verbindung steht mit der Infektion durch fakultativ pathogene Mykobakterien wie das *Mycobacterium avium, das Mycobacterium fortuitum, das Mycobacterium kansasii* und das *Mycobacterium Chelonae.*

**30.** Verfahren zum Screening von Liganden des MabA-Proteins, **gekennzeichnet dadurch, dass** dieses die folgenden Schritte umfasst:

- Herstellen eines rekombinanten Proteines, das vom MabA-Protein nach einem der Ansprüche 1 bis 16 abgeleitet ist,
- Feststellen der möglichen Bindung zwischen besagtem Protein und dem durch Messung getesteten Liganden infolge der Durchführung der Fluoreszenzanregung, insbesondere bei 300 nm, wobei die Intensität der ausgestrahlten Fluoreszenz des besagten Proteines zwischen 300 und 400 nm beträgt (im Wesentlichen gemäß der Fluoreszenzanregung des Einzeltryptophans W 145) und Vergleich mit der Intensität der Fluoreszenz, die in einem Assay in der Abwesenheit des Liganden ausgestrahlt wurde, Fixieren eines Liganden an der aktiven Stelle des MabA, wobei dies durch ein Fluoreszenzquenching **gekennzeichnet** ist.

**31.** Verfahren zum Screening von Liganden, die das MabA-Protein hemmen, **gekennzeichnet dadurch, dass** dieses die folgenden Schritte umfasst:

- Herstellen eines rekombinanten Proteines, das vom MabA-Protein nach einem der Ansprüche 1 bis 16 abgeleitet ist, in einem Reaktionsmedium, das ein Substrat wie ein β-Cetoacyl-Derivat enthält, das in Anspruch 14 definiert ist, sowie das NADPH-Coenzym und den getesteten Liganden,
- Feststellen einer potenziellen Fähigkeit des getesteten Liganden als Hemmstoff durch kinetische Messung des Absorptionsgrades, insbesondere bei 340 nm und Vergleich der Neigung der optischen Dichtekurve im Hinblick auf die Zeit mit der Neigung, die in einem Assay ohne Vorhandensein des Liganden erhalten wurde.

**32.** Verfahren zum Screening von Liganden des MabA-Proteins, **gekennzeichnet dadurch, dass** dieses die folgenden Schritte umfasst:

- Herstellen eines rekombinanten Proteines, das vom MabA-Protein nach einem der Ansprüche 1 bis 16 abgeleitet ist, mit dem getesteten Liganden,
- Analyse der Tertiärstruktur des Komplexes, der zwischen besagtem Protein und besagtem Liganden in löslicher Phase entstanden ist, insbesondere durch NMR-Spektroskopie und durch Fluoreszenz.

**33.** Verfahren zum Screening von Liganden des MabA-Proteins, **gekennzeichnet dadurch, dass** dieses die folgenden Schritte umfasst:

- Co-Kristallisation des getesteten Liganden und eines rekombinanten Proteines, das vom MabA-Protein nach einem der Ansprüche 1 bis 16 abgeleitet wurde, insbesondere unter den Kristallisationsbedingungen, die in

Anspruch 17 definiert sind und auf eine Weise, so dass Kristalle nach Anspruch 22 erhalten werden,
- oder Einweichen der Kristalle eines rekombinanten Proteines, das nach einem der Ansprüche 17 bis 22 abgeleitet ist, in optimierten Lösungen, die den potenziellen Liganden enthalten,
- Analyse der Tertiärstruktur der oben genannten Kristalle, insbesondere durch Beugung von Röntgenstrahlen (im Hinblick auf die Auswahl der Liganden, die eine optimale Kapazität aufweisen, die aktive Stelle der besagten Proteine zu besetzen und zu blockieren).

```
REMARK refinement resolution: 500.0 - 2.03 A
REMARK final    r= 0.1974 free_r= 0.2348
CRYST1   81.403  116.801   52.324  90.00 122.30  90.00 C 2
REMARK DATE:20-Nov-2001  10:28:39       created by user: martin
ATOM      1  C   GLY A   8      28.557 -10.078  12.384  1.00 44.73      A
ATOM      2  O   GLY A   8      28.079  -9.513  13.369  1.00 44.05      A
ATOM      3  N   GLY A   8      29.206 -12.350  11.625  1.00 45.14      A
ATOM      4  CA  GLY A   8      28.066 -11.442  11.948  1.00 45.13      A
ATOM      5  N   ALA A   9      29.516  -9.547  11.634  1.00 44.33      A
ATOM      6  CA  ALA A   9      30.111  -8.253  11.931  1.00 42.41      A
ATOM      7  CB  ALA A   9      31.313  -8.017  11.017  1.00 42.90      A
ATOM      8  C   ALA A   9      29.146  -7.074  11.827  1.00 40.87      A
ATOM      9  O   ALA A   9      28.654  -6.742  10.748  1.00 40.84      A
ATOM     10  N   LYS A  10      28.879  -6.446  12.965  1.00 39.44      A
ATOM     11  CA  LYS A  10      28.008  -5.283  13.003  1.00 36.92      A
ATOM     12  CB  LYS A  10      27.529  -5.025  14.431  1.00 35.55      A
ATOM     13  CG  LYS A  10      26.716  -3.748  14.590  1.00 34.35      A
ATOM     14  CD  LYS A  10      26.233  -3.583  16.017  1.00 34.93      A
ATOM     15  CE  LYS A  10      25.366  -2.347  16.165  1.00 35.37      A
ATOM     16  NZ  LYS A  10      24.780  -2.238  17.530  1.00 37.64      A
ATOM     17  C   LYS A  10      28.832  -4.097  12.516  1.00 35.69      A
ATOM     18  O   LYS A  10      29.955  -3.887  12.975  1.00 36.27      A
ATOM     19  N   PRO A  11      28.295  -3.315  11.567  1.00 34.03      A
ATOM     20  CD  PRO A  11      26.978  -3.432  10.916  1.00 34.01      A
ATOM     21  CA  PRO A  11      29.031  -2.158  11.053  1.00 31.83      A
ATOM     22  CB  PRO A  11      28.009  -1.474  10.150  1.00 32.57      A
ATOM     23  CG  PRO A  11      27.177  -2.619   9.660  1.00 34.15      A
ATOM     24  C   PRO A  11      29.460  -1.263  12.209  1.00 30.91      A
ATOM     25  O   PRO A  11      28.669  -0.984  13.112  1.00 31.60      A
ATOM     26  N   PRO A  12      30.720  -0.807  12.202  1.00 29.48      A
ATOM     27  CD  PRO A  12      31.728  -0.953  11.137  1.00 28.57      A
ATOM     28  CA  PRO A  12      31.213   0.059  13.277  1.00 28.04      A
ATOM     29  CB  PRO A  12      32.679   0.259  12.906  1.00 30.02      A
ATOM     30  CG  PRO A  12      32.645   0.213  11.401  1.00 29.76      A
ATOM     31  C   PRO A  12      30.434   1.375  13.340  1.00 27.19      A
ATOM     32  O   PRO A  12      30.087   1.952  12.310  1.00 26.47      A
ATOM     33  N   PHE A  13      30.158   1.837  14.553  1.00 25.82      A
ATOM     34  CA  PHE A  13      29.416   3.072  14.747  1.00 26.34      A
ATOM     35  CB  PHE A  13      29.184   3.323  16.239  1.00 25.18      A
ATOM     36  CG  PHE A  13      28.437   4.595  16.524  1.00 24.50      A
ATOM     37  CD1 PHE A  13      27.066   4.673  16.313  1.00 22.49      A
ATOM     38  CD2 PHE A  13      29.114   5.733  16.956  1.00 24.05      A
ATOM     39  CE1 PHE A  13      26.376   5.863  16.522  1.00 22.77      A
ATOM     40  CE2 PHE A  13      28.436   6.929  17.168  1.00 22.47      A
ATOM     41  CZ  PHE A  13      27.062   6.996  16.949  1.00 22.44      A
ATOM     42  C   PHE A  13      30.109   4.292  14.140  1.00 26.15      A
ATOM     43  O   PHE A  13      31.336   4.363  14.088  1.00 26.51      A
ATOM     44  N   VAL A  14      29.305   5.244  13.675  1.00 25.33      A
ATOM     45  CA  VAL A  14      29.805   6.488  13.093  1.00 22.18      A
ATOM     46  CB  VAL A  14      29.697   6.495  11.559  1.00 24.05      A
ATOM     47  CG1 VAL A  14      30.077   7.869  11.020  1.00 23.47      A
ATOM     48  CG2 VAL A  14      30.598   5.427  10.964  1.00 24.64      A
ATOM     49  C   VAL A  14      28.960   7.630  13.633  1.00 21.01      A
ATOM     50  O   VAL A  14      27.739   7.645  13.446  1.00 17.53      A
ATOM     51  N   SER A  15      29.605   8.579  14.313  1.00 18.10      A
ATOM     52  CA  SER A  15      28.895   9.718  14.879  1.00 17.32      A
ATOM     53  CB  SER A  15      29.788  10.489  15.851  1.00 16.29      A
ATOM     54  OG  SER A  15      29.043  11.494  16.509  1.00 15.78      A
ATOM     55  C   SER A  15      28.491  10.634  13.735  1.00 17.44      A
ATOM     56  O   SER A  15      29.346  11.148  13.010  1.00 17.96      A
ATOM     57  N   ARG A  16      27.190  10.846  13.580  1.00 15.71      A
ATOM     58  CA  ARG A  16      26.692  11.684  12.491  1.00 14.81      A
ATOM     59  CB  ARG A  16      25.552  10.967  11.755  1.00 13.92      A
ATOM     60  CG  ARG A  16      25.917   9.629  11.145  1.00 14.97      A
ATOM     61  CD  ARG A  16      26.793   9.752   9.930  1.00 14.77      A
ATOM     62  NE  ARG A  16      27.055   8.439   9.338  1.00 15.15      A
ATOM     63  CZ  ARG A  16      27.843   8.227   8.292  1.00 15.94      A
ATOM     64  NH1 ARG A  16      28.458   9.242   7.709  1.00 15.75      A
ATOM     65  NH2 ARG A  16      28.013   6.995   7.827  1.00 13.09      A
ATOM     66  C   ARG A  16      26.179  13.039  12.936  1.00 14.49      A
ATOM     67  O   ARG A  16      25.726  13.201  14.067  1.00 12.61      A
ATOM     68  N   SER A  17      26.256  14.010  12.030  1.00 15.43      A
ATOM     69  CA  SER A  17      25.720  15.341  12.292  1.00 15.06      A
ATOM     70  CB  SER A  17      26.246  16.355  11.270  1.00 16.01      A
```

Figure 1

| | | | | | | | | | | | |
|---|---|---|---|---|---|---|---|---|---|---|---|
| ATOM | 71 | OG | SER | A | 17 | 25.610 | 17.611 | 11.452 | 1.00 | 19.05 | A |
| ATOM | 72 | C | SER | A | 17 | 24.212 | 15.146 | 12.097 | 1.00 | 14.97 | A |
| ATOM | 73 | O | SER | A | 17 | 23.760 | 14.836 | 10.998 | 1.00 | 13.15 | A |
| ATOM | 74 | N | VAL | A | 18 | 23.451 | 15.329 | 13.168 | 1.00 | 14.92 | A |
| ATOM | 75 | CA | VAL | A | 18 | 22.010 | 15.123 | 13.158 | 1.00 | 13.35 | A |
| ATOM | 76 | CB | VAL | A | 18 | 21.618 | 14.080 | 14.244 | 1.00 | 12.47 | A |
| ATOM | 77 | CG1 | VAL | A | 18 | 20.096 | 13.986 | 14.384 | 1.00 | 15.18 | A |
| ATOM | 78 | CG2 | VAL | A | 18 | 22.201 | 12.722 | 13.890 | 1.00 | 12.39 | A |
| ATOM | 79 | C | VAL | A | 18 | 21.185 | 16.382 | 13.410 | 1.00 | 13.87 | A |
| ATOM | 80 | O | VAL | A | 18 | 21.526 | 17.204 | 14.259 | 1.00 | 13.59 | A |
| ATOM | 81 | N | LEU | A | 19 | 20.091 | 16.517 | 12.668 | 1.00 | 13.12 | A |
| ATOM | 82 | CA | LEU | A | 19 | 19.188 | 17.641 | 12.850 | 1.00 | 12.82 | A |
| ATOM | 83 | CB | LEU | A | 19 | 19.267 | 18.623 | 11.674 | 1.00 | 12.78 | A |
| ATOM | 84 | CG | LEU | A | 19 | 18.257 | 19.775 | 11.784 | 1.00 | 15.13 | A |
| ATOM | 85 | CD1 | LEU | A | 19 | 18.395 | 20.466 | 13.144 | 1.00 | 14.97 | A |
| ATOM | 86 | CD2 | LEU | A | 19 | 18.482 | 20.767 | 10.647 | 1.00 | 15.96 | A |
| ATOM | 87 | C | LEU | A | 19 | 17.773 | 17.102 | 12.983 | 1.00 | 13.06 | A |
| ATOM | 88 | O | LEU | A | 19 | 17.241 | 16.482 | 12.061 | 1.00 | 13.57 | A |
| ATOM | 89 | N | VAL | A | 20 | 17.179 | 17.320 | 14.146 | 1.00 | 13.85 | A |
| ATOM | 90 | CA | VAL | A | 20 | 15.825 | 16.874 | 14.420 | 1.00 | 13.99 | A |
| ATOM | 91 | CB | VAL | A | 20 | 15.756 | 16.112 | 15.763 | 1.00 | 14.53 | A |
| ATOM | 92 | CG1 | VAL | A | 20 | 14.336 | 15.613 | 16.000 | 1.00 | 11.58 | A |
| ATOM | 93 | CG2 | VAL | A | 20 | 16.764 | 14.949 | 15.769 | 1.00 | 11.26 | A |
| ATOM | 94 | C | VAL | A | 20 | 14.911 | 18.097 | 14.514 | 1.00 | 16.26 | A |
| ATOM | 95 | O | VAL | A | 20 | 14.965 | 18.837 | 15.497 | 1.00 | 16.66 | A |
| ATOM | 96 | N | THR | A | 21 | 14.077 | 18.322 | 13.502 | 1.00 | 16.25 | A |
| ATOM | 97 | CA | THR | A | 21 | 13.178 | 19.469 | 13.550 | 1.00 | 19.55 | A |
| ATOM | 98 | CB | THR | A | 21 | 12.502 | 19.726 | 12.183 | 1.00 | 18.85 | A |
| ATOM | 99 | OG1 | THR | A | 21 | 11.556 | 18.687 | 11.896 | 1.00 | 21.80 | A |
| ATOM | 100 | CG2 | THR | A | 21 | 13.548 | 19.762 | 11.089 | 1.00 | 18.93 | A |
| ATOM | 101 | C | THR | A | 21 | 12.130 | 19.208 | 14.623 | 1.00 | 20.29 | A |
| ATOM | 102 | O | THR | A | 21 | 11.694 | 18.076 | 14.810 | 1.00 | 21.50 | A |
| ATOM | 103 | N | GLY | A | 22 | 11.748 | 20.252 | 15.348 | 1.00 | 22.10 | A |
| ATOM | 104 | CA | GLY | A | 22 | 10.767 | 20.092 | 16.407 | 1.00 | 23.21 | A |
| ATOM | 105 | C | GLY | A | 22 | 11.234 | 19.090 | 17.450 | 1.00 | 23.29 | A |
| ATOM | 106 | O | GLY | A | 22 | 10.426 | 18.385 | 18.056 | 1.00 | 24.35 | A |
| ATOM | 107 | N | GLY | A | 23 | 12.543 | 19.028 | 17.674 | 1.00 | 22.96 | A |
| ATOM | 108 | CA | GLY | A | 23 | 13.075 | 18.082 | 18.638 | 1.00 | 22.74 | A |
| ATOM | 109 | C | GLY | A | 23 | 13.208 | 18.589 | 20.061 | 1.00 | 24.18 | A |
| ATOM | 110 | O | GLY | A | 23 | 14.077 | 18.123 | 20.803 | 1.00 | 23.85 | A |
| ATOM | 111 | N | ASN | A | 24 | 12.350 | 19.525 | 20.458 | 1.00 | 24.19 | A |
| ATOM | 112 | CA | ASN | A | 24 | 12.406 | 20.071 | 21.808 | 1.00 | 27.28 | A |
| ATOM | 113 | CB | ASN | A | 24 | 12.472 | 21.603 | 21.756 | 1.00 | 30.22 | A |
| ATOM | 114 | CG | ASN | A | 24 | 11.253 | 22.222 | 21.094 | 1.00 | 33.69 | A |
| ATOM | 115 | OD1 | ASN | A | 24 | 10.890 | 21.866 | 19.968 | 1.00 | 35.62 | A |
| ATOM | 116 | ND2 | ASN | A | 24 | 10.621 | 23.164 | 21.786 | 1.00 | 34.09 | A |
| ATOM | 117 | C | ASN | A | 24 | 11.239 | 19.623 | 22.684 | 1.00 | 27.64 | A |
| ATOM | 118 | O | ASN | A | 24 | 11.218 | 19.893 | 23.884 | 1.00 | 29.17 | A |
| ATOM | 119 | N | ARG | A | 25 | 10.269 | 18.934 | 22.092 | 1.00 | 27.34 | A |
| ATOM | 120 | CA | ARG | A | 25 | 9.123 | 18.449 | 22.856 | 1.00 | 28.27 | A |
| ATOM | 121 | CB | ARG | A | 25 | 8.019 | 19.519 | 22.907 | 1.00 | 30.10 | A |
| ATOM | 122 | CG | ARG | A | 25 | 8.310 | 20.670 | 23.859 | 0.00 | 31.98 | A |
| ATOM | 123 | CD | ARG | A | 25 | 7.161 | 21.669 | 23.904 | 1.00 | 35.26 | A |
| ATOM | 124 | NE | ARG | A | 25 | 5.902 | 21.060 | 24.329 | 0.00 | 34.30 | A |
| ATOM | 125 | CZ | ARG | A | 25 | 5.687 | 20.535 | 25.532 | 0.00 | 34.59 | A |
| ATOM | 126 | NH1 | ARG | A | 25 | 6.649 | 20.540 | 26.445 | 0.00 | 34.52 | A |
| ATOM | 127 | NH2 | ARG | A | 25 | 4.504 | 20.009 | 25.825 | 0.00 | 34.52 | A |
| ATOM | 128 | C | ARG | A | 25 | 8.544 | 17.141 | 22.323 | 1.00 | 26.42 | A |
| ATOM | 129 | O | ARG | A | 25 | 8.817 | 16.743 | 21.196 | 1.00 | 26.62 | A |
| ATOM | 130 | N | GLY | A | 26 | 7.751 | 16.481 | 23.162 | 1.00 | 25.93 | A |
| ATOM | 131 | CA | GLY | A | 26 | 7.111 | 15.230 | 22.798 | 1.00 | 23.93 | A |
| ATOM | 132 | C | GLY | A | 26 | 7.994 | 14.199 | 22.125 | 1.00 | 22.19 | A |
| ATOM | 133 | O | GLY | A | 26 | 9.113 | 13.925 | 22.565 | 1.00 | 21.20 | A |
| ATOM | 134 | N | ILE | A | 27 | 7.477 | 13.614 | 21.050 | 1.00 | 20.49 | A |
| ATOM | 135 | CA | ILE | A | 27 | 8.206 | 12.606 | 20.302 | 1.00 | 18.19 | A |
| ATOM | 136 | CB | ILE | A | 27 | 7.353 | 12.069 | 19.137 | 1.00 | 20.27 | A |
| ATOM | 137 | CG2 | ILE | A | 27 | 8.213 | 11.269 | 18.175 | 1.00 | 19.76 | A |
| ATOM | 138 | CG1 | ILE | A | 27 | 6.204 | 11.227 | 19.696 | 1.00 | 21.50 | A |
| ATOM | 139 | CD | ILE | A | 27 | 5.249 | 10.704 | 18.627 | 1.00 | 25.02 | A |
| ATOM | 140 | C | ILE | A | 27 | 9.537 | 13.114 | 19.754 | 1.00 | 15.67 | A |
| ATOM | 141 | O | ILE | A | 27 | 10.524 | 12.384 | 19.762 | 1.00 | 14.78 | A |
| ATOM | 142 | N | GLY | A | 28 | 9.561 | 14.354 | 19.268 | 1.00 | 13.70 | A |
| ATOM | 143 | CA | GLY | A | 28 | 10.792 | 14.902 | 18.723 | 1.00 | 14.12 | A |

Figure 1 (suite 1)

| | | | | | | | | | | | |
|---|---|---|---|---|---|---|---|---|---|---|---|
| ATOM | 144 | C | GLY | A | 28 | 11.910 | 14.927 | 19.757 | 1.00 | 14.81 | A |
| ATOM | 145 | O | GLY | A | 28 | 13.078 | 14.691 | 19.438 | 1.00 | 12.54 | A |
| ATOM | 146 | N | LEU | A | 29 | 11.546 | 15.219 | 21.001 | 1.00 | 16.36 | A |
| ATOM | 147 | CA | LEU | A | 29 | 12.516 | 15.278 | 22.092 | 1.00 | 16.41 | A |
| ATOM | 148 | CB | LEU | A | 29 | 11.865 | 15.879 | 23.340 | 1.00 | 17.88 | A |
| ATOM | 149 | CG | LEU | A | 29 | 12.758 | 15.967 | 24.583 | 1.00 | 19.05 | A |
| ATOM | 150 | CD1 | LEU | A | 29 | 14.002 | 16.786 | 24.271 | 1.00 | 20.93 | A |
| ATOM | 151 | CD2 | LEU | A | 29 | 11.972 | 16.597 | 25.726 | 1.00 | 22.59 | A |
| ATOM | 152 | C | LEU | A | 29 | 13.039 | 13.883 | 22.410 | 1.00 | 15.61 | A |
| ATOM | 153 | O | LEU | A | 29 | 14.244 | 13.683 | 22.576 | 1.00 | 15.95 | A |
| ATOM | 154 | N | ALA | A | 30 | 12.123 | 12.922 | 22.502 | 1.00 | 14.97 | A |
| ATOM | 155 | CA | ALA | A | 30 | 12.505 | 11.549 | 22.798 | 1.00 | 15.90 | A |
| ATOM | 156 | CB | ALA | A | 30 | 11.275 | 10.651 | 22.839 | 1.00 | 15.50 | A |
| ATOM | 157 | C | ALA | A | 30 | 13.479 | 11.063 | 21.738 | 1.00 | 15.00 | A |
| ATOM | 158 | O | ALA | A | 30 | 14.411 | 10.320 | 22.040 | 1.00 | 14.77 | A |
| ATOM | 159 | N | ILE | A | 31 | 13.271 | 11.501 | 20.497 | 1.00 | 13.19 | A |
| ATOM | 160 | CA | ILE | A | 31 | 14.134 | 11.111 | 19.394 | 1.00 | 11.32 | A |
| ATOM | 161 | CB | ILE | A | 31 | 13.511 | 11.495 | 18.037 | 1.00 | 13.39 | A |
| ATOM | 162 | CG2 | ILE | A | 31 | 14.537 | 11.314 | 16.931 | 1.00 | 11.93 | A |
| ATOM | 163 | CG1 | ILE | A | 31 | 12.260 | 10.647 | 17.766 | 1.00 | 14.11 | A |
| ATOM | 164 | CD | ILE | A | 31 | 11.507 | 11.041 | 16.496 | 1.00 | 14.17 | A |
| ATOM | 165 | C | ILE | A | 31 | 15.521 | 11.764 | 19.484 | 1.00 | 10.05 | A |
| ATOM | 166 | O | ILE | A | 31 | 16.540 | 11.096 | 19.304 | 1.00 | 9.87 | A |
| ATOM | 167 | N | ALA | A | 32 | 15.546 | 13.072 | 19.731 | 1.00 | 11.61 | A |
| ATOM | 168 | CA | ALA | A | 32 | 16.804 | 13.805 | 19.836 | 1.00 | 12.36 | A |
| ATOM | 169 | CB | ALA | A | 32 | 16.528 | 15.294 | 20.076 | 1.00 | 9.57 | A |
| ATOM | 170 | C | ALA | A | 32 | 17.676 | 13.241 | 20.963 | 1.00 | 11.88 | A |
| ATOM | 171 | O | ALA | A | 32 | 18.861 | 12.996 | 20.775 | 1.00 | 12.40 | A |
| ATOM | 172 | N | GLN | A | 33 | 17.084 | 13.035 | 22.130 | 1.00 | 12.57 | A |
| ATOM | 173 | CA | GLN | A | 33 | 17.831 | 12.503 | 23.263 | 1.00 | 14.61 | A |
| ATOM | 174 | CB | GLN | A | 33 | 16.967 | 12.545 | 24.521 | 1.00 | 16.53 | A |
| ATOM | 175 | CG | GLN | A | 33 | 16.563 | 13.964 | 24.873 | 1.00 | 21.81 | A |
| ATOM | 176 | CD | GLN | A | 33 | 16.042 | 14.103 | 26.281 | 1.00 | 27.28 | A |
| ATOM | 177 | OE1 | GLN | A | 33 | 15.041 | 13.490 | 26.652 | 1.00 | 27.21 | A |
| ATOM | 178 | NE2 | GLN | A | 33 | 16.727 | 14.920 | 27.084 | 1.00 | 30.14 | A |
| ATOM | 179 | C | GLN | A | 33 | 18.354 | 11.088 | 23.011 | 1.00 | 14.23 | A |
| ATOM | 180 | O | GLN | A | 33 | 19.438 | 10.735 | 23.485 | 1.00 | 14.37 | A |
| ATOM | 181 | N | ARG | A | 34 | 17.598 | 10.285 | 22.253 | 1.00 | 14.10 | A |
| ATOM | 182 | CA | ARG | A | 34 | 18.019 | 8.918 | 21.940 | 1.00 | 12.57 | A |
| ATOM | 183 | CB | ARG | A | 34 | 16.881 | 8.102 | 21.301 | 1.00 | 12.96 | A |
| ATOM | 184 | CG | ARG | A | 34 | 17.335 | 6.759 | 20.678 | 1.00 | 11.76 | A |
| ATOM | 185 | CD | ARG | A | 34 | 18.024 | 5.815 | 21.693 | 1.00 | 9.93 | A |
| ATOM | 186 | NE | ARG | A | 34 | 18.551 | 4.610 | 21.049 | 1.00 | 10.90 | A |
| ATOM | 187 | CZ | ARG | A | 34 | 17.898 | 3.452 | 20.940 | 1.00 | 13.44 | A |
| ATOM | 188 | NH1 | ARG | A | 34 | 16.675 | 3.307 | 21.441 | 1.00 | 12.30 | A |
| ATOM | 189 | NH2 | ARG | A | 34 | 18.461 | 2.438 | 20.299 | 1.00 | 15.45 | A |
| ATOM | 190 | C | ARG | A | 34 | 19.196 | 8.958 | 20.986 | 1.00 | 13.57 | A |
| ATOM | 191 | O | ARG | A | 34 | 20.189 | 8.244 | 21.183 | 1.00 | 12.62 | A |
| ATOM | 192 | N | LEU | A | 35 | 19.093 | 9.790 | 19.951 | 1.00 | 11.38 | A |
| ATOM | 193 | CA | LEU | A | 35 | 20.164 | 9.897 | 18.976 | 1.00 | 11.26 | A |
| ATOM | 194 | CB | LEU | A | 35 | 19.728 | 10.754 | 17.782 | 1.00 | 10.91 | A |
| ATOM | 195 | CG | LEU | A | 35 | 18.696 | 10.099 | 16.842 | 1.00 | 14.07 | A |
| ATOM | 196 | CD1 | LEU | A | 35 | 18.311 | 11.071 | 15.716 | 1.00 | 11.97 | A |
| ATOM | 197 | CD2 | LEU | A | 35 | 19.287 | 8.836 | 16.240 | 1.00 | 10.89 | A |
| ATOM | 198 | C | LEU | A | 35 | 21.449 | 10.455 | 19.608 | 1.00 | 12.11 | A |
| ATOM | 199 | O | LEU | A | 35 | 22.548 | 10.105 | 19.184 | 1.00 | 13.15 | A |
| ATOM | 200 | N | ALA | A | 36 | 21.301 | 11.305 | 20.622 | 1.00 | 11.64 | A |
| ATOM | 201 | CA | ALA | A | 36 | 22.456 | 11.874 | 21.322 | 1.00 | 13.44 | A |
| ATOM | 202 | CB | ALA | A | 36 | 22.009 | 13.000 | 22.253 | 1.00 | 13.60 | A |
| ATOM | 203 | C | ALA | A | 36 | 23.126 | 10.753 | 22.135 | 1.00 | 13.61 | A |
| ATOM | 204 | O | ALA | A | 36 | 24.344 | 10.583 | 22.086 | 1.00 | 13.61 | A |
| ATOM | 205 | N | ALA | A | 37 | 22.318 | 9.984 | 22.860 | 1.00 | 13.31 | A |
| ATOM | 206 | CA | ALA | A | 37 | 22.821 | 8.869 | 23.664 | 1.00 | 13.90 | A |
| ATOM | 207 | CB | ALA | A | 37 | 21.687 | 8.248 | 24.474 | 1.00 | 13.86 | A |
| ATOM | 208 | C | ALA | A | 37 | 23.483 | 7.810 | 22.784 | 1.00 | 14.79 | A |
| ATOM | 209 | O | ALA | A | 37 | 24.414 | 7.120 | 23.227 | 1.00 | 12.27 | A |
| ATOM | 210 | N | ASP | A | 38 | 23.002 | 7.691 | 21.544 | 1.00 | 13.50 | A |
| ATOM | 211 | CA | ASP | A | 38 | 23.546 | 6.744 | 20.570 | 1.00 | 12.55 | A |
| ATOM | 212 | CB | ASP | A | 38 | 22.772 | 6.775 | 19.238 | 1.00 | 12.40 | A |
| ATOM | 213 | CG | ASP | A | 38 | 21.466 | 5.987 | 19.270 | 1.00 | 13.14 | A |
| ATOM | 214 | OD1 | ASP | A | 38 | 21.224 | 5.221 | 20.222 | 1.00 | 11.29 | A |
| ATOM | 215 | OD2 | ASP | A | 38 | 20.682 | 6.138 | 18.302 | 1.00 | 15.13 | A |
| ATOM | 216 | C | ASP | A | 38 | 24.979 | 7.140 | 20.247 | 1.00 | 12.24 | A |

Figure 1 (suite 2)

| | | | | | | | | | | | |
|---|---|---|---|---|---|---|---|---|---|---|---|
| ATOM | 217 | O | ASP | A | 38 | 25.773 | 6.318 | 19.776 | 1.00 | 10.54 | A |
| ATOM | 218 | N | GLY | A | 39 | 25.285 | 8.420 | 20.444 | 1.00 | 11.48 | A |
| ATOM | 219 | CA | GLY | A | 39 | 26.622 | 8.914 | 20.161 | 1.00 | 11.43 | A |
| ATOM | 220 | C | GLY | A | 39 | 26.704 | 9.906 | 19.014 | 1.00 | 12.32 | A |
| ATOM | 221 | O | GLY | A | 39 | 27.800 | 10.310 | 18.616 | 1.00 | 11.60 | A |
| ATOM | 222 | N | HIS | A | 40 | 25.551 | 10.295 | 18.466 | 1.00 | 13.41 | A |
| ATOM | 223 | CA | HIS | A | 40 | 25.531 | 11.250 | 17.358 | 1.00 | 12.25 | A |
| ATOM | 224 | CB | HIS | A | 40 | 24.224 | 11.122 | 16.550 | 1.00 | 11.77 | A |
| ATOM | 225 | CG | HIS | A | 40 | 24.048 | 9.796 | 15.877 | 1.00 | 11.70 | A |
| ATOM | 226 | CD2 | HIS | A | 40 | 23.195 | 8.775 | 16.127 | 1.00 | 10.27 | A |
| ATOM | 227 | ND1 | HIS | A | 40 | 24.832 | 9.390 | 14.818 | 1.00 | 9.79 | A |
| ATOM | 228 | CE1 | HIS | A | 40 | 24.469 | 8.175 | 14.444 | 1.00 | 10.74 | A |
| ATOM | 229 | NE2 | HIS | A | 40 | 23.477 | 7.779 | 15.222 | 1.00 | 10.77 | A |
| ATOM | 230 | C | HIS | A | 40 | 25.639 | 12.678 | 17.896 | 1.00 | 13.34 | A |
| ATOM | 231 | O | HIS | A | 40 | 25.467 | 12.913 | 19.091 | 1.00 | 12.55 | A |
| ATOM | 232 | N | LYS | A | 41 | 25.926 | 13.613 | 16.990 | 1.00 | 14.49 | A |
| ATOM | 233 | CA | LYS | A | 41 | 26.022 | 15.044 | 17.286 | 1.00 | 14.35 | A |
| ATOM | 234 | CB | LYS | A | 41 | 27.075 | 15.692 | 16.387 | 1.00 | 16.67 | A |
| ATOM | 235 | CG | LYS | A | 41 | 28.493 | 15.226 | 16.672 | 1.00 | 18.14 | A |
| ATOM | 236 | CD | LYS | A | 41 | 29.473 | 15.751 | 15.645 | 1.00 | 23.85 | A |
| ATOM | 237 | CE | LYS | A | 41 | 29.246 | 15.108 | 14.291 | 1.00 | 25.20 | A |
| ATOM | 238 | NZ | LYS | A | 41 | 30.150 | 15.663 | 13.243 | 1.00 | 29.43 | A |
| ATOM | 239 | C | LYS | A | 41 | 24.631 | 15.551 | 16.927 | 1.00 | 14.31 | A |
| ATOM | 240 | O | LYS | A | 41 | 24.294 | 15.685 | 15.748 | 1.00 | 13.54 | A |
| ATOM | 241 | N | VAL | A | 42 | 23.830 | 15.840 | 17.942 | 1.00 | 14.64 | A |
| ATOM | 242 | CA | VAL | A | 42 | 22.451 | 16.236 | 17.711 | 1.00 | 14.35 | A |
| ATOM | 243 | CB | VAL | A | 42 | 21.505 | 15.335 | 18.548 | 1.00 | 13.50 | A |
| ATOM | 244 | CG1 | VAL | A | 42 | 20.046 | 15.710 | 18.292 | 1.00 | 12.86 | A |
| ATOM | 245 | CG2 | VAL | A | 42 | 21.752 | 13.861 | 18.201 | 1.00 | 12.03 | A |
| ATOM | 246 | C | VAL | A | 42 | 22.035 | 17.685 | 17.932 | 1.00 | 16.41 | A |
| ATOM | 247 | O | VAL | A | 42 | 22.255 | 18.260 | 19.000 | 1.00 | 18.01 | A |
| ATOM | 248 | N | ALA | A | 43 | 21.408 | 18.249 | 16.903 | 1.00 | 14.98 | A |
| ATOM | 249 | CA | ALA | A | 43 | 20.881 | 19.610 | 16.943 | 1.00 | 16.49 | A |
| ATOM | 250 | CB | ALA | A | 43 | 21.520 | 20.459 | 15.847 | 1.00 | 17.44 | A |
| ATOM | 251 | C | ALA | A | 43 | 19.371 | 19.504 | 16.712 | 1.00 | 16.71 | A |
| ATOM | 252 | O | ALA | A | 43 | 18.907 | 18.609 | 15.997 | 1.00 | 16.14 | A |
| ATOM | 253 | N | VAL | A | 44 | 18.606 | 20.405 | 17.320 | 1.00 | 16.80 | A |
| ATOM | 254 | CA | VAL | A | 44 | 17.152 | 20.404 | 17.167 | 1.00 | 17.90 | A |
| ATOM | 255 | CB | VAL | A | 44 | 16.448 | 19.987 | 18.484 | 1.00 | 18.21 | A |
| ATOM | 256 | CG1 | VAL | A | 44 | 16.983 | 18.646 | 18.975 | 1.00 | 19.11 | A |
| ATOM | 257 | CG2 | VAL | A | 44 | 16.659 | 21.048 | 19.549 | 1.00 | 18.82 | A |
| ATOM | 258 | C | VAL | A | 44 | 16.655 | 21.802 | 16.797 | 1.00 | 19.04 | A |
| ATOM | 259 | O | VAL | A | 44 | 17.324 | 22.799 | 17.079 | 1.00 | 20.03 | A |
| ATOM | 260 | N | THR | A | 45 | 15.497 | 21.879 | 16.145 | 1.00 | 18.79 | A |
| ATOM | 261 | CA | THR | A | 45 | 14.924 | 23.180 | 15.817 | 1.00 | 20.43 | A |
| ATOM | 262 | CB | THR | A | 45 | 14.363 | 23.264 | 14.382 | 1.00 | 20.44 | A |
| ATOM | 263 | OG1 | THR | A | 45 | 13.238 | 22.385 | 14.250 | 1.00 | 20.45 | A |
| ATOM | 264 | CG2 | THR | A | 45 | 15.430 | 22.905 | 13.371 | 1.00 | 21.69 | A |
| ATOM | 265 | C | THR | A | 45 | 13.774 | 23.328 | 16.789 | 1.00 | 22.74 | A |
| ATOM | 266 | O | THR | A | 45 | 13.273 | 22.335 | 17.316 | 1.00 | 20.96 | A |
| ATOM | 267 | N | HIS | A | 46 | 13.367 | 24.562 | 17.043 | 1.00 | 24.72 | A |
| ATOM | 268 | CA | HIS | A | 46 | 12.271 | 24.816 | 17.959 | 1.00 | 29.64 | A |
| ATOM | 269 | CB | HIS | A | 46 | 12.799 | 24.928 | 19.395 | 1.00 | 31.61 | A |
| ATOM | 270 | CG | HIS | A | 46 | 13.851 | 25.978 | 19.578 | 1.00 | 33.03 | A |
| ATOM | 271 | CD2 | HIS | A | 46 | 15.191 | 25.877 | 19.755 | 1.00 | 33.79 | A |
| ATOM | 272 | ND1 | HIS | A | 46 | 13.566 | 27.327 | 19.583 | 1.00 | 33.37 | A |
| ATOM | 273 | CE1 | HIS | A | 46 | 14.684 | 28.010 | 19.755 | 1.00 | 34.30 | A |
| ATOM | 274 | NE2 | HIS | A | 46 | 15.685 | 27.154 | 19.862 | 1.00 | 33.61 | A |
| ATOM | 275 | C | HIS | A | 46 | 11.555 | 26.093 | 17.548 | 1.00 | 31.11 | A |
| ATOM | 276 | O | HIS | A | 46 | 11.969 | 26.767 | 16.604 | 1.00 | 30.04 | A |
| ATOM | 277 | N | ARG | A | 47 | 10.480 | 26.418 | 18.255 | 1.00 | 34.66 | A |
| ATOM | 278 | CA | ARG | A | 47 | 9.701 | 27.611 | 17.942 | 1.00 | 38.37 | A |
| ATOM | 279 | CB | ARG | A | 47 | 8.206 | 27.283 | 18.000 | 1.00 | 38.87 | A |
| ATOM | 280 | CG | ARG | A | 47 | 7.774 | 26.180 | 17.031 | 1.00 | 40.62 | A |
| ATOM | 281 | CD | ARG | A | 47 | 8.193 | 26.496 | 15.597 | 1.00 | 40.51 | A |
| ATOM | 282 | NE | ARG | A | 47 | 7.588 | 27.733 | 15.107 | 1.00 | 43.29 | A |
| ATOM | 283 | CZ | ARG | A | 47 | 6.285 | 27.896 | 14.891 | 1.00 | 45.41 | A |
| ATOM | 284 | NH1 | ARG | A | 47 | 5.440 | 26.895 | 15.119 | 1.00 | 46.23 | A |
| ATOM | 285 | NH2 | ARG | A | 47 | 5.823 | 29.062 | 14.455 | 1.00 | 45.21 | A |
| ATOM | 286 | C | ARG | A | 47 | 10.016 | 28.776 | 18.877 | 1.00 | 40.64 | A |
| ATOM | 287 | O | ARG | A | 47 | 9.363 | 29.820 | 18.819 | 1.00 | 40.94 | A |
| ATOM | 288 | N | GLY | A | 48 | 11.021 | 28.596 | 19.731 | 1.00 | 42.31 | A |
| ATOM | 289 | CA | GLY | A | 48 | 11.398 | 29.645 | 20.659 | 1.00 | 43.97 | A |

Figure 1 (suite 3)

| | | | | | | | | | | | |
|---|---|---|---|---|---|---|---|---|---|---|---|
| ATOM | 290 | C | GLY | A | 48 | 11.482 | 29.165 | 22.094 | 1.00 | 45.86 | A |
| ATOM | 291 | O | GLY | A | 48 | 12.175 | 29.764 | 22.917 | 1.00 | 45.91 | A |
| ATOM | 292 | N | SER | A | 49 | 10.778 | 28.078 | 22.398 | 1.00 | 47.86 | A |
| ATOM | 293 | CA | SER | A | 49 | 10.772 | 27.518 | 23.747 | 1.00 | 49.27 | A |
| ATOM | 294 | CB | SER | A | 49 | 9.866 | 26.283 | 23.797 | 1.00 | 50.56 | A |
| ATOM | 295 | OG | SER | A | 49 | 9.779 | 25.755 | 25.111 | 1.00 | 51.02 | A |
| ATOM | 296 | C | SER | A | 49 | 12.185 | 27.145 | 24.207 | 1.00 | 49.96 | A |
| ATOM | 297 | O | SER | A | 49 | 12.430 | 26.965 | 25.401 | 1.00 | 50.54 | A |
| ATOM | 298 | N | GLY | A | 50 | 13.107 | 27.028 | 23.255 | 1.00 | 49.77 | A |
| ATOM | 299 | CA | GLY | A | 50 | 14.477 | 26.682 | 23.590 | 1.00 | 49.27 | A |
| ATOM | 300 | C | GLY | A | 50 | 14.749 | 25.188 | 23.579 | 1.00 | 49.57 | A |
| ATOM | 301 | O | GLY | A | 50 | 13.872 | 24.381 | 23.900 | 1.00 | 49.37 | A |
| ATOM | 302 | N | ALA | A | 51 | 15.970 | 24.819 | 23.207 | 1.00 | 48.35 | A |
| ATOM | 303 | CA | ALA | A | 51 | 16.365 | 23.418 | 23.156 | 1.00 | 48.15 | A |
| ATOM | 304 | CB | ALA | A | 51 | 17.538 | 23.242 | 22.204 | 1.00 | 47.46 | A |
| ATOM | 305 | C | ALA | A | 51 | 16.745 | 22.922 | 24.548 | 1.00 | 48.08 | A |
| ATOM | 306 | O | ALA | A | 51 | 17.226 | 23.691 | 25.384 | 1.00 | 48.43 | A |
| ATOM | 307 | N | PRO | A | 52 | 16.535 | 21.625 | 24.816 | 1.00 | 47.45 | A |
| ATOM | 308 | CD | PRO | A | 52 | 16.037 | 20.576 | 23.906 | 1.00 | 47.77 | A |
| ATOM | 309 | CA | PRO | A | 52 | 16.871 | 21.062 | 26.126 | 1.00 | 46.98 | A |
| ATOM | 310 | CB | PRO | A | 52 | 16.292 | 19.653 | 26.047 | 1.00 | 47.13 | A |
| ATOM | 311 | CG | PRO | A | 52 | 16.486 | 19.307 | 24.600 | 1.00 | 47.21 | A |
| ATOM | 312 | C | PRO | A | 52 | 18.379 | 21.067 | 26.371 | 1.00 | 46.19 | A |
| ATOM | 313 | O | PRO | A | 52 | 19.169 | 20.984 | 25.429 | 1.00 | 45.27 | A |
| ATOM | 314 | N | LYS | A | 53 | 18.770 | 21.168 | 27.637 | 1.00 | 45.43 | A |
| ATOM | 315 | CA | LYS | A | 53 | 20.183 | 21.189 | 27.998 | 1.00 | 44.82 | A |
| ATOM | 316 | CB | LYS | A | 53 | 20.344 | 21.187 | 29.524 | 1.00 | 44.81 | A |
| ATOM | 317 | CG | LYS | A | 53 | 19.592 | 22.309 | 30.217 | 1.00 | 44.03 | A |
| ATOM | 318 | CD | LYS | A | 53 | 19.892 | 22.376 | 31.709 | 1.00 | 44.09 | A |
| ATOM | 319 | CE | LYS | A | 53 | 21.297 | 22.892 | 31.980 | 1.00 | 44.68 | A |
| ATOM | 320 | NZ | LYS | A | 53 | 21.533 | 23.113 | 33.438 | 1.00 | 42.83 | A |
| ATOM | 321 | C | LYS | A | 53 | 20.913 | 19.994 | 27.404 | 1.00 | 44.10 | A |
| ATOM | 322 | O | LYS | A | 53 | 20.432 | 18.861 | 27.467 | 1.00 | 44.17 | A |
| ATOM | 323 | N | GLY | A | 54 | 22.074 | 20.253 | 26.815 | 1.00 | 43.06 | A |
| ATOM | 324 | CA | GLY | A | 54 | 22.846 | 19.176 | 26.229 | 1.00 | 43.37 | A |
| ATOM | 325 | C | GLY | A | 54 | 22.709 | 19.076 | 24.725 | 1.00 | 42.64 | A |
| ATOM | 326 | O | GLY | A | 54 | 23.501 | 18.393 | 24.073 | 1.00 | 44.87 | A |
| ATOM | 327 | N | LEU | A | 55 | 21.711 | 19.750 | 24.162 | 1.00 | 39.41 | A |
| ATOM | 328 | CA | LEU | A | 55 | 21.510 | 19.704 | 22.720 | 1.00 | 36.93 | A |
| ATOM | 329 | CB | LEU | A | 55 | 20.195 | 18.990 | 22.384 | 1.00 | 36.66 | A |
| ATOM | 330 | CG | LEU | A | 55 | 20.078 | 17.537 | 22.855 | 1.00 | 38.20 | A |
| ATOM | 331 | CD1 | LEU | A | 55 | 18.798 | 16.922 | 22.310 | 1.00 | 37.83 | A |
| ATOM | 332 | CD2 | LEU | A | 55 | 21.292 | 16.741 | 22.385 | 1.00 | 37.79 | A |
| ATOM | 333 | C | LEU | A | 55 | 21.515 | 21.084 | 22.089 | 1.00 | 34.40 | A |
| ATOM | 334 | O | LEU | A | 55 | 20.895 | 22.020 | 22.598 | 1.00 | 32.61 | A |
| ATOM | 335 | N | PHE | A | 56 | 22.227 | 21.198 | 20.973 | 1.00 | 32.34 | A |
| ATOM | 336 | CA | PHE | A | 56 | 22.326 | 22.451 | 20.241 | 1.00 | 30.13 | A |
| ATOM | 337 | CB | PHE | A | 56 | 23.363 | 22.326 | 19.127 | 1.00 | 30.06 | A |
| ATOM | 338 | CG | PHE | A | 56 | 23.570 | 23.592 | 18.346 | 1.00 | 30.24 | A |
| ATOM | 339 | CD1 | PHE | A | 56 | 24.176 | 24.696 | 18.934 | 1.00 | 30.71 | A |
| ATOM | 340 | CD2 | PHE | A | 56 | 23.163 | 23.679 | 17.019 | 1.00 | 29.29 | A |
| ATOM | 341 | CE1 | PHE | A | 56 | 24.378 | 25.873 | 18.208 | 1.00 | 30.14 | A |
| ATOM | 342 | CE2 | PHE | A | 56 | 23.358 | 24.848 | 16.285 | 1.00 | 30.97 | A |
| ATOM | 343 | CZ | PHE | A | 56 | 23.969 | 25.949 | 16.883 | 1.00 | 30.31 | A |
| ATOM | 344 | C | PHE | A | 56 | 20.962 | 22.782 | 19.638 | 1.00 | 28.67 | A |
| ATOM | 345 | O | PHE | A | 56 | 20.380 | 21.973 | 18.917 | 1.00 | 28.17 | A |
| ATOM | 346 | N | GLY | A | 57 | 20.459 | 23.974 | 19.931 | 1.00 | 25.53 | A |
| ATOM | 347 | CA | GLY | A | 57 | 19.164 | 24.362 | 19.409 | 1.00 | 24.51 | A |
| ATOM | 348 | C | GLY | A | 57 | 19.191 | 25.565 | 18.494 | 1.00 | 23.41 | A |
| ATOM | 349 | O | GLY | A | 57 | 20.040 | 26.447 | 18.626 | 1.00 | 22.83 | A |
| ATOM | 350 | N | VAL | A | 58 | 18.259 | 25.589 | 17.545 | 1.00 | 23.13 | A |
| ATOM | 351 | CA | VAL | A | 58 | 18.133 | 26.701 | 16.612 | 1.00 | 22.22 | A |
| ATOM | 352 | CB | VAL | A | 58 | 18.849 | 26.430 | 15.261 | 1.00 | 24.80 | A |
| ATOM | 353 | CG1 | VAL | A | 58 | 20.347 | 26.390 | 15.467 | 1.00 | 26.17 | A |
| ATOM | 354 | CG2 | VAL | A | 58 | 18.367 | 25.120 | 14.654 | 1.00 | 24.05 | A |
| ATOM | 355 | C | VAL | A | 58 | 16.654 | 26.922 | 16.354 | 1.00 | 22.42 | A |
| ATOM | 356 | O | VAL | A | 58 | 15.876 | 25.970 | 16.275 | 1.00 | 20.06 | A |
| ATOM | 357 | N | GLU | A | 59 | 16.256 | 28.181 | 16.240 | 1.00 | 23.28 | A |
| ATOM | 358 | CA | GLU | A | 59 | 14.860 | 28.489 | 15.994 | 1.00 | 25.11 | A |
| ATOM | 359 | CB | GLU | A | 59 | 14.568 | 29.931 | 16.398 | 1.00 | 29.66 | A |
| ATOM | 360 | CG | GLU | A | 59 | 13.124 | 30.348 | 16.239 | 1.00 | 36.25 | A |
| ATOM | 361 | CD | GLU | A | 59 | 12.826 | 31.634 | 16.985 | 1.00 | 39.99 | A |
| ATOM | 362 | OE1 | GLU | A | 59 | 13.613 | 32.595 | 16.840 | 1.00 | 40.69 | A |

Figure 1 (suite 4)

| | | | | | | | | | | | |
|---|---|---|---|---|---|---|---|---|---|---|---|
| ATOM | 363 | OE2 | GLU | A | 59 | 11.810 | 31.679 | 17.714 | 1.00 | 42.67 | A |
| ATOM | 364 | C | GLU | A | 59 | 14.576 | 28.277 | 14.516 | 1.00 | 23.68 | A |
| ATOM | 365 | O | GLU | A | 59 | 15.429 | 28.539 | 13.667 | 1.00 | 22.26 | A |
| ATOM | 366 | N | CYS | A | 60 | 13.385 | 27.784 | 14.205 | 1.00 | 23.67 | A |
| ATOM | 367 | CA | CYS | A | 60 | 13.044 | 27.549 | 12.813 | 1.00 | 24.68 | A |
| ATOM | 368 | CB | CYS | A | 60 | 13.850 | 26.371 | 12.275 | 1.00 | 25.89 | A |
| ATOM | 369 | SG | CYS | A | 60 | 13.634 | 26.085 | 10.516 | 1.00 | 25.19 | A |
| ATOM | 370 | C | CYS | A | 60 | 11.567 | 27.286 | 12.558 | 1.00 | 25.22 | A |
| ATOM | 371 | O | CYS | A | 60 | 10.967 | 26.396 | 13.163 | 1.00 | 25.05 | A |
| ATOM | 372 | N | ASP | A | 61 | 10.989 | 28.077 | 11.661 | 1.00 | 23.84 | A |
| ATOM | 373 | CA | ASP | A | 61 | 9.598 | 27.903 | 11.272 | 1.00 | 24.56 | A |
| ATOM | 374 | CB | ASP | A | 61 | 8.898 | 29.256 | 11.115 | 1.00 | 26.52 | A |
| ATOM | 375 | CG | ASP | A | 61 | 7.409 | 29.109 | 10.831 | 1.00 | 29.60 | A |
| ATOM | 376 | OD1 | ASP | A | 61 | 7.043 | 28.271 | 9.982 | 1.00 | 27.94 | A |
| ATOM | 377 | OD2 | ASP | A | 61 | 6.604 | 29.829 | 11.456 | 1.00 | 33.11 | A |
| ATOM | 378 | C | ASP | A | 61 | 9.706 | 27.220 | 9.913 | 1.00 | 22.98 | A |
| ATOM | 379 | O | ASP | A | 61 | 10.176 | 27.832 | 8.954 | 1.00 | 20.73 | A |
| ATOM | 380 | N | VAL | A | 62 | 9.284 | 25.960 | 9.828 | 1.00 | 22.05 | A |
| ATOM | 381 | CA | VAL | A | 62 | 9.386 | 25.220 | 8.570 | 1.00 | 22.66 | A |
| ATOM | 382 | CB | VAL | A | 62 | 9.080 | 23.711 | 8.766 | 1.00 | 22.34 | A |
| ATOM | 383 | CG1 | VAL | A | 62 | 10.152 | 23.084 | 9.635 | 1.00 | 25.29 | A |
| ATOM | 384 | CG2 | VAL | A | 62 | 7.709 | 23.522 | 9.389 | 1.00 | 23.35 | A |
| ATOM | 385 | C | VAL | A | 62 | 8.547 | 25.742 | 7.409 | 1.00 | 21.38 | A |
| ATOM | 386 | O | VAL | A | 62 | 8.689 | 25.265 | 6.282 | 1.00 | 20.82 | A |
| ATOM | 387 | N | THR | A | 63 | 7.679 | 26.716 | 7.666 | 1.00 | 21.22 | A |
| ATOM | 388 | CA | THR | A | 63 | 6.869 | 27.284 | 6.590 | 1.00 | 23.09 | A |
| ATOM | 389 | CB | THR | A | 63 | 5.563 | 27.922 | 7.112 | 1.00 | 23.58 | A |
| ATOM | 390 | OG1 | THR | A | 63 | 5.871 | 29.094 | 7.874 | 1.00 | 23.88 | A |
| ATOM | 391 | CG2 | THR | A | 63 | 4.800 | 26.947 | 7.990 | 1.00 | 26.15 | A |
| ATOM | 392 | C | THR | A | 63 | 7.678 | 28.373 | 5.897 | 1.00 | 23.05 | A |
| ATOM | 393 | O | THR | A | 63 | 7.289 | 28.873 | 4.844 | 1.00 | 24.84 | A |
| ATOM | 394 | N | ASP | A | 64 | 8.817 | 28.718 | 6.493 | 1.00 | 24.62 | A |
| ATOM | 395 | CA | ASP | A | 64 | 9.702 | 29.770 | 5.985 | 1.00 | 23.42 | A |
| ATOM | 396 | CB | ASP | A | 64 | 10.035 | 30.723 | 7.136 | 1.00 | 24.98 | A |
| ATOM | 397 | CG | ASP | A | 64 | 10.908 | 31.892 | 6.713 | 1.00 | 25.38 | A |
| ATOM | 398 | OD1 | ASP | A | 64 | 11.618 | 31.805 | 5.686 | 1.00 | 25.28 | A |
| ATOM | 399 | OD2 | ASP | A | 64 | 10.888 | 32.906 | 7.440 | 1.00 | 28.51 | A |
| ATOM | 400 | C | ASP | A | 64 | 11.001 | 29.208 | 5.411 | 1.00 | 23.66 | A |
| ATOM | 401 | O | ASP | A | 64 | 11.866 | 28.762 | 6.168 | 1.00 | 21.49 | A |
| ATOM | 402 | N | SER | A | 65 | 11.150 | 29.257 | 4.087 | 1.00 | 21.05 | A |
| ATOM | 403 | CA | SER | A | 65 | 12.351 | 28.746 | 3.433 | 1.00 | 23.39 | A |
| ATOM | 404 | CB | SER | A | 65 | 12.223 | 28.855 | 1.907 | 1.00 | 23.66 | A |
| ATOM | 405 | OG | SER | A | 65 | 11.378 | 27.837 | 1.384 | 1.00 | 26.84 | A |
| ATOM | 406 | C | SER | A | 65 | 13.653 | 29.419 | 3.879 | 1.00 | 23.92 | A |
| ATOM | 407 | O | SER | A | 65 | 14.704 | 28.776 | 3.910 | 1.00 | 23.33 | A |
| ATOM | 408 | N | ASP | A | 66 | 13.593 | 30.706 | 4.213 | 1.00 | 24.49 | A |
| ATOM | 409 | CA | ASP | A | 66 | 14.797 | 31.409 | 4.651 | 1.00 | 24.69 | A |
| ATOM | 410 | CB | ASP | A | 66 | 14.601 | 32.929 | 4.594 | 1.00 | 27.84 | A |
| ATOM | 411 | CG | ASP | A | 66 | 14.473 | 33.448 | 3.171 | 1.00 | 30.01 | A |
| ATOM | 412 | OD1 | ASP | A | 66 | 15.236 | 32.983 | 2.297 | 1.00 | 32.24 | A |
| ATOM | 413 | OD2 | ASP | A | 66 | 13.620 | 34.328 | 2.930 | 1.00 | 33.58 | A |
| ATOM | 414 | C | ASP | A | 66 | 15.176 | 30.992 | 6.062 | 1.00 | 22.62 | A |
| ATOM | 415 | O | ASP | A | 66 | 16.358 | 30.874 | 6.385 | 1.00 | 23.05 | A |
| ATOM | 416 | N | ALA | A | 67 | 14.168 | 30.774 | 6.900 | 1.00 | 19.93 | A |
| ATOM | 417 | CA | ALA | A | 67 | 14.396 | 30.346 | 8.272 | 1.00 | 20.15 | A |
| ATOM | 418 | CB | ALA | A | 67 | 13.083 | 30.332 | 9.050 | 1.00 | 19.42 | A |
| ATOM | 419 | C | ALA | A | 67 | 15.022 | 28.949 | 8.270 | 1.00 | 19.13 | A |
| ATOM | 420 | O | ALA | A | 67 | 15.795 | 28.602 | 9.168 | 1.00 | 18.73 | A |
| ATOM | 421 | N | VAL | A | 68 | 14.682 | 28.152 | 7.260 | 1.00 | 18.32 | A |
| ATOM | 422 | CA | VAL | A | 68 | 15.227 | 26.804 | 7.144 | 1.00 | 17.51 | A |
| ATOM | 423 | CB | VAL | A | 68 | 14.439 | 25.959 | 6.111 | 1.00 | 17.27 | A |
| ATOM | 424 | CG1 | VAL | A | 68 | 15.212 | 24.699 | 5.778 | 1.00 | 15.53 | A |
| ATOM | 425 | CG2 | VAL | A | 68 | 13.058 | 25.609 | 6.671 | 1.00 | 15.15 | A |
| ATOM | 426 | C | VAL | A | 68 | 16.677 | 26.906 | 6.703 | 1.00 | 18.82 | A |
| ATOM | 427 | O | VAL | A | 68 | 17.549 | 26.190 | 7.205 | 1.00 | 19.23 | A |
| ATOM | 428 | N | ASP | A | 69 | 16.932 | 27.806 | 5.764 | 1.00 | 19.11 | A |
| ATOM | 429 | CA | ASP | A | 69 | 18.279 | 28.020 | 5.259 | 1.00 | 20.67 | A |
| ATOM | 430 | CB | ASP | A | 69 | 18.261 | 29.095 | 4.175 | 1.00 | 22.86 | A |
| ATOM | 431 | CG | ASP | A | 69 | 19.620 | 29.317 | 3.554 | 1.00 | 24.39 | A |
| ATOM | 432 | OD1 | ASP | A | 69 | 19.777 | 30.313 | 2.818 | 1.00 | 27.00 | A |
| ATOM | 433 | OD2 | ASP | A | 69 | 20.527 | 28.491 | 3.787 | 1.00 | 22.39 | A |
| ATOM | 434 | C | ASP | A | 69 | 19.214 | 28.455 | 6.392 | 1.00 | 20.57 | A |
| ATOM | 435 | O | ASP | A | 69 | 20.356 | 27.996 | 6.478 | 1.00 | 20.97 | A |

Figure 1 (suite 5)

| | | | | | | | | | | | |
|---|---|---|---|---|---|---|---|---|---|---|---|
| ATOM | 436 | N | ARG | A | 70 | 18.723 | 29.340 | 7.256 | 1.00 | 20.03 | A |
| ATOM | 437 | CA | ARG | A | 70 | 19.520 | 29.834 | 8.375 | 1.00 | 21.17 | A |
| ATOM | 438 | CB | ARG | A | 70 | 18.825 | 31.020 | 9.054 | 1.00 | 22.17 | A |
| ATOM | 439 | CG | ARG | A | 70 | 18.632 | 32.242 | 8.158 | 1.00 | 28.85 | A |
| ATOM | 440 | CD | ARG | A | 70 | 18.312 | 33.485 | 8.977 | 1.00 | 28.60 | A |
| ATOM | 441 | NE | ARG | A | 70 | 17.029 | 33.405 | 9.672 | 1.00 | 33.05 | A |
| ATOM | 442 | CZ | ARG | A | 70 | 15.851 | 33.644 | 9.103 | 1.00 | 34.08 | A |
| ATOM | 443 | NH1 | ARG | A | 70 | 15.786 | 33.980 | 7.818 | 1.00 | 35.58 | A |
| ATOM | 444 | NH2 | ARG | A | 70 | 14.737 | 33.556 | 9.819 | 1.00 | 35.99 | A |
| ATOM | 445 | C | ARG | A | 70 | 19.770 | 28.740 | 9.415 | 1.00 | 19.83 | A |
| ATOM | 446 | O | ARG | A | 70 | 20.879 | 28.615 | 9.938 | 1.00 | 19.57 | A |
| ATOM | 447 | N | ALA | A | 71 | 18.734 | 27.963 | 9.719 | 1.00 | 17.49 | A |
| ATOM | 448 | CA | ALA | A | 71 | 18.842 | 26.889 | 10.700 | 1.00 | 17.22 | A |
| ATOM | 449 | CB | ALA | A | 71 | 17.506 | 26.168 | 10.843 | 1.00 | 16.33 | A |
| ATOM | 450 | C | ALA | A | 71 | 19.932 | 25.912 | 10.288 | 1.00 | 17.08 | A |
| ATOM | 451 | O | ALA | A | 71 | 20.829 | 25.604 | 11.080 | 1.00 | 19.16 | A |
| ATOM | 452 | N | PHE | A | 72 | 19.866 | 25.435 | 9.048 | 1.00 | 16.74 | A |
| ATOM | 453 | CA | PHE | A | 72 | 20.866 | 24.504 | 8.545 | 1.00 | 16.40 | A |
| ATOM | 454 | CB | PHE | A | 72 | 20.560 | 24.085 | 7.107 | 1.00 | 13.69 | A |
| ATOM | 455 | CG | PHE | A | 72 | 19.677 | 22.868 | 7.006 | 1.00 | 14.97 | A |
| ATOM | 456 | CD1 | PHE | A | 72 | 18.337 | 22.931 | 7.367 | 1.00 | 11.79 | A |
| ATOM | 457 | CD2 | PHE | A | 72 | 20.194 | 21.655 | 6.549 | 1.00 | 12.86 | A |
| ATOM | 458 | CE1 | PHE | A | 72 | 17.516 | 21.797 | 7.283 | 1.00 | 11.80 | A |
| ATOM | 459 | CE2 | PHE | A | 72 | 19.382 | 20.516 | 6.463 | 1.00 | 13.73 | A |
| ATOM | 460 | CZ | PHE | A | 72 | 18.040 | 20.591 | 6.827 | 1.00 | 11.88 | A |
| ATOM | 461 | C | PHE | A | 72 | 22.272 | 25.084 | 8.594 | 1.00 | 18.02 | A |
| ATOM | 462 | O | PHE | A | 72 | 23.209 | 24.407 | 9.017 | 1.00 | 16.91 | A |
| ATOM | 463 | N | THR | A | 73 | 22.421 | 26.328 | 8.148 | 1.00 | 17.98 | A |
| ATOM | 464 | CA | THR | A | 73 | 23.730 | 26.975 | 8.151 | 1.00 | 18.36 | A |
| ATOM | 465 | CB | THR | A | 73 | 23.647 | 28.401 | 7.573 | 1.00 | 18.81 | A |
| ATOM | 466 | OG1 | THR | A | 73 | 23.338 | 28.326 | 6.176 | 1.00 | 20.59 | A |
| ATOM | 467 | CG2 | THR | A | 73 | 24.977 | 29.131 | 7.756 | 1.00 | 20.50 | A |
| ATOM | 468 | C | THR | A | 73 | 24.313 | 27.039 | 9.560 | 1.00 | 17.95 | A |
| ATOM | 469 | O | THR | A | 73 | 25.499 | 26.770 | 9.756 | 1.00 | 18.54 | A |
| ATOM | 470 | N | ALA | A | 74 | 23.474 | 27.390 | 10.532 | 1.00 | 18.30 | A |
| ATOM | 471 | CA | ALA | A | 74 | 23.892 | 27.483 | 11.928 | 1.00 | 18.73 | A |
| ATOM | 472 | CB | ALA | A | 74 | 22.746 | 27.992 | 12.778 | 1.00 | 20.77 | A |
| ATOM | 473 | C | ALA | A | 74 | 24.358 | 26.116 | 12.443 | 1.00 | 20.68 | A |
| ATOM | 474 | O | ALA | A | 74 | 25.373 | 26.013 | 13.142 | 1.00 | 18.58 | A |
| ATOM | 475 | N | VAL | A | 75 | 23.612 | 25.069 | 12.096 | 1.00 | 20.24 | A |
| ATOM | 476 | CA | VAL | A | 75 | 23.963 | 23.719 | 12.513 | 1.00 | 19.00 | A |
| ATOM | 477 | CB | VAL | A | 75 | 22.852 | 22.698 | 12.121 | 1.00 | 18.18 | A |
| ATOM | 478 | CG1 | VAL | A | 75 | 23.346 | 21.264 | 12.337 | 1.00 | 15.28 | A |
| ATOM | 479 | CG2 | VAL | A | 75 | 21.603 | 22.951 | 12.960 | 1.00 | 15.82 | A |
| ATOM | 480 | C | VAL | A | 75 | 25.282 | 23.297 | 11.867 | 1.00 | 20.46 | A |
| ATOM | 481 | O | VAL | A | 75 | 26.139 | 22.701 | 12.520 | 1.00 | 19.30 | A |
| ATOM | 482 | N | GLU | A | 76 | 25.436 | 23.616 | 10.587 | 1.00 | 21.42 | A |
| ATOM | 483 | CA | GLU | A | 76 | 26.637 | 23.270 | 9.838 | 1.00 | 24.10 | A |
| ATOM | 484 | CB | GLU | A | 76 | 26.511 | 23.764 | 8.396 | 1.00 | 26.69 | A |
| ATOM | 485 | CG | GLU | A | 76 | 25.234 | 23.305 | 7.699 | 1.00 | 31.10 | A |
| ATOM | 486 | CD | GLU | A | 76 | 25.003 | 23.985 | 6.357 | 1.00 | 31.89 | A |
| ATOM | 487 | OE1 | GLU | A | 76 | 25.205 | 25.215 | 6.261 | 1.00 | 33.39 | A |
| ATOM | 488 | OE2 | GLU | A | 76 | 24.606 | 23.292 | 5.398 | 1.00 | 33.86 | A |
| ATOM | 489 | C | GLU | A | 76 | 27.886 | 23.874 | 10.476 | 1.00 | 25.12 | A |
| ATOM | 490 | O | GLU | A | 76 | 28.927 | 23.221 | 10.562 | 1.00 | 25.90 | A |
| ATOM | 491 | N | GLU | A | 77 | 27.778 | 25.121 | 10.917 | 1.00 | 25.94 | A |
| ATOM | 492 | CA | GLU | A | 77 | 28.905 | 25.802 | 11.539 | 1.00 | 28.63 | A |
| ATOM | 493 | CB | GLU | A | 77 | 28.605 | 27.294 | 11.692 | 1.00 | 29.79 | A |
| ATOM | 494 | CG | GLU | A | 77 | 28.182 | 27.970 | 10.396 | 1.00 | 34.29 | A |
| ATOM | 495 | CD | GLU | A | 77 | 28.188 | 29.485 | 10.490 | 1.00 | 36.82 | A |
| ATOM | 496 | OE1 | GLU | A | 77 | 27.640 | 30.028 | 11.472 | 1.00 | 38.81 | A |
| ATOM | 497 | OE2 | GLU | A | 77 | 28.736 | 30.132 | 9.572 | 1.00 | 39.93 | A |
| ATOM | 498 | C | GLU | A | 77 | 29.184 | 25.185 | 12.906 | 1.00 | 27.64 | A |
| ATOM | 499 | O | GLU | A | 77 | 30.330 | 25.075 | 13.333 | 1.00 | 28.46 | A |
| ATOM | 500 | N | HIS | A | 78 | 28.124 | 24.764 | 13.579 | 1.00 | 27.08 | A |
| ATOM | 501 | CA | HIS | A | 78 | 28.256 | 24.172 | 14.896 | 1.00 | 25.22 | A |
| ATOM | 502 | CB | HIS | A | 78 | 26.899 | 24.156 | 15.593 | 1.00 | 26.54 | A |
| ATOM | 503 | CG | HIS | A | 78 | 26.906 | 23.435 | 16.904 | 1.00 | 27.50 | A |
| ATOM | 504 | CD2 | HIS | A | 78 | 26.419 | 22.221 | 17.254 | 1.00 | 28.11 | A |
| ATOM | 505 | ND1 | HIS | A | 78 | 27.487 | 23.959 | 18.038 | 1.00 | 28.93 | A |
| ATOM | 506 | CE1 | HIS | A | 78 | 27.356 | 23.098 | 19.033 | 1.00 | 29.00 | A |
| ATOM | 507 | NE2 | HIS | A | 78 | 26.712 | 22.036 | 18.583 | 1.00 | 27.73 | A |
| ATOM | 508 | C | HIS | A | 78 | 28.832 | 22.758 | 14.912 | 1.00 | 24.24 | A |

Figure 1 (suite 6)

| | | | | | | | | | | | |
|---|---|---|---|---|---|---|---|---|---|---|---|
| ATOM | 509 | O | HIS | A | 78 | 29.822 | 22.492 | 15.603 | 1.00 | 23.91 | A |
| ATOM | 510 | N | GLN | A | 79 | 28.220 | 21.856 | 14.150 | 1.00 | 21.56 | A |
| ATOM | 511 | CA | GLN | A | 79 | 28.635 | 20.458 | 14.144 | 1.00 | 20.83 | A |
| ATOM | 512 | CB | GLN | A | 79 | 27.569 | 19.631 | 14.853 | 1.00 | 21.56 | A |
| ATOM | 513 | CG | GLN | A | 79 | 26.211 | 19.746 | 14.172 | 1.00 | 18.06 | A |
| ATOM | 514 | CD | GLN | A | 79 | 25.110 | 19.038 | 14.918 | 1.00 | 16.50 | A |
| ATOM | 515 | OE1 | GLN | A | 79 | 24.873 | 19.295 | 16.097 | 1.00 | 18.79 | A |
| ATOM | 516 | NE2 | GLN | A | 79 | 24.416 | 18.145 | 14.228 | 1.00 | 13.17 | A |
| ATOM | 517 | C | GLN | A | 79 | 28.902 | 19.816 | 12.792 | 1.00 | 21.02 | A |
| ATOM | 518 | O | GLN | A | 79 | 28.970 | 18.591 | 12.705 | 1.00 | 20.74 | A |
| ATOM | 519 | N | GLY | A | 80 | 29.039 | 20.620 | 11.744 | 1.00 | 20.26 | A |
| ATOM | 520 | CA | GLY | A | 80 | 29.293 | 20.063 | 10.428 | 1.00 | 20.31 | A |
| ATOM | 521 | C | GLY | A | 80 | 28.017 | 19.822 | 9.633 | 1.00 | 20.84 | A |
| ATOM | 522 | O | GLY | A | 80 | 26.926 | 19.799 | 10.201 | 1.00 | 20.12 | A |
| ATOM | 523 | N | PRO | A | 81 | 28.131 | 19.632 | 8.310 | 1.00 | 20.29 | A |
| ATOM | 524 | CD | PRO | A | 81 | 29.410 | 19.551 | 7.582 | 1.00 | 21.49 | A |
| ATOM | 525 | CA | PRO | A | 81 | 27.003 | 19.389 | 7.405 | 1.00 | 21.60 | A |
| ATOM | 526 | CB | PRO | A | 81 | 27.687 | 18.903 | 6.131 | 1.00 | 21.29 | A |
| ATOM | 527 | CG | PRO | A | 81 | 28.974 | 19.650 | 6.145 | 1.00 | 23.93 | A |
| ATOM | 528 | C | PRO | A | 81 | 26.023 | 18.354 | 7.957 | 1.00 | 21.00 | A |
| ATOM | 529 | O | PRO | A | 81 | 26.429 | 17.364 | 8.573 | 1.00 | 20.36 | A |
| ATOM | 530 | N | VAL | A | 82 | 24.734 | 18.590 | 7.741 | 1.00 | 18.90 | A |
| ATOM | 531 | CA | VAL | A | 82 | 23.720 | 17.663 | 8.208 | 1.00 | 18.43 | A |
| ATOM | 532 | CB | VAL | A | 82 | 22.314 | 18.275 | 8.102 | 1.00 | 18.67 | A |
| ATOM | 533 | CG1 | VAL | A | 82 | 21.273 | 17.249 | 8.524 | 1.00 | 18.92 | A |
| ATOM | 534 | CG2 | VAL | A | 82 | 22.220 | 19.508 | 8.991 | 1.00 | 19.76 | A |
| ATOM | 535 | C | VAL | A | 82 | 23.783 | 16.381 | 7.378 | 1.00 | 18.11 | A |
| ATOM | 536 | O | VAL | A | 82 | 23.643 | 16.404 | 6.151 | 1.00 | 16.71 | A |
| ATOM | 537 | N | GLU | A | 83 | 24.021 | 15.270 | 8.066 | 1.00 | 15.92 | A |
| ATOM | 538 | CA | GLU | A | 83 | 24.115 | 13.960 | 7.440 | 1.00 | 15.29 | A |
| ATOM | 539 | CB | GLU | A | 83 | 25.271 | 13.167 | 8.057 | 1.00 | 15.19 | A |
| ATOM | 540 | CG | GLU | A | 83 | 26.666 | 13.713 | 7.785 | 1.00 | 14.89 | A |
| ATOM | 541 | CD | GLU | A | 83 | 27.722 | 12.907 | 8.530 | 1.00 | 15.73 | A |
| ATOM | 542 | OE1 | GLU | A | 83 | 27.765 | 12.999 | 9.770 | 1.00 | 13.86 | A |
| ATOM | 543 | OE2 | GLU | A | 83 | 28.484 | 12.170 | 7.878 | 1.00 | 14.54 | A |
| ATOM | 544 | C | GLU | A | 83 | 22.822 | 13.175 | 7.654 | 1.00 | 14.11 | A |
| ATOM | 545 | O | GLU | A | 83 | 22.420 | 12.381 | 6.813 | 1.00 | 12.20 | A |
| ATOM | 546 | N | VAL | A | 84 | 22.200 | 13.395 | 8.804 | 1.00 | 13.30 | A |
| ATOM | 547 | CA | VAL | A | 84 | 20.969 | 12.721 | 9.170 | 1.00 | 12.09 | A |
| ATOM | 548 | CB | VAL | A | 84 | 21.176 | 11.807 | 10.409 | 1.00 | 14.87 | A |
| ATOM | 549 | CG1 | VAL | A | 84 | 19.839 | 11.210 | 10.858 | 1.00 | 12.03 | A |
| ATOM | 550 | CG2 | VAL | A | 84 | 22.178 | 10.691 | 10.074 | 1.00 | 13.38 | A |
| ATOM | 551 | C | VAL | A | 84 | 19.906 | 13.760 | 9.501 | 1.00 | 13.92 | A |
| ATOM | 552 | O | VAL | A | 84 | 20.044 | 14.521 | 10.463 | 1.00 | 11.66 | A |
| ATOM | 553 | N | LEU | A | 85 | 18.849 | 13.792 | 8.695 | 1.00 | 11.97 | A |
| ATOM | 554 | CA | LEU | A | 85 | 17.754 | 14.729 | 8.911 | 1.00 | 11.53 | A |
| ATOM | 555 | CB | LEU | A | 85 | 17.426 | 15.460 | 7.600 | 1.00 | 12.93 | A |
| ATOM | 556 | CG | LEU | A | 85 | 16.073 | 16.191 | 7.551 | 1.00 | 14.43 | A |
| ATOM | 557 | CD1 | LEU | A | 85 | 16.062 | 17.326 | 8.563 | 1.00 | 14.85 | A |
| ATOM | 558 | CD2 | LEU | A | 85 | 15.820 | 16.725 | 6.136 | 1.00 | 14.06 | A |
| ATOM | 559 | C | LEU | A | 85 | 16.508 | 14.002 | 9.412 | 1.00 | 12.69 | A |
| ATOM | 560 | O | LEU | A | 85 | 16.028 | 13.059 | 8.773 | 1.00 | 11.22 | A |
| ATOM | 561 | N | VAL | A | 86 | 15.995 | 14.420 | 10.560 | 1.00 | 10.53 | A |
| ATOM | 562 | CA | VAL | A | 86 | 14.782 | 13.827 | 11.099 | 1.00 | 11.06 | A |
| ATOM | 563 | CB | VAL | A | 86 | 14.970 | 13.314 | 12.545 | 1.00 | 11.32 | A |
| ATOM | 564 | CG1 | VAL | A | 86 | 13.676 | 12.691 | 13.042 | 1.00 | 11.37 | A |
| ATOM | 565 | CG2 | VAL | A | 86 | 16.107 | 12.275 | 12.598 | 1.00 | 11.11 | A |
| ATOM | 566 | C | VAL | A | 86 | 13.721 | 14.932 | 11.085 | 1.00 | 11.88 | A |
| ATOM | 567 | O | VAL | A | 86 | 13.781 | 15.888 | 11.871 | 1.00 | 13.93 | A |
| ATOM | 568 | N | SER | A | 87 | 12.763 | 14.804 | 10.173 | 1.00 | 10.34 | A |
| ATOM | 569 | CA | SER | A | 87 | 11.691 | 15.785 | 10.037 | 1.00 | 11.59 | A |
| ATOM | 570 | CB | SER | A | 87 | 11.402 | 16.006 | 8.546 | 1.00 | 11.36 | A |
| ATOM | 571 | OG | SER | A | 87 | 10.310 | 16.888 | 8.348 | 1.00 | 13.45 | A |
| ATOM | 572 | C | SER | A | 87 | 10.417 | 15.379 | 10.779 | 1.00 | 14.15 | A |
| ATOM | 573 | O | SER | A | 87 | 9.715 | 14.445 | 10.376 | 1.00 | 12.78 | A |
| ATOM | 574 | N | ASN | A | 88 | 10.132 | 16.073 | 11.876 | 1.00 | 17.34 | A |
| ATOM | 575 | CA | ASN | A | 88 | 8.937 | 15.813 | 12.672 | 1.00 | 21.45 | A |
| ATOM | 576 | CB | ASN | A | 88 | 9.210 | 16.079 | 14.152 | 1.00 | 22.49 | A |
| ATOM | 577 | CG | ASN | A | 88 | 10.055 | 14.999 | 14.799 | 1.00 | 23.19 | A |
| ATOM | 578 | OD1 | ASN | A | 88 | 9.565 | 13.907 | 15.103 | 1.00 | 26.14 | A |
| ATOM | 579 | ND2 | ASN | A | 88 | 11.336 | 15.292 | 15.006 | 1.00 | 23.94 | A |
| ATOM | 580 | C | ASN | A | 88 | 7.809 | 16.735 | 12.197 | 1.00 | 24.25 | A |
| ATOM | 581 | O | ASN | A | 88 | 8.061 | 17.840 | 11.716 | 1.00 | 25.95 | A |

Figure 1 (suite 7)

| | | | | | | | | | | | |
|---|---|---|---|---|---|---|---|---|---|---|---|
| ATOM | 582 | N | ALA | A | 89 | 6.569 | 16.275 | 12.335 | 1.00 | 26.98 | A |
| ATOM | 583 | CA | ALA | A | 89 | 5.407 | 17.058 | 11.918 | 1.00 | 30.14 | A |
| ATOM | 584 | CB | ALA | A | 89 | 4.124 | 16.351 | 12.344 | 1.00 | 30.27 | A |
| ATOM | 585 | C | ALA | A | 89 | 5.431 | 18.473 | 12.494 | 1.00 | 31.94 | A |
| ATOM | 586 | O | ALA | A | 89 | 5.395 | 18.656 | 13.709 | 1.00 | 32.94 | A |
| ATOM | 587 | N | GLY | A | 90 | 5.490 | 19.467 | 11.613 | 1.00 | 33.78 | A |
| ATOM | 588 | CA | GLY | A | 90 | 5.505 | 20.848 | 12.053 | 1.00 | 36.47 | A |
| ATOM | 589 | C | GLY | A | 90 | 4.339 | 21.168 | 12.975 | 1.00 | 39.47 | A |
| ATOM | 590 | O | GLY | A | 90 | 4.509 | 21.847 | 13.990 | 1.00 | 41.17 | A |
| ATOM | 591 | N | LEU | A | 91 | 3.152 | 20.680 | 12.626 | 1.00 | 39.03 | A |
| ATOM | 592 | CA | LEU | A | 91 | 1.960 | 20.917 | 13.431 | 1.00 | 40.84 | A |
| ATOM | 593 | CB | LEU | A | 91 | 0.917 | 21.696 | 12.623 | 1.00 | 40.30 | A |
| ATOM | 594 | CG | LEU | A | 91 | 1.213 | 23.161 | 12.287 | 1.00 | 41.14 | A |
| ATOM | 595 | CD1 | LEU | A | 91 | 0.144 | 23.698 | 11.343 | 1.00 | 40.25 | A |
| ATOM | 596 | CD2 | LEU | A | 91 | 1.255 | 23.980 | 13.568 | 1.00 | 40.55 | A |
| ATOM | 597 | C | LEU | A | 91 | 1.347 | 19.607 | 13.909 | 1.00 | 42.66 | A |
| ATOM | 598 | O | LEU | A | 91 | 1.375 | 18.601 | 13.199 | 1.00 | 42.33 | A |
| ATOM | 599 | N | SER | A | 92 | 0.793 | 19.627 | 15.118 | 1.00 | 45.25 | A |
| ATOM | 600 | CA | SER | A | 92 | 0.158 | 18.446 | 15.696 | 1.00 | 46.92 | A |
| ATOM | 601 | CB | SER | A | 92 | 0.690 | 18.191 | 17.112 | 1.00 | 48.42 | A |
| ATOM | 602 | OG | SER | A | 92 | 2.073 | 17.884 | 17.099 | 1.00 | 50.20 | A |
| ATOM | 603 | C | SER | A | 92 | -1.357 | 18.629 | 15.750 | 1.00 | 48.24 | A |
| ATOM | 604 | O | SER | A | 92 | -1.856 | 19.753 | 15.846 | 1.00 | 47.40 | A |
| ATOM | 605 | N | ALA | A | 93 | -2.085 | 17.518 | 15.691 | 1.00 | 49.52 | A |
| ATOM | 606 | CA | ALA | A | 93 | -3.542 | 17.558 | 15.735 | 1.00 | 50.21 | A |
| ATOM | 607 | CB | ALA | A | 93 | -4.112 | 16.221 | 15.283 | 1.00 | 50.72 | A |
| ATOM | 608 | C | ALA | A | 93 | -4.031 | 17.886 | 17.143 | 1.00 | 51.15 | A |
| ATOM | 609 | O | ALA | A | 93 | -4.163 | 19.055 | 17.509 | 1.00 | 51.49 | A |
| ATOM | 610 | N | ARG | A | 99 | -15.022 | 21.456 | 11.947 | 1.00 | 42.43 | A |
| ATOM | 611 | CA | ARG | A | 99 | -15.163 | 21.174 | 10.523 | 1.00 | 42.58 | A |
| ATOM | 612 | CB | ARG | A | 99 | -16.433 | 21.837 | 9.982 | 1.00 | 45.29 | A |
| ATOM | 613 | CG | ARG | A | 99 | -16.764 | 21.511 | 8.529 | 1.00 | 48.50 | A |
| ATOM | 614 | CD | ARG | A | 99 | -16.952 | 20.017 | 8.313 | 1.00 | 51.66 | A |
| ATOM | 615 | NE | ARG | A | 99 | -15.681 | 19.305 | 8.195 | 1.00 | 54.40 | A |
| ATOM | 616 | CZ | ARG | A | 99 | -15.570 | 17.982 | 8.120 | 1.00 | 55.29 | A |
| ATOM | 617 | NH1 | ARG | A | 99 | -16.656 | 17.222 | 8.154 | 1.00 | 56.59 | A |
| ATOM | 618 | NH2 | ARG | A | 99 | -14.376 | 17.417 | 8.001 | 1.00 | 55.54 | A |
| ATOM | 619 | C | ARG | A | 99 | -13.939 | 21.684 | 9.769 | 1.00 | 40.62 | A |
| ATOM | 620 | O | ARG | A | 99 | -13.337 | 22.692 | 10.147 | 1.00 | 41.33 | A |
| ATOM | 621 | N | MET | A | 100 | -13.578 | 20.985 | 8.699 | 1.00 | 37.61 | A |
| ATOM | 622 | CA | MET | A | 100 | -12.418 | 21.349 | 7.902 | 1.00 | 35.35 | A |
| ATOM | 623 | CB | MET | A | 100 | -11.981 | 20.154 | 7.050 | 1.00 | 35.87 | A |
| ATOM | 624 | CG | MET | A | 100 | -10.646 | 20.337 | 6.341 | 1.00 | 34.72 | A |
| ATOM | 625 | SD | MET | A | 100 | -9.278 | 20.698 | 7.479 | 1.00 | 34.45 | A |
| ATOM | 626 | CE | MET | A | 100 | -9.288 | 19.207 | 8.483 | 1.00 | 32.65 | A |
| ATOM | 627 | C | MET | A | 100 | -12.690 | 22.552 | 7.007 | 1.00 | 34.40 | A |
| ATOM | 628 | O | MET | A | 100 | -13.716 | 22.616 | 6.331 | 1.00 | 35.23 | A |
| ATOM | 629 | N | THR | A | 101 | -11.763 | 23.506 | 7.014 | 1.00 | 31.88 | A |
| ATOM | 630 | CA | THR | A | 101 | -11.878 | 24.708 | 6.197 | 1.00 | 29.47 | A |
| ATOM | 631 | CB | THR | A | 101 | -11.960 | 25.985 | 7.059 | 1.00 | 28.91 | A |
| ATOM | 632 | OG1 | THR | A | 101 | -10.699 | 26.203 | 7.703 | 1.00 | 29.44 | A |
| ATOM | 633 | CG2 | THR | A | 101 | -13.045 | 25.854 | 8.113 | 1.00 | 28.67 | A |
| ATOM | 634 | C | THR | A | 101 | -10.639 | 24.820 | 5.319 | 1.00 | 28.93 | A |
| ATOM | 635 | O | THR | A | 101 | -9.646 | 24.121 | 5.545 | 1.00 | 26.76 | A |
| ATOM | 636 | N | GLU | A | 102 | -10.699 | 25.703 | 4.327 | 1.00 | 26.66 | A |
| ATOM | 637 | CA | GLU | A | 102 | -9.579 | 25.915 | 3.416 | 1.00 | 27.19 | A |
| ATOM | 638 | CB | GLU | A | 102 | -9.945 | 26.954 | 2.352 | 1.00 | 27.20 | A |
| ATOM | 639 | CG | GLU | A | 102 | -8.916 | 27.084 | 1.234 | 1.00 | 27.26 | A |
| ATOM | 640 | CD | GLU | A | 102 | -9.020 | 28.406 | 0.491 | 1.00 | 30.25 | A |
| ATOM | 641 | OE1 | GLU | A | 102 | -8.422 | 28.535 | -0.599 | 1.00 | 27.88 | A |
| ATOM | 642 | OE2 | GLU | A | 102 | -9.691 | 29.324 | 1.003 | 1.00 | 32.00 | A |
| ATOM | 643 | C | GLU | A | 102 | -8.349 | 26.403 | 4.181 | 1.00 | 27.41 | A |
| ATOM | 644 | O | GLU | A | 102 | -7.228 | 25.967 | 3.910 | 1.00 | 26.58 | A |
| ATOM | 645 | N | GLU | A | 103 | -8.566 | 27.319 | 5.124 | 1.00 | 26.59 | A |
| ATOM | 646 | CA | GLU | A | 103 | -7.479 | 27.875 | 5.929 | 1.00 | 26.49 | A |
| ATOM | 647 | CB | GLU | A | 103 | -8.021 | 28.942 | 6.890 | 1.00 | 29.18 | A |
| ATOM | 648 | CG | GLU | A | 103 | -6.956 | 29.574 | 7.790 | 1.00 | 34.00 | A |
| ATOM | 649 | CD | GLU | A | 103 | -7.486 | 30.753 | 8.598 | 1.00 | 40.53 | A |
| ATOM | 650 | OE1 | GLU | A | 103 | -7.941 | 31.743 | 7.984 | 1.00 | 42.32 | A |
| ATOM | 651 | OE2 | GLU | A | 103 | -7.445 | 30.693 | 9.848 | 1.00 | 42.51 | A |
| ATOM | 652 | C | GLU | A | 103 | -6.764 | 26.781 | 6.728 | 1.00 | 24.46 | A |
| ATOM | 653 | O | GLU | A | 103 | -5.541 | 26.664 | 6.676 | 1.00 | 23.77 | A |
| ATOM | 654 | N | LYS | A | 104 | -7.535 | 25.989 | 7.464 | 1.00 | 23.12 | A |

Figure 1 (suite 8)

| | | | | | | | | | | | |
|---|---|---|---|---|---|---|---|---|---|---|---|
| ATOM | 655 | CA | LYS | A | 104 | -6.980 | 24.903 | 8.267 | 1.00 | 24.53 | A |
| ATOM | 656 | CB | LYS | A | 104 | -8.095 | 24.206 | 9.056 | 1.00 | 24.75 | A |
| ATOM | 657 | CG | LYS | A | 104 | -8.826 | 25.113 | 10.031 | 0.00 | 26.36 | A |
| ATOM | 658 | CD | LYS | A | 104 | -9.913 | 24.356 | 10.774 | 1.00 | 28.49 | A |
| ATOM | 659 | CE | LYS | A | 104 | -10.648 | 25.262 | 11.747 | 0.00 | 27.80 | A |
| ATOM | 660 | NZ | LYS | A | 104 | -11.720 | 24.533 | 12.479 | 0.00 | 28.01 | A |
| ATOM | 661 | C | LYS | A | 104 | -6.246 | 23.878 | 7.403 | 1.00 | 23.16 | A |
| ATOM | 662 | O | LYS | A | 104 | -5.187 | 23.378 | 7.784 | 1.00 | 23.47 | A |
| ATOM | 663 | N | PHE | A | 105 | -6.806 | 23.574 | 6.235 | 1.00 | 22.27 | A |
| ATOM | 664 | CA | PHE | A | 105 | -6.201 | 22.604 | 5.334 | 1.00 | 20.31 | A |
| ATOM | 665 | CB | PHE | A | 105 | -7.133 | 22.313 | 4.153 | 1.00 | 19.01 | A |
| ATOM | 666 | CG | PHE | A | 105 | -6.661 | 21.190 | 3.271 | 1.00 | 17.42 | A |
| ATOM | 667 | CD1 | PHE | A | 105 | -7.019 | 19.873 | 3.548 | 1.00 | 16.60 | A |
| ATOM | 668 | CD2 | PHE | A | 105 | -5.849 | 21.446 | 2.177 | 1.00 | 17.49 | A |
| ATOM | 669 | CE1 | PHE | A | 105 | -6.570 | 18.828 | 2.741 | 1.00 | 18.49 | A |
| ATOM | 670 | CE2 | PHE | A | 105 | -5.394 | 20.407 | 1.361 | 1.00 | 17.58 | A |
| ATOM | 671 | CZ | PHE | A | 105 | -5.754 | 19.100 | 1.644 | 1.00 | 17.57 | A |
| ATOM | 672 | C | PHE | A | 105 | -4.861 | 23.099 | 4.801 | 1.00 | 19.49 | A |
| ATOM | 673 | O | PHE | A | 105 | -3.867 | 22.370 | 4.813 | 1.00 | 17.60 | A |
| ATOM | 674 | N | GLU | A | 106 | -4.831 | 24.336 | 4.320 | 1.00 | 19.63 | A |
| ATOM | 675 | CA | GLU | A | 106 | -3.589 | 24.873 | 3.783 | 1.00 | 19.94 | A |
| ATOM | 676 | CB | GLU | A | 106 | -3.845 | 26.190 | 3.043 | 1.00 | 21.71 | A |
| ATOM | 677 | CG | GLU | A | 106 | -4.851 | 26.066 | 1.895 | 1.00 | 27.01 | A |
| ATOM | 678 | CD | GLU | A | 106 | -4.699 | 27.168 | 0.862 | 1.00 | 28.08 | A |
| ATOM | 679 | OE1 | GLU | A | 106 | -3.859 | 27.023 | -0.052 | 1.00 | 29.07 | A |
| ATOM | 680 | OE2 | GLU | A | 106 | -5.414 | 28.187 | 0.974 | 1.00 | 29.26 | A |
| ATOM | 681 | C | GLU | A | 106 | -2.513 | 25.073 | 4.858 | 1.00 | 19.81 | A |
| ATOM | 682 | O | GLU | A | 106 | -1.322 | 24.921 | 4.575 | 1.00 | 21.01 | A |
| ATOM | 683 | N | LYS | A | 107 | -2.921 | 25.410 | 6.078 | 1.00 | 18.30 | A |
| ATOM | 684 | CA | LYS | A | 107 | -1.954 | 25.606 | 7.160 | 1.00 | 20.96 | A |
| ATOM | 685 | CB | LYS | A | 107 | -2.643 | 26.119 | 8.428 | 1.00 | 20.90 | A |
| ATOM | 686 | CG | LYS | A | 107 | -3.085 | 27.565 | 8.354 | 0.00 | 24.30 | A |
| ATOM | 687 | CD | LYS | A | 107 | -3.544 | 28.056 | 9.714 | 1.00 | 28.51 | A |
| ATOM | 688 | CE | LYS | A | 107 | -3.952 | 29.518 | 9.661 | 0.00 | 27.13 | A |
| ATOM | 689 | NZ | LYS | A | 107 | -4.407 | 30.013 | 10.986 | 0.00 | 27.58 | A |
| ATOM | 690 | C | LYS | A | 107 | -1.230 | 24.293 | 7.470 | 1.00 | 19.51 | A |
| ATOM | 691 | O | LYS | A | 107 | 0.000 | 24.251 | 7.534 | 1.00 | 21.15 | A |
| ATOM | 692 | N | VAL | A | 108 | -2.000 | 23.225 | 7.655 | 1.00 | 18.30 | A |
| ATOM | 693 | CA | VAL | A | 108 | -1.418 | 21.921 | 7.945 | 1.00 | 18.91 | A |
| ATOM | 694 | CB | VAL | A | 108 | -2.520 | 20.884 | 8.252 | 1.00 | 17.46 | A |
| ATOM | 695 | CG1 | VAL | A | 108 | -1.930 | 19.481 | 8.311 | 1.00 | 16.01 | A |
| ATOM | 696 | CG2 | VAL | A | 108 | -3.187 | 21.231 | 9.573 | 1.00 | 19.18 | A |
| ATOM | 697 | C | VAL | A | 108 | -0.558 | 21.445 | 6.775 | 1.00 | 18.13 | A |
| ATOM | 698 | O | VAL | A | 108 | 0.554 | 20.960 | 6.977 | 1.00 | 18.08 | A |
| ATOM | 699 | N | ILE | A | 109 | -1.065 | 21.587 | 5.551 | 1.00 | 18.57 | A |
| ATOM | 700 | CA | ILE | A | 109 | -0.305 | 21.171 | 4.372 | 1.00 | 17.75 | A |
| ATOM | 701 | CB | ILE | A | 109 | -1.123 | 21.365 | 3.063 | 1.00 | 19.09 | A |
| ATOM | 702 | CG2 | ILE | A | 109 | -0.210 | 21.280 | 1.857 | 1.00 | 17.37 | A |
| ATOM | 703 | CG1 | ILE | A | 109 | -2.247 | 20.324 | 2.979 | 1.00 | 19.32 | A |
| ATOM | 704 | CD | ILE | A | 109 | -1.768 | 18.887 | 2.927 | 1.00 | 23.45 | A |
| ATOM | 705 | C | ILE | A | 109 | 1.006 | 21.953 | 4.239 | 1.00 | 17.28 | A |
| ATOM | 706 | O | ILE | A | 109 | 2.049 | 21.400 | 3.868 | 1.00 | 15.00 | A |
| ATOM | 707 | N | ASN | A | 110 | 0.955 | 23.246 | 4.531 | 1.00 | 17.86 | A |
| ATOM | 708 | CA | ASN | A | 110 | 2.148 | 24.069 | 4.423 | 1.00 | 17.42 | A |
| ATOM | 709 | CB | ASN | A | 110 | 1.782 | 25.549 | 4.587 | 1.00 | 18.99 | A |
| ATOM | 710 | CG | ASN | A | 110 | 2.900 | 26.471 | 4.161 | 1.00 | 18.56 | A |
| ATOM | 711 | OD1 | ASN | A | 110 | 3.723 | 26.119 | 3.314 | 1.00 | 21.26 | A |
| ATOM | 712 | ND2 | ASN | A | 110 | 2.926 | 27.666 | 4.730 | 1.00 | 18.28 | A |
| ATOM | 713 | C | ASN | A | 110 | 3.207 | 23.663 | 5.456 | 1.00 | 16.43 | A |
| ATOM | 714 | O | ASN | A | 110 | 4.385 | 23.572 | 5.142 | 1.00 | 17.24 | A |
| ATOM | 715 | N | ALA | A | 111 | 2.771 | 23.398 | 6.677 | 1.00 | 17.52 | A |
| ATOM | 716 | CA | ALA | A | 111 | 3.681 | 23.013 | 7.750 | 1.00 | 18.35 | A |
| ATOM | 717 | CB | ALA | A | 111 | 3.028 | 23.292 | 9.085 | 1.00 | 18.91 | A |
| ATOM | 718 | C | ALA | A | 111 | 4.144 | 21.553 | 7.702 | 1.00 | 18.40 | A |
| ATOM | 719 | O | ALA | A | 111 | 5.333 | 21.270 | 7.854 | 1.00 | 19.21 | A |
| ATOM | 720 | N | ASN | A | 112 | 3.206 | 20.634 | 7.481 | 1.00 | 17.41 | A |
| ATOM | 721 | CA | ASN | A | 112 | 3.521 | 19.206 | 7.463 | 1.00 | 17.98 | A |
| ATOM | 722 | CB | ASN | A | 112 | 2.333 | 18.417 | 8.010 | 1.00 | 17.28 | A |
| ATOM | 723 | CG | ASN | A | 112 | 2.139 | 18.642 | 9.493 | 1.00 | 20.88 | A |
| ATOM | 724 | OD1 | ASN | A | 112 | 2.778 | 19.513 | 10.084 | 1.00 | 21.76 | A |
| ATOM | 725 | ND2 | ASN | A | 112 | 1.262 | 17.862 | 10.105 | 1.00 | 22.78 | A |
| ATOM | 726 | C | ASN | A | 112 | 3.992 | 18.590 | 6.155 | 1.00 | 17.46 | A |
| ATOM | 727 | O | ASN | A | 112 | 4.737 | 17.607 | 6.171 | 1.00 | 18.19 | A |

Figure 1 (suite 9)

| | | | | | | | | | | | |
|---|---|---|---|---|---|---|---|---|---|---|---|
| ATOM | 728 | N | LEU | A | 113 | 3.575 | 19.137 | 5.019 | 1.00 | 15.38 | A |
| ATOM | 729 | CA | LEU | A | 113 | 4.021 | 18.577 | 3.750 | 1.00 | 13.84 | A |
| ATOM | 730 | CB | LEU | A | 113 | 2.825 | 18.335 | 2.820 | 1.00 | 13.67 | A |
| ATOM | 731 | CG | LEU | A | 113 | 3.145 | 17.751 | 1.439 | 1.00 | 11.80 | A |
| ATOM | 732 | CD1 | LEU | A | 113 | 3.885 | 16.432 | 1.588 | 1.00 | 13.33 | A |
| ATOM | 733 | CD2 | LEU | A | 113 | 1.852 | 17.549 | 0.652 | 1.00 | 16.22 | A |
| ATOM | 734 | C | LEU | A | 113 | 5.059 | 19.463 | 3.064 | 1.00 | 14.25 | A |
| ATOM | 735 | O | LEU | A | 113 | 6.155 | 19.011 | 2.727 | 1.00 | 14.64 | A |
| ATOM | 736 | N | THR | A | 114 | 4.723 | 20.728 | 2.850 | 1.00 | 13.99 | A |
| ATOM | 737 | CA | THR | A | 114 | 5.668 | 21.628 | 2.201 | 1.00 | 14.60 | A |
| ATOM | 738 | CB | THR | A | 114 | 4.999 | 22.979 | 1.847 | 1.00 | 15.51 | A |
| ATOM | 739 | OG1 | THR | A | 114 | 3.799 | 22.728 | 1.100 | 1.00 | 17.59 | A |
| ATOM | 740 | CG2 | THR | A | 114 | 5.934 | 23.830 | 0.998 | 1.00 | 11.69 | A |
| ATOM | 741 | C | THR | A | 114 | 6.853 | 21.857 | 3.152 | 1.00 | 14.23 | A |
| ATOM | 742 | O | THR | A | 114 | 7.991 | 21.982 | 2.715 | 1.00 | 14.52 | A |
| ATOM | 743 | N | GLY | A | 115 | 6.565 | 21.901 | 4.449 | 1.00 | 14.24 | A |
| ATOM | 744 | CA | GLY | A | 115 | 7.610 | 22.087 | 5.441 | 1.00 | 15.25 | A |
| ATOM | 745 | C | GLY | A | 115 | 8.589 | 20.925 | 5.351 | 1.00 | 16.07 | A |
| ATOM | 746 | O | GLY | A | 115 | 9.798 | 21.101 | 5.494 | 1.00 | 13.49 | A |
| ATOM | 747 | N | ALA | A | 116 | 8.060 | 19.730 | 5.103 | 1.00 | 15.40 | A |
| ATOM | 748 | CA | ALA | A | 116 | 8.892 | 18.542 | 4.961 | 1.00 | 15.79 | A |
| ATOM | 749 | CB | ALA | A | 116 | 8.012 | 17.292 | 4.833 | 1.00 | 15.43 | A |
| ATOM | 750 | C | ALA | A | 116 | 9.756 | 18.707 | 3.717 | 1.00 | 15.98 | A |
| ATOM | 751 | O | ALA | A | 116 | 10.933 | 18.355 | 3.719 | 1.00 | 16.41 | A |
| ATOM | 752 | N | PHE | A | 117 | 9.168 | 19.242 | 2.647 | 1.00 | 16.35 | A |
| ATOM | 753 | CA | PHE | A | 117 | 9.912 | 19.456 | 1.409 | 1.00 | 15.71 | A |
| ATOM | 754 | CB | PHE | A | 117 | 8.994 | 19.962 | 0.291 | 1.00 | 18.09 | A |
| ATOM | 755 | CG | PHE | A | 117 | 9.743 | 20.541 | -0.875 | 1.00 | 19.05 | A |
| ATOM | 756 | CD1 | PHE | A | 117 | 10.380 | 19.711 | -1.794 | 1.00 | 21.37 | A |
| ATOM | 757 | CD2 | PHE | A | 117 | 9.875 | 21.919 | -1.015 | 1.00 | 21.07 | A |
| ATOM | 758 | CE1 | PHE | A | 117 | 11.141 | 20.245 | -2.834 | 1.00 | 22.99 | A |
| ATOM | 759 | CE2 | PHE | A | 117 | 10.633 | 22.466 | -2.052 | 1.00 | 22.15 | A |
| ATOM | 760 | CZ | PHE | A | 117 | 11.268 | 21.631 | -2.961 | 1.00 | 22.35 | A |
| ATOM | 761 | C | PHE | A | 117 | 11.038 | 20.475 | 1.594 | 1.00 | 14.92 | A |
| ATOM | 762 | O | PHE | A | 117 | 12.131 | 20.314 | 1.048 | 1.00 | 15.26 | A |
| ATOM | 763 | N | ARG | A | 118 | 10.756 | 21.535 | 2.341 | 1.00 | 13.45 | A |
| ATOM | 764 | CA | ARG | A | 118 | 11.753 | 22.580 | 2.570 | 1.00 | 13.65 | A |
| ATOM | 765 | CB | ARG | A | 118 | 11.155 | 23.711 | 3.412 | 1.00 | 14.37 | A |
| ATOM | 766 | CG | ARG | A | 118 | 10.045 | 24.468 | 2.693 | 1.00 | 17.34 | A |
| ATOM | 767 | CD | ARG | A | 118 | 9.614 | 25.731 | 3.435 | 1.00 | 18.01 | A |
| ATOM | 768 | NE | ARG | A | 118 | 8.574 | 26.438 | 2.688 | 1.00 | 18.25 | A |
| ATOM | 769 | CZ | ARG | A | 118 | 7.269 | 26.279 | 2.887 | 1.00 | 17.82 | A |
| ATOM | 770 | NH1 | ARG | A | 118 | 6.831 | 25.446 | 3.822 | 1.00 | 16.69 | A |
| ATOM | 771 | NH2 | ARG | A | 118 | 6.399 | 26.932 | 2.128 | 1.00 | 18.28 | A |
| ATOM | 772 | C | ARG | A | 118 | 13.015 | 22.040 | 3.237 | 1.00 | 13.92 | A |
| ATOM | 773 | O | ARG | A | 118 | 14.115 | 22.220 | 2.718 | 1.00 | 12.36 | A |
| ATOM | 774 | N | VAL | A | 119 | 12.863 | 21.367 | 4.374 | 1.00 | 12.57 | A |
| ATOM | 775 | CA | VAL | A | 119 | 14.033 | 20.827 | 5.066 | 1.00 | 13.68 | A |
| ATOM | 776 | CB | VAL | A | 119 | 13.660 | 20.271 | 6.454 | 1.00 | 12.05 | A |
| ATOM | 777 | CG1 | VAL | A | 119 | 13.101 | 21.393 | 7.308 | 1.00 | 10.46 | A |
| ATOM | 778 | CG2 | VAL | A | 119 | 12.640 | 19.140 | 6.332 | 1.00 | 13.00 | A |
| ATOM | 779 | C | VAL | A | 119 | 14.731 | 19.759 | 4.241 | 1.00 | 13.37 | A |
| ATOM | 780 | O | VAL | A | 119 | 15.960 | 19.705 | 4.200 | 1.00 | 14.24 | A |
| ATOM | 781 | N | ALA | A | 120 | 13.952 | 18.922 | 3.558 | 1.00 | 13.48 | A |
| ATOM | 782 | CA | ALA | A | 120 | 14.523 | 17.876 | 2.720 | 1.00 | 14.31 | A |
| ATOM | 783 | CB | ALA | A | 120 | 13.402 | 16.978 | 2.152 | 1.00 | 13.43 | A |
| ATOM | 784 | C | ALA | A | 120 | 15.337 | 18.484 | 1.584 | 1.00 | 15.34 | A |
| ATOM | 785 | O | ALA | A | 120 | 16.416 | 18.000 | 1.249 | 1.00 | 14.61 | A |
| ATOM | 786 | N | GLN | A | 121 | 14.814 | 19.548 | 0.985 | 1.00 | 15.56 | A |
| ATOM | 787 | CA | GLN | A | 121 | 15.495 | 20.211 | -0.122 | 1.00 | 17.67 | A |
| ATOM | 788 | CB | GLN | A | 121 | 14.561 | 21.248 | -0.760 | 1.00 | 20.51 | A |
| ATOM | 789 | CG | GLN | A | 121 | 15.049 | 21.805 | -2.085 | 1.00 | 29.77 | A |
| ATOM | 790 | CD | GLN | A | 121 | 15.223 | 20.725 | -3.141 | 1.00 | 34.82 | A |
| ATOM | 791 | OE1 | GLN | A | 121 | 14.381 | 19.834 | -3.277 | 1.00 | 37.55 | A |
| ATOM | 792 | NE2 | GLN | A | 121 | 16.312 | 20.806 | -3.903 | 1.00 | 37.06 | A |
| ATOM | 793 | C | GLN | A | 121 | 16.789 | 20.895 | 0.333 | 1.00 | 16.74 | A |
| ATOM | 794 | O | GLN | A | 121 | 17.809 | 20.821 | -0.345 | 1.00 | 15.89 | A |
| ATOM | 795 | N | ARG | A | 122 | 16.740 | 21.559 | 1.481 | 1.00 | 14.64 | A |
| ATOM | 796 | CA | ARG | A | 122 | 17.917 | 22.251 | 2.006 | 1.00 | 16.27 | A |
| ATOM | 797 | CB | ARG | A | 122 | 17.517 | 23.126 | 3.203 | 1.00 | 17.40 | A |
| ATOM | 798 | CG | ARG | A | 122 | 18.676 | 23.866 | 3.879 | 1.00 | 17.83 | A |
| ATOM | 799 | CD | ARG | A | 122 | 19.434 | 24.783 | 2.915 | 1.00 | 18.11 | A |
| ATOM | 800 | NE | ARG | A | 122 | 20.521 | 25.497 | 3.589 | 1.00 | 18.03 | A |

Figure 1 (suite 10)

| | | | | | | | | | | | |
|---|---|---|---|---|---|---|---|---|---|---|---|
| ATOM | 801 | CZ | ARG | A | 122 | 21.681 | 24.945 | 3.944 | 1.00 | 15.84 | A |
| ATOM | 802 | NH1 | ARG | A | 122 | 21.924 | 23.667 | 3.683 | 1.00 | 15.51 | A |
| ATOM | 803 | NH2 | ARG | A | 122 | 22.595 | 25.669 | 4.573 | 1.00 | 17.47 | A |
| ATOM | 804 | C | ARG | A | 122 | 19.033 | 21.280 | 2.413 | 1.00 | 17.07 | A |
| ATOM | 805 | O | ARG | A | 122 | 20.213 | 21.633 | 2.393 | 1.00 | 17.40 | A |
| ATOM | 806 | N | ALA | A | 123 | 18.667 | 20.048 | 2.755 | 1.00 | 17.13 | A |
| ATOM | 807 | CA | ALA | A | 123 | 19.660 | 19.061 | 3.173 | 1.00 | 17.64 | A |
| ATOM | 808 | CB | ALA | A | 123 | 19.030 | 18.086 | 4.159 | 1.00 | 15.95 | A |
| ATOM | 809 | C | ALA | A | 123 | 20.268 | 18.285 | 2.016 | 1.00 | 17.49 | A |
| ATOM | 810 | O | ALA | A | 123 | 21.400 | 17.805 | 2.102 | 1.00 | 16.35 | A |
| ATOM | 811 | N | SER | A | 124 | 19.507 | 18.177 | 0.932 | 1.00 | 18.85 | A |
| ATOM | 812 | CA | SER | A | 124 | 19.906 | 17.409 | -0.243 | 1.00 | 20.53 | A |
| ATOM | 813 | CB | SER | A | 124 | 18.802 | 17.503 | -1.301 | 1.00 | 21.14 | A |
| ATOM | 814 | OG | SER | A | 124 | 19.088 | 16.647 | -2.390 | 1.00 | 29.16 | A |
| ATOM | 815 | C | SER | A | 124 | 21.259 | 17.674 | -0.909 | 1.00 | 21.05 | A |
| ATOM | 816 | O | SER | A | 124 | 21.980 | 16.728 | -1.236 | 1.00 | 19.51 | A |
| ATOM | 817 | N | ARG | A | 125 | 21.605 | 18.940 | -1.132 | 1.00 | 21.29 | A |
| ATOM | 818 | CA | ARG | A | 125 | 22.873 | 19.262 | -1.788 | 1.00 | 24.64 | A |
| ATOM | 819 | CB | ARG | A | 125 | 23.064 | 20.794 | -1.819 | 1.00 | 30.23 | A |
| ATOM | 820 | CG | ARG | A | 125 | 22.981 | 21.391 | -3.223 | 1.00 | 37.53 | A |
| ATOM | 821 | CD | ARG | A | 125 | 21.546 | 21.555 | -3.719 | 1.00 | 42.87 | A |
| ATOM | 822 | NE | ARG | A | 125 | 21.435 | 22.472 | -4.864 | 1.00 | 49.21 | A |
| ATOM | 823 | CZ | ARG | A | 125 | 20.270 | 22.879 | -5.384 | 1.00 | 52.15 | A |
| ATOM | 824 | NH1 | ARG | A | 125 | 19.103 | 22.463 | -4.876 | 1.00 | 54.37 | A |
| ATOM | 825 | NH2 | ARG | A | 125 | 20.168 | 23.715 | -6.427 | 1.00 | 56.76 | A |
| ATOM | 826 | C | ARG | A | 125 | 24.083 | 18.637 | -1.104 | 1.00 | 22.80 | A |
| ATOM | 827 | O | ARG | A | 125 | 24.836 | 17.879 | -1.715 | 1.00 | 23.04 | A |
| ATOM | 828 | N | SER | A | 126 | 24.270 | 18.980 | 0.164 | 1.00 | 21.87 | A |
| ATOM | 829 | CA | SER | A | 126 | 25.393 | 18.481 | 0.947 | 1.00 | 21.93 | A |
| ATOM | 830 | CB | SER | A | 126 | 25.329 | 19.056 | 2.367 | 1.00 | 21.24 | A |
| ATOM | 831 | OG | SER | A | 126 | 26.448 | 18.654 | 3.141 | 1.00 | 22.58 | A |
| ATOM | 832 | C | SER | A | 126 | 25.431 | 16.958 | 1.003 | 1.00 | 20.68 | A |
| ATOM | 833 | O | SER | A | 126 | 26.498 | 16.357 | 0.879 | 1.00 | 20.04 | A |
| ATOM | 834 | N | MET | A | 127 | 24.268 | 16.333 | 1.190 | 1.00 | 20.79 | A |
| ATOM | 835 | CA | MET | A | 127 | 24.201 | 14.875 | 1.263 | 1.00 | 20.39 | A |
| ATOM | 836 | CB | MET | A | 127 | 22.782 | 14.421 | 1.626 | 1.00 | 17.75 | A |
| ATOM | 837 | CG | MET | A | 127 | 22.338 | 14.839 | 3.020 | 1.00 | 16.89 | A |
| ATOM | 838 | SD | MET | A | 127 | 20.636 | 14.342 | 3.401 | 1.00 | 15.43 | A |
| ATOM | 839 | CE | MET | A | 127 | 20.532 | 14.646 | 5.137 | 1.00 | 10.54 | A |
| ATOM | 840 | C | MET | A | 127 | 24.642 | 14.222 | -0.045 | 1.00 | 21.44 | A |
| ATOM | 841 | O | MET | A | 127 | 25.353 | 13.215 | -0.038 | 1.00 | 20.02 | A |
| ATOM | 842 | N | GLN | A | 128 | 24.221 | 14.792 | -1.168 | 1.00 | 22.20 | A |
| ATOM | 843 | CA | GLN | A | 128 | 24.590 | 14.256 | -2.473 | 1.00 | 24.39 | A |
| ATOM | 844 | CB | GLN | A | 128 | 23.854 | 14.998 | -3.591 | 1.00 | 27.10 | A |
| ATOM | 845 | CG | GLN | A | 128 | 22.371 | 14.723 | -3.678 | 1.00 | 29.14 | A |
| ATOM | 846 | CD | GLN | A | 128 | 21.718 | 15.489 | -4.813 | 1.00 | 30.98 | A |
| ATOM | 847 | OE1 | GLN | A | 128 | 21.064 | 16.515 | -4.596 | 1.00 | 31.74 | A |
| ATOM | 848 | NE2 | GLN | A | 128 | 21.907 | 15.001 | -6.035 | 1.00 | 30.30 | A |
| ATOM | 849 | C | GLN | A | 128 | 26.087 | 14.404 | -2.707 | 1.00 | 25.55 | A |
| ATOM | 850 | O | GLN | A | 128 | 26.762 | 13.470 | -3.140 | 1.00 | 25.65 | A |
| ATOM | 851 | N | ARG | A | 129 | 26.598 | 15.595 | -2.427 | 1.00 | 27.10 | A |
| ATOM | 852 | CA | ARG | A | 129 | 28.012 | 15.875 | -2.625 | 1.00 | 28.10 | A |
| ATOM | 853 | CB | ARG | A | 129 | 28.296 | 17.350 | -2.326 | 1.00 | 30.05 | A |
| ATOM | 854 | CG | ARG | A | 129 | 29.747 | 17.749 | -2.518 | 1.00 | 35.40 | A |
| ATOM | 855 | CD | ARG | A | 129 | 29.938 | 19.259 | -2.458 | 1.00 | 38.37 | A |
| ATOM | 856 | NE | ARG | A | 129 | 29.303 | 19.850 | -1.284 | 1.00 | 41.42 | A |
| ATOM | 857 | CZ | ARG | A | 129 | 28.100 | 20.414 | -1.292 | 1.00 | 43.51 | A |
| ATOM | 858 | NH1 | ARG | A | 129 | 27.596 | 20.923 | -0.175 | 1.00 | 44.84 | A |
| ATOM | 859 | NH2 | ARG | A | 129 | 27.407 | 20.483 | -2.422 | 1.00 | 45.74 | A |
| ATOM | 860 | C | ARG | A | 129 | 28.904 | 14.982 | -1.766 | 1.00 | 27.39 | A |
| ATOM | 861 | O | ARG | A | 129 | 29.966 | 14.550 | -2.212 | 1.00 | 25.98 | A |
| ATOM | 862 | N | ASN | A | 130 | 28.474 | 14.706 | -0.538 | 1.00 | 25.98 | A |
| ATOM | 863 | CA | ASN | A | 130 | 29.255 | 13.866 | 0.365 | 1.00 | 24.37 | A |
| ATOM | 864 | CB | ASN | A | 130 | 29.052 | 14.312 | 1.814 | 1.00 | 26.10 | A |
| ATOM | 865 | CG | ASN | A | 130 | 29.675 | 15.662 | 2.093 | 1.00 | 27.25 | A |
| ATOM | 866 | OD1 | ASN | A | 130 | 30.838 | 15.891 | 1.767 | 1.00 | 29.25 | A |
| ATOM | 867 | ND2 | ASN | A | 130 | 28.912 | 16.559 | 2.703 | 1.00 | 27.79 | A |
| ATOM | 868 | C | ASN | A | 130 | 28.926 | 12.388 | 0.231 | 1.00 | 23.73 | A |
| ATOM | 869 | O | ASN | A | 130 | 29.427 | 11.563 | 0.991 | 1.00 | 24.46 | A |
| ATOM | 870 | N | LYS | A | 131 | 28.085 | 12.058 | -0.743 | 1.00 | 23.48 | A |
| ATOM | 871 | CA | LYS | A | 131 | 27.686 | 10.678 | -0.981 | 1.00 | 22.75 | A |
| ATOM | 872 | CB | LYS | A | 131 | 28.847 | 9.880 | -1.567 | 1.00 | 25.74 | A |
| ATOM | 873 | CG | LYS | A | 131 | 29.414 | 10.461 | -2.845 | 1.00 | 28.25 | A |

Figure 1 (suite 11)

| | | | | | | | | | | | |
|---|---|---|---|---|---|---|---|---|---|---|---|
| ATOM | 874 | CD | LYS | A | 131 | 28.412 | 10.415 | -3.972 | 1.00 | 32.71 | A |
| ATOM | 875 | CE | LYS | A | 131 | 29.060 | 10.849 | -5.280 | 1.00 | 36.00 | A |
| ATOM | 876 | NZ | LYS | A | 131 | 28.101 | 10.798 | -6.414 | 1.00 | 37.75 | A |
| ATOM | 877 | C | LYS | A | 131 | 27.191 | 9.988 | 0.284 | 1.00 | 21.44 | A |
| ATOM | 878 | O | LYS | A | 131 | 27.534 | 8.839 | 0.554 | 1.00 | 20.09 | A |
| ATOM | 879 | N | PHE | A | 132 | 26.406 | 10.706 | 1.074 | 1.00 | 19.69 | A |
| ATOM | 880 | CA | PHE | A | 132 | 25.820 | 10.136 | 2.270 | 1.00 | 18.13 | A |
| ATOM | 881 | CB | PHE | A | 132 | 26.830 | 9.971 | 3.406 | 1.00 | 18.58 | A |
| ATOM | 882 | CG | PHE | A | 132 | 26.238 | 9.308 | 4.618 | 1.00 | 20.58 | A |
| ATOM | 883 | CD1 | PHE | A | 132 | 26.231 | 7.920 | 4.732 | 1.00 | 18.23 | A |
| ATOM | 884 | CD2 | PHE | A | 132 | 25.587 | 10.063 | 5.590 | 1.00 | 18.12 | A |
| ATOM | 885 | CE1 | PHE | A | 132 | 25.582 | 7.291 | 5.790 | 1.00 | 18.86 | A |
| ATOM | 886 | CE2 | PHE | A | 132 | 24.934 | 9.446 | 6.652 | 1.00 | 21.77 | A |
| ATOM | 887 | CZ | PHE | A | 132 | 24.930 | 8.048 | 6.751 | 1.00 | 18.89 | A |
| ATOM | 888 | C | PHE | A | 132 | 24.667 | 10.968 | 2.791 | 1.00 | 17.16 | A |
| ATOM | 889 | O | PHE | A | 132 | 24.780 | 12.182 | 2.955 | 1.00 | 16.20 | A |
| ATOM | 890 | N | GLY | A | 133 | 23.562 | 10.297 | 3.086 | 1.00 | 16.04 | A |
| ATOM | 891 | CA | GLY | A | 133 | 22.416 | 11.002 | 3.610 | 1.00 | 15.57 | A |
| ATOM | 892 | C | GLY | A | 133 | 21.296 | 10.109 | 4.098 | 1.00 | 16.14 | A |
| ATOM | 893 | O | GLY | A | 133 | 20.966 | 9.090 | 3.487 | 1.00 | 15.46 | A |
| ATOM | 894 | N | ARG | A | 134 | 20.714 | 10.498 | 5.222 | 1.00 | 14.19 | A |
| ATOM | 895 | CA | ARG | A | 134 | 19.596 | 9.777 | 5.789 | 1.00 | 14.01 | A |
| ATOM | 896 | CB | ARG | A | 134 | 20.010 | 9.066 | 7.084 | 1.00 | 14.82 | A |
| ATOM | 897 | CG | ARG | A | 134 | 21.101 | 8.017 | 6.891 | 1.00 | 16.57 | A |
| ATOM | 898 | CD | ARG | A | 134 | 20.606 | 6.827 | 6.056 | 1.00 | 18.51 | A |
| ATOM | 899 | NE | ARG | A | 134 | 21.660 | 5.849 | 5.770 | 1.00 | 19.22 | A |
| ATOM | 900 | CZ | ARG | A | 134 | 22.408 | 5.840 | 4.668 | 1.00 | 23.52 | A |
| ATOM | 901 | NH1 | ARG | A | 134 | 22.234 | 6.758 | 3.725 | 1.00 | 23.76 | A |
| ATOM | 902 | NH2 | ARG | A | 134 | 23.328 | 4.898 | 4.496 | 1.00 | 25.78 | A |
| ATOM | 903 | C | ARG | A | 134 | 18.499 | 10.796 | 6.084 | 1.00 | 13.04 | A |
| ATOM | 904 | O | ARG | A | 134 | 18.660 | 11.677 | 6.926 | 1.00 | 14.69 | A |
| ATOM | 905 | N | MET | A | 135 | 17.396 | 10.697 | 5.355 | 1.00 | 11.84 | A |
| ATOM | 906 | CA | MET | A | 135 | 16.271 | 11.578 | 5.595 | 1.00 | 11.42 | A |
| ATOM | 907 | CB | MET | A | 135 | 15.755 | 12.185 | 4.285 | 1.00 | 12.68 | A |
| ATOM | 908 | CG | MET | A | 135 | 16.672 | 13.226 | 3.673 | 1.00 | 14.06 | A |
| ATOM | 909 | SD | MET | A | 135 | 15.845 | 14.199 | 2.365 | 1.00 | 14.52 | A |
| ATOM | 910 | CE | MET | A | 135 | 17.173 | 14.464 | 1.225 | 1.00 | 16.15 | A |
| ATOM | 911 | C | MET | A | 135 | 15.219 | 10.694 | 6.229 | 1.00 | 11.94 | A |
| ATOM | 912 | O | MET | A | 135 | 14.846 | 9.658 | 5.671 | 1.00 | 11.02 | A |
| ATOM | 913 | N | ILE | A | 136 | 14.762 | 11.082 | 7.411 | 1.00 | 11.08 | A |
| ATOM | 914 | CA | ILE | A | 136 | 13.765 | 10.305 | 8.116 | 1.00 | 9.91 | A |
| ATOM | 915 | CB | ILE | A | 136 | 14.360 | 9.688 | 9.393 | 1.00 | 10.62 | A |
| ATOM | 916 | CG2 | ILE | A | 136 | 13.327 | 8.814 | 10.076 | 1.00 | 10.51 | A |
| ATOM | 917 | CG1 | ILE | A | 136 | 15.602 | 8.865 | 9.026 | 1.00 | 9.01 | A |
| ATOM | 918 | CD | ILE | A | 136 | 16.474 | 8.485 | 10.233 | 1.00 | 9.16 | A |
| ATOM | 919 | C | ILE | A | 136 | 12.577 | 11.183 | 8.477 | 1.00 | 10.24 | A |
| ATOM | 920 | O | ILE | A | 136 | 12.672 | 12.074 | 9.325 | 1.00 | 10.32 | A |
| ATOM | 921 | N | PHE | A | 137 | 11.448 | 10.927 | 7.823 | 1.00 | 9.65 | A |
| ATOM | 922 | CA | PHE | A | 137 | 10.243 | 11.707 | 8.079 | 1.00 | 10.26 | A |
| ATOM | 923 | CB | PHE | A | 137 | 9.484 | 11.952 | 6.762 | 1.00 | 11.09 | A |
| ATOM | 924 | CG | PHE | A | 137 | 10.333 | 12.566 | 5.681 | 1.00 | 10.63 | A |
| ATOM | 925 | CD1 | PHE | A | 137 | 11.120 | 11.771 | 4.859 | 1.00 | 10.01 | A |
| ATOM | 926 | CD2 | PHE | A | 137 | 10.373 | 13.950 | 5.509 | 1.00 | 10.03 | A |
| ATOM | 927 | CE1 | PHE | A | 137 | 11.928 | 12.329 | 3.885 | 1.00 | 12.32 | A |
| ATOM | 928 | CE2 | PHE | A | 137 | 11.184 | 14.522 | 4.535 | 1.00 | 11.49 | A |
| ATOM | 929 | CZ | PHE | A | 137 | 11.963 | 13.717 | 3.721 | 1.00 | 9.36 | A |
| ATOM | 930 | C | PHE | A | 137 | 9.355 | 10.997 | 9.083 | 1.00 | 11.12 | A |
| ATOM | 931 | O | PHE | A | 137 | 9.055 | 9.807 | 8.934 | 1.00 | 11.07 | A |
| ATOM | 932 | N | ILE | A | 138 | 8.949 | 11.731 | 10.114 | 1.00 | 11.35 | A |
| ATOM | 933 | CA | ILE | A | 138 | 8.088 | 11.202 | 11.157 | 1.00 | 13.52 | A |
| ATOM | 934 | CB | ILE | A | 138 | 8.531 | 11.697 | 12.556 | 1.00 | 14.71 | A |
| ATOM | 935 | CG2 | ILE | A | 138 | 7.751 | 10.958 | 13.637 | 1.00 | 13.00 | A |
| ATOM | 936 | CG1 | ILE | A | 138 | 10.040 | 11.476 | 12.747 | 1.00 | 13.06 | A |
| ATOM | 937 | CD | ILE | A | 138 | 10.492 | 10.030 | 12.573 | 1.00 | 13.95 | A |
| ATOM | 938 | C | ILE | A | 138 | 6.654 | 11.672 | 10.910 | 1.00 | 14.27 | A |
| ATOM | 939 | O | ILE | A | 138 | 6.379 | 12.870 | 10.909 | 1.00 | 14.16 | A |
| ATOM | 940 | N | GLY | A | 139 | 5.746 | 10.725 | 10.697 | 1.00 | 17.53 | A |
| ATOM | 941 | CA | GLY | A | 139 | 4.364 | 11.085 | 10.448 | 1.00 | 19.64 | A |
| ATOM | 942 | C | GLY | A | 139 | 3.673 | 11.563 | 11.709 | 1.00 | 21.88 | A |
| ATOM | 943 | O | GLY | A | 139 | 4.043 | 11.167 | 12.815 | 1.00 | 21.63 | A |
| ATOM | 944 | N | SER | A | 140 | 2.666 | 12.415 | 11.554 | 1.00 | 21.89 | A |
| ATOM | 945 | CA | SER | A | 140 | 1.930 | 12.914 | 12.707 | 1.00 | 26.20 | A |
| ATOM | 946 | CB | SER | A | 140 | 0.929 | 14.003 | 12.272 | 1.00 | 27.45 | A |

Figure 1 (suite 12)

| | | | | | | | | | | | |
|---|---|---|---|---|---|---|---|---|---|---|---|
| ATOM | 947 | OG | SER | A | 140 | 0.195 | 13.613 | 11.120 | 1.00 | 28.07 | A |
| ATOM | 948 | C | SER | A | 140 | 1.199 | 11.766 | 13.403 | 1.00 | 27.37 | A |
| ATOM | 949 | O | SER | A | 140 | 0.934 | 10.728 | 12.794 | 1.00 | 25.74 | A |
| ATOM | 950 | N | VAL | A | 141 | 0.882 | 11.958 | 14.681 | 1.00 | 29.16 | A |
| ATOM | 951 | CA | VAL | A | 141 | 0.177 | 10.942 | 15.455 | 1.00 | 30.74 | A |
| ATOM | 952 | CB | VAL | A | 141 | 0.519 | 11.055 | 16.961 | 1.00 | 32.01 | A |
| ATOM | 953 | CG1 | VAL | A | 141 | -0.363 | 10.111 | 17.776 | 1.00 | 32.50 | A |
| ATOM | 954 | CG2 | VAL | A | 141 | 1.989 | 10.726 | 17.183 | 1.00 | 30.37 | A |
| ATOM | 955 | C | VAL | A | 141 | -1.336 | 11.068 | 15.280 | 1.00 | 31.79 | A |
| ATOM | 956 | O | VAL | A | 141 | -1.833 | 11.218 | 14.161 | 1.00 | 31.70 | A |
| ATOM | 957 | N | ALA | A | 150 | -9.248 | 13.852 | 10.205 | 1.00 | 28.04 | A |
| ATOM | 958 | CA | ALA | A | 150 | -9.025 | 15.146 | 9.575 | 1.00 | 27.16 | A |
| ATOM | 959 | CB | ALA | A | 150 | -8.114 | 16.007 | 10.446 | 1.00 | 28.88 | A |
| ATOM | 960 | C | ALA | A | 150 | -8.410 | 14.958 | 8.197 | 1.00 | 26.17 | A |
| ATOM | 961 | O | ALA | A | 150 | -7.355 | 14.345 | 8.056 | 1.00 | 25.22 | A |
| ATOM | 962 | N | ALA | A | 151 | -9.075 | 15.501 | 7.184 | 1.00 | 24.94 | A |
| ATOM | 963 | CA | ALA | A | 151 | -8.616 | 15.388 | 5.809 | 1.00 | 23.20 | A |
| ATOM | 964 | CB | ALA | A | 151 | -9.663 | 15.979 | 4.865 | 1.00 | 21.41 | A |
| ATOM | 965 | C | ALA | A | 151 | -7.265 | 16.048 | 5.551 | 1.00 | 23.29 | A |
| ATOM | 966 | O | ALA | A | 151 | -6.519 | 15.606 | 4.675 | 1.00 | 23.04 | A |
| ATOM | 967 | N | ASN | A | 152 | -6.943 | 17.107 | 6.292 | 1.00 | 20.88 | A |
| ATOM | 968 | CA | ASN | A | 152 | -5.668 | 17.772 | 6.061 | 1.00 | 21.43 | A |
| ATOM | 969 | CB | ASN | A | 152 | -5.660 | 19.195 | 6.649 | 1.00 | 21.19 | A |
| ATOM | 970 | CG | ASN | A | 152 | -6.068 | 19.247 | 8.102 | 1.00 | 20.33 | A |
| ATOM | 971 | OD1 | ASN | A | 152 | -6.033 | 20.314 | 8.718 | 1.00 | 23.00 | A |
| ATOM | 972 | ND2 | ASN | A | 152 | -6.462 | 18.114 | 8.661 | 1.00 | 21.40 | A |
| ATOM | 973 | C | ASN | A | 152 | -4.462 | 16.973 | 6.555 | 1.00 | 20.62 | A |
| ATOM | 974 | O | ASN | A | 152 | -3.466 | 16.866 | 5.848 | 1.00 | 18.60 | A |
| ATOM | 975 | N | TYR | A | 153 | -4.544 | 16.403 | 7.752 | 1.00 | 21.70 | A |
| ATOM | 976 | CA | TYR | A | 153 | -3.426 | 15.608 | 8.258 | 1.00 | 21.12 | A |
| ATOM | 977 | CB | TYR | A | 153 | -3.636 | 15.257 | 9.727 | 1.00 | 24.29 | A |
| ATOM | 978 | CG | TYR | A | 153 | -3.331 | 16.407 | 10.650 | 1.00 | 25.51 | A |
| ATOM | 979 | CD1 | TYR | A | 153 | -4.305 | 17.351 | 10.974 | 1.00 | 26.43 | A |
| ATOM | 980 | CE1 | TYR | A | 153 | -4.001 | 18.444 | 11.788 | 1.00 | 28.70 | A |
| ATOM | 981 | CD2 | TYR | A | 153 | -2.046 | 16.581 | 11.161 | 1.00 | 26.44 | A |
| ATOM | 982 | CE2 | TYR | A | 153 | -1.732 | 17.668 | 11.971 | 1.00 | 27.25 | A |
| ATOM | 983 | CZ | TYR | A | 153 | -2.709 | 18.594 | 12.280 | 1.00 | 27.33 | A |
| ATOM | 984 | OH | TYR | A | 153 | -2.384 | 19.673 | 13.066 | 1.00 | 28.54 | A |
| ATOM | 985 | C | TYR | A | 153 | -3.274 | 14.339 | 7.425 | 1.00 | 20.46 | A |
| ATOM | 986 | O | TYR | A | 153 | -2.163 | 13.954 | 7.060 | 1.00 | 21.29 | A |
| ATOM | 987 | N | ALA | A | 154 | -4.398 | 13.695 | 7.115 | 1.00 | 18.63 | A |
| ATOM | 988 | CA | ALA | A | 154 | -4.382 | 12.485 | 6.303 | 1.00 | 17.80 | A |
| ATOM | 989 | CB | ALA | A | 154 | -5.821 | 12.012 | 6.031 | 1.00 | 16.78 | A |
| ATOM | 990 | C | ALA | A | 154 | -3.655 | 12.758 | 4.987 | 1.00 | 16.33 | A |
| ATOM | 991 | O | ALA | A | 154 | -2.792 | 11.987 | 4.574 | 1.00 | 15.01 | A |
| ATOM | 992 | N | ALA | A | 155 | -4.002 | 13.865 | 4.334 | 1.00 | 15.66 | A |
| ATOM | 993 | CA | ALA | A | 155 | -3.373 | 14.239 | 3.068 | 1.00 | 15.55 | A |
| ATOM | 994 | CB | ALA | A | 155 | -4.077 | 15.452 | 2.473 | 1.00 | 12.62 | A |
| ATOM | 995 | C | ALA | A | 155 | -1.882 | 14.541 | 3.209 | 1.00 | 15.46 | A |
| ATOM | 996 | O | ALA | A | 155 | -1.066 | 14.108 | 2.392 | 1.00 | 12.93 | A |
| ATOM | 997 | N | SER | A | 156 | -1.528 | 15.304 | 4.237 | 1.00 | 16.10 | A |
| ATOM | 998 | CA | SER | A | 156 | -0.130 | 15.670 | 4.438 | 1.00 | 16.03 | A |
| ATOM | 999 | CB | SER | A | 156 | 0.003 | 16.719 | 5.561 | 1.00 | 17.50 | A |
| ATOM | 1000 | OG | SER | A | 156 | -0.561 | 16.261 | 6.779 | 1.00 | 22.26 | A |
| ATOM | 1001 | C | SER | A | 156 | 0.714 | 14.446 | 4.747 | 1.00 | 15.28 | A |
| ATOM | 1002 | O | SER | A | 156 | 1.792 | 14.283 | 4.186 | 1.00 | 14.36 | A |
| ATOM | 1003 | N | LYS | A | 157 | 0.223 | 13.579 | 5.625 | 1.00 | 15.94 | A |
| ATOM | 1004 | CA | LYS | A | 157 | 0.978 | 12.379 | 5.974 | 1.00 | 16.00 | A |
| ATOM | 1005 | CB | LYS | A | 157 | 0.284 | 11.597 | 7.100 | 1.00 | 19.24 | A |
| ATOM | 1006 | CG | LYS | A | 157 | -1.099 | 11.059 | 6.774 | 0.00 | 22.85 | A |
| ATOM | 1007 | CD | LYS | A | 157 | -1.618 | 10.162 | 7.897 | 1.00 | 29.16 | A |
| ATOM | 1008 | CE | LYS | A | 157 | -1.803 | 10.924 | 9.201 | 0.00 | 27.16 | A |
| ATOM | 1009 | NZ | LYS | A | 157 | -2.845 | 11.981 | 9.087 | 0.00 | 27.87 | A |
| ATOM | 1010 | C | LYS | A | 157 | 1.147 | 11.502 | 4.740 | 1.00 | 15.26 | A |
| ATOM | 1011 | O | LYS | A | 157 | 2.237 | 10.995 | 4.482 | 1.00 | 12.02 | A |
| ATOM | 1012 | N | ALA | A | 158 | 0.074 | 11.332 | 3.965 | 1.00 | 13.52 | A |
| ATOM | 1013 | CA | ALA | A | 158 | 0.163 | 10.519 | 2.753 | 1.00 | 13.70 | A |
| ATOM | 1014 | CB | ALA | A | 158 | -1.240 | 10.349 | 2.112 | 1.00 | 13.36 | A |
| ATOM | 1015 | C | ALA | A | 158 | 1.118 | 11.203 | 1.777 | 1.00 | 12.63 | A |
| ATOM | 1016 | O | ALA | A | 158 | 1.913 | 10.547 | 1.094 | 1.00 | 11.43 | A |
| ATOM | 1017 | N | GLY | A | 159 | 1.042 | 12.531 | 1.720 | 1.00 | 12.13 | A |
| ATOM | 1018 | CA | GLY | A | 159 | 1.913 | 13.278 | 0.829 | 1.00 | 12.92 | A |
| ATOM | 1019 | C | GLY | A | 159 | 3.395 | 13.169 | 1.178 | 1.00 | 12.47 | A |

Figure 1 (suite 13)

| | | | | | | | | | | | |
|---|---|---|---|---|---|---|---|---|---|---|---|
| ATOM | 1020 | O | GLY | A | 159 | 4.233 | 13.083 | 0.290 | 1.00 | 12.65 | A |
| ATOM | 1021 | N | VAL | A | 160 | 3.728 | 13.179 | 2.466 | 1.00 | 12.24 | A |
| ATOM | 1022 | CA | VAL | A | 160 | 5.138 | 13.075 | 2.873 | 1.00 | 11.18 | A |
| ATOM | 1023 | CB | VAL | A | 160 | 5.290 | 13.183 | 4.406 | 1.00 | 11.56 | A |
| ATOM | 1024 | CG1 | VAL | A | 160 | 6.734 | 12.852 | 4.831 | 1.00 | 10.76 | A |
| ATOM | 1025 | CG2 | VAL | A | 160 | 4.935 | 14.582 | 4.855 | 1.00 | 8.64 | A |
| ATOM | 1026 | C | VAL | A | 160 | 5.686 | 11.732 | 2.404 | 1.00 | 12.91 | A |
| ATOM | 1027 | O | VAL | A | 160 | 6.827 | 11.634 | 1.937 | 1.00 | 13.27 | A |
| ATOM | 1028 | N | ILE | A | 161 | 4.859 | 10.695 | 2.512 | 1.00 | 13.46 | A |
| ATOM | 1029 | CA | ILE | A | 161 | 5.277 | 9.365 | 2.086 | 1.00 | 13.87 | A |
| ATOM | 1030 | CB | ILE | A | 161 | 4.213 | 8.306 | 2.459 | 1.00 | 14.19 | A |
| ATOM | 1031 | CG2 | ILE | A | 161 | 4.508 | 6.985 | 1.753 | 1.00 | 10.60 | A |
| ATOM | 1032 | CG1 | ILE | A | 161 | 4.214 | 8.119 | 3.981 | 1.00 | 13.57 | A |
| ATOM | 1033 | CD | ILE | A | 161 | 3.086 | 7.264 | 4.525 | 1.00 | 15.93 | A |
| ATOM | 1034 | C | ILE | A | 161 | 5.557 | 9.353 | 0.591 | 1.00 | 13.80 | A |
| ATOM | 1035 | O | ILE | A | 161 | 6.574 | 8.819 | 0.146 | 1.00 | 13.00 | A |
| ATOM | 1036 | N | GLY | A | 162 | 4.669 | 9.960 | -0.191 | 1.00 | 14.82 | A |
| ATOM | 1037 | CA | GLY | A | 162 | 4.889 | 10.004 | -1.625 | 1.00 | 15.61 | A |
| ATOM | 1038 | C | GLY | A | 162 | 6.159 | 10.778 | -1.945 | 1.00 | 16.64 | A |
| ATOM | 1039 | O | GLY | A | 162 | 6.939 | 10.382 | -2.817 | 1.00 | 15.33 | A |
| ATOM | 1040 | N | MET | A | 163 | 6.371 | 11.889 | -1.241 | 1.00 | 15.45 | A |
| ATOM | 1041 | CA | MET | A | 163 | 7.562 | 12.703 | -1.471 | 1.00 | 16.08 | A |
| ATOM | 1042 | CB | MET | A | 163 | 7.521 | 13.975 | -0.617 | 1.00 | 14.24 | A |
| ATOM | 1043 | CG | MET | A | 163 | 8.717 | 14.896 | -0.846 | 1.00 | 15.17 | A |
| ATOM | 1044 | SD | MET | A | 163 | 8.729 | 16.289 | 0.290 | 1.00 | 18.70 | A |
| ATOM | 1045 | CE | MET | A | 163 | 9.161 | 15.444 | 1.846 | 1.00 | 16.49 | A |
| ATOM | 1046 | C | MET | A | 163 | 8.817 | 11.901 | -1.124 | 1.00 | 17.05 | A |
| ATOM | 1047 | O | MET | A | 163 | 9.780 | 11.873 | -1.888 | 1.00 | 18.25 | A |
| ATOM | 1048 | N | ALA | A | 164 | 8.791 | 11.248 | 0.032 | 1.00 | 15.39 | A |
| ATOM | 1049 | CA | ALA | A | 164 | 9.926 | 10.450 | 0.485 | 1.00 | 15.94 | A |
| ATOM | 1050 | CB | ALA | A | 164 | 9.576 | 9.744 | 1.792 | 1.00 | 13.42 | A |
| ATOM | 1051 | C | ALA | A | 164 | 10.314 | 9.429 | -0.569 | 1.00 | 15.19 | A |
| ATOM | 1052 | O | ALA | A | 164 | 11.487 | 9.272 | -0.907 | 1.00 | 14.97 | A |
| ATOM | 1053 | N | ARG | A | 165 | 9.316 | 8.735 | -1.100 | 1.00 | 17.18 | A |
| ATOM | 1054 | CA | ARG | A | 165 | 9.576 | 7.723 | -2.107 | 1.00 | 17.56 | A |
| ATOM | 1055 | CB | ARG | A | 165 | 8.293 | 6.938 | -2.386 | 1.00 | 20.37 | A |
| ATOM | 1056 | CG | ARG | A | 165 | 8.021 | 5.978 | -1.244 | 1.00 | 20.99 | A |
| ATOM | 1057 | CD | ARG | A | 165 | 6.706 | 5.233 | -1.311 | 1.00 | 25.23 | A |
| ATOM | 1058 | NE | ARG | A | 165 | 6.738 | 4.136 | -0.344 | 1.00 | 26.52 | A |
| ATOM | 1059 | CZ | ARG | A | 165 | 5.675 | 3.462 | 0.081 | 1.00 | 27.75 | A |
| ATOM | 1060 | NH1 | ARG | A | 165 | 4.462 | 3.766 | -0.372 | 1.00 | 26.84 | A |
| ATOM | 1061 | NH2 | ARG | A | 165 | 5.827 | 2.481 | 0.965 | 1.00 | 26.81 | A |
| ATOM | 1062 | C | ARG | A | 165 | 10.175 | 8.309 | -3.363 | 1.00 | 17.36 | A |
| ATOM | 1063 | O | ARG | A | 165 | 11.001 | 7.677 | -4.013 | 1.00 | 17.57 | A |
| ATOM | 1064 | N | SER | A | 166 | 9.782 | 9.533 | -3.692 | 1.00 | 17.65 | A |
| ATOM | 1065 | CA | SER | A | 166 | 10.321 | 10.205 | -4.865 | 1.00 | 19.54 | A |
| ATOM | 1066 | CB | SER | A | 166 | 9.538 | 11.490 | -5.139 | 1.00 | 19.35 | A |
| ATOM | 1067 | OG | SER | A | 166 | 10.127 | 12.215 | -6.200 | 1.00 | 22.67 | A |
| ATOM | 1068 | C | SER | A | 166 | 11.793 | 10.535 | -4.609 | 1.00 | 19.98 | A |
| ATOM | 1069 | O | SER | A | 166 | 12.657 | 10.305 | -5.460 | 1.00 | 20.10 | A |
| ATOM | 1070 | N | ILE | A | 167 | 12.073 | 11.083 | -3.432 | 1.00 | 17.58 | A |
| ATOM | 1071 | CA | ILE | A | 167 | 13.446 | 11.418 | -3.074 | 1.00 | 16.34 | A |
| ATOM | 1072 | CB | ILE | A | 167 | 13.510 | 12.097 | -1.681 | 1.00 | 14.77 | A |
| ATOM | 1073 | CG2 | ILE | A | 167 | 14.977 | 12.310 | -1.273 | 1.00 | 16.91 | A |
| ATOM | 1074 | CG1 | ILE | A | 167 | 12.784 | 13.447 | -1.726 | 1.00 | 17.95 | A |
| ATOM | 1075 | CD | ILE | A | 167 | 12.651 | 14.126 | -0.376 | 1.00 | 18.31 | A |
| ATOM | 1076 | C | ILE | A | 167 | 14.306 | 10.150 | -3.061 | 1.00 | 15.47 | A |
| ATOM | 1077 | O | ILE | A | 167 | 15.405 | 10.130 | -3.605 | 1.00 | 16.41 | A |
| ATOM | 1078 | N | ALA | A | 168 | 13.798 | 9.086 | -2.452 | 1.00 | 15.38 | A |
| ATOM | 1079 | CA | ALA | A | 168 | 14.550 | 7.830 | -2.397 | 1.00 | 17.39 | A |
| ATOM | 1080 | CB | ALA | A | 168 | 13.766 | 6.777 | -1.630 | 1.00 | 13.85 | A |
| ATOM | 1081 | C | ALA | A | 168 | 14.874 | 7.320 | -3.797 | 1.00 | 17.50 | A |
| ATOM | 1082 | O | ALA | A | 168 | 15.974 | 6.820 | -4.048 | 1.00 | 19.05 | A |
| ATOM | 1083 | N | ARG | A | 169 | 13.924 | 7.447 | -4.718 | 1.00 | 18.39 | A |
| ATOM | 1084 | CA | ARG | A | 169 | 14.165 | 6.980 | -6.080 | 1.00 | 21.53 | A |
| ATOM | 1085 | CB | ARG | A | 169 | 12.872 | 7.017 | -6.908 | 1.00 | 23.66 | A |
| ATOM | 1086 | CG | ARG | A | 169 | 11.908 | 5.878 | -6.609 | 1.00 | 29.85 | A |
| ATOM | 1087 | CD | ARG | A | 169 | 10.905 | 5.670 | -7.748 | 1.00 | 34.11 | A |
| ATOM | 1088 | NE | ARG | A | 169 | 9.937 | 6.759 | -7.831 | 1.00 | 38.99 | A |
| ATOM | 1089 | CZ | ARG | A | 169 | 8.938 | 6.941 | -6.972 | 1.00 | 39.80 | A |
| ATOM | 1090 | NH1 | ARG | A | 169 | 8.768 | 6.100 | -5.962 | 1.00 | 41.20 | A |
| ATOM | 1091 | NH2 | ARG | A | 169 | 8.118 | 7.975 | -7.115 | 1.00 | 41.33 | A |
| ATOM | 1092 | C | ARG | A | 169 | 15.244 | 7.789 | -6.784 | 1.00 | 20.59 | A |

Figure 1 (suite 14)

| | | | | | | | | | | | |
|---|---|---|---|---|---|---|---|---|---|---|---|
| ATOM | 1093 | O | ARG | A | 169 | 16.041 | 7.248 | -7.549 | 1.00 | 21.56 | A |
| ATOM | 1094 | N | GLU | A | 170 | 15.284 | 9.087 | -6.514 | 1.00 | 20.38 | A |
| ATOM | 1095 | CA | GLU | A | 170 | 16.261 | 9.947 | -7.162 | 1.00 | 20.18 | A |
| ATOM | 1096 | CB | GLU | A | 170 | 15.717 | 11.379 | -7.242 | 1.00 | 20.53 | A |
| ATOM | 1097 | CG | GLU | A | 170 | 16.642 | 12.356 | -7.952 | 0.00 | 20.42 | A |
| ATOM | 1098 | CD | GLU | A | 170 | 16.080 | 13.763 | -8.003 | 0.00 | 20.49 | A |
| ATOM | 1099 | OE1 | GLU | A | 170 | 14.980 | 13.949 | -8.565 | 0.00 | 20.47 | A |
| ATOM | 1100 | OE2 | GLU | A | 170 | 16.741 | 14.685 | -7.483 | 0.00 | 20.47 | A |
| ATOM | 1101 | C | GLU | A | 170 | 17.655 | 9.978 | -6.535 | 1.00 | 20.13 | A |
| ATOM | 1102 | O | GLU | A | 170 | 18.656 | 9.983 | -7.249 | 1.00 | 19.29 | A |
| ATOM | 1103 | N | LEU | A | 171 | 17.724 | 9.974 | -5.210 | 1.00 | 18.95 | A |
| ATOM | 1104 | CA | LEU | A | 171 | 19.013 | 10.080 | -4.532 | 1.00 | 19.25 | A |
| ATOM | 1105 | CB | LEU | A | 171 | 18.895 | 11.111 | -3.406 | 1.00 | 19.75 | A |
| ATOM | 1106 | CG | LEU | A | 171 | 18.285 | 12.464 | -3.793 | 1.00 | 21.22 | A |
| ATOM | 1107 | CD1 | LEU | A | 171 | 18.361 | 13.405 | -2.602 | 1.00 | 20.37 | A |
| ATOM | 1108 | CD2 | LEU | A | 171 | 19.020 | 13.054 | -4.985 | 1.00 | 21.46 | A |
| ATOM | 1109 | C | LEU | A | 171 | 19.683 | 8.824 | -3.974 | 1.00 | 19.26 | A |
| ATOM | 1110 | O | LEU | A | 171 | 20.850 | 8.877 | -3.591 | 1.00 | 18.19 | A |
| ATOM | 1111 | N | SER | A | 172 | 18.973 | 7.703 | -3.917 | 1.00 | 20.57 | A |
| ATOM | 1112 | CA | SER | A | 172 | 19.576 | 6.493 | -3.365 | 1.00 | 21.80 | A |
| ATOM | 1113 | CB | SER | A | 172 | 18.566 | 5.340 | -3.377 | 1.00 | 21.56 | A |
| ATOM | 1114 | OG | SER | A | 172 | 17.549 | 5.573 | -2.414 | 1.00 | 18.56 | A |
| ATOM | 1115 | C | SER | A | 172 | 20.862 | 6.096 | -4.084 | 1.00 | 23.20 | A |
| ATOM | 1116 | O | SER | A | 172 | 21.728 | 5.443 | -3.505 | 1.00 | 21.26 | A |
| ATOM | 1117 | N | LYS | A | 173 | 20.997 | 6.513 | -5.337 | 1.00 | 24.05 | A |
| ATOM | 1118 | CA | LYS | A | 173 | 22.193 | 6.202 | -6.104 | 1.00 | 25.51 | A |
| ATOM | 1119 | CB | LYS | A | 173 | 22.012 | 6.609 | -7.572 | 1.00 | 28.04 | A |
| ATOM | 1120 | CG | LYS | A | 173 | 21.728 | 8.088 | -7.793 | 1.00 | 30.41 | A |
| ATOM | 1121 | CD | LYS | A | 173 | 21.664 | 8.422 | -9.281 | 1.00 | 33.57 | A |
| ATOM | 1122 | CE | LYS | A | 173 | 20.522 | 7.691 | -9.977 | 1.00 | 34.12 | A |
| ATOM | 1123 | NZ | LYS | A | 173 | 19.190 | 8.082 | -9.431 | 1.00 | 35.57 | A |
| ATOM | 1124 | C | LYS | A | 173 | 23.422 | 6.904 | -5.516 | 1.00 | 24.78 | A |
| ATOM | 1125 | O | LYS | A | 173 | 24.557 | 6.518 | -5.797 | 1.00 | 24.01 | A |
| ATOM | 1126 | N | ALA | A | 174 | 23.194 | 7.929 | -4.699 | 1.00 | 22.61 | A |
| ATOM | 1127 | CA | ALA | A | 174 | 24.291 | 8.668 | -4.077 | 1.00 | 22.27 | A |
| ATOM | 1128 | CB | ALA | A | 174 | 24.080 | 10.167 | -4.252 | 1.00 | 23.92 | A |
| ATOM | 1129 | C | ALA | A | 174 | 24.391 | 8.325 | -2.596 | 1.00 | 20.69 | A |
| ATOM | 1130 | O | ALA | A | 174 | 24.952 | 9.085 | -1.800 | 1.00 | 20.16 | A |
| ATOM | 1131 | N | ASN | A | 175 | 23.833 | 7.178 | -2.231 | 1.00 | 20.99 | A |
| ATOM | 1132 | CA | ASN | A | 175 | 23.851 | 6.721 | -0.850 | 1.00 | 21.32 | A |
| ATOM | 1133 | CB | ASN | A | 175 | 25.300 | 6.571 | -0.380 | 1.00 | 23.56 | A |
| ATOM | 1134 | CG | ASN | A | 175 | 25.403 | 6.021 | 1.021 | 1.00 | 26.10 | A |
| ATOM | 1135 | OD1 | ASN | A | 175 | 24.568 | 5.220 | 1.452 | 1.00 | 28.09 | A |
| ATOM | 1136 | ND2 | ASN | A | 175 | 26.442 | 6.438 | 1.744 | 1.00 | 25.64 | A |
| ATOM | 1137 | C | ASN | A | 175 | 23.065 | 7.657 | 0.065 | 1.00 | 20.91 | A |
| ATOM | 1138 | O | ASN | A | 175 | 23.411 | 7.859 | 1.230 | 1.00 | 21.02 | A |
| ATOM | 1139 | N | VAL | A | 176 | 22.002 | 8.239 | -0.484 | 1.00 | 20.91 | A |
| ATOM | 1140 | CA | VAL | A | 176 | 21.129 | 9.129 | 0.272 | 1.00 | 19.88 | A |
| ATOM | 1141 | CB | VAL | A | 176 | 21.014 | 10.526 | -0.383 | 1.00 | 22.38 | A |
| ATOM | 1142 | CG1 | VAL | A | 176 | 20.059 | 11.402 | 0.424 | 1.00 | 21.36 | A |
| ATOM | 1143 | CG2 | VAL | A | 176 | 22.387 | 11.185 | -0.463 | 1.00 | 20.27 | A |
| ATOM | 1144 | C | VAL | A | 176 | 19.758 | 8.471 | 0.267 | 1.00 | 19.58 | A |
| ATOM | 1145 | O | VAL | A | 176 | 19.120 | 8.352 | -0.783 | 1.00 | 18.58 | A |
| ATOM | 1146 | N | THR | A | 177 | 19.312 | 8.038 | 1.441 | 1.00 | 18.58 | A |
| ATOM | 1147 | CA | THR | A | 177 | 18.025 | 7.368 | 1.564 | 1.00 | 17.44 | A |
| ATOM | 1148 | CB | THR | A | 177 | 18.151 | 6.073 | 2.409 | 1.00 | 18.42 | A |
| ATOM | 1149 | OG1 | THR | A | 177 | 18.472 | 6.408 | 3.766 | 1.00 | 16.62 | A |
| ATOM | 1150 | CG2 | THR | A | 177 | 19.253 | 5.176 | 1.839 | 1.00 | 18.69 | A |
| ATOM | 1151 | C | THR | A | 177 | 16.960 | 8.251 | 2.197 | 1.00 | 16.37 | A |
| ATOM | 1152 | O | THR | A | 177 | 17.264 | 9.246 | 2.857 | 1.00 | 16.65 | A |
| ATOM | 1153 | N | ALA | A | 178 | 15.705 | 7.871 | 1.982 | 1.00 | 15.18 | A |
| ATOM | 1154 | CA | ALA | A | 178 | 14.565 | 8.592 | 2.524 | 1.00 | 11.61 | A |
| ATOM | 1155 | CB | ALA | A | 178 | 13.965 | 9.519 | 1.457 | 1.00 | 11.68 | A |
| ATOM | 1156 | C | ALA | A | 178 | 13.541 | 7.568 | 2.979 | 1.00 | 12.03 | A |
| ATOM | 1157 | O | ALA | A | 178 | 13.021 | 6.782 | 2.174 | 1.00 | 10.40 | A |
| ATOM | 1158 | N | ASN | A | 179 | 13.264 | 7.567 | 4.275 | 1.00 | 9.65 | A |
| ATOM | 1159 | CA | ASN | A | 179 | 12.308 | 6.638 | 4.852 | 1.00 | 8.92 | A |
| ATOM | 1160 | CB | ASN | A | 179 | 13.026 | 5.548 | 5.658 | 1.00 | 6.27 | A |
| ATOM | 1161 | CG | ASN | A | 179 | 13.906 | 4.669 | 4.791 | 1.00 | 10.03 | A |
| ATOM | 1162 | OD1 | ASN | A | 179 | 13.436 | 4.054 | 3.834 | 1.00 | 9.04 | A |
| ATOM | 1163 | ND2 | ASN | A | 179 | 15.198 | 4.604 | 5.123 | 1.00 | 8.31 | A |
| ATOM | 1164 | C | ASN | A | 179 | 11.354 | 7.380 | 5.756 | 1.00 | 11.05 | A |
| ATOM | 1165 | O | ASN | A | 179 | 11.632 | 8.497 | 6.189 | 1.00 | 10.62 | A |

Figure 1 (suite 15)

| | | | | | | | | | | | |
|---|---|---|---|---|---|---|---|---|---|---|---|
| ATOM | 1166 | N | VAL | A | 180 | 10.227 | 6.745 | 6.049 | 1.00 | 11.96 | A |
| ATOM | 1167 | CA | VAL | A | 180 | 9.222 | 7.343 | 6.904 | 1.00 | 10.82 | A |
| ATOM | 1168 | CB | VAL | A | 180 | 7.897 | 7.582 | 6.131 | 1.00 | 12.99 | A |
| ATOM | 1169 | CG1 | VAL | A | 180 | 6.858 | 8.181 | 7.052 | 1.00 | 11.39 | A |
| ATOM | 1170 | CG2 | VAL | A | 180 | 8.142 | 8.478 | 4.937 | 1.00 | 12.56 | A |
| ATOM | 1171 | C | VAL | A | 180 | 8.909 | 6.435 | 8.073 | 1.00 | 11.92 | A |
| ATOM | 1172 | O | VAL | A | 180 | 8.896 | 5.215 | 7.937 | 1.00 | 11.48 | A |
| ATOM | 1173 | N | VAL | A | 181 | 8.674 | 7.041 | 9.228 | 1.00 | 13.51 | A |
| ATOM | 1174 | CA | VAL | A | 181 | 8.297 | 6.304 | 10.419 | 1.00 | 12.14 | A |
| ATOM | 1175 | CB | VAL | A | 181 | 9.164 | 6.685 | 11.624 | 1.00 | 12.88 | A |
| ATOM | 1176 | CG1 | VAL | A | 181 | 8.569 | 6.102 | 12.891 | 1.00 | 13.00 | A |
| ATOM | 1177 | CG2 | VAL | A | 181 | 10.587 | 6.174 | 11.413 | 1.00 | 12.20 | A |
| ATOM | 1178 | C | VAL | A | 181 | 6.868 | 6.753 | 10.661 | 1.00 | 13.51 | A |
| ATOM | 1179 | O | VAL | A | 181 | 6.608 | 7.951 | 10.799 | 1.00 | 13.38 | A |
| ATOM | 1180 | N | ALA | A | 182 | 5.945 | 5.796 | 10.707 | 1.00 | 13.46 | A |
| ATOM | 1181 | CA | ALA | A | 182 | 4.533 | 6.110 | 10.905 | 1.00 | 14.68 | A |
| ATOM | 1182 | CB | ALA | A | 182 | 3.697 | 5.488 | 9.773 | 1.00 | 13.81 | A |
| ATOM | 1183 | C | ALA | A | 182 | 4.010 | 5.640 | 12.248 | 1.00 | 12.86 | A |
| ATOM | 1184 | O | ALA | A | 182 | 3.590 | 4.491 | 12.390 | 1.00 | 14.28 | A |
| ATOM | 1185 | N | PRO | A | 183 | 4.010 | 6.529 | 13.251 | 1.00 | 13.88 | A |
| ATOM | 1186 | CD | PRO | A | 183 | 4.481 | 7.926 | 13.208 | 1.00 | 12.89 | A |
| ATOM | 1187 | CA | PRO | A | 183 | 3.530 | 6.185 | 14.587 | 1.00 | 15.89 | A |
| ATOM | 1188 | CB | PRO | A | 183 | 4.052 | 7.329 | 15.442 | 1.00 | 16.27 | A |
| ATOM | 1189 | CG | PRO | A | 183 | 3.957 | 8.488 | 14.508 | 1.00 | 18.68 | A |
| ATOM | 1190 | C | PRO | A | 183 | 2.016 | 6.067 | 14.660 | 1.00 | 17.75 | A |
| ATOM | 1191 | O | PRO | A | 183 | 1.290 | 6.768 | 13.948 | 1.00 | 18.14 | A |
| ATOM | 1192 | N | GLY | A | 184 | 1.556 | 5.162 | 15.515 | 1.00 | 18.42 | A |
| ATOM | 1193 | CA | GLY | A | 184 | 0.135 | 4.974 | 15.706 | 1.00 | 20.98 | A |
| ATOM | 1194 | C | GLY | A | 184 | -0.249 | 5.795 | 16.918 | 1.00 | 23.52 | A |
| ATOM | 1195 | O | GLY | A | 184 | 0.110 | 6.972 | 17.013 | 1.00 | 23.14 | A |
| ATOM | 1196 | N | TYR | A | 185 | -0.962 | 5.181 | 17.854 | 1.00 | 23.18 | A |
| ATOM | 1197 | CA | TYR | A | 185 | -1.377 | 5.879 | 19.059 | 1.00 | 25.30 | A |
| ATOM | 1198 | CB | TYR | A | 185 | -2.586 | 5.173 | 19.674 | 1.00 | 31.38 | A |
| ATOM | 1199 | CG | TYR | A | 185 | -3.316 | 5.988 | 20.717 | 1.00 | 37.86 | A |
| ATOM | 1200 | CD1 | TYR | A | 185 | -3.832 | 7.249 | 20.412 | 1.00 | 40.80 | A |
| ATOM | 1201 | CE1 | TYR | A | 185 | -4.518 | 7.999 | 21.371 | 1.00 | 42.58 | A |
| ATOM | 1202 | CD2 | TYR | A | 185 | -3.509 | 5.494 | 22.005 | 1.00 | 40.79 | A |
| ATOM | 1203 | CE2 | TYR | A | 185 | -4.196 | 6.233 | 22.968 | 1.00 | 42.45 | A |
| ATOM | 1204 | CZ | TYR | A | 185 | -4.697 | 7.481 | 22.645 | 1.00 | 42.84 | A |
| ATOM | 1205 | OH | TYR | A | 185 | -5.368 | 8.208 | 23.599 | 1.00 | 44.77 | A |
| ATOM | 1206 | C | TYR | A | 185 | -0.214 | 5.896 | 20.048 | 1.00 | 23.99 | A |
| ATOM | 1207 | O | TYR | A | 185 | 0.092 | 4.880 | 20.669 | 1.00 | 22.19 | A |
| ATOM | 1208 | N | ILE | A | 186 | 0.439 | 7.050 | 20.176 | 1.00 | 23.70 | A |
| ATOM | 1209 | CA | ILE | A | 186 | 1.573 | 7.203 | 21.086 | 1.00 | 22.87 | A |
| ATOM | 1210 | CB | ILE | A | 186 | 2.792 | 7.829 | 20.373 | 1.00 | 21.42 | A |
| ATOM | 1211 | CG2 | ILE | A | 186 | 3.984 | 7.887 | 21.334 | 1.00 | 20.99 | A |
| ATOM | 1212 | CG1 | ILE | A | 186 | 3.154 | 7.016 | 19.127 | 1.00 | 19.18 | A |
| ATOM | 1213 | CD | ILE | A | 186 | 3.550 | 5.578 | 19.414 | 1.00 | 15.85 | A |
| ATOM | 1214 | C | ILE | A | 186 | 1.191 | 8.107 | 22.250 | 1.00 | 25.12 | A |
| ATOM | 1215 | O | ILE | A | 186 | 0.534 | 9.128 | 22.061 | 1.00 | 26.62 | A |
| ATOM | 1216 | N | ASP | A | 187 | 1.611 | 7.723 | 23.449 | 1.00 | 27.41 | A |
| ATOM | 1217 | CA | ASP | A | 187 | 1.319 | 8.481 | 24.660 | 1.00 | 30.93 | A |
| ATOM | 1218 | CB | ASP | A | 187 | 1.786 | 7.686 | 25.885 | 1.00 | 30.58 | A |
| ATOM | 1219 | CG | ASP | A | 187 | 1.394 | 8.338 | 27.202 | 1.00 | 33.98 | A |
| ATOM | 1220 | OD1 | ASP | A | 187 | 2.139 | 8.163 | 28.191 | 1.00 | 35.31 | A |
| ATOM | 1221 | OD2 | ASP | A | 187 | 0.342 | 9.008 | 27.261 | 1.00 | 34.41 | A |
| ATOM | 1222 | C | ASP | A | 187 | 2.027 | 9.838 | 24.627 | 1.00 | 33.51 | A |
| ATOM | 1223 | O | ASP | A | 187 | 3.221 | 9.927 | 24.908 | 1.00 | 33.49 | A |
| ATOM | 1224 | N | THR | A | 188 | 1.292 | 10.887 | 24.274 | 1.00 | 36.05 | A |
| ATOM | 1225 | CA | THR | A | 188 | 1.851 | 12.234 | 24.223 | 1.00 | 40.80 | A |
| ATOM | 1226 | CB | THR | A | 188 | 1.965 | 12.754 | 22.773 | 1.00 | 41.38 | A |
| ATOM | 1227 | OG1 | THR | A | 188 | 0.659 | 12.835 | 22.189 | 1.00 | 42.37 | A |
| ATOM | 1228 | CG2 | THR | A | 188 | 2.834 | 11.823 | 21.932 | 1.00 | 42.25 | A |
| ATOM | 1229 | C | THR | A | 188 | 0.944 | 13.182 | 24.999 | 1.00 | 43.80 | A |
| ATOM | 1230 | O | THR | A | 188 | -0.046 | 12.752 | 25.592 | 1.00 | 44.20 | A |
| ATOM | 1231 | N | ASP | A | 189 | 1.281 | 14.469 | 24.994 | 1.00 | 46.51 | A |
| ATOM | 1232 | CA | ASP | A | 189 | 0.478 | 15.464 | 25.696 | 1.00 | 48.42 | A |
| ATOM | 1233 | CB | ASP | A | 189 | 1.153 | 16.835 | 25.621 | 1.00 | 49.08 | A |
| ATOM | 1234 | CG | ASP | A | 189 | 2.465 | 16.897 | 26.404 | 0.00 | 48.79 | A |
| ATOM | 1235 | OD1 | ASP | A | 189 | 2.657 | 16.105 | 27.403 | 0.00 | 48.67 | A |
| ATOM | 1236 | OD2 | ASP | A | 189 | 3.377 | 17.743 | 26.065 | 0.00 | 48.67 | A |
| ATOM | 1237 | C | ASP | A | 189 | -0.902 | 15.555 | 25.055 | 1.00 | 49.70 | A |

Figure 1 (suite 16)

| | | | | | | | | | | | |
|---|---|---|---|---|---|---|---|---|---|---|---|
| ATOM | 1238 | O | ASP | A | 189 | -1.921 | 15.476 | 25.740 | 1.00 | 50.30 | A |
| ATOM | 1239 | N | MET | A | 190 | -0.928 | 15.718 | 23.736 | 1.00 | 51.13 | A |
| ATOM | 1240 | CA | MET | A | 190 | -2.187 | 15.801 | 23.004 | 1.00 | 52.10 | A |
| ATOM | 1241 | CB | MET | A | 190 | -1.999 | 15.873 | 21.525 | 1.00 | 51.92 | A |
| ATOM | 1242 | CG | MET | A | 190 | -1.367 | 17.202 | 21.136 | 0.00 | 51.43 | A |
| ATOM | 1243 | SD | MET | A | 190 | -2.425 | 18.590 | 21.404 | 0.00 | 51.38 | A |
| ATOM | 1244 | CE | MET | A | 190 | -3.663 | 18.639 | 20.134 | 0.00 | 51.45 | A |
| ATOM | 1245 | C | MET | A | 190 | -2.973 | 14.522 | 23.246 | 1.00 | 53.14 | A |
| ATOM | 1246 | O | MET | A | 190 | -4.192 | 14.550 | 23.405 | 1.00 | 53.88 | A |
| ATOM | 1247 | N | THR | A | 191 | -2.260 | 13.401 | 23.272 | 1.00 | 54.34 | A |
| ATOM | 1248 | CA | THR | A | 191 | -2.878 | 12.102 | 23.497 | 1.00 | 55.94 | A |
| ATOM | 1249 | CB | THR | A | 191 | -1.826 | 10.971 | 23.445 | 1.00 | 55.78 | A |
| ATOM | 1250 | OG1 | THR | A | 191 | -1.295 | 10.876 | 22.118 | 1.00 | 57.29 | A |
| ATOM | 1251 | CG2 | THR | A | 191 | -2.448 | 9.640 | 23.829 | 1.00 | 56.56 | A |
| ATOM | 1252 | C | THR | A | 191 | -3.582 | 12.066 | 24.849 | 1.00 | 56.53 | A |
| ATOM | 1253 | O | THR | A | 191 | -4.687 | 11.535 | 24.970 | 1.00 | 56.74 | A |
| ATOM | 1254 | N | ARG | A | 192 | -2.937 | 12.637 | 25.863 | 1.00 | 57.76 | A |
| ATOM | 1255 | CA | ARG | A | 192 | -3.505 | 12.669 | 27.204 | 1.00 | 58.88 | A |
| ATOM | 1256 | CB | ARG | A | 192 | -2.446 | 13.108 | 28.219 | 1.00 | 59.03 | A |
| ATOM | 1257 | CG | ARG | A | 192 | -1.243 | 12.182 | 28.298 | 1.00 | 60.71 | A |
| ATOM | 1258 | CD | ARG | A | 192 | -0.314 | 12.576 | 29.436 | 1.00 | 63.08 | A |
| ATOM | 1259 | NE | ARG | A | 192 | 0.860 | 11.710 | 29.510 | 1.00 | 65.16 | A |
| ATOM | 1260 | CZ | ARG | A | 192 | 1.754 | 11.746 | 30.494 | 1.00 | 67.49 | A |
| ATOM | 1261 | NH1 | ARG | A | 192 | 1.612 | 12.605 | 31.497 | 1.00 | 68.73 | A |
| ATOM | 1262 | NH2 | ARG | A | 192 | 2.796 | 10.923 | 30.477 | 1.00 | 68.21 | A |
| ATOM | 1263 | C | ARG | A | 192 | -4.704 | 13.609 | 27.271 | 1.00 | 59.14 | A |
| ATOM | 1264 | O | ARG | A | 192 | -5.660 | 13.355 | 28.003 | 1.00 | 59.61 | A |
| ATOM | 1265 | N | ALA | A | 193 | -4.650 | 14.691 | 26.501 | 1.00 | 59.40 | A |
| ATOM | 1266 | CA | ALA | A | 193 | -5.733 | 15.669 | 26.475 | 1.00 | 59.47 | A |
| ATOM | 1267 | CB | ALA | A | 193 | -5.328 | 16.873 | 25.632 | 1.00 | 59.49 | A |
| ATOM | 1268 | C | ALA | A | 193 | -7.018 | 15.056 | 25.925 | 1.00 | 60.08 | A |
| ATOM | 1269 | O | ALA | A | 193 | -8.116 | 15.537 | 26.209 | 1.00 | 60.25 | A |
| ATOM | 1270 | N | LEU | A | 194 | -6.875 | 13.993 | 25.139 | 1.00 | 59.81 | A |
| ATOM | 1271 | CA | LEU | A | 194 | -8.033 | 13.336 | 24.562 | 1.00 | 60.37 | A |
| ATOM | 1272 | CB | LEU | A | 194 | -7.570 | 12.174 | 23.651 | 0.00 | 60.75 | A |
| ATOM | 1273 | CG | LEU | A | 194 | -6.743 | 12.636 | 22.451 | 0.00 | 61.07 | A |
| ATOM | 1274 | CD1 | LEU | A | 194 | -6.198 | 11.475 | 21.620 | 0.00 | 60.59 | A |
| ATOM | 1275 | CD2 | LEU | A | 194 | -7.540 | 13.505 | 21.475 | 0.00 | 60.59 | A |
| ATOM | 1276 | C | LEU | A | 194 | -8.975 | 12.782 | 25.612 | 1.00 | 60.36 | A |
| ATOM | 1277 | O | LEU | A | 194 | -8.539 | 12.345 | 26.678 | 1.00 | 60.80 | A |
| ATOM | 1278 | N | ASP | A | 195 | -10.271 | 12.798 | 25.315 | 1.00 | 60.20 | A |
| ATOM | 1279 | CA | ASP | A | 195 | -11.271 | 12.294 | 26.249 | 1.00 | 59.11 | A |
| ATOM | 1280 | CB | ASP | A | 195 | -12.676 | 12.436 | 25.658 | 1.00 | 58.45 | A |
| ATOM | 1281 | CG | ASP | A | 195 | -13.762 | 12.011 | 26.630 | 1.00 | 58.11 | A |
| ATOM | 1282 | OD1 | ASP | A | 195 | -13.868 | 12.631 | 27.709 | 1.00 | 59.99 | A |
| ATOM | 1283 | OD2 | ASP | A | 195 | -14.508 | 11.059 | 26.318 | 1.00 | 59.85 | A |
| ATOM | 1284 | C | ASP | A | 195 | -11.000 | 10.831 | 26.575 | 1.00 | 59.03 | A |
| ATOM | 1285 | O | ASP | A | 195 | -10.573 | 10.062 | 25.712 | 1.00 | 59.30 | A |
| ATOM | 1286 | N | GLU | A | 196 | -11.250 | 10.455 | 27.826 | 1.00 | 57.91 | A |
| ATOM | 1287 | CA | GLU | A | 196 | -11.035 | 9.085 | 28.275 | 1.00 | 57.31 | A |
| ATOM | 1288 | CB | GLU | A | 196 | -11.623 | 8.894 | 29.681 | 0.00 | 56.62 | A |
| ATOM | 1289 | CG | GLU | A | 196 | -10.857 | 9.659 | 30.761 | 0.00 | 54.27 | A |
| ATOM | 1290 | CD | GLU | A | 196 | -11.408 | 9.419 | 32.167 | 0.00 | 52.79 | A |
| ATOM | 1291 | OE1 | GLU | A | 196 | -12.541 | 8.822 | 32.322 | 0.00 | 52.23 | A |
| ATOM | 1292 | OE2 | GLU | A | 196 | -10.745 | 9.818 | 33.196 | 0.00 | 52.23 | A |
| ATOM | 1293 | C | GLU | A | 196 | -11.695 | 8.087 | 27.329 | 1.00 | 57.38 | A |
| ATOM | 1294 | O | GLU | A | 196 | -11.125 | 7.040 | 27.023 | 1.00 | 58.77 | A |
| ATOM | 1295 | N | ARG | A | 197 | -12.895 | 8.420 | 26.867 | 1.00 | 56.47 | A |
| ATOM | 1296 | CA | ARG | A | 197 | -13.610 | 7.536 | 25.965 | 1.00 | 55.72 | A |
| ATOM | 1297 | CB | ARG | A | 197 | -14.987 | 8.147 | 25.618 | 0.00 | 54.23 | A |
| ATOM | 1298 | CG | ARG | A | 197 | -15.931 | 8.243 | 26.816 | 0.00 | 52.06 | A |
| ATOM | 1299 | CD | ARG | A | 197 | -17.252 | 8.938 | 26.483 | 0.00 | 50.09 | A |
| ATOM | 1300 | NE | ARG | A | 197 | -18.160 | 9.030 | 27.636 | 0.00 | 47.69 | A |
| ATOM | 1301 | CZ | ARG | A | 197 | -19.373 | 9.595 | 27.590 | 0.00 | 46.35 | A |
| ATOM | 1302 | NH1 | ARG | A | 197 | -19.845 | 10.124 | 26.454 | 0.00 | 45.21 | A |
| ATOM | 1303 | NH2 | ARG | A | 197 | -20.201 | 9.679 | 28.641 | 0.00 | 45.21 | A |
| ATOM | 1304 | C | ARG | A | 197 | -12.862 | 7.270 | 24.673 | 1.00 | 55.90 | A |
| ATOM | 1305 | O | ARG | A | 197 | -12.904 | 6.160 | 24.140 | 1.00 | 55.46 | A |
| ATOM | 1306 | N | ILE | A | 198 | -12.178 | 8.291 | 24.166 | 1.00 | 56.45 | A |
| ATOM | 1307 | CA | ILE | A | 198 | -11.419 | 8.168 | 22.928 | 1.00 | 56.36 | A |
| ATOM | 1308 | CB | ILE | A | 198 | -11.047 | 9.559 | 22.366 | 1.00 | 55.91 | A |
| ATOM | 1309 | CG2 | ILE | A | 198 | -10.254 | 9.406 | 21.074 | 1.00 | 55.67 | A |
| ATOM | 1310 | CG1 | ILE | A | 198 | -12.318 | 10.373 | 22.116 | 1.00 | 55.46 | A |

Figure 1 (suite 17)

| | | | | | | | | | | | |
|---|---|---|---|---|---|---|---|---|---|---|---|
| ATOM | 1311 | CD | ILE | A | 198 | -12.058 | 11.788 | 21.652 | 1.00 | 54.82 | A |
| ATOM | 1312 | C | ILE | A | 198 | -10.139 | 7.375 | 23.158 | 1.00 | 56.33 | A |
| ATOM | 1313 | O | ILE | A | 198 | -9.709 | 6.606 | 22.299 | 1.00 | 56.04 | A |
| ATOM | 1314 | N | GLN | A | 199 | -9.538 | 7.567 | 24.326 | 1.00 | 56.57 | A |
| ATOM | 1315 | CA | GLN | A | 199 | -8.306 | 6.875 | 24.670 | 1.00 | 56.71 | A |
| ATOM | 1316 | CB | GLN | A | 199 | -7.779 | 7.381 | 26.016 | 1.00 | 57.80 | A |
| ATOM | 1317 | CG | GLN | A | 199 | -7.377 | 8.852 | 26.005 | 1.00 | 58.57 | A |
| ATOM | 1318 | CD | GLN | A | 199 | -6.823 | 9.323 | 27.337 | 1.00 | 59.69 | A |
| ATOM | 1319 | OE1 | GLN | A | 199 | -6.411 | 10.477 | 27.480 | 1.00 | 59.54 | A |
| ATOM | 1320 | NE2 | GLN | A | 199 | -6.812 | 8.432 | 28.322 | 1.00 | 60.57 | A |
| ATOM | 1321 | C | GLN | A | 199 | -8.517 | 5.367 | 24.722 | 1.00 | 56.30 | A |
| ATOM | 1322 | O | GLN | A | 199 | -7.838 | 4.614 | 24.026 | 1.00 | 56.70 | A |
| ATOM | 1323 | N | GLN | A | 200 | -9.464 | 4.931 | 25.543 | 1.00 | 55.72 | A |
| ATOM | 1324 | CA | GLN | A | 200 | -9.754 | 3.510 | 25.674 | 1.00 | 55.24 | A |
| ATOM | 1325 | CB | GLN | A | 200 | -10.665 | 3.267 | 26.882 | 1.00 | 56.08 | A |
| ATOM | 1326 | CG | GLN | A | 200 | -10.058 | 3.698 | 28.209 | 0.00 | 56.67 | A |
| ATOM | 1327 | CD | GLN | A | 200 | -10.968 | 3.419 | 29.391 | 1.00 | 57.47 | A |
| ATOM | 1328 | OE1 | GLN | A | 200 | -10.624 | 3.714 | 30.536 | 0.00 | 57.40 | A |
| ATOM | 1329 | NE2 | GLN | A | 200 | -12.137 | 2.848 | 29.120 | 0.00 | 57.40 | A |
| ATOM | 1330 | C | GLN | A | 200 | -10.414 | 2.971 | 24.409 | 1.00 | 54.21 | A |
| ATOM | 1331 | O | GLN | A | 200 | -10.287 | 1.789 | 24.086 | 1.00 | 54.85 | A |
| ATOM | 1332 | N | GLY | A | 201 | -11.113 | 3.843 | 23.691 | 1.00 | 52.75 | A |
| ATOM | 1333 | CA | GLY | A | 201 | -11.787 | 3.427 | 22.473 | 1.00 | 51.07 | A |
| ATOM | 1334 | C | GLY | A | 201 | -10.836 | 3.073 | 21.346 | 1.00 | 49.97 | A |
| ATOM | 1335 | O | GLY | A | 201 | -11.130 | 2.206 | 20.521 | 1.00 | 49.37 | A |
| ATOM | 1336 | N | ALA | A | 202 | -9.693 | 3.748 | 21.310 | 1.00 | 48.87 | A |
| ATOM | 1337 | CA | ALA | A | 202 | -8.692 | 3.507 | 20.278 | 1.00 | 47.28 | A |
| ATOM | 1338 | CB | ALA | A | 202 | -7.640 | 4.606 | 20.315 | 1.00 | 47.00 | A |
| ATOM | 1339 | C | ALA | A | 202 | -8.027 | 2.145 | 20.448 | 1.00 | 45.94 | A |
| ATOM | 1340 | O | ALA | A | 202 | -7.634 | 1.512 | 19.469 | 1.00 | 45.51 | A |
| ATOM | 1341 | N | LEU | A | 203 | -7.904 | 1.699 | 21.695 | 1.00 | 44.75 | A |
| ATOM | 1342 | CA | LEU | A | 203 | -7.279 | 0.415 | 21.992 | 1.00 | 44.53 | A |
| ATOM | 1343 | CB | LEU | A | 203 | -7.263 | 0.180 | 23.505 | 1.00 | 45.36 | A |
| ATOM | 1344 | CG | LEU | A | 203 | -6.557 | 1.263 | 24.331 | 1.00 | 46.15 | A |
| ATOM | 1345 | CD1 | LEU | A | 203 | -6.649 | 0.936 | 25.810 | 1.00 | 46.43 | A |
| ATOM | 1346 | CD2 | LEU | A | 203 | -5.106 | 1.368 | 23.900 | 1.00 | 46.75 | A |
| ATOM | 1347 | C | LEU | A | 203 | -7.995 | -0.738 | 21.292 | 1.00 | 44.47 | A |
| ATOM | 1348 | O | LEU | A | 203 | -7.435 | -1.821 | 21.120 | 1.00 | 43.34 | A |
| ATOM | 1349 | N | GLN | A | 204 | -9.234 | -0.494 | 20.883 | 1.00 | 44.95 | A |
| ATOM | 1350 | CA | GLN | A | 204 | -10.034 | -1.503 | 20.202 | 1.00 | 45.67 | A |
| ATOM | 1351 | CB | GLN | A | 204 | -11.494 | -1.043 | 20.132 | 1.00 | 46.89 | A |
| ATOM | 1352 | CG | GLN | A | 204 | -12.462 | -2.073 | 19.577 | 1.00 | 48.62 | A |
| ATOM | 1353 | CD | GLN | A | 204 | -13.916 | -1.637 | 19.705 | 1.00 | 50.07 | A |
| ATOM | 1354 | OE1 | GLN | A | 204 | -14.833 | -2.393 | 19.378 | 1.00 | 50.85 | A |
| ATOM | 1355 | NE2 | GLN | A | 204 | -14.131 | -0.412 | 20.181 | 1.00 | 49.11 | A |
| ATOM | 1356 | C | GLN | A | 204 | -9.500 | -1.759 | 18.796 | 1.00 | 45.40 | A |
| ATOM | 1357 | O | GLN | A | 204 | -9.778 | -2.796 | 18.195 | 1.00 | 45.12 | A |
| ATOM | 1358 | N | PHE | A | 205 | -8.729 | -0.808 | 18.276 | 1.00 | 43.63 | A |
| ATOM | 1359 | CA | PHE | A | 205 | -8.168 | -0.930 | 16.936 | 1.00 | 42.40 | A |
| ATOM | 1360 | CB | PHE | A | 205 | -8.390 | 0.367 | 16.146 | 1.00 | 46.04 | A |
| ATOM | 1361 | CG | PHE | A | 205 | -9.838 | 0.707 | 15.920 | 1.00 | 48.65 | A |
| ATOM | 1362 | CD1 | PHE | A | 205 | -10.669 | 1.034 | 16.988 | 1.00 | 50.01 | A |
| ATOM | 1363 | CD2 | PHE | A | 205 | -10.371 | 0.700 | 14.634 | 1.00 | 50.38 | A |
| ATOM | 1364 | CE1 | PHE | A | 205 | -12.011 | 1.349 | 16.779 | 1.00 | 51.54 | A |
| ATOM | 1365 | CE2 | PHE | A | 205 | -11.712 | 1.011 | 14.412 | 1.00 | 51.46 | A |
| ATOM | 1366 | CZ | PHE | A | 205 | -12.534 | 1.338 | 15.486 | 1.00 | 51.71 | A |
| ATOM | 1367 | C | PHE | A | 205 | -6.675 | -1.243 | 16.966 | 1.00 | 38.91 | A |
| ATOM | 1368 | O | PHE | A | 205 | -6.008 | -1.208 | 15.934 | 1.00 | 38.36 | A |
| ATOM | 1369 | N | ILE | A | 206 | -6.158 | -1.554 | 18.150 | 1.00 | 34.31 | A |
| ATOM | 1370 | CA | ILE | A | 206 | -4.737 | -1.848 | 18.308 | 1.00 | 29.53 | A |
| ATOM | 1371 | CB | ILE | A | 206 | -4.083 | -0.853 | 19.291 | 1.00 | 29.34 | A |
| ATOM | 1372 | CG2 | ILE | A | 206 | -2.572 | -1.072 | 19.331 | 1.00 | 26.82 | A |
| ATOM | 1373 | CG1 | ILE | A | 206 | -4.402 | 0.582 | 18.864 | 1.00 | 28.56 | A |
| ATOM | 1374 | CD | ILE | A | 206 | -4.000 | 1.631 | 19.887 | 1.00 | 31.15 | A |
| ATOM | 1375 | C | ILE | A | 206 | -4.501 | -3.267 | 18.822 | 1.00 | 26.82 | A |
| ATOM | 1376 | O | ILE | A | 206 | -4.755 | -3.568 | 19.985 | 1.00 | 25.24 | A |
| ATOM | 1377 | N | PRO | A | 207 | -4.014 | -4.161 | 17.953 | 1.00 | 24.20 | A |
| ATOM | 1378 | CD | PRO | A | 207 | -3.760 | -3.998 | 16.510 | 1.00 | 23.75 | A |
| ATOM | 1379 | CA | PRO | A | 207 | -3.761 | -5.539 | 18.379 | 1.00 | 23.68 | A |
| ATOM | 1380 | CB | PRO | A | 207 | -3.018 | -6.131 | 17.186 | 1.00 | 22.65 | A |
| ATOM | 1381 | CG | PRO | A | 207 | -3.689 | -5.435 | 16.025 | 1.00 | 22.64 | A |
| ATOM | 1382 | C | PRO | A | 207 | -2.955 | -5.614 | 19.676 | 1.00 | 23.27 | A |
| ATOM | 1383 | O | PRO | A | 207 | -3.296 | -6.376 | 20.579 | 1.00 | 23.28 | A |

Figure 1 (suite 18)

| | | | | | | | | | | | |
|---|---|---|---|---|---|---|---|---|---|---|---|
| ATOM | 1384 | N | ALA | A | 208 | -1.895 | -4.815 | 19.768 | 1.00 | 21.13 | A |
| ATOM | 1385 | CA | ALA | A | 208 | -1.051 | -4.799 | 20.962 | 1.00 | 22.70 | A |
| ATOM | 1386 | CB | ALA | A | 208 | 0.089 | -3.804 | 20.779 | 1.00 | 21.24 | A |
| ATOM | 1387 | C | ALA | A | 208 | -1.856 | -4.455 | 22.218 | 1.00 | 24.38 | A |
| ATOM | 1388 | O | ALA | A | 208 | -1.413 | -4.714 | 23.338 | 1.00 | 23.78 | A |
| ATOM | 1389 | N | LYS | A | 209 | -3.036 | -3.867 | 22.022 | 1.00 | 25.04 | A |
| ATOM | 1390 | CA | LYS | A | 209 | -3.931 | -3.493 | 23.120 | 1.00 | 26.68 | A |
| ATOM | 1391 | CB | LYS | A | 209 | -4.430 | -4.748 | 23.845 | 1.00 | 27.64 | A |
| ATOM | 1392 | CG | LYS | A | 209 | -5.197 | -5.715 | 22.945 | 1.00 | 29.62 | A |
| ATOM | 1393 | CD | LYS | A | 209 | -6.376 | -5.024 | 22.264 | 1.00 | 32.24 | A |
| ATOM | 1394 | CE | LYS | A | 209 | -6.992 | -5.901 | 21.185 | 1.00 | 33.49 | A |
| ATOM | 1395 | NZ | LYS | A | 209 | -8.040 | -5.166 | 20.426 | 1.00 | 35.67 | A |
| ATOM | 1396 | C | LYS | A | 209 | -3.330 | -2.524 | 24.134 | 1.00 | 26.83 | A |
| ATOM | 1397 | O | LYS | A | 209 | -3.671 | -2.557 | 25.316 | 1.00 | 26.69 | A |
| ATOM | 1398 | N | ARG | A | 210 | -2.437 | -1.660 | 23.667 | 1.00 | 26.06 | A |
| ATOM | 1399 | CA | ARG | A | 210 | -1.808 | -0.667 | 24.528 | 1.00 | 24.55 | A |
| ATOM | 1400 | CB | ARG | A | 210 | -0.635 | -1.273 | 25.302 | 1.00 | 26.17 | A |
| ATOM | 1401 | CG | ARG | A | 210 | 0.609 | -1.524 | 24.456 | 1.00 | 25.44 | A |
| ATOM | 1402 | CD | ARG | A | 210 | 1.735 | -2.093 | 25.298 | 1.00 | 25.15 | A |
| ATOM | 1403 | NE | ARG | A | 210 | 2.889 | -2.478 | 24.491 | 1.00 | 23.76 | A |
| ATOM | 1404 | CZ | ARG | A | 210 | 3,804 | -1.634 | 24.024 | 1.00 | 23.62 | A |
| ATOM | 1405 | NH1 | ARG | A | 210 | 3.713 | -0.333 | 24.282 | 1.00 | 24.72 | A |
| ATOM | 1406 | NH2 | ARG | A | 210 | 4.814 | -2.095 | 23.296 | 1.00 | 24.36 | A |
| ATOM | 1407 | C | ARG | A | 210 | -1.294 | 0.474 | 23.669 | 1.00 | 24.54 | A |
| ATOM | 1408 | O | ARG | A | 210 | -1.135 | 0.328 | 22.457 | 1.00 | 22.67 | A |
| ATOM | 1409 | N | VAL | A | 211 | -1.036 | 1.607 | 24.312 | 1.00 | 23.88 | A |
| ATOM | 1410 | CA | VAL | A | 211 | -0.519 | 2.789 | 23.633 | 1.00 | 24.22 | A |
| ATOM | 1411 | CB | VAL | A | 211 | -0.834 | 4.072 | 24.441 | 1.00 | 24.53 | A |
| ATOM | 1412 | CG1 | VAL | A | 211 | -0.403 | 5.295 | 23.665 | 1.00 | 25.39 | A |
| ATOM | 1413 | CG2 | VAL | A | 211 | -2.318 | 4.136 | 24.754 | 1.00 | 26.13 | A |
| ATOM | 1414 | C | VAL | A | 211 | 1.001 | 2.633 | 23.529 | 1.00 | 22.56 | A |
| ATOM | 1415 | O | VAL | A | 211 | 1.611 | 1.957 | 24.354 | 1.00 | 22.76 | A |
| ATOM | 1416 | N | GLY | A | 212 | 1.605 | 3.245 | 22.517 | 1.00 | 21.91 | A |
| ATOM | 1417 | CA | GLY | A | 212 | 3.047 | 3.159 | 22.362 | 1.00 | 21.21 | A |
| ATOM | 1418 | C | GLY | A | 212 | 3.712 | 4.301 | 23.114 | 1.00 | 22.14 | A |
| ATOM | 1419 | O | GLY | A | 212 | 3.059 | 5.297 | 23.428 | 1.00 | 22.96 | A |
| ATOM | 1420 | N | THR | A | 213 | 5.000 | 4.167 | 23.415 | 1.00 | 22.49 | A |
| ATOM | 1421 | CA | THR | A | 213 | 5.718 | 5.224 | 24.133 | 1.00 | 23.38 | A |
| ATOM | 1422 | CB | THR | A | 213 | 6.645 | 4.656 | 25.220 | 1.00 | 23.84 | A |
| ATOM | 1423 | OG1 | THR | A | 213 | 7.676 | 3.878 | 24.601 | 1.00 | 25.92 | A |
| ATOM | 1424 | CG2 | THR | A | 213 | 5.868 | 3.784 | 26.195 | 1.00 | 23.81 | A |
| ATOM | 1425 | C | THR | A | 213 | 6.591 | 6.006 | 23.165 | 1.00 | 24.07 | A |
| ATOM | 1426 | O | THR | A | 213 | 6.944 | 5.511 | 22.095 | 1.00 | 23.85 | A |
| ATOM | 1427 | N | PRO | A | 214 | 6.942 | 7.251 | 23.521 | 1.00 | 23.55 | A |
| ATOM | 1428 | CD | PRO | A | 214 | 6.402 | 8.086 | 24.609 | 1.00 | 24.16 | A |
| ATOM | 1429 | CA | PRO | A | 214 | 7.788 | 8.043 | 22.625 | 1.00 | 22.85 | A |
| ATOM | 1430 | CB | PRO | A | 214 | 7.897 | 9.381 | 23.354 | 1.00 | 24.75 | A |
| ATOM | 1431 | CG | PRO | A | 214 | 6.564 | 9.483 | 24.044 | 1.00 | 24.18 | A |
| ATOM | 1432 | C | PRO | A | 214 | 9.142 | 7.362 | 22.424 | 1.00 | 21.96 | A |
| ATOM | 1433 | O | PRO | A | 214 | 9.771 | 7.503 | 21.380 | 1.00 | 21.58 | A |
| ATOM | 1434 | N | ALA | A | 215 | 9.577 | 6.615 | 23.433 | 1.00 | 21.21 | A |
| ATOM | 1435 | CA | ALA | A | 215 | 10.845 | 5.896 | 23.362 | 1.00 | 20.67 | A |
| ATOM | 1436 | CB | ALA | A | 215 | 11.177 | 5.283 | 24.714 | 1.00 | 21.56 | A |
| ATOM | 1437 | C | ALA | A | 215 | 10.755 | 4.802 | 22.301 | 1.00 | 19.68 | A |
| ATOM | 1438 | O | ALA | A | 215 | 11.742 | 4.487 | 21.637 | 1.00 | 19.73 | A |
| ATOM | 1439 | N | GLU | A | 216 | 9.569 | 4.221 | 22.142 | 1.00 | 18.12 | A |
| ATOM | 1440 | CA | GLU | A | 216 | 9.397 | 3.175 | 21.143 | 1.00 | 17.48 | A |
| ATOM | 1441 | CB | GLU | A | 216 | 8.078 | 2.434 | 21.371 | 1.00 | 17.90 | A |
| ATOM | 1442 | CG | GLU | A | 216 | 8.135 | 1.523 | 22.605 | 1.00 | 19.26 | A |
| ATOM | 1443 | CD | GLU | A | 216 | 6.830 | 0.803 | 22.889 | 1.00 | 19.62 | A |
| ATOM | 1444 | OE1 | GLU | A | 216 | 5.794 | 1.478 | 23.045 | 1.00 | 19.87 | A |
| ATOM | 1445 | OE2 | GLU | A | 216 | 6.843 | -0.443 | 22.959 | 1.00 | 23.45 | A |
| ATOM | 1446 | C | GLU | A | 216 | 9.499 | 3.733 | 19.730 | 1.00 | 16.20 | A |
| ATOM | 1447 | O | GLU | A | 216 | 9.902 | 3.026 | 18.815 | 1.00 | 16.19 | A |
| ATOM | 1448 | N | VAL | A | 217 | 9.144 | 5.005 | 19.550 | 1.00 | 15.93 | A |
| ATOM | 1449 | CA | VAL | A | 217 | 9.267 | 5.628 | 18.235 | 1.00 | 14.81 | A |
| ATOM | 1450 | CB | VAL | A | 217 | 8.446 | 6.946 | 18.118 | 1.00 | 15.91 | A |
| ATOM | 1451 | CG1 | VAL | A | 217 | 8.668 | 7.560 | 16.739 | 1.00 | 12.69 | A |
| ATOM | 1452 | CG2 | VAL | A | 217 | 6.963 | 6.677 | 18.333 | 1.00 | 15.53 | A |
| ATOM | 1453 | C | VAL | A | 217 | 10.748 | 5.972 | 18.030 | 1.00 | 14.18 | A |
| ATOM | 1454 | O | VAL | A | 217 | 11.313 | 5.744 | 16.963 | 1.00 | 13.12 | A |
| ATOM | 1455 | N | ALA | A | 218 | 11.366 | 6.532 | 19.066 | 1.00 | 13.15 | A |
| ATOM | 1456 | CA | ALA | A | 218 | 12.780 | 6.915 | 19.017 | 1.00 | 13.00 | A |

Figure 1 (suite 19)

| | | | | | | | | | | | |
|---|---|---|---|---|---|---|---|---|---|---|---|
| ATOM | 1457 | CB | ALA | A | 218 | 13.205 | 7.500 | 20.363 | 1.00 | 11.76 | A |
| ATOM | 1458 | C | ALA | A | 218 | 13.654 | 5.710 | 18.662 | 1.00 | 12.56 | A |
| ATOM | 1459 | O | ALA | A | 218 | 14.597 | 5.819 | 17.884 | 1.00 | 12.29 | A |
| ATOM | 1460 | N | GLY | A | 219 | 13.330 | 4.555 | 19.233 | 1.00 | 11.87 | A |
| ATOM | 1461 | CA | GLY | A | 219 | 14.100 | 3.357 | 18.939 | 1.00 | 12.40 | A |
| ATOM | 1462 | C | GLY | A | 219 | 14.140 | 3.062 | 17.449 | 1.00 | 13.11 | A |
| ATOM | 1463 | O | GLY | A | 219 | 15.151 | 2.605 | 16.913 | 1.00 | 14.88 | A |
| ATOM | 1464 | N | VAL | A | 220 | 13.040 | 3.330 | 16.757 | 1.00 | 12.06 | A |
| ATOM | 1465 | CA | VAL | A | 220 | 13.000 | 3.066 | 15.323 | 1.00 | 12.41 | A |
| ATOM | 1466 | CB | VAL | A | 220 | 11.549 | 3.116 | 14.778 | 1.00 | 15.16 | A |
| ATOM | 1467 | CG1 | VAL | A | 220 | 11.537 | 2.702 | 13.312 | 1.00 | 15.52 | A |
| ATOM | 1468 | CG2 | VAL | A | 220 | 10.653 | 2.201 | 15.609 | 1.00 | 17.97 | A |
| ATOM | 1469 | C | VAL | A | 220 | 13.850 | 4.073 | 14.563 | 1.00 | 10.59 | A |
| ATOM | 1470 | O | VAL | A | 220 | 14.525 | 3.728 | 13.598 | 1.00 | 10.11 | A |
| ATOM | 1471 | N | VAL | A | 221 | 13.810 | 5.328 | 14.988 | 1.00 | 8.27 | A |
| ATOM | 1472 | CA | VAL | A | 221 | 14.612 | 6.345 | 14.317 | 1.00 | 10.77 | A |
| ATOM | 1473 | CB | VAL | A | 221 | 14.281 | 7.748 | 14.868 | 1.00 | 7.98 | A |
| ATOM | 1474 | CG1 | VAL | A | 221 | 15.043 | 8.814 | 14.085 | 1.00 | 8.84 | A |
| ATOM | 1475 | CG2 | VAL | A | 221 | 12.778 | 7.985 | 14.768 | 1.00 | 12.72 | A |
| ATOM | 1476 | C | VAL | A | 221 | 16.100 | 6.027 | 14.511 | 1.00 | 10.38 | A |
| ATOM | 1477 | O | VAL | A | 221 | 16.904 | 6.181 | 13.592 | 1.00 | 10.29 | A |
| ATOM | 1478 | N | SER | A | 222 | 16.455 | 5.571 | 15.709 | 1.00 | 11.81 | A |
| ATOM | 1479 | CA | SER | A | 222 | 17.847 | 5.207 | 16.004 | 1.00 | 11.68 | A |
| ATOM | 1480 | CB | SER | A | 222 | 17.949 | 4.589 | 17.404 | 1.00 | 11.60 | A |
| ATOM | 1481 | OG | SER | A | 222 | 19.281 | 4.177 | 17.672 | 1.00 | 11.75 | A |
| ATOM | 1482 | C | SER | A | 222 | 18.303 | 4.195 | 14.957 | 1.00 | 11.93 | A |
| ATOM | 1483 | O | SER | A | 222 | 19.362 | 4.335 | 14.351 | 1.00 | 10.07 | A |
| ATOM | 1484 | N | PHE | A | 223 | 17.478 | 3.180 | 14.733 | 1.00 | 10.90 | A |
| ATOM | 1485 | CA | PHE | A | 223 | 17.797 | 2.165 | 13.744 | 1.00 | 13.00 | A |
| ATOM | 1486 | CB | PHE | A | 223 | 16.700 | 1.096 | 13.692 | 1.00 | 13.06 | A |
| ATOM | 1487 | CG | PHE | A | 223 | 16.796 | 0.201 | 12.488 | 1.00 | 13.47 | A |
| ATOM | 1488 | CD1 | PHE | A | 223 | 17.875 | -0.657 | 12.332 | 1.00 | 12.72 | A |
| ATOM | 1489 | CD2 | PHE | A | 223 | 15.828 | 0.252 | 11.486 | 1.00 | 13.71 | A |
| ATOM | 1490 | CE1 | PHE | A | 223 | 17.998 | -1.451 | 11.191 | 1.00 | 14.85 | A |
| ATOM | 1491 | CE2 | PHE | A | 223 | 15.938 | -0.535 | 10.346 | 1.00 | 13.68 | A |
| ATOM | 1492 | CZ | PHE | A | 223 | 17.023 | -1.387 | 10.196 | 1.00 | 14.01 | A |
| ATOM | 1493 | C | PHE | A | 223 | 17.957 | 2.774 | 12.357 | 1.00 | 11.43 | A |
| ATOM | 1494 | O | PHE | A | 223 | 18.932 | 2.509 | 11.655 | 1.00 | 11.31 | A |
| ATOM | 1495 | N | LEU | A | 224 | 16.995 | 3.598 | 11.956 | 1.00 | 11.82 | A |
| ATOM | 1496 | CA | LEU | A | 224 | 17.054 | 4.210 | 10.631 | 1.00 | 12.00 | A |
| ATOM | 1497 | CB | LEU | A | 224 | 15.741 | 4.945 | 10.326 | 1.00 | 11.46 | A |
| ATOM | 1498 | CG | LEU | A | 224 | 14.555 | 4.010 | 10.046 | 1.00 | 12.36 | A |
| ATOM | 1499 | CD1 | LEU | A | 224 | 13.299 | 4.841 | 9.748 | 1.00 | 10.87 | A |
| ATOM | 1500 | CD2 | LEU | A | 224 | 14.892 | 3.100 | 8.871 | 1.00 | 9.91 | A |
| ATOM | 1501 | C | LEU | A | 224 | 18.242 | 5.150 | 10.449 | 1.00 | 12.44 | A |
| ATOM | 1502 | O | LEU | A | 224 | 18.720 | 5.344 | 9.336 | 1.00 | 13.30 | A |
| ATOM | 1503 | N | ALA | A | 225 | 18.724 | 5.731 | 11.540 | 1.00 | 12.87 | A |
| ATOM | 1504 | CA | ALA | A | 225 | 19.874 | 6.634 | 11.453 | 1.00 | 14.05 | A |
| ATOM | 1505 | CB | ALA | A | 225 | 19.798 | 7.680 | 12.556 | 1.00 | 14.94 | A |
| ATOM | 1506 | C | ALA | A | 225 | 21.198 | 5.865 | 11.546 | 1.00 | 16.31 | A |
| ATOM | 1507 | O | ALA | A | 225 | 22.263 | 6.416 | 11.276 | 1.00 | 20.25 | A |
| ATOM | 1508 | N | SER | A | 226 | 21.126 | 4.581 | 11.887 | 1.00 | 14.78 | A |
| ATOM | 1509 | CA | SER | A | 226 | 22.329 | 3.759 | 12.039 | 1.00 | 13.37 | A |
| ATOM | 1510 | CB | SER | A | 226 | 22.042 | 2.605 | 12.997 | 1.00 | 11.54 | A |
| ATOM | 1511 | OG | SER | A | 226 | 21.307 | 1.593 | 12.331 | 1.00 | 10.86 | A |
| ATOM | 1512 | C | SER | A | 226 | 22.901 | 3.184 | 10.745 | 1.00 | 14.81 | A |
| ATOM | 1513 | O | SER | A | 226 | 22.285 | 3.251 | 9.680 | 1.00 | 15.76 | A |
| ATOM | 1514 | N | GLU | A | 227 | 24.087 | 2.594 | 10.858 | 1.00 | 15.68 | A |
| ATOM | 1515 | CA | GLU | A | 227 | 24.770 | 1.978 | 9.725 | 1.00 | 17.13 | A |
| ATOM | 1516 | CB | GLU | A | 227 | 26.212 | 1.639 | 10.112 | 1.00 | 19.25 | A |
| ATOM | 1517 | CG | GLU | A | 227 | 27.074 | 2.864 | 10.382 | 1.00 | 19.74 | A |
| ATOM | 1518 | CD | GLU | A | 227 | 27.436 | 3.589 | 9.111 | 1.00 | 22.70 | A |
| ATOM | 1519 | OE1 | GLU | A | 227 | 28.346 | 3.115 | 8.400 | 1.00 | 23.52 | A |
| ATOM | 1520 | OE2 | GLU | A | 227 | 26.802 | 4.622 | 8.811 | 1.00 | 22.66 | A |
| ATOM | 1521 | C | GLU | A | 227 | 24.059 | 0.711 | 9.239 | 1.00 | 18.24 | A |
| ATOM | 1522 | O | GLU | A | 227 | 24.287 | 0.257 | 8.120 | 1.00 | 17.79 | A |
| ATOM | 1523 | N | ASP | A | 228 | 23.201 | 0.146 | 10.081 | 1.00 | 15.31 | A |
| ATOM | 1524 | CA | ASP | A | 228 | 22.454 | -1.065 | 9.726 | 1.00 | 15.06 | A |
| ATOM | 1525 | CB | ASP | A | 228 | 21.844 | -1.686 | 10.980 | 1.00 | 14.06 | A |
| ATOM | 1526 | CG | ASP | A | 228 | 22.837 | -2.529 | 11.758 | 1.00 | 16.84 | A |
| ATOM | 1527 | OD1 | ASP | A | 228 | 22.730 | -2.559 | 13.002 | 1.00 | 15.62 | A |
| ATOM | 1528 | OD2 | ASP | A | 228 | 23.702 | -3.167 | 11.116 | 1.00 | 13.83 | A |
| ATOM | 1529 | C | ASP | A | 228 | 21.328 | -0.841 | 8.713 | 1.00 | 14.88 | A |

Figure 1 (suite 20)

| | | | | | | | | | | | |
|---|---|---|---|---|---|---|---|---|---|---|---|
| ATOM | 1530 | O | ASP | A | 228 | 20.845 | -1.797 | 8.101 | 1.00 | 15.10 | A |
| ATOM | 1531 | N | ALA | A | 229 | 20.918 | 0.411 | 8.531 | 1.00 | 13.79 | A |
| ATOM | 1532 | CA | ALA | A | 229 | 19.810 | 0.731 | 7.625 | 1.00 | 14.58 | A |
| ATOM | 1533 | CB | ALA | A | 229 | 18.940 | 1.812 | 8.269 | 1.00 | 14.59 | A |
| ATOM | 1534 | C | ALA | A | 229 | 20.186 | 1.152 | 6.206 | 1.00 | 14.91 | A |
| ATOM | 1535 | O | ALA | A | 229 | 19.335 | 1.637 | 5.455 | 1.00 | 13.14 | A |
| ATOM | 1536 | N | SER | A | 230 | 21.444 | 0.944 | 5.830 | 1.00 | 14.75 | A |
| ATOM | 1537 | CA | SER | A | 230 | 21.942 | 1.347 | 4.517 | 1.00 | 18.10 | A |
| ATOM | 1538 | CB | SER | A | 230 | 23.419 | 0.963 | 4.392 | 1.00 | 16.96 | A |
| ATOM | 1539 | OG | SER | A | 230 | 23.604 | -0.419 | 4.669 | 1.00 | 21.70 | A |
| ATOM | 1540 | C | SER | A | 230 | 21.186 | 0.860 | 3.271 | 1.00 | 17.32 | A |
| ATOM | 1541 | O | SER | A | 230 | 21.159 | 1.563 | 2.262 | 1.00 | 18.39 | A |
| ATOM | 1542 | N | TYR | A | 231 | 20.580 | -0.322 | 3.320 | 1.00 | 14.83 | A |
| ATOM | 1543 | CA | TYR | A | 231 | 19.871 | -0.815 | 2.141 | 1.00 | 15.89 | A |
| ATOM | 1544 | CB | TYR | A | 231 | 20.229 | -2.282 | 1.872 | 1.00 | 16.41 | A |
| ATOM | 1545 | CG | TYR | A | 231 | 20.124 | -2.670 | 0.409 | 1.00 | 17.87 | A |
| ATOM | 1546 | CD1 | TYR | A | 231 | 20.817 | -1.956 | -0.570 | 1.00 | 18.93 | A |
| ATOM | 1547 | CE1 | TYR | A | 231 | 20.738 | -2.313 | -1.914 | 1.00 | 19.50 | A |
| ATOM | 1548 | CD2 | TYR | A | 231 | 19.345 | -3.755 | 0.004 | 1.00 | 17.16 | A |
| ATOM | 1549 | CE2 | TYR | A | 231 | 19.260 | -4.121 | -1.340 | 1.00 | 18.72 | A |
| ATOM | 1550 | CZ | TYR | A | 231 | 19.960 | -3.396 | -2.291 | 1.00 | 21.53 | A |
| ATOM | 1551 | OH | TYR | A | 231 | 19.893 | -3.751 | -3.619 | 1.00 | 21.39 | A |
| ATOM | 1552 | C | TYR | A | 231 | 18.350 | -0.651 | 2.235 | 1.00 | 15.13 | A |
| ATOM | 1553 | O | TYR | A | 231 | 17.599 | -1.279 | 1.492 | 1.00 | 14.58 | A |
| ATOM | 1554 | N | ILE | A | 232 | 17.904 | 0.191 | 3.159 | 1.00 | 14.48 | A |
| ATOM | 1555 | CA | ILE | A | 232 | 16.486 | 0.467 | 3.314 | 1.00 | 13.88 | A |
| ATOM | 1556 | CB | ILE | A | 232 | 16.044 | 0.409 | 4.785 | 1.00 | 14.90 | A |
| ATOM | 1557 | CG2 | ILE | A | 232 | 14.571 | 0.777 | 4.891 | 1.00 | 12.11 | A |
| ATOM | 1558 | CG1 | ILE | A | 232 | 16.292 | -0.995 | 5.355 | 1.00 | 17.03 | A |
| ATOM | 1559 | CD | ILE | A | 232 | 15.879 | -1.147 | 6.806 | 1.00 | 17.74 | A |
| ATOM | 1560 | C | ILE | A | 232 | 16.260 | 1.887 | 2.797 | 1.00 | 12.70 | A |
| ATOM | 1561 | O | ILE | A | 232 | 16.863 | 2.836 | 3.299 | 1.00 | 11.87 | A |
| ATOM | 1562 | N | SER | A | 233 | 15.413 | 2.026 | 1.781 | 1.00 | 12.77 | A |
| ATOM | 1563 | CA | SER | A | 233 | 15.113 | 3.339 | 1.221 | 1.00 | 13.71 | A |
| ATOM | 1564 | CB | SER | A | 233 | 16.160 | 3.737 | 0.180 | 1.00 | 14.41 | A |
| ATOM | 1565 | OG | SER | A | 233 | 16.096 | 5.124 | -0.111 | 1.00 | 16.79 | A |
| ATOM | 1566 | C | SER | A | 233 | 13.726 | 3.323 | 0.577 | 1.00 | 13.59 | A |
| ATOM | 1567 | O | SER | A | 233 | 13.380 | 2.390 | -0.151 | 1.00 | 14.09 | A |
| ATOM | 1568 | N | GLY | A | 234 | 12.941 | 4.357 | 0.866 | 1.00 | 12.52 | A |
| ATOM | 1569 | CA | GLY | A | 234 | 11.602 | 4.463 | 0.315 | 1.00 | 13.35 | A |
| ATOM | 1570 | C | GLY | A | 234 | 10.622 | 3.613 | 1.086 | 1.00 | 13.52 | A |
| ATOM | 1571 | O | GLY | A | 234 | 9.530 | 3.304 | 0.599 | 1.00 | 15.12 | A |
| ATOM | 1572 | N | ALA | A | 235 | 11.005 | 3.251 | 2.304 | 1.00 | 12.40 | A |
| ATOM | 1573 | CA | ALA | A | 235 | 10.178 | 2.416 | 3.156 | 1.00 | 11.39 | A |
| ATOM | 1574 | CB | ALA | A | 235 | 11.063 | 1.378 | 3.877 | 1.00 | 8.92 | A |
| ATOM | 1575 | C | ALA | A | 235 | 9.396 | 3.216 | 4.181 | 1.00 | 11.43 | A |
| ATOM | 1576 | O | ALA | A | 235 | 9.801 | 4.312 | 4.571 | 1.00 | 9.78 | A |
| ATOM | 1577 | N | VAL | A | 236 | 8.267 | 2.656 | 4.606 | 1.00 | 10.72 | A |
| ATOM | 1578 | CA | VAL | A | 236 | 7.426 | 3.267 | 5.619 | 1.00 | 11.96 | A |
| ATOM | 1579 | CB | VAL | A | 236 | 5.971 | 3.513 | 5.127 | 1.00 | 13.74 | A |
| ATOM | 1580 | CG1 | VAL | A | 236 | 5.131 | 4.071 | 6.273 | 1.00 | 13.62 | A |
| ATOM | 1581 | CG2 | VAL | A | 236 | 5.965 | 4.473 | 3.957 | 1.00 | 9.57 | A |
| ATOM | 1582 | C | VAL | A | 236 | 7.357 | 2.274 | 6.763 | 1.00 | 13.54 | A |
| ATOM | 1583 | O | VAL | A | 236 | 6.829 | 1.173 | 6.600 | 1.00 | 13.00 | A |
| ATOM | 1584 | N | ILE | A | 237 | 7.884 | 2.648 | 7.921 | 1.00 | 11.49 | A |
| ATOM | 1585 | CA | ILE | A | 237 | 7.836 | 1.731 | 9.042 | 1.00 | 13.11 | A |
| ATOM | 1586 | CB | ILE | A | 237 | 9.221 | 1.587 | 9.729 | 1.00 | 13.46 | A |
| ATOM | 1587 | CG2 | ILE | A | 237 | 9.130 | 0.577 | 10.869 | 1.00 | 12.87 | A |
| ATOM | 1588 | CG1 | ILE | A | 237 | 10.260 | 1.128 | 8.697 | 1.00 | 14.72 | A |
| ATOM | 1589 | CD | ILE | A | 237 | 11.626 | 0.791 | 9.277 | 1.00 | 17.74 | A |
| ATOM | 1590 | C | ILE | A | 237 | 6.799 | 2.151 | 10.062 | 1.00 | 11.70 | A |
| ATOM | 1591 | O | ILE | A | 237 | 6.899 | 3.207 | 10.680 | 1.00 | 14.22 | A |
| ATOM | 1592 | N | PRO | A | 238 | 5.764 | 1.321 | 10.241 | 1.00 | 13.77 | A |
| ATOM | 1593 | CD | PRO | A | 238 | 5.424 | 0.131 | 9.439 | 1.00 | 11.22 | A |
| ATOM | 1594 | CA | PRO | A | 238 | 4.707 | 1.625 | 11.203 | 1.00 | 12.82 | A |
| ATOM | 1595 | CB | PRO | A | 238 | 3.526 | 0.823 | 10.666 | 1.00 | 14.23 | A |
| ATOM | 1596 | CG | PRO | A | 238 | 4.191 | -0.400 | 10.142 | 1.00 | 13.12 | A |
| ATOM | 1597 | C | PRO | A | 238 | 5.112 | 1.198 | 12.611 | 1.00 | 14.02 | A |
| ATOM | 1598 | O | PRO | A | 238 | 5.627 | 0.097 | 12.810 | 1.00 | 12.20 | A |
| ATOM | 1599 | N | VAL | A | 239 | 4.904 | 2.091 | 13.573 | 1.00 | 13.77 | A |
| ATOM | 1600 | CA | VAL | A | 239 | 5.209 | 1.824 | 14.977 | 1.00 | 15.10 | A |
| ATOM | 1601 | CB | VAL | A | 239 | 6.234 | 2.852 | 15.539 | 1.00 | 16.14 | A |
| ATOM | 1602 | CG1 | VAL | A | 239 | 6.644 | 2.459 | 16.949 | 1.00 | 18.24 | A |

Figure 1 (suite 21)

| | | | | | | | | | | | |
|---|---|---|---|---|---|---|---|---|---|---|---|
| ATOM | 1603 | CG2 | VAL | A | 239 | 7.433 | 2.934 | 14.629 | 1.00 | 15.98 | A |
| ATOM | 1604 | C | VAL | A | 239 | 3.857 | 2.042 | 15.616 | 1.00 | 13.91 | A |
| ATOM | 1605 | O | VAL | A | 239 | 3.599 | 3.087 | 16.219 | 1.00 | 13.72 | A |
| ATOM | 1606 | N | ASP | A | 240 | 2.989 | 1.044 | 15.476 | 1.00 | 13.39 | A |
| ATOM | 1607 | CA | ASP | A | 240 | 1.620 | 1.173 | 15.953 | 1.00 | 15.72 | A |
| ATOM | 1608 | CB | ASP | A | 240 | 0.728 | 1.531 | 14.765 | 1.00 | 16.17 | A |
| ATOM | 1609 | CG | ASP | A | 240 | 0.877 | 0.547 | 13.609 | 1.00 | 17.37 | A |
| ATOM | 1610 | OD1 | ASP | A | 240 | 1.387 | -0.574 | 13.833 | 1.00 | 17.57 | A |
| ATOM | 1611 | OD2 | ASP | A | 240 | 0.478 | 0.887 | 12.479 | 1.00 | 19.02 | A |
| ATOM | 1612 | C | ASP | A | 240 | 1.010 | -0.029 | 16.656 | 1.00 | 16.35 | A |
| ATOM | 1613 | O | ASP | A | 240 | -0.211 | -0.090 | 16.823 | 1.00 | 17.69 | A |
| ATOM | 1614 | N | GLY | A | 241 | 1.838 | -0.978 | 17.068 | 1.00 | 18.27 | A |
| ATOM | 1615 | CA | GLY | A | 241 | 1.302 | -2.147 | 17.735 | 1.00 | 19.02 | A |
| ATOM | 1616 | C | GLY | A | 241 | 0.361 | -2.908 | 16.819 | 1.00 | 20.37 | A |
| ATOM | 1617 | O | GLY | A | 241 | -0.564 | -3.573 | 17.285 | 1.00 | 19.21 | A |
| ATOM | 1618 | N | GLY | A | 242 | 0.592 | -2.797 | 15.515 | 1.00 | 20.61 | A |
| ATOM | 1619 | CA | GLY | A | 242 | -0.233 | -3.496 | 14.546 | 1.00 | 22.92 | A |
| ATOM | 1620 | C | GLY | A | 242 | -1.503 | -2.784 | 14.114 | 1.00 | 24.90 | A |
| ATOM | 1621 | O | GLY | A | 242 | -2.257 | -3.308 | 13.292 | 1.00 | 24.39 | A |
| ATOM | 1622 | N | MET | A | 243 | -1.744 | -1.593 | 14.656 | 1.00 | 27.36 | A |
| ATOM | 1623 | CA | MET | A | 243 | -2.946 | -0.834 | 14.327 | 1.00 | 30.21 | A |
| ATOM | 1624 | CB | MET | A | 243 | -2.884 | 0.571 | 14.933 | 1.00 | 31.72 | A |
| ATOM | 1625 | CG | MET | A | 243 | -4.178 | 1.354 | 14.765 | 1.00 | 36.26 | A |
| ATOM | 1626 | SD | MET | A | 243 | -4.006 | 3.118 | 15.068 | 1.00 | 41.87 | A |
| ATOM | 1627 | CE | MET | A | 243 | -4.332 | 3.214 | 16.812 | 1.00 | 41.19 | A |
| ATOM | 1628 | C | MET | A | 243 | -3.154 | -0.712 | 12.821 | 1.00 | 31.25 | A |
| ATOM | 1629 | O | MET | A | 243 | -4.220 | -1.041 | 12.302 | 1.00 | 31.01 | A |
| ATOM | 1630 | N | GLY | A | 244 | -2.130 | -0.232 | 12.124 | 1.00 | 33.20 | A |
| ATOM | 1631 | CA | GLY | A | 244 | -2.230 | -0.068 | 10.686 | 1.00 | 33.96 | A |
| ATOM | 1632 | C | GLY | A | 244 | -2.357 | -1.384 | 9.947 | 1.00 | 34.81 | A |
| ATOM | 1633 | O | GLY | A | 244 | -2.341 | -1.405 | 8.717 | 1.00 | 35.30 | A |
| ATOM | 1634 | N | GLY | B | 8 | 17.002 | 9.814 | 26.472 | 1.00 | 48.25 | B |
| ATOM | 1635 | CA | GLY | B | 8 | 17.482 | 8.990 | 25.377 | 1.00 | 46.70 | B |
| ATOM | 1636 | C | GLY | B | 8 | 17.844 | 7.598 | 25.853 | 1.00 | 45.80 | B |
| ATOM | 1637 | O | GLY | B | 8 | 18.777 | 6.976 | 25.348 | 1.00 | 45.72 | B |
| ATOM | 1638 | N | ALA | B | 9 | 17.090 | 7.109 | 26.830 | 1.00 | 45.16 | B |
| ATOM | 1639 | CA | ALA | B | 9 | 17.326 | 5.792 | 27.401 | 1.00 | 42.57 | B |
| ATOM | 1640 | CB | ALA | B | 9 | 16.499 | 5.623 | 28.674 | 1.00 | 43.36 | B |
| ATOM | 1641 | C | ALA | B | 9 | 17.031 | 4.647 | 26.435 | 1.00 | 41.02 | B |
| ATOM | 1642 | O | ALA | B | 9 | 15.879 | 4.386 | 26.083 | 1.00 | 40.37 | B |
| ATOM | 1643 | N | LYS | B | 10 | 18.088 | 3.972 | 26.004 | 1.00 | 39.44 | B |
| ATOM | 1644 | CA | LYS | B | 10 | 17.954 | 2.830 | 25.115 | 1.00 | 38.32 | B |
| ATOM | 1645 | CB | LYS | B | 10 | 19.274 | 2.572 | 24.390 | 1.00 | 37.37 | B |
| ATOM | 1646 | CG | LYS | B | 10 | 19.258 | 1.356 | 23.486 | 1.00 | 38.04 | B |
| ATOM | 1647 | CD | LYS | B | 10 | 20.638 | 1.090 | 22.911 | 1.00 | 40.15 | B |
| ATOM | 1648 | CE | LYS | B | 10 | 20.602 | -0.011 | 21.863 | 1.00 | 39.99 | B |
| ATOM | 1649 | NZ | LYS | B | 10 | 20.010 | -1.263 | 22.401 | 1.00 | 43.63 | B |
| ATOM | 1650 | C | LYS | B | 10 | 17.616 | 1.648 | 26.020 | 1.00 | 37.52 | B |
| ATOM | 1651 | O | LYS | B | 10 | 18.265 | 1.445 | 27.047 | 1.00 | 37.33 | B |
| ATOM | 1652 | N | PRO | B | 11 | 16.584 | 0.866 | 25.665 | 1.00 | 36.88 | B |
| ATOM | 1653 | CD | PRO | B | 11 | 15.710 | 0.994 | 24.485 | 1.00 | 37.28 | B |
| ATOM | 1654 | CA | PRO | B | 11 | 16.195 | -0.290 | 26.480 | 1.00 | 35.53 | B |
| ATOM | 1655 | CB | PRO | B | 11 | 15.132 | -0.974 | 25.623 | 1.00 | 36.57 | B |
| ATOM | 1656 | CG | PRO | B | 11 | 14.508 | 0.176 | 24.889 | 1.00 | 37.17 | B |
| ATOM | 1657 | C | PRO | B | 11 | 17.394 | -1.195 | 26.737 | 1.00 | 34.26 | B |
| ATOM | 1658 | O | PRO | B | 11 | 18.196 | -1.444 | 25.835 | 1.00 | 35.10 | B |
| ATOM | 1659 | N | PRO | B | 12 | 17.542 | -1.688 | 27.977 | 1.00 | 32.54 | B |
| ATOM | 1660 | CD | PRO | B | 12 | 16.675 | -1.490 | 29.151 | 1.00 | 31.43 | B |
| ATOM | 1661 | CA | PRO | B | 12 | 18.670 | -2.566 | 28.300 | 1.00 | 30.62 | B |
| ATOM | 1662 | CB | PRO | B | 12 | 18.525 | -2.768 | 29.806 | 1.00 | 31.94 | B |
| ATOM | 1663 | CG | PRO | B | 12 | 17.048 | -2.664 | 30.017 | 1.00 | 32.74 | B |
| ATOM | 1664 | C | PRO | B | 12 | 18.614 | -3.877 | 27.517 | 1.00 | 29.49 | B |
| ATOM | 1665 | O | PRO | B | 12 | 17.546 | -4.461 | 27.347 | 1.00 | 28.62 | B |
| ATOM | 1666 | N | PHE | B | 13 | 19.769 | -4.327 | 27.039 | 1.00 | 28.80 | B |
| ATOM | 1667 | CA | PHE | B | 13 | 19.856 | -5.559 | 26.269 | 1.00 | 28.71 | B |
| ATOM | 1668 | CB | PHE | B | 13 | 21.302 | -5.811 | 25.829 | 1.00 | 27.38 | B |
| ATOM | 1669 | CG | PHE | B | 13 | 21.483 | -7.077 | 25.034 | 1.00 | 26.82 | B |
| ATOM | 1670 | CD1 | PHE | B | 13 | 21.093 | -7.139 | 23.703 | 1.00 | 25.07 | B |
| ATOM | 1671 | CD2 | PHE | B | 13 | 22.017 | -8.218 | 25.630 | 1.00 | 26.81 | B |
| ATOM | 1672 | CE1 | PHE | B | 13 | 21.230 | -8.317 | 22.969 | 1.00 | 26.37 | B |
| ATOM | 1673 | CE2 | PHE | B | 13 | 22.160 | -9.403 | 24.911 | 1.00 | 26.66 | B |
| ATOM | 1674 | CZ | PHE | B | 13 | 21.765 | -9.455 | 23.576 | 1.00 | 26.59 | B |
| ATOM | 1675 | C | PHE | B | 13 | 19.354 | -6.768 | 27.045 | 1.00 | 28.45 | B |

Figure 1 (suite 22)

| | | | | | | | | | | | |
|---|---|---|---|---|---|---|---|---|---|---|---|
| ATOM | 1676 | O | PHE | B | 13 | 19.440 | -6.817 | 28.271 | 1.00 | 31.15 | B |
| ATOM | 1677 | N | VAL | B | 14 | 18.825 | -7.744 | 26.312 | 1.00 | 27.65 | B |
| ATOM | 1678 | CA | VAL | B | 14 | 18.318 | -8.985 | 26.893 | 1.00 | 24.38 | B |
| ATOM | 1679 | CB | VAL | B | 14 | 16.780 | -8.973 | 26.983 | 1.00 | 26.02 | B |
| ATOM | 1680 | CG1 | VAL | B | 14 | 16.268 | -10.334 | 27.407 | 1.00 | 24.96 | B |
| ATOM | 1681 | CG2 | VAL | B | 14 | 16.325 | -7.907 | 27.964 | 1.00 | 27.11 | B |
| ATOM | 1682 | C | VAL | B | 14 | 18.749 | -10.119 | 25.973 | 1.00 | 23.50 | B |
| ATOM | 1683 | O | VAL | B | 14 | 18.443 | -10.099 | 24.779 | 1.00 | 21.48 | B |
| ATOM | 1684 | N | SER | B | 15 | 19.472 | -11.098 | 26.512 | 1.00 | 20.53 | B |
| ATOM | 1685 | CA | SER | B | 15 | 19.922 | -12.221 | 25.697 | 1.00 | 19.71 | B |
| ATOM | 1686 | CB | SER | B | 15 | 21.049 | -12.990 | 26.397 | 1.00 | 20.51 | B |
| ATOM | 1687 | OG | SER | B | 15 | 21.629 | -13.939 | 25.516 | 1.00 | 18.61 | B |
| ATOM | 1688 | C | SER | B | 15 | 18.732 | -13.147 | 25.471 | 1.00 | 19.57 | B |
| ATOM | 1689 | O | SER | B | 15 | 18.231 | -13.769 | 26.408 | 1.00 | 19.92 | B |
| ATOM | 1690 | N | ARG | B | 16 | 18.281 | -13.240 | 24.225 | 1.00 | 19.38 | B |
| ATOM | 1691 | CA | ARG | B | 16 | 17.128 | -14.081 | 23.901 | 1.00 | 16.85 | B |
| ATOM | 1692 | CB | ARG | B | 16 | 16.221 | -13.366 | 22.890 | 1.00 | 16.15 | B |
| ATOM | 1693 | CG | ARG | B | 16 | 15.691 | -12.021 | 23.341 | 1.00 | 17.30 | B |
| ATOM | 1694 | CD | ARG | B | 16 | 14.683 | -12.151 | 24.449 | 1.00 | 16.35 | B |
| ATOM | 1695 | NE | ARG | B | 16 | 14.137 | -10.850 | 24.811 | 1.00 | 17.46 | B |
| ATOM | 1696 | CZ | ARG | B | 16 | 13.247 | -10.650 | 25.774 | 1.00 | 17.91 | B |
| ATOM | 1697 | NH1 | ARG | B | 16 | 12.793 | -11.673 | 26.483 | 1.00 | 18.46 | B |
| ATOM | 1698 | NH2 | ARG | B | 16 | 12.808 | -9.422 | 26.026 | 1.00 | 17.98 | B |
| ATOM | 1699 | C | ARG | B | 16 | 17.500 | -15.434 | 23.321 | 1.00 | 15.97 | B |
| ATOM | 1700 | O | ARG | B | 16 | 18.539 | -15.583 | 22.684 | 1.00 | 13.61 | B |
| ATOM | 1701 | N | SER | B | 17 | 16.635 | -16.418 | 23.554 | 1.00 | 17.26 | B |
| ATOM | 1702 | CA | SER | B | 17 | 16.826 | -17.750 | 22.992 | 1.00 | 17.05 | B |
| ATOM | 1703 | CB | SER | B | 17 | 15.956 | -18.780 | 23.722 | 1.00 | 17.23 | B |
| ATOM | 1704 | OG | SER | B | 17 | 16.038 | -20.037 | 23.073 | 1.00 | 20.37 | B |
| ATOM | 1705 | C | SER | B | 17 | 16.347 | -17.587 | 21.550 | 1.00 | 16.25 | B |
| ATOM | 1706 | O | SER | B | 17 | 15.171 | -17.309 | 21.308 | 1.00 | 15.82 | B |
| ATOM | 1707 | N | VAL | B | 18 | 17.263 | -17.752 | 20.605 | 1.00 | 16.51 | B |
| ATOM | 1708 | CA | VAL | B | 18 | 16.971 | -17.576 | 19.195 | 1.00 | 16.36 | B |
| ATOM | 1709 | CB | VAL | B | 18 | 17.919 | -16.516 | 18.584 | 1.00 | 16.14 | B |
| ATOM | 1710 | CG1 | VAL | B | 18 | 17.691 | -16.397 | 17.077 | 1.00 | 17.11 | B |
| ATOM | 1711 | CG2 | VAL | B | 18 | 17.697 | -15.163 | 19.262 | 1.00 | 15.03 | B |
| ATOM | 1712 | C | VAL | B | 18 | 17.068 | -18.830 | 18.328 | 1.00 | 17.42 | B |
| ATOM | 1713 | O | VAL | B | 18 | 17.960 | -19.660 | 18.501 | 1.00 | 16.40 | B |
| ATOM | 1714 | N | LEU | B | 19 | 16.136 | -18.949 | 17.387 | 1.00 | 16.92 | B |
| ATOM | 1715 | CA | LEU | B | 19 | 16.146 | -20.057 | 16.449 | 1.00 | 15.95 | B |
| ATOM | 1716 | CB | LEU | B | 19 | 15.002 | -21.038 | 16.729 | 1.00 | 16.03 | B |
| ATOM | 1717 | CG | LEU | B | 19 | 14.978 | -22.244 | 15.780 | 1.00 | 18.62 | B |
| ATOM | 1718 | CD1 | LEU | B | 19 | 16.348 | -22.925 | 15.778 | 1.00 | 18.18 | B |
| ATOM | 1719 | CD2 | LEU | B | 19 | 13.895 | -23.220 | 16.201 | 1.00 | 16.61 | B |
| ATOM | 1720 | C | LEU | B | 19 | 16.020 | -19.479 | 15.051 | 1.00 | 15.42 | B |
| ATOM | 1721 | O | LEU | B | 19 | 15.057 | -18.775 | 14.741 | 1.00 | 15.31 | B |
| ATOM | 1722 | N | VAL | B | 20 | 17.015 | -19.760 | 14.219 | 1.00 | 15.31 | B |
| ATOM | 1723 | CA | VAL | B | 20 | 17.048 | -19.286 | 12.847 | 1.00 | 16.58 | B |
| ATOM | 1724 | CB | VAL | B | 20 | 18.369 | -18.520 | 12.566 | 1.00 | 16.48 | B |
| ATOM | 1725 | CG1 | VAL | B | 20 | 18.414 | -18.067 | 11.110 | 1.00 | 14.91 | B |
| ATOM | 1726 | CG2 | VAL | B | 20 | 18.498 | -17.317 | 13.510 | 1.00 | 13.51 | B |
| ATOM | 1727 | C | VAL | B | 20 | 16.972 | -20.480 | 11.891 | 1.00 | 18.58 | B |
| ATOM | 1728 | O | VAL | B | 20 | 17.946 | -21.226 | 11.765 | 1.00 | 19.59 | B |
| ATOM | 1729 | N | THR | B | 21 | 15.831 | -20.669 | 11.220 | 1.00 | 18.71 | B |
| ATOM | 1730 | CA | THR | B | 21 | 15.704 | -21.787 | 10.285 | 1.00 | 19.08 | B |
| ATOM | 1731 | CB | THR | B | 21 | 14.237 | -22.009 | 9.805 | 1.00 | 20.84 | B |
| ATOM | 1732 | OG1 | THR | B | 21 | 13.825 | -20.928 | 8.960 | 1.00 | 23.98 | B |
| ATOM | 1733 | CG2 | THR | B | 21 | 13.301 | -22.105 | 10.985 | 1.00 | 20.72 | B |
| ATOM | 1734 | C | THR | B | 21 | 16.586 | -21.533 | 9.073 | 1.00 | 19.80 | B |
| ATOM | 1735 | O | THR | B | 21 | 16.686 | -20.403 | 8.596 | 1.00 | 19.23 | B |
| ATOM | 1736 | N | GLY | B | 22 | 17.230 | -22.586 | 8.580 | 1.00 | 18.52 | B |
| ATOM | 1737 | CA | GLY | B | 22 | 18.112 | -22.446 | 7.435 | 1.00 | 21.03 | B |
| ATOM | 1738 | C | GLY | B | 22 | 19.239 | -21.470 | 7.731 | 1.00 | 22.36 | B |
| ATOM | 1739 | O | GLY | B | 22 | 19.719 | -20.762 | 6.840 | 1.00 | 21.78 | B |
| ATOM | 1740 | N | GLY | B | 23 | 19.677 | -21.442 | 8.985 | 1.00 | 22.00 | B |
| ATOM | 1741 | CA | GLY | B | 23 | 20.732 | -20.518 | 9.374 | 1.00 | 22.72 | B |
| ATOM | 1742 | C | GLY | B | 23 | 22.159 | -20.984 | 9.156 | 1.00 | 24.03 | B |
| ATOM | 1743 | O | GLY | B | 23 | 23.084 | -20.464 | 9.787 | 1.00 | 23.12 | B |
| ATOM | 1744 | N | ASN | B | 24 | 22.353 | -21.940 | 8.252 | 1.00 | 24.26 | B |
| ATOM | 1745 | CA | ASN | B | 24 | 23.690 | -22.456 | 7.983 | 1.00 | 26.03 | B |
| ATOM | 1746 | CB | ASN | B | 24 | 23.656 | -23.985 | 7.884 | 1.00 | 28.43 | B |
| ATOM | 1747 | CG | ASN | B | 24 | 22.867 | -24.474 | 6.681 | 1.00 | 30.79 | B |
| ATOM | 1748 | OD1 | ASN | B | 24 | 21.667 | -24.210 | 6.556 | 1.00 | 32.13 | B |

Figure 1 (suite 23)

| | | | | | | | | | | | |
|---|---|---|---|---|---|---|---|---|---|---|---|
| ATOM | 1749 | ND2 | ASN | B | 24 | 23.539 | -25.189 | 5.786 | 1.00 | 33.15 | B |
| ATOM | 1750 | C | ASN | B | 24 | 24.322 | -21.892 | 6.720 | 1.00 | 25.75 | B |
| ATOM | 1751 | O | ASN | B | 24 | 25.504 | -22.110 | 6.474 | 1.00 | 26.43 | B |
| ATOM | 1752 | N | ARG | B | 25 | 23.552 | -21.164 | 5.918 | 1.00 | 25.84 | B |
| ATOM | 1753 | CA | ARG | B | 25 | 24.101 | -20.618 | 4.681 | 1.00 | 27.35 | B |
| ATOM | 1754 | CB | ARG | B | 25 | 23.978 | -21.664 | 3.571 | 1.00 | 29.73 | B |
| ATOM | 1755 | CG | ARG | B | 25 | 22.584 | -22.244 | 3.439 | 0.00 | 30.22 | B |
| ATOM | 1756 | CD | ARG | B | 25 | 22.579 | -23.464 | 2.540 | 1.00 | 32.11 | B |
| ATOM | 1757 | NE | ARG | B | 25 | 21.247 | -24.051 | 2.436 | 0.00 | 31.62 | B |
| ATOM | 1758 | CZ | ARG | B | 25 | 20.966 | -25.150 | 1.744 | 0.00 | 31.81 | B |
| ATOM | 1759 | NH1 | ARG | B | 25 | 21.926 | -25.789 | 1.089 | 0.00 | 31.79 | B |
| ATOM | 1760 | NH2 | ARG | B | 25 | 19.723 | -25.611 | 1.706 | 0.00 | 31.79 | B |
| ATOM | 1761 | C | ARG | B | 25 | 23.481 | -19.302 | 4.210 | 1.00 | 25.71 | B |
| ATOM | 1762 | O | ARG | B | 25 | 22.421 | -18.896 | 4.678 | 1.00 | 26.45 | B |
| ATOM | 1763 | N | GLY | B | 26 | 24.166 | -18.643 | 3.282 | 1.00 | 24.23 | B |
| ATOM | 1764 | CA | GLY | B | 26 | 23.685 | -17.385 | 2.739 | 1.00 | 24.03 | B |
| ATOM | 1765 | C | GLY | B | 26 | 23.176 | -16.391 | 3.764 | 1.00 | 22.00 | B |
| ATOM | 1766 | O | GLY | B | 26 | 23.824 | -16.128 | 4.778 | 1.00 | 22.01 | B |
| ATOM | 1767 | N | ILE | B | 27 | 22.004 | -15.831 | 3.490 | 1.00 | 22.24 | B |
| ATOM | 1768 | CA | ILE | B | 27 | 21.395 | -14.853 | 4.378 | 1.00 | 21.22 | B |
| ATOM | 1769 | CB | ILE | B | 27 | 20.083 | -14.298 | 3.765 | 1.00 | 22.98 | B |
| ATOM | 1770 | CG2 | ILE | B | 27 | 19.306 | -13.501 | 4.804 | 1.00 | 22.26 | B |
| ATOM | 1771 | CG1 | ILE | B | 27 | 20.415 | -13.428 | 2.551 | 1.00 | 22.72 | B |
| ATOM | 1772 | CD | ILE | B | 27 | 19.196 | -13.023 | 1.737 | 1.00 | 26.93 | B |
| ATOM | 1773 | C | ILE | B | 27 | 21.110 | -15.405 | 5.773 | 1.00 | 19.76 | B |
| ATOM | 1774 | O | ILE | B | 27 | 21.345 | -14.720 | 6.766 | 1.00 | 18.63 | B |
| ATOM | 1775 | N | GLY | B | 28 | 20.602 | -16.633 | 5.853 | 1.00 | 17.13 | B |
| ATOM | 1776 | CA | GLY | B | 28 | 20.304 | -17.213 | 7.152 | 1.00 | 17.63 | B |
| ATOM | 1777 | C | GLY | B | 28 | 21.542 | -17.266 | 8.038 | 1.00 | 18.32 | B |
| ATOM | 1778 | O | GLY | B | 28 | 21.474 | -17.042 | 9.249 | 1.00 | 17.12 | B |
| ATOM | 1779 | N | LEU | B | 29 | 22.680 | -17.571 | 7.426 | 1.00 | 17.92 | B |
| ATOM | 1780 | CA | LEU | B | 29 | 23.942 | -17.646 | 8.154 | 1.00 | 17.83 | B |
| ATOM | 1781 | CB | LEU | B | 29 | 25.048 | -18.176 | 7.235 | 1.00 | 18.69 | B |
| ATOM | 1782 | CG | LEU | B | 29 | 26.464 | -18.131 | 7.823 | 1.00 | 19.31 | B |
| ATOM | 1783 | CD1 | LEU | B | 29 | 26.497 | -18.904 | 9.122 | 1.00 | 20.48 | B |
| ATOM | 1784 | CD2 | LEU | B | 29 | 27.464 | -18.707 | 6.829 | 1.00 | 19.95 | B |
| ATOM | 1785 | C | LEU | B | 29 | 24.320 | -16.260 | 8.671 | 1.00 | 17.30 | B |
| ATOM | 1786 | O | LEU | B | 29 | 24.627 | -16.087 | 9.848 | 1.00 | 15.18 | B |
| ATOM | 1787 | N | ALA | B | 30 | 24.289 | -15.274 | 7.779 | 1.00 | 17.42 | B |
| ATOM | 1788 | CA | ALA | B | 30 | 24.626 | -13.902 | 8.142 | 1.00 | 18.02 | B |
| ATOM | 1789 | CB | ALA | B | 30 | 24.441 | -12.978 | 6.944 | 1.00 | 18.70 | B |
| ATOM | 1790 | C | ALA | B | 30 | 23.753 | -13.451 | 9.302 | 1.00 | 17.69 | B |
| ATOM | 1791 | O | ALA | B | 30 | 24.219 | -12.762 | 10.213 | 1.00 | 18.99 | B |
| ATOM | 1792 | N | ILE | B | 31 | 22.485 | -13.852 | 9.278 | 1.00 | 15.54 | B |
| ATOM | 1793 | CA | ILE | B | 31 | 21.562 | -13.492 | 10.340 | 1.00 | 14.90 | B |
| ATOM | 1794 | CB | ILE | B | 31 | 20.107 | -13.892 | 9.976 | 1.00 | 15.42 | B |
| ATOM | 1795 | CG2 | ILE | B | 31 | 19.216 | -13.757 | 11.192 | 1.00 | 13.00 | B |
| ATOM | 1796 | CG1 | ILE | B | 31 | 19.593 | -13.019 | 8.821 | 1.00 | 15.16 | B |
| ATOM | 1797 | CD | ILE | B | 31 | 18.241 | -13.455 | 8.268 | 1.00 | 17.36 | B |
| ATOM | 1798 | C | ILE | B | 31 | 21.948 | -14.169 | 11.659 | 1.00 | 14.82 | B |
| ATOM | 1799 | O | ILE | B | 31 | 21.997 | -13.528 | 12.706 | 1.00 | 13.67 | B |
| ATOM | 1800 | N | ALA | B | 32 | 22.213 | -15.467 | 11.599 | 1.00 | 15.14 | B |
| ATOM | 1801 | CA | ALA | B | 32 | 22.582 | -16.228 | 12.792 | 1.00 | 16.29 | B |
| ATOM | 1802 | CB | ALA | B | 32 | 22.779 | -17.704 | 12.431 | 1.00 | 15.57 | B |
| ATOM | 1803 | C | ALA | B | 32 | 23.856 | -15.678 | 13.455 | 1.00 | 15.44 | B |
| ATOM | 1804 | O | ALA | B | 32 | 23.909 | -15.520 | 14.670 | 1.00 | 13.35 | B |
| ATOM | 1805 | N | GLN | B | 33 | 24.871 | -15.391 | 12.649 | 1.00 | 16.20 | B |
| ATOM | 1806 | CA | GLN | B | 33 | 26.137 | -14.869 | 13.163 | 1.00 | 18.06 | B |
| ATOM | 1807 | CB | GLN | B | 33 | 27.169 | -14.796 | 12.041 | 1.00 | 20.14 | B |
| ATOM | 1808 | CG | GLN | B | 33 | 27.466 | -16.146 | 11.417 | 1.00 | 26.85 | B |
| ATOM | 1809 | CD | GLN | B | 33 | 28.567 | -16.084 | 10.378 | 1.00 | 31.51 | B |
| ATOM | 1810 | OE1 | GLN | B | 33 | 28.579 | -15.200 | 9.519 | 1.00 | 35.59 | B |
| ATOM | 1811 | NE2 | GLN | B | 33 | 29.495 | -17.035 | 10.442 | 1.00 | 34.71 | B |
| ATOM | 1812 | C | GLN | B | 33 | 25.950 | -13.494 | 13.788 | 1.00 | 17.87 | B |
| ATOM | 1813 | O | GLN | B | 33 | 26.517 | -13.204 | 14.843 | 1.00 | 17.87 | B |
| ATOM | 1814 | N | ARG | B | 34 | 25.147 | -12.650 | 13.139 | 1.00 | 15.16 | B |
| ATOM | 1815 | CA | ARG | B | 34 | 24.887 | -11.312 | 13.653 | 1.00 | 13.71 | B |
| ATOM | 1816 | CB | ARG | B | 34 | 24.042 | -10.495 | 12.661 | 1.00 | 12.60 | B |
| ATOM | 1817 | CG | ARG | B | 34 | 23.515 | -9.173 | 13.236 | 1.00 | 10.69 | B |
| ATOM | 1818 | CD | ARG | B | 34 | 24.641 | -8.201 | 13.630 | 1.00 | 8.96 | B |
| ATOM | 1819 | NE | ARG | B | 34 | 24.106 | -7.005 | 14.286 | 1.00 | 11.92 | B |
| ATOM | 1820 | CZ | ARG | B | 34 | 23.893 | -5.837 | 13.683 | 1.00 | 12.62 | B |
| ATOM | 1821 | NH1 | ARG | B | 34 | 24.178 | -5.690 | 12.404 | 1.00 | 12.07 | B |

Figure 1 (suite 24)

| | | | | | | | | | | | |
|---|---|---|---|---|---|---|---|---|---|---|---|
| ATOM | 1822 | NH2 | ARG | B | 34 | 23.375 | -4.821 | 14.363 | 1.00 | 13.57 | B |
| ATOM | 1823 | C | ARG | B | 34 | 24.177 | -11.378 | 14.997 | 1.00 | 13.96 | B |
| ATOM | 1824 | O | ARG | B | 34 | 24.585 | -10.708 | 15.950 | 1.00 | 13.93 | B |
| ATOM | 1825 | N | LEU | B | 35 | 23.116 | -12.180 | 15.085 | 1.00 | 11.52 | B |
| ATOM | 1826 | CA | LEU | B | 35 | 22.378 | -12.300 | 16.336 | 1.00 | 13.06 | B |
| ATOM | 1827 | CB | LEU | B | 35 | 21.105 | -13.137 | 16.138 | 1.00 | 12.24 | B |
| ATOM | 1828 | CG | LEU | B | 35 | 20.047 | -12.479 | 15.228 | 1.00 | 15.85 | B |
| ATOM | 1829 | CD1 | LEU | B | 35 | 18.863 | -13.427 | 15.005 | 1.00 | 15.14 | B |
| ATOM | 1830 | CD2 | LEU | B | 35 | 19.563 | -11.195 | 15.877 | 1.00 | 12.26 | B |
| ATOM | 1831 | C | LEU | B | 35 | 23.240 | -12.903 | 17.458 | 1.00 | 13.57 | B |
| ATOM | 1832 | O | LEU | B | 35 | 23.032 | -12.610 | 18.633 | 1.00 | 15.62 | B |
| ATOM | 1833 | N | ALA | B | 36 | 24.205 | -13.739 | 17.091 | 1.00 | 13.64 | B |
| ATOM | 1834 | CA | ALA | B | 36 | 25.087 | -14.338 | 18.087 | 1.00 | 14.53 | B |
| ATOM | 1835 | CB | ALA | B | 36 | 25.877 | -15.492 | 17.468 | 1.00 | 12.53 | B |
| ATOM | 1836 | C | ALA | B | 36 | 26.032 | -13.228 | 18.577 | 1.00 | 16.19 | B |
| ATOM | 1837 | O | ALA | B | 36 | 26.274 | -13.090 | 19.772 | 1.00 | 17.42 | B |
| ATOM | 1838 | N | ALA | B | 37 | 26.536 | -12.417 | 17.649 | 1.00 | 15.43 | B |
| ATOM | 1839 | CA | ALA | B | 37 | 27.430 | -11.310 | 18.003 | 1.00 | 16.04 | B |
| ATOM | 1840 | CB | ALA | B | 37 | 28.049 | -10.703 | 16.740 | 1.00 | 14.41 | B |
| ATOM | 1841 | C | ALA | B | 37 | 26.693 | -10.237 | 18.811 | 1.00 | 15.27 | B |
| ATOM | 1842 | O | ALA | B | 37 | 27.321 | -9.481 | 19.569 | 1.00 | 13.38 | B |
| ATOM | 1843 | N | ASP | B | 38 | 25.367 | -10.183 | 18.657 | 1.00 | 16.01 | B |
| ATOM | 1844 | CA | ASP | B | 38 | 24.517 | -9.233 | 19.389 | 1.00 | 14.39 | B |
| ATOM | 1845 | CB | ASP | B | 38 | 23.067 | -9.226 | 18.864 | 1.00 | 13.82 | B |
| ATOM | 1846 | CG | ASP | B | 38 | 22.885 | -8.425 | 17.582 | 1.00 | 13.63 | B |
| ATOM | 1847 | OD1 | ASP | B | 38 | 23.774 | -7.641 | 17.211 | 1.00 | 11.17 | B |
| ATOM | 1848 | OD2 | ASP | B | 38 | 21.814 | -8.577 | 16.952 | 1.00 | 16.12 | B |
| ATOM | 1849 | C | ASP | B | 38 | 24.444 | -9.645 | 20.853 | 1.00 | 15.11 | B |
| ATOM | 1850 | O | ASP | B | 38 | 24.080 | -8.848 | 21.720 | 1.00 | 13.62 | B |
| ATOM | 1851 | N | GLY | B | 39 | 24.730 | -10.913 | 21.121 | 1.00 | 15.44 | B |
| ATOM | 1852 | CA | GLY | B | 39 | 24.682 | -11.396 | 22.490 | 1.00 | 14.70 | B |
| ATOM | 1853 | C | GLY | B | 39 | 23.566 | -12.377 | 22.783 | 1.00 | 14.87 | B |
| ATOM | 1854 | O | GLY | B | 39 | 23.355 | -12.749 | 23.934 | 1.00 | 13.69 | B |
| ATOM | 1855 | N | HIS | B | 40 | 22.839 | -12.788 | 21.746 | 1.00 | 16.75 | B |
| ATOM | 1856 | CA | HIS | B | 40 | 21.735 | -13.738 | 21.904 | 1.00 | 15.28 | B |
| ATOM | 1857 | CB | HIS | B | 40 | 20.722 | -13.581 | 20.757 | 1.00 | 14.70 | B |
| ATOM | 1858 | CG | HIS | B | 40 | 20.048 | -12.247 | 20.708 | 1.00 | 15.47 | B |
| ATOM | 1859 | CD2 | HIS | B | 40 | 20.078 | -11.264 | 19.779 | 1.00 | 13.05 | B |
| ATOM | 1860 | ND1 | HIS | B | 40 | 19.204 | -11.800 | 21.707 | 1.00 | 13.99 | B |
| ATOM | 1861 | CE1 | HIS | B | 40 | 18.749 | -10.605 | 21.390 | 1.00 | 16.09 | B |
| ATOM | 1862 | NE2 | HIS | B | 40 | 19.264 | -10.254 | 20.222 | 1.00 | 13.60 | B |
| ATOM | 1863 | C | HIS | B | 40 | 22.275 | -15.172 | 21.872 | 1.00 | 16.11 | B |
| ATOM | 1864 | O | HIS | B | 40 | 23.400 | -15.406 | 21.423 | 1.00 | 14.09 | B |
| ATOM | 1865 | N | LYS | B | 41 | 21.453 | -16.113 | 22.338 | 1.00 | 16.45 | B |
| ATOM | 1866 | CA | LYS | B | 41 | 21.784 | -17.536 | 22.333 | 1.00 | 18.48 | B |
| ATOM | 1867 | CB | LYS | B | 41 | 21.113 | -18.251 | 23.507 | 1.00 | 20.12 | B |
| ATOM | 1868 | CG | LYS | B | 41 | 21.263 | -17.558 | 24.840 | 1.00 | 26.57 | B |
| ATOM | 1869 | CD | LYS | B | 41 | 20.607 | -18.364 | 25.940 | 1.00 | 29.73 | B |
| ATOM | 1870 | CE | LYS | B | 41 | 20.399 | -17.533 | 27.194 | 1.00 | 33.61 | B |
| ATOM | 1871 | NZ | LYS | B | 41 | 19.332 | -16.509 | 27.013 | 1.00 | 36.28 | B |
| ATOM | 1872 | C | LYS | B | 41 | 21.169 | -18.026 | 21.031 | 1.00 | 16.86 | B |
| ATOM | 1873 | O | LYS | B | 41 | 19.955 | -18.214 | 20.943 | 1.00 | 16.47 | B |
| ATOM | 1874 | N | VAL | B | 42 | 22.002 | -18.242 | 20.025 | 1.00 | 17.78 | B |
| ATOM | 1875 | CA | VAL | B | 42 | 21.504 | -18.636 | 18.720 | 1.00 | 17.47 | B |
| ATOM | 1876 | CB | VAL | B | 42 | 22.156 | -17.760 | 17.617 | 1.00 | 15.95 | B |
| ATOM | 1877 | CG1 | VAL | B | 42 | 21.606 | -18.135 | 16.245 | 1.00 | 18.97 | B |
| ATOM | 1878 | CG2 | VAL | B | 42 | 21.900 | -16.282 | 17.908 | 1.00 | 15.38 | B |
| ATOM | 1879 | C | VAL | B | 42 | 21.648 | -20.098 | 18.314 | 1.00 | 19.06 | B |
| ATOM | 1880 | O | VAL | B | 42 | 22.712 | -20.702 | 18.444 | 1.00 | 20.35 | B |
| ATOM | 1881 | N | ALA | B | 43 | 20.550 | -20.648 | 17.808 | 1.00 | 19.63 | B |
| ATOM | 1882 | CA | ALA | B | 43 | 20.503 | -22.017 | 17.322 | 1.00 | 21.20 | B |
| ATOM | 1883 | CB | ALA | B | 43 | 19.581 | -22.864 | 18.198 | 1.00 | 21.04 | B |
| ATOM | 1884 | C | ALA | B | 43 | 19.951 | -21.935 | 15.906 | 1.00 | 21.48 | B |
| ATOM | 1885 | O | ALA | B | 43 | 19.164 | -21.036 | 15.588 | 1.00 | 20.09 | B |
| ATOM | 1886 | N | VAL | B | 44 | 20.370 | -22.856 | 15.051 | 1.00 | 20.25 | B |
| ATOM | 1887 | CA | VAL | B | 44 | 19.891 | -22.867 | 13.682 | 1.00 | 21.57 | B |
| ATOM | 1888 | CB | VAL | B | 44 | 20.987 | -22.415 | 12.697 | 1.00 | 21.52 | B |
| ATOM | 1889 | CG1 | VAL | B | 44 | 21.514 | -21.044 | 13.089 | 1.00 | 22.10 | B |
| ATOM | 1890 | CG2 | VAL | B | 44 | 22.112 | -23.440 | 12.666 | 1.00 | 20.99 | B |
| ATOM | 1891 | C | VAL | B | 44 | 19.452 | -24.266 | 13.270 | 1.00 | 22.26 | B |
| ATOM | 1892 | O | VAL | B | 44 | 19.812 | -25.258 | 13.908 | 1.00 | 22.12 | B |
| ATOM | 1893 | N | THR | B | 45 | 18.656 | -24.333 | 12.211 | 1.00 | 21.96 | B |
| ATOM | 1894 | CA | THR | B | 45 | 18.222 | -25.613 | 11.675 | 1.00 | 21.89 | B |

Figure 1 (suite 25)

| | | | | | | | | | | | |
|---|---|---|---|---|---|---|---|---|---|---|---|
| ATOM | 1895 | CB | THR | B | 45 | 16.698 | -25.681 | 11.454 | 1.00 | 21.52 | B |
| ATOM | 1896 | OG1 | THR | B | 45 | 16.329 | -24.762 | 10.421 | 1.00 | 21.86 | B |
| ATOM | 1897 | CG2 | THR | B | 45 | 15.952 | -25.347 | 12.730 | 1.00 | 23.35 | B |
| ATOM | 1898 | C | THR | B | 45 | 18.903 | -25.641 | 10.322 | 1.00 | 23.06 | B |
| ATOM | 1899 | O | THR | B | 45 | 19.226 | -24.587 | 9.776 | 1.00 | 21.96 | B |
| ATOM | 1900 | N | HIS | B | 46 | 19.141 | -26.835 | 9.794 | 1.00 | 24.87 | B |
| ATOM | 1901 | CA | HIS | B | 46 | 19.778 | -26.995 | 8.494 | 1.00 | 28.33 | B |
| ATOM | 1902 | CB | HIS | B | 46 | 21.293 | -27.143 | 8.651 | 1.00 | 29.32 | B |
| ATOM | 1903 | CG | HIS | B | 46 | 21.696 | -28.208 | 9.623 | 1.00 | 29.97 | B |
| ATOM | 1904 | CD2 | HIS | B | 46 | 22.112 | -29.481 | 9.426 | 1.00 | 27.92 | B |
| ATOM | 1905 | ND1 | HIS | B | 46 | 21.649 | -28.026 | 10.988 | 1.00 | 29.57 | B |
| ATOM | 1906 | CE1 | HIS | B | 46 | 22.018 | -29.142 | 11.591 | 1.00 | 29.31 | B |
| ATOM | 1907 | NE2 | HIS | B | 46 | 22.303 | -30.041 | 10.666 | 1.00 | 28.30 | B |
| ATOM | 1908 | C | HIS | B | 46 | 19.204 | -28.237 | 7.822 | 1.00 | 31.51 | B |
| ATOM | 1909 | O | HIS | B | 46 | 18.432 | -28.981 | 8.436 | 1.00 | 30.15 | B |
| ATOM | 1910 | N | ARG | B | 47 | 19.582 | -28.456 | 6.567 | 1.00 | 35.26 | B |
| ATOM | 1911 | CA | ARG | B | 47 | 19.097 | -29.604 | 5.805 | 1.00 | 39.73 | B |
| ATOM | 1912 | CB | ARG | B | 47 | 18.912 | -29.219 | 4.336 | 1.00 | 42.06 | B |
| ATOM | 1913 | CG | ARG | B | 47 | 17.899 | -28.108 | 4.112 | 1.00 | 44.72 | B |
| ATOM | 1914 | CD | ARG | B | 47 | 16.512 | -28.513 | 4.595 | 1.00 | 47.62 | B |
| ATOM | 1915 | NE | ARG | B | 47 | 15.984 | -29.665 | 3.867 | 1.00 | 50.29 | B |
| ATOM | 1916 | CZ | ARG | B | 47 | 15.785 | -29.699 | 2.552 | 1.00 | 51.54 | B |
| ATOM | 1917 | NH1 | ARG | B | 47 | 16.070 | -28.640 | 1.801 | 1.00 | 51.52 | B |
| ATOM | 1918 | NH2 | ARG | B | 47 | 15.299 | -30.797 | 1.984 | 1.00 | 51.81 | B |
| ATOM | 1919 | C | ARG | B | 47 | 20.036 | -30.802 | 5.903 | 1.00 | 41.55 | B |
| ATOM | 1920 | O | ARG | B | 47 | 19.656 | -31.925 | 5.561 | 1.00 | 41.65 | B |
| ATOM | 1921 | N | GLY | B | 48 | 21.261 | -30.564 | 6.366 | 1.00 | 42.79 | B |
| ATOM | 1922 | CA | GLY | B | 48 | 22.211 | -31.653 | 6.502 | 1.00 | 44.59 | B |
| ATOM | 1923 | C | GLY | B | 48 | 23.670 | -31.276 | 6.320 | 1.00 | 45.50 | B |
| ATOM | 1924 | O | GLY | B | 48 | 24.553 | -32.036 | 6.722 | 1.00 | 46.40 | B |
| ATOM | 1925 | N | SER | B | 49 | 23.934 | -30.116 | 5.723 | 1.00 | 45.76 | B |
| ATOM | 1926 | CA | SER | B | 49 | 25.312 | -29.683 | 5.499 | 1.00 | 46.76 | B |
| ATOM | 1927 | CB | SER | B | 49 | 25.357 | -28.488 | 4.542 | 1.00 | 47.15 | B |
| ATOM | 1928 | OG | SER | B | 49 | 24.931 | -27.298 | 5.183 | 1.00 | 50.33 | B |
| ATOM | 1929 | C | SER | B | 49 | 26.007 | -29.307 | 6.806 | 1.00 | 46.93 | B |
| ATOM | 1930 | O | SER | B | 49 | 27.210 | -29.036 | 6.822 | 1.00 | 47.22 | B |
| ATOM | 1931 | N | GLY | B | 50 | 25.250 | -29.285 | 7.898 | 1.00 | 47.43 | B |
| ATOM | 1932 | CA | GLY | B | 50 | 25.826 | -28.947 | 9.188 | 1.00 | 47.70 | B |
| ATOM | 1933 | C | GLY | B | 50 | 25.644 | -27.491 | 9.572 | 1.00 | 48.62 | B |
| ATOM | 1934 | O | GLY | B | 50 | 25.522 | -26.622 | 8.709 | 1.00 | 47.80 | B |
| ATOM | 1935 | N | ALA | B | 51 | 25.632 | -27.225 | 10.875 | 1.00 | 49.01 | B |
| ATOM | 1936 | CA | ALA | B | 51 | 25.460 | -25.869 | 11.386 | 1.00 | 50.25 | B |
| ATOM | 1937 | CB | ALA | B | 51 | 24.818 | -25.914 | 12.765 | 1.00 | 49.90 | B |
| ATOM | 1938 | C | ALA | B | 51 | 26.789 | -25.119 | 11.456 | 1.00 | 51.12 | B |
| ATOM | 1939 | O | ALA | B | 51 | 27.862 | -25.724 | 11.390 | 1.00 | 51.89 | B |
| ATOM | 1940 | N | PRO | B | 52 | 26.733 | -23.783 | 11.581 | 1.00 | 51.18 | B |
| ATOM | 1941 | CD | PRO | B | 52 | 25.532 | -22.938 | 11.462 | 1.00 | 51.52 | B |
| ATOM | 1942 | CA | PRO | B | 52 | 27.940 | -22.954 | 11.661 | 1.00 | 50.93 | B |
| ATOM | 1943 | CB | PRO | B | 52 | 27.378 | -21.537 | 11.731 | 1.00 | 51.37 | B |
| ATOM | 1944 | CG | PRO | B | 52 | 26.112 | -21.645 | 10.949 | 1.00 | 52.18 | B |
| ATOM | 1945 | C | PRO | B | 52 | 28.779 | -23.298 | 12.890 | 1.00 | 50.38 | B |
| ATOM | 1946 | O | PRO | B | 52 | 28.252 | -23.452 | 13.993 | 1.00 | 49.99 | B |
| ATOM | 1947 | N | LYS | B | 53 | 30.087 | -23.414 | 12.696 | 1.00 | 49.76 | B |
| ATOM | 1948 | CA | LYS | B | 53 | 30.984 | -23.741 | 13.796 | 1.00 | 48.48 | B |
| ATOM | 1949 | CB | LYS | B | 53 | 32.438 | -23.681 | 13.323 | 1.00 | 49.41 | B |
| ATOM | 1950 | CG | LYS | B | 53 | 33.417 | -24.360 | 14.263 | 1.00 | 49.40 | B |
| ATOM | 1951 | CD | LYS | B | 53 | 34.822 | -24.345 | 13.690 | 1.00 | 50.61 | B |
| ATOM | 1952 | CE | LYS | B | 53 | 35.762 | -25.201 | 14.521 | 1.00 | 50.24 | B |
| ATOM | 1953 | NZ | LYS | B | 53 | 35.330 | -26.625 | 14.529 | 1.00 | 50.60 | B |
| ATOM | 1954 | C | LYS | B | 53 | 30.771 | -22.776 | 14.961 | 1.00 | 46.88 | B |
| ATOM | 1955 | O | LYS | B | 53 | 30.826 | -21.557 | 14.793 | 1.00 | 46.90 | B |
| ATOM | 1956 | N | GLY | B | 54 | 30.515 | -23.331 | 16.139 | 1.00 | 45.33 | B |
| ATOM | 1957 | CA | GLY | B | 54 | 30.297 | -22.506 | 17.312 | 1.00 | 43.20 | B |
| ATOM | 1958 | C | GLY | B | 54 | 28.834 | -22.373 | 17.688 | 1.00 | 42.33 | B |
| ATOM | 1959 | O | GLY | B | 54 | 28.504 | -22.231 | 18.868 | 1.00 | 41.73 | B |
| ATOM | 1960 | N | LEU | B | 55 | 27.954 | -22.425 | 16.690 | 1.00 | 39.62 | B |
| ATOM | 1961 | CA | LEU | B | 55 | 26.520 | -22.296 | 16.935 | 1.00 | 38.79 | B |
| ATOM | 1962 | CB | LEU | B | 55 | 25.875 | -21.395 | 15.874 | 1.00 | 38.30 | B |
| ATOM | 1963 | CG | LEU | B | 55 | 26.310 | -19.925 | 15.879 | 1.00 | 39.74 | B |
| ATOM | 1964 | CD1 | LEU | B | 55 | 25.583 | -19.170 | 14.775 | 1.00 | 37.56 | B |
| ATOM | 1965 | CD2 | LEU | B | 55 | 26.016 | -19.299 | 17.239 | 1.00 | 37.44 | B |
| ATOM | 1966 | C | LEU | B | 55 | 25.795 | -23.635 | 16.974 | 1.00 | 36.65 | B |
| ATOM | 1967 | O | LEU | B | 55 | 26.063 | -24.532 | 16.174 | 1.00 | 36.32 | B |

Figure 1 (suite 26)

| | | | | | | | | | | | |
|---|---|---|---|---|---|---|---|---|---|---|---|
| ATOM | 1968 | N | PHE | B | 56 | 24.867 | -23.755 | 17.914 | 1.00 | 35.52 | B |
| ATOM | 1969 | CA | PHE | B | 56 | 24.092 | -24.974 | 18.077 | 1.00 | 33.72 | B |
| ATOM | 1970 | CB | PHE | B | 56 | 23.233 | -24.880 | 19.334 | 1.00 | 33.49 | B |
| ATOM | 1971 | CG | PHE | B | 56 | 22.436 | -26.116 | 19.610 | 1.00 | 33.88 | B |
| ATOM | 1972 | CD1 | PHE | B | 56 | 23.054 | -27.263 | 20.095 | 1.00 | 34.46 | B |
| ATOM | 1973 | CD2 | PHE | B | 56 | 21.067 | -26.140 | 19.374 | 1.00 | 32.48 | B |
| ATOM | 1974 | CE1 | PHE | B | 56 | 22.317 | -28.418 | 20.345 | 1.00 | 35.16 | B |
| ATOM | 1975 | CE2 | PHE | B | 56 | 20.320 | -27.287 | 19.619 | 1.00 | 34.66 | B |
| ATOM | 1976 | CZ | PHE | B | 56 | 20.946 | -28.430 | 20.106 | 1.00 | 35.24 | B |
| ATOM | 1977 | C | PHE | B | 56 | 23.193 | -25.205 | 16.864 | 1.00 | 32.80 | B |
| ATOM | 1978 | O | PHE | B | 56 | 22.410 | -24.334 | 16.484 | 1.00 | 31.30 | B |
| ATOM | 1979 | N | GLY | B | 57 | 23.307 | -26.383 | 16.263 | 1.00 | 30.91 | B |
| ATOM | 1980 | CA | GLY | B | 57 | 22.493 | -26.691 | 15.107 | 1.00 | 30.75 | B |
| ATOM | 1981 | C | GLY | B | 57 | 21.683 | -27.960 | 15.269 | 1.00 | 31.06 | B |
| ATOM | 1982 | O | GLY | B | 57 | 22.004 | -28.817 | 16.093 | 1.00 | 30.11 | B |
| ATOM | 1983 | N | VAL | B | 58 | 20.613 | -28.066 | 14.488 | 1.00 | 29.86 | B |
| ATOM | 1984 | CA | VAL | B | 58 | 19.750 | -29.241 | 14.500 | 1.00 | 30.53 | B |
| ATOM | 1985 | CB | VAL | B | 58 | 18.599 | -29.128 | 15.537 | 1.00 | 31.41 | B |
| ATOM | 1986 | CG1 | VAL | B | 58 | 19.158 | -29.195 | 16.944 | 1.00 | 33.18 | B |
| ATOM | 1987 | CG2 | VAL | B | 58 | 17.828 | -27.834 | 15.334 | 1.00 | 30.79 | B |
| ATOM | 1988 | C | VAL | B | 58 | 19.150 | -29.386 | 13.112 | 1.00 | 31.11 | B |
| ATOM | 1989 | O | VAL | B | 58 | 18.750 | -28.400 | 12.487 | 1.00 | 29.71 | B |
| ATOM | 1990 | N | GLU | B | 59 | 19.107 | -30.614 | 12.617 | 1.00 | 30.57 | B |
| ATOM | 1991 | CA | GLU | B | 59 | 18.554 | -30.848 | 11.298 | 1.00 | 30.80 | B |
| ATOM | 1992 | CB | GLU | B | 59 | 18.979 | -32.217 | 10.778 | 1.00 | 32.19 | B |
| ATOM | 1993 | CG | GLU | B | 59 | 18.525 | -32.493 | 9.363 | 1.00 | 35.94 | B |
| ATOM | 1994 | CD | GLU | B | 59 | 18.962 | -33.852 | 8.886 | 1.00 | 38.74 | B |
| ATOM | 1995 | OE1 | GLU | B | 59 | 18.680 | -34.839 | 9.597 | 1.00 | 41.59 | B |
| ATOM | 1996 | OE2 | GLU | B | 59 | 19.583 | -33.933 | 7.805 | 1.00 | 38.25 | B |
| ATOM | 1997 | C | GLU | B | 59 | 17.037 | -30.762 | 11.366 | 1.00 | 29.95 | B |
| ATOM | 1998 | O | GLU | B | 59 | 16.423 | -31.146 | 12.361 | 1.00 | 31.16 | B |
| ATOM | 1999 | N | CYS | B | 60 | 16.432 | -30.243 | 10.308 | 1.00 | 29.00 | B |
| ATOM | 2000 | CA | CYS | B | 60 | 14.984 | -30.114 | 10.271 | 1.00 | 28.01 | B |
| ATOM | 2001 | CB | CYS | B | 60 | 14.520 | -29.029 | 11.244 | 1.00 | 27.68 | B |
| ATOM | 2002 | SG | CYS | B | 60 | 12.732 | -28.708 | 11.200 | 1.00 | 27.51 | B |
| ATOM | 2003 | C | CYS | B | 60 | 14.480 | -29.770 | 8.888 | 1.00 | 28.01 | B |
| ATOM | 2004 | O | CYS | B | 60 | 15.008 | -28.873 | 8.234 | 1.00 | 27.17 | B |
| ATOM | 2005 | N | ASP | B | 61 | 13.473 | -30.507 | 8.439 | 1.00 | 26.81 | B |
| ATOM | 2006 | CA | ASP | B | 61 | 12.855 | -30.238 | 7.151 | 1.00 | 28.87 | B |
| ATOM | 2007 | CB | ASP | B | 61 | 12.537 | -31.532 | 6.399 | 1.00 | 30.56 | B |
| ATOM | 2008 | CG | ASP | B | 61 | 11.945 | -31.269 | 5.026 | 1.00 | 32.17 | B |
| ATOM | 2009 | OD1 | ASP | B | 61 | 11.171 | -30.300 | 4.892 | 1.00 | 34.55 | B |
| ATOM | 2010 | OD2 | ASP | B | 61 | 12.242 | -32.027 | 4.083 | 1.00 | 34.88 | B |
| ATOM | 2011 | C | ASP | B | 61 | 11.558 | -29.554 | 7.553 | 1.00 | 26.92 | B |
| ATOM | 2012 | O | ASP | B | 61 | 10.720 | -30.164 | 8.221 | 1.00 | 27.08 | B |
| ATOM | 2013 | N | VAL | B | 62 | 11.391 | -28.292 | 7.173 | 1.00 | 25.54 | B |
| ATOM | 2014 | CA | VAL | B | 62 | 10.180 | -27.569 | 7.547 | 1.00 | 24.38 | B |
| ATOM | 2015 | CB | VAL | B | 62 | 10.307 | -26.052 | 7.269 | 1.00 | 24.16 | B |
| ATOM | 2016 | CG1 | VAL | B | 62 | 11.447 | -25.480 | 8.092 | 1.00 | 24.85 | B |
| ATOM | 2017 | CG2 | VAL | B | 62 | 10.526 | -25.798 | 5.790 | 1.00 | 23.67 | B |
| ATOM | 2018 | C | VAL | B | 62 | 8.904 | -28.095 | 6.893 | 1.00 | 22.96 | B |
| ATOM | 2019 | O | VAL | B | 62 | 7.815 | -27.604 | 7.188 | 1.00 | 21.45 | B |
| ATOM | 2020 | N | THR | B | 63 | 9.028 | -29.083 | 6.009 | 1.00 | 22.85 | B |
| ATOM | 2021 | CA | THR | B | 63 | 7.840 | -29.662 | 5.383 | 1.00 | 23.72 | B |
| ATOM | 2022 | CB | THR | B | 63 | 8.125 | -30.277 | 3.988 | 1.00 | 23.26 | B |
| ATOM | 2023 | OG1 | THR | B | 63 | 9.014 | -31.395 | 4.117 | 1.00 | 22.86 | B |
| ATOM | 2024 | CG2 | THR | B | 63 | 8.729 | -29.242 | 3.053 | 1.00 | 23.10 | B |
| ATOM | 2025 | C | THR | B | 63 | 7.318 | -30.771 | 6.282 | 1.00 | 24.11 | B |
| ATOM | 2026 | O | THR | B | 63 | 6.254 | -31.336 | 6.031 | 1.00 | 24.90 | B |
| ATOM | 2027 | N | ASP | B | 64 | 8.069 | -31.060 | 7.345 | 1.00 | 25.76 | B |
| ATOM | 2028 | CA | ASP | B | 64 | 7.724 | -32.124 | 8.294 | 1.00 | 25.92 | B |
| ATOM | 2029 | CB | ASP | B | 64 | 8.884 | -33.120 | 8.377 | 1.00 | 27.82 | B |
| ATOM | 2030 | CG | ASP | B | 64 | 8.571 | -34.328 | 9.247 | 1.00 | 28.07 | B |
| ATOM | 2031 | OD1 | ASP | B | 64 | 7.794 | -34.212 | 10.220 | 1.00 | 26.50 | B |
| ATOM | 2032 | OD2 | ASP | B | 64 | 9.132 | -35.403 | 8.958 | 1.00 | 32.34 | B |
| ATOM | 2033 | C | ASP | B | 64 | 7.426 | -31.602 | 9.695 | 1.00 | 25.09 | B |
| ATOM | 2034 | O | ASP | B | 64 | 8.347 | -31.255 | 10.433 | 1.00 | 25.36 | B |
| ATOM | 2035 | N | SER | B | 65 | 6.149 | -31.582 | 10.071 | 1.00 | 24.94 | B |
| ATOM | 2036 | CA | SER | B | 65 | 5.732 | -31.097 | 11.387 | 1.00 | 26.56 | B |
| ATOM | 2037 | CB | SER | B | 65 | 4.203 | -31.141 | 11.513 | 1.00 | 27.10 | B |
| ATOM | 2038 | OG | SER | B | 65 | 3.583 | -30.207 | 10.641 | 1.00 | 29.89 | B |
| ATOM | 2039 | C | SER | B | 65 | 6.355 | -31.847 | 12.566 | 1.00 | 27.18 | B |
| ATOM | 2040 | O | SER | B | 65 | 6.518 | -31.278 | 13.646 | 1.00 | 26.85 | B |

Figure 1 (suite 27)

| | | | | | | | | | | | |
|---|---|---|---|---|---|---|---|---|---|---|---|
| ATOM | 2041 | N | ASP | B | 66 | 6.687 | -33.122 | 12.372 | 1.00 | 27.84 | B |
| ATOM | 2042 | CA | ASP | B | 66 | 7.291 | -33.904 | 13.448 | 1.00 | 27.67 | B |
| ATOM | 2043 | CB | ASP | B | 66 | 7.221 | -35.401 | 13.142 | 1.00 | 30.67 | B |
| ATOM | 2044 | CG | ASP | B | 66 | 5.795 | -35.918 | 13.096 | 1.00 | 33.99 | B |
| ATOM | 2045 | OD1 | ASP | B | 66 | 5.053 | -35.712 | 14.083 | 1.00 | 34.79 | B |
| ATOM | 2046 | OD2 | ASP | B | 66 | 5.420 | -36.533 | 12.074 | 1.00 | 37.05 | B |
| ATOM | 2047 | C | ASP | B | 66 | 8.740 | -33.482 | 13.626 | 1.00 | 25.14 | B |
| ATOM | 2048 | O | ASP | B | 66 | 9.229 | -33.362 | 14.747 | 1.00 | 24.38 | B |
| ATOM | 2049 | N | ALA | B | 67 | 9.418 | -33.258 | 12.508 | 1.00 | 24.12 | B |
| ATOM | 2050 | CA | ALA | B | 67 | 10.804 | -32.830 | 12.531 | 1.00 | 25.24 | B |
| ATOM | 2051 | CB | ALA | B | 67 | 11.361 | -32.795 | 11.120 | 1.00 | 25.50 | B |
| ATOM | 2052 | C | ALA | B | 67 | 10.879 | -31.443 | 13.167 | 1.00 | 25.79 | B |
| ATOM | 2053 | O | ALA | B | 67 | 11.815 | -31.133 | 13.906 | 1.00 | 27.24 | B |
| ATOM | 2054 | N | VAL | B | 68 | 9.885 | -30.609 | 12.882 | 1.00 | 25.19 | B |
| ATOM | 2055 | CA | VAL | B | 68 | 9.853 | -29.264 | 13.441 | 1.00 | 23.73 | B |
| ATOM | 2056 | CB | VAL | B | 68 | 8.703 | -28.427 | 12.839 | 1.00 | 22.28 | B |
| ATOM | 2057 | CG1 | VAL | B | 68 | 8.563 | -27.113 | 13.590 | 1.00 | 19.62 | B |
| ATOM | 2058 | CG2 | VAL | B | 68 | 8.973 | -28.174 | 11.365 | 1.00 | 20.52 | B |
| ATOM | 2059 | C | VAL | B | 68 | 9.684 | -29.350 | 14.950 | 1.00 | 24.76 | B |
| ATOM | 2060 | O | VAL | B | 68 | 10.371 | -28.654 | 15.704 | 1.00 | 24.21 | B |
| ATOM | 2061 | N | ASP | B | 69 | 8.771 | -30.208 | 15.390 | 1.00 | 23.95 | B |
| ATOM | 2062 | CA | ASP | B | 69 | 8.543 | -30.381 | 16.813 | 1.00 | 25.64 | B |
| ATOM | 2063 | CB | ASP | B | 69 | 7.413 | -31.377 | 17.065 | 1.00 | 28.56 | B |
| ATOM | 2064 | CG | ASP | B | 69 | 7.314 | -31.776 | 18.522 | 1.00 | 30.71 | B |
| ATOM | 2065 | OD1 | ASP | B | 69 | 7.608 | -32.949 | 18.833 | 1.00 | 34.06 | B |
| ATOM | 2066 | OD2 | ASP | B | 69 | 6.956 | -30.918 | 19.358 | 1.00 | 30.17 | B |
| ATOM | 2067 | C | ASP | B | 69 | 9.815 | -30.872 | 17.502 | 1.00 | 25.70 | B |
| ATOM | 2068 | O | ASP | B | 69 | 10.166 | -30.389 | 18.574 | 1.00 | 26.79 | B |
| ATOM | 2069 | N | ARG | B | 70 | 10.501 | -31.832 | 16.886 | 1.00 | 24.80 | B |
| ATOM | 2070 | CA | ARG | B | 70 | 11.734 | -32.356 | 17.463 | 1.00 | 25.86 | B |
| ATOM | 2071 | CB | ARG | B | 70 | 12.232 | -33.576 | 16.682 | 1.00 | 26.53 | B |
| ATOM | 2072 | CG | ARG | B | 70 | 11.346 | -34.806 | 16.815 | 1.00 | 31.70 | B |
| ATOM | 2073 | CD | ARG | B | 70 | 12.078 | -36.047 | 16.336 | 1.00 | 33.09 | B |
| ATOM | 2074 | NE | ARG | B | 70 | 12.424 | -35.988 | 14.919 | 1.00 | 33.97 | B |
| ATOM | 2075 | CZ | ARG | B | 70 | 11.561 | -36.193 | 13.928 | 1.00 | 34.18 | B |
| ATOM | 2076 | NH1 | ARG | B | 70 | 10.290 | -36.473 | 14.193 | 1.00 | 32.66 | B |
| ATOM | 2077 | NH2 | ARG | B | 70 | 11.972 | -36.121 | 12.670 | 1.00 | 33.58 | B |
| ATOM | 2078 | C | ARG | B | 70 | 12.825 | -31.284 | 17.474 | 1.00 | 25.38 | B |
| ATOM | 2079 | O | ARG | B | 70 | 13.610 | -31.201 | 18.420 | 1.00 | 23.73 | B |
| ATOM | 2080 | N | ALA | B | 71 | 12.874 | -30.473 | 16.418 | 1.00 | 24.86 | B |
| ATOM | 2081 | CA | ALA | B | 71 | 13.866 | -29.406 | 16.323 | 1.00 | 24.15 | B |
| ATOM | 2082 | CB | ALA | B | 71 | 13.725 | -28.665 | 15.000 | 1.00 | 24.77 | B |
| ATOM | 2083 | C | ALA | B | 71 | 13.700 | -28.440 | 17.491 | 1.00 | 22.68 | B |
| ATOM | 2084 | O | ALA | B | 71 | 14.662 | -28.151 | 18.205 | 1.00 | 25.60 | B |
| ATOM | 2085 | N | PHE | B | 72 | 12.482 | -27.945 | 17.695 | 1.00 | 21.55 | B |
| ATOM | 2086 | CA | PHE | B | 72 | 12.231 | -27.026 | 18.796 | 1.00 | 22.49 | B |
| ATOM | 2087 | CB | PHE | B | 72 | 10.768 | -26.571 | 18.811 | 1.00 | 19.74 | B |
| ATOM | 2088 | CG | PHE | B | 72 | 10.505 | -25.341 | 17.971 | 1.00 | 18.62 | B |
| ATOM | 2089 | CD1 | PHE | B | 72 | 10.562 | -25.404 | 16.584 | 1.00 | 15.62 | B |
| ATOM | 2090 | CD2 | PHE | B | 72 | 10.212 | -24.120 | 18.577 | 1.00 | 18.05 | B |
| ATOM | 2091 | CE1 | PHE | B | 72 | 10.335 | -24.263 | 15.802 | 1.00 | 18.34 | B |
| ATOM | 2092 | CE2 | PHE | B | 72 | 9.984 | -22.972 | 17.808 | 1.00 | 17.25 | B |
| ATOM | 2093 | CZ | PHE | B | 72 | 10.043 | -23.044 | 16.418 | 1.00 | 15.98 | B |
| ATOM | 2094 | C | PHE | B | 72 | 12.583 | -27.655 | 20.139 | 1.00 | 24.54 | B |
| ATOM | 2095 | O | PHE | B | 72 | 13.225 | -27.026 | 20.980 | 1.00 | 24.63 | B |
| ATOM | 2096 | N | THR | B | 73 | 12.163 | -28.899 | 20.335 | 1.00 | 25.46 | B |
| ATOM | 2097 | CA | THR | B | 73 | 12.437 | -29.603 | 21.583 | 1.00 | 27.12 | B |
| ATOM | 2098 | CB | THR | B | 73 | 11.904 | -31.052 | 21.524 | 1.00 | 28.63 | B |
| ATOM | 2099 | OG1 | THR | B | 73 | 10.476 | -31.028 | 21.404 | 1.00 | 28.45 | B |
| ATOM | 2100 | CG2 | THR | B | 73 | 12.289 | -31.822 | 22.786 | 1.00 | 29.50 | B |
| ATOM | 2101 | C | THR | B | 73 | 13.928 | -29.627 | 21.917 | 1.00 | 25.55 | B |
| ATOM | 2102 | O | THR | B | 73 | 14.318 | -29.360 | 23.054 | 1.00 | 25.97 | B |
| ATOM | 2103 | N | ALA | B | 74 | 14.754 | -29.939 | 20.922 | 1.00 | 25.54 | B |
| ATOM | 2104 | CA | ALA | B | 74 | 16.200 | -30.000 | 21.107 | 1.00 | 26.32 | B |
| ATOM | 2105 | CB | ALA | B | 74 | 16.859 | -30.567 | 19.860 | 1.00 | 26.92 | B |
| ATOM | 2106 | C | ALA | B | 74 | 16.794 | -28.626 | 21.432 | 1.00 | 28.05 | B |
| ATOM | 2107 | O | ALA | B | 74 | 17.782 | -28.523 | 22.165 | 1.00 | 28.11 | B |
| ATOM | 2108 | N | VAL | B | 75 | 16.198 | -27.572 | 20.882 | 1.00 | 26.37 | B |
| ATOM | 2109 | CA | VAL | B | 75 | 16.686 | -26.223 | 21.137 | 1.00 | 25.12 | B |
| ATOM | 2110 | CB | VAL | B | 75 | 16.121 | -25.214 | 20.097 | 1.00 | 24.00 | B |
| ATOM | 2111 | CG1 | VAL | B | 75 | 16.480 | -23.781 | 20.488 | 1.00 | 20.80 | B |
| ATOM | 2112 | CG2 | VAL | B | 75 | 16.689 | -25.529 | 18.722 | 1.00 | 22.40 | B |
| ATOM | 2113 | C | VAL | B | 75 | 16.281 | -25.790 | 22.539 | 1.00 | 25.68 | B |

Figure 1 (suite 28)

| | | | | | | | | | | | |
|---|---|---|---|---|---|---|---|---|---|---|---|
| ATOM | 2114 | O | VAL | B | 75 | 17.093 | -25.242 | 23.284 | 1.00 | 25.11 | B |
| ATOM | 2115 | N | GLU | B | 76 | 15.027 | -26.050 | 22.895 | 1.00 | 26.54 | B |
| ATOM | 2116 | CA | GLU | B | 76 | 14.500 | -25.680 | 24.202 | 1.00 | 29.64 | B |
| ATOM | 2117 | CB | GLU | B | 76 | 13.037 | -26.124 | 24.316 | 1.00 | 32.19 | B |
| ATOM | 2118 | CG | GLU | B | 76 | 12.152 | -25.614 | 23.174 | 1.00 | 35.80 | B |
| ATOM | 2119 | CD | GLU | B | 76 | 10.752 | -26.220 | 23.173 | 1.00 | 37.33 | B |
| ATOM | 2120 | OE1 | GLU | B | 76 | 10.630 | -27.459 | 23.274 | 1.00 | 37.82 | B |
| ATOM | 2121 | OE2 | GLU | B | 76 | 9.771 | -25.456 | 23.057 | 1.00 | 39.06 | B |
| ATOM | 2122 | C | GLU | B | 76 | 15.320 | -26.293 | 25.341 | 1.00 | 31.59 | B |
| ATOM | 2123 | O | GLU | B | 76 | 15.573 | -25.643 | 26.358 | 1.00 | 30.88 | B |
| ATOM | 2124 | N | GLU | B | 77 | 15.734 | -27.543 | 25.164 | 1.00 | 32.78 | B |
| ATOM | 2125 | CA | GLU | B | 77 | 16.519 | -28.236 | 26.177 | 1.00 | 34.20 | B |
| ATOM | 2126 | CB | GLU | B | 77 | 16.530 | -29.739 | 25.884 | 1.00 | 35.46 | B |
| ATOM | 2127 | CG | GLU | B | 77 | 15.148 | -30.376 | 25.903 | 0.00 | 37.13 | B |
| ATOM | 2128 | CD | GLU | B | 77 | 15.181 | -31.859 | 25.592 | 1.00 | 39.36 | B |
| ATOM | 2129 | OE1 | GLU | B | 77 | 15.633 | -32.224 | 24.487 | 0.00 | 38.50 | B |
| ATOM | 2130 | OE2 | GLU | B | 77 | 14.755 | -32.659 | 26.451 | 0.00 | 38.50 | B |
| ATOM | 2131 | C | GLU | B | 77 | 17.948 | -27.694 | 26.215 | 1.00 | 34.34 | B |
| ATOM | 2132 | O | GLU | B | 77 | 18.593 | -27.681 | 27.263 | 1.00 | 34.82 | B |
| ATOM | 2133 | N | HIS | B | 78 | 18.425 | -27.227 | 25.069 | 1.00 | 33.65 | B |
| ATOM | 2134 | CA | HIS | B | 78 | 19.770 | -26.691 | 24.956 | 1.00 | 33.54 | B |
| ATOM | 2135 | CB | HIS | B | 78 | 20.214 | -26.719 | 23.496 | 1.00 | 34.94 | B |
| ATOM | 2136 | CG | HIS | B | 78 | 21.544 | -26.078 | 23.255 | 1.00 | 36.75 | B |
| ATOM | 2137 | CD2 | HIS | B | 78 | 21.874 | -24.891 | 22.692 | 1.00 | 36.55 | B |
| ATOM | 2138 | ND1 | HIS | B | 78 | 22.736 | -26.669 | 23.623 | 1.00 | 37.54 | B |
| ATOM | 2139 | CE1 | HIS | B | 78 | 23.738 | -25.877 | 23.294 | 1.00 | 37.63 | B |
| ATOM | 2140 | NE2 | HIS | B | 78 | 23.243 | -24.789 | 22.727 | 1.00 | 37.74 | B |
| ATOM | 2141 | C | HIS | B | 78 | 19.945 | -25.271 | 25.492 | 1.00 | 32.92 | B |
| ATOM | 2142 | O | HIS | B | 78 | 20.880 | -24.997 | 26.249 | 1.00 | 34.44 | B |
| ATOM | 2143 | N | GLN | B | 79 | 19.051 | -24.369 | 25.103 | 1.00 | 30.90 | B |
| ATOM | 2144 | CA | GLN | B | 79 | 19.166 | -22.973 | 25.514 | 1.00 | 28.31 | B |
| ATOM | 2145 | CB | GLN | B | 79 | 19.610 | -22.139 | 24.316 | 1.00 | 29.46 | B |
| ATOM | 2146 | CG | GLN | B | 79 | 18.686 | -22.296 | 23.114 | 1.00 | 26.17 | B |
| ATOM | 2147 | CD | GLN | B | 79 | 19.153 | -21.524 | 21.908 | 1.00 | 25.64 | B |
| ATOM | 2148 | OE1 | GLN | B | 79 | 20.277 | -21.704 | 21.435 | 1.00 | 27.22 | B |
| ATOM | 2149 | NE2 | GLN | B | 79 | 18.288 | -20.659 | 21.392 | 1.00 | 23.78 | B |
| ATOM | 2150 | C | GLN | B | 79 | 17.901 | -22.364 | 26.090 | 1.00 | 27.73 | B |
| ATOM | 2151 | O | GLN | B | 79 | 17.845 | -21.156 | 26.318 | 1.00 | 28.01 | B |
| ATOM | 2152 | N | GLY | B | 80 | 16.885 | -23.186 | 26.324 | 1.00 | 26.34 | B |
| ATOM | 2153 | CA | GLY | B | 80 | 15.645 | -22.662 | 26.863 | 1.00 | 25.80 | B |
| ATOM | 2154 | C | GLY | B | 80 | 14.632 | -22.379 | 25.765 | 1.00 | 25.41 | B |
| ATOM | 2155 | O | GLY | B | 80 | 14.986 | -22.351 | 24.588 | 1.00 | 26.98 | B |
| ATOM | 2156 | N | PRO | B | 81 | 13.361 | -22.155 | 26.124 | 1.00 | 25.22 | B |
| ATOM | 2157 | CD | PRO | B | 81 | 12.887 | -22.081 | 27.517 | 1.00 | 25.63 | B |
| ATOM | 2158 | CA | PRO | B | 81 | 12.263 | -21.872 | 25.191 | 1.00 | 25.07 | B |
| ATOM | 2159 | CB | PRO | B | 81 | 11.142 | -21.406 | 26.116 | 1.00 | 24.62 | B |
| ATOM | 2160 | CG | PRO | B | 81 | 11.389 | -22.194 | 27.350 | 1.00 | 27.66 | B |
| ATOM | 2161 | C | PRO | B | 81 | 12.604 | -20.817 | 24.137 | 1.00 | 23.40 | B |
| ATOM | 2162 | O | PRO | B | 81 | 13.145 | -19.761 | 24.460 | 1.00 | 22.20 | B |
| ATOM | 2163 | N | VAL | B | 82 | 12.289 | -21.113 | 22.880 | 1.00 | 21.04 | B |
| ATOM | 2164 | CA | VAL | B | 82 | 12.547 | -20.186 | 21.788 | 1.00 | 19.29 | B |
| ATOM | 2165 | CB | VAL | B | 82 | 12.199 | -20.832 | 20.430 | 1.00 | 17.86 | B |
| ATOM | 2166 | CG1 | VAL | B | 82 | 12.315 | -19.812 | 19.318 | 1.00 | 18.89 | B |
| ATOM | 2167 | CG2 | VAL | B | 82 | 13.144 | -21.997 | 20.162 | 1.00 | 18.38 | B |
| ATOM | 2168 | C | VAL | B | 82 | 11.727 | -18.910 | 21.987 | 1.00 | 18.59 | B |
| ATOM | 2169 | O | VAL | B | 82 | 10.499 | -18.958 | 22.090 | 1.00 | 18.02 | B |
| ATOM | 2170 | N | GLU | B | 83 | 12.423 | -17.776 | 22.055 | 1.00 | 17.06 | B |
| ATOM | 2171 | CA | GLU | B | 83 | 11.795 | -16.472 | 22.259 | 1.00 | 16.32 | B |
| ATOM | 2172 | CB | GLU | B | 83 | 12.591 | -15.657 | 23.278 | 1.00 | 16.68 | B |
| ATOM | 2173 | CG | GLU | B | 83 | 12.579 | -16.205 | 24.680 | 1.00 | 19.55 | B |
| ATOM | 2174 | CD | GLU | B | 83 | 13.526 | -15.440 | 25.581 | 1.00 | 19.42 | B |
| ATOM | 2175 | OE1 | GLU | B | 83 | 14.757 | -15.571 | 25.392 | 1.00 | 19.69 | B |
| ATOM | 2176 | OE2 | GLU | B | 83 | 13.035 | -14.706 | 26.460 | 1.00 | 20.69 | B |
| ATOM | 2177 | C | GLU | B | 83 | 11.741 | -15.675 | 20.971 | 1.00 | 15.04 | B |
| ATOM | 2178 | O | GLU | B | 83 | 10.833 | -14.881 | 20.754 | 1.00 | 14.92 | B |
| ATOM | 2179 | N | VAL | B | 84 | 12.743 | -15.878 | 20.131 | 1.00 | 15.50 | B |
| ATOM | 2180 | CA | VAL | B | 84 | 12.835 | -15.181 | 18.865 | 1.00 | 14.90 | B |
| ATOM | 2181 | CB | VAL | B | 84 | 14.055 | -14.230 | 18.836 | 1.00 | 15.55 | B |
| ATOM | 2182 | CG1 | VAL | B | 84 | 14.151 | -13.544 | 17.485 | 1.00 | 15.95 | B |
| ATOM | 2183 | CG2 | VAL | B | 84 | 13.934 | -13.202 | 19.952 | 1.00 | 15.73 | B |
| ATOM | 2184 | C | VAL | B | 84 | 12.987 | -16.204 | 17.762 | 1.00 | 13.98 | B |
| ATOM | 2185 | O | VAL | B | 84 | 13.963 | -16.953 | 17.732 | 1.00 | 13.56 | B |
| ATOM | 2186 | N | LEU | B | 85 | 12.013 | -16.237 | 16.857 | 1.00 | 14.03 | B |

Figure 1 (suite 29)

| | | | | | | | | | | | |
|---|---|---|---|---|---|---|---|---|---|---|---|
| ATOM | 2187 | CA | LEU | B | 85 | 12.044 | -17.169 | 15.739 | 1.00 | 13.23 | B |
| ATOM | 2188 | CB | LEU | B | 85 | 10.722 | -17.943 | 15.652 | 1.00 | 15.04 | B |
| ATOM | 2189 | CG | LEU | B | 85 | 10.466 | -18.745 | 14.365 | 1.00 | 15.96 | B |
| ATOM | 2190 | CD1 | LEU | B | 85 | 11.485 | -19.867 | 14.245 | 1.00 | 17.22 | B |
| ATOM | 2191 | CD2 | LEU | B | 85 | 9.041 | -19.317 | 14.382 | 1.00 | 16.96 | B |
| ATOM | 2192 | C | LEU | B | 85 | 12.278 | -16.435 | 14.428 | 1.00 | 13.49 | B |
| ATOM | 2193 | O | LEU | B | 85 | 11.526 | -15.522 | 14.065 | 1.00 | 11.35 | B |
| ATOM | 2194 | N | VAL | B | 86 | 13.328 | -16.825 | 13.721 | 1.00 | 13.24 | B |
| ATOM | 2195 | CA | VAL | B | 86 | 13.630 | -16.229 | 12.434 | 1.00 | 12.65 | B |
| ATOM | 2196 | CB | VAL | B | 86 | 15.084 | -15.737 | 12.357 | 1.00 | 12.59 | B |
| ATOM | 2197 | CG1 | VAL | B | 86 | 15.355 | -15.143 | 10.989 | 1.00 | 13.22 | B |
| ATOM | 2198 | CG2 | VAL | B | 86 | 15.337 | -14.707 | 13.455 | 1.00 | 11.38 | B |
| ATOM | 2199 | C | VAL | B | 86 | 13.402 | -17.315 | 11.389 | 1.00 | 14.54 | B |
| ATOM | 2200 | O | VAL | B | 86 | 14.178 | -18.269 | 11.281 | 1.00 | 13.23 | B |
| ATOM | 2201 | N | SER | B | 87 | 12.318 | -17.170 | 10.631 | 1.00 | 14.13 | B |
| ATOM | 2202 | CA | SER | B | 87 | 11.967 | -18.137 | 9.602 | 1.00 | 16.39 | B |
| ATOM | 2203 | CB | SER | B | 87 | 10.443 | -18.331 | 9.587 | 1.00 | 16.97 | B |
| ATOM | 2204 | OG | SER | B | 87 | 10.038 | -19.239 | 8.575 | 1.00 | 19.02 | B |
| ATOM | 2205 | C | SER | B | 87 | 12.476 | -17.725 | 8.225 | 1.00 | 19.08 | B |
| ATOM | 2206 | O | SER | B | 87 | 11.978 | -16.768 | 7.621 | 1.00 | 17.15 | B |
| ATOM | 2207 | N | ASN | B | 88 | 13.484 | -18.450 | 7.746 | 1.00 | 21.73 | B |
| ATOM | 2208 | CA | ASN | B | 88 | 14.090 | -18.212 | 6.438 | 1.00 | 26.89 | B |
| ATOM | 2209 | CB | ASN | B | 88 | 15.591 | -18.509 | 6.484 | 1.00 | 28.55 | B |
| ATOM | 2210 | CG | ASN | B | 88 | 16.390 | -17.400 | 7.124 | 1.00 | 28.54 | B |
| ATOM | 2211 | OD1 | ASN | B | 88 | 16.608 | -16.349 | 6.518 | 1.00 | 30.49 | B |
| ATOM | 2212 | ND2 | ASN | B | 88 | 16.828 | -17.622 | 8.359 | 1.00 | 27.92 | B |
| ATOM | 2213 | C | ASN | B | 88 | 13.440 | -19.127 | 5.406 | 1.00 | 30.25 | B |
| ATOM | 2214 | O | ASN | B | 88 | 13.084 | -20.264 | 5.715 | 1.00 | 30.93 | B |
| ATOM | 2215 | N | ALA | B | 89 | 13.295 | -18.629 | 4.182 | 1.00 | 33.44 | B |
| ATOM | 2216 | CA | ALA | B | 89 | 12.683 | -19.396 | 3.097 | 1.00 | 36.59 | B |
| ATOM | 2217 | CB | ALA | B | 89 | 12.782 | -18.612 | 1.793 | 1.00 | 37.17 | B |
| ATOM | 2218 | C | ALA | B | 89 | 13.320 | -20.775 | 2.919 | 1.00 | 37.97 | B |
| ATOM | 2219 | O | ALA | B | 89 | 14.537 | -20.890 | 2.789 | 1.00 | 38.60 | B |
| ATOM | 2220 | N | GLY | B | 90 | 12.488 | -21.813 | 2.904 | 1.00 | 39.68 | B |
| ATOM | 2221 | CA | GLY | B | 90 | 12.986 | -23.166 | 2.733 | 1.00 | 41.52 | B |
| ATOM | 2222 | C | GLY | B | 90 | 13.725 | -23.359 | 1.418 | 1.00 | 42.99 | B |
| ATOM | 2223 | O | GLY | B | 90 | 14.935 | -23.593 | 1.401 | 1.00 | 44.34 | B |
| ATOM | 2224 | N | LEU | B | 91 | 12.998 | -23.261 | 0.310 | 1.00 | 43.05 | B |
| ATOM | 2225 | CA | LEU | B | 91 | 13.592 | -23.420 | -1.011 | 1.00 | 43.03 | B |
| ATOM | 2226 | CB | LEU | B | 91 | 12.620 | -24.146 | -1.943 | 1.00 | 42.11 | B |
| ATOM | 2227 | CG | LEU | B | 91 | 12.146 | -25.525 | -1.473 | 1.00 | 42.74 | B |
| ATOM | 2228 | CD1 | LEU | B | 91 | 11.095 | -26.066 | -2.431 | 1.00 | 40.87 | B |
| ATOM | 2229 | CD2 | LEU | B | 91 | 13.332 | -26.472 | -1.381 | 1.00 | 42.21 | B |
| ATOM | 2230 | C | LEU | B | 91 | 13.936 | -22.052 | -1.588 | 1.00 | 43.31 | B |
| ATOM | 2231 | O | LEU | B | 91 | 13.488 | -21.024 | -1.076 | 1.00 | 43.63 | B |
| ATOM | 2232 | N | MET | B | 100 | 6.331 | -24.149 | -14.973 | 1.00 | 40.22 | B |
| ATOM | 2233 | CA | MET | B | 100 | 5.662 | -24.301 | -13.687 | 1.00 | 38.24 | B |
| ATOM | 2234 | CB | MET | B | 100 | 4.923 | -23.013 | -13.322 | 1.00 | 38.40 | B |
| ATOM | 2235 | CG | MET | B | 100 | 4.255 | -23.047 | -11.958 | 1.00 | 38.93 | B |
| ATOM | 2236 | SD | MET | B | 100 | 5.448 | -23.239 | -10.621 | 1.00 | 40.64 | B |
| ATOM | 2237 | CE | MET | B | 100 | 6.308 | -21.683 | -10.747 | 1.00 | 37.80 | B |
| ATOM | 2238 | C | MET | B | 100 | 4.667 | -25.451 | -13.737 | 1.00 | 37.77 | B |
| ATOM | 2239 | O | MET | B | 100 | 3.856 | -25.536 | -14.657 | 1.00 | 38.79 | B |
| ATOM | 2240 | N | THR | B | 101 | 4.727 | -26.330 | -12.741 | 1.00 | 35.26 | B |
| ATOM | 2241 | CA | THR | B | 101 | 3.823 | -27.471 | -12.675 | 1.00 | 33.51 | B |
| ATOM | 2242 | CB | THR | B | 101 | 4.575 | -28.798 | -12.892 | 1.00 | 33.61 | B |
| ATOM | 2243 | OG1 | THR | B | 101 | 5.498 | -29.006 | -11.816 | 1.00 | 33.03 | B |
| ATOM | 2244 | CG2 | THR | B | 101 | 5.339 | -28.766 | -14.206 | 1.00 | 33.87 | B |
| ATOM | 2245 | C | THR | B | 101 | 3.161 | -27.516 | -11.308 | 1.00 | 31.33 | B |
| ATOM | 2246 | O | THR | B | 101 | 3.531 | -26.764 | -10.410 | 1.00 | 32.40 | B |
| ATOM | 2247 | N | GLU | B | 102 | 2.181 | -28.400 | -11.155 | 1.00 | 29.72 | B |
| ATOM | 2248 | CA | GLU | B | 102 | 1.483 | -28.542 | -9.886 | 1.00 | 29.12 | B |
| ATOM | 2249 | CB | GLU | B | 102 | 0.360 | -29.573 | -9.999 | 1.00 | 28.57 | B |
| ATOM | 2250 | CG | GLU | B | 102 | -0.425 | -29.745 | -8.708 | 1.00 | 28.54 | B |
| ATOM | 2251 | CD | GLU | B | 102 | -1.271 | -31.003 | -8.694 | 1.00 | 31.26 | B |
| ATOM | 2252 | OE1 | GLU | B | 102 | -2.139 | -31.127 | -7.802 | 1.00 | 30.34 | B |
| ATOM | 2253 | OE2 | GLU | B | 102 | -1.056 | -31.877 | -9.563 | 1.00 | 33.91 | B |
| ATOM | 2254 | C | GLU | B | 102 | 2.450 | -28.994 | -8.791 | 1.00 | 28.97 | B |
| ATOM | 2255 | O | GLU | B | 102 | 2.402 | -28.499 | -7.664 | 1.00 | 27.58 | B |
| ATOM | 2256 | N | GLU | B | 103 | 3.316 | -29.946 | -9.127 | 1.00 | 27.52 | B |
| ATOM | 2257 | CA | GLU | B | 103 | 4.283 | -30.462 | -8.165 | 1.00 | 26.37 | B |
| ATOM | 2258 | CB | GLU | B | 103 | 5.079 | -31.626 | -8.775 | 1.00 | 27.47 | B |
| ATOM | 2259 | CG | GLU | B | 103 | 5.982 | -32.346 | -7.773 | 1.00 | 30.94 | B |

Figure 1 (suite 30)

| | | | | | | | | | | | |
|---|---|---|---|---|---|---|---|---|---|---|---|
| ATOM | 2260 | CD | GLU | B | 103 | 6.704 | -33.556 | -8.363 | 1.00 | 34.61 | B |
| ATOM | 2261 | OE1 | GLU | B | 103 | 7.418 | -34.247 | -7.603 | 1.00 | 34.61 | B |
| ATOM | 2262 | OE2 | GLU | B | 103 | 6.563 | -33.817 | -9.579 | 1.00 | 34.66 | B |
| ATOM | 2263 | C | GLU | B | 103 | 5.241 | -29.357 | -7.716 | 1.00 | 25.06 | B |
| ATOM | 2264 | O | GLU | B | 103 | 5.449 | -29.156 | -6.520 | 1.00 | 25.46 | B |
| ATOM | 2265 | N | LYS | B | 104 | 5.816 | -28.640 | -8.675 | 1.00 | 22.69 | B |
| ATOM | 2266 | CA | LYS | B | 104 | 6.755 | -27.567 | -8.358 | 1.00 | 24.43 | B |
| ATOM | 2267 | CB | LYS | B | 104 | 7.373 | -27.006 | -9.641 | 1.00 | 24.59 | B |
| ATOM | 2268 | CG | LYS | B | 104 | 8.467 | -25.981 | -9.393 | 0.00 | 27.57 | B |
| ATOM | 2269 | CD | LYS | B | 104 | 9.128 | -25.558 | -10.690 | 1.00 | 31.39 | B |
| ATOM | 2270 | CE | LYS | B | 104 | 10.239 | -24.549 | -10.443 | 0.00 | 30.17 | B |
| ATOM | 2271 | NZ | LYS | B | 104 | 9.733 | -23.310 | -9.792 | 0.00 | 30.62 | B |
| ATOM | 2272 | C | LYS | B | 104 | 6.099 | -26.441 | -7.558 | 1.00 | 22.54 | B |
| ATOM | 2273 | O | LYS | B | 104 | 6.729 | -25.843 | -6.689 | 1.00 | 22.22 | B |
| ATOM | 2274 | N | PHE | B | 105 | 4.831 | -26.160 | -7.845 | 1.00 | 23.39 | B |
| ATOM | 2275 | CA | PHE | B | 105 | 4.105 | -25.113 | -7.133 | 1.00 | 20.87 | B |
| ATOM | 2276 | CB | PHE | B | 105 | 2.759 | -24.832 | -7.809 | 1.00 | 20.81 | B |
| ATOM | 2277 | CG | PHE | B | 105 | 1.979 | -23.716 | -7.165 | 1.00 | 20.72 | B |
| ATOM | 2278 | CD1 | PHE | B | 105 | 2.185 | -22.393 | -7.551 | 1.00 | 20.45 | B |
| ATOM | 2279 | CD2 | PHE | B | 105 | 1.053 | -23.984 | -6.167 | 1.00 | 19.98 | B |
| ATOM | 2280 | CE1 | PHE | B | 105 | 1.473 | -21.356 | -6.950 | 1.00 | 20.99 | B |
| ATOM | 2281 | CE2 | PHE | B | 105 | 0.337 | -22.954 | -5.559 | 1.00 | 18.14 | B |
| ATOM | 2282 | CZ | PHE | B | 105 | 0.547 | -21.640 | -5.950 | 1.00 | 19.57 | B |
| ATOM | 2283 | C | PHE | B | 105 | 3.850 | -25.528 | -5.688 | 1.00 | 20.14 | B |
| ATOM | 2284 | O | PHE | B | 105 | 4.091 | -24.759 | -4.760 | 1.00 | 19.49 | B |
| ATOM | 2285 | N | GLU | B | 106 | 3.348 | -26.744 | -5.495 | 1.00 | 20.74 | B |
| ATOM | 2286 | CA | GLU | B | 106 | 3.055 | -27.220 | -4.148 | 1.00 | 21.20 | B |
| ATOM | 2287 | CB | GLU | B | 106 | 2.247 | -28.521 | -4.198 | 1.00 | 22.33 | B |
| ATOM | 2288 | CG | GLU | B | 106 | 0.917 | -28.390 | -4.939 | 1.00 | 25.36 | B |
| ATOM | 2289 | CD | GLU | B | 106 | -0.024 | -29.543 | -4.661 | 1.00 | 26.28 | B |
| ATOM | 2290 | OE1 | GLU | B | 106 | -0.762 | -29.486 | -3.652 | 1.00 | 27.07 | B |
| ATOM | 2291 | OE2 | GLU | B | 106 | -0.013 | -30.513 | -5.447 | 1.00 | 29.08 | B |
| ATOM | 2292 | C | GLU | B | 106 | 4.314 | -27.430 | -3.306 | 1.00 | 20.82 | B |
| ATOM | 2293 | O | GLU | B | 106 | 4.275 | -27.281 | -2.087 | 1.00 | 21.79 | B |
| ATOM | 2294 | N | LYS | B | 107 | 5.420 | -27.776 | -3.957 | 1.00 | 21.33 | B |
| ATOM | 2295 | CA | LYS | B | 107 | 6.678 | -27.999 | -3.247 | 1.00 | 23.03 | B |
| ATOM | 2296 | CB | LYS | B | 107 | 7.751 | -28.507 | -4.217 | 1.00 | 24.57 | B |
| ATOM | 2297 | CG | LYS | B | 107 | 9.048 | -28.927 | -3.547 | 0.00 | 27.44 | B |
| ATOM | 2298 | CD | LYS | B | 107 | 10.006 | -29.559 | -4.542 | 1.00 | 31.85 | B |
| ATOM | 2299 | CE | LYS | B | 107 | 11.289 | -30.001 | -3.860 | 0.00 | 30.46 | B |
| ATOM | 2300 | NZ | LYS | B | 107 | 12.230 | -30.639 | -4.819 | 0.00 | 31.00 | B |
| ATOM | 2301 | C | LYS | B | 107 | 7.130 | -26.690 | -2.594 | 1.00 | 22.20 | B |
| ATOM | 2302 | O | LYS | B | 107 | 7.436 | -26.651 | -1.403 | 1.00 | 21.74 | B |
| ATOM | 2303 | N | VAL | B | 108 | 7.151 | -25.616 | -3.376 | 1.00 | 22.17 | B |
| ATOM | 2304 | CA | VAL | B | 108 | 7.556 | -24.312 | -2.863 | 1.00 | 22.27 | B |
| ATOM | 2305 | CB | VAL | B | 108 | 7.618 | -23.270 | -4.001 | 1.00 | 22.55 | B |
| ATOM | 2306 | CG1 | VAL | B | 108 | 7.761 | -21.859 | -3.431 | 1.00 | 23.48 | B |
| ATOM | 2307 | CG2 | VAL | B | 108 | 8.792 | -23.587 | -4.913 | 1.00 | 24.47 | B |
| ATOM | 2308 | C | VAL | B | 108 | 6.596 | -23.837 | -1.777 | 1.00 | 21.06 | B |
| ATOM | 2309 | O | VAL | B | 108 | 7.023 | -23.356 | -0.725 | 1.00 | 19.52 | B |
| ATOM | 2310 | N | ILE | B | 109 | 5.298 | -23.976 | -2.033 | 1.00 | 19.56 | B |
| ATOM | 2311 | CA | ILE | B | 109 | 4.287 | -23.568 | -1.067 | 1.00 | 19.05 | B |
| ATOM | 2312 | CB | ILE | B | 109 | 2.854 | -23.805 | -1.622 | 1.00 | 18.73 | B |
| ATOM | 2313 | CG2 | ILE | B | 109 | 1.844 | -23.780 | -0.492 | 1.00 | 16.27 | B |
| ATOM | 2314 | CG1 | ILE | B | 109 | 2.523 | -22.757 | -2.692 | 1.00 | 19.15 | B |
| ATOM | 2315 | CD | ILE | B | 109 | 2.480 | -21.329 | -2.171 | 1.00 | 21.38 | B |
| ATOM | 2316 | C | ILE | B | 109 | 4.418 | -24.317 | 0.259 | 1.00 | 18.69 | B |
| ATOM | 2317 | O | ILE | B | 109 | 4.279 | -23.734 | 1.336 | 1.00 | 17.31 | B |
| ATOM | 2318 | N | ASN | B | 110 | 4.673 | -25.617 | 0.178 | 1.00 | 18.85 | B |
| ATOM | 2319 | CA | ASN | B | 110 | 4.795 | -26.433 | 1.381 | 1.00 | 18.79 | B |
| ATOM | 2320 | CB | ASN | B | 110 | 4.832 | -27.913 | 0.995 | 1.00 | 20.20 | B |
| ATOM | 2321 | CG | ASN | B | 110 | 4.659 | -28.825 | 2.183 | 1.00 | 20.87 | B |
| ATOM | 2322 | OD1 | ASN | B | 110 | 4.013 | -28.467 | 3.169 | 1.00 | 21.65 | B |
| ATOM | 2323 | ND2 | ASN | B | 110 | 5.220 | -30.021 | 2.092 | 1.00 | 23.53 | B |
| ATOM | 2324 | C | ASN | B | 110 | 6.031 | -26.067 | 2.210 | 1.00 | 18.01 | B |
| ATOM | 2325 | O | ASN | B | 110 | 5.965 | -26.003 | 3.434 | 1.00 | 20.04 | B |
| ATOM | 2326 | N | ALA | B | 111 | 7.144 | -25.815 | 1.531 | 1.00 | 17.72 | B |
| ATOM | 2327 | CA | ALA | B | 111 | 8.399 | -25.451 | 2.188 | 1.00 | 18.48 | B |
| ATOM | 2328 | CB | ALA | B | 111 | 9.559 | -25.669 | 1.228 | 1.00 | 15.96 | B |
| ATOM | 2329 | C | ALA | B | 111 | 8.415 | -24.004 | 2.691 | 1.00 | 19.55 | B |
| ATOM | 2330 | O | ALA | B | 111 | 8.732 | -23.749 | 3.853 | 1.00 | 21.58 | B |
| ATOM | 2331 | N | ASN | B | 112 | 8.057 | -23.067 | 1.814 | 1.00 | 20.76 | B |
| ATOM | 2332 | CA | ASN | B | 112 | 8.073 | -21.640 | 2.143 | 1.00 | 20.85 | B |

Figure 1 (suite 31)

| | | | | | | | | | | | |
|---|---|---|---|---|---|---|---|---|---|---|---|
| ATOM | 2333 | CB | ASN | B | 112 | 8.339 | -20.829 | 0.874 | 1.00 | 21.67 | B |
| ATOM | 2334 | CG | ASN | B | 112 | 9.760 | -20.989 | 0.379 | 1.00 | 26.60 | B |
| ATOM | 2335 | OD1 | ASN | B | 112 | 10.477 | -21.884 | 0.823 | 1.00 | 26.81 | B |
| ATOM | 2336 | ND2 | ASN | B | 112 | 10.178 | -20.125 | -0.541 | 1.00 | 27.43 | B |
| ATOM | 2337 | C | ASN | B | 112 | 6.871 | -21.058 | 2.875 | 1.00 | 20.41 | B |
| ATOM | 2338 | O | ASN | B | 112 | 7.039 | -20.193 | 3.734 | 1.00 | 22.63 | B |
| ATOM | 2339 | N | LEU | B | 113 | 5.661 | -21.504 | 2.550 | 1.00 | 18.17 | B |
| ATOM | 2340 | CA | LEU | B | 113 | 4.490 | -20.967 | 3.232 | 1.00 | 16.68 | B |
| ATOM | 2341 | CB | LEU | B | 113 | 3.347 | -20.741 | 2.232 | 1.00 | 15.09 | B |
| ATOM | 2342 | CG | LEU | B | 113 | 2.015 | -20.224 | 2.792 | 1.00 | 15.26 | B |
| ATOM | 2343 | CD1 | LEU | B | 113 | 2.233 | -18.968 | 3.635 | 1.00 | 16.94 | B |
| ATOM | 2344 | CD2 | LEU | B | 113 | 1.071 | -19.921 | 1.629 | 1.00 | 16.28 | B |
| ATOM | 2345 | C | LEU | B | 113 | 4.031 | -21.860 | 4.376 | 1.00 | 16.87 | B |
| ATOM | 2346 | O | LEU | B | 113 | 3.972 | -21.427 | 5.529 | 1.00 | 16.86 | B |
| ATOM | 2347 | N | THR | B | 114 | 3.702 | -23.108 | 4.068 | 1.00 | 16.69 | B |
| ATOM | 2348 | CA | THR | B | 114 | 3.258 | -24.015 | 5.117 | 1.00 | 16.46 | B |
| ATOM | 2349 | CB | THR | B | 114 | 2.792 | -25.360 | 4.526 | 1.00 | 17.74 | B |
| ATOM | 2350 | OG1 | THR | B | 114 | 1.804 | -25.111 | 3.515 | 1.00 | 18.48 | B |
| ATOM | 2351 | CG2 | THR | B | 114 | 2.174 | -26.235 | 5.615 | 1.00 | 15.01 | B |
| ATOM | 2352 | C | THR | B | 114 | 4.411 | -24.249 | 6.103 | 1.00 | 16.30 | B |
| ATOM | 2353 | O | THR | B | 114 | 4.196 | -24.372 | 7.307 | 1.00 | 17.05 | B |
| ATOM | 2354 | N | GLY | B | 115 | 5.631 | -24.300 | 5.581 | 1.00 | 16.06 | B |
| ATOM | 2355 | CA | GLY | B | 115 | 6.783 | -24.496 | 6.442 | 1.00 | 17.58 | B |
| ATOM | 2356 | C | GLY | B | 115 | 6.877 | -23.362 | 7.450 | 1.00 | 18.08 | B |
| ATOM | 2357 | O | GLY | B | 115 | 7.200 | -23.582 | 8.618 | 1.00 | 18.88 | B |
| ATOM | 2358 | N | ALA | B | 116 | 6.578 | -22.145 | 7.000 | 1.00 | 16.92 | B |
| ATOM | 2359 | CA | ALA | B | 116 | 6.621 | -20.974 | 7.873 | 1.00 | 16.78 | B |
| ATOM | 2360 | CB | ALA | B | 116 | 6.402 | -19.698 | 7.064 | 1.00 | 16.75 | B |
| ATOM | 2361 | C | ALA | B | 116 | 5.540 | -21.112 | 8.925 | 1.00 | 16.21 | B |
| ATOM | 2362 | O | ALA | B | 116 | 5.723 | -20.724 | 10.076 | 1.00 | 15.23 | B |
| ATOM | 2363 | N | PHE | B | 117 | 4.395 | -21.656 | 8.518 | 1.00 | 16.26 | B |
| ATOM | 2364 | CA | PHE | B | 117 | 3.286 | -21.853 | 9.443 | 1.00 | 15.35 | B |
| ATOM | 2365 | CB | PHE | B | 117 | 2.040 | -22.347 | 8.699 | 1.00 | 18.07 | B |
| ATOM | 2366 | CG | PHE | B | 117 | 1.060 | -23.067 | 9.585 | 1.00 | 18.77 | B |
| ATOM | 2367 | CD1 | PHE | B | 117 | 0.313 | -22.373 | 10.528 | 1.00 | 21.24 | B |
| ATOM | 2368 | CD2 | PHE | B | 117 | 0.926 | -24.450 | 9.513 | 1.00 | 20.34 | B |
| ATOM | 2369 | CE1 | PHE | B | 117 | -0.553 | -23.044 | 11.392 | 1.00 | 23.21 | B |
| ATOM | 2370 | CE2 | PHE | B | 117 | 0.060 | -25.134 | 10.374 | 1.00 | 22.43 | B |
| ATOM | 2371 | CZ | PHE | B | 117 | -0.677 | -24.432 | 11.312 | 1.00 | 22.35 | B |
| ATOM | 2372 | C | PHE | B | 117 | 3.654 | -22.878 | 10.507 | 1.00 | 14.76 | B |
| ATOM | 2373 | O | PHE | B | 117 | 3.328 | -22.712 | 11.680 | 1.00 | 15.78 | B |
| ATOM | 2374 | N | ARG | B | 118 | 4.313 | -23.950 | 10.085 | 1.00 | 14.73 | B |
| ATOM | 2375 | CA | ARG | B | 118 | 4.698 | -25.012 | 11.009 | 1.00 | 16.30 | B |
| ATOM | 2376 | CB | ARG | B | 118 | 5.402 | -26.144 | 10.257 | 1.00 | 15.31 | B |
| ATOM | 2377 | CG | ARG | B | 118 | 4.500 | -26.886 | 9.270 | 1.00 | 17.31 | B |
| ATOM | 2378 | CD | ARG | B | 118 | 5.167 | -28.145 | 8.721 | 1.00 | 19.04 | B |
| ATOM | 2379 | NE | ARG | B | 118 | 4.294 | -28.854 | 7.784 | 1.00 | 20.26 | B |
| ATOM | 2380 | CZ | ARG | B | 118 | 4.253 | -28.620 | 6.477 | 1.00 | 19.79 | B |
| ATOM | 2381 | NH1 | ARG | B | 118 | 5.045 | -27.700 | 5.944 | 1.00 | 20.81 | B |
| ATOM | 2382 | NH2 | ARG | B | 118 | 3.401 | -29.290 | 5.706 | 1.00 | 18.36 | B |
| ATOM | 2383 | C | ARG | B | 118 | 5.587 | -24.508 | 12.141 | 1.00 | 17.22 | B |
| ATOM | 2384 | O | ARG | B | 118 | 5.278 | -24.718 | 13.314 | 1.00 | 18.34 | B |
| ATOM | 2385 | N | VAL | B | 119 | 6.685 | -23.840 | 11.796 | 1.00 | 15.34 | B |
| ATOM | 2386 | CA | VAL | B | 119 | 7.585 | -23.334 | 12.828 | 1.00 | 16.17 | B |
| ATOM | 2387 | CB | VAL | B | 119 | 8.873 | -22.750 | 12.213 | 1.00 | 15.78 | B |
| ATOM | 2388 | CG1 | VAL | B | 119 | 9.607 | -23.839 | 11.462 | 1.00 | 15.34 | B |
| ATOM | 2389 | CG2 | VAL | B | 119 | 8.549 | -21.583 | 11.277 | 1.00 | 13.90 | B |
| ATOM | 2390 | C | VAL | B | 119 | 6.879 | -22.291 | 13.676 | 1.00 | 17.36 | B |
| ATOM | 2391 | O | VAL | B | 119 | 7.041 | -22.261 | 14.893 | 1.00 | 18.09 | B |
| ATOM | 2392 | N | ALA | B | 120 | 6.071 | -21.450 | 13.033 | 1.00 | 18.38 | B |
| ATOM | 2393 | CA | ALA | B | 120 | 5.333 | -20.414 | 13.748 | 1.00 | 17.52 | B |
| ATOM | 2394 | CB | ALA | B | 120 | 4.566 | -19.528 | 12.749 | 1.00 | 16.09 | B |
| ATOM | 2395 | C | ALA | B | 120 | 4.369 | -21.029 | 14.758 | 1.00 | 17.87 | B |
| ATOM | 2396 | O | ALA | B | 120 | 4.262 | -20.569 | 15.891 | 1.00 | 16.18 | B |
| ATOM | 2397 | N | GLN | B | 121 | 3.664 | -22.074 | 14.347 | 1.00 | 19.15 | B |
| ATOM | 2398 | CA | GLN | B | 121 | 2.714 | -22.731 | 15.237 | 1.00 | 20.60 | B |
| ATOM | 2399 | CB | GLN | B | 121 | 1.903 | -23.781 | 14.465 | 1.00 | 24.92 | B |
| ATOM | 2400 | CG | GLN | B | 121 | 0.918 | -24.553 | 15.329 | 1.00 | 32.37 | B |
| ATOM | 2401 | CD | GLN | B | 121 | 0.095 | -25.547 | 14.532 | 1.00 | 36.19 | B |
| ATOM | 2402 | OE1 | GLN | B | 121 | 0.639 | -26.397 | 13.821 | 1.00 | 38.14 | B |
| ATOM | 2403 | NE2 | GLN | B | 121 | -1.229 | -25.448 | 14.649 | 1.00 | 39.06 | B |
| ATOM | 2404 | C | GLN | B | 121 | 3.431 | -23.394 | 16.416 | 1.00 | 20.04 | B |
| ATOM | 2405 | O | GLN | B | 121 | 2.993 | -23.283 | 17.557 | 1.00 | 20.43 | B |

Figure 1 (suite 32)

| | | | | | | | | | | | |
|---|---|---|---|---|---|---|---|---|---|---|---|
| ATOM | 2406 | N | ARG | B | 122 | 4.533 | -24.082 | 16.136 | 1.00 | 18.68 | B |
| ATOM | 2407 | CA | ARG | B | 122 | 5.289 | -24.756 | 17.189 | 1.00 | 20.92 | B |
| ATOM | 2408 | CB | ARG | B | 122 | 6.404 | -25.604 | 16.565 | 1.00 | 19.94 | B |
| ATOM | 2409 | CG | ARG | B | 122 | 7.317 | -26.312 | 17.572 | 1.00 | 21.44 | B |
| ATOM | 2410 | CD | ARG | B | 122 | 6.573 | -27.344 | 18.418 | 1.00 | 22.43 | B |
| ATOM | 2411 | NE | ARG | B | 122 | 7.476 | -28.072 | 19.313 | 1.00 | 22.41 | B |
| ATOM | 2412 | CZ | ARG | B | 122 | 7.995 | -27.576 | 20.435 | 1.00 | 23.35 | B |
| ATOM | 2413 | NH1 | ARG | B | 122 | 7.702 | -26.338 | 20.820 | 1.00 | 22.24 | B |
| ATOM | 2414 | NH2 | ARG | B | 122 | 8.813 | -28.319 | 21.173 | 1.00 | 21.45 | B |
| ATOM | 2415 | C | ARG | B | 122 | 5.877 | -23.761 | 18.198 | 1.00 | 21.64 | B |
| ATOM | 2416 | O | ARG | B | 122 | 6.068 | -24.095 | 19.368 | 1.00 | 20.61 | B |
| ATOM | 2417 | N | ALA | B | 123 | 6.128 | -22.530 | 17.752 | 1.00 | 22.12 | B |
| ATOM | 2418 | CA | ALA | B | 123 | 6.704 | -21.506 | 18.625 | 1.00 | 22.04 | B |
| ATOM | 2419 | CB | ALA | B | 123 | 7.524 | -20.514 | 17.791 | 1.00 | 20.74 | B |
| ATOM | 2420 | C | ALA | B | 123 | 5.688 | -20.743 | 19.465 | 1.00 | 22.30 | B |
| ATOM | 2421 | O | ALA | B | 123 | 5.975 | -20.357 | 20.597 | 1.00 | 21.65 | B |
| ATOM | 2422 | N | SER | B | 124 | 4.499 | -20.531 | 18.908 | 1.00 | 24.04 | B |
| ATOM | 2423 | CA | SER | B | 124 | 3.445 | -19.764 | 19.570 | 1.00 | 24.46 | B |
| ATOM | 2424 | CB | SER | B | 124 | 2.173 | -19.799 | 18.715 | 1.00 | 24.25 | B |
| ATOM | 2425 | OG | SER | B | 124 | 1.730 | -21.130 | 18.513 | 1.00 | 30.38 | B |
| ATOM | 2426 | C | SER | B | 124 | 3.083 | -20.101 | 21.013 | 1.00 | 24.79 | B |
| ATOM | 2427 | O | SER | B | 124 | 2.925 | -19.202 | 21.836 | 1.00 | 23.04 | B |
| ATOM | 2428 | N | ARG | B | 125 | 2.933 | -21.384 | 21.325 | 1.00 | 26.66 | B |
| ATOM | 2429 | CA | ARG | B | 125 | 2.565 | -21.793 | 22.678 | 1.00 | 28.70 | B |
| ATOM | 2430 | CB | ARG | B | 125 | 2.563 | -23.322 | 22.781 | 1.00 | 32.86 | B |
| ATOM | 2431 | CG | ARG | B | 125 | 2.474 | -23.855 | 24.208 | 1.00 | 39.32 | B |
| ATOM | 2432 | CD | ARG | B | 125 | 2.480 | -25.384 | 24.248 | 1.00 | 43.06 | B |
| ATOM | 2433 | NE | ARG | B | 125 | 3.581 | -25.972 | 23.483 | 1.00 | 47.21 | B |
| ATOM | 2434 | CZ | ARG | B | 125 | 4.874 | -25.790 | 23.749 | 1.00 | 48.34 | B |
| ATOM | 2435 | NH1 | ARG | B | 125 | 5.249 | -25.029 | 24.772 | 1.00 | 49.11 | B |
| ATOM | 2436 | NH2 | ARG | B | 125 | 5.797 | -26.372 | 22.992 | 1.00 | 49.67 | B |
| ATOM | 2437 | C | ARG | B | 125 | 3.461 | -21.210 | 23.772 | 1.00 | 26.71 | B |
| ATOM | 2438 | O | ARG | B | 125 | 2.977 | -20.568 | 24.701 | 1.00 | 27.85 | B |
| ATOM | 2439 | N | SER | B | 126 | 4.763 | -21.439 | 23.661 | 1.00 | 25.91 | B |
| ATOM | 2440 | CA | SER | B | 126 | 5.706 | -20.947 | 24.659 | 1.00 | 25.09 | B |
| ATOM | 2441 | CB | SER | B | 126 | 7.112 | -21.470 | 24.344 | 1.00 | 25.38 | B |
| ATOM | 2442 | OG | SER | B | 126 | 8.033 | -21.118 | 25.362 | 1.00 | 27.01 | B |
| ATOM | 2443 | C | SER | B | 126 | 5.711 | -19.418 | 24.731 | 1.00 | 25.23 | B |
| ATOM | 2444 | O | SER | B | 126 | 5.672 | -18.841 | 25.819 | 1.00 | 24.36 | B |
| ATOM | 2445 | N | MET | B | 127 | 5.745 | -18.767 | 23.572 | 1.00 | 23.39 | B |
| ATOM | 2446 | CA | MET | B | 127 | 5.756 | -17.307 | 23.519 | 1.00 | 24.16 | B |
| ATOM | 2447 | CB | MET | B | 127 | 5.814 | -16.835 | 22.068 | 1.00 | 22.17 | B |
| ATOM | 2448 | CG | MET | B | 127 | 7.039 | -17.325 | 21.325 | 1.00 | 20.96 | B |
| ATOM | 2449 | SD | MET | B | 127 | 7.039 | -16.825 | 19.601 | 1.00 | 19.44 | B |
| ATOM | 2450 | CE | MET | B | 127 | 8.741 | -17.150 | 19.146 | 1.00 | 15.61 | B |
| ATOM | 2451 | C | MET | B | 127 | 4.555 | -16.679 | 24.217 | 1.00 | 23.47 | B |
| ATOM | 2452 | O | MET | B | 127 | 4.714 | -15.760 | 25.020 | 1.00 | 23.72 | B |
| ATOM | 2453 | N | GLN | B | 128 | 3.355 | -17.168 | 23.917 | 1.00 | 25.18 | B |
| ATOM | 2454 | CA | GLN | B | 128 | 2.153 | -16.621 | 24.542 | 1.00 | 28.03 | B |
| ATOM | 2455 | CB | GLN | B | 128 | 0.890 | -17.307 | 24.009 | 1.00 | 29.24 | B |
| ATOM | 2456 | CG | GLN | B | 128 | 0.777 | -17.384 | 22.505 | 1.00 | 33.90 | B |
| ATOM | 2457 | CD | GLN | B | 128 | -0.508 | -18.069 | 22.060 | 1.00 | 36.76 | B |
| ATOM | 2458 | OE1 | GLN | B | 128 | -0.629 | -18.511 | 20.914 | 1.00 | 38.26 | B |
| ATOM | 2459 | NE2 | GLN | B | 128 | -1.478 | -18.149 | 22.964 | 1.00 | 37.25 | B |
| ATOM | 2460 | C | GLN | B | 128 | 2.214 | -16.840 | 26.044 | 1.00 | 28.84 | B |
| ATOM | 2461 | O | GLN | B | 128 | 1.882 | -15.961 | 26.832 | 1.00 | 28.83 | B |
| ATOM | 2462 | N | ARG | B | 129 | 2.639 | -18.034 | 26.430 | 1.00 | 31.14 | B |
| ATOM | 2463 | CA | ARG | B | 129 | 2.728 | -18.401 | 27.835 | 1.00 | 33.70 | B |
| ATOM | 2464 | CB | ARG | B | 129 | 3.114 | -19.876 | 27.940 | 1.00 | 35.23 | B |
| ATOM | 2465 | CG | ARG | B | 129 | 2.780 | -20.535 | 29.260 | 1.00 | 40.46 | B |
| ATOM | 2466 | CD | ARG | B | 129 | 2.732 | -22.040 | 29.066 | 1.00 | 43.52 | B |
| ATOM | 2467 | NE | ARG | B | 129 | 3.955 | -22.525 | 28.432 | 1.00 | 46.02 | B |
| ATOM | 2468 | CZ | ARG | B | 129 | 4.005 | -23.551 | 27.589 | 1.00 | 46.43 | B |
| ATOM | 2469 | NH1 | ARG | B | 129 | 5.165 | -23.919 | 27.064 | 1.00 | 47.43 | B |
| ATOM | 2470 | NH2 | ARG | B | 129 | 2.896 | -24.202 | 27.262 | 1.00 | 47.31 | B |
| ATOM | 2471 | C | ARG | B | 129 | 3.724 | -17.528 | 28.602 | 1.00 | 33.12 | B |
| ATOM | 2472 | O | ARG | B | 129 | 3.474 | -17.156 | 29.748 | 1.00 | 34.86 | B |
| ATOM | 2473 | N | ASN | B | 130 | 4.844 | -17.193 | 27.969 | 1.00 | 30.94 | B |
| ATOM | 2474 | CA | ASN | B | 130 | 5.854 | -16.361 | 28.622 | 1.00 | 29.78 | B |
| ATOM | 2475 | CB | ASN | B | 130 | 7.254 | -16.753 | 28.144 | 1.00 | 30.53 | B |
| ATOM | 2476 | CG | ASN | B | 130 | 7.597 | -18.191 | 28.482 | 1.00 | 33.43 | B |
| ATOM | 2477 | OD1 | ASN | B | 130 | 7.552 | -18.590 | 29.643 | 1.00 | 35.11 | B |
| ATOM | 2478 | ND2 | ASN | B | 130 | 7.940 | -18.976 | 27.468 | 1.00 | 34.95 | B |

Figure 1 (suite 33)

| | | | | | | | | | | | |
|---|---|---|---|---|---|---|---|---|---|---|---|
| ATOM | 2479 | C | ASN | B | 130 | 5.624 | -14.874 | 28.377 | 1.00 | 28.18 | B |
| ATOM | 2480 | O | ASN | B | 130 | 6.399 | -14.034 | 28.836 | 1.00 | 28.33 | B |
| ATOM | 2481 | N | LYS | B | 131 | 4.557 | -14.555 | 27.651 | 1.00 | 25.90 | B |
| ATOM | 2482 | CA | LYS | B | 131 | 4.220 | -13.169 | 27.350 | 1.00 | 24.63 | B |
| ATOM | 2483 | CB | LYS | B | 131 | 3.832 | -12.425 | 28.629 | 1.00 | 26.99 | B |
| ATOM | 2484 | CG | LYS | B | 131 | 2.576 | -12.962 | 29.302 | 1.00 | 28.01 | B |
| ATOM | 2485 | CD | LYS | B | 131 | 1.365 | -12.849 | 28.390 | 1.00 | 29.65 | B |
| ATOM | 2486 | CE | LYS | B | 131 | 0.100 | -13.277 | 29.115 | 0.00 | 29.09 | B |
| ATOM | 2487 | NZ | LYS | B | 131 | -1.111 | -13.123 | 28.264 | 0.00 | 29.28 | B |
| ATOM | 2488 | C | LYS | B | 131 | 5.348 | -12.421 | 26.645 | 1.00 | 22.91 | B |
| ATOM | 2489 | O | LYS | B | 131 | 5.634 | -11.263 | 26.951 | 1.00 | 20.26 | B |
| ATOM | 2490 | N | PHE | B | 132 | 6.006 | -13.107 | 25.723 | 1.00 | 21.27 | B |
| ATOM | 2491 | CA | PHE | B | 132 | 7.065 | -12.508 | 24.926 | 1.00 | 21.14 | B |
| ATOM | 2492 | CB | PHE | B | 132 | 8.388 | -12.365 | 25.684 | 1.00 | 19.09 | B |
| ATOM | 2493 | CG | PHE | B | 132 | 9.478 | -11.758 | 24.841 | 1.00 | 19.66 | B |
| ATOM | 2494 | CD1 | PHE | B | 132 | 9.641 | -10.377 | 24.781 | 1.00 | 19.02 | B |
| ATOM | 2495 | CD2 | PHE | B | 132 | 10.268 | -12.559 | 24.022 | 1.00 | 19.88 | B |
| ATOM | 2496 | CE1 | PHE | B | 132 | 10.565 | -9.798 | 23.913 | 1.00 | 20.12 | B |
| ATOM | 2497 | CE2 | PHE | B | 132 | 11.198 | -11.995 | 23.146 | 1.00 | 22.33 | B |
| ATOM | 2498 | CZ | PHE | B | 132 | 11.344 | -10.602 | 23.092 | 1.00 | 20.66 | B |
| ATOM | 2499 | C | PHE | B | 132 | 7.326 | -13.354 | 23.699 | 1.00 | 20.24 | B |
| ATOM | 2500 | O | PHE | B | 132 | 7.544 | -14.563 | 23.792 | 1.00 | 19.62 | B |
| ATOM | 2501 | N | GLY | B | 133 | 7.336 | -12.707 | 22.547 | 1.00 | 19.05 | B |
| ATOM | 2502 | CA | GLY | B | 133 | 7.585 | -13.439 | 21.331 | 1.00 | 19.09 | B |
| ATOM | 2503 | C | GLY | B | 133 | 7.894 | -12.534 | 20.166 | 1.00 | 20.18 | B |
| ATOM | 2504 | O | GLY | B | 133 | 7.267 | -11.488 | 19.976 | 1.00 | 19.75 | B |
| ATOM | 2505 | N | ARG | B | 134 | 8.882 | -12.938 | 19.383 | 1.00 | 17.88 | B |
| ATOM | 2506 | CA | ARG | B | 134 | 9.256 | -12.183 | 18.212 | 1.00 | 18.18 | B |
| ATOM | 2507 | CB | ARG | B | 134 | 10.565 | -11.419 | 18.456 | 1.00 | 19.10 | B |
| ATOM | 2508 | CG | ARG | B | 134 | 10.494 | -10.424 | 19.614 | 1.00 | 20.41 | B |
| ATOM | 2509 | CD | ARG | B | 134 | 9.660 | -9.184 | 19.263 | 1.00 | 18.87 | B |
| ATOM | 2510 | NE | ARG | B | 134 | 9.548 | -8.242 | 20.382 | 1.00 | 17.22 | B |
| ATOM | 2511 | CZ | ARG | B | 134 | 8.593 | -8.277 | 21.308 | 1.00 | 21.94 | B |
| ATOM | 2512 | NH1 | ARG | B | 134 | 7.647 | -9.206 | 21.264 | 1.00 | 20.69 | B |
| ATOM | 2513 | NH2 | ARG | B | 134 | 8.573 | -7.374 | 22.282 | 1.00 | 22.19 | B |
| ATOM | 2514 | C | ARG | B | 134 | 9.412 | -13.160 | 17.059 | 1.00 | 15.98 | B |
| ATOM | 2515 | O | ARG | B | 134 | 10.356 | -13.946 | 17.017 | 1.00 | 17.41 | B |
| ATOM | 2516 | N | MET | B | 135 | 8.454 | -13.133 | 16.143 | 1.00 | 14.33 | B |
| ATOM | 2517 | CA | MET | B | 135 | 8.512 | -13.984 | 14.970 | 1.00 | 15.20 | B |
| ATOM | 2518 | CB | MET | B | 135 | 7.154 | -14.634 | 14.687 | 1.00 | 16.57 | B |
| ATOM | 2519 | CG | MET | B | 135 | 6.727 | -15.661 | 15.705 | 1.00 | 16.61 | B |
| ATOM | 2520 | SD | MET | B | 135 | 5.238 | -16.566 | 15.173 | 1.00 | 18.74 | B |
| ATOM | 2521 | CE | MET | B | 135 | 4.444 | -16.823 | 16.727 | 1.00 | 19.55 | B |
| ATOM | 2522 | C | MET | B | 135 | 8.906 | -13.081 | 13.821 | 1.00 | 14.60 | B |
| ATOM | 2523 | O | MET | B | 135 | 8.271 | -12.052 | 13.579 | 1.00 | 12.42 | B |
| ATOM | 2524 | N | ILE | B | 136 | 9.965 | -13.466 | 13.123 | 1.00 | 12.93 | B |
| ATOM | 2525 | CA | ILE | B | 136 | 10.465 | -12.689 | 12.014 | 1.00 | 13.57 | B |
| ATOM | 2526 | CB | ILE | B | 136 | 11.846 | -12.077 | 12.366 | 1.00 | 13.61 | B |
| ATOM | 2527 | CG2 | ILE | B | 136 | 12.320 | -11.174 | 11.237 | 1.00 | 13.49 | B |
| ATOM | 2528 | CG1 | ILE | B | 136 | 11.733 | -11.290 | 13.678 | 1.00 | 12.32 | B |
| ATOM | 2529 | CD | ILE | B | 136 | 13.087 | -10.823 | 14.249 | 1.00 | 13.70 | B |
| ATOM | 2530 | C | ILE | B | 136 | 10.587 | -13.571 | 10.781 | 1.00 | 13.66 | B |
| ATOM | 2531 | O | ILE | B | 136 | 11.419 | -14.486 | 10.727 | 1.00 | 13.20 | B |
| ATOM | 2532 | N | PHE | B | 137 | 9.752 | -13.296 | 9.781 | 1.00 | 13.11 | B |
| ATOM | 2533 | CA | PHE | B | 137 | 9.776 | -14.076 | 8.552 | 1.00 | 12.82 | B |
| ATOM | 2534 | CB | PHE | B | 137 | 8.343 | -14.324 | 8.045 | 1.00 | 11.99 | B |
| ATOM | 2535 | CG | PHE | B | 137 | 7.448 | -14.958 | 9.064 | 1.00 | 10.95 | B |
| ATOM | 2536 | CD1 | PHE | B | 137 | 6.787 | -14.185 | 10.005 | 1.00 | 10.39 | B |
| ATOM | 2537 | CD2 | PHE | B | 137 | 7.298 | -16.343 | 9.108 | 1.00 | 11.93 | B |
| ATOM | 2538 | CE1 | PHE | B | 137 | 5.993 | -14.772 | 10.979 | 1.00 | 12.09 | B |
| ATOM | 2539 | CE2 | PHE | B | 137 | 6.509 | -16.940 | 10.079 | 1.00 | 10.64 | B |
| ATOM | 2540 | CZ | PHE | B | 137 | 5.854 | -16.158 | 11.017 | 1.00 | 8.11 | B |
| ATOM | 2541 | C | PHE | B | 137 | 10.594 | -13.371 | 7.484 | 1.00 | 13.81 | B |
| ATOM | 2542 | O | PHE | B | 137 | 10.396 | -12.185 | 7.214 | 1.00 | 14.50 | B |
| ATOM | 2543 | N | ILE | B | 138 | 11.514 | -14.110 | 6.878 | 1.00 | 16.06 | B |
| ATOM | 2544 | CA | ILE | B | 138 | 12.373 | -13.572 | 5.836 | 1.00 | 18.30 | B |
| ATOM | 2545 | CB | ILE | B | 138 | 13.841 | -14.042 | 6.008 | 1.00 | 20.13 | B |
| ATOM | 2546 | CG2 | ILE | B | 138 | 14.738 | -13.313 | 5.007 | 1.00 | 19.23 | B |
| ATOM | 2547 | CG1 | ILE | B | 138 | 14.312 | -13.807 | 7.453 | 1.00 | 19.09 | B |
| ATOM | 2548 | CD | ILE | B | 138 | 14.258 | -12.363 | 7.910 | 1.00 | 18.73 | B |
| ATOM | 2549 | C | ILE | B | 138 | 11.884 | -14.060 | 4.480 | 1.00 | 21.85 | B |
| ATOM | 2550 | O | ILE | B | 138 | 11.937 | -15.258 | 4.189 | 1.00 | 20.92 | B |
| ATOM | 2551 | N | GLY | B | 139 | 11.411 | -13.136 | 3.651 | 1.00 | 23.04 | B |

Figure 1 (suite 34)

| | | | | | | | | | | | |
|---|---|---|---|---|---|---|---|---|---|---|---|
| ATOM | 2552 | CA | GLY | B | 139 | 10.931 | -13.521 | 2.341 | 1.00 | 26.00 | B |
| ATOM | 2553 | C | GLY | B | 139 | 12.091 | -13.992 | 1.491 | 1.00 | 28.48 | B |
| ATOM | 2554 | O | GLY | B | 139 | 13.239 | -13.624 | 1.744 | 1.00 | 27.34 | B |
| ATOM | 2555 | N | SER | B | 140 | 11.807 | -14.816 | 0.489 | 1.00 | 29.78 | B |
| ATOM | 2556 | CA | SER | B | 140 | 12.859 | -15.302 | -0.391 | 1.00 | 33.13 | B |
| ATOM | 2557 | CB | SER | B | 140 | 12.322 | -16.416 | -1.303 | 1.00 | 32.36 | B |
| ATOM | 2558 | OG | SER | B | 140 | 11.186 | -15.991 | -2.037 | 1.00 | 33.27 | B |
| ATOM | 2559 | C | SER | B | 140 | 13.387 | -14.132 | -1.223 | 1.00 | 34.95 | B |
| ATOM | 2560 | O | SER | B | 140 | 12.718 | -13.107 | -1.364 | 1.00 | 35.32 | B |
| ATOM | 2561 | N | VAL | B | 141 | 14.593 | -14.284 | -1.761 | 1.00 | 38.32 | B |
| ATOM | 2562 | CA | VAL | B | 141 | 15.204 | -13.234 | -2.570 | 1.00 | 40.52 | B |
| ATOM | 2563 | CB | VAL | B | 141 | 16.746 | -13.333 | -2.527 | 1.00 | 40.88 | B |
| ATOM | 2564 | CG1 | VAL | B | 141 | 17.370 | -12.320 | -3.483 | 1.00 | 42.71 | B |
| ATOM | 2565 | CG2 | VAL | B | 141 | 17.234 | -13.086 | -1.107 | 1.00 | 40.19 | B |
| ATOM | 2566 | C | VAL | B | 141 | 14.741 | -13.289 | -4.021 | 1.00 | 41.83 | B |
| ATOM | 2567 | O | VAL | B | 141 | 14.408 | -12.259 | -4.614 | 1.00 | 44.39 | B |
| ATOM | 2568 | N | ALA | B | 150 | 7.654 | -18.191 | -11.533 | 1.00 | 32.52 | B |
| ATOM | 2569 | CA | ALA | B | 150 | 7.802 | -17.485 | -10.267 | 1.00 | 29.90 | B |
| ATOM | 2570 | CB | ALA | B | 150 | 8.305 | -16.069 | -10.520 | 1.00 | 30.58 | B |
| ATOM | 2571 | C | ALA | B | 150 | 6.487 | -17.445 | -9.498 | 1.00 | 27.89 | B |
| ATOM | 2572 | O | ALA | B | 150 | 6.448 | -17.032 | -8.341 | 1.00 | 28.29 | B |
| ATOM | 2573 | N | ALA | B | 151 | 5.413 | -17.886 | -10.143 | 1.00 | 25.12 | B |
| ATOM | 2574 | CA | ALA | B | 151 | 4.097 | -17.895 | -9.522 | 1.00 | 22.98 | B |
| ATOM | 2575 | CB | ALA | B | 151 | 3.095 | -18.613 | -10.429 | 1.00 | 22.09 | B |
| ATOM | 2576 | C | ALA | B | 151 | 4.094 | -18.537 | -8.142 | 1.00 | 22.44 | B |
| ATOM | 2577 | O | ALA | B | 151 | 3.392 | -18.075 | -7.245 | 1.00 | 21.79 | B |
| ATOM | 2578 | N | ASN | B | 152 | 4.869 | -19.607 | -7.969 | 1.00 | 21.68 | B |
| ATOM | 2579 | CA | ASN | B | 152 | 4.906 | -20.282 | -6.675 | 1.00 | 21.67 | B |
| ATOM | 2580 | CB | ASN | B | 152 | 5.521 | -21.692 | -6.800 | 1.00 | 21.15 | B |
| ATOM | 2581 | CG | ASN | B | 152 | 6.852 | -21.708 | -7.536 | 1.00 | 22.76 | B |
| ATOM | 2582 | OD1 | ASN | B | 152 | 7.462 | -22.770 | -7.693 | 1.00 | 24.94 | B |
| ATOM | 2583 | ND2 | ASN | B | 152 | 7.306 | -20.548 | -7.996 | 1.00 | 20.48 | B |
| ATOM | 2584 | C | ASN | B | 152 | 5.628 | -19.468 | -5.604 | 1.00 | 21.46 | B |
| ATOM | 2585 | O | ASN | B | 152 | 5.149 | -19.369 | -4.474 | 1.00 | 20.62 | B |
| ATOM | 2586 | N | TYR | B | 153 | 6.769 | -18.876 | -5.951 | 1.00 | 21.00 | B |
| ATOM | 2587 | CA | TYR | B | 153 | 7.501 | -18.059 | -4.983 | 1.00 | 22.20 | B |
| ATOM | 2588 | CB | TYR | B | 153 | 8.891 | -17.696 | -5.512 | 1.00 | 24.19 | B |
| ATOM | 2589 | CG | TYR | B | 153 | 9.865 | -18.849 | -5.457 | 1.00 | 27.87 | B |
| ATOM | 2590 | CD1 | TYR | B | 153 | 9.898 | -19.810 | -6.468 | 1.00 | 29.45 | B |
| ATOM | 2591 | CE1 | TYR | B | 153 | 10.760 | -20.903 | -6.394 | 1.00 | 31.51 | B |
| ATOM | 2592 | CD2 | TYR | B | 153 | 10.721 | -19.009 | -4.365 | 1.00 | 27.27 | B |
| ATOM | 2593 | CE2 | TYR | B | 153 | 11.585 | -20.100 | -4.280 | 1.00 | 30.07 | B |
| ATOM | 2594 | CZ | TYR | B | 153 | 11.599 | -21.043 | -5.296 | 1.00 | 31.10 | B |
| ATOM | 2595 | OH | TYR | B | 153 | 12.445 | -22.126 | -5.216 | 1.00 | 29.88 | B |
| ATOM | 2596 | C | TYR | B | 153 | 6.707 | -16.789 | -4.686 | 1.00 | 21.14 | B |
| ATOM | 2597 | O | TYR | B | 153 | 6.563 | -16.393 | -3.533 | 1.00 | 21.69 | B |
| ATOM | 2598 | N | ALA | B | 154 | 6.189 | -16.157 | -5.734 | 1.00 | 18.31 | B |
| ATOM | 2599 | CA | ALA | B | 154 | 5.393 | -14.946 | -5.573 | 1.00 | 17.82 | B |
| ATOM | 2600 | CB | ALA | B | 154 | 4.849 | -14.494 | -6.931 | 1.00 | 17.28 | B |
| ATOM | 2601 | C | ALA | B | 154 | 4.240 | -15.212 | -4.605 | 1.00 | 17.14 | B |
| ATOM | 2602 | O | ALA | B | 154 | 3.972 | -14.404 | -3.711 | 1.00 | 16.00 | B |
| ATOM | 2603 | N | ALA | B | 155 | 3.565 | -16.350 | -4.777 | 1.00 | 14.43 | B |
| ATOM | 2604 | CA | ALA | B | 155 | 2.441 | -16.713 | -3.914 | 1.00 | 15.62 | B |
| ATOM | 2605 | CB | ALA | B | 155 | 1.699 | -17.917 | -4.500 | 1.00 | 16.27 | B |
| ATOM | 2606 | C | ALA | B | 155 | 2.855 | -17.014 | -2.475 | 1.00 | 15.64 | B |
| ATOM | 2607 | O | ALA | B | 155 | 2.187 | -16.600 | -1.526 | 1.00 | 13.77 | B |
| ATOM | 2608 | N | SER | B | 156 | 3.950 | -17.748 | -2.308 | 1.00 | 15.82 | B |
| ATOM | 2609 | CA | SER | B | 156 | 4.412 | -18.087 | -0.964 | 1.00 | 18.48 | B |
| ATOM | 2610 | CB | SER | B | 156 | 5.561 | -19.112 | -1.028 | 1.00 | 19.77 | B |
| ATOM | 2611 | OG | SER | B | 156 | 6.582 | -18.695 | -1.916 | 1.00 | 22.63 | B |
| ATOM | 2612 | C | SER | B | 156 | 4.849 | -16.836 | -0.204 | 1.00 | 18.43 | B |
| ATOM | 2613 | O | SER | B | 156 | 4.504 | -16.663 | 0.962 | 1.00 | 17.71 | B |
| ATOM | 2614 | N | LYS | B | 157 | 5.593 | -15.961 | -0.869 | 1.00 | 19.19 | B |
| ATOM | 2615 | CA | LYS | B | 157 | 6.060 | -14.730 | -0.229 | 1.00 | 19.75 | B |
| ATOM | 2616 | CB | LYS | B | 157 | 6.905 | -13.902 | -1.203 | 1.00 | 23.50 | B |
| ATOM | 2617 | CG | LYS | B | 157 | 8.088 | -14.649 | -1.776 | 1.00 | 29.75 | B |
| ATOM | 2618 | CD | LYS | B | 157 | 8.904 | -13.785 | -2.736 | 1.00 | 32.53 | B |
| ATOM | 2619 | CE | LYS | B | 157 | 9.652 | -12.685 | -2.002 | 1.00 | 32.95 | B |
| ATOM | 2620 | NZ | LYS | B | 157 | 10.651 | -12.018 | -2.890 | 1.00 | 36.13 | B |
| ATOM | 2621 | C | LYS | B | 157 | 4.871 | -13.901 | 0.237 | 1.00 | 15.82 | B |
| ATOM | 2622 | O | LYS | B | 157 | 4.821 | -13.461 | 1.383 | 1.00 | 13.71 | B |
| ATOM | 2623 | N | ALA | B | 158 | 3.906 | -13.691 | -0.655 | 1.00 | 13.50 | B |
| ATOM | 2624 | CA | ALA | B | 158 | 2.721 | -12.905 | -0.301 | 1.00 | 13.54 | B |

Figure 1 (suite 35)

| | | | | | | | | | | | |
|---|---|---|---|---|---|---|---|---|---|---|---|
| ATOM | 2625 | CB | ALA | B | 158 | 1.848 | -12.662 | -1.538 | 1.00 | 13.34 | B |
| ATOM | 2626 | C | ALA | B | 158 | 1.917 | -13.607 | 0.775 | 1.00 | 13.76 | B |
| ATOM | 2627 | O | ALA | B | 158 | 1.368 | -12.963 | 1.674 | 1.00 | 14.41 | B |
| ATOM | 2628 | N | GLY | B | 159 | 1.849 | -14.935 | 0.684 | 1.00 | 12.75 | B |
| ATOM | 2629 | CA | GLY | B | 159 | 1.110 | -15.699 | 1.672 | 1.00 | 12.80 | B |
| ATOM | 2630 | C | GLY | B | 159 | 1.698 | -15.562 | 3.067 | 1.00 | 13.96 | B |
| ATOM | 2631 | O | GLY | B | 159 | 0.966 | -15.434 | 4.047 | 1.00 | 13.50 | B |
| ATOM | 2632 | N | VAL | B | 160 | 3.027 | -15.593 | 3.162 | 1.00 | 13.88 | B |
| ATOM | 2633 | CA | VAL | B | 160 | 3.701 | -15.456 | 4.458 | 1.00 | 14.94 | B |
| ATOM | 2634 | CB | VAL | B | 160 | 5.235 | -15.543 | 4.292 | 1.00 | 15.41 | B |
| ATOM | 2635 | CG1 | VAL | B | 160 | 5.937 | -15.185 | 5.603 | 1.00 | 17.83 | B |
| ATOM | 2636 | CG2 | VAL | B | 160 | 5.620 | -16.945 | 3.856 | 1.00 | 14.12 | B |
| ATOM | 2637 | C | VAL | B | 160 | 3.332 | -14.120 | 5.098 | 1.00 | 14.13 | B |
| ATOM | 2638 | O | VAL | B | 160 | 3.065 | -14.042 | 6.301 | 1.00 | 17.06 | B |
| ATOM | 2639 | N | ILE | B | 161 | 3.305 | -13.070 | 4.286 | 1.00 | 14.26 | B |
| ATOM | 2640 | CA | ILE | B | 161 | 2.948 | -11.752 | 4.793 | 1.00 | 13.94 | B |
| ATOM | 2641 | CB | ILE | B | 161 | 3.112 | -10.683 | 3.690 | 1.00 | 15.34 | B |
| ATOM | 2642 | CG2 | ILE | B | 161 | 2.424 | -9.378 | 4.102 | 1.00 | 14.87 | B |
| ATOM | 2643 | CG1 | ILE | B | 161 | 4.609 | -10.465 | 3.428 | 1.00 | 15.04 | B |
| ATOM | 2644 | CD | ILE | B | 161 | 4.925 | -9.639 | 2.195 | 1.00 | 16.74 | B |
| ATOM | 2645 | C | ILE | B | 161 | 1.525 | -11.778 | 5.331 | 1.00 | 14.90 | B |
| ATOM | 2646 | O | ILE | B | 161 | 1.262 | -11.277 | 6.420 | 1.00 | 13.37 | B |
| ATOM | 2647 | N | GLY | B | 162 | 0.605 | -12.386 | 4.583 | 1.00 | 15.27 | B |
| ATOM | 2648 | CA | GLY | B | 162 | -0.765 | -12.466 | 5.054 | 1.00 | 15.79 | B |
| ATOM | 2649 | C | GLY | B | 162 | -0.849 | -13.228 | 6.366 | 1.00 | 17.03 | B |
| ATOM | 2650 | O | GLY | B | 162 | -1.584 | -12.847 | 7.276 | 1.00 | 14.47 | B |
| ATOM | 2651 | N | MET | B | 163 | -0.082 | -14.310 | 6.473 | 1.00 | 16.74 | B |
| ATOM | 2652 | CA | MET | B | 163 | -0.086 | -15.117 | 7.688 | 1.00 | 17.21 | B |
| ATOM | 2653 | CB | MET | B | 163 | 0.724 | -16.397 | 7.475 | 1.00 | 16.37 | B |
| ATOM | 2654 | CG | MET | B | 163 | 0.694 | -17.334 | 8.669 | 1.00 | 17.76 | B |
| ATOM | 2655 | SD | MET | B | 163 | 1.828 | -18.702 | 8.465 | 1.00 | 20.36 | B |
| ATOM | 2656 | CE | MET | B | 163 | 3.427 | -17.850 | 8.668 | 1.00 | 17.93 | B |
| ATOM | 2657 | C | MET | B | 163 | 0.491 | -14.348 | 8.879 | 1.00 | 18.19 | B |
| ATOM | 2658 | O | MET | B | 163 | -0.060 | -14.384 | 9.983 | 1.00 | 18.95 | B |
| ATOM | 2659 | N | ALA | B | 164 | 1.606 | -13.660 | 8.646 | 1.00 | 17.51 | B |
| ATOM | 2660 | CA | ALA | B | 164 | 2.265 | -12.881 | 9.693 | 1.00 | 17.68 | B |
| ATOM | 2661 | CB | ALA | B | 164 | 3.496 | -12.168 | 9.123 | 1.00 | 15.44 | B |
| ATOM | 2662 | C | ALA | B | 164 | 1.302 | -11.868 | 10.279 | 1.00 | 17.20 | B |
| ATOM | 2663 | O | ALA | B | 164 | 1.190 | -11.730 | 11.494 | 1.00 | 18.23 | B |
| ATOM | 2664 | N | ARG | B | 165 | 0.594 | -11.161 | 9.404 | 1.00 | 18.43 | B |
| ATOM | 2665 | CA | ARG | B | 165 | -0.364 | -10.157 | 9.846 | 1.00 | 19.07 | B |
| ATOM | 2666 | CB | ARG | B | 165 | -0.886 | -9.369 | 8.639 | 1.00 | 20.52 | B |
| ATOM | 2667 | CG | ARG | B | 165 | 0.190 | -8.427 | 8.116 | 1.00 | 23.01 | B |
| ATOM | 2668 | CD | ARG | B | 165 | -0.180 | -7.622 | 6.883 | 1.00 | 27.09 | B |
| ATOM | 2669 | NE | ARG | B | 165 | 0.802 | -6.553 | 6.691 | 1.00 | 29.08 | B |
| ATOM | 2670 | CZ | ARG | B | 165 | 0.979 | -5.866 | 5.567 | 1.00 | 30.84 | B |
| ATOM | 2671 | NH1 | ARG | B | 165 | 0.237 | -6.125 | 4.498 | 1.00 | 30.46 | B |
| ATOM | 2672 | NH2 | ARG | B | 165 | 1.905 | -4.912 | 5.513 | 1.00 | 31.88 | B |
| ATOM | 2673 | C | ARG | B | 165 | -1.496 | -10.764 | 10.648 | 1.00 | 18.55 | B |
| ATOM | 2674 | O | ARG | B | 165 | -2.015 | -10.137 | 11.572 | 1.00 | 19.30 | B |
| ATOM | 2675 | N | SER | B | 166 | -1.866 | -11.995 | 10.311 | 1.00 | 18.96 | B |
| ATOM | 2676 | CA | SER | B | 166 | -2.928 | -12.688 | 11.030 | 1.00 | 20.28 | B |
| ATOM | 2677 | CB | SER | B | 166 | -3.322 | -13.974 | 10.293 | 1.00 | 23.04 | B |
| ATOM | 2678 | OG | SER | B | 166 | -4.227 | -14.738 | 11.071 | 1.00 | 25.20 | B |
| ATOM | 2679 | C | SER | B | 166 | -2.408 | -13.027 | 12.423 | 1.00 | 20.25 | B |
| ATOM | 2680 | O | SER | B | 166 | -3.091 | -12.817 | 13.425 | 1.00 | 20.24 | B |
| ATOM | 2681 | N | ILE | B | 167 | -1.190 | -13.558 | 12.477 | 1.00 | 18.31 | B |
| ATOM | 2682 | CA | ILE | B | 167 | -0.569 | -13.899 | 13.752 | 1.00 | 18.83 | B |
| ATOM | 2683 | CB | ILE | B | 167 | 0.831 | -14.541 | 13.550 | 1.00 | 18.53 | B |
| ATOM | 2684 | CG2 | ILE | B | 167 | 1.522 | -14.710 | 14.906 | 1.00 | 18.91 | B |
| ATOM | 2685 | CG1 | ILE | B | 167 | 0.690 | -15.892 | 12.841 | 1.00 | 18.34 | B |
| ATOM | 2686 | CD | ILE | B | 167 | 2.000 | -16.521 | 12.420 | 1.00 | 17.40 | B |
| ATOM | 2687 | C | ILE | B | 167 | -0.407 | -12.637 | 14.598 | 1.00 | 19.31 | B |
| ATOM | 2688 | O | ILE | B | 167 | -0.695 | -12.637 | 15.789 | 1.00 | 18.83 | B |
| ATOM | 2689 | N | ALA | B | 168 | 0.054 | -11.557 | 13.978 | 1.00 | 19.50 | B |
| ATOM | 2690 | CA | ALA | B | 168 | 0.243 | -10.304 | 14.707 | 1.00 | 19.81 | B |
| ATOM | 2691 | CB | ALA | B | 168 | 0.848 | -9.246 | 13.792 | 1.00 | 18.78 | B |
| ATOM | 2692 | C | ALA | B | 168 | -1.086 | -9.809 | 15.259 | 1.00 | 20.69 | B |
| ATOM | 2693 | O | ALA | B | 168 | -1.173 | -9.352 | 16.401 | 1.00 | 20.68 | B |
| ATOM | 2694 | N | ARG | B | 169 | -2.130 | -9.907 | 14.446 | 1.00 | 21.86 | B |
| ATOM | 2695 | CA | ARG | B | 169 | -3.437 | -9.446 | 14.877 | 1.00 | 25.77 | B |
| ATOM | 2696 | CB | ARG | B | 169 | -4.435 | -9.554 | 13.719 | 1.00 | 28.78 | B |
| ATOM | 2697 | CG | ARG | B | 169 | -5.664 | -8.680 | 13.904 | 1.00 | 37.34 | B |

Figure 1 (suite 36)

| | | | | | | | | | | | |
|---|---|---|---|---|---|---|---|---|---|---|---|
| ATOM | 2698 | CD | ARG | B | 169 | -6.612 | -8.715 | 12.708 | 1.00 | 41.23 | B |
| ATOM | 2699 | NE | ARG | B | 169 | -5.967 | -8.331 | 11.454 | 1.00 | 44.69 | B |
| ATOM | 2700 | CZ | ARG | B | 169 | -5.379 | -9.187 | 10.623 | 1.00 | 46.95 | B |
| ATOM | 2701 | NH1 | ARG | B | 169 | -5.354 | -10.483 | 10.913 | 1.00 | 47.94 | B |
| ATOM | 2702 | NH2 | ARG | B | 169 | -4.820 | -8.750 | 9.500 | 1.00 | 45.61 | B |
| ATOM | 2703 | C | ARG | B | 169 | -3.920 | -10.249 | 16.080 | 1.00 | 25.76 | B |
| ATOM | 2704 | O | ARG | B | 169 | -4.596 | -9.726 | 16.960 | 1.00 | 26.32 | B |
| ATOM | 2705 | N | GLU | B | 170 | -3.533 | -11.518 | 16.132 | 1.00 | 25.55 | B |
| ATOM | 2706 | CA | GLU | B | 170 | -3.959 | -12.403 | 17.208 | 1.00 | 26.96 | B |
| ATOM | 2707 | CB | GLU | B | 170 | -4.050 | -13.838 | 16.676 | 1.00 | 28.60 | B |
| ATOM | 2708 | CG | GLU | B | 170 | -4.628 | -14.836 | 17.661 | 1.00 | 33.91 | B |
| ATOM | 2709 | CD | GLU | B | 170 | -4.478 | -16.275 | 17.191 | 1.00 | 39.03 | B |
| ATOM | 2710 | OE1 | GLU | B | 170 | -4.853 | -16.576 | 16.035 | 1.00 | 41.87 | B |
| ATOM | 2711 | OE2 | GLU | B | 170 | -3.988 | -17.112 | 17.982 | 1.00 | 41.37 | B |
| ATOM | 2712 | C | GLU | B | 170 | -3.125 | -12.415 | 18.493 | 1.00 | 25.51 | B |
| ATOM | 2713 | O | GLU | B | 170 | -3.680 | -12.401 | 19.587 | 1.00 | 24.49 | B |
| ATOM | 2714 | N | LEU | B | 171 | -1.803 | -12.430 | 18.366 | 1.00 | 23.20 | B |
| ATOM | 2715 | CA | LEU | B | 171 | -0.945 | -12.527 | 19.545 | 1.00 | 22.33 | B |
| ATOM | 2716 | CB | LEU | B | 171 | 0.117 | -13.602 | 19.295 | 1.00 | 22.32 | B |
| ATOM | 2717 | CG | LEU | B | 171 | -0.440 | -14.969 | 18.887 | 1.00 | 23.86 | B |
| ATOM | 2718 | CD1 | LEU | B | 171 | 0.690 | -15.936 | 18.570 | 1.00 | 23.20 | B |
| ATOM | 2719 | CD2 | LEU | B | 171 | -1.305 | -15.499 | 20.014 | 1.00 | 25.67 | B |
| ATOM | 2720 | C | LEU | B | 171 | -0.257 | -11.266 | 20.066 | 1.00 | 22.28 | B |
| ATOM | 2721 | O | LEU | B | 171 | 0.359 | -11.303 | 21.135 | 1.00 | 20.54 | B |
| ATOM | 2722 | N | SER | B | 172 | -0.353 | -10.160 | 19.334 | 1.00 | 21.49 | B |
| ATOM | 2723 | CA | SER | B | 172 | 0.291 | -8.927 | 19.770 | 1.00 | 22.29 | B |
| ATOM | 2724 | CB | SER | B | 172 | -0.016 | -7.788 | 18.796 | 1.00 | 22.21 | B |
| ATOM | 2725 | OG | SER | B | 172 | 0.677 | -7.983 | 17.575 | 1.00 | 22.36 | B |
| ATOM | 2726 | C | SER | B | 172 | -0.111 | -8.531 | 21.184 | 1.00 | 22.61 | B |
| ATOM | 2727 | O | SER | B | 172 | 0.689 | -7.961 | 21.922 | 1.00 | 21.75 | B |
| ATOM | 2728 | N | LYS | B | 173 | -1.343 | -8.846 | 21.567 | 1.00 | 23.20 | B |
| ATOM | 2729 | CA | LYS | B | 173 | -1.820 | -8.520 | 22.903 | 1.00 | 23.74 | B |
| ATOM | 2730 | CB | LYS | B | 173 | -3.313 | -8.849 | 23.029 | 1.00 | 25.00 | B |
| ATOM | 2731 | CG | LYS | B | 173 | -3.646 | -10.324 | 22.812 | 1.00 | 28.11 | B |
| ATOM | 2732 | CD | LYS | B | 173 | -5.104 | -10.639 | 23.148 | 1.00 | 30.37 | B |
| ATOM | 2733 | CE | LYS | B | 173 | -6.072 | -9.953 | 22.200 | 1.00 | 32.62 | B |
| ATOM | 2734 | NZ | LYS | B | 173 | -5.919 | -10.441 | 20.798 | 1.00 | 33.67 | B |
| ATOM | 2735 | C | LYS | B | 173 | -1.029 | -9.279 | 23.976 | 1.00 | 23.62 | B |
| ATOM | 2736 | O | LYS | B | 173 | -1.047 | -8.907 | 25.150 | 1.00 | 22.26 | B |
| ATOM | 2737 | N | ALA | B | 174 | -0.339 | -10.342 | 23.570 | 1.00 | 22.36 | B |
| ATOM | 2738 | CA | ALA | B | 174 | 0.456 | -11.145 | 24.499 | 1.00 | 22.72 | B |
| ATOM | 2739 | CB | ALA | B | 174 | 0.240 | -12.631 | 24.227 | 1.00 | 23.59 | B |
| ATOM | 2740 | C | ALA | B | 174 | 1.931 | -10.804 | 24.350 | 1.00 | 23.27 | B |
| ATOM | 2741 | O | ALA | B | 174 | 2.809 | -11.575 | 24.752 | 1.00 | 23.26 | B |
| ATOM | 2742 | N | ASN | B | 175 | 2.194 | -9.644 | 23.761 | 1.00 | 23.53 | B |
| ATOM | 2743 | CA | ASN | B | 175 | 3.553 | -9.181 | 23.535 | 1.00 | 22.47 | B |
| ATOM | 2744 | CB | ASN | B | 175 | 4.284 | -9.015 | 24.867 | 1.00 | 24.64 | B |
| ATOM | 2745 | CG | ASN | B | 175 | 5.675 | -8.458 | 24.692 | 1.00 | 26.56 | B |
| ATOM | 2746 | OD1 | ASN | B | 175 | 5.938 | -7.708 | 23.747 | 1.00 | 25.07 | B |
| ATOM | 2747 | ND2 | ASN | B | 175 | 6.575 | -8.808 | 25.608 | 1.00 | 26.51 | B |
| ATOM | 2748 | C | ASN | B | 175 | 4.315 | -10.122 | 22.606 | 1.00 | 22.45 | B |
| ATOM | 2749 | O | ASN | B | 175 | 5.533 | -10.308 | 22.727 | 1.00 | 21.92 | B |
| ATOM | 2750 | N | VAL | B | 176 | 3.574 | -10.719 | 21.677 | 1.00 | 21.65 | B |
| ATOM | 2751 | CA | VAL | B | 176 | 4.135 | -11.619 | 20.680 | 1.00 | 20.70 | B |
| ATOM | 2752 | CB | VAL | B | 176 | 3.453 | -13.005 | 20.716 | 1.00 | 22.34 | B |
| ATOM | 2753 | CG1 | VAL | B | 176 | 4.039 | -13.903 | 19.638 | 1.00 | 22.86 | B |
| ATOM | 2754 | CG2 | VAL | B | 176 | 3.637 | -13.640 | 22.075 | 1.00 | 19.79 | B |
| ATOM | 2755 | C | VAL | B | 176 | 3.869 | -10.960 | 19.329 | 1.00 | 20.95 | B |
| ATOM | 2756 | O | VAL | B | 176 | 2.727 | -10.864 | 18.881 | 1.00 | 19.16 | B |
| ATOM | 2757 | N | THR | B | 177 | 4.931 | -10.491 | 18.687 | 1.00 | 21.28 | B |
| ATOM | 2758 | CA | THR | B | 177 | 4.801 | -9.824 | 17.403 | 1.00 | 19.31 | B |
| ATOM | 2759 | CB | THR | B | 177 | 5.621 | -8.520 | 17.379 | 1.00 | 19.38 | B |
| ATOM | 2760 | OG1 | THR | B | 177 | 7.018 | -8.830 | 17.477 | 1.00 | 20.93 | B |
| ATOM | 2761 | CG2 | THR | B | 177 | 5.224 | -7.629 | 18.543 | 1.00 | 19.01 | B |
| ATOM | 2762 | C | THR | B | 177 | 5.260 | -10.692 | 16.243 | 1.00 | 19.00 | B |
| ATOM | 2763 | O | THR | B | 177 | 6.041 | -11.638 | 16.416 | 1.00 | 17.05 | B |
| ATOM | 2764 | N | ALA | B | 178 | 4.767 | -10.358 | 15.056 | 1.00 | 16.75 | B |
| ATOM | 2765 | CA | ALA | B | 178 | 5.128 | -11.072 | 13.841 | 1.00 | 14.50 | B |
| ATOM | 2766 | CB | ALA | B | 178 | 3.986 | -12.017 | 13.409 | 1.00 | 15.85 | B |
| ATOM | 2767 | C | ALA | B | 178 | 5.383 | -10.018 | 12.776 | 1.00 | 13.91 | B |
| ATOM | 2768 | O | ALA | B | 178 | 4.508 | -9.204 | 12.476 | 1.00 | 13.29 | B |
| ATOM | 2769 | N | ASN | B | 179 | 6.592 | -10.019 | 12.225 | 1.00 | 12.60 | B |
| ATOM | 2770 | CA | ASN | B | 179 | 6.973 | -9.057 | 11.204 | 1.00 | 13.37 | B |

Figure 1 (suite 37)

| | | | | | | | | | | | |
|---|---|---|---|---|---|---|---|---|---|---|---|
| ATOM | 2771 | CB | ASN | B | 179 | 7.914 | -8.002 | 11.789 | 1.00 | 11.29 | B |
| ATOM | 2772 | CG | ASN | B | 179 | 7.232 | -7.117 | 12.813 | 1.00 | 13.13 | B |
| ATOM | 2773 | OD1 | ASN | B | 179 | 6.259 | -6.430 | 12.499 | 1.00 | 10.95 | B |
| ATOM | 2774 | ND2 | ASN | B | 179 | 7.744 | -7.119 | 14.046 | 1.00 | 10.53 | B |
| ATOM | 2775 | C | ASN | B | 179 | 7.663 | -9.764 | 10.064 | 1.00 | 14.02 | B |
| ATOM | 2776 | O | ASN | B | 179 | 8.102 | -10.902 | 10.200 | 1.00 | 14.15 | B |
| ATOM | 2777 | N | VAL | B | 180 | 7.767 | -9.086 | 8.933 | 1.00 | 14.08 | B |
| ATOM | 2778 | CA | VAL | B | 180 | 8.411 | -9.682 | 7.784 | 1.00 | 13.17 | B |
| ATOM | 2779 | CB | VAL | B | 180 | 7.388 | -9.963 | 6.655 | 1.00 | 14.47 | B |
| ATOM | 2780 | CG1 | VAL | B | 180 | 8.094 | -10.569 | 5.460 | 1.00 | 11.10 | B |
| ATOM | 2781 | CG2 | VAL | B | 180 | 6.293 | -10.901 | 7.157 | 1.00 | 13.46 | B |
| ATOM | 2782 | C | VAL | B | 180 | 9.506 | -8.788 | 7.225 | 1.00 | 13.68 | B |
| ATOM | 2783 | O | VAL | B | 180 | 9.357 | -7.574 | 7.158 | 1.00 | 14.14 | B |
| ATOM | 2784 | N | VAL | B | 181 | 10.612 | -9.405 | 6.834 | 1.00 | 12.71 | B |
| ATOM | 2785 | CA | VAL | B | 181 | 11.722 | -8.689 | 6.234 | 1.00 | 12.67 | B |
| ATOM | 2786 | CB | VAL | B | 181 | 13.064 | -9.067 | 6.900 | 1.00 | 12.97 | B |
| ATOM | 2787 | CG1 | VAL | B | 181 | 14.215 | -8.445 | 6.141 | 1.00 | 12.54 | B |
| ATOM | 2788 | CG2 | VAL | B | 181 | 13.063 | -8.599 | 8.344 | 1.00 | 12.04 | B |
| ATOM | 2789 | C | VAL | B | 181 | 11.715 | -9.146 | 4.788 | 1.00 | 13.31 | B |
| ATOM | 2790 | O | VAL | B | 181 | 11.834 | -10.345 | 4.513 | 1.00 | 13.50 | B |
| ATOM | 2791 | N | ALA | B | 182 | 11.572 | -8.199 | 3.865 | 1.00 | 13.49 | B |
| ATOM | 2792 | CA | ALA | B | 182 | 11.521 | -8.523 | 2.445 | 1.00 | 14.71 | B |
| ATOM | 2793 | CB | ALA | B | 182 | 10.240 | -7.950 | 1.825 | 1.00 | 14.21 | B |
| ATOM | 2794 | C | ALA | B | 182 | 12.731 | -8.029 | 1.674 | 1.00 | 14.50 | B |
| ATOM | 2795 | O | ALA | B | 182 | 12.767 | -6.890 | 1.210 | 1.00 | 12.36 | B |
| ATOM | 2796 | N | PRO | B | 183 | 13.746 | -8.892 | 1.521 | 1.00 | 16.03 | B |
| ATOM | 2797 | CD | PRO | B | 183 | 13.874 | -10.244 | 2.096 | 1.00 | 17.07 | B |
| ATOM | 2798 | CA | PRO | B | 183 | 14.958 | -8.518 | 0.794 | 1.00 | 17.83 | B |
| ATOM | 2799 | CB | PRO | B | 183 | 15.927 | -9.646 | 1.139 | 1.00 | 18.22 | B |
| ATOM | 2800 | CG | PRO | B | 183 | 15.015 | -10.826 | 1.291 | 1.00 | 19.19 | B |
| ATOM | 2801 | C | PRO | B | 183 | 14.733 | -8.409 | -0.704 | 1.00 | 19.04 | B |
| ATOM | 2802 | O | PRO | B | 183 | 13.910 | -9.130 | -1.272 | 1.00 | 18.44 | B |
| ATOM | 2803 | N | GLY | B | 184 | 15.463 | -7.495 | -1.331 | 1.00 | 19.55 | B |
| ATOM | 2804 | CA | GLY | B | 184 | 15.367 | -7.322 | -2.763 | 1.00 | 22.67 | B |
| ATOM | 2805 | C | GLY | B | 184 | 16.482 | -8.163 | -3.343 | 1.00 | 24.82 | B |
| ATOM | 2806 | O | GLY | B | 184 | 16.585 | -9.357 | -3.037 | 1.00 | 24.72 | B |
| ATOM | 2807 | N | TYR | B | 185 | 17.327 | -7.557 | -4.168 | 1.00 | 26.16 | B |
| ATOM | 2808 | CA | TYR | B | 185 | 18.442 | -8.283 | -4.749 | 1.00 | 26.68 | B |
| ATOM | 2809 | CB | TYR | B | 185 | 18.881 | -7.652 | -6.071 | 1.00 | 30.43 | B |
| ATOM | 2810 | CG | TYR | B | 185 | 17.965 | -7.961 | -7.233 | 1.00 | 34.61 | B |
| ATOM | 2811 | CD1 | TYR | B | 185 | 16.904 | -7.119 | -7.560 | 1.00 | 37.00 | B |
| ATOM | 2812 | CE1 | TYR | B | 185 | 16.049 | -7.417 | -8.626 | 1.00 | 39.20 | B |
| ATOM | 2813 | CD2 | TYR | B | 185 | 18.150 | -9.114 | -7.999 | 1.00 | 37.54 | B |
| ATOM | 2814 | CE2 | TYR | B | 185 | 17.306 | -9.421 | -9.061 | 1.00 | 38.33 | B |
| ATOM | 2815 | CZ | TYR | B | 185 | 16.259 | -8.570 | -9.370 | 1.00 | 39.54 | B |
| ATOM | 2816 | OH | TYR | B | 185 | 15.429 | -8.876 | -10.422 | 1.00 | 41.80 | B |
| ATOM | 2817 | C | TYR | B | 185 | 19.605 | -8.279 | -3.768 | 1.00 | 26.75 | B |
| ATOM | 2818 | O | TYR | B | 185 | 20.192 | -7.234 | -3.487 | 1.00 | 23.34 | B |
| ATOM | 2819 | N | ILE | B | 186 | 19.918 | -9.458 | -3.242 | 1.00 | 25.52 | B |
| ATOM | 2820 | CA | ILE | B | 186 | 21.008 | -9.620 | -2.291 | 1.00 | 26.20 | B |
| ATOM | 2821 | CB | ILE | B | 186 | 20.502 | -10.204 | -0.966 | 1.00 | 22.70 | B |
| ATOM | 2822 | CG2 | ILE | B | 186 | 21.656 | -10.321 | 0.025 | 1.00 | 22.60 | B |
| ATOM | 2823 | CG1 | ILE | B | 186 | 19.384 | -9.323 | -0.408 | 1.00 | 21.56 | B |
| ATOM | 2824 | CD | ILE | B | 186 | 19.817 | -7.898 | -0.094 | 1.00 | 18.42 | B |
| ATOM | 2825 | C | ILE | B | 186 | 22.033 | -10.581 | -2.879 | 1.00 | 28.76 | B |
| ATOM | 2826 | O | ILE | B | 186 | 21.676 | -11.643 | -3.394 | 1.00 | 28.77 | B |
| ATOM | 2827 | N | ASP | B | 187 | 23.305 | -10.212 | -2.802 | 1.00 | 31.03 | B |
| ATOM | 2828 | CA | ASP | B | 187 | 24.365 | -11.054 | -3.339 | 1.00 | 34.80 | B |
| ATOM | 2829 | CB | ASP | B | 187 | 25.596 | -10.203 | -3.642 | 1.00 | 37.12 | B |
| ATOM | 2830 | CG | ASP | B | 187 | 26.748 | -11.024 | -4.173 | 1.00 | 40.07 | B |
| ATOM | 2831 | OD1 | ASP | B | 187 | 26.547 | -11.745 | -5.174 | 1.00 | 41.17 | B |
| ATOM | 2832 | OD2 | ASP | B | 187 | 27.851 | -10.949 | -3.589 | 1.00 | 41.81 | B |
| ATOM | 2833 | C | ASP | B | 187 | 24.752 | -12.193 | -2.396 | 1.00 | 35.68 | B |
| ATOM | 2834 | O | ASP | B | 187 | 25.438 | -11.975 | -1.398 | 1.00 | 35.33 | B |
| ATOM | 2835 | N | THR | B | 188 | 24.314 | -13.408 | -2.716 | 1.00 | 37.81 | B |
| ATOM | 2836 | CA | THR | B | 188 | 24.635 | -14.574 | -1.895 | 1.00 | 41.35 | B |
| ATOM | 2837 | CB | THR | B | 188 | 23.346 | -15.199 | -1.347 | 1.00 | 41.22 | B |
| ATOM | 2838 | OG1 | THR | B | 188 | 22.604 | -15.774 | -2.410 | 1.00 | 40.93 | B |
| ATOM | 2839 | CG2 | THR | B | 188 | 22.439 | -14.189 | -0.649 | 1.00 | 41.42 | B |
| ATOM | 2840 | C | THR | B | 188 | 25.381 | -15.624 | -2.711 | 1.00 | 43.44 | B |
| ATOM | 2841 | O | THR | B | 188 | 25.752 | -15.383 | -3.859 | 1.00 | 43.89 | B |
| ATOM | 2842 | N | ASP | B | 189 | 25.572 | -16.763 | -2.072 | 1.00 | 45.61 | B |
| ATOM | 2843 | CA | ASP | B | 189 | 26.269 | -17.909 | -2.671 | 1.00 | 47.03 | B |

Figure 1 (suite 38)

| | | | | | | | | | | | |
|---|---|---|---|---|---|---|---|---|---|---|---|
| ATOM | 2844 | CB | ASP | B | 189 | 26.280 | -19.083 | -1.693 | 1.00 | 47.92 | B |
| ATOM | 2845 | CG | ASP | B | 189 | 27.152 | -18.829 | -0.463 | 0.00 | 47.56 | B |
| ATOM | 2846 | OD1 | ASP | B | 189 | 28.135 | -18.002 | -0.530 | 0.00 | 47.66 | B |
| ATOM | 2847 | OD2 | ASP | B | 189 | 26.902 | -19.443 | 0.644 | 0.00 | 47.66 | B |
| ATOM | 2848 | C | ASP | B | 189 | 25.562 | -18.339 | -3.956 | 1.00 | 47.18 | B |
| ATOM | 2849 | O | ASP | B | 189 | 24.332 | -18.444 | -4.000 | 1.00 | 48.59 | B |
| ATOM | 2850 | N | ALA | B | 202 | 19.875 | -5.017 | -15.370 | 1.00 | 55.89 | B |
| ATOM | 2851 | CA | ALA | B | 202 | 18.678 | -5.074 | -14.539 | 1.00 | 55.72 | B |
| ATOM | 2852 | CB | ALA | B | 202 | 18.303 | -6.523 | -14.266 | 1.00 | 56.33 | B |
| ATOM | 2853 | C | ALA | B | 202 | 18.912 | -4.339 | -13.226 | 1.00 | 55.69 | B |
| ATOM | 2854 | O | ALA | B | 202 | 17.969 | -3.893 | -12.571 | 1.00 | 56.03 | B |
| ATOM | 2855 | N | LEU | B | 203 | 20.178 | -4.215 | -12.847 | 1.00 | 54.67 | B |
| ATOM | 2856 | CA | LEU | B | 203 | 20.541 | -3.533 | -11.614 | 1.00 | 53.55 | B |
| ATOM | 2857 | CB | LEU | B | 203 | 22.053 | -3.621 | -11.399 | 1.00 | 54.09 | B |
| ATOM | 2858 | CG | LEU | B | 203 | 22.604 | -3.149 | -10.055 | 1.00 | 53.44 | B |
| ATOM | 2859 | CD1 | LEU | B | 203 | 21.965 | -3.952 | -8.936 | 1.00 | 54.33 | B |
| ATOM | 2860 | CD2 | LEU | B | 203 | 24.116 | -3.320 | -10.039 | 1.00 | 54.66 | B |
| ATOM | 2861 | C | LEU | B | 203 | 20.106 | -2.072 | -11.684 | 1.00 | 52.90 | B |
| ATOM | 2862 | O | LEU | B | 203 | 19.979 | -1.401 | -10.660 | 1.00 | 53.12 | B |
| ATOM | 2863 | N | GLN | B | 204 | 19.877 | -1.588 | -12.903 | 1.00 | 51.35 | B |
| ATOM | 2864 | CA | GLN | B | 204 | 19.455 | -0.212 | -13.091 | 1.00 | 49.57 | B |
| ATOM | 2865 | CB | GLN | B | 204 | 19.505 | 0.150 | -14.590 | 1.00 | 49.64 | B |
| ATOM | 2866 | CG | GLN | B | 204 | 20.921 | 0.142 | -15.166 | 0.00 | 48.76 | B |
| ATOM | 2867 | CD | GLN | B | 204 | 20.965 | 0.525 | -16.646 | 0.00 | 48.24 | B |
| ATOM | 2868 | OE1 | GLN | B | 204 | 22.041 | 0.560 | -17.240 | 0.00 | 48.14 | B |
| ATOM | 2869 | NE2 | GLN | B | 204 | 19.851 | 0.820 | -17.285 | 0.00 | 48.14 | B |
| ATOM | 2870 | C | GLN | B | 204 | 18.047 | 0.027 | -12.582 | 1.00 | 48.06 | B |
| ATOM | 2871 | O | GLN | B | 204 | 17.609 | 1.172 | -12.451 | 1.00 | 48.88 | B |
| ATOM | 2872 | N | PHE | B | 205 | 17.336 | -1.058 | -12.295 | 1.00 | 46.07 | B |
| ATOM | 2873 | CA | PHE | B | 205 | 15.968 | -0.979 | -11.794 | 1.00 | 43.74 | B |
| ATOM | 2874 | CB | PHE | B | 205 | 15.242 | -2.306 | -12.037 | 1.00 | 43.49 | B |
| ATOM | 2875 | CG | PHE | B | 205 | 13.805 | -2.306 | -11.598 | 0.00 | 43.02 | B |
| ATOM | 2876 | CD1 | PHE | B | 205 | 12.876 | -1.469 | -12.207 | 0.00 | 43.03 | B |
| ATOM | 2877 | CD2 | PHE | B | 205 | 13.378 | -3.148 | -10.575 | 0.00 | 43.03 | B |
| ATOM | 2878 | CE1 | PHE | B | 205 | 11.542 | -1.470 | -11.806 | 0.00 | 42.86 | B |
| ATOM | 2879 | CE2 | PHE | B | 205 | 12.045 | -3.157 | -10.166 | 0.00 | 42.86 | B |
| ATOM | 2880 | CZ | PHE | B | 205 | 11.126 | -2.316 | -10.783 | 0.00 | 42.77 | B |
| ATOM | 2881 | C | PHE | B | 205 | 15.992 | -0.671 | -10.301 | 1.00 | 41.75 | B |
| ATOM | 2882 | O | PHE | B | 205 | 14.971 | -0.325 | -9.707 | 1.00 | 42.91 | B |
| ATOM | 2883 | N | ILE | B | 206 | 17.168 | -0.802 | -9.699 | 1.00 | 39.02 | B |
| ATOM | 2884 | CA | ILE | B | 206 | 17.333 | -0.536 | -8.275 | 1.00 | 35.56 | B |
| ATOM | 2885 | CB | ILE | B | 206 | 18.349 | -1.508 | -7.651 | 1.00 | 35.05 | B |
| ATOM | 2886 | CG2 | ILE | B | 206 | 18.475 | -1.242 | -6.154 | 1.00 | 35.65 | B |
| ATOM | 2887 | CG1 | ILE | B | 206 | 17.907 | -2.949 | -7.911 | 1.00 | 35.42 | B |
| ATOM | 2888 | CD | ILE | B | 206 | 18.874 | -3.994 | -7.386 | 1.00 | 35.44 | B |
| ATOM | 2889 | C | ILE | B | 206 | 17.823 | 0.896 | -8.071 | 1.00 | 33.11 | B |
| ATOM | 2890 | O | ILE | B | 206 | 18.905 | 1.260 | -8.526 | 1.00 | 32.97 | B |
| ATOM | 2891 | N | PRO | B | 207 | 17.025 | 1.729 | -7.387 | 1.00 | 30.99 | B |
| ATOM | 2892 | CD | PRO | B | 207 | 15.685 | 1.449 | -6.838 | 1.00 | 30.23 | B |
| ATOM | 2893 | CA | PRO | B | 207 | 17.411 | 3.122 | -7.141 | 1.00 | 29.47 | B |
| ATOM | 2894 | CB | PRO | B | 207 | 16.337 | 3.607 | -6.174 | 1.00 | 28.86 | B |
| ATOM | 2895 | CG | PRO | B | 207 | 15.126 | 2.841 | -6.630 | 1.00 | 29.53 | B |
| ATOM | 2896 | C | PRO | B | 207 | 18.821 | 3.245 | -6.562 | 1.00 | 28.93 | B |
| ATOM | 2897 | O | PRO | B | 207 | 19.579 | 4.141 | -6.938 | 1.00 | 28.26 | B |
| ATOM | 2898 | N | ALA | B | 208 | 19.162 | 2.343 | -5.644 | 1.00 | 27.59 | B |
| ATOM | 2899 | CA | ALA | B | 208 | 20.483 | 2.338 | -5.018 | 1.00 | 27.75 | B |
| ATOM | 2900 | CB | ALA | B | 208 | 20.518 | 1.314 | -3.885 | 1.00 | 26.29 | B |
| ATOM | 2901 | C | ALA | B | 208 | 21.568 | 2.019 | -6.048 | 1.00 | 28.17 | B |
| ATOM | 2902 | O | ALA | B | 208 | 22.751 | 2.296 | -5.830 | 1.00 | 28.24 | B |
| ATOM | 2903 | N | LYS | B | 209 | 21.150 | 1.439 | -7.170 | 1.00 | 28.42 | B |
| ATOM | 2904 | CA | LYS | B | 209 | 22.047 | 1.066 | -8.259 | 1.00 | 28.51 | B |
| ATOM | 2905 | CB | LYS | B | 209 | 22.661 | 2.318 | -8.898 | 1.00 | 30.44 | B |
| ATOM | 2906 | CG | LYS | B | 209 | 21.626 | 3.210 | -9.587 | 1.00 | 32.77 | B |
| ATOM | 2907 | CD | LYS | B | 209 | 20.852 | 2.425 | -10.643 | 1.00 | 35.73 | B |
| ATOM | 2908 | CE | LYS | B | 209 | 19.560 | 3.124 | -11.050 | 1.00 | 37.75 | B |
| ATOM | 2909 | NZ | LYS | B | 209 | 19.808 | 4.445 | -11.686 | 1.00 | 40.50 | B |
| ATOM | 2910 | C | LYS | B | 209 | 23.143 | 0.100 | -7.827 | 1.00 | 28.36 | B |
| ATOM | 2911 | O | LYS | B | 209 | 24.242 | 0.098 | -8.382 | 1.00 | 27.11 | B |
| ATOM | 2912 | N | ARG | B | 210 | 22.830 | -0.722 | -6.831 | 1.00 | 27.24 | B |
| ATOM | 2913 | CA | ARG | B | 210 | 23.765 | -1.719 | -6.331 | 1.00 | 25.85 | B |
| ATOM | 2914 | CB | ARG | B | 210 | 24.737 | -1.106 | -5.311 | 1.00 | 27.31 | B |
| ATOM | 2915 | CG | ARG | B | 210 | 24.153 | -0.856 | -3.920 | 1.00 | 26.93 | B |
| ATOM | 2916 | CD | ARG | B | 210 | 25.212 | -0.261 | -3.000 | 1.00 | 26.38 | B |

Figure 1 (suite 39)

| | | | | | | | | | | | |
|---|---|---|---|---|---|---|---|---|---|---|---|
| ATOM | 2917 | NE | ARG | B | 210 | 24.702 | 0.094 | -1.677 | 1.00 | 24.82 | B |
| ATOM | 2918 | CZ | ARG | B | 210 | 24.347 | -0.782 | -0.743 | 1.00 | 24.26 | B |
| ATOM | 2919 | NH1 | ARG | B | 210 | 24.440 | -2.087 | -0.975 | 1.00 | 21.63 | B |
| ATOM | 2920 | NH2 | ARG | B | 210 | 23.907 | -0.352 | 0.431 | 1.00 | 24.83 | B |
| ATOM | 2921 | C | ARG | B | 210 | 22.975 | -2.843 | -5.675 | 1.00 | 26.27 | B |
| ATOM | 2922 | O | ARG | B | 210 | 21.793 | -2.686 | -5.357 | 1.00 | 24.84 | B |
| ATOM | 2923 | N | VAL | B | 211 | 23.632 | -3.979 | -5.485 | 1.00 | 25.10 | B |
| ATOM | 2924 | CA | VAL | B | 211 | 22.995 | -5.122 | -4.857 | 1.00 | 25.90 | B |
| ATOM | 2925 | CB | VAL | B | 211 | 23.615 | -6.450 | -5.359 | 1.00 | 26.64 | B |
| ATOM | 2926 | CG1 | VAL | B | 211 | 25.038 | -6.595 | -4.843 | 1.00 | 26.09 | B |
| ATOM | 2927 | CG2 | VAL | B | 211 | 22.761 | -7.618 | -4.922 | 1.00 | 28.01 | B |
| ATOM | 2928 | C | VAL | B | 211 | 23.213 | -4.991 | -3.351 | 1.00 | 24.04 | B |
| ATOM | 2929 | O | VAL | B | 211 | 24.149 | -4.321 | -2.914 | 1.00 | 23.30 | B |
| ATOM | 2930 | N | GLY | B | 212 | 22.338 | -5.607 | -2.566 | 1.00 | 23.69 | B |
| ATOM | 2931 | CA | GLY | B | 212 | 22.478 | -5.547 | -1.124 | 1.00 | 23.07 | B |
| ATOM | 2932 | C | GLY | B | 212 | 23.343 | -6.699 | -0.649 | 1.00 | 24.74 | B |
| ATOM | 2933 | O | GLY | B | 212 | 23.579 | -7.650 | -1.397 | 1.00 | 23.47 | B |
| ATOM | 2934 | N | THR | B | 213 | 23.824 | -6.621 | 0.587 | 1.00 | 23.66 | B |
| ATOM | 2935 | CA | THR | B | 213 | 24.659 | -7.684 | 1.134 | 1.00 | 23.00 | B |
| ATOM | 2936 | CB | THR | B | 213 | 25.924 | -7.132 | 1.811 | 1.00 | 22.89 | B |
| ATOM | 2937 | OG1 | THR | B | 213 | 25.556 | -6.451 | 3.016 | 1.00 | 22.98 | B |
| ATOM | 2938 | CG2 | THR | B | 213 | 26.651 | -6.171 | 0.890 | 1.00 | 23.97 | B |
| ATOM | 2939 | C | THR | B | 213 | 23.884 | -8.448 | 2.191 | 1.00 | 22.89 | B |
| ATOM | 2940 | O | THR | B | 213 | 22.874 | -7.967 | 2.704 | 1.00 | 19.76 | B |
| ATOM | 2941 | N | PRO | B | 214 | 24.339 | -9.666 | 2.519 | 1.00 | 23.70 | B |
| ATOM | 2942 | CD | PRO | B | 214 | 25.324 | -10.481 | 1.779 | 1.00 | 23.09 | B |
| ATOM | 2943 | CA | PRO | B | 214 | 23.657 | -10.469 | 3.536 | 1.00 | 22.41 | B |
| ATOM | 2944 | CB | PRO | B | 214 | 24.478 | -11.757 | 3.557 | 1.00 | 22.67 | B |
| ATOM | 2945 | CG | PRO | B | 214 | 24.896 | -11.894 | 2.123 | 1.00 | 21.60 | B |
| ATOM | 2946 | C | PRO | B | 214 | 23.661 | -9.744 | 4.884 | 1.00 | 21.46 | B |
| ATOM | 2947 | O | PRO | B | 214 | 22.711 | -9.843 | 5.661 | 1.00 | 21.19 | B |
| ATOM | 2948 | N | ALA | B | 215 | 24.734 | -9.004 | 5.147 | 1.00 | 21.03 | B |
| ATOM | 2949 | CA | ALA | B | 215 | 24.862 | -8.256 | 6.395 | 1.00 | 19.81 | B |
| ATOM | 2950 | CB | ALA | B | 215 | 26.260 | -7.655 | 6.497 | 1.00 | 20.64 | B |
| ATOM | 2951 | C | ALA | B | 215 | 23.808 | -7.153 | 6.484 | 1.00 | 19.97 | B |
| ATOM | 2952 | O | ALA | B | 215 | 23.323 | -6.832 | 7.570 | 1.00 | 18.90 | B |
| ATOM | 2953 | N | GLU | B | 216 | 23.456 | -6.571 | 5.341 | 1.00 | 17.73 | B |
| ATOM | 2954 | CA | GLU | B | 216 | 22.452 | -5.511 | 5.326 | 1.00 | 18.91 | B |
| ATOM | 2955 | CB | GLU | B | 216 | 22.469 | -4.789 | 3.979 | 1.00 | 19.59 | B |
| ATOM | 2956 | CG | GLU | B | 216 | 23.725 | -3.936 | 3.807 | 1.00 | 21.35 | B |
| ATOM | 2957 | CD | GLU | B | 216 | 23.781 | -3.214 | 2.481 | 1.00 | 21.23 | B |
| ATOM | 2958 | OE1 | GLU | B | 216 | 23.726 | -3.890 | 1.432 | 1.00 | 21.28 | B |
| ATOM | 2959 | OE2 | GLU | B | 216 | 23.885 | -1.968 | 2.489 | 1.00 | 21.50 | B |
| ATOM | 2960 | C | GLU | B | 216 | 21.064 | -6.048 | 5.651 | 1.00 | 18.63 | B |
| ATOM | 2961 | O | GLU | B | 216 | 20.192 | -5.303 | 6.109 | 1.00 | 20.35 | B |
| ATOM | 2962 | N | VAL | B | 217 | 20.869 | -7.346 | 5.427 | 1.00 | 16.63 | B |
| ATOM | 2963 | CA | VAL | B | 217 | 19.606 | -8.000 | 5.741 | 1.00 | 15.24 | B |
| ATOM | 2964 | CB | VAL | B | 217 | 19.418 | -9.306 | 4.926 | 1.00 | 15.84 | B |
| ATOM | 2965 | CG1 | VAL | B | 217 | 18.193 | -10.049 | 5.417 | 1.00 | 16.45 | B |
| ATOM | 2966 | CG2 | VAL | B | 217 | 19.277 | -8.982 | 3.446 | 1.00 | 19.21 | B |
| ATOM | 2967 | C | VAL | B | 217 | 19.655 | -8.352 | 7.225 | 1.00 | 13.94 | B |
| ATOM | 2968 | O | VAL | B | 217 | 18.710 | -8.099 | 7.971 | 1.00 | 12.18 | B |
| ATOM | 2969 | N | ALA | B | 218 | 20.770 | -8.951 | 7.638 | 1.00 | 12.78 | B |
| ATOM | 2970 | CA | ALA | B | 218 | 20.980 | -9.347 | 9.026 | 1.00 | 12.90 | B |
| ATOM | 2971 | CB | ALA | B | 218 | 22.394 | -9.912 | 9.188 | 1.00 | 10.44 | B |
| ATOM | 2972 | C | ALA | B | 218 | 20.779 | -8.156 | 9.965 | 1.00 | 12.71 | B |
| ATOM | 2973 | O | ALA | B | 218 | 20.206 | -8.288 | 11.048 | 1.00 | 15.39 | B |
| ATOM | 2974 | N | GLY | B | 219 | 21.249 | -6.990 | 9.540 | 1.00 | 14.52 | B |
| ATOM | 2975 | CA | GLY | B | 219 | 21.106 | -5.796 | 10.362 | 1.00 | 14.80 | B |
| ATOM | 2976 | C | GLY | B | 219 | 19.659 | -5.474 | 10.700 | 1.00 | 14.83 | B |
| ATOM | 2977 | O | GLY | B | 219 | 19.343 | -5.027 | 11.807 | 1.00 | 13.27 | B |
| ATOM | 2978 | N | VAL | B | 220 | 18.765 | -5.701 | 9.744 | 1.00 | 12.96 | B |
| ATOM | 2979 | CA | VAL | B | 220 | 17.352 | -5.423 | 9.971 | 1.00 | 12.77 | B |
| ATOM | 2980 | CB | VAL | B | 220 | 16.565 | -5.456 | 8.641 | 1.00 | 16.15 | B |
| ATOM | 2981 | CG1 | VAL | B | 220 | 15.157 | -4.917 | 8.854 | 1.00 | 14.91 | B |
| ATOM | 2982 | CG2 | VAL | B | 220 | 17.301 | -4.642 | 7.592 | 1.00 | 19.30 | B |
| ATOM | 2983 | C | VAL | B | 220 | 16.774 | -6.449 | 10.935 | 1.00 | 10.79 | B |
| ATOM | 2984 | O | VAL | B | 220 | 15.959 | -6.123 | 11.791 | 1.00 | 9.23 | B |
| ATOM | 2985 | N | VAL | B | 221 | 17.199 | -7.700 | 10.797 | 1.00 | 10.86 | B |
| ATOM | 2986 | CA | VAL | B | 221 | 16.720 | -8.746 | 11.696 | 1.00 | 10.68 | B |
| ATOM | 2987 | CB | VAL | B | 221 | 17.225 | -10.145 | 11.250 | 1.00 | 9.89 | B |
| ATOM | 2988 | CG1 | VAL | B | 221 | 16.681 | -11.217 | 12.180 | 1.00 | 8.98 | B |
| ATOM | 2989 | CG2 | VAL | B | 221 | 16.781 | -10.418 | 9.816 | 1.00 | 13.64 | B |

Figure 1 (suite 40)

| | | | | | | | | | | |
|---|---|---|---|---|---|---|---|---|---|---|
| ATOM | 2990 | C | VAL | B | 221 | 17.214 | -8.437 | 13.110 | 1.00 | 10.44 | B |
| ATOM | 2991 | O | VAL | B | 221 | 16.470 | -8.569 | 14.077 | 1.00 | 11.42 | B |
| ATOM | 2992 | N | SER | B | 222 | 18.470 | -8.008 | 13.225 | 1.00 | 11.81 | B |
| ATOM | 2993 | CA | SER | B | 222 | 19.024 | -7.663 | 14.540 | 1.00 | 12.78 | B |
| ATOM | 2994 | CB | SER | B | 222 | 20.431 | -7.060 | 14.393 | 1.00 | 12.02 | B |
| ATOM | 2995 | OG | SER | B | 222 | 20.945 | -6.653 | 15.656 | 1.00 | 10.39 | B |
| ATOM | 2996 | C | SER | B | 222 | 18.090 | -6.651 | 15.201 | 1.00 | 12.71 | B |
| ATOM | 2997 | O | SER | B | 222 | 17.722 | -6.796 | 16.361 | 1.00 | 13.20 | B |
| ATOM | 2998 | N | PHE | B | 223 | 17.695 | -5.632 | 14.443 | 1.00 | 11.86 | B |
| ATOM | 2999 | CA | PHE | B | 223 | 16.785 | -4.605 | 14.955 | 1.00 | 12.61 | B |
| ATOM | 3000 | CB | PHE | B | 223 | 16.551 | -3.528 | 13.886 | 1.00 | 12.25 | B |
| ATOM | 3001 | CG | PHE | B | 223 | 15.407 | -2.606 | 14.200 | 1.00 | 15.51 | B |
| ATOM | 3002 | CD1 | PHE | B | 223 | 15.455 | -1.765 | 15.310 | 1.00 | 13.76 | B |
| ATOM | 3003 | CD2 | PHE | B | 223 | 14.263 | -2.598 | 13.398 | 1.00 | 13.95 | B |
| ATOM | 3004 | CE1 | PHE | B | 223 | 14.377 | -0.929 | 15.623 | 1.00 | 15.97 | B |
| ATOM | 3005 | CE2 | PHE | B | 223 | 13.179 | -1.769 | 13.700 | 1.00 | 15.64 | B |
| ATOM | 3006 | CZ | PHE | B | 223 | 13.234 | -0.934 | 14.812 | 1.00 | 16.87 | B |
| ATOM | 3007 | C | PHE | B | 223 | 15.439 | -5.198 | 15.390 | 1.00 | 12.23 | B |
| ATOM | 3008 | O | PHE | B | 223 | 14.964 | -4.956 | 16.503 | 1.00 | 11.84 | B |
| ATOM | 3009 | N | LEU | B | 224 | 14.819 | -5.978 | 14.512 | 1.00 | 11.81 | B |
| ATOM | 3010 | CA | LEU | B | 224 | 13.525 | -6.577 | 14.846 | 1.00 | 12.22 | B |
| ATOM | 3011 | CB | LEU | B | 224 | 12.945 | -7.312 | 13.630 | 1.00 | 12.58 | B |
| ATOM | 3012 | CG | LEU | B | 224 | 12.434 | -6.372 | 12.531 | 1.00 | 11.82 | B |
| ATOM | 3013 | CD1 | LEU | B | 224 | 11.885 | -7.181 | 11.364 | 1.00 | 14.57 | B |
| ATOM | 3014 | CD2 | LEU | B | 224 | 11.356 | -5.468 | 13.105 | 1.00 | 11.19 | B |
| ATOM | 3015 | C | LEU | B | 224 | 13.607 | -7.520 | 16.042 | 1.00 | 12.40 | B |
| ATOM | 3016 | O | LEU | B | 224 | 12.634 | -7.698 | 16.771 | 1.00 | 14.37 | B |
| ATOM | 3017 | N | ALA | B | 225 | 14.772 | -8.120 | 16.257 | 1.00 | 13.34 | B |
| ATOM | 3018 | CA | ALA | B | 225 | 14.932 | -9.023 | 17.395 | 1.00 | 14.76 | B |
| ATOM | 3019 | CB | ALA | B | 225 | 16.014 | -10.052 | 17.089 | 1.00 | 13.56 | B |
| ATOM | 3020 | C | ALA | B | 225 | 15.265 | -8.274 | 18.694 | 1.00 | 17.27 | B |
| ATOM | 3021 | O | ALA | B | 225 | 15.254 | -8.864 | 19.776 | 1.00 | 20.63 | B |
| ATOM | 3022 | N | SER | B | 226 | 15.524 | -6.970 | 18.596 | 1.00 | 15.40 | B |
| ATOM | 3023 | CA | SER | B | 226 | 15.890 | -6.175 | 19.774 | 1.00 | 16.90 | B |
| ATOM | 3024 | CB | SER | B | 226 | 16.827 | -5.030 | 19.363 | 1.00 | 15.39 | B |
| ATOM | 3025 | OG | SER | B | 226 | 16.127 | -4.024 | 18.644 | 1.00 | 12.49 | B |
| ATOM | 3026 | C | SER | B | 226 | 14.732 | -5.589 | 20.579 | 1.00 | 17.32 | B |
| ATOM | 3027 | O | SER | B | 226 | 13.564 | -5.655 | 20.179 | 1.00 | 16.83 | B |
| ATOM | 3028 | N | GLU | B | 227 | 15.074 | -5.005 | 21.723 | 1.00 | 16.28 | B |
| ATOM | 3029 | CA | GLU | B | 227 | 14.091 | -4.378 | 22.597 | 1.00 | 17.51 | B |
| ATOM | 3030 | CB | GLU | B | 227 | 14.736 | -4.035 | 23.943 | 1.00 | 17.36 | B |
| ATOM | 3031 | CG | GLU | B | 227 | 15.174 | -5.263 | 24.736 | 1.00 | 19.09 | B |
| ATOM | 3032 | CD | GLU | B | 227 | 14.000 | -6.056 | 25.272 | 1.00 | 22.46 | B |
| ATOM | 3033 | OE1 | GLU | B | 227 | 13.380 | -5.614 | 26.263 | 1.00 | 25.10 | B |
| ATOM | 3034 | OE2 | GLU | B | 227 | 13.689 | -7.118 | 24.698 | 1.00 | 23.05 | B |
| ATOM | 3035 | C | GLU | B | 227 | 13.487 | -3.113 | 21.977 | 1.00 | 17.29 | B |
| ATOM | 3036 | O | GLU | B | 227 | 12.440 | -2.648 | 22.418 | 1.00 | 18.76 | B |
| ATOM | 3037 | N | ASP | B | 228 | 14.145 | -2.568 | 20.958 | 1.00 | 16.74 | B |
| ATOM | 3038 | CA | ASP | B | 228 | 13.671 | -1.360 | 20.268 | 1.00 | 16.66 | B |
| ATOM | 3039 | CB | ASP | B | 228 | 14.803 | -0.735 | 19.446 | 1.00 | 16.67 | B |
| ATOM | 3040 | CG | ASP | B | 228 | 15.746 | 0.117 | 20.278 | 1.00 | 14.83 | B |
| ATOM | 3041 | OD1 | ASP | B | 228 | 16.935 | 0.178 | 19.913 | 1.00 | 16.75 | B |
| ATOM | 3042 | OD2 | ASP | B | 228 | 15.299 | 0.736 | 21.263 | 1.00 | 14.12 | B |
| ATOM | 3043 | C | ASP | B | 228 | 12.496 | -1.609 | 19.313 | 1.00 | 16.89 | B |
| ATOM | 3044 | O | ASP | B | 228 | 11.860 | -0.658 | 18.853 | 1.00 | 17.54 | B |
| ATOM | 3045 | N | ALA | B | 229 | 12.210 | -2.870 | 19.005 | 1.00 | 14.65 | B |
| ATOM | 3046 | CA | ALA | B | 229 | 11.132 | -3.181 | 18.061 | 1.00 | 16.45 | B |
| ATOM | 3047 | CB | ALA | B | 229 | 11.617 | -4.240 | 17.072 | 1.00 | 14.50 | B |
| ATOM | 3048 | C | ALA | B | 229 | 9.807 | -3.625 | 18.676 | 1.00 | 16.29 | B |
| ATOM | 3049 | O | ALA | B | 229 | 8.934 | -4.131 | 17.969 | 1.00 | 14.67 | B |
| ATOM | 3050 | N | SER | B | 230 | 9.652 | -3.403 | 19.976 | 1.00 | 17.21 | B |
| ATOM | 3051 | CA | SER | B | 230 | 8.463 | -3.817 | 20.714 | 1.00 | 18.51 | B |
| ATOM | 3052 | CB | SER | B | 230 | 8.605 | -3.410 | 22.190 | 1.00 | 19.70 | B |
| ATOM | 3053 | OG | SER | B | 230 | 8.969 | -2.038 | 22.315 | 1.00 | 21.98 | B |
| ATOM | 3054 | C | SER | B | 230 | 7.103 | -3.358 | 20.190 | 1.00 | 17.36 | B |
| ATOM | 3055 | O | SER | B | 230 | 6.140 | -4.126 | 20.238 | 1.00 | 20.09 | B |
| ATOM | 3056 | N | TYR | B | 231 | 7.007 | -2.128 | 19.690 | 1.00 | 17.86 | B |
| ATOM | 3057 | CA | TYR | B | 231 | 5.722 | -1.635 | 19.192 | 1.00 | 16.10 | B |
| ATOM | 3058 | CB | TYR | B | 231 | 5.521 | -0.160 | 19.564 | 1.00 | 16.32 | B |
| ATOM | 3059 | CG | TYR | B | 231 | 4.060 | 0.233 | 19.753 | 1.00 | 18.12 | B |
| ATOM | 3060 | CD1 | TYR | B | 231 | 3.237 | -0.472 | 20.633 | 1.00 | 17.54 | B |
| ATOM | 3061 | CE1 | TYR | B | 231 | 1.906 | -0.093 | 20.840 | 1.00 | 16.86 | B |
| ATOM | 3062 | CD2 | TYR | B | 231 | 3.516 | 1.327 | 19.079 | 1.00 | 17.35 | B |

Figure 1 (suite 41)

| | | | | | | | | | | | |
|---|---|---|---|---|---|---|---|---|---|---|---|
| ATOM | 3063 | CE2 | TYR | B | 231 | 2.188 | 1.716 | 19.281 | 1.00 | 16.69 | B |
| ATOM | 3064 | CZ | TYR | B | 231 | 1.393 | 1.002 | 20.160 | 1.00 | 17.90 | B |
| ATOM | 3065 | OH | TYR | B | 231 | 0.091 | 1.391 | 20.375 | 1.00 | 18.97 | B |
| ATOM | 3066 | C | TYR | B | 231 | 5.556 | -1.816 | 17.687 | 1.00 | 15.29 | B |
| ATOM | 3067 | O | TYR | B | 231 | 4.687 | -1.199 | 17.071 | 1.00 | 13.42 | B |
| ATOM | 3068 | N | ILE | B | 232 | 6.403 | -2.646 | 17.089 | 1.00 | 14.24 | B |
| ATOM | 3069 | CA | ILE | B | 232 | 6.283 | -2.919 | 15.665 | 1.00 | 13.46 | B |
| ATOM | 3070 | CB | ILE | B | 232 | 7.634 | -2.894 | 14.945 | 1.00 | 13.94 | B |
| ATOM | 3071 | CG2 | ILE | B | 232 | 7.422 | -3.155 | 13.455 | 1.00 | 14.45 | B |
| ATOM | 3072 | CG1 | ILE | B | 232 | 8.324 | -1.540 | 15.161 | 1.00 | 16.47 | B |
| ATOM | 3073 | CD | ILE | B | 232 | 9.635 | -1.410 | 14.426 | 1.00 | 16.61 | B |
| ATOM | 3074 | C | ILE | B | 232 | 5.702 | -4.324 | 15.533 | 1.00 | 11.95 | B |
| ATOM | 3075 | O | ILE | B | 232 | 6.278 | -5.285 | 16.049 | 1.00 | 13.51 | B |
| ATOM | 3076 | N | SER | B | 233 | 4.558 | -4.436 | 14.859 | 1.00 | 13.23 | B |
| ATOM | 3077 | CA | SER | B | 233 | 3.918 | -5.736 | 14.656 | 1.00 | 14.53 | B |
| ATOM | 3078 | CB | SER | B | 233 | 3.042 | -6.091 | 15.861 | 1.00 | 13.70 | B |
| ATOM | 3079 | OG | SER | B | 233 | 2.670 | -7.457 | 15.845 | 1.00 | 14.88 | B |
| ATOM | 3080 | C | SER | B | 233 | 3.071 | -5.729 | 13.380 | 1.00 | 13.28 | B |
| ATOM | 3081 | O | SER | B | 233 | 2.353 | -4.769 | 13.114 | 1.00 | 14.47 | B |
| ATOM | 3082 | N | GLY | B | 234 | 3.184 | -6.799 | 12.590 | 1.00 | 14.82 | B |
| ATOM | 3083 | CA | GLY | B | 234 | 2.436 | -6.915 | 11.348 | 1.00 | 13.64 | B |
| ATOM | 3084 | C | GLY | B | 234 | 3.036 | -6.092 | 10.227 | 1.00 | 13.60 | B |
| ATOM | 3085 | O | GLY | B | 234 | 2.414 | -5.892 | 9.179 | 1.00 | 13.21 | B |
| ATOM | 3086 | N | ALA | B | 235 | 4.262 | -5.632 | 10.435 | 1.00 | 13.14 | B |
| ATOM | 3087 | CA | ALA | B | 235 | 4.947 | -4.804 | 9.453 | 1.00 | 13.56 | B |
| ATOM | 3088 | CB | ALA | B | 235 | 5.849 | -3.789 | 10.182 | 1.00 | 10.18 | B |
| ATOM | 3089 | C | ALA | B | 235 | 5.771 | -5.593 | 8.451 | 1.00 | 14.07 | B |
| ATOM | 3090 | O | ALA | B | 235 | 6.223 | -6.704 | 8.737 | 1.00 | 12.52 | B |
| ATOM | 3091 | N | VAL | B | 236 | 5.952 | -5.019 | 7.266 | 1.00 | 13.62 | B |
| ATOM | 3092 | CA | VAL | B | 236 | 6.760 | -5.644 | 6.231 | 1.00 | 13.08 | B |
| ATOM | 3093 | CB | VAL | B | 236 | 5.966 | -5.899 | 4.916 | 1.00 | 14.47 | B |
| ATOM | 3094 | CG1 | VAL | B | 236 | 6.896 | -6.495 | 3.860 | 1.00 | 13.75 | B |
| ATOM | 3095 | CG2 | VAL | B | 236 | 4.805 | -6.846 | 5.171 | 1.00 | 12.41 | B |
| ATOM | 3096 | C | VAL | B | 236 | 7.870 | -4.650 | 5.932 | 1.00 | 14.50 | B |
| ATOM | 3097 | O | VAL | B | 236 | 7.611 | -3.573 | 5.386 | 1.00 | 13.40 | B |
| ATOM | 3098 | N | ILE | B | 237 | 9.103 | -4.995 | 6.293 | 1.00 | 11.99 | B |
| ATOM | 3099 | CA | ILE | B | 237 | 10.211 | -4.085 | 6.040 | 1.00 | 14.33 | B |
| ATOM | 3100 | CB | ILE | B | 237 | 11.152 | -3.976 | 7.273 | 1.00 | 15.01 | B |
| ATOM | 3101 | CG2 | ILE | B | 237 | 12.253 | -2.936 | 7.007 | 1.00 | 13.31 | B |
| ATOM | 3102 | CG1 | ILE | B | 237 | 10.331 | -3.587 | 8.506 | 1.00 | 15.76 | B |
| ATOM | 3103 | CD | ILE | B | 237 | 11.151 | -3.352 | 9.760 | 1.00 | 18.86 | B |
| ATOM | 3104 | C | ILE | B | 237 | 11.015 | -4.502 | 4.828 | 1.00 | 13.62 | B |
| ATOM | 3105 | O | ILE | B | 237 | 11.636 | -5.567 | 4.809 | 1.00 | 14.79 | B |
| ATOM | 3106 | N | PRO | B | 238 | 10.994 | -3.673 | 3.776 | 1.00 | 14.27 | B |
| ATOM | 3107 | CD | PRO | B | 238 | 10.140 | -2.486 | 3.601 | 1.00 | 14.07 | B |
| ATOM | 3108 | CA | PRO | B | 238 | 11.733 | -3.962 | 2.547 | 1.00 | 14.14 | B |
| ATOM | 3109 | CB | PRO | B | 238 | 11.005 | -3.114 | 1.512 | 1.00 | 15.55 | B |
| ATOM | 3110 | CG | PRO | B | 238 | 10.662 | -1.900 | 2.299 | 1.00 | 15.53 | B |
| ATOM | 3111 | C | PRO | B | 238 | 13.199 | -3.559 | 2.684 | 1.00 | 14.33 | B |
| ATOM | 3112 | O | PRO | B | 238 | 13.501 | -2.469 | 3.172 | 1.00 | 14.70 | B |
| ATOM | 3113 | N | VAL | B | 239 | 14.095 | -4.454 | 2.277 | 1.00 | 13.08 | B |
| ATOM | 3114 | CA | VAL | B | 239 | 15.540 | -4.215 | 2.315 | 1.00 | 16.18 | B |
| ATOM | 3115 | CB | VAL | B | 239 | 16.260 | -5.218 | 3.252 | 1.00 | 16.22 | B |
| ATOM | 3116 | CG1 | VAL | B | 239 | 17.729 | -4.839 | 3.392 | 1.00 | 15.88 | B |
| ATOM | 3117 | CG2 | VAL | B | 239 | 15.580 | -5.240 | 4.607 | 1.00 | 17.34 | B |
| ATOM | 3118 | C | VAL | B | 239 | 15.945 | -4.481 | 0.874 | 1.00 | 17.63 | B |
| ATOM | 3119 | O | VAL | B | 239 | 16.455 | -5.551 | 0.538 | 1.00 | 16.58 | B |
| ATOM | 3120 | N | ASP | B | 240 | 15.706 | -3.489 | 0.024 | 1.00 | 18.72 | B |
| ATOM | 3121 | CA | ASP | B | 240 | 15.956 | -3.625 | -1.400 | 1.00 | 19.99 | B |
| ATOM | 3122 | CB | ASP | B | 240 | 14.627 | -3.925 | -2.095 | 1.00 | 18.56 | B |
| ATOM | 3123 | CG | ASP | B | 240 | 13.553 | -2.923 | -1.724 | 1.00 | 17.40 | B |
| ATOM | 3124 | OD1 | ASP | B | 240 | 13.895 | -1.742 | -1.511 | 1.00 | 20.05 | B |
| ATOM | 3125 | OD2 | ASP | B | 240 | 12.372 | -3.302 | -1.641 | 1.00 | 20.53 | B |
| ATOM | 3126 | C | ASP | B | 240 | 16.585 | -2.410 | -2.069 | 1.00 | 20.52 | B |
| ATOM | 3127 | O | ASP | B | 240 | 16.593 | -2.318 | -3.297 | 1.00 | 22.35 | B |
| ATOM | 3128 | N | GLY | B | 241 | 17.101 | -1.478 | -1.279 | 1.00 | 20.38 | B |
| ATOM | 3129 | CA | GLY | B | 241 | 17.707 | -0.300 | -1.868 | 1.00 | 21.59 | B |
| ATOM | 3130 | C | GLY | B | 241 | 16.692 | 0.478 | -2.684 | 1.00 | 23.34 | B |
| ATOM | 3131 | O | GLY | B | 241 | 17.030 | 1.066 | -3.713 | 1.00 | 23.48 | B |
| ATOM | 3132 | N | GLY | B | 242 | 15.442 | 0.467 | -2.228 | 1.00 | 24.13 | B |
| ATOM | 3133 | CA | GLY | B | 242 | 14.384 | 1.184 | -2.917 | 1.00 | 27.94 | B |
| ATOM | 3134 | C | GLY | B | 242 | 13.711 | 0.454 | -4.070 | 1.00 | 30.64 | B |
| ATOM | 3135 | O | GLY | B | 242 | 12.677 | 0.905 | -4.567 | 1.00 | 29.25 | B |

Figure 1 (suite 42)

| | | | | | | | | | | | |
|---|---|---|---|---|---|---|---|---|---|---|---|
| ATOM | 3136 | N | MET | B | 243 | 14.281 | -0.670 | -4.497 | 1.00 | 34.19 | B |
| ATOM | 3137 | CA | MET | B | 243 | 13.724 | -1.434 | -5.616 | 1.00 | 36.93 | B |
| ATOM | 3138 | CB | MET | B | 243 | 14.368 | -2.816 | -5.712 | 1.00 | 39.13 | B |
| ATOM | 3139 | CG | MET | B | 243 | 13.826 | -3.631 | -6.875 | 1.00 | 43.70 | B |
| ATOM | 3140 | SD | MET | B | 243 | 14.208 | -5.382 | -6.787 | 1.00 | 48.51 | B |
| ATOM | 3141 | CE | MET | B | 243 | 12.996 | -5.916 | -5.559 | 1.00 | 47.29 | B |
| ATOM | 3142 | C | MET | B | 243 | 12.214 | -1.618 | -5.545 | 1.00 | 37.62 | B |
| ATOM | 3143 | O | MET | B | 243 | 11.485 | -1.201 | -6.446 | 1.00 | 37.87 | B |
| ATOM | 3144 | N | GLY | B | 244 | 11.750 | -2.257 | -4.477 | 1.00 | 37.95 | B |
| ATOM | 3145 | CA | GLY | B | 244 | 10.327 | -2.496 | -4.319 | 1.00 | 39.12 | B |
| ATOM | 3146 | C | GLY | B | 244 | 9.491 | -1.231 | -4.326 | 1.00 | 40.01 | B |
| ATOM | 3147 | O | GLY | B | 244 | 8.306 | -1.264 | -3.987 | 1.00 | 40.20 | B |
| ATOM | 3148 | CS+1 | CS1 | C | 1 | 28.017 | 4.543 | 19.973 | 1.00 | 39.77 | C |
| ATOM | 3149 | CS+1 | CS1 | C | 2 | 24.702 | -6.974 | 23.879 | 1.00 | 39.94 | C |
| ATOM | 3150 | CS+1 | CS1 | C | 3 | 26.739 | 12.516 | 22.115 | 0.50 | 24.88 | C |
| ATOM | 3151 | CS+1 | CS1 | C | 4 | 26.718 | -15.015 | 22.111 | 0.50 | 32.10 | C |
| ATOM | 3152 | OH2 | TIP | S | 1 | 1.999 | 3.685 | 3.164 | 1.00 | 45.55 | S |
| ATOM | 3153 | OH2 | TIP | S | 2 | 10.616 | -15.319 | 27.312 | 1.00 | 18.97 | S |
| ATOM | 3154 | OH2 | TIP | S | 3 | 4.272 | -2.670 | 6.856 | 1.00 | 13.25 | S |
| ATOM | 3155 | OH2 | TIP | S | 4 | 12.420 | 25.843 | 0.038 | 1.00 | 23.54 | S |
| ATOM | 3156 | OH2 | TIP | S | 5 | 28.864 | 12.791 | 5.442 | 1.00 | 15.66 | S |
| ATOM | 3157 | OH2 | TIP | S | 6 | 22.858 | 3.268 | 1.071 | 1.00 | 31.62 | S |
| ATOM | 3158 | OH2 | TIP | S | 7 | 1.874 | 8.457 | 11.241 | 1.00 | 21.72 | S |
| ATOM | 3159 | OH2 | TIP | S | 8 | 7.191 | 15.867 | 18.341 | 1.00 | 35.87 | S |
| ATOM | 3160 | OH2 | TIP | S | 9 | 18.732 | -3.268 | 23.830 | 1.00 | 20.48 | S |
| ATOM | 3161 | OH2 | TIP | S | 10 | 7.461 | 0.296 | 3.026 | 1.00 | 11.03 | S |
| ATOM | 3162 | OH2 | TIP | S | 11 | 8.643 | -10.064 | 15.482 | 1.00 | 14.64 | S |
| ATOM | 3163 | OH2 | TIP | S | 12 | 10.866 | 4.587 | -3.443 | 1.00 | 16.24 | S |
| ATOM | 3164 | OH2 | TIP | S | 13 | 22.771 | 20.738 | 1.586 | 1.00 | 20.81 | S |
| ATOM | 3165 | OH2 | TIP | S | 14 | 18.622 | 4.400 | 5.323 | 1.00 | 26.22 | S |
| ATOM | 3166 | OH2 | TIP | S | 15 | 10.917 | -21.623 | 8.064 | 1.00 | 21.60 | S |
| ATOM | 3167 | OH2 | TIP | S | 16 | 21.613 | 5.603 | 15.562 | 1.00 | 13.93 | S |
| ATOM | 3168 | OH2 | TIP | S | 17 | -1.029 | -27.165 | -1.819 | 1.00 | 15.32 | S |
| ATOM | 3169 | OH2 | TIP | S | 18 | 5.118 | -5.793 | 22.087 | 1.00 | 25.04 | S |
| ATOM | 3170 | OH2 | TIP | S | 19 | 25.376 | 3.221 | 13.682 | 1.00 | 14.07 | S |
| ATOM | 3171 | OH2 | TIP | S | 20 | 3.805 | -1.836 | 13.482 | 1.00 | 19.99 | S |
| ATOM | 3172 | OH2 | TIP | S | 21 | 20.253 | -2.408 | 5.459 | 1.00 | 15.94 | S |
| ATOM | 3173 | OH2 | TIP | S | 22 | 14.604 | 5.064 | 22.714 | 1.00 | 14.00 | S |
| ATOM | 3174 | OH2 | TIP | S | 23 | 11.354 | -11.074 | -0.041 | 1.00 | 26.72 | S |
| ATOM | 3175 | OH2 | TIP | S | 24 | 23.016 | -6.196 | 21.209 | 1.00 | 30.36 | S |
| ATOM | 3176 | OH2 | TIP | S | 25 | -2.080 | 24.781 | -0.586 | 1.00 | 18.27 | S |
| ATOM | 3177 | OH2 | TIP | S | 26 | 20.494 | 23.736 | 24.868 | 1.00 | 21.50 | S |
| ATOM | 3178 | OH2 | TIP | S | 27 | 26.171 | 5.582 | 12.822 | 1.00 | 18.41 | S |
| ATOM | 3179 | OH2 | TIP | S | 28 | -1.039 | 2.455 | 18.019 | 1.00 | 15.04 | S |
| ATOM | 3180 | OH2 | TIP | S | 29 | 4.159 | -27.049 | 13.922 | 1.00 | 18.02 | S |
| ATOM | 3181 | OH2 | TIP | S | 30 | 2.589 | -6.230 | 21.223 | 1.00 | 24.37 | S |
| ATOM | 3182 | OH2 | TIP | S | 31 | 19.298 | -8.055 | 18.442 | 1.00 | 12.26 | S |
| ATOM | 3183 | OH2 | TIP | S | 32 | 11.043 | 23.881 | 14.298 | 1.00 | 25.06 | S |
| ATOM | 3184 | OH2 | TIP | S | 33 | 11.414 | 0.558 | -0.203 | 1.00 | 20.51 | S |
| ATOM | 3185 | OH2 | TIP | S | 34 | 14.072 | -0.652 | 1.396 | 1.00 | 15.43 | S |
| ATOM | 3186 | OH2 | TIP | S | 35 | 25.284 | 6.171 | 10.227 | 1.00 | 13.60 | S |
| ATOM | 3187 | OH2 | TIP | S | 36 | 14.408 | 24.661 | 1.472 | 1.00 | 17.73 | S |
| ATOM | 3188 | OH2 | TIP | S | 37 | 26.762 | 0.834 | 14.054 | 1.00 | 23.69 | S |
| ATOM | 3189 | OH2 | TIP | S | 38 | 8.937 | -0.156 | 18.953 | 1.00 | 22.63 | S |
| ATOM | 3190 | OH2 | TIP | S | 39 | 9.920 | 19.346 | 9.173 | 1.00 | 29.73 | S |
| ATOM | 3191 | OH2 | TIP | S | 40 | 18.066 | -5.520 | 22.455 | 1.00 | 18.74 | S |
| ATOM | 3192 | OH2 | TIP | S | 41 | 24.929 | -17.502 | 20.543 | 1.00 | 20.36 | S |
| ATOM | 3193 | OH2 | TIP | S | 42 | 9.163 | -18.961 | 24.390 | 1.00 | 18.38 | S |
| ATOM | 3194 | OH2 | TIP | S | 43 | 1.317 | 26.494 | 8.454 | 1.00 | 20.92 | S |
| ATOM | 3195 | OH2 | TIP | S | 44 | 25.665 | 16.490 | 4.529 | 1.00 | 25.58 | S |
| ATOM | 3196 | OH2 | TIP | S | 45 | 9.128 | 25.658 | 20.726 | 1.00 | 27.97 | S |
| ATOM | 3197 | OH2 | TIP | S | 46 | 5.847 | -23.374 | 22.064 | 1.00 | 20.44 | S |
| ATOM | 3198 | OH2 | TIP | S | 47 | 2.187 | -28.236 | 12.031 | 1.00 | 27.13 | S |
| ATOM | 3199 | OH2 | TIP | S | 48 | 32.494 | 8.779 | 14.289 | 1.00 | 25.03 | S |
| ATOM | 3200 | OH2 | TIP | S | 49 | -0.327 | 25.034 | 1.705 | 1.00 | 20.32 | S |
| ATOM | 3201 | OH2 | TIP | S | 50 | 11.712 | 30.849 | 12.608 | 1.00 | 22.35 | S |
| ATOM | 3202 | OH2 | TIP | S | 51 | 24.631 | 15.094 | 20.905 | 1.00 | 18.69 | S |
| ATOM | 3203 | OH2 | TIP | S | 52 | 13.022 | 1.609 | 22.328 | 1.00 | 21.57 | S |
| ATOM | 3204 | OH2 | TIP | S | 53 | 19.391 | -18.172 | 3.471 | 1.00 | 22.56 | S |
| ATOM | 3205 | OH2 | TIP | S | 54 | -8.866 | 30.116 | -2.556 | 1.00 | 30.99 | S |
| ATOM | 3206 | OH2 | TIP | S | 55 | 14.171 | -33.509 | 19.786 | 1.00 | 24.43 | S |
| ATOM | 3207 | OH2 | TIP | S | 56 | 1.350 | 4.367 | 6.588 | 1.00 | 27.07 | S |
| ATOM | 3208 | OH2 | TIP | S | 57 | 7.087 | 19.390 | 9.020 | 1.00 | 18.33 | S |

Figure 1 (suite 43)

| | | | | | | | | | | | |
|---|---|---|---|---|---|---|---|---|---|---|---|
| ATOM | 3209 | OH2 | TIP | S | 58 | 0.845 | -10.171 | 0.965 | 1.00 | 25.25 | S |
| ATOM | 3210 | OH2 | TIP | S | 59 | 8.695 | -6.762 | 17.316 | 1.00 | 20.32 | S |
| ATOM | 3211 | OH2 | TIP | S | 60 | 16.871 | 7.618 | 5.645 | 1.00 | 13.51 | S |
| ATOM | 3212 | OH2 | TIP | S | 61 | 14.866 | -8.608 | 22.818 | 1.00 | 20.81 | S |
| ATOM | 3213 | OH2 | TIP | S | 62 | 4.516 | -30.892 | -1.995 | 1.00 | 22.77 | S |
| ATOM | 3214 | OH2 | TIP | S | 63 | 1.205 | 28.404 | 6.721 | 1.00 | 24.95 | S |
| ATOM | 3215 | OH2 | TIP | S | 64 | 17.097 | 5.323 | 7.036 | 1.00 | 24.18 | S |
| ATOM | 3216 | OH2 | TIP | S | 65 | 26.673 | 13.742 | 4.087 | 1.00 | 16.42 | S |
| ATOM | 3217 | OH2 | TIP | S | 66 | 26.603 | -3.932 | -6.686 | 1.00 | 30.81 | S |
| ATOM | 3218 | OH2 | TIP | S | 67 | 17.436 | -2.479 | 21.436 | 1.00 | 26.07 | S |
| ATOM | 3219 | OH2 | TIP | S | 68 | 28.480 | 20.411 | 2.439 | 1.00 | 24.90 | S |
| ATOM | 3220 | OH2 | TIP | S | 69 | 8.335 | 24.695 | 12.358 | 1.00 | 26.27 | S |
| ATOM | 3221 | OH2 | TIP | S | 70 | 28.275 | -26.123 | 15.282 | 1.00 | 29.19 | S |
| ATOM | 3222 | OH2 | TIP | S | 71 | 22.574 | 18.488 | 4.655 | 1.00 | 13.27 | S |
| ATOM | 3223 | OH2 | TIP | S | 72 | 21.930 | -27.504 | 4.683 | 1.00 | 28.86 | S |
| ATOM | 3224 | OH2 | TIP | S | 73 | -4.118 | 28.924 | 5.610 | 1.00 | 25.34 | S |
| ATOM | 3225 | OH2 | TIP | S | 74 | 24.336 | -4.198 | 8.779 | 1.00 | 21.22 | S |
| ATOM | 3226 | OH2 | TIP | S | 75 | 6.177 | 8.687 | -5.007 | 1.00 | 29.11 | S |
| ATOM | 3227 | OH2 | TIP | S | 76 | 6.031 | 16.693 | 8.352 | 1.00 | 22.68 | S |
| ATOM | 3228 | OH2 | TIP | S | 77 | 26.916 | 4.316 | 3.896 | 1.00 | 32.50 | S |
| ATOM | 3229 | OH2 | TIP | S | 78 | 20.363 | 21.499 | -0.916 | 1.00 | 23.82 | S |
| ATOM | 3230 | OH2 | TIP | S | 79 | -3.593 | -9.219 | 19.619 | 1.00 | 20.91 | S |
| ATOM | 3231 | OH2 | TIP | S | 80 | 4.886 | 14.470 | 20.139 | 1.00 | 25.80 | S |
| ATOM | 3232 | OH2 | TIP | S | 81 | 13.859 | -27.223 | 5.463 | 1.00 | 25.43 | S |
| ATOM | 3233 | OH2 | TIP | S | 82 | 1.623 | -27.370 | -0.642 | 1.00 | 23.15 | S |
| ATOM | 3234 | OH2 | TIP | S | 83 | 28.891 | 16.295 | 8.760 | 1.00 | 25.49 | S |
| ATOM | 3235 | OH2 | TIP | S | 84 | 10.003 | -7.798 | 15.501 | 1.00 | 23.22 | S |
| ATOM | 3236 | OH2 | TIP | S | 85 | 8.660 | -11.011 | 1.096 | 1.00 | 34.34 | S |
| ATOM | 3237 | OH2 | TIP | S | 86 | 28.769 | -6.662 | 17.101 | 1.00 | 23.36 | S |
| ATOM | 3238 | OH2 | TIP | S | 87 | 9.074 | -6.855 | 25.772 | 1.00 | 31.54 | S |
| ATOM | 3239 | OH2 | TIP | S | 88 | 3.513 | -4.225 | 19.211 | 1.00 | 19.82 | S |
| ATOM | 3240 | OH2 | TIP | S | 89 | 27.473 | -9.536 | 4.211 | 1.00 | 24.57 | S |
| ATOM | 3241 | OH2 | TIP | S | 90 | 8.259 | 0.497 | 0.302 | 1.00 | 23.56 | S |
| ATOM | 3242 | OH2 | TIP | S | 91 | 3.257 | 6.561 | -1.998 | 1.00 | 33.20 | S |
| ATOM | 3243 | OH2 | TIP | S | 92 | 20.123 | -6.138 | 20.069 | 1.00 | 19.90 | S |
| ATOM | 3244 | OH2 | TIP | S | 93 | 20.293 | -4.110 | 16.436 | 1.00 | 19.16 | S |
| ATOM | 3245 | OH2 | TIP | S | 94 | 4.723 | -11.907 | -3.941 | 1.00 | 18.08 | S |
| ATOM | 3246 | OH2 | TIP | S | 95 | 20.613 | 11.685 | 25.598 | 1.00 | 24.53 | S |
| ATOM | 3247 | OH2 | TIP | S | 96 | -5.095 | 29.910 | -0.921 | 1.00 | 27.39 | S |
| ATOM | 3248 | OH2 | TIP | S | 97 | -0.969 | 28.392 | 4.819 | 1.00 | 32.34 | S |
| ATOM | 3249 | OH2 | TIP | S | 98 | 8.158 | 15.171 | 8.168 | 1.00 | 28.13 | S |
| ATOM | 3250 | OH2 | TIP | S | 99 | 10.128 | -21.789 | 5.329 | 1.00 | 20.14 | S |
| ATOM | 3251 | OH2 | TIP | S | 100 | 19.859 | -11.052 | 29.405 | 1.00 | 27.24 | S |
| ATOM | 3252 | OH2 | TIP | S | 101 | -1.676 | -32.376 | -4.914 | 1.00 | 26.41 | S |
| ATOM | 3253 | OH2 | TIP | S | 102 | 7.974 | -30.737 | 24.269 | 1.00 | 51.26 | S |
| ATOM | 3254 | OH2 | TIP | S | 103 | 5.606 | -29.218 | 15.121 | 1.00 | 26.23 | S |
| ATOM | 3255 | OH2 | TIP | S | 104 | 24.243 | 0.129 | 13.054 | 1.00 | 25.36 | S |
| ATOM | 3256 | OH2 | TIP | S | 105 | 14.303 | -33.270 | 9.329 | 1.00 | 38.58 | S |
| ATOM | 3257 | OH2 | TIP | S | 106 | -2.028 | 28.921 | -1.297 | 1.00 | 33.82 | S |
| ATOM | 3258 | OH2 | TIP | S | 107 | -4.154 | -32.776 | -8.185 | 1.00 | 34.53 | S |
| ATOM | 3259 | OH2 | TIP | S | 108 | -2.324 | 12.468 | 11.993 | 1.00 | 36.85 | S |
| ATOM | 3260 | OH2 | TIP | S | 109 | 1.179 | 7.777 | 0.709 | 1.00 | 17.87 | S |
| ATOM | 3261 | OH2 | TIP | S | 110 | 19.482 | 1.891 | -0.089 | 1.00 | 19.59 | S |
| ATOM | 3262 | OH2 | TIP | S | 111 | -0.238 | 27.853 | 2.490 | 1.00 | 24.88 | S |
| ATOM | 3263 | OH2 | TIP | S | 112 | 17.403 | -8.054 | 23.423 | 1.00 | 22.40 | S |
| ATOM | 3264 | OH2 | TIP | S | 113 | 25.733 | 3.505 | 6.385 | 1.00 | 29.87 | S |
| ATOM | 3265 | OH2 | TIP | S | 114 | 13.976 | -18.813 | 26.940 | 1.00 | 31.16 | S |
| ATOM | 3266 | OH2 | TIP | S | 115 | 8.833 | -20.890 | 21.246 | 1.00 | 14.93 | S |
| ATOM | 3267 | OH2 | TIP | S | 116 | 24.501 | 22.906 | 1.057 | 1.00 | 35.96 | S |
| ATOM | 3268 | OH2 | TIP | S | 117 | 21.414 | 3.779 | -1.147 | 1.00 | 30.87 | S |
| ATOM | 3269 | OH2 | TIP | S | 118 | 28.998 | -13.976 | 15.483 | 1.00 | 23.57 | S |
| ATOM | 3270 | OH2 | TIP | S | 119 | 18.731 | 6.848 | -7.099 | 1.00 | 25.17 | S |
| ATOM | 3271 | OH2 | TIP | S | 120 | 20.010 | 1.728 | 16.800 | 1.00 | 22.88 | S |
| ATOM | 3272 | OH2 | TIP | S | 121 | 6.894 | -30.742 | -0.620 | 1.00 | 29.47 | S |
| ATOM | 3273 | OH2 | TIP | S | 122 | 20.174 | -17.026 | 1.268 | 1.00 | 30.49 | S |
| ATOM | 3274 | OH2 | TIP | S | 123 | 8.643 | -28.845 | -0.416 | 1.00 | 31.35 | S |
| ATOM | 3275 | OH2 | TIP | S | 124 | 4.944 | -31.072 | -4.553 | 1.00 | 32.74 | S |
| ATOM | 3276 | OH2 | TIP | S | 125 | -10.961 | 28.830 | 4.996 | 1.00 | 33.03 | S |
| ATOM | 3277 | OH2 | TIP | S | 126 | 30.092 | 8.788 | 5.004 | 1.00 | 33.78 | S |
| ATOM | 3278 | OH2 | TIP | S | 127 | 17.445 | -4.803 | -4.414 | 1.00 | 20.44 | S |
| ATOM | 3279 | OH2 | TIP | S | 128 | 23.112 | -4.584 | 17.484 | 1.00 | 30.23 | S |
| ATOM | 3280 | OH2 | TIP | S | 129 | -6.744 | 21.445 | 11.698 | 1.00 | 31.58 | S |
| ATOM | 3281 | OH2 | TIP | S | 130 | 8.973 | -16.266 | 25.362 | 1.00 | 20.07 | S |

*Figure 1 (suite 44)*

| | | | | | | | | | | | |
|---|---|---|---|---|---|---|---|---|---|---|---|
| ATOM | 3282 | OH2 | TIP | S | 131 | 12.772 | 13.622 | -6.319 | 1.00 | 34.15 | S |
| ATOM | 3283 | OH2 | TIP | S | 132 | -3.116 | 9.249 | 5.359 | 1.00 | 29.44 | S |
| ATOM | 3284 | OH2 | TIP | S | 133 | 26.881 | -19.295 | 2.513 | 1.00 | 33.28 | S |
| ATOM | 3285 | OH2 | TIP | S | 134 | 7.429 | -9.285 | -0.530 | 1.00 | 26.08 | S |
| ATOM | 3286 | OH2 | TIP | S | 135 | 22.324 | 30.847 | 10.082 | 1.00 | 28.18 | S |
| ATOM | 3287 | OH2 | TIP | S | 136 | 1.898 | -2.552 | 8.170 | 1.00 | 25.83 | S |
| ATOM | 3288 | OH2 | TIP | S | 137 | 4.952 | 29.654 | 3.610 | 1.00 | 19.80 | S |
| ATOM | 3289 | OH2 | TIP | S | 138 | 4.730 | -10.048 | -1.783 | 1.00 | 32.11 | S |
| ATOM | 3290 | OH2 | TIP | S | 139 | 22.848 | 3.688 | 6.970 | 1.00 | 38.11 | S |
| ATOM | 3291 | OH2 | TIP | S | 140 | 14.761 | -14.049 | 28.549 | 1.00 | 34.69 | S |
| ATOM | 3292 | OH2 | TIP | S | 141 | 25.134 | 3.816 | 18.696 | 1.00 | 24.08 | S |
| ATOM | 3293 | OH2 | TIP | S | 142 | 16.166 | -26.138 | 8.270 | 1.00 | 24.18 | S |
| ATOM | 3294 | OH2 | TIP | S | 143 | 8.520 | 22.387 | 13.511 | 1.00 | 32.86 | S |
| ATOM | 3295 | OH2 | TIP | S | 144 | 15.947 | -33.236 | 7.410 | 1.00 | 30.70 | S |
| ATOM | 3296 | OH2 | TIP | S | 145 | 1.001 | 3.721 | 11.463 | 1.00 | 30.99 | S |
| ATOM | 3297 | OH2 | TIP | S | 146 | 19.402 | -30.453 | 22.808 | 1.00 | 30.06 | S |
| ATOM | 3298 | OH2 | TIP | S | 147 | 2.545 | -8.887 | -0.354 | 1.00 | 29.15 | S |
| ATOM | 3299 | OH2 | TIP | S | 148 | 6.125 | -2.484 | 3.591 | 1.00 | 36.81 | S |
| ATOM | 3300 | OH2 | TIP | S | 149 | 30.037 | 15.301 | 6.097 | 1.00 | 34.72 | S |
| ATOM | 3301 | OH2 | TIP | S | 150 | 2.692 | 14.176 | 15.786 | 1.00 | 37.96 | S |
| ATOM | 3302 | OH2 | TIP | S | 151 | 14.515 | -32.146 | 13.729 | 1.00 | 32.82 | S |
| ATOM | 3303 | OH2 | TIP | S | 152 | 9.610 | -36.868 | 11.279 | 1.00 | 41.95 | S |
| ATOM | 3304 | OH2 | TIP | S | 153 | 5.665 | -34.453 | 2.968 | 1.00 | 38.59 | S |
| ATOM | 3305 | OH2 | TIP | S | 154 | 23.178 | -21.702 | 21.829 | 1.00 | 30.09 | S |
| ATOM | 3306 | OH2 | TIP | S | 155 | -1.201 | -8.663 | 3.473 | 1.00 | 33.00 | S |
| ATOM | 3307 | OH2 | TIP | S | 156 | -3.830 | -11.232 | 6.982 | 1.00 | 30.35 | S |
| ATOM | 3308 | OH2 | TIP | S | 157 | 24.938 | -7.342 | 10.309 | 1.00 | 23.44 | S |
| ATOM | 3309 | OH2 | TIP | S | 158 | 16.368 | 29.902 | 11.388 | 1.00 | 27.04 | S |
| ATOM | 3310 | OH2 | TIP | S | 159 | -1.341 | -6.872 | 11.300 | 1.00 | 14.83 | S |
| ATOM | 3311 | OH2 | TIP | S | 160 | 30.760 | 11.420 | 9.093 | 1.00 | 32.59 | S |
| ATOM | 3312 | OH2 | TIP | S | 161 | 23.476 | 3.734 | 15.955 | 1.00 | 23.82 | S |
| ATOM | 3313 | OH2 | TIP | S | 162 | 26.214 | 27.933 | 14.693 | 1.00 | 30.10 | S |
| ATOM | 3314 | OH2 | TIP | S | 163 | 22.151 | 4.306 | 25.213 | 1.00 | 29.14 | S |
| ATOM | 3315 | OH2 | TIP | S | 164 | 11.738 | -35.020 | 8.005 | 1.00 | 38.12 | S |
| ATOM | 3316 | OH2 | TIP | S | 165 | 4.778 | -18.873 | -12.768 | 1.00 | 35.23 | S |
| ATOM | 3317 | OH2 | TIP | S | 166 | 30.502 | 22.934 | 8.137 | 1.00 | 42.60 | S |
| ATOM | 3318 | OH2 | TIP | S | 167 | 2.607 | 14.207 | 8.337 | 1.00 | 29.81 | S |
| ATOM | 3319 | OH2 | TIP | S | 168 | 9.085 | 2.348 | 25.949 | 1.00 | 25.77 | S |
| ATOM | 3320 | OH2 | TIP | S | 169 | 21.392 | 5.439 | 8.382 | 1.00 | 36.39 | S |
| ATOM | 3321 | OH2 | TIP | S | 170 | 1.780 | -2.781 | 11.266 | 1.00 | 34.39 | S |
| ATOM | 3322 | OH2 | TIP | S | 171 | 19.734 | 10.347 | 27.642 | 1.00 | 35.25 | S |
| ATOM | 3323 | OH2 | TIP | S | 172 | 29.690 | -12.928 | 8.733 | 1.00 | 50.23 | S |
| ATOM | 3324 | OH2 | TIP | S | 173 | 27.727 | -11.269 | 7.668 | 1.00 | 46.10 | S |
| ATOM | 3325 | OH2 | TIP | S | 174 | 5.410 | 17.042 | 15.669 | 1.00 | 42.21 | S |
| ATOM | 3326 | OH2 | TIP | S | 175 | 6.076 | 31.520 | 7.133 | 1.00 | 38.34 | S |
| ATOM | 3327 | OH2 | TIP | S | 176 | 9.093 | 7.280 | 26.421 | 1.00 | 28.81 | S |
| ATOM | 3328 | OH2 | TIP | S | 177 | 13.074 | 11.869 | 26.280 | 1.00 | 47.11 | S |
| ATOM | 3329 | OH2 | TIP | S | 178 | 15.685 | 11.427 | 29.581 | 1.00 | 47.20 | S |
| ATOM | 3330 | OH2 | TIP | S | 179 | 10.850 | -11.175 | 28.516 | 1.00 | 37.36 | S |
| ATOM | 3331 | OH2 | TIP | S | 180 | 19.187 | -20.970 | 4.242 | 1.00 | 38.61 | S |
| ATOM | 3332 | OH2 | TIP | S | 181 | 4.028 | -1.322 | -4.810 | 1.00 | 46.22 | S |
| ATOM | 3333 | OH2 | TIP | S | 182 | 7.873 | 18.402 | 17.920 | 1.00 | 40.67 | S |
| ATOM | 3334 | OH2 | TIP | S | 183 | 7.076 | 14.293 | 15.717 | 1.00 | 48.95 | S |
| ATOM | 3335 | OH2 | TIP | S | 184 | 10.024 | 30.496 | 14.711 | 1.00 | 41.11 | S |
| ATOM | 3336 | OH2 | TIP | S | 185 | 24.994 | 30.881 | 11.708 | 1.00 | 40.24 | S |
| ATOM | 3337 | OH2 | TIP | S | 186 | 31.169 | 24.245 | 17.272 | 1.00 | 40.69 | S |
| ATOM | 3338 | OH2 | TIP | S | 187 | -6.751 | 19.480 | 18.331 | 1.00 | 50.67 | S |
| ATOM | 3339 | OH2 | TIP | S | 188 | 3.855 | 10.646 | 6.741 | 1.00 | 19.27 | S |
| ATOM | 3340 | OH2 | TIP | S | 189 | -2.085 | 1.514 | 26.909 | 1.00 | 27.84 | S |
| ATOM | 3341 | OH2 | TIP | S | 190 | 26.440 | -3.683 | 6.999 | 1.00 | 44.01 | S |
| ATOM | 3342 | OH2 | TIP | S | 191 | 30.217 | 1.717 | 9.822 | 1.00 | 46.01 | S |
| ATOM | 3343 | OH2 | TIP | S | 192 | 7.493 | -1.348 | 6.867 | 1.00 | 41.64 | S |
| ATOM | 3344 | OH2 | TIP | S | 193 | -0.399 | -1.896 | 5.465 | 1.00 | 39.06 | S |
| ATOM | 3345 | OH2 | TIP | S | 194 | -5.402 | -2.523 | 9.679 | 1.00 | 39.06 | S |
| ATOM | 3346 | OH2 | TIP | S | 195 | 13.225 | -12.534 | 30.097 | 1.00 | 42.02 | S |
| ATOM | 3347 | OH2 | TIP | S | 196 | 9.342 | -13.524 | 29.233 | 1.00 | 38.74 | S |
| ATOM | 3348 | OH2 | TIP | S | 197 | 10.600 | -8.628 | 27.877 | 1.00 | 36.17 | S |
| ATOM | 3349 | OH2 | TIP | S | 198 | 21.448 | -2.867 | 23.943 | 1.00 | 35.12 | S |
| ATOM | 3350 | OH2 | TIP | S | 199 | 16.165 | -17.404 | 27.188 | 1.00 | 34.24 | S |
| ATOM | 3351 | OH2 | TIP | S | 200 | 25.006 | -21.444 | 19.546 | 1.00 | 43.74 | S |
| ATOM | 3352 | OH2 | TIP | S | 201 | 32.504 | -28.332 | 15.263 | 1.00 | 44.50 | S |
| ATOM | 3353 | OH2 | TIP | S | 202 | 13.047 | -39.920 | 14.059 | 1.00 | 54.11 | S |
| ATOM | 3354 | OH2 | TIP | S | 203 | 15.295 | -24.831 | 5.913 | 1.00 | 40.66 | S |

Figure 1 (suite 45)

| | | | | | | | | | | | |
|---|---|---|---|---|---|---|---|---|---|---|---|
| ATOM | 3355 | OH2 | TIP | S | 204 | 15.812 | -25.237 | -6.506 | 1.00 | 42.95 | |
| ATOM | 3356 | OH2 | TIP | S | 205 | -1.883 | -31.135 | -1.926 | 1.00 | 36.12 | |
| ATOM | 3357 | OH2 | TIP | S | 206 | 2.475 | -30.159 | -0.547 | 1.00 | 35.27 | S |
| ATOM | 3358 | OH2 | TIP | S | 207 | 0.700 | -19.322 | 15.415 | 1.00 | 38.57 | S |
| ATOM | 3359 | OH2 | TIP | S | 208 | 11.149 | -6.262 | 21.333 | 1.00 | 41.17 | S |
| ATOM | 3360 | OH2 | TIP | S | 209 | 27.288 | -5.096 | 4.238 | 1.00 | 49.23 | S |
| ATOM | 3361 | OH2 | TIP | S | 210 | 21.317 | 2.039 | 19.764 | 1.00 | 29.40 | S |
| ATOM | 3362 | OH2 | TIP | S | 211 | 0.870 | 6.839 | 7.567 | 1.00 | 28.05 | S |
| ATOM | 3363 | OH2 | TIP | S | 212 | 18.158 | 2.168 | 29.537 | 1.00 | 41.88 | S |
| ATOM | 3364 | OH2 | TIP | S | 213 | 9.134 | -17.237 | -0.572 | 1.00 | 44.90 | S |
| ATOM | 3365 | OH2 | TIP | S | 214 | 24.984 | 14.920 | 23.592 | 1.00 | 28.30 | S |
| ATOM | 3366 | OH2 | TIP | S | 215 | 0.439 | 15.182 | 8.978 | 1.00 | 34.52 | S |
| ATOM | 3367 | OH2 | TIP | S | 216 | 10.936 | -5.905 | 24.207 | 1.00 | 32.05 | S |
| ATOM | 3368 | OH2 | TIP | S | 217 | 29.846 | 14.236 | 11.030 | 1.00 | 32.46 | S |
| ATOM | 3369 | OH2 | TIP | S | 218 | 15.060 | -4.303 | 27.945 | 1.00 | 43.42 | S |
| ATOM | 3370 | OH2 | TIP | S | 219 | 6.890 | -6.862 | 0.135 | 1.00 | 35.09 | S |
| ATOM | 3371 | OH2 | TIP | S | 220 | 21.864 | 4.566 | 22.761 | 1.00 | 36.83 | S |
| ATOM | 3372 | OH2 | TIP | S | 221 | 16.080 | 26.689 | 2.684 | 1.00 | 24.35 | S |
| ATOM | 3373 | OH2 | TIP | S | 222 | 16.332 | -16.635 | 3.959 | 1.00 | 44.15 | S |
| ATOM | 3374 | OH2 | TIP | S | 223 | 15.404 | 6.864 | 24.627 | 1.00 | 30.61 | S |
| ATOM | 3375 | OH2 | TIP | S | 224 | 4.632 | 0.144 | 5.552 | 1.00 | 36.13 | S |
| ATOM | 3376 | OH2 | TIP | S | 225 | 16.546 | -19.618 | 1.379 | 1.00 | 47.74 | S |
| ATOM | 3377 | OH2 | TIP | S | 226 | 4.785 | 12.188 | 15.137 | 1.00 | 28.17 | S |
| ATOM | 3378 | OH2 | TIP | S | 227 | 9.549 | -17.218 | 6.257 | 1.00 | 33.26 | S |
| ATOM | 3379 | OH2 | TIP | S | 228 | 9.186 | 20.256 | 11.685 | 1.00 | 37.08 | S |
| ATOM | 3380 | OH2 | TIP | S | 229 | 11.861 | 10.311 | -8.402 | 1.00 | 46.18 | S |
| ATOM | 3381 | OH2 | TIP | S | 230 | 22.849 | 13.004 | 26.008 | 1.00 | 38.51 | S |
| ATOM | 3382 | OH2 | TIP | S | 231 | 4.625 | -32.096 | 4.121 | 1.00 | 30.03 | S |
| ATOM | 3383 | OH2 | TIP | S | 232 | 3.497 | -6.239 | 1.305 | 1.00 | 41.17 | S |
| ATOM | 3384 | OH2 | TIP | S | 233 | 25.731 | -5.527 | 20.339 | 1.00 | 40.18 | S |
| ATOM | 3385 | OH2 | TIP | S | 234 | 5.524 | 14.143 | 13.640 | 1.00 | 38.12 | S |
| ATOM | 3386 | OH2 | TIP | S | 235 | 2.367 | -31.914 | -5.657 | 1.00 | 30.23 | S |
| ATOM | 3387 | OH2 | TIP | S | 236 | -3.695 | -16.219 | 13.330 | 1.00 | 41.87 | S |
| ATOM | 3388 | OH2 | TIP | S | 237 | 20.124 | -3.453 | 19.734 | 1.00 | 48.27 | S |
| ATOM | 3389 | OH2 | TIP | S | 238 | 27.207 | 0.628 | 16.671 | 1.00 | 41.79 | S |
| ATOM | 3390 | OH2 | TIP | S | 239 | 10.044 | -22.899 | 22.586 | 1.00 | 35.79 | S |
| ATOM | 3391 | OH2 | TIP | S | 240 | 24.265 | 29.243 | 15.909 | 1.00 | 44.93 | S |
| ATOM | 3392 | OH2 | TIP | S | 241 | -0.047 | 1.376 | 28.625 | 1.00 | 46.26 | S |
| ATOM | 3393 | OH2 | TIP | S | 242 | 2.565 | 27.065 | 10.842 | 1.00 | 37.25 | S |
| ATOM | 3394 | OH2 | TIP | S | 243 | 28.138 | -17.240 | 19.703 | 1.00 | 34.45 | S |
| ATOM | 3395 | OH2 | TIP | S | 244 | 17.257 | 0.355 | 17.279 | 1.00 | 30.95 | S |
| ATOM | 3396 | OH2 | TIP | S | 245 | 28.493 | -4.973 | 8.163 | 1.00 | 44.55 | S |
| ATOM | 3397 | OH2 | TIP | S | 246 | 18.184 | -2.026 | 18.307 | 1.00 | 35.36 | S |
| ATOM | 3398 | OH2 | TIP | S | 247 | 15.664 | -34.252 | 17.819 | 1.00 | 47.99 | S |
| ATOM | 3399 | OH2 | TIP | S | 248 | 22.255 | 28.389 | 1.686 | 1.00 | 44.91 | S |
| ATOM | 3400 | OH2 | TIP | S | 249 | 26.409 | -15.500 | 4.304 | 1.00 | 40.70 | S |
| ATOM | 3401 | OH2 | TIP | S | 250 | 21.762 | 24.045 | -0.060 | 1.00 | 36.10 | S |
| ATOM | 3402 | OH2 | TIP | S | 251 | 13.321 | -24.365 | -12.367 | 1.00 | 50.71 | S |
| ATOM | 3403 | OH2 | TIP | S | 252 | 2.408 | 1.759 | 26.931 | 1.00 | 39.54 | S |
| ATOM | 3404 | OH2 | TIP | S | 253 | 30.522 | 11.389 | 3.923 | 1.00 | 46.45 | S |
| ATOM | 3405 | OH2 | TIP | S | 254 | 21.571 | 26.334 | 21.320 | 1.00 | 39.41 | S |
| ATOM | 3406 | OH2 | TIP | S | 255 | 28.934 | -4.129 | 18.585 | 1.00 | 32.38 | S |
| ATOM | 3407 | OH2 | TIP | S | 256 | 14.498 | -20.875 | -6.569 | 1.00 | 42.38 | S |
| ATOM | 3408 | OH2 | TIP | S | 257 | 27.194 | 1.669 | 18.988 | 1.00 | 39.47 | S |
| ATOM | 3409 | OH2 | TIP | S | 258 | 5.689 | -30.318 | 22.217 | 1.00 | 43.77 | S |
| ATOM | 3410 | OH2 | TIP | S | 259 | 29.393 | -3.138 | 2.993 | 1.00 | 55.63 | S |
| ATOM | 3411 | OH2 | TIP | S | 260 | 12.064 | -7.755 | 19.433 | 1.00 | 36.19 | S |
| ATOM | 3412 | OH2 | TIP | S | 261 | 11.468 | -29.351 | 0.182 | 1.00 | 42.15 | S |
| ATOM | 3413 | OH2 | TIP | S | 262 | 2.793 | -24.109 | 20.019 | 1.00 | 27.19 | S |
| ATOM | 3414 | OH2 | TIP | S | 263 | 15.146 | -16.894 | 30.023 | 1.00 | 39.72 | S |
| ATOM | 3415 | OH2 | TIP | S | 264 | -1.627 | 30.209 | 1.601 | 1.00 | 40.65 | S |
| ATOM | 3416 | OH2 | TIP | S | 265 | 21.828 | -3.117 | 28.431 | 1.00 | 49.90 | S |
| ATOM | 3417 | OH2 | TIP | S | 266 | 23.833 | 2.666 | -2.401 | 1.00 | 40.48 | S |
| ATOM | 3418 | OH2 | TIP | S | 267 | 24.119 | 18.986 | 20.778 | 1.00 | 34.31 | S |
| ATOM | 3419 | OH2 | TIP | S | 268 | 23.888 | 20.891 | 4.776 | 1.00 | 34.05 | S |
| ATOM | 3420 | OH2 | TIP | S | 269 | -5.985 | -13.437 | 13.992 | 1.00 | 41.21 | S |
| ATOM | 3421 | OH2 | TIP | S | 270 | 7.539 | 17.753 | 25.495 | 1.00 | 35.61 | S |
| ATOM | 3422 | OH2 | TIP | S | 271 | 4.929 | -32.984 | -12.055 | 1.00 | 46.60 | S |
| ATOM | 3423 | OH2 | TIP | S | 272 | 19.421 | 16.348 | 26.586 | 1.00 | 44.90 | S |
| ATOM | 3424 | OH2 | TIP | S | 273 | -5.126 | 29.424 | 3.157 | 1.00 | 42.56 | S |
| ATOM | 3425 | OH2 | TIP | S | 274 | 15.188 | 35.041 | 0.002 | 1.00 | 38.03 | S |
| ATOM | 3426 | OH2 | TIP | S | 275 | 2.958 | 8.574 | 8.206 | 1.00 | 35.94 | S |
| ATOM | 3427 | OH2 | TIP | S | 276 | 13.387 | 8.738 | 25.418 | 1.00 | 46.28 | S |

Figure 1 (suite 46)

```
ATOM   3428  OH2 TIP S 277       2.595   1.361   6.779  1.00 45.47      S
ATOM   3429  OH2 TIP S 278      -2.089 -16.600  15.553  1.00 35.70      S
ATOM   3430  OH2 TIP S 279      18.258  28.154  19.965  1.00 41.52      S
ATOM   3431  OH2 TIP S 280     -11.660  16.480   7.205  1.00 36.10      S
ATOM   3432  OH2 TIP S 281       3.766 -30.040  16.830  1.00 43.33      S
ATOM   3433  OH2 TIP S 282      11.128  -3.111  25.163  1.00 38.91      S
ATOM   3434  OH2 TIP S 283       9.307 -33.179  20.725  1.00 39.55      S
ATOM   3435  OH2 TIP S 284      13.282 -22.861   6.607  1.00 36.05      S
ATOM   3436  OH2 TIP S 285      -0.826   7.845   5.105  1.00 33.41      S
ATOM   3437  OH2 TIP S 286      12.902  35.421  16.812  1.00 48.67      S
ATOM   3438  OH2 TIP S 287       3.775  32.150   4.458  1.00 47.99      S
ATOM   3439  OH2 TIP S 288      26.897  -3.990  -2.326  1.00 39.71      S
ATOM   3440  OH2 TIP S 289      31.544   0.903  17.057  1.00 44.92      S
ATOM   3441  OH2 TIP S 290      31.752  11.192  11.679  1.00 44.01      S
ATOM   3442  OH2 TIP S 291      29.313  -7.603   4.202  1.00 45.29      S
ATOM   3443  OH2 TIP S 292      -1.997  20.614  19.103  1.00 37.97      S
ATOM   3444  OH2 TIP S 293       3.951  29.380  11.031  1.00 44.47      S
ATOM   3445  OH2 TIP S 294       7.420 -13.015   2.578  1.00 24.94      S
ATOM   3446  OH2 TIP S 295       1.172  -1.918   3.233  1.00 47.41      S
ATOM   3447  OH2 TIP S 296      15.445 -14.913   1.778  1.00 48.50      S
ATOM   3448  OH2 TIP S 297      11.829   1.453  -7.636  1.00 46.65      S
ATOM   3449  OH2 TIP S 298       4.839  14.662   9.465  1.00 43.52      S
ATOM   3450  OH2 TIP S 299      20.801  20.622  34.631  1.00 47.72      S
ATOM   3451  OH2 TIP S 300       3.579  -3.390   3.521  1.00 45.77      S
ATOM   3452  OH2 TIP S 301      14.974  14.541  -5.141  1.00 37.03      S
ATOM   3453  OH2 TIP S 302      23.536  -4.099  23.236  1.00 34.08      S
ATOM   3454  OH2 TIP S 303      14.927   2.709 -10.320  1.00 41.32      S
ATOM   3455  OH2 TIP S 304      20.026 -27.159  -0.520  1.00 45.54      S
ATOM   3456  OH2 TIP S 305      32.279   9.746   7.528  1.00 48.79      S
ATOM   3457  OH2 TIP S 306      20.024  12.429  29.467  1.00 52.22      S
ATOM   3458  OH2 TIP S 307     -11.011  30.690   8.145  1.00 40.47      S
ATOM   3459  OH2 TIP S 308      -7.184  -1.953  13.670  1.00 43.28      S
ATOM   3460  OH2 TIP S 309      17.087  27.813   0.558  1.00 33.08      S
ATOM   3461  OH2 TIP S 310       4.584  -0.055  27.309  1.00 40.10      S
ATOM   3462  OH2 TIP S 311       9.381 -19.064   4.380  1.00 38.66      S
ATOM   3463  OH2 TIP S 312      21.214  -1.441  26.029  1.00 51.98      S
ATOM   3464  OH2 TIP S 313      25.982  19.100  18.523  1.00 34.38      S
ATOM   3465  OH2 TIP S 314      12.061 -28.229   2.756  1.00 47.15      S
ATOM   3466  OH2 TIP S 315      29.798   1.634  19.587  1.00 44.38      S
ATOM   3467  OH2 TIP S 316      26.769   1.072   5.979  1.00 33.94      S
ATOM   3468  OH2 TIP S 317      25.073  10.661  -7.667  1.00 47.47      S
ATOM   3469  OH2 TIP S 318       0.652  -0.626  10.097  1.00 51.78      S
ATOM   3470  OH2 TIP S 319      10.308  -1.480  -1.490  1.00 45.26      S
ATOM   3471  OH2 TIP S 320      -3.204  -9.728   4.730  1.00 47.98      S
ATOM   3472  OH2 TIP S 321      -0.412  -6.260  25.918  1.00 48.99      S
ATOM   3473  OH2 TIP S 322      25.131  27.774   4.328  1.00 45.49      S
ATOM   3474  OH2 TIP S 323     -12.821  17.616   4.983  1.00 50.61      S
ATOM   3475  OH2 TIP S 324     -12.793  20.162  12.345  1.00 41.52      S
ATOM   3476  OH2 TIP S 325      24.342  26.941   1.698  1.00 41.17      S
ATOM   3477  OH2 TIP S 326      -7.365  -5.025  17.431  1.00 39.48      S
ATOM   3478  OH2 TIP S 327       2.892 -27.982  17.989  1.00 41.13      S
ATOM   3479  OH2 TIP S 328       9.872   6.526 -11.214  1.00 42.90      S
ATOM   3480  OH2 TIP S 329      -6.200  12.028   9.697  1.00 50.57      S
ATOM   3481  OH2 TIP S 330      23.866 -15.421  26.329  1.00 40.42      S
ATOM   3482  OH2 TIP S 331       1.096 -30.176 -13.571  1.00 43.43      S
ATOM   3483  OH2 TIP S 332       8.434 -15.636   1.992  1.00 40.17      S
ATOM   3484  OH2 TIP S 333      32.949   3.069   9.650  1.00 41.86      S
ATOM   3485  OH2 TIP S 334       7.922 -33.872   4.448  1.00 47.95      S
ATOM   3486  OH2 TIP S 335      17.865  30.540  17.212  1.00 45.88      S
ATOM   3487  OH2 TIP S 336      32.372  -4.686  12.364  1.00 47.64      S
ATOM   3488  OH2 TIP S 337      -7.753  32.425  -4.214  1.00 45.41      S
ATOM   3489  OH2 TIP S 338       8.880  -5.207  -0.854  1.00 48.73      S
ATOM   3490  OH2 TIP S 339       4.667  11.210  26.924  1.00 40.85      S
ATOM   3491  OH2 TIP S 340      22.102  28.486  19.256  1.00 44.58      S
ATOM   3492  OH2 TIP S 341      12.923  34.748   7.433  1.00 46.74      S
END
```

Figure 1 (suite 47)

```
REMARK coordinates from restrained individual B-factor refinement
REMARK refinement resolution: 500.0 - 2.0 A
REMARK starting r= 0.2074 free_r= 0.2640
REMARK final    r= 0.2065 free_r= 0.2645
REMARK B rmsd for bonded mainchain atoms=  1.376  target= 1.5
REMARK B rmsd for bonded sidechain atoms=  2.030  target= 2.0
REMARK B rmsd for angle mainchain atoms=  2.342  target= 2.0
REMARK B rmsd for angle sidechain atoms=  3.164  target= 2.5
REMARK rweight=  0.1000 (with wa= 3.8334)
REMARK target= mlf  steps= 30
REMARK sg= C2 a= 82.23 b= 118.61 c= 53.17 alpha= 90 beta= 122.74 gamma= 90
REMARK parameter file 1  : CNS_TOPPAR:protein_rep.param
REMARK parameter file 2  : CNS_TOPPAR:water.param
REMARK molecular structure file: generate_easy.mtf
REMARK input coordinates: generate_easy.pdb
REMARK reflection file= 39a1.cv
REMARK ncs= none
REMARK B-correction resolution: 6.0 - 2.0
REMARK initial B-factor correction applied to fobs :
REMARK   B11=   3.144 B22=   1.061 B33=  -4.206
REMARK   B12=   0.000 B13=   0.003 B23=   0.000
REMARK B-factor correction applied to coordinate array B:    0.994
REMARK bulk solvent: density level= 0.316088 e/A^3, B-factor= 51.7425 A^2
REMARK reflections with |Fobs|/sigma_F < 0.0 rejected
REMARK reflections with |Fobs| > 10000 * rms(Fobs) rejected
REMARK theoretical total number of refl. in resol. range:     28906 ( 100.0 % )
REMARK number of unobserved reflections (no entry or |F|=0):  15942 (  55.2 % )
REMARK number of reflections rejected:                            0 (   0.0 % )
REMARK total number of reflections used:                      12964 (  44.8 % )
REMARK number of reflections in working set:                  11763 (  40.7 % )
REMARK number of reflections in test set:                      1201 (   4.2 % )
CRYST1   82.230  118.610   53.170  90.00 122.74  90.00 C 2
REMARK FILENAME="bindividual.pdb"
REMARK DATE:30-Jul-2001  14:15:27       created by user: martin
REMARK VERSION:1.0
ATOM      1  CB  ALA A   9      30.422  -9.176  11.235  1.00 41.11      A
ATOM      2  C   ALA A   9      28.942  -7.624  12.534  1.00 39.86      A
ATOM      3  O   ALA A   9      28.288  -7.326  11.531  1.00 40.73      A
ATOM      4  N   ALA A   9      28.897 -10.074  12.965  1.00 40.08      A
ATOM      5  CA  ALA A   9      29.758  -8.915  12.589  1.00 40.38      A
ATOM      6  N   LYS A  10      28.980  -6.864  13.622  1.00 38.53      A
ATOM      7  CA  LYS A  10      28.254  -5.605  13.697  1.00 35.83      A
ATOM      8  CB  LYS A  10      27.841  -5.331  15.145  1.00 36.55      A
ATOM      9  CG  LYS A  10      27.543  -6.615  15.926  1.00 38.44      A
ATOM     10  CD  LYS A  10      26.591  -6.405  17.093  1.00 39.25      A
ATOM     11  CE  LYS A  10      27.131  -5.430  18.117  1.00 42.03      A
ATOM     12  NZ  LYS A  10      26.167  -5.232  19.244  1.00 44.49      A
ATOM     13  C   LYS A  10      29.167  -4.501  13.171  1.00 33.26      A
ATOM     14  O   LYS A  10      30.343  -4.427  13.527  1.00 33.45      A
ATOM     15  N   PRO A  11      28.640  -3.637  12.298  1.00 30.71      A
ATOM     16  CD  PRO A  11      27.233  -3.579  11.870  1.00 30.30      A
ATOM     17  CA  PRO A  11      29.402  -2.534  11.712  1.00 29.27      A
ATOM     18  CB  PRO A  11      28.433  -1.964  10.690  1.00 29.81      A
ATOM     19  CG  PRO A  11      27.118  -2.170  11.355  1.00 29.71      A
ATOM     20  C   PRO A  11      29.835  -1.500  12.749  1.00 27.64      A
ATOM     21  O   PRO A  11      29.119  -1.227  13.711  1.00 27.72      A
ATOM     22  N   PRO A  12      31.016  -0.901  12.554  1.00 26.23      A
ATOM     23  CD  PRO A  12      31.966  -1.151  11.453  1.00 25.43      A
ATOM     24  CA  PRO A  12      31.541   0.100  13.483  1.00 25.88      A
ATOM     25  CB  PRO A  12      33.008   0.189  13.083  1.00 26.21      A
ATOM     26  CG  PRO A  12      32.945  -0.014  11.599  1.00 26.51      A
ATOM     27  C   PRO A  12      30.839   1.458  13.455  1.00 25.75      A
ATOM     28  O   PRO A  12      30.800   2.143  12.429  1.00 26.55      A
ATOM     29  N   PHE A  13      30.299   1.833  14.609  1.00 23.89      A
ATOM     30  CA  PHE A  13      29.590   3.092  14.806  1.00 21.57      A
ATOM     31  CB  PHE A  13      29.286   3.257  16.292  1.00 20.43      A
ATOM     32  CG  PHE A  13      28.509   4.490  16.623  1.00 21.08      A
ATOM     33  CD1 PHE A  13      27.118   4.467  16.644  1.00 21.88      A
ATOM     34  CD2 PHE A  13      29.162   5.677  16.924  1.00 21.43      A
ATOM     35  CE1 PHE A  13      26.381   5.614  16.964  1.00 22.20      A
ATOM     36  CE2 PHE A  13      28.441   6.824  17.241  1.00 22.78      A
ATOM     37  CZ  PHE A  13      27.041   6.792  17.262  1.00 22.17      A
ATOM     38  C   PHE A  13      30.347   4.327  14.315  1.00 21.79      A
```

Figure 2

| | | | | | | | | | | | |
|---|---|---|---|---|---|---|---|---|---|---|---|
| ATOM | 39 | O | PHE | A | 13 | 31.578 | 4.398 | 14.384 | 1.00 | 21.26 | A |
| ATOM | 40 | N | VAL | A | 14 | 29.591 | 5.302 | 13.821 | 1.00 | 21.12 | A |
| ATOM | 41 | CA | VAL | A | 14 | 30.149 | 6.564 | 13.342 | 1.00 | 20.43 | A |
| ATOM | 42 | CB | VAL | A | 14 | 30.206 | 6.628 | 11.792 | 1.00 | 22.40 | A |
| ATOM | 43 | CG1 | VAL | A | 14 | 30.972 | 5.427 | 11.249 | 1.00 | 21.40 | A |
| ATOM | 44 | CG2 | VAL | A | 14 | 28.802 | 6.661 | 11.213 | 1.00 | 23.48 | A |
| ATOM | 45 | C | VAL | A | 14 | 29.244 | 7.683 | 13.857 | 1.00 | 20.02 | A |
| ATOM | 46 | O | VAL | A | 14 | 28.030 | 7.679 | 13.621 | 1.00 | 21.40 | A |
| ATOM | 47 | N | SER | A | 15 | 29.829 | 8.622 | 14.590 | 1.00 | 17.33 | A |
| ATOM | 48 | CA | SER | A | 15 | 29.063 | 9.727 | 15.136 | 1.00 | 15.61 | A |
| ATOM | 49 | CB | SER | A | 15 | 29.928 | 10.543 | 16.090 | 1.00 | 15.00 | A |
| ATOM | 50 | OG | SER | A | 15 | 29.122 | 11.388 | 16.889 | 1.00 | 17.16 | A |
| ATOM | 51 | C | SER | A | 15 | 28.614 | 10.585 | 13.968 | 1.00 | 13.87 | A |
| ATOM | 52 | O | SER | A | 15 | 29.435 | 10.986 | 13.151 | 1.00 | 15.94 | A |
| ATOM | 53 | N | ARG | A | 16 | 27.318 | 10.861 | 13.881 | 1.00 | 12.84 | A |
| ATOM | 54 | CA | ARG | A | 16 | 26.789 | 11.657 | 12.767 | 1.00 | 12.21 | A |
| ATOM | 55 | CB | ARG | A | 16 | 25.659 | 10.898 | 12.065 | 1.00 | 11.08 | A |
| ATOM | 56 | CG | ARG | A | 16 | 26.030 | 9.556 | 11.481 | 1.00 | 9.87 | A |
| ATOM | 57 | CD | ARG | A | 16 | 26.816 | 9.694 | 10.194 | 1.00 | 9.78 | A |
| ATOM | 58 | NE | ARG | A | 16 | 27.113 | 8.383 | 9.630 | 1.00 | 9.03 | A |
| ATOM | 59 | CZ | ARG | A | 16 | 27.900 | 8.186 | 8.582 | 1.00 | 9.68 | A |
| ATOM | 60 | NH1 | ARG | A | 16 | 28.469 | 9.218 | 7.981 | 1.00 | 8.04 | A |
| ATOM | 61 | NH2 | ARG | A | 16 | 28.121 | 6.955 | 8:139 | 1.00 | 10.81 | A |
| ATOM | 62 | C | ARG | A | 16 | 26.233 | 13.018 | 13.164 | 1.00 | 11.68 | A |
| ATOM | 63 | O | ARG | A | 16 | 25.737 | 13.198 | 14.278 | 1.00 | 11.20 | A |
| ATOM | 64 | N | SER | A | 17 | 26.322 | 13.974 | 12.246 | 1.00 | 10.35 | A |
| ATOM | 65 | CA | SER | A | 17 | 25.738 | 15.296 | 12.473 | 1:00 | 10.79 | A |
| ATOM | 66 | CB | SER | A | 17 | 26.328 | 16.302 | 11.484 | 1.00 | 11.64 | A |
| ATOM | 67 | OG | SER | A | 17 | 25.704 | 17.571 | 11.622 | 1.00 | 16.03 | A |
| ATOM | 68 | C | SER | A | 17 | 24.214 | 15.110 | 12.226 | 1.00 | 9.64 | A |
| ATOM | 69 | O | SER | A | 17 | 23.777 | 14.832 | 11.106 | 1.00 | 7.76 | A |
| ATOM | 70 | N | VAL | A | 18 | 23.421 | 15.259 | 13.279 | 1.00 | 8.54 | A |
| ATOM | 71 | CA | VAL | A | 18 | 21.982 | 15.048 | 13.208 | 1.00 | 9.00 | A |
| ATOM | 72 | CB | VAL | A | 18 | 21.563 | 13.945 | 14.192 | 1.00 | 8.69 | A |
| ATOM | 73 | CG1 | VAL | A | 18 | 20.025 | 13.791 | 14.202 | 1.00 | 9.96 | A |
| ATOM | 74 | CG2 | VAL | A | 18 | 22.246 | 12.643 | 13.821 | 1.00 | 6.80 | A |
| ATOM | 75 | C | VAL | A | 18 | 21.141 | 16.271 | 13.536 | 1.00 | 12.16 | A |
| ATOM | 76 | O | VAL | A | 18 | 21.435 | 17.011 | 14.476 | 1.00 | 14.34 | A |
| ATOM | 77 | N | LEU | A | 19 | 20.071 | 16.467 | 12.773 | 1.00 | 13.28 | A |
| ATOM | 78 | CA | LEU | A | 19 | 19.181 | 17.592 | 13.012 | 1.00 | 14.60 | A |
| ATOM | 79 | CB | LEU | A | 19 | 19.204 | 18.563 | 11.833 | 1.00 | 14.72 | A |
| ATOM | 80 | CG | LEU | A | 19 | 18.246 | 19.754 | 11.930 | 1.00 | 12.88 | A |
| ATOM | 81 | CD1 | LEU | A | 19 | 18.597 | 20.595 | 13.137 | 1.00 | 10.65 | A |
| ATOM | 82 | CD2 | LEU | A | 19 | 18.338 | 20.577 | 10.653 | 1.00 | 14.48 | A |
| ATOM | 83 | C | LEU | A | 19 | 17.769 | 17.078 | 13.203 | 1.00 | 16.61 | A |
| ATOM | 84 | O | LEU | A | 19 | 17.240 | 16.378 | 12.341 | 1.00 | 20.56 | A |
| ATOM | 85 | N | VAL | A | 20 | 17.153 | 17.433 | 14.322 | 1.00 | 16.18 | A |
| ATOM | 86 | CA | VAL | A | 20 | 15.798 | 16.999 | 14.610 | 1.00 | 15.84 | A |
| ATOM | 87 | CB | VAL | A | 20 | 15.756 | 16.164 | 15.902 | 1.00 | 15.27 | A |
| ATOM | 88 | CG1 | VAL | A | 20 | 14.332 | 15.656 | 16.152 | 1.00 | 12.33 | A |
| ATOM | 89 | CG2 | VAL | A | 20 | 16.749 | 15.011 | 15.801 | 1.00 | 13.35 | A |
| ATOM | 90 | C | VAL | A | 20 | 14.844 | 18.190 | 14.755 | 1.00 | 17.29 | A |
| ATOM | 91 | O | VAL | A | 20 | 14.819 | 18.857 | 15.794 | 1.00 | 19.31 | A |
| ATOM | 92 | N | THR | A | 21 | 14.057 | 18.443 | 13.712 | 1.00 | 16.49 | A |
| ATOM | 93 | CA | THR | A | 21 | 13.101 | 19.538 | 13.722 | 1.00 | 16.36 | A |
| ATOM | 94 | CB | THR | A | 21 | 12.392 | 19.699 | 12.352 | 1.00 | 14.57 | A |
| ATOM | 95 | OG1 | THR | A | 21 | 11.524 | 18.585 | 12.128 | 1.00 | 14.64 | A |
| ATOM | 96 | CG2 | THR | A | 21 | 13.404 | 19.775 | 11.226 | 1.00 | 10.98 | A |
| ATOM | 97 | C | THR | A | 21 | 12.050 | 19.254 | 14.782 | 1.00 | 17.72 | A |
| ATOM | 98 | O | THR | A | 21 | 11.659 | 18.110 | 14.979 | 1.00 | 18.56 | A |
| ATOM | 99 | N | GLY | A | 22 | 11.603 | 20.300 | 15.467 | 1.00 | 19.24 | A |
| ATOM | 100 | CA | GLY | A | 22 | 10.600 | 20.130 | 16.501 | 1.00 | 20.64 | A |
| ATOM | 101 | C | GLY | A | 22 | 11.003 | 19.105 | 17.536 | 1.00 | 22.52 | A |
| ATOM | 102 | O | GLY | A | 22 | 10.166 | 18.351 | 18.022 | 1.00 | 22.82 | A |
| ATOM | 103 | N | GLY | A | 23 | 12.286 | 19.083 | 17.883 | 1.00 | 24.34 | A |
| ATOM | 104 | CA | GLY | A | 23 | 12.765 | 18.128 | 18.864 | 1.00 | 27.47 | A |
| ATOM | 105 | C | GLY | A | 23 | 13.006 | 18.691 | 20.252 | 1.00 | 29.20 | A |
| ATOM | 106 | O | GLY | A | 23 | 13.963 | 18.306 | 20.923 | 1.00 | 29.04 | A |
| ATOM | 107 | N | ASN | A | 24 | 12.138 | 19.597 | 20.690 | 1.00 | 31.59 | A |
| ATOM | 108 | CA | ASN | A | 24 | 12.263 | 20.207 | 22.011 | 1.00 | 33.62 | A |
| ATOM | 109 | CB | ASN | A | 24 | 12.220 | 21.728 | 21.895 | 1.00 | 35.66 | A |
| ATOM | 110 | CG | ASN | A | 24 | 10.959 | 22.218 | 21.206 | 1.00 | 37.64 | A |
| ATOM | 111 | OD1 | ASN | A | 24 | 9.852 | 22.026 | 21.706 | 1.00 | 39.46 | A |

Figure 2 (suite 1)

| | | | | | | | | | | | |
|---|---|---|---|---|---|---|---|---|---|---|---|
| ATOM | 112 | ND2 | ASN | A | 24 | 11.122 | 22.844 | 20.045 | 1.00 | 39.31 | A |
| ATOM | 113 | C | ASN | A | 24 | 11.141 | 19.745 | 22.932 | 1.00 | 34.20 | A |
| ATOM | 114 | O | ASN | A | 24 | 11.142 | 20.065 | 24.120 | 1.00 | 34.62 | A |
| ATOM | 115 | N | ARG | A | 25 | 10.181 | 19.009 | 22.376 | 1.00 | 34.65 | A |
| ATOM | 116 | CA | ARG | A | 25 | 9.053 | 18.496 | 23.154 | 1.00 | 36.55 | A |
| ATOM | 117 | CB | ARG | A | 25 | 7.976 | 19.586 | 23.297 | 1.00 | 41.80 | A |
| ATOM | 118 | CG | ARG | A | 25 | 7.447 | 20.088 | 21.954 | 1.00 | 50.52 | A |
| ATOM | 119 | CD | ARG | A | 25 | 6.399 | 21.191 | 22.100 | 1.00 | 57.48 | A |
| ATOM | 120 | NE | ARG | A | 25 | 5.890 | 21.675 | 20.807 | 1.00 | 64.33 | A |
| ATOM | 121 | CZ | ARG | A | 25 | 4.968 | 22.636 | 20.680 | 1.00 | 67.46 | A |
| ATOM | 122 | NH1 | ARG | A | 25 | 4.440 | 23.235 | 21.755 | 1.00 | 69.17 | A |
| ATOM | 123 | NH2 | ARG | A | 25 | 4.503 | 23.076 | 19.500 | 1.00 | 68.63 | A |
| ATOM | 124 | C | ARG | A | 25 | 8.410 | 17.261 | 22.504 | 1.00 | 33.84 | A |
| ATOM | 125 | O | ARG | A | 25 | 8.669 | 16.953 | 21.341 | 1.00 | 32.14 | A |
| ATOM | 126 | N | GLY | A | 26 | 7.592 | 16.549 | 23.275 | 1.00 | 32.23 | A |
| ATOM | 127 | CA | GLY | A | 26 | 6.905 | 15.370 | 22.767 | 1.00 | 31.25 | A |
| ATOM | 128 | C | GLY | A | 26 | 7.778 | 14.242 | 22.238 | 1.00 | 30.73 | A |
| ATOM | 129 | O | GLY | A | 26 | 8.733 | 13.815 | 22.897 | 1.00 | 32.11 | A |
| ATOM | 130 | N | ILE | A | 27 | 7.443 | 13.748 | 21.047 | 1.00 | 27.69 | A |
| ATOM | 131 | CA | ILE | A | 27 | 8.192 | 12.662 | 20.428 | 1.00 | 24.40 | A |
| ATOM | 132 | CB | ILE | A | 27 | 7.392 | 12.033 | 19.262 | 1.00 | 23.88 | A |
| ATOM | 133 | CG2 | ILE | A | 27 | 8.315 | 11.267 | 18.322 | 1.00 | 23.97 | A |
| ATOM | 134 | CG1 | ILE | A | 27 | 6.305 | 11.126 | 19.830 | 1.00 | 24.22 | A |
| ATOM | 135 | CD | ILE | A | 27 | 5.480 | 10.402 | 18.767 | 1.00 | 25.78 | A |
| ATOM | 136 | C | ILE | A | 27 | 9.550 | 13.122 | 19.912 | 1.00 | 22.64 | A |
| ATOM | 137 | O | ILE | A | 27 | 10.498 | 12.337 | 19.868 | 1.00 | 23.45 | A |
| ATOM | 138 | N | GLY | A | 28 | 9.639 | 14.389 | 19.518 | 1.00 | 20.14 | A |
| ATOM | 139 | CA | GLY | A | 28 | 10.890 | 14.919 | 19.005 | 1.00 | 18.36 | A |
| ATOM | 140 | C | GLY | A | 28 | 11.965 | 14.915 | 20.075 | 1.00 | 19.52 | A |
| ATOM | 141 | O | GLY | A | 28 | 13.125 | 14.585 | 19.808 | 1.00 | 18.13 | A |
| ATOM | 142 | N | LEU | A | 29 | 11.580 | 15.270 | 21.299 | 1.00 | 18.76 | A |
| ATOM | 143 | CA | LEU | A | 29 | 12.537 | 15.299 | 22.391 | 1.00 | 18.46 | A |
| ATOM | 144 | CB | LEU | A | 29 | 11.874 | 15.734 | 23.700 | 1.00 | 20.11 | A |
| ATOM | 145 | CG | LEU | A | 29 | 12.880 | 15.920 | 24.845 | 1.00 | 19.58 | A |
| ATOM | 146 | CD1 | LEU | A | 29 | 13.828 | 17.056 | 24.486 | 1.00 | 19.30 | A |
| ATOM | 147 | CD2 | LEU | A | 29 | 12.160 | 16.212 | 26.152 | 1.00 | 19.75 | A |
| ATOM | 148 | C | LEU | A | 29 | 13.137 | 13.918 | 22.562 | 1.00 | 17.11 | A |
| ATOM | 149 | O | LEU | A | 29 | 14.357 | 13.764 | 22.527 | 1.00 | 18.05 | A |
| ATOM | 150 | N | ALA | A | 30 | 12.276 | 12.919 | 22.737 | 1.00 | 15.43 | A |
| ATOM | 151 | CA | ALA | A | 30 | 12.720 | 11.539 | 22.912 | 1.00 | 14.80 | A |
| ATOM | 152 | CB | ALA | A | 30 | 11.515 | 10.605 | 23.019 | 1.00 | 13.25 | A |
| ATOM | 153 | C | ALA | A | 30 | 13.628 | 11.094 | 21.769 | 1.00 | 15.35 | A |
| ATOM | 154 | O | ALA | A | 30 | 14.658 | 10.460 | 21.998 | 1.00 | 16.89 | A |
| ATOM | 155 | N | ILE | A | 31 | 13.257 | 11.417 | 20.535 | 1.00 | 13.96 | A |
| ATOM | 156 | CA | ILE | A | 31 | 14.097 | 11.029 | 19.415 | 1.00 | 12.97 | A |
| ATOM | 157 | CB | ILE | A | 31 | 13.492 | 11.463 | 18.051 | 1.00 | 11.97 | A |
| ATOM | 158 | CG2 | ILE | A | 31 | 14.508 | 11.234 | 16.934 | 1.00 | 9.66 | A |
| ATOM | 159 | CG1 | ILE | A | 31 | 12.213 | 10.664 | 17.762 | 1.00 | 12.97 | A |
| ATOM | 160 | CD | ILE | A | 31 | 11.539 | 10.994 | 16.403 | 1.00 | 11.85 | A |
| ATOM | 161 | C | ILE | A | 31 | 15.465 | 11.683 | 19.580 | 1.00 | 13.32 | A |
| ATOM | 162 | O | ILE | A | 31 | 16.497 | 11.011 | 19.521 | 1.00 | 14.01 | A |
| ATOM | 163 | N | ALA | A | 32 | 15.454 | 12.999 | 19.784 | 1.00 | 14.26 | A |
| ATOM | 164 | CA | ALA | A | 32 | 16.669 | 13.792 | 19.952 | 1.00 | 16.52 | A |
| ATOM | 165 | CB | ALA | A | 32 | 16.299 | 15.261 | 20.174 | 1.00 | 13.84 | A |
| ATOM | 166 | C | ALA | A | 32 | 17.551 | 13.281 | 21.107 | 1.00 | 18.89 | A |
| ATOM | 167 | O | ALA | A | 32 | 18.765 | 13.096 | 20.943 | 1.00 | 18.83 | A |
| ATOM | 168 | N | GLN | A | 33 | 16.951 | 13.057 | 22.271 | 1.00 | 17.50 | A |
| ATOM | 169 | CA | GLN | A | 33 | 17.712 | 12.550 | 23.396 | 1.00 | 19.47 | A |
| ATOM | 170 | CB | GLN | A | 33 | 16.840 | 12.477 | 24.642 | 1.00 | 22.43 | A |
| ATOM | 171 | CG | GLN | A | 33 | 16.445 | 13.835 | 25.143 | 1.00 | 28.78 | A |
| ATOM | 172 | CD | GLN | A | 33 | 15.724 | 13.767 | 26.459 | 1.00 | 33.41 | A |
| ATOM | 173 | OE1 | GLN | A | 33 | 14.742 | 13.028 | 26.602 | 1.00 | 36.79 | A |
| ATOM | 174 | NE2 | GLN | A | 33 | 16.198 | 14.540 | 27.438 | 1.00 | 35.24 | A |
| ATOM | 175 | C | GLN | A | 33 | 18.289 | 11.172 | 23.097 | 1.00 | 17.80 | A |
| ATOM | 176 | O | GLN | A | 33 | 19.472 | 10.919 | 23.352 | 1.00 | 17.24 | A |
| ATOM | 177 | N | ARG | A | 34 | 17.461 | 10.287 | 22.550 | 1.00 | 16.06 | A |
| ATOM | 178 | CA | ARG | A | 34 | 17.919 | 8.942 | 22.234 | 1.00 | 13.76 | A |
| ATOM | 179 | CB | ARG | A | 34 | 16.769 | 8.078 | 21.684 | 1.00 | 13.03 | A |
| ATOM | 180 | CG | ARG | A | 34 | 17.200 | 6.735 | 21.044 | 1.00 | 10.92 | A |
| ATOM | 181 | CD | ARG | A | 34 | 18.050 | 5.864 | 21.982 | 1.00 | 12.05 | A |
| ATOM | 182 | NE | ARG | A | 34 | 18.603 | 4.713 | 21.269 | 1.00 | 10.97 | A |
| ATOM | 183 | CZ | ARG | A | 34 | 18.062 | 3.495 | 21.272 | 1.00 | 8.66 | A |
| ATOM | 184 | NH1 | ARG | A | 34 | 16.961 | 3.246 | 21.968 | 1.00 | 5.88 | A |

Figure 2 (suite 2)

| | | | | | | | | | | | |
|---|---|---|---|---|---|---|---|---|---|---|---|
| ATOM | 185 | NH2 | ARG | A | 34 | 18.594 | 2.542 | 20.530 | 1.00 | 4.18 | A |
| ATOM | 186 | C | ARG | A | 34 | 19.069 | 8.986 | 21.242 | 1.00 | 13.25 | A |
| ATOM | 187 | O | ARG | A | 34 | 20.043 | 8.243 | 21.397 | 1.00 | 12.88 | A |
| ATOM | 188 | N | LEU | A | 35 | 18.987 | 9.849 | 20.234 | 1.00 | 12.30 | A |
| ATOM | 189 | CA | LEU | A | 35 | 20.080 | 9.897 | 19.265 | 1.00 | 12.14 | A |
| ATOM | 190 | CB | LEU | A | 35 | 19.698 | 10.715 | 18.025 | 1.00 | 12.88 | A |
| ATOM | 191 | CG | LEU | A | 35 | 18.696 | 10.058 | 17.065 | 1.00 | 16.08 | A |
| ATOM | 192 | CD1 | LEU | A | 35 | 18.592 | 10.869 | 15.770 | 1.00 | 15.56 | A |
| ATOM | 193 | CD2 | LEU | A | 35 | 19.169 | 8.634 | 16.737 | 1.00 | 18.24 | A |
| ATOM | 194 | C | LEU | A | 35 | 21.348 | 10.458 | 19.907 | 1.00 | 11.90 | A |
| ATOM | 195 | O | LEU | A | 35 | 22.461 | 10.218 | 19.413 | 1.00 | 11.88 | A |
| ATOM | 196 | N | ALA | A | 36 | 21.172 | 11.195 | 21.009 | 1.00 | 10.57 | A |
| ATOM | 197 | CA | ALA | A | 36 | 22.293 | 11.779 | 21.745 | 1.00 | 9.70 | A |
| ATOM | 198 | CB | ALA | A | 36 | 21.797 | 12.862 | 22.732 | 1.00 | 6.77 | A |
| ATOM | 199 | C | ALA | A | 36 | 22.962 | 10.626 | 22.492 | 1.00 | 8.75 | A |
| ATOM | 200 | O | ALA | A | 36 | 24.170 | 10.423 | 22.386 | 1.00 | 7.78 | A |
| ATOM | 201 | N | ALA | A | 37 | 22.163 | 9.859 | 23.226 | 1.00 | 8.83 | A |
| ATOM | 202 | CA | ALA | A | 37 | 22.669 | 8.696 | 23.951 | 1.00 | 10.16 | A |
| ATOM | 203 | CB | ALA | A | 37 | 21.532 | 7.946 | 24.598 | 1.00 | 9.94 | A |
| ATOM | 204 | C | ALA | A | 37 | 23.448 | 7.746 | 23.035 | 1.00 | 10.94 | A |
| ATOM | 205 | O | ALA | A | 37 | 24.439 | 7.171 | 23.462 | 1.00 | 15.00 | A |
| ATOM | 206 | N | ASP | A | 38 | 23.016 | 7.574 | 21.787 | 1.00 | 10.32 | A |
| ATOM | 207 | CA | ASP | A | 38 | 23.728 | 6.685 | 20.871 | 1.00 | 9.49 | A |
| ATOM | 208 | CB | ASP | A | 38 | 23.005 | 6.514 | 19.523 | 1.00 | 12.16 | A |
| ATOM | 209 | CG | ASP | A | 38 | 21.656 | 5.802 | 19.624 | 1.00 | 12.71 | A |
| ATOM | 210 | OD1 | ASP | A | 38 | 21.420 | 5.000 | 20.558 | 1.00 | 13.07 | A |
| ATOM | 211 | OD2 | ASP | A | 38 | 20.832 | 6.042 | 18.714 | 1.00 | 13.70 | A |
| ATOM | 212 | C | ASP | A | 38 | 25.096 | 7.246 | 20.542 | 1.00 | 10.87 | A |
| ATOM | 213 | O | ASP | A | 38 | 25.880 | 6.586 | 19.868 | 1.00 | 13.39 | A |
| ATOM | 214 | N | GLY | A | 39 | 25.377 | 8.478 | 20.962 | 1.00 | 10.83 | A |
| ATOM | 215 | CA | GLY | A | 39 | 26.674 | 9.063 | 20.651 | 1.00 | 10.80 | A |
| ATOM | 216 | C | GLY | A | 39 | 26.772 | 9.907 | 19.381 | 1.00 | 12.39 | A |
| ATOM | 217 | O | GLY | A | 39 | 27.870 | 10.112 | 18.855 | 1.00 | 11.52 | A |
| ATOM | 218 | N | HIS | A | 40 | 25.631 | 10.398 | 18.889 | 1.00 | 13.36 | A |
| ATOM | 219 | CA | HIS | A | 40 | 25.572 | 11.251 | 17.687 | 1.00 | 11.69 | A |
| ATOM | 220 | CB | HIS | A | 40 | 24.250 | 11.051 | 16.921 | 1.00 | 10.41 | A |
| ATOM | 221 | CG | HIS | A | 40 | 24.151 | 9.770 | 16.153 | 1.00 | 10.64 | A |
| ATOM | 222 | CD2 | HIS | A | 40 | 23.319 | 8.709 | 16.294 | 1.00 | 8.70 | A |
| ATOM | 223 | ND1 | HIS | A | 40 | 24.947 | 9.492 | 15.063 | 1.00 | 10.35 | A |
| ATOM | 224 | CE1 | HIS | A | 40 | 24.608 | 8.316 | 14.564 | 1.00 | 11.71 | A |
| ATOM | 225 | NE2 | HIS | A | 40 | 23.622 | 7.820 | 15.293 | 1.00 | 9.13 | A |
| ATOM | 226 | C | HIS | A | 40 | 25.595 | 12.724 | 18.109 | 1.00 | 11.15 | A |
| ATOM | 227 | O | HIS | A | 40 | 25.107 | 13.065 | 19.186 | 1.00 | 9.74 | A |
| ATOM | 228 | N | LYS | A | 41 | 26.136 | 13.588 | 17.252 | 1.00 | 11.35 | A |
| ATOM | 229 | CA | LYS | A | 41 | 26.146 | 15.037 | 17.497 | 1.00 | 11.83 | A |
| ATOM | 230 | CB | LYS | A | 41 | 27.176 | 15.707 | 16.590 | 1.00 | 13.00 | A |
| ATOM | 231 | CG | LYS | A | 41 | 28.605 | 15.285 | 16.907 | 1.00 | 16.98 | A |
| ATOM | 232 | CD | LYS | A | 41 | 29.603 | 15.666 | 15.827 | 1.00 | 18.28 | A |
| ATOM | 233 | CE | LYS | A | 41 | 29.393 | 14.848 | 14.567 | 1.00 | 21.61 | A |
| ATOM | 234 | NZ | LYS | A | 41 | 30.431 | 15.137 | 13.529 | 1.00 | 22.84 | A |
| ATOM | 235 | C | LYS | A | 41 | 24.723 | 15.501 | 17.134 | 1.00 | 12.43 | A |
| ATOM | 236 | O | LYS | A | 41 | 24.342 | 15.494 | 15.964 | 1.00 | 13.92 | A |
| ATOM | 237 | N | VAL | A | 42 | 23.944 | 15.917 | 18.126 | 1.00 | 11.66 | A |
| ATOM | 238 | CA | VAL | A | 42 | 22.555 | 16.284 | 17.878 | 1.00 | 10.78 | A |
| ATOM | 239 | CB | VAL | A | 42 | 21.630 | 15.386 | 18.737 | 1.00 | 9.17 | A |
| ATOM | 240 | CG1 | VAL | A | 42 | 20.194 | 15.882 | 18.688 | 1.00 | 7.55 | A |
| ATOM | 241 | CG2 | VAL | A | 42 | 21.733 | 13.946 | 18.257 | 1.00 | 8.30 | A |
| ATOM | 242 | C | VAL | A | 42 | 22.090 | 17.720 | 18.066 | 1.00 | 12.10 | A |
| ATOM | 243 | O | VAL | A | 42 | 22.271 | 18.308 | 19.122 | 1.00 | 13.74 | A |
| ATOM | 244 | N | ALA | A | 43 | 21.435 | 18.254 | 17.041 | 1.00 | 13.69 | A |
| ATOM | 245 | CA | ALA | A | 43 | 20.892 | 19.608 | 17.084 | 1.00 | 14.92 | A |
| ATOM | 246 | CB | ALA | A | 43 | 21.506 | 20.464 | 15.967 | 1.00 | 12.07 | A |
| ATOM | 247 | C | ALA | A | 43 | 19.370 | 19.532 | 16.910 | 1.00 | 16.04 | A |
| ATOM | 248 | O | ALA | A | 43 | 18.867 | 18.650 | 16.230 | 1.00 | 17.32 | A |
| ATOM | 249 | N | VAL | A | 44 | 18.643 | 20.451 | 17.531 | 1.00 | 17.57 | A |
| ATOM | 250 | CA | VAL | A | 44 | 17.190 | 20.488 | 17.413 | 1.00 | 18.05 | A |
| ATOM | 251 | CB | VAL | A | 44 | 16.508 | 20.146 | 18.759 | 1.00 | 18.96 | A |
| ATOM | 252 | CG1 | VAL | A | 44 | 16.977 | 18.805 | 19.260 | 1.00 | 16.87 | A |
| ATOM | 253 | CG2 | VAL | A | 44 | 16.802 | 21.223 | 19.769 | 1.00 | 18.29 | A |
| ATOM | 254 | C | VAL | A | 44 | 16.724 | 21.887 | 16.981 | 1.00 | 18.01 | A |
| ATOM | 255 | O | VAL | A | 44 | 17.454 | 22.876 | 17.124 | 1.00 | 18.07 | A |
| ATOM | 256 | N | THR | A | 45 | 15.511 | 21.962 | 16.445 | 1.00 | 17.93 | A |
| ATOM | 257 | CA | THR | A | 45 | 14.931 | 23.232 | 16.025 | 1.00 | 19.70 | A |

Figure 2 (suite 3)

| | | | | | | | | | | | |
|---|---|---|---|---|---|---|---|---|---|---|---|
| ATOM | 258 | CB | THR | A | 45 | 14.420 | 23.174 | 14.569 | 1.00 | 18.60 | A |
| ATOM | 259 | OG1 | THR | A | 45 | 13.351 | 22.224 | 14.464 | 1.00 | 17.49 | A |
| ATOM | 260 | CG2 | THR | A | 45 | 15.530 | 22.763 | 13.637 | 1.00 | 17.18 | A |
| ATOM | 261 | C | THR | A | 45 | 13.758 | 23.486 | 16.970 | 1.00 | 22.36 | A |
| ATOM | 262 | O | THR | A | 45 | 13.087 | 22.541 | 17.387 | 1.00 | 22.98 | A |
| ATOM | 263 | N | HIS | A | 46 | 13.525 | 24.745 | 17.330 | 1.00 | 25.80 | A |
| ATOM | 264 | CA | HIS | A | 46 | 12.428 | 25.091 | 18.238 | 1.00 | 29.57 | A |
| ATOM | 265 | CB | HIS | A | 46 | 12.959 | 25.288 | 19.663 | 1.00 | 30.37 | A |
| ATOM | 266 | CG | HIS | A | 46 | 14.003 | 26.357 | 19.781 | 1.00 | 33.94 | A |
| ATOM | 267 | CD2 | HIS | A | 46 | 14.002 | 27.655 | 19.390 | 1.00 | 35.14 | A |
| ATOM | 268 | ND1 | HIS | A | 46 | 15.236 | 26.133 | 20.355 | 1.00 | 35.00 | A |
| ATOM | 269 | CE1 | HIS | A | 46 | 15.951 | 27.244 | 20.311 | 1.00 | 35.25 | A |
| ATOM | 270 | NE2 | HIS | A | 46 | 15.225 | 28.182 | 19.729 | 1.00 | 35.70 | A |
| ATOM | 271 | C | HIS | A | 46 | 11.705 | 26.353 | 17.783 | 1.00 | 31.87 | A |
| ATOM | 272 | O | HIS | A | 46 | 12.147 | 27.032 | 16.855 | 1.00 | 31.90 | A |
| ATOM | 273 | N | ARG | A | 47 | 10.593 | 26.668 | 18.437 | 1.00 | 35.77 | A |
| ATOM | 274 | CA | ARG | A | 47 | 9.825 | 27.858 | 18.085 | 1.00 | 39.38 | A |
| ATOM | 275 | CB | ARG | A | 47 | 8.327 | 27.608 | 18.281 | 1.00 | 40.84 | A |
| ATOM | 276 | CG | ARG | A | 47 | 7.774 | 26.443 | 17.475 | 1.00 | 44.05 | A |
| ATOM | 277 | CD | ARG | A | 47 | 7.894 | 26.676 | 15.974 | 1.00 | 46.42 | A |
| ATOM | 278 | NE | ARG | A | 47 | 6.614 | 27.003 | 15.343 | 1.00 | 49.64 | A |
| ATOM | 279 | CZ | ARG | A | 47 | 5.998 | 28.180 | 15.434 | 1.00 | 52.02 | A |
| ATOM | 280 | NH1 | ARG | A | 47 | 6.536 | 29.171 | 16.136 | 1.00 | 52.80 | A |
| ATOM | 281 | NH2 | ARG | A | 47 | 4.842 | 28.370 | 14.809 | 1.00 | 52.67 | A |
| ATOM | 282 | C | ARG | A | 47 | 10.253 | 29.045 | 18.940 | 1.00 | 40.66 | A |
| ATOM | 283 | O | ARG | A | 47 | 9.652 | 30.111 | 18.869 | 1.00 | 42.53 | A |
| ATOM | 284 | N | GLY | A | 48 | 11.290 | 28.854 | 19.748 | 1.00 | 41.95 | A |
| ATOM | 285 | CA | GLY | A | 48 | 11.769 | 29.919 | 20.607 | 1.00 | 42.65 | A |
| ATOM | 286 | C | GLY | A | 48 | 11.780 | 29.477 | 22.056 | 1.00 | 43.64 | A |
| ATOM | 287 | O | GLY | A | 48 | 12.489 | 30.051 | 22.882 | 1.00 | 42.74 | A |
| ATOM | 288 | N | SER | A | 49 | 10.999 | 28.444 | 22.359 | 1.00 | 44.77 | A |
| ATOM | 289 | CA | SER | A | 49 | 10.899 | 27.906 | 23.714 | 1.00 | 46.53 | A |
| ATOM | 290 | CB | SER | A | 49 | 9.864 | 26.772 | 23.753 | 1.00 | 48.20 | A |
| ATOM | 291 | OG | SER | A | 49 | 10.162 | 25.762 | 22.798 | 1.00 | 50.23 | A |
| ATOM | 292 | C | SER | A | 49 | 12.231 | 27.403 | 24.277 | 1.00 | 46.47 | A |
| ATOM | 293 | O | SER | A | 49 | 12.317 | 27.036 | 25.451 | 1.00 | 47.41 | A |
| ATOM | 294 | N | GLY | A | 50 | 13.264 | 27.379 | 23.442 | 1.00 | 45.76 | A |
| ATOM | 295 | CA | GLY | A | 50 | 14.567 | 26.930 | 23.898 | 1.00 | 45.02 | A |
| ATOM | 296 | C | GLY | A | 50 | 14.801 | 25.434 | 23.791 | 1.00 | 44.71 | A |
| ATOM | 297 | O | GLY | A | 50 | 13.864 | 24.634 | 23.888 | 1.00 | 45.02 | A |
| ATOM | 298 | N | ALA | A | 51 | 16.063 | 25.058 | 23.602 | 1.00 | 43.13 | A |
| ATOM | 299 | CA | ALA | A | 51 | 16.449 | 23.655 | 23.477 | 1.00 | 42.29 | A |
| ATOM | 300 | CB | ALA | A | 51 | 17.644 | 23.538 | 22.534 | 1.00 | 42.51 | A |
| ATOM | 301 | C | ALA | A | 51 | 16.781 | 23.005 | 24.829 | 1.00 | 41.22 | A |
| ATOM | 302 | O | ALA | A | 51 | 17.281 | 23.661 | 25.745 | 1.00 | 41.48 | A |
| ATOM | 303 | N | PRO | A | 52 | 16.510 | 21.697 | 24.963 | 1.00 | 39.90 | A |
| ATOM | 304 | CD | PRO | A | 52 | 16.086 | 20.765 | 23.905 | 1.00 | 39.31 | A |
| ATOM | 305 | CA | PRO | A | 52 | 16.789 | 20.972 | 26.208 | 1.00 | 38.92 | A |
| ATOM | 306 | CB | PRO | A | 52 | 16.296 | 19.566 | 25.900 | 1.00 | 38.48 | A |
| ATOM | 307 | CG | PRO | A | 52 | 16.584 | 19.438 | 24.437 | 1.00 | 39.13 | A |
| ATOM | 308 | C | PRO | A | 52 | 18.280 | 20.998 | 26.545 | 1.00 | 38.57 | A |
| ATOM | 309 | O | PRO | A | 52 | 19.129 | 21.011 | 25.648 | 1.00 | 39.03 | A |
| ATOM | 310 | N | LYS | A | 53 | 18.595 | 20.996 | 27.837 | 1.00 | 37.67 | A |
| ATOM | 311 | CA | LYS | A | 53 | 19.984 | 21.037 | 28.280 | 1.00 | 35.90 | A |
| ATOM | 312 | CB | LYS | A | 53 | 20.078 | 20.843 | 29.800 | 1.00 | 36.66 | A |
| ATOM | 313 | CG | LYS | A | 53 | 19.534 | 22.005 | 30.623 | 0.00 | 36.47 | A |
| ATOM | 314 | CD | LYS | A | 53 | 18.017 | 22.083 | 30.577 | 0.00 | 36.59 | A |
| ATOM | 315 | CE | LYS | A | 53 | 17.379 | 20.893 | 31.281 | 0.00 | 36.60 | A |
| ATOM | 316 | NZ | LYS | A | 53 | 15.893 | 20.993 | 31.312 | 0.00 | 36.63 | A |
| ATOM | 317 | C | LYS | A | 53 | 20.835 | 19.993 | 27.579 | 1.00 | 34.46 | A |
| ATOM | 318 | O | LYS | A | 53 | 20.393 | 18.871 | 27.338 | 1.00 | 35.40 | A |
| ATOM | 319 | N | GLY | A | 54 | 22.057 | 20.374 | 27.237 | 1.00 | 32.76 | A |
| ATOM | 320 | CA | GLY | A | 54 | 22.953 | 19.445 | 26.579 | 1.00 | 32.34 | A |
| ATOM | 321 | C | GLY | A | 54 | 22.880 | 19.411 | 25.067 | 1.00 | 32.21 | A |
| ATOM | 322 | O | GLY | A | 54 | 23.858 | 19.030 | 24.420 | 1.00 | 32.47 | A |
| ATOM | 323 | N | LEU | A | 55 | 21.740 | 19.808 | 24.500 | 1.00 | 31.34 | A |
| ATOM | 324 | CA | LEU | A | 55 | 21.556 | 19.797 | 23.048 | 1.00 | 30.34 | A |
| ATOM | 325 | CB | LEU | A | 55 | 20.224 | 19.125 | 22.689 | 1.00 | 30.13 | A |
| ATOM | 326 | CG | LEU | A | 55 | 20.017 | 17.653 | 23.067 | 1.00 | 29.86 | A |
| ATOM | 327 | CD1 | LEU | A | 55 | 18.605 | 17.229 | 22.712 | 1.00 | 27.31 | A |
| ATOM | 328 | CD2 | LEU | A | 55 | 21.036 | 16.777 | 22.340 | 1.00 | 29.67 | A |
| ATOM | 329 | C | LEU | A | 55 | 21.597 | 21.175 | 22.394 | 1.00 | 30.27 | A |
| ATOM | 330 | O | LEU | A | 55 | 21.013 | 22.134 | 22.898 | 1.00 | 30.32 | A |

Figure 2 (suite 4)

| | | | | | | | | | | | |
|---|---|---|---|---|---|---|---|---|---|---|---|
| ATOM | 331 | N | PHE | A | 56 | 22.282 | 21.262 | 21.259 | 1.00 | 30.12 | A |
| ATOM | 332 | CA | PHE | A | 56 | 22.374 | 22.509 | 20.508 | 1.00 | 31.66 | A |
| ATOM | 333 | CB | PHE | A | 56 | 23.494 | 22.414 | 19.470 | 1.00 | 33.62 | A |
| ATOM | 334 | CG | PHE | A | 56 | 23.665 | 23.653 | 18.636 | 1.00 | 34.80 | A |
| ATOM | 335 | CD1 | PHE | A | 56 | 24.081 | 24.845 | 19.213 | 1.00 | 36.08 | A |
| ATOM | 336 | CD2 | PHE | A | 56 | 23.416 | 23.625 | 17.267 | 1.00 | 37.31 | A |
| ATOM | 337 | CE1 | PHE | A | 56 | 24.249 | 25.994 | 18.438 | 1.00 | 37.15 | A |
| ATOM | 338 | CE2 | PHE | A | 56 | 23.580 | 24.766 | 16.483 | 1.00 | 37.74 | A |
| ATOM | 339 | CZ | PHE | A | 56 | 23.997 | 25.953 | 17.070 | 1.00 | 38.26 | A |
| ATOM | 340 | C | PHE | A | 56 | 21.045 | 22.774 | 19.793 | 1.00 | 32.44 | A |
| ATOM | 341 | O | PHE | A | 56 | 20.567 | 21.937 | 19.022 | 1.00 | 32.88 | A |
| ATOM | 342 | N | GLY | A | 57 | 20.453 | 23.938 | 20.051 | 1.00 | 32.20 | A |
| ATOM | 343 | CA | GLY | A | 57 | 19.190 | 24.288 | 19.424 | 1.00 | 31.04 | A |
| ATOM | 344 | C | GLY | A | 57 | 19.293 | 25.494 | 18.509 | 1.00 | 30.73 | A |
| ATOM | 345 | O | GLY | A | 57 | 20.230 | 26.283 | 18.607 | 1.00 | 30.35 | A |
| ATOM | 346 | N | VAL | A | 58 | 18.335 | 25.625 | 17.599 | 1.00 | 30.43 | A |
| ATOM | 347 | CA | VAL | A | 58 | 18.293 | 26.749 | 16.666 | 1.00 | 29.91 | A |
| ATOM | 348 | CB | VAL | A | 58 | 18.899 | 26.383 | 15.314 | 1.00 | 29.80 | A |
| ATOM | 349 | CG1 | VAL | A | 58 | 19.156 | 27.646 | 14.517 | 1.00 | 30.30 | A |
| ATOM | 350 | CG2 | VAL | A | 58 | 20.173 | 25.595 | 15.513 | 1.00 | 30.52 | A |
| ATOM | 351 | C | VAL | A | 58 | 16.829 | 27.096 | 16.445 | 1.00 | 29.02 | A |
| ATOM | 352 | O | VAL | A | 58 | 15.990 | 26.207 | 16.391 | 1.00 | 30.16 | A |
| ATOM | 353 | N | GLU | A | 59 | 16.509 | 28.376 | 16.326 | 1.00 | 29.02 | A |
| ATOM | 354 | CA | GLU | A | 59 | 15.114 | 28.758 | 16.122 | 1.00 | 30.73 | A |
| ATOM | 355 | CB | GLU | A | 59 | 14.904 | 30.202 | 16.567 | 1.00 | 32.88 | A |
| ATOM | 356 | CG | GLU | A | 59 | 13.504 | 30.499 | 17.051 | 1.00 | 38.59 | A |
| ATOM | 357 | CD | GLU | A | 59 | 13.467 | 31.700 | 17.985 | 1.00 | 42.88 | A |
| ATOM | 358 | OE1 | GLU | A | 59 | 12.377 | 31.995 | 18.538 | 1.00 | 43.65 | A |
| ATOM | 359 | OE2 | GLU | A | 59 | 14.535 | 32.340 | 18.164 | 1.00 | 43.95 | A |
| ATOM | 360 | C | GLU | A | 59 | 14.725 | 28.575 | 14.650 | 1.00 | 28.85 | A |
| ATOM | 361 | O | GLU | A | 59 | 15.450 | 28.999 | 13.749 | 1.00 | 27.33 | A |
| ATOM | 362 | N | VAL | A | 60 | 13.590 | 27.930 | 14.401 | 1.00 | 27.55 | A |
| ATOM | 363 | CA | VAL | A | 60 | 13.177 | 27.686 | 13.022 | 1.00 | 29.73 | A |
| ATOM | 364 | CB | VAL | A | 60 | 13.751 | 26.346 | 12.483 | 1.00 | 29.66 | A |
| ATOM | 365 | CG1 | VAL | A | 60 | 13.624 | 26.305 | 10.974 | 1.00 | 28.53 | A |
| ATOM | 366 | CG2 | VAL | A | 60 | 15.203 | 26.167 | 12.904 | 1.00 | 30.78 | A |
| ATOM | 367 | C | VAL | A | 60 | 11.671 | 27.616 | 12.800 | 1.00 | 30.43 | A |
| ATOM | 368 | O | VAL | A | 60 | 10.922 | 27.154 | 13.658 | 1.00 | 31.73 | A |
| ATOM | 369 | N | ASP | A | 61 | 11.248 | 28.076 | 11.629 | 1.00 | 31.13 | A |
| ATOM | 370 | CA | ASP | A | 61 | 9.853 | 28.029 | 11.216 | 1.00 | 32.77 | A |
| ATOM | 371 | CB | ASP | A | 61 | 9.281 | 29.439 | 11.072 | 1.00 | 35.63 | A |
| ATOM | 372 | CG | ASP | A | 61 | 7.803 | 29.440 | 10.701 | 1.00 | 38.86 | A |
| ATOM | 373 | OD1 | ASP | A | 61 | 7.418 | 28.703 | 9.767 | 1.00 | 39.87 | A |
| ATOM | 374 | OD2 | ASP | A | 61 | 7.026 | 30.183 | 11.339 | 1.00 | 40.64 | A |
| ATOM | 375 | C | ASP | A | 61 | 9.934 | 27.356 | 9.846 | 1.00 | 33.27 | A |
| ATOM | 376 | O | ASP | A | 61 | 10.457 | 27.945 | 8.894 | 1.00 | 33.52 | A |
| ATOM | 377 | N | VAL | A | 62 | 9.445 | 26.121 | 9.753 | 1.00 | 32.93 | A |
| ATOM | 378 | CA | VAL | A | 62 | 9.489 | 25.367 | 8.499 | 1.00 | 32.43 | A |
| ATOM | 379 | CB | VAL | A | 62 | 9.031 | 23.899 | 8.703 | 1.00 | 31.81 | A |
| ATOM | 380 | CG1 | VAL | A | 62 | 10.091 | 23.133 | 9.463 | 1.00 | 31.21 | A |
| ATOM | 381 | CG2 | VAL | A | 62 | 7.699 | 23.856 | 9.450 | 1.00 | 29.38 | A |
| ATOM | 382 | C | VAL | A | 62 | 8.646 | 25.997 | 7.392 | 1.00 | 33.44 | A |
| ATOM | 383 | O | VAL | A | 62 | 8.665 | 25.544 | 6.242 | 1.00 | 33.58 | A |
| ATOM | 384 | N | THR | A | 63 | 7.909 | 27.044 | 7.742 | 1.00 | 33.72 | A |
| ATOM | 385 | CA | THR | A | 63 | 7.082 | 27.746 | 6.774 | 1.00 | 33.80 | A |
| ATOM | 386 | CB | THR | A | 63 | 5.879 | 28.403 | 7.461 | 1.00 | 34.82 | A |
| ATOM | 387 | OG1 | THR | A | 63 | 4.955 | 27.388 | 7.868 | 1.00 | 35.76 | A |
| ATOM | 388 | CG2 | THR | A | 63 | 5.192 | 29.379 | 6.529 | 1.00 | 35.94 | A |
| ATOM | 389 | C | THR | A | 63 | 7.901 | 28.825 | 6.080 | 1.00 | 33.45 | A |
| ATOM | 390 | O | THR | A | 63 | 7.526 | 29.298 | 5.010 | 1.00 | 33.69 | A |
| ATOM | 391 | N | ASP | A | 64 | 9.027 | 29.194 | 6.689 | 1.00 | 33.71 | A |
| ATOM | 392 | CA | ASP | A | 64 | 9.910 | 30.236 | 6.155 | 1.00 | 34.12 | A |
| ATOM | 393 | CB | ASP | A | 64 | 10.290 | 31.220 | 7.267 | 1.00 | 35.61 | A |
| ATOM | 394 | CG | ASP | A | 64 | 11.098 | 32.400 | 6.755 | 1.00 | 36.75 | A |
| ATOM | 395 | OD1 | ASP | A | 64 | 12.053 | 32.185 | 5.972 | 1.00 | 35.47 | A |
| ATOM | 396 | OD2 | ASP | A | 64 | 10.778 | 33.542 | 7.149 | 1.00 | 39.25 | A |
| ATOM | 397 | C | ASP | A | 64 | 11.188 | 29.675 | 5.535 | 1.00 | 33.05 | A |
| ATOM | 398 | O | ASP | A | 64 | 12.127 | 29.300 | 6.242 | 1.00 | 33.08 | A |
| ATOM | 399 | N | SER | A | 65 | 11.231 | 29.643 | 4.212 | 1.00 | 31.95 | A |
| ATOM | 400 | CA | SER | A | 65 | 12.394 | 29.117 | 3.516 | 1.00 | 32.25 | A |
| ATOM | 401 | CB | SER | A | 65 | 12.232 | 29.314 | 2.010 | 1.00 | 31.49 | A |
| ATOM | 402 | OG | SER | A | 65 | 13.361 | 28.819 | 1.320 | 1.00 | 32.20 | A |
| ATOM | 403 | C | SER | A | 65 | 13.710 | 29.746 | 3.979 | 1.00 | 31.93 | A |

Figure 2. (suite 5)

| | | | | | | | | | | | |
|---|---|---|---|---|---|---|---|---|---|---|---|
| ATOM | 404 | O | SER | A | 65 | 14.723 | 29.060 | 4.093 | 1.00 | 32.07 | A |
| ATOM | 405 | N | ASP | A | 66 | 13.698 | 31.048 | 4.247 | 1.00 | 32.46 | A |
| ATOM | 406 | CA | ASP | A | 66 | 14.914 | 31.729 | 4.678 | 1.00 | 31.86 | A |
| ATOM | 407 | CB | ASP | A | 66 | 14.754 | 33.247 | 4.563 | 1.00 | 35.02 | A |
| ATOM | 408 | CG | ASP | A | 66 | 14.694 | 33.721 | 3.116 | 1.00 | 37.66 | A |
| ATOM | 409 | OD1 | ASP | A | 66 | 15.623 | 33.397 | 2.339 | 1.00 | 38.87 | A |
| ATOM | 410 | OD2 | ASP | A | 66 | 13.719 | 34.419 | 2.759 | 1.00 | 39.18 | A |
| ATOM | 411 | C | ASP | A | 66 | 15.295 | 31.353 | 6.099 | 1.00 | 30.66 | A |
| ATOM | 412 | O | ASP | A | 66 | 16.475 | 31.278 | 6.428 | 1.00 | 30.74 | A |
| ATOM | 413 | N | ALA | A | 67 | 14.296 | 31.120 | 6.940 | 1.00 | 28.27 | A |
| ATOM | 414 | CA | ALA | A | 67 | 14.553 | 30.735 | 8.316 | 1.00 | 27.52 | A |
| ATOM | 415 | CB | ALA | A | 67 | 13.256 | 30.762 | 9.121 | 1.00 | 27.23 | A |
| ATOM | 416 | C | ALA | A | 67 | 15.153 | 29.327 | 8.334 | 1.00 | 28.33 | A |
| ATOM | 417 | O | ALA | A | 67 | 15.891 | 28.966 | 9.258 | 1.00 | 29.04 | A |
| ATOM | 418 | N | VAL | A | 68 | 14.829 | 28.539 | 7.310 | 1.00 | 26.43 | A |
| ATOM | 419 | CA | VAL | A | 68 | 15.326 | 27.176 | 7.211 | 1.00 | 24.13 | A |
| ATOM | 420 | CB | VAL | A | 68 | 14.483 | 26.329 | 6.211 | 1.00 | 22.92 | A |
| ATOM | 421 | CG1 | VAL | A | 68 | 15.191 | 25.021 | 5.902 | 1.00 | 18.66 | A |
| ATOM | 422 | CG2 | VAL | A | 68 | 13.095 | 26.059 | 6.789 | 1.00 | 18.79 | A |
| ATOM | 423 | C | VAL | A | 68 | 16.771 | 27.197 | 6.756 | 1.00 | 24.60 | A |
| ATOM | 424 | O | VAL | A | 68 | 17.564 | 26.355 | 7.167 | 1.00 | 25.70 | A |
| ATOM | 425 | N | ASP | A | 69 | 17.114 | 28.158 | 5.908 | 1.00 | 24.37 | A |
| ATOM | 426 | CA | ASP | A | 69 | 18.482 | 28.264 | 5.412 | 1.00 | 25.76 | A |
| ATOM | 427 | CB | ASP | A | 69 | 18.571 | 29.306 | 4.304 | 1.00 | 27.60 | A |
| ATOM | 428 | CG | ASP | A | 69 | 19.920 | 29.309 | 3.620 | 1.00 | 28.25 | A |
| ATOM | 429 | OD1 | ASP | A | 69 | 20.251 | 30.328 | 2.983 | 1.00 | 30.79 | A |
| ATOM | 430 | OD2 | ASP | A | 69 | 20.642 | 28.295 | 3.708 | 1.00 | 28.60 | A |
| ATOM | 431 | C | ASP | A | 69 | 19.409 | 28.683 | 6.548 | 1.00 | 26.02 | A |
| ATOM | 432 | O | ASP | A | 69 | 20.514 | 28.162 | 6.690 | 1.00 | 26.04 | A |
| ATOM | 433 | N | ARG | A | 70 | 18.954 | 29.641 | 7.348 | 1.00 | 26.01 | A |
| ATOM | 434 | CA | ARG | A | 70 | 19.744 | 30.124 | 8.469 | 1.00 | 27.00 | A |
| ATOM | 435 | CB | ARG | A | 70 | 19.041 | 31.307 | 9.142 | 1.00 | 29.03 | A |
| ATOM | 436 | CG | ARG | A | 70 | 19.292 | 32.651 | 8.470 | 1.00 | 31.66 | A |
| ATOM | 437 | CD | ARG | A | 70 | 18.224 | 33.652 | 8.879 | 1.00 | 35.30 | A |
| ATOM | 438 | NE | ARG | A | 70 | 17.908 | 33.555 | 10.302 | 1.00 | 38.57 | A |
| ATOM | 439 | CZ | ARG | A | 70 | 16.678 | 33.652 | 10.805 | 1.00 | 40.60 | A |
| ATOM | 440 | NH1 | ARG | A | 70 | 15.638 | 33.849 | 10.001 | 1.00 | 38.52 | A |
| ATOM | 441 | NH2 | ARG | A | 70 | 16.488 | 33.554 | 12.117 | 1.00 | 40.85 | A |
| ATOM | 442 | C | ARG | A | 70 | 19.988 | 29.014 | 9.481 | 1.00 | 25.32 | A |
| ATOM | 443 | O | ARG | A | 70 | 21.106 | 28.831 | 9.946 | 1.00 | 25.06 | A |
| ATOM | 444 | N | ALA | A | 71 | 18.936 | 28.272 | 9.810 | 1.00 | 23.96 | A |
| ATOM | 445 | CA | ALA | A | 71 | 19.042 | 27.184 | 10.768 | 1.00 | 21.97 | A |
| ATOM | 446 | CB | ALA | A | 71 | 17.717 | 26.463 | 10.883 | 1.00 | 20.85 | A |
| ATOM | 447 | C | ALA | A | 71 | 20.144 | 26.208 | 10.366 | 1.00 | 21.51 | A |
| ATOM | 448 | O | ALA | A | 71 | 21.066 | 25.961 | 11.140 | 1.00 | 21.16 | A |
| ATOM | 449 | N | PHE | A | 72 | 20.065 | 25.674 | 9.152 | 1.00 | 19.81 | A |
| ATOM | 450 | CA | PHE | A | 72 | 21.071 | 24.727 | 8.688 | 1.00 | 18.98 | A |
| ATOM | 451 | CB | PHE | A | 72 | 20.735 | 24.208 | 7.279 | 1.00 | 15.62 | A |
| ATOM | 452 | CG | PHE | A | 72 | 19.769 | 23.037 | 7.267 | 1.00 | 12.64 | A |
| ATOM | 453 | CD1 | PHE | A | 72 | 18.446 | 23.190 | 7.690 | 1.00 | 10.54 | A |
| ATOM | 454 | CD2 | PHE | A | 72 | 20.181 | 21.783 | 6.813 | 1.00 | 11.70 | A |
| ATOM | 455 | CE1 | PHE | A | 72 | 17.548 | 22.111 | 7.660 | 1.00 | 8.46 | A |
| ATOM | 456 | CE2 | PHE | A | 72 | 19.288 | 20.696 | 6.779 | 1.00 | 9.72 | A |
| ATOM | 457 | CZ | PHE | A | 72 | 17.970 | 20.867 | 7.204 | 1.00 | 8.12 | A |
| ATOM | 458 | C | PHE | A | 72 | 22.485 | 25.299 | 8.697 | 1.00 | 20.19 | A |
| ATOM | 459 | O | PHE | A | 72 | 23.443 | 24.572 | 8.962 | 1.00 | 21.83 | A |
| ATOM | 460 | N | THR | A | 73 | 22.633 | 26.590 | 8.414 | 1.00 | 20.23 | A |
| ATOM | 461 | CA | THR | A | 73 | 23.965 | 27.179 | 8.409 | 1.00 | 18.77 | A |
| ATOM | 462 | CB | THR | A | 73 | 23.972 | 28.559 | 7.695 | 1.00 | 19.86 | A |
| ATOM | 463 | OG1 | THR | A | 73 | 23.871 | 28.356 | 6.276 | 1.00 | 21.11 | A |
| ATOM | 464 | CG2 | THR | A | 73 | 25.275 | 29.315 | 7.982 | 1.00 | 19.14 | A |
| ATOM | 465 | C | THR | A | 73 | 24.552 | 27.295 | 9.822 | 1.00 | 17.23 | A |
| ATOM | 466 | O | THR | A | 73 | 25.752 | 27.112 | 10.017 | 1.00 | 15.66 | A |
| ATOM | 467 | N | ALA | A | 74 | 23.712 | 27.596 | 10.805 | 1.00 | 16.34 | A |
| ATOM | 468 | CA | ALA | A | 74 | 24.190 | 27.694 | 12.174 | 1.00 | 17.98 | A |
| ATOM | 469 | CB | ALA | A | 74 | 23.075 | 28.169 | 13.089 | 1.00 | 16.63 | A |
| ATOM | 470 | C | ALA | A | 74 | 24.662 | 26.295 | 12.593 | 1.00 | 20.31 | A |
| ATOM | 471 | O | ALA | A | 74 | 25.746 | 26.128 | 13.166 | 1.00 | 21.52 | A |
| ATOM | 472 | N | VAL | A | 75 | 23.852 | 25.288 | 12.282 | 1.00 | 19.33 | A |
| ATOM | 473 | CA | VAL | A | 75 | 24.184 | 23.918 | 12.614 | 1.00 | 19.29 | A |
| ATOM | 474 | CB | VAL | A | 75 | 22.987 | 22.977 | 12.282 | 1.00 | 18.48 | A |
| ATOM | 475 | CG1 | VAL | A | 75 | 23.382 | 21.513 | 12.453 | 1.00 | 16.30 | A |
| ATOM | 476 | CG2 | VAL | A | 75 | 21.813 | 23.303 | 13.196 | 1.00 | 15.34 | A |

Figure 2 (suite 6)

| | | | | | | | | | | | |
|---|---|---|---|---|---|---|---|---|---|---|---|
| ATOM | 477 | C | VAL | A | 75 | 25.448 | 23.448 | 11.886 | 1.00 | 21.32 | A |
| ATOM | 478 | O | VAL | A | 75 | 26.292 | 22.776 | 12.474 | 1.00 | 21.31 | A |
| ATOM | 479 | N | GLU | A | 76 | 25.588 | 23.801 | 10.615 | 1.00 | 23.29 | A |
| ATOM | 480 | CA | GLU | A | 76 | 26.757 | 23.370 | 9.851 | 1.00 | 26.20 | A |
| ATOM | 481 | CB | GLU | A | 76 | 26.624 | 23.801 | 8.386 | 1.00 | 27.40 | A |
| ATOM | 482 | CG | GLU | A | 76 | 25.513 | 23.085 | 7.629 | 1.00 | 31.27 | A |
| ATOM | 483 | CD | GLU | A | 76 | 25.030 | 23.861 | 6.410 | 1.00 | 34.75 | A |
| ATOM | 484 | OE1 | GLU | A | 76 | 24.706 | 25.062 | 6.572 | 1.00 | 35.30 | A |
| ATOM | 485 | OE2 | GLU | A | 76 | 24.966 | 23.274 | 5.302 | 1.00 | 34.70 | A |
| ATOM | 486 | C | GLU | A | 76 | 28.071 | 23.897 | 10.430 | 1.00 | 28.19 | A |
| ATOM | 487 | O | GLU | A | 76 | 29.127 | 23.259 | 10.292 | 1.00 | 27.84 | A |
| ATOM | 488 | N | GLU | A | 77 | 28.008 | 25.055 | 11.084 | 1.00 | 29.96 | A |
| ATOM | 489 | CA | GLU | A | 77 | 29.201 | 25.646 | 11.676 | 1.00 | 31.94 | A |
| ATOM | 490 | CB | GLU | A | 77 | 29.078 | 27.171 | 11.710 | 1.00 | 33.85 | A |
| ATOM | 491 | CG | GLU | A | 77 | 28.834 | 27.775 | 10.337 | 1.00 | 38.46 | A |
| ATOM | 492 | CD | GLU | A | 77 | 28.847 | 29.292 | 10.340 | 1.00 | 41.16 | A |
| ATOM | 493 | OE1 | GLU | A | 77 | 28.278 | 29.894 | 11.279 | 1.00 | 43.29 | A |
| ATOM | 494 | OE2 | GLU | A | 77 | 29.414 | 29.882 | 9.391 | 1.00 | 43.21 | A |
| ATOM | 495 | C | GLU | A | 77 | 29.445 | 25.100 | 13.080 | 1.00 | 31.56 | A |
| ATOM | 496 | O | GLU | A | 77 | 30.576 | 25.072 | 13.558 | 1.00 | 33.09 | A |
| ATOM | 497 | N | HIS | A | 78 | 28.384 | 24.646 | 13.730 | 1.00 | 30.22 | A |
| ATOM | 498 | CA | HIS | A | 78 | 28.497 | 24.107 | 15.071 | 1.00 | 29.57 | A |
| ATOM | 499 | CB | HIS | A | 78 | 27.157 | 24.215 | 15.790 | 1.00 | 31.26 | A |
| ATOM | 500 | CG | HIS | A | 78 | 27.092 | 23.442 | 17.072 | 1.00 | 32.68 | A |
| ATOM | 501 | CD2 | HIS | A | 78 | 26.690 | 22.175 | 17.329 | 1.00 | 33.49 | A |
| ATOM | 502 | ND1 | HIS | A | 78 | 27.474 | 23.976 | 18.283 | 1.00 | 33.95 | A |
| ATOM | 503 | CE1 | HIS | A | 78 | 27.305 | 23.072 | 19.233 | 1.00 | 34.12 | A |
| ATOM | 504 | NE2 | HIS | A | 78 | 26.831 | 21.971 | 18.681 | 1.00 | 33.04 | A |
| ATOM | 505 | C | HIS | A | 78 | 28.945 | 22.654 | 15.122 | 1.00 | 29.36 | A |
| ATOM | 506 | O | HIS | A | 78 | 29.661 | 22.272 | 16.042 | 1.00 | 32.16 | A |
| ATOM | 507 | N | GLN | A | 79 | 28.531 | 21.842 | 14.150 | 1.00 | 27.79 | A |
| ATOM | 508 | CA | GLN | A | 79 | 28.876 | 20.419 | 14.172 | 1.00 | 25.84 | A |
| ATOM | 509 | CB | GLN | A | 79 | 27.793 | 19.661 | 14.928 | 1.00 | 23.90 | A |
| ATOM | 510 | CG | GLN | A | 79 | 26.430 | 19.831 | 14.285 | 1.00 | 21.34 | A |
| ATOM | 511 | CD | GLN | A | 79 | 25.348 | 19.075 | 15.001 | 1.00 | 18.75 | A |
| ATOM | 512 | OE1 | GLN | A | 79 | 25.074 | 19.322 | 16.172 | 1.00 | 18.68 | A |
| ATOM | 513 | NE2 | GLN | A | 79 | 24.719 | 18.145 | 14.297 | 1.00 | 18.96 | A |
| ATOM | 514 | C | GLN | A | 79 | 29.074 | 19.738 | 12.823 | 1.00 | 25.19 | A |
| ATOM | 515 | O | GLN | A | 79 | 28.977 | 18.518 | 12.737 | 1.00 | 25.32 | A |
| ATOM | 516 | N | GLY | A | 80 | 29.342 | 20.506 | 11.776 | 1.00 | 25.31 | A |
| ATOM | 517 | CA | GLY | A | 80 | 29.544 | 19.903 | 10.469 | 1.00 | 25.21 | A |
| ATOM | 518 | C | GLY | A | 80 | 28.255 | 19.706 | 9.686 | 1.00 | 25.87 | A |
| ATOM | 519 | O | GLY | A | 80 | 27.164 | 19.716 | 10.263 | 1.00 | 25.05 | A |
| ATOM | 520 | N | PRO | A | 81 | 28.349 | 19.503 | 8.364 | 1.00 | 25.96 | A |
| ATOM | 521 | CD | PRO | A | 81 | 29.570 | 19.160 | 7.617 | 1.00 | 25.04 | A |
| ATOM | 522 | CA | PRO | A | 81 | 27.158 | 19.308 | 7.531 | 1.00 | 26.35 | A |
| ATOM | 523 | CB | PRO | A | 81 | 27.736 | 18.778 | 6.216 | 1.00 | 26.12 | A |
| ATOM | 524 | CG | PRO | A | 81 | 29.127 | 19.331 | 6.197 | 1.00 | 25.01 | A |
| ATOM | 525 | C | PRO | A | 81 | 26.198 | 18.303 | 8.170 | 1.00 | 26.26 | A |
| ATOM | 526 | O | PRO | A | 81 | 26.632 | 17.355 | 8.827 | 1.00 | 26.52 | A |
| ATOM | 527 | N | VAL | A | 82 | 24.900 | 18.523 | 7.989 | 1.00 | 25.08 | A |
| ATOM | 528 | CA | VAL | A | 82 | 23.898 | 17.619 | 8.527 | 1.00 | 24.24 | A |
| ATOM | 529 | CB | VAL | A | 82 | 22.515 | 18.264 | 8.488 | 1.00 | 24.93 | A |
| ATOM | 530 | CG1 | VAL | A | 82 | 21.446 | 17.241 | 8.877 | 1.00 | 24.92 | A |
| ATOM | 531 | CG2 | VAL | A | 82 | 22.490 | 19.456 | 9.426 | 1.00 | 25.11 | A |
| ATOM | 532 | C | VAL | A | 82 | 23.900 | 16.349 | 7.674 | 1.00 | 24.20 | A |
| ATOM | 533 | O | VAL | A | 82 | 23.828 | 16.415 | 6.450 | 1.00 | 25.52 | A |
| ATOM | 534 | N | GLU | A | 83 | 23.987 | 15.196 | 8.328 | 1.00 | 22.45 | A |
| ATOM | 535 | CA | GLU | A | 83 | 24.041 | 13.918 | 7.631 | 1.00 | 18.70 | A |
| ATOM | 536 | CB | GLU | A | 83 | 25.234 | 13.114 | 8.149 | 1.00 | 18.70 | A |
| ATOM | 537 | CG | GLU | A | 83 | 26.574 | 13.822 | 8.028 | 1.00 | 19.34 | A |
| ATOM | 538 | CD | GLU | A | 83 | 27.681 | 13.098 | 8.775 | 1.00 | 20.88 | A |
| ATOM | 539 | OE1 | GLU | A | 83 | 27.719 | 13.182 | 10.026 | 1.00 | 24.26 | A |
| ATOM | 540 | OE2 | GLU | A | 83 | 28.502 | 12.437 | 8.111 | 1.00 | 19.32 | A |
| ATOM | 541 | C | GLU | A | 83 | 22.765 | 13.117 | 7.839 | 1.00 | 17.01 | A |
| ATOM | 542 | O | GLU | A | 83 | 22.387 | 12.297 | 7.012 | 1.00 | 16.78 | A |
| ATOM | 543 | N | VAL | A | 84 | 22.112 | 13.361 | 8.964 | 1.00 | 14.72 | A |
| ATOM | 544 | CA | VAL | A | 84 | 20.893 | 12.668 | 9.309 | 1.00 | 13.64 | A |
| ATOM | 545 | CB | VAL | A | 84 | 21.132 | 11.705 | 10.507 | 1.00 | 12.18 | A |
| ATOM | 546 | CG1 | VAL | A | 84 | 19.827 | 11.144 | 11.028 | 1.00 | 7.20 | A |
| ATOM | 547 | CG2 | VAL | A | 84 | 22.035 | 10.578 | 10.067 | 1.00 | 9.14 | A |
| ATOM | 548 | C | VAL | A | 84 | 19.855 | 13.716 | 9.667 | 1.00 | 15.60 | A |
| ATOM | 549 | O | VAL | A | 84 | 19.973 | 14.401 | 10.690 | 1.00 | 18.17 | A |

Figure 2 (suite 7)

| | | | | | | | | | | | |
|---|---|---|---|---|---|---|---|---|---|---|---|
| ATOM | 550 | N | LEU | A | 85 | 18.850 | 13.840 | 8.801 | 1.00 | 16.29 | A |
| ATOM | 551 | CA | LEU | A | 85 | 17.755 | 14.786 | 8.981 | 1.00 | 15.24 | A |
| ATOM | 552 | CB | LEU | A | 85 | 17.425 | 15.461 | 7.651 | 1.00 | 13.96 | A |
| ATOM | 553 | CG | LEU | A | 85 | 16.039 | 16.112 | 7.572 | 1.00 | 15.58 | A |
| ATOM | 554 | CD1 | LEU | A | 85 | 15.920 | 17.221 | 8.597 | 1.00 | 14.82 | A |
| ATOM | 555 | CD2 | LEU | A | 85 | 15.799 | 16.636 | 6.158 | 1.00 | 16.77 | A |
| ATOM | 556 | C | LEU | A | 85 | 16.506 | 14.095 | 9.513 | 1.00 | 15.67 | A |
| ATOM | 557 | O | LEU | A | 85 | 15.963 | 13.187 | 8.885 | 1.00 | 15.78 | A |
| ATOM | 558 | N | VAL | A | 86 | 16.054 | 14.526 | 10.679 | 1.00 | 15.72 | A |
| ATOM | 559 | CA | VAL | A | 86 | 14.858 | 13.962 | 11.276 | 1.00 | 16.19 | A |
| ATOM | 560 | CB | VAL | A | 86 | 15.116 | 13.486 | 12.719 | 1.00 | 15.20 | A |
| ATOM | 561 | CG1 | VAL | A | 86 | 13.812 | 13.023 | 13.352 | 1.00 | 12.18 | A |
| ATOM | 562 | CG2 | VAL | A | 86 | 16.134 | 12.349 | 12.716 | 1.00 | 11.16 | A |
| ATOM | 563 | C | VAL | A | 86 | 13.759 | 15.015 | 11.285 | 1.00 | 18.33 | A |
| ATOM | 564 | O | VAL | A | 86 | 13.702 | 15.863 | 12.176 | 1.00 | 19.61 | A |
| ATOM | 565 | N | SER | A | 87 | 12.891 | 14.938 | 10.281 | 1.00 | 19.30 | A |
| ATOM | 566 | CA | SER | A | 87 | 11.770 | 15.849 | 10.106 | 1.00 | 18.78 | A |
| ATOM | 567 | CB | SER | A | 87 | 11.387 | 15.880 | 8.629 | 1.00 | 18.34 | A |
| ATOM | 568 | OG | SER | A | 87 | 10.300 | 16.756 | 8.405 | 1.00 | 20.73 | A |
| ATOM | 569 | C | SER | A | 87 | 10.548 | 15.470 | 10.942 | 1.00 | 19.77 | A |
| ATOM | 570 | O | SER | A | 87 | 9.936 | 14.437 | 10.720 | 1.00 | 19.19 | A |
| ATOM | 571 | N | ASN | A | 88 | 10.200 | 16.318 | 11.900 | 1.00 | 22.39 | A |
| ATOM | 572 | CA | ASN | A | 88 | 9.041 | 16.101 | 12.769 | 1.00 | 24.29 | A |
| ATOM | 573 | CB | ASN | A | 88 | 9.433 | 16.340 | 14.226 | 1.00 | 26.33 | A |
| ATOM | 574 | CG | ASN | A | 88 | 9.584 | 15.060 | 15.016 | 1.00 | 28.09 | A |
| ATOM | 575 | OD1 | ASN | A | 88 | 8.601 | 14.374 | 15.316 | 1.00 | 28.53 | A |
| ATOM | 576 | ND2 | ASN | A | 88 | 10.820 | 14.732 | 15.368 | 1.00 | 28.84 | A |
| ATOM | 577 | C | ASN | A | 88 | 7.902 | 17.067 | 12.417 | 1.00 | 24.93 | A |
| ATOM | 578 | O | ASN | A | 88 | 8.083 | 18.011 | 11.647 | 1.00 | 25.97 | A |
| ATOM | 579 | N | ALA | A | 89 | 6.739 | 16.825 | 13.010 | 1.00 | 26.22 | A |
| ATOM | 580 | CA | ALA | A | 89 | 5.540 | 17.645 | 12.824 | 1.00 | 26.71 | A |
| ATOM | 581 | CB | ALA | A | 89 | 4.840 | 17.843 | 14.176 | 1.00 | 26.01 | A |
| ATOM | 582 | C | ALA | A | 89 | 5.808 | 19.004 | 12.190 | 1.00 | 26.72 | A |
| ATOM | 583 | O | ALA | A | 89 | 5.221 | 20.008 | 12.601 | 1.00 | 27.99 | A |
| ATOM | 584 | N | MET | A | 100 | -13.817 | 21.691 | 9.259 | 1.00 | 45.63 | A |
| ATOM | 585 | CA | MET | A | 100 | -12.674 | 21.714 | 8.352 | 1.00 | 44.93 | A |
| ATOM | 586 | CB | MET | A | 100 | -12.591 | 20.395 | 7.577 | 1.00 | 45.18 | A |
| ATOM | 587 | CG | MET | A | 100 | -11.344 | 20.231 | 6.707 | 1.00 | 44.63 | A |
| ATOM | 588 | SD | MET | A | 100 | -9.824 | 19.959 | 7.662 | 1.00 | 44.50 | A |
| ATOM | 589 | CE | MET | A | 100 | -8.973 | 21.499 | 7.406 | 1.00 | 44.00 | A |
| ATOM | 590 | C | MET | A | 100 | -12.827 | 22.871 | 7.376 | 1.00 | 44.30 | A |
| ATOM | 591 | O | MET | A | 100 | -13.769 | 22.899 | 6.587 | 1.00 | 45.24 | A |
| ATOM | 592 | N | THR | A | 101 | -11.907 | 23.828 | 7.428 | 1.00 | 42.94 | A |
| ATOM | 593 | CA | THR | A | 101 | -11.968 | 24.974 | 6.528 | 1.00 | 41.58 | A |
| ATOM | 594 | CB | THR | A | 101 | -12.127 | 26.285 | 7.314 | 1.00 | 42.36 | A |
| ATOM | 595 | OG1 | THR | A | 101 | -10.962 | 26.512 | 8.118 | 1.00 | 44.01 | A |
| ATOM | 596 | CG2 | THR | A | 101 | -13.350 | 26.208 | 8.216 | 1.00 | 42.43 | A |
| ATOM | 597 | C | THR | A | 101 | -10.720 | 25.059 | 5.652 | 1.00 | 40.75 | A |
| ATOM | 598 | O | THR | A | 101 | -9.679 | 24.491 | 5.988 | 1.00 | 39.59 | A |
| ATOM | 599 | N | GLU | A | 102 | -10.830 | 25.761 | 4.528 | 1.00 | 39.89 | A |
| ATOM | 600 | CA | GLU | A | 102 | -9.707 | 25.902 | 3.609 | 1.00 | 39.87 | A |
| ATOM | 601 | CB | GLU | A | 102 | -10.088 | 26.794 | 2.422 | 1.00 | 42.29 | A |
| ATOM | 602 | CG | GLU | A | 102 | -8.936 | 27.013 | 1.446 | 1.00 | 48.38 | A |
| ATOM | 603 | CD | GLU | A | 102 | -9.294 | 27.911 | 0.266 | 1.00 | 51.80 | A |
| ATOM | 604 | OE1 | GLU | A | 102 | -10.092 | 27.482 | -0.599 | 1.00 | 54.49 | A |
| ATOM | 605 | OE2 | GLU | A | 102 | -8.773 | 29.048 | 0.205 | 1.00 | 53.40 | A |
| ATOM | 606 | C | GLU | A | 102 | -8.488 | 26.478 | 4.325 | 1.00 | 38.88 | A |
| ATOM | 607 | O | GLU | A | 102 | -7.346 | 26.172 | 3.974 | 1.00 | 38.59 | A |
| ATOM | 608 | N | GLU | A | 103 | -8.734 | 27.313 | 5.331 | 1.00 | 37.86 | A |
| ATOM | 609 | CA | GLU | A | 103 | -7.653 | 27.916 | 6.108 | 1.00 | 36.21 | A |
| ATOM | 610 | CB | GLU | A | 103 | -8.218 | 28.926 | 7.112 | 1.00 | 36.47 | A |
| ATOM | 611 | CG | GLU | A | 103 | -7.168 | 29.664 | 7.920 | 0.00 | 36.49 | A |
| ATOM | 612 | CD | GLU | A | 103 | -7.776 | 30.688 | 8.859 | 0.00 | 36.56 | A |
| ATOM | 613 | OE1 | GLU | A | 103 | -8.458 | 31.613 | 8.370 | 0.00 | 36.58 | A |
| ATOM | 614 | OE2 | GLU | A | 103 | -7.573 | 30.566 | 10.085 | 0.00 | 36.58 | A |
| ATOM | 615 | C | GLU | A | 103 | -6.895 | 26.824 | 6.855 | 1.00 | 34.53 | A |
| ATOM | 616 | O | GLU | A | 103 | -5.674 | 26.702 | 6.739 | 1.00 | 33.29 | A |
| ATOM | 617 | N | LYS | A | 104 | -7.633 | 26.030 | 7.623 | 1.00 | 33.32 | A |
| ATOM | 618 | CA | LYS | A | 104 | -7.037 | 24.948 | 8.386 | 1.00 | 33.29 | A |
| ATOM | 619 | CB | LYS | A | 104 | -8.093 | 24.251 | 9.239 | 1.00 | 35.06 | A |
| ATOM | 620 | CG | LYS | A | 104 | -8.689 | 25.128 | 10.320 | 1.00 | 38.98 | A |
| ATOM | 621 | CD | LYS | A | 104 | -9.655 | 24.337 | 11.187 | 1.00 | 42.34 | A |
| ATOM | 622 | CE | LYS | A | 104 | -10.393 | 25.239 | 12.165 | 1.00 | 44.35 | A |

Figure 2 (suite 8)

| | | | | | | | | | | | |
|---|---|---|---|---|---|---|---|---|---|---|---|
| ATOM | 623 | NZ | LYS | A | 104 | -11.480 | 24.500 | 12.871 | 1.00 | 45.84 | A |
| ATOM | 624 | C | LYS | A | 104 | -6.378 | 23.927 | 7.478 | 1.00 | 32.17 | A |
| ATOM | 625 | O | LYS | A | 104 | -5.365 | 23.332 | 7.841 | 1.00 | 34.00 | A |
| ATOM | 626 | N | PHE | A | 105 | -6.941 | 23.723 | 6.294 | 1.00 | 29.76 | A |
| ATOM | 627 | CA | PHE | A | 105 | -6.371 | 22.742 | 5.384 | 1.00 | 26.50 | A |
| ATOM | 628 | CB | PHE | A | 105 | -7.277 | 22.516 | 4.169 | 1.00 | 22.88 | A |
| ATOM | 629 | CG | PHE | A | 105 | -6.844 | 21.369 | 3.307 | 1.00 | 17.76 | A |
| ATOM | 630 | CD1 | PHE | A | 105 | -7.250 | 20.071 | 3.603 | 1.00 | 17.29 | A |
| ATOM | 631 | CD2 | PHE | A | 105 | -6.000 | 21.576 | 2.226 | 1.00 | 15.03 | A |
| ATOM | 632 | CE1 | PHE | A | 105 | -6.817 | 18.991 | 2.830 | 1.00 | 15.45 | A |
| ATOM | 633 | CE2 | PHE | A | 105 | -5.557 | 20.504 | 1.445 | 1.00 | 15.57 | A |
| ATOM | 634 | CZ | PHE | A | 105 | -5.967 | 19.207 | 1.748 | 1.00 | 13.25 | A |
| ATOM | 635 | C | PHE | A | 105 | -5.001 | 23.169 | 4.901 | 1.00 | 26.45 | A |
| ATOM | 636 | O | PHE | A | 105 | -4.045 | 22.392 | 4.956 | 1.00 | 25.86 | A |
| ATOM | 637 | N | GLU | A | 106 | -4.914 | 24.406 | 4.420 | 1.00 | 26.99 | A |
| ATOM | 638 | CA | GLU | A | 106 | -3.661 | 24.933 | 3.901 | 1.00 | 27.20 | A |
| ATOM | 639 | CB | GLU | A | 106 | -3.900 | 26.271 | 3.201 | 1.00 | 28.36 | A |
| ATOM | 640 | CG | GLU | A | 106 | -5.047 | 26.230 | 2.214 | 1.00 | 32.86 | A |
| ATOM | 641 | CD | GLU | A | 106 | -4.944 | 27.300 | 1.140 | 1.00 | 35.94 | A |
| ATOM | 642 | OE1 | GLU | A | 106 | -4.068 | 27.167 | 0.252 | 1.00 | 37.44 | A |
| ATOM | 643 | OE2 | GLU | A | 106 | -5.735 | 28.271 | 1.186 | 1.00 | 36.25 | A |
| ATOM | 644 | C | GLU | A | 106 | -2.606 | 25.086 | 4.987 | 1.00 | 25.96 | A |
| ATOM | 645 | O | GLU | A | 106 | -1.415 | 24.947 | 4.719 | 1.00 | 25.06 | A |
| ATOM | 646 | N | LYS | A | 107 | -3.035 | 25.367 | 6.212 | 1.00 | 25.24 | A |
| ATOM | 647 | CA | LYS | A | 107 | -2.081 | 25.513 | 7.302 | 1.00 | 25.50 | A |
| ATOM | 648 | CB | LYS | A | 107 | -2.789 | 25.859 | 8.615 | 1.00 | 27.35 | A |
| ATOM | 649 | CG | LYS | A | 107 | -2.635 | 27.310 | 9.032 | 1.00 | 30.84 | A |
| ATOM | 650 | CD | LYS | A | 107 | -3.080 | 27.532 | 10.488 | 1.00 | 33.87 | A |
| ATOM | 651 | CE | LYS | A | 107 | -2.119 | 26.890 | 11.504 | 1.00 | 33.04 | A |
| ATOM | 652 | NZ | LYS | A | 107 | -0.746 | 27.484 | 11.472 | 1.00 | 31.90 | A |
| ATOM | 653 | C | LYS | A | 107 | -1.298 | 24.214 | 7.475 | 1.00 | 23.67 | A |
| ATOM | 654 | O | LYS | A | 107 | -0.071 | 24.199 | 7.346 | 1.00 | 22.28 | A |
| ATOM | 655 | N | VAL | A | 108 | -2.022 | 23.131 | 7.754 | 1.00 | 21.32 | A |
| ATOM | 656 | CA | VAL | A | 108 | -1.428 | 21.816 | 7.949 | 1.00 | 19.26 | A |
| ATOM | 657 | CB | VAL | A | 108 | -2.520 | 20.762 | 8.140 | 1.00 | 18.21 | A |
| ATOM | 658 | CG1 | VAL | A | 108 | -1.910 | 19.379 | 8.207 | 1.00 | 14.51 | A |
| ATOM | 659 | CG2 | VAL | A | 108 | -3.302 | 21.068 | 9.411 | 1.00 | 17.32 | A |
| ATOM | 660 | C | VAL | A | 108 | -0.540 | 21.412 | 6.782 | 1.00 | 19.84 | A |
| ATOM | 661 | O | VAL | A | 108 | 0.575 | 20.909 | 6.972 | 1.00 | 19.91 | A |
| ATOM | 662 | N | ILE | A | 109 | -1.029 | 21.643 | 5.573 | 1.00 | 20.13 | A |
| ATOM | 663 | CA | ILE | A | 109 | -0.272 | 21.300 | 4.381 | 1.00 | 22.01 | A |
| ATOM | 664 | CB | ILE | A | 109 | -1.102 | 21.580 | 3.105 | 1.00 | 22.59 | A |
| ATOM | 665 | CG2 | ILE | A | 109 | -0.269 | 21.359 | 1.864 | 1.00 | 20.76 | A |
| ATOM | 666 | CG1 | ILE | A | 109 | -2.330 | 20.672 | 3.084 | 1.00 | 23.42 | A |
| ATOM | 667 | CD | ILE | A | 109 | -1.996 | 19.194 | 3.102 | 1.00 | 26.08 | A |
| ATOM | 668 | C | ILE | A | 109 | 1.033 | 22.084 | 4.317 | 1.00 | 23.26 | A |
| ATOM | 669 | O | ILE | A | 109 | 2.115 | 21.514 | 4.165 | 1.00 | 24.70 | A |
| ATOM | 670 | N | ASN | A | 110 | 0.923 | 23.400 | 4.447 | 1.00 | 24.87 | A |
| ATOM | 671 | CA | ASN | A | 110 | 2.080 | 24.281 | 4.385 | 1.00 | 24.73 | A |
| ATOM | 672 | CB | ASN | A | 110 | 1.624 | 25.720 | 4.635 | 1.00 | 26.05 | A |
| ATOM | 673 | CG | ASN | A | 110 | 2.694 | 26.735 | 4.320 | 1.00 | 27.18 | A |
| ATOM | 674 | OD1 | ASN | A | 110 | 3.355 | 26.661 | 3.288 | 1.00 | 29.59 | A |
| ATOM | 675 | ND2 | ASN | A | 110 | 2.858 | 27.705 | 5.205 | 1.00 | 29.56 | A |
| ATOM | 676 | C | ASN | A | 110 | 3.140 | 23.861 | 5.400 | 1.00 | 24.45 | A |
| ATOM | 677 | O | ASN | A | 110 | 4.326 | 23.819 | 5.091 | 1.00 | 25.13 | A |
| ATOM | 678 | N | ALA | A | 111 | 2.698 | 23.532 | 6.606 | 1.00 | 22.78 | A |
| ATOM | 679 | CA | ALA | A | 111 | 3.600 | 23.114 | 7.662 | 1.00 | 23.03 | A |
| ATOM | 680 | CB | ALA | A | 111 | 2.910 | 23.279 | 9.013 | 1.00 | 21.82 | A |
| ATOM | 681 | C | ALA | A | 111 | 4.098 | 21.668 | 7.493 | 1.00 | 24.51 | A |
| ATOM | 682 | O | ALA | A | 111 | 5.261 | 21.443 | 7.145 | 1.00 | 24.00 | A |
| ATOM | 683 | N | ASN | A | 112 | 3.203 | 20.704 | 7.723 | 1.00 | 25.14 | A |
| ATOM | 684 | CA | ASN | A | 112 | 3.501 | 19.272 | 7.645 | 1.00 | 23.24 | A |
| ATOM | 685 | CB | ASN | A | 112 | 2.305 | 18.463 | 8.135 | 1.00 | 22.49 | A |
| ATOM | 686 | CG | ASN | A | 112 | 2.043 | 18.661 | 9.603 | 1.00 | 24.17 | A |
| ATOM | 687 | OD1 | ASN | A | 112 | 2.927 | 19.093 | 10.341 | 1.00 | 26.63 | A |
| ATOM | 688 | ND2 | ASN | A | 112 | 0.833 | 18.334 | 10.046 | 1.00 | 23.37 | A |
| ATOM | 689 | C | ASN | A | 112 | 3.941 | 18.674 | 6.319 | 1.00 | 23.04 | A |
| ATOM | 690 | O | ASN | A | 112 | 4.531 | 17.597 | 6.305 | 1.00 | 23.47 | A |
| ATOM | 691 | N | LEU | A | 113 | 3.658 | 19.336 | 5.205 | 1.00 | 22.27 | A |
| ATOM | 692 | CA | LEU | A | 113 | 4.040 | 18.767 | 3.924 | 1.00 | 20.61 | A |
| ATOM | 693 | CB | LEU | A | 113 | 2.810 | 18.619 | 3.031 | 1.00 | 21.12 | A |
| ATOM | 694 | CG | LEU | A | 113 | 3.098 | 18.203 | 1.588 | 1.00 | 21.78 | A |
| ATOM | 695 | CD1 | LEU | A | 113 | 3.874 | 16.897 | 1.588 | 1.00 | 21.70 | A |

Figure 2 (suite 9)

| | | | | | | | | | | | |
|---|---|---|---|---|---|---|---|---|---|---|---|
| ATOM | 696 | CD2 | LEU | A | 113 | 1.793 | 18.067 | 0.820 | 1.00 | 22.32 | A |
| ATOM | 697 | C | LEU | A | 113 | 5.097 | 19.577 | 3.212 | 1.00 | 21.29 | A |
| ATOM | 698 | O | LEU | A | 113 | 6.159 | 19.070 | 2.870 | 1.00 | 22.82 | A |
| ATOM | 699 | N | THR | A | 114 | 4.801 | 20.842 | 2.973 | 1.00 | 21.39 | A |
| ATOM | 700 | CA | THR | A | 114 | 5.754 | 21.703 | 2.306 | 1.00 | 21.79 | A |
| ATOM | 701 | CB | THR | A | 114 | 5.078 | 23.000 | 1.876 | 1.00 | 22.36 | A |
| ATOM | 702 | OG1 | THR | A | 114 | 3.892 | 22.675 | 1.144 | 1.00 | 24.39 | A |
| ATOM | 703 | CG2 | THR | A | 114 | 6.004 | 23.820 | 0.988 | 1.00 | 23.08 | A |
| ATOM | 704 | C | THR | A | 114 | 6.899 | 21.991 | 3.280 | 1.00 | 21.28 | A |
| ATOM | 705 | O | THR | A | 114 | 8.000 | 22.362 | 2.874 | 1.00 | 21.07 | A |
| ATOM | 706 | N | GLY | A | 115 | 6.624 | 21.817 | 4.570 | 1.00 | 21.02 | A |
| ATOM | 707 | CA | GLY | A | 115 | 7.653 | 22.027 | 5.570 | 1.00 | 19.28 | A |
| ATOM | 708 | C | GLY | A | 115 | 8.698 | 20.953 | 5.350 | 1.00 | 18.29 | A |
| ATOM | 709 | O | GLY | A | 115 | 9.880 | 21.234 | 5.142 | 1.00 | 16.16 | A |
| ATOM | 710 | N | ALA | A | 116 | 8.248 | 19.704 | 5.365 | 1.00 | 18.26 | A |
| ATOM | 711 | CA | ALA | A | 116 | 9.149 | 18.587 | 5.165 | 1.00 | 17.60 | A |
| ATOM | 712 | CB | ALA | A | 116 | 8.357 | 17.291 | 5.078 | 1.00 | 18.47 | A |
| ATOM | 713 | C | ALA | A | 116 | 9.963 | 18.801 | 3.896 | 1.00 | 17.35 | A |
| ATOM | 714 | O | ALA | A | 116 | 11.179 | 18.585 | 3.886 | 1.00 | 16.68 | A |
| ATOM | 715 | N | PHE | A | 117 | 9.293 | 19.242 | 2.834 | 1.00 | 16.63 | A |
| ATOM | 716 | CA | PHE | A | 117 | 9.954 | 19.470 | 1.555 | 1.00 | 15.25 | A |
| ATOM | 717 | CB | PHE | A | 117 | 8.936 | 19.904 | 0.474 | 1.00 | 15.60 | A |
| ATOM | 718 | CG | PHE | A | 117 | 9.583 | 20.379 | -0.800 | 1.00 | 12.80 | A |
| ATOM | 719 | CD1 | PHE | A | 117 | 10.250 | 19.483 | -1.631 | 1.00 | 12.44 | A |
| ATOM | 720 | CD2 | PHE | A | 117 | 9.646 | 21.736 | -1.089 | 1.00 | 13.00 | A |
| ATOM | 721 | CE1 | PHE | A | 117 | 10.987 | 19.930 | -2.723 | 1.00 | 13.85 | A |
| ATOM | 722 | CE2 | PHE | A | 117 | 10.381 | 22.202 | -2.183 | 1.00 | 13.81 | A |
| ATOM | 723 | CZ | PHE | A | 117 | 11.056 | 21.297 | -3.001 | 1.00 | 15.67 | A |
| ATOM | 724 | C | PHE | A | 117 | 11.041 | 20.529 | 1.679 | 1.00 | 16.06 | A |
| ATOM | 725 | O | PHE | A | 117 | 12.107 | 20.408 | 1.079 | 1.00 | 16.35 | A |
| ATOM | 726 | N | ARG | A | 118 | 10.778 | 21.573 | 2.450 | 1.00 | 17.41 | A |
| ATOM | 727 | CA | ARG | A | 118 | 11.767 | 22.628 | 2.599 | 1.00 | 20.16 | A |
| ATOM | 728 | CB | ARG | A | 118 | 11.179 | 23.808 | 3.375 | 1.00 | 21.94 | A |
| ATOM | 729 | CG | ARG | A | 118 | 10.126 | 24.594 | 2.585 | 1.00 | 25.10 | A |
| ATOM | 730 | CD | ARG | A | 118 | 9.746 | 25.914 | 3.273 | 1.00 | 27.44 | A |
| ATOM | 731 | NE | ARG | A | 118 | 8.689 | 26.603 | 2.540 | 1.00 | 29.24 | A |
| ATOM | 732 | CZ | ARG | A | 118 | 7.389 | 26.396 | 2.722 | 1.00 | 30.37 | A |
| ATOM | 733 | NH1 | ARG | A | 118 | 6.969 | 25.520 | 3.623 | 1.00 | 31.74 | A |
| ATOM | 734 | NH2 | ARG | A | 118 | 6.504 | 27.060 | 1.993 | 1.00 | 31.96 | A |
| ATOM | 735 | C | ARG | A | 118 | 13.069 | 22.166 | 3.250 | 1.00 | 20.54 | A |
| ATOM | 736 | O | ARG | A | 118 | 14.142 | 22.327 | 2.669 | 1.00 | 22.26 | A |
| ATOM | 737 | N | VAL | A | 119 | 12.990 | 21.580 | 4.439 | 1.00 | 20.19 | A |
| ATOM | 738 | CA | VAL | A | 119 | 14.204 | 21.131 | 5.108 | 1.00 | 19.56 | A |
| ATOM | 739 | CB | VAL | A | 119 | 13.909 | 20.606 | 6.521 | 1.00 | 18.64 | A |
| ATOM | 740 | CG1 | VAL | A | 119 | 13.279 | 21.709 | 7.355 | 1.00 | 18.95 | A |
| ATOM | 741 | CG2 | VAL | A | 119 | 12.999 | 19.414 | 6.456 | 1.00 | 18.96 | A |
| ATOM | 742 | C | VAL | A | 119 | 14.921 | 20.051 | 4.310 | 1.00 | 19.45 | A |
| ATOM | 743 | O | VAL | A | 119 | 16.150 | 20.006 | 4.279 | 1.00 | 20.30 | A |
| ATOM | 744 | N | ALA | A | 120 | 14.156 | 19.194 | 3.644 | 1.00 | 19.68 | A |
| ATOM | 745 | CA | ALA | A | 120 | 14.754 | 18.123 | 2.856 | 1.00 | 18.69 | A |
| ATOM | 746 | CB | ALA | A | 120 | 13.680 | 17.170 | 2.369 | 1.00 | 16.95 | A |
| ATOM | 747 | C | ALA | A | 120 | 15.550 | 18.664 | 1.681 | 1.00 | 18.76 | A |
| ATOM | 748 | O | ALA | A | 120 | 16.612 | 18.145 | 1.353 | 1.00 | 19.04 | A |
| ATOM | 749 | N | GLN | A | 121 | 15.046 | 19.711 | 1.042 | 1.00 | 20.23 | A |
| ATOM | 750 | CA | GLN | A | 121 | 15.760 | 20.282 | -0.095 | 1.00 | 21.94 | A |
| ATOM | 751 | CB | GLN | A | 121 | 14.860 | 21.268 | -0.860 | 1.00 | 24.53 | A |
| ATOM | 752 | CG | GLN | A | 121 | 15.533 | 21.837 | -2.107 | 1.00 | 29.39 | A |
| ATOM | 753 | CD | GLN | A | 121 | 14.754 | 22.966 | -2.747 | 1.00 | 33.32 | A |
| ATOM | 754 | OE1 | GLN | A | 121 | 14.211 | 23.833 | -2.056 | 1.00 | 33.95 | A |
| ATOM | 755 | NE2 | GLN | A | 121 | 14.710 | 22.976 | -4.080 | 1.00 | 34.52 | A |
| ATOM | 756 | C | GLN | A | 121 | 17.051 | 20.985 | 0.371 | 1.00 | 19.87 | A |
| ATOM | 757 | O | GLN | A | 121 | 18.067 | 20.987 | -0.332 | 1.00 | 16.85 | A |
| ATOM | 758 | N | ARG | A | 122 | 17.010 | 21.570 | 1.565 | 1.00 | 18.91 | A |
| ATOM | 759 | CA | ARG | A | 122 | 18.185 | 22.252 | 2.099 | 1.00 | 18.75 | A |
| ATOM | 760 | CB | ARG | A | 122 | 17.793 | 23.137 | 3.290 | 1.00 | 19.05 | A |
| ATOM | 761 | CG | ARG | A | 122 | 18.309 | 24.571 | 3.188 | 1.00 | 20.89 | A |
| ATOM | 762 | CD | ARG | A | 122 | 19.797 | 24.617 | 2.849 | 1.00 | 20.06 | A |
| ATOM | 763 | NE | ARG | A | 122 | 20.540 | 25.353 | 3.863 | 1.00 | 21.80 | A |
| ATOM | 764 | CZ | ARG | A | 122 | 21.843 | 25.209 | 4.100 | 1.00 | 20.76 | A |
| ATOM | 765 | NH1 | ARG | A | 122 | 22.564 | 24.349 | 3.387 | 1.00 | 19.53 | A |
| ATOM | 766 | NH2 | ARG | A | 122 | 22.416 | 25.912 | 5.070 | 1.00 | 18.72 | A |
| ATOM | 767 | C | ARG | A | 122 | 19.281 | 21.262 | 2.527 | 1.00 | 17.94 | A |
| ATOM | 768 | O | ARG | A | 122 | 20.465 | 21.575 | 2.460 | 1.00 | 17.41 | A |

Figure 2 (suite 10)

| | | | | | | | | | | | |
|---|---|---|---|---|---|---|---|---|---|---|---|
| ATOM | 769 | N | ALA | A | 123 | 18.884 | 20.065 | 2.952 | 1.00 | 17.08 | A |
| ATOM | 770 | CA | ALA | A | 123 | 19.844 | 19.057 | 3.389 | 1.00 | 15.78 | A |
| ATOM | 771 | CB | ALA | A | 123 | 19.196 | 18.133 | 4.401 | 1.00 | 15.48 | A |
| ATOM | 772 | C | ALA | A | 123 | 20.397 | 18.235 | 2.247 | 1.00 | 15.22 | A |
| ATOM | 773 | O | ALA | A | 123 | 21.522 | 17.753 | 2.311 | 1.00 | 14.31 | A |
| ATOM | 774 | N | SER | A | 124 | 19.612 | 18.087 | 1.192 | 1.00 | 17.23 | A |
| ATOM | 775 | CA | SER | A | 124 | 20.011 | 17.269 | 0.055 | 1.00 | 19.49 | A |
| ATOM | 776 | CB | SER | A | 124 | 18.910 | 17.286 | -0.986 | 1.00 | 18.94 | A |
| ATOM | 777 | OG | SER | A | 124 | 18.617 | 18.622 | -1.298 | 1.00 | 25.45 | A |
| ATOM | 778 | C | SER | A | 124 | 21.327 | 17.605 | -0.616 | 1.00 | 20.19 | A |
| ATOM | 779 | O | SER | A | 124 | 22.091 | 16.707 | -0.955 | 1.00 | 20.88 | A |
| ATOM | 780 | N | ARG | A | 125 | 21.580 | 18.890 | -0.831 | 1.00 | 21.56 | A |
| ATOM | 781 | CA | ARG | A | 125 | 22.813 | 19.336 | -1.479 | 1.00 | 24.09 | A |
| ATOM | 782 | CB | ARG | A | 125 | 22.915 | 20.862 | -1.417 | 1.00 | 26.41 | A |
| ATOM | 783 | CG | ARG | A | 125 | 21.735 | 21.607 | -2.007 | 0.00 | 25.80 | A |
| ATOM | 784 | CD | ARG | A | 125 | 21.944 | 23.102 | -1.852 | 0.00 | 26.18 | A |
| ATOM | 785 | NE | ARG | A | 125 | 23.211 | 23.526 | -2.441 | 0.00 | 26.24 | A |
| ATOM | 786 | CZ | ARG | A | 125 | 23.688 | 24.764 | -2.381 | 0.00 | 26.33 | A |
| ATOM | 787 | NH1 | ARG | A | 125 | 23.003 | 25.710 | -1.754 | 0.00 | 26.37 | A |
| ATOM | 788 | NH2 | ARG | A | 125 | 24.850 | 25.058 | -2.949 | 0.00 | 26.37 | A |
| ATOM | 789 | C | ARG | A | 125 | 24.073 | 18.737 | -0.843 | 1.00 | 25.17 | A |
| ATOM | 790 | O | ARG | A | 125 | 24.860 | 18.057 | -1.510 | 1.00 | 24.89 | A |
| ATOM | 791 | N | SER | A | 126 | 24.256 | 19.003 | 0.449 | 1.00 | 25.59 | A |
| ATOM | 792 | CA | SER | A | 126 | 25.414 | 18.518 | 1.186 | 1.00 | 25.31 | A |
| ATOM | 793 | CB | SER | A | 126 | 25.366 | 19.041 | 2.622 | 1.00 | 25.34 | A |
| ATOM | 794 | OG | SER | A | 126 | 26.533 | 18.664 | 3.335 | 1.00 | 30.02 | A |
| ATOM | 795 | C | SER | A | 126 | 25.507 | 16.995 | 1.199 | 1.00 | 25.59 | A |
| ATOM | 796 | O | SER | A | 126 | 26.573 | 16.422 | 0.960 | 1.00 | 24.31 | A |
| ATOM | 797 | N | MET | A | 127 | 24.385 | 16.343 | 1.485 | 1.00 | 26.53 | A |
| ATOM | 798 | CA | MET | A | 127 | 24.343 | 14.888 | 1.532 | 1.00 | 26.94 | A |
| ATOM | 799 | CB | MET | A | 127 | 22.935 | 14.418 | 1.876 | 1.00 | 24.10 | A |
| ATOM | 800 | CG | MET | A | 127 | 22.491 | 14.828 | 3.252 | 1.00 | 22.06 | A |
| ATOM | 801 | SD | MET | A | 127 | 20.793 | 14.358 | 3.583 | 1.00 | 22.79 | A |
| ATOM | 802 | CE | MET | A | 127 | 20.695 | 14.710 | 5.318 | 1.00 | 22.56 | A |
| ATOM | 803 | C | MET | A | 127 | 24.782 | 14.268 | 0.215 | 1.00 | 28.77 | A |
| ATOM | 804 | O | MET | A | 127 | 25.502 | 13.268 | 0.198 | 1.00 | 28.73 | A |
| ATOM | 805 | N | GLN | A | 128 | 24.355 | 14.875 | -0.885 | 1.00 | 31.09 | A |
| ATOM | 806 | CA | GLN | A | 128 | 24.685 | 14.378 | -2.208 | 1.00 | 33.19 | A |
| ATOM | 807 | CB | GLN | A | 128 | 23.920 | 15.168 | -3.266 | 1.00 | 35.53 | A |
| ATOM | 808 | CG | GLN | A | 128 | 23.741 | 14.425 | -4.573 | 1.00 | 40.28 | A |
| ATOM | 809 | CD | GLN | A | 128 | 22.899 | 15.202 | -5.571 | 1.00 | 43.73 | A |
| ATOM | 810 | OE1 | GLN | A | 128 | 21.853 | 15.757 | -5.221 | 1.00 | 43.94 | A |
| ATOM | 811 | NE2 | GLN | A | 128 | 23.348 | 15.238 | -6.823 | 1.00 | 44.88 | A |
| ATOM | 812 | C | GLN | A | 128 | 26.179 | 14.453 | -2.487 | 1.00 | 33.66 | A |
| ATOM | 813 | O | GLN | A | 128 | 26.769 | 13.486 | -2.957 | 1.00 | 35.28 | A |
| ATOM | 814 | N | ARG | A | 129 | 26.798 | 15.592 | -2.194 | 1.00 | 34.14 | A |
| ATOM | 815 | CA | ARG | A | 129 | 28.229 | 15.748 | -2.438 | 1.00 | 35.32 | A |
| ATOM | 816 | CB | ARG | A | 129 | 28.677 | 17.191 | -2.181 | 1.00 | 37.93 | A |
| ATOM | 817 | CG | ARG | A | 129 | 28.293 | 18.178 | -3.268 | 1.00 | 42.36 | A |
| ATOM | 818 | CD | ARG | A | 129 | 28.772 | 19.570 | -2.906 | 1.00 | 46.27 | A |
| ATOM | 819 | NE | ARG | A | 129 | 28.441 | 19.894 | -1.520 | 1.00 | 49.78 | A |
| ATOM | 820 | CZ | ARG | A | 129 | 28.655 | 21.077 | -0.952 | 1.00 | 51.75 | A |
| ATOM | 821 | NH1 | ARG | A | 129 | 28.324 | 21.272 | 0.321 | 1.00 | 52.27 | A |
| ATOM | 822 | NH2 | ARG | A | 129 | 29.190 | 22.066 | -1.659 | 1.00 | 53.22 | A |
| ATOM | 823 | C | ARG | A | 129 | 29.089 | 14.816 | -1.598 | 1.00 | 33.84 | A |
| ATOM | 824 | O | ARG | A | 129 | 30.079 | 14.274 | -2.092 | 1.00 | 34.50 | A |
| ATOM | 825 | N | ASN | A | 130 | 28.726 | 14.635 | -0.333 | 1.00 | 31.20 | A |
| ATOM | 826 | CA | ASN | A | 130 | 29.498 | 13.764 | 0.545 | 1.00 | 30.14 | A |
| ATOM | 827 | CB | ASN | A | 130 | 29.305 | 14.182 | 2.000 | 1.00 | 31.54 | A |
| ATOM | 828 | CG | ASN | A | 130 | 29.774 | 15.594 | 2.257 | 1.00 | 33.96 | A |
| ATOM | 829 | OD1 | ASN | A | 130 | 30.902 | 15.942 | 1.920 | 1.00 | 36.05 | A |
| ATOM | 830 | ND2 | ASN | A | 130 | 28.912 | 16.419 | 2.850 | 1.00 | 35.36 | A |
| ATOM | 831 | C | ASN | A | 130 | 29.112 | 12.303 | 0.370 | 1.00 | 28.67 | A |
| ATOM | 832 | O | ASN | A | 130 | 29.583 | 11.431 | 1.098 | 1.00 | 28.09 | A |
| ATOM | 833 | N | LYS | A | 131 | 28.261 | 12.044 | -0.615 | 1.00 | 27.98 | A |
| ATOM | 834 | CA | LYS | A | 131 | 27.791 | 10.699 | -0.891 | 1.00 | 26.63 | A |
| ATOM | 835 | CB | LYS | A | 131 | 28.878 | 9.879 | -1.586 | 1.00 | 28.68 | A |
| ATOM | 836 | CG | LYS | A | 131 | 28.888 | 10.026 | -3.104 | 1.00 | 31.33 | A |
| ATOM | 837 | CD | LYS | A | 131 | 29.008 | 11.481 | -3.541 | 1.00 | 33.33 | A |
| ATOM | 838 | CE | LYS | A | 131 | 29.041 | 11.591 | -5.053 | 1.00 | 34.77 | A |
| ATOM | 839 | NZ | LYS | A | 131 | 30.188 | 10.824 | -5.622 | 1.00 | 38.14 | A |
| ATOM | 840 | C | LYS | A | 131 | 27.297 | 9.971 | 0.354 | 1.00 | 25.13 | A |
| ATOM | 841 | O | LYS | A | 131 | 27.597 | 8.794 | 0.556 | 1.00 | 26.37 | A |

Figure 2 (suite 11)

| | | | | | | | | | | | |
|---|---|---|---|---|---|---|---|---|---|---|---|
| ATOM | 842 | N | PHE | A | 132 | 26.555 | 10.685 | 1.193 | 1.00 | 20.64 | A |
| ATOM | 843 | CA | PHE | A | 132 | 25.962 | 10.097 | 2.387 | 1.00 | 19.01 | A |
| ATOM | 844 | CB | PHE | A | 132 | 26.964 | 9.949 | 3.533 | 1.00 | 14.67 | A |
| ATOM | 845 | CG | PHE | A | 132 | 26.365 | 9.312 | 4.758 | 1.00 | 10.73 | A |
| ATOM | 846 | CD1 | PHE | A | 132 | 26.279 | 7.926 | 4.865 | 1.00 | 8.33 | A |
| ATOM | 847 | CD2 | PHE | A | 132 | 25.800 | 10.094 | 5.757 | 1.00 | 8.90 | A |
| ATOM | 848 | CE1 | PHE | A | 132 | 25.628 | 7.323 | 5.952 | 1.00 | 6.87 | A |
| ATOM | 849 | CE2 | PHE | A | 132 | 25.147 | 9.505 | 6.847 | 1.00 | 8.63 | A |
| ATOM | 850 | CZ | PHE | A | 132 | 25.060 | 8.109 | 6.941 | 1.00 | 7.39 | A |
| ATOM | 851 | C | PHE | A | 132 | 24.791 | 10.934 | 2.888 | 1.00 | 19.73 | A |
| ATOM | 852 | O | PHE | A | 132 | 24.840 | 12.164 | 2.878 | 1.00 | 19.80 | A |
| ATOM | 853 | N | GLY | A | 133 | 23.744 | 10.260 | 3.345 | 1.00 | 19.58 | A |
| ATOM | 854 | CA | GLY | A | 133 | 22.595 | 10.978 | 3.851 | 1.00 | 19.60 | A |
| ATOM | 855 | C | GLY | A | 133 | 21.487 | 10.064 | 4.324 | 1.00 | 20.52 | A |
| ATOM | 856 | O | GLY | A | 133 | 21.261 | 8.988 | 3.769 | 1.00 | 21.57 | A |
| ATOM | 857 | N | ARG | A | 134 | 20.793 | 10.497 | 5.365 | 1.00 | 19.64 | A |
| ATOM | 858 | CA | ARG | A | 134 | 19.689 | 9.739 | 5.910 | 1.00 | 19.97 | A |
| ATOM | 859 | CB | ARG | A | 134 | 20.124 | 8.981 | 7.160 | 1.00 | 19.40 | A |
| ATOM | 860 | CG | ARG | A | 134 | 21.132 | 7.892 | 6.899 | 1.00 | 21.81 | A |
| ATOM | 861 | CD | ARG | A | 134 | 20.477 | 6.574 | 6.547 | 1.00 | 24.08 | A |
| ATOM | 862 | NE | ARG | A | 134 | 21.415 | 5.465 | 6.704 | 1.00 | 25.86 | A |
| ATOM | 863 | CZ | ARG | A | 134 | 22.377 | 5.159 | 5.839 | 1.00 | 27.86 | A |
| ATOM | 864 | NH1 | ARG | A | 134 | 22.536 | 5.875 | 4.729 | 1.00 | 27.76 | A |
| ATOM | 865 | NH2 | ARG | A | 134 | 23.195 | 4.143 | 6.098 | 1.00 | 28.42 | A |
| ATOM | 866 | C | ARG | A | 134 | 18.567 | 10.698 | 6.265 | 1.00 | 20.18 | A |
| ATOM | 867 | O | ARG | A | 134 | 18.643 | 11.414 | 7.264 | 1.00 | 22.02 | A |
| ATOM | 868 | N | MET | A | 135 | 17.532 | 10.722 | 5.433 | 1.00 | 20.30 | A |
| ATOM | 869 | CA | MET | A | 135 | 16.381 | 11.577 | 5.676 | 1.00 | 18.99 | A |
| ATOM | 870 | CB | MET | A | 135 | 15.902 | 12.222 | 4.379 | 1.00 | 20.44 | A |
| ATOM | 871 | CG | MET | A | 135 | 16.808 | 13.327 | 3.881 | 1.00 | 24.17 | A |
| ATOM | 872 | SD | MET | A | 135 | 16.165 | 14.177 | 2.429 | 1.00 | 28.97 | A |
| ATOM | 873 | CE | MET | A | 135 | 17.611 | 14.158 | 1.352 | 1.00 | 28.83 | A |
| ATOM | 874 | C | MET | A | 135 | 15.293 | 10.698 | 6.248 | 1.00 | 18.69 | A |
| ATOM | 875 | O | MET | A | 135 | 14.865 | 9.743 | 5.607 | 1.00 | 20.22 | A |
| ATOM | 876 | N | ILE | A | 136 | 14.858 | 11.019 | 7.461 | 1.00 | 16.75 | A |
| ATOM | 877 | CA | ILE | A | 136 | 13.829 | 10.258 | 8.137 | 1.00 | 13.34 | A |
| ATOM | 878 | CB | ILE | A | 136 | 14.389 | 9.641 | 9.436 | 1.00 | 12.60 | A |
| ATOM | 879 | CG2 | ILE | A | 136 | 13.319 | 8.833 | 10.147 | 1.00 | 9.67 | A |
| ATOM | 880 | CG1 | ILE | A | 136 | 15.598 | 8.764 | 9.102 | 1.00 | 10.24 | A |
| ATOM | 881 | CD | ILE | A | 136 | 16.285 | 8.179 | 10.315 | 1.00 | 9.43 | A |
| ATOM | 882 | C | ILE | A | 136 | 12.658 | 11.174 | 8.480 | 1.00 | 14.33 | A |
| ATOM | 883 | O | ILE | A | 136 | 12.816 | 12.151 | 9.205 | 1.00 | 15.22 | A |
| ATOM | 884 | N | PHE | A | 137 | 11.474 | 10.860 | 7.960 | 1.00 | 14.53 | A |
| ATOM | 885 | CA | PHE | A | 137 | 10.300 | 11.674 | 8.247 | 1.00 | 12.73 | A |
| ATOM | 886 | CB | PHE | A | 137 | 9.543 | 11.990 | 6.956 | 1.00 | 9.16 | A |
| ATOM | 887 | CG | PHE | A | 137 | 10.387 | 12.623 | 5.901 | 1.00 | 7.83 | A |
| ATOM | 888 | CD1 | PHE | A | 137 | 11.143 | 11.848 | 5.038 | 1.00 | 8.30 | A |
| ATOM | 889 | CD2 | PHE | A | 137 | 10.440 | 14.002 | 5.771 | 1.00 | 7.36 | A |
| ATOM | 890 | CE1 | PHE | A | 137 | 11.936 | 12.440 | 4.054 | 1.00 | 8.23 | A |
| ATOM | 891 | CE2 | PHE | A | 137 | 11.226 | 14.594 | 4.799 | 1.00 | 5.85 | A |
| ATOM | 892 | CZ | PHE | A | 137 | 11.977 | 13.810 | 3.939 | 1.00 | 6.45 | A |
| ATOM | 893 | C | PHE | A | 137 | 9.383 | 10.934 | 9.216 | 1.00 | 13.22 | A |
| ATOM | 894 | O | PHE | A | 137 | 9.149 | 9.741 | 9.066 | 1.00 | 14.22 | A |
| ATOM | 895 | N | ILE | A | 138 | 8.878 | 11.638 | 10.219 | 1.00 | 13.04 | A |
| ATOM | 896 | CA | ILE | A | 138 | 7.983 | 11.027 | 11.182 | 1.00 | 14.87 | A |
| ATOM | 897 | CB | ILE | A | 138 | 8.337 | 11.426 | 12.649 | 1.00 | 14.74 | A |
| ATOM | 898 | CG2 | ILE | A | 138 | 7.189 | 11.054 | 13.588 | 1.00 | 13.97 | A |
| ATOM | 899 | CG1 | ILE | A | 138 | 9.595 | 10.697 | 13.121 | 1.00 | 12.18 | A |
| ATOM | 900 | CD | ILE | A | 138 | 10.823 | 11.123 | 12.443 | 1.00 | 15.15 | A |
| ATOM | 901 | C | ILE | A | 138 | 6.558 | 11.477 | 10.893 | 1.00 | 17.05 | A |
| ATOM | 902 | O | ILE | A | 138 | 6.267 | 12.675 | 10.877 | 1.00 | 17.13 | A |
| ATOM | 903 | N | GLY | A | 139 | 5.668 | 10.524 | 10.646 | 1.00 | 18.55 | A |
| ATOM | 904 | CA | GLY | A | 139 | 4.292 | 10.890 | 10.402 | 1.00 | 20.69 | A |
| ATOM | 905 | C | GLY | A | 139 | 3.749 | 11.426 | 11.713 | 1.00 | 23.13 | A |
| ATOM | 906 | O | GLY | A | 139 | 4.346 | 11.206 | 12.773 | 1.00 | 24.66 | A |
| ATOM | 907 | N | SER | A | 140 | 2.631 | 12.137 | 11.653 | 1.00 | 23.36 | A |
| ATOM | 908 | CA | SER | A | 140 | 2.020 | 12.682 | 12.852 | 1.00 | 25.95 | A |
| ATOM | 909 | CB | SER | A | 140 | 1.079 | 13.828 | 12.471 | 1.00 | 27.12 | A |
| ATOM | 910 | OG | SER | A | 140 | 0.505 | 14.411 | 13.629 | 1.00 | 32.70 | A |
| ATOM | 911 | C | SER | A | 140 | 1.241 | 11.593 | 13.610 | 1.00 | 26.56 | A |
| ATOM | 912 | O | SER | A | 140 | 0.880 | 10.560 | 13.040 | 1.00 | 25.96 | A |
| ATOM | 913 | N | VAL | A | 141 | 0.979 | 11.827 | 14.893 | 1.00 | 27.29 | A |
| ATOM | 914 | CA | VAL | A | 141 | 0.240 | 10.862 | 15.706 | 1.00 | 27.62 | A |

Figure 2 (suite 12)

| | | | | | | | | | | | |
|---|---|---|---|---|---|---|---|---|---|---|---|
| ATOM | 915 | CB | VAL | A | 141 | 0.447 | 11.118 | 17.209 | 1.00 | 26.45 | A |
| ATOM | 916 | CG1 | VAL | A | 141 | -0.452 | 10.191 | 18.023 | 1.00 | 26.29 | A |
| ATOM | 917 | CG2 | VAL | A | 141 | 1.913 | 10.908 | 17.575 | 1.00 | 25.56 | A |
| ATOM | 918 | C | VAL | A | 141 | -1.262 | 10.906 | 15.421 | 1.00 | 28.95 | A |
| ATOM | 919 | O | VAL | A | 141 | -1.694 | 11.319 | 14.343 | 1.00 | 30.54 | A |
| ATOM | 920 | N | GLN | A | 150 | -10.246 | 15.356 | 9.762 | 1.00 | 38.28 | A |
| ATOM | 921 | CA | GLN | A | 150 | -8.849 | 14.957 | 9.772 | 1.00 | 36.55 | A |
| ATOM | 922 | CB | GLN | A | 150 | -8.689 | 13.670 | 10.585 | 1.00 | 38.06 | A |
| ATOM | 923 | CG | GLN | A | 150 | -9.623 | 12.547 | 10.165 | 1.00 | 40.32 | A |
| ATOM | 924 | CD | GLN | A | 150 | -8.916 | 11.422 | 9.418 | 1.00 | 42.57 | A |
| ATOM | 925 | OE1 | GLN | A | 150 | -9.564 | 10.494 | 8.922 | 1.00 | 43.88 | A |
| ATOM | 926 | NE2 | GLN | A | 150 | -7.586 | 11.493 | 9.341 | 1.00 | 41.24 | A |
| ATOM | 927 | C | GLN | A | 150 | -8.319 | 14.756 | 8.356 | 1.00 | 35.16 | A |
| ATOM | 928 | O | GLN | A | 150 | -7.344 | 14.043 | 8.154 | 1.00 | 36.86 | A |
| ATOM | 929 | N | ALA | A | 151 | -8.948 | 15.395 | 7.375 | 1.00 | 32.57 | A |
| ATOM | 930 | CA | ALA | A | 151 | -8.517 | 15.255 | 5.988 | 1.00 | 30.34 | A |
| ATOM | 931 | CB | ALA | A | 151 | -9.603 | 15.773 | 5.044 | 1.00 | 29.55 | A |
| ATOM | 932 | C | ALA | A | 151 | -7.198 | 15.972 | 5.710 | 1.00 | 29.06 | A |
| ATOM | 933 | O | ALA | A | 151 | -6.449 | 15.590 | 4.805 | 1.00 | 28.46 | A |
| ATOM | 934 | N | ASN | A | 152 | -6.918 | 17.019 | 6.474 | 1.00 | 27.34 | A |
| ATOM | 935 | CA | ASN | A | 152 | -5.684 | 17.766 | 6.278 | 1.00 | 26.26 | A |
| ATOM | 936 | CB | ASN | A | 152 | -5.754 | 19.118 | 7.006 | 1.00 | 25.51 | A |
| ATOM | 937 | CG | ASN | A | 152 | -6.411 | 19.022 | 8.370 | 1.00 | 23.66 | A |
| ATOM | 938 | OD1 | ASN | A | 152 | -6.590 | 20.027 | 9.045 | 1.00 | 23.90 | A |
| ATOM | 939 | ND2 | ASN | A | 152 | -6.778 | 17.816 | 8.778 | 1.00 | 21.72 | A |
| ATOM | 940 | C | ASN | A | 152 | -4.466 | 16.960 | 6.734 | 1.00 | 26.44 | A |
| ATOM | 941 | O | ASN | A | 152 | -3.462 | 16.873 | 6.020 | 1.00 | 26.33 | A |
| ATOM | 942 | N | TYR | A | 153 | -4.555 | 16.354 | 7.912 | 1.00 | 25.09 | A |
| ATOM | 943 | CA | TYR | A | 153 | -3.443 | 15.562 | 8.404 | 1.00 | 25.23 | A |
| ATOM | 944 | CB | TYR | A | 153 | -3.666 | 15.187 | 9.868 | 1.00 | 26.67 | A |
| ATOM | 945 | CG | TYR | A | 153 | -3.538 | 16.374 | 10.794 | 1.00 | 28.38 | A |
| ATOM | 946 | CD1 | TYR | A | 153 | -4.664 | 16.967 | 11.363 | 1.00 | 29.05 | A |
| ATOM | 947 | CE1 | TYR | A | 153 | -4.550 | 18.088 | 12.185 | 1.00 | 30.91 | A |
| ATOM | 948 | CD2 | TYR | A | 153 | -2.288 | 16.931 | 11.072 | 1.00 | 29.84 | A |
| ATOM | 949 | CE2 | TYR | A | 153 | -2.164 | 18.056 | 11.892 | 1.00 | 30.91 | A |
| ATOM | 950 | CZ | TYR | A | 153 | -3.299 | 18.626 | 12.442 | 1.00 | 31.13 | A |
| ATOM | 951 | OH | TYR | A | 153 | -3.182 | 19.735 | 13.244 | 1.00 | 33.21 | A |
| ATOM | 952 | C | TYR | A | 153 | -3.269 | 14.315 | 7.542 | 1.00 | 24.27 | A |
| ATOM | 953 | O | TYR | A | 153 | -2.140 | 13.930 | 7.205 | 1.00 | 23.57 | A |
| ATOM | 954 | N | ALA | A | 154 | -4.390 | 13.695 | 7.176 | 1.00 | 22.07 | A |
| ATOM | 955 | CA | ALA | A | 154 | -4.363 | 12.506 | 6.333 | 1.00 | 20.00 | A |
| ATOM | 956 | CB | ALA | A | 154 | -5.771 | 12.025 | 6.059 | 1.00 | 19.51 | A |
| ATOM | 957 | C | ALA | A | 154 | -3.656 | 12.808 | 5.020 | 1.00 | 18.98 | A |
| ATOM | 958 | O | ALA | A | 154 | -2.791 | 12.051 | 4.583 | 1.00 | 20.09 | A |
| ATOM | 959 | N | ALA | A | 155 | -4.011 | 13.927 | 4.403 | 1.00 | 16.54 | A |
| ATOM | 960 | CA | ALA | A | 155 | -3.415 | 14.313 | 3.132 | 1.00 | 15.93 | A |
| ATOM | 961 | CB | ALA | A | 155 | -4.150 | 15.527 | 2.558 | 1.00 | 15.61 | A |
| ATOM | 962 | C | ALA | A | 155 | -1.929 | 14.613 | 3.275 | 1.00 | 15.88 | A |
| ATOM | 963 | O | ALA | A | 155 | -1.101 | 14.153 | 2.473 | 1.00 | 14.43 | A |
| ATOM | 964 | N | SER | A | 156 | -1.600 | 15.395 | 4.298 | 1.00 | 15.99 | A |
| ATOM | 965 | CA | SER | A | 156 | -0.214 | 15.758 | 4.578 | 1.00 | 15.93 | A |
| ATOM | 966 | CB | SER | A | 156 | -0.149 | 16.681 | 5.801 | 1.00 | 17.69 | A |
| ATOM | 967 | OG | SER | A | 156 | -0.826 | 16.099 | 6.907 | 1.00 | 21.33 | A |
| ATOM | 968 | C | SER | A | 156 | 0.605 | 14.494 | 4.823 | 1.00 | 13.65 | A |
| ATOM | 969 | O | SER | A | 156 | 1.655 | 14.296 | 4.202 | 1.00 | 13.33 | A |
| ATOM | 970 | N | LYS | A | 157 | 0.114 | 13.628 | 5.704 | 1.00 | 12.29 | A |
| ATOM | 971 | CA | LYS | A | 157 | 0.827 | 12.388 | 5.998 | 1.00 | 14.11 | A |
| ATOM | 972 | CB | LYS | A | 157 | 0.104 | 11.582 | 7.080 | 1.00 | 15.50 | A |
| ATOM | 973 | CG | LYS | A | 157 | 0.789 | 10.270 | 7.378 | 1.00 | 19.65 | A |
| ATOM | 974 | CD | LYS | A | 157 | -0.006 | 9.404 | 8.358 | 1.00 | 24.86 | A |
| ATOM | 975 | CE | LYS | A | 157 | 0.653 | 8.017 | 8.481 | 1.00 | 25.87 | A |
| ATOM | 976 | NZ | LYS | A | 157 | -0.056 | 7.129 | 9.456 | 1.00 | 29.60 | A |
| ATOM | 977 | C | LYS | A | 157 | 1.037 | 11.513 | 4.761 | 1.00 | 11.73 | A |
| ATOM | 978 | O | LYS | A | 157 | 2.123 | 10.974 | 4.552 | 1.00 | 12.86 | A |
| ATOM | 979 | N | ALA | A | 158 | 0.007 | 11.376 | 3.936 | 1.00 | 8.47 | A |
| ATOM | 980 | CA | ALA | A | 158 | 0.132 | 10.564 | 2.737 | 1.00 | 6.47 | A |
| ATOM | 981 | CB | ALA | A | 158 | -1.234 | 10.312 | 2.132 | 1.00 | 7.13 | A |
| ATOM | 982 | C | ALA | A | 158 | 1.021 | 11.287 | 1.751 | 1.00 | 7.04 | A |
| ATOM | 983 | O | ALA | A | 158 | 1.701 | 10.669 | 0.927 | 1.00 | 5.70 | A |
| ATOM | 984 | N | GLY | A | 159 | 1.021 | 12.612 | 1.852 | 1.00 | 7.47 | A |
| ATOM | 985 | CA | GLY | A | 159 | 1.840 | 13.415 | 0.969 | 1.00 | 7.76 | A |
| ATOM | 986 | C | GLY | A | 159 | 3.326 | 13.258 | 1.229 | 1.00 | 8.87 | A |
| ATOM | 987 | O | GLY | A | 159 | 4.113 | 13.135 | 0.286 | 1.00 | 8.49 | A |

Figure 2 (suite 13)

| | | | | | | | | | | | |
|---|---|---|---|---|---|---|---|---|---|---|---|
| ATOM | 988 | N | VAL | A | 160 | 3.723 | 13.268 | 2.503 | 1.00 | 10.15 | A |
| ATOM | 989 | CA | VAL | A | 160 | 5.141 | 13.118 | 2.846 | 1.00 | 8.69 | A |
| ATOM | 990 | CB | VAL | A | 160 | 5.371 | 13.221 | 4.368 | 1.00 | 6.61 | A |
| ATOM | 991 | CG1 | VAL | A | 160 | 6.858 | 13.220 | 4.664 | 1.00 | 4.46 | A |
| ATOM | 992 | CG2 | VAL | A | 160 | 4.735 | 14.482 | 4.909 | 1.00 | 4.90 | A |
| ATOM | 993 | C | VAL | A | 160 | 5.667 | 11.766 | 2.339 | 1.00 | 10.02 | A |
| ATOM | 994 | O | VAL | A | 160 | 6.752 | 11.690 | 1.747 | 1.00 | 9.07 | A |
| ATOM | 995 | N | ILE | A | 161 | 4.891 | 10.703 | 2.561 | 1.00 | 10.31 | A |
| ATOM | 996 | CA | ILE | A | 161 | 5.289 | 9.375 | 2.097 | 1.00 | 11.05 | A |
| ATOM | 997 | CB | ILE | A | 161 | 4.193 | 8.321 | 2.381 | 1.00 | 9.98 | A |
| ATOM | 998 | CG2 | ILE | A | 161 | 4.450 | 7.055 | 1.575 | 1.00 | 7.38 | A |
| ATOM | 999 | CG1 | ILE | A | 161 | 4.152 | 8.016 | 3.876 | 1.00 | 7.73 | A |
| ATOM | 1000 | CD | ILE | A | 161 | 2.999 | 7.165 | 4.269 | 1.00 | 7.69 | A |
| ATOM | 1001 | C | ILE | A | 161 | 5.550 | 9.433 | 0.600 | 1.00 | 13.32 | A |
| ATOM | 1002 | O | ILE | A | 161 | 6.578 | 8.956 | 0.126 | 1.00 | 13.88 | A |
| ATOM | 1003 | N | GLY | A | 162 | 4.622 | 10.028 | -0.145 | 1.00 | 15.87 | A |
| ATOM | 1004 | CA | GLY | A | 162 | 4.812 | 10.139 | -1.580 | 1.00 | 16.58 | A |
| ATOM | 1005 | C | GLY | A | 162 | 6.083 | 10.912 | -1.893 | 1.00 | 17.83 | A |
| ATOM | 1006 | O | GLY | A | 162 | 6.817 | 10.584 | -2.831 | 1.00 | 17.56 | A |
| ATOM | 1007 | N | MET | A | 163 | 6.346 | 11.943 | -1.094 | 1.00 | 17.55 | A |
| ATOM | 1008 | CA | MET | A | 163 | 7.535 | 12.763 | -1.283 | 1.00 | 17.89 | A |
| ATOM | 1009 | CB | MET | A | 163 | 7.491 | 13.990 | -0.380 | 1.00 | 19.84 | A |
| ATOM | 1010 | CG | MET | A | 163 | 8.469 | 15.061 | -0.818 | 1.00 | 23.15 | A |
| ATOM | 1011 | SD | MET | A | 163 | 8.902 | 16.184 | 0.491 | 1.00 | 28.77 | A |
| ATOM | 1012 | CE | MET | A | 163 | 7.313 | 16.903 | 0.857 | 1.00 | 28.55 | A |
| ATOM | 1013 | C | MET | A | 163 | 8.810 | 11.974 | -0.992 | 1.00 | 16.73 | A |
| ATOM | 1014 | O | MET | A | 163 | 9.773 | 12.028 | -1.762 | 1.00 | 15.03 | A |
| ATOM | 1015 | N | ALA | A | 164 | 8.813 | 11.241 | 0.118 | 1.00 | 16.07 | A |
| ATOM | 1016 | CA | ALA | A | 164 | 9.978 | 10.445 | 0.490 | 1.00 | 17.25 | A |
| ATOM | 1017 | CB | ALA | A | 164 | 9.734 | 9.747 | 1.821 | 1.00 | 16.56 | A |
| ATOM | 1018 | C | ALA | A | 164 | 10.323 | 9.420 | -0.600 | 1.00 | 17.74 | A |
| ATOM | 1019 | O | ALA | A | 164 | 11.467 | 9.355 | -1.064 | 1.00 | 16.05 | A |
| ATOM | 1020 | N | ARG | A | 165 | 9.332 | 8.628 | -1.008 | 1.00 | 18.89 | A |
| ATOM | 1021 | CA | ARG | A | 165 | 9.533 | 7.622 | -2.054 | 1.00 | 20.19 | A |
| ATOM | 1022 | CB | ARG | A | 165 | 8.193 | 7.018 | -2.473 | 1.00 | 21.70 | A |
| ATOM | 1023 | CG | ARG | A | 165 | 7.454 | 6.252 | -1.378 | 1.00 | 25.05 | A |
| ATOM | 1024 | CD | ARG | A | 165 | 8.053 | 4.885 | -1.176 | 1.00 | 29.58 | A |
| ATOM | 1025 | NE | ARG | A | 165 | 7.267 | 4.070 | -0.255 | 1.00 | 32.91 | A |
| ATOM | 1026 | CZ | ARG | A | 165 | 6.061 | 3.584 | -0.526 | 1.00 | 34.40 | A |
| ATOM | 1027 | NH1 | ARG | A | 165 | 5.494 | 3.827 | -1.701 | 1.00 | 36.19 | A |
| ATOM | 1028 | NH2 | ARG | A | 165 | 5.418 | 2.860 | 0.382 | 1.00 | 35.14 | A |
| ATOM | 1029 | C | ARG | A | 165 | 10.202 | 8.259 | -3.273 | 1.00 | 20.36 | A |
| ATOM | 1030 | O | ARG | A | 165 | 11.085 | 7.660 | -3.889 | 1.00 | 19.91 | A |
| ATOM | 1031 | N | SER | A | 166 | 9.775 | 9.475 | -3.616 | 1.00 | 20.28 | A |
| ATOM | 1032 | CA | SER | A | 166 | 10.340 | 10.191 | -4.753 | 1.00 | 20.96 | A |
| ATOM | 1033 | CB | SER | A | 166 | 9.568 | 11.495 | -4.995 | 1.00 | 23.91 | A |
| ATOM | 1034 | OG | SER | A | 166 | 10.030 | 12.165 | -6.161 | 1.00 | 24.04 | A |
| ATOM | 1035 | C | SER | A | 166 | 11.817 | 10.488 | -4.484 | 1.00 | 21.97 | A |
| ATOM | 1036 | O | SER | A | 166 | 12.680 | 10.169 | -5.304 | 1.00 | 22.64 | A |
| ATOM | 1037 | N | ILE | A | 167 | 12.106 | 11.098 | -3.335 | 1.00 | 22.19 | A |
| ATOM | 1038 | CA | ILE | A | 167 | 13.484 | 11.407 | -2.959 | 1.00 | 20.45 | A |
| ATOM | 1039 | CB | ILE | A | 167 | 13.607 | 11.945 | -1.503 | 1.00 | 20.32 | A |
| ATOM | 1040 | CG2 | ILE | A | 167 | 15.051 | 12.342 | -1.244 | 1.00 | 19.41 | A |
| ATOM | 1041 | CG1 | ILE | A | 167 | 12.700 | 13.157 | -1.260 | 1.00 | 19.60 | A |
| ATOM | 1042 | CD | ILE | A | 167 | 13.076 | 14.383 | -2.046 | 1.00 | 21.78 | A |
| ATOM | 1043 | C | ILE | A | 167 | 14.323 | 10.124 | -3.015 | 1.00 | 20.64 | A |
| ATOM | 1044 | O | ILE | A | 167 | 15.426 | 10.111 | -3.570 | 1.00 | 21.10 | A |
| ATOM | 1045 | N | ALA | A | 168 | 13.794 | 9.049 | -2.430 | 1.00 | 20.17 | A |
| ATOM | 1046 | CA | ALA | A | 168 | 14.494 | 7.764 | -2.386 | 1.00 | 19.91 | A |
| ATOM | 1047 | CB | ALA | A | 168 | 13.634 | 6.721 | -1.698 | 1.00 | 17.44 | A |
| ATOM | 1048 | C | ALA | A | 168 | 14.864 | 7.283 | -3.771 | 1.00 | 21.14 | A |
| ATOM | 1049 | O | ALA | A | 168 | 15.967 | 6.791 | -3.998 | 1.00 | 19.07 | A |
| ATOM | 1050 | N | ARG | A | 169 | 13.932 | 7.450 | -4.702 | 1.00 | 23.96 | A |
| ATOM | 1051 | CA | ARG | A | 169 | 14.128 | 7.014 | -6.078 | 1.00 | 26.56 | A |
| ATOM | 1052 | CB | ARG | A | 169 | 12.786 | 7.078 | -6.821 | 1.00 | 29.24 | A |
| ATOM | 1053 | CG | ARG | A | 169 | 12.787 | 6.483 | -8.220 | 1.00 | 34.66 | A |
| ATOM | 1054 | CD | ARG | A | 169 | 11.387 | 6.524 | -8.845 | 1.00 | 40.11 | A |
| ATOM | 1055 | NE | ARG | A | 169 | 10.391 | 5.784 | -8.061 | 1.00 | 43.67 | A |
| ATOM | 1056 | CZ | ARG | A | 169 | 9.435 | 6.346 | -7.319 | 1.00 | 46.07 | A |
| ATOM | 1057 | NH1 | ARG | A | 169 | 9.329 | 7.668 | -7.252 | 1.00 | 45.87 | A |
| ATOM | 1058 | NH2 | ARG | A | 169 | 8.578 | 5.584 | -6.641 | 1.00 | 45.55 | A |
| ATOM | 1059 | C | ARG | A | 169 | 15.187 | 7.829 | -6.816 | 1.00 | 26.22 | A |
| ATOM | 1060 | O | ARG | A | 169 | 15.830 | 7.334 | -7.739 | 1.00 | 27.13 | A |

Figure 2 (suite 14)

| | | | | | | | | | | | |
|---|---|---|---|---|---|---|---|---|---|---|---|
| ATOM | 1061 | N | GLU | A | 170 | 15.380 | 9.076 | -6.405 | 1.00 | 26.21 | A |
| ATOM | 1062 | CA | GLU | A | 170 | 16.350 | 9.932 | -7.072 | 1.00 | 26.01 | A |
| ATOM | 1063 | CB | GLU | A | 170 | 15.807 | 11.364 | -7.149 | 1.00 | 25.40 | A |
| ATOM | 1064 | CG | GLU | A | 170 | 16.677 | 12.312 | -7.955 | 0.00 | 25.67 | A |
| ATOM | 1065 | CD | GLU | A | 170 | 16.848 | 11.860 | -9.391 | 0.00 | 25.63 | A |
| ATOM | 1066 | OE1 | GLU | A | 170 | 15.826 | 11.728 | -10.098 | 0.00 | 25.65 | A |
| ATOM | 1067 | OE2 | GLU | A | 170 | 18.001 | 11.634 | -9.813 | 0.00 | 25.65 | A |
| ATOM | 1068 | C | GLU | A | 170 | 17.754 | 9.960 | -6.457 | 1.00 | 26.91 | A |
| ATOM | 1069 | O | GLU | A | 170 | 18.748 | 9.905 | -7.183 | 1.00 | 27.93 | A |
| ATOM | 1070 | N | LEU | A | 171 | 17.841 | 10.025 | -5.129 | 1.00 | 26.01 | A |
| ATOM | 1071 | CA | LEU | A | 171 | 19.140 | 10.122 | -4.464 | 1.00 | 25.52 | A |
| ATOM | 1072 | CB | LEU | A | 171 | 19.042 | 11.114 | -3.295 | 1.00 | 24.70 | A |
| ATOM | 1073 | CG | LEU | A | 171 | 18.536 | 12.512 | -3.655 | 1.00 | 23.06 | A |
| ATOM | 1074 | CD1 | LEU | A | 171 | 18.576 | 13.406 | -2.436 | 1.00 | 22.25 | A |
| ATOM | 1075 | CD2 | LEU | A | 171 | 19.395 | 13.088 | -4.766 | 1.00 | 23.40 | A |
| ATOM | 1076 | C | LEU | A | 171 | 19.823 | 8.841 | -3.973 | 1.00 | 25.56 | A |
| ATOM | 1077 | O | LEU | A | 171 | 21.019 | 8.862 | -3.687 | 1.00 | 25.70 | A |
| ATOM | 1078 | N | SER | A | 172 | 19.087 | 7.738 | -3.870 | 1.00 | 24.68 | A |
| ATOM | 1079 | CA | SER | A | 172 | 19.680 | 6.487 | -3.406 | 1.00 | 24.41 | A |
| ATOM | 1080 | CB | SER | A | 172 | 18.670 | 5.339 | -3.506 | 1.00 | 22.49 | A |
| ATOM | 1081 | OG | SER | A | 172 | 17.697 | 5.428 | -2.476 | 1.00 | 19.70 | A |
| ATOM | 1082 | C | SER | A | 172 | 20.962 | 6.110 | -4.147 | 1.00 | 26.22 | A |
| ATOM | 1083 | O | SER | A | 172 | 21.812 | 5.416 | -3.598 | 1.00 | 27.23 | A |
| ATOM | 1084 | N | LYS | A | 173 | 21.119 | 6.562 | -5.385 | 1.00 | 27.79 | A |
| ATOM | 1085 | CA | LYS | A | 173 | 22.333 | 6.234 | -6.128 | 1.00 | 28.30 | A |
| ATOM | 1086 | CB | LYS | A | 173 | 22.196 | 6.627 | -7.604 | 1.00 | 29.63 | A |
| ATOM | 1087 | CG | LYS | A | 173 | 22.018 | 8.119 | -7.855 | 1.00 | 31.72 | A |
| ATOM | 1088 | CD | LYS | A | 173 | 21.985 | 8.412 | -9.355 | 1.00 | 32.38 | A |
| ATOM | 1089 | CE | LYS | A | 173 | 20.673 | 9.070 | -9.769 | 1.00 | 34.40 | A |
| ATOM | 1090 | NZ | LYS | A | 173 | 19.490 | 8.182 | -9.536 | 1.00 | 34.56 | A |
| ATOM | 1091 | C | LYS | A | 173 | 23.518 | 6.962 | -5.502 | 1.00 | 27.99 | A |
| ATOM | 1092 | O | LYS | A | 173 | 24.658 | 6.512 | -5.593 | 1.00 | 27.84 | A |
| ATOM | 1093 | N | ALA | A | 174 | 23.235 | 8.094 | -4.867 | 1.00 | 27.77 | A |
| ATOM | 1094 | CA | ALA | A | 174 | 24.267 | 8.887 | -4.213 | 1.00 | 26.86 | A |
| ATOM | 1095 | CB | ALA | A | 174 | 23.954 | 10.371 | -4.331 | 1.00 | 25.40 | A |
| ATOM | 1096 | C | ALA | A | 174 | 24.338 | 8.479 | -2.750 | 1.00 | 26.81 | A |
| ATOM | 1097 | O | ALA | A | 174 | 24.889 | 9.195 | -1.910 | 1.00 | 26.68 | A |
| ATOM | 1098 | N | ASN | A | 175 | 23.749 | 7.330 | -2.445 | 1.00 | 26.09 | A |
| ATOM | 1099 | CA | ASN | A | 175 | 23.781 | 6.806 | -1.091 | 1.00 | 26.02 | A |
| ATOM | 1100 | CB | ASN | A | 175 | 25.237 | 6.642 | -0.665 | 1.00 | 28.90 | A |
| ATOM | 1101 | CG | ASN | A | 175 | 25.375 | 5.882 | 0.612 | 1.00 | 31.56 | A |
| ATOM | 1102 | OD1 | ASN | A | 175 | 24.837 | 4.783 | 0.743 | 1.00 | 35.07 | A |
| ATOM | 1103 | ND2 | ASN | A | 175 | 26.097 | 6.453 | 1.573 | 1.00 | 32.67 | A |
| ATOM | 1104 | C | ASN | A | 175 | 23.017 | 7.627 | -0.049 | 1.00 | 25.41 | A |
| ATOM | 1105 | O | ASN | A | 175 | 23.321 | 7.567 | 1.148 | 1.00 | 25.22 | A |
| ATOM | 1106 | N | VAL | A | 176 | 22.040 | 8.408 | -0.498 | 1.00 | 23.03 | A |
| ATOM | 1107 | CA | VAL | A | 176 | 21.222 | 9.189 | 0.422 | 1.00 | 20.45 | A |
| ATOM | 1108 | CB | VAL | A | 176 | 21.049 | 10.659 | -0.027 | 1.00 | 18.92 | A |
| ATOM | 1109 | CG1 | VAL | A | 176 | 20.195 | 11.387 | 0.973 | 1.00 | 17.81 | A |
| ATOM | 1110 | CG2 | VAL | A | 176 | 22.390 | 11.348 | -0.156 | 1.00 | 17.70 | A |
| ATOM | 1111 | C | VAL | A | 176 | 19.853 | 8.521 | 0.413 | 1.00 | 20.99 | A |
| ATOM | 1112 | O | VAL | A | 176 | 19.166 | 8.501 | -0.615 | 1.00 | 22.41 | A |
| ATOM | 1113 | N | THR | A | 177 | 19.458 | 7.960 | 1.546 | 1.00 | 19.48 | A |
| ATOM | 1114 | CA | THR | A | 177 | 18.172 | 7.291 | 1.623 | 1.00 | 18.34 | A |
| ATOM | 1115 | CB | THR | A | 177 | 18.283 | 5.967 | 2.406 | 1.00 | 18.02 | A |
| ATOM | 1116 | OG1 | THR | A | 177 | 18.528 | 6.240 | 3.792 | 1.00 | 16.14 | A |
| ATOM | 1117 | CG2 | THR | A | 177 | 19.419 | 5.132 | 1.851 | 1.00 | 17.24 | A |
| ATOM | 1118 | C | THR | A | 177 | 17.111 | 8.167 | 2.285 | 1.00 | 17.62 | A |
| ATOM | 1119 | O | THR | A | 177 | 17.428 | 9.173 | 2.932 | 1.00 | 17.25 | A |
| ATOM | 1120 | N | ALA | A | 178 | 15.851 | 7.775 | 2.104 | 1.00 | 15.02 | A |
| ATOM | 1121 | CA | ALA | A | 178 | 14.721 | 8.483 | 2.682 | 1.00 | 12.93 | A |
| ATOM | 1122 | CB | ALA | A | 178 | 14.154 | 9.469 | 1.677 | 1.00 | 11.82 | A |
| ATOM | 1123 | C | ALA | A | 178 | 13.659 | 7.462 | 3.088 | 1.00 | 12.71 | A |
| ATOM | 1124 | O | ALA | A | 178 | 13.173 | 6.703 | 2.259 | 1.00 | 14.63 | A |
| ATOM | 1125 | N | ASN | A | 179 | 13.316 | 7.431 | 4.368 | 1.00 | 10.59 | A |
| ATOM | 1126 | CA | ASN | A | 179 | 12.316 | 6.503 | 4.861 | 1.00 | 9.81 | A |
| ATOM | 1127 | CB | ASN | A | 179 | 12.974 | 5.392 | 5.686 | 1.00 | 7.35 | A |
| ATOM | 1128 | CG | ASN | A | 179 | 13.922 | 4.542 | 4.853 | 1.00 | 11.10 | A |
| ATOM | 1129 | OD1 | ASN | A | 179 | 13.514 | 3.936 | 3.858 | 1.00 | 13.45 | A |
| ATOM | 1130 | ND2 | ASN | A | 179 | 15.195 | 4.501 | 5.243 | 1.00 | 8.43 | A |
| ATOM | 1131 | C | ASN | A | 179 | 11.323 | 7.271 | 5.709 | 1.00 | 11.12 | A |
| ATOM | 1132 | O | ASN | A | 179 | 11.532 | 8.446 | 6.014 | 1.00 | 13.02 | A |
| ATOM | 1133 | N | VAL | A | 180 | 10.241 | 6.608 | 6.093 | 1.00 | 10.73 | A |

Figure 2 (suite 15)

| | | | | | | | | | | | |
|---|---|---|---|---|---|---|---|---|---|---|---|
| ATOM | 1134 | CA | VAL | A | 180 | 9.223 | 7.245 | 6.894 | 1.00 | 9.17 | A |
| ATOM | 1135 | CB | VAL | A | 180 | 7.956 | 7.525 | 6.052 | 1.00 | 9.51 | A |
| ATOM | 1136 | CG1 | VAL | A | 180 | 6.816 | 7.999 | 6.948 | 1.00 | 5.62 | A |
| ATOM | 1137 | CG2 | VAL | A | 180 | 8.269 | 8.570 | 4.986 | 1.00 | 11.44 | A |
| ATOM | 1138 | C | VAL | A | 180 | 8.833 | 6.374 | 8.062 | 1.00 | 9.59 | A |
| ATOM | 1139 | O | VAL | A | 180 | 8.671 | 5.169 | 7.918 | 1.00 | 9.52 | A |
| ATOM | 1140 | N | VAL | A | 181 | 8.679 | 6.993 | 9.223 | 1.00 | 9.11 | A |
| ATOM | 1141 | CA | VAL | A | 181 | 8.271 | 6.273 | 10.410 | 1.00 | 8.66 | A |
| ATOM | 1142 | CB | VAL | A | 181 | 9.141 | 6.657 | 11.633 | 1.00 | 6.41 | A |
| ATOM | 1143 | CG1 | VAL | A | 181 | 8.554 | 6.072 | 12.905 | 1.00 | 3.86 | A |
| ATOM | 1144 | CG2 | VAL | A | 181 | 10.560 | 6.170 | 11.425 | 1.00 | 2.71 | A |
| ATOM | 1145 | C | VAL | A | 181 | 6.841 | 6.716 | 10.631 | 1.00 | 10.38 | A |
| ATOM | 1146 | O | VAL | A | 181 | 6.570 | 7.915 | 10.680 | 1.00 | 11.11 | A |
| ATOM | 1147 | N | ALA | A | 182 | 5.933 | 5.754 | 10.764 | 1.00 | 11.23 | A |
| ATOM | 1148 | CA | ALA | A | 182 | 4.519 | 6.061 | 10.949 | 1.00 | 12.44 | A |
| ATOM | 1149 | CB | ALA | A | 182 | 3.700 | 5.439 | 9.811 | 1.00 | 9.88 | A |
| ATOM | 1150 | C | ALA | A | 182 | 3.982 | 5.590 | 12.291 | 1.00 | 13.34 | A |
| ATOM | 1151 | O | ALA | A | 182 | 3.591 | 4.443 | 12.453 | 1.00 | 13.77 | A |
| ATOM | 1152 | N | PRO | A | 183 | 3.933 | 6.487 | 13.270 | 1.00 | 14.72 | A |
| ATOM | 1153 | CD | PRO | A | 183 | 4.457 | 7.863 | 13.284 | 1.00 | 15.25 | A |
| ATOM | 1154 | CA | PRO | A | 183 | 3.430 | 6.100 | 14.586 | 1.00 | 15.67 | A |
| ATOM | 1155 | CB | PRO | A | 183 | 4.034 | 7.155 | 15.497 | 1.00 | 13.25 | A |
| ATOM | 1156 | CG | PRO | A | 183 | 3.981 | 8.370 | 14.629 | 1.00 | 15.98 | A |
| ATOM | 1157 | C | PRO | A | 183 | 1.908 | 6.060 | 14.687 | 1.00 | 17.53 | A |
| ATOM | 1158 | O | PRO | A | 183 | 1.205 | 6.862 | 14.068 | 1.00 | 17.01 | A |
| ATOM | 1159 | N | GLY | A | 184 | 1.415 | 5.113 | 15.478 | 1.00 | 18.43 | A |
| ATOM | 1160 | CA | GLY | A | 184 | -0.004 | 4.982 | 15.697 | 1.00 | 20.08 | A |
| ATOM | 1161 | C | GLY | A | 184 | -0.355 | 5.794 | 16.920 | 1.00 | 23.37 | A |
| ATOM | 1162 | O | GLY | A | 184 | -0.040 | 6.980 | 16.988 | 1.00 | 22.67 | A |
| ATOM | 1163 | N | TYR | A | 185 | -1.002 | 5.158 | 17.891 | 1.00 | 27.32 | A |
| ATOM | 1164 | CA | TYR | A | 185 | -1.393 | 5.829 | 19.128 | 1.00 | 30.00 | A |
| ATOM | 1165 | CB | TYR | A | 185 | -2.571 | 5.088 | 19.765 | 1.00 | 34.24 | A |
| ATOM | 1166 | CG | TYR | A | 185 | -3.283 | 5.839 | 20.872 | 1.00 | 40.67 | A |
| ATOM | 1167 | CD1 | TYR | A | 185 | -3.772 | 7.135 | 20.669 | 1.00 | 43.46 | A |
| ATOM | 1168 | CE1 | TYR | A | 185 | -4.484 | 7.809 | 21.670 | 1.00 | 44.42 | A |
| ATOM | 1169 | CD2 | TYR | A | 185 | -3.518 | 5.237 | 22.109 | 1.00 | 42.90 | A |
| ATOM | 1170 | CE2 | TYR | A | 185 | -4.230 | 5.900 | 23.113 | 1.00 | 44.95 | A |
| ATOM | 1171 | CZ | TYR | A | 185 | -4.708 | 7.184 | 22.888 | 1.00 | 45.32 | A |
| ATOM | 1172 | OH | TYR | A | 185 | -5.414 | 7.831 | 23.882 | 1.00 | 46.25 | A |
| ATOM | 1173 | C | TYR | A | 185 | -0.200 | 5.838 | 20.082 | 1.00 | 29.88 | A |
| ATOM | 1174 | O | TYR | A | 185 | 0.230 | 4.787 | 20.553 | 1.00 | 28.30 | A |
| ATOM | 1175 | N | ILE | A | 186 | 0.340 | 7.026 | 20.349 | 1.00 | 31.06 | A |
| ATOM | 1176 | CA | ILE | A | 186 | 1.488 | 7.179 | 21.240 | 1.00 | 32.31 | A |
| ATOM | 1177 | CB | ILE | A | 186 | 2.707 | 7.747 | 20.485 | 1.00 | 30.89 | A |
| ATOM | 1178 | CG2 | ILE | A | 186 | 3.942 | 7.742 | 21.399 | 1.00 | 30.93 | A |
| ATOM | 1179 | CG1 | ILE | A | 186 | 2.971 | 6.922 | 19.221 | 1.00 | 29.31 | A |
| ATOM | 1180 | CD | ILE | A | 186 | 3.334 | 5.475 | 19.480 | 1.00 | 28.25 | A |
| ATOM | 1181 | C | ILE | A | 186 | 1.157 | 8.114 | 22.399 | 1.00 | 35.03 | A |
| ATOM | 1182 | O | ILE | A | 186 | 0.702 | 9.235 | 22.193 | 1.00 | 35.64 | A |
| ATOM | 1183 | N | ASP | A | 187 | 1.391 | 7.645 | 23.619 | 1.00 | 38.53 | A |
| ATOM | 1184 | CA | ASP | A | 187 | 1.116 | 8.430 | 24.811 | 1.00 | 41.89 | A |
| ATOM | 1185 | CB | ASP | A | 187 | 1.246 | 7.544 | 26.051 | 1.00 | 42.72 | A |
| ATOM | 1186 | CG | ASP | A | 187 | 1.005 | 8.303 | 27.339 | 1.00 | 44.14 | A |
| ATOM | 1187 | OD1 | ASP | A | 187 | 1.970 | 8.883 | 27.880 | 1.00 | 43.75 | A |
| ATOM | 1188 | OD2 | ASP | A | 187 | -0.156 | 8.329 | 27.805 | 1.00 | 45.68 | A |
| ATOM | 1189 | C | ASP | A | 187 | 2.050 | 9.629 | 24.915 | 1.00 | 45.00 | A |
| ATOM | 1190 | O | ASP | A | 187 | 3.131 | 9.547 | 25.501 | 1.00 | 44.01 | A |
| ATOM | 1191 | N | THR | A | 188 | 1.623 | 10.744 | 24.335 | 1.00 | 49.44 | A |
| ATOM | 1192 | CA | THR | A | 188 | 2.405 | 11.969 | 24.359 | 1.00 | 55.62 | A |
| ATOM | 1193 | CB | THR | A | 188 | 2.281 | 12.739 | 23.026 | 1.00 | 55.99 | A |
| ATOM | 1194 | OG1 | THR | A | 188 | 0.902 | 13.017 | 22.755 | 1.00 | 56.20 | A |
| ATOM | 1195 | CG2 | THR | A | 188 | 2.861 | 11.917 | 21.880 | 1.00 | 57.10 | A |
| ATOM | 1196 | C | THR | A | 188 | 1.958 | 12.870 | 25.506 | 1.00 | 59.36 | A |
| ATOM | 1197 | O | THR | A | 188 | 1.613 | 12.385 | 26.584 | 1.00 | 59.85 | A |
| ATOM | 1198 | N | ASP | A | 189 | 1.961 | 14.179 | 25.280 | 1.00 | 63.86 | A |
| ATOM | 1199 | CA | ASP | A | 189 | 1.565 | 15.114 | 26.326 | 1.00 | 67.94 | A |
| ATOM | 1200 | CB | ASP | A | 189 | 2.489 | 16.333 | 26.326 | 1.00 | 69.62 | A |
| ATOM | 1201 | CG | ASP | A | 189 | 3.691 | 16.149 | 27.234 | 1.00 | 71.40 | A |
| ATOM | 1202 | OD1 | ASP | A | 189 | 3.483 | 15.943 | 28.452 | 1.00 | 71.44 | A |
| ATOM | 1203 | OD2 | ASP | A | 189 | 4.836 | 16.209 | 26.733 | 1.00 | 71.94 | A |
| ATOM | 1204 | C | ASP | A | 189 | 0.115 | 15.575 | 26.278 | 1.00 | 69.81 | A |
| ATOM | 1205 | O | ASP | A | 189 | -0.549 | 15.629 | 27.313 | 1.00 | 70.64 | A |
| ATOM | 1206 | N | MET | A | 190 | -0.381 | 15.910 | 25.090 | 1.00 | 71.30 | A |

Figure 2 (suite 16)

| | | | | | | | | | | | |
|---|---|---|---|---|---|---|---|---|---|---|---|
| ATOM | 1207 | CA | MET | A | 190 | -1.759 | 16.368 | 24.972 | 1.00 | 72.17 | A |
| ATOM | 1208 | CB | MET | A | 190 | -1.853 | 17.518 | 23.963 | 1.00 | 72.03 | A |
| ATOM | 1209 | CG | MET | A | 190 | -1.023 | 18.737 | 24.339 | 0.00 | 72.17 | A |
| ATOM | 1210 | SD | MET | A | 190 | -1.471 | 19.423 | 25.947 | 0.00 | 72.19 | A |
| ATOM | 1211 | CE | MET | A | 190 | -2.667 | 20.669 | 25.465 | 0.00 | 72.23 | A |
| ATOM | 1212 | C | MET | A | 190 | -2.697 | 15.241 | 24.569 | 1.00 | 72.76 | A |
| ATOM | 1213 | O | MET | A | 190 | -3.901 | 15.324 | 24.792 | 1.00 | 72.90 | A |
| ATOM | 1214 | N | THR | A | 191 | -2.142 | 14.185 | 23.984 | 1.00 | 73.78 | A |
| ATOM | 1215 | CA | THR | A | 191 | -2.946 | 13.044 | 23.560 | 1.00 | 75.06 | A |
| ATOM | 1216 | CB | THR | A | 191 | -2.077 | 11.960 | 22.889 | 1.00 | 75.48 | A |
| ATOM | 1217 | OG1 | THR | A | 191 | -2.912 | 10.896 | 22.415 | 1.00 | 75.79 | A |
| ATOM | 1218 | CG2 | THR | A | 191 | -1.082 | 11.393 | 23.882 | 1.00 | 76.10 | A |
| ATOM | 1219 | C | THR | A | 191 | -3.674 | 12.411 | 24.744 | 1.00 | 75.54 | A |
| ATOM | 1220 | O | THR | A | 191 | -4.818 | 11.973 | 24.619 | 1.00 | 75.24 | A |
| ATOM | 1221 | N | ARG | A | 192 | -3.007 | 12.364 | 25.893 | 1.00 | 76.37 | A |
| ATOM | 1222 | CA | ARG | A | 192 | -3.602 | 11.781 | 27.089 | 1.00 | 77.54 | A |
| ATOM | 1223 | CB | ARG | A | 192 | -2.523 | 11.536 | 28.155 | 1.00 | 78.09 | A |
| ATOM | 1224 | CG | ARG | A | 192 | -1.473 | 12.634 | 28.283 | 1.00 | 78.57 | A |
| ATOM | 1225 | CD | ARG | A | 192 | -1.754 | 13.583 | 29.442 | 1.00 | 79.88 | A |
| ATOM | 1226 | NE | ARG | A | 192 | -2.957 | 14.386 | 29.240 | 1.00 | 80.88 | A |
| ATOM | 1227 | CZ | ARG | A | 192 | -3.372 | 15.327 | 30.081 | 1.00 | 81.51 | A |
| ATOM | 1228 | NH1 | ARG | A | 192 | -2.681 | 15.585 | 31.184 | 1.00 | 82.34 | A |
| ATOM | 1229 | NH2 | ARG | A | 192 | -4.479 | 16.011 | 29.822 | 1.00 | 81.83 | A |
| ATOM | 1230 | C | ARG | A | 192 | -4.724 | 12.650 | 27.646 | 1.00 | 78.01 | A |
| ATOM | 1231 | O | ARG | A | 192 | -5.316 | 12.334 | 28.680 | 1.00 | 77.97 | A |
| ATOM | 1232 | N | ALA | A | 193 | -5.017 | 13.743 | 26.946 | 1.00 | 78.41 | A |
| ATOM | 1233 | CA | ALA | A | 193 | -6.074 | 14.663 | 27.351 | 1.00 | 78.88 | A |
| ATOM | 1234 | CB | ALA | A | 193 | -5.728 | 16.089 | 26.919 | 1.00 | 78.80 | A |
| ATOM | 1235 | C | ALA | A | 193 | -7.405 | 14.238 | 26.738 | 1.00 | 78.85 | A |
| ATOM | 1236 | O | ALA | A | 193 | -8.462 | 14.744 | 27.114 | 1.00 | 79.23 | A |
| ATOM | 1237 | N | LEU | A | 194 | -7.345 | 13.305 | 25.792 | 1.00 | 78.66 | A |
| ATOM | 1238 | CA | LEU | A | 194 | -8.544 | 12.812 | 25.125 | 1.00 | 78.53 | A |
| ATOM | 1239 | CB | LEU | A | 194 | -8.165 | 11.778 | 24.059 | 1.00 | 78.72 | A |
| ATOM | 1240 | CG | LEU | A | 194 | -7.329 | 12.292 | 22.879 | 1.00 | 78.55 | A |
| ATOM | 1241 | CD1 | LEU | A | 194 | -6.863 | 11.120 | 22.031 | 1.00 | 78.64 | A |
| ATOM | 1242 | CD2 | LEU | A | 194 | -8.149 | 13.267 | 22.047 | 1.00 | 77.50 | A |
| ATOM | 1243 | C | LEU | A | 194 | -9.523 | 12.200 | 26.124 | 1.00 | 78.37 | A |
| ATOM | 1244 | O | LEU | A | 194 | -9.128 | 11.708 | 27.184 | 1.00 | 78.28 | A |
| ATOM | 1245 | N | ASP | A | 195 | -10.805 | 12.240 | 25.774 | 1.00 | 78.15 | A |
| ATOM | 1246 | CA | ASP | A | 195 | -11.858 | 11.705 | 26.628 | 1.00 | 77.91 | A |
| ATOM | 1247 | CB | ASP | A | 195 | -13.224 | 11.922 | 25.958 | 1.00 | 78.08 | A |
| ATOM | 1248 | CG | ASP | A | 195 | -14.393 | 11.618 | 26.884 | 1.00 | 78.42 | A |
| ATOM | 1249 | OD1 | ASP | A | 195 | -14.456 | 12.216 | 27.978 | 1.00 | 78.58 | A |
| ATOM | 1250 | OD2 | ASP | A | 195 | -15.253 | 10.788 | 26.515 | 1.00 | 78.92 | A |
| ATOM | 1251 | C | ASP | A | 195 | -11.632 | 10.217 | 26.905 | 1.00 | 77.44 | A |
| ATOM | 1252 | O | ASP | A | 195 | -11.190 | 9.470 | 26.028 | 1.00 | 77.24 | A |
| ATOM | 1253 | N | GLU | A | 196 | -11.932 | 9.797 | 28.131 | 1.00 | 76.66 | A |
| ATOM | 1254 | CA | GLU | A | 196 | -11.774 | 8.405 | 28.534 | 0.00 | 75.73 | A |
| ATOM | 1255 | CB | GLU | A | 196 | -12.452 | 8.168 | 29.885 | 0.00 | 75.79 | A |
| ATOM | 1256 | CG | GLU | A | 196 | -11.913 | 9.035 | 31.010 | 0.00 | 75.75 | A |
| ATOM | 1257 | CD | GLU | A | 196 | -12.603 | 8.766 | 32.333 | 0.00 | 75.75 | A |
| ATOM | 1258 | OE1 | GLU | A | 196 | -13.836 | 8.951 | 32.411 | 0.00 | 75.74 | A |
| ATOM | 1259 | OE2 | GLU | A | 196 | -11.911 | 8.370 | 33.295 | 0.00 | 75.74 | A |
| ATOM | 1260 | C | GLU | A | 196 | -12.366 | 7.459 | 27.494 | 1.00 | 75.12 | A |
| ATOM | 1261 | O | GLU | A | 196 | -11.869 | 6.350 | 27.301 | 1.00 | 75.61 | A |
| ATOM | 1262 | N | ARG | A | 197 | -13.430 | 7.899 | 26.831 | 1.00 | 73.49 | A |
| ATOM | 1263 | CA | ARG | A | 197 | -14.076 | 7.084 | 25.814 | 0.00 | 72.23 | A |
| ATOM | 1264 | CB | ARG | A | 197 | -15.449 | 7.663 | 25.464 | 0.00 | 72.20 | A |
| ATOM | 1265 | CG | ARG | A | 197 | -16.412 | 7.711 | 26.639 | 0.00 | 72.07 | A |
| ATOM | 1266 | CD | ARG | A | 197 | -17.759 | 8.285 | 26.233 | 0.00 | 71.97 | A |
| ATOM | 1267 | NE | ARG | A | 197 | -18.683 | 8.349 | 27.362 | 0.00 | 71.88 | A |
| ATOM | 1268 | CZ | ARG | A | 197 | -19.926 | 8.813 | 27.286 | 0.00 | 71.84 | A |
| ATOM | 1269 | NH1 | ARG | A | 197 | -20.401 | 9.257 | 26.130 | 0.00 | 71.81 | A |
| ATOM | 1270 | NH2 | ARG | A | 197 | -20.696 | 8.834 | 28.365 | 0.00 | 71.81 | A |
| ATOM | 1271 | C | ARG | A | 197 | -13.207 | 7.022 | 24.566 | 1.00 | 71.25 | A |
| ATOM | 1272 | O | ARG | A | 197 | -13.134 | 5.988 | 23.902 | 1.00 | 71.24 | A |
| ATOM | 1273 | N | ILE | A | 198 | -12.549 | 8.135 | 24.252 | 1.00 | 69.99 | A |
| ATOM | 1274 | CA | ILE | A | 198 | -11.681 | 8.205 | 23.085 | 0.00 | 68.79 | A |
| ATOM | 1275 | CB | ILE | A | 198 | -11.072 | 9.615 | 22.918 | 0.00 | 68.57 | A |
| ATOM | 1276 | CG2 | ILE | A | 198 | -10.047 | 9.612 | 21.792 | 0.00 | 68.57 | A |
| ATOM | 1277 | CG1 | ILE | A | 198 | -12.178 | 10.631 | 22.628 | 0.00 | 68.29 | A |
| ATOM | 1278 | CD | ILE | A | 198 | -12.913 | 10.378 | 21.331 | 1.00 | 67.44 | A |
| ATOM | 1279 | C | ILE | A | 198 | -10.550 | 7.203 | 23.235 | 1.00 | 68.02 | A |

Figure 2 (suite 17)

| | | | | | | | | | | | |
|---|---|---|---|---|---|---|---|---|---|---|---|
| ATOM | 1280 | O | ILE | A | 198 | -10.390 | 6.304 | 22.412 | 1.00 | 67.64 | A |
| ATOM | 1281 | N | GLN | A | 199 | -9.775 | 7.361 | 24.302 | 1.00 | 67.57 | A |
| ATOM | 1282 | CA | GLN | A | 199 | -8.647 | 6.481 | 24.583 | 1.00 | 66.92 | A |
| ATOM | 1283 | CB | GLN | A | 199 | -8.029 | 6.840 | 25.935 | 1.00 | 67.52 | A |
| ATOM | 1284 | CG | GLN | A | 199 | -7.777 | 8.326 | 26.124 | 1.00 | 68.09 | A |
| ATOM | 1285 | CD | GLN | A | 199 | -7.208 | 8.653 | 27.489 | 1.00 | 68.06 | A |
| ATOM | 1286 | OE1 | GLN | A | 199 | -7.122 | 9.821 | 27.872 | 1.00 | 68.81 | A |
| ATOM | 1287 | NE2 | GLN | A | 199 | -6.810 | 7.623 | 28.229 | 1.00 | 67.37 | A |
| ATOM | 1288 | C | GLN | A | 199 | -9.065 | 5.011 | 24.593 | 1.00 | 66.11 | A |
| ATOM | 1289 | O | GLN | A | 199 | -8.585 | 4.215 | 23.785 | 1.00 | 66.88 | A |
| ATOM | 1290 | N | GLN | A | 200 | -9.955 | 4.652 | 25.511 | 1.00 | 64.43 | A |
| ATOM | 1291 | CA | GLN | A | 200 | -10.419 | 3.274 | 25.616 | 1.00 | 62.87 | A |
| ATOM | 1292 | CB | GLN | A | 200 | -11.423 | 3.138 | 26.765 | 1.00 | 64.71 | A |
| ATOM | 1293 | CG | GLN | A | 200 | -10.814 | 3.309 | 28.155 | 1.00 | 66.27 | A |
| ATOM | 1294 | CD | GLN | A | 200 | -11.823 | 3.062 | 29.262 | 1.00 | 68.02 | A |
| ATOM | 1295 | OE1 | GLN | A | 200 | -11.487 | 3.099 | 30.449 | 1.00 | 67.87 | A |
| ATOM | 1296 | NE2 | GLN | A | 200 | -13.071 | 2.808 | 28.877 | 1.00 | 68.56 | A |
| ATOM | 1297 | C | GLN | A | 200 | -11.053 | 2.798 | 24.316 | 1.00 | 60.59 | A |
| ATOM | 1298 | O | GLN | A | 200 | -11.090 | 1.601 | 24.031 | 1.00 | 60.18 | A |
| ATOM | 1299 | N | GLY | A | 201 | -11.546 | 3.744 | 23.526 | 1.00 | 58.37 | A |
| ATOM | 1300 | CA | GLY | A | 201 | -12.165 | 3.394 | 22.264 | 1.00 | 55.58 | A |
| ATOM | 1301 | C | GLY | A | 201 | -11.143 | 2.955 | 21.234 | 1.00 | 53.42 | A |
| ATOM | 1302 | O | GLY | A | 201 | -11.320 | 1.938 | 20.570 | 1.00 | 53.17 | A |
| ATOM | 1303 | N | ALA | A | 202 | -10.067 | 3.724 | 21.104 | 1.00 | 51.91 | A |
| ATOM | 1304 | CA | ALA | A | 202 | -9.014 | 3.417 | 20.143 | 1.00 | 50.07 | A |
| ATOM | 1305 | CB | ALA | A | 202 | -7.998 | 4.555 | 20.105 | 1.00 | 49.03 | A |
| ATOM | 1306 | C | ALA | A | 202 | -8.313 | 2.098 | 20.456 | 1.00 | 49.65 | A |
| ATOM | 1307 | O | ALA | A | 202 | -7.813 | 1.428 | 19.551 | 1.00 | 48.96 | A |
| ATOM | 1308 | N | LEU | A | 203 | -8.274 | 1.729 | 21.733 | 1.00 | 48.71 | A |
| ATOM | 1309 | CA | LEU | A | 203 | -7.630 | 0.486 | 22.145 | 1.00 | 48.69 | A |
| ATOM | 1310 | CB | LEU | A | 203 | -7.644 | 0.347 | 23.675 | 1.00 | 48.69 | A |
| ATOM | 1311 | CG | LEU | A | 203 | -6.748 | 1.258 | 24.527 | 1.00 | 47.95 | A |
| ATOM | 1312 | CD1 | LEU | A | 203 | -7.093 | 1.081 | 25.993 | 1.00 | 47.20 | A |
| ATOM | 1313 | CD2 | LEU | A | 203 | -5.284 | 0.937 | 24.286 | 1.00 | 47.14 | A |
| ATOM | 1314 | C | LEU | A | 203 | -8.315 | -0.726 | 21.518 | 1.00 | 48.86 | A |
| ATOM | 1315 | O | LEU | A | 203 | -7.749 | -1.822 | 21.485 | 1.00 | 49.41 | A |
| ATOM | 1316 | N | GLN | A | 204 | -9.532 | -0.528 | 21.020 | 1.00 | 48.43 | A |
| ATOM | 1317 | CA | GLN | A | 204 | -10.279 | -1.612 | 20.397 | 1.00 | 48.04 | A |
| ATOM | 1318 | CB | GLN | A | 204 | -11.769 | -1.266 | 20.320 | 0.00 | 48.15 | A |
| ATOM | 1319 | CG | GLN | A | 204 | -12.637 | -2.403 | 19.796 | 0.00 | 48.25 | A |
| ATOM | 1320 | CD | GLN | A | 204 | -14.116 | -2.064 | 19.787 | 0.00 | 48.31 | A |
| ATOM | 1321 | OE1 | GLN | A | 204 | -14.949 | -2.887 | 19.407 | 0.00 | 48.34 | A |
| ATOM | 1322 | NE2 | GLN | A | 204 | -14.449 | -0.849 | 20.205 | 0.00 | 48.34 | A |
| ATOM | 1323 | C | GLN | A | 204 | -9.742 | -1.892 | 19.000 | 1.00 | 47.74 | A |
| ATOM | 1324 | O | GLN | A | 204 | -9.912 | -2.990 | 18.469 | 1.00 | 48.64 | A |
| ATOM | 1325 | N | PHE | A | 205 | -9.082 | -0.899 | 18.412 | 1.00 | 46.29 | A |
| ATOM | 1326 | CA | PHE | A | 205 | -8.525 | -1.040 | 17.068 | 1.00 | 44.65 | A |
| ATOM | 1327 | CB | PHE | A | 205 | -8.724 | 0.254 | 16.270 | 1.00 | 48.14 | A |
| ATOM | 1328 | CG | PHE | A | 205 | -10.162 | 0.600 | 16.014 | 1.00 | 50.70 | A |
| ATOM | 1329 | CD1 | PHE | A | 205 | -11.019 | 0.902 | 17.065 | 1.00 | 52.05 | A |
| ATOM | 1330 | CD2 | PHE | A | 205 | -10.662 | 0.612 | 14.714 | 1.00 | 53.09 | A |
| ATOM | 1331 | CE1 | PHE | A | 205 | -12.356 | 1.209 | 16.827 | 1.00 | 54.34 | A |
| ATOM | 1332 | CE2 | PHE | A | 205 | -12.002 | 0.918 | 14.461 | 1.00 | 53.93 | A |
| ATOM | 1333 | CZ | PHE | A | 205 | -12.851 | 1.216 | 15.518 | 1.00 | 54.52 | A |
| ATOM | 1334 | C | PHE | A | 205 | -7.037 | -1.373 | 17.104 | 1.00 | 41.73 | A |
| ATOM | 1335 | O | PHE | A | 205 | -6.387 | -1.451 | 16.059 | 1.00 | 40.79 | A |
| ATOM | 1336 | N | ILE | A | 206 | -6.506 | -1.565 | 18.309 | 1.00 | 37.84 | A |
| ATOM | 1337 | CA | ILE | A | 206 | -5.091 | -1.873 | 18.489 | 1.00 | 34.85 | A |
| ATOM | 1338 | CB | ILE | A | 206 | -4.452 | -0.909 | 19.509 | 1.00 | 34.76 | A |
| ATOM | 1339 | CG2 | ILE | A | 206 | -2.961 | -1.209 | 19.655 | 1.00 | 33.20 | A |
| ATOM | 1340 | CG1 | ILE | A | 206 | -4.658 | 0.534 | 19.043 | 1.00 | 34.78 | A |
| ATOM | 1341 | CD | ILE | A | 206 | -4.169 | 1.583 | 20.014 | 1.00 | 34.48 | A |
| ATOM | 1342 | C | ILE | A | 206 | -4.859 | -3.316 | 18.945 | 1.00 | 33.55 | A |
| ATOM | 1343 | O | ILE | A | 206 | -5.192 | -3.694 | 20.070 | 1.00 | 34.52 | A |
| ATOM | 1344 | N | PRO | A | 207 | -4.277 | -4.142 | 18.067 | 1.00 | 30.95 | A |
| ATOM | 1345 | CD | PRO | A | 207 | -3.863 | -3.843 | 16.686 | 1.00 | 30.13 | A |
| ATOM | 1346 | CA | PRO | A | 207 | -4.008 | -5.538 | 18.394 | 1.00 | 29.81 | A |
| ATOM | 1347 | CB | PRO | A | 207 | -3.215 | -6.024 | 17.190 | 1.00 | 28.93 | A |
| ATOM | 1348 | CG | PRO | A | 207 | -3.779 | -5.213 | 16.079 | 1.00 | 28.71 | A |
| ATOM | 1349 | C | PRO | A | 207 | -3.232 | -5.682 | 19.689 | 1.00 | 30.21 | A |
| ATOM | 1350 | O | PRO | A | 207 | -3.588 | -6.502 | 20.532 | 1.00 | 31.79 | A |
| ATOM | 1351 | N | ALA | A | 208 | -2.178 | -4.883 | 19.849 | 1.00 | 29.22 | A |
| ATOM | 1352 | CA | ALA | A | 208 | -1.351 | -4.944 | 21.051 | 1.00 | 29.26 | A |

Figure 2 (suite 18)

| | | | | | | | | | | | | |
|---|---|---|---|---|---|---|---|---|---|---|---|---|
| ATOM | 1353 | CB | ALA | A | 208 | -0.146 | -4.038 | 20.894 | 1.00 | 28.24 | | A |
| ATOM | 1354 | C | ALA | A | 208 | -2.123 | -4.576 | 22.320 | 1.00 | 29.77 | | A |
| ATOM | 1355 | O | ALA | A | 208 | -1.622 | -4.739 | 23.437 | 1.00 | 29.77 | | A |
| ATOM | 1356 | N | LYS | A | 209 | -3.345 | -4.081 | 22.142 | 1.00 | 29.08 | | A |
| ATOM | 1357 | CA | LYS | A | 209 | -4.201 | -3.687 | 23.261 | 1.00 | 29.14 | | A |
| ATOM | 1358 | CB | LYS | A | 209 | -4.670 | -4.927 | 24.017 | 1.00 | 28.42 | | A |
| ATOM | 1359 | CG | LYS | A | 209 | -5.671 | -5.739 | 23.219 | 1.00 | 31.26 | | A |
| ATOM | 1360 | CD | LYS | A | 209 | -6.931 | -4.910 | 22.958 | 1.00 | 34.16 | | A |
| ATOM | 1361 | CE | LYS | A | 209 | -7.867 | -5.569 | 21.953 | 1.00 | 34.88 | | A |
| ATOM | 1362 | NZ | LYS | A | 209 | -7.254 | -5.647 | 20.591 | 1.00 | 37.59 | | A |
| ATOM | 1363 | C | LYS | A | 209 | -3.580 | -2.684 | 24.237 | 1.00 | 28.89 | | A |
| ATOM | 1364 | O | LYS | A | 209 | -4.014 | -2.578 | 25.385 | 1.00 | 29.62 | | A |
| ATOM | 1365 | N | ARG | A | 210 | -2.571 | -1.948 | 23.781 | 1.00 | 27.82 | | A |
| ATOM | 1366 | CA | ARG | A | 210 | -1.930 | -0.947 | 24.624 | 1.00 | 26.31 | | A |
| ATOM | 1367 | CB | ARG | A | 210 | -0.782 | -1.561 | 25.437 | 1.00 | 24.06 | | A |
| ATOM | 1368 | CG | ARG | A | 210 | 0.494 | -1.765 | 24.640 | 1.00 | 20.68 | | A |
| ATOM | 1369 | CD | ARG | A | 210 | 1.654 | -2.166 | 25.526 | 1.00 | 17.43 | | A |
| ATOM | 1370 | NE | ARG | A | 210 | 2.787 | -2.604 | 24.717 | 1.00 | 19.17 | | A |
| ATOM | 1371 | CZ | ARG | A | 210 | 3.705 | -1.794 | 24.200 | 1.00 | 16.05 | | A |
| ATOM | 1372 | NH1 | ARG | A | 210 | 3.637 | -0.488 | 24.413 | 1.00 | 17.50 | | A |
| ATOM | 1373 | NH2 | ARG | A | 210 | 4.680 | -2.290 | 23.454 | 1.00 | 13.74 | | A |
| ATOM | 1374 | C | ARG | A | 210 | -1.381 | 0.194 | 23.781 | 1.00 | 26.23 | | A |
| ATOM | 1375 | O | ARG | A | 210 | -1.192 | 0.056 | 22.570 | 1.00 | 26.25 | | A |
| ATOM | 1376 | N | VAL | A | 211 | -1.111 | 1.310 | 24.446 | 1.00 | 26.23 | | A |
| ATOM | 1377 | CA | VAL | A | 211 | -0.573 | 2.506 | 23.809 | 1.00 | 25.87 | | A |
| ATOM | 1378 | CB | VAL | A | 211 | -0.926 | 3.756 | 24.645 | 1.00 | 26.41 | | A |
| ATOM | 1379 | CG1 | VAL | A | 211 | -0.515 | 5.020 | 23.913 | 1.00 | 26.14 | | A |
| ATOM | 1380 | CG2 | VAL | A | 211 | -2.412 | 3.768 | 24.943 | 1.00 | 27.87 | | A |
| ATOM | 1381 | C | VAL | A | 211 | 0.948 | 2.393 | 23.715 | 1.00 | 24.39 | | A |
| ATOM | 1382 | O | VAL | A | 211 | 1.568 | 1.776 | 24.568 | 1.00 | 26.29 | | A |
| ATOM | 1383 | N | GLY | A | 212 | 1.546 | 2.979 | 22.684 | 1.00 | 23.51 | | A |
| ATOM | 1384 | CA | GLY | A | 212 | 2.994 | 2.927 | 22.546 | 1.00 | 23.01 | | A |
| ATOM | 1385 | C | GLY | A | 212 | 3.620 | 4.139 | 23.220 | 1.00 | 22.10 | | A |
| ATOM | 1386 | O | GLY | A | 212 | 2.917 | 5.102 | 23.511 | 1.00 | 22.04 | | A |
| ATOM | 1387 | N | THR | A | 213 | 4.928 | 4.106 | 23.474 | 1.00 | 20.14 | | A |
| ATOM | 1388 | CA | THR | A | 213 | 5.602 | 5.231 | 24.121 | 1.00 | 19.14 | | A |
| ATOM | 1389 | CB | THR | A | 213 | 6.409 | 4.767 | 25.352 | 1.00 | 17.15 | | A |
| ATOM | 1390 | OG1 | THR | A | 213 | 7.564 | 4.025 | 24.932 | 1.00 | 18.02 | | A |
| ATOM | 1391 | CG2 | THR | A | 213 | 5.552 | 3.900 | 26.232 | 1.00 | 12.94 | | A |
| ATOM | 1392 | C | THR | A | 213 | 6.544 | 5.978 | 23.178 | 1.00 | 20.21 | | A |
| ATOM | 1393 | O | THR | A | 213 | 6.885 | 5.491 | 22.096 | 1.00 | 20.16 | | A |
| ATOM | 1394 | N | PRO | A | 214 | 6.974 | 7.188 | 23.571 | 1.00 | 20.16 | | A |
| ATOM | 1395 | CD | PRO | A | 214 | 6.466 | 8.083 | 24.624 | 1.00 | 19.13 | | A |
| ATOM | 1396 | CA | PRO | A | 214 | 7.879 | 7.908 | 22.671 | 1.00 | 18.72 | | A |
| ATOM | 1397 | CB | PRO | A | 214 | 8.023 | 9.265 | 23.350 | 1.00 | 18.13 | | A |
| ATOM | 1398 | CG | PRO | A | 214 | 6.702 | 9.444 | 24.009 | 1.00 | 17.57 | | A |
| ATOM | 1399 | C | PRO | A | 214 | 9.210 | 7.170 | 22.494 | 1.00 | 18.55 | | A |
| ATOM | 1400 | O | PRO | A | 214 | 9.852 | 7.259 | 21.442 | 1.00 | 19.10 | | A |
| ATOM | 1401 | N | ALA | A | 215 | 9.614 | 6.429 | 23.521 | 1.00 | 17.79 | | A |
| ATOM | 1402 | CA | ALA | A | 215 | 10.853 | 5.666 | 23.455 | 1.00 | 16.94 | | A |
| ATOM | 1403 | CB | ALA | A | 215 | 11.112 | 4.981 | 24.773 | 1.00 | 14.37 | | A |
| ATOM | 1404 | C | ALA | A | 215 | 10.747 | 4.630 | 22.339 | 1.00 | 16.62 | | A |
| ATOM | 1405 | O | ALA | A | 215 | 11.679 | 4.466 | 21.538 | 1.00 | 16.72 | | A |
| ATOM | 1406 | N | GLU | A | 216 | 9.603 | 3.948 | 22.280 | 1.00 | 14.65 | | A |
| ATOM | 1407 | CA | GLU | A | 216 | 9.367 | 2.933 | 21.256 | 1.00 | 15.22 | | A |
| ATOM | 1408 | CB | GLU | A | 216 | 8.029 | 2.225 | 21.499 | 1.00 | 14.21 | | A |
| ATOM | 1409 | CG | GLU | A | 216 | 8.027 | 1.401 | 22.783 | 1.00 | 15.82 | | A |
| ATOM | 1410 | CD | GLU | A | 216 | 6.699 | 0.721 | 23.067 | 1.00 | 19.30 | | A |
| ATOM | 1411 | OE1 | GLU | A | 216 | 5.702 | 1.434 | 23.332 | 1.00 | 20.51 | | A |
| ATOM | 1412 | OE2 | GLU | A | 216 | 6.653 | -0.530 | 23.023 | 1.00 | 18.27 | | A |
| ATOM | 1413 | C | GLU | A | 216 | 9.421 | 3.514 | 19.848 | 1.00 | 14.13 | | A |
| ATOM | 1414 | O | GLU | A | 216 | 9.597 | 2.781 | 18.877 | 1.00 | 15.13 | | A |
| ATOM | 1415 | N | VAL | A | 217 | 9.282 | 4.830 | 19.735 | 1.00 | 12.43 | | A |
| ATOM | 1416 | CA | VAL | A | 217 | 9.355 | 5.481 | 18.431 | 1.00 | 10.47 | | A |
| ATOM | 1417 | CB | VAL | A | 217 | 8.429 | 6.744 | 18.354 | 1.00 | 11.10 | | A |
| ATOM | 1418 | CG1 | VAL | A | 217 | 8.727 | 7.541 | 17.083 | 1.00 | 7.14 | | A |
| ATOM | 1419 | CG2 | VAL | A | 217 | 6.947 | 6.321 | 18.371 | 1.00 | 5.20 | | A |
| ATOM | 1420 | C | VAL | A | 217 | 10.798 | 5.900 | 18.194 | 1.00 | 10.48 | | A |
| ATOM | 1421 | O | VAL | A | 217 | 11.332 | 5.740 | 17.088 | 1.00 | 12.29 | | A |
| ATOM | 1422 | N | ALA | A | 218 | 11.429 | 6.421 | 19.247 | 1.00 | 9.58 | | A |
| ATOM | 1423 | CA | ALA | A | 218 | 12.821 | 6.880 | 19.178 | 1.00 | 6.73 | | A |
| ATOM | 1424 | CB | ALA | A | 218 | 13.238 | 7.453 | 20.506 | 1.00 | 8.03 | | A |
| ATOM | 1425 | C | ALA | A | 218 | 13.740 | 5.736 | 18.794 | 1.00 | 4.88 | | A |

Figure 2 (suite 19)

| | | | | | | | | | | | |
|---|---|---|---|---|---|---|---|---|---|---|---|
| ATOM | 1426 | O | ALA | A | 218 | 14.704 | 5.902 | 18.040 | 1.00 | 3.63 | A |
| ATOM | 1427 | N | GLY | A | 219 | 13.423 | 4.560 | 19.311 | 1.00 | 6.23 | A |
| ATOM | 1428 | CA | GLY | A | 219 | 14.226 | 3.389 | 19.007 | 1.00 | 6.97 | A |
| ATOM | 1429 | C | GLY | A | 219 | 14.304 | 3.094 | 17.525 | 1.00 | 7.86 | A |
| ATOM | 1430 | O | GLY | A | 219 | 15.380 | 2.782 | 17.012 | 1.00 | 9.02 | A |
| ATOM | 1431 | N | VAL | A | 220 | 13.175 | 3.205 | 16.826 | 1.00 | 7.52 | A |
| ATOM | 1432 | CA | VAL | A | 220 | 13.147 | 2.918 | 15.401 | 1.00 | 6.64 | A |
| ATOM | 1433 | CB | VAL | A | 220 | 11.703 | 2.953 | 14.846 | 1.00 | 9.49 | A |
| ATOM | 1434 | CG1 | VAL | A | 220 | 11.667 | 2.328 | 13.457 | 1.00 | 8.43 | A |
| ATOM | 1435 | CG2 | VAL | A | 220 | 10.758 | 2.208 | 15.789 | 1.00 | 10.10 | A |
| ATOM | 1436 | C | VAL | A | 220 | 13.994 | 3.931 | 14.663 | 1.00 | 8.08 | A |
| ATOM | 1437 | O | VAL | A | 220 | 14.736 | 3.583 | 13.739 | 1.00 | 7.30 | A |
| ATOM | 1438 | N | VAL | A | 221 | 13.884 | 5.193 | 15.070 | 1.00 | 7.48 | A |
| ATOM | 1439 | CA | VAL | A | 221 | 14.663 | 6.246 | 14.440 | 1.00 | 7.02 | A |
| ATOM | 1440 | CB | VAL | A | 221 | 14.293 | 7.615 | 15.004 | 1.00 | 8.65 | A |
| ATOM | 1441 | CG1 | VAL | A | 221 | 15.148 | 8.710 | 14.348 | 1.00 | 4.58 | A |
| ATOM | 1442 | CG2 | VAL | A | 221 | 12.817 | 7.854 | 14.780 | 1.00 | 9.89 | A |
| ATOM | 1443 | C | VAL | A | 221 | 16.142 | 5.996 | 14.681 | 1.00 | 8.26 | A |
| ATOM | 1444 | O | VAL | A | 221 | 16.976 | 6.209 | 13.787 | 1.00 | 7.51 | A |
| ATOM | 1445 | N | SER | A | 222 | 16.469 | 5.551 | 15.896 | 1.00 | 6.95 | A |
| ATOM | 1446 | CA | SER | A | 222 | 17.859 | 5.248 | 16.226 | 1.00 | 5.02 | A |
| ATOM | 1447 | CB | SER | A | 222 | 17.960 | 4.689 | 17.650 | 1.00 | 6.45 | A |
| ATOM | 1448 | OG | SER | A | 222 | 19.254 | 4.188 | 17.911 | 1.00 | 1.00 | A |
| ATOM | 1449 | C | SER | A | 222 | 18.341 | 4.222 | 15.207 | 1.00 | 4.42 | A |
| ATOM | 1450 | O | SER | A | 222 | 19.390 | 4.392 | 14.576 | 1.00 | 2.47 | A |
| ATOM | 1451 | N | PHE | A | 223 | 17.562 | 3.162 | 15.023 | 1.00 | 3.35 | A |
| ATOM | 1452 | CA | PHE | A | 223 | 17.941 | 2.155 | 14.044 | 1.00 | 7.82 | A |
| ATOM | 1453 | CB | PHE | A | 223 | 16.931 | 1.006 | 14.027 | 1.00 | 9.22 | A |
| ATOM | 1454 | CG | PHE | A | 223 | 17.036 | 0.139 | 12.805 | 1.00 | 12.74 | A |
| ATOM | 1455 | CD1 | PHE | A | 223 | 18.102 | -0.745 | 12.654 | 1.00 | 12.05 | A |
| ATOM | 1456 | CD2 | PHE | A | 223 | 16.115 | 0.276 | 11.758 | 1.00 | 12.11 | A |
| ATOM | 1457 | CE1 | PHE | A | 223 | 18.265 | -1.473 | 11.483 | 1.00 | 13.16 | A |
| ATOM | 1458 | CE2 | PHE | A | 223 | 16.267 | -0.444 | 10.582 | 1.00 | 11.93 | A |
| ATOM | 1459 | CZ | PHE | A | 223 | 17.346 | -1.321 | 10.439 | 1.00 | 14.42 | A |
| ATOM | 1460 | C | PHE | A | 223 | 18.072 | 2.739 | 12.625 | 1.00 | 9.49 | A |
| ATOM | 1461 | O | PHE | A | 223 | 19.045 | 2.455 | 11.926 | 1.00 | 7.54 | A |
| ATOM | 1462 | N | LEU | A | 224 | 17.106 | 3.550 | 12.193 | 1.00 | 10.29 | A |
| ATOM | 1463 | CA | LEU | A | 224 | 17.163 | 4.108 | 10.837 | 1.00 | 12.29 | A |
| ATOM | 1464 | CB | LEU | A | 224 | 15.850 | 4.830 | 10.484 | 1.00 | 9.95 | A |
| ATOM | 1465 | CG | LEU | A | 224 | 14.669 | 3.869 | 10.281 | 1.00 | 9.49 | A |
| ATOM | 1466 | CD1 | LEU | A | 224 | 13.352 | 4.608 | 10.329 | 1.00 | 10.49 | A |
| ATOM | 1467 | CD2 | LEU | A | 224 | 14.835 | 3.139 | 8.961 | 1.00 | 10.17 | A |
| ATOM | 1468 | C | LEU | A | 224 | 18.348 | 5.030 | 10.651 | 1.00 | 13.40 | A |
| ATOM | 1469 | O | LEU | A | 224 | 18.771 | 5.304 | 9.523 | 1.00 | 14.15 | A |
| ATOM | 1470 | N | ALA | A | 225 | 18.905 | 5.473 | 11.774 | 1.00 | 15.56 | A |
| ATOM | 1471 | CA | ALA | A | 225 | 20.057 | 6.374 | 11.769 | 1.00 | 15.19 | A |
| ATOM | 1472 | CB | ALA | A | 225 | 19.977 | 7.303 | 12.966 | 1.00 | 13.49 | A |
| ATOM | 1473 | C | ALA | A | 225 | 21.392 | 5.619 | 11.778 | 1.00 | 14.41 | A |
| ATOM | 1474 | O | ALA | A | 225 | 22.405 | 6.132 | 11.312 | 1.00 | 13.62 | A |
| ATOM | 1475 | N | SER | A | 226 | 21.377 | 4.393 | 12.292 | 1.00 | 15.80 | A |
| ATOM | 1476 | CA | SER | A | 226 | 22.584 | 3.566 | 12.389 | 1.00 | 16.98 | A |
| ATOM | 1477 | CB | SER | A | 226 | 22.319 | 2.374 | 13.309 | 1.00 | 16.97 | A |
| ATOM | 1478 | OG | SER | A | 226 | 21.672 | 1.320 | 12.604 | 1.00 | 15.58 | A |
| ATOM | 1479 | C | SER | A | 226 | 23.094 | 3.034 | 11.053 | 1.00 | 18.42 | A |
| ATOM | 1480 | O | SER | A | 226 | 22.442 | 3.179 | 10.020 | 1.00 | 22.00 | A |
| ATOM | 1481 | N | GLU | A | 227 | 24.256 | 2.391 | 11.093 | 1.00 | 19.42 | A |
| ATOM | 1482 | CA | GLU | A | 227 | 24.878 | 1.817 | 9.903 | 1.00 | 20.73 | A |
| ATOM | 1483 | CB | GLU | A | 227 | 26.366 | 1.571 | 10.152 | 1.00 | 21.44 | A |
| ATOM | 1484 | CG | GLU | A | 227 | 27.152 | 2.823 | 10.504 | 1.00 | 22.98 | A |
| ATOM | 1485 | CD | GLU | A | 227 | 27.375 | 3.717 | 9.308 | 1.00 | 24.77 | A |
| ATOM | 1486 | OE1 | GLU | A | 227 | 28.131 | 3.307 | 8.403 | 1.00 | 26.71 | A |
| ATOM | 1487 | OE2 | GLU | A | 227 | 26.793 | 4.822 | 9.270 | 1.00 | 25.16 | A |
| ATOM | 1488 | C | GLU | A | 227 | 24.209 | 0.499 | 9.517 | 1.00 | 22.12 | A |
| ATOM | 1489 | O | GLU | A | 227 | 24.507 | -0.082 | 8.471 | 1.00 | 21.93 | A |
| ATOM | 1490 | N | ASP | A | 228 | 23.300 | 0.032 | 10.362 | 1.00 | 22.16 | A |
| ATOM | 1491 | CA | ASP | A | 228 | 22.599 | -1.211 | 10.084 | 1.00 | 22.71 | A |
| ATOM | 1492 | CB | ASP | A | 228 | 21.999 | -1.783 | 11.376 | 1.00 | 20.81 | A |
| ATOM | 1493 | CG | ASP | A | 228 | 22.944 | -2.739 | 12.088 | 1.00 | 21.04 | A |
| ATOM | 1494 | OD1 | ASP | A | 228 | 22.745 | -2.975 | 13.300 | 1.00 | 20.22 | A |
| ATOM | 1495 | OD2 | ASP | A | 228 | 23.874 | -3.264 | 11.434 | 1.00 | 19.57 | A |
| ATOM | 1496 | C | ASP | A | 228 | 21.492 | -1.061 | 9.045 | 1.00 | 22.93 | A |
| ATOM | 1497 | O | ASP | A | 228 | 21.042 | -2.050 | 8.478 | 1.00 | 26.52 | A |
| ATOM | 1498 | N | ALA | A | 229 | 21.061 | 0.167 | 8.782 | 1.00 | 21.70 | A |

Figure 2 (suite 20)

| | | | | | | | | | | | |
|---|---|---|---|---|---|---|---|---|---|---|---|
| ATOM | 1499 | CA | ALA | A | 229 | 19.968 | 0.403 | 7.841 | 1.00 | 20.26 | A |
| ATOM | 1500 | CB | ALA | A | 229 | 18.953 | 1.349 | 8.485 | 1.00 | 17.02 | A |
| ATOM | 1501 | C | ALA | A | 229 | 20.399 | 0.951 | 6.478 | 1.00 | 20.00 | A |
| ATOM | 1502 | O | ALA | A | 229 | 19.636 | 1.655 | 5.818 | 1.00 | 18.75 | A |
| ATOM | 1503 | N | SER | A | 230 | 21.604 | 0.611 | 6.040 | 1.00 | 19.58 | A |
| ATOM | 1504 | CA | SER | A | 230 | 22.094 | 1.132 | 4.772 | 1.00 | 20.55 | A |
| ATOM | 1505 | CB | SER | A | 230 | 23.586 | 0.837 | 4.628 | 1.00 | 19.79 | A |
| ATOM | 1506 | OG | SER | A | 230 | 23.837 | -0.559 | 4.642 | 1.00 | 19.61 | A |
| ATOM | 1507 | C | SER | A | 230 | 21.374 | 0.648 | 3.512 | 1.00 | 21.47 | A |
| ATOM | 1508 | O | SER | A | 230 | 21.577 | 1.216 | 2.437 | 1.00 | 22.78 | A |
| ATOM | 1509 | N | TYR | A | 231 | 20.540 | -0.385 | 3.618 | 1.00 | 19.92 | A |
| ATOM | 1510 | CA | TYR | A | 231 | 19.861 | -0.880 | 2.425 | 1.00 | 18.84 | A |
| ATOM | 1511 | CB | TYR | A | 231 | 20.213 | -2.358 | 2.183 | 1.00 | 19.73 | A |
| ATOM | 1512 | CG | TYR | A | 231 | 20.145 | -2.758 | 0.722 | 1.00 | 20.05 | A |
| ATOM | 1513 | CD1 | TYR | A | 231 | 20.933 | -2.116 | -0.228 | 1.00 | 20.82 | A |
| ATOM | 1514 | CE1 | TYR | A | 231 | 20.864 | -2.458 | -1.581 | 1.00 | 20.42 | A |
| ATOM | 1515 | CD2 | TYR | A | 231 | 19.280 | -3.765 | 0.283 | 1.00 | 21.47 | A |
| ATOM | 1516 | CE2 | TYR | A | 231 | 19.204 | -4.117 | -1.073 | 1.00 | 20.44 | A |
| ATOM | 1517 | CZ | TYR | A | 231 | 20.001 | -3.455 | -1.993 | 1.00 | 20.86 | A |
| ATOM | 1518 | OH | TYR | A | 231 | 19.943 | -3.774 | -3.329 | 1.00 | 21.54 | A |
| ATOM | 1519 | C | TYR | A | 231 | 18.343 | -0.693 | 2.454 | 1.00 | 17.26 | A |
| ATOM | 1520 | O | TYR | A | 231 | 17.619 | -1.267 | 1.642 | 1.00 | 17.19 | A |
| ATOM | 1521 | N | ILE | A | 232 | 17.864 | 0.112 | 3.393 | 1.00 | 14.22 | A |
| ATOM | 1522 | CA | ILE | A | 232 | 16.439 | 0.390 | 3.495 | 1.00 | 12.13 | A |
| ATOM | 1523 | CB | ILE | A | 232 | 15.961 | 0.342 | 4.955 | 1.00 | 10.05 | A |
| ATOM | 1524 | CG2 | ILE | A | 232 | 14.519 | 0.774 | 5.039 | 1.00 | 8.61 | A |
| ATOM | 1525 | CG1 | ILE | A | 232 | 16.128 | -1.072 | 5.509 | 1.00 | 12.21 | A |
| ATOM | 1526 | CD | ILE | A | 232 | 15.551 | -1.262 | 6.899 | 1.00 | 13.52 | A |
| ATOM | 1527 | C | ILE | A | 232 | 16.184 | 1.789 | 2.926 | 1.00 | 12.43 | A |
| ATOM | 1528 | O | ILE | A | 232 | 16.698 | 2.777 | 3.440 | 1.00 | 10.23 | A |
| ATOM | 1529 | N | SER | A | 233 | 15.390 | 1.872 | 1.863 | 1.00 | 13.45 | A |
| ATOM | 1530 | CA | SER | A | 233 | 15.111 | 3.164 | 1.258 | 1.00 | 15.34 | A |
| ATOM | 1531 | CB | SER | A | 233 | 16.209 | 3.492 | 0.237 | 1.00 | 16.47 | A |
| ATOM | 1532 | OG | SER | A | 233 | 16.091 | 4.818 | -0.248 | 1.00 | 17.42 | A |
| ATOM | 1533 | C | SER | A | 233 | 13.734 | 3.253 | 0.590 | 1.00 | 16.35 | A |
| ATOM | 1534 | O | SER | A | 233 | 13.433 | 2.513 | -0.352 | 1.00 | 18.84 | A |
| ATOM | 1535 | N | GLY | A | 234 | 12.908 | 4.177 | 1.067 | 1.00 | 14.92 | A |
| ATOM | 1536 | CA | GLY | A | 234 | 11.591 | 4.351 | 0.487 | 1.00 | 12.07 | A |
| ATOM | 1537 | C | GLY | A | 234 | 10.586 | 3.468 | 1.187 | 1.00 | 11.98 | A |
| ATOM | 1538 | O | GLY | A | 234 | 9.565 | 3.084 | 0.608 | 1.00 | 13.98 | A |
| ATOM | 1539 | N | ALA | A | 235 | 10.874 | 3.153 | 2.437 | 1.00 | 10.45 | A |
| ATOM | 1540 | CA | ALA | A | 235 | 10.010 | 2.299 | 3.221 | 1.00 | 8.86 | A |
| ATOM | 1541 | CB | ALA | A | 235 | 10.857 | 1.276 | 4.002 | 1.00 | 8.64 | A |
| ATOM | 1542 | C | ALA | A | 235 | 9.168 | 3.115 | 4.177 | 1.00 | 7.73 | A |
| ATOM | 1543 | O | ALA | A | 235 | 9.491 | 4.259 | 4.478 | 1.00 | 8.58 | A |
| ATOM | 1544 | N | VAL | A | 236 | 8.073 | 2.520 | 4.633 | 1.00 | 6.68 | A |
| ATOM | 1545 | CA | VAL | A | 236 | 7.200 | 3.165 | 5.592 | 1.00 | 8.75 | A |
| ATOM | 1546 | CB | VAL | A | 236 | 5.773 | 3.410 | 5.034 | 1.00 | 8.76 | A |
| ATOM | 1547 | CG1 | VAL | A | 236 | 4.912 | 4.093 | 6.099 | 1.00 | 5.68 | A |
| ATOM | 1548 | CG2 | VAL | A | 236 | 5.836 | 4.270 | 3.766 | 1.00 | 8.93 | A |
| ATOM | 1549 | C | VAL | A | 236 | 7.114 | 2.177 | 6.738 | 1.00 | 10.46 | A |
| ATOM | 1550 | O | VAL | A | 236 | 6.417 | 1.167 | 6.653 | 1.00 | 13.26 | A |
| ATOM | 1551 | N | ILE | A | 237 | 7.845 | 2.451 | 7.806 | 1.00 | 10.69 | A |
| ATOM | 1552 | CA | ILE | A | 237 | 7.833 | 1.562 | 8.940 | 1.00 | 10.63 | A |
| ATOM | 1553 | CB | ILE | A | 237 | 9.219 | 1.520 | 9.610 | 1.00 | 11.15 | A |
| ATOM | 1554 | CG2 | ILE | A | 237 | 9.226 | 0.492 | 10.747 | 1.00 | 10.58 | A |
| ATOM | 1555 | CG1 | ILE | A | 237 | 10.269 | 1.157 | 8.551 | 1.00 | 11.88 | A |
| ATOM | 1556 | CD | ILE | A | 237 | 11.664 | 0.883 | 9.085 | 1.00 | 12.97 | A |
| ATOM | 1557 | C | ILE | A | 237 | 6.768 | 1.993 | 9.930 | 1.00 | 11.28 | A |
| ATOM | 1558 | O | ILE | A | 237 | 6.840 | 3.067 | 10.524 | 1.00 | 13.26 | A |
| ATOM | 1559 | N | PRO | A | 238 | 5.731 | 1.173 | 10.093 | 1.00 | 10.85 | A |
| ATOM | 1560 | CD | PRO | A | 238 | 5.342 | -0.015 | 9.314 | 1.00 | 8.19 | A |
| ATOM | 1561 | CA | PRO | A | 238 | 4.683 | 1.545 | 11.042 | 1.00 | 11.24 | A |
| ATOM | 1562 | CB | PRO | A | 238 | 3.475 | 0.774 | 10.531 | 1.00 | 8.10 | A |
| ATOM | 1563 | CG | PRO | A | 238 | 4.103 | -0.500 | 10.056 | 1.00 | 8.19 | A |
| ATOM | 1564 | C | PRO | A | 238 | 5.098 | 1.111 | 12.450 | 1.00 | 12.98 | A |
| ATOM | 1565 | O | PRO | A | 238 | 5.723 | 0.056 | 12.642 | 1.00 | 13.25 | A |
| ATOM | 1566 | N | VAL | A | 239 | 4.748 | 1.935 | 13.426 | 1.00 | 12.33 | A |
| ATOM | 1567 | CA | VAL | A | 239 | 5.064 | 1.674 | 14.819 | 1.00 | 13.51 | A |
| ATOM | 1568 | CB | VAL | A | 239 | 6.148 | 2.675 | 15.311 | 1.00 | 14.42 | A |
| ATOM | 1569 | CG1 | VAL | A | 239 | 6.535 | 2.378 | 16.761 | 1.00 | 14.52 | A |
| ATOM | 1570 | CG2 | VAL | A | 239 | 7.365 | 2.601 | 14.385 | 1.00 | 11.48 | A |
| ATOM | 1571 | C | VAL | A | 239 | 3.723 | 1.950 | 15.464 | 1.00 | 14.09 | A |

Figure 2 (suite 21)

| | | | | | | | | | | | | |
|---|---|---|---|---|---|---|---|---|---|---|---|---|
| ATOM | 1572 | O | VAL | A | 239 | 3.512 | 3.008 | 16.051 | 1.00 | 14.25 | | A |
| ATOM | 1573 | N | ASP | A | 240 | 2.822 | 0.979 | 15.353 | 1.00 | 14.20 | | A |
| ATOM | 1574 | CA | ASP | A | 240 | 1.453 | 1.139 | 15.826 | 1.00 | 15.48 | | A |
| ATOM | 1575 | CB | ASP | A | 240 | 0.562 | 1.414 | 14.626 | 1.00 | 15.14 | | A |
| ATOM | 1576 | CG | ASP | A | 240 | 0.707 | 0.347 | 13.578 | 1.00 | 14.56 | | A |
| ATOM | 1577 | OD1 | ASP | A | 240 | 0.710 | -0.835 | 13.961 | 1.00 | 17.45 | | A |
| ATOM | 1578 | OD2 | ASP | A | 240 | 0.830 | 0.669 | 12.384 | 1.00 | 17.25 | | A |
| ATOM | 1579 | C | ASP | A | 240 | 0.870 | -0.057 | 16.553 | 1.00 | 17.51 | | A |
| ATOM | 1580 | O | ASP | A | 240 | -0.351 | -0.157 | 16.688 | 1.00 | 16.29 | | A |
| ATOM | 1581 | N | GLY | A | 241 | 1.719 | -0.975 | 16.995 | 1.00 | 18.79 | | A |
| ATOM | 1582 | CA | GLY | A | 241 | 1.206 | -2.133 | 17.701 | 1.00 | 19.42 | | A |
| ATOM | 1583 | C | GLY | A | 241 | 0.271 | -2.984 | 16.858 | 1.00 | 19.98 | | A |
| ATOM | 1584 | O | GLY | A | 241 | -0.586 | -3.685 | 17.393 | 1.00 | 19.83 | | A |
| ATOM | 1585 | N | GLY | A | 242 | 0.425 | -2.921 | 15.543 | 1.00 | 20.21 | | A |
| ATOM | 1586 | CA | GLY | A | 242 | -0.415 | -3.724 | 14.674 | 1.00 | 21.61 | | A |
| ATOM | 1587 | C | GLY | A | 242 | -1.596 | -2.995 | 14.065 | 1.00 | 22.48 | | A |
| ATOM | 1588 | O | GLY | A | 242 | -2.154 | -3.440 | 13.064 | 1.00 | 23.53 | | A |
| ATOM | 1589 | N | MET | A | 243 | -1.979 | -1.873 | 14.659 | 1.00 | 24.19 | | A |
| ATOM | 1590 | CA | MET | A | 243 | -3.105 | -1.088 | 14.161 | 1.00 | 27.09 | | A |
| ATOM | 1591 | CB | MET | A | 243 | -3.168 | 0.250 | 14.879 | 1.00 | 29.40 | | A |
| ATOM | 1592 | CG | MET | A | 243 | -4.162 | 1.208 | 14.286 | 1.00 | 32.67 | | A |
| ATOM | 1593 | SD | MET | A | 243 | -4.022 | 2.805 | 15.051 | 1.00 | 38.19 | | A |
| ATOM | 1594 | CE | MET | A | 243 | -4.984 | 2.552 | 16.537 | 1.00 | 37.58 | | A |
| ATOM | 1595 | C | MET | A | 243 | -3.029 | -0.829 | 12.666 | 1.00 | 28.76 | | A |
| ATOM | 1596 | O | MET | A | 243 | -3.927 | -1.201 | 11.917 | 1.00 | 29.46 | | A |
| ATOM | 1597 | N | GLY | A | 244 | -1.962 | -0.164 | 12.240 | 1.00 | 30.64 | | A |
| ATOM | 1598 | CA | GLY | A | 244 | -1.795 | 0.135 | 10.831 | 1.00 | 31.65 | | A |
| ATOM | 1599 | C | GLY | A | 244 | -2.182 | -1.035 | 9.947 | 1.00 | 31.67 | | A |
| ATOM | 1600 | O | GLY | A | 244 | -2.804 | -0.843 | 8.904 | 1.00 | 32.36 | | A |
| ATOM | 1601 | N | ALA | B | 9 | 17.277 | 7.252 | 26.136 | 1.00 | 34.94 | | B |
| ATOM | 1602 | CA | ALA | B | 9 | 17.480 | 6.151 | 27.072 | 1.00 | 34.45 | | B |
| ATOM | 1603 | CB | ALA | B | 9 | 16.467 | 6.237 | 28.219 | 1.00 | 35.29 | | B |
| ATOM | 1604 | C | ALA | B | 9 | 17.352 | 4.807 | 26.364 | 1.00 | 33.03 | | B |
| ATOM | 1605 | O | ALA | B | 9 | 16.250 | 4.359 | 26.049 | 1.00 | 32.99 | | B |
| ATOM | 1606 | N | LYS | B | 10 | 18.493 | 4.175 | 26.120 | 1.00 | 32.07 | | B |
| ATOM | 1607 | CA | LYS | B | 10 | 18.549 | 2.880 | 25.453 | 1.00 | 31.12 | | B |
| ATOM | 1608 | CB | LYS | B | 10 | 19.962 | 2.662 | 24.913 | 1.00 | 31.53 | | B |
| ATOM | 1609 | CG | LYS | B | 10 | 20.450 | 3.836 | 24.059 | 1.00 | 31.59 | | B |
| ATOM | 1610 | CD | LYS | B | 10 | 21.948 | 3.802 | 23.778 | 1.00 | 32.86 | | B |
| ATOM | 1611 | CE | LYS | B | 10 | 22.779 | 4.077 | 25.040 | 1.00 | 34.28 | | B |
| ATOM | 1612 | NZ | LYS | B | 10 | 24.255 | 4.183 | 24.735 | 1.00 | 33.73 | | B |
| ATOM | 1613 | C | LYS | B | 10 | 18.173 | 1.789 | 26.452 | 1.00 | 29.69 | | B |
| ATOM | 1614 | O | LYS | B | 10 | 18.826 | 1.626 | 27.474 | 1.00 | 31.05 | | B |
| ATOM | 1615 | N | PRO | B | 11 | 17.109 | 1.027 | 26.168 | 1.00 | 28.50 | | B |
| ATOM | 1616 | CD | PRO | B | 11 | 16.405 | 0.906 | 24.880 | 1.00 | 29.73 | | B |
| ATOM | 1617 | CA | PRO | B | 11 | 16.687 | -0.034 | 27.081 | 1.00 | 28.36 | | B |
| ATOM | 1618 | CB | PRO | B | 11 | 15.448 | -0.603 | 26.396 | 1.00 | 27.78 | | B |
| ATOM | 1619 | CG | PRO | B | 11 | 15.802 | -0.486 | 24.968 | 1.00 | 28.34 | | B |
| ATOM | 1620 | C | PRO | B | 11 | 17.787 | -1.073 | 27.257 | 1.00 | 27.66 | | B |
| ATOM | 1621 | O | PRO | B | 11 | 18.622 | -1.260 | 26.375 | 1.00 | 27.86 | | B |
| ATOM | 1622 | N | PRO | B | 12 | 17.799 | -1.761 | 28.407 | 1.00 | 26.40 | | B |
| ATOM | 1623 | CD | PRO | B | 12 | 16.943 | -1.536 | 29.586 | 1.00 | 25.86 | | B |
| ATOM | 1624 | CA | PRO | B | 12 | 18.813 | -2.778 | 28.684 | 1.00 | 25.37 | | B |
| ATOM | 1625 | CB | PRO | B | 12 | 18.636 | -3.034 | 30.182 | 1.00 | 25.58 | | B |
| ATOM | 1626 | CG | PRO | B | 12 | 17.179 | -2.788 | 30.397 | 1.00 | 25.43 | | B |
| ATOM | 1627 | C | PRO | B | 12 | 18.733 | -4.053 | 27.856 | 1.00 | 24.15 | | B |
| ATOM | 1628 | O | PRO | B | 12 | 17.667 | -4.650 | 27.701 | 1.00 | 24.84 | | B |
| ATOM | 1629 | N | PHE | B | 13 | 19.879 | -4.468 | 27.332 | 1.00 | 22.52 | | B |
| ATOM | 1630 | CA | PHE | B | 13 | 19.965 | -5.681 | 26.534 | 1.00 | 22.74 | | B |
| ATOM | 1631 | CB | PHE | B | 13 | 21.384 | -5.859 | 26.017 | 1.00 | 23.45 | | B |
| ATOM | 1632 | CG | PHE | B | 13 | 21.542 | -7.014 | 25.085 | 1.00 | 26.25 | | B |
| ATOM | 1633 | CD1 | PHE | B | 13 | 21.146 | -6.911 | 23.757 | 1.00 | 26.86 | | B |
| ATOM | 1634 | CD2 | PHE | B | 13 | 22.074 | -8.214 | 25.536 | 1.00 | 28.34 | | B |
| ATOM | 1635 | CE1 | PHE | B | 13 | 21.276 | -7.992 | 22.888 | 1.00 | 29.43 | | B |
| ATOM | 1636 | CE2 | PHE | B | 13 | 22.207 | -9.300 | 24.675 | 1.00 | 29.67 | | B |
| ATOM | 1637 | CZ | PHE | B | 13 | 21.807 | -9.187 | 23.346 | 1.00 | 28.80 | | B |
| ATOM | 1638 | C | PHE | B | 13 | 19.569 | -6.919 | 27.345 | 1.00 | 22.77 | | B |
| ATOM | 1639 | O | PHE | B | 13 | 19.955 | -7.063 | 28.501 | 1.00 | 21.33 | | B |
| ATOM | 1640 | N | VAL | B | 14 | 18.783 | -7.799 | 26.726 | 1.00 | 23.53 | | B |
| ATOM | 1641 | CA | VAL | B | 14 | 18.322 | -9.046 | 27.346 | 1.00 | 22.86 | | B |
| ATOM | 1642 | CB | VAL | B | 14 | 16.780 | -9.080 | 27.505 | 1.00 | 23.67 | | B |
| ATOM | 1643 | CG1 | VAL | B | 14 | 16.350 | -10.419 | 28.083 | 1.00 | 22.29 | | B |
| ATOM | 1644 | CG2 | VAL | B | 14 | 16.302 | -7.936 | 28.384 | 1.00 | 21.97 | | B |

Figure 2 (suite 22)

| | | | | | | | | | | | |
|---|---|---|---|---|---|---|---|---|---|---|---|
| ATOM | 1645 | C | VAL | B | 14 | 18.696 | -10.202 | 26.425 | 1.00 | 23.02 | B |
| ATOM | 1646 | O | VAL | B | 14 | 18.210 | -10.268 | 25.294 | 1.00 | 25.03 | B |
| ATOM | 1647 | N | SER | B | 15 | 19.553 | -11.108 | 26.888 | 1.00 | 21.62 | B |
| ATOM | 1648 | CA | SER | B | 15 | 19.938 | -12.248 | 26.055 | 1.00 | 20.09 | B |
| ATOM | 1649 | CB | SER | B | 15 | 21.029 | -13.074 | 26.723 | 1.00 | 18.99 | B |
| ATOM | 1650 | OG | SER | B | 15 | 21.499 | -14.058 | 25.820 | 1.00 | 19.43 | B |
| ATOM | 1651 | C | SER | B | 15 | 18.723 | -13.137 | 25.806 | 1.00 | 19.14 | B |
| ATOM | 1652 | O | SER | B | 15 | 18.122 | -13.646 | 26.751 | 1.00 | 21.26 | B |
| ATOM | 1653 | N | ARG | B | 16 | 18.371 | -13.330 | 24.537 | 1.00 | 17.17 | B |
| ATOM | 1654 | CA | ARG | B | 16 | 17.205 | -14.141 | 24.183 | 1.00 | 14.27 | B |
| ATOM | 1655 | CB | ARG | B | 16 | 16.305 | -13.389 | 23.193 | 1.00 | 12.50 | B |
| ATOM | 1656 | CG | ARG | B | 16 | 15.785 | -12.032 | 23.648 | 1.00 | 10.39 | B |
| ATOM | 1657 | CD | ARG | B | 16 | 14.782 | -12.161 | 24.771 | 1.00 | 10.42 | B |
| ATOM | 1658 | NE | ARG | B | 16 | 14.185 | -10.872 | 25.110 | 1.00 | 9.32 | B |
| ATOM | 1659 | CZ | ARG | B | 16 | 13.291 | -10.698 | 26.078 | 1.00 | 8.94 | B |
| ATOM | 1660 | NH1 | ARG | B | 16 | 12.898 | -11.738 | 26.805 | 1.00 | 6.65 | B |
| ATOM | 1661 | NH2 | ARG | B | 16 | 12.779 | -9.492 | 26.308 | 1.00 | 3.63 | B |
| ATOM | 1662 | C | ARG | B | 16 | 17.548 | -15.487 | 23.557 | 1.00 | 14.02 | B |
| ATOM | 1663 | O | ARG | B | 16 | 18.579 | -15.640 | 22.897 | 1.00 | 13.75 | B |
| ATOM | 1664 | N | SER | B | 17 | 16.669 | -16.461 | 23.778 | 1.00 | 13.74 | B |
| ATOM | 1665 | CA | SER | B | 17 | 16.813 | -17.788 | 23.181 | 1.00 | 13.76 | B |
| ATOM | 1666 | CB | SER | B | 17 | 15.908 | -18.793 | 23.895 | 1.00 | 14.93 | B |
| ATOM | 1667 | OG | SER | B | 17 | 15.964 | -20.057 | 23.262 | 1.00 | 17.61 | B |
| ATOM | 1668 | C | SER | B | 17 | 16.318 | -17.579 | 21.749 | 1.00 | 12.96 | B |
| ATOM | 1669 | O | SER | B | 17 | 15.167 | -17.207 | 21.533 | 1.00 | 12.15 | B |
| ATOM | 1670 | N | VAL | B | 18 | 17.178 | -17.814 | 20.775 | 1.00 | 12.16 | B |
| ATOM | 1671 | CA | VAL | B | 18 | 16.809 | -17.586 | 19.392 | 1.00 | 12.61 | B |
| ATOM | 1672 | CB | VAL | B | 18 | 17.670 | -16.450 | 18.808 | 1.00 | 12.16 | B |
| ATOM | 1673 | CG1 | VAL | B | 18 | 17.515 | -16.367 | 17.282 | 1.00 | 12.47 | B |
| ATOM | 1674 | CG2 | VAL | B | 18 | 17.278 | -15.147 | 19.466 | 1.00 | 11.67 | B |
| ATOM | 1675 | C | VAL | B | 18 | 16.963 | -18.812 | 18.523 | 1.00 | 13.46 | B |
| ATOM | 1676 | O | VAL | B | 18 | 17.938 | -19.550 | 18.655 | 1.00 | 14.69 | B |
| ATOM | 1677 | N | LEU | B | 19 | 15.999 | -19.027 | 17.628 | 1.00 | 13.90 | B |
| ATOM | 1678 | CA | LEU | B | 19 | 16.065 | -20.163 | 16.724 | 1.00 | 13.96 | B |
| ATOM | 1679 | CB | LEU | B | 19 | 14.866 | -21.096 | 16.929 | 1.00 | 13.71 | B |
| ATOM | 1680 | CG | LEU | B | 19 | 15.069 | -22.607 | 16.702 | 1.00 | 13.58 | B |
| ATOM | 1681 | CD1 | LEU | B | 19 | 13.738 | -23.240 | 16.327 | 1.00 | 11.86 | B |
| ATOM | 1682 | CD2 | LEU | B | 19 | 16.069 | -22.862 | 15.614 | 1.00 | 10.66 | B |
| ATOM | 1683 | C | LEU | B | 19 | 16.056 | -19.621 | 15.310 | 1.00 | 14.36 | B |
| ATOM | 1684 | O | LEU | B | 19 | 15.191 | -18.830 | 14.949 | 1.00 | 16.70 | B |
| ATOM | 1685 | N | VAL | B | 20 | 17.022 | -20.028 | 14.505 | 1.00 | 14.10 | B |
| ATOM | 1686 | CA | VAL | B | 20 | 17.082 | -19.561 | 13.134 | 1.00 | 13.73 | B |
| ATOM | 1687 | CB | VAL | B | 20 | 18.364 | -18.715 | 12.894 | 1.00 | 12.99 | B |
| ATOM | 1688 | CG1 | VAL | B | 20 | 18.443 | -18.267 | 11.438 | 1.00 | 10.98 | B |
| ATOM | 1689 | CG2 | VAL | B | 20 | 18.364 | -17.506 | 13.820 | 1.00 | 10.14 | B |
| ATOM | 1690 | C | VAL | B | 20 | 17.067 | -20.779 | 12.209 | 1.00 | 15.54 | B |
| ATOM | 1691 | O | VAL | B | 20 | 18.074 | -21.478 | 12.063 | 1.00 | 16.77 | B |
| ATOM | 1692 | N | THR | B | 21 | 15.916 | -21.027 | 11.590 | 1.00 | 15.04 | B |
| ATOM | 1693 | CA | THR | B | 21 | 15.760 | -22.154 | 10.684 | 1.00 | 16.20 | B |
| ATOM | 1694 | CB | THR | B | 21 | 14.293 | -22.344 | 10.286 | 1.00 | 16.10 | B |
| ATOM | 1695 | OG1 | THR | B | 21 | 13.923 | -21.326 | 9.354 | 1.00 | 16.37 | B |
| ATOM | 1696 | CG2 | THR | B | 21 | 13.398 | -22.242 | 11.507 | 1.00 | 16.43 | B |
| ATOM | 1697 | C | THR | B | 21 | 16.575 | -21.930 | 9.419 | 1.00 | 17.00 | B |
| ATOM | 1698 | O | THR | B | 21 | 16.553 | -20.852 | 8.849 | 1.00 | 16.86 | B |
| ATOM | 1699 | N | GLY | B | 22 | 17.293 | -22.954 | 8.979 | 1.00 | 18.43 | B |
| ATOM | 1700 | CA | GLY | B | 22 | 18.099 | -22.808 | 7.786 | 1.00 | 21.23 | B |
| ATOM | 1701 | C | GLY | B | 22 | 19.191 | -21.767 | 7.942 | 1.00 | 24.20 | B |
| ATOM | 1702 | O | GLY | B | 22 | 19.421 | -20.967 | 7.036 | 1.00 | 24.23 | B |
| ATOM | 1703 | N | GLY | B | 23 | 19.874 | -21.778 | 9.086 | 1.00 | 26.18 | B |
| ATOM | 1704 | CA | GLY | B | 23 | 20.925 | -20.807 | 9.316 | 1.00 | 26.81 | B |
| ATOM | 1705 | C | GLY | B | 23 | 22.325 | -21.376 | 9.224 | 1.00 | 28.86 | B |
| ATOM | 1706 | O | GLY | B | 23 | 23.094 | -21.267 | 10.178 | 1.00 | 30.93 | B |
| ATOM | 1707 | N | ASN | B | 24 | 22.665 | -21.971 | 8.083 | 1.00 | 29.51 | B |
| ATOM | 1708 | CA | ASN | B | 24 | 23.989 | -22.558 | 7.890 | 1.00 | 29.20 | B |
| ATOM | 1709 | CB | ASN | B | 24 | 23.911 | -24.074 | 7.963 | 1.00 | 30.63 | B |
| ATOM | 1710 | CG | ASN | B | 24 | 23.063 | -24.653 | 6.855 | 1.00 | 32.64 | B |
| ATOM | 1711 | OD1 | ASN | B | 24 | 21.855 | -24.421 | 6.799 | 1.00 | 32.95 | B |
| ATOM | 1712 | ND2 | ASN | B | 24 | 23.693 | -25.403 | 5.955 | 1.00 | 34.50 | B |
| ATOM | 1713 | C | ASN | B | 24 | 24.563 | -22.162 | 6.540 | 1.00 | 29.81 | B |
| ATOM | 1714 | O | ASN | B | 24 | 25.679 | -22.549 | 6.193 | 1.00 | 30.73 | B |
| ATOM | 1715 | N | ARG | B | 25 | 23.787 | -21.405 | 5.774 | 1.00 | 29.99 | B |
| ATOM | 1716 | CA | ARG | B | 25 | 24.224 | -20.926 | 4.463 | 1.00 | 30.70 | B |
| ATOM | 1717 | CB | ARG | B | 25 | 23.944 | -21.973 | 3.370 | 1.00 | 32.68 | B |

Figure 2 (suite 23)

| | | | | | | | | | | | |
|---|---|---|---|---|---|---|---|---|---|---|---|
| ATOM | 1718 | CG | ARG | B | 25 | 22.622 | -22.699 | 3.512 | 1.00 | 37.36 | B |
| ATOM | 1719 | CD | ARG | B | 25 | 22.166 | -23.359 | 2.205 | 1.00 | 42.30 | B |
| ATOM | 1720 | NE | ARG | B | 25 | 23.159 | -24.253 | 1.606 | 1.00 | 46.85 | B |
| ATOM | 1721 | CZ | ARG | B | 25 | 24.129 | -23.858 | 0.783 | 1.00 | 48.73 | B |
| ATOM | 1722 | NH1 | ARG | B | 25 | 24.241 | -22.576 | 0.455 | 1.00 | 50.14 | B |
| ATOM | 1723 | NH2 | ARG | B | 25 | 24.984 | -24.746 | 0.283 | 1.00 | 48.92 | B |
| ATOM | 1724 | C | ARG | B | 25 | 23.563 | -19.587 | 4.104 | 1.00 | 28.34 | B |
| ATOM | 1725 | O | ARG | B | 25 | 22.505 | -19.245 | 4.628 | 1.00 | 27.36 | B |
| ATOM | 1726 | N | GLY | B | 26 | 24.213 | -18.826 | 3.227 | 1.00 | 25.95 | B |
| ATOM | 1727 | CA | GLY | B | 26 | 23.690 | -17.540 | 2.809 | 1.00 | 25.89 | B |
| ATOM | 1728 | C | GLY | B | 26 | 23.107 | -16.674 | 3.911 | 1.00 | 26.24 | B |
| ATOM | 1729 | O | GLY | B | 26 | 23.606 | -16.655 | 5.034 | 1.00 | 28.72 | B |
| ATOM | 1730 | N | ILE | B | 27 | 22.041 | -15.954 | 3.577 | 1.00 | 24.56 | B |
| ATOM | 1731 | CA | ILE | B | 27 | 21.358 | -15.057 | 4.506 | 1.00 | 23.63 | B |
| ATOM | 1732 | CB | ILE | B | 27 | 19.976 | -14.663 | 3.940 | 1.00 | 22.93 | B |
| ATOM | 1733 | CG2 | ILE | B | 27 | 19.150 | -13.948 | 4.985 | 1.00 | 21.67 | B |
| ATOM | 1734 | CG1 | ILE | B | 27 | 20.174 | -13.791 | 2.702 | 1.00 | 24.61 | B |
| ATOM | 1735 | CD | ILE | B | 27 | 18.921 | -13.618 | 1.861 | 1.00 | 28.25 | B |
| ATOM | 1736 | C | ILE | B | 27 | 21.184 | -15.600 | 5.925 | 1.00 | 23.14 | B |
| ATOM | 1737 | O | ILE | B | 27 | 21.385 | -14.872 | 6.901 | 1.00 | 23.00 | B |
| ATOM | 1738 | N | GLY | B | 28 | 20.805 | -16.872 | 6.035 | 1.00 | 21.76 | B |
| ATOM | 1739 | CA | GLY | B | 28 | 20.596 | -17.476 | 7.341 | 1.00 | 21.01 | B |
| ATOM | 1740 | C | GLY | B | 28 | 21.848 | -17.489 | 8.202 | 1.00 | 20.97 | B |
| ATOM | 1741 | O | GLY | B | 28 | 21.817 | -17.126 | 9.381 | 1.00 | 18.85 | B |
| ATOM | 1742 | N | LEU | B | 29 | 22.958 | -17.914 | 7.610 | 1.00 | 20.00 | B |
| ATOM | 1743 | CA | LEU | B | 29 | 24.214 | -17.961 | 8.334 | 1.00 | 20.06 | B |
| ATOM | 1744 | CB | LEU | B | 29 | 25.350 | -18.402 | 7.401 | 1.00 | 19.25 | B |
| ATOM | 1745 | CG | LEU | B | 29 | 26.736 | -18.492 | 8.059 | 1.00 | 19.01 | B |
| ATOM | 1746 | CD1 | LEU | B | 29 | 26.655 | -19.319 | 9.334 | 1.00 | 16.33 | B |
| ATOM | 1747 | CD2 | LEU | B | 29 | 27.726 | -19.109 | 7.085 | 1.00 | 18.24 | B |
| ATOM | 1748 | C | LEU | B | 29 | 24.512 | -16.581 | 8.920 | 1.00 | 19.86 | B |
| ATOM | 1749 | O | LEU | B | 29 | 24.779 | -16.449 | 10.114 | 1.00 | 20.21 | B |
| ATOM | 1750 | N | ALA | B | 30 | 24.444 | -15.558 | 8.069 | 1.00 | 18.98 | B |
| ATOM | 1751 | CA | ALA | B | 30 | 24.703 | -14.183 | 8.487 | 1.00 | 17.83 | B |
| ATOM | 1752 | CB | ALA | B | 30 | 24.565 | -13.222 | 7.292 | 1.00 | 13.68 | B |
| ATOM | 1753 | C | ALA | B | 30 | 23.753 | -13.778 | 9.607 | 1.00 | 17.49 | B |
| ATOM | 1754 | O | ALA | B | 30 | 24.151 | -13.095 | 10.544 | 1.00 | 20.27 | B |
| ATOM | 1755 | N | ILE | B | 31 | 22.501 | -14.207 | 9.530 | 1.00 | 15.71 | B |
| ATOM | 1756 | CA | ILE | B | 31 | 21.563 | -13.844 | 10.574 | 1.00 | 15.88 | B |
| ATOM | 1757 | CB | ILE | B | 31 | 20.121 | -14.214 | 10.172 | 1.00 | 14.67 | B |
| ATOM | 1758 | CG2 | ILE | B | 31 | 19.177 | -14.001 | 11.349 | 1.00 | 12.54 | B |
| ATOM | 1759 | CG1 | ILE | B | 31 | 19.700 | -13.358 | 8.971 | 1.00 | 16.54 | B |
| ATOM | 1760 | CD | ILE | B | 31 | 18.294 | -13.673 | 8.414 | 1.00 | 18.92 | B |
| ATOM | 1761 | C | ILE | B | 31 | 21.919 | -14.475 | 11.923 | 1.00 | 16.88 | B |
| ATOM | 1762 | O | ILE | B | 31 | 21.849 | -13.811 | 12.958 | 1.00 | 17.91 | B |
| ATOM | 1763 | N | ALA | B | 32 | 22.296 | -15.752 | 11.909 | 1.00 | 18.64 | B |
| ATOM | 1764 | CA | ALA | B | 32 | 22.668 | -16.461 | 13.134 | 1.00 | 19.71 | B |
| ATOM | 1765 | CB | ALA | B | 32 | 22.849 | -17.948 | 12.850 | 1.00 | 17.06 | B |
| ATOM | 1766 | C | ALA | B | 32 | 23.963 | -15.876 | 13.709 | 1.00 | 21.48 | B |
| ATOM | 1767 | O | ALA | B | 32 | 24.034 | -15.523 | 14.893 | 1.00 | 21.93 | B |
| ATOM | 1768 | N | GLN | B | 33 | 24.988 | -15.762 | 12.874 | 1.00 | 21.37 | B |
| ATOM | 1769 | CA | GLN | B | 33 | 26.237 | -15.212 | 13.357 | 1.00 | 22.59 | B |
| ATOM | 1770 | CB | GLN | B | 33 | 27.264 | -15.133 | 12.230 | 1.00 | 24.60 | B |
| ATOM | 1771 | CG | GLN | B | 33 | 27.584 | -16.503 | 11.655 | 1.00 | 31.74 | B |
| ATOM | 1772 | CD | GLN | B | 33 | 28.870 | -16.543 | 10.847 | 1.00 | 35.18 | B |
| ATOM | 1773 | OE1 | GLN | B | 33 | 29.120 | -15.678 | 9.997 | 1.00 | 36.53 | B |
| ATOM | 1774 | NE2 | GLN | B | 33 | 29.692 | -17.567 | 11.099 | 1.00 | 36.43 | B |
| ATOM | 1775 | C | GLN | B | 33 | 26.005 | -13.841 | 13.975 | 1.00 | 22.63 | B |
| ATOM | 1776 | O | GLN | B | 33 | 26.409 | -13.597 | 15.120 | 1.00 | 22.91 | B |
| ATOM | 1777 | N | ARG | B | 34 | 25.331 | -12.958 | 13.242 | 1.00 | 20.54 | B |
| ATOM | 1778 | CA | ARG | B | 34 | 25.074 | -11.621 | 13.757 | 1.00 | 17.00 | B |
| ATOM | 1779 | CB | ARG | B | 34 | 24.339 | -10.762 | 12.721 | 1.00 | 15.73 | B |
| ATOM | 1780 | CG | ARG | B | 34 | 23.803 | -9.410 | 13.252 | 1.00 | 11.32 | B |
| ATOM | 1781 | CD | ARG | B | 34 | 24.888 | -8.548 | 13.913 | 1.00 | 9.99 | B |
| ATOM | 1782 | NE | ARG | B | 34 | 24.310 | -7.332 | 14.488 | 1.00 | 10.62 | B |
| ATOM | 1783 | CZ | ARG | B | 34 | 24.265 | -6.154 | 13.870 | 1.00 | 7.33 | B |
| ATOM | 1784 | NH1 | ARG | B | 34 | 24.777 | -6.011 | 12.661 | 1.00 | 9.25 | B |
| ATOM | 1785 | NH2 | ARG | B | 34 | 23.656 | -5.133 | 14.441 | 1.00 | 1.94 | B |
| ATOM | 1786 | C | ARG | B | 34 | 24.279 | -11.676 | 15.049 | 1.00 | 17.49 | B |
| ATOM | 1787 | O | ARG | B | 34 | 24.588 | -10.959 | 16.003 | 1.00 | 17.06 | B |
| ATOM | 1788 | N | LEU | B | 35 | 23.261 | -12.524 | 15.111 | 1.00 | 15.67 | B |
| ATOM | 1789 | CA | LEU | B | 35 | 22.485 | -12.567 | 16.334 | 1.00 | 15.07 | B |
| ATOM | 1790 | CB | LEU | B | 35 | 21.239 | -13.453 | 16.174 | 1.00 | 15.19 | B |

Figure 2 (suite 24)

| | | | | | | | | | | | |
|---|---|---|---|---|---|---|---|---|---|---|---|
| ATOM | 1791 | CG | LEU | B | 35 | 20.051 | -12.843 | 15.408 | 1.00 | 17.48 | B |
| ATOM | 1792 | CD1 | LEU | B | 35 | 18.781 | -13.671 | 15.635 | 1.00 | 16.69 | B |
| ATOM | 1793 | CD2 | LEU | B | 35 | 19.803 | -11.420 | 15.901 | 1.00 | 18.65 | B |
| ATOM | 1794 | C | LEU | B | 35 | 23.358 | -13.042 | 17.492 | 1.00 | 15.55 | B |
| ATOM | 1795 | O | LEU | B | 35 | 23.150 | -12.633 | 18.638 | 1.00 | 15.35 | B |
| ATOM | 1796 | N | ALA | B | 36 | 24.345 | -13.886 | 17.185 | 1.00 | 14.62 | B |
| ATOM | 1797 | CA | ALA | B | 36 | 25.260 | -14.405 | 18.203 | 1.00 | 14.76 | B |
| ATOM | 1798 | CB | ALA | B | 36 | 26.140 | -15.487 | 17.611 | 1.00 | 13.09 | B |
| ATOM | 1799 | C | ALA | B | 36 | 26.114 | -13.250 | 18.757 | 1.00 | 15.95 | B |
| ATOM | 1800 | O | ALA | B | 36 | 26.197 | -13.052 | 19.975 | 1.00 | 17.03 | B |
| ATOM | 1801 | N | ALA | B | 37 | 26.724 | -12.478 | 17.859 | 1.00 | 15.59 | B |
| ATOM | 1802 | CA | ALA | B | 37 | 27.532 | -11.324 | 18.252 | 1.00 | 14.90 | B |
| ATOM | 1803 | CB | ALA | B | 37 | 28.015 | -10.590 | 17.030 | 1.00 | 12.45 | B |
| ATOM | 1804 | C | ALA | B | 37 | 26.718 | -10.374 | 19.134 | 1.00 | 16.15 | B |
| ATOM | 1805 | O | ALA | B | 37 | 27.254 | -9.769 | 20.053 | 1.00 | 17.87 | B |
| ATOM | 1806 | N | ASP | B | 38 | 25.422 | -10.245 | 18.859 | 1.00 | 15.76 | B |
| ATOM | 1807 | CA | ASP | B | 38 | 24.575 | -9.368 | 19.657 | 1.00 | 14.97 | B |
| ATOM | 1808 | CB | ASP | B | 38 | 23.124 | -9.341 | 19.144 | 1.00 | 14.84 | B |
| ATOM | 1809 | CG | ASP | B | 38 | 22.940 | -8.506 | 17.883 | 1.00 | 11.34 | B |
| ATOM | 1810 | OD1 | ASP | B | 38 | 23.902 | -7.864 | 17.406 | 1.00 | 7.50 | B |
| ATOM | 1811 | OD2 | ASP | B | 38 | 21.802 | -8.500 | 17.371 | 1.00 | 11.71 | B |
| ATOM | 1812 | C | ASP | B | 38 | 24.546 | -9.866 | 21.085 | 1.00 | 16.07 | B |
| ATOM | 1813 | O | ASP | B | 38 | 24.286 | -9.095 | 22.001 | 1.00 | 17.54 | B |
| ATOM | 1814 | N | GLY | B | 39 | 24.785 | -11.161 | 21.278 | 1.00 | 15.57 | B |
| ATOM | 1815 | CA | GLY | B | 39 | 24.762 | -11.700 | 22.628 | 1.00 | 15.80 | B |
| ATOM | 1816 | C | GLY | B | 39 | 23.581 | -12.594 | 22.982 | 1.00 | 16.07 | B |
| ATOM | 1817 | O | GLY | B | 39 | 23.289 | -12.793 | 24.169 | 1.00 | 14.57 | B |
| ATOM | 1818 | N | HIS | B | 40 | 22.887 | -13.116 | 21.969 | 1.00 | 14.63 | B |
| ATOM | 1819 | CA | HIS | B | 40 | 21.766 | -14.033 | 22.208 | 1.00 | 14.29 | B |
| ATOM | 1820 | CB | HIS | B | 40 | 20.661 | -13.877 | 21.157 | 1.00 | 13.81 | B |
| ATOM | 1821 | CG | HIS | B | 40 | 20.006 | -12.534 | 21.136 | 1.00 | 15.12 | B |
| ATOM | 1822 | CD2 | HIS | B | 40 | 18.989 | -12.031 | 21.873 | 1.00 | 13.59 | B |
| ATOM | 1823 | ND1 | HIS | B | 40 | 20.353 | -11.549 | 20.233 | 1.00 | 14.30 | B |
| ATOM | 1824 | CE1 | HIS | B | 40 | 19.573 | -10.499 | 20.415 | 1.00 | 13.58 | B |
| ATOM | 1825 | NE2 | HIS | B | 40 | 18.736 | -10.765 | 21.403 | 1.00 | 14.06 | B |
| ATOM | 1826 | C | HIS | B | 40 | 22.258 | -15.483 | 22.106 | 1.00 | 12.84 | B |
| ATOM | 1827 | O | HIS | B | 40 | 23.273 | -15.762 | 21.453 | 1.00 | 10.61 | B |
| ATOM | 1828 | N | LYS | B | 41 | 21.528 | -16.396 | 22.743 | 1.00 | 11.61 | B |
| ATOM | 1829 | CA | LYS | B | 41 | 21.844 | -17.826 | 22.668 | 1.00 | 12.58 | B |
| ATOM | 1830 | CB | LYS | B | 41 | 21.217 | -18.561 | 23.841 | 1.00 | 11.69 | B |
| ATOM | 1831 | CG | LYS | B | 41 | 21.093 | -17.718 | 25.087 | 1.00 | 14.26 | B |
| ATOM | 1832 | CD | LYS | B | 41 | 20.670 | -18.559 | 26.277 | 1.00 | 18.63 | B |
| ATOM | 1833 | CE | LYS | B | 41 | 20.114 | -17.697 | 27.402 | 1.00 | 23.03 | B |
| ATOM | 1834 | NZ | LYS | B | 41 | 18.829 | -17.027 | 27.011 | 1.00 | 24.58 | B |
| ATOM | 1835 | C | LYS | B | 41 | 21.208 | -18.319 | 21.351 | 1.00 | 13.17 | B |
| ATOM | 1836 | O | LYS | B | 41 | 20.007 | -18.563 | 21.286 | 1.00 | 13.54 | B |
| ATOM | 1837 | N | VAL | B | 42 | 22.011 | -18.456 | 20.306 | 1.00 | 11.24 | B |
| ATOM | 1838 | CA | VAL | B | 42 | 21.504 | -18.862 | 19.003 | 1.00 | 11.02 | B |
| ATOM | 1839 | CB | VAL | B | 42 | 22.233 | -18.069 | 17.890 | 1.00 | 9.55 | B |
| ATOM | 1840 | CG1 | VAL | B | 42 | 21.827 | -18.568 | 16.515 | 1.00 | 7.93 | B |
| ATOM | 1841 | CG2 | VAL | B | 42 | 21.920 | -16.593 | 18.029 | 1.00 | 8.14 | B |
| ATOM | 1842 | C | VAL | B | 42 | 21.575 | -20.352 | 18.650 | 1.00 | 13.12 | B |
| ATOM | 1843 | O | VAL | B | 42 | 22.551 | -21.041 | 18.943 | 1.00 | 15.61 | B |
| ATOM | 1844 | N | ALA | B | 43 | 20.526 | -20.830 | 17.992 | 1.00 | 14.72 | B |
| ATOM | 1845 | CA | ALA | B | 43 | 20.445 | -22.208 | 17.536 | 1.00 | 16.03 | B |
| ATOM | 1846 | CB | ALA | B | 43 | 19.455 | -22.987 | 18.390 | 1.00 | 15.23 | B |
| ATOM | 1847 | C | ALA | B | 43 | 19.973 | -22.173 | 16.087 | 1.00 | 16.55 | B |
| ATOM | 1848 | O | ALA | B | 43 | 19.053 | -21.439 | 15.758 | 1.00 | 17.87 | B |
| ATOM | 1849 | N | VAL | B | 44 | 20.606 | -22.945 | 15.217 | 1.00 | 18.27 | B |
| ATOM | 1850 | CA | VAL | B | 44 | 20.183 | -22.987 | 13.825 | 1.00 | 21.23 | B |
| ATOM | 1851 | CB | VAL | B | 44 | 21.333 | -22.586 | 12.889 | 1.00 | 20.58 | B |
| ATOM | 1852 | CG1 | VAL | B | 44 | 22.012 | -21.354 | 13.422 | 1.00 | 21.68 | B |
| ATOM | 1853 | CG2 | VAL | B | 44 | 22.313 | -23.730 | 12.740 | 1.00 | 20.08 | B |
| ATOM | 1854 | C | VAL | B | 44 | 19.710 | -24.396 | 13.428 | 1.00 | 22.45 | B |
| ATOM | 1855 | O | VAL | B | 44 | 20.095 | -25.388 | 14.054 | 1.00 | 23.10 | B |
| ATOM | 1856 | N | THR | B | 45 | 18.868 | -24.476 | 12.398 | 1.00 | 23.98 | B |
| ATOM | 1857 | CA | THR | B | 45 | 18.389 | -25.766 | 11.888 | 1.00 | 23.83 | B |
| ATOM | 1858 | CB | THR | B | 45 | 16.852 | -25.808 | 11.659 | 1.00 | 22.54 | B |
| ATOM | 1859 | OG1 | THR | B | 45 | 16.512 | -25.019 | 10.510 | 1.00 | 19.70 | B |
| ATOM | 1860 | CG2 | THR | B | 45 | 16.107 | -25.298 | 12.880 | 1.00 | 20.99 | B |
| ATOM | 1861 | C | THR | B | 45 | 19.058 | -25.946 | 10.533 | 1.00 | 25.40 | B |
| ATOM | 1862 | O | THR | B | 45 | 19.386 | -24.962 | 9.874 | 1.00 | 25.60 | B |
| ATOM | 1863 | N | HIS | B | 46 | 19.260 | -27.190 | 10.115 | 1.00 | 28.57 | B |

Figure 2 (suite 25)

| | | | | | | | | | | | |
|---|---|---|---|---|---|---|---|---|---|---|---|
| ATOM | 1864 | CA | HIS | B | 46 | 19.896 | -27.450 | 8.829 | 1.00 | 32.87 | B |
| ATOM | 1865 | CB | HIS | B | 46 | 21.418 | -27.509 | 9.012 | 1.00 | 35.34 | B |
| ATOM | 1866 | CG | HIS | B | 46 | 21.874 | -28.609 | 9.921 | 1.00 | 36.89 | B |
| ATOM | 1867 | CD2 | HIS | B | 46 | 22.039 | -28.644 | 11.263 | 1.00 | 37.01 | B |
| ATOM | 1868 | ND1 | HIS | B | 46 | 22.165 | -29.877 | 9.465 | 1.00 | 37.90 | B |
| ATOM | 1869 | CE1 | HIS | B | 46 | 22.489 | -30.646 | 10.489 | 1.00 | 38.55 | B |
| ATOM | 1870 | NE2 | HIS | B | 46 | 22.421 | -29.923 | 11.592 | 1.00 | 37.89 | B |
| ATOM | 1871 | C | HIS | B | 46 | 19.384 | -28.749 | 8.209 | 1.00 | 34.58 | B |
| ATOM | 1872 | O | HIS | B | 46 | 18.646 | -29.502 | 8.845 | 1.00 | 33.28 | B |
| ATOM | 1873 | N | ARG | B | 47 | 19.770 | -28.996 | 6.962 | 1.00 | 37.38 | B |
| ATOM | 1874 | CA | ARG | B | 47 | 19.360 | -30.201 | 6.249 | 1.00 | 40.81 | B |
| ATOM | 1875 | CB | ARG | B | 47 | 19.530 | -30.008 | 4.741 | 1.00 | 41.97 | B |
| ATOM | 1876 | CG | ARG | B | 47 | 18.572 | -29.008 | 4.110 | 1.00 | 44.55 | B |
| ATOM | 1877 | CD | ARG | B | 47 | 17.148 | -29.549 | 4.067 | 1.00 | 47.06 | B |
| ATOM | 1878 | NE | ARG | B | 47 | 16.320 | -28.840 | 3.089 | 1.00 | 49.93 | B |
| ATOM | 1879 | CZ | ARG | B | 47 | 16.615 | -28.739 | 1.794 | 1.00 | 50.54 | B |
| ATOM | 1880 | NH1 | ARG | B | 47 | 17.719 | -29.299 | 1.318 | 1.00 | 51.19 | B |
| ATOM | 1881 | NH2 | ARG | B | 47 | 15.809 | -28.082 | 0.970 | 1.00 | 50.73 | B |
| ATOM | 1882 | C | ARG | B | 47 | 20.219 | -31.369 | 6.707 | 1.00 | 41.88 | B |
| ATOM | 1883 | O | ARG | B | 47 | 19.720 | -32.345 | 7.265 | 1.00 | 42.19 | B |
| ATOM | 1884 | N | GLY | B | 48 | 21.520 | -31.252 | 6.460 | 1.00 | 42.97 | B |
| ATOM | 1885 | CA | GLY | B | 48 | 22.458 | -32.286 | 6.852 | 1.00 | 43.87 | B |
| ATOM | 1886 | C | GLY | B | 48 | 23.873 | -31.746 | 6.800 | 1.00 | 44.99 | B |
| ATOM | 1887 | O | GLY | B | 48 | 24.789 | -32.284 | 7.426 | 1.00 | 45.07 | B |
| ATOM | 1888 | N | SER | B | 49 | 24.046 | -30.662 | 6.052 | 1.00 | 45.73 | B |
| ATOM | 1889 | CA | SER | B | 49 | 25.348 | -30.033 | 5.897 | 1.00 | 46.96 | B |
| ATOM | 1890 | CB | SER | B | 49 | 25.197 | -28.739 | 5.097 | 1.00 | 47.81 | B |
| ATOM | 1891 | OG | SER | B | 49 | 26.454 | -28.128 | 4.860 | 1.00 | 48.73 | B |
| ATOM | 1892 | C | SER | B | 49 | 26.028 | -29.741 | 7.234 | 1.00 | 47.99 | B |
| ATOM | 1893 | O | SER | B | 49 | 27.240 | -29.546 | 7.284 | 1.00 | 49.41 | B |
| ATOM | 1894 | N | GLY | B | 50 | 25.256 | -29.709 | 8.315 | 1.00 | 48.62 | B |
| ATOM | 1895 | CA | GLY | B | 50 | 25.835 | -29.436 | 9.621 | 1.00 | 48.52 | B |
| ATOM | 1896 | C | GLY | B | 50 | 25.801 | -27.965 | 10.007 | 1.00 | 48.87 | B |
| ATOM | 1897 | O | GLY | B | 50 | 25.835 | -27.084 | 9.145 | 1.00 | 49.98 | B |
| ATOM | 1898 | N | ALA | B | 51 | 25.748 | -27.697 | 11.309 | 1.00 | 47.59 | B |
| ATOM | 1899 | CA | ALA | B | 51 | 25.688 | -26.330 | 11.818 | 1.00 | 46.62 | B |
| ATOM | 1900 | CB | ALA | B | 51 | 25.147 | -26.342 | 13.232 | 1.00 | 46.20 | B |
| ATOM | 1901 | C | ALA | B | 51 | 27.023 | -25.586 | 11.787 | 1.00 | 46.45 | B |
| ATOM | 1902 | O | ALA | B | 51 | 28.093 | -26.194 | 11.870 | 1.00 | 46.75 | B |
| ATOM | 1903 | N | PRO | B | 52 | 26.975 | -24.249 | 11.666 | 1.00 | 45.61 | B |
| ATOM | 1904 | CD | PRO | B | 52 | 25.805 | -23.353 | 11.618 | 1.00 | 45.35 | B |
| ATOM | 1905 | CA | PRO | B | 52 | 28.222 | -23.482 | 11.636 | 1.00 | 45.57 | B |
| ATOM | 1906 | CB | PRO | B | 52 | 27.742 | -22.051 | 11.399 | 1.00 | 44.91 | B |
| ATOM | 1907 | CG | PRO | B | 52 | 26.395 | -22.034 | 12.072 | 1.00 | 45.29 | B |
| ATOM | 1908 | C | PRO | B | 52 | 28.971 | -23.639 | 12.958 | 1.00 | 45.62 | B |
| ATOM | 1909 | O | PRO | B | 52 | 28.371 | -23.576 | 14.036 | 1.00 | 45.55 | B |
| ATOM | 1910 | N | LYS | B | 53 | 30.281 | -23.855 | 12.865 | 1.00 | 45.23 | B |
| ATOM | 1911 | CA | LYS | B | 53 | 31.124 | -24.029 | 14.044 | 1.00 | 43.08 | B |
| ATOM | 1912 | CB | LYS | B | 53 | 32.604 | -24.021 | 13.636 | 1.00 | 43.84 | B |
| ATOM | 1913 | CG | LYS | B | 53 | 33.576 | -24.223 | 14.790 | 1.00 | 44.49 | B |
| ATOM | 1914 | CD | LYS | B | 53 | 35.023 | -24.229 | 14.320 | 0.00 | 44.37 | B |
| ATOM | 1915 | CE | LYS | B | 53 | 35.425 | -22.885 | 13.735 | 0.00 | 44.46 | B |
| ATOM | 1916 | NZ | LYS | B | 53 | 36.851 | -22.873 | 13.311 | 0.00 | 44.46 | B |
| ATOM | 1917 | C | LYS | B | 53 | 30.865 | -22.935 | 15.074 | 1.00 | 40.97 | B |
| ATOM | 1918 | O | LYS | B | 53 | 30.952 | -21.750 | 14.763 | 1.00 | 40.39 | B |
| ATOM | 1919 | N | GLY | B | 54 | 30.538 | -23.339 | 16.296 | 1.00 | 38.93 | B |
| ATOM | 1920 | CA | GLY | B | 54 | 30.281 | -22.376 | 17.349 | 1.00 | 37.18 | B |
| ATOM | 1921 | C | GLY | B | 54 | 28.829 | -22.306 | 17.775 | 1.00 | 37.06 | B |
| ATOM | 1922 | O | GLY | B | 54 | 28.528 | -22.035 | 18.939 | 1.00 | 36.18 | B |
| ATOM | 1923 | N | LEU | B | 55 | 27.923 | -22.564 | 16.835 | 1.00 | 37.45 | B |
| ATOM | 1924 | CA | LEU | B | 55 | 26.492 | -22.503 | 17.119 | 1.00 | 36.51 | B |
| ATOM | 1925 | CB | LEU | B | 55 | 25.782 | -21.692 | 16.032 | 1.00 | 36.61 | B |
| ATOM | 1926 | CG | LEU | B | 55 | 26.307 | -20.270 | 15.814 | 1.00 | 36.79 | B |
| ATOM | 1927 | CD1 | LEU | B | 55 | 25.525 | -19.599 | 14.697 | 1.00 | 36.99 | B |
| ATOM | 1928 | CD2 | LEU | B | 55 | 26.197 | -19.474 | 17.110 | 1.00 | 37.16 | B |
| ATOM | 1929 | C | LEU | B | 55 | 25.824 | -23.868 | 17.254 | 1.00 | 35.40 | B |
| ATOM | 1930 | O | LEU | B | 55 | 26.216 | -24.836 | 16.605 | 1.00 | 35.81 | B |
| ATOM | 1931 | N | PHE | B | 56 | 24.806 | -23.931 | 18.103 | 1.00 | 33.91 | B |
| ATOM | 1932 | CA | PHE | B | 56 | 24.080 | -25.170 | 18.324 | 1.00 | 34.22 | B |
| ATOM | 1933 | CB | PHE | B | 56 | 23.173 | -25.040 | 19.550 | 1.00 | 33.31 | B |
| ATOM | 1934 | CG | PHE | B | 56 | 22.522 | -26.331 | 19.972 | 1.00 | 33.23 | B |
| ATOM | 1935 | CD1 | PHE | B | 56 | 23.293 | -27.450 | 20.275 | 1.00 | 33.83 | B |
| ATOM | 1936 | CD2 | PHE | B | 56 | 21.141 | -26.423 | 20.098 | 1.00 | 33.16 | B |

Figure 2 (suite 26)

| | | | | | | | | | | | |
|---|---|---|---|---|---|---|---|---|---|---|---|
| ATOM | 1937 | CE1 | PHE | B | 56 | 22.695 | -28.640 | 20.707 | 1.00 | 31.05 | B |
| ATOM | 1938 | CE2 | PHE | B | 56 | 20.536 | -27.607 | 20.528 | 1.00 | 31.64 | B |
| ATOM | 1939 | CZ | PHE | B | 56 | 21.318 | -28.716 | 20.831 | 1.00 | 30.48 | B |
| ATOM | 1940 | C | PHE | B | 56 | 23.243 | -25.518 | 17.101 | 1.00 | 35.07 | B |
| ATOM | 1941 | O | PHE | B | 56 | 22.342 | -24.772 | 16.715 | 1.00 | 35.42 | B |
| ATOM | 1942 | N | GLY | B | 57 | 23.549 | -26.656 | 16.492 | 1.00 | 35.03 | B |
| ATOM | 1943 | CA | GLY | B | 57 | 22.804 | -27.085 | 15.326 | 1.00 | 34.97 | B |
| ATOM | 1944 | C | GLY | B | 57 | 21.787 | -28.161 | 15.654 | 1.00 | 35.38 | B |
| ATOM | 1945 | O | GLY | B | 57 | 21.905 | -28.855 | 16.662 | 1.00 | 35.83 | B |
| ATOM | 1946 | N | VAL | B | 58 | 20.778 | -28.283 | 14.799 | 1.00 | 34.86 | B |
| ATOM | 1947 | CA | VAL | B | 58 | 19.726 | -29.275 | 14.956 | 1.00 | 34.82 | B |
| ATOM | 1948 | CB | VAL | B | 58 | 18.592 | -28.756 | 15.833 | 1.00 | 33.94 | B |
| ATOM | 1949 | CG1 | VAL | B | 58 | 17.459 | -29.749 | 15.840 | 1.00 | 35.23 | B |
| ATOM | 1950 | CG2 | VAL | B | 58 | 19.094 | -28.537 | 17.240 | 1.00 | 35.95 | B |
| ATOM | 1951 | C | VAL | B | 58 | 19.171 | -29.563 | 13.575 | 1.00 | 35.44 | B |
| ATOM | 1952 | O | VAL | B | 58 | 18.764 | -28.647 | 12.868 | 1.00 | 35.99 | B |
| ATOM | 1953 | N | GLU | B | 59 | 19.165 | -30.831 | 13.184 | 1.00 | 35.86 | B |
| ATOM | 1954 | CA | GLU | B | 59 | 18.663 | -31.207 | 11.871 | 1.00 | 37.18 | B |
| ATOM | 1955 | CB | GLU | B | 59 | 19.095 | -32.632 | 11.544 | 1.00 | 39.57 | B |
| ATOM | 1956 | CG | GLU | B | 59 | 18.946 | -33.015 | 10.088 | 1.00 | 43.94 | B |
| ATOM | 1957 | CD | GLU | B | 59 | 19.500 | -34.401 | 9.801 | 1.00 | 47.30 | B |
| ATOM | 1958 | OE1 | GLU | B | 59 | 19.527 | -34.802 | 8.618 | 1.00 | 49.96 | B |
| ATOM | 1959 | OE2 | GLU | B | 59 | 19.908 | -35.092 | 10.761 | 1.00 | 49.65 | B |
| ATOM | 1960 | C | GLU | B | 59 | 17.141 | -31.093 | 11.846 | 1.00 | 36.18 | B |
| ATOM | 1961 | O | GLU | B | 59 | 16.459 | -31.550 | 12.760 | 1.00 | 36.41 | B |
| ATOM | 1962 | N | VAL | B | 60 | 16.603 | -30.465 | 10.811 | 1.00 | 35.70 | B |
| ATOM | 1963 | CA | VAL | B | 60 | 15.159 | -30.308 | 10.726 | 1.00 | 36.11 | B |
| ATOM | 1964 | CB | VAL | B | 60 | 14.698 | -28.958 | 11.329 | 1.00 | 36.33 | B |
| ATOM | 1965 | CG1 | VAL | B | 60 | 13.185 | -28.928 | 11.428 | 1.00 | 34.94 | B |
| ATOM | 1966 | CG2 | VAL | B | 60 | 15.313 | -28.754 | 12.705 | 1.00 | 37.27 | B |
| ATOM | 1967 | C | VAL | B | 60 | 14.643 | -30.385 | 9.297 | 1.00 | 35.98 | B |
| ATOM | 1968 | O | VAL | B | 60 | 15.343 | -30.058 | 8.342 | 1.00 | 35.83 | B |
| ATOM | 1969 | N | ASP | B | 61 | 13.404 | -30.833 | 9.171 | 1.00 | 36.87 | B |
| ATOM | 1970 | CA | ASP | B | 61 | 12.735 | -30.947 | 7.886 | 1.00 | 37.12 | B |
| ATOM | 1971 | CB | ASP | B | 61 | 12.488 | -32.412 | 7.560 | 1.00 | 39.69 | B |
| ATOM | 1972 | CG | ASP | B | 61 | 11.765 | -32.602 | 6.248 | 1.00 | 42.02 | B |
| ATOM | 1973 | OD1 | ASP | B | 61 | 11.379 | -33.754 | 5.960 | 1.00 | 44.55 | B |
| ATOM | 1974 | OD2 | ASP | B | 61 | 11.586 | -31.608 | 5.506 | 1.00 | 42.70 | B |
| ATOM | 1975 | C | ASP | B | 61 | 11.401 | -30.216 | 8.043 | 1.00 | 36.50 | B |
| ATOM | 1976 | O | ASP | B | 61 | 10.449 | -30.763 | 8.614 | 1.00 | 35.04 | B |
| ATOM | 1977 | N | VAL | B | 62 | 11.344 | -28.978 | 7.555 | 1.00 | 35.28 | B |
| ATOM | 1978 | CA | VAL | B | 62 | 10.136 | -28.168 | 7.671 | 1.00 | 34.75 | B |
| ATOM | 1979 | CB | VAL | B | 62 | 10.310 | -26.781 | 6.996 | 1.00 | 34.30 | B |
| ATOM | 1980 | CG1 | VAL | B | 62 | 11.297 | -25.956 | 7.778 | 1.00 | 33.93 | B |
| ATOM | 1981 | CG2 | VAL | B | 62 | 10.784 | -26.943 | 5.563 | 1.00 | 36.20 | B |
| ATOM | 1982 | C | VAL | B | 62 | 8.882 | -28.839 | 7.119 | 1.00 | 33.46 | B |
| ATOM | 1983 | O | VAL | B | 62 | 7.791 | -28.624 | 7.642 | 1.00 | 33.33 | B |
| ATOM | 1984 | N | THR | B | 63 | 9.026 | -29.655 | 6.077 | 1.00 | 33.47 | B |
| ATOM | 1985 | CA | THR | B | 63 | 7.860 | -30.336 | 5.515 | 1.00 | 33.80 | B |
| ATOM | 1986 | CB | THR | B | 63 | 8.168 | -31.018 | 4.168 | 1.00 | 33.89 | B |
| ATOM | 1987 | OG1 | THR | B | 63 | 9.098 | -32.085 | 4.374 | 1.00 | 35.95 | B |
| ATOM | 1988 | CG2 | THR | B | 63 | 8.752 | -30.026 | 3.183 | 1.00 | 34.12 | B |
| ATOM | 1989 | C | THR | B | 63 | 7.352 | -31.407 | 6.470 | 1.00 | 32.81 | B |
| ATOM | 1990 | O | THR | B | 63 | 6.231 | -31.881 | 6.329 | 1.00 | 33.79 | B |
| ATOM | 1991 | N | ASP | B | 64 | 8.181 | -31.771 | 7.444 | 1.00 | 33.52 | B |
| ATOM | 1992 | CA | ASP | B | 64 | 7.845 | -32.797 | 8.434 | 1.00 | 33.28 | B |
| ATOM | 1993 | CB | ASP | B | 64 | 9.029 | -33.753 | 8.616 | 1.00 | 33.73 | B |
| ATOM | 1994 | CG | ASP | B | 64 | 8.680 | -34.965 | 9.468 | 1.00 | 33.49 | B |
| ATOM | 1995 | OD1 | ASP | B | 64 | 7.947 | -34.806 | 10.477 | 1.00 | 28.87 | B |
| ATOM | 1996 | OD2 | ASP | B | 64 | 9.159 | -36.072 | 9.125 | 1.00 | 34.01 | B |
| ATOM | 1997 | C | ASP | B | 64 | 7.490 | -32.182 | 9.787 | 1.00 | 32.35 | B |
| ATOM | 1998 | O | ASP | B | 64 | 8.364 | -31.744 | 10.530 | 1.00 | 32.36 | B |
| ATOM | 1999 | N | SER | B | 65 | 6.205 | -32.181 | 10.107 | 1.00 | 32.05 | B |
| ATOM | 2000 | CA | SER | B | 65 | 5.712 | -31.614 | 11.353 | 1.00 | 32.58 | B |
| ATOM | 2001 | CB | SER | B | 65 | 4.190 | -31.645 | 11.346 | 1.00 | 31.63 | B |
| ATOM | 2002 | OG | SER | B | 65 | 3.666 | -31.277 | 12.606 | 1.00 | 31.99 | B |
| ATOM | 2003 | C | SER | B | 65 | 6.222 | -32.320 | 12.605 | 1.00 | 34.31 | B |
| ATOM | 2004 | O | SER | B | 65 | 6.188 | -31.760 | 13.707 | 1.00 | 33.93 | B |
| ATOM | 2005 | N | ASP | B | 66 | 6.681 | -33.555 | 12.441 | 1.00 | 36.32 | B |
| ATOM | 2006 | CA | ASP | B | 66 | 7.176 | -34.329 | 13.573 | 1.00 | 36.47 | B |
| ATOM | 2007 | CB | ASP | B | 66 | 7.045 | -35.827 | 13.277 | 1.00 | 41.39 | B |
| ATOM | 2008 | CG | ASP | B | 66 | 7.291 | -36.690 | 14.503 | 1.00 | 45.30 | B |
| ATOM | 2009 | OD1 | ASP | B | 66 | 8.449 | -37.126 | 14.707 | 1.00 | 47.03 | B |

Figure 2 (suite 27)

| | | | | | | | | | | | |
|---|---|---|---|---|---|---|---|---|---|---|---|
| ATOM | 2010 | OD2 | ASP | B | 66 | 6.323 | -36.918 | 15.267 | 1.00 | 46.90 | B |
| ATOM | 2011 | C | ASP | B | 66 | 8.624 | -33.959 | 13.859 | 1.00 | 34.25 | B |
| ATOM | 2012 | O | ASP | B | 66 | 9.031 | -33.844 | 15.013 | 1.00 | 32.42 | B |
| ATOM | 2013 | N | ALA | B | 67 | 9.395 | -33.765 | 12.795 | 1.00 | 32.33 | B |
| ATOM | 2014 | CA | ALA | B | 67 | 10.787 | -33.391 | 12.931 | 1.00 | 31.65 | B |
| ATOM | 2015 | CB | ALA | B | 67 | 11.508 | -33.560 | 11.601 | 1.00 | 30.52 | B |
| ATOM | 2016 | C | ALA | B | 67 | 10.863 | -31.939 | 13.404 | 1.00 | 32.59 | B |
| ATOM | 2017 | O | ALA | B | 67 | 11.911 | -31.481 | 13.856 | 1.00 | 32.71 | B |
| ATOM | 2018 | N | VAL | B | 68 | 9.751 | -31.212 | 13.293 | 1.00 | 32.40 | B |
| ATOM | 2019 | CA | VAL | B | 68 | 9.722 | -29.826 | 13.741 | 1.00 | 31.44 | B |
| ATOM | 2020 | CB | VAL | B | 68 | 8.593 | -29.016 | 13.051 | 1.00 | 29.71 | B |
| ATOM | 2021 | CG1 | VAL | B | 68 | 8.330 | -27.720 | 13.812 | 1.00 | 26.63 | B |
| ATOM | 2022 | CG2 | VAL | B | 68 | 8.987 | -28.702 | 11.614 | 1.00 | 26.88 | B |
| ATOM | 2023 | C | VAL | B | 68 | 9.504 | -29.830 | 15.246 | 1.00 | 32.81 | B |
| ATOM | 2024 | O | VAL | B | 68 | 10.052 | -28.992 | 15.958 | 1.00 | 34.59 | B |
| ATOM | 2025 | N | ASP | B | 69 | 8.707 | -30.775 | 15.733 | 1.00 | 33.50 | B |
| ATOM | 2026 | CA | ASP | B | 69 | 8.460 | -30.867 | 17.169 | 1.00 | 33.57 | B |
| ATOM | 2027 | CB | ASP | B | 69 | 7.341 | -31.861 | 17.475 | 1.00 | 34.62 | B |
| ATOM | 2028 | CG | ASP | B | 69 | 7.036 | -31.952 | 18.968 | 1.00 | 35.90 | B |
| ATOM | 2029 | OD1 | ASP | B | 69 | 6.172 | -32.765 | 19.358 | 1.00 | 37.75 | B |
| ATOM | 2030 | OD2 | ASP | B | 69 | 7.658 | -31.208 | 19.755 | 1.00 | 35.51 | B |
| ATOM | 2031 | C | ASP | B | 69 | 9.727 | -31.315 | 17.895 | 1.00 | 32.64 | B |
| ATOM | 2032 | O | ASP | B | 69 | 10.041 | -30.812 | 18.972 | 1.00 | 32.46 | B |
| ATOM | 2033 | N | ARG | B | 70 | 10.453 | -32.261 | 17.309 | 1.00 | 31.33 | B |
| ATOM | 2034 | CA | ARG | B | 70 | 11.670 | -32.744 | 17.939 | 1.00 | 32.12 | B |
| ATOM | 2035 | CB | ARG | B | 70 | 12.257 | -33.934 | 17.164 | 1.00 | 32.22 | B |
| ATOM | 2036 | CG | ARG | B | 70 | 11.361 | -35.169 | 17.162 | 1.00 | 34.30 | B |
| ATOM | 2037 | CD | ARG | B | 70 | 12.125 | -36.431 | 16.786 | 1.00 | 35.78 | B |
| ATOM | 2038 | NE | ARG | B | 70 | 12.793 | -36.334 | 15.490 | 1.00 | 36.14 | B |
| ATOM | 2039 | CZ | ARG | B | 70 | 12.192 | -36.491 | 14.313 | 1.00 | 34.84 | B |
| ATOM | 2040 | NH1 | ARG | B | 70 | 10.894 | -36.755 | 14.262 | 1.00 | 34.83 | B |
| ATOM | 2041 | NH2 | ARG | B | 70 | 12.893 | -36.394 | 13.188 | 1.00 | 32.81 | B |
| ATOM | 2042 | C | ARG | B | 70 | 12.702 | -31.629 | 18.049 | 1.00 | 32.05 | B |
| ATOM | 2043 | O | ARG | B | 70 | 13.243 | -31.384 | 19.132 | 1.00 | 32.39 | B |
| ATOM | 2044 | N | ALA | B | 71 | 12.959 | -30.947 | 16.932 | 1.00 | 30.19 | B |
| ATOM | 2045 | CA | ALA | B | 71 | 13.927 | -29.858 | 16.905 | 1.00 | 28.26 | B |
| ATOM | 2046 | CB | ALA | B | 71 | 13.871 | -29.146 | 15.577 | 1.00 | 26.90 | B |
| ATOM | 2047 | C | ALA | B | 71 | 13.710 | -28.866 | 18.044 | 1.00 | 28.10 | B |
| ATOM | 2048 | O | ALA | B | 71 | 14.622 | -28.617 | 18.832 | 1.00 | 26.90 | B |
| ATOM | 2049 | N | PHE | B | 72 | 12.508 | -28.307 | 18.146 | 1.00 | 26.96 | B |
| ATOM | 2050 | CA | PHE | B | 72 | 12.237 | -27.350 | 19.213 | 1.00 | 27.63 | B |
| ATOM | 2051 | CB | PHE | B | 72 | 10.766 | -26.900 | 19.206 | 1.00 | 26.41 | B |
| ATOM | 2052 | CG | PHE | B | 72 | 10.508 | -25.681 | 18.357 | 1.00 | 25.65 | B |
| ATOM | 2053 | CD1 | PHE | B | 72 | 10.563 | -25.752 | 16.965 | 1.00 | 24.21 | B |
| ATOM | 2054 | CD2 | PHE | B | 72 | 10.267 | -24.447 | 18.954 | 1.00 | 25.81 | B |
| ATOM | 2055 | CE1 | PHE | B | 72 | 10.389 | -24.613 | 16.184 | 1.00 | 23.65 | B |
| ATOM | 2056 | CE2 | PHE | B | 72 | 10.093 | -23.301 | 18.182 | 1.00 | 25.67 | B |
| ATOM | 2057 | CZ | PHE | B | 72 | 10.156 | -23.385 | 16.796 | 1.00 | 24.39 | B |
| ATOM | 2058 | C | PHE | B | 72 | 12.580 | -27.924 | 20.580 | 1.00 | 28.85 | B |
| ATOM | 2059 | O | PHE | B | 72 | 13.218 | -27.256 | 21.396 | 1.00 | 29.18 | B |
| ATOM | 2060 | N | THR | B | 73 | 12.161 | -29.159 | 20.835 | 1.00 | 29.69 | B |
| ATOM | 2061 | CA | THR | B | 73 | 12.430 | -29.783 | 22.123 | 1.00 | 29.34 | B |
| ATOM | 2062 | CB | THR | B | 73 | 11.827 | -31.200 | 22.196 | 1.00 | 29.95 | B |
| ATOM | 2063 | OG1 | THR | B | 73 | 10.398 | -31.096 | 22.254 | 1.00 | 29.29 | B |
| ATOM | 2064 | CG2 | THR | B | 73 | 12.324 | -31.937 | 23.445 | 1.00 | 28.72 | B |
| ATOM | 2065 | C | THR | B | 73 | 13.920 | -29.835 | 22.439 | 1.00 | 28.65 | B |
| ATOM | 2066 | O | THR | B | 73 | 14.317 | -29.619 | 23.581 | 1.00 | 28.62 | B |
| ATOM | 2067 | N | ALA | B | 74 | 14.742 | -30.112 | 21.434 | 1.00 | 28.15 | B |
| ATOM | 2068 | CA | ALA | B | 74 | 16.186 | -30.161 | 21.643 | 1.00 | 29.78 | B |
| ATOM | 2069 | CB | ALA | B | 74 | 16.889 | -30.604 | 20.377 | 1.00 | 28.12 | B |
| ATOM | 2070 | C | ALA | B | 74 | 16.675 | -28.773 | 22.054 | 1.00 | 31.71 | B |
| ATOM | 2071 | O | ALA | B | 74 | 17.393 | -28.621 | 23.048 | 1.00 | 33.87 | B |
| ATOM | 2072 | N | VAL | B | 75 | 16.268 | -27.764 | 21.289 | 1.00 | 31.11 | B |
| ATOM | 2073 | CA | VAL | B | 75 | 16.650 | -26.390 | 21.562 | 1.00 | 31.07 | B |
| ATOM | 2074 | CB | VAL | B | 75 | 16.048 | -25.441 | 20.496 | 1.00 | 29.90 | B |
| ATOM | 2075 | CG1 | VAL | B | 75 | 16.226 | -23.993 | 20.905 | 1.00 | 27.82 | B |
| ATOM | 2076 | CG2 | VAL | B | 75 | 16.724 | -25.688 | 19.160 | 1.00 | 29.38 | B |
| ATOM | 2077 | C | VAL | B | 75 | 16.207 | -25.947 | 22.956 | 1.00 | 31.82 | B |
| ATOM | 2078 | O | VAL | B | 75 | 16.986 | -25.369 | 23.707 | 1.00 | 31.20 | B |
| ATOM | 2079 | N | GLU | B | 76 | 14.957 | -26.225 | 23.303 | 1.00 | 33.28 | B |
| ATOM | 2080 | CA | GLU | B | 76 | 14.441 | -25.828 | 24.606 | 1.00 | 35.53 | B |
| ATOM | 2081 | CB | GLU | B | 76 | 13.011 | -26.336 | 24.776 | 1.00 | 37.17 | B |
| ATOM | 2082 | CG | GLU | B | 76 | 12.074 | -25.893 | 23.660 | 1.00 | 40.82 | B |

Figure 2 (suite 28)

| | | | | | | | | | | | |
|---|---|---|---|---|---|---|---|---|---|---|---|
| ATOM | 2083 | CD | GLU | B | 76 | 10.804 | -26.729 | 23.586 | 1.00 | 43.67 | B |
| ATOM | 2084 | OE1 | GLU | B | 76 | 10.007 | -26.706 | 24.550 | 1.00 | 44.86 | B |
| ATOM | 2085 | OE2 | GLU | B | 76 | 10.607 | -27.415 | 22.560 | 1.00 | 44.65 | B |
| ATOM | 2086 | C | GLU | B | 76 | 15.323 | -26.357 | 25.733 | 1.00 | 36.71 | B |
| ATOM | 2087 | O | GLU | B | 76 | 15.688 | -25.618 | 26.651 | 1.00 | 36.12 | B |
| ATOM | 2088 | N | GLU | B | 77 | 15.679 | -27.635 | 25.660 | 1.00 | 37.24 | B |
| ATOM | 2089 | CA | GLU | B | 77 | 16.514 | -28.221 | 26.696 | 1.00 | 38.03 | B |
| ATOM | 2090 | CB | GLU | B | 77 | 16.627 | -29.732 | 26.495 | 1.00 | 40.95 | B |
| ATOM | 2091 | CG | GLU | B | 77 | 15.359 | -30.486 | 26.880 | 1.00 | 45.32 | B |
| ATOM | 2092 | CD | GLU | B | 77 | 15.466 | -31.977 | 26.626 | 1.00 | 48.12 | B |
| ATOM | 2093 | OE1 | GLU | B | 77 | 16.341 | -32.622 | 27.247 | 1.00 | 50.98 | B |
| ATOM | 2094 | OE2 | GLU | B | 77 | 14.677 | -32.500 | 25.803 | 1.00 | 49.22 | B |
| ATOM | 2095 | C | GLU | B | 77 | 17.897 | -27.588 | 26.727 | 1.00 | 36.31 | B |
| ATOM | 2096 | O | GLU | B | 77 | 18.394 | -27.216 | 27.790 | 1.00 | 37.14 | B |
| ATOM | 2097 | N | HIS | B | 78 | 18.508 | -27.452 | 25.560 | 1.00 | 33.62 | B |
| ATOM | 2098 | CA | HIS | B | 78 | 19.833 | -26.871 | 25.479 | 1.00 | 32.19 | B |
| ATOM | 2099 | CB | HIS | B | 78 | 20.307 | -26.829 | 24.026 | 1.00 | 33.08 | B |
| ATOM | 2100 | CG | HIS | B | 78 | 21.600 | -26.097 | 23.826 | 1.00 | 33.30 | B |
| ATOM | 2101 | CD2 | HIS | B | 78 | 21.858 | -24.785 | 23.608 | 1.00 | 33.71 | B |
| ATOM | 2102 | ND1 | HIS | B | 78 | 22.824 | -26.730 | 23.828 | 1.00 | 34.91 | B |
| ATOM | 2103 | CE1 | HIS | B | 78 | 23.779 | -25.842 | 23.618 | 1.00 | 34.10 | B |
| ATOM | 2104 | NE2 | HIS | B | 78 | 23.220 | -24.654 | 23.481 | 1.00 | 34.12 | B |
| ATOM | 2105 | C | HIS | B | 78 | 19.894 | -25.466 | 26.049 | 1.00 | 31.22 | B |
| ATOM | 2106 | O | HIS | B | 78 | 20.552 | -25.230 | 27.057 | 1.00 | 33.16 | B |
| ATOM | 2107 | N | GLN | B | 79 | 19.183 | -24.545 | 25.409 | 1.00 | 29.23 | B |
| ATOM | 2108 | CA | GLN | B | 79 | 19.202 | -23.133 | 25.781 | 1.00 | 26.12 | B |
| ATOM | 2109 | CB | GLN | B | 79 | 19.660 | -22.324 | 24.565 | 1.00 | 27.20 | B |
| ATOM | 2110 | CG | GLN | B | 79 | 18.775 | -22.578 | 23.346 | 1.00 | 24.01 | B |
| ATOM | 2111 | CD | GLN | B | 79 | 19.067 | -21.666 | 22.187 | 1.00 | 22.51 | B |
| ATOM | 2112 | OE1 | GLN | B | 79 | 20.123 | -21.747 | 21.552 | 1.00 | 19.59 | B |
| ATOM | 2113 | NE2 | GLN | B | 79 | 18.122 | -20.781 | 21.896 | 1.00 | 23.88 | B |
| ATOM | 2114 | C | GLN | B | 79 | 17.922 | -22.501 | 26.316 | 1.00 | 24.02 | B |
| ATOM | 2115 | O | GLN | B | 79 | 17.899 | -21.306 | 26.571 | 1.00 | 24.79 | B |
| ATOM | 2116 | N | GLY | B | 80 | 16.857 | -23.268 | 26.478 | 1.00 | 23.05 | B |
| ATOM | 2117 | CA | GLY | B | 80 | 15.626 | -22.671 | 26.979 | 1.00 | 23.13 | B |
| ATOM | 2118 | C | GLY | B | 80 | 14.559 | -22.409 | 25.913 | 1.00 | 23.96 | B |
| ATOM | 2119 | O | GLY | B | 80 | 14.853 | -22.370 | 24.712 | 1.00 | 23.18 | B |
| ATOM | 2120 | N | PRO | B | 81 | 13.294 | -22.233 | 26.328 | 1.00 | 24.17 | B |
| ATOM | 2121 | CD | PRO | B | 81 | 12.840 | -22.196 | 27.729 | 1.00 | 23.01 | B |
| ATOM | 2122 | CA | PRO | B | 81 | 12.175 | -21.973 | 25.411 | 1.00 | 24.58 | B |
| ATOM | 2123 | CB | PRO | B | 81 | 11.086 | -21.457 | 26.346 | 1.00 | 24.28 | B |
| ATOM | 2124 | CG | PRO | B | 81 | 11.335 | -22.239 | 27.585 | 1.00 | 23.16 | B |
| ATOM | 2125 | C | PRO | B | 81 | 12.529 | -20.945 | 24.334 | 1.00 | 24.76 | B |
| ATOM | 2126 | O | PRO | B | 81 | 13.153 | -19.924 | 24.632 | 1.00 | 26.08 | B |
| ATOM | 2127 | N | VAL | B | 82 | 12.144 | -21.214 | 23.091 | 1.00 | 23.35 | B |
| ATOM | 2128 | CA | VAL | B | 82 | 12.414 | -20.281 | 22.005 | 1.00 | 20.74 | B |
| ATOM | 2129 | CB | VAL | B | 82 | 12.016 | -20.890 | 20.655 | 1.00 | 21.39 | B |
| ATOM | 2130 | CG1 | VAL | B | 82 | 12.071 | -19.835 | 19.561 | 1.00 | 19.50 | B |
| ATOM | 2131 | CG2 | VAL | B | 82 | 12.948 | -22.040 | 20.331 | 1.00 | 19.90 | B |
| ATOM | 2132 | C | VAL | B | 82 | 11.627 | -18.988 | 22.249 | 1.00 | 20.50 | B |
| ATOM | 2133 | O | VAL | B | 82 | 10.430 | -19.023 | 22.543 | 1.00 | 19.83 | B |
| ATOM | 2134 | N | GLU | B | 83 | 12.321 | -17.854 | 22.136 | 1.00 | 19.98 | B |
| ATOM | 2135 | CA | GLU | B | 83 | 11.744 | -16.532 | 22.366 | 1.00 | 17.65 | B |
| ATOM | 2136 | CB | GLU | B | 83 | 12.618 | -15.757 | 23.341 | 1.00 | 17.75 | B |
| ATOM | 2137 | CG | GLU | B | 83 | 12.615 | -16.329 | 24.747 | 1.00 | 19.45 | B |
| ATOM | 2138 | CD | GLU | B | 83 | 13.552 | -15.570 | 25.681 | 1.00 | 19.87 | B |
| ATOM | 2139 | OE1 | GLU | B | 83 | 14.792 | -15.652 | 25.490 | 1.00 | 18.89 | B |
| ATOM | 2140 | OE2 | GLU | B | 83 | 13.042 | -14.885 | 26.594 | 1.00 | 17.85 | B |
| ATOM | 2141 | C | GLU | B | 83 | 11.592 | -15.723 | 21.088 | 1.00 | 16.60 | B |
| ATOM | 2142 | O | GLU | B | 83 | 10.609 | -15.015 | 20.904 | 1.00 | 16.28 | B |
| ATOM | 2143 | N | VAL | B | 84 | 12.585 | -15.799 | 20.216 | 1.00 | 14.10 | B |
| ATOM | 2144 | CA | VAL | B | 84 | 12.500 | -15.089 | 18.967 | 1.00 | 14.37 | B |
| ATOM | 2145 | CB | VAL | B | 84 | 13.442 | -13.833 | 18.909 | 1.00 | 14.20 | B |
| ATOM | 2146 | CG1 | VAL | B | 84 | 14.070 | -13.587 | 20.257 | 1.00 | 15.04 | B |
| ATOM | 2147 | CG2 | VAL | B | 84 | 14.493 | -13.983 | 17.825 | 1.00 | 11.88 | B |
| ATOM | 2148 | C | VAL | B | 84 | 12.849 | -16.105 | 17.896 | 1.00 | 15.83 | B |
| ATOM | 2149 | O | VAL | B | 84 | 13.919 | -16.724 | 17.917 | 1.00 | 15.59 | B |
| ATOM | 2150 | N | LEU | B | 85 | 11.900 | -16.288 | 16.981 | 1.00 | 16.05 | B |
| ATOM | 2151 | CA | LEU | B | 85 | 12.015 | -17.224 | 15.874 | 1.00 | 15.31 | B |
| ATOM | 2152 | CB | LEU | B | 85 | 10.694 | -17.971 | 15.714 | 1.00 | 15.38 | B |
| ATOM | 2153 | CG | LEU | B | 85 | 10.497 | -18.818 | 14.457 | 1.00 | 18.06 | B |
| ATOM | 2154 | CD1 | LEU | B | 85 | 11.457 | -19.994 | 14.439 | 1.00 | 17.56 | B |
| ATOM | 2155 | CD2 | LEU | B | 85 | 9.064 | -19.308 | 14.441 | 1.00 | 20.33 | B |

Figure 2 (suite 29)

| | | | | | | | | | | | |
|---|---|---|---|---|---|---|---|---|---|---|---|
| ATOM | 2156 | C | LEU | B | 85 | 12.338 | -16.489 | 14.583 | 1.00 | 14.07 | B |
| ATOM | 2157 | O | LEU | B | 85 | 11.667 | -15.528 | 14.233 | 1.00 | 14.43 | B |
| ATOM | 2158 | N | VAL | B | 86 | 13.376 | -16.928 | 13.885 | 1.00 | 12.55 | B |
| ATOM | 2159 | CA | VAL | B | 86 | 13.748 | -16.306 | 12.624 | 1.00 | 12.50 | B |
| ATOM | 2160 | CB | VAL | B | 86 | 15.219 | -15.842 | 12.638 | 1.00 | 11.63 | B |
| ATOM | 2161 | CG1 | VAL | B | 86 | 15.594 | -15.236 | 11.300 | 1.00 | 8.49 | B |
| ATOM | 2162 | CG2 | VAL | B | 86 | 15.423 | -14.829 | 13.750 | 1.00 | 8.13 | B |
| ATOM | 2163 | C | VAL | B | 86 | 13.544 | -17.384 | 11.574 | 1.00 | 14.82 | B |
| ATOM | 2164 | O | VAL | B | 86 | 14.376 | -18.282 | 11.420 | 1.00 | 14.49 | B |
| ATOM | 2165 | N | SER | B | 87 | 12.411 | -17.289 | 10.874 | 1.00 | 17.45 | B |
| ATOM | 2166 | CA | SER | B | 87 | 12.003 | -18.236 | 9.841 | 1.00 | 17.74 | B |
| ATOM | 2167 | CB | SER | B | 87 | 10.479 | -18.300 | 9.793 | 1.00 | 18.64 | B |
| ATOM | 2168 | OG | SER | B | 87 | 10.039 | -19.167 | 8.764 | 1.00 | 23.03 | B |
| ATOM | 2169 | C | SER | B | 87 | 12.535 | -17.896 | 8.461 | 1.00 | 18.79 | B |
| ATOM | 2170 | O | SER | B | 87 | 11.988 | -17.047 | 7.771 | 1.00 | 19.58 | B |
| ATOM | 2171 | N | ASN | B | 88 | 13.598 | -18.579 | 8.060 | 1.00 | 20.59 | B |
| ATOM | 2172 | CA | ASN | B | 88 | 14.215 | -18.363 | 6.763 | 1.00 | 23.45 | B |
| ATOM | 2173 | CB | ASN | B | 88 | 15.700 | -18.713 | 6.837 | 1.00 | 24.25 | B |
| ATOM | 2174 | CG | ASN | B | 88 | 16.579 | -17.646 | 6.251 | 1.00 | 27.69 | B |
| ATOM | 2175 | OD1 | ASN | B | 88 | 16.629 | -16.529 | 6.753 | 1.00 | 31.82 | B |
| ATOM | 2176 | ND2 | ASN | B | 88 | 17.286 | -17.981 | 5.183 | 1.00 | 31.23 | B |
| ATOM | 2177 | C | ASN | B | 88 | 13.528 | -19.249 | 5.728 | 1.00 | 25.95 | B |
| ATOM | 2178 | O | ASN | B | 88 | 13.368 | -20.459 | 5.936 | 1.00 | 27.50 | B |
| ATOM | 2179 | N | ALA | B | 89 | 13.127 | -18.655 | 4.610 | 1.00 | 27.96 | B |
| ATOM | 2180 | CA | ALA | B | 89 | 12.450 | -19.408 | 3.558 | 1.00 | 30.43 | B |
| ATOM | 2181 | CB | ALA | B | 89 | 11.830 | -18.444 | 2.561 | 1.00 | 29.36 | B |
| ATOM | 2182 | C | ALA | B | 89 | 13.373 | -20.394 | 2.830 | 1.00 | 32.48 | B |
| ATOM | 2183 | O | ALA | B | 89 | 14.409 | -20.002 | 2.290 | 1.00 | 33.08 | B |
| ATOM | 2184 | N | GLY | B | 90 | 12.986 | -21.670 | 2.816 | 1.00 | 33.96 | B |
| ATOM | 2185 | CA | GLY | B | 90 | 13.774 | -22.693 | 2.140 | 1.00 | 36.27 | B |
| ATOM | 2186 | C | GLY | B | 90 | 14.190 | -22.335 | 0.717 | 1.00 | 38.99 | B |
| ATOM | 2187 | O | GLY | B | 90 | 13.513 | -22.662 | -0.268 | 1.00 | 39.85 | B |
| ATOM | 2188 | N | MET | B | 100 | 7.253 | -24.765 | -15.031 | 1.00 | 39.53 | B |
| ATOM | 2189 | CA | MET | B | 100 | 6.340 | -24.640 | -13.902 | 1.00 | 38.51 | B |
| ATOM | 2190 | CB | MET | B | 100 | 5.615 | -23.296 | -13.951 | 1.00 | 38.23 | B |
| ATOM | 2191 | CG | MET | B | 100 | 4.827 | -22.971 | -12.690 | 1.00 | 39.50 | B |
| ATOM | 2192 | SD | MET | B | 100 | 5.864 | -22.941 | -11.205 | 1.00 | 40.84 | B |
| ATOM | 2193 | CE | MET | B | 100 | 5.214 | -24.331 | -10.332 | 1.00 | 40.16 | B |
| ATOM | 2194 | C | MET | B | 100 | 5.323 | -25.770 | -13.943 | 1.00 | 38.29 | B |
| ATOM | 2195 | O | MET | B | 100 | 4.488 | -25.830 | -14.846 | 1.00 | 38.89 | B |
| ATOM | 2196 | N | THR | B | 101 | 5.393 | -26.660 | -12.957 | 1.00 | 37.05 | B |
| ATOM | 2197 | CA | THR | B | 101 | 4.488 | -27.800 | -12.888 | 1.00 | 35.82 | B |
| ATOM | 2198 | CB | THR | B | 101 | 5.269 | -29.133 | -12.958 | 1.00 | 36.64 | B |
| ATOM | 2199 | OG1 | THR | B | 101 | 6.026 | -29.309 | -11.752 | 1.00 | 37.40 | B |
| ATOM | 2200 | CG2 | THR | B | 101 | 6.229 | -29.131 | -14.144 | 1.00 | 35.93 | B |
| ATOM | 2201 | C | THR | B | 101 | 3.698 | -27.779 | -11.589 | 1.00 | 35.38 | B |
| ATOM | 2202 | O | THR | B | 101 | 4.026 | -27.035 | -10.668 | 1.00 | 34.76 | B |
| ATOM | 2203 | N | GLU | B | 102 | 2.657 | -28.602 | -11.518 | 1.00 | 34.88 | B |
| ATOM | 2204 | CA | GLU | B | 102 | 1.833 | -28.681 | -10.322 | 1.00 | 36.07 | B |
| ATOM | 2205 | CB | GLU | B | 102 | 0.633 | -29.597 | -10.569 | 1.00 | 36.57 | B |
| ATOM | 2206 | CG | GLU | B | 102 | -0.192 | -29.886 | -9.321 | 1.00 | 40.25 | B |
| ATOM | 2207 | CD | GLU | B | 102 | -1.539 | -30.517 | -9.639 | 1.00 | 42.60 | B |
| ATOM | 2208 | OE1 | GLU | B | 102 | -2.283 | -29.933 | -10.461 | 1.00 | 45.67 | B |
| ATOM | 2209 | OE2 | GLU | B | 102 | -1.858 | -31.582 | -9.066 | 1.00 | 41.25 | B |
| ATOM | 2210 | C | GLU | B | 102 | 2.664 | -29.215 | -9.155 | 1.00 | 37.17 | B |
| ATOM | 2211 | O | GLU | B | 102 | 2.432 | -28.872 | -7.994 | 1.00 | 36.43 | B |
| ATOM | 2212 | N | GLU | B | 103 | 3.636 | -30.063 | -9.473 | 1.00 | 37.95 | B |
| ATOM | 2213 | CA | GLU | B | 103 | 4.499 | -30.636 | -8.452 | 1.00 | 37.38 | B |
| ATOM | 2214 | CB | GLU | B | 103 | 5.300 | -31.812 | -9.035 | 1.00 | 39.40 | B |
| ATOM | 2215 | CG | GLU | B | 103 | 6.001 | -32.681 | -7.988 | 1.00 | 44.78 | B |
| ATOM | 2216 | CD | GLU | B | 103 | 6.695 | -33.916 | -8.580 | 1.00 | 47.37 | B |
| ATOM | 2217 | OE1 | GLU | B | 103 | 7.648 | -33.758 | -9.381 | 1.00 | 48.17 | B |
| ATOM | 2218 | OE2 | GLU | B | 103 | 6.283 | -35.048 | -8.236 | 1.00 | 47.89 | B |
| ATOM | 2219 | C | GLU | B | 103 | 5.429 | -29.531 | -7.945 | 1.00 | 35.61 | B |
| ATOM | 2220 | O | GLU | B | 103 | 5.593 | -29.351 | -6.738 | 1.00 | 34.94 | B |
| ATOM | 2221 | N | LYS | B | 104 | 6.024 | -28.778 | -8.866 | 1.00 | 33.60 | B |
| ATOM | 2222 | CA | LYS | B | 104 | 6.916 | -27.699 | -8.466 | 1.00 | 32.55 | B |
| ATOM | 2223 | CB | LYS | B | 104 | 7.513 | -26.989 | -9.683 | 1.00 | 33.69 | B |
| ATOM | 2224 | CG | LYS | B | 104 | 8.687 | -27.720 | -10.304 | 1.00 | 38.07 | B |
| ATOM | 2225 | CD | LYS | B | 104 | 9.646 | -26.760 | -11.003 | 1.00 | 41.33 | B |
| ATOM | 2226 | CE | LYS | B | 104 | 8.979 | -26.026 | -12.160 | 1.00 | 46.12 | B |
| ATOM | 2227 | NZ | LYS | B | 104 | 8.473 | -26.948 | -13.230 | 1.00 | 48.48 | B |
| ATOM | 2228 | C | LYS | B | 104 | 6.186 | -26.688 | -7.597 | 1.00 | 31.34 | B |

Figure 2 (suite 30)

| | | | | | | | | | | | |
|---|---|---|---|---|---|---|---|---|---|---|---|
| ATOM | 2229 | O | LYS | B | 104 | 6.749 | -26.177 | -6.630 | 1.00 | 33.28 | B |
| ATOM | 2230 | N | PHE | B | 105 | 4.929 | -26.406 | -7.931 | 1.00 | 28.58 | B |
| ATOM | 2231 | CA | PHE | B | 105 | 4.143 | -25.447 | -7.163 | 1.00 | 26.13 | B |
| ATOM | 2232 | CB | PHE | B | 105 | 2.763 | -25.233 | -7.800 | 1.00 | 22.29 | B |
| ATOM | 2233 | CG | PHE | B | 105 | 2.013 | -24.070 | -7.224 | 1.00 | 18.50 | B |
| ATOM | 2234 | CD1 | PHE | B | 105 | 2.270 | -22.776 | -7.665 | 1.00 | 18.93 | B |
| ATOM | 2235 | CD2 | PHE | B | 105 | 1.087 | -24.258 | -6.213 | 1.00 | 17.84 | B |
| ATOM | 2236 | CE1 | PHE | B | 105 | 1.612 | -21.683 | -7.098 | 1.00 | 17.45 | B |
| ATOM | 2237 | CE2 | PHE | B | 105 | 0.424 | -23.173 | -5.636 | 1.00 | 18.18 | B |
| ATOM | 2238 | CZ | PHE | B | 105 | 0.687 | -21.882 | -6.081 | 1.00 | 16.21 | B |
| ATOM | 2239 | C | PHE | B | 105 | 3.954 | -25.909 | -5.721 | 1.00 | 26.78 | B |
| ATOM | 2240 | O | PHE | B | 105 | 4.250 | -25.174 | -4.778 | 1.00 | 26.56 | B |
| ATOM | 2241 | N | GLU | B | 106 | 3.452 | -27.129 | -5.556 | 1.00 | 26.98 | B |
| ATOM | 2242 | CA | GLU | B | 106 | 3.210 | -27.675 | -4.229 | 1.00 | 27.72 | B |
| ATOM | 2243 | CB | GLU | B | 106 | 2.518 | -29.037 | -4.321 | 1.00 | 27.66 | B |
| ATOM | 2244 | CG | GLU | B | 106 | 1.258 | -29.069 | -5.160 | 1.00 | 32.00 | B |
| ATOM | 2245 | CD | GLU | B | 106 | 0.383 | -30.275 | -4.838 | 1.00 | 34.67 | B |
| ATOM | 2246 | OE1 | GLU | B | 106 | -0.304 | -30.244 | -3.790 | 1.00 | 36.83 | B |
| ATOM | 2247 | OE2 | GLU | B | 106 | 0.389 | -31.253 | -5.620 | 1.00 | 35.41 | B |
| ATOM | 2248 | C | GLU | B | 106 | 4.491 | -27.825 | -3.407 | 1.00 | 27.40 | B |
| ATOM | 2249 | O | GLU | B | 106 | 4.461 | -27.704 | -2.183 | 1.00 | 27.59 | B |
| ATOM | 2250 | N | LYS | B | 107 | 5.613 | -28.091 | -4.067 | 1.00 | 26.39 | B |
| ATOM | 2251 | CA | LYS | B | 107 | 6.858 | -28.263 | -3.333 | 1.00 | 27.37 | B |
| ATOM | 2252 | CB | LYS | B | 107 | 7.985 | -28.694 | -4.274 | 1.00 | 30.80 | B |
| ATOM | 2253 | CG | LYS | B | 107 | 9.324 | -28.893 | -3.567 | 1.00 | 36.49 | B |
| ATOM | 2254 | CD | LYS | B | 107 | 10.335 | -29.664 | -4.426 | 1.00 | 39.01 | B |
| ATOM | 2255 | CE | LYS | B | 107 | 10.693 | -28.919 | -5.702 | 1.00 | 41.16 | B |
| ATOM | 2256 | NZ | LYS | B | 107 | 11.609 | -29.711 | -6.576 | 1.00 | 41.28 | B |
| ATOM | 2257 | C | LYS | B | 107 | 7.224 | -26.967 | -2.617 | 1.00 | 26.14 | B |
| ATOM | 2258 | O | LYS | B | 107 | 7.330 | -26.936 | -1.386 | 1.00 | 25.42 | B |
| ATOM | 2259 | N | VAL | B | 108 | 7.393 | -25.896 | -3.388 | 1.00 | 23.44 | B |
| ATOM | 2260 | CA | VAL | B | 108 | 7.732 | -24.598 | -2.827 | 1.00 | 21.39 | B |
| ATOM | 2261 | CB | VAL | B | 108 | 7.881 | -23.571 | -3.961 | 1.00 | 20.59 | B |
| ATOM | 2262 | CG1 | VAL | B | 108 | 7.864 | -22.155 | -3.409 | 1.00 | 17.73 | B |
| ATOM | 2263 | CG2 | VAL | B | 108 | 9.174 | -23.845 | -4.716 | 1.00 | 17.57 | B |
| ATOM | 2264 | C | VAL | B | 108 | 6.713 | -24.104 | -1.779 | 1.00 | 21.97 | B |
| ATOM | 2265 | O | VAL | B | 108 | 7.093 | -23.555 | -0.737 | 1.00 | 22.12 | B |
| ATOM | 2266 | N | ILE | B | 109 | 5.428 | -24.307 | -2.046 | 1.00 | 20.56 | B |
| ATOM | 2267 | CA | ILE | B | 109 | 4.384 | -23.884 | -1.117 | 1.00 | 22.01 | B |
| ATOM | 2268 | CB | ILE | B | 109 | 2.949 | -24.086 | -1.723 | 1.00 | 21.91 | B |
| ATOM | 2269 | CG2 | ILE | B | 109 | 1.877 | -23.870 | -0.658 | 1.00 | 18.94 | B |
| ATOM | 2270 | CG1 | ILE | B | 109 | 2.726 | -23.117 | -2.884 | 1.00 | 21.87 | B |
| ATOM | 2271 | CD | ILE | B | 109 | 2.757 | -21.653 | -2.476 | 1.00 | 22.52 | B |
| ATOM | 2272 | C | ILE | B | 109 | 4.475 | -24.675 | 0.184 | 1.00 | 23.14 | B |
| ATOM | 2273 | O | ILE | B | 109 | 4.334 | -24.121 | 1.274 | 1.00 | 25.00 | B |
| ATOM | 2274 | N | ASN | B | 110 | 4.711 | -25.976 | 0.061 | 1.00 | 23.16 | B |
| ATOM | 2275 | CA | ASN | B | 110 | 4.808 | -26.860 | 1.215 | 1.00 | 21.91 | B |
| ATOM | 2276 | CB | ASN | B | 110 | 4.919 | -28.305 | 0.733 | 1.00 | 21.65 | B |
| ATOM | 2277 | CG | ASN | B | 110 | 4.845 | -29.303 | 1.863 | 1.00 | 23.14 | B |
| ATOM | 2278 | OD1 | ASN | B | 110 | 3.994 | -29.203 | 2.750 | 1.00 | 24.10 | B |
| ATOM | 2279 | ND2 | ASN | B | 110 | 5.733 | -30.288 | 1.833 | 1.00 | 25.64 | B |
| ATOM | 2280 | C | ASN | B | 110 | 6.007 | -26.502 | 2.089 | 1.00 | 21.80 | B |
| ATOM | 2281 | O | ASN | B | 110 | 5.903 | -26.439 | 3.313 | 1.00 | 21.93 | B |
| ATOM | 2282 | N | ALA | B | 111 | 7.144 | -26.259 | 1.456 | 1.00 | 21.36 | B |
| ATOM | 2283 | CA | ALA | B | 111 | 8.349 | -25.911 | 2.187 | 1.00 | 22.50 | B |
| ATOM | 2284 | CB | ALA | B | 111 | 9.551 | -25.995 | 1.259 | 1.00 | 20.09 | B |
| ATOM | 2285 | C | ALA | B | 111 | 8.262 | -24.512 | 2.813 | 1.00 | 24.13 | B |
| ATOM | 2286 | O | ALA | B | 111 | 8.165 | -24.368 | 4.037 | 1.00 | 24.28 | B |
| ATOM | 2287 | N | ASN | B | 112 | 8.274 | -23.497 | 1.951 | 1.00 | 24.68 | B |
| ATOM | 2288 | CA | ASN | B | 112 | 8.233 | -22.089 | 2.347 | 1.00 | 23.60 | B |
| ATOM | 2289 | CB | ASN | B | 112 | 8.477 | -21.218 | 1.117 | 1.00 | 23.62 | B |
| ATOM | 2290 | CG | ASN | B | 112 | 9.798 | -21.514 | 0.455 | 1.00 | 25.80 | B |
| ATOM | 2291 | OD1 | ASN | B | 112 | 10.077 | -21.047 | -0.652 | 1.00 | 28.50 | B |
| ATOM | 2292 | ND2 | ASN | B | 112 | 10.630 | -22.296 | 1.133 | 1.00 | 25.82 | B |
| ATOM | 2293 | C | ASN | B | 112 | 6.991 | -21.564 | 3.069 | 1.00 | 22.81 | B |
| ATOM | 2294 | O | ASN | B | 112 | 7.109 | -20.780 | 4.008 | 1.00 | 24.31 | B |
| ATOM | 2295 | N | LEU | B | 113 | 5.804 | -21.978 | 2.648 | 1.00 | 21.52 | B |
| ATOM | 2296 | CA | LEU | B | 113 | 4.600 | -21.455 | 3.279 | 1.00 | 20.08 | B |
| ATOM | 2297 | CB | LEU | B | 113 | 3.501 | -21.280 | 2.231 | 1.00 | 19.46 | B |
| ATOM | 2298 | CG | LEU | B | 113 | 2.187 | -20.674 | 2.708 | 1.00 | 19.09 | B |
| ATOM | 2299 | CD1 | LEU | B | 113 | 2.443 | -19.349 | 3.380 | 1.00 | 20.36 | B |
| ATOM | 2300 | CD2 | LEU | B | 113 | 1.265 | -20.498 | 1.514 | 1.00 | 21.04 | B |
| ATOM | 2301 | C | LEU | B | 113 | 4.087 | -22.275 | 4.445 | 1.00 | 20.60 | B |

Figure 2 (suite 31)

| | | | | | | | | | | | |
|---|---|---|---|---|---|---|---|---|---|---|---|
| ATOM | 2302 | O | LEU | B | 113 | 3.943 | -21.763 | 5.550 | 1.00 | 20.67 | B |
| ATOM | 2303 | N | THR | B | 114 | 3.793 | -23.545 | 4.214 | 1.00 | 20.54 | B |
| ATOM | 2304 | CA | THR | B | 114 | 3.314 | -24.377 | 5.305 | 1.00 | 20.68 | B |
| ATOM | 2305 | CB | THR | B | 114 | 2.679 | -25.669 | 4.774 | 1.00 | 21.19 | B |
| ATOM | 2306 | OG1 | THR | B | 114 | 1.557 | -25.324 | 3.951 | 1.00 | 21.25 | B |
| ATOM | 2307 | CG2 | THR | B | 114 | 2.199 | -26.542 | 5.927 | 1.00 | 20.80 | B |
| ATOM | 2308 | C | THR | B | 114 | 4.479 | -24.680 | 6.253 | 1.00 | 19.38 | B |
| ATOM | 2309 | O | THR | B | 114 | 4.283 | -24.944 | 7.443 | 1.00 | 17.72 | B |
| ATOM | 2310 | N | GLY | B | 115 | 5.695 | -24.631 | 5.722 | 1.00 | 19.25 | B |
| ATOM | 2311 | CA | GLY | B | 115 | 6.852 | -24.841 | 6.568 | 1.00 | 18.96 | B |
| ATOM | 2312 | C | GLY | B | 115 | 6.806 | -23.735 | 7.612 | 1.00 | 19.07 | B |
| ATOM | 2313 | O | GLY | B | 115 | 6.782 | -23.994 | 8.821 | 1.00 | 19.57 | B |
| ATOM | 2314 | N | ALA | B | 116 | 6.758 | -22.490 | 7.140 | 1.00 | 17.44 | B |
| ATOM | 2315 | CA | ALA | B | 116 | 6.703 | -21.350 | 8.040 | 1.00 | 17.21 | B |
| ATOM | 2316 | CB | ALA | B | 116 | 6.546 | -20.063 | 7.248 | 1.00 | 15.20 | B |
| ATOM | 2317 | C | ALA | B | 116 | 5.565 | -21.490 | 9.046 | 1.00 | 17.74 | B |
| ATOM | 2318 | O | ALA | B | 116 | 5.699 | -21.089 | 10.199 | 1.00 | 18.58 | B |
| ATOM | 2319 | N | PHE | B | 117 | 4.448 | -22.071 | 8.616 | 1.00 | 17.92 | B |
| ATOM | 2320 | CA | PHE | B | 117 | 3.299 | -22.237 | 9.500 | 1.00 | 17.53 | B |
| ATOM | 2321 | CB | PHE | B | 117 | 2.067 | -22.734 | 8.717 | 1.00 | 16.05 | B |
| ATOM | 2322 | CG | PHE | B | 117 | 0.951 | -23.214 | 9.598 | 1.00 | 12.30 | B |
| ATOM | 2323 | CD1 | PHE | B | 117 | 0.186 | -22.316 | 10.326 | 1.00 | 13.26 | B |
| ATOM | 2324 | CD2 | PHE | B | 117 | 0.740 | -24.571 | 9.783 | 1.00 | 14.73 | B |
| ATOM | 2325 | CE1 | PHE | B | 117 | -0.767 | -22.756 | 11.237 | 1.00 | 13.80 | B |
| ATOM | 2326 | CE2 | PHE | B | 117 | -0.214 | -25.031 | 10.696 | 1.00 | 14.70 | B |
| ATOM | 2327 | CZ | PHE | B | 117 | -0.968 | -24.118 | 11.426 | 1.00 | 15.29 | B |
| ATOM | 2328 | C | PHE | B | 117 | 3.618 | -23.220 | 10.610 | 1.00 | 19.06 | B |
| ATOM | 2329 | O | PHE | B | 117 | 3.255 | -23.002 | 11.768 | 1.00 | 19.44 | B |
| ATOM | 2330 | N | ARG | B | 118 | 4.295 | -24.306 | 10.254 | 1.00 | 19.60 | B |
| ATOM | 2331 | CA | ARG | B | 118 | 4.643 | -25.325 | 11.237 | 1.00 | 20.74 | B |
| ATOM | 2332 | CB | ARG | B | 118 | 5.338 | -26.506 | 10.554 | 1.00 | 21.41 | B |
| ATOM | 2333 | CG | ARG | B | 118 | 4.458 | -27.203 | 9.520 | 1.00 | 24.84 | B |
| ATOM | 2334 | CD | ARG | B | 118 | 5.038 | -28.532 | 9.048 | 1.00 | 26.56 | B |
| ATOM | 2335 | NE | ARG | B | 118 | 4.045 | -29.289 | 8.292 | 1.00 | 28.93 | B |
| ATOM | 2336 | CZ | ARG | B | 118 | 3.892 | -29.242 | 6.971 | 1.00 | 30.02 | B |
| ATOM | 2337 | NH1 | ARG | B | 118 | 4.677 | -28.476 | 6.220 | 1.00 | 27.45 | B |
| ATOM | 2338 | NH2 | ARG | B | 118 | 2.932 | -29.960 | 6.401 | 1.00 | 30.83 | B |
| ATOM | 2339 | C | ARG | B | 118 | 5.524 | -24.778 | 12.355 | 1.00 | 20.79 | B |
| ATOM | 2340 | O | ARG | B | 118 | 5.121 | -24.760 | 13.528 | 1.00 | 19.12 | B |
| ATOM | 2341 | N | VAL | B | 119 | 6.720 | -24.323 | 11.996 | 1.00 | 20.71 | B |
| ATOM | 2342 | CA | VAL | B | 119 | 7.627 | -23.791 | 13.001 | 1.00 | 21.55 | B |
| ATOM | 2343 | CB | VAL | B | 119 | 8.916 | -23.239 | 12.356 | 1.00 | 21.22 | B |
| ATOM | 2344 | CG1 | VAL | B | 119 | 9.713 | -24.377 | 11.735 | 1.00 | 21.19 | B |
| ATOM | 2345 | CG2 | VAL | B | 119 | 8.570 | -22.230 | 11.288 | 1.00 | 22.83 | B |
| ATOM | 2346 | C | VAL | B | 119 | 6.939 | -22.699 | 13.817 | 1.00 | 21.36 | B |
| ATOM | 2347 | O | VAL | B | 119 | 7.184 | -22.567 | 15.014 | 1.00 | 23.61 | B |
| ATOM | 2348 | N | ALA | B | 120 | 6.048 | -21.941 | 13.185 | 1.00 | 20.42 | B |
| ATOM | 2349 | CA | ALA | B | 120 | 5.357 | -20.868 | 13.890 | 1.00 | 20.13 | B |
| ATOM | 2350 | CB | ALA | B | 120 | 4.699 | -19.915 | 12.896 | 1.00 | 18.01 | B |
| ATOM | 2351 | C | ALA | B | 120 | 4.324 | -21.411 | 14.860 | 1.00 | 20.70 | B |
| ATOM | 2352 | O | ALA | B | 120 | 4.123 | -20.859 | 15.941 | 1.00 | 21.30 | B |
| ATOM | 2353 | N | GLN | B | 121 | 3.661 | -22.494 | 14.479 | 1.00 | 22.65 | B |
| ATOM | 2354 | CA | GLN | B | 121 | 2.656 | -23.083 | 15.357 | 1.00 | 22.64 | B |
| ATOM | 2355 | CB | GLN | B | 121 | 1.882 | -24.182 | 14.628 | 0.00 | 22.18 | B |
| ATOM | 2356 | CG | GLN | B | 121 | 0.750 | -24.787 | 15.446 | 0.00 | 22.36 | B |
| ATOM | 2357 | CD | GLN | B | 121 | -0.334 | -23.779 | 15.783 | 0.00 | 22.33 | B |
| ATOM | 2358 | OE1 | GLN | B | 121 | -0.086 | -22.786 | 16.469 | 0.00 | 22.35 | B |
| ATOM | 2359 | NE2 | GLN | B | 121 | -1.546 | -24.031 | 15.301 | 0.00 | 22.35 | B |
| ATOM | 2360 | C | GLN | B | 121 | 3.373 | -23.670 | 16.567 | 1.00 | 20.67 | B |
| ATOM | 2361 | O | GLN | B | 121 | 2.939 | -23.508 | 17.710 | 1.00 | 18.85 | B |
| ATOM | 2362 | N | ARG | B | 122 | 4.481 | -24.349 | 16.296 | 1.00 | 20.59 | B |
| ATOM | 2363 | CA | ARG | B | 122 | 5.284 | -24.968 | 17.347 | 1.00 | 22.42 | B |
| ATOM | 2364 | CB | ARG | B | 122 | 6.505 | -25.669 | 16.732 | 1.00 | 22.84 | B |
| ATOM | 2365 | CG | ARG | B | 122 | 6.927 | -26.959 | 17.418 | 1.00 | 24.36 | B |
| ATOM | 2366 | CD | ARG | B | 122 | 6.651 | -26.899 | 18.905 | 1.00 | 27.76 | B |
| ATOM | 2367 | NE | ARG | B | 122 | 7.481 | -27.832 | 19.654 | 1.00 | 31.48 | B |
| ATOM | 2368 | CZ | ARG | B | 122 | 7.644 | -27.777 | 20.970 | 1.00 | 32.24 | B |
| ATOM | 2369 | NH1 | ARG | B | 122 | 7.027 | -26.836 | 21.677 | 1.00 | 32.89 | B |
| ATOM | 2370 | NH2 | ARG | B | 122 | 8.444 | -28.642 | 21.574 | 1.00 | 33.66 | B |
| ATOM | 2371 | C | ARG | B | 122 | 5.751 | -23.904 | 18.352 | 1.00 | 22.90 | B |
| ATOM | 2372 | O | ARG | B | 122 | 5.614 | -24.075 | 19.565 | 1.00 | 22.79 | B |
| ATOM | 2373 | N | ALA | B | 123 | 6.278 | -22.796 | 17.837 | 1.00 | 22.03 | B |
| ATOM | 2374 | CA | ALA | B | 123 | 6.776 | -21.721 | 18.683 | 1.00 | 22.20 | B |

Figure 2 (suite 32)

| | | | | | | | | | | | |
|---|---|---|---|---|---|---|---|---|---|---|---|
| ATOM | 2375 | CB | ALA | B | 123 | 7.516 | -20.710 | 17.836 | 1.00 | 21.31 | B |
| ATOM | 2376 | C | ALA | B | 123 | 5.736 | -21.002 | 19.527 | 1.00 | 23.32 | B |
| ATOM | 2377 | O | ALA | B | 123 | 5.967 | -20.736 | 20.705 | 1.00 | 24.42 | B |
| ATOM | 2378 | N | SER | B | 124 | 4.592 | -20.690 | 18.929 | 1.00 | 25.28 | B |
| ATOM | 2379 | CA | SER | B | 124 | 3.534 | -19.942 | 19.611 | 1.00 | 27.88 | B |
| ATOM | 2380 | CB | SER | B | 124 | 2.271 | -19.928 | 18.746 | 1.00 | 28.56 | B |
| ATOM | 2381 | OG | SER | B | 124 | 1.707 | -21.227 | 18.650 | 1.00 | 30.97 | B |
| ATOM | 2382 | C | SER | B | 124 | 3.161 | -20.394 | 21.019 | 1.00 | 28.61 | B |
| ATOM | 2383 | O | SER | B | 124 | 2.844 | -19.577 | 21.883 | 1.00 | 27.43 | B |
| ATOM | 2384 | N | ARG | B | 125 | 3.189 | -21.700 | 21.233 | 1.00 | 31.56 | B |
| ATOM | 2385 | CA | ARG | B | 125 | 2.830 | -22.304 | 22.511 | 1.00 | 33.63 | B |
| ATOM | 2386 | CB | ARG | B | 125 | 3.148 | -23.800 | 22.456 | 1.00 | 38.27 | B |
| ATOM | 2387 | CG | ARG | B | 125 | 2.591 | -24.649 | 23.595 | 1.00 | 42.86 | B |
| ATOM | 2388 | CD | ARG | B | 125 | 3.027 | -26.090 | 23.378 | 1.00 | 46.16 | B |
| ATOM | 2389 | NE | ARG | B | 125 | 2.757 | -26.511 | 22.003 | 1.00 | 49.29 | B |
| ATOM | 2390 | CZ | ARG | B | 125 | 3.475 | -27.409 | 21.329 | 1.00 | 51.16 | B |
| ATOM | 2391 | NH1 | ARG | B | 125 | 4.522 | -27.993 | 21.900 | 1.00 | 51.19 | B |
| ATOM | 2392 | NH2 | ARG | B | 125 | 3.149 | -27.718 | 20.077 | 1.00 | 51.79 | B |
| ATOM | 2393 | C | ARG | B | 125 | 3.504 | -21.676 | 23.728 | 1.00 | 32.37 | B |
| ATOM | 2394 | O | ARG | B | 125 | 2.830 | -21.180 | 24.629 | 1.00 | 32.77 | B |
| ATOM | 2395 | N | SER | B | 126 | 4.830 | -21.698 | 23.762 | 1.00 | 29.72 | B |
| ATOM | 2396 | CA | SER | B | 126 | 5.547 | -21.139 | 24.897 | 1.00 | 28.74 | B |
| ATOM | 2397 | CB | SER | B | 126 | 6.960 | -21.722 | 24.966 | 1.00 | 27.59 | B |
| ATOM | 2398 | OG | SER | B | 126 | 7.596 | -21.682 | 23.704 | 1.00 | 31.21 | B |
| ATOM | 2399 | C | SER | B | 126 | 5.602 | -19.612 | 24.920 | 1.00 | 28.79 | B |
| ATOM | 2400 | O | SER | B | 126 | 5.615 | -19.007 | 25.994 | 1.00 | 29.36 | B |
| ATOM | 2401 | N | MET | B | 127 | 5.626 | -18.984 | 23.749 | 1.00 | 28.12 | B |
| ATOM | 2402 | CA | MET | B | 127 | 5.676 | -17.529 | 23.697 | 1.00 | 26.34 | B |
| ATOM | 2403 | CB | MET | B | 127 | 5.783 | -17.047 | 22.256 | 1.00 | 23.81 | B |
| ATOM | 2404 | CG | MET | B | 127 | 7.015 | -17.568 | 21.556 | 1.00 | 21.92 | B |
| ATOM | 2405 | SD | MET | B | 127 | 7.253 | -16.868 | 19.917 | 1.00 | 21.64 | B |
| ATOM | 2406 | CE | MET | B | 127 | 8.943 | -17.384 | 19.590 | 1.00 | 19.62 | B |
| ATOM | 2407 | C | MET | B | 127 | 4.461 | -16.908 | 24.363 | 1.00 | 27.45 | B |
| ATOM | 2408 | O | MET | B | 127 | 4.577 | -15.906 | 25.068 | 1.00 | 28.07 | B |
| ATOM | 2409 | N | GLN | B | 128 | 3.297 | -17.510 | 24.158 | 1.00 | 29.24 | B |
| ATOM | 2410 | CA | GLN | B | 128 | 2.075 | -16.993 | 24.759 | 1.00 | 33.42 | B |
| ATOM | 2411 | CB | GLN | B | 128 | 0.862 | -17.773 | 24.264 | 1.00 | 36.43 | B |
| ATOM | 2412 | CG | GLN | B | 128 | 0.514 | -17.535 | 22.818 | 1.00 | 41.92 | B |
| ATOM | 2413 | CD | GLN | B | 128 | -0.639 | -18.401 | 22.365 | 1.00 | 45.81 | B |
| ATOM | 2414 | OE1 | GLN | B | 128 | -1.703 | -18.420 | 22.994 | 1.00 | 48.09 | B |
| ATOM | 2415 | NE2 | GLN | B | 128 | -0.439 | -19.123 | 21.266 | 1.00 | 47.43 | B |
| ATOM | 2416 | C | GLN | B | 128 | 2.124 | -17.082 | 26.272 | 1.00 | 34.57 | B |
| ATOM | 2417 | O | GLN | B | 128 | 1.770 | -16.134 | 26.974 | 1.00 | 35.61 | B |
| ATOM | 2418 | N | ARG | B | 129 | 2.551 | -18.232 | 26.775 | 1.00 | 35.20 | B |
| ATOM | 2419 | CA | ARG | B | 129 | 2.624 | -18.430 | 28.211 | 1.00 | 36.70 | B |
| ATOM | 2420 | CB | ARG | B | 129 | 3.016 | -19.871 | 28.540 | 1.00 | 38.94 | B |
| ATOM | 2421 | CG | ARG | B | 129 | 1.851 | -20.833 | 28.595 | 1.00 | 42.24 | B |
| ATOM | 2422 | CD | ARG | B | 129 | 2.307 | -22.168 | 29.144 | 1.00 | 47.04 | B |
| ATOM | 2423 | NE | ARG | B | 129 | 3.301 | -22.798 | 28.278 | 1.00 | 50.90 | B |
| ATOM | 2424 | CZ | ARG | B | 129 | 3.929 | -23.934 | 28.563 | 1.00 | 52.09 | B |
| ATOM | 2425 | NH1 | ARG | B | 129 | 4.817 | -24.434 | 27.713 | 1.00 | 52.94 | B |
| ATOM | 2426 | NH2 | ARG | B | 129 | 3.675 | -24.567 | 29.701 | 1.00 | 53.78 | B |
| ATOM | 2427 | C | ARG | B | 129 | 3.603 | -17.486 | 28.878 | 1.00 | 35.78 | B |
| ATOM | 2428 | O | ARG | B | 129 | 3.308 | -16.922 | 29.931 | 1.00 | 36.77 | B |
| ATOM | 2429 | N | ASN | B | 130 | 4.765 | -17.307 | 28.266 | 1.00 | 33.48 | B |
| ATOM | 2430 | CA | ASN | B | 130 | 5.775 | -16.444 | 28.851 | 1.00 | 33.17 | B |
| ATOM | 2431 | CB | ASN | B | 130 | 7.161 | -16.891 | 28.388 | 1.00 | 34.36 | B |
| ATOM | 2432 | CG | ASN | B | 130 | 7.431 | -18.336 | 28.730 | 1.00 | 35.05 | B |
| ATOM | 2433 | OD1 | ASN | B | 130 | 7.152 | -18.770 | 29.842 | 1.00 | 35.71 | B |
| ATOM | 2434 | ND2 | ASN | B | 130 | 7.973 | -19.091 | 27.779 | 1.00 | 36.85 | B |
| ATOM | 2435 | C | ASN | B | 130 | 5.568 | -14.962 | 28.571 | 1.00 | 31.28 | B |
| ATOM | 2436 | O | ASN | B | 130 | 6.413 | -14.140 | 28.915 | 1.00 | 32.21 | B |
| ATOM | 2437 | N | LYS | B | 131 | 4.436 | -14.627 | 27.962 | 1.00 | 29.33 | B |
| ATOM | 2438 | CA | LYS | B | 131 | 4.112 | -13.244 | 27.646 | 1.00 | 26.82 | B |
| ATOM | 2439 | CB | LYS | B | 131 | 3.760 | -12.484 | 28.924 | 1.00 | 30.06 | B |
| ATOM | 2440 | CG | LYS | B | 131 | 2.266 | -12.440 | 29.230 | 1.00 | 35.94 | B |
| ATOM | 2441 | CD | LYS | B | 131 | 1.612 | -13.819 | 29.113 | 1.00 | 40.50 | B |
| ATOM | 2442 | CE | LYS | B | 131 | 0.099 | -13.739 | 29.329 | 1.00 | 42.66 | B |
| ATOM | 2443 | NZ | LYS | B | 131 | -0.585 | -15.028 | 29.014 | 1.00 | 44.71 | B |
| ATOM | 2444 | C | LYS | B | 131 | 5.212 | -12.506 | 26.888 | 1.00 | 24.77 | B |
| ATOM | 2445 | O | LYS | B | 131 | 5.426 | -11.311 | 27.097 | 1.00 | 24.62 | B |
| ATOM | 2446 | N | PHE | B | 132 | 5.917 | -13.230 | 26.023 | 1.00 | 20.94 | B |
| ATOM | 2447 | CA | PHE | B | 132 | 6.960 | -12.645 | 25.196 | 1.00 | 18.76 | B |

Figure 2 (suite 33)

| | | | | | | | | | | | |
|---|---|---|---|---|---|---|---|---|---|---|---|
| ATOM | 2448 | CB | PHE | B | 132 | 8.288 | -12.506 | 25.935 | 1.00 | 13.95 | B |
| ATOM | 2449 | CG | PHE | B | 132 | 9.353 | -11.846 | 25.102 | 1.00 | 10.30 | B |
| ATOM | 2450 | CD1 | PHE | B | 132 | 9.349 | -10.466 | 24.915 | 1.00 | 6.92 | B |
| ATOM | 2451 | CD2 | PHE | B | 132 | 10.280 | -12.611 | 24.402 | 1.00 | 10.47 | B |
| ATOM | 2452 | CE1 | PHE | B | 132 | 10.245 | -9.855 | 24.037 | 1.00 | 6.90 | B |
| ATOM | 2453 | CE2 | PHE | B | 132 | 11.187 | -12.012 | 23.515 | 1.00 | 11.14 | B |
| ATOM | 2454 | CZ | PHE | B | 132 | 11.164 | -10.624 | 23.332 | 1.00 | 9.62 | B |
| ATOM | 2455 | C | PHE | B | 132 | 7.195 | -13.504 | 23.970 | 1.00 | 20.37 | B |
| ATOM | 2456 | O | PHE | B | 132 | 7.189 | -14.726 | 24.046 | 1.00 | 21.42 | B |
| ATOM | 2457 | N | GLY | B | 133 | 7.418 | -12.857 | 22.838 | 1.00 | 21.10 | B |
| ATOM | 2458 | CA | GLY | B | 133 | 7.656 | -13.603 | 21.622 | 1.00 | 20.55 | B |
| ATOM | 2459 | C | GLY | B | 133 | 7.877 | -12.698 | 20.431 | 1.00 | 19.24 | B |
| ATOM | 2460 | O | GLY | B | 133 | 7.224 | -11.669 | 20.280 | 1.00 | 19.14 | B |
| ATOM | 2461 | N | ARG | B | 134 | 8.813 | -13.083 | 19.580 | 1.00 | 17.73 | B |
| ATOM | 2462 | CA | ARG | B | 134 | 9.103 | -12.306 | 18.399 | 1.00 | 18.33 | B |
| ATOM | 2463 | CB | ARG | B | 134 | 10.291 | -11.369 | 18.664 | 1.00 | 17.84 | B |
| ATOM | 2464 | CG | ARG | B | 134 | 10.068 | -10.455 | 19.863 | 1.00 | 19.54 | B |
| ATOM | 2465 | CD | ARG | B | 134 | 10.007 | -8.979 | 19.484 | 1.00 | 20.00 | B |
| ATOM | 2466 | NE | ARG | B | 134 | 9.766 | -8.120 | 20.644 | 1.00 | 22.87 | B |
| ATOM | 2467 | CZ | ARG | B | 134 | 8.705 | -8.222 | 21.448 | 1.00 | 26.30 | B |
| ATOM | 2468 | NH1 | ARG | B | 134 | 7.784 | -9.146 | 21.225 | 1.00 | 29.02 | B |
| ATOM | 2469 | NH2 | ARG | B | 134 | 8.547 | -7.394 | 22.473 | 1.00 | 27.25 | B |
| ATOM | 2470 | C | ARG | B | 134 | 9.387 | -13.233 | 17.223 | 1.00 | 17.75 | B |
| ATOM | 2471 | O | ARG | B | 134 | 10.447 | -13.847 | 17.127 | 1.00 | 17.95 | B |
| ATOM | 2472 | N | MET | B | 135 | 8.411 | -13.339 | 16.333 | 1.00 | 17.58 | B |
| ATOM | 2473 | CA | MET | B | 135 | 8.549 | -14.169 | 15.155 | 1.00 | 17.63 | B |
| ATOM | 2474 | CB | MET | B | 135 | 7.229 | -14.865 | 14.843 | 1.00 | 18.35 | B |
| ATOM | 2475 | CG | MET | B | 135 | 6.807 | -15.826 | 15.931 | 1.00 | 21.90 | B |
| ATOM | 2476 | SD | MET | B | 135 | 5.320 | -16.747 | 15.533 | 1.00 | 27.02 | B |
| ATOM | 2477 | CE | MET | B | 135 | 4.274 | -16.375 | 16.957 | 1.00 | 27.90 | B |
| ATOM | 2478 | C | MET | B | 135 | 8.946 | -13.260 | 14.015 | 1.00 | 16.50 | B |
| ATOM | 2479 | O | MET | B | 135 | 8.338 | -12.213 | 13.811 | 1.00 | 18.47 | B |
| ATOM | 2480 | N | ILE | B | 136 | 9.973 | -13.654 | 13.280 | 1.00 | 14.35 | B |
| ATOM | 2481 | CA | ILE | B | 136 | 10.451 | -12.869 | 12.159 | 1.00 | 13.22 | B |
| ATOM | 2482 | CB | ILE | B | 136 | 11.828 | -12.247 | 12.483 | 1.00 | 12.87 | B |
| ATOM | 2483 | CG2 | ILE | B | 136 | 12.298 | -11.390 | 11.320 | 1.00 | 11.01 | B |
| ATOM | 2484 | CG1 | ILE | B | 136 | 11.740 | -11.449 | 13.790 | 1.00 | 13.64 | B |
| ATOM | 2485 | CD | ILE | B | 136 | 13.033 | -10.748 | 14.198 | 1.00 | 12.66 | B |
| ATOM | 2486 | C | ILE | B | 136 | 10.597 | -13.800 | 10.958 | 1.00 | 13.67 | B |
| ATOM | 2487 | O | ILE | B | 136 | 11.370 | -14.761 | 11.006 | 1.00 | 12.30 | B |
| ATOM | 2488 | N | PHE | B | 137 | 9.865 | -13.522 | 9.882 | 1.00 | 13.73 | B |
| ATOM | 2489 | CA | PHE | B | 137 | 9.948 | -14.361 | 8.687 | 1.00 | 12.38 | B |
| ATOM | 2490 | CB | PHE | B | 137 | 8.541 | -14.694 | 8.172 | 1.00 | 7.90 | B |
| ATOM | 2491 | CG | PHE | B | 137 | 7.641 | -15.313 | 9.208 | 1.00 | 5.47 | B |
| ATOM | 2492 | CD1 | PHE | B | 137 | 7.018 | -14.527 | 10.172 | 1.00 | 6.90 | B |
| ATOM | 2493 | CD2 | PHE | B | 137 | 7.414 | -16.691 | 9.221 | 1.00 | 2.18 | B |
| ATOM | 2494 | CE1 | PHE | B | 137 | 6.170 | -15.111 | 11.142 | 1.00 | 7.71 | B |
| ATOM | 2495 | CE2 | PHE | B | 137 | 6.582 | -17.283 | 10.175 | 1.00 | 1.15 | B |
| ATOM | 2496 | CZ | PHE | B | 137 | 5.954 | -16.496 | 11.139 | 1.00 | 3.76 | B |
| ATOM | 2497 | C | PHE | B | 137 | 10.740 | -13.625 | 7.609 | 1.00 | 14.00 | B |
| ATOM | 2498 | O | PHE | B | 137 | 10.553 | -12.434 | 7.413 | 1.00 | 16.42 | B |
| ATOM | 2499 | N | ILE | B | 138 | 11.636 | -14.320 | 6.921 | 1.00 | 15.72 | B |
| ATOM | 2500 | CA | ILE | B | 138 | 12.416 | -13.682 | 5.870 | 1.00 | 19.13 | B |
| ATOM | 2501 | CB | ILE | B | 138 | 13.940 | -13.963 | 6.013 | 1.00 | 18.75 | B |
| ATOM | 2502 | CG2 | ILE | B | 138 | 14.656 | -13.632 | 4.713 | 1.00 | 16.52 | B |
| ATOM | 2503 | CG1 | ILE | B | 138 | 14.546 | -13.115 | 7.134 | 1.00 | 19.13 | B |
| ATOM | 2504 | CD | ILE | B | 138 | 14.083 | -13.475 | 8.499 | 1.00 | 22.04 | B |
| ATOM | 2505 | C | ILE | B | 138 | 11.954 | -14.199 | 4.516 | 1.00 | 23.04 | B |
| ATOM | 2506 | O | ILE | B | 138 | 11.977 | -15.403 | 4.268 | 1.00 | 23.90 | B |
| ATOM | 2507 | N | GLY | B | 139 | 11.529 | -13.285 | 3.647 | 1.00 | 24.92 | B |
| ATOM | 2508 | CA | GLY | B | 139 | 11.072 | -13.676 | 2.332 | 1.00 | 28.70 | B |
| ATOM | 2509 | C | GLY | B | 139 | 12.226 | -14.211 | 1.513 | 1.00 | 31.78 | B |
| ATOM | 2510 | O | GLY | B | 139 | 13.355 | -14.258 | 2.005 | 1.00 | 32.78 | B |
| ATOM | 2511 | N | SER | B | 140 | 11.949 | -14.617 | 0.274 | 1.00 | 33.57 | B |
| ATOM | 2512 | CA | SER | B | 140 | 12.983 | -15.139 | -0.618 | 1.00 | 34.64 | B |
| ATOM | 2513 | CB | SER | B | 140 | 12.366 | -16.049 | -1.681 | 1.00 | 35.90 | B |
| ATOM | 2514 | OG | SER | B | 140 | 11.621 | -17.098 | -1.088 | 1.00 | 39.07 | B |
| ATOM | 2515 | C | SER | B | 140 | 13.688 | -13.976 | -1.302 | 1.00 | 34.77 | B |
| ATOM | 2516 | O | SER | B | 140 | 13.340 | -12.818 | -1.087 | 1.00 | 34.54 | B |
| ATOM | 2517 | N | VAL | B | 141 | 14.675 | -14.291 | -2.132 | 1.00 | 36.27 | B |
| ATOM | 2518 | CA | VAL | B | 141 | 15.430 | -13.272 | -2.853 | 1.00 | 36.89 | B |
| ATOM | 2519 | CB | VAL | B | 141 | 16.936 | -13.555 | -2.773 | 1.00 | 36.37 | B |
| ATOM | 2520 | CG1 | VAL | B | 141 | 17.699 | -12.584 | -3.664 | 1.00 | 36.22 | B |

Figure 2 (suite 34)

| | | | | | | | | | | | |
|---|---|---|---|---|---|---|---|---|---|---|---|
| ATOM | 2521 | CG2 | VAL | B | 141 | 17.402 | -13.444 | -1.325 | 1.00 | 35.68 | B |
| ATOM | 2522 | C | VAL | B | 141 | 15.019 | -13.194 | -4.322 | 1.00 | 38.39 | B |
| ATOM | 2523 | O | VAL | B | 141 | 13.911 | -12.755 | -4.649 | 1.00 | 39.97 | B |
| ATOM | 2524 | N | GLN | B | 150 | 8.833 | -16.418 | -10.185 | 1.00 | 27.33 | B |
| ATOM | 2525 | CA | GLN | B | 150 | 8.090 | -17.664 | -10.319 | 1.00 | 26.83 | B |
| ATOM | 2526 | CB | GLN | B | 150 | 8.922 | -18.833 | -9.787 | 0.00 | 26.86 | B |
| ATOM | 2527 | CG | GLN | B | 150 | 10.242 | -19.040 | -10.513 | 0.00 | 26.89 | B |
| ATOM | 2528 | CD | GLN | B | 150 | 10.064 | -19.409 | -11.977 | 0.00 | 26.91 | B |
| ATOM | 2529 | OE1 | GLN | B | 150 | 9.503 | -18.643 | -12.760 | 0.00 | 26.92 | B |
| ATOM | 2530 | NE2 | GLN | B | 150 | 10.545 | -20.588 | -12.351 | 0.00 | 26.92 | B |
| ATOM | 2531 | C | GLN | B | 150 | 6.769 | -17.580 | -9.563 | 1.00 | 26.35 | B |
| ATOM | 2532 | O | GLN | B | 150 | 6.676 | -16.945 | -8.514 | 1.00 | 25.87 | B |
| ATOM | 2533 | N | ALA | B | 151 | 5.745 | -18.234 | -10.093 | 1.00 | 25.82 | B |
| ATOM | 2534 | CA | ALA | B | 151 | 4.440 | -18.204 | -9.466 | 1.00 | 24.46 | B |
| ATOM | 2535 | CB | ALA | B | 151 | 3.430 | -18.900 | -10.352 | 1.00 | 24.45 | B |
| ATOM | 2536 | C | ALA | B | 151 | 4.452 | -18.837 | -8.086 | 1.00 | 24.96 | B |
| ATOM | 2537 | O | ALA | B | 151 | 3.769 | -18.359 | -7.184 | 1.00 | 25.30 | B |
| ATOM | 2538 | N | ASN | B | 152 | 5.213 | -19.917 | -7.920 | 1.00 | 25.85 | B |
| ATOM | 2539 | CA | ASN | B | 152 | 5.286 | -20.605 | -6.628 | 1.00 | 26.54 | B |
| ATOM | 2540 | CB | ASN | B | 152 | 6.029 | -21.956 | -6.755 | 1.00 | 25.88 | B |
| ATOM | 2541 | CG | ASN | B | 152 | 7.194 | -21.909 | -7.741 | 1.00 | 26.71 | B |
| ATOM | 2542 | OD1 | ASN | B | 152 | 7.781 | -20.852 | -7.990 | 1.00 | 27.83 | B |
| ATOM | 2543 | ND2 | ASN | B | 152 | 7.538 | -23.065 | -8.300 | 1.00 | 26.34 | B |
| ATOM | 2544 | C | ASN | B | 152 | 5.947 | -19.733 | -5.554 | 1.00 | 26.98 | B |
| ATOM | 2545 | O | ASN | B | 152 | 5.520 | -19.722 | -4.396 | 1.00 | 27.73 | B |
| ATOM | 2546 | N | TYR | B | 153 | 6.975 | -18.987 | -5.938 | 1.00 | 27.04 | B |
| ATOM | 2547 | CA | TYR | B | 153 | 7.650 | -18.119 | -4.985 | 1.00 | 27.79 | B |
| ATOM | 2548 | CB | TYR | B | 153 | 9.028 | -17.721 | -5.529 | 1.00 | 28.92 | B |
| ATOM | 2549 | CG | TYR | B | 153 | 10.005 | -18.881 | -5.501 | 1.00 | 32.33 | B |
| ATOM | 2550 | CD1 | TYR | B | 153 | 10.566 | -19.387 | -6.678 | 1.00 | 33.86 | B |
| ATOM | 2551 | CE1 | TYR | B | 153 | 11.429 | -20.497 | -6.655 | 1.00 | 34.87 | B |
| ATOM | 2552 | CD2 | TYR | B | 153 | 10.329 | -19.511 | -4.297 | 1.00 | 34.51 | B |
| ATOM | 2553 | CE2 | TYR | B | 153 | 11.187 | -20.620 | -4.260 | 1.00 | 36.40 | B |
| ATOM | 2554 | CZ | TYR | B | 153 | 11.734 | -21.107 | -5.442 | 1.00 | 36.54 | B |
| ATOM | 2555 | OH | TYR | B | 153 | 12.598 | -22.183 | -5.396 | 1.00 | 36.46 | B |
| ATOM | 2556 | C | TYR | B | 153 | 6.788 | -16.895 | -4.666 | 1.00 | 27.09 | B |
| ATOM | 2557 | O | TYR | B | 153 | 6.688 | -16.471 | -3.510 | 1.00 | 27.36 | B |
| ATOM | 2558 | N | ALA | B | 154 | 6.145 | -16.343 | -5.690 | 1.00 | 25.31 | B |
| ATOM | 2559 | CA | ALA | B | 154 | 5.275 | -15.189 | -5.506 | 1.00 | 23.01 | B |
| ATOM | 2560 | CB | ALA | B | 154 | 4.675 | -14.784 | -6.835 | 1.00 | 23.17 | B |
| ATOM | 2561 | C | ALA | B | 154 | 4.163 | -15.528 | -4.511 | 1.00 | 21.70 | B |
| ATOM | 2562 | O | ALA | B | 154 | 3.890 | -14.762 | -3.581 | 1.00 | 20.90 | B |
| ATOM | 2563 | N | ALA | B | 155 | 3.535 | -16.684 | -4.709 | 1.00 | 19.37 | B |
| ATOM | 2564 | CA | ALA | B | 155 | 2.456 | -17.140 | -3.839 | 1.00 | 17.91 | B |
| ATOM | 2565 | CB | ALA | B | 155 | 1.844 | -18.416 | -4.400 | 1.00 | 16.58 | B |
| ATOM | 2566 | C | ALA | B | 155 | 2.957 | -17.368 | -2.410 | 1.00 | 17.50 | B |
| ATOM | 2567 | O | ALA | B | 155 | 2.320 | -16.936 | -1.439 | 1.00 | 14.44 | B |
| ATOM | 2568 | N | SER | B | 156 | 4.099 | -18.042 | -2.289 | 1.00 | 16.63 | B |
| ATOM | 2569 | CA | SER | B | 156 | 4.703 | -18.307 | -0.984 | 1.00 | 17.74 | B |
| ATOM | 2570 | CB | SER | B | 156 | 6.041 | -19.034 | -1.159 | 1.00 | 17.64 | B |
| ATOM | 2571 | OG | SER | B | 156 | 5.837 | -20.374 | -1.571 | 1.00 | 21.18 | B |
| ATOM | 2572 | C | SER | B | 156 | 4.930 | -17.028 | -0.172 | 1.00 | 18.42 | B |
| ATOM | 2573 | O | SER | B | 156 | 4.462 | -16.917 | 0.969 | 1.00 | 17.17 | B |
| ATOM | 2574 | N | LYS | B | 157 | 5.651 | -16.073 | -0.762 | 1.00 | 18.28 | B |
| ATOM | 2575 | CA | LYS | B | 157 | 5.944 | -14.807 | -0.092 | 1.00 | 19.60 | B |
| ATOM | 2576 | CB | LYS | B | 157 | 6.711 | -13.860 | -1.018 | 1.00 | 22.02 | B |
| ATOM | 2577 | CG | LYS | B | 157 | 8.168 | -14.193 | -1.225 | 1.00 | 25.26 | B |
| ATOM | 2578 | CD | LYS | B | 157 | 8.864 | -13.073 | -2.014 | 1.00 | 29.23 | B |
| ATOM | 2579 | CE | LYS | B | 157 | 10.371 | -13.329 | -2.124 | 1.00 | 31.76 | B |
| ATOM | 2580 | NZ | LYS | B | 157 | 11.098 | -12.281 | -2.913 | 1.00 | 32.96 | B |
| ATOM | 2581 | C | LYS | B | 157 | 4.689 | -14.093 | 0.382 | 1.00 | 19.14 | B |
| ATOM | 2582 | O | LYS | B | 157 | 4.526 | -13.822 | 1.574 | 1.00 | 20.02 | B |
| ATOM | 2583 | N | ALA | B | 158 | 3.815 | -13.771 | -0.567 | 1.00 | 18.04 | B |
| ATOM | 2584 | CA | ALA | B | 158 | 2.568 | -13.078 | -0.260 | 1.00 | 15.49 | B |
| ATOM | 2585 | CB | ALA | B | 158 | 1.717 | -12.946 | -1.519 | 1.00 | 14.08 | B |
| ATOM | 2586 | C | ALA | B | 158 | 1.830 | -13.868 | 0.798 | 1.00 | 15.15 | B |
| ATOM | 2587 | O | ALA | B | 158 | 1.159 | -13.303 | 1.659 | 1.00 | 14.92 | B |
| ATOM | 2588 | N | GLY | B | 159 | 1.974 | -15.188 | 0.732 | 1.00 | 14.35 | B |
| ATOM | 2589 | CA | GLY | B | 159 | 1.325 | -16.047 | 1.699 | 1.00 | 14.40 | B |
| ATOM | 2590 | C | GLY | B | 159 | 1.803 | -15.855 | 3.132 | 1.00 | 14.89 | B |
| ATOM | 2591 | O | GLY | B | 159 | 0.979 | -15.726 | 4.045 | 1.00 | 13.41 | B |
| ATOM | 2592 | N | VAL | B | 160 | 3.123 | -15.831 | 3.345 | 1.00 | 15.33 | B |
| ATOM | 2593 | CA | VAL | B | 160 | 3.655 | -15.660 | 4.702 | 1.00 | 15.29 | B |

Figure 2 (suite 35)

| | | | | | | | | | | | |
|---|---|---|---|---|---|---|---|---|---|---|---|
| ATOM | 2594 | CB | VAL | B | 160 | 5.197 | -15.751 | 4.760 | 1.00 | 15.30 | B |
| ATOM | 2595 | CG1 | VAL | B | 160 | 5.643 | -15.808 | 6.210 | 1.00 | 14.68 | B |
| ATOM | 2596 | CG2 | VAL | B | 160 | 5.688 | -16.991 | 4.026 | 1.00 | 16.55 | B |
| ATOM | 2597 | C | VAL | B | 160 | 3.239 | -14.311 | 5.258 | 1.00 | 15.45 | B |
| ATOM | 2598 | O | VAL | B | 160 | 2.798 | -14.211 | 6.405 | 1.00 | 16.30 | B |
| ATOM | 2599 | N | ILE | B | 161 | 3.370 | -13.270 | 4.445 | 1.00 | 14.47 | B |
| ATOM | 2600 | CA | ILE | B | 161 | 2.975 | -11.945 | 4.897 | 1.00 | 13.04 | B |
| ATOM | 2601 | CB | ILE | B | 161 | 3.090 | -10.895 | 3.779 | 1.00 | 11.20 | B |
| ATOM | 2602 | CG2 | ILE | B | 161 | 2.522 | -9.560 | 4.250 | 1.00 | 10.31 | B |
| ATOM | 2603 | CG1 | ILE | B | 161 | 4.552 | -10.744 | 3.364 | 1.00 | 7.93 | B |
| ATOM | 2604 | CD | ILE | B | 161 | 4.742 | -9.814 | 2.203 | 1.00 | 9.26 | B |
| ATOM | 2605 | C | ILE | B | 161 | 1.539 | -12.018 | 5.372 | 1.00 | 13.91 | B |
| ATOM | 2606 | O | ILE | B | 161 | 1.216 | -11.536 | 6.453 | 1.00 | 15.99 | B |
| ATOM | 2607 | N | GLY | B | 162 | 0.677 | -12.643 | 4.581 | 1.00 | 13.80 | B |
| ATOM | 2608 | CA | GLY | B | 162 | -0.712 | -12.749 | 4.991 | 1.00 | 16.62 | B |
| ATOM | 2609 | C | GLY | B | 162 | -0.866 | -13.477 | 6.316 | 1.00 | 18.32 | B |
| ATOM | 2610 | O | GLY | B | 162 | -1.617 | -13.051 | 7.199 | 1.00 | 17.37 | B |
| ATOM | 2611 | N | MET | B | 163 | -0.152 | -14.592 | 6.447 | 1.00 | 18.71 | B |
| ATOM | 2612 | CA | MET | B | 163 | -0.188 | -15.393 | 7.664 | 1.00 | 19.07 | B |
| ATOM | 2613 | CB | MET | B | 163 | 0.635 | -16.671 | 7.476 | 1.00 | 19.88 | B |
| ATOM | 2614 | CG | MET | B | 163 | 0.647 | -17.591 | 8.691 | 1.00 | 20.21 | B |
| ATOM | 2615 | SD | MET | B | 163 | 1.698 | -19.049 | 8.464 | 1.00 | 20.77 | B |
| ATOM | 2616 | CE | MET | B | 163 | 3.324 | -18.334 | 8.779 | 1.00 | 14.50 | B |
| ATOM | 2617 | C | MET | B | 163 | 0.368 | -14.607 | 8.846 | 1.00 | 19.03 | B |
| ATOM | 2618 | O | MET | B | 163 | -0.233 | -14.574 | 9.925 | 1.00 | 17.41 | B |
| ATOM | 2619 | N | ALA | B | 164 | 1.516 | -13.968 | 8.629 | 1.00 | 18.20 | B |
| ATOM | 2620 | CA | ALA | B | 164 | 2.170 | -13.194 | 9.674 | 1.00 | 17.95 | B |
| ATOM | 2621 | CB | ALA | B | 164 | 3.392 | -12.490 | 9.115 | 1.00 | 17.02 | B |
| ATOM | 2622 | C | ALA | B | 164 | 1.219 | -12.184 | 10.306 | 1.00 | 19.00 | B |
| ATOM | 2623 | O | ALA | B | 164 | 1.099 | -12.115 | 11.531 | 1.00 | 21.75 | B |
| ATOM | 2624 | N | ARG | B | 165 | 0.533 | -11.408 | 9.478 | 1.00 | 18.20 | B |
| ATOM | 2625 | CA | ARG | B | 165 | -0.400 | -10.420 | 9.999 | 1.00 | 17.57 | B |
| ATOM | 2626 | CB | ARG | B | 165 | -0.959 | -9.570 | 8.867 | 1.00 | 18.37 | B |
| ATOM | 2627 | CG | ARG | B | 165 | 0.126 | -8.838 | 8.121 | 1.00 | 21.31 | B |
| ATOM | 2628 | CD | ARG | B | 165 | -0.346 | -7.534 | 7.535 | 1.00 | 24.86 | B |
| ATOM | 2629 | NE | ARG | B | 165 | 0.738 | -6.912 | 6.786 | 1.00 | 29.92 | B |
| ATOM | 2630 | CZ | ARG | B | 165 | 0.636 | -5.764 | 6.128 | 1.00 | 31.69 | B |
| ATOM | 2631 | NH1 | ARG | B | 165 | -0.512 | -5.098 | 6.125 | 1.00 | 33.70 | B |
| ATOM | 2632 | NH2 | ARG | B | 165 | 1.683 | -5.288 | 5.468 | 1.00 | 31.69 | B |
| ATOM | 2633 | C | ARG | B | 165 | -1.530 | -11.071 | 10.767 | 1.00 | 16.98 | B |
| ATOM | 2634 | O | ARG | B | 165 | -2.091 | -10.470 | 11.679 | 1.00 | 17.07 | B |
| ATOM | 2635 | N | SER | B | 166 | -1.868 | -12.303 | 10.404 | 1.00 | 17.56 | B |
| ATOM | 2636 | CA | SER | B | 166 | -2.931 | -13.015 | 11.096 | 1.00 | 17.47 | B |
| ATOM | 2637 | CB | SER | B | 166 | -3.292 | -14.294 | 10.343 | 1.00 | 17.88 | B |
| ATOM | 2638 | OG | SER | B | 166 | -4.342 | -14.983 | 10.992 | 1.00 | 19.19 | B |
| ATOM | 2639 | C | SER | B | 166 | -2.428 | -13.335 | 12.505 | 1.00 | 18.20 | B |
| ATOM | 2640 | O | SER | B | 166 | -3.132 | -13.110 | 13.490 | 1.00 | 18.19 | B |
| ATOM | 2641 | N | ILE | B | 167 | -1.204 | -13.851 | 12.600 | 1.00 | 18.17 | B |
| ATOM | 2642 | CA | ILE | B | 167 | -0.611 | -14.157 | 13.905 | 1.00 | 18.52 | B |
| ATOM | 2643 | CB | ILE | B | 167 | 0.851 | -14.664 | 13.805 | 1.00 | 19.81 | B |
| ATOM | 2644 | CG2 | ILE | B | 167 | 1.337 | -15.023 | 15.202 | 1.00 | 20.05 | B |
| ATOM | 2645 | CG1 | ILE | B | 167 | 0.978 | -15.859 | 12.847 | 1.00 | 18.47 | B |
| ATOM | 2646 | CD | ILE | B | 167 | 0.358 | -17.128 | 13.353 | 1.00 | 19.27 | B |
| ATOM | 2647 | C | ILE | B | 167 | -0.547 | -12.867 | 14.737 | 1.00 | 18.42 | B |
| ATOM | 2648 | O | ILE | B | 167 | -0.999 | -12.829 | 15.883 | 1.00 | 18.17 | B |
| ATOM | 2649 | N | ALA | B | 168 | 0.029 | -11.823 | 14.140 | 1.00 | 17.54 | B |
| ATOM | 2650 | CA | ALA | B | 168 | 0.180 | -10.521 | 14.794 | 1.00 | 18.65 | B |
| ATOM | 2651 | CB | ALA | B | 168 | 0.734 | -9.488 | 13.801 | 1.00 | 16.97 | B |
| ATOM | 2652 | C | ALA | B | 168 | -1.134 | -10.020 | 15.354 | 1.00 | 19.73 | B |
| ATOM | 2653 | O | ALA | B | 168 | -1.208 | -9.549 | 16.489 | 1.00 | 18.22 | B |
| ATOM | 2654 | N | ARG | B | 169 | -2.178 | -10.140 | 14.543 | 1.00 | 21.81 | B |
| ATOM | 2655 | CA | ARG | B | 169 | -3.496 | -9.674 | 14.919 | 1.00 | 24.17 | B |
| ATOM | 2656 | CB | ARG | B | 169 | -4.434 | -9.803 | 13.712 | 1.00 | 25.57 | B |
| ATOM | 2657 | CG | ARG | B | 169 | -5.674 | -8.924 | 13.767 | 1.00 | 30.70 | B |
| ATOM | 2658 | CD | ARG | B | 169 | -6.491 | -9.014 | 12.470 | 1.00 | 34.88 | B |
| ATOM | 2659 | NE | ARG | B | 169 | -5.789 | -8.476 | 11.299 | 1.00 | 39.03 | B |
| ATOM | 2660 | CZ | ARG | B | 169 | -5.417 | -9.193 | 10.235 | 1.00 | 40.65 | B |
| ATOM | 2661 | NH1 | ARG | B | 169 | -5.670 | -10.495 | 10.173 | 1.00 | 41.23 | B |
| ATOM | 2662 | NH2 | ARG | B | 169 | -4.794 | -8.603 | 9.219 | 1.00 | 39.83 | B |
| ATOM | 2663 | C | ARG | B | 169 | -4.045 | -10.439 | 16.120 | 1.00 | 25.32 | B |
| ATOM | 2664 | O | ARG | B | 169 | -4.876 | -9.922 | 16.866 | 1.00 | 26.76 | B |
| ATOM | 2665 | N | GLU | B | 170 | -3.563 | -11.659 | 16.327 | 1.00 | 24.14 | B |
| ATOM | 2666 | CA | GLU | B | 170 | -4.054 | -12.469 | 17.432 | 1.00 | 24.38 | B |

Figure 2 (suite 36)

| | | | | | | | | | | | |
|---|---|---|---|---|---|---|---|---|---|---|---|
| ATOM | 2667 | CB | GLU | B | 170 | -4.355 | -13.888 | 16.920 | 1.00 | 24.04 | B |
| ATOM | 2668 | CG | GLU | B | 170 | -4.963 | -14.814 | 17.954 | 0.00 | 24.19 | B |
| ATOM | 2669 | CD | GLU | B | 170 | -5.229 | -16.201 | 17.404 | 0.00 | 24.17 | B |
| ATOM | 2670 | OE1 | GLU | B | 170 | -5.980 | -16.316 | 16.412 | 0.00 | 24.18 | B |
| ATOM | 2671 | OE2 | GLU | B | 170 | -4.688 | -17.176 | 17.966 | 0.00 | 24.18 | B |
| ATOM | 2672 | C | GLU | B | 170 | -3.139 | -12.548 | 18.671 | 1.00 | 24.66 | B |
| ATOM | 2673 | O | GLU | B | 170 | -3.618 | -12.532 | 19.807 | 1.00 | 24.39 | B |
| ATOM | 2674 | N | LEU | B | 171 | -1.830 | -12.623 | 18.463 | 1.00 | 23.05 | B |
| ATOM | 2675 | CA | LEU | B | 171 | -0.919 | -12.753 | 19.593 | 1.00 | 23.37 | B |
| ATOM | 2676 | CB | LEU | B | 171 | 0.165 | -13.778 | 19.254 | 1.00 | 22.38 | B |
| ATOM | 2677 | CG | LEU | B | 171 | -0.403 | -15.144 | 18.861 | 1.00 | 21.38 | B |
| ATOM | 2678 | CD1 | LEU | B | 171 | 0.730 | -16.153 | 18.713 | 1.00 | 21.53 | B |
| ATOM | 2679 | CD2 | LEU | B | 171 | -1.399 | -15.600 | 19.918 | 1.00 | 20.29 | B |
| ATOM | 2680 | C | LEU | B | 171 | -0.265 | -11.485 | 20.140 | 1.00 | 23.40 | B |
| ATOM | 2681 | O | LEU | B | 171 | 0.444 | -11.544 | 21.148 | 1.00 | 21.74 | B |
| ATOM | 2682 | N | SER | B | 172 | -0.505 | -10.344 | 19.500 | 1.00 | 23.19 | B |
| ATOM | 2683 | CA | SER | B | 172 | 0.088 | -9.099 | 19.974 | 1.00 | 23.56 | B |
| ATOM | 2684 | CB | SER | B | 172 | -0.217 | -7.941 | 19.015 | 1.00 | 22.80 | B |
| ATOM | 2685 | OG | SER | B | 172 | 0.707 | -7.904 | 17.938 | 1.00 | 20.40 | B |
| ATOM | 2686 | C | SER | B | 172 | -0.363 | -8.725 | 21.383 | 1.00 | 23.74 | B |
| ATOM | 2687 | O | SER | B | 172 | 0.398 | -8.128 | 22.134 | 1.00 | 23.72 | B |
| ATOM | 2688 | N | LYS | B | 173 | -1.588 | -9.070 | 21.754 | 1.00 | 24.21 | B |
| ATOM | 2689 | CA | LYS | B | 173 | -2.053 | -8.720 | 23.089 | 1.00 | 25.73 | B |
| ATOM | 2690 | CB | LYS | B | 173 | -3.539 | -9.051 | 23.258 | 1.00 | 27.90 | B |
| ATOM | 2691 | CG | LYS | B | 173 | -3.862 | -10.530 | 23.124 | 1.00 | 32.63 | B |
| ATOM | 2692 | CD | LYS | B | 173 | -5.303 | -10.807 | 23.509 | 1.00 | 35.92 | B |
| ATOM | 2693 | CE | LYS | B | 173 | -5.633 | -12.289 | 23.401 | 1.00 | 39.11 | B |
| ATOM | 2694 | NZ | LYS | B | 173 | -6.958 | -12.604 | 24.020 | 1.00 | 41.06 | B |
| ATOM | 2695 | C | LYS | B | 173 | -1.232 | -9.455 | 24.146 | 1.00 | 25.46 | B |
| ATOM | 2696 | O | LYS | B | 173 | -1.093 | -8.976 | 25.266 | 1.00 | 25.96 | B |
| ATOM | 2697 | N | ALA | B | 174 | -0.697 | -10.620 | 23.792 | 1.00 | 24.50 | B |
| ATOM | 2698 | CA | ALA | B | 174 | 0.123 | -11.396 | 24.719 | 1.00 | 23.20 | B |
| ATOM | 2699 | CB | ALA | B | 174 | -0.025 | -12.885 | 24.448 | 1.00 | 22.05 | B |
| ATOM | 2700 | C | ALA | B | 174 | 1.575 | -10.984 | 24.542 | 1.00 | 24.14 | B |
| ATOM | 2701 | O | ALA | B | 174 | 2.495 | -11.718 | 24.907 | 1.00 | 24.34 | B |
| ATOM | 2702 | N | ASN | B | 175 | 1.774 | -9.807 | 23.961 | 1.00 | 24.21 | B |
| ATOM | 2703 | CA | ASN | B | 175 | 3.112 | -9.284 | 23.734 | 1.00 | 24.80 | B |
| ATOM | 2704 | CB | ASN | B | 175 | 3.828 | -9.098 | 25.075 | 1.00 | 25.91 | B |
| ATOM | 2705 | CG | ASN | B | 175 | 5.173 | -8.424 | 24.925 | 1.00 | 28.94 | B |
| ATOM | 2706 | OD1 | ASN | B | 175 | 5.297 | -7.413 | 24.226 | 1.00 | 32.13 | B |
| ATOM | 2707 | ND2 | ASN | B | 175 | 6.193 | -8.975 | 25.585 | 1.00 | 29.32 | B |
| ATOM | 2708 | C | ASN | B | 175 | 3.937 | -10.170 | 22.806 | 1.00 | 24.42 | B |
| ATOM | 2709 | O | ASN | B | 175 | 5.167 | -10.201 | 22.890 | 1.00 | 25.23 | B |
| ATOM | 2710 | N | VAL | B | 176 | 3.253 | -10.901 | 21.930 | 1.00 | 23.52 | B |
| ATOM | 2711 | CA | VAL | B | 176 | 3.923 | -11.761 | 20.957 | 1.00 | 23.27 | B |
| ATOM | 2712 | CB | VAL | B | 176 | 3.362 | -13.196 | 20.971 | 1.00 | 23.31 | B |
| ATOM | 2713 | CG1 | VAL | B | 176 | 4.104 | -14.037 | 19.963 | 1.00 | 23.75 | B |
| ATOM | 2714 | CG2 | VAL | B | 176 | 3.497 | -13.797 | 22.357 | 1.00 | 22.20 | B |
| ATOM | 2715 | C | VAL | B | 176 | 3.679 | -11.137 | 19.585 | 1.00 | 23.82 | B |
| ATOM | 2716 | O | VAL | B | 176 | 2.537 | -11.041 | 19.126 | 1.00 | 23.95 | B |
| ATOM | 2717 | N | THR | B | 177 | 4.754 | -10.707 | 18.937 | 1.00 | 22.37 | B |
| ATOM | 2718 | CA | THR | B | 177 | 4.640 | -10.056 | 17.642 | 1.00 | 21.50 | B |
| ATOM | 2719 | CB | THR | B | 177 | 5.387 | -8.708 | 17.647 | 1.00 | 21.19 | B |
| ATOM | 2720 | OG1 | THR | B | 177 | 6.776 | -8.924 | 17.944 | 1.00 | 19.49 | B |
| ATOM | 2721 | CG2 | THR | B | 177 | 4.785 | -7.782 | 18.691 | 1.00 | 20.61 | B |
| ATOM | 2722 | C | THR | B | 177 | 5.143 | -10.866 | 16.457 | 1.00 | 21.13 | B |
| ATOM | 2723 | O | THR | B | 177 | 5.930 | -11.798 | 16.610 | 1.00 | 21.68 | B |
| ATOM | 2724 | N | ALA | B | 178 | 4.682 | -10.489 | 15.267 | 1.00 | 19.19 | B |
| ATOM | 2725 | CA | ALA | B | 178 | 5.086 | -11.153 | 14.038 | 1.00 | 16.91 | B |
| ATOM | 2726 | CB | ALA | B | 178 | 4.022 | -12.162 | 13.605 | 1.00 | 16.89 | B |
| ATOM | 2727 | C | ALA | B | 178 | 5.302 | -10.112 | 12.945 | 1.00 | 15.53 | B |
| ATOM | 2728 | O | ALA | B | 178 | 4.407 | -9.328 | 12.634 | 1.00 | 15.54 | B |
| ATOM | 2729 | N | ASN | B | 179 | 6.498 | -10.099 | 12.373 | 1.00 | 13.30 | B |
| ATOM | 2730 | CA | ASN | B | 179 | 6.815 | -9.160 | 11.312 | 1.00 | 13.89 | B |
| ATOM | 2731 | CB | ASN | B | 179 | 7.727 | -8.042 | 11.828 | 1.00 | 12.26 | B |
| ATOM | 2732 | CG | ASN | B | 179 | 7.065 | -7.202 | 12.905 | 1.00 | 12.79 | B |
| ATOM | 2733 | OD1 | ASN | B | 179 | 6.055 | -6.544 | 12.666 | 1.00 | 13.53 | B |
| ATOM | 2734 | ND2 | ASN | B | 179 | 7.626 | -7.228 | 14.097 | 1.00 | 13.49 | B |
| ATOM | 2735 | C | ASN | B | 179 | 7.512 | -9.928 | 10.212 | 1.00 | 14.71 | B |
| ATOM | 2736 | O | ASN | B | 179 | 7.977 | -11.045 | 10.431 | 1.00 | 18.43 | B |
| ATOM | 2737 | N | VAL | B | 180 | 7.593 | -9.329 | 9.033 | 1.00 | 13.15 | B |
| ATOM | 2738 | CA | VAL | B | 180 | 8.222 | -9.974 | 7.893 | 1.00 | 11.96 | B |
| ATOM | 2739 | CB | VAL | B | 180 | 7.167 | -10.286 | 6.794 | 1.00 | 12.43 | B |

Figure 2 (suite 37)

| | | | | | | | | | | | |
|---|---|---|---|---|---|---|---|---|---|---|---|
| ATOM | 2740 | CG1 | VAL | B | 180 | 7.853 | -10.757 | 5.529 | 1.00 | 11.10 | B |
| ATOM | 2741 | CG2 | VAL | B | 180 | 6.174 | -11.341 | 7.298 | 1.00 | 10.24 | B |
| ATOM | 2742 | C | VAL | B | 180 | 9.305 | -9.093 | 7.283 | 1.00 | 11.86 | B |
| ATOM | 2743 | O | VAL | B | 180 | 9.100 | -7.900 | 7.089 | 1.00 | 14.54 | B |
| ATOM | 2744 | N | VAL | B | 181 | 10.453 | -9.686 | 6.975 | 1.00 | 10.06 | B |
| ATOM | 2745 | CA | VAL | B | 181 | 11.542 | -8.954 | 6.361 | 1.00 | 9.63 | B |
| ATOM | 2746 | CB | VAL | B | 181 | 12.892 | -9.288 | 7.031 | 1.00 | 6.16 | B |
| ATOM | 2747 | CG1 | VAL | B | 181 | 14.030 | -8.679 | 6.253 | 1.00 | 1.08 | B |
| ATOM | 2748 | CG2 | VAL | B | 181 | 12.889 | -8.774 | 8.452 | 1.00 | 6.11 | B |
| ATOM | 2749 | C | VAL | B | 181 | 11.579 | -9.367 | 4.901 | 1.00 | 12.67 | B |
| ATOM | 2750 | O | VAL | B | 181 | 11.739 | -10.548 | 4.597 | 1.00 | 14.35 | B |
| ATOM | 2751 | N | ALA | B | 182 | 11.449 | -8.397 | 4.000 | 1.00 | 12.84 | B |
| ATOM | 2752 | CA | ALA | B | 182 | 11.441 | -8.686 | 2.569 | 1.00 | 12.54 | B |
| ATOM | 2753 | CB | ALA | B | 182 | 10.191 | -8.081 | 1.927 | 1.00 | 10.65 | B |
| ATOM | 2754 | C | ALA | B | 182 | 12.688 | -8.202 | 1.839 | 1.00 | 12.38 | B |
| ATOM | 2755 | O | ALA | B | 182 | 12.789 | -7.044 | 1.448 | 1.00 | 14.26 | B |
| ATOM | 2756 | N | PRO | B | 183 | 13.659 | -9.091 | 1.644 | 1.00 | 12.33 | B |
| ATOM | 2757 | CD | PRO | B | 183 | 13.780 | -10.445 | 2.216 | 1.00 | 12.51 | B |
| ATOM | 2758 | CA | PRO | B | 183 | 14.884 | -8.701 | 0.950 | 1.00 | 13.15 | B |
| ATOM | 2759 | CB | PRO | B | 183 | 15.860 | -9.806 | 1.346 | 1.00 | 11.89 | B |
| ATOM | 2760 | CG | PRO | B | 183 | 14.965 | -11.007 | 1.456 | 1.00 | 12.07 | B |
| ATOM | 2761 | C | PRO | B | 183 | 14.691 | -8.606 | -0.562 | 1.00 | 14.98 | B |
| ATOM | 2762 | O | PRO | B | 183 | 13.885 | -9.325 | -1.136 | 1.00 | 14.01 | B |
| ATOM | 2763 | N | GLY | B | 184 | 15.432 | -7.701 | -1.192 | 1.00 | 16.59 | B |
| ATOM | 2764 | CA | GLY | B | 184 | 15.350 | -7.532 | -2.626 | 1.00 | 17.02 | B |
| ATOM | 2765 | C | GLY | B | 184 | 16.477 | -8.342 | -3.211 | 1.00 | 19.76 | B |
| ATOM | 2766 | O | GLY | B | 184 | 16.630 | -9.506 | -2.867 | 1.00 | 19.23 | B |
| ATOM | 2767 | N | TYR | B | 185 | 17.282 | -7.741 | -4.077 | 1.00 | 22.77 | B |
| ATOM | 2768 | CA | TYR | B | 185 | 18.391 | -8.467 | -4.673 | 1.00 | 26.48 | B |
| ATOM | 2769 | CB | TYR | B | 185 | 18.782 | -7.836 | -6.012 | 1.00 | 32.48 | B |
| ATOM | 2770 | CG | TYR | B | 185 | 17.846 | -8.206 | -7.143 | 1.00 | 39.11 | B |
| ATOM | 2771 | CD1 | TYR | B | 185 | 16.483 | -7.918 | -7.069 | 1.00 | 42.16 | B |
| ATOM | 2772 | CE1 | TYR | B | 185 | 15.608 | -8.275 | -8.102 | 1.00 | 44.97 | B |
| ATOM | 2773 | CD2 | TYR | B | 185 | 18.318 | -8.859 | -8.280 | 1.00 | 42.39 | B |
| ATOM | 2774 | CE2 | TYR | B | 185 | 17.451 | -9.220 | -9.321 | 1.00 | 44.59 | B |
| ATOM | 2775 | CZ | TYR | B | 185 | 16.100 | -8.923 | -9.222 | 1.00 | 45.10 | B |
| ATOM | 2776 | OH | TYR | B | 185 | 15.242 | -9.265 | -10.241 | 1.00 | 45.91 | B |
| ATOM | 2777 | C | TYR | B | 185 | 19.597 | -8.510 | -3.741 | 1.00 | 26.86 | B |
| ATOM | 2778 | O | TYR | B | 185 | 20.348 | -7.538 | -3.625 | 1.00 | 25.29 | B |
| ATOM | 2779 | N | ILE | B | 186 | 19.776 | -9.640 | -3.064 | 1.00 | 26.49 | B |
| ATOM | 2780 | CA | ILE | B | 186 | 20.907 | -9.776 | -2.167 | 1.00 | 28.27 | B |
| ATOM | 2781 | CB | ILE | B | 186 | 20.506 | -10.360 | -0.802 | 1.00 | 25.94 | B |
| ATOM | 2782 | CG2 | ILE | B | 186 | 21.747 | -10.501 | 0.076 | 1.00 | 26.12 | B |
| ATOM | 2783 | CG1 | ILE | B | 186 | 19.490 | -9.446 | -0.109 | 1.00 | 24.78 | B |
| ATOM | 2784 | CD | ILE | B | 186 | 20.017 | -8.053 | 0.215 | 1.00 | 23.06 | B |
| ATOM | 2785 | C | ILE | B | 186 | 21.975 | -10.666 | -2.785 | 1.00 | 31.30 | B |
| ATOM | 2786 | O | ILE | B | 186 | 21.687 | -11.744 | -3.306 | 1.00 | 31.58 | B |
| ATOM | 2787 | N | ASP | B | 187 | 23.213 | -10.191 | -2.721 | 1.00 | 34.76 | B |
| ATOM | 2788 | CA | ASP | B | 187 | 24.362 | -10.901 | -3.252 | 1.00 | 37.21 | B |
| ATOM | 2789 | CB | ASP | B | 187 | 25.544 | -9.943 | -3.349 | 1.00 | 39.01 | B |
| ATOM | 2790 | CG | ASP | B | 187 | 26.751 | -10.577 | -3.980 | 1.00 | 41.19 | B |
| ATOM | 2791 | OD1 | ASP | B | 187 | 27.830 | -9.944 | -3.962 | 1.00 | 42.80 | B |
| ATOM | 2792 | OD2 | ASP | B | 187 | 26.618 | -11.705 | -4.500 | 1.00 | 42.66 | B |
| ATOM | 2793 | C | ASP | B | 187 | 24.717 | -12.062 | -2.331 | 1.00 | 39.40 | B |
| ATOM | 2794 | O | ASP | B | 187 | 24.879 | -11.874 | -1.126 | 1.00 | 39.41 | B |
| ATOM | 2795 | N | THR | B | 188 | 24.833 | -13.256 | -2.905 | 1.00 | 41.81 | B |
| ATOM | 2796 | CA | THR | B | 188 | 25.168 | -14.458 | -2.147 | 1.00 | 43.13 | B |
| ATOM | 2797 | CB | THR | B | 188 | 24.075 | -14.795 | -1.119 | 1.00 | 44.38 | B |
| ATOM | 2798 | OG1 | THR | B | 188 | 24.517 | -15.879 | -0.294 | 1.00 | 46.48 | B |
| ATOM | 2799 | CG2 | THR | B | 188 | 22.783 | -15.202 | -1.827 | 1.00 | 44.23 | B |
| ATOM | 2800 | C | THR | B | 188 | 25.327 | -15.655 | -3.079 | 1.00 | 43.49 | B |
| ATOM | 2801 | O | THR | B | 188 | 26.334 | -16.360 | -3.039 | 1.00 | 44.69 | B |
| ATOM | 2802 | N | LEU | B | 203 | 19.799 | -4.676 | -13.173 | 1.00 | 57.60 | B |
| ATOM | 2803 | CA | LEU | B | 203 | 20.335 | -4.038 | -11.977 | 1.00 | 56.83 | B |
| ATOM | 2804 | CB | LEU | B | 203 | 21.865 | -4.156 | -11.965 | 1.00 | 56.68 | B |
| ATOM | 2805 | CG | LEU | B | 203 | 22.656 | -3.878 | -10.679 | 1.00 | 55.40 | B |
| ATOM | 2806 | CD1 | LEU | B | 203 | 24.104 | -4.283 | -10.892 | 1.00 | 54.84 | B |
| ATOM | 2807 | CD2 | LEU | B | 203 | 22.572 | -2.412 | -10.298 | 1.00 | 55.69 | B |
| ATOM | 2808 | C | LEU | B | 203 | 19.919 | -2.572 | -11.963 | 1.00 | 57.44 | B |
| ATOM | 2809 | O | LEU | B | 203 | 19.814 | -1.959 | -10.901 | 1.00 | 58.78 | B |
| ATOM | 2810 | N | GLN | B | 204 | 19.675 | -2.017 | -13.149 | 1.00 | 56.96 | B |
| ATOM | 2811 | CA | GLN | B | 204 | 19.270 | -0.619 | -13.280 | 1.00 | 55.19 | B |
| ATOM | 2812 | CB | GLN | B | 204 | 19.156 | -0.240 | -14.759 | 0.00 | 55.20 | B |

Figure 2 (suite 38)

| | | | | | | | | | | | |
|---|---|---|---|---|---|---|---|---|---|---|---|
| ATOM | 2813 | CG | GLN | B | 204 | 20.462 | -0.347 | -15.529 | 0.00 | 55.05 | B |
| ATOM | 2814 | CD | GLN | B | 204 | 21.538 | 0.570 | -14.981 | 0.00 | 55.01 | B |
| ATOM | 2815 | OE1 | GLN | B | 204 | 21.374 | 1.789 | -14.951 | 0.00 | 54.97 | B |
| ATOM | 2816 | NE2 | GLN | B | 204 | 22.647 | -0.015 | -14.544 | 0.00 | 54.97 | B |
| ATOM | 2817 | C | GLN | B | 204 | 17.945 | -0.326 | -12.574 | 1.00 | 53.86 | B |
| ATOM | 2818 | O | GLN | B | 204 | 17.707 | 0.797 | -12.128 | 1.00 | 55.74 | B |
| ATOM | 2819 | N | PHE | B | 205 | 17.090 | -1.341 | -12.474 | 1.00 | 51.29 | B |
| ATOM | 2820 | CA | PHE | B | 205 | 15.782 | -1.205 | -11.830 | 1.00 | 47.47 | B |
| ATOM | 2821 | CB | PHE | B | 205 | 14.886 | -2.397 | -12.183 | 0.00 | 47.71 | B |
| ATOM | 2822 | CG | PHE | B | 205 | 14.549 | -2.500 | -13.644 | 0.00 | 47.42 | B |
| ATOM | 2823 | CD1 | PHE | B | 205 | 15.534 | -2.774 | -14.586 | 0.00 | 47.39 | B |
| ATOM | 2824 | CD2 | PHE | B | 205 | 13.238 | -2.331 | -14.078 | 0.00 | 47.39 | B |
| ATOM | 2825 | CE1 | PHE | B | 205 | 15.219 | -2.880 | -15.940 | 0.00 | 47.32 | B |
| ATOM | 2826 | CE2 | PHE | B | 205 | 12.911 | -2.435 | -15.427 | 0.00 | 47.32 | B |
| ATOM | 2827 | CZ | PHE | B | 205 | 13.904 | -2.710 | -16.361 | 0.00 | 47.31 | B |
| ATOM | 2828 | C | PHE | B | 205 | 15.900 | -1.107 | -10.310 | 1.00 | 45.53 | B |
| ATOM | 2829 | O | PHE | B | 205 | 14.895 | -1.234 | -9.599 | 1.00 | 44.89 | B |
| ATOM | 2830 | N | ILE | B | 206 | 17.120 | -0.894 | -9.814 | 1.00 | 43.23 | B |
| ATOM | 2831 | CA | ILE | B | 206 | 17.361 | -0.773 | -8.374 | 1.00 | 41.76 | B |
| ATOM | 2832 | CB | ILE | B | 206 | 18.437 | -1.756 | -7.883 | 1.00 | 41.01 | B |
| ATOM | 2833 | CG2 | ILE | B | 206 | 18.570 | -1.663 | -6.371 | 1.00 | 40.33 | B |
| ATOM | 2834 | CG1 | ILE | B | 206 | 18.065 | -3.180 | -8.272 | 1.00 | 39.69 | B |
| ATOM | 2835 | CD | ILE | B | 206 | 19.100 | -4.195 | -7.852 | 1.00 | 40.41 | B |
| ATOM | 2836 | C | ILE | B | 206 | 17.834 | 0.632 | -8.027 | 1.00 | 40.98 | B |
| ATOM | 2837 | O | ILE | B | 206 | 18.961 | 1.011 | -8.338 | 1.00 | 41.44 | B |
| ATOM | 2838 | N | PRO | B | 207 | 16.980 | 1.416 | -7.356 | 1.00 | 39.78 | B |
| ATOM | 2839 | CD | PRO | B | 207 | 15.703 | 1.011 | -6.746 | 1.00 | 39.06 | B |
| ATOM | 2840 | CA | PRO | B | 207 | 17.326 | 2.787 | -6.975 | 1.00 | 38.63 | B |
| ATOM | 2841 | CB | PRO | B | 207 | 16.288 | 3.118 | -5.911 | 1.00 | 39.20 | B |
| ATOM | 2842 | CG | PRO | B | 207 | 15.101 | 2.337 | -6.358 | 1.00 | 39.44 | B |
| ATOM | 2843 | C | PRO | B | 207 | 18.751 | 2.927 | -6.455 | 1.00 | 38.38 | B |
| ATOM | 2844 | O | PRO | B | 207 | 19.515 | 3.740 | -6.968 | 1.00 | 39.98 | B |
| ATOM | 2845 | N | ALA | B | 208 | 19.107 | 2.135 | -5.443 | 1.00 | 37.33 | B |
| ATOM | 2846 | CA | ALA | B | 208 | 20.448 | 2.197 | -4.860 | 1.00 | 35.98 | B |
| ATOM | 2847 | CB | ALA | B | 208 | 20.552 | 1.255 | -3.675 | 1.00 | 34.73 | B |
| ATOM | 2848 | C | ALA | B | 208 | 21.518 | 1.862 | -5.891 | 1.00 | 35.72 | B |
| ATOM | 2849 | O | ALA | B | 208 | 22.705 | 2.110 | -5.667 | 1.00 | 35.13 | B |
| ATOM | 2850 | N | LYS | B | 209 | 21.085 | 1.291 | -7.016 | 1.00 | 35.26 | B |
| ATOM | 2851 | CA | LYS | B | 209 | 21.975 | 0.927 | -8.121 | 1.00 | 34.76 | B |
| ATOM | 2852 | CB | LYS | B | 209 | 22.698 | 2.172 | -8.646 | 0.00 | 34.87 | B |
| ATOM | 2853 | CG | LYS | B | 209 | 21.777 | 3.237 | -9.228 | 0.00 | 34.81 | B |
| ATOM | 2854 | CD | LYS | B | 209 | 21.039 | 2.731 | -10.459 | 0.00 | 34.81 | B |
| ATOM | 2855 | CE | LYS | B | 209 | 20.135 | 3.806 | -11.043 | 0.00 | 34.79 | B |
| ATOM | 2856 | NZ | LYS | B | 209 | 19.409 | 3.328 | -12.252 | 0.00 | 34.78 | B |
| ATOM | 2857 | C | LYS | B | 209 | 23.006 | -0.144 | -7.759 | 1.00 | 34.86 | B |
| ATOM | 2858 | O | LYS | B | 209 | 24.044 | -0.244 | -8.414 | 1.00 | 35.48 | B |
| ATOM | 2859 | N | ARG | B | 210 | 22.726 | -0.944 | -6.730 | 1.00 | 34.58 | B |
| ATOM | 2860 | CA | ARG | B | 210 | 23.649 | -2.000 | -6.302 | 1.00 | 33.50 | B |
| ATOM | 2861 | CB | ARG | B | 210 | 24.731 | -1.420 | -5.381 | 1.00 | 31.31 | B |
| ATOM | 2862 | CG | ARG | B | 210 | 24.229 | -1.037 | -3.997 | 1.00 | 29.26 | B |
| ATOM | 2863 | CD | ARG | B | 210 | 25.341 | -0.426 | -3.137 | 1.00 | 28.36 | B |
| ATOM | 2864 | NE | ARG | B | 210 | 24.856 | -0.066 | -1.807 | 1.00 | 26.53 | B |
| ATOM | 2865 | CZ | ARG | B | 210 | 24.554 | -0.945 | -0.855 | 1.00 | 25.95 | B |
| ATOM | 2866 | NH1 | ARG | B | 210 | 24.692 | -2.246 | -1.071 | 1.00 | 25.91 | B |
| ATOM | 2867 | NH2 | ARG | B | 210 | 24.088 | -0.526 | 0.309 | 1.00 | 26.12 | B |
| ATOM | 2868 | C | ARG | B | 210 | 22.908 | -3.118 | -5.561 | 1.00 | 33.48 | B |
| ATOM | 2869 | O | ARG | B | 210 | 21.769 | -2.929 | -5.127 | 1.00 | 34.49 | B |
| ATOM | 2870 | N | VAL | B | 211 | 23.542 | -4.279 | -5.421 | 1.00 | 32.44 | B |
| ATOM | 2871 | CA | VAL | B | 211 | 22.909 | -5.383 | -4.697 | 1.00 | 31.70 | B |
| ATOM | 2872 | CB | VAL | B | 211 | 23.415 | -6.774 | -5.162 | 1.00 | 30.22 | B |
| ATOM | 2873 | CG1 | VAL | B | 211 | 22.927 | -7.064 | -6.573 | 1.00 | 30.43 | B |
| ATOM | 2874 | CG2 | VAL | B | 211 | 24.922 | -6.826 | -5.093 | 1.00 | 27.66 | B |
| ATOM | 2875 | C | VAL | B | 211 | 23.224 | -5.239 | -3.216 | 1.00 | 31.05 | B |
| ATOM | 2876 | O | VAL | B | 211 | 24.235 | -4.639 | -2.851 | 1.00 | 31.40 | B |
| ATOM | 2877 | N | GLY | B | 212 | 22.354 | -5.776 | -2.368 | 1.00 | 29.81 | B |
| ATOM | 2878 | CA | GLY | B | 212 | 22.581 | -5.699 | -0.938 | 1.00 | 27.78 | B |
| ATOM | 2879 | C | GLY | B | 212 | 23.395 | -6.898 | -0.487 | 1.00 | 26.05 | B |
| ATOM | 2880 | O | GLY | B | 212 | 23.440 | -7.918 | -1.182 | 1.00 | 25.24 | B |
| ATOM | 2881 | N | THR | B | 213 | 24.051 | -6.781 | 0.663 | 1.00 | 23.16 | B |
| ATOM | 2882 | CA | THR | B | 213 | 24.847 | -7.881 | 1.187 | 1.00 | 21.32 | B |
| ATOM | 2883 | CB | THR | B | 213 | 26.156 | -7.390 | 1.807 | 1.00 | 19.76 | B |
| ATOM | 2884 | OG1 | THR | B | 213 | 25.868 | -6.499 | 2.890 | 1.00 | 20.49 | B |
| ATOM | 2885 | CG2 | THR | B | 213 | 26.979 | -6.675 | 0.779 | 1.00 | 19.67 | B |

Figure 2 (suite 39)

| | | | | | | | | | | | |
|---|---|---|---|---|---|---|---|---|---|---|---|
| ATOM | 2886 | C | THR | B | 213 | 24.049 | -8.599 | 2.259 | 1.00 | 20.76 | B |
| ATOM | 2887 | O | THR | B | 213 | 23.076 | -8.061 | 2.787 | 1.00 | 19.93 | B |
| ATOM | 2888 | N | PRO | B | 214 | 24.444 | -9.835 | 2.592 | 1.00 | 20.67 | B |
| ATOM | 2889 | CD | PRO | B | 214 | 25.454 | -10.673 | 1.923 | 1.00 | 20.24 | B |
| ATOM | 2890 | CA | PRO | B | 214 | 23.726 | -10.595 | 3.619 | 1.00 | 18.45 | B |
| ATOM | 2891 | CB | PRO | B | 214 | 24.460 | -11.931 | 3.629 | 1.00 | 17.85 | B |
| ATOM | 2892 | CG | PRO | B | 214 | 24.950 | -12.058 | 2.221 | 1.00 | 19.97 | B |
| ATOM | 2893 | C | PRO | B | 214 | 23.744 | -9.906 | 4.987 | 1.00 | 17.23 | B |
| ATOM | 2894 | O | PRO | B | 214 | 22.847 | -10.107 | 5.809 | 1.00 | 16.42 | B |
| ATOM | 2895 | N | ALA | B | 215 | 24.765 | -9.088 | 5.220 | 1.00 | 15.79 | B |
| ATOM | 2896 | CA | ALA | B | 215 | 24.907 | -8.381 | 6.489 | 1.00 | 16.15 | B |
| ATOM | 2897 | CB | ALA | B | 215 | 26.317 | -7.811 | 6.606 | 1.00 | 13.12 | B |
| ATOM | 2898 | C | ALA | B | 215 | 23.871 | -7.262 | 6.637 | 1.00 | 16.78 | B |
| ATOM | 2899 | O | ALA | B | 215 | 23.411 | -6.971 | 7.748 | 1.00 | 14.51 | B |
| ATOM | 2900 | N | GLU | B | 216 | 23.504 | -6.648 | 5.512 | 1.00 | 15.98 | B |
| ATOM | 2901 | CA | GLU | B | 216 | 22.526 | -5.564 | 5.514 | 1.00 | 16.29 | B |
| ATOM | 2902 | CB | GLU | B | 216 | 22.532 | -4.846 | 4.161 | 1.00 | 16.44 | B |
| ATOM | 2903 | CG | GLU | B | 216 | 23.834 | -4.087 | 3.923 | 1.00 | 19.17 | B |
| ATOM | 2904 | CD | GLU | B | 216 | 23.861 | -3.303 | 2.627 | 1.00 | 20.82 | B |
| ATOM | 2905 | OE1 | GLU | B | 216 | 23.857 | -3.935 | 1.544 | 1.00 | 24.88 | B |
| ATOM | 2906 | OE2 | GLU | B | 216 | 23.893 | -2.052 | 2.693 | 1.00 | 19.73 | B |
| ATOM | 2907 | C | GLU | B | 216 | 21.128 | -6.056 | 5.864 | 1.00 | 15.91 | B |
| ATOM | 2908 | O | GLU | B | 216 | 20.248 | -5.265 | 6.209 | 1.00 | 17.75 | B |
| ATOM | 2909 | N | VAL | B | 217 | 20.929 | -7.368 | 5.796 | 1.00 | 14.30 | B |
| ATOM | 2910 | CA | VAL | B | 217 | 19.644 | -7.958 | 6.135 | 1.00 | 13.78 | B |
| ATOM | 2911 | CB | VAL | B | 217 | 19.351 | -9.193 | 5.242 | 1.00 | 14.33 | B |
| ATOM | 2912 | CG1 | VAL | B | 217 | 18.015 | -9.810 | 5.606 | 1.00 | 11.17 | B |
| ATOM | 2913 | CG2 | VAL | B | 217 | 19.359 | -8.778 | 3.781 | 1.00 | 13.80 | B |
| ATOM | 2914 | C | VAL | B | 217 | 19.710 | -8.370 | 7.603 | 1.00 | 13.60 | B |
| ATOM | 2915 | O | VAL | B | 217 | 18.740 | -8.226 | 8.356 | 1.00 | 13.97 | B |
| ATOM | 2916 | N | ALA | B | 218 | 20.873 | -8.869 | 8.005 | 1.00 | 13.37 | B |
| ATOM | 2917 | CA | ALA | B | 218 | 21.097 | -9.300 | 9.382 | 1.00 | 11.98 | B |
| ATOM | 2918 | CB | ALA | B | 218 | 22.525 | -9.772 | 9.542 | 1.00 | 12.54 | B |
| ATOM | 2919 | C | ALA | B | 218 | 20.818 | -8.152 | 10.342 | 1.00 | 11.59 | B |
| ATOM | 2920 | O | ALA | B | 218 | 20.174 | -8.327 | 11.382 | 1.00 | 9.25 | B |
| ATOM | 2921 | N | GLY | B | 219 | 21.304 | -6.970 | 9.981 | 1.00 | 12.05 | B |
| ATOM | 2922 | CA | GLY | B | 219 | 21.097 | -5.801 | 10.823 | 1.00 | 14.58 | B |
| ATOM | 2923 | C | GLY | B | 219 | 19.626 | -5.552 | 11.098 | 1.00 | 14.85 | B |
| ATOM | 2924 | O | GLY | B | 219 | 19.231 | -5.215 | 12.222 | 1.00 | 15.30 | B |
| ATOM | 2925 | N | VAL | B | 220 | 18.809 | -5.732 | 10.065 | 1.00 | 13.64 | B |
| ATOM | 2926 | CA | VAL | B | 220 | 17.376 | -5.530 | 10.194 | 1.00 | 11.99 | B |
| ATOM | 2927 | CB | VAL | B | 220 | 16.659 | -5.716 | 8.834 | 1.00 | 12.62 | B |
| ATOM | 2928 | CG1 | VAL | B | 220 | 15.208 | -5.262 | 8.948 | 1.00 | 12.49 | B |
| ATOM | 2929 | CG2 | VAL | B | 220 | 17.387 | -4.932 | 7.742 | 1.00 | 12.48 | B |
| ATOM | 2930 | C | VAL | B | 220 | 16.814 | -6.525 | 11.196 | 1.00 | 11.11 | B |
| ATOM | 2931 | O | VAL | B | 220 | 16.082 | -6.144 | 12.115 | 1.00 | 10.50 | B |
| ATOM | 2932 | N | VAL | B | 221 | 17.163 | -7.799 | 11.019 | 1.00 | 11.26 | B |
| ATOM | 2933 | CA | VAL | B | 221 | 16.693 | -8.854 | 11.913 | 1.00 | 11,59 | B |
| ATOM | 2934 | CB | VAL | B | 221 | 17.225 | -10.234 | 11.487 | 1.00 | 12.51 | B |
| ATOM | 2935 | CG1 | VAL | B | 221 | 16.691 | -11.314 | 12.420 | 1.00 | 9.41 | B |
| ATOM | 2936 | CG2 | VAL | B | 221 | 16.813 | -10.522 | 10.055 | 1.00 | 12.76 | B |
| ATOM | 2937 | C | VAL | B | 221 | 17.171 | -8.556 | 13.322 | 1.00 | 12.66 | B |
| ATOM | 2938 | O | VAL | B | 221 | 16.409 | -8.681 | 14.293 | 1.00 | 12.24 | B |
| ATOM | 2939 | N | SER | B | 222 | 18.438 | -8.160 | 13.439 | 1.00 | 11.72 | B |
| ATOM | 2940 | CA | SER | B | 222 | 18.981 | -7.818 | 14.748 | 1.00 | 11.52 | B |
| ATOM | 2941 | CB | SER | B | 222 | 20.401 | -7.267 | 14.611 | 1.00 | 14.90 | B |
| ATOM | 2942 | OG | SER | B | 222 | 20.904 | -6.866 | 15.865 | 1.00 | 15.56 | B |
| ATOM | 2943 | C | SER | B | 222 | 18.065 | -6.765 | 15.370 | 1.00 | 11.54 | B |
| ATOM | 2944 | O | SER | B | 222 | 17.612 | -6.909 | 16.518 | 1.00 | 11.06 | B |
| ATOM | 2945 | N | PHE | B | 223 | 17.779 | -5.708 | 14.612 | 1.00 | 8.63 | B |
| ATOM | 2946 | CA | PHE | B | 223 | 16.897 | -4.675 | 15.125 | 1.00 | 8.48 | B |
| ATOM | 2947 | CB | PHE | B | 223 | 16.703 | -3.558 | 14.101 | 1.00 | 11.04 | B |
| ATOM | 2948 | CG | PHE | B | 223 | 15.501 | -2.698 | 14.383 | 1.00 | 11.34 | B |
| ATOM | 2949 | CD1 | PHE | B | 223 | 15.441 | -1.930 | 15.546 | 1.00 | 8.49 | B |
| ATOM | 2950 | CD2 | PHE | B | 223 | 14.389 | -2.736 | 13.539 | 1.00 | 8.19 | B |
| ATOM | 2951 | CE1 | PHE | B | 223 | 14.294 | -1.224 | 15.870 | 1.00 | 8.99 | B |
| ATOM | 2952 | CE2 | PHE | B | 223 | 13.234 | -2.033 | 13.857 | 1.00 | 8.19 | B |
| ATOM | 2953 | CZ | PHE | B | 223 | 13.184 | -1.278 | 15.024 | 1.00 | 10.25 | B |
| ATOM | 2954 | C | PHE | B | 223 | 15.530 | -5.243 | 15.517 | 1.00 | 10.39 | B |
| ATOM | 2955 | O | PHE | B | 223 | 14.981 | -4.882 | 16.564 | 1.00 | 11.38 | B |
| ATOM | 2956 | N | LEU | B | 224 | 14.973 | -6.129 | 14.694 | 1.00 | 10.91 | B |
| ATOM | 2957 | CA | LEU | B | 224 | 13.663 | -6.690 | 15.020 | 1.00 | 12.84 | B |
| ATOM | 2958 | CB | LEU | B | 224 | 13.058 | -7.409 | 13.804 | 1.00 | 11.87 | B |

Figure 2 (suite 40)

| | | | | | | | | | | | |
|---|---|---|---|---|---|---|---|---|---|---|---|
| ATOM | 2959 | CG | LEU | B | 224 | 12.555 | -6.437 | 12.729 | 1.00 | 12.78 | B |
| ATOM | 2960 | CD1 | LEU | B | 224 | 12.140 | -7.192 | 11.480 | 1.00 | 14.87 | B |
| ATOM | 2961 | CD2 | LEU | B | 224 | 11.389 | -5.638 | 13.280 | 1.00 | 9.82 | B |
| ATOM | 2962 | C | LEU | B | 224 | 13.694 | -7.620 | 16.225 | 1.00 | 13.24 | B |
| ATOM | 2963 | O | LEU | B | 224 | 12.654 | -7.936 | 16.806 | 1.00 | 12.69 | B |
| ATOM | 2964 | N | ALA | B | 225 | 14.891 | -8.040 | 16.616 | 1.00 | 13.98 | B |
| ATOM | 2965 | CA | ALA | B | 225 | 15.024 | -8.935 | 17.762 | 1.00 | 14.97 | B |
| ATOM | 2966 | CB | ALA | B | 225 | 16.145 | -9.935 | 17.509 | 1.00 | 14.11 | B |
| ATOM | 2967 | C | ALA | B | 225 | 15.287 | -8.170 | 19.059 | 1.00 | 14.17 | B |
| ATOM | 2968 | O | ALA | B | 225 | 15.068 | -8.691 | 20.155 | 1.00 | 11.90 | B |
| ATOM | 2969 | N | SER | B | 226 | 15.737 | -6.926 | 18.921 | 1.00 | 15.24 | B |
| ATOM | 2970 | CA | SER | B | 226 | 16.063 | -6.075 | 20.071 | 1.00 | 16.86 | B |
| ATOM | 2971 | CB | SER | B | 226 | 16.893 | -4.872 | 19.610 | 1.00 | 17.00 | B |
| ATOM | 2972 | OG | SER | B | 226 | 16.094 | -3.943 | 18.889 | 1.00 | 15.97 | B |
| ATOM | 2973 | C | SER | B | 226 | 14.876 | -5.560 | 20.872 | 1.00 | 16.91 | B |
| ATOM | 2974 | O | SER | B | 226 | 13.729 | -5.672 | 20.457 | 1.00 | 19.35 | B |
| ATOM | 2975 | N | GLU | B | 227 | 15.171 | -4.978 | 22.026 | 1.00 | 18.23 | B |
| ATOM | 2976 | CA | GLU | B | 227 | 14.145 | -4.417 | 22.901 | 1.00 | 19.32 | B |
| ATOM | 2977 | CB | GLU | B | 227 | 14.736 | -4.098 | 24.272 | 1.00 | 20.22 | B |
| ATOM | 2978 | CG | GLU | B | 227 | 15.207 | -5.295 | 25.026 | 1.00 | 22.05 | B |
| ATOM | 2979 | CD | GLU | B | 227 | 14.056 | -6.105 | 25.552 | 1.00 | 26.31 | B |
| ATOM | 2980 | OE1 | GLU | B | 227 | 13.329 | -5.591 | 26.430 | 1.00 | 29.43 | B |
| ATOM | 2981 | OE2 | GLU | B | 227 | 13.875 | -7.247 | 25.082 | 1.00 | 28.49 | B |
| ATOM | 2982 | C | GLU | B | 227 | 13.553 | -3.136 | 22.328 | 1.00 | 18.28 | B |
| ATOM | 2983 | O | GLU | B | 227 | 12.569 | -2.627 | 22.856 | 1.00 | 18.22 | B |
| ATOM | 2984 | N | ASP | B | 228 | 14.165 | -2.616 | 21.267 | 1.00 | 18.38 | B |
| ATOM | 2985 | CA | ASP | B | 228 | 13.705 | -1.383 | 20.625 | 1.00 | 19.69 | B |
| ATOM | 2986 | CB | ASP | B | 228 | 14.869 | -0.705 | 19.874 | 1.00 | 18.65 | B |
| ATOM | 2987 | CG | ASP | B | 228 | 15.740 | 0.154 | 20.787 | 1.00 | 19.68 | B |
| ATOM | 2988 | OD1 | ASP | B | 228 | 16.942 | 0.338 | 20.476 | 1.00 | 16.40 | B |
| ATOM | 2989 | OD2 | ASP | B | 228 | 15.215 | 0.656 | 21.809 | 1.00 | 19.23 | B |
| ATOM | 2990 | C | ASP | B | 228 | 12.542 | -1.578 | 19.654 | 1.00 | 19.51 | B |
| ATOM | 2991 | O | ASP | B | 228 | 11.886 | -0.610 | 19.275 | 1.00 | 20.67 | B |
| ATOM | 2992 | N | ALA | B | 229 | 12.286 | -2.822 | 19.260 | 1.00 | 18.58 | B |
| ATOM | 2993 | CA | ALA | B | 229 | 11.219 | -3.121 | 18.304 | 1.00 | 18.90 | B |
| ATOM | 2994 | CB | ALA | B | 229 | 11.723 | -4.141 | 17.285 | 1.00 | 17.68 | B |
| ATOM | 2995 | C | ALA | B | 229 | 9.921 | -3.627 | 18.930 | 1.00 | 18.65 | B |
| ATOM | 2996 | O | ALA | B | 229 | 9.101 | -4.227 | 18.245 | 1.00 | 16.90 | B |
| ATOM | 2997 | N | SER | B | 230 | 9.720 | -3.355 | 20.213 | 1.00 | 18.07 | B |
| ATOM | 2998 | CA | SER | B | 230 | 8.537 | -3.844 | 20.906 | 1.00 | 18.42 | B |
| ATOM | 2999 | CB | SER | B | 230 | 8.611 | -3.488 | 22.384 | 1.00 | 19.44 | B |
| ATOM | 3000 | OG | SER | B | 230 | 8.598 | -2.082 | 22.562 | 1.00 | 21.78 | B |
| ATOM | 3001 | C | SER | B | 230 | 7.185 | -3.384 | 20.381 | 1.00 | 19.18 | B |
| ATOM | 3002 | O | SER | B | 230 | 6.176 | -4.050 | 20.625 | 1.00 | 20.61 | B |
| ATOM | 3003 | N | TYR | B | 231 | 7.135 | -2.260 | 19.675 | 1.00 | 18.10 | B |
| ATOM | 3004 | CA | TYR | B | 231 | 5.841 | -1.786 | 19.192 | 1.00 | 17.34 | B |
| ATOM | 3005 | CB | TYR | B | 231 | 5.600 | -0.342 | 19.658 | 1.00 | 14.86 | B |
| ATOM | 3006 | CG | TYR | B | 231 | 4.133 | 0.044 | 19.756 | 1.00 | 14.70 | B |
| ATOM | 3007 | CD1 | TYR | B | 231 | 3.241 | -0.723 | 20.507 | 1.00 | 13.86 | B |
| ATOM | 3008 | CE1 | TYR | B | 231 | 1.900 | -0.335 | 20.663 | 1.00 | 14.17 | B |
| ATOM | 3009 | CD2 | TYR | B | 231 | 3.648 | 1.211 | 19.152 | 1.00 | 13.83 | B |
| ATOM | 3010 | CE2 | TYR | B | 231 | 2.311 | 1.608 | 19.302 | 1.00 | 12.33 | B |
| ATOM | 3011 | CZ | TYR | B | 231 | 1.442 | 0.832 | 20.060 | 1.00 | 14.21 | B |
| ATOM | 3012 | OH | TYR | B | 231 | 0.118 | 1.212 | 20.221 | 1.00 | 12.15 | B |
| ATOM | 3013 | C | TYR | B | 231 | 5.674 | -1.889 | 17.678 | 1.00 | 16.13 | B |
| ATOM | 3014 | O | TYR | B | 231 | 4.908 | -1.135 | 17.076 | 1.00 | 16.69 | B |
| ATOM | 3015 | N | ILE | B | 232 | 6.401 | -2.813 | 17.062 | 1.00 | 14.27 | B |
| ATOM | 3016 | CA | ILE | B | 232 | 6.289 | -3.014 | 15.623 | 1.00 | 13.30 | B |
| ATOM | 3017 | CB | ILE | B | 232 | 7.650 | -2.938 | 14.946 | 1.00 | 11.24 | B |
| ATOM | 3018 | CG2 | ILE | B | 232 | 7.528 | -3.379 | 13.499 | 1.00 | 13.21 | B |
| ATOM | 3019 | CG1 | ILE | B | 232 | 8.202 | -1.521 | 15.082 | 1.00 | 10.28 | B |
| ATOM | 3020 | CD | ILE | B | 232 | 9.553 | -1.322 | 14.450 | 1.00 | 12.70 | B |
| ATOM | 3021 | C | ILE | B | 232 | 5.689 | -4.391 | 15.367 | 1.00 | 13.53 | B |
| ATOM | 3022 | O | ILE | B | 232 | 6.377 | -5.395 | 15.485 | 1.00 | 15.17 | B |
| ATOM | 3023 | N | SER | B | 233 | 4.409 | -4.442 | 15.020 | 1.00 | 13.64 | B |
| ATOM | 3024 | CA | SER | B | 233 | 3.757 | -5.726 | 14.773 | 1.00 | 15.80 | B |
| ATOM | 3025 | CB | SER | B | 233 | 2.788 | -6.042 | 15.917 | 1.00 | 16.89 | B |
| ATOM | 3026 | OG | SER | B | 233 | 2.390 | -7.401 | 15.887 | 1.00 | 16.56 | B |
| ATOM | 3027 | C | SER | B | 233 | 2.996 | -5.786 | 13.448 | 1.00 | 15.27 | B |
| ATOM | 3028 | O | SER | B | 233 | 2.279 | -4.859 | 13.095 | 1.00 | 15.59 | B |
| ATOM | 3029 | N | GLY | B | 234 | 3.150 | -6.886 | 12.721 | 1.00 | 14.64 | B |
| ATOM | 3030 | CA | GLY | B | 234 | 2.434 | -7.035 | 11.468 | 1.00 | 12.75 | B |
| ATOM | 3031 | C | GLY | B | 234 | 2.995 | -6.220 | 10.331 | 1.00 | 10.83 | B |

Figure 2 (suite 41)

| | | | | | | | | | | | |
|---|---|---|---|---|---|---|---|---|---|---|---|
| ATOM | 3032 | O | GLY | B | 234 | 2.314 | -6.011 | 9.327 | 1.00 | 10.90 | B |
| ATOM | 3033 | N | ALA | B | 235 | 4.241 | -5.783 | 10.477 | 1.00 | 9.74 | B |
| ATOM | 3034 | CA | ALA | B | 235 | 4.891 | -4.974 | 9.457 | 1.00 | 8.92 | B |
| ATOM | 3035 | CB | ALA | B | 235 | 5.792 | -3.922 | 10.114 | 1.00 | 9.35 | B |
| ATOM | 3036 | C | ALA | B | 235 | 5.698 | -5.769 | 8.452 | 1.00 | 9.28 | B |
| ATOM | 3037 | O | ALA | B | 235 | 6.073 | -6.908 | 8.692 | 1.00 | 11.38 | B |
| ATOM | 3038 | N | VAL | B | 236 | 5.960 | -5.134 | 7.318 | 1.00 | 9.46 | B |
| ATOM | 3039 | CA | VAL | B | 236 | 6.736 | -5.716 | 6.243 | 1.00 | 7.52 | B |
| ATOM | 3040 | CB | VAL | B | 236 | 5.895 | -5.840 | 4.934 | 1.00 | 6.09 | B |
| ATOM | 3041 | CG1 | VAL | B | 236 | 6.731 | -6.461 | 3.826 | 1.00 | 2.24 | B |
| ATOM | 3042 | CG2 | VAL | B | 236 | 4.654 | -6.687 | 5.178 | 1.00 | 5.40 | B |
| ATOM | 3043 | C | VAL | B | 236 | 7.871 | -4.723 | 6.007 | 1.00 | 10.25 | B |
| ATOM | 3044 | O | VAL | B | 236 | 7.654 | -3.641 | 5.463 | 1.00 | 12.24 | B |
| ATOM | 3045 | N | ILE | B | 237 | 9.079 | -5.063 | 6.435 | 1.00 | 10.91 | B |
| ATOM | 3046 | CA | ILE | B | 237 | 10.178 | -4.151 | 6.223 | 1.00 | 12.89 | B |
| ATOM | 3047 | CB | ILE | B | 237 | 11.113 | -4.060 | 7.456 | 1.00 | 13.00 | B |
| ATOM | 3048 | CG2 | ILE | B | 237 | 12.355 | -3.267 | 7.106 | 1.00 | 13.92 | B |
| ATOM | 3049 | CG1 | ILE | B | 237 | 10.419 | -3.327 | 8.599 | 1.00 | 12.80 | B |
| ATOM | 3050 | CD | ILE | B | 237 | 9.568 | -4.194 | 9.465 | 1.00 | 16.58 | B |
| ATOM | 3051 | C | ILE | B | 237 | 10.981 | -4.586 | 5.013 | 1.00 | 14.77 | B |
| ATOM | 3052 | O | ILE | B | 237 | 11.642 | -5.624 | 5.028 | 1.00 | 16.43 | B |
| ATOM | 3053 | N | PRO | B | 238 | 10.923 | -3.799 | 3.934 | 1.00 | 14.81 | B |
| ATOM | 3054 | CD | PRO | B | 238 | 10.060 | -2.618 | 3.745 | 1.00 | 14.71 | B |
| ATOM | 3055 | CA | PRO | B | 238 | 11.663 | -4.117 | 2.709 | 1.00 | 14.37 | B |
| ATOM | 3056 | CB | PRO | B | 238 | 10.948 | -3.276 | 1.654 | 1.00 | 12.75 | B |
| ATOM | 3057 | CG | PRO | B | 238 | 10.560 | -2.054 | 2.426 | 1.00 | 14.23 | B |
| ATOM | 3058 | C | PRO | B | 238 | 13.145 | -3.743 | 2.857 | 1.00 | 15.08 | B |
| ATOM | 3059 | O | PRO | B | 238 | 13.485 | -2.758 | 3.521 | 1.00 | 16.72 | B |
| ATOM | 3060 | N | VAL | B | 239 | 14.019 | -4.533 | 2.245 | 1.00 | 11.37 | B |
| ATOM | 3061 | CA | VAL | B | 239 | 15.448 | -4.293 | 2.309 | 1.00 | 12.31 | B |
| ATOM | 3062 | CB | VAL | B | 239 | 16.135 | -5.286 | 3.310 | 1.00 | 13.17 | B |
| ATOM | 3063 | CG1 | VAL | B | 239 | 17.641 | -4.999 | 3.417 | 1.00 | 8.93 | B |
| ATOM | 3064 | CG2 | VAL | B | 239 | 15.466 | -5.176 | 4.691 | 1.00 | 11.00 | B |
| ATOM | 3065 | C | VAL | B | 239 | 15.891 | -4.556 | 0.887 | 1.00 | 14.82 | B |
| ATOM | 3066 | O | VAL | B | 239 | 16.576 | -5.535 | 0.599 | 1.00 | 15.76 | B |
| ATOM | 3067 | N | ASP | B | 240 | 15.489 | -3.652 | 0.003 | 1.00 | 16.57 | B |
| ATOM | 3068 | CA | ASP | B | 240 | 15.752 | -3.758 | -1.425 | 1.00 | 17.93 | B |
| ATOM | 3069 | CB | ASP | B | 240 | 14.427 | -3.960 | -2.131 | 1.00 | 14.97 | B |
| ATOM | 3070 | CG | ASP | B | 240 | 13.389 | -2.966 | -1.659 | 1.00 | 14.64 | B |
| ATOM | 3071 | OD1 | ASP | B | 240 | 13.737 | -1.770 | -1.565 | 1.00 | 13.44 | B |
| ATOM | 3072 | OD2 | ASP | B | 240 | 12.241 | -3.368 | -1.372 | 1.00 | 15.00 | B |
| ATOM | 3073 | C | ASP | B | 240 | 16.410 | -2.513 | -1.992 | 1.00 | 21.59 | B |
| ATOM | 3074 | O | ASP | B | 240 | 16.645 | -2.421 | -3.198 | 1.00 | 23.06 | B |
| ATOM | 3075 | N | GLY | B | 241 | 16.677 | -1.540 | -1.134 | 1.00 | 24.96 | B |
| ATOM | 3076 | CA | GLY | B | 241 | 17.303 | -0.321 | -1.605 | 1.00 | 26.89 | B |
| ATOM | 3077 | C | GLY | B | 241 | 16.457 | 0.485 | -2.581 | 1.00 | 27.44 | B |
| ATOM | 3078 | O | GLY | B | 241 | 16.992 | 1.045 | -3.540 | 1.00 | 28.00 | B |
| ATOM | 3079 | N | GLY | B | 242 | 15.148 | 0.547 | -2.347 | 1.00 | 27.15 | B |
| ATOM | 3080 | CA | GLY | B | 242 | 14.278 | 1.315 | -3.224 | 1.00 | 29.09 | B |
| ATOM | 3081 | C | GLY | B | 242 | 13.412 | 0.488 | -4.157 | 1.00 | 30.74 | B |
| ATOM | 3082 | O | GLY | B | 242 | 12.254 | 0.827 | -4.396 | 1.00 | 31.22 | B |
| ATOM | 3083 | N | MET | B | 243 | 13.972 | -0.596 | -4.685 | 1.00 | 33.44 | B |
| ATOM | 3084 | CA | MET | B | 243 | 13.259 | -1.487 | -5.598 | 1.00 | 36.67 | B |
| ATOM | 3085 | CB | MET | B | 243 | 14.086 | -2.748 | -5.827 | 1.00 | 40.03 | B |
| ATOM | 3086 | CG | MET | B | 243 | 13.292 | -3.931 | -6.341 | 1.00 | 43.82 | B |
| ATOM | 3087 | SD | MET | B | 243 | 14.322 | -5.399 | -6.471 | 1.00 | 50.43 | B |
| ATOM | 3088 | CE | MET | B | 243 | 15.220 | -5.027 | -7.993 | 1.00 | 49.99 | B |
| ATOM | 3089 | C | MET | B | 243 | 11.884 | -1.878 | -5.070 | 1.00 | 37.74 | B |
| ATOM | 3090 | O | MET | B | 243 | 11.762 | -2.396 | -3.962 | 1.00 | 39.47 | B |
| ATOM | 3091 | N | GLY | | 244 | 10.848 | -1.646 | -5.868 | 1.00 | 38.47 | |
| ATOM | 3092 | CA | GLY | | 244 | 9.509 | -1.991 | -5.423 | 1.00 | 39.54 | |
| ATOM | 3093 | C | GLY | | 244 | 9.175 | -1.318 | -4.102 | 1.00 | 39.55 | |
| ATOM | 3094 | O | GLY | | 244 | 9.897 | -0.424 | -3.651 | 1.00 | 38.25 | |
| ATOM | 3095 | OH2 | TIP | S | 1 | -1.056 | 2.249 | 17.865 | 1.00 | 1.80 | S |
| ATOM | 3096 | OH2 | TIP | S | 2 | 21.926 | 5.634 | 15.965 | 1.00 | 6.22 | S |
| ATOM | 3097 | OH2 | TIP | S | 3 | 11.103 | 4.116 | -3.706 | 1.00 | 25.81 | S |
| ATOM | 3098 | OH2 | TIP | S | 4 | 19.967 | -2.584 | 5.590 | 1.00 | 10.13 | S |
| ATOM | 3099 | OH2 | TIP | S | 5 | 19.571 | -8.073 | 18.532 | 1.00 | 21.65 | S |
| ATOM | 3100 | OH2 | TIP | S | 6 | 25.299 | -7.311 | 10.537 | 1.00 | 36.47 | S |
| ATOM | 3101 | OH2 | TIP | S | 7 | 1.665 | 8.317 | 11.249 | 1.00 | 25.73 | S |
| ATOM | 3102 | OH2 | TIP | S | 8 | 8.989 | -0.055 | -7.515 | 1.00 | 33.45 | S |
| ATOM | 3103 | OH2 | TIP | S | 9 | 14.106 | -27.585 | 5.601 | 1.00 | 18.66 | S |
| ATOM | 3104 | OH2 | TIP | S | 10 | 9.830 | 19.340 | 9.518 | 1.00 | 26.40 | S |

Figure 2 (suite 42)

| | | | | | | | | | | | |
|---|---|---|---|---|---|---|---|---|---|---|---|
| ATOM | 3105 | OH2 | TIP | S | 11 | 7.723 | 19.218 | 14.510 | 1.00 | 37.90 | S |
| ATOM | 3106 | OH2 | TIP | S | 12 | 6.830 | 8.402 | -5.694 | 1.00 | 33.23 | S |
| ATOM | 3107 | OH2 | TIP | S | 17 | 16.701 | -26.276 | 8.088 | 1.00 | 16.94 | S |
| ATOM | 3108 | OH2 | TIP | S | 18 | 14.764 | 5.018 | 23.205 | 1.00 | 22.10 | S |
| ATOM | 3109 | OH2 | TIP | S | 20 | 8.858 | -0.214 | 18.727 | 1.00 | 21.55 | S |
| ATOM | 3110 | OH2 | TIP | S | 21 | -0.184 | 16.340 | 30.292 | 1.00 | 61.45 | S |
| ATOM | 3111 | OH2 | TIP | S | 22 | 29.315 | 16.174 | 9.303 | 1.00 | 34.45 | S |
| ATOM | 3112 | OH2 | TIP | S | 23 | 7.289 | 16.969 | 25.970 | 1.00 | 28.73 | S |
| ATOM | 3113 | OH2 | TIP | S | 26 | 5.326 | 12.156 | 26.476 | 1.00 | 49.73 | S |
| ATOM | 3114 | OH2 | TIP | S | 27 | 23.222 | -21.802 | 22.533 | 1.00 | 30.20 | S |
| ATOM | 3115 | OH2 | TIP | S | 29 | 17.654 | -4.955 | -4.329 | 1.00 | 17.49 | S |
| ATOM | 3116 | OH2 | TIP | S | 31 | 11.069 | -10.743 | 28.590 | 1.00 | 27.23 | S |
| ATOM | 3117 | OH2 | TIP | S | 32 | 7.184 | 16.096 | 18.764 | 1.00 | 27.13 | S |
| ATOM | 3118 | OH2 | TIP | S | 33 | 12.397 | -40.194 | 12.763 | 1.00 | 62.36 | S |
| ATOM | 3119 | OH2 | TIP | S | 34 | 29.178 | 13.072 | 19.894 | 1.00 | 26.99 | S |
| ATOM | 3120 | OH2 | TIP | S | 35 | 28.256 | -7.017 | 20.671 | 1.00 | 1.07 | S |
| ATOM | 3121 | OH2 | TIP | S | 36 | 15.270 | -8.718 | 23.440 | 1.00 | 30.66 | S |
| ATOM | 3122 | OH2 | TIP | S | 37 | 7.328 | 19.410 | 17.790 | 1.00 | 26.00 | S |
| ATOM | 3123 | OH2 | TIP | S | 38 | 23.873 | 3.117 | 16.236 | 1.00 | 16.26 | S |
| ATOM | 3124 | OH2 | TIP | S | 39 | 3.906 | -1.723 | 13.829 | 1.00 | 28.49 | S |
| ATOM | 3125 | OH2 | TIP | S | 41 | 6.735 | 19.664 | 9.237 | 1.00 | 29.06 | S |
| ATOM | 3126 | OH2 | TIP | S | 42 | 8.770 | -18.944 | 24.824 | 1.00 | 35.28 | S |
| ATOM | 3127 | OH2 | TIP | S | 43 | 14.376 | -0.619 | 1.416 | 1.00 | 13.83 | S |
| ATOM | 3128 | OH2 | TIP | S | 45 | 0.694 | -24.085 | 27.493 | 1.00 | 39.29 | S |
| ATOM | 3129 | OH2 | TIP | S | 46 | 18.485 | 4.145 | 5.960 | 1.00 | 21.66 | S |
| ATOM | 3130 | OH2 | TIP | S | 48 | 18.261 | -5.318 | 22.695 | 1.00 | 38.32 | S |
| ATOM | 3131 | OH2 | TIP | S | 51 | 29.857 | 8.075 | 5.771 | 1.00 | 32.01 | S |
| ATOM | 3132 | OH2 | TIP | S | 52 | 27.488 | -21.123 | 21.428 | 1.00 | 35.44 | S |
| ATOM | 3133 | OH2 | TIP | S | 58 | 23.096 | -6.389 | 21.308 | 1.00 | 26.51 | S |
| ATOM | 3134 | OH2 | TIP | S | 59 | 6.228 | 8.843 | -8.817 | 1.00 | 49.10 | S |
| ATOM | 3135 | OH2 | TIP | S | 62 | 3.848 | -5.739 | 21.947 | 1.00 | 31.03 | S |
| ATOM | 3136 | OH2 | TIP | S | 67 | -1.706 | 1.527 | 27.351 | 1.00 | 39.43 | S |
| ATOM | 3137 | OH2 | TIP | S | 68 | 19.766 | -11.663 | 29.402 | 1.00 | 27.43 | S |
| ATOM | 3138 | OH2 | TIP | S | 69 | 11.429 | -10.704 | -0.537 | 1.00 | 29.52 | S |
| ATOM | 3139 | OH2 | TIP | S | 150 | 24.650 | -4.072 | 8.649 | 1.00 | 31.41 | S |
| ATOM | 3140 | OH2 | TIP | S | 151 | 26.364 | 5.469 | 13.370 | 1.00 | 38.22 | S |
| ATOM | 3141 | OH2 | TIP | S | 152 | 28.818 | 12.790 | 5.811 | 1.00 | 35.91 | S |
| ATOM | 3142 | OH2 | TIP | S | 153 | 25.347 | 16.316 | 4.335 | 1.00 | 27.60 | S |
| ATOM | 3143 | OH2 | TIP | S | 157 | 6.000 | 14.176 | 15.348 | 1.00 | 36.55 | S |
| ATOM | 3144 | OH2 | TIP | S | 158 | -1.644 | 29.071 | -1.333 | 1.00 | 25.18 | S |
| ATOM | 3145 | OH2 | TIP | S | 159 | 22.981 | 3.647 | 1.752 | 1.00 | 29.93 | S |
| ATOM | 3146 | OH2 | TIP | S | 162 | 13.177 | 1.671 | 23.093 | 1.00 | 33.47 | S |
| ATOM | 3147 | OH2 | TIP | S | 163 | 26.736 | -15.188 | 22.365 | 1.00 | 5.93 | S |
| ATOM | 3148 | OH2 | TIP | S | 164 | 21.705 | -28.169 | 5.543 | 1.00 | 25.07 | S |
| ATOM | 3149 | OH2 | TIP | S | 166 | 6.048 | -23.357 | 22.534 | 1.00 | 21.49 | S |
| ATOM | 3150 | OH2 | TIP | S | 168 | -4.305 | -11.015 | 7.385 | 1.00 | 24.20 | S |
| END | | | | | | | | | | | |

Figure 2 (suite 43)

```
REMARK coordinates from restrained individual B-factor refinement
REMARK refinement resolution: 500.0 - 2.3 A
REMARK starting r= 0.2260 free_r= 0.2767
REMARK final    r= 0.2254 free_r= 0.2759
REMARK B rmsd for bonded mainchain atoms=  1.319  target= 1.5
REMARK B rmsd for bonded sidechain atoms=  1.818  target= 2.0
REMARK B rmsd for angle mainchain atoms=  2.152  target= 2.0
REMARK B rmsd for angle sidechain atoms=  2.582  target= 2.5
REMARK rweight=  0.1000 (with wa= 3.90087)
REMARK target= mlf  steps= 30
REMARK sg= C2 a= 81.072 b= 117.022 c= 51.618 alpha= 90 beta= 122.42 gamma= 90
REMARK parameter file 1  : CNS_TOPPAR:protein_rep.param
REMARK parameter file 2  : CNS_TOPPAR:water.param
REMARK parameter file 3  : CNS_TOPPAR:ion.param
REMARK parameter file 4  : /RX/martin/CNS/nap.par
REMARK molecular structure file: generate_easy.mtf
REMARK input coordinates: refine.pdb
REMARK reflection file= 49c3mnlf.cv
REMARK ncs= none
REMARK B-correction resolution: 6.0 - 2.3
REMARK initial B-factor correction applied to fobs :
REMARK   B11=   2.246 B22=   6.031 B33=  -8.276
REMARK   B12=   0.000 B13=  -2.565 B23=   0.000
REMARK B-factor correction applied to coordinate array B:     0.690
REMARK bulk solvent: density level= 0.347632 e/A^3, B-factor= 46.8324 A^2
REMARK reflections with |Fobs|/sigma_F < 0.0 rejected
REMARK reflections with |Fobs| > 10000 * rms(Fobs) rejected
REMARK theoretical total number of refl. in resol. range:     18057 ( 100.0 % )
REMARK number of unobserved reflections (no entry or |F|=0):    668 (   3.7 % )
REMARK number of reflections rejected:                            0 (   0.0 % )
REMARK total number of reflections used:                      17389 (  96.3 % )
REMARK number of reflections in working set:                  15835 (  87.7 % )
REMARK number of reflections in test set:                      1554 (   8.6 % )
CRYST1   81.072  117.022   51.618  90.00 122.42  90.00 C 2
REMARK FILENAME="bindividual.pdb"
REMARK DATE:07-Feb-2002  10:31:05       created by user: martin
REMARK VERSION:1.0
ATOM      1  CB  LYS A  10      27.491  -5.774  13.800  1.00 38.75
ATOM      2  CG  LYS A  10      27.252  -4.528  14.627  1.00 39.33
ATOM      3  CD  LYS A  10      26.122  -3.677  14.085  1.00 40.29
ATOM      4  CE  LYS A  10      25.907  -2.461  14.965  1.00 38.66
ATOM      5  NZ  LYS A  10      25.611  -2.877  16.358  1.00 37.64
ATOM      6  C   LYS A  10      28.643  -4.339  12.092  1.00 39.08
ATOM      7  O   LYS A  10      29.664  -4.210  12.770  1.00 41.53
ATOM      8  N   LYS A  10      28.247  -6.771  11.666  1.00 36.06
ATOM      9  CA  LYS A  10      27.707  -5.530  12.307  1.00 39.40
ATOM     10  N   PRO A  11      28.296  -3.447  11.143  1.00 38.76
ATOM     11  CD  PRO A  11      27.049  -3.485  10.356  1.00 37.31
ATOM     12  CA  PRO A  11      29.082  -2.252  10.802  1.00 38.06
ATOM     13  CB  PRO A  11      28.167  -1.499   9.835  1.00 37.56
ATOM     14  CG  PRO A  11      27.393  -2.604   9.176  1.00 37.26
ATOM     15  C   PRO A  11      29.474  -1.392  12.000  1.00 38.16
ATOM     16  O   PRO A  11      28.684  -1.205  12.930  1.00 38.88
ATOM     17  N   PRO A  12      30.708  -0.857  11.992  1.00 37.68
ATOM     18  CD  PRO A  12      31.738  -0.988  10.945  1.00 35.87
ATOM     19  CA  PRO A  12      31.180  -0.012  13.092  1.00 36.06
ATOM     20  CB  PRO A  12      32.665   0.151  12.782  1.00 36.46
ATOM     21  CG  PRO A  12      32.694   0.130  11.293  1.00 36.69
ATOM     22  C   PRO A  12      30.430   1.327  13.123  1.00 36.25
ATOM     23  O   PRO A  12      30.086   1.882  12.078  1.00 34.13
ATOM     24  N   PHE A  13      30.181   1.837  14.325  1.00 35.07
ATOM     25  CA  PHE A  13      29.460   3.092  14.495  1.00 34.62
ATOM     26  CB  PHE A  13      29.278   3.394  15.982  1.00 33.43
ATOM     27  CG  PHE A  13      28.442   4.612  16.260  1.00 32.71
ATOM     28  CD1 PHE A  13      27.053   4.572  16.122  1.00 31.91
ATOM     29  CD2 PHE A  13      29.042   5.809  16.640  1.00 32.73
ATOM     30  CE1 PHE A  13      26.272   5.712  16.361  1.00 31.52
ATOM     31  CE2 PHE A  13      28.275   6.950  16.879  1.00 32.00
ATOM     32  CZ  PHE A  13      26.887   6.903  16.738  1.00 32.01
ATOM     33  C   PHE A  13      30.150   4.274  13.830  1.00 35.40
ATOM     34  O   PHE A  13      31.379   4.378  13.817  1.00 36.09
ATOM     35  N   VAL A  14      29.336   5.160  13.267  1.00 35.11
ATOM     36  CA  VAL A  14      29.816   6.378  12.621  1.00 32.10
```

Figure 3

| | | | | | | | | | | |
|---|---|---|---|---|---|---|---|---|---|---|
| ATOM | 37 | CB | VAL | A | 14 | 29.616 | 6.323 | 11.093 | 1.00 | 32.59 |
| ATOM | 38 | CG1 | VAL | A | 14 | 29.958 | 7.674 | 10.470 | 1.00 | 31.43 |
| ATOM | 39 | CG2 | VAL | A | 14 | 30.497 | 5.235 | 10.504 | 1.00 | 29.66 |
| ATOM | 40 | C | VAL | A | 14 | 28.989 | 7.526 | 13.204 | 1.00 | 30.96 |
| ATOM | 41 | O | VAL | A | 14 | 27.767 | 7.560 | 13.048 | 1.00 | 31.06 |
| ATOM | 42 | N | SER | A | 15 | 29.648 | 8.451 | 13.898 | 1.00 | 28.13 |
| ATOM | 43 | CA | SER | A | 15 | 28.945 | 9.578 | 14.497 | 1.00 | 24.93 |
| ATOM | 44 | CB | SER | A | 15 | 29.868 | 10.328 | 15.451 | 1.00 | 24.08 |
| ATOM | 45 | OG | SER | A | 15 | 29.111 | 11.142 | 16.334 | 1.00 | 25.61 |
| ATOM | 46 | C | SER | A | 15 | 28.469 | 10.493 | 13.381 | 1.00 | 24.41 |
| ATOM | 47 | O | SER | A | 15 | 29.277 | 11.023 | 12.605 | 1.00 | 24.81 |
| ATOM | 48 | N | ARG | A | 16 | 27.155 | 10.673 | 13.291 | 1.00 | 22.84 |
| ATOM | 49 | CA | ARG | A | 16 | 26.575 | 11.498 | 12.231 | 1.00 | 21.32 |
| ATOM | 50 | CB | ARG | A | 16 | 25.425 | 10.742 | 11.548 | 1.00 | 20.17 |
| ATOM | 51 | CG | ARG | A | 16 | 25.807 | 9.417 | 10.912 | 1.00 | 19.52 |
| ATOM | 52 | CD | ARG | A | 16 | 26.481 | 9.592 | 9.579 | 1.00 | 18.61 |
| ATOM | 53 | NE | ARG | A | 16 | 26.762 | 8.306 | 8.943 | 1.00 | 18.67 |
| ATOM | 54 | CZ | ARG | A | 16 | 27.613 | 8.144 | 7.938 | 1.00 | 19.00 |
| ATOM | 55 | NH1 | ARG | A | 16 | 28.268 | 9.194 | 7.462 | 1.00 | 21.34 |
| ATOM | 56 | NH2 | ARG | A | 16 | 27.811 | 6.942 | 7.405 | 1.00 | 16.58 |
| ATOM | 57 | C | ARG | A | 16 | 26.051 | 12.847 | 12.712 | 1.00 | 19.93 |
| ATOM | 58 | O | ARG | A | 16 | 25.687 | 13.012 | 13.879 | 1.00 | 19.91 |
| ATOM | 59 | N | SER | A | 17 | 26.035 | 13.809 | 11.795 | 1.00 | 18.91 |
| ATOM | 60 | CA | SER | A | 17 | 25.513 | 15.143 | 12.069 | 1.00 | 19.92 |
| ATOM | 61 | CB | SER | A | 17 | 26.087 | 16.153 | 11.062 | 1.00 | 20.83 |
| ATOM | 62 | OG | SER | A | 17 | 25.437 | 17.418 | 11.145 | 1.00 | 22.89 |
| ATOM | 63 | C | SER | A | 17 | 24.000 | 14.994 | 11.892 | 1.00 | 19.85 |
| ATOM | 64 | O | SER | A | 17 | 23.510 | 14.763 | 10.787 | 1.00 | 18.77 |
| ATOM | 65 | N | VAL | A | 18 | 23.265 | 15.111 | 12.986 | 1.00 | 22.07 |
| ATOM | 66 | CA | VAL | A | 18 | 21.822 | 14.923 | 12.938 | 1.00 | 22.73 |
| ATOM | 67 | CB | VAL | A | 18 | 21.367 | 13.863 | 13.974 | 1.00 | 23.78 |
| ATOM | 68 | CG1 | VAL | A | 18 | 19.861 | 13.689 | 13.906 | 1.00 | 24.46 |
| ATOM | 69 | CG2 | VAL | A | 18 | 22.081 | 12.546 | 13.734 | 1.00 | 23.72 |
| ATOM | 70 | C | VAL | A | 18 | 20.982 | 16.159 | 13.202 | 1.00 | 23.10 |
| ATOM | 71 | O | VAL | A | 18 | 21.213 | 16.898 | 14.164 | 1.00 | 24.45 |
| ATOM | 72 | N | LEU | A | 19 | 19.983 | 16.358 | 12.352 | 1.00 | 21.24 |
| ATOM | 73 | CA | LEU | A | 19 | 19.054 | 17.470 | 12.516 | 1.00 | 19.97 |
| ATOM | 74 | CB | LEU | A | 19 | 19.077 | 18.410 | 11.303 | 1.00 | 16.77 |
| ATOM | 75 | CG | LEU | A | 19 | 17.937 | 19.438 | 11.349 | 1.00 | 16.81 |
| ATOM | 76 | CD1 | LEU | A | 19 | 17.888 | 20.099 | 12.707 | 1.00 | 11.04 |
| ATOM | 77 | CD2 | LEU | A | 19 | 18.117 | 20.465 | 10.253 | 1.00 | 15.55 |
| ATOM | 78 | C | LEU | A | 19 | 17.644 | 16.918 | 12.679 | 1.00 | 20.20 |
| ATOM | 79 | O | LEU | A | 19 | 17.110 | 16.275 | 11.773 | 1.00 | 18.61 |
| ATOM | 80 | N | VAL | A | 20 | 17.043 | 17.170 | 13.833 | 1.00 | 20.27 |
| ATOM | 81 | CA | VAL | A | 20 | 15.688 | 16.703 | 14.084 | 1.00 | 22.70 |
| ATOM | 82 | CB | VAL | A | 20 | 15.592 | 15.890 | 15.392 | 1.00 | 21.68 |
| ATOM | 83 | CG1 | VAL | A | 20 | 14.163 | 15.413 | 15.581 | 1.00 | 22.14 |
| ATOM | 84 | CG2 | VAL | A | 20 | 16.577 | 14.721 | 15.368 | 1.00 | 20.16 |
| ATOM | 85 | C | VAL | A | 20 | 14.714 | 17.877 | 14.204 | 1.00 | 24.10 |
| ATOM | 86 | O | VAL | A | 20 | 14.643 | 18.521 | 15.255 | 1.00 | 25.55 |
| ATOM | 87 | N | THR | A | 21 | 13.961 | 18.154 | 13.142 | 1.00 | 24.57 |
| ATOM | 88 | CA | THR | A | 21 | 12.989 | 19.246 | 13.184 | 1.00 | 24.47 |
| ATOM | 89 | CB | THR | A | 21 | 12.351 | 19.488 | 11.816 | 1.00 | 24.25 |
| ATOM | 90 | OG1 | THR | A | 21 | 11.480 | 18.393 | 11.503 | 1.00 | 21.99 |
| ATOM | 91 | CG2 | THR | A | 21 | 13.420 | 19.624 | 10.742 | 1.00 | 19.73 |
| ATOM | 92 | C | THR | A | 21 | 11.878 | 18.865 | 14.156 | 1.00 | 25.71 |
| ATOM | 93 | O | THR | A | 21 | 11.227 | 17.847 | 13.979 | 1.00 | 28.18 |
| ATOM | 94 | N | GLY | A | 22 | 11.664 | 19.684 | 15.176 | 1.00 | 26.63 |
| ATOM | 95 | CA | GLY | A | 22 | 10.633 | 19.389 | 16.150 | 1.00 | 27.86 |
| ATOM | 96 | C | GLY | A | 22 | 11.155 | 18.503 | 17.267 | 1.00 | 30.21 |
| ATOM | 97 | O | GLY | A | 22 | 10.384 | 17.799 | 17.927 | 1.00 | 28.53 |
| ATOM | 98 | N | GLY | A | 23 | 12.468 | 18.552 | 17.488 | 1.00 | 31.04 |
| ATOM | 99 | CA | GLY | A | 23 | 13.085 | 17.732 | 18.514 | 1.00 | 33.79 |
| ATOM | 100 | C | GLY | A | 23 | 13.195 | 18.319 | 19.910 | 1.00 | 36.05 |
| ATOM | 101 | O | GLY | A | 23 | 14.020 | 17.866 | 20.712 | 1.00 | 36.61 |
| ATOM | 102 | N | ASN | A | 24 | 12.366 | 19.311 | 20.219 | 1.00 | 37.19 |
| ATOM | 103 | CA | ASN | A | 24 | 12.399 | 19.939 | 21.537 | 1.00 | 38.34 |
| ATOM | 104 | CB | ASN | A | 24 | 12.282 | 21.458 | 21.398 | 1.00 | 40.88 |
| ATOM | 105 | CG | ASN | A | 24 | 11.050 | 21.880 | 20.602 | 1.00 | 43.56 |
| ATOM | 106 | OD1 | ASN | A | 24 | 10.991 | 21.714 | 19.378 | 1.00 | 43.65 |
| ATOM | 107 | ND2 | ASN | A | 24 | 10.057 | 22.420 | 21.300 | 1.00 | 44.02 |
| ATOM | 108 | C | ASN | A | 24 | 11.292 | 19.434 | 22.459 | 1.00 | 38.18 |
| ATOM | 109 | O | ASN | A | 24 | 11.358 | 19.615 | 23.673 | 1.00 | 37.25 |

Figure 3 (suite 1)

| | | | | | | | | | | |
|---|---|---|---|---|---|---|---|---|---|---|
| ATOM | 110 | N | ARG | A | 25 | 10.279 | 18.795 | 21.886 | 1.00 | 38.30 |
| ATOM | 111 | CA | ARG | A | 25 | 9.167 | 18.293 | 22.684 | 1.00 | 38.42 |
| ATOM | 112 | CB | ARG | A | 25 | 8.045 | 19.342 | 22.736 | 1.00 | 38.98 |
| ATOM | 113 | CG | ARG | A | 25 | 8.402 | 20.606 | 23.509 | 1.00 | 39.65 |
| ATOM | 114 | CD | ARG | A | 25 | 7.254 | 21.606 | 23.514 | 0.00 | 39.68 |
| ATOM | 115 | NE | ARG | A | 25 | 7.576 | 22.814 | 24.271 | 0.00 | 39.89 |
| ATOM | 116 | CZ | ARG | A | 25 | 7.793 | 22.843 | 25.582 | 0.00 | 39.96 |
| ATOM | 117 | NH1 | ARG | A | 25 | 7.724 | 21.728 | 26.296 | 0.00 | 40.01 |
| ATOM | 118 | NH2 | ARG | A | 25 | 8.082 | 23.990 | 26.183 | 0.00 | 40.01 |
| ATOM | 119 | C | ARG | A | 25 | 8.596 | 16.968 | 22.181 | 1.00 | 38.13 |
| ATOM | 120 | O | ARG | A | 25 | 8.941 | 16.496 | 21.099 | 1.00 | 39.18 |
| ATOM | 121 | N | GLY | A | 26 | 7.723 | 16.379 | 22.991 | 1.00 | 37.30 |
| ATOM | 122 | CA | GLY | A | 26 | 7.083 | 15.125 | 22.639 | 1.00 | 36.42 |
| ATOM | 123 | C | GLY | A | 26 | 7.975 | 14.031 | 22.084 | 1.00 | 36.94 |
| ATOM | 124 | O | GLY | A | 26 | 8.972 | 13.644 | 22.704 | 1.00 | 36.72 |
| ATOM | 125 | N | ILE | A | 27 | 7.599 | 13.534 | 20.907 | 1.00 | 35.86 |
| ATOM | 126 | CA | ILE | A | 27 | 8.323 | 12.466 | 20.235 | 1.00 | 34.14 |
| ATOM | 127 | CB | ILE | A | 27 | 7.456 | 11.818 | 19.132 | 1.00 | 34.72 |
| ATOM | 128 | CG2 | ILE | A | 27 | 8.264 | 10.773 | 18.390 | 1.00 | 36.29 |
| ATOM | 129 | CG1 | ILE | A | 27 | 6.214 | 11.169 | 19.756 | 1.00 | 34.62 |
| ATOM | 130 | CD | ILE | A | 27 | 5.313 | 10.470 | 18.755 | 1.00 | 36.03 |
| ATOM | 131 | C | ILE | A | 27 | 9.628 | 12.955 | 19.622 | 1.00 | 33.13 |
| ATOM | 132 | O | ILE | A | 27 | 10.625 | 12.229 | 19.613 | 1.00 | 31.24 |
| ATOM | 133 | N | GLY | A | 28 | 9.618 | 14.181 | 19.103 | 1.00 | 31.46 |
| ATOM | 134 | CA | GLY | A | 28 | 10.825 | 14.736 | 18.515 | 1.00 | 30.16 |
| ATOM | 135 | C | GLY | A | 28 | 11.928 | 14.766 | 19.559 | 1.00 | 28.97 |
| ATOM | 136 | O | GLY | A | 28 | 13.092 | 14.478 | 19.272 | 1.00 | 28.45 |
| ATOM | 137 | N | LEU | A | 29 | 11.556 | 15.104 | 20.787 | 1.00 | 27.56 |
| ATOM | 138 | CA | LEU | A | 29 | 12.517 | 15.151 | 21.879 | 1.00 | 26.57 |
| ATOM | 139 | CB | LEU | A | 29 | 11.844 | 15.681 | 23.151 | 1.00 | 24.78 |
| ATOM | 140 | CG | LEU | A | 29 | 12.798 | 15.812 | 24.342 | 1.00 | 26.99 |
| ATOM | 141 | CD1 | LEU | A | 29 | 13.853 | 16.854 | 24.019 | 1.00 | 25.55 |
| ATOM | 142 | CD2 | LEU | A | 29 | 12.031 | 16.195 | 25.601 | 1.00 | 26.91 |
| ATOM | 143 | C | LEU | A | 29 | 13.107 | 13.749 | 22.135 | 1.00 | 23.74 |
| ATOM | 144 | O | LEU | A | 29 | 14.325 | 13.587 | 22.207 | 1.00 | 22.55 |
| ATOM | 145 | N | ALA | A | 30 | 12.234 | 12.751 | 22.272 | 1.00 | 22.69 |
| ATOM | 146 | CA | ALA | A | 30 | 12.645 | 11.357 | 22.505 | 1.00 | 22.45 |
| ATOM | 147 | CB | ALA | A | 30 | 11.414 | 10.440 | 22.588 | 1.00 | 21.26 |
| ATOM | 148 | C | ALA | A | 30 | 13.561 | 10.872 | 21.399 | 1.00 | 19.07 |
| ATOM | 149 | O | ALA | A | 30 | 14.481 | 10.111 | 21.645 | 1.00 | 20.90 |
| ATOM | 150 | N | ILE | A | 31 | 13.310 | 11.315 | 20.179 | 1.00 | 18.40 |
| ATOM | 151 | CA | ILE | A | 31 | 14.139 | 10.912 | 19.058 | 1.00 | 18.29 |
| ATOM | 152 | CB | ILE | A | 31 | 13.507 | 11.320 | 17.716 | 1.00 | 18.68 |
| ATOM | 153 | CG2 | ILE | A | 31 | 14.467 | 11.033 | 16.575 | 1.00 | 16.30 |
| ATOM | 154 | CG1 | ILE | A | 31 | 12.187 | 10.575 | 17.512 | 1.00 | 18.56 |
| ATOM | 155 | CD | ILE | A | 31 | 11.537 | 10.844 | 16.166 | 1.00 | 22.89 |
| ATOM | 156 | C | ILE | A | 31 | 15.521 | 11.545 | 19.140 | 1.00 | 17.70 |
| ATOM | 157 | O | ILE | A | 31 | 16.532 | 10.884 | 18.893 | 1.00 | 19.71 |
| ATOM | 158 | N | ALA | A | 32 | 15.556 | 12.831 | 19.470 | 1.00 | 17.61 |
| ATOM | 159 | CA | ALA | A | 32 | 16.803 | 13.580 | 19.561 | 1.00 | 18.56 |
| ATOM | 160 | CB | ALA | A | 32 | 16.498 | 15.072 | 19.761 | 1.00 | 14.94 |
| ATOM | 161 | C | ALA | A | 32 | 17.695 | 13.061 | 20.694 | 1.00 | 19.46 |
| ATOM | 162 | O | ALA | A | 32 | 18.897 | 12.867 | 20.507 | 1.00 | 20.46 |
| ATOM | 163 | N | GLN | A | 33 | 17.109 | 12.843 | 21.864 | 1.00 | 20.18 |
| ATOM | 164 | CA | GLN | A | 33 | 17.878 | 12.336 | 22.992 | 1.00 | 22.59 |
| ATOM | 165 | CB | GLN | A | 33 | 17.008 | 12.228 | 24.240 | 1.00 | 22.56 |
| ATOM | 166 | CG | GLN | A | 33 | 16.386 | 13.531 | 24.657 | 1.00 | 30.15 |
| ATOM | 167 | CD | GLN | A | 33 | 16.547 | 13.807 | 26.127 | 1.00 | 34.83 |
| ATOM | 168 | OE1 | GLN | A | 33 | 17.659 | 14.051 | 26.608 | 1.00 | 39.65 |
| ATOM | 169 | NE2 | GLN | A | 33 | 15.439 | 13.766 | 26.861 | 1.00 | 36.40 |
| ATOM | 170 | C | GLN | A | 33 | 18.452 | 10.967 | 22.661 | 1.00 | 21.28 |
| ATOM | 171 | O | GLN | A | 33 | 19.631 | 10.707 | 22.911 | 1.00 | 21.88 |
| ATOM | 172 | N | ARG | A | 34 | 17.612 | 10.105 | 22.084 | 1.00 | 20.18 |
| ATOM | 173 | CA | ARG | A | 34 | 18.019 | 8.760 | 21.721 | 1.00 | 17.67 |
| ATOM | 174 | CB | ARG | A | 34 | 16.841 | 7.976 | 21.115 | 1.00 | 17.31 |
| ATOM | 175 | CG | ARG | A | 34 | 17.236 | 6.609 | 20.545 | 1.00 | 14.05 |
| ATOM | 176 | CD | ARG | A | 34 | 17.819 | 5.694 | 21.642 | 1.00 | 16.60 |
| ATOM | 177 | NE | ARG | A | 34 | 18.433 | 4.512 | 21.047 | 1.00 | 13.62 |
| ATOM | 178 | CZ | ARG | A | 34 | 17.765 | 3.426 | 20.671 | 1.00 | 14.14 |
| ATOM | 179 | NH1 | ARG | A | 34 | 16.454 | 3.353 | 20.847 | 1.00 | 14.30 |
| ATOM | 180 | NH2 | ARG | A | 34 | 18.403 | 2.434 | 20.067 | 1.00 | 14.48 |
| ATOM | 181 | C | ARG | A | 34 | 19.157 | 8.812 | 20.731 | 1.00 | 18.37 |
| ATOM | 182 | O | ARG | A | 34 | 20.117 | 8.052 | 20.844 | 1.00 | 18.34 |

Figure 3 (suite 2)

| | | | | | | | | | | |
|---|---|---|---|---|---|---|---|---|---|---|
| ATOM | 183 | N | LEU | A | 35 | 19.065 | 9.711 | 19.759 | 1.00 | 17.24 |
| ATOM | 184 | CA | LEU | A | 35 | 20.126 | 9.799 | 18.770 | 1.00 | 18.65 |
| ATOM | 185 | CB | LEU | A | 35 | 19.701 | 10.665 | 17.587 | 1.00 | 14.91 |
| ATOM | 186 | CG | LEU | A | 35 | 18.721 | 9.993 | 16.617 | 1.00 | 17.51 |
| ATOM | 187 | CD1 | LEU | A | 35 | 18.472 | 10.886 | 15.394 | 1.00 | 16.06 |
| ATOM | 188 | CD2 | LEU | A | 35 | 19.316 | 8.648 | 16.167 | 1.00 | 15.89 |
| ATOM | 189 | C | LEU | A | 35 | 21.426 | 10.329 | 19.360 | 1.00 | 21.05 |
| ATOM | 190 | O | LEU | A | 35 | 22.504 | 10.085 | 18.816 | 1.00 | 22.14 |
| ATOM | 191 | N | ALA | A | 36 | 21.322 | 11.060 | 20.465 | 1.00 | 20.67 |
| ATOM | 192 | CA | ALA | A | 36 | 22.504 | 11.607 | 21.109 | 1.00 | 19.90 |
| ATOM | 193 | CB | ALA | A | 36 | 22.114 | 12.734 | 22.076 | 1.00 | 21.21 |
| ATOM | 194 | C | ALA | A | 36 | 23.152 | 10.458 | 21.861 | 1.00 | 19.19 |
| ATOM | 195 | O | ALA | A | 36 | 24.353 | 10.210 | 21.726 | 1.00 | 17.48 |
| ATOM | 196 | N | ALA | A | 37 | 22.340 | 9.751 | 22.645 | 1.00 | 18.83 |
| ATOM | 197 | CA | ALA | A | 37 | 22.825 | 8.615 | 23.413 | 1.00 | 19.53 |
| ATOM | 198 | CB | ALA | A | 37 | 21.684 | 7.967 | 24.184 | 1.00 | 18.88 |
| ATOM | 199 | C | ALA | A | 37 | 23.497 | 7.602 | 22.478 | 1.00 | 20.40 |
| ATOM | 200 | O | ALA | A | 37 | 24.323 | 6.817 | 22.911 | 1.00 | 20.79 |
| ATOM | 201 | N | ASP | A | 38 | 23.156 | 7.634 | 21.193 | 1.00 | 19.28 |
| ATOM | 202 | CA | ASP | A | 38 | 23.781 | 6.738 | 20.229 | 1.00 | 18.92 |
| ATOM | 203 | CB | ASP | A | 38 | 22.970 | 6.647 | 18.934 | 1.00 | 19.11 |
| ATOM | 204 | CG | ASP | A | 38 | 21.716 | 5.800 | 19.068 | 1.00 | 19.08 |
| ATOM | 205 | OD1 | ASP | A | 38 | 21.400 | 5.324 | 20.181 | 1.00 | 20.57 |
| ATOM | 206 | OD2 | ASP | A | 38 | 21.039 | 5.619 | 18.034 | 1.00 | 22.25 |
| ATOM | 207 | C | ASP | A | 38 | 25.160 | 7.283 | 19.880 | 1.00 | 19.73 |
| ATOM | 208 | O | ASP | A | 38 | 25.952 | 6.604 | 19.233 | 1.00 | 16.40 |
| ATOM | 209 | N | GLY | A | 39 | 25.435 | 8.526 | 20.277 | 1.00 | 19.72 |
| ATOM | 210 | CA | GLY | A | 39 | 26.732 | 9.116 | 19.985 | 1.00 | 19.60 |
| ATOM | 211 | C | GLY | A | 39 | 26.761 | 9.921 | 18.703 | 1.00 | 18.78 |
| ATOM | 212 | O | GLY | A | 39 | 27.824 | 10.190 | 18.138 | 1.00 | 17.21 |
| ATOM | 213 | N | HIS | A | 40 | 25.580 | 10.295 | 18.228 | 1.00 | 19.29 |
| ATOM | 214 | CA | HIS | A | 40 | 25.466 | 11.116 | 17.025 | 1.00 | 17.55 |
| ATOM | 215 | CB | HIS | A | 40 | 24.123 | 10.881 | 16.349 | 1.00 | 16.11 |
| ATOM | 216 | CG | HIS | A | 40 | 24.036 | 9.620 | 15.552 | 1.00 | 14.51 |
| ATOM | 217 | CD2 | HIS | A | 40 | 23.160 | 8.587 | 15.620 | 1.00 | 10.17 |
| ATOM | 218 | ND1 | HIS | A | 40 | 24.835 | 9.374 | 14.458 | 1.00 | 13.42 |
| ATOM | 219 | CE1 | HIS | A | 40 | 24.449 | 8.247 | 13.881 | 1.00 | 13.66 |
| ATOM | 220 | NE2 | HIS | A | 40 | 23.433 | 7.754 | 14.566 | 1.00 | 9.29 |
| ATOM | 221 | C | HIS | A | 40 | 25.516 | 12.590 | 17.476 | 1.00 | 19.50 |
| ATOM | 222 | O | HIS | A | 40 | 25.221 | 12.895 | 18.636 | 1.00 | 20.32 |
| ATOM | 223 | N | LYS | A | 41 | 25.882 | 13.493 | 16.565 | 1.00 | 18.33 |
| ATOM | 224 | CA | LYS | A | 41 | 25.927 | 14.930 | 16.876 | 1.00 | 19.66 |
| ATOM | 225 | CB | LYS | A | 41 | 26.951 | 15.640 | 15.975 | 1.00 | 21.76 |
| ATOM | 226 | CG | LYS | A | 41 | 28.400 | 15.218 | 16.252 | 1.00 | 20.86 |
| ATOM | 227 | CD | LYS | A | 41 | 29.359 | 15.686 | 15.172 | 1.00 | 22.55 |
| ATOM | 228 | CE | LYS | A | 41 | 29.099 | 14.992 | 13.854 | 1.00 | 21.97 |
| ATOM | 229 | NZ | LYS | A | 41 | 30.073 | 15.414 | 12.809 | 1.00 | 22.82 |
| ATOM | 230 | C | LYS | A | 41 | 24.506 | 15.409 | 16.591 | 1.00 | 19.42 |
| ATOM | 231 | O | LYS | A | 41 | 24.096 | 15.515 | 15.438 | 1.00 | 19.86 |
| ATOM | 232 | N | VAL | A | 42 | 23.767 | 15.715 | 17.645 | 1.00 | 19.08 |
| ATOM | 233 | CA | VAL | A | 42 | 22.367 | 16.066 | 17.479 | 1.00 | 20.53 |
| ATOM | 234 | CB | VAL | A | 42 | 21.510 | 15.131 | 18.363 | 1.00 | 18.92 |
| ATOM | 235 | CG1 | VAL | A | 42 | 20.035 | 15.308 | 18.050 | 1.00 | 18.55 |
| ATOM | 236 | CG2 | VAL | A | 42 | 21.945 | 13.688 | 18.148 | 1.00 | 18.97 |
| ATOM | 237 | C | VAL | A | 42 | 21.857 | 17.497 | 17.695 | 1.00 | 22.99 |
| ATOM | 238 | O | VAL | A | 42 | 21.954 | 18.051 | 18.790 | 1.00 | 24.89 |
| ATOM | 239 | N | ALA | A | 43 | 21.252 | 18.054 | 16.647 | 1.00 | 24.46 |
| ATOM | 240 | CA | ALA | A | 43 | 20.667 | 19.390 | 16.696 | 1.00 | 25.69 |
| ATOM | 241 | CB | ALA | A | 43 | 21.292 | 20.279 | 15.609 | 1.00 | 27.37 |
| ATOM | 242 | C | ALA | A | 43 | 19.144 | 19.303 | 16.493 | 1.00 | 26.71 |
| ATOM | 243 | O | ALA | A | 43 | 18.651 | 18.446 | 15.752 | 1.00 | 27.74 |
| ATOM | 244 | N | VAL | A | 44 | 18.401 | 20.179 | 17.162 | 1.00 | 25.92 |
| ATOM | 245 | CA | VAL | A | 44 | 16.953 | 20.207 | 17.019 | 1.00 | 26.10 |
| ATOM | 246 | CB | VAL | A | 44 | 16.246 | 19.891 | 18.345 | 1.00 | 25.47 |
| ATOM | 247 | CG1 | VAL | A | 44 | 16.669 | 18.527 | 18.838 | 1.00 | 28.83 |
| ATOM | 248 | CG2 | VAL | A | 44 | 16.576 | 20.943 | 19.376 | 1.00 | 26.29 |
| ATOM | 249 | C | VAL | A | 44 | 16.503 | 21.592 | 16.561 | 1.00 | 26.67 |
| ATOM | 250 | O | VAL | A | 44 | 17.215 | 22.580 | 16.742 | 1.00 | 25.00 |
| ATOM | 251 | N | THR | A | 45 | 15.328 | 21.662 | 15.948 | 1.00 | 26.93 |
| ATOM | 252 | CA | THR | A | 45 | 14.787 | 22.939 | 15.522 | 1.00 | 27.66 |
| ATOM | 253 | CB | THR | A | 45 | 14.159 | 22.862 | 14.124 | 1.00 | 27.27 |
| ATOM | 254 | OG1 | THR | A | 45 | 13.055 | 21.944 | 14.138 | 1.00 | 26.00 |
| ATOM | 255 | CG2 | THR | A | 45 | 15.196 | 22.419 | 13.101 | 1.00 | 24.99 |

Figure 3 (suite 3)

| | | | | | | | | | | |
|---|---|---|---|---|---|---|---|---|---|---|
| ATOM | 256 | C | THR | A | 45 | 13.703 | 23.266 | 16.530 | 1.00 | 30.45 |
| ATOM | 257 | O | THR | A | 45 | 13.236 | 22.383 | 17.250 | 1.00 | 29.10 |
| ATOM | 258 | N | HIS | A | 46 | 13.323 | 24.535 | 16.610 | 1.00 | 34.47 |
| ATOM | 259 | CA | HIS | A | 46 | 12.268 | 24.956 | 17.529 | 1.00 | 39.46 |
| ATOM | 260 | CB | HIS | A | 46 | 12.791 | 24.984 | 18.974 | 1.00 | 39.93 |
| ATOM | 261 | CG | HIS | A | 46 | 13.876 | 25.989 | 19.213 | 1.00 | 43.09 |
| ATOM | 262 | CD2 | HIS | A | 46 | 15.213 | 25.836 | 19.364 | 1.00 | 43.64 |
| ATOM | 263 | ND1 | HIS | A | 46 | 13.631 | 27.342 | 19.316 | 1.00 | 42.79 |
| ATOM | 264 | CE1 | HIS | A | 46 | 14.771 | 27.978 | 19.520 | 1.00 | 44.74 |
| ATOM | 265 | NE2 | HIS | A | 46 | 15.746 | 27.088 | 19.555 | 1.00 | 44.48 |
| ATOM | 266 | C | HIS | A | 46 | 11.738 | 26.321 | 17.101 | 1.00 | 40.97 |
| ATOM | 267 | O | HIS | A | 46 | 12.289 | 26.950 | 16.195 | 1.00 | 41.45 |
| ATOM | 268 | N | ARG | A | 47 | 10.664 | 26.774 | 17.733 | 1.00 | 44.38 |
| ATOM | 269 | CA | ARG | A | 47 | 10.082 | 28.062 | 17.377 | 1.00 | 48.27 |
| ATOM | 270 | CB | ARG | A | 47 | 8.568 | 27.926 | 17.200 | 1.00 | 49.77 |
| ATOM | 271 | CG | ARG | A | 47 | 8.148 | 27.208 | 15.921 | 1.00 | 51.74 |
| ATOM | 272 | CD | ARG | A | 47 | 6.645 | 26.957 | 15.903 | 1.00 | 55.69 |
| ATOM | 273 | NE | ARG | A | 47 | 6.207 | 26.280 | 14.684 | 1.00 | 58.10 |
| ATOM | 274 | CZ | ARG | A | 47 | 5.010 | 25.719 | 14.523 | 1.00 | 59.64 |
| ATOM | 275 | NH1 | ARG | A | 47 | 4.115 | 25.748 | 15.504 | 1.00 | 60.10 |
| ATOM | 276 | NH2 | ARG | A | 47 | 4.709 | 25.117 | 13.379 | 1.00 | 60.84 |
| ATOM | 277 | C | ARG | A | 47 | 10.383 | 29.159 | 18.391 | 1.00 | 50.61 |
| ATOM | 278 | O | ARG | A | 47 | 9.747 | 30.216 | 18.375 | 1.00 | 52.67 |
| ATOM | 279 | N | GLY | A | 48 | 11.355 | 28.917 | 19.265 | 1.00 | 51.57 |
| ATOM | 280 | CA | GLY | A | 48 | 11.709 | 29.914 | 20.261 | 1.00 | 51.16 |
| ATOM | 281 | C | GLY | A | 48 | 11.814 | 29.355 | 21.666 | 1.00 | 51.66 |
| ATOM | 282 | O | GLY | A | 48 | 12.650 | 29.802 | 22.451 | 1.00 | 51.50 |
| ATOM | 283 | N | SER | A | 49 | 10.965 | 28.379 | 21.982 | 1.00 | 52.00 |
| ATOM | 284 | CA | SER | A | 49 | 10.952 | 27.739 | 23.298 | 1.00 | 52.39 |
| ATOM | 285 | CB | SER | A | 49 | 9.972 | 26.562 | 23.302 | 1.00 | 53.51 |
| ATOM | 286 | OG | SER | A | 49 | 10.385 | 25.547 | 22.396 | 1.00 | 54.88 |
| ATOM | 287 | C | SER | A | 49 | 12.342 | 27.233 | 23.676 | 1.00 | 52.62 |
| ATOM | 288 | O | SER | A | 49 | 12.625 | 26.968 | 24.848 | 1.00 | 51.90 |
| ATOM | 289 | N | GLY | A | 50 | 13.196 | 27.083 | 22.668 | 1.00 | 52.82 |
| ATOM | 290 | CA | GLY | A | 50 | 14.551 | 26.624 | 22.902 | 1.00 | 52.12 |
| ATOM | 291 | C | GLY | A | 50 | 14.729 | 25.119 | 22.873 | 1.00 | 51.35 |
| ATOM | 292 | O | GLY | A | 50 | 13.762 | 24.357 | 22.787 | 1.00 | 50.62 |
| ATOM | 293 | N | ALA | A | 51 | 15.986 | 24.696 | 22.948 | 1.00 | 50.28 |
| ATOM | 294 | CA | ALA | A | 51 | 16.327 | 23.283 | 22.941 | 1.00 | 50.21 |
| ATOM | 295 | CB | ALA | A | 51 | 17.415 | 23.023 | 21.918 | 1.00 | 50.36 |
| ATOM | 296 | C | ALA | A | 51 | 16.805 | 22.879 | 24.335 | 1.00 | 49.33 |
| ATOM | 297 | O | ALA | A | 51 | 17.357 | 23.699 | 25.072 | 1.00 | 50.61 |
| ATOM | 298 | N | PRO | A | 52 | 16.595 | 21.608 | 24.716 | 1.00 | 48.00 |
| ATOM | 299 | CD | PRO | A | 52 | 15.882 | 20.577 | 23.940 | 1.00 | 47.40 |
| ATOM | 300 | CA | PRO | A | 52 | 17.002 | 21.088 | 26.028 | 1.00 | 46.58 |
| ATOM | 301 | CB | PRO | A | 52 | 16.271 | 19.751 | 26.107 | 1.00 | 46.58 |
| ATOM | 302 | CG | PRO | A | 52 | 16.254 | 19.309 | 24.675 | 1.00 | 47.61 |
| ATOM | 303 | C | PRO | A | 52 | 18.516 | 20.937 | 26.213 | 1.00 | 45.71 |
| ATOM | 304 | O | PRO | A | 52 | 19.263 | 20.729 | 25.252 | 1.00 | 45.21 |
| ATOM | 305 | N | LYS | A | 53 | 18.953 | 21.045 | 27.463 | 1.00 | 44.87 |
| ATOM | 306 | CA | LYS | A | 53 | 20.364 | 20.930 | 27.802 | 1.00 | 44.10 |
| ATOM | 307 | CB | LYS | A | 53 | 20.540 | 20.849 | 29.320 | 1.00 | 43.39 |
| ATOM | 308 | CG | LYS | A | 53 | 19.775 | 21.915 | 30.082 | 1.00 | 40.19 |
| ATOM | 309 | CD | LYS | A | 53 | 19.886 | 21.734 | 31.591 | 1.00 | 37.66 |
| ATOM | 310 | CE | LYS | A | 53 | 21.253 | 22.138 | 32.110 | 1.00 | 35.71 |
| ATOM | 311 | NZ | LYS | A | 53 | 21.331 | 22.050 | 33.599 | 1.00 | 31.79 |
| ATOM | 312 | C | LYS | A | 53 | 20.931 | 19.678 | 27.165 | 1.00 | 43.98 |
| ATOM | 313 | O | LYS | A | 53 | 20.355 | 18.596 | 27.288 | 1.00 | 45.36 |
| ATOM | 314 | N | GLY | A | 54 | 22.052 | 19.826 | 26.474 | 1.00 | 42.51 |
| ATOM | 315 | CA | GLY | A | 54 | 22.669 | 18.676 | 25.850 | 1.00 | 41.75 |
| ATOM | 316 | C | GLY | A | 54 | 22.402 | 18.580 | 24.368 | 1.00 | 40.41 |
| ATOM | 317 | O | GLY | A | 54 | 22.815 | 17.622 | 23.717 | 1.00 | 41.41 |
| ATOM | 318 | N | LEU | A | 55 | 21.716 | 19.571 | 23.819 | 1.00 | 39.04 |
| ATOM | 319 | CA | LEU | A | 55 | 21.415 | 19.547 | 22.399 | 1.00 | 37.60 |
| ATOM | 320 | CB | LEU | A | 55 | 20.002 | 19.001 | 22.171 | 1.00 | 37.10 |
| ATOM | 321 | CG | LEU | A | 55 | 19.703 | 17.581 | 22.675 | 1.00 | 38.21 |
| ATOM | 322 | CD1 | LEU | A | 55 | 18.228 | 17.265 | 22.473 | 1.00 | 37.16 |
| ATOM | 323 | CD2 | LEU | A | 55 | 20.567 | 16.571 | 21.931 | 1.00 | 37.95 |
| ATOM | 324 | C | LEU | A | 55 | 21.549 | 20.923 | 21.777 | 1.00 | 36.35 |
| ATOM | 325 | O | LEU | A | 55 | 21.137 | 21.922 | 22.360 | 1.00 | 35.36 |
| ATOM | 326 | N | PHE | A | 56 | 22.133 | 20.968 | 20.586 | 1.00 | 36.93 |
| ATOM | 327 | CA | PHE | A | 56 | 22.315 | 22.224 | 19.880 | 1.00 | 36.06 |
| ATOM | 328 | CB | PHE | A | 56 | 23.428 | 22.093 | 18.842 | 1.00 | 36.53 |

Figure 3 (suite 4)

| | | | | | | | | | | |
|---|---|---|---|---|---|---|---|---|---|---|
| ATOM | 329 | CG | PHE | A | 56 | 23.669 | 23.351 | 18.056 | 1.00 | 35.48 |
| ATOM | 330 | CD1 | PHE | A | 56 | 24.135 | 24.501 | 18.689 | 1.00 | 35.86 |
| ATOM | 331 | CD2 | PHE | A | 56 | 23.430 | 23.387 | 16.684 | 1.00 | 34.37 |
| ATOM | 332 | CE1 | PHE | A | 56 | 24.361 | 25.673 | 17.964 | 1.00 | 36.99 |
| ATOM | 333 | CE2 | PHE | A | 56 | 23.651 | 24.546 | 15.950 | 1.00 | 36.57 |
| ATOM | 334 | CZ | PHE | A | 56 | 24.120 | 25.696 | 16.590 | 1.00 | 36.84 |
| ATOM | 335 | C | PHE | A | 56 | 21.028 | 22.642 | 19.182 | 1.00 | 36.09 |
| ATOM | 336 | O | PHE | A | 56 | 20.699 | 22.134 | 18.108 | 1.00 | 36.06 |
| ATOM | 337 | N | GLY | A | 57 | 20.309 | 23.575 | 19.791 | 1.00 | 35.88 |
| ATOM | 338 | CA | GLY | A | 57 | 19.069 | 24.045 | 19.204 | 1.00 | 36.35 |
| ATOM | 339 | C | GLY | A | 57 | 19.228 | 25.174 | 18.201 | 1.00 | 36.84 |
| ATOM | 340 | O | GLY | A | 57 | 20.153 | 25.976 | 18.282 | 1.00 | 36.78 |
| ATOM | 341 | N | VAL | A | 58 | 18.328 | 25.203 | 17.226 | 1.00 | 36.48 |
| ATOM | 342 | CA | VAL | A | 58 | 18.301 | 26.236 | 16.207 | 1.00 | 36.36 |
| ATOM | 343 | CB | VAL | A | 58 | 18.892 | 25.752 | 14.858 | 1.00 | 35.99 |
| ATOM | 344 | CG1 | VAL | A | 58 | 20.309 | 25.271 | 15.065 | 1.00 | 36.65 |
| ATOM | 345 | CG2 | VAL | A | 58 | 18.031 | 24.662 | 14.250 | 1.00 | 34.12 |
| ATOM | 346 | C | VAL | A | 58 | 16.828 | 26.578 | 16.042 | 1.00 | 36.87 |
| ATOM | 347 | O | VAL | A | 58 | 15.972 | 25.693 | 16.066 | 1.00 | 37.79 |
| ATOM | 348 | N | GLU | A | 59 | 16.533 | 27.862 | 15.902 | 1.00 | 37.55 |
| ATOM | 349 | CA | GLU | A | 59 | 15.159 | 28.321 | 15.767 | 1.00 | 37.92 |
| ATOM | 350 | CB | GLU | A | 59 | 15.047 | 29.714 | 16.378 | 1.00 | 40.85 |
| ATOM | 351 | CG | GLU | A | 59 | 13.677 | 30.354 | 16.341 | 1.00 | 44.82 |
| ATOM | 352 | CD | GLU | A | 59 | 13.710 | 31.774 | 16.891 | 1.00 | 46.55 |
| ATOM | 353 | OE1 | GLU | A | 59 | 14.110 | 31.956 | 18.065 | 1.00 | 48.07 |
| ATOM | 354 | OE2 | GLU | A | 59 | 13.343 | 32.706 | 16.145 | 1.00 | 48.97 |
| ATOM | 355 | C | GLU | A | 59 | 14.776 | 28.346 | 14.298 | 1.00 | 36.27 |
| ATOM | 356 | O | GLU | A | 59 | 15.586 | 28.704 | 13.449 | 1.00 | 35.15 |
| ATOM | 357 | N | VAL | A | 60 | 13.547 | 27.948 | 13.993 | 1.00 | 35.55 |
| ATOM | 358 | CA | VAL | A | 60 | 13.095 | 27.939 | 12.607 | 1.00 | 35.70 |
| ATOM | 359 | CB | VAL | A | 60 | 14.022 | 27.071 | 11.714 | 1.00 | 36.53 |
| ATOM | 360 | CG1 | VAL | A | 60 | 14.289 | 25.727 | 12.380 | 1.00 | 34.74 |
| ATOM | 361 | CG2 | VAL | A | 60 | 13.387 | 26.867 | 10.339 | 1.00 | 34.06 |
| ATOM | 362 | C | VAL | A | 60 | 11.668 | 27.450 | 12.420 | 1.00 | 36.16 |
| ATOM | 363 | O | VAL | A | 60 | 11.203 | 26.552 | 13.120 | 1.00 | 36.56 |
| ATOM | 364 | N | ASP | A | 61 | 10.972 | 28.067 | 11.472 | 1.00 | 36.07 |
| ATOM | 365 | CA | ASP | A | 61 | 9.611 | 27.681 | 11.148 | 1.00 | 35.52 |
| ATOM | 366 | CB | ASP | A | 61 | 8.711 | 28.905 | 11.045 | 1.00 | 36.81 |
| ATOM | 367 | CG | ASP | A | 61 | 7.244 | 28.541 | 11.073 | 1.00 | 38.37 |
| ATOM | 368 | OD1 | ASP | A | 61 | 6.722 | 28.262 | 12.172 | 1.00 | 39.87 |
| ATOM | 369 | OD2 | ASP | A | 61 | 6.620 | 28.516 | 9.996 | 1.00 | 38.64 |
| ATOM | 370 | C | ASP | A | 61 | 9.760 | 27.039 | 9.786 | 1.00 | 34.26 |
| ATOM | 371 | O | ASP | A | 61 | 10.240 | 27.681 | 8.848 | 1.00 | 33.99 |
| ATOM | 372 | N | VAL | A | 62 | 9.366 | 25.776 | 9.667 | 1.00 | 33.78 |
| ATOM | 373 | CA | VAL | A | 62 | 9.519 | 25.083 | 8.394 | 1.00 | 34.24 |
| ATOM | 374 | CB | VAL | A | 62 | 9.311 | 23.564 | 8.557 | 1.00 | 34.13 |
| ATOM | 375 | CG1 | VAL | A | 62 | 10.446 | 22.997 | 9.395 | 1.00 | 32.47 |
| ATOM | 376 | CG2 | VAL | A | 62 | 7.965 | 23.269 | 9.213 | 1.00 | 32.59 |
| ATOM | 377 | C | VAL | A | 62 | 8.650 | 25.612 | 7.258 | 1.00 | 34.81 |
| ATOM | 378 | O | VAL | A | 62 | 8.795 | 25.182 | 6.110 | 1.00 | 36.21 |
| ATOM | 379 | N | THR | A | 63 | 7.759 | 26.553 | 7.560 | 1.00 | 34.08 |
| ATOM | 380 | CA | THR | A | 63 | 6.925 | 27.123 | 6.508 | 1.00 | 33.90 |
| ATOM | 381 | CB | THR | A | 63 | 5.647 | 27.770 | 7.059 | 1.00 | 35.45 |
| ATOM | 382 | OG1 | THR | A | 63 | 5.984 | 28.984 | 7.740 | 1.00 | 37.84 |
| ATOM | 383 | CG2 | THR | A | 63 | 4.932 | 26.816 | 8.020 | 1.00 | 36.29 |
| ATOM | 384 | C | THR | A | 63 | 7.732 | 28.202 | 5.799 | 1.00 | 31.88 |
| ATOM | 385 | O | THR | A | 63 | 7.400 | 28.607 | 4.691 | 1.00 | 32.13 |
| ATOM | 386 | N | ASP | A | 64 | 8.808 | 28.642 | 6.445 | 1.00 | 29.53 |
| ATOM | 387 | CA | ASP | A | 64 | 9.688 | 29.685 | 5.912 | 1.00 | 29.13 |
| ATOM | 388 | CB | ASP | A | 64 | 10.086 | 30.605 | 7.064 | 1.00 | 30.08 |
| ATOM | 389 | CG | ASP | A | 64 | 11.012 | 31.728 | 6.645 | 1.00 | 30.92 |
| ATOM | 390 | OD1 | ASP | A | 64 | 11.661 | 31.658 | 5.575 | 1.00 | 28.41 |
| ATOM | 391 | OD2 | ASP | A | 64 | 11.097 | 32.692 | 7.430 | 1.00 | 32.67 |
| ATOM | 392 | C | ASP | A | 64 | 10.943 | 29.092 | 5.276 | 1.00 | 29.14 |
| ATOM | 393 | O | ASP | A | 64 | 11.814 | 28.600 | 5.987 | 1.00 | 30.75 |
| ATOM | 394 | N | SER | A | 65 | 11.044 | 29.159 | 3.951 | 1.00 | 28.62 |
| ATOM | 395 | CA | SER | A | 65 | 12.195 | 28.619 | 3.229 | 1.00 | 30.71 |
| ATOM | 396 | CB | SER | A | 65 | 11.958 | 28.665 | 1.713 | 1.00 | 31.43 |
| ATOM | 397 | OG | SER | A | 65 | 11.135 | 27.594 | 1.264 | 1.00 | 32.38 |
| ATOM | 398 | C | SER | A | 65 | 13.522 | 29.309 | 3.534 | 1.00 | 32.02 |
| ATOM | 399 | O | SER | A | 65 | 14.583 | 28.753 | 3.249 | 1.00 | 32.99 |
| ATOM | 400 | N | ASP | A | 66 | 13.474 | 30.518 | 4.092 | 1.00 | 33.53 |
| ATOM | 401 | CA | ASP | A | 66 | 14.703 | 31.246 | 4.422 | 1.00 | 34.56 |

Figure 3 (suite 5)

| | | | | | | | | | | |
|---|---|---|---|---|---|---|---|---|---|---|
| ATOM | 402 | CB | ASP | A | 66 | 14.481 | 32.759 | 4.333 | 1.00 | 38.16 |
| ATOM | 403 | CG | ASP | A | 66 | 14.333 | 33.235 | 2.911 | 1.00 | 40.10 |
| ATOM | 404 | OD1 | ASP | A | 66 | 15.122 | 32.771 | 2.058 | 1.00 | 41.74 |
| ATOM | 405 | OD2 | ASP | A | 66 | 13.441 | 34.071 | 2.650 | 1.00 | 43.26 |
| ATOM | 406 | C | ASP | A | 66 | 15.240 | 30.902 | 5.801 | 1.00 | 33.51 |
| ATOM | 407 | O | ASP | A | 66 | 16.449 | 30.859 | 6.002 | 1.00 | 34.62 |
| ATOM | 408 | N | ALA | A | 67 | 14.339 | 30.680 | 6.751 | 1.00 | 32.49 |
| ATOM | 409 | CA | ALA | A | 67 | 14.723 | 30.318 | 8.108 | 1.00 | 33.33 |
| ATOM | 410 | CB | ALA | A | 67 | 13.535 | 30.502 | 9.052 | 1.00 | 34.62 |
| ATOM | 411 | C | ALA | A | 67 | 15.222 | 28.862 | 8.160 | 1.00 | 34.35 |
| ATOM | 412 | O | ALA | A | 67 | 15.821 | 28.429 | 9.155 | 1.00 | 34.81 |
| ATOM | 413 | N | VAL | A | 68 | 14.961 | 28.114 | 7.089 | 1.00 | 32.94 |
| ATOM | 414 | CA | VAL | A | 68 | 15.385 | 26.722 | 6.988 | 1.00 | 32.39 |
| ATOM | 415 | CB | VAL | A | 68 | 14.454 | 25.906 | 6.051 | 1.00 | 30.54 |
| ATOM | 416 | CG1 | VAL | A | 68 | 15.107 | 24.595 | 5.692 | 1.00 | 29.89 |
| ATOM | 417 | CG2 | VAL | A | 68 | 13.109 | 25.658 | 6.728 | 1.00 | 27.50 |
| ATOM | 418 | C | VAL | A | 68 | 16.800 | 26.710 | 6.429 | 1.00 | 33.99 |
| ATOM | 419 | O | VAL | A | 68 | 17.642 | 25.913 | 6.848 | 1.00 | 34.18 |
| ATOM | 420 | N | ASP | A | 69 | 17.053 | 27.602 | 5.476 | 1.00 | 35.27 |
| ATOM | 421 | CA | ASP | A | 69 | 18.372 | 27.721 | 4.867 | 1.00 | 35.56 |
| ATOM | 422 | CB | ASP | A | 69 | 18.345 | 28.757 | 3.751 | 1.00 | 36.64 |
| ATOM | 423 | CG | ASP | A | 69 | 19.726 | 29.079 | 3.230 | 1.00 | 38.88 |
| ATOM | 424 | OD1 | ASP | A | 69 | 20.035 | 30.286 | 3.092 | 1.00 | 41.11 |
| ATOM | 425 | OD2 | ASP | A | 69 | 20.500 | 28.130 | 2.959 | 1.00 | 38.68 |
| ATOM | 426 | C | ASP | A | 69 | 19.365 | 28.167 | 5.931 | 1.00 | 35.09 |
| ATOM | 427 | O | ASP | A | 69 | 20.511 | 27.716 | 5.962 | 1.00 | 36.11 |
| ATOM | 428 | N | ARG | A | 70 | 18.908 | 29.061 | 6.803 | 1.00 | 34.51 |
| ATOM | 429 | CA | ARG | A | 70 | 19.737 | 29.587 | 7.874 | 1.00 | 33.27 |
| ATOM | 430 | CB | ARG | A | 70 | 19.102 | 30.845 | 8.479 | 1.00 | 34.22 |
| ATOM | 431 | CG | ARG | A | 70 | 19.301 | 32.112 | 7.650 | 1.00 | 37.73 |
| ATOM | 432 | CD | ARG | A | 70 | 18.811 | 33.350 | 8.403 | 1.00 | 37.35 |
| ATOM | 433 | NE | ARG | A | 70 | 17.358 | 33.504 | 8.361 | 1.00 | 38.15 |
| ATOM | 434 | CZ | ARG | A | 70 | 16.660 | 33.756 | 7.254 | 1.00 | 38.79 |
| ATOM | 435 | NH1 | ARG | A | 70 | 17.281 | 33.885 | 6.085 | 1.00 | 38.14 |
| ATOM | 436 | NH2 | ARG | A | 70 | 15.340 | 33.885 | 7.317 | 1.00 | 34.84 |
| ATOM | 437 | C | ARG | A | 70 | 19.944 | 28.562 | 8.971 | 1.00 | 32.85 |
| ATOM | 438 | O | ARG | A | 70 | 21.007 | 28.509 | 9.586 | 1.00 | 34.24 |
| ATOM | 439 | N | ALA | A | 71 | 18.916 | 27.760 | 9.232 | 1.00 | 32.17 |
| ATOM | 440 | CA | ALA | A | 71 | 18.990 | 26.742 | 10.268 | 1.00 | 29.60 |
| ATOM | 441 | CB | ALA | A | 71 | 17.646 | 26.070 | 10.417 | 1.00 | 30.00 |
| ATOM | 442 | C | ALA | A | 71 | 20.065 | 25.726 | 9.895 | 1.00 | 30.10 |
| ATOM | 443 | O | ALA | A | 71 | 20.934 | 25.397 | 10.702 | 1.00 | 29.21 |
| ATOM | 444 | N | PHE | A | 72 | 20.017 | 25.257 | 8.654 | 1.00 | 28.98 |
| ATOM | 445 | CA | PHE | A | 72 | 20.986 | 24.289 | 8.178 | 1.00 | 28.81 |
| ATOM | 446 | CB | PHE | A | 72 | 20.636 | 23.839 | 6.760 | 1.00 | 26.26 |
| ATOM | 447 | CG | PHE | A | 72 | 19.645 | 22.705 | 6.714 | 1.00 | 26.35 |
| ATOM | 448 | CD1 | PHE | A | 72 | 18.355 | 22.865 | 7.224 | 1.00 | 23.47 |
| ATOM | 449 | CD2 | PHE | A | 72 | 20.008 | 21.472 | 6.173 | 1.00 | 24.99 |
| ATOM | 450 | CE1 | PHE | A | 72 | 17.437 | 21.820 | 7.196 | 1.00 | 23.12 |
| ATOM | 451 | CE2 | PHE | A | 72 | 19.102 | 20.415 | 6.137 | 1.00 | 27.72 |
| ATOM | 452 | CZ | PHE | A | 72 | 17.806 | 20.590 | 6.653 | 1.00 | 25.27 |
| ATOM | 453 | C | PHE | A | 72 | 22.416 | 24.815 | 8.214 | 1.00 | 29.31 |
| ATOM | 454 | O | PHE | A | 72 | 23.341 | 24.105 | 8.643 | 1.00 | 29.33 |
| ATOM | 455 | N | THR | A | 73 | 22.605 | 26.054 | 7.766 | 1.00 | 29.73 |
| ATOM | 456 | CA | THR | A | 73 | 23.940 | 26.633 | 7.755 | 1.00 | 27.78 |
| ATOM | 457 | CB | THR | A | 73 | 23.955 | 28.017 | 7.085 | 1.00 | 27.54 |
| ATOM | 458 | OG1 | THR | A | 73 | 23.609 | 27.881 | 5.698 | 1.00 | 24.18 |
| ATOM | 459 | CG2 | THR | A | 73 | 25.349 | 28.642 | 7.187 | 1.00 | 28.24 |
| ATOM | 460 | C | THR | A | 73 | 24.510 | 26.734 | 9.155 | 1.00 | 26.66 |
| ATOM | 461 | O | THR | A | 73 | 25.691 | 26.478 | 9.362 | 1.00 | 26.54 |
| ATOM | 462 | N | ALA | A | 74 | 23.677 | 27.087 | 10.125 | 1.00 | 27.61 |
| ATOM | 463 | CA | ALA | A | 74 | 24.168 | 27.200 | 11.494 | 1.00 | 28.96 |
| ATOM | 464 | CB | ALA | A | 74 | 23.108 | 27.812 | 12.392 | 1.00 | 27.76 |
| ATOM | 465 | C | ALA | A | 74 | 24.569 | 25.820 | 12.013 | 1.00 | 31.17 |
| ATOM | 466 | O | ALA | A | 74 | 25.534 | 25.684 | 12.771 | 1.00 | 34.67 |
| ATOM | 467 | N | VAL | A | 75 | 23.822 | 24.798 | 11.603 | 1.00 | 30.71 |
| ATOM | 468 | CA | VAL | A | 75 | 24.114 | 23.436 | 12.011 | 1.00 | 29.97 |
| ATOM | 469 | CB | VAL | A | 75 | 22.934 | 22.475 | 11.669 | 1.00 | 27.67 |
| ATOM | 470 | CG1 | VAL | A | 75 | 23.423 | 21.025 | 11.631 | 1.00 | 26.60 |
| ATOM | 471 | CG2 | VAL | A | 75 | 21.830 | 22.620 | 12.714 | 1.00 | 24.37 |
| ATOM | 472 | C | VAL | A | 75 | 25.386 | 22.963 | 11.309 | 1.00 | 32.73 |
| ATOM | 473 | O | VAL | A | 75 | 26.205 | 22.269 | 11.909 | 1.00 | 33.93 |
| ATOM | 474 | N | GLU | A | 76 | 25.559 | 23.341 | 10.045 | 1.00 | 33.39 |

Figure 3 (suite 6)

| | | | | | | | | | | |
|---|---|---|---|---|---|---|---|---|---|---|
| ATOM | 475 | CA | GLU | A | 76 | 26.745 | 22.927 | 9.314 | 1.00 | 35.84 |
| ATOM | 476 | CB | GLU | A | 76 | 26.664 | 23.334 | 7.845 | 1.00 | 36.69 |
| ATOM | 477 | CG | GLU | A | 76 | 27.873 | 22.856 | 7.054 | 1.00 | 39.77 |
| ATOM | 478 | CD | GLU | A | 76 | 27.764 | 23.107 | 5.564 | 1.00 | 43.36 |
| ATOM | 479 | OE1 | GLU | A | 76 | 26.649 | 22.965 | 5.005 | 1.00 | 44.60 |
| ATOM | 480 | OE2 | GLU | A | 76 | 28.805 | 23.417 | 4.941 | 1.00 | 44.95 |
| ATOM | 481 | C | GLU | A | 76 | 28.033 | 23.489 | 9.907 | 1.00 | 37.61 |
| ATOM | 482 | O | GLU | A | 76 | 29.096 | 22.883 | 9.769 | 1.00 | 38.53 |
| ATOM | 483 | N | GLU | A | 77 | 27.941 | 24.646 | 10.559 | 1.00 | 39.47 |
| ATOM | 484 | CA | GLU | A | 77 | 29.111 | 25.281 | 11.159 | 1.00 | 40.67 |
| ATOM | 485 | CB | GLU | A | 77 | 28.885 | 26.793 | 11.326 | 1.00 | 42.81 |
| ATOM | 486 | CG | GLU | A | 77 | 28.432 | 27.510 | 10.054 | 1.00 | 48.28 |
| ATOM | 487 | CD | GLU | A | 77 | 28.180 | 29.007 | 10.256 | 1.00 | 51.06 |
| ATOM | 488 | OE1 | GLU | A | 77 | 27.590 | 29.390 | 11.291 | 1.00 | 52.64 |
| ATOM | 489 | OE2 | GLU | A | 77 | 28.555 | 29.804 | 9.366 | 1.00 | 53.43 |
| ATOM | 490 | C | GLU | A | 77 | 29.380 | 24.662 | 12.516 | 1.00 | 40.43 |
| ATOM | 491 | O | GLU | A | 77 | 30.530 | 24.438 | 12.897 | 1.00 | 41.99 |
| ATOM | 492 | N | HIS | A | 78 | 28.303 | 24.378 | 13.237 | 1.00 | 38.59 |
| ATOM | 493 | CA | HIS | A | 78 | 28.396 | 23.799 | 14.570 | 1.00 | 37.71 |
| ATOM | 494 | CB | HIS | A | 78 | 27.037 | 23.867 | 15.264 | 1.00 | 38.65 |
| ATOM | 495 | CG | HIS | A | 78 | 27.012 | 23.206 | 16.609 | 1.00 | 39.57 |
| ATOM | 496 | CD2 | HIS | A | 78 | 26.486 | 22.027 | 17.013 | 1.00 | 39.47 |
| ATOM | 497 | ND1 | HIS | A | 78 | 27.583 | 23.773 | 17.728 | 1.00 | 40.44 |
| ATOM | 498 | CE1 | HIS | A | 78 | 27.406 | 22.972 | 18.764 | 1.00 | 40.13 |
| ATOM | 499 | NE2 | HIS | A | 78 | 26.743 | 21.906 | 18.358 | 1.00 | 39.37 |
| ATOM | 500 | C | HIS | A | 78 | 28.887 | 22.355 | 14.662 | 1.00 | 36.34 |
| ATOM | 501 | O | HIS | A | 78 | 29.712 | 22.036 | 15.519 | 1.00 | 37.02 |
| ATOM | 502 | N | GLN | A | 79 | 28.381 | 21.489 | 13.788 | 1.00 | 33.26 |
| ATOM | 503 | CA | GLN | A | 79 | 28.720 | 20.071 | 13.848 | 1.00 | 30.56 |
| ATOM | 504 | CB | GLN | A | 79 | 27.585 | 19.331 | 14.546 | 1.00 | 29.33 |
| ATOM | 505 | CG | GLN | A | 79 | 26.258 | 19.591 | 13.833 | 1.00 | 28.60 |
| ATOM | 506 | CD | GLN | A | 79 | 25.083 | 18.843 | 14.408 | 1.00 | 26.36 |
| ATOM | 507 | OE1 | GLN | A | 79 | 24.731 | 18.998 | 15.577 | 1.00 | 22.77 |
| ATOM | 508 | NE2 | GLN | A | 79 | 24.455 | 18.029 | 13.572 | 1.00 | 26.46 |
| ATOM | 509 | C | GLN | A | 79 | 28.958 | 19.412 | 12.507 | 1.00 | 30.38 |
| ATOM | 510 | O | GLN | A | 79 | 29.151 | 18.206 | 12.452 | 1.00 | 30.90 |
| ATOM | 511 | N | GLY | A | 80 | 28.938 | 20.184 | 11.427 | 1.00 | 30.93 |
| ATOM | 512 | CA | GLY | A | 80 | 29.146 | 19.606 | 10.109 | 1.00 | 31.44 |
| ATOM | 513 | C | GLY | A | 80 | 27.839 | 19.489 | 9.339 | 1.00 | 32.90 |
| ATOM | 514 | O | GLY | A | 80 | 26.767 | 19.518 | 9.944 | 1.00 | 33.62 |
| ATOM | 515 | N | PRO | A | 81 | 27.888 | 19.349 | 8.003 | 1.00 | 32.99 |
| ATOM | 516 | CD | PRO | A | 81 | 29.104 | 19.132 | 7.201 | 1.00 | 32.22 |
| ATOM | 517 | CA | PRO | A | 81 | 26.682 | 19.232 | 7.174 | 1.00 | 32.81 |
| ATOM | 518 | CB | PRO | A | 81 | 27.235 | 18.808 | 5.818 | 1.00 | 33.18 |
| ATOM | 519 | CG | PRO | A | 81 | 28.617 | 19.416 | 5.810 | 1.00 | 33.63 |
| ATOM | 520 | C | PRO | A | 81 | 25.696 | 18.210 | 7.731 | 1.00 | 32.39 |
| ATOM | 521 | O | PRO | A | 81 | 26.089 | 17.275 | 8.417 | 1.00 | 33.55 |
| ATOM | 522 | N | VAL | A | 82 | 24.412 | 18.398 | 7.451 | 1.00 | 31.46 |
| ATOM | 523 | CA | VAL | A | 82 | 23.405 | 17.460 | 7.927 | 1.00 | 31.06 |
| ATOM | 524 | CB | VAL | A | 82 | 21.988 | 18.039 | 7.769 | 1.00 | 32.44 |
| ATOM | 525 | CG1 | VAL | A | 82 | 20.941 | 16.941 | 7.971 | 1.00 | 33.70 |
| ATOM | 526 | CG2 | VAL | A | 82 | 21.783 | 19.152 | 8.781 | 1.00 | 31.86 |
| ATOM | 527 | C | VAL | A | 82 | 23.519 | 16.153 | 7.142 | 1.00 | 29.52 |
| ATOM | 528 | O | VAL | A | 82 | 23.398 | 16.138 | 5.918 | 1.00 | 29.35 |
| ATOM | 529 | N | GLU | A | 83 | 23.761 | 15.066 | 7.863 | 1.00 | 29.74 |
| ATOM | 530 | CA | GLU | A | 83 | 23.920 | 13.745 | 7.264 | 1.00 | 27.70 |
| ATOM | 531 | CB | GLU | A | 83 | 25.041 | 13.003 | 7.984 | 1.00 | 27.80 |
| ATOM | 532 | CG | GLU | A | 83 | 26.368 | 13.728 | 7.919 | 1.00 | 27.64 |
| ATOM | 533 | CD | GLU | A | 83 | 27.492 | 12.918 | 8.506 | 1.00 | 27.14 |
| ATOM | 534 | OE1 | GLU | A | 83 | 27.581 | 12.824 | 9.750 | 1.00 | 27.46 |
| ATOM | 535 | OE2 | GLU | A | 83 | 28.278 | 12.367 | 7.712 | 1.00 | 26.28 |
| ATOM | 536 | C | GLU | A | 83 | 22.633 | 12.932 | 7.332 | 1.00 | 26.73 |
| ATOM | 537 | O | GLU | A | 83 | 22.285 | 12.212 | 6.401 | 1.00 | 27.36 |
| ATOM | 538 | N | VAL | A | 84 | 21.938 | 13.049 | 8.453 | 1.00 | 25.12 |
| ATOM | 539 | CA | VAL | A | 84 | 20.689 | 12.350 | 8.644 | 1.00 | 24.41 |
| ATOM | 540 | CB | VAL | A | 84 | 20.829 | 11.212 | 9.694 | 1.00 | 26.40 |
| ATOM | 541 | CG1 | VAL | A | 84 | 21.626 | 11.686 | 10.852 | 1.00 | 28.62 |
| ATOM | 542 | CG2 | VAL | A | 84 | 19.462 | 10.759 | 10.177 | 1.00 | 25.08 |
| ATOM | 543 | C | VAL | A | 84 | 19.675 | 13.380 | 9.106 | 1.00 | 23.68 |
| ATOM | 544 | O | VAL | A | 84 | 19.837 | 14.007 | 10.151 | 1.00 | 24.48 |
| ATOM | 545 | N | LEU | A | 85 | 18.644 | 13.567 | 8.294 | 1.00 | 21.80 |
| ATOM | 546 | CA | LEU | A | 85 | 17.585 | 14.524 | 8.588 | 1.00 | 20.97 |
| ATOM | 547 | CB | LEU | A | 85 | 17.227 | 15.307 | 7.314 | 1.00 | 20.48 |

Figure 3 (suite 7)

| | | | | | | | | | | |
|---|---|---|---|---|---|---|---|---|---|---|
| ATOM | 548 | CG | LEU | A | 85 | 15.826 | 15.922 | 7.190 | 1.00 | 20.87 |
| ATOM | 549 | CD1 | LEU | A | 85 | 15.586 | 16.948 | 8.284 | 1.00 | 18.65 |
| ATOM | 550 | CD2 | LEU | A | 85 | 15.682 | 16.556 | 5.815 | 1.00 | 22.06 |
| ATOM | 551 | C | LEU | A | 85 | 16.351 | 13.805 | 9.114 | 1.00 | 20.85 |
| ATOM | 552 | O | LEU | A | 85 | 15.880 | 12.847 | 8.511 | 1.00 | 20.16 |
| ATOM | 553 | N | VAL | A | 86 | 15.844 | 14.259 | 10.247 | 1.00 | 20.94 |
| ATOM | 554 | CA | VAL | A | 86 | 14.652 | 13.675 | 10.825 | 1.00 | 20.86 |
| ATOM | 555 | CB | VAL | A | 86 | 14.909 | 13.202 | 12.290 | 1.00 | 22.26 |
| ATOM | 556 | CG1 | VAL | A | 86 | 13.620 | 12.772 | 12.938 | 1.00 | 18.75 |
| ATOM | 557 | CG2 | VAL | A | 86 | 15.920 | 12.039 | 12.304 | 1.00 | 19.76 |
| ATOM | 558 | C | VAL | A | 86 | 13.545 | 14.734 | 10.794 | 1.00 | 23.10 |
| ATOM | 559 | O | VAL | A | 86 | 13.383 | 15.519 | 11.729 | 1.00 | 22.68 |
| ATOM | 560 | N | SER | A | 87 | 12.793 | 14.762 | 9.696 | 1.00 | 24.21 |
| ATOM | 561 | CA | SER | A | 87 | 11.698 | 15.709 | 9.559 | 1.00 | 23.65 |
| ATOM | 562 | CB | SER | A | 87 | 11.289 | 15.815 | 8.097 | 1.00 | 21.58 |
| ATOM | 563 | OG | SER | A | 87 | 10.339 | 16.849 | 7.907 | 1.00 | 23.62 |
| ATOM | 564 | C | SER | A | 87 | 10.531 | 15.214 | 10.419 | 1.00 | 25.81 |
| ATOM | 565 | O | SER | A | 87 | 9.811 | 14.284 | 10.059 | 1.00 | 24.72 |
| ATOM | 566 | N | ASN | A | 88 | 10.365 | 15.848 | 11.570 | 1.00 | 27.52 |
| ATOM | 567 | CA | ASN | A | 88 | 9.331 | 15.491 | 12.519 | 1.00 | 29.45 |
| ATOM | 568 | CB | ASN | A | 88 | 10.010 | 15.092 | 13.836 | 1.00 | 28.55 |
| ATOM | 569 | CG | ASN | A | 88 | 9.040 | 14.889 | 14.961 | 1.00 | 27.45 |
| ATOM | 570 | OD1 | ASN | A | 88 | 8.556 | 15.844 | 15.557 | 1.00 | 30.89 |
| ATOM | 571 | ND2 | ASN | A | 88 | 8.749 | 13.641 | 15.268 | 1.00 | 29.74 |
| ATOM | 572 | C | ASN | A | 88 | 8.350 | 16.651 | 12.727 | 1.00 | 31.89 |
| ATOM | 573 | O | ASN | A | 88 | 7.193 | 16.445 | 13.089 | 1.00 | 31.90 |
| ATOM | 574 | N | ALA | A | 89 | 8.818 | 17.871 | 12.483 | 1.00 | 34.33 |
| ATOM | 575 | CA | ALA | A | 89 | 7.993 | 19.061 | 12.652 | 1.00 | 36.16 |
| ATOM | 576 | CB | ALA | A | 89 | 8.672 | 20.254 | 11.979 | 1.00 | 35.01 |
| ATOM | 577 | C | ALA | A | 89 | 6.590 | 18.855 | 12.081 | 1.00 | 38.12 |
| ATOM | 578 | O | ALA | A | 89 | 6.430 | 18.582 | 10.891 | 1.00 | 38.88 |
| ATOM | 579 | N | GLY | A | 90 | 5.576 | 18.982 | 12.934 | 1.00 | 39.69 |
| ATOM | 580 | CA | GLY | A | 90 | 4.212 | 18.799 | 12.475 | 1.00 | 41.37 |
| ATOM | 581 | C | GLY | A | 90 | 3.138 | 19.433 | 13.342 | 1.00 | 43.10 |
| ATOM | 582 | O | GLY | A | 90 | 3.422 | 20.024 | 14.385 | 1.00 | 43.36 |
| ATOM | 583 | N | LEU | A | 91 | 1.891 | 19.303 | 12.898 | 1.00 | 44.10 |
| ATOM | 584 | CA | LEU | A | 91 | 0.743 | 19.853 | 13.611 | 1.00 | 45.99 |
| ATOM | 585 | CB | LEU | A | 91 | 0.830 | 21.381 | 13.661 | 1.00 | 45.38 |
| ATOM | 586 | CG | LEU | A | 91 | 1.002 | 22.120 | 12.333 | 1.00 | 46.36 |
| ATOM | 587 | CD1 | LEU | A | 91 | -0.345 | 22.306 | 11.641 | 1.00 | 46.92 |
| ATOM | 588 | CD2 | LEU | A | 91 | 1.637 | 23.471 | 12.609 | 1.00 | 46.85 |
| ATOM | 589 | C | LEU | A | 91 | -0.542 | 19.423 | 12.921 | 1.00 | 46.75 |
| ATOM | 590 | O | LEU | A | 91 | -0.510 | 18.893 | 11.810 | 1.00 | 48.02 |
| ATOM | 591 | N | SER | A | 92 | -1.672 | 19.657 | 13.577 | 1.00 | 47.47 |
| ATOM | 592 | CA | SER | A | 92 | -2.954 | 19.270 | 13.012 | 1.00 | 48.31 |
| ATOM | 593 | CB | SER | A | 92 | -3.411 | 17.942 | 13.625 | 1.00 | 48.52 |
| ATOM | 594 | OG | SER | A | 92 | -3.610 | 18.061 | 15.027 | 1.00 | 47.53 |
| ATOM | 595 | C | SER | A | 92 | -4.050 | 20.317 | 13.203 | 1.00 | 48.78 |
| ATOM | 596 | O | SER | A | 92 | -3.887 | 21.296 | 13.931 | 1.00 | 48.31 |
| ATOM | 597 | N | ALA | A | 93 | -5.169 | 20.081 | 12.528 | 1.00 | 49.99 |
| ATOM | 598 | CA | ALA | A | 93 | -6.343 | 20.942 | 12.569 | 1.00 | 50.31 |
| ATOM | 599 | CB | ALA | A | 93 | -6.151 | 22.142 | 11.651 | 1.00 | 49.90 |
| ATOM | 600 | C | ALA | A | 93 | -7.482 | 20.068 | 12.064 | 1.00 | 51.33 |
| ATOM | 601 | O | ALA | A | 93 | -7.877 | 20.159 | 10.900 | 1.00 | 51.54 |
| ATOM | 602 | N | ASP | A | 94 | -7.991 | 19.207 | 12.942 | 1.00 | 52.25 |
| ATOM | 603 | CA | ASP | A | 94 | -9.068 | 18.289 | 12.588 | 1.00 | 52.73 |
| ATOM | 604 | CB | ASP | A | 94 | -9.116 | 17.108 | 13.564 | 1.00 | 53.52 |
| ATOM | 605 | CG | ASP | A | 94 | -7.874 | 16.243 | 13.495 | 1.00 | 55.60 |
| ATOM | 606 | OD1 | ASP | A | 94 | -7.250 | 16.174 | 12.410 | 1.00 | 55.28 |
| ATOM | 607 | OD2 | ASP | A | 94 | -7.537 | 15.618 | 14.524 | 1.00 | 57.42 |
| ATOM | 608 | C | ASP | A | 94 | -10.447 | 18.924 | 12.521 | 1.00 | 52.88 |
| ATOM | 609 | O | ASP | A | 94 | -10.728 | 19.919 | 13.192 | 1.00 | 53.19 |
| ATOM | 610 | N | ALA | A | 95 | -11.300 | 18.316 | 11.701 | 1.00 | 53.47 |
| ATOM | 611 | CA | ALA | A | 95 | -12.677 | 18.752 | 11.495 | 1.00 | 53.43 |
| ATOM | 612 | CB | ALA | A | 95 | -12.704 | 20.217 | 11.089 | 1.00 | 52.45 |
| ATOM | 613 | C | ALA | A | 95 | -13.290 | 17.890 | 10.391 | 1.00 | 53.67 |
| ATOM | 614 | O | ALA | A | 95 | -12.563 | 17.285 | 9.596 | 1.00 | 53.46 |
| ATOM | 615 | N | PHE | A | 96 | -14.617 | 17.822 | 10.341 | 1.00 | 53.28 |
| ATOM | 616 | CA | PHE | A | 96 | -15.283 | 17.043 | 9.302 | 1.00 | 53.07 |
| ATOM | 617 | CB | PHE | A | 96 | -16.784 | 16.921 | 9.590 | 1.00 | 53.95 |
| ATOM | 618 | CG | PHE | A | 96 | -17.112 | 16.181 | 10.860 | 1.00 | 54.98 |
| ATOM | 619 | CD1 | PHE | A | 96 | -16.888 | 16.765 | 12.103 | 1.00 | 56.45 |
| ATOM | 620 | CD2 | PHE | A | 96 | -17.671 | 14.905 | 10.812 | 1.00 | 55.80 |

Figure 3 (suite 8)

| | | | | | | | | | | | |
|---|---|---|---|---|---|---|---|---|---|---|---|
| ATOM | 621 | CE1 | PHE | A | 96 | -17.219 | 16.090 | 13.283 | 1.00 | 56.92 |
| ATOM | 622 | CE2 | PHE | A | 96 | -18.006 | 14.220 | 11.985 | 1.00 | 56.56 |
| ATOM | 623 | CZ | PHE | A | 96 | -17.780 | 14.814 | 13.221 | 1.00 | 57.26 |
| ATOM | 624 | C | PHE | A | 96 | -15.080 | 17.768 | 7.972 | 1.00 | 52.31 |
| ATOM | 625 | O | PHE | A | 96 | -15.006 | 18.995 | 7.945 | 1.00 | 51.35 |
| ATOM | 626 | N | LEU | A | 97 | -14.985 | 17.019 | 6.875 | 1.00 | 52.49 |
| ATOM | 627 | CA | LEU | A | 97 | -14.794 | 17.623 | 5.551 | 1.00 | 53.11 |
| ATOM | 628 | CB | LEU | A | 97 | -15.004 | 16.582 | 4.445 | 1.00 | 51.47 |
| ATOM | 629 | CG | LEU | A | 97 | -14.969 | 17.101 | 3.001 | 1.00 | 50.77 |
| ATOM | 630 | CD1 | LEU | A | 97 | -13.607 | 17.706 | 2.693 | 1.00 | 50.34 |
| ATOM | 631 | CD2 | LEU | A | 97 | -15.270 | 15.963 | 2.043 | 1.00 | 49.70 |
| ATOM | 632 | C | LEU | A | 97 | -15.773 | 18.775 | 5.346 | 1.00 | 53.57 |
| ATOM | 633 | O | LEU | A | 97 | -15.430 | 19.816 | 4.784 | 1.00 | 53.32 |
| ATOM | 634 | N | MET | A | 98 | -17.001 | 18.563 | 5.805 | 1.00 | 55.19 |
| ATOM | 635 | CA | MET | A | 98 | -18.064 | 19.553 | 5.706 | 1.00 | 55.81 |
| ATOM | 636 | CB | MET | A | 98 | -19.372 | 18.936 | 6.222 | 1.00 | 58.14 |
| ATOM | 637 | CG | MET | A | 98 | -20.521 | 19.918 | 6.432 | 1.00 | 62.07 |
| ATOM | 638 | SD | MET | A | 98 | -22.061 | 19.100 | 6.930 | 1.00 | 65.51 |
| ATOM | 639 | CE | MET | A | 98 | -23.001 | 19.173 | 5.383 | 1.00 | 65.81 |
| ATOM | 640 | C | MET | A | 98 | -17.707 | 20.797 | 6.522 | 1.00 | 55.21 |
| ATOM | 641 | O | MET | A | 98 | -17.963 | 21.930 | 6.097 | 1.00 | 54.92 |
| ATOM | 642 | N | ARG | A | 99 | -17.101 | 20.569 | 7.687 | 1.00 | 53.69 |
| ATOM | 643 | CA | ARG | A | 99 | -16.716 | 21.642 | 8.594 | 1.00 | 52.24 |
| ATOM | 644 | CB | ARG | A | 99 | -17.203 | 21.309 | 10.009 | 1.00 | 52.65 |
| ATOM | 645 | CG | ARG | A | 99 | -18.662 | 20.882 | 10.073 | 0.00 | 52.62 |
| ATOM | 646 | CD | ARG | A | 99 | -19.049 | 20.400 | 11.464 | 0.00 | 52.73 |
| ATOM | 647 | NE | ARG | A | 99 | -20.432 | 19.932 | 11.510 | 0.00 | 52.77 |
| ATOM | 648 | CZ | ARG | A | 99 | -21.024 | 19.445 | 12.596 | 0.00 | 52.80 |
| ATOM | 649 | NH1 | ARG | A | 99 | -20.356 | 19.359 | 13.738 | 0.00 | 52.82 |
| ATOM | 650 | NH2 | ARG | A | 99 | -22.287 | 19.043 | 12.540 | 0.00 | 52.82 |
| ATOM | 651 | C | ARG | A | 99 | -15.209 | 21.930 | 8.628 | 1.00 | 51.45 |
| ATOM | 652 | O | ARG | A | 99 | -14.681 | 22.336 | 9.662 | 1.00 | 51.72 |
| ATOM | 653 | N | MET | A | 100 | -14.514 | 21.710 | 7.515 | 1.00 | 49.18 |
| ATOM | 654 | CA | MET | A | 100 | -13.081 | 21.989 | 7.465 | 1.00 | 46.67 |
| ATOM | 655 | CB | MET | A | 100 | -12.276 | 20.758 | 7.035 | 1.00 | 46.70 |
| ATOM | 656 | CG | MET | A | 100 | -10.770 | 21.026 | 6.974 | 1.00 | 46.77 |
| ATOM | 657 | SD | MET | A | 100 | -9.778 | 19.595 | 6.524 | 1.00 | 47.29 |
| ATOM | 658 | CE | MET | A | 100 | -9.755 | 18.720 | 8.079 | 1.00 | 46.97 |
| ATOM | 659 | C | MET | A | 100 | -12.844 | 23.120 | 6.479 | 1.00 | 45.55 |
| ATOM | 660 | O | MET | A | 100 | -13.285 | 23.068 | 5.333 | 1.00 | 46.25 |
| ATOM | 661 | N | THR | A | 101 | -12.126 | 24.138 | 6.933 | 1.00 | 44.49 |
| ATOM | 662 | CA | THR | A | 101 | -11.854 | 25.312 | 6.119 | 1.00 | 42.26 |
| ATOM | 663 | CB | THR | A | 101 | -11.750 | 26.555 | 7.017 | 1.00 | 42.28 |
| ATOM | 664 | OG1 | THR | A | 101 | -10.507 | 26.529 | 7.727 | 1.00 | 43.08 |
| ATOM | 665 | CG2 | THR | A | 101 | -12.881 | 26.561 | 8.034 | 1.00 | 41.34 |
| ATOM | 666 | C | THR | A | 101 | -10.593 | 25.220 | 5.274 | 1.00 | 41.06 |
| ATOM | 667 | O | THR | A | 101 | -9.675 | 24.462 | 5.583 | 1.00 | 41.63 |
| ATOM | 668 | N | GLU | A | 102 | -10.565 | 26.004 | 4.203 | 1.00 | 39.43 |
| ATOM | 669 | CA | GLU | A | 102 | -9.423 | 26.067 | 3.306 | 1.00 | 38.09 |
| ATOM | 670 | CB | GLU | A | 102 | -9.649 | 27.156 | 2.251 | 1.00 | 37.31 |
| ATOM | 671 | CG | GLU | A | 102 | -8.534 | 27.298 | 1.231 | 1.00 | 36.77 |
| ATOM | 672 | CD | GLU | A | 102 | -8.496 | 28.679 | 0.583 | 1.00 | 37.58 |
| ATOM | 673 | OE1 | GLU | A | 102 | -7.983 | 28.801 | -0.549 | 1.00 | 37.18 |
| ATOM | 674 | OE2 | GLU | A | 102 | -8.965 | 29.652 | 1.211 | 1.00 | 38.79 |
| ATOM | 675 | C | GLU | A | 102 | -8.192 | 26.418 | 4.136 | 1.00 | 37.74 |
| ATOM | 676 | O | GLU | A | 102 | -7.090 | 25.950 | 3.853 | 1.00 | 37.62 |
| ATOM | 677 | N | GLU | A | 103 | -8.393 | 27.243 | 5.162 | 1.00 | 36.97 |
| ATOM | 678 | CA | GLU | A | 103 | -7.308 | 27.671 | 6.040 | 1.00 | 37.74 |
| ATOM | 679 | CB | GLU | A | 103 | -7.789 | 28.783 | 6.984 | 1.00 | 41.01 |
| ATOM | 680 | CG | GLU | A | 103 | -6.759 | 29.154 | 8.043 | 1.00 | 45.45 |
| ATOM | 681 | CD | GLU | A | 103 | -7.190 | 30.310 | 8.927 | 1.00 | 49.29 |
| ATOM | 682 | OE1 | GLU | A | 103 | -8.252 | 30.207 | 9.583 | 1.00 | 51.41 |
| ATOM | 683 | OE2 | GLU | A | 103 | -6.454 | 31.321 | 8.972 | 1.00 | 49.96 |
| ATOM | 684 | C | GLU | A | 103 | -6.717 | 26.520 | 6.867 | 1.00 | 35.91 |
| ATOM | 685 | O | GLU | A | 103 | -5.498 | 26.405 | 6.997 | 1.00 | 34.42 |
| ATOM | 686 | N | LYS | A | 104 | -7.576 | 25.687 | 7.443 | 1.00 | 33.36 |
| ATOM | 687 | CA | LYS | A | 104 | -7.100 | 24.556 | 8.228 | 1.00 | 33.31 |
| ATOM | 688 | CB | LYS | A | 104 | -8.260 | 23.861 | 8.939 | 1.00 | 32.29 |
| ATOM | 689 | CG | LYS | A | 104 | -8.713 | 24.572 | 10.193 | 1.00 | 37.41 |
| ATOM | 690 | CD | LYS | A | 104 | -9.531 | 23.643 | 11.073 | 1.00 | 40.80 |
| ATOM | 691 | CE | LYS | A | 104 | -9.781 | 24.254 | 12.441 | 1.00 | 41.97 |
| ATOM | 692 | NZ | LYS | A | 104 | -10.370 | 23.254 | 13.389 | 1.00 | 42.60 |
| ATOM | 693 | C | LYS | A | 104 | -6.356 | 23.546 | 7.356 | 1.00 | 31.88 |

Figure 3 (suite 9)

| | | | | | | | | | | |
|---|---|---|---|---|---|---|---|---|---|---|
| ATOM | 694 | O | LYS | A | 104 | -5.379 | 22.942 | 7.795 | 1.00 | 33.48 |
| ATOM | 695 | N | PHE | A | 105 | -6.805 | 23.375 | 6.119 | 1.00 | 29.89 |
| ATOM | 696 | CA | PHE | A | 105 | -6.161 | 22.432 | 5.218 | 1.00 | 28.79 |
| ATOM | 697 | CB | PHE | A | 105 | -7.015 | 22.222 | 3.966 | 1.00 | 27.90 |
| ATOM | 698 | CG | PHE | A | 105 | -6.539 | 21.103 | 3.095 | 1.00 | 28.42 |
| ATOM | 699 | CD1 | PHE | A | 105 | -6.855 | 19.783 | 3.404 | 1.00 | 30.17 |
| ATOM | 700 | CD2 | PHE | A | 105 | -5.752 | 21.361 | 1.981 | 1.00 | 29.17 |
| ATOM | 701 | CE1 | PHE | A | 105 | -6.392 | 18.731 | 2.608 | 1.00 | 31.18 |
| ATOM | 702 | CE2 | PHE | A | 105 | -5.282 | 20.328 | 1.180 | 1.00 | 29.69 |
| ATOM | 703 | CZ | PHE | A | 105 | -5.602 | 19.005 | 1.493 | 1.00 | 30.87 |
| ATOM | 704 | C | PHE | A | 105 | -4.773 | 22.921 | 4.806 | 1.00 | 27.73 |
| ATOM | 705 | O | PHE | A | 105 | -3.764 | 22.277 | 5.093 | 1.00 | 26.87 |
| ATOM | 706 | N | GLU | A | 106 | -4.738 | 24.054 | 4.111 | 1.00 | 27.57 |
| ATOM | 707 | CA | GLU | A | 106 | -3.493 | 24.650 | 3.645 | 1.00 | 28.68 |
| ATOM | 708 | CB | GLU | A | 106 | -3.780 | 25.976 | 2.936 | 1.00 | 29.17 |
| ATOM | 709 | CG | GLU | A | 106 | -3.995 | 25.815 | 1.448 | 1.00 | 34.78 |
| ATOM | 710 | CD | GLU | A | 106 | -4.413 | 27.100 | 0.767 | 1.00 | 35.27 |
| ATOM | 711 | OE1 | GLU | A | 106 | -4.331 | 27.164 | -0.474 | 1.00 | 38.35 |
| ATOM | 712 | OE2 | GLU | A | 106 | -4.832 | 28.043 | 1.467 | 1.00 | 38.25 |
| ATOM | 713 | C | GLU | A | 106 | -2.498 | 24.874 | 4.774 | 1.00 | 27.74 |
| ATOM | 714 | O | GLU | A | 106 | -1.288 | 24.771 | 4.571 | 1.00 | 26.43 |
| ATOM | 715 | N | LYS | A | 107 | -3.010 | 25.181 | 5.961 | 1.00 | 26.18 |
| ATOM | 716 | CA | LYS | A | 107 | -2.146 | 25.397 | 7.110 | 1.00 | 27.95 |
| ATOM | 717 | CB | LYS | A | 107 | -2.984 | 25.751 | 8.343 | 1.00 | 29.05 |
| ATOM | 718 | CG | LYS | A | 107 | -2.180 | 26.261 | 9.514 | 1.00 | 30.42 |
| ATOM | 719 | CD | LYS | A | 107 | -3.060 | 26.524 | 10.736 | 1.00 | 31.28 |
| ATOM | 720 | CE | LYS | A | 107 | -4.022 | 27.675 | 10.493 | 0.00 | 30.99 |
| ATOM | 721 | NZ | LYS | A | 107 | -3.297 | 28.949 | 10.234 | 0.00 | 31.10 |
| ATOM | 722 | C | LYS | A | 107 | -1.367 | 24.101 | 7.354 | 1.00 | 27.13 |
| ATOM | 723 | O | LYS | A | 107 | -0.147 | 24.064 | 7.200 | 1.00 | 29.03 |
| ATOM | 724 | N | VAL | A | 108 | -2.091 | 23.040 | 7.714 | 1.00 | 25.47 |
| ATOM | 725 | CA | VAL | A | 108 | -1.503 | 21.734 | 7.979 | 1.00 | 23.61 |
| ATOM | 726 | CB | VAL | A | 108 | -2.597 | 20.701 | 8.265 | 1.00 | 23.11 |
| ATOM | 727 | CG1 | VAL | A | 108 | -2.000 | 19.302 | 8.343 | 1.00 | 23.60 |
| ATOM | 728 | CG2 | VAL | A | 108 | -3.288 | 21.047 | 9.572 | 1.00 | 22.61 |
| ATOM | 729 | C | VAL | A | 108 | -0.635 | 21.243 | 6.835 | 1.00 | 23.45 |
| ATOM | 730 | O | VAL | A | 108 | 0.422 | 20.654 | 7.053 | 1.00 | 23.97 |
| ATOM | 731 | N | ILE | A | 109 | -1.071 | 21.500 | 5.612 | 1.00 | 24.00 |
| ATOM | 732 | CA | ILE | A | 109 | -0.315 | 21.075 | 4.445 | 1.00 | 22.60 |
| ATOM | 733 | CB | ILE | A | 109 | -1.141 | 21.271 | 3.156 | 1.00 | 21.59 |
| ATOM | 734 | CG2 | ILE | A | 109 | -0.248 | 21.255 | 1.939 | 1.00 | 18.01 |
| ATOM | 735 | CG1 | ILE | A | 109 | -2.201 | 20.178 | 3.055 | 1.00 | 23.30 |
| ATOM | 736 | CD | ILE | A | 109 | -1.619 | 18.781 | 2.853 | 1.00 | 22.32 |
| ATOM | 737 | C | ILE | A | 109 | 1.018 | 21.801 | 4.299 | 1.00 | 24.03 |
| ATOM | 738 | O | ILE | A | 109 | 2.029 | 21.196 | 3.930 | 1.00 | 25.56 |
| ATOM | 739 | N | ASN | A | 110 | 1.016 | 23.098 | 4.587 | 1.00 | 24.87 |
| ATOM | 740 | CA | ASN | A | 110 | 2.213 | 23.925 | 4.459 | 1.00 | 25.23 |
| ATOM | 741 | CB | ASN | A | 110 | 1.830 | 25.396 | 4.683 | 1.00 | 25.06 |
| ATOM | 742 | CG | ASN | A | 110 | 2.887 | 26.368 | 4.199 | 1.00 | 23.28 |
| ATOM | 743 | OD1 | ASN | A | 110 | 3.536 | 26.151 | 3.182 | 1.00 | 25.75 |
| ATOM | 744 | ND2 | ASN | A | 110 | 3.040 | 27.466 | 4.917 | 1.00 | 25.22 |
| ATOM | 745 | C | ASN | A | 110 | 3.266 | 23.486 | 5.465 | 1.00 | 25.85 |
| ATOM | 746 | O | ASN | A | 110 | 4.436 | 23.287 | 5.124 | 1.00 | 26.39 |
| ATOM | 747 | N | ALA | A | 111 | 2.826 | 23.316 | 6.703 | 1.00 | 25.24 |
| ATOM | 748 | CA | ALA | A | 111 | 3.694 | 22.910 | 7.796 | 1.00 | 25.28 |
| ATOM | 749 | CB | ALA | A | 111 | 2.974 | 23.129 | 9.118 | 1.00 | 25.88 |
| ATOM | 750 | C | ALA | A | 111 | 4.197 | 21.469 | 7.729 | 1.00 | 25.55 |
| ATOM | 751 | O | ALA | A | 111 | 5.390 | 21.214 | 7.897 | 1.00 | 26.70 |
| ATOM | 752 | N | ASN | A | 112 | 3.287 | 20.530 | 7.496 | 1.00 | 24.93 |
| ATOM | 753 | CA | ASN | A | 112 | 3.645 | 19.116 | 7.465 | 1.00 | 24.90 |
| ATOM | 754 | CB | ASN | A | 112 | 2.417 | 18.269 | 7.827 | 1.00 | 26.43 |
| ATOM | 755 | CG | ASN | A | 112 | 1.896 | 18.576 | 9.210 | 1.00 | 27.63 |
| ATOM | 756 | OD1 | ASN | A | 112 | 2.411 | 19.459 | 9.893 | 1.00 | 29.04 |
| ATOM | 757 | ND2 | ASN | A | 112 | 0.870 | 17.852 | 9.635 | 1.00 | 31.91 |
| ATOM | 758 | C | ASN | A | 112 | 4.239 | 18.611 | 6.164 | 1.00 | 23.66 |
| ATOM | 759 | O | ASN | A | 112 | 5.256 | 17.929 | 6.168 | 1.00 | 26.52 |
| ATOM | 760 | N | LEU | A | 113 | 3.618 | 18.948 | 5.045 | 1.00 | 22.66 |
| ATOM | 761 | CA | LEU | A | 113 | 4.101 | 18.470 | 3.766 | 1.00 | 20.48 |
| ATOM | 762 | CB | LEU | A | 113 | 2.906 | 18.177 | 2.859 | 1.00 | 18.83 |
| ATOM | 763 | CG | LEU | A | 113 | 3.214 | 17.595 | 1.481 | 1.00 | 19.11 |
| ATOM | 764 | CD1 | LEU | A | 113 | 4.000 | 16.311 | 1.652 | 1.00 | 16.85 |
| ATOM | 765 | CD2 | LEU | A | 113 | 1.911 | 17.347 | 0.713 | 1.00 | 19.28 |
| ATOM | 766 | C | LEU | A | 113 | 5.104 | 19.375 | 3.045 | 1.00 | 20.90 |

Figure 3 (suite 10)

| | | | | | | | | | | |
|---|---|---|---|---|---|---|---|---|---|---|
| ATOM | 767 | O | LEU | A | 113 | 6.156 | 18.904 | 2.599 | 1.00 | 21.34 |
| ATOM | 768 | N | THR | A | 114 | 4.788 | 20.660 | 2.903 | 1.00 | 20.83 |
| ATOM | 769 | CA | THR | A | 114 | 5.713 | 21.565 | 2.223 | 1.00 | 21.18 |
| ATOM | 770 | CB | THR | A | 114 | 5.076 | 22.956 | 1.935 | 1.00 | 23.91 |
| ATOM | 771 | OG1 | THR | A | 114 | 3.809 | 22.777 | 1.287 | 1.00 | 25.43 |
| ATOM | 772 | CG2 | THR | A | 114 | 5.980 | 23.774 | 0.994 | 1.00 | 22.04 |
| ATOM | 773 | C | THR | A | 114 | 6.909 | 21.724 | 3.147 | 1.00 | 21.45 |
| ATOM | 774 | O | THR | A | 114 | 8.045 | 21.839 | 2.697 | 1.00 | 22.69 |
| ATOM | 775 | N | GLY | A | 115 | 6.647 | 21.714 | 4.447 | 1.00 | 21.80 |
| ATOM | 776 | CA | GLY | A | 115 | 7.733 | 21.819 | 5.394 | 1.00 | 20.61 |
| ATOM | 777 | C | GLY | A | 115 | 8.703 | 20.666 | 5.170 | 1.00 | 20.43 |
| ATOM | 778 | O | GLY | A | 115 | 9.919 | 20.852 | 5.072 | 1.00 | 19.81 |
| ATOM | 779 | N | ALA | A | 116 | 8.156 | 19.463 | 5.065 | 1.00 | 20.29 |
| ATOM | 780 | CA | ALA | A | 116 | 8.983 | 18.288 | 4.859 | 1.00 | 21.73 |
| ATOM | 781 | CB | ALA | A | 116 | 8.111 | 17.038 | 4.779 | 1.00 | 18.86 |
| ATOM | 782 | C | ALA | A | 116 | 9.768 | 18.473 | 3.576 | 1.00 | 23.23 |
| ATOM | 783 | O | ALA | A | 116 | 10.901 | 18.010 | 3.459 | 1.00 | 25.44 |
| ATOM | 784 | N | PHE | A | 117 | 9.169 | 19.159 | 2.609 | 1.00 | 23.52 |
| ATOM | 785 | CA | PHE | A | 117 | 9.848 | 19.386 | 1.343 | 1.00 | 23.14 |
| ATOM | 786 | CB | PHE | A | 117 | 8.894 | 19.913 | 0.279 | 1.00 | 21.79 |
| ATOM | 787 | CG | PHE | A | 117 | 9.609 | 20.465 | -0.928 | 1.00 | 23.00 |
| ATOM | 788 | CD1 | PHE | A | 117 | 10.120 | 19.613 | -1.901 | 1.00 | 21.13 |
| ATOM | 789 | CD2 | PHE | A | 117 | 9.830 | 21.840 | -1.057 | 1.00 | 18.79 |
| ATOM | 790 | CE1 | PHE | A | 117 | 10.838 | 20.120 | -2.979 | 1.00 | 18.72 |
| ATOM | 791 | CE2 | PHE | A | 117 | 10.542 | 22.351 | -2.127 | 1.00 | 18.79 |
| ATOM | 792 | CZ | PHE | A | 117 | 11.048 | 21.498 | -3.089 | 1.00 | 20.42 |
| ATOM | 793 | C | PHE | A | 117 | 10.978 | 20.398 | 1.493 | 1.00 | 22.48 |
| ATOM | 794 | O | PHE | A | 117 | 12.036 | 20.254 | 0.882 | 1.00 | 21.49 |
| ATOM | 795 | N | ARG | A | 118 | 10.748 | 21.436 | 2.287 | 1.00 | 22.22 |
| ATOM | 796 | CA | ARG | A | 118 | 11.777 | 22.449 | 2.468 | 1.00 | 22.92 |
| ATOM | 797 | CB | ARG | A | 118 | 11.248 | 23.594 | 3.336 | 1.00 | 23.27 |
| ATOM | 798 | CG | ARG | A | 118 | 10.128 | 24.361 | 2.636 | 1.00 | 23.10 |
| ATOM | 799 | CD | ARG | A | 118 | 9.775 | 25.694 | 3.311 | 1.00 | 24.63 |
| ATOM | 800 | NE | ARG | A | 118 | 8.666 | 26.338 | 2.613 | 1.00 | 23.86 |
| ATOM | 801 | CZ | ARG | A | 118 | 7.383 | 26.138 | 2.901 | 1.00 | 26.99 |
| ATOM | 802 | NH1 | ARG | A | 118 | 7.037 | 25.317 | 3.885 | 1.00 | 26.10 |
| ATOM | 803 | NH2 | ARG | A | 118 | 6.442 | 26.752 | 2.196 | 1.00 | 26.07 |
| ATOM | 804 | C | ARG | A | 118 | 13.046 | 21.844 | 3.055 | 1.00 | 23.54 |
| ATOM | 805 | O | ARG | A | 118 | 14.087 | 21.840 | 2.400 | 1.00 | 22.80 |
| ATOM | 806 | N | VAL | A | 119 | 12.962 | 21.304 | 4.267 | 1.00 | 24.01 |
| ATOM | 807 | CA | VAL | A | 119 | 14.134 | 20.696 | 4.889 | 1.00 | 23.16 |
| ATOM | 808 | CB | VAL | A | 119 | 13.785 | 20.094 | 6.265 | 1.00 | 22.24 |
| ATOM | 809 | CG1 | VAL | A | 119 | 13.342 | 21.200 | 7.195 | 1.00 | 21.77 |
| ATOM | 810 | CG2 | VAL | A | 119 | 12.679 | 19.046 | 6.139 | 1.00 | 22.60 |
| ATOM | 811 | C | VAL | A | 119 | 14.749 | 19.633 | 3.982 | 1.00 | 23.38 |
| ATOM | 812 | O | VAL | A | 119 | 15.959 | 19.608 | 3.778 | 1.00 | 27.84 |
| ATOM | 813 | N | ALA | A | 120 | 13.919 | 18.775 | 3.403 | 1.00 | 24.35 |
| ATOM | 814 | CA | ALA | A | 120 | 14.422 | 17.728 | 2.520 | 1.00 | 24.26 |
| ATOM | 815 | CB | ALA | A | 120 | 13.275 | 16.856 | 2.043 | 1.00 | 21.60 |
| ATOM | 816 | C | ALA | A | 120 | 15.170 | 18.279 | 1.314 | 1.00 | 23.93 |
| ATOM | 817 | O | ALA | A | 120 | 16.169 | 17.715 | 0.882 | 1.00 | 23.22 |
| ATOM | 818 | N | GLN | A | 121 | 14.667 | 19.369 | 0.754 | 1.00 | 25.14 |
| ATOM | 819 | CA | GLN | A | 121 | 15.287 | 19.967 | -0.423 | 1.00 | 27.71 |
| ATOM | 820 | CB | GLN | A | 121 | 14.290 | 20.938 | -1.078 | 1.00 | 29.44 |
| ATOM | 821 | CG | GLN | A | 121 | 14.653 | 21.428 | -2.478 | 1.00 | 31.51 |
| ATOM | 822 | CD | GLN | A | 121 | 15.596 | 22.611 | -2.461 | 1.00 | 35.04 |
| ATOM | 823 | OE1 | GLN | A | 121 | 15.509 | 23.476 | -1.581 | 1.00 | 35.62 |
| ATOM | 824 | NE2 | GLN | A | 121 | 16.493 | 22.670 | -3.443 | 1.00 | 37.12 |
| ATOM | 825 | C | GLN | A | 121 | 16.597 | 20.674 | -0.045 | 1.00 | 26.96 |
| ATOM | 826 | O | GLN | A | 121 | 17.547 | 20.701 | -0.822 | 1.00 | 28.13 |
| ATOM | 827 | N | ARG | A | 122 | 16.642 | 21.228 | 1.158 | 1.00 | 27.08 |
| ATOM | 828 | CA | ARG | A | 122 | 17.833 | 21.911 | 1.638 | 1.00 | 28.77 |
| ATOM | 829 | CB | ARG | A | 122 | 17.494 | 22.737 | 2.874 | 1.00 | 28.83 |
| ATOM | 830 | CG | ARG | A | 122 | 18.695 | 23.396 | 3.518 | 1.00 | 31.87 |
| ATOM | 831 | CD | ARG | A | 122 | 19.338 | 24.444 | 2.618 | 1.00 | 32.88 |
| ATOM | 832 | NE | ARG | A | 122 | 20.346 | 25.199 | 3.354 | 1.00 | 32.80 |
| ATOM | 833 | CZ | ARG | A | 122 | 21.545 | 24.727 | 3.677 | 1.00 | 32.67 |
| ATOM | 834 | NH1 | ARG | A | 122 | 21.895 | 23.502 | 3.315 | 1.00 | 31.70 |
| ATOM | 835 | NH2 | ARG | A | 122 | 22.380 | 25.463 | 4.399 | 1.00 | 32.74 |
| ATOM | 836 | C | ARG | A | 122 | 18.952 | 20.920 | 1.972 | 1.00 | 30.00 |
| ATOM | 837 | O | ARG | A | 122 | 20.109 | 21.136 | 1.606 | 1.00 | 30.77 |
| ATOM | 838 | N | ALA | A | 123 | 18.608 | 19.829 | 2.654 | 1.00 | 28.91 |
| ATOM | 839 | CA | ALA | A | 123 | 19.605 | 18.825 | 3.020 | 1.00 | 30.22 |

Figure 3 (suite 11)

| | | | | | | | | | | | |
|---|---|---|---|---|---|---|---|---|---|---|---|
| ATOM | 840 | CB | ALA | A | 123 | 18.990 | 17.799 | 3.959 | 1.00 | 26.72 |
| ATOM | 841 | C | ALA | A | 123 | 20.166 | 18.125 | 1.790 | 1.00 | 30.18 |
| ATOM | 842 | O | ALA | A | 123 | 21.312 | 17.684 | 1.780 | 1.00 | 30.59 |
| ATOM | 843 | N | SER | A | 124 | 19.346 | 18.045 | 0.751 | 1.00 | 32.19 |
| ATOM | 844 | CA | SER | A | 124 | 19.698 | 17.377 | -0.492 | 1.00 | 34.41 |
| ATOM | 845 | CB | SER | A | 124 | 18.734 | 17.817 | -1.594 | 1.00 | 36.31 |
| ATOM | 846 | OG | SER | A | 124 | 19.085 | 17.237 | -2.839 | 1.00 | 37.74 |
| ATOM | 847 | C | SER | A | 124 | 21.131 | 17.538 | -0.992 | 1.00 | 35.61 |
| ATOM | 848 | O | SER | A | 124 | 21.924 | 16.598 | -0.947 | 1.00 | 36.34 |
| ATOM | 849 | N | ARG | A | 125 | 21.441 | 18.729 | -1.491 | 1.00 | 37.00 |
| ATOM | 850 | CA | ARG | A | 125 | 22.748 | 19.059 | -2.050 | 1.00 | 36.33 |
| ATOM | 851 | CB | ARG | A | 125 | 22.905 | 20.581 | -2.082 | 1.00 | 40.67 |
| ATOM | 852 | CG | ARG | A | 125 | 21.696 | 21.321 | -2.666 | 1.00 | 46.97 |
| ATOM | 853 | CD | ARG | A | 125 | 21.774 | 22.830 | -2.411 | 1.00 | 51.59 |
| ATOM | 854 | NE | ARG | A | 125 | 21.836 | 23.131 | -0.980 | 1.00 | 54.75 |
| ATOM | 855 | CZ | ARG | A | 125 | 21.959 | 24.354 | -0.467 | 1.00 | 56.58 |
| ATOM | 856 | NH1 | ARG | A | 125 | 22.034 | 25.413 | -1.266 | 1.00 | 58.31 |
| ATOM | 857 | NH2 | ARG | A | 125 | 22.014 | 24.518 | 0.851 | 1.00 | 57.43 |
| ATOM | 858 | C | ARG | A | 125 | 23.951 | 18.439 | -1.338 | 1.00 | 34.05 |
| ATOM | 859 | O | ARG | A | 125 | 24.711 | 17.681 | -1.926 | 1.00 | 33.29 |
| ATOM | 860 | N | SER | A | 126 | 24.126 | 18.774 | -0.070 | 1.00 | 33.18 |
| ATOM | 861 | CA | SER | A | 126 | 25.253 | 18.274 | 0.701 | 1.00 | 33.32 |
| ATOM | 862 | CB | SER | A | 126 | 25.187 | 18.840 | 2.119 | 1.00 | 33.56 |
| ATOM | 863 | OG | SER | A | 126 | 26.338 | 18.495 | 2.863 | 1.00 | 37.42 |
| ATOM | 864 | C | SER | A | 126 | 25.305 | 16.742 | 0.736 | 1.00 | 33.36 |
| ATOM | 865 | O | SER | A | 126 | 26.346 | 16.149 | 0.460 | 1.00 | 33.36 |
| ATOM | 866 | N | MET | A | 127 | 24.178 | 16.112 | 1.069 | 1.00 | 32.40 |
| ATOM | 867 | CA | MET | A | 127 | 24.088 | 14.657 | 1.131 | 1.00 | 31.00 |
| ATOM | 868 | CB | MET | A | 127 | 22.664 | 14.232 | 1.505 | 1.00 | 27.94 |
| ATOM | 869 | CG | MET | A | 127 | 22.224 | 14.666 | 2.895 | 1.00 | 25.21 |
| ATOM | 870 | SD | MET | A | 127 | 20.457 | 14.346 | 3.184 | 1.00 | 24.58 |
| ATOM | 871 | CE | MET | A | 127 | 20.332 | 14.607 | 4.910 | 1.00 | 22.20 |
| ATOM | 872 | C | MET | A | 127 | 24.475 | 14.055 | -0.218 | 1.00 | 31.71 |
| ATOM | 873 | O | MET | A | 127 | 25.222 | 13.080 | -0.283 | 1.00 | 31.13 |
| ATOM | 874 | N | GLN | A | 128 | 23.967 | 14.644 | -1.294 | 1.00 | 32.77 |
| ATOM | 875 | CA | GLN | A | 128 | 24.271 | 14.166 | -2.638 | 1.00 | 34.17 |
| ATOM | 876 | CB | GLN | A | 128 | 23.559 | 15.011 | -3.691 | 1.00 | 36.22 |
| ATOM | 877 | CG | GLN | A | 128 | 22.133 | 14.628 | -3.995 | 1.00 | 38.15 |
| ATOM | 878 | CD | GLN | A | 128 | 21.556 | 15.470 | -5.119 | 1.00 | 40.49 |
| ATOM | 879 | OE1 | GLN | A | 128 | 21.209 | 16.635 | -4.923 | 1.00 | 40.10 |
| ATOM | 880 | NE2 | GLN | A | 128 | 21.471 | 14.886 | -6.313 | 1.00 | 42.21 |
| ATOM | 881 | C | GLN | A | 128 | 25.755 | 14.235 | -2.934 | 1.00 | 34.35 |
| ATOM | 882 | O | GLN | A | 128 | 26.327 | 13.314 | -3.513 | 1.00 | 34.51 |
| ATOM | 883 | N | ARG | A | 129 | 26.368 | 15.349 | -2.555 | 1.00 | 33.87 |
| ATOM | 884 | CA | ARG | A | 129 | 27.784 | 15.559 | -2.798 | 1.00 | 34.52 |
| ATOM | 885 | CB | ARG | A | 129 | 28.182 | 16.980 | -2.384 | 1.00 | 35.60 |
| ATOM | 886 | CG | ARG | A | 129 | 29.503 | 17.447 | -2.969 | 1.00 | 36.43 |
| ATOM | 887 | CD | ARG | A | 129 | 29.828 | 18.869 | -2.542 | 1.00 | 39.82 |
| ATOM | 888 | NE | ARG | A | 129 | 28.662 | 19.750 | -2.593 | 1.00 | 41.44 |
| ATOM | 889 | CZ | ARG | A | 129 | 28.015 | 20.176 | -1.515 | 1.00 | 42.01 |
| ATOM | 890 | NH1 | ARG | A | 129 | 28.423 | 19.804 | -0.309 | 1.00 | 43.02 |
| ATOM | 891 | NH2 | ARG | A | 129 | 26.963 | 20.973 | -1.638 | 1.00 | 42.88 |
| ATOM | 892 | C | ARG | A | 129 | 28.640 | 14.555 | -2.043 | 1.00 | 34.13 |
| ATOM | 893 | O | ARG | A | 129 | 29.522 | 13.931 | -2.628 | 1.00 | 33.66 |
| ATOM | 894 | N | ASN | A | 130 | 28.366 | 14.408 | -0.749 | 1.00 | 33.56 |
| ATOM | 895 | CA | ASN | A | 130 | 29.111 | 13.497 | 0.117 | 1.00 | 34.50 |
| ATOM | 896 | CB | ASN | A | 130 | 28.988 | 13.949 | 1.576 | 1.00 | 34.85 |
| ATOM | 897 | CG | ASN | A | 130 | 29.607 | 15.316 | 1.816 | 1.00 | 37.77 |
| ATOM | 898 | OD1 | ASN | A | 130 | 30.797 | 15.516 | 1.581 | 1.00 | 40.62 |
| ATOM | 899 | ND2 | ASN | A | 130 | 28.802 | 16.266 | 2.283 | 1.00 | 39.35 |
| ATOM | 900 | C | ASN | A | 130 | 28.661 | 12.042 | -0.004 | 1.00 | 34.68 |
| ATOM | 901 | O | ASN | A | 130 | 28.983 | 11.217 | 0.847 | 1.00 | 34.01 |
| ATOM | 902 | N | LYS | A | 131 | 27.919 | 11.741 | -1.066 | 1.00 | 34.67 |
| ATOM | 903 | CA | LYS | A | 131 | 27.410 | 10.400 | -1.305 | 1.00 | 34.70 |
| ATOM | 904 | CB | LYS | A | 131 | 28.500 | 9.509 | -1.894 | 1.00 | 35.51 |
| ATOM | 905 | CG | LYS | A | 131 | 28.394 | 9.267 | -3.395 | 1.00 | 35.95 |
| ATOM | 906 | CD | LYS | A | 131 | 29.287 | 10.194 | -4.189 | 1.00 | 36.56 |
| ATOM | 907 | CE | LYS | A | 131 | 28.717 | 11.596 | -4.254 | 1.00 | 38.43 |
| ATOM | 908 | NZ | LYS | A | 131 | 27.481 | 11.639 | -5.093 | 1.00 | 38.91 |
| ATOM | 909 | C | LYS | A | 131 | 26.847 | 9.729 | -0.053 | 1.00 | 35.01 |
| ATOM | 910 | O | LYS | A | 131 | 27.003 | 8.522 | 0.122 | 1.00 | 36.73 |
| ATOM | 911 | N | PHE | A | 132 | 26.210 | 10.503 | 0.820 | 1.00 | 32.09 |
| ATOM | 912 | CA | PHE | A | 132 | 25.611 | 9.941 | 2.023 | 1.00 | 28.98 |

Figure 3 (suite 12)

| | | | | | | | | | | |
|---|---|---|---|---|---|---|---|---|---|---|
| ATOM | 913 | CB | PHE | A | 132 | 26.640 | 9.744 | 3.141 | 1.00 | 29.57 |
| ATOM | 914 | CG | PHE | A | 132 | 26.051 | 9.116 | 4.380 | 1.00 | 28.76 |
| ATOM | 915 | CD1 | PHE | A | 132 | 25.933 | 7.734 | 4.489 | 1.00 | 28.98 |
| ATOM | 916 | CD2 | PHE | A | 132 | 25.514 | 9.908 | 5.388 | 1.00 | 30.38 |
| ATOM | 917 | CE1 | PHE | A | 132 | 25.281 | 7.148 | 5.580 | 1.00 | 28.94 |
| ATOM | 918 | CE2 | PHE | A | 132 | 24.858 | 9.331 | 6.484 | 1.00 | 30.64 |
| ATOM | 919 | CZ | PHE | A | 132 | 24.741 | 7.947 | 6.575 | 1.00 | 30.26 |
| ATOM | 920 | C | PHE | A | 132 | 24.463 | 10.769 | 2.591 | 1.00 | 27.22 |
| ATOM | 921 | O | PHE | A | 132 | 24.642 | 11.923 | 2.989 | 1.00 | 26.03 |
| ATOM | 922 | N | GLY | A | 133 | 23.290 | 10.150 | 2.667 | 1.00 | 25.90 |
| ATOM | 923 | CA | GLY | A | 133 | 22.139 | 10.833 | 3.214 | 1.00 | 26.01 |
| ATOM | 924 | C | GLY | A | 133 | 21.006 | 9.923 | 3.656 | 1.00 | 25.69 |
| ATOM | 925 | O | GLY | A | 133 | 20.609 | 8.991 | 2.947 | 1.00 | 26.18 |
| ATOM | 926 | N | ARG | A | 134 | 20.495 | 10.195 | 4.848 | 1.00 | 23.44 |
| ATOM | 927 | CA | ARG | A | 134 | 19.379 | 9.447 | 5.388 | 1.00 | 23.51 |
| ATOM | 928 | CB | ARG | A | 134 | 19.787 | 8.647 | 6.641 | 1.00 | 23.31 |
| ATOM | 929 | CG | ARG | A | 134 | 20.942 | 7.662 | 6.431 | 1.00 | 23.90 |
| ATOM | 930 | CD | ARG | A | 134 | 20.590 | 6.578 | 5.418 | 1.00 | 25.00 |
| ATOM | 931 | NE | ARG | A | 134 | 21.708 | 5.679 | 5.136 | 1.00 | 23.26 |
| ATOM | 932 | CZ | ARG | A | 134 | 22.472 | 5.748 | 4.048 | 1.00 | 26.81 |
| ATOM | 933 | NH1 | ARG | A | 134 | 22.245 | 6.680 | 3.135 | 1.00 | 26.66 |
| ATOM | 934 | NH2 | ARG | A | 134 | 23.453 | 4.868 | 3.856 | 1.00 | 25.61 |
| ATOM | 935 | C | ARG | A | 134 | 18.303 | 10.463 | 5.762 | 1.00 | 23.10 |
| ATOM | 936 | O | ARG | A | 134 | 18.496 | 11.290 | 6.655 | 1.00 | 24.41 |
| ATOM | 937 | N | MET | A | 135 | 17.178 | 10.420 | 5.061 | 1.00 | 22.34 |
| ATOM | 938 | CA | MET | A | 135 | 16.070 | 11.317 | 5.370 | 1.00 | 21.23 |
| ATOM | 939 | CB | MET | A | 135 | 15.554 | 12.000 | 4.103 | 1.00 | 22.05 |
| ATOM | 940 | CG | MET | A | 135 | 16.531 | 12.975 | 3.473 | 1.00 | 22.70 |
| ATOM | 941 | SD | MET | A | 135 | 15.755 | 13.894 | 2.146 | 1.00 | 24.85 |
| ATOM | 942 | CE | MET | A | 135 | 17.002 | 13.838 | 0.845 | 1.00 | 28.31 |
| ATOM | 943 | C | MET | A | 135 | 14.962 | 10.490 | 6.000 | 1.00 | 20.85 |
| ATOM | 944 | O | MET | A | 135 | 14.418 | 9.592 | 5.367 | 1.00 | 23.23 |
| ATOM | 945 | N | ILE | A | 136 | 14.629 | 10.791 | 7.246 | 1.00 | 18.50 |
| ATOM | 946 | CA | ILE | A | 136 | 13.591 | 10.062 | 7.939 | 1.00 | 17.43 |
| ATOM | 947 | CB | ILE | A | 136 | 14.158 | 9.416 | 9.213 | 1.00 | 17.40 |
| ATOM | 948 | CG2 | ILE | A | 136 | 13.132 | 8.488 | 9.829 | 1.00 | 16.14 |
| ATOM | 949 | CG1 | ILE | A | 136 | 15.427 | 8.638 | 8.856 | 1.00 | 18.24 |
| ATOM | 950 | CD | ILE | A | 136 | 16.236 | 8.182 | 10.042 | 1.00 | 18.53 |
| ATOM | 951 | C | ILE | A | 136 | 12.392 | 10.948 | 8.287 | 1.00 | 18.67 |
| ATOM | 952 | O | ILE | A | 136 | 12.419 | 11.715 | 9.254 | 1.00 | 16.20 |
| ATOM | 953 | N | PHE | A | 137 | 11.329 | 10.812 | 7.495 | 1.00 | 18.64 |
| ATOM | 954 | CA | PHE | A | 137 | 10.104 | 11.578 | 7.695 | 1.00 | 18.38 |
| ATOM | 955 | CB | PHE | A | 137 | 9.404 | 11.782 | 6.350 | 1.00 | 14.15 |
| ATOM | 956 | CG | PHE | A | 137 | 10.284 | 12.385 | 5.298 | 1.00 | 16.58 |
| ATOM | 957 | CD1 | PHE | A | 137 | 10.948 | 11.583 | 4.382 | 1.00 | 15.58 |
| ATOM | 958 | CD2 | PHE | A | 137 | 10.475 | 13.765 | 5.237 | 1.00 | 14.34 |
| ATOM | 959 | CE1 | PHE | A | 137 | 11.791 | 12.142 | 3.419 | 1.00 | 14.96 |
| ATOM | 960 | CE2 | PHE | A | 137 | 11.310 | 14.325 | 4.284 | 1.00 | 13.78 |
| ATOM | 961 | CZ | PHE | A | 137 | 11.970 | 13.511 | 3.372 | 1.00 | 13.51 |
| ATOM | 962 | C | PHE | A | 137 | 9.139 | 10.907 | 8.676 | 1.00 | 19.94 |
| ATOM | 963 | O | PHE | A | 137 | 8.690 | 9.789 | 8.447 | 1.00 | 22.12 |
| ATOM | 964 | N | ILE | A | 138 | 8.826 | 11.583 | 9.771 | 1.00 | 19.99 |
| ATOM | 965 | CA | ILE | A | 138 | 7.897 | 11.041 | 10.739 | 1.00 | 22.91 |
| ATOM | 966 | CB | ILE | A | 138 | 8.055 | 11.729 | 12.115 | 1.00 | 21.86 |
| ATOM | 967 | CG2 | ILE | A | 138 | 7.196 | 11.034 | 13.164 | 1.00 | 21.20 |
| ATOM | 968 | CG1 | ILE | A | 138 | 9.522 | 11.680 | 12.550 | 1.00 | 21.47 |
| ATOM | 969 | CD | ILE | A | 138 | 10.147 | 10.286 | 12.533 | 1.00 | 18.73 |
| ATOM | 970 | C | ILE | A | 138 | 6.486 | 11.258 | 10.197 | 1.00 | 26.84 |
| ATOM | 971 | O | ILE | A | 138 | 5.963 | 12.379 | 10.211 | 1.00 | 27.99 |
| ATOM | 972 | N | GLY | A | 139 | 5.894 | 10.174 | 9.689 | 1.00 | 29.61 |
| ATOM | 973 | CA | GLY | A | 139 | 4.552 | 10.223 | 9.127 | 1.00 | 30.82 |
| ATOM | 974 | C | GLY | A | 139 | 3.480 | 9.799 | 10.115 | 1.00 | 32.28 |
| ATOM | 975 | O | GLY | A | 139 | 3.527 | 10.199 | 11.279 | 1.00 | 33.02 |
| ATOM | 976 | N | SER | A | 140 | 2.522 | 8.989 | 9.659 | 1.00 | 32.42 |
| ATOM | 977 | CA | SER | A | 140 | 1.425 | 8.520 | 10.515 | 1.00 | 33.61 |
| ATOM | 978 | CB | SER | A | 140 | 0.531 | 9.705 | 10.918 | 1.00 | 36.01 |
| ATOM | 979 | OG | SER | A | 140 | -0.544 | 9.311 | 11.761 | 1.00 | 37.45 |
| ATOM | 980 | C | SER | A | 140 | 0.559 | 7.440 | 9.847 | 1.00 | 33.81 |
| ATOM | 981 | O | SER | A | 140 | 0.426 | 7.386 | 8.622 | 1.00 | 31.26 |
| ATOM | 982 | N | VAL | A | 141 | -0.031 | 6.583 | 10.672 | 1.00 | 35.86 |
| ATOM | 983 | CA | VAL | A | 141 | -0.888 | 5.511 | 10.185 | 1.00 | 38.62 |
| ATOM | 984 | CB | VAL | A | 141 | -1.478 | 4.694 | 11.359 | 1.00 | 39.84 |
| ATOM | 985 | CG1 | VAL | A | 141 | -2.384 | 3.597 | 10.830 | 1.00 | 39.43 |

Figure 3 (suite 13)

| | | | | | | | | | | |
|---|---|---|---|---|---|---|---|---|---|---|
| ATOM | 986 | CG2 | VAL | A | 141 | -0.353 | 4.093 | 12.182 | 1.00 | 41.97 |
| ATOM | 987 | C | VAL | A | 141 | -2.043 | 6.070 | 9.354 | 1.00 | 39.46 |
| ATOM | 988 | O | VAL | A | 141 | -2.302 | 5.604 | 8.237 | 1.00 | 38.42 |
| ATOM | 989 | N | SER | A | 142 | -2.722 | 7.079 | 9.901 | 1.00 | 40.24 |
| ATOM | 990 | CA | SER | A | 142 | -3.864 | 7.701 | 9.238 | 1.00 | 42.18 |
| ATOM | 991 | CB | SER | A | 142 | -4.239 | 9.014 | 9.933 | 1.00 | 41.50 |
| ATOM | 992 | OG | SER | A | 142 | -3.351 | 10.058 | 9.580 | 1.00 | 42.24 |
| ATOM | 993 | C | SER | A | 142 | -3.607 | 7.968 | 7.763 | 1.00 | 41.83 |
| ATOM | 994 | O | SER | A | 142 | -4.536 | 7.951 | 6.955 | 1.00 | 44.02 |
| ATOM | 995 | N | GLY | A | 143 | -2.349 | 8.216 | 7.415 | 1.00 | 41.59 |
| ATOM | 996 | CA | GLY | A | 143 | -2.000 | 8.480 | 6.029 | 1.00 | 41.28 |
| ATOM | 997 | C | GLY | A | 143 | -2.609 | 7.492 | 5.047 | 1.00 | 39.84 |
| ATOM | 998 | O | GLY | A | 143 | -3.229 | 7.890 | 4.060 | 1.00 | 39.96 |
| ATOM | 999 | N | LEU | A | 144 | -2.441 | 6.199 | 5.316 | 1.00 | 39.86 |
| ATOM | 1000 | CA | LEU | A | 144 | -2.975 | 5.160 | 4.424 | 1.00 | 39.91 |
| ATOM | 1001 | CB | LEU | A | 144 | -1.852 | 4.229 | 3.968 | 1.00 | 38.97 |
| ATOM | 1002 | CG | LEU | A | 144 | -0.824 | 4.895 | 3.057 | 1.00 | 38.18 |
| ATOM | 1003 | CD1 | LEU | A | 144 | 0.332 | 3.943 | 2.773 | 1.00 | 39.19 |
| ATOM | 1004 | CD2 | LEU | A | 144 | -1.499 | 5.303 | 1.767 | 1.00 | 39.05 |
| ATOM | 1005 | C | LEU | A | 144 | -4.080 | 4.336 | 5.067 | 1.00 | 41.06 |
| ATOM | 1006 | O | LEU | A | 144 | -4.678 | 3.465 | 4.416 | 1.00 | 40.52 |
| ATOM | 1007 | N | TRP | A | 145 | -4.354 | 4.612 | 6.337 | 1.00 | 41.51 |
| ATOM | 1008 | CA | TRP | A | 145 | -5.393 | 3.900 | 7.068 | 1.00 | 42.99 |
| ATOM | 1009 | CB | TRP | A | 145 | -4.964 | 3.722 | 8.531 | 1.00 | 45.40 |
| ATOM | 1010 | CG | TRP | A | 145 | -5.896 | 2.868 | 9.344 | 1.00 | 49.29 |
| ATOM | 1011 | CD2 | TRP | A | 145 | -6.382 | 3.144 | 10.666 | 1.00 | 51.54 |
| ATOM | 1012 | CE2 | TRP | A | 145 | -7.186 | 2.046 | 11.052 | 1.00 | 51.71 |
| ATOM | 1013 | CE3 | TRP | A | 145 | -6.218 | 4.213 | 11.561 | 1.00 | 53.30 |
| ATOM | 1014 | CD1 | TRP | A | 145 | -6.409 | 1.651 | 8.994 | 1.00 | 49.19 |
| ATOM | 1015 | NE1 | TRP | A | 145 | -7.182 | 1.152 | 10.014 | 1.00 | 50.75 |
| ATOM | 1016 | CZ2 | TRP | A | 145 | -7.821 | 1.982 | 12.299 | 1.00 | 53.37 |
| ATOM | 1017 | CZ3 | TRP | A | 145 | -6.849 | 4.151 | 12.804 | 1.00 | 53.08 |
| ATOM | 1018 | CH2 | TRP | A | 145 | -7.643 | 3.042 | 13.159 | 1.00 | 55.08 |
| ATOM | 1019 | C | TRP | A | 145 | -6.710 | 4.672 | 6.987 | 1.00 | 43.02 |
| ATOM | 1020 | O | TRP | A | 145 | -7.792 | 4.085 | 7.022 | 1.00 | 41.30 |
| ATOM | 1021 | N | GLY | A | 146 | -6.604 | 5.994 | 6.877 | 1.00 | 43.95 |
| ATOM | 1022 | CA | GLY | A | 146 | -7.786 | 6.833 | 6.794 | 1.00 | 45.52 |
| ATOM | 1023 | C | GLY | A | 146 | -8.507 | 6.981 | 8.121 | 1.00 | 46.22 |
| ATOM | 1024 | O | GLY | A | 146 | -9.238 | 6.087 | 8.543 | 1.00 | 46.34 |
| ATOM | 1025 | N | ILE | A | 147 | -8.286 | 8.104 | 8.798 | 1.00 | 47.46 |
| ATOM | 1026 | CA | ILE | A | 147 | -8.942 | 8.363 | 10.076 | 1.00 | 47.62 |
| ATOM | 1027 | CB | ILE | A | 147 | -7.940 | 8.839 | 11.166 | 1.00 | 48.36 |
| ATOM | 1028 | CG2 | ILE | A | 147 | -8.690 | 9.204 | 12.448 | 1.00 | 48.02 |
| ATOM | 1029 | CG1 | ILE | A | 147 | -6.923 | 7.739 | 11.464 | 1.00 | 46.70 |
| ATOM | 1030 | CD | ILE | A | 147 | -5.994 | 8.071 | 12.609 | 1.00 | 46.42 |
| ATOM | 1031 | C | ILE | A | 147 | -9.979 | 9.455 | 9.852 | 1.00 | 47.46 |
| ATOM | 1032 | O | ILE | A | 147 | -9.780 | 10.351 | 9.028 | 1.00 | 47.48 |
| ATOM | 1033 | N | GLY | A | 148 | -11.088 | 9.370 | 10.578 | 1.00 | 47.57 |
| ATOM | 1034 | CA | GLY | A | 148 | -12.135 | 10.363 | 10.435 | 1.00 | 48.17 |
| ATOM | 1035 | C | GLY | A | 148 | -11.697 | 11.742 | 10.890 | 1.00 | 47.88 |
| ATOM | 1036 | O | GLY | A | 148 | -10.921 | 11.885 | 11.839 | 1.00 | 48.54 |
| ATOM | 1037 | N | ASN | A | 149 | -12.182 | 12.762 | 10.192 | 1.00 | 47.41 |
| ATOM | 1038 | CA | ASN | A | 149 | -11.872 | 14.146 | 10.527 | 1.00 | 46.83 |
| ATOM | 1039 | CB | ASN | A | 149 | -12.430 | 14.473 | 11.915 | 1.00 | 48.25 |
| ATOM | 1040 | CG | ASN | A | 149 | -13.907 | 14.124 | 12.051 | 1.00 | 50.79 |
| ATOM | 1041 | OD1 | ASN | A | 149 | -14.461 | 14.153 | 13.152 | 1.00 | 52.44 |
| ATOM | 1042 | ND2 | ASN | A | 149 | -14.551 | 13.794 | 10.931 | 1.00 | 51.06 |
| ATOM | 1043 | C | ASN | A | 149 | -10.373 | 14.439 | 10.488 | 1.00 | 45.62 |
| ATOM | 1044 | O | ASN | A | 149 | -9.815 | 14.994 | 11.434 | 1.00 | 46.56 |
| ATOM | 1045 | N | GLN | A | 150 | -9.725 | 14.068 | 9.390 | 1.00 | 43.23 |
| ATOM | 1046 | CA | GLN | A | 150 | -8.297 | 14.306 | 9.234 | 1.00 | 40.59 |
| ATOM | 1047 | CB | GLN | A | 150 | -7.483 | 13.144 | 9.814 | 1.00 | 43.17 |
| ATOM | 1048 | CG | GLN | A | 150 | -7.361 | 13.141 | 11.338 | 1.00 | 46.54 |
| ATOM | 1049 | CD | GLN | A | 150 | -6.308 | 12.165 | 11.847 | 1.00 | 47.88 |
| ATOM | 1050 | OE1 | GLN | A | 150 | -5.943 | 12.183 | 13.027 | 1.00 | 49.38 |
| ATOM | 1051 | NE2 | GLN | A | 150 | -5.820 | 11.306 | 10.960 | 1.00 | 48.24 |
| ATOM | 1052 | C | GLN | A | 150 | -7.929 | 14.484 | 7.777 | 1.00 | 37.61 |
| ATOM | 1053 | O | GLN | A | 150 | -6.804 | 14.195 | 7.387 | 1.00 | 37.52 |
| ATOM | 1054 | N | ALA | A | 151 | -8.869 | 14.969 | 6.972 | 1.00 | 33.43 |
| ATOM | 1055 | CA | ALA | A | 151 | -8.609 | 15.150 | 5.554 | 1.00 | 30.22 |
| ATOM | 1056 | CB | ALA | A | 151 | -9.801 | 15.789 | 4.885 | 1.00 | 29.22 |
| ATOM | 1057 | C | ALA | A | 151 | -7.355 | 15.977 | 5.275 | 1.00 | 28.29 |
| ATOM | 1058 | O | ALA | A | 151 | -6.734 | 15.837 | 4.221 | 1.00 | 29.90 |

Figure 3 (suite 14)

| | | | | | | | | | | |
|---|---|---|---|---|---|---|---|---|---|---|
| ATOM | 1059 | N | ASN | A | 152 | -6.986 | 16.850 | 6.205 | 1.00 | 24.84 |
| ATOM | 1060 | CA | ASN | A | 152 | -5.800 | 17.675 | 6.004 | 1.00 | 23.47 |
| ATOM | 1061 | CB | ASN | A | 152 | -5.952 | 19.032 | 6.707 | 1.00 | 24.18 |
| ATOM | 1062 | CG | ASN | A | 152 | -6.295 | 18.899 | 8.172 | 1.00 | 25.01 |
| ATOM | 1063 | OD1 | ASN | A | 152 | -6.047 | 19.808 | 8.965 | 1.00 | 29.17 |
| ATOM | 1064 | ND2 | ASN | A | 152 | -6.879 | 17.772 | 8.542 | 1.00 | 24.68 |
| ATOM | 1065 | C | ASN | A | 152 | -4.556 | 16.948 | 6.509 | 1.00 | 22.55 |
| ATOM | 1066 | O | ASN | A | 152 | -3.557 | 16.861 | 5.808 | 1.00 | 24.15 |
| ATOM | 1067 | N | TYR | A | 153 | -4.627 | 16.419 | 7.723 | 1.00 | 22.54 |
| ATOM | 1068 | CA | TYR | A | 153 | -3.509 | 15.681 | 8.301 | 1.00 | 23.84 |
| ATOM | 1069 | CB | TYR | A | 153 | -3.877 | 15.222 | 9.708 | 1.00 | 26.11 |
| ATOM | 1070 | CG | TYR | A | 153 | -2.712 | 14.749 | 10.529 | 1.00 | 32.82 |
| ATOM | 1071 | CD1 | TYR | A | 153 | -1.798 | 15.659 | 11.060 | 1.00 | 36.43 |
| ATOM | 1072 | CE1 | TYR | A | 153 | -0.724 | 15.229 | 11.845 | 1.00 | 39.09 |
| ATOM | 1073 | CD2 | TYR | A | 153 | -2.525 | 13.385 | 10.797 | 1.00 | 35.67 |
| ATOM | 1074 | CE2 | TYR | A | 153 | -1.456 | 12.941 | 11.578 | 1.00 | 39.17 |
| ATOM | 1075 | CZ | TYR | A | 153 | -0.557 | 13.871 | 12.103 | 1.00 | 41.16 |
| ATOM | 1076 | OH | TYR | A | 153 | 0.498 | 13.449 | 12.890 | 1.00 | 42.42 |
| ATOM | 1077 | C | TYR | A | 153 | -3.218 | 14.469 | 7.400 | 1.00 | 20.95 |
| ATOM | 1078 | O | TYR | A | 153 | -2.138 | 14.346 | 6.829 | 1.00 | 21.62 |
| ATOM | 1079 | N | ALA | A | 154 | -4.197 | 13.587 | 7.259 | 1.00 | 19.92 |
| ATOM | 1080 | CA | ALA | A | 154 | -4.044 | 12.403 | 6.415 | 1.00 | 20.01 |
| ATOM | 1081 | CB | ALA | A | 154 | -5.402 | 11.730 | 6.197 | 1.00 | 16.82 |
| ATOM | 1082 | C | ALA | A | 154 | -3.429 | 12.753 | 5.073 | 1.00 | 19.37 |
| ATOM | 1083 | O | ALA | A | 154 | -2.481 | 12.106 | 4.629 | 1.00 | 21.06 |
| ATOM | 1084 | N | ALA | A | 155 | -3.969 | 13.771 | 4.416 | 1.00 | 19.59 |
| ATOM | 1085 | CA | ALA | A | 155 | -3.442 | 14.162 | 3.117 | 1.00 | 18.09 |
| ATOM | 1086 | CB | ALA | A | 155 | -4.263 | 15.302 | 2.528 | 1.00 | 17.02 |
| ATOM | 1087 | C | ALA | A | 155 | -1.970 | 14.562 | 3.220 | 1.00 | 18.22 |
| ATOM | 1088 | O | ALA | A | 155 | -1.214 | 14.403 | 2.254 | 1.00 | 16.36 |
| ATOM | 1089 | N | SER | A | 156 | -1.568 | 15.067 | 4.387 | 1.00 | 18.09 |
| ATOM | 1090 | CA | SER | A | 156 | -0.184 | 15.482 | 4.599 | 1.00 | 19.29 |
| ATOM | 1091 | CB | SER | A | 156 | -0.086 | 16.497 | 5.761 | 1.00 | 21.03 |
| ATOM | 1092 | OG | SER | A | 156 | -0.110 | 15.870 | 7.037 | 1.00 | 21.08 |
| ATOM | 1093 | C | SER | A | 156 | 0.709 | 14.273 | 4.875 | 1.00 | 18.77 |
| ATOM | 1094 | O | SER | A | 156 | 1.774 | 14.132 | 4.279 | 1.00 | 19.85 |
| ATOM | 1095 | N | LYS | A | 157 | 0.268 | 13.396 | 5.768 | 1.00 | 20.27 |
| ATOM | 1096 | CA | LYS | A | 157 | 1.046 | 12.204 | 6.105 | 1.00 | 20.77 |
| ATOM | 1097 | CB | LYS | A | 157 | 0.381 | 11.439 | 7.238 | 1.00 | 21.14 |
| ATOM | 1098 | CG | LYS | A | 157 | 0.117 | 12.245 | 8.489 | 1.00 | 27.34 |
| ATOM | 1099 | CD | LYS | A | 157 | 1.392 | 12.618 | 9.261 | 1.00 | 28.75 |
| ATOM | 1100 | CE | LYS | A | 157 | 2.178 | 13.743 | 8.596 | 1.00 | 27.60 |
| ATOM | 1101 | NZ | LYS | A | 157 | 3.212 | 14.295 | 9.525 | 1.00 | 31.18 |
| ATOM | 1102 | C | LYS | A | 157 | 1.196 | 11.291 | 4.891 | 1.00 | 21.85 |
| ATOM | 1103 | O | LYS | A | 157 | 2.234 | 10.655 | 4.719 | 1.00 | 20.90 |
| ATOM | 1104 | N | ALA | A | 158 | 0.165 | 11.243 | 4.046 | 1.00 | 21.00 |
| ATOM | 1105 | CA | ALA | A | 158 | 0.195 | 10.410 | 2.845 | 1.00 | 21.20 |
| ATOM | 1106 | CB | ALA | A | 158 | -1.234 | 10.184 | 2.316 | 1.00 | 18.89 |
| ATOM | 1107 | C | ALA | A | 158 | 1.051 | 11.072 | 1.778 | 1.00 | 20.96 |
| ATOM | 1108 | O | ALA | A | 158 | 1.673 | 10.391 | 0.946 | 1.00 | 20.64 |
| ATOM | 1109 | N | GLY | A | 159 | 1.070 | 12.406 | 1.804 | 1.00 | 19.85 |
| ATOM | 1110 | CA | GLY | A | 159 | 1.861 | 13.161 | 0.847 | 1.00 | 20.29 |
| ATOM | 1111 | C | GLY | A | 159 | 3.351 | 13.005 | 1.100 | 1.00 | 20.62 |
| ATOM | 1112 | O | GLY | A | 159 | 4.159 | 13.037 | 0.175 | 1.00 | 19.85 |
| ATOM | 1113 | N | VAL | A | 160 | 3.722 | 12.834 | 2.361 | 1.00 | 21.63 |
| ATOM | 1114 | CA | VAL | A | 160 | 5.125 | 12.666 | 2.705 | 1.00 | 21.98 |
| ATOM | 1115 | CB | VAL | A | 160 | 5.334 | 12.727 | 4.231 | 1.00 | 23.14 |
| ATOM | 1116 | CG1 | VAL | A | 160 | 6.791 | 12.494 | 4.558 | 1.00 | 22.15 |
| ATOM | 1117 | CG2 | VAL | A | 160 | 4.902 | 14.093 | 4.762 | 1.00 | 20.32 |
| ATOM | 1118 | C | VAL | A | 160 | 5.636 | 11.334 | 2.165 | 1.00 | 22.41 |
| ATOM | 1119 | O | VAL | A | 160 | 6.770 | 11.241 | 1.683 | 1.00 | 23.31 |
| ATOM | 1120 | N | ILE | A | 161 | 4.788 | 10.305 | 2.234 | 1.00 | 22.52 |
| ATOM | 1121 | CA | ILE | A | 161 | 5.147 | 8.982 | 1.732 | 1.00 | 20.14 |
| ATOM | 1122 | CB | ILE | A | 161 | 3.984 | 7.960 | 1.912 | 1.00 | 20.94 |
| ATOM | 1123 | CG2 | ILE | A | 161 | 4.354 | 6.616 | 1.311 | 1.00 | 19.56 |
| ATOM | 1124 | CG1 | ILE | A | 161 | 3.675 | 7.771 | 3.391 | 1.00 | 23.35 |
| ATOM | 1125 | CD | ILE | A | 161 | 2.507 | 6.804 | 3.645 | 1.00 | 23.63 |
| ATOM | 1126 | C | ILE | A | 161 | 5.469 | 9.104 | 0.252 | 1.00 | 18.82 |
| ATOM | 1127 | O | ILE | A | 161 | 6.511 | 8.651 | -0.206 | 1.00 | 18.23 |
| ATOM | 1128 | N | GLY | A | 162 | 4.564 | 9.721 | -0.500 | 1.00 | 19.40 |
| ATOM | 1129 | CA | GLY | A | 162 | 4.792 | 9.896 | -1.926 | 1.00 | 19.06 |
| ATOM | 1130 | C | GLY | A | 162 | 6.021 | 10.737 | -2.241 | 1.00 | 19.58 |
| ATOM | 1131 | O | GLY | A | 162 | 6.739 | 10.468 | -3.218 | 1.00 | 17.54 |

Figure 3 (suite 15)

| | | | | | | | | | | |
|---|---|---|---|---|---|---|---|---|---|---|
| ATOM | 1132 | N | MET | A | 163 | 6.271 | 11.765 | -1.434 | 1.00 | 19.24 |
| ATOM | 1133 | CA | MET | A | 163 | 7.443 | 12.611 | -1.673 | 1.00 | 21.65 |
| ATOM | 1134 | CB | MET | A | 163 | 7.418 | 13.857 | -0.774 | 1.00 | 21.13 |
| ATOM | 1135 | CG | MET | A | 163 | 8.606 | 14.806 | -1.018 | 1.00 | 23.13 |
| ATOM | 1136 | SD | MET | A | 163 | 8.619 | 16.254 | 0.071 | 1.00 | 24.77 |
| ATOM | 1137 | CE | MET | A | 163 | 9.382 | 15.583 | 1.535 | 1.00 | 28.40 |
| ATOM | 1138 | C | MET | A | 163 | 8.724 | 11.810 | -1.411 | 1.00 | 23.01 |
| ATOM | 1139 | O | MET | A | 163 | 9.647 | 11.796 | -2.232 | 1.00 | 21.16 |
| ATOM | 1140 | N | ALA | A | 164 | 8.762 | 11.140 | -0.262 | 1.00 | 24.55 |
| ATOM | 1141 | CA | ALA | A | 164 | 9.912 | 10.330 | 0.116 | 1.00 | 25.27 |
| ATOM | 1142 | CB | ALA | A | 164 | 9.650 | 9.652 | 1.438 | 1.00 | 25.88 |
| ATOM | 1143 | C | ALA | A | 164 | 10.239 | 9.289 | -0.945 | 1.00 | 25.94 |
| ATOM | 1144 | O | ALA | A | 164 | 11.405 | 9.121 | -1.325 | 1.00 | 28.99 |
| ATOM | 1145 | N | ARG | A | 165 | 9.222 | 8.600 | -1.451 | 1.00 | 25.40 |
| ATOM | 1146 | CA | ARG | A | 165 | 9.480 | 7.577 | -2.452 | 1.00 | 26.17 |
| ATOM | 1147 | CB | ARG | A | 165 | 8.222 | 6.730 | -2.714 | 1.00 | 26.36 |
| ATOM | 1148 | CG | ARG | A | 165 | 7.884 | 5.782 | -1.545 | 1.00 | 24.95 |
| ATOM | 1149 | CD | ARG | A | 165 | 6.869 | 4.693 | -1.922 | 1.00 | 23.82 |
| ATOM | 1150 | NE | ARG | A | 165 | 6.627 | 3.786 | -0.797 | 1.00 | 26.72 |
| ATOM | 1151 | CZ | ARG | A | 165 | 5.719 | 2.807 | -0.777 | 1.00 | 27.93 |
| ATOM | 1152 | NH1 | ARG | A | 165 | 4.939 | 2.583 | -1.832 | 1.00 | 25.86 |
| ATOM | 1153 | NH2 | ARG | A | 165 | 5.587 | 2.048 | 0.311 | 1.00 | 24.81 |
| ATOM | 1154 | C | ARG | A | 165 | 10.022 | 8.152 | -3.742 | 1.00 | 26.76 |
| ATOM | 1155 | O | ARG | A | 165 | 10.815 | 7.499 | -4.433 | 1.00 | 27.40 |
| ATOM | 1156 | N | SER | A | 166 | 9.607 | 9.372 | -4.074 | 1.00 | 26.41 |
| ATOM | 1157 | CA | SER | A | 166 | 10.096 | 10.010 | -5.291 | 1.00 | 26.44 |
| ATOM | 1158 | CB | SER | A | 166 | 9.315 | 11.293 | -5.599 | 1.00 | 26.07 |
| ATOM | 1159 | OG | SER | A | 166 | 9.808 | 11.894 | -6.786 | 1.00 | 22.81 |
| ATOM | 1160 | C | SER | A | 166 | 11.570 | 10.339 | -5.068 | 1.00 | 27.17 |
| ATOM | 1161 | O | SER | A | 166 | 12.405 | 10.131 | -5.953 | 1.00 | 28.03 |
| ATOM | 1162 | N | ILE | A | 167 | 11.880 | 10.859 | -3.883 | 1.00 | 25.55 |
| ATOM | 1163 | CA | ILE | A | 167 | 13.255 | 11.180 | -3.533 | 1.00 | 26.35 |
| ATOM | 1164 | CB | ILE | A | 167 | 13.365 | 11.752 | -2.102 | 1.00 | 25.69 |
| ATOM | 1165 | CG2 | ILE | A | 167 | 14.830 | 11.918 | -1.727 | 1.00 | 24.10 |
| ATOM | 1166 | CG1 | ILE | A | 167 | 12.604 | 13.074 | -2.004 | 1.00 | 26.96 |
| ATOM | 1167 | CD | ILE | A | 167 | 12.692 | 13.737 | -0.653 | 1.00 | 26.77 |
| ATOM | 1168 | C | ILE | A | 167 | 14.055 | 9.878 | -3.577 | 1.00 | 27.35 |
| ATOM | 1169 | O | ILE | A | 167 | 15.113 | 9.806 | -4.197 | 1.00 | 28.74 |
| ATOM | 1170 | N | ALA | A | 168 | 13.539 | 8.847 | -2.915 | 1.00 | 26.34 |
| ATOM | 1171 | CA | ALA | A | 168 | 14.219 | 7.550 | -2.885 | 1.00 | 26.52 |
| ATOM | 1172 | CB | ALA | A | 168 | 13.327 | 6.497 | -2.230 | 1.00 | 23.43 |
| ATOM | 1173 | C | ALA | A | 168 | 14.587 | 7.106 | -4.281 | 1.00 | 25.94 |
| ATOM | 1174 | O | ALA | A | 168 | 15.721 | 6.698 | -4.536 | 1.00 | 23.36 |
| ATOM | 1175 | N | ARG | A | 169 | 13.626 | 7.219 | -5.193 | 1.00 | 28.04 |
| ATOM | 1176 | CA | ARG | A | 169 | 13.826 | 6.795 | -6.572 | 1.00 | 30.43 |
| ATOM | 1177 | CB | ARG | A | 169 | 12.502 | 6.841 | -7.334 | 1.00 | 32.56 |
| ATOM | 1178 | CG | ARG | A | 169 | 12.453 | 5.904 | -8.546 | 1.00 | 37.06 |
| ATOM | 1179 | CD | ARG | A | 169 | 11.598 | 4.679 | -8.232 | 1.00 | 38.45 |
| ATOM | 1180 | NE | ARG | A | 169 | 11.600 | 3.689 | -9.304 | 1.00 | 41.17 |
| ATOM | 1181 | CZ | ARG | A | 169 | 10.836 | 2.598 | -9.320 | 1.00 | 42.22 |
| ATOM | 1182 | NH1 | ARG | A | 169 | 9.992 | 2.347 | -8.324 | 1.00 | 40.64 |
| ATOM | 1183 | NH2 | ARG | A | 169 | 10.927 | 1.745 | -10.333 | 1.00 | 43.83 |
| ATOM | 1184 | C | ARG | A | 169 | 14.855 | 7.631 | -7.321 | 1.00 | 31.19 |
| ATOM | 1185 | O | ARG | A | 169 | 15.312 | 7.250 | -8.401 | 1.00 | 31.61 |
| ATOM | 1186 | N | GLU | A | 170 | 15.227 | 8.767 | -6.749 | 1.00 | 31.09 |
| ATOM | 1187 | CA | GLU | A | 170 | 16.179 | 9.638 | -7.409 | 1.00 | 32.53 |
| ATOM | 1188 | CB | GLU | A | 170 | 15.619 | 11.064 | -7.463 | 1.00 | 33.83 |
| ATOM | 1189 | CG | GLU | A | 170 | 16.592 | 12.086 | -8.017 | 1.00 | 38.07 |
| ATOM | 1190 | CD | GLU | A | 170 | 16.080 | 13.505 | -7.898 | 1.00 | 40.66 |
| ATOM | 1191 | OE1 | GLU | A | 170 | 16.921 | 14.431 | -7.928 | 1.00 | 41.97 |
| ATOM | 1192 | OE2 | GLU | A | 170 | 14.849 | 13.694 | -7.783 | 1.00 | 43.13 |
| ATOM | 1193 | C | GLU | A | 170 | 17.574 | 9.667 | -6.790 | 1.00 | 31.99 |
| ATOM | 1194 | O | GLU | A | 170 | 18.569 | 9.552 | -7.503 | 1.00 | 32.11 |
| ATOM | 1195 | N | LEU | A | 171 | 17.643 | 9.815 | -5.472 | 1.00 | 30.26 |
| ATOM | 1196 | CA | LEU | A | 171 | 18.928 | 9.913 | -4.787 | 1.00 | 30.59 |
| ATOM | 1197 | CB | LEU | A | 171 | 18.831 | 10.992 | -3.704 | 1.00 | 31.27 |
| ATOM | 1198 | CG | LEU | A | 171 | 18.249 | 12.346 | -4.131 | 1.00 | 30.94 |
| ATOM | 1199 | CD1 | LEU | A | 171 | 18.041 | 13.215 | -2.900 | 1.00 | 30.69 |
| ATOM | 1200 | CD2 | LEU | A | 171 | 19.173 | 13.025 | -5.128 | 1.00 | 27.03 |
| ATOM | 1201 | C | LEU | A | 171 | 19.505 | 8.629 | -4.162 | 1.00 | 30.02 |
| ATOM | 1202 | O | LEU | A | 171 | 20.542 | 8.686 | -3.495 | 1.00 | 28.65 |
| ATOM | 1203 | N | SER | A | 172 | 18.856 | 7.484 | -4.367 | 1.00 | 29.58 |
| ATOM | 1204 | CA | SER | A | 172 | 19.352 | 6.233 | -3.783 | 1.00 | 30.31 |

Figure 3 (suite 16)

| | | | | | | | | | | |
|---|---|---|---|---|---|---|---|---|---|---|
| ATOM | 1205 | CB | SER | A | 172 | 18.284 | 5.143 | -3.863 | 1.00 | 28.89 |
| ATOM | 1206 | OG | SER | A | 172 | 17.317 | 5.319 | -2.840 | 1.00 | 29.41 |
| ATOM | 1207 | C | SER | A | 172 | 20.645 | 5.767 | -4.445 | 1.00 | 30.68 |
| ATOM | 1208 | O | SER | A | 172 | 21.489 | 5.144 | -3.805 | 1.00 | 32.87 |
| ATOM | 1209 | N | LYS | A | 173 | 20.795 | 6.082 | -5.725 | 1.00 | 31.39 |
| ATOM | 1210 | CA | LYS | A | 173 | 21.995 | 5.738 | -6.482 | 1.00 | 30.87 |
| ATOM | 1211 | CB | LYS | A | 173 | 21.797 | 6.063 | -7.969 | 1.00 | 31.77 |
| ATOM | 1212 | CG | LYS | A | 173 | 21.509 | 7.552 | -8.232 | 1.00 | 32.44 |
| ATOM | 1213 | CD | LYS | A | 173 | 21.553 | 7.906 | -9.712 | 1.00 | 33.80 |
| ATOM | 1214 | CE | LYS | A | 173 | 20.374 | 7.326 | -10.484 | 1.00 | 35.52 |
| ATOM | 1215 | NZ | LYS | A | 173 | 19.054 | 7.867 | -10.024 | 1.00 | 36.38 |
| ATOM | 1216 | C | LYS | A | 173 | 23.171 | 6.569 | -5.947 | 1.00 | 29.06 |
| ATOM | 1217 | O | LYS | A | 173 | 24.301 | 6.420 | -6.396 | 1.00 | 29.26 |
| ATOM | 1218 | N | ALA | A | 174 | 22.894 | 7.447 | -4.992 | 1.00 | 28.23 |
| ATOM | 1219 | CA | ALA | A | 174 | 23.925 | 8.303 | -4.412 | 1.00 | 27.55 |
| ATOM | 1220 | CB | ALA | A | 174 | 23.573 | 9.769 | -4.634 | 1.00 | 27.05 |
| ATOM | 1221 | C | ALA | A | 174 | 24.063 | 8.029 | -2.929 | 1.00 | 25.75 |
| ATOM | 1222 | O | ALA | A | 174 | 24.635 | 8.836 | -2.190 | 1.00 | 26.19 |
| ATOM | 1223 | N | ASN | A | 175 | 23.524 | 6.894 | -2.500 | 1.00 | 26.35 |
| ATOM | 1224 | CA | ASN | A | 175 | 23.571 | 6.490 | -1.103 | 1.00 | 26.62 |
| ATOM | 1225 | CB | ASN | A | 175 | 25.033 | 6.319 | -0.657 | 1.00 | 28.12 |
| ATOM | 1226 | CG | ASN | A | 175 | 25.160 | 5.785 | 0.759 | 1.00 | 31.80 |
| ATOM | 1227 | OD1 | ASN | A | 175 | 24.327 | 4.995 | 1.223 | 1.00 | 32.23 |
| ATOM | 1228 | ND2 | ASN | A | 175 | 26.211 | 6.207 | 1.456 | 1.00 | 30.18 |
| ATOM | 1229 | C | ASN | A | 175 | 22.821 | 7.501 | -0.236 | 1.00 | 26.14 |
| ATOM | 1230 | O | ASN | A | 175 | 23.154 | 7.731 | 0.928 | 1.00 | 26.45 |
| ATOM | 1231 | N | VAL | A | 176 | 21.800 | 8.111 | -0.828 | 1.00 | 25.76 |
| ATOM | 1232 | CA | VAL | A | 176 | 20.944 | 9.062 | -0.117 | 1.00 | 25.20 |
| ATOM | 1233 | CB | VAL | A | 176 | 20.906 | 10.448 | -0.818 | 1.00 | 26.27 |
| ATOM | 1234 | CG1 | VAL | A | 176 | 19.980 | 11.391 | -0.050 | 1.00 | 26.07 |
| ATOM | 1235 | CG2 | VAL | A | 176 | 22.307 | 11.036 | -0.877 | 1.00 | 24.73 |
| ATOM | 1236 | C | VAL | A | 176 | 19.550 | 8.433 | -0.143 | 1.00 | 24.81 |
| ATOM | 1237 | O | VAL | A | 176 | 18.899 | 8.359 | -1.194 | 1.00 | 22.12 |
| ATOM | 1238 | N | THR | A | 177 | 19.106 | 7.964 | 1.016 | 1.00 | 24.50 |
| ATOM | 1239 | CA | THR | A | 177 | 17.819 | 7.291 | 1.127 | 1.00 | 22.56 |
| ATOM | 1240 | CB | THR | A | 177 | 17.978 | 5.935 | 1.851 | 1.00 | 23.81 |
| ATOM | 1241 | OG1 | THR | A | 177 | 18.303 | 6.158 | 3.231 | 1.00 | 22.55 |
| ATOM | 1242 | CG2 | THR | A | 177 | 19.094 | 5.122 | 1.206 | 1.00 | 23.39 |
| ATOM | 1243 | C | THR | A | 177 | 16.743 | 8.094 | 1.848 | 1.00 | 22.34 |
| ATOM | 1244 | O | THR | A | 177 | 17.044 | 8.955 | 2.684 | 1.00 | 22.71 |
| ATOM | 1245 | N | ALA | A | 178 | 15.489 | 7.787 | 1.512 | 1.00 | 20.79 |
| ATOM | 1246 | CA | ALA | A | 178 | 14.319 | 8.435 | 2.101 | 1.00 | 21.24 |
| ATOM | 1247 | CB | ALA | A | 178 | 13.638 | 9.333 | 1.061 | 1.00 | 20.55 |
| ATOM | 1248 | C | ALA | A | 178 | 13.318 | 7.406 | 2.624 | 1.00 | 20.98 |
| ATOM | 1249 | O | ALA | A | 178 | 12.769 | 6.613 | 1.860 | 1.00 | 20.50 |
| ATOM | 1250 | N | ASN | A | 179 | 13.070 | 7.428 | 3.926 | 1.00 | 20.46 |
| ATOM | 1251 | CA | ASN | A | 179 | 12.129 | 6.487 | 4.521 | 1.00 | 19.71 |
| ATOM | 1252 | CB | ASN | A | 179 | 12.879 | 5.395 | 5.312 | 1.00 | 17.07 |
| ATOM | 1253 | CG | ASN | A | 179 | 13.804 | 4.556 | 4.427 | 1.00 | 17.63 |
| ATOM | 1254 | OD1 | ASN | A | 179 | 13.364 | 3.932 | 3.464 | 1.00 | 17.09 |
| ATOM | 1255 | ND2 | ASN | A | 179 | 15.092 | 4.549 | 4.754 | 1.00 | 16.62 |
| ATOM | 1256 | C | ASN | A | 179 | 11.159 | 7.206 | 5.440 | 1.00 | 19.41 |
| ATOM | 1257 | O | ASN | A | 179 | 11.459 | 8.280 | 5.969 | 1.00 | 19.68 |
| ATOM | 1258 | N | VAL | A | 180 | 10.001 | 6.592 | 5.635 | 1.00 | 20.19 |
| ATOM | 1259 | CA | VAL | A | 180 | 8.962 | 7.138 | 6.489 | 1.00 | 20.78 |
| ATOM | 1260 | CB | VAL | A | 180 | 7.618 | 7.245 | 5.727 | 1.00 | 21.45 |
| ATOM | 1261 | CG1 | VAL | A | 180 | 6.562 | 7.861 | 6.626 | 1.00 | 19.51 |
| ATOM | 1262 | CG2 | VAL | A | 180 | 7.799 | 8.050 | 4.439 | 1.00 | 20.49 |
| ATOM | 1263 | C | VAL | A | 180 | 8.719 | 6.223 | 7.681 | 1.00 | 21.61 |
| ATOM | 1264 | O | VAL | A | 180 | 8.663 | 5.006 | 7.525 | 1.00 | 19.79 |
| ATOM | 1265 | N | VAL | A | 181 | 8.579 | 6.810 | 8.866 | 1.00 | 21.26 |
| ATOM | 1266 | CA | VAL | A | 181 | 8.279 | 6.050 | 10.068 | 1.00 | 21.02 |
| ATOM | 1267 | CB | VAL | A | 181 | 9.200 | 6.444 | 11.249 | 1.00 | 20.42 |
| ATOM | 1268 | CG1 | VAL | A | 181 | 8.787 | 5.711 | 12.508 | 1.00 | 16.40 |
| ATOM | 1269 | CG2 | VAL | A | 181 | 10.643 | 6.101 | 10.911 | 1.00 | 18.15 |
| ATOM | 1270 | C | VAL | A | 181 | 6.834 | 6.436 | 10.366 | 1.00 | 22.79 |
| ATOM | 1271 | O | VAL | A | 181 | 6.544 | 7.593 | 10.665 | 1.00 | 23.96 |
| ATOM | 1272 | N | ALA | A | 182 | 5.929 | 5.467 | 10.267 | 1.00 | 22.95 |
| ATOM | 1273 | CA | ALA | A | 182 | 4.511 | 5.717 | 10.473 | 1.00 | 23.66 |
| ATOM | 1274 | CB | ALA | A | 182 | 3.714 | 5.027 | 9.376 | 1.00 | 23.83 |
| ATOM | 1275 | C | ALA | A | 182 | 3.979 | 5.296 | 11.829 | 1.00 | 23.91 |
| ATOM | 1276 | O | ALA | A | 182 | 3.523 | 4.176 | 12.006 | 1.00 | 26.07 |
| ATOM | 1277 | N | PRO | A | 183 | 4.004 | 6.202 | 12.805 | 1.00 | 23.30 |

Figure 3 (suite 17)

| | | | | | | | | | | |
|---|---|---|---|---|---|---|---|---|---|---|
| ATOM | 1278 | CD | PRO | A | 183 | 4.624 | 7.537 | 12.833 | 1.00 | 22.63 |
| ATOM | 1279 | CA | PRO | A | 183 | 3.499 | 5.833 | 14.123 | 1.00 | 22.95 |
| ATOM | 1280 | CB | PRO | A | 183 | 4.132 | 6.877 | 15.027 | 1.00 | 22.75 |
| ATOM | 1281 | CG | PRO | A | 183 | 4.150 | 8.074 | 14.158 | 1.00 | 24.80 |
| ATOM | 1282 | C | PRO | A | 183 | 1.972 | 5.834 | 14.231 | 1.00 | 24.33 |
| ATOM | 1283 | O | PRO | A | 183 | 1.276 | 6.564 | 13.518 | 1.00 | 23.50 |
| ATOM | 1284 | N | GLY | A | 184 | 1.472 | 4.986 | 15.121 | 1.00 | 24.23 |
| ATOM | 1285 | CA | GLY | A | 184 | 0.051 | 4.905 | 15.372 | 1.00 | 27.27 |
| ATOM | 1286 | C | GLY | A | 184 | -0.154 | 5.766 | 16.602 | 1.00 | 29.47 |
| ATOM | 1287 | O | GLY | A | 184 | 0.470 | 6.824 | 16.715 | 1.00 | 29.06 |
| ATOM | 1288 | N | TYR | A | 185 | -0.993 | 5.333 | 17.534 | 1.00 | 30.15 |
| ATOM | 1289 | CA | TYR | A | 185 | -1.219 | 6.123 | 18.738 | 1.00 | 31.23 |
| ATOM | 1290 | CB | TYR | A | 185 | -2.531 | 5.719 | 19.415 | 1.00 | 35.26 |
| ATOM | 1291 | CG | TYR | A | 185 | -3.739 | 6.265 | 18.696 | 1.00 | 38.51 |
| ATOM | 1292 | CD1 | TYR | A | 185 | -4.136 | 5.737 | 17.472 | 1.00 | 40.59 |
| ATOM | 1293 | CE1 | TYR | A | 185 | -5.219 | 6.264 | 16.775 | 1.00 | 42.99 |
| ATOM | 1294 | CD2 | TYR | A | 185 | -4.459 | 7.341 | 19.214 | 1.00 | 39.38 |
| ATOM | 1295 | CE2 | TYR | A | 185 | -5.545 | 7.877 | 18.524 | 1.00 | 42.18 |
| ATOM | 1296 | CZ | TYR | A | 185 | -5.916 | 7.332 | 17.304 | 1.00 | 42.50 |
| ATOM | 1297 | OH | TYR | A | 185 | -6.973 | 7.855 | 16.599 | 1.00 | 45.88 |
| ATOM | 1298 | C | TYR | A | 185 | -0.079 | 6.030 | 19.734 | 1.00 | 29.89 |
| ATOM | 1299 | O | TYR | A | 185 | 0.239 | 4.949 | 20.234 | 1.00 | 27.29 |
| ATOM | 1300 | N | ILE | A | 186 | 0.535 | 7.180 | 20.011 | 1.00 | 29.69 |
| ATOM | 1301 | CA | ILE | A | 186 | 1.648 | 7.261 | 20.949 | 1.00 | 30.76 |
| ATOM | 1302 | CB | ILE | A | 186 | 2.887 | 7.867 | 20.280 | 1.00 | 28.11 |
| ATOM | 1303 | CG2 | ILE | A | 186 | 4.099 | 7.714 | 21.189 | 1.00 | 26.99 |
| ATOM | 1304 | CG1 | ILE | A | 186 | 3.142 | 7.161 | 18.950 | 1.00 | 26.89 |
| ATOM | 1305 | CD | ILE | A | 186 | 3.425 | 5.690 | 19.098 | 1.00 | 23.17 |
| ATOM | 1306 | C | ILE | A | 186 | 1.258 | 8.121 | 22.140 | 1.00 | 33.27 |
| ATOM | 1307 | O | ILE | A | 186 | 0.583 | 9.139 | 21.985 | 1.00 | 32.95 |
| ATOM | 1308 | N | ASP | A | 187 | 1.697 | 7.717 | 23.325 | 1.00 | 37.68 |
| ATOM | 1309 | CA | ASP | A | 187 | 1.380 | 8.437 | 24.550 | 1.00 | 42.05 |
| ATOM | 1310 | CB | ASP | A | 187 | 1.728 | 7.581 | 25.766 | 1.00 | 43.38 |
| ATOM | 1311 | CG | ASP | A | 187 | 1.084 | 8.090 | 27.037 | 1.00 | 44.84 |
| ATOM | 1312 | OD1 | ASP | A | 187 | 1.497 | 7.650 | 28.130 | 1.00 | 47.35 |
| ATOM | 1313 | OD2 | ASP | A | 187 | 0.157 | 8.924 | 26.943 | 1.00 | 43.52 |
| ATOM | 1314 | C | ASP | A | 187 | 2.122 | 9.765 | 24.641 | 1.00 | 44.87 |
| ATOM | 1315 | O | ASP | A | 187 | 3.267 | 9.819 | 25.093 | 1.00 | 44.45 |
| ATOM | 1316 | N | THR | A | 188 | 1.458 | 10.833 | 24.212 | 1.00 | 48.93 |
| ATOM | 1317 | CA | THR | A | 188 | 2.046 | 12.166 | 24.240 | 1.00 | 53.70 |
| ATOM | 1318 | CB | THR | A | 188 | 2.230 | 12.716 | 22.828 | 1.00 | 54.21 |
| ATOM | 1319 | OG1 | THR | A | 188 | 0.947 | 12.871 | 22.209 | 1.00 | 53.66 |
| ATOM | 1320 | CG2 | THR | A | 188 | 3.086 | 11.765 | 22.000 | 1.00 | 55.43 |
| ATOM | 1321 | C | THR | A | 188 | 1.156 | 13.138 | 25.001 | 1.00 | 56.59 |
| ATOM | 1322 | O | THR | A | 188 | 0.026 | 12.803 | 25.369 | 1.00 | 57.46 |
| ATOM | 1323 | N | ASP | A | 189 | 1.668 | 14.344 | 25.230 | 1.00 | 59.38 |
| ATOM | 1324 | CA | ASP | A | 189 | 0.917 | 15.370 | 25.946 | 1.00 | 61.90 |
| ATOM | 1325 | CB | ASP | A | 189 | 1.706 | 16.683 | 25.972 | 1.00 | 61.72 |
| ATOM | 1326 | CG | ASP | A | 189 | 3.052 | 16.539 | 26.655 | 0.00 | 61.71 |
| ATOM | 1327 | OD1 | ASP | A | 189 | 3.078 | 16.165 | 27.847 | 0.00 | 61.66 |
| ATOM | 1328 | OD2 | ASP | A | 189 | 4.083 | 16.800 | 26.001 | 0.00 | 61.66 |
| ATOM | 1329 | C | ASP | A | 189 | -0.439 | 15.586 | 25.281 | 1.00 | 63.40 |
| ATOM | 1330 | O | ASP | A | 189 | -1.451 | 15.763 | 25.962 | 1.00 | 64.05 |
| ATOM | 1331 | N | MET | A | 190 | -0.453 | 15.563 | 23.949 | 1.00 | 65.16 |
| ATOM | 1332 | CA | MET | A | 190 | -1.687 | 15.743 | 23.187 | 1.00 | 66.37 |
| ATOM | 1333 | CB | MET | A | 190 | -1.408 | 15.632 | 21.683 | 1.00 | 66.16 |
| ATOM | 1334 | CG | MET | A | 190 | -0.399 | 16.642 | 21.157 | 0.00 | 66.11 |
| ATOM | 1335 | SD | MET | A | 190 | -0.926 | 18.349 | 21.404 | 0.00 | 66.04 |
| ATOM | 1336 | CE | MET | A | 190 | -1.806 | 18.658 | 19.875 | 0.00 | 66.00 |
| ATOM | 1337 | C | MET | A | 190 | -2.714 | 14.686 | 23.592 | 1.00 | 67.21 |
| ATOM | 1338 | O | MET | A | 190 | -3.821 | 15.012 | 24.017 | 1.00 | 66.74 |
| ATOM | 1339 | N | THR | A | 191 | -2.332 | 13.418 | 23.464 | 1.00 | 68.47 |
| ATOM | 1340 | CA | THR | A | 191 | -3.212 | 12.310 | 23.808 | 1.00 | 70.19 |
| ATOM | 1341 | CB | THR | A | 191 | -2.457 | 10.968 | 23.726 | 1.00 | 70.83 |
| ATOM | 1342 | OG1 | THR | A | 191 | -1.836 | 10.850 | 22.438 | 1.00 | 70.10 |
| ATOM | 1343 | CG2 | THR | A | 191 | -3.422 | 9.800 | 23.924 | 1.00 | 71.95 |
| ATOM | 1344 | C | THR | A | 191 | -3.808 | 12.469 | 25.208 | 1.00 | 71.27 |
| ATOM | 1345 | O | THR | A | 191 | -4.919 | 12.008 | 25.471 | 1.00 | 71.28 |
| ATOM | 1346 | N | ARG | A | 192 | -3.070 | 13.121 | 26.103 | 1.00 | 72.57 |
| ATOM | 1347 | CA | ARG | A | 192 | -3.548 | 13.344 | 27.466 | 1.00 | 73.56 |
| ATOM | 1348 | CB | ARG | A | 192 | -2.387 | 13.724 | 28.394 | 1.00 | 73.29 |
| ATOM | 1349 | CG | ARG | A | 192 | -1.811 | 12.558 | 29.187 | 1.00 | 73.08 |
| ATOM | 1350 | CD | ARG | A | 192 | -1.136 | 11.534 | 28.287 | 1.00 | 72.56 |

Figure 3 (suite 18)

| | | | | | | | | | | |
|---|---|---|---|---|---|---|---|---|---|---|
| ATOM | 1351 | NE | ARG | A | 192 | -0.757 | 10.324 | 29.015 | 1.00 | 73.00 |
| ATOM | 1352 | CZ | ARG | A | 192 | 0.071 | 10.292 | 30.056 | 1.00 | 73.62 |
| ATOM | 1353 | NH1 | ARG | A | 192 | 0.623 | 11.410 | 30.513 | 1.00 | 74.08 |
| ATOM | 1354 | NH2 | ARG | A | 192 | 0.349 | 9.134 | 30.643 | 1.00 | 72.84 |
| ATOM | 1355 | C | ARG | A | 192 | -4.604 | 14.445 | 27.504 | 1.00 | 74.52 |
| ATOM | 1356 | O | ARG | A | 192 | -5.434 | 14.491 | 28.416 | 1.00 | 74.98 |
| ATOM | 1357 | N | ALA | A | 193 | -4.566 | 15.329 | 26.510 | 1.00 | 75.07 |
| ATOM | 1358 | CA | ALA | A | 193 | -5.521 | 16.432 | 26.424 | 1.00 | 75.62 |
| ATOM | 1359 | CB | ALA | A | 193 | -5.146 | 17.359 | 25.267 | 1.00 | 75.64 |
| ATOM | 1360 | C | ALA | A | 193 | -6.944 | 15.906 | 26.235 | 1.00 | 75.69 |
| ATOM | 1361 | O | ALA | A | 193 | -7.916 | 16.556 | 26.626 | 1.00 | 75.33 |
| ATOM | 1362 | N | LEU | A | 194 | -7.058 | 14.727 | 25.630 | 1.00 | 75.77 |
| ATOM | 1363 | CA | LEU | A | 194 | -8.356 | 14.112 | 25.389 | 1.00 | 76.09 |
| ATOM | 1364 | CB | LEU | A | 194 | -8.211 | 12.955 | 24.395 | 1.00 | 75.93 |
| ATOM | 1365 | CG | LEU | A | 194 | -7.605 | 13.303 | 23.033 | 0.00 | 75.68 |
| ATOM | 1366 | CD1 | LEU | A | 194 | -7.392 | 12.031 | 22.229 | 0.00 | 75.56 |
| ATOM | 1367 | CD2 | LEU | A | 194 | -8.519 | 14.264 | 22.291 | 0.00 | 75.56 |
| ATOM | 1368 | C | LEU | A | 194 | -8.937 | 13.596 | 26.702 | 1.00 | 76.25 |
| ATOM | 1369 | O | LEU | A | 194 | -8.289 | 13.669 | 27.750 | 1.00 | 75.95 |
| ATOM | 1370 | N | ASP | A | 195 | -10.163 | 13.083 | 26.646 | 1.00 | 76.30 |
| ATOM | 1371 | CA | ASP | A | 195 | -10.815 | 12.544 | 27.836 | 1.00 | 75.93 |
| ATOM | 1372 | CB | ASP | A | 195 | -12.329 | 12.452 | 27.615 | 1.00 | 75.54 |
| ATOM | 1373 | CG | ASP | A | 195 | -12.938 | 13.773 | 27.188 | 0.00 | 75.36 |
| ATOM | 1374 | OD1 | ASP | A | 195 | -12.776 | 14.770 | 27.923 | 0.00 | 75.21 |
| ATOM | 1375 | OD2 | ASP | A | 195 | -13.582 | 13.814 | 26.118 | 0.00 | 75.21 |
| ATOM | 1376 | C | ASP | A | 195 | -10.235 | 11.154 | 28.117 | 1.00 | 75.91 |
| ATOM | 1377 | O | ASP | A | 195 | -9.044 | 10.915 | 27.901 | 1.00 | 75.04 |
| ATOM | 1378 | N | GLU | A | 196 | -11.073 | 10.240 | 28.600 | 1.00 | 75.83 |
| ATOM | 1379 | CA | GLU | A | 196 | -10.624 | 8.883 | 28.889 | 1.00 | 75.46 |
| ATOM | 1380 | CB | GLU | A | 196 | -11.226 | 8.383 | 30.207 | 1.00 | 75.46 |
| ATOM | 1381 | CG | GLU | A | 196 | -12.741 | 8.285 | 30.212 | 0.00 | 74.96 |
| ATOM | 1382 | CD | GLU | A | 196 | -13.259 | 7.448 | 31.363 | 0.00 | 74.78 |
| ATOM | 1383 | OE1 | GLU | A | 196 | -12.943 | 6.240 | 31.408 | 0.00 | 74.65 |
| ATOM | 1384 | OE2 | GLU | A | 196 | -13.979 | 7.995 | 32.224 | 0.00 | 74.65 |
| ATOM | 1385 | C | GLU | A | 196 | -11.004 | 7.929 | 27.759 | 1.00 | 75.35 |
| ATOM | 1386 | O | GLU | A | 196 | -10.194 | 7.110 | 27.326 | 1.00 | 75.44 |
| ATOM | 1387 | N | ARG | A | 197 | -12.240 | 8.042 | 27.282 | 1.00 | 75.04 |
| ATOM | 1388 | CA | ARG | A | 197 | -12.722 | 7.186 | 26.204 | 1.00 | 74.96 |
| ATOM | 1389 | CB | ARG | A | 197 | -14.255 | 7.150 | 26.207 | 1.00 | 75.05 |
| ATOM | 1390 | CG | ARG | A | 197 | -14.908 | 8.513 | 26.049 | 0.00 | 74.35 |
| ATOM | 1391 | CD | ARG | A | 197 | -16.432 | 8.435 | 26.070 | 0.00 | 73.96 |
| ATOM | 1392 | NE | ARG | A | 197 | -16.963 | 7.970 | 27.351 | 0.00 | 73.56 |
| ATOM | 1393 | CZ | ARG | A | 197 | -17.111 | 6.693 | 27.689 | 0.00 | 73.37 |
| ATOM | 1394 | NH1 | ARG | A | 197 | -16.772 | 5.732 | 26.840 | 0.00 | 73.24 |
| ATOM | 1395 | NH2 | ARG | A | 197 | -17.600 | 6.375 | 28.880 | 0.00 | 73.24 |
| ATOM | 1396 | C | ARG | A | 197 | -12.218 | 7.653 | 24.838 | 1.00 | 74.78 |
| ATOM | 1397 | O | ARG | A | 197 | -12.075 | 6.851 | 23.916 | 1.00 | 74.27 |
| ATOM | 1398 | N | ILE | A | 198 | -11.946 | 8.950 | 24.716 | 1.00 | 74.53 |
| ATOM | 1399 | CA | ILE | A | 198 | -11.462 | 9.520 | 23.460 | 1.00 | 73.91 |
| ATOM | 1400 | CB | ILE | A | 198 | -11.252 | 11.050 | 23.579 | 1.00 | 73.93 |
| ATOM | 1401 | CG2 | ILE | A | 198 | -10.928 | 11.639 | 22.214 | 0.00 | 73.78 |
| ATOM | 1402 | CG1 | ILE | A | 198 | -12.506 | 11.710 | 24.161 | 0.00 | 73.69 |
| ATOM | 1403 | CD | ILE | A | 198 | -13.760 | 11.518 | 23.332 | 1.00 | 73.41 |
| ATOM | 1404 | C | ILE | A | 198 | -10.132 | 8.885 | 23.068 | 1.00 | 73.11 |
| ATOM | 1405 | O | ILE | A | 198 | -9.773 | 8.836 | 21.892 | 1.00 | 73.12 |
| ATOM | 1406 | N | GLN | A | 199 | -9.410 | 8.399 | 24.071 | 1.00 | 72.12 |
| ATOM | 1407 | CA | GLN | A | 199 | -8.112 | 7.773 | 23.862 | 1.00 | 70.42 |
| ATOM | 1408 | CB | GLN | A | 199 | -7.081 | 8.408 | 24.805 | 1.00 | 71.32 |
| ATOM | 1409 | CG | GLN | A | 199 | -7.548 | 8.499 | 26.260 | 1.00 | 71.85 |
| ATOM | 1410 | CD | GLN | A | 199 | -6.510 | 9.115 | 27.186 | 1.00 | 72.73 |
| ATOM | 1411 | OE1 | GLN | A | 199 | -6.763 | 9.314 | 28.377 | 1.00 | 72.64 |
| ATOM | 1412 | NE2 | GLN | A | 199 | -5.334 | 9.416 | 26.643 | 1.00 | 73.63 |
| ATOM | 1413 | C | GLN | A | 199 | -8.194 | 6.269 | 24.103 | 1.00 | 68.60 |
| ATOM | 1414 | O | GLN | A | 199 | -7.418 | 5.497 | 23.543 | 1.00 | 68.12 |
| ATOM | 1415 | N | GLN | A | 200 | -9.145 | 5.862 | 24.936 | 1.00 | 66.90 |
| ATOM | 1416 | CA | GLN | A | 200 | -9.328 | 4.452 | 25.255 | 1.00 | 65.67 |
| ATOM | 1417 | CB | GLN | A | 200 | -10.003 | 4.313 | 26.628 | 1.00 | 66.35 |
| ATOM | 1418 | CG | GLN | A | 200 | -10.137 | 2.881 | 27.131 | 1.00 | 67.05 |
| ATOM | 1419 | CD | GLN | A | 200 | -11.455 | 2.239 | 26.742 | 1.00 | 67.63 |
| ATOM | 1420 | OE1 | GLN | A | 200 | -11.626 | 1.024 | 26.864 | 1.00 | 67.98 |
| ATOM | 1421 | NE2 | GLN | A | 200 | -12.401 | 3.055 | 26.285 | 1.00 | 66.63 |
| ATOM | 1422 | C | GLN | A | 200 | -10.155 | 3.754 | 24.177 | 1.00 | 63.79 |
| ATOM | 1423 | O | GLN | A | 200 | -10.217 | 2.526 | 24.127 | 1.00 | 63.22 |

Figure 3 (suite 19)

| | | | | | | | | | | |
|---|---|---|---|---|---|---|---|---|---|---|
| ATOM | 1424 | N | GLY | A | 201 | -10.779 | 4.545 | 23.309 | 1.00 | 62.00 |
| ATOM | 1425 | CA | GLY | A | 201 | -11.588 | 3.985 | 22.241 | 1.00 | 59.62 |
| ATOM | 1426 | C | GLY | A | 201 | -10.768 | 3.646 | 21.009 | 1.00 | 58.70 |
| ATOM | 1427 | O | GLY | A | 201 | -11.313 | 3.235 | 19.983 | 1.00 | 59.13 |
| ATOM | 1428 | N | ALA | A | 202 | -9.455 | 3.826 | 21.105 | 1.00 | 56.32 |
| ATOM | 1429 | CA | ALA | A | 202 | -8.558 | 3.524 | 19.997 | 1.00 | 54.25 |
| ATOM | 1430 | CB | ALA | A | 202 | -7.461 | 4.576 | 19.912 | 1.00 | 54.49 |
| ATOM | 1431 | C | ALA | A | 202 | -7.943 | 2.145 | 20.196 | 1.00 | 52.55 |
| ATOM | 1432 | O | ALA | A | 202 | -7.608 | 1.455 | 19.234 | 1.00 | 52.36 |
| ATOM | 1433 | N | LEU | A | 203 | -7.799 | 1.752 | 21.456 | 1.00 | 50.56 |
| ATOM | 1434 | CA | LEU | A | 203 | -7.223 | 0.459 | 21.797 | 1.00 | 49.88 |
| ATOM | 1435 | CB | LEU | A | 203 | -7.257 | 0.256 | 23.314 | 1.00 | 49.29 |
| ATOM | 1436 | CG | LEU | A | 203 | -6.467 | 1.266 | 24.151 | 1.00 | 50.15 |
| ATOM | 1437 | CD1 | LEU | A | 203 | -6.553 | 0.889 | 25.623 | 1.00 | 50.81 |
| ATOM | 1438 | CD2 | LEU | A | 203 | -5.015 | 1.285 | 23.703 | 1.00 | 50.35 |
| ATOM | 1439 | C | LEU | A | 203 | -7.948 | -0.691 | 21.107 | 1.00 | 49.33 |
| ATOM | 1440 | O | LEU | A | 203 | -7.353 | -1.726 | 20.814 | 1.00 | 49.85 |
| ATOM | 1441 | N | GLN | A | 204 | -9.236 | -0.503 | 20.852 | 1.00 | 49.48 |
| ATOM | 1442 | CA | GLN | A | 204 | -10.063 | -1.514 | 20.198 | 1.00 | 49.73 |
| ATOM | 1443 | CB | GLN | A | 204 | -11.528 | -1.059 | 20.220 | 1.00 | 51.90 |
| ATOM | 1444 | CG | GLN | A | 204 | -12.553 | -2.101 | 19.777 | 1.00 | 54.06 |
| ATOM | 1445 | CD | GLN | A | 204 | -13.958 | -1.519 | 19.648 | 1.00 | 54.61 |
| ATOM | 1446 | OE1 | GLN | A | 204 | -14.466 | -0.873 | 20.568 | 1.00 | 54.86 |
| ATOM | 1447 | NE2 | GLN | A | 204 | -14.591 | -1.751 | 18.501 | 1.00 | 55.72 |
| ATOM | 1448 | C | GLN | A | 204 | -9.595 | -1.710 | 18.754 | 1.00 | 48.54 |
| ATOM | 1449 | O | GLN | A | 204 | -9.922 | -2.711 | 18.112 | 1.00 | 47.66 |
| ATOM | 1450 | N | PHE | A | 205 | -8.819 | -0.748 | 18.260 | 1.00 | 46.46 |
| ATOM | 1451 | CA | PHE | A | 205 | -8.301 | -0.778 | 16.894 | 1.00 | 44.66 |
| ATOM | 1452 | CB | PHE | A | 205 | -8.570 | 0.569 | 16.216 | 1.00 | 47.15 |
| ATOM | 1453 | CG | PHE | A | 205 | -10.029 | 0.917 | 16.101 | 1.00 | 49.74 |
| ATOM | 1454 | CD1 | PHE | A | 205 | -10.420 | 2.220 | 15.802 | 1.00 | 51.43 |
| ATOM | 1455 | CD2 | PHE | A | 205 | -11.013 | -0.058 | 16.275 | 1.00 | 51.47 |
| ATOM | 1456 | CE1 | PHE | A | 205 | -11.768 | 2.553 | 15.676 | 1.00 | 52.67 |
| ATOM | 1457 | CE2 | PHE | A | 205 | -12.367 | 0.259 | 16.153 | 1.00 | 52.62 |
| ATOM | 1458 | CZ | PHE | A | 205 | -12.746 | 1.569 | 15.852 | 1.00 | 53.25 |
| ATOM | 1459 | C | PHE | A | 205 | -6.802 | -1.084 | 16.834 | 1.00 | 41.13 |
| ATOM | 1460 | O | PHE | A | 205 | -6.170 | -0.934 | 15.789 | 1.00 | 40.62 |
| ATOM | 1461 | N | ILE | A | 206 | -6.244 | -1.525 | 17.954 | 1.00 | 38.26 |
| ATOM | 1462 | CA | ILE | A | 206 | -4.822 | -1.836 | 18.038 | 1.00 | 34.80 |
| ATOM | 1463 | CB | ILE | A | 206 | -4.100 | -0.877 | 19.019 | 1.00 | 34.07 |
| ATOM | 1464 | CG2 | ILE | A | 206 | -2.598 | -1.107 | 18.962 | 1.00 | 33.11 |
| ATOM | 1465 | CG1 | ILE | A | 206 | -4.396 | 0.581 | 18.659 | 1.00 | 31.61 |
| ATOM | 1466 | CD | ILE | A | 206 | -3.957 | 1.559 | 19.722 | 1.00 | 30.85 |
| ATOM | 1467 | C | ILE | A | 206 | -4.592 | -3.264 | 18.530 | 1.00 | 33.40 |
| ATOM | 1468 | O | ILE | A | 206 | -4.872 | -3.584 | 19.686 | 1.00 | 34.72 |
| ATOM | 1469 | N | PRO | A | 207 | -4.077 | -4.143 | 17.659 | 1.00 | 31.73 |
| ATOM | 1470 | CD | PRO | A | 207 | -3.819 | -4.008 | 16.214 | 1.00 | 31.31 |
| ATOM | 1471 | CA | PRO | A | 207 | -3.838 | -5.515 | 18.095 | 1.00 | 30.17 |
| ATOM | 1472 | CB | PRO | A | 207 | -3.077 | -6.112 | 16.920 | 1.00 | 28.83 |
| ATOM | 1473 | CG | PRO | A | 207 | -3.735 | -5.457 | 15.762 | 1.00 | 29.24 |
| ATOM | 1474 | C | PRO | A | 207 | -3.050 | -5.571 | 19.394 | 1.00 | 29.68 |
| ATOM | 1475 | O | PRO | A | 207 | -3.403 | -6.319 | 20.306 | 1.00 | 30.59 |
| ATOM | 1476 | N | ALA | A | 208 | -1.987 | -4.775 | 19.480 | 1.00 | 28.48 |
| ATOM | 1477 | CA | ALA | A | 208 | -1.151 | -4.760 | 20.675 | 1.00 | 28.28 |
| ATOM | 1478 | CB | ALA | A | 208 | -0.055 | -3.728 | 20.533 | 1.00 | 28.15 |
| ATOM | 1479 | C | ALA | A | 208 | -1.989 | -4.471 | 21.909 | 1.00 | 28.69 |
| ATOM | 1480 | O | ALA | A | 208 | -1.600 | -4.800 | 23.028 | 1.00 | 28.45 |
| ATOM | 1481 | N | LYS | A | 209 | -3.154 | -3.866 | 21.694 | 1.00 | 30.41 |
| ATOM | 1482 | CA | LYS | A | 209 | -4.067 | -3.527 | 22.781 | 1.00 | 31.35 |
| ATOM | 1483 | CB | LYS | A | 209 | -4.456 | -4.791 | 23.554 | 1.00 | 32.54 |
| ATOM | 1484 | CG | LYS | A | 209 | -5.406 | -5.707 | 22.784 | 1.00 | 33.85 |
| ATOM | 1485 | CD | LYS | A | 209 | -6.759 | -5.027 | 22.547 | 1.00 | 34.43 |
| ATOM | 1486 | CE | LYS | A | 209 | -7.133 | -4.977 | 21.065 | 1.00 | 34.46 |
| ATOM | 1487 | NZ | LYS | A | 209 | -7.186 | -6.326 | 20.427 | 1.00 | 30.13 |
| ATOM | 1488 | C | LYS | A | 209 | -3.476 | -2.490 | 23.732 | 1.00 | 31.95 |
| ATOM | 1489 | O | LYS | A | 209 | -3.833 | -2.425 | 24.911 | 1.00 | 32.91 |
| ATOM | 1490 | N | ARG | A | 210 | -2.554 | -1.686 | 23.219 | 1.00 | 30.40 |
| ATOM | 1491 | CA | ARG | A | 210 | -1.951 | -0.639 | 24.024 | 1.00 | 29.80 |
| ATOM | 1492 | CB | ARG | A | 210 | -0.861 | -1.201 | 24.940 | 1.00 | 29.86 |
| ATOM | 1493 | CG | ARG | A | 210 | 0.434 | -1.547 | 24.233 | 1.00 | 28.45 |
| ATOM | 1494 | CD | ARG | A | 210 | 1.509 | -1.914 | 25.233 | 1.00 | 25.31 |
| ATOM | 1495 | NE | ARG | A | 210 | 2.715 | -2.387 | 24.559 | 1.00 | 25.07 |
| ATOM | 1496 | CZ | ARG | A | 210 | 3.575 | -1.602 | 23.917 | 1.00 | 22.22 |

Figure 3 (suite 20)

| | | | | | | | | | | |
|---|---|---|---|---|---|---|---|---|---|---|
| ATOM | 1497 | NH1 | ARG | A | 210 | 3.368 | -0.288 | 23.863 | 1.00 | 20.69 |
| ATOM | 1498 | NH2 | ARG | A | 210 | 4.639 | -2.131 | 23.330 | 1.00 | 20.90 |
| ATOM | 1499 | C | ARG | A | 210 | -1.366 | 0.466 | 23.164 | 1.00 | 28.74 |
| ATOM | 1500 | O | ARG | A | 210 | -1.148 | 0.306 | 21.961 | 1.00 | 28.41 |
| ATOM | 1501 | N | VAL | A | 211 | -1.136 | 1.599 | 23.808 | 1.00 | 29.45 |
| ATOM | 1502 | CA | VAL | A | 211 | -0.568 | 2.775 | 23.164 | 1.00 | 28.88 |
| ATOM | 1503 | CB | VAL | A | 211 | -1.029 | 4.060 | 23.901 | 1.00 | 30.36 |
| ATOM | 1504 | CG1 | VAL | A | 211 | -0.175 | 5.239 | 23.495 | 1.00 | 30.84 |
| ATOM | 1505 | CG2 | VAL | A | 211 | -2.497 | 4.345 | 23.565 | 1.00 | 31.53 |
| ATOM | 1506 | C | VAL | A | 211 | 0.958 | 2.677 | 23.200 | 1.00 | 27.77 |
| ATOM | 1507 | O | VAL | A | 211 | 1.539 | 2.242 | 24.192 | 1.00 | 29.52 |
| ATOM | 1508 | N | GLY | A | 212 | 1.606 | 3.072 | 22.115 | 1.00 | 26.63 |
| ATOM | 1509 | CA | GLY | A | 212 | 3.056 | 3.021 | 22.074 | 1.00 | 26.43 |
| ATOM | 1510 | C | GLY | A | 212 | 3.662 | 4.146 | 22.889 | 1.00 | 26.40 |
| ATOM | 1511 | O | GLY | A | 212 | 2.936 | 4.935 | 23.495 | 1.00 | 28.28 |
| ATOM | 1512 | N | THR | A | 213 | 4.987 | 4.219 | 22.922 | 1.00 | 25.23 |
| ATOM | 1513 | CA | THR | A | 213 | 5.668 | 5.277 | 23.665 | 1.00 | 25.44 |
| ATOM | 1514 | CB | THR | A | 213 | 6.569 | 4.719 | 24.782 | 1.00 | 23.97 |
| ATOM | 1515 | OG1 | THR | A | 213 | 7.626 | 3.959 | 24.190 | 1.00 | 24.84 |
| ATOM | 1516 | CG2 | THR | A | 213 | 5.785 | 3.841 | 25.734 | 1.00 | 23.87 |
| ATOM | 1517 | C | THR | A | 213 | 6.575 | 6.033 | 22.709 | 1.00 | 24.95 |
| ATOM | 1518 | O | THR | A | 213 | 6.876 | 5.549 | 21.618 | 1.00 | 23.48 |
| ATOM | 1519 | N | PRO | A | 214 | 7.012 | 7.241 | 23.099 | 1.00 | 25.53 |
| ATOM | 1520 | CD | PRO | A | 214 | 6.491 | 8.071 | 24.200 | 1.00 | 25.92 |
| ATOM | 1521 | CA | PRO | A | 214 | 7.904 | 8.026 | 22.232 | 1.00 | 24.23 |
| ATOM | 1522 | CB | PRO | A | 214 | 8.045 | 9.344 | 22.990 | 1.00 | 23.44 |
| ATOM | 1523 | CG | PRO | A | 214 | 6.706 | 9.475 | 23.653 | 1.00 | 25.96 |
| ATOM | 1524 | C | PRO | A | 214 | 9.235 | 7.286 | 22.068 | 1.00 | 22.20 |
| ATOM | 1525 | O | PRO | A | 214 | 9.879 | 7.349 | 21.024 | 1.00 | 22.02 |
| ATOM | 1526 | N | ALA | A | 215 | 9.628 | 6.559 | 23.104 | 1.00 | 21.59 |
| ATOM | 1527 | CA | ALA | A | 215 | 10.865 | 5.788 | 23.060 | 1.00 | 22.16 |
| ATOM | 1528 | CB | ALA | A | 215 | 11.159 | 5.200 | 24.436 | 1.00 | 21.65 |
| ATOM | 1529 | C | ALA | A | 215 | 10.769 | 4.673 | 22.010 | 1.00 | 23.55 |
| ATOM | 1530 | O | ALA | A | 215 | 11.746 | 4.386 | 21.318 | 1.00 | 24.84 |
| ATOM | 1531 | N | GLU | A | 216 | 9.596 | 4.051 | 21.872 | 1.00 | 23.38 |
| ATOM | 1532 | CA | GLU | A | 216 | 9.438 | 2.980 | 20.885 | 1.00 | 22.85 |
| ATOM | 1533 | CB | GLU | A | 216 | 8.107 | 2.256 | 21.096 | 1.00 | 22.66 |
| ATOM | 1534 | CG | GLU | A | 216 | 8.120 | 1.429 | 22.371 | 1.00 | 24.30 |
| ATOM | 1535 | CD | GLU | A | 216 | 6.760 | 0.864 | 22.747 | 1.00 | 23.24 |
| ATOM | 1536 | OE1 | GLU | A | 216 | 5.814 | 1.658 | 22.904 | 1.00 | 22.54 |
| ATOM | 1537 | OE2 | GLU | A | 216 | 6.648 | -0.377 | 22.897 | 1.00 | 26.14 |
| ATOM | 1538 | C | GLU | A | 216 | 9.570 | 3.498 | 19.464 | 1.00 | 22.88 |
| ATOM | 1539 | O | GLU | A | 216 | 9.982 | 2.769 | 18.560 | 1.00 | 22.51 |
| ATOM | 1540 | N | VAL | A | 217 | 9.225 | 4.768 | 19.273 | 1.00 | 23.16 |
| ATOM | 1541 | CA | VAL | A | 217 | 9.349 | 5.409 | 17.963 | 1.00 | 22.45 |
| ATOM | 1542 | CB | VAL | A | 217 | 8.490 | 6.715 | 17.877 | 1.00 | 21.94 |
| ATOM | 1543 | CG1 | VAL | A | 217 | 8.618 | 7.332 | 16.507 | 1.00 | 22.67 |
| ATOM | 1544 | CG2 | VAL | A | 217 | 7.029 | 6.409 | 18.176 | 1.00 | 21.63 |
| ATOM | 1545 | C | VAL | A | 217 | 10.834 | 5.779 | 17.783 | 1.00 | 22.70 |
| ATOM | 1546 | O | VAL | A | 217 | 11.415 | 5.576 | 16.709 | 1.00 | 20.49 |
| ATOM | 1547 | N | ALA | A | 218 | 11.432 | 6.321 | 18.848 | 1.00 | 21.01 |
| ATOM | 1548 | CA | ALA | A | 218 | 12.847 | 6.729 | 18.852 | 1.00 | 20.10 |
| ATOM | 1549 | CB | ALA | A | 218 | 13.262 | 7.179 | 20.250 | 1.00 | 17.08 |
| ATOM | 1550 | C | ALA | A | 218 | 13.720 | 5.571 | 18.414 | 1.00 | 19.64 |
| ATOM | 1551 | O | ALA | A | 218 | 14.701 | 5.748 | 17.682 | 1.00 | 19.97 |
| ATOM | 1552 | N | GLY | A | 219 | 13.343 | 4.375 | 18.861 | 1.00 | 19.88 |
| ATOM | 1553 | CA | GLY | A | 219 | 14.096 | 3.185 | 18.510 | 1.00 | 19.84 |
| ATOM | 1554 | C | GLY | A | 219 | 14.149 | 2.975 | 17.017 | 1.00 | 17.61 |
| ATOM | 1555 | O | GLY | A | 219 | 15.211 | 2.738 | 16.452 | 1.00 | 19.63 |
| ATOM | 1556 | N | VAL | A | 220 | 12.999 | 3.074 | 16.368 | 1.00 | 18.57 |
| ATOM | 1557 | CA | VAL | A | 220 | 12.922 | 2.879 | 14.928 | 1.00 | 18.27 |
| ATOM | 1558 | CB | VAL | A | 220 | 11.457 | 2.940 | 14.452 | 1.00 | 18.21 |
| ATOM | 1559 | CG1 | VAL | A | 220 | 11.354 | 2.526 | 12.985 | 1.00 | 17.32 |
| ATOM | 1560 | CG2 | VAL | A | 220 | 10.608 | 2.040 | 15.327 | 1.00 | 21.48 |
| ATOM | 1561 | C | VAL | A | 220 | 13.744 | 3.914 | 14.170 | 1.00 | 17.75 |
| ATOM | 1562 | O | VAL | A | 220 | 14.429 | 3.589 | 13.209 | 1.00 | 20.13 |
| ATOM | 1563 | N | VAL | A | 221 | 13.675 | 5.169 | 14.598 | 1.00 | 20.26 |
| ATOM | 1564 | CA | VAL | A | 221 | 14.444 | 6.215 | 13.928 | 1.00 | 18.92 |
| ATOM | 1565 | CB | VAL | A | 221 | 14.069 | 7.608 | 14.469 | 1.00 | 18.88 |
| ATOM | 1566 | CG1 | VAL | A | 221 | 14.886 | 8.677 | 13.758 | 1.00 | 19.47 |
| ATOM | 1567 | CG2 | VAL | A | 221 | 12.572 | 7.857 | 14.248 | 1.00 | 18.74 |
| ATOM | 1568 | C | VAL | A | 221 | 15.937 | 5.937 | 14.145 | 1.00 | 20.15 |
| ATOM | 1569 | O | VAL | A | 221 | 16.740 | 6.080 | 13.226 | 1.00 | 19.50 |

Figure 3 (suite 21)

| | | | | | | | | | | |
|---|---|---|---|---|---|---|---|---|---|---|
| ATOM | 1570 | N | SER | A | 222 | 16.304 | 5.528 | 15.358 | 1.00 | 19.36 |
| ATOM | 1571 | CA | SER | A | 222 | 17.690 | 5.188 | 15.641 | 1.00 | 18.94 |
| ATOM | 1572 | CB | SER | A | 222 | 17.833 | 4.642 | 17.051 | 1.00 | 20.48 |
| ATOM | 1573 | OG | SER | A | 222 | 19.133 | 4.106 | 17.217 | 1.00 | 17.17 |
| ATOM | 1574 | C | SER | A | 222 | 18.163 | 4.121 | 14.668 | 1.00 | 19.66 |
| ATOM | 1575 | O | SER | A | 222 | 19.251 | 4.212 | 14.094 | 1.00 | 19.47 |
| ATOM | 1576 | N | PHE | A | 223 | 17.340 | 3.098 | 14.477 | 1.00 | 19.56 |
| ATOM | 1577 | CA | PHE | A | 223 | 17.715 | 2.027 | 13.563 | 1.00 | 20.01 |
| ATOM | 1578 | CB | PHE | A | 223 | 16.652 | 0.911 | 13.595 | 1.00 | 18.67 |
| ATOM | 1579 | CG | PHE | A | 223 | 16.616 | 0.075 | 12.349 | 1.00 | 19.08 |
| ATOM | 1580 | CD1 | PHE | A | 223 | 17.694 | -0.744 | 12.008 | 1.00 | 16.49 |
| ATOM | 1581 | CD2 | PHE | A | 223 | 15.528 | 0.151 | 11.485 | 1.00 | 16.24 |
| ATOM | 1582 | CE1 | PHE | A | 223 | 17.685 | -1.469 | 10.823 | 1.00 | 14.42 |
| ATOM | 1583 | CE2 | PHE | A | 223 | 15.509 | -0.568 | 10.298 | 1.00 | 13.96 |
| ATOM | 1584 | CZ | PHE | A | 223 | 16.587 | -1.380 | 9.962 | 1.00 | 14.23 |
| ATOM | 1585 | C | PHE | A | 223 | 17.881 | 2.584 | 12.147 | 1.00 | 19.30 |
| ATOM | 1586 | O | PHE | A | 223 | 18.858 | 2.285 | 11.459 | 1.00 | 17.98 |
| ATOM | 1587 | N | LEU | A | 224 | 16.930 | 3.404 | 11.708 | 1.00 | 19.46 |
| ATOM | 1588 | CA | LEU | A | 224 | 17.016 | 3.976 | 10.364 | 1.00 | 19.17 |
| ATOM | 1589 | CB | LEU | A | 224 | 15.690 | 4.636 | 9.991 | 1.00 | 18.83 |
| ATOM | 1590 | CG | LEU | A | 224 | 14.573 | 3.662 | 9.583 | 1.00 | 20.77 |
| ATOM | 1591 | CD1 | LEU | A | 224 | 13.239 | 4.389 | 9.508 | 1.00 | 19.92 |
| ATOM | 1592 | CD2 | LEU | A | 224 | 14.918 | 3.029 | 8.243 | 1.00 | 19.95 |
| ATOM | 1593 | C | LEU | A | 224 | 18.183 | 4.960 | 10.200 | 1.00 | 20.54 |
| ATOM | 1594 | O | LEU | A | 224 | 18.629 | 5.211 | 9.086 | 1.00 | 19.06 |
| ATOM | 1595 | N | ALA | A | 225 | 18.678 | 5.509 | 11.307 | 1.00 | 19.84 |
| ATOM | 1596 | CA | ALA | A | 225 | 19.811 | 6.425 | 11.244 | 1.00 | 21.49 |
| ATOM | 1597 | CB | ALA | A | 225 | 19.765 | 7.390 | 12.420 | 1.00 | 20.88 |
| ATOM | 1598 | C | ALA | A | 225 | 21.154 | 5.681 | 11.244 | 1.00 | 22.38 |
| ATOM | 1599 | O | ALA | A | 225 | 22.146 | 6.188 | 10.716 | 1.00 | 24.95 |
| ATOM | 1600 | N | SER | A | 226 | 21.182 | 4.485 | 11.829 | 1.00 | 21.84 |
| ATOM | 1601 | CA | SER | A | 226 | 22.409 | 3.687 | 11.920 | 1.00 | 23.42 |
| ATOM | 1602 | CB | SER | A | 226 | 22.228 | 2.562 | 12.951 | 1.00 | 22.36 |
| ATOM | 1603 | OG | SER | A | 226 | 21.422 | 1.512 | 12.439 | 1.00 | 21.91 |
| ATOM | 1604 | C | SER | A | 226 | 22.878 | 3.079 | 10.601 | 1.00 | 24.46 |
| ATOM | 1605 | O | SER | A | 226 | 22.184 | 3.141 | 9.583 | 1.00 | 23.46 |
| ATOM | 1606 | N | GLU | A | 227 | 24.066 | 2.481 | 10.636 | 1.00 | 24.36 |
| ATOM | 1607 | CA | GLU | A | 227 | 24.669 | 1.838 | 9.466 | 1.00 | 24.93 |
| ATOM | 1608 | CB | GLU | A | 227 | 26.131 | 1.482 | 9.779 | 1.00 | 24.22 |
| ATOM | 1609 | CG | GLU | A | 227 | 27.032 | 2.678 | 10.019 | 1.00 | 24.51 |
| ATOM | 1610 | CD | GLU | A | 227 | 27.377 | 3.393 | 8.734 | 1.00 | 27.17 |
| ATOM | 1611 | OE1 | GLU | A | 227 | 28.302 | 2.935 | 8.016 | 1.00 | 27.71 |
| ATOM | 1612 | OE2 | GLU | A | 227 | 26.708 | 4.403 | 8.435 | 1.00 | 26.30 |
| ATOM | 1613 | C | GLU | A | 227 | 23.941 | 0.576 | 8.959 | 1.00 | 24.08 |
| ATOM | 1614 | O | GLU | A | 227 | 24.231 | 0.100 | 7.865 | 1.00 | 25.50 |
| ATOM | 1615 | N | ASP | A | 228 | 23.004 | 0.047 | 9.743 | 1.00 | 22.88 |
| ATOM | 1616 | CA | ASP | A | 228 | 22.247 | -1.163 | 9.379 | 1.00 | 22.39 |
| ATOM | 1617 | CB | ASP | A | 228 | 21.711 | -1.850 | 10.643 | 1.00 | 20.91 |
| ATOM | 1618 | CG | ASP | A | 228 | 22.739 | -2.732 | 11.314 | 1.00 | 23.14 |
| ATOM | 1619 | OD1 | ASP | A | 228 | 22.620 | -2.964 | 12.538 | 1.00 | 27.49 |
| ATOM | 1620 | OD2 | ASP | A | 228 | 23.656 | -3.205 | 10.616 | 1.00 | 23.27 |
| ATOM | 1621 | C | ASP | A | 228 | 21.061 | -0.969 | 8.424 | 1.00 | 23.20 |
| ATOM | 1622 | O | ASP | A | 228 | 20.488 | -1.947 | 7.934 | 1.00 | 23.49 |
| ATOM | 1623 | N | ALA | A | 229 | 20.677 | 0.273 | 8.159 | 1.00 | 24.20 |
| ATOM | 1624 | CA | ALA | A | 229 | 19.531 | 0.517 | 7.284 | 1.00 | 24.88 |
| ATOM | 1625 | CB | ALA | A | 229 | 18.611 | 1.558 | 7.924 | 1.00 | 25.71 |
| ATOM | 1626 | C | ALA | A | 229 | 19.913 | 0.958 | 5.880 | 1.00 | 23.13 |
| ATOM | 1627 | O | ALA | A | 229 | 19.064 | 1.439 | 5.127 | 1.00 | 21.29 |
| ATOM | 1628 | N | SER | A | 230 | 21.181 | 0.780 | 5.520 | 1.00 | 21.49 |
| ATOM | 1629 | CA | SER | A | 230 | 21.671 | 1.193 | 4.203 | 1.00 | 20.96 |
| ATOM | 1630 | CB | SER | A | 230 | 23.154 | 0.844 | 4.070 | 1.00 | 20.19 |
| ATOM | 1631 | OG | SER | A | 230 | 23.328 | -0.566 | 3.950 | 1.00 | 24.08 |
| ATOM | 1632 | C | SER | A | 230 | 20.944 | 0.653 | 2.965 | 1.00 | 19.26 |
| ATOM | 1633 | O | SER | A | 230 | 20.991 | 1.279 | 1.905 | 1.00 | 20.04 |
| ATOM | 1634 | N | TYR | A | 231 | 20.296 | -0.504 | 3.058 | 1.00 | 17.57 |
| ATOM | 1635 | CA | TYR | A | 231 | 19.625 | -1.021 | 1.865 | 1.00 | 18.22 |
| ATOM | 1636 | CB | TYR | A | 231 | 19.937 | -2.507 | 1.644 | 1.00 | 16.59 |
| ATOM | 1637 | CG | TYR | A | 231 | 19.773 | -2.918 | 0.189 | 1.00 | 17.55 |
| ATOM | 1638 | CD1 | TYR | A | 231 | 20.341 | -2.156 | -0.827 | 1.00 | 18.46 |
| ATOM | 1639 | CE1 | TYR | A | 231 | 20.244 | -2.546 | -2.162 | 1.00 | 19.61 |
| ATOM | 1640 | CD2 | TYR | A | 231 | 19.088 | -4.087 | -0.165 | 1.00 | 16.40 |
| ATOM | 1641 | CE2 | TYR | A | 231 | 18.984 | -4.492 | -1.495 | 1.00 | 18.17 |
| ATOM | 1642 | CZ | TYR | A | 231 | 19.569 | -3.714 | -2.490 | 1.00 | 20.89 |

Figure 3 (suite 22)

| | | | | | | | | | | | |
|---|---|---|---|---|---|---|---|---|---|---|---|
| ATOM | 1643 | OH | TYR | A | 231 | 19.510 | -4.104 | -3.809 | 1.00 | 22.96 |
| ATOM | 1644 | C | TYR | A | 231 | 18.113 | -0.803 | 1.882 | 1.00 | 16.01 |
| ATOM | 1645 | O | TYR | A | 231 | 17.408 | -1.234 | 0.978 | 1.00 | 15.50 |
| ATOM | 1646 | N | ILE | A | 232 | 17.636 | -0.141 | 2.926 | 1.00 | 15.80 |
| ATOM | 1647 | CA | ILE | A | 232 | 16.229 | 0.187 | 3.066 | 1.00 | 15.99 |
| ATOM | 1648 | CB | ILE | A | 232 | 15.796 | 0.143 | 4.531 | 1.00 | 14.69 |
| ATOM | 1649 | CG2 | ILE | A | 232 | 14.338 | 0.588 | 4.648 | 1.00 | 14.41 |
| ATOM | 1650 | CG1 | ILE | A | 232 | 16.027 | -1.267 | 5.089 | 1.00 | 17.74 |
| ATOM | 1651 | CD | ILE | A | 232 | 15.538 | -1.470 | 6.515 | 1.00 | 17.04 |
| ATOM | 1652 | C | ILE | A | 232 | 16.021 | 1.621 | 2.556 | 1.00 | 16.49 |
| ATOM | 1653 | O | ILE | A | 232 | 16.630 | 2.565 | 3.065 | 1.00 | 15.16 |
| ATOM | 1654 | N | SER | A | 233 | 15.163 | 1.767 | 1.553 | 1.00 | 18.64 |
| ATOM | 1655 | CA | SER | A | 233 | 14.848 | 3.065 | 0.968 | 1.00 | 19.20 |
| ATOM | 1656 | CB | SER | A | 233 | 15.916 | 3.455 | -0.053 | 1.00 | 21.26 |
| ATOM | 1657 | OG | SER | A | 233 | 15.620 | 4.697 | -0.658 | 1.00 | 21.05 |
| ATOM | 1658 | C | SER | A | 233 | 13.480 | 3.029 | 0.284 | 1.00 | 19.87 |
| ATOM | 1659 | O | SER | A | 233 | 13.178 | 2.108 | -0.486 | 1.00 | 19.81 |
| ATOM | 1660 | N | GLY | A | 234 | 12.657 | 4.038 | 0.573 | 1.00 | 18.53 |
| ATOM | 1661 | CA | GLY | A | 234 | 11.339 | 4.122 | -0.022 | 1.00 | 16.68 |
| ATOM | 1662 | C | GLY | A | 234 | 10.346 | 3.375 | 0.830 | 1.00 | 16.81 |
| ATOM | 1663 | O | GLY | A | 234 | 9.211 | 3.125 | 0.426 | 1.00 | 16.34 |
| ATOM | 1664 | N | ALA | A | 235 | 10.778 | 3.032 | 2.032 | 1.00 | 15.22 |
| ATOM | 1665 | CA | ALA | A | 235 | 9.941 | 2.274 | 2.940 | 1.00 | 16.22 |
| ATOM | 1666 | CB | ALA | A | 235 | 10.801 | 1.277 | 3.716 | 1.00 | 18.83 |
| ATOM | 1667 | C | ALA | A | 235 | 9.128 | 3.093 | 3.927 | 1.00 | 16.37 |
| ATOM | 1668 | O | ALA | A | 235 | 9.529 | 4.177 | 4.353 | 1.00 | 13.28 |
| ATOM | 1669 | N | VAL | A | 236 | 7.976 | 2.533 | 4.284 | 1.00 | 16.91 |
| ATOM | 1670 | CA | VAL | A | 236 | 7.077 | 3.123 | 5.246 | 1.00 | 17.93 |
| ATOM | 1671 | CB | VAL | A | 236 | 5.649 | 3.356 | 4.669 | 1.00 | 19.00 |
| ATOM | 1672 | CG1 | VAL | A | 236 | 4.750 | 3.952 | 5.738 | 1.00 | 17.26 |
| ATOM | 1673 | CG2 | VAL | A | 236 | 5.708 | 4.268 | 3.464 | 1.00 | 16.71 |
| ATOM | 1674 | C | VAL | A | 236 | 6.998 | 2.074 | 6.321 | 1.00 | 18.86 |
| ATOM | 1675 | O | VAL | A | 236 | 6.384 | 1.033 | 6.127 | 1.00 | 22.72 |
| ATOM | 1676 | N | ILE | A | 237 | 7.629 | 2.345 | 7.453 | 1.00 | 17.47 |
| ATOM | 1677 | CA | ILE | A | 237 | 7.638 | 1.407 | 8.554 | 1.00 | 15.78 |
| ATOM | 1678 | CB | ILE | A | 237 | 9.043 | 1.300 | 9.166 | 1.00 | 16.18 |
| ATOM | 1679 | CG2 | ILE | A | 237 | 9.035 | 0.290 | 10.306 | 1.00 | 14.81 |
| ATOM | 1680 | CG1 | ILE | A | 237 | 10.050 | 0.910 | 8.076 | 1.00 | 15.22 |
| ATOM | 1681 | CD | ILE | A | 237 | 11.457 | 0.608 | 8.589 | 1.00 | 14.49 |
| ATOM | 1682 | C | ILE | A | 237 | 6.657 | 1.780 | 9.652 | 1.00 | 16.44 |
| ATOM | 1683 | O | ILE | A | 237 | 6.772 | 2.841 | 10.275 | 1.00 | 13.61 |
| ATOM | 1684 | N | PRO | A | 238 | 5.669 | 0.903 | 9.914 | 1.00 | 16.75 |
| ATOM | 1685 | CD | PRO | A | 238 | 5.211 | -0.243 | 9.108 | 1.00 | 15.08 |
| ATOM | 1686 | CA | PRO | A | 238 | 4.701 | 1.219 | 10.964 | 1.00 | 17.32 |
| ATOM | 1687 | CB | PRO | A | 238 | 3.485 | 0.393 | 10.561 | 1.00 | 15.50 |
| ATOM | 1688 | CG | PRO | A | 238 | 4.093 | -0.805 | 9.951 | 1.00 | 16.12 |
| ATOM | 1689 | C | PRO | A | 238 | 5.205 | 0.895 | 12.372 | 1.00 | 17.46 |
| ATOM | 1690 | O | PRO | A | 238 | 5.999 | -0.027 | 12.573 | 1.00 | 15.97 |
| ATOM | 1691 | N | VAL | A | 239 | 4.731 | 1.675 | 13.337 | 1.00 | 17.78 |
| ATOM | 1692 | CA | VAL | A | 239 | 5.090 | 1.528 | 14.740 | 1.00 | 18.45 |
| ATOM | 1693 | CB | VAL | A | 239 | 6.199 | 2.550 | 15.143 | 1.00 | 19.47 |
| ATOM | 1694 | CG1 | VAL | A | 239 | 6.655 | 2.302 | 16.554 | 1.00 | 19.85 |
| ATOM | 1695 | CG2 | VAL | A | 239 | 7.372 | 2.458 | 14.177 | 1.00 | 19.53 |
| ATOM | 1696 | C | VAL | A | 239 | 3.770 | 1.881 | 15.396 | 1.00 | 18.46 |
| ATOM | 1697 | O | VAL | A | 239 | 3.658 | 2.831 | 16.173 | 1.00 | 18.55 |
| ATOM | 1698 | N | ASP | A | 240 | 2.761 | 1.092 | 15.045 | 1.00 | 19.42 |
| ATOM | 1699 | CA | ASP | A | 240 | 1.400 | 1.287 | 15.509 | 1.00 | 18.80 |
| ATOM | 1700 | CB | ASP | A | 240 | 0.505 | 1.530 | 14.304 | 1.00 | 20.01 |
| ATOM | 1701 | CG | ASP | A | 240 | 0.667 | 0.456 | 13.263 | 1.00 | 20.60 |
| ATOM | 1702 | OD1 | ASP | A | 240 | 1.082 | -0.653 | 13.659 | 1.00 | 18.90 |
| ATOM | 1703 | OD2 | ASP | A | 240 | 0.385 | 0.702 | 12.065 | 1.00 | 22.97 |
| ATOM | 1704 | C | ASP | A | 240 | 0.856 | 0.095 | 16.264 | 1.00 | 20.29 |
| ATOM | 1705 | O | ASP | A | 240 | -0.360 | -0.043 | 16.398 | 1.00 | 20.46 |
| ATOM | 1706 | N | GLY | A | 241 | 1.735 | -0.774 | 16.754 | 1.00 | 21.06 |
| ATOM | 1707 | CA | GLY | A | 241 | 1.255 | -1.941 | 17.473 | 1.00 | 20.92 |
| ATOM | 1708 | C | GLY | A | 241 | 0.363 | -2.797 | 16.584 | 1.00 | 22.72 |
| ATOM | 1709 | O | GLY | A | 241 | -0.504 | -3.515 | 17.071 | 1.00 | 23.64 |
| ATOM | 1710 | N | GLY | A | 242 | 0.569 | -2.706 | 15.276 | 1.00 | 24.00 |
| ATOM | 1711 | CA | GLY | A | 242 | -0.206 | -3.490 | 14.335 | 1.00 | 23.14 |
| ATOM | 1712 | C | GLY | A | 242 | -1.515 | -2.875 | 13.886 | 1.00 | 24.74 |
| ATOM | 1713 | O | GLY | A | 242 | -2.251 | -3.488 | 13.115 | 1.00 | 25.97 |
| ATOM | 1714 | N | MET | A | 243 | -1.808 | -1.668 | 14.353 | 1.00 | 24.24 |
| ATOM | 1715 | CA | MET | A | 243 | -3.051 | -0.978 | 14.006 | 1.00 | 25.42 |

Figure 3 (suite 23)

| | | | | | | | | | | |
|---|---|---|---|---|---|---|---|---|---|---|
| ATOM | 1716 | CB | MET | A | 243 | -3.097 | 0.372 | 14.721 | 1.00 | 27.16 |
| ATOM | 1717 | CG | MET | A | 243 | -4.272 | 1.239 | 14.334 | 1.00 | 26.23 |
| ATOM | 1718 | SD | MET | A | 243 | -4.087 | 2.914 | 14.954 | 1.00 | 31.70 |
| ATOM | 1719 | CE | MET | A | 243 | -4.991 | 2.797 | 16.466 | 1.00 | 33.90 |
| ATOM | 1720 | C | MET | A | 243 | -3.361 | -0.739 | 12.519 | 1.00 | 25.54 |
| ATOM | 1721 | O | MET | A | 243 | -4.487 | -0.968 | 12.076 | 1.00 | 25.69 |
| ATOM | 1722 | N | GLY | A | 244 | -2.372 | -0.287 | 11.754 | 1.00 | 24.69 |
| ATOM | 1723 | CA | GLY | A | 244 | -2.597 | 0.028 | 10.351 | 1.00 | 25.06 |
| ATOM | 1724 | C | GLY | A | 244 | -2.589 | -1.016 | 9.246 | 1.00 | 24.97 |
| ATOM | 1725 | O | GLY | A | 244 | -2.528 | -0.663 | 8.067 | 1.00 | 24.27 |
| ATOM | 1726 | N | MET | A | 245 | -2.655 | -2.294 | 9.583 | 1.00 | 27.29 |
| ATOM | 1727 | CA | MET | A | 245 | -2.656 | -3.317 | 8.534 | 1.00 | 28.40 |
| ATOM | 1728 | CB | MET | A | 245 | -2.644 | -4.709 | 9.166 | 1.00 | 28.47 |
| ATOM | 1729 | CG | MET | A | 245 | -1.426 | -4.960 | 10.062 | 1.00 | 26.77 |
| ATOM | 1730 | SD | MET | A | 245 | -1.410 | -6.611 | 10.791 | 1.00 | 28.00 |
| ATOM | 1731 | CE | MET | A | 245 | -2.767 | -6.461 | 11.933 | 1.00 | 25.55 |
| ATOM | 1732 | C | MET | A | 245 | -3.858 | -3.160 | 7.579 | 1.00 | 29.48 |
| ATOM | 1733 | O | MET | A | 245 | -4.983 | -2.870 | 7.997 | 1.00 | 28.17 |
| ATOM | 1734 | N | GLY | A | 246 | -3.594 | -3.335 | 6.288 | 1.00 | 29.90 |
| ATOM | 1735 | CA | GLY | A | 246 | -4.625 | -3.200 | 5.281 | 1.00 | 29.51 |
| ATOM | 1736 | C | GLY | A | 246 | -3.977 | -3.013 | 3.925 | 1.00 | 30.03 |
| ATOM | 1737 | O | GLY | A | 246 | -2.854 | -3.469 | 3.702 | 1.00 | 28.94 |
| ATOM | 1738 | N | HIS | A | 247 | -4.670 | -2.338 | 3.016 | 1.00 | 29.74 |
| ATOM | 1739 | CA | HIS | A | 247 | -4.131 | -2.110 | 1.678 | 1.00 | 31.69 |
| ATOM | 1740 | CB | HIS | A | 247 | -4.196 | -3.409 | 0.855 | 1.00 | 31.36 |
| ATOM | 1741 | CG | HIS | A | 247 | -3.659 | -3.272 | -0.537 | 1.00 | 33.77 |
| ATOM | 1742 | CD2 | HIS | A | 247 | -2.390 | -3.296 | -1.010 | 1.00 | 34.17 |
| ATOM | 1743 | ND1 | HIS | A | 247 | -4.470 | -3.042 | -1.629 | 1.00 | 33.27 |
| ATOM | 1744 | CE1 | HIS | A | 247 | -3.724 | -2.931 | -2.714 | 1.00 | 32.18 |
| ATOM | 1745 | NE2 | HIS | A | 247 | -2.458 | -3.080 | -2.366 | 1.00 | 34.50 |
| ATOM | 1746 | C | HIS | A | 247 | -4.868 | -0.984 | 0.947 | 1.00 | 31.58 |
| ATOM | 1747 | OT1 | HIS | A | 247 | -5.854 | -0.472 | 1.516 | 1.00 | 31.24 |
| ATOM | 1748 | OXT | HIS | A | 247 | -4.449 | -0.623 | -0.179 | 1.00 | 30.42 |
| ATOM | 1749 | CB | ALA | B | 9 | 17.154 | 5.553 | 27.771 | 1.00 | 43.71 |
| ATOM | 1750 | C | ALA | B | 9 | 16.540 | 4.268 | 25.728 | 1.00 | 42.48 |
| ATOM | 1751 | O | ALA | B | 9 | 15.476 | 3.647 | 25.686 | 1.00 | 41.28 |
| ATOM | 1752 | N | ALA | B | 9 | 15.236 | 6.258 | 26.374 | 1.00 | 45.05 |
| ATOM | 1753 | CA | ALA | B | 9 | 16.603 | 5.652 | 26.356 | 1.00 | 43.66 |
| ATOM | 1754 | N | LYS | B | 10 | 17.677 | 3.790 | 25.230 | 1.00 | 42.03 |
| ATOM | 1755 | CA | LYS | B | 10 | 17.724 | 2.472 | 24.612 | 1.00 | 41.62 |
| ATOM | 1756 | CB | LYS | B | 10 | 19.099 | 2.217 | 23.993 | 1.00 | 39.98 |
| ATOM | 1757 | CG | LYS | B | 10 | 19.235 | 0.835 | 23.386 | 1.00 | 39.59 |
| ATOM | 1758 | CD | LYS | B | 10 | 20.367 | 0.769 | 22.375 | 1.00 | 38.11 |
| ATOM | 1759 | CE | LYS | B | 10 | 20.397 | -0.585 | 21.693 | 1.00 | 36.74 |
| ATOM | 1760 | NZ | LYS | B | 10 | 21.273 | -0.580 | 20.494 | 1.00 | 35.01 |
| ATOM | 1761 | C | LYS | B | 10 | 17.418 | 1.396 | 25.650 | 1.00 | 41.79 |
| ATOM | 1762 | O | LYS | B | 10 | 18.100 | 1.297 | 26.671 | 1.00 | 41.07 |
| ATOM | 1763 | N | PRO | B | 11 | 16.374 | 0.584 | 25.411 | 1.00 | 42.08 |
| ATOM | 1764 | CD | PRO | B | 11 | 15.491 | 0.536 | 24.234 | 1.00 | 41.28 |
| ATOM | 1765 | CA | PRO | B | 11 | 16.024 | -0.473 | 26.365 | 1.00 | 41.25 |
| ATOM | 1766 | CB | PRO | B | 11 | 14.868 | -1.195 | 25.675 | 1.00 | 41.25 |
| ATOM | 1767 | CG | PRO | B | 11 | 14.267 | -0.135 | 24.802 | 1.00 | 42.09 |
| ATOM | 1768 | C | PRO | B | 11 | 17.229 | -1.383 | 26.569 | 1.00 | 40.21 |
| ATOM | 1769 | O | PRO | B | 11 | 17.984 | -1.633 | 25.630 | 1.00 | 40.86 |
| ATOM | 1770 | N | PRO | B | 12 | 17.428 | -1.883 | 27.799 | 1.00 | 39.08 |
| ATOM | 1771 | CD | PRO | B | 12 | 16.620 | -1.701 | 29.018 | 1.00 | 37.96 |
| ATOM | 1772 | CA | PRO | B | 12 | 18.574 | -2.762 | 28.052 | 1.00 | 37.90 |
| ATOM | 1773 | CB | PRO | B | 12 | 18.529 | -2.961 | 29.566 | 1.00 | 37.87 |
| ATOM | 1774 | CG | PRO | B | 12 | 17.069 | -2.863 | 29.872 | 1.00 | 38.28 |
| ATOM | 1775 | C | PRO | B | 12 | 18.494 | -4.074 | 27.270 | 1.00 | 36.67 |
| ATOM | 1776 | O | PRO | B | 12 | 17.421 | -4.655 | 27.096 | 1.00 | 36.05 |
| ATOM | 1777 | N | PHE | B | 13 | 19.645 | -4.525 | 26.791 | 1.00 | 35.84 |
| ATOM | 1778 | CA | PHE | B | 13 | 19.736 | -5.752 | 26.014 | 1.00 | 35.92 |
| ATOM | 1779 | CB | PHE | B | 13 | 21.181 | -5.964 | 25.569 | 1.00 | 36.09 |
| ATOM | 1780 | CG | PHE | B | 13 | 21.394 | -7.225 | 24.794 | 1.00 | 36.10 |
| ATOM | 1781 | CD1 | PHE | B | 13 | 21.135 | -7.266 | 23.430 | 1.00 | 33.67 |
| ATOM | 1782 | CD2 | PHE | B | 13 | 21.842 | -8.381 | 25.435 | 1.00 | 36.30 |
| ATOM | 1783 | CE1 | PHE | B | 13 | 21.317 | -8.442 | 22.708 | 1.00 | 34.31 |
| ATOM | 1784 | CE2 | PHE | B | 13 | 22.029 | -9.564 | 24.723 | 1.00 | 35.76 |
| ATOM | 1785 | CZ | PHE | B | 13 | 21.765 | -9.595 | 23.355 | 1.00 | 34.64 |
| ATOM | 1786 | C | PHE | B | 13 | 19.265 | -6.986 | 26.782 | 1.00 | 34.95 |
| ATOM | 1787 | O | PHE | B | 13 | 19.500 | -7.115 | 27.986 | 1.00 | 34.24 |
| ATOM | 1788 | N | VAL | B | 14 | 18.592 | -7.884 | 26.070 | 1.00 | 33.15 |

Figure 3 (suite 24)

| | | | | | | | | | | |
|---|---|---|---|---|---|---|---|---|---|---|
| ATOM | 1789 | CA | VAL | B | 14 | 18.115 | -9.137 | 26.647 | 1.00 | 31.02 |
| ATOM | 1790 | CB | VAL | B | 14 | 16.574 | -9.212 | 26.701 | 1.00 | 31.39 |
| ATOM | 1791 | CG1 | VAL | B | 14 | 16.149 | -10.492 | 27.410 | 1.00 | 32.22 |
| ATOM | 1792 | CG2 | VAL | B | 14 | 16.011 | -8.003 | 27.421 | 1.00 | 31.20 |
| ATOM | 1793 | C | VAL | B | 14 | 18.612 | -10.254 | 25.741 | 1.00 | 29.74 |
| ATOM | 1794 | O | VAL | B | 14 | 18.442 | -10.194 | 24.522 | 1.00 | 29.85 |
| ATOM | 1795 | N | SER | B | 15 | 19.249 | -11.261 | 26.329 | 1.00 | 26.64 |
| ATOM | 1796 | CA | SER | B | 15 | 19.754 | -12.384 | 25.544 | 1.00 | 23.65 |
| ATOM | 1797 | CB | SER | B | 15 | 20.898 | -13.084 | 26.275 | 1.00 | 20.78 |
| ATOM | 1798 | OG | SER | B | 15 | 21.610 | -13.913 | 25.384 | 1.00 | 20.22 |
| ATOM | 1799 | C | SER | B | 15 | 18.591 | -13.343 | 25.351 | 1.00 | 24.03 |
| ATOM | 1800 | O | SER | B | 15 | 18.166 | -14.030 | 26.292 | 1.00 | 25.45 |
| ATOM | 1801 | N | ARG | B | 16 | 18.077 | -13.374 | 24.125 | 1.00 | 22.86 |
| ATOM | 1802 | CA | ARG | B | 16 | 16.932 | -14.211 | 23.772 | 1.00 | 19.51 |
| ATOM | 1803 | CB | ARG | B | 16 | 16.021 | -13.460 | 22.795 | 1.00 | 18.85 |
| ATOM | 1804 | CG | ARG | B | 16 | 15.513 | -12.113 | 23.280 | 1.00 | 18.34 |
| ATOM | 1805 | CD | ARG | B | 16 | 14.530 | -12.229 | 24.418 | 1.00 | 18.76 |
| ATOM | 1806 | NE | ARG | B | 16 | 13.809 | -10.972 | 24.622 | 1.00 | 23.70 |
| ATOM | 1807 | CZ | ARG | B | 16 | 13.005 | -10.724 | 25.653 | 1.00 | 23.91 |
| ATOM | 1808 | NH1 | ARG | B | 16 | 12.816 | -11.650 | 26.582 | 1.00 | 25.26 |
| ATOM | 1809 | NH2 | ARG | B | 16 | 12.392 | -9.549 | 25.757 | 1.00 | 22.66 |
| ATOM | 1810 | C | ARG | B | 16 | 17.319 | -15.542 | 23.131 | 1.00 | 18.90 |
| ATOM | 1811 | O | ARG | B | 16 | 18.354 | -15.657 | 22.466 | 1.00 | 17.81 |
| ATOM | 1812 | N | SER | B | 17 | 16.471 | -16.549 | 23.335 | 1.00 | 19.17 |
| ATOM | 1813 | CA | SER | B | 17 | 16.698 | -17.851 | 22.721 | 1.00 | 18.57 |
| ATOM | 1814 | CB | SER | B | 17 | 15.856 | -18.930 | 23.410 | 1.00 | 19.35 |
| ATOM | 1815 | OG | SER | B | 17 | 16.007 | -20.186 | 22.754 | 1.00 | 23.63 |
| ATOM | 1816 | C | SER | B | 17 | 16.233 | -17.665 | 21.277 | 1.00 | 18.08 |
| ATOM | 1817 | O | SER | B | 17 | 15.065 | -17.353 | 21.026 | 1.00 | 15.62 |
| ATOM | 1818 | N | VAL | B | 18 | 17.136 | -17.860 | 20.329 | 1.00 | 17.06 |
| ATOM | 1819 | CA | VAL | B | 18 | 16.796 | -17.661 | 18.936 | 1.00 | 18.80 |
| ATOM | 1820 | CB | VAL | B | 18 | 17.658 | -16.535 | 18.303 | 1.00 | 19.98 |
| ATOM | 1821 | CG1 | VAL | B | 18 | 17.352 | -16.412 | 16.817 | 1.00 | 20.28 |
| ATOM | 1822 | CG2 | VAL | B | 18 | 17.399 | -15.213 | 19.011 | 1.00 | 17.75 |
| ATOM | 1823 | C | VAL | B | 18 | 16.952 | -18.877 | 18.046 | 1.00 | 19.81 |
| ATOM | 1824 | O | VAL | B | 18 | 17.953 | -19.605 | 18.111 | 1.00 | 18.57 |
| ATOM | 1825 | N | LEU | B | 19 | 15.959 | -19.075 | 17.187 | 1.00 | 19.76 |
| ATOM | 1826 | CA | LEU | B | 19 | 15.995 | -20.170 | 16.238 | 1.00 | 19.20 |
| ATOM | 1827 | CB | LEU | B | 19 | 14.848 | -21.155 | 16.484 | 1.00 | 16.68 |
| ATOM | 1828 | CG | LEU | B | 19 | 14.700 | -22.209 | 15.385 | 1.00 | 16.71 |
| ATOM | 1829 | CD1 | LEU | B | 19 | 16.035 | -22.868 | 15.120 | 1.00 | 17.94 |
| ATOM | 1830 | CD2 | LEU | B | 19 | 13.680 | -23.245 | 15.796 | 1.00 | 17.88 |
| ATOM | 1831 | C | LEU | B | 19 | 15.872 | -19.581 | 14.851 | 1.00 | 20.24 |
| ATOM | 1832 | O | LEU | B | 19 | 14.907 | -18.875 | 14.551 | 1.00 | 22.09 |
| ATOM | 1833 | N | VAL | B | 20 | 16.854 | -19.857 | 14.006 | 1.00 | 20.16 |
| ATOM | 1834 | CA | VAL | B | 20 | 16.832 | -19.364 | 12.642 | 1.00 | 21.61 |
| ATOM | 1835 | CB | VAL | B | 20 | 18.128 | -18.578 | 12.299 | 1.00 | 22.44 |
| ATOM | 1836 | CG1 | VAL | B | 20 | 17.994 | -17.950 | 10.923 | 1.00 | 22.78 |
| ATOM | 1837 | CG2 | VAL | B | 20 | 18.409 | -17.522 | 13.370 | 1.00 | 21.24 |
| ATOM | 1838 | C | VAL | B | 20 | 16.734 | -20.559 | 11.691 | 1.00 | 22.59 |
| ATOM | 1839 | O | VAL | B | 20 | 17.695 | -21.312 | 11.551 | 1.00 | 22.76 |
| ATOM | 1840 | N | THR | B | 21 | 15.589 | -20.738 | 11.034 | 1.00 | 21.44 |
| ATOM | 1841 | CA | THR | B | 21 | 15.441 | -21.859 | 10.112 | 1.00 | 23.20 |
| ATOM | 1842 | CB | THR | B | 21 | 13.970 | -22.128 | 9.763 | 1.00 | 22.66 |
| ATOM | 1843 | OG1 | THR | B | 21 | 13.530 | -21.181 | 8.785 | 1.00 | 26.15 |
| ATOM | 1844 | CG2 | THR | B | 21 | 13.108 | -22.032 | 11.007 | 1.00 | 21.36 |
| ATOM | 1845 | C | THR | B | 21 | 16.205 | -21.604 | 8.827 | 1.00 | 23.77 |
| ATOM | 1846 | O | THR | B | 21 | 16.048 | -20.564 | 8.205 | 1.00 | 25.68 |
| ATOM | 1847 | N | GLY | B | 22 | 17.034 | -22.561 | 8.423 | 1.00 | 25.29 |
| ATOM | 1848 | CA | GLY | B | 22 | 17.821 | -22.382 | 7.220 | 1.00 | 27.78 |
| ATOM | 1849 | C | GLY | B | 22 | 18.943 | -21.386 | 7.466 | 1.00 | 31.31 |
| ATOM | 1850 | O | GLY | B | 22 | 19.330 | -20.638 | 6.571 | 1.00 | 32.05 |
| ATOM | 1851 | N | GLY | B | 23 | 19.480 | -21.382 | 8.682 | 1.00 | 33.62 |
| ATOM | 1852 | CA | GLY | B | 23 | 20.542 | -20.445 | 9.014 | 1.00 | 36.28 |
| ATOM | 1853 | C | GLY | B | 23 | 21.975 | -20.941 | 8.889 | 1.00 | 38.02 |
| ATOM | 1854 | O | GLY | B | 23 | 22.839 | -20.527 | 9.666 | 1.00 | 37.93 |
| ATOM | 1855 | N | ASN | B | 24 | 22.237 | -21.802 | 7.907 | 1.00 | 38.84 |
| ATOM | 1856 | CA | ASN | B | 24 | 23.576 | -22.350 | 7.700 | 1.00 | 37.63 |
| ATOM | 1857 | CB | ASN | B | 24 | 23.516 | -23.879 | 7.688 | 1.00 | 40.22 |
| ATOM | 1858 | CG | ASN | B | 24 | 22.649 | -24.415 | 6.565 | 1.00 | 41.64 |
| ATOM | 1859 | OD1 | ASN | B | 24 | 21.423 | -24.262 | 6.576 | 1.00 | 41.57 |
| ATOM | 1860 | ND2 | ASN | B | 24 | 23.285 | -25.034 | 5.578 | 1.00 | 42.65 |
| ATOM | 1861 | C | ASN | B | 24 | 24.224 | -21.868 | 6.406 | 1.00 | 37.51 |

Figure 3 (suite 25)

| | | | | | | | | | | |
|---|---|---|---|---|---|---|---|---|---|---|
| ATOM | 1862 | O | ASN | B | 24 | 25.425 | -22.042 | 6.207 | 1.00 | 39.57 |
| ATOM | 1863 | N | ARG | B | 25 | 23.434 | -21.270 | 5.520 | 1.00 | 35.02 |
| ATOM | 1864 | CA | ARG | B | 25 | 23.974 | -20.772 | 4.261 | 1.00 | 32.76 |
| ATOM | 1865 | CB | ARG | B | 25 | 23.673 | -21.771 | 3.134 | 1.00 | 33.60 |
| ATOM | 1866 | CG | ARG | B | 25 | 24.429 | -23.089 | 3.254 | 0.00 | 33.45 |
| ATOM | 1867 | CD | ARG | B | 25 | 25.934 | -22.868 | 3.180 | 0.00 | 33.63 |
| ATOM | 1868 | NE | ARG | B | 25 | 26.695 | -24.110 | 3.298 | 0.00 | 33.70 |
| ATOM | 1869 | CZ | ARG | B | 25 | 26.619 | -25.124 | 2.441 | 0.00 | 33.75 |
| ATOM | 1870 | NH1 | ARG | B | 25 | 25.811 | -25.053 | 1.391 | 0.00 | 33.78 |
| ATOM | 1871 | NH2 | ARG | B | 25 | 27.357 | -26.209 | 2.629 | 0.00 | 33.78 |
| ATOM | 1872 | C | ARG | B | 25 | 23.434 | -19.379 | 3.893 | 1.00 | 31.39 |
| ATOM | 1873 | O | ARG | B | 25 | 22.454 | -18.915 | 4.466 | 1.00 | 31.05 |
| ATOM | 1874 | N | GLY | B | 26 | 24.092 | -18.721 | 2.943 | 1.00 | 28.47 |
| ATOM | 1875 | CA | GLY | B | 26 | 23.683 | -17.399 | 2.497 | 1.00 | 29.70 |
| ATOM | 1876 | C | GLY | B | 26 | 23.118 | -16.457 | 3.550 | 1.00 | 30.10 |
| ATOM | 1877 | O | GLY | B | 26 | 23.713 | -16.264 | 4.612 | 1.00 | 29.40 |
| ATOM | 1878 | N | ILE | B | 27 | 21.960 | -15.873 | 3.241 | 1.00 | 29.44 |
| ATOM | 1879 | CA | ILE | B | 27 | 21.276 | -14.941 | 4.132 | 1.00 | 28.41 |
| ATOM | 1880 | CB | ILE | B | 27 | 19.892 | -14.525 | 3.546 | 1.00 | 29.50 |
| ATOM | 1881 | CG2 | ILE | B | 27 | 19.056 | -13.817 | 4.605 | 1.00 | 30.18 |
| ATOM | 1882 | CG1 | ILE | B | 27 | 20.087 | -13.623 | 2.326 | 1.00 | 30.67 |
| ATOM | 1883 | CD | ILE | B | 27 | 18.783 | -13.251 | 1.615 | 1.00 | 26.26 |
| ATOM | 1884 | C | ILE | B | 27 | 21.060 | -15.517 | 5.525 | 1.00 | 26.94 |
| ATOM | 1885 | O | ILE | B | 27 | 21.373 | -14.868 | 6.529 | 1.00 | 26.93 |
| ATOM | 1886 | N | GLY | B | 28 | 20.509 | -16.729 | 5.586 | 1.00 | 24.90 |
| ATOM | 1887 | CA | GLY | B | 28 | 20.251 | -17.362 | 6.869 | 1.00 | 23.60 |
| ATOM | 1888 | C | GLY | B | 28 | 21.482 | -17.433 | 7.763 | 1.00 | 23.80 |
| ATOM | 1889 | O | GLY | B | 28 | 21.407 | -17.213 | 8.976 | 1.00 | 21.64 |
| ATOM | 1890 | N | LEU | B | 29 | 22.623 | -17.740 | 7.157 | 1.00 | 22.57 |
| ATOM | 1891 | CA | LEU | B | 29 | 23.880 | -17.839 | 7.897 | 1.00 | 24.09 |
| ATOM | 1892 | CB | LEU | B | 29 | 24.992 | -18.377 | 6.975 | 1.00 | 22.20 |
| ATOM | 1893 | CG | LEU | B | 29 | 26.413 | -18.428 | 7.546 | 1.00 | 24.33 |
| ATOM | 1894 | CD1 | LEU | B | 29 | 26.438 | -19.140 | 8.882 | 1.00 | 23.15 |
| ATOM | 1895 | CD2 | LEU | B | 29 | 27.309 | -19.126 | 6.547 | 1.00 | 25.85 |
| ATOM | 1896 | C | LEU | B | 29 | 24.240 | -16.458 | 8.449 | 1.00 | 21.49 |
| ATOM | 1897 | O | LEU | B | 29 | 24.513 | -16.311 | 9.639 | 1.00 | 20.10 |
| ATOM | 1898 | N | ALA | B | 30 | 24.212 | -15.456 | 7.573 | 1.00 | 20.50 |
| ATOM | 1899 | CA | ALA | B | 30 | 24.504 | -14.069 | 7.943 | 1.00 | 19.62 |
| ATOM | 1900 | CB | ALA | B | 30 | 24.381 | -13.158 | 6.709 | 1.00 | 18.40 |
| ATOM | 1901 | C | ALA | B | 30 | 23.555 | -13.601 | 9.057 | 1.00 | 18.67 |
| ATOM | 1902 | O | ALA | B | 30 | 23.959 | -12.873 | 9.964 | 1.00 | 20.36 |
| ATOM | 1903 | N | ILE | B | 31 | 22.300 | -14.033 | 9.019 | 1.00 | 17.44 |
| ATOM | 1904 | CA | ILE | B | 31 | 21.375 | -13.635 | 10.074 | 1.00 | 17.69 |
| ATOM | 1905 | CB | ILE | B | 31 | 19.912 | -14.036 | 9.740 | 1.00 | 16.30 |
| ATOM | 1906 | CG2 | ILE | B | 31 | 18.994 | -13.792 | 10.934 | 1.00 | 15.41 |
| ATOM | 1907 | CG1 | ILE | B | 31 | 19.400 | -13.218 | 8.559 | 1.00 | 17.45 |
| ATOM | 1908 | CD | ILE | B | 31 | 17.975 | -13.596 | 8.135 | 1.00 | 10.76 |
| ATOM | 1909 | C | ILE | B | 31 | 21.761 | -14.262 | 11.412 | 1.00 | 19.82 |
| ATOM | 1910 | O | ILE | B | 31 | 21.821 | -13.571 | 12.431 | 1.00 | 22.95 |
| ATOM | 1911 | N | ALA | B | 32 | 22.014 | -15.570 | 11.418 | 1.00 | 20.38 |
| ATOM | 1912 | CA | ALA | B | 32 | 22.365 | -16.266 | 12.656 | 1.00 | 22.62 |
| ATOM | 1913 | CB | ALA | B | 32 | 22.511 | -17.767 | 12.397 | 1.00 | 21.68 |
| ATOM | 1914 | C | ALA | B | 32 | 23.645 | -15.725 | 13.283 | 1.00 | 23.91 |
| ATOM | 1915 | O | ALA | B | 32 | 23.709 | -15.529 | 14.495 | 1.00 | 24.11 |
| ATOM | 1916 | N | GLN | B | 33 | 24.662 | -15.500 | 12.458 | 1.00 | 24.17 |
| ATOM | 1917 | CA | GLN | B | 33 | 25.929 | -14.973 | 12.945 | 1.00 | 26.15 |
| ATOM | 1918 | CB | GLN | B | 33 | 26.942 | -14.902 | 11.807 | 1.00 | 25.41 |
| ATOM | 1919 | CG | GLN | B | 33 | 27.630 | -16.226 | 11.535 | 1.00 | 29.07 |
| ATOM | 1920 | CD | GLN | B | 33 | 28.509 | -16.188 | 10.302 | 1.00 | 30.66 |
| ATOM | 1921 | OE1 | GLN | B | 33 | 29.483 | -16.931 | 10.203 | 1.00 | 33.78 |
| ATOM | 1922 | NE2 | GLN | B | 33 | 28.159 | -15.335 | 9.346 | 1.00 | 31.87 |
| ATOM | 1923 | C | GLN | B | 33 | 25.747 | -13.597 | 13.567 | 1.00 | 27.83 |
| ATOM | 1924 | O | GLN | B | 33 | 26.289 | -13.318 | 14.645 | 1.00 | 29.99 |
| ATOM | 1925 | N | ARG | B | 34 | 24.970 | -12.742 | 12.902 | 1.00 | 26.39 |
| ATOM | 1926 | CA | ARG | B | 34 | 24.732 | -11.399 | 13.413 | 1.00 | 24.75 |
| ATOM | 1927 | CB | ARG | B | 34 | 23.909 | -10.574 | 12.409 | 1.00 | 22.05 |
| ATOM | 1928 | CG | ARG | B | 34 | 23.415 | -9.225 | 12.941 | 1.00 | 19.97 |
| ATOM | 1929 | CD | ARG | B | 34 | 24.550 | -8.337 | 13.462 | 1.00 | 19.05 |
| ATOM | 1930 | NE | ARG | B | 34 | 24.022 | -7.165 | 14.165 | 1.00 | 20.18 |
| ATOM | 1931 | CZ | ARG | B | 34 | 23.730 | -6.004 | 13.581 | 1.00 | 18.46 |
| ATOM | 1932 | NH1 | ARG | B | 34 | 23.923 | -5.835 | 12.281 | 1.00 | 17.13 |
| ATOM | 1933 | NH2 | ARG | B | 34 | 23.212 | -5.024 | 14.299 | 1.00 | 19.34 |
| ATOM | 1934 | C | ARG | B | 34 | 24.018 | -11.462 | 14.749 | 1.00 | 24.72 |

Figure 3 (suite 26)

| | | | | | | | | | | |
|---|---|---|---|---|---|---|---|---|---|---|
| ATOM | 1935 | O | ARG | B | 34 | 24.406 | -10.780 | 15.702 | 1.00 | 25.12 |
| ATOM | 1936 | N | LEU | B | 35 | 22.979 | -12.283 | 14.833 | 1.00 | 23.47 |
| ATOM | 1937 | CA | LEU | B | 35 | 22.234 | -12.388 | 16.083 | 1.00 | 22.95 |
| ATOM | 1938 | CB | LEU | B | 35 | 21.019 | -13.307 | 15.907 | 1.00 | 22.64 |
| ATOM | 1939 | CG | LEU | B | 35 | 19.923 | -12.811 | 14.953 | 1.00 | 21.87 |
| ATOM | 1940 | CD1 | LEU | B | 35 | 18.723 | -13.739 | 15.013 | 1.00 | 20.95 |
| ATOM | 1941 | CD2 | LEU | B | 35 | 19.497 | -11.396 | 15.352 | 1.00 | 22.30 |
| ATOM | 1942 | C | LEU | B | 35 | 23.116 | -12.889 | 17.219 | 1.00 | 24.02 |
| ATOM | 1943 | O | LEU | B | 35 | 22.921 | -12.508 | 18.381 | 1.00 | 24.92 |
| ATOM | 1944 | N | ALA | B | 36 | 24.080 | -13.751 | 16.891 | 1.00 | 22.75 |
| ATOM | 1945 | CA | ALA | B | 36 | 24.998 | -14.280 | 17.902 | 1.00 | 23.33 |
| ATOM | 1946 | CB | ALA | B | 36 | 25.843 | -15.416 | 17.321 | 1.00 | 22.14 |
| ATOM | 1947 | C | ALA | B | 36 | 25.895 | -13.131 | 18.361 | 1.00 | 22.12 |
| ATOM | 1948 | O | ALA | B | 36 | 26.051 | -12.897 | 19.555 | 1.00 | 22.95 |
| ATOM | 1949 | N | ALA | B | 37 | 26.469 | -12.410 | 17.401 | 1.00 | 20.31 |
| ATOM | 1950 | CA | ALA | B | 37 | 27.325 | -11.266 | 17.709 | 1.00 | 19.99 |
| ATOM | 1951 | CB | ALA | B | 37 | 27.783 | -10.580 | 16.423 | 1.00 | 16.43 |
| ATOM | 1952 | C | ALA | B | 37 | 26.538 | -10.297 | 18.581 | 1.00 | 19.90 |
| ATOM | 1953 | O | ALA | B | 37 | 27.099 | -9.665 | 19.467 | 1.00 | 21.32 |
| ATOM | 1954 | N | ASP | B | 38 | 25.231 | -10.198 | 18.343 | 1.00 | 21.79 |
| ATOM | 1955 | CA | ASP | B | 38 | 24.381 | -9.319 | 19.144 | 1.00 | 22.07 |
| ATOM | 1956 | CB | ASP | B | 38 | 22.947 | -9.275 | 18.606 | 1.00 | 21.59 |
| ATOM | 1957 | CG | ASP | B | 38 | 22.779 | -8.394 | 17.376 | 1.00 | 19.07 |
| ATOM | 1958 | OD1 | ASP | B | 38 | 23.753 | -7.781 | 16.877 | 1.00 | 18.08 |
| ATOM | 1959 | OD2 | ASP | B | 38 | 21.621 | -8.324 | 16.906 | 1.00 | 20.42 |
| ATOM | 1960 | C | ASP | B | 38 | 24.320 | -9.836 | 20.569 | 1.00 | 24.49 |
| ATOM | 1961 | O | ASP | B | 38 | 23.820 | -9.152 | 21.455 | 1.00 | 25.93 |
| ATOM | 1962 | N | GLY | B | 39 | 24.792 | -11.057 | 20.794 | 1.00 | 24.49 |
| ATOM | 1963 | CA | GLY | B | 39 | 24.755 | -11.608 | 22.141 | 1.00 | 23.90 |
| ATOM | 1964 | C | GLY | B | 39 | 23.553 | -12.489 | 22.458 | 1.00 | 22.88 |
| ATOM | 1965 | O | GLY | B | 39 | 23.256 | -12.756 | 23.621 | 1.00 | 23.42 |
| ATOM | 1966 | N | HIS | B | 40 | 22.844 | -12.929 | 21.427 | 1.00 | 23.38 |
| ATOM | 1967 | CA | HIS | B | 40 | 21.691 | -13.812 | 21.618 | 1.00 | 21.93 |
| ATOM | 1968 | CB | HIS | B | 40 | 20.700 | -13.657 | 20.467 | 1.00 | 18.41 |
| ATOM | 1969 | CG | HIS | B | 40 | 19.919 | -12.384 | 20.500 | 1.00 | 16.78 |
| ATOM | 1970 | CD2 | HIS | B | 40 | 19.735 | -11.429 | 19.557 | 1.00 | 13.48 |
| ATOM | 1971 | ND1 | HIS | B | 40 | 19.164 | -12.004 | 21.586 | 1.00 | 15.71 |
| ATOM | 1972 | CE1 | HIS | B | 40 | 18.544 | -10.870 | 21.310 | 1.00 | 15.44 |
| ATOM | 1973 | NE2 | HIS | B | 40 | 18.875 | -10.502 | 20.085 | 1.00 | 13.87 |
| ATOM | 1974 | C | HIS | B | 40 | 22.190 | -15.262 | 21.617 | 1.00 | 21.70 |
| ATOM | 1975 | O | HIS | B | 40 | 23.317 | -15.525 | 21.188 | 1.00 | 21.22 |
| ATOM | 1976 | N | LYS | B | 41 | 21.351 | -16.181 | 22.098 | 1.00 | 20.32 |
| ATOM | 1977 | CA | LYS | B | 41 | 21.659 | -17.620 | 22.106 | 1.00 | 21.42 |
| ATOM | 1978 | CB | LYS | B | 41 | 20.960 | -18.312 | 23.278 | 1.00 | 21.95 |
| ATOM | 1979 | CG | LYS | B | 41 | 21.594 | -18.008 | 24.613 | 1.00 | 24.24 |
| ATOM | 1980 | CD | LYS | B | 41 | 20.769 | -18.499 | 25.768 | 1.00 | 25.91 |
| ATOM | 1981 | CE | LYS | B | 41 | 19.449 | -17.755 | 25.867 | 1.00 | 27.29 |
| ATOM | 1982 | NZ | LYS | B | 41 | 18.682 | -18.164 | 27.077 | 1.00 | 30.66 |
| ATOM | 1983 | C | LYS | B | 41 | 21.105 | -18.146 | 20.785 | 1.00 | 22.88 |
| ATOM | 1984 | O | LYS | B | 41 | 19.897 | -18.348 | 20.645 | 1.00 | 22.36 |
| ATOM | 1985 | N | VAL | B | 42 | 21.988 | -18.380 | 19.824 | 1.00 | 22.46 |
| ATOM | 1986 | CA | VAL | B | 42 | 21.556 | -18.801 | 18.504 | 1.00 | 24.04 |
| ATOM | 1987 | CB | VAL | B | 42 | 22.346 | -18.017 | 17.425 | 1.00 | 22.50 |
| ATOM | 1988 | CG1 | VAL | B | 42 | 21.880 | -18.405 | 16.030 | 1.00 | 22.48 |
| ATOM | 1989 | CG2 | VAL | B | 42 | 22.161 | -16.512 | 17.644 | 1.00 | 21.82 |
| ATOM | 1990 | C | VAL | B | 42 | 21.603 | -20.299 | 18.169 | 1.00 | 26.77 |
| ATOM | 1991 | O | VAL | B | 42 | 22.500 | -21.033 | 18.590 | 1.00 | 27.62 |
| ATOM | 1992 | N | ALA | B | 43 | 20.610 | -20.729 | 17.391 | 1.00 | 28.60 |
| ATOM | 1993 | CA | ALA | B | 43 | 20.482 | -22.104 | 16.932 | 1.00 | 29.48 |
| ATOM | 1994 | CB | ALA | B | 43 | 19.576 | -22.894 | 17.867 | 1.00 | 30.00 |
| ATOM | 1995 | C | ALA | B | 43 | 19.866 | -22.044 | 15.546 | 1.00 | 30.55 |
| ATOM | 1996 | O | ALA | B | 43 | 18.911 | -21.305 | 15.329 | 1.00 | 31.96 |
| ATOM | 1997 | N | VAL | B | 44 | 20.412 | -22.808 | 14.611 | 1.00 | 30.32 |
| ATOM | 1998 | CA | VAL | B | 44 | 19.881 | -22.838 | 13.260 | 1.00 | 31.96 |
| ATOM | 1999 | CB | VAL | B | 44 | 20.957 | -22.449 | 12.231 | 1.00 | 31.68 |
| ATOM | 2000 | CG1 | VAL | B | 44 | 21.546 | -21.091 | 12.602 | 1.00 | 31.37 |
| ATOM | 2001 | CG2 | VAL | B | 44 | 22.039 | -23.516 | 12.172 | 1.00 | 30.17 |
| ATOM | 2002 | C | VAL | B | 44 | 19.367 | -24.241 | 12.918 | 1.00 | 34.54 |
| ATOM | 2003 | O | VAL | B | 44 | 19.636 | -25.207 | 13.637 | 1.00 | 33.21 |
| ATOM | 2004 | N | THR | B | 45 | 18.618 | -24.339 | 11.823 | 1.00 | 35.50 |
| ATOM | 2005 | CA | THR | B | 45 | 18.087 | -25.613 | 11.354 | 1.00 | 36.18 |
| ATOM | 2006 | CB | THR | B | 45 | 16.555 | -25.585 | 11.219 | 1.00 | 35.68 |
| ATOM | 2007 | OG1 | THR | B | 45 | 16.184 | -24.689 | 10.168 | 1.00 | 35.21 |

Figure 3 (suite 27)

| | | | | | | | | | | |
|---|---|---|---|---|---|---|---|---|---|---|
| ATOM | 2008 | CG2 | THR | B | 45 | 15.912 | -25.123 | 12.506 | 1.00 | 35.44 |
| ATOM | 2009 | C | THR | B | 45 | 18.695 | -25.812 | 9.972 | 1.00 | 38.54 |
| ATOM | 2010 | O | THR | B | 45 | 18.871 | -24.851 | 9.227 | 1.00 | 38.94 |
| ATOM | 2011 | N | HIS | B | 46 | 19.022 | -27.049 | 9.624 | 1.00 | 41.43 |
| ATOM | 2012 | CA | HIS | B | 46 | 19.634 | -27.315 | 8.325 | 1.00 | 44.35 |
| ATOM | 2013 | CB | HIS | B | 46 | 21.147 | -27.405 | 8.501 | 1.00 | 45.53 |
| ATOM | 2014 | CG | HIS | B | 46 | 21.563 | -28.418 | 9.519 | 1.00 | 48.45 |
| ATOM | 2015 | CD2 | HIS | B | 46 | 21.742 | -28.314 | 10.857 | 1.00 | 49.14 |
| ATOM | 2016 | ND1 | HIS | B | 46 | 21.757 | -29.747 | 9.209 | 1.00 | 49.27 |
| ATOM | 2017 | CE1 | HIS | B | 46 | 22.037 | -30.418 | 10.312 | 1.00 | 49.26 |
| ATOM | 2018 | NE2 | HIS | B | 46 | 22.033 | -29.572 | 11.327 | 1.00 | 51.24 |
| ATOM | 2019 | C | HIS | B | 46 | 19.113 | -28.602 | 7.700 | 1.00 | 46.14 |
| ATOM | 2020 | O | HIS | B | 46 | 18.351 | -29.340 | 8.323 | 1.00 | 45.27 |
| ATOM | 2021 | N | ARG | B | 47 | 19.534 | -28.859 | 6.464 | 1.00 | 49.84 |
| ATOM | 2022 | CA | ARG | B | 47 | 19.137 | -30.058 | 5.733 | 1.00 | 53.46 |
| ATOM | 2023 | CB | ARG | B | 47 | 19.170 | -29.790 | 4.228 | 1.00 | 55.79 |
| ATOM | 2024 | CG | ARG | B | 47 | 18.463 | -28.518 | 3.808 | 1.00 | 58.41 |
| ATOM | 2025 | CD | ARG | B | 47 | 18.605 | -28.290 | 2.313 | 1.00 | 61.09 |
| ATOM | 2026 | NE | ARG | B | 47 | 18.040 | -27.006 | 1.909 | 1.00 | 63.78 |
| ATOM | 2027 | CZ | ARG | B | 47 | 17.936 | -26.594 | 0.648 | 1.00 | 64.88 |
| ATOM | 2028 | NH1 | ARG | B | 47 | 18.360 | -27.365 | -0.346 | 1.00 | 64.74 |
| ATOM | 2029 | NH2 | ARG | B | 47 | 17.408 | -25.407 | 0.380 | 0.00 | 64.58 |
| ATOM | 2030 | C | ARG | B | 47 | 20.118 | -31.183 | 6.058 | 1.00 | 54.68 |
| ATOM | 2031 | O | ARG | B | 47 | 19.756 | -32.198 | 6.663 | 1.00 | 55.73 |
| ATOM | 2032 | N | GLY | B | 48 | 21.367 | -30.991 | 5.647 | 1.00 | 54.36 |
| ATOM | 2033 | CA | GLY | B | 48 | 22.391 | -31.986 | 5.900 | 1.00 | 54.32 |
| ATOM | 2034 | C | GLY | B | 48 | 23.776 | -31.373 | 5.943 | 1.00 | 54.26 |
| ATOM | 2035 | O | GLY | B | 48 | 24.661 | -31.880 | 6.634 | 1.00 | 53.82 |
| ATOM | 2036 | N | SER | B | 49 | 23.957 | -30.278 | 5.207 | 1.00 | 54.07 |
| ATOM | 2037 | CA | SER | B | 49 | 25.238 | -29.579 | 5.137 | 1.00 | 54.22 |
| ATOM | 2038 | CB | SER | B | 49 | 25.072 | -28.262 | 4.377 | 0.00 | 54.34 |
| ATOM | 2039 | OG | SER | B | 49 | 24.105 | -27.435 | 4.997 | 0.00 | 54.32 |
| ATOM | 2040 | C | SER | B | 49 | 25.833 | -29.303 | 6.519 | 1.00 | 54.74 |
| ATOM | 2041 | O | SER | B | 49 | 27.052 | -29.187 | 6.668 | 1.00 | 54.87 |
| ATOM | 2042 | N | GLY | B | 50 | 24.969 | -29.194 | 7.523 | 1.00 | 54.38 |
| ATOM | 2043 | CA | GLY | B | 50 | 25.430 | -28.943 | 8.874 | 1.00 | 54.13 |
| ATOM | 2044 | C | GLY | B | 50 | 25.428 | -27.477 | 9.261 | 1.00 | 54.26 |
| ATOM | 2045 | O | GLY | B | 50 | 25.563 | -26.595 | 8.412 | 1.00 | 53.00 |
| ATOM | 2046 | N | ALA | B | 51 | 25.281 | -27.221 | 10.557 | 1.00 | 53.98 |
| ATOM | 2047 | CA | ALA | B | 51 | 25.267 | -25.860 | 11.076 | 1.00 | 54.35 |
| ATOM | 2048 | CB | ALA | B | 51 | 24.746 | -25.848 | 12.505 | 1.00 | 53.44 |
| ATOM | 2049 | C | ALA | B | 51 | 26.663 | -25.263 | 11.035 | 1.00 | 54.44 |
| ATOM | 2050 | O | ALA | B | 51 | 27.660 | -25.983 | 11.089 | 1.00 | 54.87 |
| ATOM | 2051 | N | PRO | B | 52 | 26.753 | -23.929 | 10.936 | 1.00 | 54.53 |
| ATOM | 2052 | CD | PRO | B | 52 | 25.664 | -22.938 | 10.860 | 1.00 | 53.44 |
| ATOM | 2053 | CA | PRO | B | 52 | 28.063 | -23.277 | 10.894 | 1.00 | 54.36 |
| ATOM | 2054 | CB | PRO | B | 52 | 27.712 | -21.829 | 10.571 | 1.00 | 53.89 |
| ATOM | 2055 | CG | PRO | B | 52 | 26.378 | -21.661 | 11.220 | 1.00 | 54.44 |
| ATOM | 2056 | C | PRO | B | 52 | 28.775 | -23.437 | 12.239 | 1.00 | 54.60 |
| ATOM | 2057 | O | PRO | B | 52 | 28.141 | -23.406 | 13.299 | 1.00 | 55.52 |
| ATOM | 2058 | N | LYS | B | 53 | 30.090 | -23.620 | 12.187 | 1.00 | 54.23 |
| ATOM | 2059 | CA | LYS | B | 53 | 30.903 | -23.809 | 13.388 | 1.00 | 53.80 |
| ATOM | 2060 | CB | LYS | B | 53 | 32.390 | -23.814 | 13.000 | 1.00 | 54.36 |
| ATOM | 2061 | CG | LYS | B | 53 | 33.342 | -24.303 | 14.078 | 1.00 | 53.83 |
| ATOM | 2062 | CD | LYS | B | 53 | 34.766 | -24.366 | 13.539 | 1.00 | 54.25 |
| ATOM | 2063 | CE | LYS | B | 53 | 35.738 | -24.902 | 14.578 | 1.00 | 53.91 |
| ATOM | 2064 | NZ | LYS | B | 53 | 37.147 | -24.873 | 14.091 | 1.00 | 54.71 |
| ATOM | 2065 | C | LYS | B | 53 | 30.638 | -22.736 | 14.444 | 1.00 | 52.35 |
| ATOM | 2066 | O | LYS | B | 53 | 30.642 | -21.544 | 14.148 | 1.00 | 53.09 |
| ATOM | 2067 | N | GLY | B | 54 | 30.398 | -23.164 | 15.676 | 1.00 | 50.78 |
| ATOM | 2068 | CA | GLY | B | 54 | 30.151 | -22.212 | 16.740 | 1.00 | 49.28 |
| ATOM | 2069 | C | GLY | B | 54 | 28.688 | -22.095 | 17.103 | 1.00 | 48.04 |
| ATOM | 2070 | O | GLY | B | 54 | 28.344 | -21.821 | 18.253 | 1.00 | 48.25 |
| ATOM | 2071 | N | LEU | B | 55 | 27.823 | -22.307 | 16.118 | 1.00 | 46.91 |
| ATOM | 2072 | CA | LEU | B | 55 | 26.383 | -22.222 | 16.330 | 1.00 | 44.56 |
| ATOM | 2073 | CB | LEU | B | 55 | 25.716 | -21.555 | 15.118 | 1.00 | 43.43 |
| ATOM | 2074 | CG | LEU | B | 55 | 26.153 | -20.124 | 14.773 | 1.00 | 43.19 |
| ATOM | 2075 | CD1 | LEU | B | 55 | 25.466 | -19.665 | 13.496 | 1.00 | 40.81 |
| ATOM | 2076 | CD2 | LEU | B | 55 | 25.810 | -19.187 | 15.921 | 1.00 | 42.09 |
| ATOM | 2077 | C | LEU | B | 55 | 25.765 | -23.601 | 16.574 | 1.00 | 43.07 |
| ATOM | 2078 | O | LEU | B | 55 | 26.246 | -24.611 | 16.058 | 1.00 | 42.93 |
| ATOM | 2079 | N | PHE | B | 56 | 24.694 | -23.627 | 17.363 | 1.00 | 41.65 |
| ATOM | 2080 | CA | PHE | B | 56 | 23.990 | -24.864 | 17.691 | 1.00 | 40.11 |

Figure 3 (suite 28)

| | | | | | | | | | | |
|---|---|---|---|---|---|---|---|---|---|---|
| ATOM | 2081 | CB | PHE | B | 56 | 23.200 | -24.667 | 18.980 | 1.00 | 39.87 |
| ATOM | 2082 | CG | PHE | B | 56 | 22.556 | -25.919 | 19.503 | 1.00 | 39.45 |
| ATOM | 2083 | CD1 | PHE | B | 56 | 23.334 | -26.975 | 19.975 | 1.00 | 39.46 |
| ATOM | 2084 | CD2 | PHE | B | 56 | 21.168 | -26.021 | 19.578 | 1.00 | 40.18 |
| ATOM | 2085 | CE1 | PHE | B | 56 | 22.743 | -28.107 | 20.518 | 1.00 | 38.48 |
| ATOM | 2086 | CE2 | PHE | B | 56 | 20.562 | -27.154 | 20.120 | 1.00 | 41.14 |
| ATOM | 2087 | CZ | PHE | B | 56 | 21.353 | -28.200 | 20.592 | 1.00 | 40.75 |
| ATOM | 2088 | C | PHE | B | 56 | 23.030 | -25.275 | 16.575 | 1.00 | 39.99 |
| ATOM | 2089 | O | PHE | B | 56 | 21.998 | -24.635 | 16.374 | 1.00 | 37.97 |
| ATOM | 2090 | N | GLY | B | 57 | 23.366 | -26.346 | 15.862 | 1.00 | 38.48 |
| ATOM | 2091 | CA | GLY | B | 57 | 22.509 | -26.812 | 14.784 | 1.00 | 39.37 |
| ATOM | 2092 | C | GLY | B | 57 | 21.565 | -27.961 | 15.128 | 1.00 | 40.02 |
| ATOM | 2093 | O | GLY | B | 57 | 21.645 | -28.553 | 16.201 | 1.00 | 39.95 |
| ATOM | 2094 | N | VAL | B | 58 | 20.652 | -28.252 | 14.204 | 1.00 | 40.15 |
| ATOM | 2095 | CA | VAL | B | 58 | 19.673 | -29.331 | 14.332 | 1.00 | 40.36 |
| ATOM | 2096 | CB | VAL | B | 58 | 18.478 | -28.949 | 15.253 | 1.00 | 39.47 |
| ATOM | 2097 | CG1 | VAL | B | 58 | 18.952 | -28.679 | 16.657 | 1.00 | 39.25 |
| ATOM | 2098 | CG2 | VAL | B | 58 | 17.759 | -27.736 | 14.705 | 1.00 | 39.99 |
| ATOM | 2099 | C | VAL | B | 58 | 19.127 | -29.603 | 12.931 | 1.00 | 41.09 |
| ATOM | 2100 | O | VAL | B | 58 | 18.839 | -28.667 | 12.185 | 1.00 | 42.22 |
| ATOM | 2101 | N | GLU | B | 59 | 19.000 | -30.873 | 12.560 | 1.00 | 40.68 |
| ATOM | 2102 | CA | GLU | B | 59 | 18.475 | -31.207 | 11.240 | 1.00 | 38.75 |
| ATOM | 2103 | CB | GLU | B | 59 | 18.876 | -32.627 | 10.845 | 1.00 | 39.43 |
| ATOM | 2104 | CG | GLU | B | 59 | 20.331 | -32.959 | 11.143 | 0.00 | 39.37 |
| ATOM | 2105 | CD | GLU | B | 59 | 20.692 | -34.390 | 10.799 | 0.00 | 39.50 |
| ATOM | 2106 | OE1 | GLU | B | 59 | 20.064 | -35.313 | 11.357 | 0.00 | 39.51 |
| ATOM | 2107 | OE2 | GLU | B | 59 | 21.605 | -34.592 | 9.972 | 0.00 | 39.51 |
| ATOM | 2108 | C | GLU | B | 59 | 16.954 | -31.092 | 11.295 | 1.00 | 38.17 |
| ATOM | 2109 | O | GLU | B | 59 | 16.306 | -31.605 | 12.210 | 1.00 | 35.97 |
| ATOM | 2110 | N | VAL | B | 60 | 16.382 | -30.384 | 10.334 | 1.00 | 37.50 |
| ATOM | 2111 | CA | VAL | B | 60 | 14.938 | -30.227 | 10.306 | 1.00 | 38.32 |
| ATOM | 2112 | CB | VAL | B | 60 | 14.447 | -29.043 | 11.165 | 1.00 | 39.41 |
| ATOM | 2113 | CG1 | VAL | B | 60 | 12.983 | -29.246 | 11.501 | 1.00 | 39.25 |
| ATOM | 2114 | CG2 | VAL | B | 60 | 15.283 | -28.888 | 12.424 | 1.00 | 40.23 |
| ATOM | 2115 | C | VAL | B | 60 | 14.486 | -29.940 | 8.899 | 1.00 | 37.09 |
| ATOM | 2116 | O | VAL | B | 60 | 15.157 | -29.225 | 8.159 | 1.00 | 36.84 |
| ATOM | 2117 | N | ASP | B | 61 | 13.351 | -30.514 | 8.529 | 1.00 | 37.31 |
| ATOM | 2118 | CA | ASP | B | 61 | 12.776 | -30.275 | 7.217 | 1.00 | 36.05 |
| ATOM | 2119 | CB | ASP | B | 61 | 12.579 | -31.587 | 6.464 | 1.00 | 37.92 |
| ATOM | 2120 | CG | ASP | B | 61 | 12.058 | -31.373 | 5.058 | 1.00 | 39.99 |
| ATOM | 2121 | OD1 | ASP | B | 61 | 12.074 | -32.328 | 4.255 | 1.00 | 42.49 |
| ATOM | 2122 | OD2 | ASP | B | 61 | 11.622 | -30.244 | 4.754 | 1.00 | 42.95 |
| ATOM | 2123 | C | ASP | B | 61 | 11.441 | -29.604 | 7.521 | 1.00 | 35.04 |
| ATOM | 2124 | O | ASP | B | 61 | 10.560 | -30.209 | 8.146 | 1.00 | 35.28 |
| ATOM | 2125 | N | VAL | B | 62 | 11.306 | -28.345 | 7.110 | 1.00 | 33.84 |
| ATOM | 2126 | CA | VAL | B | 62 | 10.088 | -27.581 | 7.378 | 1.00 | 32.00 |
| ATOM | 2127 | CB | VAL | B | 62 | 10.185 | -26.138 | 6.813 | 1.00 | 30.81 |
| ATOM | 2128 | CG1 | VAL | B | 62 | 11.180 | -25.338 | 7.619 | 1.00 | 31.87 |
| ATOM | 2129 | CG2 | VAL | B | 62 | 10.607 | -26.170 | 5.356 | 1.00 | 30.88 |
| ATOM | 2130 | C | VAL | B | 62 | 8.805 | -28.229 | 6.870 | 1.00 | 30.74 |
| ATOM | 2131 | O | VAL | B | 62 | 7.717 | -27.901 | 7.348 | 1.00 | 31.75 |
| ATOM | 2132 | N | THR | B | 63 | 8.912 | -29.141 | 5.909 | 1.00 | 29.84 |
| ATOM | 2133 | CA | THR | B | 63 | 7.707 | -29.803 | 5.408 | 1.00 | 29.47 |
| ATOM | 2134 | CB | THR | B | 63 | 7.918 | -30.476 | 4.032 | 1.00 | 29.03 |
| ATOM | 2135 | OG1 | THR | B | 63 | 8.829 | -31.575 | 4.161 | 1.00 | 31.05 |
| ATOM | 2136 | CG2 | THR | B | 63 | 8.459 | -29.474 | 3.021 | 1.00 | 28.04 |
| ATOM | 2137 | C | THR | B | 63 | 7.232 | -30.873 | 6.380 | 1.00 | 28.56 |
| ATOM | 2138 | O | THR | B | 63 | 6.107 | -31.344 | 6.265 | 1.00 | 30.19 |
| ATOM | 2139 | N | ASP | B | 64 | 8.076 | -31.237 | 7.347 | 1.00 | 28.69 |
| ATOM | 2140 | CA | ASP | B | 64 | 7.745 | -32.272 | 8.335 | 1.00 | 27.45 |
| ATOM | 2141 | CB | ASP | B | 64 | 8.934 | -33.221 | 8.506 | 1.00 | 28.14 |
| ATOM | 2142 | CG | ASP | B | 64 | 8.630 | -34.413 | 9.420 | 1.00 | 25.75 |
| ATOM | 2143 | OD1 | ASP | B | 64 | 7.820 | -34.303 | 10.370 | 1.00 | 20.08 |
| ATOM | 2144 | OD2 | ASP | B | 64 | 9.246 | -35.474 | 9.195 | 1.00 | 27.49 |
| ATOM | 2145 | C | ASP | B | 64 | 7.380 | -31.714 | 9.699 | 1.00 | 28.63 |
| ATOM | 2146 | O | ASP | B | 64 | 8.253 | -31.300 | 10.451 | 1.00 | 27.05 |
| ATOM | 2147 | N | SER | B | 65 | 6.095 | -31.749 | 10.034 | 1.00 | 30.81 |
| ATOM | 2148 | CA | SER | B | 65 | 5.627 | -31.244 | 11.316 | 1.00 | 34.09 |
| ATOM | 2149 | CB | SER | B | 65 | 4.099 | -31.211 | 11.343 | 1.00 | 33.25 |
| ATOM | 2150 | OG | SER | B | 65 | 3.603 | -30.236 | 10.440 | 1.00 | 36.91 |
| ATOM | 2151 | C | SER | B | 65 | 6.137 | -32.029 | 12.520 | 1.00 | 36.19 |
| ATOM | 2152 | O | SER | B | 65 | 6.079 | -31.536 | 13.649 | 1.00 | 35.84 |
| ATOM | 2153 | N | ASP | B | 66 | 6.621 | -33.248 | 12.283 | 1.00 | 38.11 |

Figure 3 (suite 29)

| | | | | | | | | | | |
|---|---|---|---|---|---|---|---|---|---|---|
| ATOM | 2154 | CA | ASP | B | 66 | 7.153 | -34.093 | 13.359 | 1.00 | 38.29 |
| ATOM | 2155 | CB | ASP | B | 66 | 7.136 | -35.581 | 12.953 | 1.00 | 42.40 |
| ATOM | 2156 | CG | ASP | B | 66 | 5.720 | -36.148 | 12.799 | 1.00 | 47.02 |
| ATOM | 2157 | OD1 | ASP | B | 66 | 4.984 | -36.223 | 13.809 | 1.00 | 47.93 |
| ATOM | 2158 | OD2 | ASP | B | 66 | 5.350 | -36.525 | 11.661 | 1.00 | 50.42 |
| ATOM | 2159 | C | ASP | B | 66 | 8.590 | -33.675 | 13.663 | 1.00 | 36.02 |
| ATOM | 2160 | O | ASP | B | 66 | 8.966 | -33.503 | 14.820 | 1.00 | 34.94 |
| ATOM | 2161 | N | ALA | B | 67 | 9.389 | -33.528 | 12.610 | 1.00 | 35.81 |
| ATOM | 2162 | CA | ALA | B | 67 | 10.780 | -33.119 | 12.747 | 1.00 | 36.08 |
| ATOM | 2163 | CB | ALA | B | 67 | 11.469 | -33.137 | 11.389 | 1.00 | 33.55 |
| ATOM | 2164 | C | ALA | B | 67 | 10.824 | -31.712 | 13.348 | 1.00 | 38.08 |
| ATOM | 2165 | O | ALA | B | 67 | 11.670 | -31.408 | 14.201 | 1.00 | 40.35 |
| ATOM | 2166 | N | VAL | B | 68 | 9.900 | -30.863 | 12.907 | 1.00 | 37.10 |
| ATOM | 2167 | CA | VAL | B | 68 | 9.811 | -29.498 | 13.401 | 1.00 | 37.10 |
| ATOM | 2168 | CB | VAL | B | 68 | 8.707 | -28.703 | 12.656 | 1.00 | 34.52 |
| ATOM | 2169 | CG1 | VAL | B | 68 | 8.428 | -27.389 | 13.364 | 1.00 | 31.27 |
| ATOM | 2170 | CG2 | VAL | B | 68 | 9.136 | -28.453 | 11.227 | 1.00 | 32.87 |
| ATOM | 2171 | C | VAL | B | 68 | 9.510 | -29.486 | 14.888 | 1.00 | 37.88 |
| ATOM | 2172 | O | VAL | B | 68 | 10.067 | -28.681 | 15.629 | 1.00 | 39.05 |
| ATOM | 2173 | N | ASP | B | 69 | 8.630 | -30.377 | 15.326 | 1.00 | 39.53 |
| ATOM | 2174 | CA | ASP | B | 69 | 8.269 | -30.438 | 16.738 | 1.00 | 40.97 |
| ATOM | 2175 | CB | ASP | B | 69 | 7.040 | -31.324 | 16.942 | 1.00 | 41.89 |
| ATOM | 2176 | CG | ASP | B | 69 | 6.639 | -31.433 | 18.406 | 1.00 | 44.55 |
| ATOM | 2177 | OD1 | ASP | B | 69 | 7.415 | -32.004 | 19.196 | 1.00 | 45.61 |
| ATOM | 2178 | OD2 | ASP | B | 69 | 5.551 | -30.941 | 18.776 | 1.00 | 46.50 |
| ATOM | 2179 | C | ASP | B | 69 | 9.398 | -30.945 | 17.631 | 1.00 | 41.35 |
| ATOM | 2180 | O | ASP | B | 69 | 9.496 | -30.542 | 18.794 | 1.00 | 41.81 |
| ATOM | 2181 | N | ARG | B | 70 | 10.246 | -31.828 | 17.106 | 1.00 | 40.72 |
| ATOM | 2182 | CA | ARG | B | 70 | 11.343 | -32.351 | 17.911 | 1.00 | 40.79 |
| ATOM | 2183 | CB | ARG | B | 70 | 11.690 | -33.792 | 17.508 | 1.00 | 42.32 |
| ATOM | 2184 | CG | ARG | B | 70 | 12.198 | -33.980 | 16.087 | 1.00 | 46.38 |
| ATOM | 2185 | CD | ARG | B | 70 | 12.788 | -35.383 | 15.910 | 1.00 | 48.39 |
| ATOM | 2186 | NE | ARG | B | 70 | 13.467 | -35.531 | 14.625 | 1.00 | 50.58 |
| ATOM | 2187 | CZ | ARG | B | 70 | 12.866 | -35.902 | 13.499 | 1.00 | 50.62 |
| ATOM | 2188 | NH1 | ARG | B | 70 | 11.569 | -36.173 | 13.498 | 1.00 | 49.38 |
| ATOM | 2189 | NH2 | ARG | B | 70 | 13.560 | -35.985 | 12.369 | 1.00 | 52.00 |
| ATOM | 2190 | C | ARG | B | 70 | 12.577 | -31.458 | 17.821 | 1.00 | 39.76 |
| ATOM | 2191 | O | ARG | B | 70 | 13.467 | -31.524 | 18.669 | 1.00 | 40.46 |
| ATOM | 2192 | N | ALA | B | 71 | 12.630 | -30.620 | 16.794 | 1.00 | 37.22 |
| ATOM | 2193 | CA | ALA | B | 71 | 13.749 | -29.700 | 16.645 | 1.00 | 34.96 |
| ATOM | 2194 | CB | ALA | B | 71 | 13.744 | -29.078 | 15.265 | 1.00 | 33.97 |
| ATOM | 2195 | C | ALA | B | 71 | 13.588 | -28.620 | 17.705 | 1.00 | 34.23 |
| ATOM | 2196 | O | ALA | B | 71 | 14.567 | -28.159 | 18.297 | 1.00 | 33.88 |
| ATOM | 2197 | N | PHE | B | 72 | 12.347 | -28.223 | 17.958 | 1.00 | 32.07 |
| ATOM | 2198 | CA | PHE | B | 72 | 12.109 | -27.202 | 18.958 | 1.00 | 32.77 |
| ATOM | 2199 | CB | PHE | B | 72 | 10.647 | -26.740 | 18.933 | 1.00 | 31.04 |
| ATOM | 2200 | CG | PHE | B | 72 | 10.392 | -25.588 | 17.992 | 1.00 | 32.39 |
| ATOM | 2201 | CD1 | PHE | B | 72 | 10.572 | -25.738 | 16.616 | 1.00 | 30.97 |
| ATOM | 2202 | CD2 | PHE | B | 72 | 10.000 | -24.341 | 18.487 | 1.00 | 31.08 |
| ATOM | 2203 | CE1 | PHE | B | 72 | 10.367 | -24.671 | 15.747 | 1.00 | 30.38 |
| ATOM | 2204 | CE2 | PHE | B | 72 | 9.793 | -23.263 | 17.629 | 1.00 | 31.80 |
| ATOM | 2205 | CZ | PHE | B | 72 | 9.978 | -23.428 | 16.254 | 1.00 | 32.02 |
| ATOM | 2206 | C | PHE | B | 72 | 12.485 | -27.708 | 20.342 | 1.00 | 34.11 |
| ATOM | 2207 | O | PHE | B | 72 | 13.141 | -26.999 | 21.111 | 1.00 | 33.31 |
| ATOM | 2208 | N | THR | B | 73 | 12.079 | -28.937 | 20.658 | 1.00 | 34.11 |
| ATOM | 2209 | CA | THR | B | 73 | 12.385 | -29.522 | 21.959 | 1.00 | 33.15 |
| ATOM | 2210 | CB | THR | B | 73 | 11.741 | -30.921 | 22.115 | 1.00 | 34.16 |
| ATOM | 2211 | OG1 | THR | B | 73 | 10.334 | -30.838 | 21.848 | 1.00 | 33.91 |
| ATOM | 2212 | CG2 | THR | B | 73 | 11.933 | -31.438 | 23.537 | 1.00 | 34.89 |
| ATOM | 2213 | C | THR | B | 73 | 13.898 | -29.643 | 22.168 | 1.00 | 32.36 |
| ATOM | 2214 | O | THR | B | 73 | 14.384 | -29.503 | 23.282 | 1.00 | 32.94 |
| ATOM | 2215 | N | ALA | B | 74 | 14.639 | -29.897 | 21.098 | 1.00 | 30.84 |
| ATOM | 2216 | CA | ALA | B | 74 | 16.087 | -30.020 | 21.204 | 1.00 | 32.65 |
| ATOM | 2217 | CB | ALA | B | 74 | 16.673 | -30.469 | 19.879 | 1.00 | 32.14 |
| ATOM | 2218 | C | ALA | B | 74 | 16.685 | -28.677 | 21.605 | 1.00 | 35.41 |
| ATOM | 2219 | O | ALA | B | 74 | 17.458 | -28.583 | 22.566 | 1.00 | 37.63 |
| ATOM | 2220 | N | VAL | B | 75 | 16.327 | -27.645 | 20.845 | 1.00 | 34.66 |
| ATOM | 2221 | CA | VAL | B | 75 | 16.784 | -26.284 | 21.094 | 1.00 | 33.27 |
| ATOM | 2222 | CB | VAL | B | 75 | 16.193 | -25.304 | 20.039 | 1.00 | 31.92 |
| ATOM | 2223 | CG1 | VAL | B | 75 | 16.453 | -23.872 | 20.452 | 1.00 | 31.32 |
| ATOM | 2224 | CG2 | VAL | B | 75 | 16.788 | -25.583 | 18.661 | 1.00 | 27.26 |
| ATOM | 2225 | C | VAL | B | 75 | 16.291 | -25.874 | 22.477 | 1.00 | 34.64 |
| ATOM | 2226 | O | VAL | B | 75 | 17.007 | -25.228 | 23.245 | 1.00 | 34.97 |

Figure 3 (suite 30)

| | | | | | | | | | | |
|---|---|---|---|---|---|---|---|---|---|---|
| ATOM | 2227 | N | GLU | B | 76 | 15.053 | -26.257 | 22.772 | 1.00 | 35.42 |
| ATOM | 2228 | CA | GLU | B | 76 | 14.405 | -25.957 | 24.042 | 1.00 | 36.60 |
| ATOM | 2229 | CB | GLU | B | 76 | 12.994 | -26.562 | 24.024 | 1.00 | 36.97 |
| ATOM | 2230 | CG | GLU | B | 76 | 12.430 | -27.083 | 25.345 | 1.00 | 41.17 |
| ATOM | 2231 | CD | GLU | B | 76 | 12.190 | -25.999 | 26.366 | 1.00 | 41.24 |
| ATOM | 2232 | OE1 | GLU | B | 76 | 11.361 | -26.205 | 27.283 | 1.00 | 39.45 |
| ATOM | 2233 | OE2 | GLU | B | 76 | 12.845 | -24.944 | 26.261 | 1.00 | 43.43 |
| ATOM | 2234 | C | GLU | B | 76 | 15.226 | -26.476 | 25.223 | 1.00 | 38.43 |
| ATOM | 2235 | O | GLU | B | 76 | 15.238 | -25.874 | 26.303 | 1.00 | 38.79 |
| ATOM | 2236 | N | GLU | B | 77 | 15.927 | -27.585 | 25.013 | 1.00 | 38.15 |
| ATOM | 2237 | CA | GLU | B | 77 | 16.744 | -28.161 | 26.073 | 1.00 | 39.80 |
| ATOM | 2238 | CB | GLU | B | 77 | 16.881 | -29.678 | 25.887 | 1.00 | 40.46 |
| ATOM | 2239 | CG | GLU | B | 77 | 15.610 | -30.464 | 26.202 | 1.00 | 41.37 |
| ATOM | 2240 | CD | GLU | B | 77 | 15.779 | -31.957 | 25.998 | 0.00 | 41.21 |
| ATOM | 2241 | OE1 | GLU | B | 77 | 16.077 | -32.373 | 24.858 | 0.00 | 41.34 |
| ATOM | 2242 | OE2 | GLU | B | 77 | 15.614 | -32.714 | 26.978 | 0.00 | 41.34 |
| ATOM | 2243 | C | GLU | B | 77 | 18.126 | -27.520 | 26.095 | 1.00 | 39.58 |
| ATOM | 2244 | O | GLU | B | 77 | 18.771 | -27.442 | 27.135 | 1.00 | 40.34 |
| ATOM | 2245 | N | HIS | B | 78 | 18.565 | -27.050 | 24.938 | 1.00 | 39.00 |
| ATOM | 2246 | CA | HIS | B | 78 | 19.868 | -26.438 | 24.816 | 1.00 | 38.00 |
| ATOM | 2247 | CB | HIS | B | 78 | 20.262 | -26.378 | 23.344 | 1.00 | 38.52 |
| ATOM | 2248 | CG | HIS | B | 78 | 21.586 | -25.725 | 23.098 | 1.00 | 38.82 |
| ATOM | 2249 | CD2 | HIS | B | 78 | 21.908 | -24.571 | 22.465 | 1.00 | 38.83 |
| ATOM | 2250 | ND1 | HIS | B | 78 | 22.776 | -26.271 | 23.526 | 1.00 | 39.20 |
| ATOM | 2251 | CE1 | HIS | B | 78 | 23.774 | -25.484 | 23.167 | 1.00 | 38.53 |
| ATOM | 2252 | NE2 | HIS | B | 78 | 23.275 | -24.446 | 22.521 | 1.00 | 38.43 |
| ATOM | 2253 | C | HIS | B | 78 | 19.955 | -25.042 | 25.412 | 1.00 | 38.17 |
| ATOM | 2254 | O | HIS | B | 78 | 20.581 | -24.832 | 26.452 | 1.00 | 39.63 |
| ATOM | 2255 | N | GLN | B | 79 | 19.304 | -24.096 | 24.747 | 1.00 | 37.25 |
| ATOM | 2256 | CA | GLN | B | 79 | 19.333 | -22.693 | 25.132 | 1.00 | 33.50 |
| ATOM | 2257 | CB | GLN | B | 79 | 19.658 | -21.872 | 23.893 | 1.00 | 34.67 |
| ATOM | 2258 | CG | GLN | B | 79 | 18.762 | -22.256 | 22.723 | 1.00 | 34.80 |
| ATOM | 2259 | CD | GLN | B | 79 | 19.133 | -21.581 | 21.436 | 1.00 | 35.28 |
| ATOM | 2260 | OE1 | GLN | B | 79 | 20.245 | -21.749 | 20.921 | 1.00 | 34.89 |
| ATOM | 2261 | NE2 | GLN | B | 79 | 18.197 | -20.807 | 20.894 | 1.00 | 36.57 |
| ATOM | 2262 | C | GLN | B | 79 | 18.052 | -22.172 | 25.744 | 1.00 | 32.72 |
| ATOM | 2263 | O | GLN | B | 79 | 17.937 | -20.979 | 26.011 | 1.00 | 32.82 |
| ATOM | 2264 | N | GLY | B | 80 | 17.083 | -23.052 | 25.965 | 1.00 | 32.57 |
| ATOM | 2265 | CA | GLY | B | 80 | 15.821 | -22.605 | 26.532 | 1.00 | 31.61 |
| ATOM | 2266 | C | GLY | B | 80 | 14.767 | -22.340 | 25.459 | 1.00 | 31.60 |
| ATOM | 2267 | O | GLY | B | 80 | 15.083 | -22.266 | 24.265 | 1.00 | 32.15 |
| ATOM | 2268 | N | PRO | B | 81 | 13.504 | -22.172 | 25.859 | 1.00 | 28.16 |
| ATOM | 2269 | CD | PRO | B | 81 | 13.101 | -22.057 | 27.269 | 1.00 | 29.00 |
| ATOM | 2270 | CA | PRO | B | 81 | 12.369 | -21.917 | 24.968 | 1.00 | 29.07 |
| ATOM | 2271 | CB | PRO | B | 81 | 11.265 | -21.493 | 25.934 | 1.00 | 30.07 |
| ATOM | 2272 | CG | PRO | B | 81 | 11.616 | -22.230 | 27.187 | 1.00 | 29.41 |
| ATOM | 2273 | C | PRO | B | 81 | 12.619 | -20.851 | 23.900 | 1.00 | 29.26 |
| ATOM | 2274 | O | PRO | B | 81 | 12.941 | -19.710 | 24.226 | 1.00 | 27.31 |
| ATOM | 2275 | N | VAL | B | 82 | 12.458 | -21.229 | 22.632 | 1.00 | 29.35 |
| ATOM | 2276 | CA | VAL | B | 82 | 12.634 | -20.302 | 21.523 | 1.00 | 28.83 |
| ATOM | 2277 | CB | VAL | B | 82 | 12.194 | -20.938 | 20.192 | 1.00 | 30.61 |
| ATOM | 2278 | CG1 | VAL | B | 82 | 12.209 | -19.896 | 19.068 | 1.00 | 31.76 |
| ATOM | 2279 | CG2 | VAL | B | 82 | 13.118 | -22.072 | 19.845 | 1.00 | 30.36 |
| ATOM | 2280 | C | VAL | B | 82 | 11.781 | -19.057 | 21.781 | 1.00 | 29.43 |
| ATOM | 2281 | O | VAL | B | 82 | 10.558 | -19.143 | 21.964 | 1.00 | 29.57 |
| ATOM | 2282 | N | GLU | B | 83 | 12.432 | -17.900 | 21.813 | 1.00 | 28.56 |
| ATOM | 2283 | CA | GLU | B | 83 | 11.733 | -16.650 | 22.058 | 1.00 | 26.71 |
| ATOM | 2284 | CB | GLU | B | 83 | 12.491 | -15.820 | 23.087 | 1.00 | 27.62 |
| ATOM | 2285 | CG | GLU | B | 83 | 12.550 | -16.413 | 24.482 | 1.00 | 25.93 |
| ATOM | 2286 | CD | GLU | B | 83 | 13.565 | -15.679 | 25.344 | 1.00 | 27.69 |
| ATOM | 2287 | OE1 | GLU | B | 83 | 14.787 | -15.802 | 25.054 | 1.00 | 25.03 |
| ATOM | 2288 | OE2 | GLU | B | 83 | 13.139 | -14.971 | 26.288 | 1.00 | 24.70 |
| ATOM | 2289 | C | GLU | B | 83 | 11.615 | -15.851 | 20.778 | 1.00 | 24.76 |
| ATOM | 2290 | O | GLU | B | 83 | 10.664 | -15.101 | 20.587 | 1.00 | 26.60 |
| ATOM | 2291 | N | VAL | B | 84 | 12.589 | -16.018 | 19.900 | 1.00 | 24.49 |
| ATOM | 2292 | CA | VAL | B | 84 | 12.612 | -15.284 | 18.648 | 1.00 | 25.08 |
| ATOM | 2293 | CB | VAL | B | 84 | 13.775 | -14.275 | 18.624 | 1.00 | 24.67 |
| ATOM | 2294 | CG1 | VAL | B | 84 | 13.844 | -13.579 | 17.274 | 1.00 | 25.81 |
| ATOM | 2295 | CG2 | VAL | B | 84 | 13.597 | -13.280 | 19.758 | 1.00 | 24.72 |
| ATOM | 2296 | C | VAL | B | 84 | 12.790 | -16.243 | 17.495 | 1.00 | 24.89 |
| ATOM | 2297 | O | VAL | B | 84 | 13.837 | -16.867 | 17.353 | 1.00 | 26.64 |
| ATOM | 2298 | N | LEU | B | 85 | 11.762 | -16.353 | 16.666 | 1.00 | 24.08 |
| ATOM | 2299 | CA | LEU | B | 85 | 11.810 | -17.248 | 15.523 | 1.00 | 23.37 |

Figure 3 (suite 31)

| | | | | | | | | | | |
|---|---|---|---|---|---|---|---|---|---|---|
| ATOM | 2300 | CB | LEU | B | 85 | 10.523 | -18.075 | 15.452 | 1.00 | 22.55 |
| ATOM | 2301 | CG | LEU | B | 85 | 10.359 | -18.944 | 14.203 | 1.00 | 23.77 |
| ATOM | 2302 | CD1 | LEU | B | 85 | 11.572 | -19.839 | 14.016 | 1.00 | 23.48 |
| ATOM | 2303 | CD2 | LEU | B | 85 | 9.094 | -19.775 | 14.331 | 1.00 | 24.51 |
| ATOM | 2304 | C | LEU | B | 85 | 12.004 | -16.515 | 14.213 | 1.00 | 22.22 |
| ATOM | 2305 | O | LEU | B | 85 | 11.242 | -15.610 | 13.881 | 1.00 | 21.53 |
| ATOM | 2306 | N | VAL | B | 86 | 13.038 | -16.902 | 13.481 | 1.00 | 21.03 |
| ATOM | 2307 | CA | VAL | B | 86 | 13.314 | -16.320 | 12.183 | 1.00 | 20.08 |
| ATOM | 2308 | CB | VAL | B | 86 | 14.787 | -15.847 | 12.043 | 1.00 | 20.32 |
| ATOM | 2309 | CG1 | VAL | B | 86 | 15.030 | -15.385 | 10.642 | 1.00 | 15.90 |
| ATOM | 2310 | CG2 | VAL | B | 86 | 15.099 | -14.719 | 13.048 | 1.00 | 16.67 |
| ATOM | 2311 | C | VAL | B | 86 | 13.085 | -17.426 | 11.160 | 1.00 | 21.54 |
| ATOM | 2312 | O | VAL | B | 86 | 13.918 | -18.321 | 11.009 | 1.00 | 21.49 |
| ATOM | 2313 | N | SER | B | 87 | 11.955 | -17.379 | 10.463 | 1.00 | 22.84 |
| ATOM | 2314 | CA | SER | B | 87 | 11.679 | -18.387 | 9.459 | 1.00 | 23.28 |
| ATOM | 2315 | CB | SER | B | 87 | 10.175 | -18.633 | 9.357 | 1.00 | 21.23 |
| ATOM | 2316 | OG | SER | B | 87 | 9.868 | -19.392 | 8.197 | 1.00 | 26.00 |
| ATOM | 2317 | C | SER | B | 87 | 12.260 | -17.935 | 8.118 | 1.00 | 24.78 |
| ATOM | 2318 | O | SER | B | 87 | 11.687 | -17.104 | 7.411 | 1.00 | 25.03 |
| ATOM | 2319 | N | ASN | B | 88 | 13.415 | -18.494 | 7.785 | 1.00 | 25.31 |
| ATOM | 2320 | CA | ASN | B | 88 | 14.111 | -18.167 | 6.554 | 1.00 | 28.19 |
| ATOM | 2321 | CB | ASN | B | 88 | 15.535 | -17.682 | 6.895 | 1.00 | 28.64 |
| ATOM | 2322 | CG | ASN | B | 88 | 16.386 | -17.440 | 5.665 | 1.00 | 29.48 |
| ATOM | 2323 | OD1 | ASN | B | 88 | 17.191 | -18.279 | 5.279 | 1.00 | 31.19 |
| ATOM | 2324 | ND2 | ASN | B | 88 | 16.202 | -16.295 | 5.035 | 1.00 | 32.27 |
| ATOM | 2325 | C | ASN | B | 88 | 14.153 | -19.374 | 5.620 | 1.00 | 29.69 |
| ATOM | 2326 | O | ASN | B | 88 | 14.351 | -19.234 | 4.418 | 1.00 | 31.08 |
| ATOM | 2327 | N | ALA | B | 89 | 13.957 | -20.563 | 6.179 | 1.00 | 31.81 |
| ATOM | 2328 | CA | ALA | B | 89 | 13.978 | -21.795 | 5.390 | 1.00 | 35.27 |
| ATOM | 2329 | CB | ALA | B | 89 | 13.478 | -22.965 | 6.245 | 1.00 | 35.33 |
| ATOM | 2330 | C | ALA | B | 89 | 13.130 | -21.668 | 4.120 | 1.00 | 37.47 |
| ATOM | 2331 | O | ALA | B | 89 | 11.977 | -21.250 | 4.176 | 1.00 | 37.34 |
| ATOM | 2332 | N | GLY | B | 90 | 13.701 | -22.026 | 2.973 | 1.00 | 39.41 |
| ATOM | 2333 | CA | GLY | B | 90 | 12.948 | -21.922 | 1.740 | 1.00 | 41.67 |
| ATOM | 2334 | C | GLY | B | 90 | 13.608 | -22.491 | 0.498 | 1.00 | 42.85 |
| ATOM | 2335 | O | GLY | B | 90 | 14.677 | -23.100 | 0.571 | 1.00 | 42.17 |
| ATOM | 2336 | N | LEU | B | 91 | 12.956 | -22.280 | -0.645 | 1.00 | 44.04 |
| ATOM | 2337 | CA | LEU | B | 91 | 13.439 | -22.758 | -1.939 | 1.00 | 45.37 |
| ATOM | 2338 | CB | LEU | B | 91 | 13.414 | -24.292 | -1.974 | 1.00 | 44.81 |
| ATOM | 2339 | CG | LEU | B | 91 | 12.063 | -25.002 | -1.834 | 1.00 | 45.93 |
| ATOM | 2340 | CD1 | LEU | B | 91 | 11.376 | -25.108 | -3.194 | 1.00 | 46.59 |
| ATOM | 2341 | CD2 | LEU | B | 91 | 12.292 | -26.390 | -1.265 | 1.00 | 45.80 |
| ATOM | 2342 | C | LEU | B | 91 | 12.581 | -22.193 | -3.076 | 1.00 | 45.54 |
| ATOM | 2343 | O | LEU | B | 91 | 11.592 | -21.501 | -2.836 | 1.00 | 46.40 |
| ATOM | 2344 | N | SER | B | 92 | 12.949 | -22.503 | -4.313 | 1.00 | 46.32 |
| ATOM | 2345 | CA | SER | B | 92 | 12.209 | -21.996 | -5.456 | 1.00 | 46.97 |
| ATOM | 2346 | CB | SER | B | 92 | 12.692 | -20.579 | -5.778 | 1.00 | 47.71 |
| ATOM | 2347 | OG | SER | B | 92 | 14.096 | -20.551 | -5.991 | 1.00 | 47.82 |
| ATOM | 2348 | C | SER | B | 92 | 12.311 | -22.862 | -6.712 | 1.00 | 46.96 |
| ATOM | 2349 | O | SER | B | 92 | 13.375 | -23.383 | -7.039 | 1.00 | 47.96 |
| ATOM | 2350 | N | ALA | B | 93 | 11.189 | -23.001 | -7.410 | 1.00 | 46.34 |
| ATOM | 2351 | CA | ALA | B | 93 | 11.119 | -23.765 | -8.650 | 1.00 | 46.18 |
| ATOM | 2352 | CB | ALA | B | 93 | 10.360 | -25.074 | -8.433 | 1.00 | 46.17 |
| ATOM | 2353 | C | ALA | B | 93 | 10.374 | -22.884 | -9.638 | 1.00 | 46.81 |
| ATOM | 2354 | O | ALA | B | 93 | 9.160 | -23.016 | -9.808 | 1.00 | 45.71 |
| ATOM | 2355 | N | ASP | B | 94 | 11.113 | -21.979 | -10.276 | 1.00 | 48.00 |
| ATOM | 2356 | CA | ASP | B | 94 | 10.553 | -21.036 | -11.241 | 1.00 | 48.10 |
| ATOM | 2357 | CB | ASP | B | 94 | 11.537 | -19.890 | -11.494 | 1.00 | 49.58 |
| ATOM | 2358 | CG | ASP | B | 94 | 12.130 | -19.333 | -10.217 | 1.00 | 51.20 |
| ATOM | 2359 | OD1 | ASP | B | 94 | 11.364 | -19.089 | -9.258 | 1.00 | 52.44 |
| ATOM | 2360 | OD2 | ASP | B | 94 | 13.364 | -19.133 | -10.181 | 1.00 | 50.84 |
| ATOM | 2361 | C | ASP | B | 94 | 10.212 | -21.673 | -12.580 | 1.00 | 48.72 |
| ATOM | 2362 | O | ASP | B | 94 | 10.702 | -22.752 | -12.912 | 1.00 | 48.60 |
| ATOM | 2363 | N | ALA | B | 95 | 9.380 | -20.971 | -13.348 | 1.00 | 48.98 |
| ATOM | 2364 | CA | ALA | B | 95 | 8.941 | -21.399 | -14.677 | 1.00 | 48.27 |
| ATOM | 2365 | CB | ALA | B | 95 | 8.566 | -22.871 | -14.670 | 1.00 | 48.35 |
| ATOM | 2366 | C | ALA | B | 95 | 7.731 | -20.572 | -15.082 | 1.00 | 48.30 |
| ATOM | 2367 | O | ALA | B | 95 | 7.079 | -19.963 | -14.233 | 1.00 | 47.66 |
| ATOM | 2368 | N | PHE | B | 96 | 7.430 | -20.546 | -16.377 | 1.00 | 47.96 |
| ATOM | 2369 | CA | PHE | B | 96 | 6.265 | -19.812 | -16.858 | 1.00 | 47.96 |
| ATOM | 2370 | CB | PHE | B | 96 | 6.212 | -19.818 | -18.388 | 1.00 | 50.08 |
| ATOM | 2371 | CG | PHE | B | 96 | 7.365 | -19.105 | -19.044 | 1.00 | 51.96 |
| ATOM | 2372 | CD1 | PHE | B | 96 | 8.540 | -19.785 | -19.353 | 1.00 | 53.04 |

Figure 3 (suite 32)

| | | | | | | | | | | |
|---|---|---|---|---|---|---|---|---|---|---|
| ATOM | 2373 | CD2 | PHE | B | 96 | 7.272 | -17.751 | -19.360 | 1.00 | 52.71 |
| ATOM | 2374 | CE1 | PHE | B | 96 | 9.607 | -19.126 | -19.971 | 1.00 | 52.76 |
| ATOM | 2375 | CE2 | PHE | B | 96 | 8.332 | -17.085 | -19.977 | 1.00 | 52.54 |
| ATOM | 2376 | CZ | PHE | B | 96 | 9.501 | -17.775 | -20.283 | 1.00 | 52.17 |
| ATOM | 2377 | C | PHE | B | 96 | 5.016 | -20.499 | -16.304 | 1.00 | 46.78 |
| ATOM | 2378 | O | PHE | B | 96 | 5.063 | -21.674 | -15.939 | 1.00 | 45.47 |
| ATOM | 2379 | N | LEU | B | 97 | 3.903 | -19.772 | -16.242 | 1.00 | 46.20 |
| ATOM | 2380 | CA | LEU | B | 97 | 2.653 | -20.331 | -15.721 | 1.00 | 46.42 |
| ATOM | 2381 | CB | LEU | B | 97 | 1.550 | -19.263 | -15.714 | 1.00 | 45.38 |
| ATOM | 2382 | CG | LEU | B | 97 | 0.130 | -19.676 | -15.303 | 1.00 | 45.55 |
| ATOM | 2383 | CD1 | LEU | B | 97 | 0.105 | -20.191 | -13.870 | 1.00 | 45.38 |
| ATOM | 2384 | CD2 | LEU | B | 97 | -0.792 | -18.475 | -15.447 | 1.00 | 45.84 |
| ATOM | 2385 | C | LEU | B | 97 | 2.187 | -21.542 | -16.528 | 1.00 | 46.18 |
| ATOM | 2386 | O | LEU | B | 97 | 1.804 | -22.565 | -15.956 | 1.00 | 46.68 |
| ATOM | 2387 | N | MET | B | 98 | 2.223 | -21.428 | -17.853 | 1.00 | 45.79 |
| ATOM | 2388 | CA | MET | B | 98 | 1.793 | -22.524 | -18.716 | 1.00 | 46.27 |
| ATOM | 2389 | CB | MET | B | 98 | 1.719 | -22.045 | -20.174 | 1.00 | 45.50 |
| ATOM | 2390 | CG | MET | B | 98 | 1.143 | -23.062 | -21.147 | 0.00 | 45.82 |
| ATOM | 2391 | SD | MET | B | 98 | 2.348 | -24.277 | -21.709 | 0.00 | 45.76 |
| ATOM | 2392 | CE | MET | B | 98 | 2.938 | -23.479 | -23.202 | 0.00 | 45.81 |
| ATOM | 2393 | C | MET | B | 98 | 2.709 | -23.753 | -18.600 | 1.00 | 46.07 |
| ATOM | 2394 | O | MET | B | 98 | 2.259 | -24.887 | -18.795 | 1.00 | 45.06 |
| ATOM | 2395 | N | ARG | B | 99 | 3.982 | -23.523 | -18.269 | 1.00 | 44.99 |
| ATOM | 2396 | CA | ARG | B | 99 | 4.960 | -24.606 | -18.132 | 1.00 | 43.96 |
| ATOM | 2397 | CB | ARG | B | 99 | 6.363 | -24.115 | -18.521 | 1.00 | 43.58 |
| ATOM | 2398 | CG | ARG | B | 99 | 6.483 | -23.612 | -19.951 | 0.00 | 43.84 |
| ATOM | 2399 | CD | ARG | B | 99 | 7.903 | -23.161 | -20.251 | 0.00 | 43.89 |
| ATOM | 2400 | NE | ARG | B | 99 | 8.049 | -22.691 | -21.624 | 0.00 | 44.01 |
| ATOM | 2401 | CZ | ARG | B | 99 | 9.190 | -22.255 | -22.148 | 0.00 | 44.05 |
| ATOM | 2402 | NH1 | ARG | B | 99 | 10.293 | -22.227 | -21.412 | 0.00 | 44.09 |
| ATOM | 2403 | NH2 | ARG | B | 99 | 9.229 | -21.847 | -23.408 | 0.00 | 44.09 |
| ATOM | 2404 | C | ARG | B | 99 | 5.012 | -25.201 | -16.725 | 1.00 | 41.86 |
| ATOM | 2405 | O | ARG | B | 99 | 5.213 | -26.405 | -16.568 | 1.00 | 42.64 |
| ATOM | 2406 | N | MET | B | 100 | 4.829 | -24.362 | -15.711 | 1.00 | 38.84 |
| ATOM | 2407 | CA | MET | B | 100 | 4.870 | -24.807 | -14.323 | 1.00 | 35.50 |
| ATOM | 2408 | CB | MET | B | 100 | 4.556 | -23.638 | -13.391 | 1.00 | 34.98 |
| ATOM | 2409 | CG | MET | B | 100 | 4.675 | -23.986 | -11.927 | 1.00 | 32.85 |
| ATOM | 2410 | SD | MET | B | 100 | 4.538 | -22.547 | -10.873 | 1.00 | 34.31 |
| ATOM | 2411 | CE | MET | B | 100 | 5.943 | -21.572 | -11.451 | 1.00 | 29.20 |
| ATOM | 2412 | C | MET | B | 100 | 3.901 | -25.956 | -14.051 | 1.00 | 34.33 |
| ATOM | 2413 | O | MET | B | 100 | 2.794 | -25.989 | -14.586 | 1.00 | 33.61 |
| ATOM | 2414 | N | THR | B | 101 | 4.322 | -26.881 | -13.191 | 1.00 | 33.73 |
| ATOM | 2415 | CA | THR | B | 101 | 3.523 | -28.054 | -12.853 | 1.00 | 32.17 |
| ATOM | 2416 | CB | THR | B | 101 | 4.387 | -29.331 | -12.893 | 1.00 | 33.77 |
| ATOM | 2417 | OG1 | THR | B | 101 | 5.313 | -29.321 | -11.793 | 1.00 | 34.85 |
| ATOM | 2418 | CG2 | THR | B | 101 | 5.172 | -29.393 | -14.193 | 1.00 | 32.68 |
| ATOM | 2419 | C | THR | B | 101 | 2.905 | -27.962 | -11.471 | 1.00 | 31.61 |
| ATOM | 2420 | O | THR | B | 101 | 3.288 | -27.112 | -10.666 | 1.00 | 31.29 |
| ATOM | 2421 | N | GLU | B | 102 | 1.954 | -28.851 | -11.198 | 1.00 | 29.70 |
| ATOM | 2422 | CA | GLU | B | 102 | 1.293 | -28.894 | -9.901 | 1.00 | 32.21 |
| ATOM | 2423 | CB | GLU | B | 102 | 0.164 | -29.922 | -9.903 | 1.00 | 31.57 |
| ATOM | 2424 | CG | GLU | B | 102 | -0.494 | -30.060 | -8.539 | 1.00 | 33.13 |
| ATOM | 2425 | CD | GLU | B | 102 | -1.323 | -31.320 | -8.390 | 1.00 | 33.82 |
| ATOM | 2426 | OE1 | GLU | B | 102 | -2.045 | -31.431 | -7.377 | 1.00 | 34.58 |
| ATOM | 2427 | OE2 | GLU | B | 102 | -1.246 | -32.201 | -9.274 | 1.00 | 37.41 |
| ATOM | 2428 | C | GLU | B | 102 | 2.284 | -29.272 | -8.799 | 1.00 | 33.74 |
| ATOM | 2429 | O | GLU | B | 102 | 2.196 | -28.789 | -7.663 | 1.00 | 32.86 |
| ATOM | 2430 | N | GLU | B | 103 | 3.217 | -30.154 | -9.144 | 1.00 | 34.85 |
| ATOM | 2431 | CA | GLU | B | 103 | 4.220 | -30.623 | -8.204 | 1.00 | 36.71 |
| ATOM | 2432 | CB | GLU | B | 103 | 5.016 | -31.770 | -8.840 | 1.00 | 38.74 |
| ATOM | 2433 | CG | GLU | B | 103 | 5.938 | -32.522 | -7.887 | 1.00 | 41.92 |
| ATOM | 2434 | CD | GLU | B | 103 | 6.685 | -33.663 | -8.573 | 1.00 | 43.15 |
| ATOM | 2435 | OE1 | GLU | B | 103 | 6.026 | -34.637 | -9.005 | 1.00 | 45.67 |
| ATOM | 2436 | OE2 | GLU | B | 103 | 7.929 | -33.583 | -8.688 | 1.00 | 42.57 |
| ATOM | 2437 | C | GLU | B | 103 | 5.153 | -29.481 | -7.781 | 1.00 | 37.05 |
| ATOM | 2438 | O | GLU | B | 103 | 5.351 | -29.242 | -6.586 | 1.00 | 36.94 |
| ATOM | 2439 | N | LYS | B | 104 | 5.714 | -28.770 | -8.758 | 1.00 | 36.23 |
| ATOM | 2440 | CA | LYS | B | 104 | 6.620 | -27.661 | -8.464 | 1.00 | 36.48 |
| ATOM | 2441 | CB | LYS | B | 104 | 7.204 | -27.098 | -9.758 | 1.00 | 36.98 |
| ATOM | 2442 | CG | LYS | B | 104 | 8.076 | -28.109 | -10.486 | 1.00 | 42.79 |
| ATOM | 2443 | CD | LYS | B | 104 | 8.678 | -27.564 | -11.773 | 1.00 | 44.40 |
| ATOM | 2444 | CE | LYS | B | 104 | 9.354 | -28.679 | -12.558 | 1.00 | 44.69 |
| ATOM | 2445 | NZ | LYS | B | 104 | 9.947 | -28.195 | -13.839 | 1.00 | 47.48 |

Figure 3 (suite 33)

| | | | | | | | | | | |
|---|---|---|---|---|---|---|---|---|---|---|
| ATOM | 2446 | C | LYS | B | 104 | 5.933 | -26.553 | -7.669 | 1.00 | 35.54 |
| ATOM | 2447 | O | LYS | B | 104 | 6.546 | -25.934 | -6.799 | 1.00 | 36.74 |
| ATOM | 2448 | N | PHE | B | 105 | 4.659 | -26.305 | -7.955 | 1.00 | 33.64 |
| ATOM | 2449 | CA | PHE | B | 105 | 3.933 | -25.273 | -7.233 | 1.00 | 31.30 |
| ATOM | 2450 | CB | PHE | B | 105 | 2.544 | -25.048 | -7.837 | 1.00 | 28.17 |
| ATOM | 2451 | CG | PHE | B | 105 | 1.786 | -23.910 | -7.205 | 1.00 | 27.79 |
| ATOM | 2452 | CD1 | PHE | B | 105 | 1.973 | -22.601 | -7.645 | 1.00 | 29.14 |
| ATOM | 2453 | CD2 | PHE | B | 105 | 0.918 | -24.138 | -6.150 | 1.00 | 26.74 |
| ATOM | 2454 | CE1 | PHE | B | 105 | 1.306 | -21.536 | -7.039 | 1.00 | 27.44 |
| ATOM | 2455 | CE2 | PHE | B | 105 | 0.246 | -23.081 | -5.535 | 1.00 | 27.47 |
| ATOM | 2456 | CZ | PHE | B | 105 | 0.443 | -21.775 | -5.982 | 1.00 | 25.56 |
| ATOM | 2457 | C | PHE | B | 105 | 3.779 | -25.690 | -5.777 | 1.00 | 30.67 |
| ATOM | 2458 | O | PHE | B | 105 | 4.125 | -24.936 | -4.865 | 1.00 | 31.44 |
| ATOM | 2459 | N | GLU | B | 106 | 3.262 | -26.893 | -5.560 | 1.00 | 28.45 |
| ATOM | 2460 | CA | GLU | B | 106 | 3.041 | -27.387 | -4.208 | 1.00 | 28.68 |
| ATOM | 2461 | CB | GLU | B | 106 | 2.264 | -28.711 | -4.247 | 1.00 | 28.98 |
| ATOM | 2462 | CG | GLU | B | 106 | 0.957 | -28.600 | -5.005 | 1.00 | 33.71 |
| ATOM | 2463 | CD | GLU | B | 106 | -0.063 | -29.636 | -4.586 | 1.00 | 34.95 |
| ATOM | 2464 | OE1 | GLU | B | 106 | -0.470 | -29.637 | -3.408 | 1.00 | 37.19 |
| ATOM | 2465 | OE2 | GLU | B | 106 | -0.463 | -30.449 | -5.437 | 1.00 | 38.33 |
| ATOM | 2466 | C | GLU | B | 106 | 4.323 | -27.554 | -3.400 | 1.00 | 27.10 |
| ATOM | 2467 | O | GLU | B | 106 | 4.306 | -27.417 | -2.180 | 1.00 | 25.93 |
| ATOM | 2468 | N | LYS | B | 107 | 5.428 | -27.840 | -4.080 | 1.00 | 26.11 |
| ATOM | 2469 | CA | LYS | B | 107 | 6.715 | -28.026 | -3.413 | 1.00 | 25.79 |
| ATOM | 2470 | CB | LYS | B | 107 | 7.777 | -28.501 | -4.417 | 1.00 | 24.81 |
| ATOM | 2471 | CG | LYS | B | 107 | 9.133 | -28.784 | -3.792 | 0.00 | 25.20 |
| ATOM | 2472 | CD | LYS | B | 107 | 10.131 | -29.263 | -4.829 | 0.00 | 25.10 |
| ATOM | 2473 | CE | LYS | B | 107 | 11.477 | -29.571 | -4.197 | 0.00 | 25.13 |
| ATOM | 2474 | NZ | LYS | B | 107 | 12.469 | -30.028 | -5.207 | 0.00 | 25.11 |
| ATOM | 2475 | C | LYS | B | 107 | 7.171 | -26.725 | -2.764 | 1.00 | 24.76 |
| ATOM | 2476 | O | LYS | B | 107 | 7.574 | -26.711 | -1.602 | 1.00 | 23.53 |
| ATOM | 2477 | N | VAL | B | 108 | 7.105 | -25.629 | -3.518 | 1.00 | 24.22 |
| ATOM | 2478 | CA | VAL | B | 108 | 7.514 | -24.333 | -2.995 | 1.00 | 20.70 |
| ATOM | 2479 | CB | VAL | B | 108 | 7.564 | -23.284 | -4.101 | 1.00 | 21.32 |
| ATOM | 2480 | CG1 | VAL | B | 108 | 7.855 | -21.921 | -3.515 | 1.00 | 22.49 |
| ATOM | 2481 | CG2 | VAL | B | 108 | 8.646 | -23.653 | -5.104 | 1.00 | 23.04 |
| ATOM | 2482 | C | VAL | B | 108 | 6.551 | -23.907 | -1.915 | 1.00 | 19.41 |
| ATOM | 2483 | O | VAL | B | 108 | 6.962 | -23.417 | -0.863 | 1.00 | 18.44 |
| ATOM | 2484 | N | ILE | B | 109 | 5.265 | -24.141 | -2.158 | 1.00 | 20.40 |
| ATOM | 2485 | CA | ILE | B | 109 | 4.224 | -23.782 | -1.195 | 1.00 | 18.92 |
| ATOM | 2486 | CB | ILE | B | 109 | 2.814 | -24.089 | -1.752 | 1.00 | 17.57 |
| ATOM | 2487 | CG2 | ILE | B | 109 | 1.780 | -23.950 | -0.660 | 1.00 | 17.33 |
| ATOM | 2488 | CG1 | ILE | B | 109 | 2.498 | -23.173 | -2.936 | 1.00 | 19.00 |
| ATOM | 2489 | CD | ILE | B | 109 | 2.435 | -21.683 | -2.570 | 1.00 | 22.52 |
| ATOM | 2490 | C | ILE | B | 109 | 4.370 | -24.493 | 0.140 | 1.00 | 20.59 |
| ATOM | 2491 | O | ILE | B | 109 | 4.251 | -23.874 | 1.202 | 1.00 | 23.24 |
| ATOM | 2492 | N | ASN | B | 110 | 4.624 | -25.796 | 0.091 | 1.00 | 20.99 |
| ATOM | 2493 | CA | ASN | B | 110 | 4.766 | -26.604 | 1.307 | 1.00 | 22.09 |
| ATOM | 2494 | CB | ASN | B | 110 | 4.844 | -28.087 | 0.917 | 1.00 | 22.84 |
| ATOM | 2495 | CG | ASN | B | 110 | 4.769 | -29.018 | 2.099 | 1.00 | 22.29 |
| ATOM | 2496 | OD1 | ASN | B | 110 | 4.146 | -28.724 | 3.118 | 1.00 | 20.82 |
| ATOM | 2497 | ND2 | ASN | B | 110 | 5.398 | -30.174 | 1.958 | 1.00 | 24.06 |
| ATOM | 2498 | C | ASN | B | 110 | 5.986 | -26.201 | 2.129 | 1.00 | 21.53 |
| ATOM | 2499 | O | ASN | B | 110 | 5.892 | -26.003 | 3.334 | 1.00 | 21.91 |
| ATOM | 2500 | N | ALA | B | 111 | 7.130 | -26.071 | 1.473 | 1.00 | 20.67 |
| ATOM | 2501 | CA | ALA | B | 111 | 8.354 | -25.679 | 2.166 | 1.00 | 21.79 |
| ATOM | 2502 | CB | ALA | B | 111 | 9.557 | -25.828 | 1.235 | 1.00 | 20.43 |
| ATOM | 2503 | C | ALA | B | 111 | 8.285 | -24.241 | 2.690 | 1.00 | 23.41 |
| ATOM | 2504 | O | ALA | B | 111 | 8.344 | -24.010 | 3.901 | 1.00 | 24.41 |
| ATOM | 2505 | N | ASN | B | 112 | 8.142 | -23.291 | 1.764 | 1.00 | 24.03 |
| ATOM | 2506 | CA | ASN | B | 112 | 8.102 | -21.858 | 2.070 | 1.00 | 23.29 |
| ATOM | 2507 | CB | ASN | B | 112 | 8.191 | -21.046 | 0.778 | 1.00 | 25.39 |
| ATOM | 2508 | CG | ASN | B | 112 | 9.462 | -21.306 | 0.028 | 1.00 | 26.15 |
| ATOM | 2509 | OD1 | ASN | B | 112 | 10.304 | -22.081 | 0.481 | 1.00 | 28.76 |
| ATOM | 2510 | ND2 | ASN | B | 112 | 9.619 | -20.665 | -1.128 | 1.00 | 27.90 |
| ATOM | 2511 | C | ASN | B | 112 | 6.921 | -21.340 | 2.863 | 1.00 | 23.51 |
| ATOM | 2512 | O | ASN | B | 112 | 7.090 | -20.607 | 3.834 | 1.00 | 22.70 |
| ATOM | 2513 | N | LEU | B | 113 | 5.719 | -21.710 | 2.454 | 1.00 | 23.78 |
| ATOM | 2514 | CA | LEU | B | 113 | 4.540 | -21.211 | 3.131 | 1.00 | 21.95 |
| ATOM | 2515 | CB | LEU | B | 113 | 3.434 | -20.968 | 2.103 | 1.00 | 19.20 |
| ATOM | 2516 | CG | LEU | B | 113 | 2.104 | -20.434 | 2.631 | 1.00 | 19.62 |
| ATOM | 2517 | CD1 | LEU | B | 113 | 2.334 | -19.356 | 3.691 | 1.00 | 17.87 |
| ATOM | 2518 | CD2 | LEU | B | 113 | 1.298 | -19.890 | 1.464 | 1.00 | 20.01 |

Figure 3 (suite 34)

| | | | | | | | | | | |
|---|---|---|---|---|---|---|---|---|---|---|
| ATOM | 2519 | C | LEU | B | 113 | 4.059 | -22.106 | 4.265 | 1.00 | 22.51 |
| ATOM | 2520 | O | LEU | B | 113 | 3.982 | -21.666 | 5.421 | 1.00 | 22.62 |
| ATOM | 2521 | N | THR | B | 114 | 3.730 | -23.356 | 3.960 | 1.00 | 20.95 |
| ATOM | 2522 | CA | THR | B | 114 | 3.270 | -24.240 | 5.023 | 1.00 | 22.27 |
| ATOM | 2523 | CB | THR | B | 114 | 2.765 | -25.597 | 4.471 | 1.00 | 21.90 |
| ATOM | 2524 | OG1 | THR | B | 114 | 1.788 | -25.352 | 3.454 | 1.00 | 23.26 |
| ATOM | 2525 | CG2 | THR | B | 114 | 2.112 | -26.414 | 5.576 | 1.00 | 17.00 |
| ATOM | 2526 | C | THR | B | 114 | 4.431 | -24.467 | 5.983 | 1.00 | 21.54 |
| ATOM | 2527 | O | THR | B | 114 | 4.230 | -24.719 | 7.173 | 1.00 | 23.38 |
| ATOM | 2528 | N | GLY | B | 115 | 5.647 | -24.348 | 5.467 | 1.00 | 22.12 |
| ATOM | 2529 | CA | GLY | B | 115 | 6.822 | -24.520 | 6.306 | 1.00 | 22.02 |
| ATOM | 2530 | C | GLY | B | 115 | 6.869 | -23.457 | 7.388 | 1.00 | 21.15 |
| ATOM | 2531 | O | GLY | B | 115 | 7.191 | -23.735 | 8.549 | 1.00 | 20.31 |
| ATOM | 2532 | N | ALA | B | 116 | 6.530 | -22.233 | 7.004 | 1.00 | 20.18 |
| ATOM | 2533 | CA | ALA | B | 116 | 6.529 | -21.105 | 7.927 | 1.00 | 19.88 |
| ATOM | 2534 | CB | ALA | B | 116 | 6.308 | -19.801 | 7.159 | 1.00 | 18.23 |
| ATOM | 2535 | C | ALA | B | 116 | 5.436 | -21.308 | 8.948 | 1.00 | 20.53 |
| ATOM | 2536 | O | ALA | B | 116 | 5.621 | -21.032 | 10.136 | 1.00 | 20.25 |
| ATOM | 2537 | N | PHE | B | 117 | 4.283 | -21.791 | 8.492 | 1.00 | 20.35 |
| ATOM | 2538 | CA | PHE | B | 117 | 3.188 | -22.041 | 9.420 | 1.00 | 19.97 |
| ATOM | 2539 | CB | PHE | B | 117 | 1.971 | -22.639 | 8.708 | 1.00 | 17.71 |
| ATOM | 2540 | CG | PHE | B | 117 | 1.039 | -23.360 | 9.643 | 1.00 | 20.39 |
| ATOM | 2541 | CD1 | PHE | B | 117 | 0.228 | -22.654 | 10.516 | 1.00 | 22.22 |
| ATOM | 2542 | CD2 | PHE | B | 117 | 1.060 | -24.752 | 9.731 | 1.00 | 21.70 |
| ATOM | 2543 | CE1 | PHE | B | 117 | -0.540 | -23.316 | 11.467 | 1.00 | 21.61 |
| ATOM | 2544 | CE2 | PHE | B | 117 | 0.292 | -25.425 | 10.681 | 1.00 | 20.42 |
| ATOM | 2545 | CZ | PHE | B | 117 | -0.505 | -24.707 | 11.549 | 1.00 | 20.85 |
| ATOM | 2546 | C | PHE | B | 117 | 3.628 | -23.013 | 10.518 | 1.00 | 21.32 |
| ATOM | 2547 | O | PHE | B | 117 | 3.402 | -22.767 | 11.703 | 1.00 | 21.45 |
| ATOM | 2548 | N | ARG | B | 118 | 4.253 | -24.117 | 10.114 | 1.00 | 21.34 |
| ATOM | 2549 | CA | ARG | B | 118 | 4.698 | -25.146 | 11.059 | 1.00 | 21.95 |
| ATOM | 2550 | CB | ARG | B | 118 | 5.400 | -26.279 | 10.303 | 1.00 | 21.93 |
| ATOM | 2551 | CG | ARG | B | 118 | 4.487 | -27.009 | 9.331 | 1.00 | 23.11 |
| ATOM | 2552 | CD | ARG | B | 118 | 5.140 | -28.236 | 8.710 | 1.00 | 23.64 |
| ATOM | 2553 | NE | ARG | B | 118 | 4.254 | -28.896 | 7.751 | 1.00 | 23.42 |
| ATOM | 2554 | CZ | ARG | B | 118 | 4.342 | -28.748 | 6.433 | 1.00 | 21.79 |
| ATOM | 2555 | NH1 | ARG | B | 118 | 5.275 | -27.968 | 5.913 | 1.00 | 20.46 |
| ATOM | 2556 | NH2 | ARG | B | 118 | 3.500 | -29.380 | 5.629 | 1.00 | 21.84 |
| ATOM | 2557 | C | ARG | B | 118 | 5.597 | -24.628 | 12.181 | 1.00 | 22.51 |
| ATOM | 2558 | O | ARG | B | 118 | 5.321 | -24.848 | 13.368 | 1.00 | 21.38 |
| ATOM | 2559 | N | VAL | B | 119 | 6.677 | -23.944 | 11.814 | 1.00 | 21.85 |
| ATOM | 2560 | CA | VAL | B | 119 | 7.581 | -23.407 | 12.823 | 1.00 | 21.34 |
| ATOM | 2561 | CB | VAL | B | 119 | 8.836 | -22.803 | 12.168 | 1.00 | 21.43 |
| ATOM | 2562 | CG1 | VAL | B | 119 | 9.599 | -23.897 | 11.447 | 1.00 | 19.75 |
| ATOM | 2563 | CG2 | VAL | B | 119 | 8.450 | -21.707 | 11.172 | 1.00 | 20.37 |
| ATOM | 2564 | C | VAL | B | 119 | 6.850 | -22.362 | 13.659 | 1.00 | 22.16 |
| ATOM | 2565 | O | VAL | B | 119 | 7.023 | -22.300 | 14.871 | 1.00 | 23.39 |
| ATOM | 2566 | N | ALA | B | 120 | 5.996 | -21.571 | 13.010 | 1.00 | 22.92 |
| ATOM | 2567 | CA | ALA | B | 120 | 5.226 | -20.529 | 13.697 | 1.00 | 22.68 |
| ATOM | 2568 | CB | ALA | B | 120 | 4.539 | -19.611 | 12.665 | 1.00 | 20.63 |
| ATOM | 2569 | C | ALA | B | 120 | 4.187 | -21.109 | 14.652 | 1.00 | 22.39 |
| ATOM | 2570 | O | ALA | B | 120 | 3.945 | -20.565 | 15.722 | 1.00 | 21.67 |
| ATOM | 2571 | N | GLN | B | 121 | 3.558 | -22.212 | 14.268 | 1.00 | 24.11 |
| ATOM | 2572 | CA | GLN | B | 121 | 2.554 | -22.830 | 15.131 | 1.00 | 24.84 |
| ATOM | 2573 | CB | GLN | B | 121 | 1.756 | -23.879 | 14.338 | 1.00 | 24.95 |
| ATOM | 2574 | CG | GLN | B | 121 | 0.492 | -24.386 | 15.039 | 1.00 | 27.04 |
| ATOM | 2575 | CD | GLN | B | 121 | 0.767 | -25.462 | 16.073 | 1.00 | 27.14 |
| ATOM | 2576 | OE1 | GLN | B | 121 | -0.002 | -25.639 | 17.022 | 1.00 | 29.92 |
| ATOM | 2577 | NE2 | GLN | B | 121 | 1.860 | -26.198 | 15.890 | 1.00 | 28.24 |
| ATOM | 2578 | C | GLN | B | 121 | 3.251 | -23.474 | 16.333 | 1.00 | 26.99 |
| ATOM | 2579 | O | GLN | B | 121 | 2.810 | -23.335 | 17.478 | 1.00 | 26.49 |
| ATOM | 2580 | N | ARG | B | 122 | 4.351 | -24.171 | 16.058 | 1.00 | 27.35 |
| ATOM | 2581 | CA | ARG | B | 122 | 5.121 | -24.843 | 17.097 | 1.00 | 30.63 |
| ATOM | 2582 | CB | ARG | B | 122 | 6.277 | -25.628 | 16.463 | 1.00 | 30.55 |
| ATOM | 2583 | CG | ARG | B | 122 | 7.173 | -26.335 | 17.464 | 1.00 | 35.32 |
| ATOM | 2584 | CD | ARG | B | 122 | 6.476 | -27.500 | 18.170 | 1.00 | 36.97 |
| ATOM | 2585 | NE | ARG | B | 122 | 7.414 | -28.245 | 19.008 | 1.00 | 39.64 |
| ATOM | 2586 | CZ | ARG | B | 122 | 7.844 | -27.841 | 20.202 | 1.00 | 40.89 |
| ATOM | 2587 | NH1 | ARG | B | 122 | 7.415 | -26.696 | 20.714 | 1.00 | 40.87 |
| ATOM | 2588 | NH2 | ARG | B | 122 | 8.715 | -28.576 | 20.880 | 1.00 | 41.19 |
| ATOM | 2589 | C | ARG | B | 122 | 5.662 | -23.856 | 18.134 | 1.00 | 30.83 |
| ATOM | 2590 | O | ARG | B | 122 | 5.782 | -24.188 | 19.316 | 1.00 | 28.63 |
| ATOM | 2591 | N | ALA | B | 123 | 5.965 | -22.636 | 17.696 | 1.00 | 30.84 |

Figure 3 (suite 35)

| | | | | | | | | | | |
|---|---|---|---|---|---|---|---|---|---|---|
| ATOM | 2592 | CA | ALA | B | 123 | 6.497 | -21.627 | 18.606 | 1.00 | 31.21 |
| ATOM | 2593 | CB | ALA | B | 123 | 7.427 | -20.677 | 17.847 | 1.00 | 29.98 |
| ATOM | 2594 | C | ALA | B | 123 | 5.442 | -20.825 | 19.353 | 1.00 | 31.90 |
| ATOM | 2595 | O | ALA | B | 123 | 5.692 | -20.376 | 20.472 | 1.00 | 31.17 |
| ATOM | 2596 | N | SER | B | 124 | 4.264 | -20.657 | 18.754 | 1.00 | 32.92 |
| ATOM | 2597 | CA | SER | B | 124 | 3.209 | -19.852 | 19.373 | 1.00 | 33.92 |
| ATOM | 2598 | CB | SER | B | 124 | 1.918 | -19.921 | 18.544 | 1.00 | 34.41 |
| ATOM | 2599 | OG | SER | B | 124 | 1.204 | -21.124 | 18.749 | 1.00 | 34.18 |
| ATOM | 2600 | C | SER | B | 124 | 2.903 | -20.198 | 20.824 | 1.00 | 34.96 |
| ATOM | 2601 | O | SER | B | 124 | 2.620 | -19.312 | 21.634 | 1.00 | 35.99 |
| ATOM | 2602 | N | ARG | B | 125 | 2.959 | -21.483 | 21.152 | 1.00 | 35.34 |
| ATOM | 2603 | CA | ARG | B | 125 | 2.686 | -21.952 | 22.509 | 1.00 | 36.10 |
| ATOM | 2604 | CB | ARG | B | 125 | 2.912 | -23.471 | 22.575 | 1.00 | 40.25 |
| ATOM | 2605 | CG | ARG | B | 125 | 2.812 | -24.090 | 23.977 | 1.00 | 43.17 |
| ATOM | 2606 | CD | ARG | B | 125 | 3.433 | -25.498 | 24.028 | 1.00 | 46.47 |
| ATOM | 2607 | NE | ARG | B | 125 | 4.876 | -25.483 | 23.764 | 1.00 | 49.73 |
| ATOM | 2608 | CZ | ARG | B | 125 | 5.799 | -25.029 | 24.613 | 1.00 | 50.68 |
| ATOM | 2609 | NH1 | ARG | B | 125 | 5.441 | -24.552 | 25.801 | 1.00 | 50.91 |
| ATOM | 2610 | NH2 | ARG | B | 125 | 7.081 | -25.034 | 24.264 | 1.00 | 49.04 |
| ATOM | 2611 | C | ARG | B | 125 | 3.540 | -21.267 | 23.587 | 1.00 | 34.26 |
| ATOM | 2612 | O | ARG | B | 125 | 3.017 | -20.560 | 24.450 | 1.00 | 33.58 |
| ATOM | 2613 | N | SER | B | 126 | 4.849 | -21.496 | 23.532 | 1.00 | 31.96 |
| ATOM | 2614 | CA | SER | B | 126 | 5.796 | -20.952 | 24.509 | 1.00 | 31.29 |
| ATOM | 2615 | CB | SER | B | 126 | 7.200 | -21.480 | 24.207 | 1.00 | 30.09 |
| ATOM | 2616 | OG | SER | B | 126 | 8.086 | -21.194 | 25.272 | 1.00 | 31.85 |
| ATOM | 2617 | C | SER | B | 126 | 5.825 | -19.425 | 24.574 | 1.00 | 31.55 |
| ATOM | 2618 | O | SER | B | 126 | 5.673 | -18.843 | 25.644 | 1.00 | 31.07 |
| ATOM | 2619 | N | MET | B | 127 | 6.032 | -18.786 | 23.427 | 1.00 | 30.98 |
| ATOM | 2620 | CA | MET | B | 127 | 6.061 | -17.329 | 23.339 | 1.00 | 29.95 |
| ATOM | 2621 | CB | MET | B | 127 | 6.160 | -16.889 | 21.881 | 1.00 | 27.73 |
| ATOM | 2622 | CG | MET | B | 127 | 7.367 | -17.414 | 21.163 | 1.00 | 28.73 |
| ATOM | 2623 | SD | MET | B | 127 | 7.312 | -16.901 | 19.454 | 1.00 | 31.50 |
| ATOM | 2624 | CE | MET | B | 127 | 9.013 | -17.086 | 18.961 | 1.00 | 22.51 |
| ATOM | 2625 | C | MET | B | 127 | 4.800 | -16.721 | 23.936 | 1.00 | 29.37 |
| ATOM | 2626 | O | MET | B | 127 | 4.857 | -15.749 | 24.686 | 1.00 | 27.98 |
| ATOM | 2627 | N | GLN | B | 128 | 3.655 | -17.301 | 23.604 | 1.00 | 30.05 |
| ATOM | 2628 | CA | GLN | B | 128 | 2.393 | -16.785 | 24.106 | 1.00 | 32.26 |
| ATOM | 2629 | CB | GLN | B | 128 | 1.227 | -17.506 | 23.427 | 1.00 | 34.36 |
| ATOM | 2630 | CG | GLN | B | 128 | -0.149 | -17.008 | 23.851 | 1.00 | 40.12 |
| ATOM | 2631 | CD | GLN | B | 128 | -1.281 | -17.665 | 23.072 | 1.00 | 42.87 |
| ATOM | 2632 | OE1 | GLN | B | 128 | -2.457 | -17.484 | 23.400 | 1.00 | 45.34 |
| ATOM | 2633 | NE2 | GLN | B | 128 | -0.932 | -18.428 | 22.035 | 1.00 | 43.30 |
| ATOM | 2634 | C | GLN | B | 128 | 2.276 | -16.909 | 25.617 | 1.00 | 32.98 |
| ATOM | 2635 | O | GLN | B | 128 | 1.786 | -15.995 | 26.279 | 1.00 | 34.70 |
| ATOM | 2636 | N | ARG | B | 129 | 2.732 | -18.029 | 26.168 | 1.00 | 31.98 |
| ATOM | 2637 | CA | ARG | B | 129 | 2.648 | -18.242 | 27.609 | 1.00 | 32.51 |
| ATOM | 2638 | CB | ARG | B | 129 | 2.931 | -19.713 | 27.951 | 1.00 | 32.15 |
| ATOM | 2639 | CG | ARG | B | 129 | 1.912 | -20.690 | 27.381 | 0.00 | 32.38 |
| ATOM | 2640 | CD | ARG | B | 129 | 0.522 | -20.507 | 27.989 | 0.00 | 32.41 |
| ATOM | 2641 | NE | ARG | B | 129 | 0.465 | -20.883 | 29.401 | 0.00 | 32.48 |
| ATOM | 2642 | CZ | ARG | B | 129 | 0.800 | -20.084 | 30.411 | 0.00 | 32.51 |
| ATOM | 2643 | NH1 | ARG | B | 129 | 1.218 | -18.847 | 30.179 | 0.00 | 32.53 |
| ATOM | 2644 | NH2 | ARG | B | 129 | 0.715 | -20.524 | 31.660 | 0.00 | 32.53 |
| ATOM | 2645 | C | ARG | B | 129 | 3.608 | -17.341 | 28.378 | 1.00 | 32.22 |
| ATOM | 2646 | O | ARG | B | 129 | 3.247 | -16.763 | 29.405 | 1.00 | 30.15 |
| ATOM | 2647 | N | ASN | B | 130 | 4.831 | -17.220 | 27.878 | 1.00 | 31.67 |
| ATOM | 2648 | CA | ASN | B | 130 | 5.832 | -16.387 | 28.532 | 1.00 | 31.66 |
| ATOM | 2649 | CB | ASN | B | 130 | 7.233 | -16.857 | 28.141 | 1.00 | 32.51 |
| ATOM | 2650 | CG | ASN | B | 130 | 7.584 | -18.200 | 28.756 | 1.00 | 32.98 |
| ATOM | 2651 | OD1 | ASN | B | 130 | 7.725 | -18.322 | 29.973 | 1.00 | 37.07 |
| ATOM | 2652 | ND2 | ASN | B | 130 | 7.720 | -19.215 | 27.919 | 1.00 | 32.70 |
| ATOM | 2653 | C | ASN | B | 130 | 5.661 | -14.910 | 28.195 | 1.00 | 30.97 |
| ATOM | 2654 | O | ASN | B | 130 | 6.544 | -14.105 | 28.458 | 1.00 | 30.98 |
| ATOM | 2655 | N | LYS | B | 131 | 4.519 | -14.570 | 27.606 | 1.00 | 30.94 |
| ATOM | 2656 | CA | LYS | B | 131 | 4.205 | -13.198 | 27.225 | 1.00 | 30.94 |
| ATOM | 2657 | CB | LYS | B | 131 | 3.799 | -12.386 | 28.452 | 1.00 | 31.84 |
| ATOM | 2658 | CG | LYS | B | 131 | 2.291 | -12.309 | 28.691 | 1.00 | 33.57 |
| ATOM | 2659 | CD | LYS | B | 131 | 1.767 | -13.430 | 29.583 | 1.00 | 35.76 |
| ATOM | 2660 | CE | LYS | B | 131 | 0.390 | -13.071 | 30.149 | 1.00 | 37.63 |
| ATOM | 2661 | NZ | LYS | B | 131 | -0.198 | -14.117 | 31.042 | 1.00 | 38.07 |
| ATOM | 2662 | C | LYS | B | 131 | 5.299 | -12.446 | 26.466 | 1.00 | 29.77 |
| ATOM | 2663 | O | LYS | B | 131 | 5.509 | -11.255 | 26.689 | 1.00 | 32.99 |
| ATOM | 2664 | N | PHE | B | 132 | 5.999 | -13.144 | 25.583 | 1.00 | 27.39 |

Figure 3 (suite 36)

| | | | | | | | | | | |
|---|---|---|---|---|---|---|---|---|---|---|
| ATOM | 2665 | CA | PHE | B | 132 | 7.035 | -12.536 | 24.755 | 1.00 | 26.07 |
| ATOM | 2666 | CB | PHE | B | 132 | 8.394 | -12.432 | 25.470 | 1.00 | 23.05 |
| ATOM | 2667 | CG | PHE | B | 132 | 9.475 | -11.826 | 24.601 | 1.00 | 20.00 |
| ATOM | 2668 | CD1 | PHE | B | 132 | 9.527 | -10.454 | 24.386 | 1.00 | 19.18 |
| ATOM | 2669 | CD2 | PHE | B | 132 | 10.373 | -12.636 | 23.912 | 1.00 | 21.62 |
| ATOM | 2670 | CE1 | PHE | B | 132 | 10.450 | -9.897 | 23.493 | 1.00 | 20.74 |
| ATOM | 2671 | CE2 | PHE | B | 132 | 11.300 | -12.091 | 23.018 | 1.00 | 20.95 |
| ATOM | 2672 | CZ | PHE | B | 132 | 11.336 | -10.718 | 22.808 | 1.00 | 19.00 |
| ATOM | 2673 | C | PHE | B | 132 | 7.215 | -13.388 | 23.516 | 1.00 | 24.82 |
| ATOM | 2674 | O | PHE | B | 132 | 7.104 | -14.598 | 23.583 | 1.00 | 25.96 |
| ATOM | 2675 | N | GLY | B | 133 | 7.508 | -12.758 | 22.388 | 1.00 | 24.87 |
| ATOM | 2676 | CA | GLY | B | 133 | 7.698 | -13.521 | 21.172 | 1.00 | 25.39 |
| ATOM | 2677 | C | GLY | B | 133 | 7.880 | -12.653 | 19.947 | 1.00 | 25.26 |
| ATOM | 2678 | O | GLY | B | 133 | 7.139 | -11.694 | 19.728 | 1.00 | 25.47 |
| ATOM | 2679 | N | ARG | B | 134 | 8.884 | -12.989 | 19.150 | 1.00 | 24.77 |
| ATOM | 2680 | CA | ARG | B | 134 | 9.164 | -12.259 | 17.929 | 1.00 | 23.46 |
| ATOM | 2681 | CB | ARG | B | 134 | 10.455 | -11.438 | 18.059 | 1.00 | 23.00 |
| ATOM | 2682 | CG | ARG | B | 134 | 10.451 | -10.424 | 19.189 | 1.00 | 20.89 |
| ATOM | 2683 | CD | ARG | B | 134 | 9.629 | -9.189 | 18.824 | 1.00 | 21.93 |
| ATOM | 2684 | NE | ARG | B | 134 | 9.638 | -8.178 | 19.880 | 1.00 | 23.22 |
| ATOM | 2685 | CZ | ARG | B | 134 | 8.814 | -8.167 | 20.923 | 1.00 | 26.72 |
| ATOM | 2686 | NH1 | ARG | B | 134 | 7.894 | -9.116 | 21.063 | 1.00 | 28.27 |
| ATOM | 2687 | NH2 | ARG | B | 134 | 8.901 | -7.203 | 21.832 | 1.00 | 28.73 |
| ATOM | 2688 | C | ARG | B | 134 | 9.325 | -13.273 | 16.818 | 1.00 | 23.15 |
| ATOM | 2689 | O | ARG | B | 134 | 10.260 | -14.068 | 16.814 | 1.00 | 24.62 |
| ATOM | 2690 | N | MET | B | 135 | 8.383 | -13.263 | 15.890 | 1.00 | 24.99 |
| ATOM | 2691 | CA | MET | B | 135 | 8.430 | -14.152 | 14.745 | 1.00 | 24.15 |
| ATOM | 2692 | CB | MET | B | 135 | 7.053 | -14.774 | 14.469 | 1.00 | 25.04 |
| ATOM | 2693 | CG | MET | B | 135 | 6.511 | -15.652 | 15.585 | 1.00 | 25.25 |
| ATOM | 2694 | SD | MET | B | 135 | 5.036 | -16.591 | 15.059 | 1.00 | 30.03 |
| ATOM | 2695 | CE | MET | B | 135 | 4.269 | -16.950 | 16.606 | 1.00 | 23.03 |
| ATOM | 2696 | C | MET | B | 135 | 8.824 | -13.238 | 13.602 | 1.00 | 23.07 |
| ATOM | 2697 | O | MET | B | 135 | 8.145 | -12.255 | 13.325 | 1.00 | 21.19 |
| ATOM | 2698 | N | ILE | B | 136 | 9.926 | -13.560 | 12.945 | 1.00 | 23.40 |
| ATOM | 2699 | CA | ILE | B | 136 | 10.421 | -12.756 | 11.851 | 1.00 | 21.75 |
| ATOM | 2700 | CB | ILE | B | 136 | 11.788 | -12.148 | 12.228 | 1.00 | 20.79 |
| ATOM | 2701 | CG2 | ILE | B | 136 | 12.334 | -11.323 | 11.070 | 1.00 | 19.94 |
| ATOM | 2702 | CG1 | ILE | B | 136 | 11.627 | -11.331 | 13.518 | 1.00 | 20.78 |
| ATOM | 2703 | CD | ILE | B | 136 | 12.903 | -11.009 | 14.245 | 1.00 | 13.03 |
| ATOM | 2704 | C | ILE | B | 136 | 10.559 | -13.626 | 10.615 | 1.00 | 22.08 |
| ATOM | 2705 | O | ILE | B | 136 | 11.530 | -14.377 | 10.476 | 1.00 | 22.09 |
| ATOM | 2706 | N | PHE | B | 137 | 9.584 | -13.519 | 9.715 | 1.00 | 20.58 |
| ATOM | 2707 | CA | PHE | B | 137 | 9.603 | -14.306 | 8.488 | 1.00 | 19.18 |
| ATOM | 2708 | CB | PHE | B | 137 | 8.177 | -14.595 | 8.000 | 1.00 | 14.55 |
| ATOM | 2709 | CG | PHE | B | 137 | 7.287 | -15.191 | 9.044 | 1.00 | 16.43 |
| ATOM | 2710 | CD1 | PHE | B | 137 | 6.590 | -14.379 | 9.924 | 1.00 | 17.52 |
| ATOM | 2711 | CD2 | PHE | B | 137 | 7.150 | -16.575 | 9.157 | 1.00 | 15.15 |
| ATOM | 2712 | CE1 | PHE | B | 137 | 5.761 | -14.936 | 10.904 | 1.00 | 18.48 |
| ATOM | 2713 | CE2 | PHE | B | 137 | 6.334 | -17.133 | 10.127 | 1.00 | 15.06 |
| ATOM | 2714 | CZ | PHE | B | 137 | 5.638 | -16.319 | 11.001 | 1.00 | 16.05 |
| ATOM | 2715 | C | PHE | B | 137 | 10.362 | -13.587 | 7.389 | 1.00 | 18.23 |
| ATOM | 2716 | O | PHE | B | 137 | 10.212 | -12.384 | 7.209 | 1.00 | 19.17 |
| ATOM | 2717 | N | ILE | B | 138 | 11.190 | -14.325 | 6.663 | 1.00 | 18.75 |
| ATOM | 2718 | CA | ILE | B | 138 | 11.933 | -13.752 | 5.570 | 1.00 | 20.82 |
| ATOM | 2719 | CB | ILE | B | 138 | 13.344 | -14.389 | 5.426 | 1.00 | 21.39 |
| ATOM | 2720 | CG2 | ILE | B | 138 | 14.111 | -13.704 | 4.300 | 1.00 | 20.99 |
| ATOM | 2721 | CG1 | ILE | B | 138 | 14.126 | -14.287 | 6.744 | 1.00 | 18.59 |
| ATOM | 2722 | CD | ILE | B | 138 | 14.164 | -12.898 | 7.363 | 1.00 | 16.82 |
| ATOM | 2723 | C | ILE | B | 138 | 11.119 | -14.031 | 4.314 | 1.00 | 23.91 |
| ATOM | 2724 | O | ILE | B | 138 | 10.728 | -15.172 | 4.053 | 1.00 | 24.74 |
| ATOM | 2725 | N | GLY | B | 139 | 10.841 | -12.983 | 3.547 | 1.00 | 27.28 |
| ATOM | 2726 | CA | GLY | B | 139 | 10.068 | -13.138 | 2.332 | 1.00 | 29.75 |
| ATOM | 2727 | C | GLY | B | 139 | 10.851 | -12.647 | 1.135 | 1.00 | 32.37 |
| ATOM | 2728 | O | GLY | B | 139 | 11.934 | -13.175 | 0.846 | 1.00 | 35.06 |
| ATOM | 2729 | N | SER | B | 140 | 10.301 | -11.648 | 0.439 | 1.00 | 33.25 |
| ATOM | 2730 | CA | SER | B | 140 | 10.938 | -11.046 | -0.735 | 1.00 | 33.95 |
| ATOM | 2731 | CB | SER | B | 140 | 11.372 | -12.117 | -1.735 | 1.00 | 35.94 |
| ATOM | 2732 | OG | SER | B | 140 | 10.248 | -12.747 | -2.335 | 1.00 | 37.28 |
| ATOM | 2733 | C | SER | B | 140 | 10.002 | -10.078 | -1.451 | 1.00 | 34.18 |
| ATOM | 2734 | O | SER | B | 140 | 8.792 | -10.043 | -1.197 | 1.00 | 32.46 |
| ATOM | 2735 | N | VAL | B | 141 | 10.580 | -9.305 | -2.363 | 1.00 | 36.58 |
| ATOM | 2736 | CA | VAL | B | 141 | 9.837 | -8.327 | -3.148 | 1.00 | 38.51 |
| ATOM | 2737 | CB | VAL | B | 141 | 10.779 | -7.495 | -4.043 | 1.00 | 38.15 |

Figure 3 (suite 37)

| | | | | | | | | | | |
|---|---|---|---|---|---|---|---|---|---|---|
| ATOM | 2738 | CG1 | VAL | B | 141 | 10.013 | -6.345 | -4.663 | 1.00 | 38.73 |
| ATOM | 2739 | CG2 | VAL | B | 141 | 11.946 | -6.988 | -3.242 | 1.00 | 40.58 |
| ATOM | 2740 | C | VAL | B | 141 | 8.830 | -9.015 | -4.064 | 1.00 | 38.02 |
| ATOM | 2741 | O | VAL | B | 141 | 7.746 | -8.484 | -4.312 | 1.00 | 38.82 |
| ATOM | 2742 | N | SER | B | 142 | 9.208 | -10.187 | -4.573 | 1.00 | 38.25 |
| ATOM | 2743 | CA | SER | B | 142 | 8.357 | -10.959 | -5.477 | 1.00 | 38.47 |
| ATOM | 2744 | CB | SER | B | 142 | 8.939 | -12.357 | -5.700 | 1.00 | 37.85 |
| ATOM | 2745 | OG | SER | B | 142 | 8.177 | -13.075 | -6.655 | 1.00 | 40.31 |
| ATOM | 2746 | C | SER | B | 142 | 6.948 | -11.080 | -4.918 | 1.00 | 37.84 |
| ATOM | 2747 | O | SER | B | 142 | 5.970 | -10.966 | -5.657 | 1.00 | 38.77 |
| ATOM | 2748 | N | GLY | B | 143 | 6.855 | -11.303 | -3.611 | 1.00 | 36.63 |
| ATOM | 2749 | CA | GLY | B | 143 | 5.562 | -11.430 | -2.966 | 1.00 | 37.56 |
| ATOM | 2750 | C | GLY | B | 143 | 4.519 | -10.399 | -3.380 | 1.00 | 36.87 |
| ATOM | 2751 | O | GLY | B | 143 | 3.397 | -10.762 | -3.723 | 1.00 | 38.29 |
| ATOM | 2752 | N | LEU | B | 144 | 4.866 | -9.114 | -3.352 | 1.00 | 37.92 |
| ATOM | 2753 | CA | LEU | B | 144 | 3.895 | -8.073 | -3.721 | 1.00 | 37.85 |
| ATOM | 2754 | CB | LEU | B | 144 | 3.689 | -7.117 | -2.554 | 1.00 | 35.75 |
| ATOM | 2755 | CG | LEU | B | 144 | 3.081 | -7.796 | -1.327 | 1.00 | 35.45 |
| ATOM | 2756 | CD1 | LEU | B | 144 | 3.159 | -6.875 | -0.114 | 1.00 | 35.29 |
| ATOM | 2757 | CD2 | LEU | B | 144 | 1.633 | -8.180 | -1.638 | 1.00 | 35.42 |
| ATOM | 2758 | C | LEU | B | 144 | 4.277 | -7.290 | -4.969 | 1.00 | 38.19 |
| ATOM | 2759 | O | LEU | B | 144 | 3.512 | -6.433 | -5.437 | 1.00 | 36.69 |
| ATOM | 2760 | N | TRP | B | 145 | 5.446 | -7.598 | -5.520 | 1.00 | 38.69 |
| ATOM | 2761 | CA | TRP | B | 145 | 5.914 | -6.918 | -6.718 | 1.00 | 40.88 |
| ATOM | 2762 | CB | TRP | B | 145 | 7.428 | -6.731 | -6.648 | 1.00 | 41.08 |
| ATOM | 2763 | CG | TRP | B | 145 | 7.947 | -5.685 | -7.579 | 1.00 | 44.07 |
| ATOM | 2764 | CD2 | TRP | B | 145 | 8.833 | -5.884 | -8.684 | 1.00 | 44.75 |
| ATOM | 2765 | CE2 | TRP | B | 145 | 9.048 | -4.621 | -9.279 | 1.00 | 44.32 |
| ATOM | 2766 | CE3 | TRP | B | 145 | 9.465 | -7.008 | -9.232 | 1.00 | 45.49 |
| ATOM | 2767 | CD1 | TRP | B | 145 | 7.669 | -4.346 | -7.547 | 1.00 | 44.66 |
| ATOM | 2768 | NE1 | TRP | B | 145 | 8.326 | -3.702 | -8.565 | 1.00 | 44.22 |
| ATOM | 2769 | CZ2 | TRP | B | 145 | 9.875 | -4.448 | -10.397 | 1.00 | 44.87 |
| ATOM | 2770 | CZ3 | TRP | B | 145 | 10.287 | -6.837 | -10.345 | 1.00 | 45.69 |
| ATOM | 2771 | CH2 | TRP | B | 145 | 10.483 | -5.565 | -10.915 | 1.00 | 45.79 |
| ATOM | 2772 | C | TRP | B | 145 | 5.535 | -7.722 | -7.964 | 1.00 | 41.44 |
| ATOM | 2773 | O | TRP | B | 145 | 5.464 | -7.182 | -9.072 | 1.00 | 40.12 |
| ATOM | 2774 | N | GLY | B | 146 | 5.301 | -9.018 | -7.770 | 1.00 | 42.61 |
| ATOM | 2775 | CA | GLY | B | 146 | 4.919 | -9.891 | -8.869 | 1.00 | 43.92 |
| ATOM | 2776 | C | GLY | B | 146 | 5.969 | -10.068 | -9.951 | 1.00 | 44.56 |
| ATOM | 2777 | O | GLY | B | 146 | 5.844 | -9.503 | -11.039 | 1.00 | 45.47 |
| ATOM | 2778 | N | ILE | B | 147 | 7.003 | -10.855 | -9.662 | 1.00 | 44.77 |
| ATOM | 2779 | CA | ILE | B | 147 | 8.071 | -11.107 | -10.630 | 1.00 | 43.88 |
| ATOM | 2780 | CB | ILE | B | 147 | 9.397 | -11.493 | -9.920 | 1.00 | 43.97 |
| ATOM | 2781 | CG2 | ILE | B | 147 | 10.523 | -11.612 | -10.943 | 1.00 | 44.38 |
| ATOM | 2782 | CG1 | ILE | B | 147 | 9.750 | -10.437 | -8.869 | 1.00 | 43.42 |
| ATOM | 2783 | CD | ILE | B | 147 | 11.076 | -10.667 | -8.172 | 1.00 | 43.63 |
| ATOM | 2784 | C | ILE | B | 147 | 7.657 | -12.245 | -11.566 | 1.00 | 42.48 |
| ATOM | 2785 | O | ILE | B | 147 | 6.985 | -13.191 | -11.147 | 1.00 | 42.12 |
| ATOM | 2786 | N | GLY | B | 148 | 8.057 | -12.147 | -12.829 | 1.00 | 41.20 |
| ATOM | 2787 | CA | GLY | B | 148 | 7.704 | -13.170 | -13.799 | 1.00 | 41.92 |
| ATOM | 2788 | C | GLY | B | 148 | 8.333 | -14.543 | -13.571 | 1.00 | 42.51 |
| ATOM | 2789 | O | GLY | B | 148 | 9.364 | -14.683 | -12.898 | 1.00 | 40.96 |
| ATOM | 2790 | N | ASN | B | 149 | 7.691 | -15.567 | -14.126 | 1.00 | 42.53 |
| ATOM | 2791 | CA | ASN | B | 149 | 8.177 | -16.936 | -14.019 | 1.00 | 44.12 |
| ATOM | 2792 | CB | ASN | B | 149 | 9.513 | -17.068 | -14.760 | 1.00 | 45.94 |
| ATOM | 2793 | CG | ASN | B | 149 | 9.385 | -16.791 | -16.250 | 1.00 | 49.63 |
| ATOM | 2794 | OD1 | ASN | B | 149 | 8.804 | -15.778 | -16.662 | 1.00 | 49.63 |
| ATOM | 2795 | ND2 | ASN | B | 149 | 9.931 | -17.690 | -17.067 | 1.00 | 48.80 |
| ATOM | 2796 | C | ASN | B | 149 | 8.340 | -17.451 | -12.586 | 1.00 | 43.98 |
| ATOM | 2797 | O | ASN | B | 149 | 9.318 | -18.137 | -12.282 | 1.00 | 44.04 |
| ATOM | 2798 | N | GLN | B | 150 | 7.398 | -17.124 | -11.705 | 1.00 | 41.49 |
| ATOM | 2799 | CA | GLN | B | 150 | 7.473 | -17.609 | -10.334 | 1.00 | 39.83 |
| ATOM | 2800 | CB | GLN | B | 150 | 8.620 | -16.925 | -9.577 | 1.00 | 43.38 |
| ATOM | 2801 | CG | GLN | B | 150 | 8.642 | -15.407 | -9.617 | 1.00 | 46.22 |
| ATOM | 2802 | CD | GLN | B | 150 | 9.976 | -14.853 | -9.139 | 1.00 | 47.76 |
| ATOM | 2803 | OE1 | GLN | B | 150 | 11.022 | -15.116 | -9.742 | 1.00 | 48.78 |
| ATOM | 2804 | NE2 | GLN | B | 150 | 9.948 | -14.089 | -8.052 | 1.00 | 48.03 |
| ATOM | 2805 | C | GLN | B | 150 | 6.170 | -17.488 | -9.562 | 1.00 | 36.62 |
| ATOM | 2806 | O | GLN | B | 150 | 6.140 | -17.022 | -8.428 | 1.00 | 35.61 |
| ATOM | 2807 | N | ALA | B | 151 | 5.093 | -17.954 | -10.185 | 1.00 | 33.32 |
| ATOM | 2808 | CA | ALA | B | 151 | 3.772 | -17.919 | -9.580 | 1.00 | 30.99 |
| ATOM | 2809 | CB | ALA | B | 151 | 2.758 | -18.570 | -10.517 | 1.00 | 30.35 |
| ATOM | 2810 | C | ALA | B | 151 | 3.751 | -18.620 | -8.229 | 1.00 | 29.13 |

Figure 3 (suite 38)

| | | | | | | | | | | |
|---|---|---|---|---|---|---|---|---|---|---|
| ATOM | 2811 | O | ALA | B | 151 | 2.980 | -18.252 | -7.345 | 1.00 | 27.67 |
| ATOM | 2812 | N | ASN | B | 152 | 4.590 | -19.639 | -8.076 | 1.00 | 26.81 |
| ATOM | 2813 | CA | ASN | B | 152 | 4.654 | -20.389 | -6.830 | 1.00 | 25.44 |
| ATOM | 2814 | CB | ASN | B | 152 | 5.269 | -21.791 | -7.050 | 1.00 | 24.51 |
| ATOM | 2815 | CG | ASN | B | 152 | 6.602 | -21.757 | -7.794 | 1.00 | 24.10 |
| ATOM | 2816 | OD1 | ASN | B | 152 | 7.346 | -22.743 | -7.792 | 1.00 | 30.20 |
| ATOM | 2817 | ND2 | ASN | B | 152 | 6.902 | -20.643 | -8.443 | 1.00 | 21.52 |
| ATOM | 2818 | C | ASN | B | 152 | 5.426 | -19.636 | -5.757 | 1.00 | 25.04 |
| ATOM | 2819 | O | ASN | B | 152 | 4.971 | -19.541 | -4.619 | 1.00 | 25.81 |
| ATOM | 2820 | N | TYR | B | 153 | 6.593 | -19.105 | -6.114 | 1.00 | 25.37 |
| ATOM | 2821 | CA | TYR | B | 153 | 7.405 | -18.345 | -5.163 | 1.00 | 25.86 |
| ATOM | 2822 | CB | TYR | B | 153 | 8.745 | -17.951 | -5.786 | 1.00 | 27.80 |
| ATOM | 2823 | CG | TYR | B | 153 | 9.749 | -17.508 | -4.754 | 1.00 | 34.01 |
| ATOM | 2824 | CD1 | TYR | B | 153 | 10.329 | -18.436 | -3.883 | 1.00 | 36.35 |
| ATOM | 2825 | CE1 | TYR | B | 153 | 11.235 | -18.037 | -2.906 | 1.00 | 38.54 |
| ATOM | 2826 | CD2 | TYR | B | 153 | 10.103 | -16.160 | -4.618 | 1.00 | 35.08 |
| ATOM | 2827 | CE2 | TYR | B | 153 | 11.008 | -15.753 | -3.640 | 1.00 | 36.93 |
| ATOM | 2828 | CZ | TYR | B | 153 | 11.571 | -16.698 | -2.787 | 1.00 | 39.09 |
| ATOM | 2829 | OH | TYR | B | 153 | 12.476 | -16.316 | -1.820 | 1.00 | 40.30 |
| ATOM | 2830 | C | TYR | B | 153 | 6.660 | -17.072 | -4.736 | 1.00 | 25.31 |
| ATOM | 2831 | O | TYR | B | 153 | 6.590 | -16.741 | -3.549 | 1.00 | 23.46 |
| ATOM | 2832 | N | ALA | B | 154 | 6.097 | -16.378 | -5.721 | 1.00 | 23.27 |
| ATOM | 2833 | CA | ALA | B | 154 | 5.349 | -15.145 | -5.487 | 1.00 | 21.90 |
| ATOM | 2834 | CB | ALA | B | 154 | 4.852 | -14.591 | -6.810 | 1.00 | 21.46 |
| ATOM | 2835 | C | ALA | B | 154 | 4.176 | -15.410 | -4.557 | 1.00 | 20.50 |
| ATOM | 2836 | O | ALA | B | 154 | 3.890 | -14.625 | -3.646 | 1.00 | 23.70 |
| ATOM | 2837 | N | ALA | B | 155 | 3.508 | -16.533 | -4.779 | 1.00 | 19.15 |
| ATOM | 2838 | CA | ALA | B | 155 | 2.358 | -16.915 | -3.962 | 1.00 | 18.95 |
| ATOM | 2839 | CB | ALA | B | 155 | 1.584 | -18.045 | -4.646 | 1.00 | 14.65 |
| ATOM | 2840 | C | ALA | B | 155 | 2.752 | -17.320 | -2.542 | 1.00 | 17.43 |
| ATOM | 2841 | O | ALA | B | 155 | 1.995 | -17.085 | -1.599 | 1.00 | 16.12 |
| ATOM | 2842 | N | SER | B | 156 | 3.932 | -17.919 | -2.371 | 1.00 | 18.80 |
| ATOM | 2843 | CA | SER | B | 156 | 4.358 | -18.322 | -1.025 | 1.00 | 20.28 |
| ATOM | 2844 | CB | SER | B | 156 | 5.529 | -19.329 | -1.091 | 1.00 | 21.93 |
| ATOM | 2845 | OG | SER | B | 156 | 6.769 | -18.729 | -1.457 | 1.00 | 24.23 |
| ATOM | 2846 | C | SER | B | 156 | 4.763 | -17.090 | -0.221 | 1.00 | 20.26 |
| ATOM | 2847 | O | SER | B | 156 | 4.364 | -16.910 | 0.932 | 1.00 | 21.32 |
| ATOM | 2848 | N | LYS | B | 157 | 5.538 | -16.223 | -0.853 | 1.00 | 22.12 |
| ATOM | 2849 | CA | LYS | B | 157 | 6.003 | -15.009 | -0.203 | 1.00 | 21.82 |
| ATOM | 2850 | CB | LYS | B | 157 | 6.990 | -14.286 | -1.117 | 1.00 | 24.27 |
| ATOM | 2851 | CG | LYS | B | 157 | 8.146 | -15.145 | -1.613 | 1.00 | 29.71 |
| ATOM | 2852 | CD | LYS | B | 157 | 9.162 | -15.510 | -0.504 | 1.00 | 34.37 |
| ATOM | 2853 | CE | LYS | B | 157 | 8.657 | -16.579 | 0.456 | 1.00 | 34.60 |
| ATOM | 2854 | NZ | LYS | B | 157 | 9.652 | -16.871 | 1.541 | 1.00 | 38.73 |
| ATOM | 2855 | C | LYS | B | 157 | 4.840 | -14.088 | 0.168 | 1.00 | 21.52 |
| ATOM | 2856 | O | LYS | B | 157 | 4.847 | -13.481 | 1.233 | 1.00 | 22.67 |
| ATOM | 2857 | N | ALA | B | 158 | 3.837 | -13.985 | -0.699 | 1.00 | 19.32 |
| ATOM | 2858 | CA | ALA | B | 158 | 2.689 | -13.123 | -0.399 | 1.00 | 19.26 |
| ATOM | 2859 | CB | ALA | B | 158 | 1.820 | -12.919 | -1.652 | 1.00 | 17.30 |
| ATOM | 2860 | C | ALA | B | 158 | 1.881 | -13.767 | 0.710 | 1.00 | 18.45 |
| ATOM | 2861 | O | ALA | B | 158 | 1.298 | -13.081 | 1.560 | 1.00 | 18.58 |
| ATOM | 2862 | N | GLY | B | 159 | 1.866 | -15.099 | 0.705 | 1.00 | 19.23 |
| ATOM | 2863 | CA | GLY | B | 159 | 1.140 | -15.835 | 1.723 | 1.00 | 19.72 |
| ATOM | 2864 | C | GLY | B | 159 | 1.708 | -15.669 | 3.123 | 1.00 | 19.80 |
| ATOM | 2865 | O | GLY | B | 159 | 0.952 | -15.634 | 4.089 | 1.00 | 19.58 |
| ATOM | 2866 | N | VAL | B | 160 | 3.029 | -15.557 | 3.261 | 1.00 | 20.59 |
| ATOM | 2867 | CA | VAL | B | 160 | 3.599 | -15.403 | 4.606 | 1.00 | 21.10 |
| ATOM | 2868 | CB | VAL | B | 160 | 5.151 | -15.450 | 4.600 | 1.00 | 23.13 |
| ATOM | 2869 | CG1 | VAL | B | 160 | 5.666 | -15.424 | 6.022 | 1.00 | 22.47 |
| ATOM | 2870 | CG2 | VAL | B | 160 | 5.640 | -16.712 | 3.907 | 1.00 | 23.03 |
| ATOM | 2871 | C | VAL | B | 160 | 3.149 | -14.090 | 5.227 | 1.00 | 20.68 |
| ATOM | 2872 | O | VAL | B | 160 | 2.880 | -14.012 | 6.425 | 1.00 | 20.30 |
| ATOM | 2873 | N | ILE | B | 161 | 3.073 | -13.053 | 4.397 | 1.00 | 21.09 |
| ATOM | 2874 | CA | ILE | B | 161 | 2.632 | -11.739 | 4.848 | 1.00 | 17.10 |
| ATOM | 2875 | CB | ILE | B | 161 | 2.571 | -10.724 | 3.665 | 1.00 | 17.79 |
| ATOM | 2876 | CG2 | ILE | B | 161 | 1.820 | -9.447 | 4.098 | 1.00 | 12.70 |
| ATOM | 2877 | CG1 | ILE | B | 161 | 3.991 | -10.410 | 3.183 | 1.00 | 16.24 |
| ATOM | 2878 | CD | ILE | B | 161 | 4.061 | -9.663 | 1.860 | 1.00 | 16.08 |
| ATOM | 2879 | C | ILE | B | 161 | 1.255 | -11.885 | 5.448 | 1.00 | 15.92 |
| ATOM | 2880 | O | ILE | B | 161 | 1.040 | -11.557 | 6.608 | 1.00 | 18.30 |
| ATOM | 2881 | N | GLY | B | 162 | 0.311 | -12.387 | 4.663 | 1.00 | 16.69 |
| ATOM | 2882 | CA | GLY | B | 162 | -1.039 | -12.561 | 5.187 | 1.00 | 15.72 |
| ATOM | 2883 | C | GLY | B | 162 | -1.058 | -13.387 | 6.461 | 1.00 | 16.79 |

Figure 3 (suite 39)

| | | | | | | | | | | |
|---|---|---|---|---|---|---|---|---|---|---|
| ATOM | 2884 | O | GLY | B | 162 | -1.790 | -13.079 | 7.413 | 1.00 | 15.14 |
| ATOM | 2885 | N | MET | B | 163 | -0.249 | -14.446 | 6.493 | 1.00 | 17.63 |
| ATOM | 2886 | CA | MET | B | 163 | -0.202 | -15.295 | 7.684 | 1.00 | 19.64 |
| ATOM | 2887 | CB | MET | B | 163 | 0.677 | -16.537 | 7.456 | 1.00 | 17.61 |
| ATOM | 2888 | CG | MET | B | 163 | 0.727 | -17.450 | 8.676 | 1.00 | 19.60 |
| ATOM | 2889 | SD | MET | B | 163 | 1.815 | -18.909 | 8.515 | 1.00 | 19.27 |
| ATOM | 2890 | CE | MET | B | 163 | 3.473 | -18.155 | 8.594 | 1.00 | 15.00 |
| ATOM | 2891 | C | MET | B | 163 | 0.345 | -14.490 | 8.852 | 1.00 | 20.11 |
| ATOM | 2892 | O | MET | B | 163 | -0.183 | -14.543 | 9.966 | 1.00 | 20.12 |
| ATOM | 2893 | N | ALA | B | 164 | 1.412 | -13.744 | 8.588 | 1.00 | 19.62 |
| ATOM | 2894 | CA | ALA | B | 164 | 2.033 | -12.919 | 9.616 | 1.00 | 19.58 |
| ATOM | 2895 | CB | ALA | B | 164 | 3.167 | -12.130 | 9.014 | 1.00 | 18.67 |
| ATOM | 2896 | C | ALA | B | 164 | 1.003 | -11.976 | 10.218 | 1.00 | 20.48 |
| ATOM | 2897 | O | ALA | B | 164 | 0.863 | -11.888 | 11.436 | 1.00 | 23.97 |
| ATOM | 2898 | N | ARG | B | 165 | 0.259 | -11.290 | 9.358 | 1.00 | 21.04 |
| ATOM | 2899 | CA | ARG | B | 165 | -0.742 | -10.343 | 9.821 | 1.00 | 20.92 |
| ATOM | 2900 | CB | ARG | B | 165 | -1.267 | -9.526 | 8.631 | 1.00 | 22.49 |
| ATOM | 2901 | CG | ARG | B | 165 | -0.196 | -8.523 | 8.149 | 1.00 | 22.71 |
| ATOM | 2902 | CD | ARG | B | 165 | -0.611 | -7.597 | 7.017 | 1.00 | 21.34 |
| ATOM | 2903 | NE | ARG | B | 165 | 0.462 | -6.633 | 6.775 | 1.00 | 24.05 |
| ATOM | 2904 | CZ | ARG | B | 165 | 0.484 | -5.716 | 5.812 | 1.00 | 26.40 |
| ATOM | 2905 | NH1 | ARG | B | 165 | -0.525 | -5.601 | 4.949 | 1.00 | 28.23 |
| ATOM | 2906 | NH2 | ARG | B | 165 | 1.537 | -4.908 | 5.709 | 1.00 | 28.55 |
| ATOM | 2907 | C | ARG | B | 165 | -1.874 | -10.947 | 10.629 | 1.00 | 22.73 |
| ATOM | 2908 | O | ARG | B | 165 | -2.352 | -10.325 | 11.586 | 1.00 | 26.19 |
| ATOM | 2909 | N | SER | B | 166 | -2.303 | -12.154 | 10.268 | 1.00 | 22.48 |
| ATOM | 2910 | CA | SER | B | 166 | -3.372 | -12.813 | 11.013 | 1.00 | 21.16 |
| ATOM | 2911 | CB | SER | B | 166 | -3.825 | -14.100 | 10.307 | 1.00 | 22.17 |
| ATOM | 2912 | OG | SER | B | 166 | -4.733 | -14.826 | 11.130 | 1.00 | 22.65 |
| ATOM | 2913 | C | SER | B | 166 | -2.834 | -13.149 | 12.394 | 1.00 | 19.55 |
| ATOM | 2914 | O | SER | B | 166 | -3.521 | -12.972 | 13.393 | 1.00 | 19.99 |
| ATOM | 2915 | N | ILE | B | 167 | -1.601 | -13.648 | 12.438 | 1.00 | 19.44 |
| ATOM | 2916 | CA | ILE | B | 167 | -0.942 | -13.980 | 13.701 | 1.00 | 20.27 |
| ATOM | 2917 | CB | ILE | B | 167 | 0.460 | -14.604 | 13.449 | 1.00 | 21.35 |
| ATOM | 2918 | CG2 | ILE | B | 167 | 1.248 | -14.682 | 14.756 | 1.00 | 22.18 |
| ATOM | 2919 | CG1 | ILE | B | 167 | 0.298 | -15.987 | 12.804 | 1.00 | 20.76 |
| ATOM | 2920 | CD | ILE | B | 167 | 1.596 | -16.687 | 12.461 | 1.00 | 20.04 |
| ATOM | 2921 | C | ILE | B | 167 | -0.784 | -12.685 | 14.502 | 1.00 | 21.38 |
| ATOM | 2922 | O | ILE | B | 167 | -1.254 | -12.581 | 15.633 | 1.00 | 19.86 |
| ATOM | 2923 | N | ALA | B | 168 | -0.131 | -11.697 | 13.902 | 1.00 | 21.59 |
| ATOM | 2924 | CA | ALA | B | 168 | 0.068 | -10.402 | 14.559 | 1.00 | 23.63 |
| ATOM | 2925 | CB | ALA | B | 168 | 0.610 | -9.364 | 13.553 | 1.00 | 21.65 |
| ATOM | 2926 | C | ALA | B | 168 | -1.224 | -9.891 | 15.183 | 1.00 | 24.37 |
| ATOM | 2927 | O | ALA | B | 168 | -1.221 | -9.445 | 16.326 | 1.00 | 23.85 |
| ATOM | 2928 | N | ARG | B | 169 | -2.335 | -9.966 | 14.453 | 1.00 | 26.12 |
| ATOM | 2929 | CA | ARG | B | 169 | -3.589 | -9.473 | 15.013 | 1.00 | 28.62 |
| ATOM | 2930 | CB | ARG | B | 169 | -4.676 | -9.355 | 13.930 | 1.00 | 31.23 |
| ATOM | 2931 | CG | ARG | B | 169 | -5.857 | -8.460 | 14.380 | 1.00 | 34.88 |
| ATOM | 2932 | CD | ARG | B | 169 | -6.898 | -8.206 | 13.293 | 1.00 | 36.47 |
| ATOM | 2933 | NE | ARG | B | 169 | -6.426 | -7.344 | 12.208 | 1.00 | 38.72 |
| ATOM | 2934 | CZ | ARG | B | 169 | -6.434 | -6.010 | 12.231 | 1.00 | 40.50 |
| ATOM | 2935 | NH1 | ARG | B | 169 | -6.892 | -5.355 | 13.291 | 1.00 | 39.73 |
| ATOM | 2936 | NH2 | ARG | B | 169 | -5.993 | -5.329 | 11.180 | 1.00 | 39.84 |
| ATOM | 2937 | C | ARG | B | 169 | -4.102 | -10.328 | 16.167 | 1.00 | 29.55 |
| ATOM | 2938 | O | ARG | B | 169 | -4.884 | -9.859 | 16.996 | 1.00 | 29.74 |
| ATOM | 2939 | N | GLU | B | 170 | -3.647 | -11.576 | 16.233 | 1.00 | 29.44 |
| ATOM | 2940 | CA | GLU | B | 170 | -4.074 | -12.494 | 17.284 | 1.00 | 29.76 |
| ATOM | 2941 | CB | GLU | B | 170 | -4.164 | -13.918 | 16.721 | 1.00 | 29.37 |
| ATOM | 2942 | CG | GLU | B | 170 | -4.764 | -14.942 | 17.671 | 1.00 | 27.62 |
| ATOM | 2943 | CD | GLU | B | 170 | -4.407 | -16.368 | 17.279 | 1.00 | 30.47 |
| ATOM | 2944 | OE1 | GLU | B | 170 | -4.525 | -16.702 | 16.076 | 1.00 | 30.26 |
| ATOM | 2945 | OE2 | GLU | B | 170 | -4.013 | -17.154 | 18.174 | 1.00 | 30.23 |
| ATOM | 2946 | C | GLU | B | 170 | -3.154 | -12.514 | 18.508 | 1.00 | 29.22 |
| ATOM | 2947 | O | GLU | B | 170 | -3.619 | -12.526 | 19.653 | 1.00 | 28.01 |
| ATOM | 2948 | N | LEU | B | 171 | -1.846 | -12.517 | 18.269 | 1.00 | 27.97 |
| ATOM | 2949 | CA | LEU | B | 171 | -0.897 | -12.588 | 19.377 | 1.00 | 27.45 |
| ATOM | 2950 | CB | LEU | B | 171 | 0.129 | -13.685 | 19.087 | 1.00 | 26.92 |
| ATOM | 2951 | CG | LEU | B | 171 | -0.466 | -15.046 | 18.725 | 1.00 | 23.41 |
| ATOM | 2952 | CD1 | LEU | B | 171 | 0.647 | -16.073 | 18.615 | 1.00 | 24.64 |
| ATOM | 2953 | CD2 | LEU | B | 171 | -1.469 | -15.459 | 19.783 | 1.00 | 24.15 |
| ATOM | 2954 | C | LEU | B | 171 | -0.164 | -11.302 | 19.796 | 1.00 | 27.79 |
| ATOM | 2955 | O | LEU | B | 171 | 0.692 | -11.347 | 20.688 | 1.00 | 26.41 |
| ATOM | 2956 | N | SER | B | 172 | -0.482 | -10.167 | 19.169 | 1.00 | 26.58 |

Figure 3 (suite 40)

| | | | | | | | | | | |
|---|---|---|---|---|---|---|---|---|---|---|
| ATOM | 2957 | CA | SER | B | 172 | 0.174 | -8.907 | 19.537 | 1.00 | 26.34 |
| ATOM | 2958 | CB | SER | B | 172 | -0.214 | -7.776 | 18.572 | 1.00 | 24.14 |
| ATOM | 2959 | OG | SER | B | 172 | 0.550 | -7.821 | 17.374 | 1.00 | 22.25 |
| ATOM | 2960 | C | SER | B | 172 | -0.202 | -8.510 | 20.965 | 1.00 | 26.89 |
| ATOM | 2961 | O | SER | B | 172 | 0.635 | -8.035 | 21.727 | 1.00 | 26.33 |
| ATOM | 2962 | N | LYS | B | 173 | -1.459 | -8.721 | 21.331 | 1.00 | 27.07 |
| ATOM | 2963 | CA | LYS | B | 173 | -1.925 | -8.371 | 22.665 | 1.00 | 28.54 |
| ATOM | 2964 | CB | LYS | B | 173 | -3.439 | -8.596 | 22.777 | 1.00 | 28.43 |
| ATOM | 2965 | CG | LYS | B | 173 | -3.870 | -10.038 | 22.552 | 1.00 | 30.42 |
| ATOM | 2966 | CD | LYS | B | 173 | -5.311 | -10.276 | 22.992 | 1.00 | 32.95 |
| ATOM | 2967 | CE | LYS | B | 173 | -6.299 | -9.547 | 22.105 | 1.00 | 33.59 |
| ATOM | 2968 | NZ | LYS | B | 173 | -6.262 | -10.100 | 20.727 | 1.00 | 36.12 |
| ATOM | 2969 | C | LYS | B | 173 | -1.212 | -9.181 | 23.743 | 1.00 | 28.44 |
| ATOM | 2970 | O | LYS | B | 173 | -1.394 | -8.944 | 24.928 | 1.00 | 29.39 |
| ATOM | 2971 | N | ALA | B | 174 | -0.402 | -10.144 | 23.330 | 1.00 | 27.76 |
| ATOM | 2972 | CA | ALA | B | 174 | 0.314 | -10.975 | 24.283 | 1.00 | 26.15 |
| ATOM | 2973 | CB | ALA | B | 174 | -0.037 | -12.451 | 24.058 | 1.00 | 24.47 |
| ATOM | 2974 | C | ALA | B | 174 | 1.809 | -10.758 | 24.132 | 1.00 | 26.48 |
| ATOM | 2975 | O | ALA | B | 174 | 2.608 | -11.580 | 24.567 | 1.00 | 27.83 |
| ATOM | 2976 | N | ASN | B | 175 | 2.170 | -9.633 | 23.518 | 1.00 | 28.64 |
| ATOM | 2977 | CA | ASN | B | 175 | 3.562 | -9.258 | 23.272 | 1.00 | 27.36 |
| ATOM | 2978 | CB | ASN | B | 175 | 4.332 | -9.096 | 24.591 | 1.00 | 29.14 |
| ATOM | 2979 | CG | ASN | B | 175 | 5.771 | -8.628 | 24.376 | 1.00 | 29.06 |
| ATOM | 2980 | OD1 | ASN | B | 175 | 6.060 | -7.882 | 23.433 | 1.00 | 29.98 |
| ATOM | 2981 | ND2 | ASN | B | 175 | 6.677 | -9.062 | 25.252 | 1.00 | 27.19 |
| ATOM | 2982 | C | ASN | B | 175 | 4.245 | -10.273 | 22.371 | 1.00 | 26.69 |
| ATOM | 2983 | O | ASN | B | 175 | 5.442 | -10.537 | 22.490 | 1.00 | 26.61 |
| ATOM | 2984 | N | VAL | B | 176 | 3.458 | -10.849 | 21.469 | 1.00 | 27.01 |
| ATOM | 2985 | CA | VAL | B | 176 | 3.951 | -11.815 | 20.499 | 1.00 | 25.61 |
| ATOM | 2986 | CB | VAL | B | 176 | 3.215 | -13.163 | 20.603 | 1.00 | 26.93 |
| ATOM | 2987 | CG1 | VAL | B | 176 | 3.627 | -14.072 | 19.449 | 1.00 | 25.47 |
| ATOM | 2988 | CG2 | VAL | B | 176 | 3.543 | -13.818 | 21.932 | 1.00 | 26.40 |
| ATOM | 2989 | C | VAL | B | 176 | 3.637 | -11.175 | 19.160 | 1.00 | 26.33 |
| ATOM | 2990 | O | VAL | B | 176 | 2.494 | -11.182 | 18.701 | 1.00 | 26.94 |
| ATOM | 2991 | N | THR | B | 177 | 4.665 | -10.606 | 18.549 | 1.00 | 25.51 |
| ATOM | 2992 | CA | THR | B | 177 | 4.535 | -9.913 | 17.289 | 1.00 | 23.68 |
| ATOM | 2993 | CB | THR | B | 177 | 5.367 | -8.633 | 17.334 | 1.00 | 23.92 |
| ATOM | 2994 | OG1 | THR | B | 177 | 6.731 | -8.965 | 17.635 | 1.00 | 22.47 |
| ATOM | 2995 | CG2 | THR | B | 177 | 4.837 | -7.700 | 18.412 | 1.00 | 22.93 |
| ATOM | 2996 | C | THR | B | 177 | 4.975 | -10.755 | 16.096 | 1.00 | 23.27 |
| ATOM | 2997 | O | THR | B | 177 | 5.700 | -11.744 | 16.243 | 1.00 | 20.28 |
| ATOM | 2998 | N | ALA | B | 178 | 4.535 | -10.345 | 14.912 | 1.00 | 21.41 |
| ATOM | 2999 | CA | ALA | B | 178 | 4.878 | -11.045 | 13.681 | 1.00 | 22.03 |
| ATOM | 3000 | CB | ALA | B | 178 | 3.737 | -11.999 | 13.275 | 1.00 | 22.23 |
| ATOM | 3001 | C | ALA | B | 178 | 5.150 | -10.055 | 12.560 | 1.00 | 20.99 |
| ATOM | 3002 | O | ALA | B | 178 | 4.255 | -9.323 | 12.132 | 1.00 | 19.37 |
| ATOM | 3003 | N | ASN | B | 179 | 6.380 | -10.054 | 12.064 | 1.00 | 19.91 |
| ATOM | 3004 | CA | ASN | B | 179 | 6.761 | -9.146 | 10.997 | 1.00 | 17.90 |
| ATOM | 3005 | CB | ASN | B | 179 | 7.724 | -8.085 | 11.540 | 1.00 | 16.04 |
| ATOM | 3006 | CG | ASN | B | 179 | 7.108 | -7.257 | 12.651 | 1.00 | 15.83 |
| ATOM | 3007 | OD1 | ASN | B | 179 | 6.185 | -6.493 | 12.424 | 1.00 | 13.33 |
| ATOM | 3008 | ND2 | ASN | B | 179 | 7.610 | -7.415 | 13.858 | 1.00 | 18.80 |
| ATOM | 3009 | C | ASN | B | 179 | 7.403 | -9.882 | 9.851 | 1.00 | 18.50 |
| ATOM | 3010 | O | ASN | B | 179 | 7.770 | -11.045 | 9.977 | 1.00 | 21.12 |
| ATOM | 3011 | N | VAL | B | 180 | 7.553 | -9.192 | 8.731 | 1.00 | 20.44 |
| ATOM | 3012 | CA | VAL | B | 180 | 8.156 | -9.770 | 7.549 | 1.00 | 18.50 |
| ATOM | 3013 | CB | VAL | B | 180 | 7.104 | -10.013 | 6.447 | 1.00 | 18.36 |
| ATOM | 3014 | CG1 | VAL | B | 180 | 7.793 | -10.353 | 5.142 | 1.00 | 16.83 |
| ATOM | 3015 | CG2 | VAL | B | 180 | 6.160 | -11.164 | 6.866 | 1.00 | 19.20 |
| ATOM | 3016 | C | VAL | B | 180 | 9.224 | -8.850 | 6.975 | 1.00 | 19.84 |
| ATOM | 3017 | O | VAL | B | 180 | 9.030 | -7.633 | 6.874 | 1.00 | 21.10 |
| ATOM | 3018 | N | VAL | B | 181 | 10.352 | -9.449 | 6.606 | 1.00 | 19.06 |
| ATOM | 3019 | CA | VAL | B | 181 | 11.455 | -8.742 | 5.996 | 1.00 | 17.28 |
| ATOM | 3020 | CB | VAL | B | 181 | 12.805 | -9.194 | 6.577 | 1.00 | 18.16 |
| ATOM | 3021 | CG1 | VAL | B | 181 | 13.950 | -8.566 | 5.799 | 1.00 | 16.75 |
| ATOM | 3022 | CG2 | VAL | B | 181 | 12.885 | -8.820 | 8.043 | 1.00 | 15.31 |
| ATOM | 3023 | C | VAL | B | 181 | 11.392 | -9.137 | 4.539 | 1.00 | 19.01 |
| ATOM | 3024 | O | VAL | B | 181 | 11.581 | -10.303 | 4.210 | 1.00 | 18.46 |
| ATOM | 3025 | N | ALA | B | 182 | 11.122 | -8.174 | 3.664 | 1.00 | 17.71 |
| ATOM | 3026 | CA | ALA | B | 182 | 11.027 | -8.466 | 2.247 | 1.00 | 17.68 |
| ATOM | 3027 | CB | ALA | B | 182 | 9.809 | -7.766 | 1.644 | 1.00 | 13.26 |
| ATOM | 3028 | C | ALA | B | 182 | 12.287 | -8.041 | 1.513 | 1.00 | 18.39 |
| ATOM | 3029 | O | ALA | B | 182 | 12.360 | -6.941 | 0.961 | 1.00 | 17.64 |

Figure 3 (suite 41)

| | | | | | | | | | | | |
|---|---|---|---|---|---|---|---|---|---|---|---|
| ATOM | 3030 | N | PRO | B | 183 | 13.298 | -8.921 | 1.476 | 1.00 | 20.26 |
| ATOM | 3031 | CD | PRO | B | 183 | 13.457 | -10.219 | 2.152 | 1.00 | 20.15 |
| ATOM | 3032 | CA | PRO | B | 183 | 14.525 | -8.550 | 0.779 | 1.00 | 20.79 |
| ATOM | 3033 | CB | PRO | B | 183 | 15.524 | -9.595 | 1.264 | 1.00 | 20.22 |
| ATOM | 3034 | CG | PRO | B | 183 | 14.681 | -10.780 | 1.460 | 1.00 | 21.75 |
| ATOM | 3035 | C | PRO | B | 183 | 14.327 | -8.601 | -0.709 | 1.00 | 22.87 |
| ATOM | 3036 | O | PRO | B | 183 | 13.404 | -9.252 | -1.196 | 1.00 | 21.23 |
| ATOM | 3037 | N | GLY | B | 184 | 15.197 | -7.898 | -1.421 | 1.00 | 23.74 |
| ATOM | 3038 | CA | GLY | B | 184 | 15.140 | -7.876 | -2.863 | 1.00 | 27.03 |
| ATOM | 3039 | C | GLY | B | 184 | 16.326 | -8.678 | -3.342 | 1.00 | 28.66 |
| ATOM | 3040 | O | GLY | B | 184 | 16.663 | -9.696 | -2.744 | 1.00 | 27.26 |
| ATOM | 3041 | N | TYR | B | 185 | 16.978 | -8.229 | -4.404 | 1.00 | 30.59 |
| ATOM | 3042 | CA | TYR | B | 185 | 18.129 | -8.959 | -4.907 | 1.00 | 32.81 |
| ATOM | 3043 | CB | TYR | B | 185 | 18.453 | -8.509 | -6.333 | 1.00 | 36.11 |
| ATOM | 3044 | CG | TYR | B | 185 | 17.514 | -9.118 | -7.357 | 1.00 | 40.44 |
| ATOM | 3045 | CD1 | TYR | B | 185 | 17.218 | -8.461 | -8.546 | 1.00 | 42.60 |
| ATOM | 3046 | CE1 | TYR | B | 185 | 16.348 | -9.026 | -9.486 | 1.00 | 45.24 |
| ATOM | 3047 | CD2 | TYR | B | 185 | 16.920 | -10.364 | -7.129 | 1.00 | 42.48 |
| ATOM | 3048 | CE2 | TYR | B | 185 | 16.052 | -10.938 | -8.059 | 1.00 | 44.35 |
| ATOM | 3049 | CZ | TYR | B | 185 | 15.772 | -10.265 | -9.233 | 1.00 | 44.96 |
| ATOM | 3050 | OH | TYR | B | 185 | 14.922 | -10.842 | -10.151 | 1.00 | 46.18 |
| ATOM | 3051 | C | TYR | B | 185 | 19.330 | -8.800 | -3.988 | 1.00 | 33.01 |
| ATOM | 3052 | O | TYR | B | 185 | 19.856 | -7.695 | -3.805 | 1.00 | 32.04 |
| ATOM | 3053 | N | ILE | B | 186 | 19.729 | -9.922 | -3.387 | 1.00 | 31.71 |
| ATOM | 3054 | CA | ILE | B | 186 | 20.865 | -9.972 | -2.481 | 1.00 | 31.74 |
| ATOM | 3055 | CB | ILE | B | 186 | 20.435 | -10.457 | -1.087 | 1.00 | 29.44 |
| ATOM | 3056 | CG2 | ILE | B | 186 | 21.624 | -10.458 | -0.143 | 1.00 | 27.68 |
| ATOM | 3057 | CG1 | ILE | B | 186 | 19.315 | -9.561 | -0.560 | 1.00 | 27.76 |
| ATOM | 3058 | CD | ILE | B | 186 | 19.628 | -8.074 | -0.654 | 1.00 | 25.09 |
| ATOM | 3059 | C | ILE | B | 186 | 21.929 | -10.916 | -3.031 | 1.00 | 33.15 |
| ATOM | 3060 | O | ILE | B | 186 | 21.616 | -11.987 | -3.550 | 1.00 | 32.02 |
| ATOM | 3061 | N | ASP | B | 187 | 23.187 | -10.513 | -2.908 | 1.00 | 35.82 |
| ATOM | 3062 | CA | ASP | B | 187 | 24.300 | -11.312 | -3.403 | 1.00 | 40.52 |
| ATOM | 3063 | CB | ASP | B | 187 | 25.539 | -10.426 | -3.527 | 1.00 | 42.18 |
| ATOM | 3064 | CG | ASP | B | 187 | 26.613 | -11.047 | -4.388 | 1.00 | 44.53 |
| ATOM | 3065 | OD1 | ASP | B | 187 | 26.310 | -11.397 | -5.548 | 1.00 | 45.06 |
| ATOM | 3066 | OD2 | ASP | B | 187 | 27.760 | -11.177 | -3.911 | 1.00 | 46.12 |
| ATOM | 3067 | C | ASP | B | 187 | 24.611 | -12.524 | -2.513 | 1.00 | 42.04 |
| ATOM | 3068 | O | ASP | B | 187 | 24.899 | -12.379 | -1.326 | 1.00 | 42.04 |
| ATOM | 3069 | N | THR | B | 188 | 24.541 | -13.719 | -3.095 | 1.00 | 44.22 |
| ATOM | 3070 | CA | THR | B | 188 | 24.826 | -14.959 | -2.372 | 1.00 | 45.74 |
| ATOM | 3071 | CB | THR | B | 188 | 23.532 | -15.605 | -1.809 | 1.00 | 46.17 |
| ATOM | 3072 | OG1 | THR | B | 188 | 22.588 | -15.803 | -2.873 | 1.00 | 44.39 |
| ATOM | 3073 | CG2 | THR | B | 188 | 22.911 | -14.723 | -0.735 | 1.00 | 45.67 |
| ATOM | 3074 | C | THR | B | 188 | 25.516 | -15.968 | -3.295 | 1.00 | 47.35 |
| ATOM | 3075 | O | THR | B | 188 | 25.324 | -17.185 | -3.175 | 1.00 | 49.06 |
| ATOM | 3076 | N | ALA | B | 202 | 18.425 | -5.709 | -14.708 | 1.00 | 55.07 |
| ATOM | 3077 | CA | ALA | B | 202 | 17.936 | -6.099 | -13.389 | 1.00 | 54.43 |
| ATOM | 3078 | CB | ALA | B | 202 | 18.562 | -7.424 | -12.972 | 1.00 | 55.02 |
| ATOM | 3079 | C | ALA | B | 202 | 18.253 | -5.018 | -12.360 | 1.00 | 53.99 |
| ATOM | 3080 | O | ALA | B | 202 | 17.412 | -4.681 | -11.523 | 1.00 | 52.98 |
| ATOM | 3081 | N | LEU | B | 203 | 19.472 | -4.483 | -12.428 | 1.00 | 53.07 |
| ATOM | 3082 | CA | LEU | B | 203 | 19.909 | -3.425 | -11.520 | 1.00 | 51.60 |
| ATOM | 3083 | CB | LEU | B | 203 | 21.425 | -3.230 | -11.602 | 1.00 | 51.62 |
| ATOM | 3084 | CG | LEU | B | 203 | 22.341 | -4.086 | -10.727 | 1.00 | 50.74 |
| ATOM | 3085 | CD1 | LEU | B | 203 | 23.787 | -3.764 | -11.064 | 1.00 | 50.35 |
| ATOM | 3086 | CD2 | LEU | B | 203 | 22.063 | -3.814 | -9.257 | 1.00 | 49.19 |
| ATOM | 3087 | C | LEU | B | 203 | 19.223 | -2.112 | -11.870 | 1.00 | 51.68 |
| ATOM | 3088 | O | LEU | B | 203 | 19.116 | -1.212 | -11.037 | 1.00 | 51.88 |
| ATOM | 3089 | N | GLN | B | 204 | 18.772 | -2.005 | -13.114 | 1.00 | 50.78 |
| ATOM | 3090 | CA | GLN | B | 204 | 18.092 | -0.808 | -13.580 | 1.00 | 51.24 |
| ATOM | 3091 | CB | GLN | B | 204 | 17.771 | -0.937 | -15.072 | 1.00 | 52.56 |
| ATOM | 3092 | CG | GLN | B | 204 | 18.983 | -1.260 | -15.934 | 0.00 | 52.15 |
| ATOM | 3093 | CD | GLN | B | 204 | 18.629 | -1.479 | -17.393 | 0.00 | 52.29 |
| ATOM | 3094 | OE1 | GLN | B | 204 | 19.493 | -1.798 | -18.210 | 0.00 | 52.25 |
| ATOM | 3095 | NE2 | GLN | B | 204 | 17.355 | -1.308 | -17.727 | 0.00 | 52.25 |
| ATOM | 3096 | C | GLN | B | 204 | 16.801 | -0.613 | -12.791 | 1.00 | 50.33 |
| ATOM | 3097 | O | GLN | B | 204 | 16.299 | 0.507 | -12.677 | 1.00 | 51.45 |
| ATOM | 3098 | N | PHE | B | 205 | 16.274 | -1.710 | -12.249 | 1.00 | 47.81 |
| ATOM | 3099 | CA | PHE | B | 205 | 15.040 | -1.679 | -11.467 | 1.00 | 45.50 |
| ATOM | 3100 | CB | PHE | B | 205 | 14.317 | -3.028 | -11.557 | 1.00 | 45.59 |
| ATOM | 3101 | CG | PHE | B | 205 | 13.786 | -3.347 | -12.923 | 0.00 | 45.66 |
| ATOM | 3102 | CD1 | PHE | B | 205 | 14.650 | -3.584 | -13.986 | 0.00 | 45.70 |

Figure 3 (suite 42)

| | | | | | | | | | | |
|---|---|---|---|---|---|---|---|---|---|---|
| ATOM | 3103 | CD2 | PHE | B | 205 | 12.415 | -3.415 | -13.146 | 0.00 | 45.70 |
| ATOM | 3104 | CE1 | PHE | B | 205 | 14.156 | -3.886 | -15.253 | 0.00 | 45.72 |
| ATOM | 3105 | CE2 | PHE | B | 205 | 11.910 | -3.716 | -14.408 | 0.00 | 45.72 |
| ATOM | 3106 | CZ | PHE | B | 205 | 12.783 | -3.952 | -15.464 | 0.00 | 45.72 |
| ATOM | 3107 | C | PHE | B | 205 | 15.289 | -1.348 | -9.998 | 1.00 | 43.46 |
| ATOM | 3108 | O | PHE | B | 205 | 14.352 | -1.203 | -9.215 | 1.00 | 42.75 |
| ATOM | 3109 | N | ILE | B | 206 | 16.555 | -1.238 | -9.625 | 1.00 | 41.65 |
| ATOM | 3110 | CA | ILE | B | 206 | 16.904 | -0.925 | -8.248 | 1.00 | 39.49 |
| ATOM | 3111 | CB | ILE | B | 206 | 18.011 | -1.860 | -7.732 | 1.00 | 39.17 |
| ATOM | 3112 | CG2 | ILE | B | 206 | 18.288 | -1.576 | -6.259 | 1.00 | 39.39 |
| ATOM | 3113 | CG1 | ILE | B | 206 | 17.582 | -3.317 | -7.906 | 1.00 | 38.15 |
| ATOM | 3114 | CD | ILE | B | 206 | 18.687 | -4.310 | -7.614 | 1.00 | 38.00 |
| ATOM | 3115 | C | ILE | B | 206 | 17.396 | 0.512 | -8.158 | 1.00 | 37.14 |
| ATOM | 3116 | O | ILE | B | 206 | 18.422 | 0.861 | -8.734 | 1.00 | 38.05 |
| ATOM | 3117 | N | PRO | B | 207 | 16.660 | 1.373 | -7.444 | 1.00 | 34.93 |
| ATOM | 3118 | CD | PRO | B | 207 | 15.338 | 1.196 | -6.822 | 1.00 | 34.69 |
| ATOM | 3119 | CA | PRO | B | 207 | 17.103 | 2.762 | -7.334 | 1.00 | 33.99 |
| ATOM | 3120 | CB | PRO | B | 207 | 16.131 | 3.345 | -6.312 | 1.00 | 32.98 |
| ATOM | 3121 | CG | PRO | B | 207 | 14.870 | 2.630 | -6.645 | 1.00 | 34.52 |
| ATOM | 3122 | C | PRO | B | 207 | 18.565 | 2.876 | -6.902 | 1.00 | 33.32 |
| ATOM | 3123 | O | PRO | B | 207 | 19.364 | 3.518 | -7.575 | 1.00 | 33.54 |
| ATOM | 3124 | N | ALA | B | 208 | 18.909 | 2.237 | -5.787 | 1.00 | 33.82 |
| ATOM | 3125 | CA | ALA | B | 208 | 20.272 | 2.279 | -5.263 | 1.00 | 33.11 |
| ATOM | 3126 | CB | ALA | B | 208 | 20.352 | 1.485 | -3.973 | 1.00 | 32.44 |
| ATOM | 3127 | C | ALA | B | 208 | 21.330 | 1.782 | -6.249 | 1.00 | 32.76 |
| ATOM | 3128 | O | ALA | B | 208 | 22.523 | 1.800 | -5.945 | 1.00 | 31.93 |
| ATOM | 3129 | N | LYS | B | 209 | 20.891 | 1.335 | -7.423 | 1.00 | 32.52 |
| ATOM | 3130 | CA | LYS | B | 209 | 21.795 | 0.850 | -8.469 | 1.00 | 32.79 |
| ATOM | 3131 | CB | LYS | B | 209 | 22.507 | 2.033 | -9.129 | 1.00 | 32.96 |
| ATOM | 3132 | CG | LYS | B | 209 | 21.549 | 3.058 | -9.737 | 1.00 | 36.13 |
| ATOM | 3133 | CD | LYS | B | 209 | 20.566 | 2.392 | -10.691 | 1.00 | 34.43 |
| ATOM | 3134 | CE | LYS | B | 209 | 19.446 | 3.339 | -11.085 | 1.00 | 35.82 |
| ATOM | 3135 | NZ | LYS | B | 209 | 18.322 | 2.596 | -11.741 | 1.00 | 34.69 |
| ATOM | 3136 | C | LYS | B | 209 | 22.838 | -0.175 | -8.012 | 1.00 | 32.31 |
| ATOM | 3137 | O | LYS | B | 209 | 23.979 | -0.163 | -8.479 | 1.00 | 30.35 |
| ATOM | 3138 | N | ARG | B | 210 | 22.444 | -1.058 | -7.102 | 1.00 | 32.09 |
| ATOM | 3139 | CA | ARG | B | 210 | 23.336 | -2.096 | -6.610 | 1.00 | 31.59 |
| ATOM | 3140 | CB | ARG | B | 210 | 24.396 | -1.509 | -5.676 | 1.00 | 33.03 |
| ATOM | 3141 | CG | ARG | B | 210 | 23.883 | -1.106 | -4.300 | 1.00 | 34.43 |
| ATOM | 3142 | CD | ARG | B | 210 | 25.021 | -0.577 | -3.443 | 1.00 | 32.95 |
| ATOM | 3143 | NE | ARG | B | 210 | 24.576 | -0.201 | -2.104 | 1.00 | 32.05 |
| ATOM | 3144 | CZ | ARG | B | 210 | 24.130 | -1.059 | -1.192 | 1.00 | 30.52 |
| ATOM | 3145 | NH1 | ARG | B | 210 | 24.067 | -2.358 | -1.472 | 1.00 | 27.64 |
| ATOM | 3146 | NH2 | ARG | B | 210 | 23.756 | -0.621 | 0.001 | 1.00 | 28.08 |
| ATOM | 3147 | C | ARG | B | 210 | 22.534 | -3.146 | -5.863 | 1.00 | 31.22 |
| ATOM | 3148 | O | ARG | B | 210 | 21.460 | -2.858 | -5.330 | 1.00 | 31.87 |
| ATOM | 3149 | N | VAL | B | 211 | 23.055 | -4.367 | -5.841 | 1.00 | 30.35 |
| ATOM | 3150 | CA | VAL | B | 211 | 22.404 | -5.462 | -5.138 | 1.00 | 29.82 |
| ATOM | 3151 | CB | VAL | B | 211 | 22.914 | -6.836 | -5.639 | 1.00 | 32.02 |
| ATOM | 3152 | CG1 | VAL | B | 211 | 22.720 | -6.943 | -7.139 | 1.00 | 32.20 |
| ATOM | 3153 | CG2 | VAL | B | 211 | 24.386 | -7.008 | -5.283 | 1.00 | 29.50 |
| ATOM | 3154 | C | VAL | B | 211 | 22.742 | -5.324 | -3.658 | 1.00 | 29.10 |
| ATOM | 3155 | O | VAL | B | 211 | 23.725 | -4.676 | -3.296 | 1.00 | 27.89 |
| ATOM | 3156 | N | GLY | B | 212 | 21.925 | -5.924 | -2.805 | 1.00 | 27.61 |
| ATOM | 3157 | CA | GLY | B | 212 | 22.173 | -5.842 | -1.380 | 1.00 | 26.92 |
| ATOM | 3158 | C | GLY | B | 212 | 23.021 | -7.003 | -0.891 | 1.00 | 26.64 |
| ATOM | 3159 | O | GLY | B | 212 | 23.198 | -7.995 | -1.599 | 1.00 | 26.31 |
| ATOM | 3160 | N | THR | B | 213 | 23.546 | -6.879 | 0.323 | 1.00 | 25.77 |
| ATOM | 3161 | CA | THR | B | 213 | 24.381 | -7.916 | 0.906 | 1.00 | 24.61 |
| ATOM | 3162 | CB | THR | B | 213 | 25.679 | -7.333 | 1.509 | 1.00 | 24.52 |
| ATOM | 3163 | OG1 | THR | B | 213 | 25.375 | -6.605 | 2.707 | 1.00 | 25.43 |
| ATOM | 3164 | CG2 | THR | B | 213 | 26.348 | -6.418 | 0.523 | 1.00 | 25.97 |
| ATOM | 3165 | C | THR | B | 213 | 23.630 | -8.611 | 2.023 | 1.00 | 23.53 |
| ATOM | 3166 | O | THR | B | 213 | 22.662 | -8.074 | 2.553 | 1.00 | 21.69 |
| ATOM | 3167 | N | PRO | B | 214 | 24.061 | -9.834 | 2.380 | 1.00 | 24.32 |
| ATOM | 3168 | CD | PRO | B | 214 | 25.006 | -10.653 | 1.594 | 1.00 | 22.90 |
| ATOM | 3169 | CA | PRO | B | 214 | 23.447 | -10.630 | 3.448 | 1.00 | 22.29 |
| ATOM | 3170 | CB | PRO | B | 214 | 24.300 | -11.896 | 3.454 | 1.00 | 22.47 |
| ATOM | 3171 | CG | PRO | B | 214 | 24.633 | -12.068 | 2.003 | 1.00 | 21.88 |
| ATOM | 3172 | C | PRO | B | 214 | 23.478 | -9.893 | 4.790 | 1.00 | 20.77 |
| ATOM | 3173 | O | PRO | B | 214 | 22.548 | -9.983 | 5.582 | 1.00 | 21.07 |
| ATOM | 3174 | N | ALA | B | 215 | 24.547 | -9.150 | 5.036 | 1.00 | 21.86 |
| ATOM | 3175 | CA | ALA | B | 215 | 24.679 | -8.405 | 6.282 | 1.00 | 22.57 |

Figure 3 (suite 43)

| | | | | | | | | | | |
|---|---|---|---|---|---|---|---|---|---|---|
| ATOM | 3176 | CB | ALA | B | 215 | 26.088 | -7.843 | 6.398 | 1.00 | 21.01 |
| ATOM | 3177 | C | ALA | B | 215 | 23.647 | -7.271 | 6.360 | 1.00 | 24.31 |
| ATOM | 3178 | O | ALA | B | 215 | 23.260 | -6.846 | 7.452 | 1.00 | 25.54 |
| ATOM | 3179 | N | GLU | B | 216 | 23.208 | -6.780 | 5.204 | 1.00 | 23.45 |
| ATOM | 3180 | CA | GLU | B | 216 | 22.214 | -5.719 | 5.168 | 1.00 | 23.01 |
| ATOM | 3181 | CB | GLU | B | 216 | 22.141 | -5.110 | 3.768 | 1.00 | 23.62 |
| ATOM | 3182 | CG | GLU | B | 216 | 23.405 | -4.341 | 3.419 | 1.00 | 26.44 |
| ATOM | 3183 | CD | GLU | B | 216 | 23.329 | -3.640 | 2.082 | 1.00 | 27.62 |
| ATOM | 3184 | OE1 | GLU | B | 216 | 23.006 | -4.314 | 1.080 | 1.00 | 25.54 |
| ATOM | 3185 | OE2 | GLU | B | 216 | 23.599 | -2.415 | 2.037 | 1.00 | 28.06 |
| ATOM | 3186 | C | GLU | B | 216 | 20.864 | -6.255 | 5.612 | 1.00 | 22.14 |
| ATOM | 3187 | O | GLU | B | 216 | 20.110 | -5.565 | 6.292 | 1.00 | 23.52 |
| ATOM | 3188 | N | VAL | B | 217 | 20.563 | -7.493 | 5.235 | 1.00 | 22.53 |
| ATOM | 3189 | CA | VAL | B | 217 | 19.314 | -8.130 | 5.649 | 1.00 | 22.41 |
| ATOM | 3190 | CB | VAL | B | 217 | 19.086 | -9.466 | 4.899 | 1.00 | 22.53 |
| ATOM | 3191 | CG1 | VAL | B | 217 | 17.918 | -10.223 | 5.519 | 1.00 | 18.40 |
| ATOM | 3192 | CG2 | VAL | B | 217 | 18.849 | -9.206 | 3.422 | 1.00 | 21.28 |
| ATOM | 3193 | C | VAL | B | 217 | 19.431 | -8.451 | 7.136 | 1.00 | 22.60 |
| ATOM | 3194 | O | VAL | B | 217 | 18.539 | -8.153 | 7.931 | 1.00 | 23.87 |
| ATOM | 3195 | N | ALA | B | 218 | 20.549 | -9.067 | 7.501 | 1.00 | 23.47 |
| ATOM | 3196 | CA | ALA | B | 218 | 20.800 | -9.459 | 8.883 | 1.00 | 24.19 |
| ATOM | 3197 | CB | ALA | B | 218 | 22.209 | -10.033 | 9.012 | 1.00 | 24.34 |
| ATOM | 3198 | C | ALA | B | 218 | 20.609 | -8.317 | 9.870 | 1.00 | 23.87 |
| ATOM | 3199 | O | ALA | B | 218 | 20.091 | -8.516 | 10.976 | 1.00 | 23.91 |
| ATOM | 3200 | N | GLY | B | 219 | 21.026 | -7.120 | 9.467 | 1.00 | 22.61 |
| ATOM | 3201 | CA | GLY | B | 219 | 20.891 | -5.967 | 10.340 | 1.00 | 21.46 |
| ATOM | 3202 | C | GLY | B | 219 | 19.445 | -5.678 | 10.660 | 1.00 | 21.35 |
| ATOM | 3203 | O | GLY | B | 219 | 19.098 | -5.362 | 11.794 | 1.00 | 23.23 |
| ATOM | 3204 | N | VAL | B | 220 | 18.591 | -5.795 | 9.654 | 1.00 | 19.84 |
| ATOM | 3205 | CA | VAL | B | 220 | 17.169 | -5.539 | 9.828 | 1.00 | 18.45 |
| ATOM | 3206 | CB | VAL | B | 220 | 16.444 | -5.623 | 8.480 | 1.00 | 17.61 |
| ATOM | 3207 | CG1 | VAL | B | 220 | 15.044 | -5.027 | 8.596 | 1.00 | 16.70 |
| ATOM | 3208 | CG2 | VAL | B | 220 | 17.251 | -4.898 | 7.420 | 1.00 | 18.60 |
| ATOM | 3209 | C | VAL | B | 220 | 16.589 | -6.562 | 10.796 | 1.00 | 18.52 |
| ATOM | 3210 | O | VAL | B | 220 | 15.886 | -6.202 | 11.746 | 1.00 | 18.82 |
| ATOM | 3211 | N | VAL | B | 221 | 16.890 | -7.840 | 10.561 | 1.00 | 19.89 |
| ATOM | 3212 | CA | VAL | B | 221 | 16.410 | -8.908 | 11.443 | 1.00 | 18.87 |
| ATOM | 3213 | CB | VAL | B | 221 | 16.967 | -10.295 | 11.026 | 1.00 | 20.40 |
| ATOM | 3214 | CG1 | VAL | B | 221 | 16.463 | -11.363 | 11.994 | 1.00 | 21.04 |
| ATOM | 3215 | CG2 | VAL | B | 221 | 16.548 | -10.626 | 9.599 | 1.00 | 20.78 |
| ATOM | 3216 | C | VAL | B | 221 | 16.888 | -8.585 | 12.850 | 1.00 | 19.07 |
| ATOM | 3217 | O | VAL | B | 221 | 16.117 | -8.653 | 13.810 | 1.00 | 19.23 |
| ATOM | 3218 | N | SER | B | 222 | 18.167 | -8.228 | 12.966 | 1.00 | 17.97 |
| ATOM | 3219 | CA | SER | B | 222 | 18.751 | -7.855 | 14.252 | 1.00 | 17.54 |
| ATOM | 3220 | CB | SER | B | 222 | 20.166 | -7.305 | 14.053 | 1.00 | 18.08 |
| ATOM | 3221 | OG | SER | B | 222 | 20.703 | -6.883 | 15.292 | 1.00 | 18.17 |
| ATOM | 3222 | C | SER | B | 222 | 17.879 | -6.778 | 14.894 | 1.00 | 19.17 |
| ATOM | 3223 | O | SER | B | 222 | 17.515 | -6.864 | 16.077 | 1.00 | 18.33 |
| ATOM | 3224 | N | PHE | B | 223 | 17.556 | -5.745 | 14.114 | 1.00 | 17.40 |
| ATOM | 3225 | CA | PHE | B | 223 | 16.705 | -4.694 | 14.632 | 1.00 | 16.66 |
| ATOM | 3226 | CB | PHE | B | 223 | 16.424 | -3.625 | 13.564 | 1.00 | 18.64 |
| ATOM | 3227 | CG | PHE | B | 223 | 15.216 | -2.780 | 13.868 | 1.00 | 19.54 |
| ATOM | 3228 | CD1 | PHE | B | 223 | 15.126 | -2.084 | 15.065 | 1.00 | 18.69 |
| ATOM | 3229 | CD2 | PHE | B | 223 | 14.138 | -2.740 | 12.985 | 1.00 | 21.07 |
| ATOM | 3230 | CE1 | PHE | B | 223 | 13.984 | -1.363 | 15.386 | 1.00 | 18.36 |
| ATOM | 3231 | CE2 | PHE | B | 223 | 12.984 | -2.019 | 13.295 | 1.00 | 21.20 |
| ATOM | 3232 | CZ | PHE | B | 223 | 12.907 | -1.332 | 14.496 | 1.00 | 21.47 |
| ATOM | 3233 | C | PHE | B | 223 | 15.390 | -5.320 | 15.082 | 1.00 | 16.54 |
| ATOM | 3234 | O | PHE | B | 223 | 14.965 | -5.139 | 16.221 | 1.00 | 17.24 |
| ATOM | 3235 | N | LEU | B | 224 | 14.750 | -6.063 | 14.183 | 1.00 | 15.68 |
| ATOM | 3236 | CA | LEU | B | 224 | 13.466 | -6.695 | 14.502 | 1.00 | 16.78 |
| ATOM | 3237 | CB | LEU | B | 224 | 12.890 | -7.388 | 13.258 | 1.00 | 16.35 |
| ATOM | 3238 | CG | LEU | B | 224 | 12.282 | -6.446 | 12.199 | 1.00 | 16.26 |
| ATOM | 3239 | CD1 | LEU | B | 224 | 11.712 | -7.245 | 11.048 | 1.00 | 11.66 |
| ATOM | 3240 | CD2 | LEU | B | 224 | 11.176 | -5.608 | 12.839 | 1.00 | 15.77 |
| ATOM | 3241 | C | LEU | B | 224 | 13.485 | -7.649 | 15.693 | 1.00 | 18.23 |
| ATOM | 3242 | O | LEU | B | 224 | 12.460 | -7.846 | 16.340 | 1.00 | 17.08 |
| ATOM | 3243 | N | ALA | B | 225 | 14.646 | -8.226 | 16.002 | 1.00 | 21.38 |
| ATOM | 3244 | CA | ALA | B | 225 | 14.753 | -9.129 | 17.153 | 1.00 | 23.58 |
| ATOM | 3245 | CB | ALA | B | 225 | 15.792 | -10.211 | 16.870 | 1.00 | 21.10 |
| ATOM | 3246 | C | ALA | B | 225 | 15.117 | -8.392 | 18.452 | 1.00 | 26.02 |
| ATOM | 3247 | O | ALA | B | 225 | 15.083 | -8.974 | 19.548 | 1.00 | 26.28 |
| ATOM | 3248 | N | SER | B | 226 | 15.458 | -7.112 | 18.336 | 1.00 | 27.12 |

Figure 3 (suite 44)

| | | | | | | | | | | |
|---|---|---|---|---|---|---|---|---|---|---|
| ATOM | 3249 | CA | SER | B | 226 | 15.856 | -6.323 | 19.499 | 1.00 | 27.11 |
| ATOM | 3250 | CB | SER | B | 226 | 16.760 | -5.166 | 19.057 | 1.00 | 28.10 |
| ATOM | 3251 | OG | SER | B | 226 | 16.027 | -4.211 | 18.306 | 1.00 | 27.36 |
| ATOM | 3252 | C | SER | B | 226 | 14.695 | -5.759 | 20.296 | 1.00 | 27.29 |
| ATOM | 3253 | O | SER | B | 226 | 13.536 | -5.915 | 19.929 | 1.00 | 26.55 |
| ATOM | 3254 | N | GLU | B | 227 | 15.036 | -5.087 | 21.393 | 1.00 | 27.13 |
| ATOM | 3255 | CA | GLU | B | 227 | 14.061 | -4.457 | 22.275 | 1.00 | 26.18 |
| ATOM | 3256 | CB | GLU | B | 227 | 14.729 | -4.118 | 23.609 | 1.00 | 25.23 |
| ATOM | 3257 | CG | GLU | B | 227 | 15.286 | -5.326 | 24.331 | 1.00 | 25.01 |
| ATOM | 3258 | CD | GLU | B | 227 | 14.208 | -6.119 | 25.033 | 1.00 | 26.77 |
| ATOM | 3259 | OE1 | GLU | B | 227 | 13.736 | -5.674 | 26.100 | 1.00 | 28.38 |
| ATOM | 3260 | OE2 | GLU | B | 227 | 13.817 | -7.176 | 24.508 | 1.00 | 26.16 |
| ATOM | 3261 | C | GLU | B | 227 | 13.430 | -3.185 | 21.680 | 1.00 | 26.21 |
| ATOM | 3262 | O | GLU | B | 227 | 12.423 | -2.703 | 22.195 | 1.00 | 28.75 |
| ATOM | 3263 | N | ASP | B | 228 | 14.013 | -2.657 | 20.603 | 1.00 | 25.18 |
| ATOM | 3264 | CA | ASP | B | 228 | 13.510 | -1.443 | 19.944 | 1.00 | 24.68 |
| ATOM | 3265 | CB | ASP | B | 228 | 14.628 | -0.769 | 19.118 | 1.00 | 23.08 |
| ATOM | 3266 | CG | ASP | B | 228 | 15.621 | 0.001 | 19.966 | 1.00 | 24.24 |
| ATOM | 3267 | OD1 | ASP | B | 228 | 16.762 | 0.206 | 19.496 | 1.00 | 27.32 |
| ATOM | 3268 | OD2 | ASP | B | 228 | 15.267 | 0.416 | 21.085 | 1.00 | 23.01 |
| ATOM | 3269 | C | ASP | B | 228 | 12.342 | -1.716 | 18.994 | 1.00 | 25.75 |
| ATOM | 3270 | O | ASP | B | 228 | 11.727 | -0.786 | 18.471 | 1.00 | 26.91 |
| ATOM | 3271 | N | ALA | B | 229 | 12.027 | -2.984 | 18.767 | 1.00 | 25.73 |
| ATOM | 3272 | CA | ALA | B | 229 | 10.963 | -3.319 | 17.826 | 1.00 | 24.68 |
| ATOM | 3273 | CB | ALA | B | 229 | 11.466 | -4.411 | 16.863 | 1.00 | 24.05 |
| ATOM | 3274 | C | ALA | B | 229 | 9.640 | -3.745 | 18.434 | 1.00 | 22.67 |
| ATOM | 3275 | O | ALA | B | 229 | 8.764 | -4.209 | 17.714 | 1.00 | 21.73 |
| ATOM | 3276 | N | SER | B | 230 | 9.488 | -3.567 | 19.742 | 1.00 | 22.59 |
| ATOM | 3277 | CA | SER | B | 230 | 8.274 | -3.980 | 20.450 | 1.00 | 21.56 |
| ATOM | 3278 | CB | SER | B | 230 | 8.375 | -3.600 | 21.931 | 1.00 | 22.28 |
| ATOM | 3279 | OG | SER | B | 230 | 8.578 | -2.199 | 22.112 | 1.00 | 26.26 |
| ATOM | 3280 | C | SER | B | 230 | 6.933 | -3.483 | 19.898 | 1.00 | 21.27 |
| ATOM | 3281 | O | SER | B | 230 | 5.924 | -4.198 | 19.982 | 1.00 | 21.97 |
| ATOM | 3282 | N | TYR | B | 231 | 6.904 | -2.276 | 19.334 | 1.00 | 19.91 |
| ATOM | 3283 | CA | TYR | B | 231 | 5.647 | -1.751 | 18.813 | 1.00 | 19.60 |
| ATOM | 3284 | CB | TYR | B | 231 | 5.492 | -0.272 | 19.164 | 1.00 | 18.36 |
| ATOM | 3285 | CG | TYR | B | 231 | 4.045 | 0.119 | 19.381 | 1.00 | 18.93 |
| ATOM | 3286 | CD1 | TYR | B | 231 | 3.244 | -0.585 | 20.280 | 1.00 | 17.26 |
| ATOM | 3287 | CE1 | TYR | B | 231 | 1.922 | -0.229 | 20.495 | 1.00 | 17.90 |
| ATOM | 3288 | CD2 | TYR | B | 231 | 3.481 | 1.190 | 18.697 | 1.00 | 17.48 |
| ATOM | 3289 | CE2 | TYR | B | 231 | 2.161 | 1.555 | 18.903 | 1.00 | 17.53 |
| ATOM | 3290 | CZ | TYR | B | 231 | 1.387 | 0.843 | 19.806 | 1.00 | 18.33 |
| ATOM | 3291 | OH | TYR | B | 231 | 0.087 | 1.228 | 20.054 | 1.00 | 17.45 |
| ATOM | 3292 | C | TYR | B | 231 | 5.482 | -1.938 | 17.316 | 1.00 | 18.32 |
| ATOM | 3293 | O | TYR | B | 231 | 4.607 | -1.336 | 16.708 | 1.00 | 16.10 |
| ATOM | 3294 | N | ILE | B | 232 | 6.335 | -2.761 | 16.720 | 1.00 | 18.74 |
| ATOM | 3295 | CA | ILE | B | 232 | 6.241 | -3.034 | 15.298 | 1.00 | 17.63 |
| ATOM | 3296 | CB | ILE | B | 232 | 7.626 | -3.000 | 14.627 | 1.00 | 19.30 |
| ATOM | 3297 | CG2 | ILE | B | 232 | 7.481 | -3.215 | 13.119 | 1.00 | 18.64 |
| ATOM | 3298 | CG1 | ILE | B | 232 | 8.313 | -1.654 | 14.916 | 1.00 | 19.43 |
| ATOM | 3299 | CD | ILE | B | 232 | 9.558 | -1.409 | 14.097 | 1.00 | 17.98 |
| ATOM | 3300 | C | ILE | B | 232 | 5.633 | -4.438 | 15.153 | 1.00 | 16.67 |
| ATOM | 3301 | O | ILE | B | 232 | 6.217 | -5.423 | 15.605 | 1.00 | 13.29 |
| ATOM | 3302 | N | SER | B | 233 | 4.448 | -4.517 | 14.556 | 1.00 | 17.08 |
| ATOM | 3303 | CA | SER | B | 233 | 3.779 | -5.799 | 14.358 | 1.00 | 19.41 |
| ATOM | 3304 | CB | SER | B | 233 | 2.923 | -6.147 | 15.579 | 1.00 | 20.68 |
| ATOM | 3305 | OG | SER | B | 233 | 2.529 | -7.506 | 15.566 | 1.00 | 17.43 |
| ATOM | 3306 | C | SER | B | 233 | 2.904 | -5.711 | 13.123 | 1.00 | 19.48 |
| ATOM | 3307 | O | SER | B | 233 | 2.202 | -4.713 | 12.925 | 1.00 | 19.32 |
| ATOM | 3308 | N | GLY | B | 234 | 2.964 | -6.750 | 12.290 | 1.00 | 20.62 |
| ATOM | 3309 | CA | GLY | B | 234 | 2.172 | -6.795 | 11.072 | 1.00 | 18.59 |
| ATOM | 3310 | C | GLY | B | 234 | 2.894 | -6.122 | 9.934 | 1.00 | 17.69 |
| ATOM | 3311 | O | GLY | B | 234 | 2.413 | -6.092 | 8.804 | 1.00 | 18.72 |
| ATOM | 3312 | N | ALA | B | 235 | 4.084 | -5.605 | 10.232 | 1.00 | 18.08 |
| ATOM | 3313 | CA | ALA | B | 235 | 4.890 | -4.879 | 9.248 | 1.00 | 16.92 |
| ATOM | 3314 | CB | ALA | B | 235 | 5.913 | -4.008 | 9.967 | 1.00 | 17.62 |
| ATOM | 3315 | C | ALA | B | 235 | 5.606 | -5.729 | 8.217 | 1.00 | 18.76 |
| ATOM | 3316 | O | ALA | B | 235 | 5.969 | -6.882 | 8.466 | 1.00 | 17.73 |
| ATOM | 3317 | N | VAL | B | 236 | 5.799 | -5.131 | 7.046 | 1.00 | 17.88 |
| ATOM | 3318 | CA | VAL | B | 236 | 6.495 | -5.753 | 5.942 | 1.00 | 18.94 |
| ATOM | 3319 | CB | VAL | B | 236 | 5.607 | -5.900 | 4.692 | 1.00 | 20.55 |
| ATOM | 3320 | CG1 | VAL | B | 236 | 6.422 | -6.468 | 3.538 | 1.00 | 21.02 |
| ATOM | 3321 | CG2 | VAL | B | 236 | 4.457 | -6.817 | 4.989 | 1.00 | 21.71 |

Figure 3 (suite 45)

| | | | | | | | | | | |
|---|---|---|---|---|---|---|---|---|---|---|
| ATOM | 3322 | C | VAL | B | 236 | 7.623 | -4.800 | 5.618 | 1.00 | 19.27 |
| ATOM | 3323 | O | VAL | B | 236 | 7.435 | -3.837 | 4.880 | 1.00 | 22.92 |
| ATOM | 3324 | N | ILE | B | 237 | 8.800 | -5.073 | 6.168 | 1.00 | 19.56 |
| ATOM | 3325 | CA | ILE | B | 237 | 9.946 | -4.215 | 5.952 | 1.00 | 18.59 |
| ATOM | 3326 | CB | ILE | B | 237 | 10.805 | -4.158 | 7.221 | 1.00 | 19.39 |
| ATOM | 3327 | CG2 | ILE | B | 237 | 11.922 | -3.115 | 7.056 | 1.00 | 16.12 |
| ATOM | 3328 | CG1 | ILE | B | 237 | 9.897 | -3.834 | 8.418 | 1.00 | 19.02 |
| ATOM | 3329 | CD | ILE | B | 237 | 10.610 | -3.580 | 9.726 | 1.00 | 19.06 |
| ATOM | 3330 | C | ILE | B | 237 | 10.808 | -4.597 | 4.762 | 1.00 | 19.26 |
| ATOM | 3331 | O | ILE | B | 237 | 11.474 | -5.641 | 4.755 | 1.00 | 17.33 |
| ATOM | 3332 | N | PRO | B | 238 | 10.801 | -3.749 | 3.720 | 1.00 | 19.99 |
| ATOM | 3333 | CD | PRO | B | 238 | 9.933 | -2.571 | 3.538 | 1.00 | 19.07 |
| ATOM | 3334 | CA | PRO | B | 238 | 11.586 | -3.989 | 2.510 | 1.00 | 19.96 |
| ATOM | 3335 | CB | PRO | B | 238 | 10.888 | -3.114 | 1.473 | 1.00 | 17.49 |
| ATOM | 3336 | CG | PRO | B | 238 | 10.483 | -1.956 | 2.263 | 1.00 | 17.85 |
| ATOM | 3337 | C | PRO | B | 238 | 13.059 | -3.618 | 2.702 | 1.00 | 20.52 |
| ATOM | 3338 | O | PRO | B | 238 | 13.387 | -2.653 | 3.399 | 1.00 | 20.22 |
| ATOM | 3339 | N | VAL | B | 239 | 13.931 | -4.399 | 2.068 | 1.00 | 19.70 |
| ATOM | 3340 | CA | VAL | B | 239 | 15.378 | -4.224 | 2.134 | 1.00 | 19.66 |
| ATOM | 3341 | CB | VAL | B | 239 | 15.979 | -5.223 | 3.141 | 1.00 | 20.15 |
| ATOM | 3342 | CG1 | VAL | B | 239 | 17.457 | -5.014 | 3.276 | 1.00 | 19.79 |
| ATOM | 3343 | CG2 | VAL | B | 239 | 15.293 | -5.067 | 4.483 | 1.00 | 20.11 |
| ATOM | 3344 | C | VAL | B | 239 | 15.812 | -4.552 | 0.718 | 1.00 | 21.04 |
| ATOM | 3345 | O | VAL | B | 239 | 16.494 | -5.542 | 0.459 | 1.00 | 21.23 |
| ATOM | 3346 | N | ASP | B | 240 | 15.398 | -3.689 | -0.199 | 1.00 | 22.16 |
| ATOM | 3347 | CA | ASP | B | 240 | 15.634 | -3.869 | -1.619 | 1.00 | 21.86 |
| ATOM | 3348 | CB | ASP | B | 240 | 14.291 | -4.151 | -2.282 | 1.00 | 23.18 |
| ATOM | 3349 | CG | ASP | B | 240 | 13.224 | -3.154 | -1.846 | 1.00 | 23.07 |
| ATOM | 3350 | OD1 | ASP | B | 240 | 13.593 | -1.996 | -1.538 | 1.00 | 20.69 |
| ATOM | 3351 | OD2 | ASP | B | 240 | 12.030 | -3.514 | -1.802 | 1.00 | 25.58 |
| ATOM | 3352 | C | ASP | B | 240 | 16.256 | -2.675 | -2.323 | 1.00 | 22.91 |
| ATOM | 3353 | O | ASP | B | 240 | 16.202 | -2.598 | -3.546 | 1.00 | 23.20 |
| ATOM | 3354 | N | GLY | B | 241 | 16.841 | -1.742 | -1.586 | 1.00 | 23.92 |
| ATOM | 3355 | CA | GLY | B | 241 | 17.388 | -0.578 | -2.264 | 1.00 | 24.67 |
| ATOM | 3356 | C | GLY | B | 241 | 16.252 | 0.170 | -2.958 | 1.00 | 26.79 |
| ATOM | 3357 | O | GLY | B | 241 | 16.450 | 0.835 | -3.978 | 1.00 | 27.17 |
| ATOM | 3358 | N | GLY | B | 242 | 15.050 | 0.029 | -2.405 | 1.00 | 25.97 |
| ATOM | 3359 | CA | GLY | B | 242 | 13.871 | 0.687 | -2.942 | 1.00 | 26.14 |
| ATOM | 3360 | C | GLY | B | 242 | 13.305 | 0.114 | -4.223 | 1.00 | 27.13 |
| ATOM | 3361 | O | GLY | B | 242 | 12.618 | 0.822 | -4.954 | 1.00 | 29.00 |
| ATOM | 3362 | N | MET | B | 243 | 13.574 | -1.159 | -4.498 | 1.00 | 27.17 |
| ATOM | 3363 | CA | MET | B | 243 | 13.099 | -1.799 | -5.728 | 1.00 | 28.09 |
| ATOM | 3364 | CB | MET | B | 243 | 13.894 | -3.073 | -5.996 | 1.00 | 29.92 |
| ATOM | 3365 | CG | MET | B | 243 | 13.210 | -4.031 | -6.975 | 1.00 | 32.72 |
| ATOM | 3366 | SD | MET | B | 243 | 13.954 | -5.681 | -7.005 | 1.00 | 37.16 |
| ATOM | 3367 | CE | MET | B | 243 | 14.917 | -5.581 | -8.533 | 1.00 | 38.08 |
| ATOM | 3368 | C | MET | B | 243 | 11.615 | -2.151 | -5.815 | 1.00 | 27.91 |
| ATOM | 3369 | O | MET | B | 243 | 11.016 | -2.049 | -6.889 | 1.00 | 26.63 |
| ATOM | 3370 | N | GLY | B | 244 | 11.030 | -2.572 | -4.698 | 1.00 | 27.28 |
| ATOM | 3371 | CA | GLY | B | 244 | 9.632 | -2.973 | -4.709 | 1.00 | 26.06 |
| ATOM | 3372 | C | GLY | B | 244 | 8.538 | -1.949 | -4.450 | 1.00 | 25.90 |
| ATOM | 3373 | O | GLY | B | 244 | 7.392 | -2.337 | -4.198 | 1.00 | 23.49 |
| ATOM | 3374 | N | MET | B | 245 | 8.868 | -0.661 | -4.510 | 1.00 | 25.98 |
| ATOM | 3375 | CA | MET | B | 245 | 7.873 | 0.394 | -4.277 | 1.00 | 28.32 |
| ATOM | 3376 | CB | MET | B | 245 | 8.508 | 1.783 | -4.443 | 1.00 | 28.83 |
| ATOM | 3377 | CG | MET | B | 245 | 9.681 | 2.072 | -3.508 | 1.00 | 26.79 |
| ATOM | 3378 | SD | MET | B | 245 | 10.356 | 3.745 | -3.743 | 1.00 | 29.49 |
| ATOM | 3379 | CE | MET | B | 245 | 11.073 | 3.580 | -5.326 | 1.00 | 28.83 |
| ATOM | 3380 | C | MET | B | 245 | 6.690 | 0.262 | -5.243 | 1.00 | 29.05 |
| ATOM | 3381 | O | MET | B | 245 | 6.872 | 0.017 | -6.437 | 1.00 | 29.15 |
| ATOM | 3382 | N | GLY | B | 246 | 5.481 | 0.418 | -4.711 | 1.00 | 29.29 |
| ATOM | 3383 | CA | GLY | B | 246 | 4.282 | 0.320 | -5.525 | 1.00 | 29.76 |
| ATOM | 3384 | C | GLY | B | 246 | 3.055 | 0.153 | -4.652 | 1.00 | 29.73 |
| ATOM | 3385 | O | GLY | B | 246 | 3.065 | 0.556 | -3.487 | 1.00 | 28.95 |
| ATOM | 3386 | N | HIS | B | 247 | 1.997 | -0.439 | -5.206 | 1.00 | 28.72 |
| ATOM | 3387 | CA | HIS | B | 247 | 0.767 | -0.660 | -4.442 | 1.00 | 28.75 |
| ATOM | 3388 | CB | HIS | B | 247 | -0.106 | 0.612 | -4.445 | 1.00 | 28.29 |
| ATOM | 3389 | CG | HIS | B | 247 | -1.320 | 0.514 | -3.567 | 1.00 | 29.06 |
| ATOM | 3390 | CD2 | HIS | B | 247 | -1.445 | 0.501 | -2.218 | 1.00 | 29.15 |
| ATOM | 3391 | ND1 | HIS | B | 247 | -2.598 | 0.383 | -4.070 | 1.00 | 28.47 |
| ATOM | 3392 | CE1 | HIS | B | 247 | -3.457 | 0.294 | -3.071 | 1.00 | 26.88 |
| ATOM | 3393 | NE2 | HIS | B | 247 | -2.784 | 0.362 | -1.936 | 1.00 | 29.90 |
| ATOM | 3394 | C | HIS | B | 247 | -0.044 | -1.845 | -4.976 | 1.00 | 28.17 |

Figure 3 (suite 46)

| | | | | | | | | | | |
|---|---|---|---|---|---|---|---|---|---|---|
| ATOM | 3395 | OT1 | HIS | B | 247 | 0.143 | -2.201 | -6.154 | 1.00 | 28.14 |
| ATOM | 3396 | OXT | HIS | B | 247 | -0.862 | -2.396 | -4.205 | 1.00 | 27.00 |
| ATOM | 3397 | CS+1 | CS1 | C | 1 | 28.083 | 4.431 | 19.672 | 1.00 | 49.29 |
| ATOM | 3398 | CS+1 | CS1 | C | 2 | 26.723 | 12.390 | 21.828 | 0.50 | 34.54 |
| ATOM | 3399 | CS+1 | CS1 | C | 3 | 24.633 | -7.144 | 23.673 | 1.00 | 46.60 |
| ATOM | 3400 | CS+1 | CS1 | C | 4 | 26.719 | -15.154 | 21.813 | 0.50 | 37.95 |
| ATOM | 3401 | K+1 | K1 | D | 1 | 20.556 | 23.647 | 24.672 | 1.00 | 59.83 |
| ATOM | 3402 | OH2 | TIP | S | 2 | 24.805 | 5.788 | 10.005 | 1.00 | 22.95 |
| ATOM | 3403 | OH2 | TIP | S | 3 | 30.044 | 1.618 | 9.864 | 1.00 | 35.51 |
| ATOM | 3404 | OH2 | TIP | S | 4 | 9.901 | 18.911 | 9.218 | 1.00 | 21.80 |
| ATOM | 3405 | OH2 | TIP | S | 5 | 12.899 | 1.579 | 22.236 | 1.00 | 23.08 |
| ATOM | 3406 | OH2 | TIP | S | 6 | 21.556 | 5.548 | 15.485 | 1.00 | 33.59 |
| ATOM | 3407 | OH2 | TIP | S | 7 | 7.047 | 19.266 | 8.653 | 1.00 | 20.88 |
| ATOM | 3408 | OH2 | TIP | S | 8 | 12.256 | 25.585 | -0.244 | 1.00 | 30.52 |
| ATOM | 3409 | OH2 | TIP | S | 9 | 16.733 | 7.683 | 5.057 | 1.00 | 31.46 |
| ATOM | 3410 | OH2 | TIP | S | 10 | -0.444 | 24.686 | 2.311 | 1.00 | 23.34 |
| ATOM | 3411 | OH2 | TIP | S | 11 | -1.850 | 30.091 | 1.753 | 1.00 | 18.45 |
| ATOM | 3412 | OH2 | TIP | S | 12 | -1.849 | 24.839 | -0.422 | 1.00 | 31.87 |
| ATOM | 3413 | OH2 | TIP | S | 13 | 22.721 | 20.714 | 0.994 | 1.00 | 20.04 |
| ATOM | 3414 | OH2 | TIP | S | 14 | 25.330 | 27.355 | 4.101 | 1.00 | 29.92 |
| ATOM | 3415 | OH2 | TIP | S | 15 | 13.867 | -0.819 | 1.139 | 1.00 | 17.51 |
| ATOM | 3416 | OH2 | TIP | S | 16 | 3.780 | -1.965 | 13.134 | 1.00 | 21.92 |
| ATOM | 3417 | OH2 | TIP | S | 17 | 9.960 | -22.037 | 5.212 | 1.00 | 21.32 |
| ATOM | 3418 | OH2 | TIP | S | 18 | 2.010 | -28.633 | 11.953 | 1.00 | 19.93 |
| ATOM | 3419 | OH2 | TIP | S | 19 | 8.549 | -6.955 | 17.148 | 1.00 | 20.43 |
| ATOM | 3420 | OH2 | TIP | S | 20 | 8.423 | -10.277 | 15.282 | 1.00 | 28.34 |
| ATOM | 3421 | OH2 | TIP | S | 21 | 19.115 | -8.345 | 18.306 | 1.00 | 16.58 |
| ATOM | 3422 | OH2 | TIP | S | 22 | 5.454 | -5.545 | 22.191 | 1.00 | 30.07 |
| ATOM | 3423 | OH2 | TIP | S | 23 | 33.688 | -11.437 | 13.986 | 1.00 | 30.10 |
| ATOM | 3424 | OH2 | TIP | S | 24 | 31.262 | -12.869 | 8.660 | 1.00 | 35.66 |
| ATOM | 3425 | OH2 | TIP | S | 25 | 27.301 | -10.069 | 4.217 | 1.00 | 38.06 |
| ATOM | 3426 | OH2 | TIP | S | 26 | 26.597 | 13.769 | 3.679 | 1.00 | 21.20 |
| ATOM | 3427 | OH2 | TIP | S | 27 | -4.915 | 29.771 | -1.172 | 1.00 | 23.21 |
| ATOM | 3428 | OH2 | TIP | S | 28 | 18.244 | 4.328 | 5.326 | 1.00 | 38.36 |
| ATOM | 3429 | OH2 | TIP | S | 29 | 16.660 | 5.332 | 7.096 | 1.00 | 33.30 |
| ATOM | 3430 | OH2 | TIP | S | 30 | -1.073 | 2.608 | 17.566 | 1.00 | 17.95 |
| ATOM | 3431 | OH2 | TIP | S | 31 | 26.568 | 4.116 | 3.667 | 1.00 | 24.70 |
| ATOM | 3432 | OH2 | TIP | S | 32 | 18.387 | -2.627 | 20.792 | 1.00 | 34.99 |
| ATOM | 3433 | OH2 | TIP | S | 33 | 14.462 | -32.104 | 13.877 | 1.00 | 35.65 |
| ATOM | 3434 | OH2 | TIP | S | 34 | 11.089 | -21.774 | 7.873 | 1.00 | 22.95 |
| ATOM | 3435 | OH2 | TIP | S | 35 | 3.627 | -26.936 | 14.058 | 1.00 | 33.15 |
| ATOM | 3436 | OH2 | TIP | S | 36 | 4.837 | -18.850 | -12.497 | 1.00 | 24.06 |
| ATOM | 3437 | OH2 | TIP | S | 37 | 5.889 | -23.356 | 22.010 | 1.00 | 20.76 |
| ATOM | 3438 | OH2 | TIP | S | 38 | 10.550 | -5.833 | -1.267 | 1.00 | 26.47 |
| ATOM | 3439 | OH2 | TIP | S | 39 | 24.666 | -7.266 | 10.099 | 1.00 | 20.62 |
| ATOM | 3440 | OH2 | TIP | S | 40 | 14.762 | 10.910 | 28.648 | 1.00 | 28.52 |
| ATOM | 3441 | OH2 | TIP | S | 41 | 21.377 | 3.425 | -1.378 | 1.00 | 25.67 |
| ATOM | 3442 | OH2 | TIP | S | 42 | 16.347 | -19.789 | 1.470 | 1.00 | 32.94 |
| ATOM | 3443 | OH2 | TIP | S | 43 | 32.290 | 13.569 | 9.449 | 1.00 | 40.93 |
| ATOM | 3444 | OH2 | TIP | S | 44 | -8.057 | 21.918 | 16.075 | 1.00 | 38.21 |
| ATOM | 3445 | OH2 | TIP | S | 45 | 3.076 | 7.965 | 6.895 | 1.00 | 33.85 |
| ATOM | 3446 | OH2 | TIP | S | 46 | 26.702 | 0.589 | 13.711 | 1.00 | 53.05 |
| ATOM | 3447 | OH2 | TIP | S | 47 | 14.619 | 24.547 | 1.139 | 1.00 | 29.71 |
| ATOM | 3448 | OH2 | TIP | S | 48 | 22.657 | 18.086 | 3.974 | 1.00 | 29.21 |
| ATOM | 3449 | OH2 | TIP | S | 49 | 29.466 | 14.837 | 10.484 | 1.00 | 31.35 |
| ATOM | 3450 | OH2 | TIP | S | 50 | 5.077 | 29.487 | 3.615 | 1.00 | 18.35 |
| ATOM | 3451 | OH2 | TIP | S | 51 | 1.306 | 28.154 | 6.710 | 1.00 | 25.28 |
| ATOM | 3452 | OH2 | TIP | S | 52 | 19.160 | 1.563 | -0.157 | 1.00 | 28.11 |
| ATOM | 3453 | OH2 | TIP | S | 53 | -7.109 | -2.226 | 13.624 | 1.00 | 38.34 |
| ATOM | 3454 | OH2 | TIP | S | 54 | 2.867 | -6.187 | 21.162 | 1.00 | 28.75 |
| ATOM | 3455 | OH2 | TIP | S | 55 | 8.840 | -0.120 | 18.493 | 1.00 | 24.84 |
| ATOM | 3456 | OH2 | TIP | S | 56 | 7.118 | 0.111 | 2.708 | 1.00 | 24.92 |
| ATOM | 3457 | OH2 | TIP | S | 57 | -5.990 | -2.323 | 10.167 | 1.00 | 24.69 |
| ATOM | 3458 | OH2 | TIP | S | 58 | 15.254 | -4.530 | 28.157 | 1.00 | 33.32 |
| ATOM | 3459 | OH2 | TIP | S | 59 | 10.723 | -11.914 | 28.168 | 1.00 | 31.77 |
| ATOM | 3460 | OH2 | TIP | S | 60 | 22.986 | -21.844 | 21.498 | 1.00 | 33.79 |
| ATOM | 3461 | OH2 | TIP | S | 61 | 15.576 | -33.861 | 7.343 | 1.00 | 27.21 |
| ATOM | 3462 | OH2 | TIP | S | 62 | 3.968 | -33.461 | 8.965 | 1.00 | 36.19 |
| ATOM | 3463 | OH2 | TIP | S | 63 | 5.407 | -29.464 | 15.167 | 1.00 | 26.78 |
| ATOM | 3464 | OH2 | TIP | S | 64 | 9.392 | -18.929 | 24.140 | 1.00 | 21.74 |
| ATOM | 3465 | OH2 | TIP | S | 65 | 13.817 | -27.332 | 5.423 | 1.00 | 25.48 |
| ATOM | 3466 | OH2 | TIP | S | 66 | 9.826 | -20.540 | -6.924 | 1.00 | 28.13 |
| ATOM | 3467 | OH2 | TIP | S | 67 | 4.360 | -31.071 | -1.982 | 1.00 | 35.41 |

Figure 3 (suite 47)

| | | | | | | | | | | |
|---|---|---|---|---|---|---|---|---|---|---|
| ATOM | 3468 | OH2 | TIP | S | 68 | 7.093 | -30.975 | -0.410 | 1.00 | 30.31 |
| ATOM | 3469 | OH2 | TIP | S | 69 | 7.291 | -5.259 | -16.950 | 1.00 | 32.62 |
| ATOM | 3470 | OH2 | TIP | S | 70 | -3.311 | -9.291 | 19.508 | 1.00 | 24.81 |
| ATOM | 3471 | OH2 | TIP | S | 71 | -3.312 | -19.620 | 19.369 | 1.00 | 35.26 |
| ATOM | 3472 | OH2 | TIP | S | 72 | 12.346 | 12.372 | 26.196 | 1.00 | 46.73 |
| ATOM | 3473 | OH2 | TIP | S | 73 | 13.770 | -33.780 | 9.081 | 1.00 | 28.40 |
| ATOM | 3474 | OH2 | TIP | S | 74 | 9.580 | -36.798 | 14.882 | 1.00 | 33.27 |
| ATOM | 3475 | OH2 | TIP | S | 75 | 1.046 | -27.551 | 1.775 | 1.00 | 14.10 |
| ATOM | 3476 | OH2 | TIP | S | 76 | 8.736 | -21.027 | 21.505 | 1.00 | 22.75 |
| ATOM | 3477 | OH2 | TIP | S | 77 | 9.466 | -14.075 | 28.726 | 1.00 | 34.35 |
| ATOM | 3478 | OH2 | TIP | S | 78 | 13.570 | -13.670 | -1.952 | 1.00 | 35.26 |
| ATOM | 3479 | OH2 | TIP | S | 79 | 21.286 | -14.037 | -10.713 | 1.00 | 31.62 |
| ATOM | 3480 | OH2 | TIP | S | 80 | 11.879 | -1.294 | -9.108 | 1.00 | 49.45 |
| ATOM | 3481 | OH2 | TIP | S | 81 | 2.005 | -3.156 | 10.626 | 1.00 | 33.73 |
| ATOM | 3482 | OH2 | TIP | S | 82 | 3.886 | -2.801 | 6.331 | 1.00 | 7.68 |
| ATOM | 3483 | OH2 | TIP | S | 83 | 8.788 | 35.227 | 15.838 | 1.00 | 30.73 |
| ATOM | 3484 | OH2 | TIP | S | 84 | 11.283 | 31.152 | 12.613 | 1.00 | 34.15 |
| ATOM | 3485 | OH2 | TIP | S | 85 | 15.534 | 33.200 | 9.930 | 1.00 | 31.26 |
| ATOM | 3486 | OH2 | TIP | S | 86 | -11.267 | 15.834 | 7.217 | 1.00 | 27.45 |
| ATOM | 3487 | OH2 | TIP | S | 87 | 23.648 | 13.130 | -6.062 | 1.00 | 47.12 |
| ATOM | 3488 | OH2 | TIP | S | 88 | 30.541 | 10.675 | 3.832 | 1.00 | 49.90 |
| ATOM | 3489 | OH2 | TIP | S | 89 | 6.861 | 27.562 | 21.590 | 1.00 | 29.45 |
| ATOM | 3490 | OH2 | TIP | S | 90 | 20.294 | -4.360 | 16.329 | 1.00 | 16.67 |
| ATOM | 3491 | OH2 | TIP | S | 91 | 31.628 | 11.340 | 11.607 | 1.00 | 33.38 |
| ATOM | 3492 | OH2 | TIP | S | 92 | 7.571 | 19.113 | 18.010 | 1.00 | 38.94 |
| ATOM | 3493 | OH2 | TIP | S | 93 | 4.390 | 18.481 | 17.598 | 1.00 | 48.86 |
| ATOM | 3494 | OH2 | TIP | S | 94 | 7.014 | 22.256 | 15.165 | 1.00 | 28.79 |
| ATOM | 3495 | OH2 | TIP | S | 95 | 7.687 | 23.691 | 13.221 | 1.00 | 32.68 |
| ATOM | 3496 | OH2 | TIP | S | 96 | 4.770 | 14.526 | 19.412 | 1.00 | 32.98 |
| ATOM | 3497 | OH2 | TIP | S | 97 | 28.347 | 13.243 | 19.793 | 1.00 | 44.20 |
| ATOM | 3498 | OH2 | TIP | S | 98 | 7.819 | 16.200 | 8.374 | 1.00 | 30.78 |
| ATOM | 3499 | OH2 | TIP | S | 99 | 2.681 | 12.055 | 12.243 | 1.00 | 58.41 |
| ATOM | 3500 | OH2 | TIP | S | 100 | -2.323 | 10.915 | 14.249 | 1.00 | 32.86 |
| ATOM | 3501 | OH2 | TIP | S | 101 | 0.897 | 11.441 | 15.570 | 1.00 | 57.24 |
| ATOM | 3502 | OH2 | TIP | S | 102 | -12.793 | -11.386 | 14.731 | 1.00 | 37.36 |
| ATOM | 3503 | OH2 | TIP | S | 103 | 2.561 | 2.053 | 26.486 | 1.00 | 33.59 |
| ATOM | 3504 | OH2 | TIP | S | 104 | 9.321 | 7.166 | 25.631 | 1.00 | 35.63 |
| ATOM | 3505 | OH2 | TIP | S | 105 | 18.153 | 6.999 | 24.379 | 1.00 | 34.26 |
| ATOM | 3506 | OH2 | TIP | S | 106 | 17.252 | -5.133 | -4.494 | 1.00 | 32.68 |
| ATOM | 3507 | OH2 | TIP | S | 107 | 2.117 | -2.804 | 7.971 | 1.00 | 23.84 |
| ATOM | 3508 | OH2 | TIP | S | 108 | 0.905 | 6.033 | 6.588 | 1.00 | 35.53 |
| ATOM | 3509 | OH2 | TIP | S | 109 | -1.809 | 2.221 | 7.805 | 1.00 | 26.71 |
| ATOM | 3510 | OH2 | TIP | S | 110 | 31.327 | 4.490 | 21.257 | 1.00 | 27.43 |
| ATOM | 3511 | OH2 | TIP | S | 111 | 24.899 | 15.120 | 20.380 | 1.00 | 23.02 |
| ATOM | 3512 | OH2 | TIP | S | 112 | 20.579 | -16.631 | 28.270 | 1.00 | 36.51 |
| ATOM | 3513 | OH2 | TIP | S | 113 | 25.411 | -17.113 | 23.758 | 1.00 | 21.37 |
| ATOM | 3514 | OH2 | TIP | S | 114 | 1.515 | -27.409 | -0.458 | 1.00 | 19.14 |
| ATOM | 3515 | OH2 | TIP | S | 115 | 9.018 | -0.538 | -7.783 | 1.00 | 33.68 |
| ATOM | 3516 | OH2 | TIP | S | 116 | 9.636 | 26.386 | 20.024 | 1.00 | 50.24 |
| ATOM | 3517 | OH2 | TIP | S | 117 | 12.045 | 35.049 | 15.300 | 1.00 | 56.83 |
| ATOM | 3518 | OH2 | TIP | S | 118 | -3.599 | 28.704 | 5.505 | 1.00 | 33.39 |
| ATOM | 3519 | OH2 | TIP | S | 119 | 29.917 | -8.775 | 11.714 | 1.00 | 37.22 |
| ATOM | 3520 | OH2 | TIP | S | 120 | 24.137 | -3.898 | 25.140 | 1.00 | 34.55 |
| ATOM | 3521 | OH2 | TIP | S | 121 | 26.629 | -27.184 | 16.790 | 1.00 | 35.24 |
| ATOM | 3522 | OH2 | TIP | S | 122 | 27.014 | -4.417 | -6.003 | 1.00 | 30.58 |
| ATOM | 3523 | OH2 | TIP | S | 123 | 3.109 | -4.246 | 18.828 | 1.00 | 17.61 |
| ATOM | 3524 | OH2 | TIP | S | 124 | -3.599 | -2.056 | 28.229 | 1.00 | 41.01 |
| ATOM | 3525 | OH2 | TIP | S | 125 | 10.085 | -7.737 | 15.326 | 1.00 | 26.21 |
| ATOM | 3526 | OH2 | TIP | S | 126 | -1.000 | -2.978 | 1.801 | 1.00 | 29.55 |
| ATOM | 3527 | OH2 | TIP | S | 127 | 13.264 | 8.314 | 25.059 | 1.00 | 48.01 |
| ATOM | 3528 | OH2 | TIP | S | 128 | 20.011 | -18.478 | 30.197 | 1.00 | 39.67 |
| ATOM | 3529 | OH2 | TIP | S | 129 | 13.173 | -12.360 | 29.536 | 1.00 | 35.49 |
| ATOM | 3530 | OH2 | TIP | S | 130 | 20.797 | -16.810 | 0.544 | 1.00 | 40.49 |
| ATOM | 3531 | OH2 | TIP | S | 131 | -1.893 | -30.512 | -0.755 | 1.00 | 42.21 |
| ATOM | 3532 | OH2 | TIP | S | 132 | 28.312 | -26.251 | 15.235 | 1.00 | 29.77 |
| ATOM | 3533 | OH2 | TIP | S | 133 | 29.069 | 19.628 | 2.346 | 1.00 | 37.15 |
| ATOM | 3534 | OH2 | TIP | S | 134 | 18.835 | -22.021 | 3.787 | 1.00 | 52.90 |
| ATOM | 3535 | OH2 | TIP | S | 135 | 11.980 | -25.129 | -11.870 | 1.00 | 31.15 |
| ATOM | 3536 | OH2 | TIP | S | 136 | 25.969 | 5.485 | 12.281 | 1.00 | 22.04 |
| ATOM | 3537 | OH2 | TIP | S | 137 | 16.632 | -23.687 | 3.049 | 1.00 | 46.76 |
| ATOM | 3538 | OH2 | TIP | S | 138 | 18.173 | -21.460 | 0.580 | 1.00 | 40.88 |
| ATOM | 3539 | OH2 | TIP | S | 139 | 5.688 | -1.702 | 4.816 | 1.00 | 47.98 |
| ATOM | 3540 | OH2 | TIP | S | 140 | 22.648 | 3.275 | 0.933 | 1.00 | 29.92 |
| ATOM | 3541 | OH2 | TIP | S | 141 | 14.618 | -14.383 | 28.653 | 1.00 | 40.28 |

Figure 3 (suite 48)